(19) Europäisches Patentamt
European Patent Office
Office européen des brevets

(11) EP 4 431 511 A1

(12) **EUROPEAN PATENT APPLICATION**
published in accordance with Art. 153(4) EPC

(43) Date of publication:
**18.09.2024 Bulletin 2024/38**

(21) Application number: **22891988.2**

(22) Date of filing: **08.11.2022**

(51) International Patent Classification (IPC):
***C07D 487/14*** $^{(2006.01)}$ ***C07D 471/14*** $^{(2006.01)}$
***A61K 31/519*** $^{(2006.01)}$ ***A61P 35/00*** $^{(2006.01)}$

(52) Cooperative Patent Classification (CPC):
**A61K 31/519; A61P 35/00; C07D 471/14; C07D 487/14**

(86) International application number:
**PCT/CN2022/130700**

(87) International publication number:
**WO 2023/083194 (19.05.2023 Gazette 2023/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **09.11.2021 CN 202111322616**
**06.04.2022 CN 202210360177**

(71) Applicant: **Hangzhou Glubio Pharmaceutical Co. Ltd.**
**Hangzhou, Zhejiang 310018 (CN)**

(72) Inventors:
- **FU, Liqiang**
  **Shanghai 201210 (CN)**
- **KONG, Linglong**
  **Shanghai 201210 (CN)**
- **LU, Gang**
  **San Diego, California 92121 (US)**
- **XIA, Yifeng**
  **San Diego, California 92121 (US)**
- **LU, Chin-Chun**
  **San Diego, California 92121 (US)**

(74) Representative: **Cabinet Laurent & Charras**
**Le Contemporain**
**50 Chemin de la Bruyère**
**69574 Dardilly Cedex (FR)**

# (54) WEE1 PROTEIN KINASE DEGRADATION AGENT AND USE THEREOF

(57) A compound for degrading a Wee1 protein kinase or a pharmaceutically acceptable salt thereof, and the use thereof in the treatment of proliferative diseases.

EP 4 431 511 A1

## Description

### FIELD OF THE PRESENT APPLICATION

The present application belongs to the field of medicine. The invention provides compounds that degrade Weel protein kinase or pharmaceutically acceptable salts thereof, and uses thereof in the treatment of proliferative disorders. The invention also provides pharmaceutical compositions comprising a compound described herein or a salt thereof, and a pharmaceutically acceptable carrier.

### BACKGROUND

Cell cycle refers to the entire process of cell division, which is divided into interphase composed of DNA synthesis prophase ($G_1$ phase), DNA synthesis phase (S phase) and DNA synthesis anaphase ($G_2$ phase), and mitosis (M phase) composed of prophase, prometaphase, metaphase, anaphase, and telophase. Cell cycle checkpoint is a key point that regulates the cell cycleand ensures that every event of the cell cycle (such as DNA replication and chromosome segregation) is completed in an orderly and quality manner. The main cell cycle checkpoints are: 1) the $G_1$/S checkpoint, which checks and controls cells from the stationary $G_1$ phase to the DNA synthesis phase; 2) the S-phase checkpoint, which checks whether DNA replication is completed; 3) the $G_2$/M checkpoint, which checks and controls cells entering mitosis; 4) the Mid-anaphase checkpoint, which checks whether the spindle is properly assembled. The checkpoints will promptly sense and initiate repair when there are abnormalities such as DNA damage in certain processes of the cell cycle.

Damaged DNA is usually repaired at the $G_1$/S checkpoint. P53 protein can repair DNA damage and prevent cells from entering S phase. Accumulation of irreversible DNA damage results in p53-dependent cell apoptosis. In normal cells, p53 protein is maintained at a low level. However, loss-of-funciton mutations in p53, which frequently occur in tumor cells, make $G_1$/S checkpoint defective.

Thus, in p53 mutated tumor cells, cell cycle regulation (e.g. arresting the cell cycle or eliminating cells) relies on the $G_2$/M checkpoint. Weel protein kinase is a key regulator for arresting the $G_2$/M phase. Weel protein kinase is highly expressed in many cancer types. Inhibition or down-regulation of Weel protein kinase can lead to "mitotic catastrophe", resulting in the apoptosis of tumor cells. The Weel kinase inhibitor AZD1775 has shown certain clinical activities in the treatment of tumors. However, there are still deficiencies such as low cell activity, large clinical toxicity and side effects, and poor patient tolerance.

The ubiquitin-proteasome system (UPS) is a major protein degradation system with high specificity and selectivity. The proteolysis targeting chimera (PROTAC) refers to a compound composed of a ligand of a protein of interest (POI), a ligand of an E3 ubiquitin ligase, and a linker connecting the two ligands. In other words, one end of the PROTAC molecule can bind to a POI, and the other end can bind to an E3 ubiquitin ligase, thus brining an E3 ubiquitin ligase in close proximity to a POI. This allows the E3 ubiquitin ligase targetted by the PROTAC molecule to "tag" multiple ubiquitin proteins to the POI through a process called polyubiquitination. Next, the tagged POI (polyubiquitinated POI) is recognized and degraded by the proteasome. This process does not require the POI ligand to occupy the binding site for a long time. A PROTAC molecule can rapidly induce the ubiquitination of the POI only by briefly occupying the POI site and E3 ubiquitin ligase site at the same time. Moreover, PROTAC can be recycled after the POI is degraded by the proteasome.

However, when compared to the drug-like properties of small-molecule inhibitors, many PROTAC drugs in general have several disadvantages including poor water solubility, poor membrane permeability, poor metabolic stability, low oral bioavailability, etc.

The proteolytic degradation of Weel can effectively blocks downstream signaling. The removal of Weel protein can favorably eliminate Weel kinase activity and any protein interaction or skeletal structure function associated with Weel, providing wide therapeutic applications for the treatment of tumors.

The compound or a pharmaceutically acceptable salt thereof of the invention can effectively degrade Weel protein kinase, and showed improved water solubility, membrane permeability, metabolic stability and oral bioavailability.

### SUMMARY OF THE INVENTION

The present invention provides proteolysis targeting chimera. The first embodiment of the present invention provides a compound represented by structural formula (I), or a derivative, pharmaceutically acceptable salt, isomer, solvate, hydrates adduct, complex or prodrug thereof:

W-L-D (I),

wherein:

W is a Weel kinase binding ligand with the following structure:

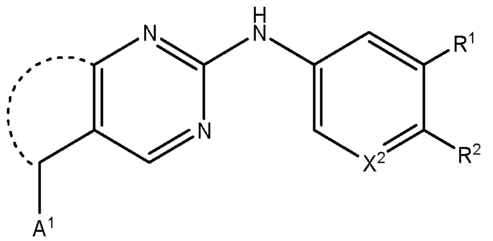

(I-W),

wherein:

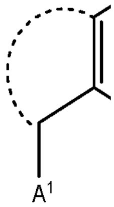

represents an optionally substituted cyclic group;

$A^1$ is selected from O=, halogen, hydroxyl, nitro, cyano, amino, mercapto, $C_1$-$C_6$ alkyl, $C_1$-$C_6$ alkoxy, (mono- and di-$C_1$-$C_6$ alkylamino) $C_0$-$C_4$ alkyl;

$X^2$, for each occurrence, is independently selected from $CR^{2'}$ or N;

$R^{2'}$, for each occurrence, is independently selected from absence, hydrogen, deuterium, halogen, hydroxyl, carbonyl, nitro, cyano, amino, mercapto, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono-and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^2$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^1$ and $R^2$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl;

any one of $R^1$, $R^2$, $R^1$ and $R^2$ together or the ring systems formed by $R^1$ and $R^2$ together is linked to L groups;

L is a linker and has the following structure;

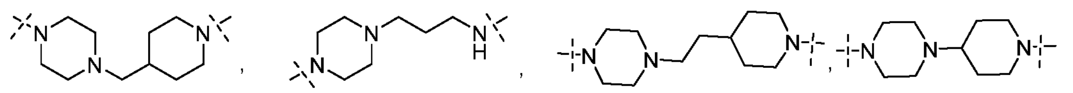

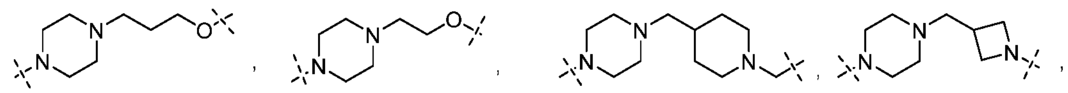

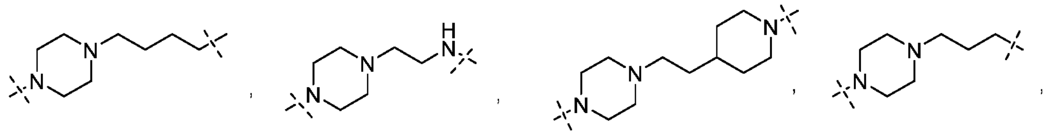

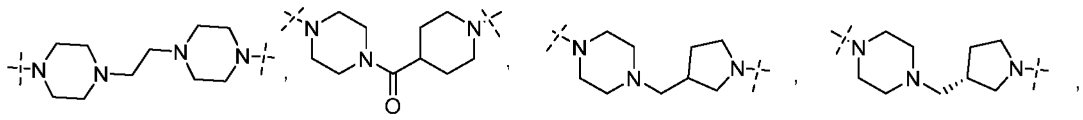

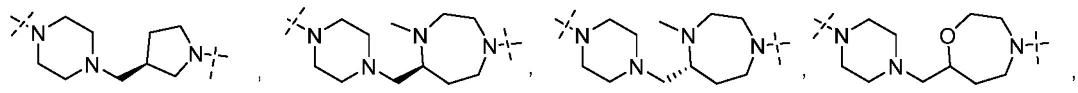

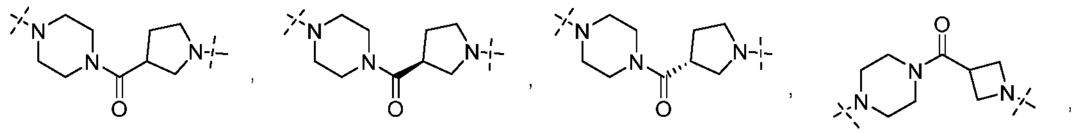

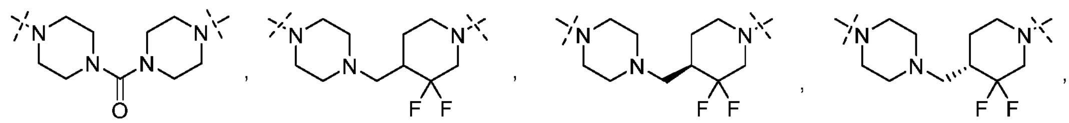

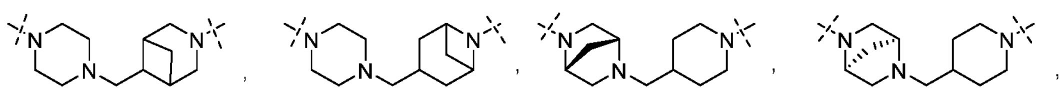

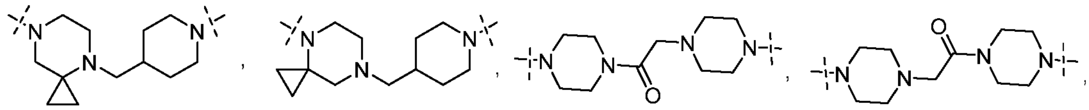

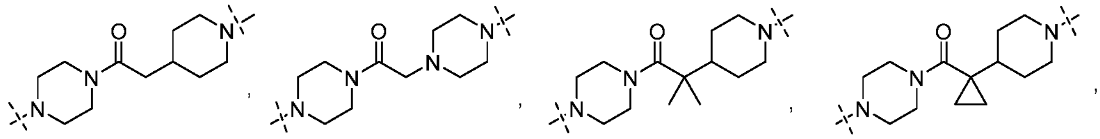

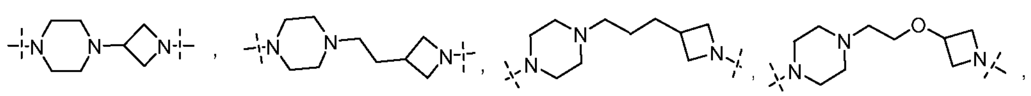

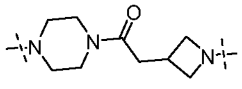, 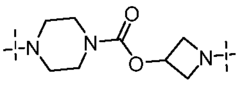, 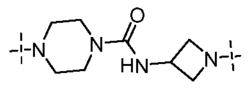, 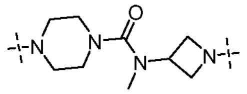,

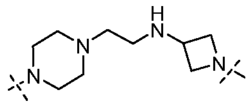, 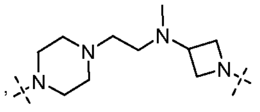, 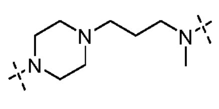, 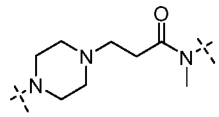,

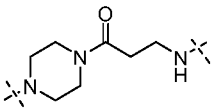, 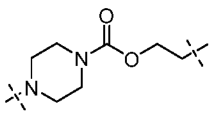, 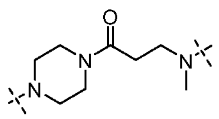, 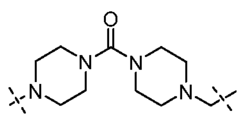,

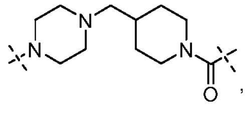, 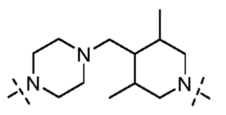, 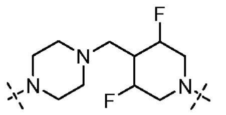, 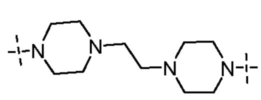,

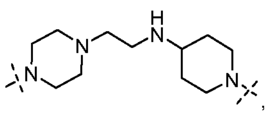, 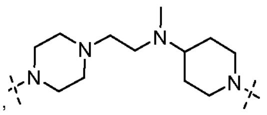, 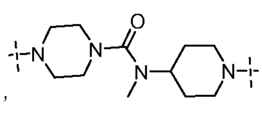, 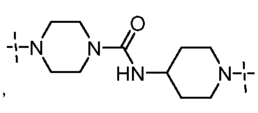,

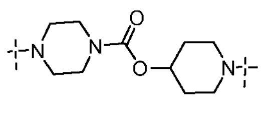, 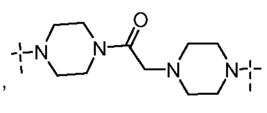, 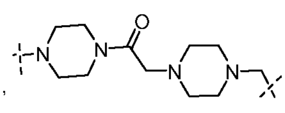, 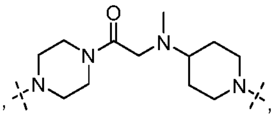,

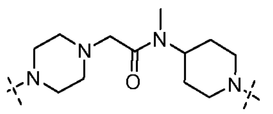, 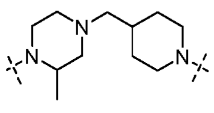, 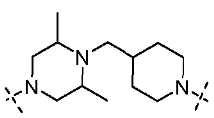, 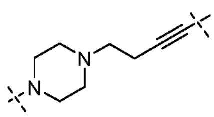,

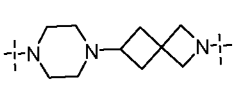, 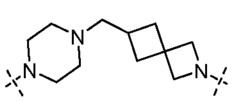, 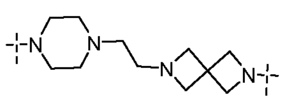, 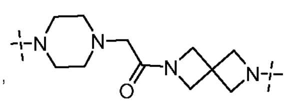,

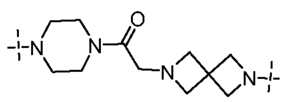, 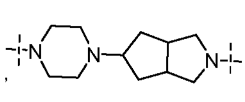, 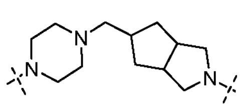, 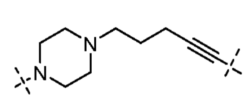,

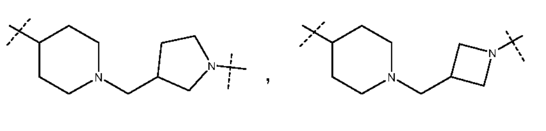, 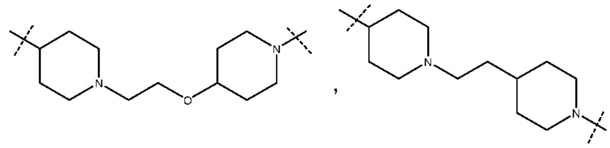,

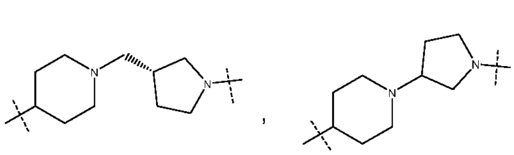, 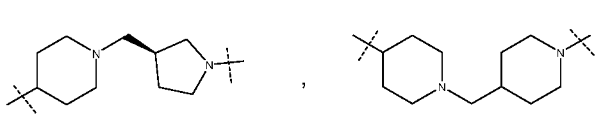,

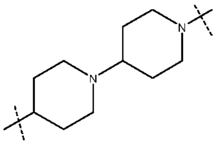 , 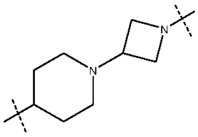 , 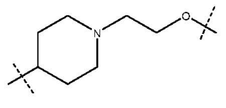 , 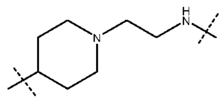

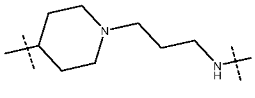 , 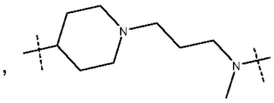 , 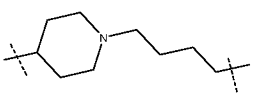 , 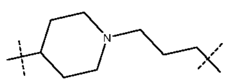 ,

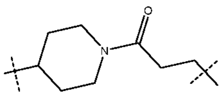 , 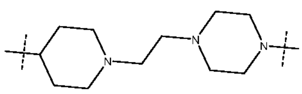 , 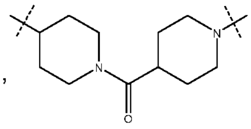 , 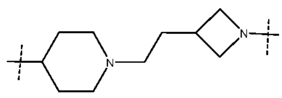 ,

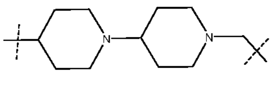 , 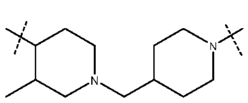 , 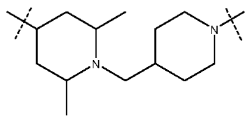 , 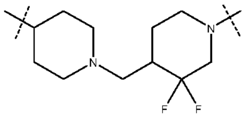 ,

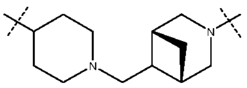 , 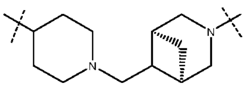 , 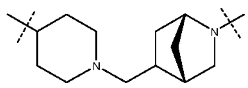 , 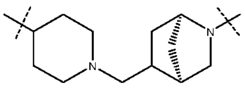 ,

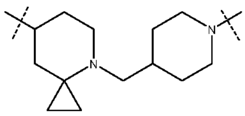 , 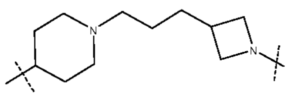 , 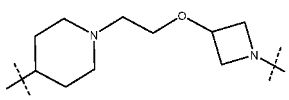 , 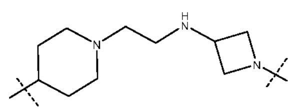

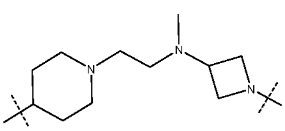 , 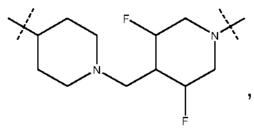 , 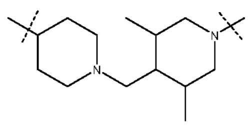 , 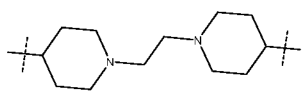 ,

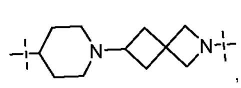 , 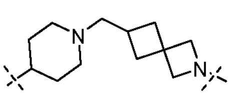 , 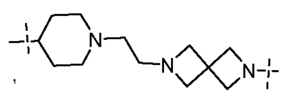 , 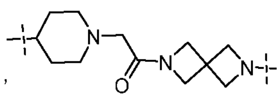 ,

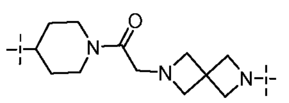 , 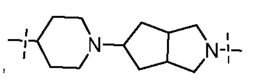 , 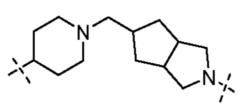 ,

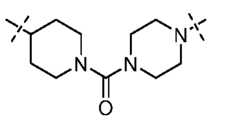 , 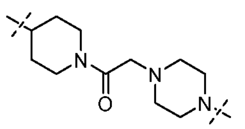 , 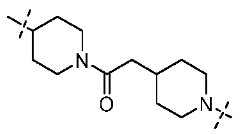 , 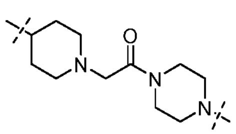 ,

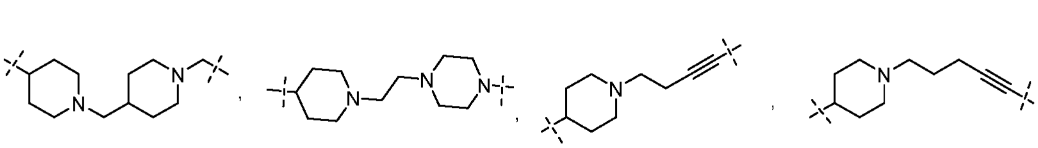 , , , ,

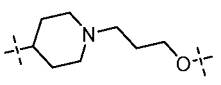 , 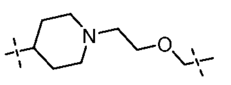 , 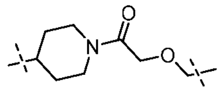 , 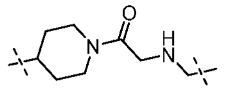 ,

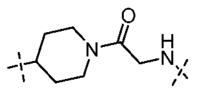 , 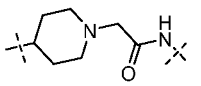 , 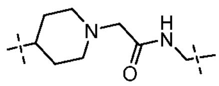 , 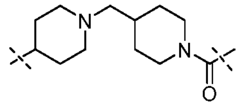 ,

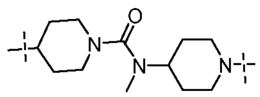 , 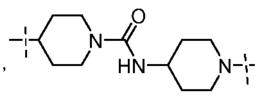 , 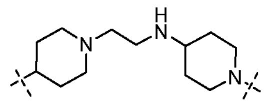 , 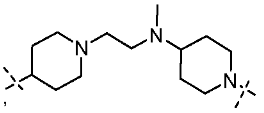 ,

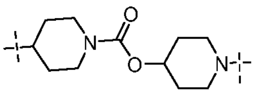 , 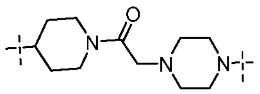 , 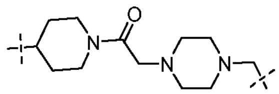 , 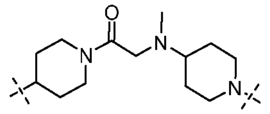 ,

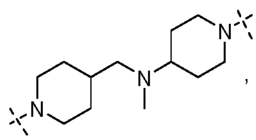 , 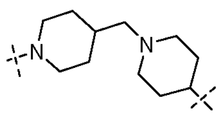 , 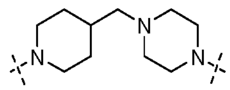 , 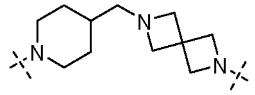 ,

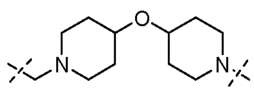 , 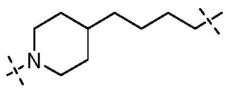 , 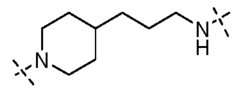 , 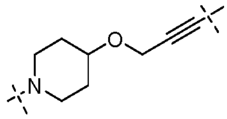 ,

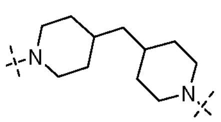 , 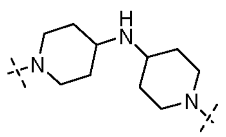 , 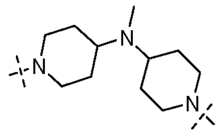 , 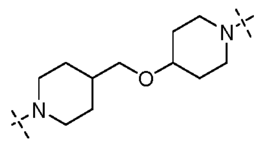 ,

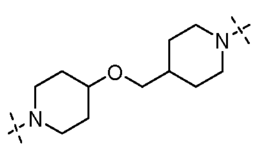 , 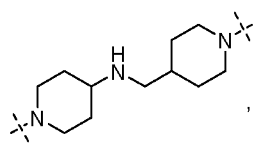 , 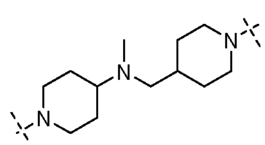 , 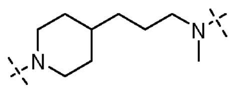 ,

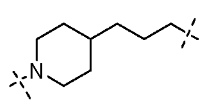 , 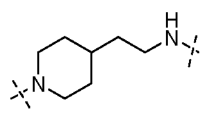 , 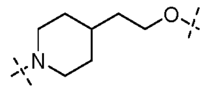 , 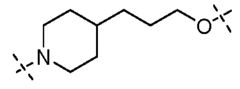 ,

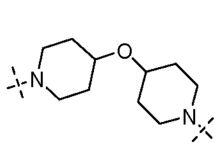 , 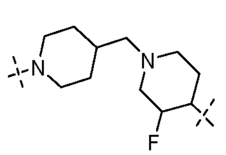 , 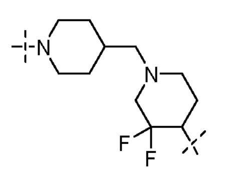 , 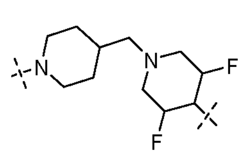

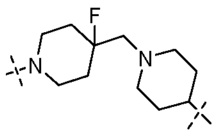 , 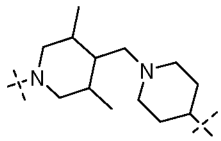 , 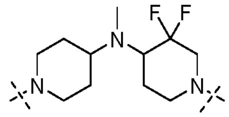 ,

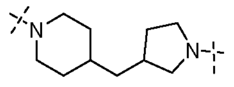 , 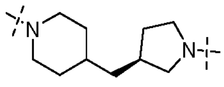 , 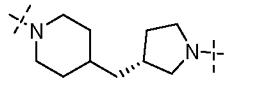 , 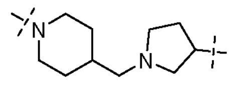

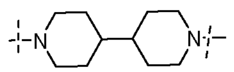 , 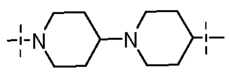 , 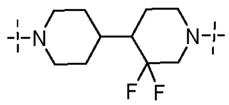 , 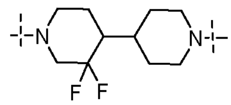

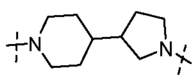 , 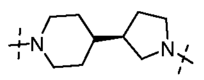 , 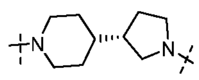 , 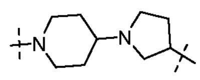 ,

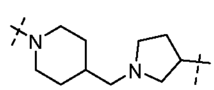 , 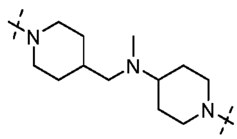 , 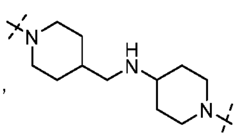 ,

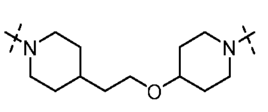 , 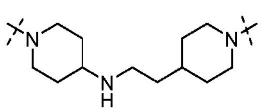 , 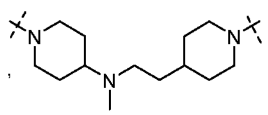 , 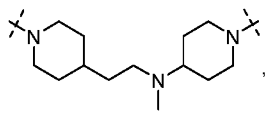 ,

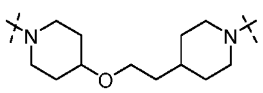 , 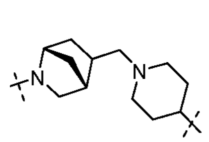 , 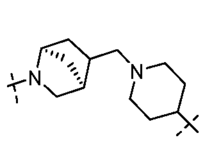 , 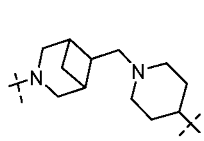 ,

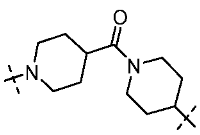 , 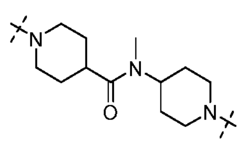 , 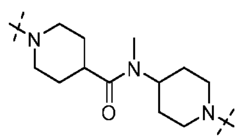 ,

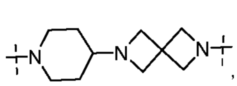 , 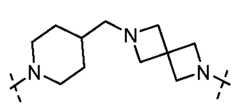 , 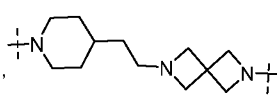 , 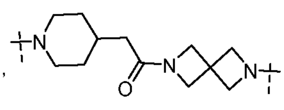 ,

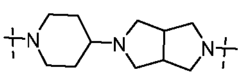 , 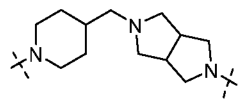 ,

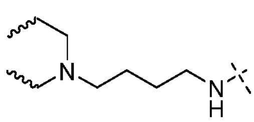 , 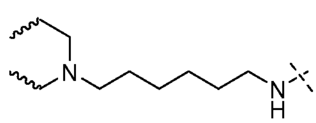 , 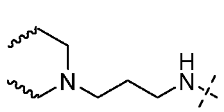 , 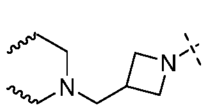 ,

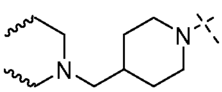 , 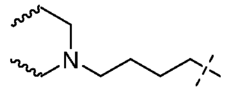 , 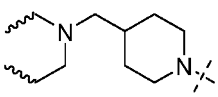 , 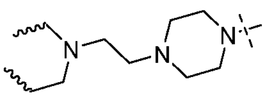 ,

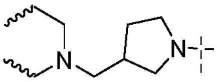 , 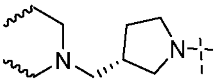 , 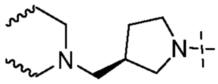 , 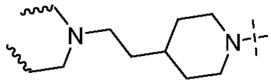 ,

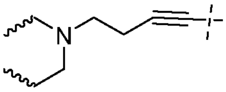 , 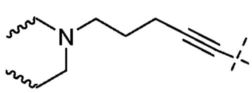 , 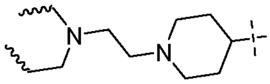 , 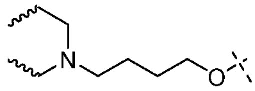 ,

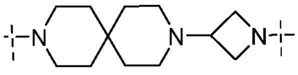 , 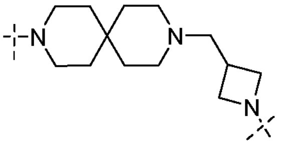 , 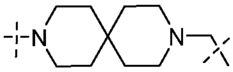 , 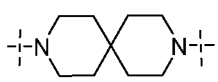 ,

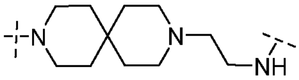 , 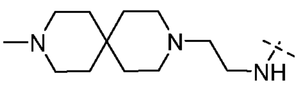 , 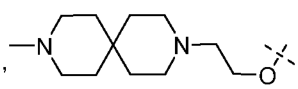 ,

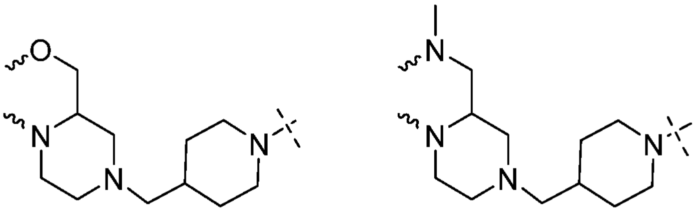 ,

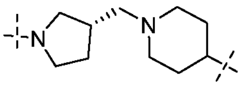 , 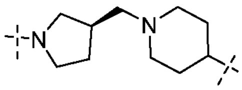 , 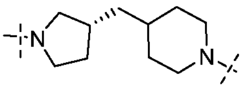 , 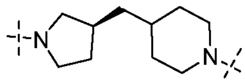 ,

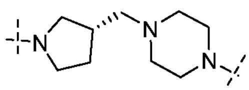 , 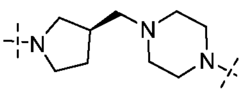 , 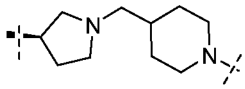 , 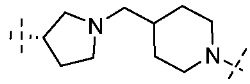 ,

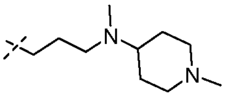 , 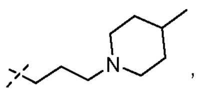 , 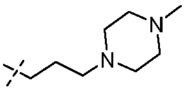 , 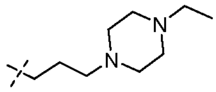 , 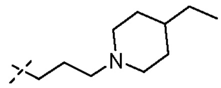 ,

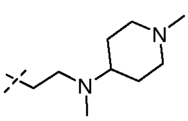 , 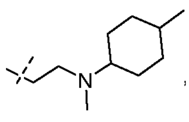 , 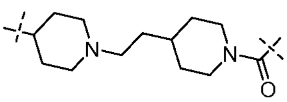 , 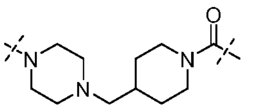 ,

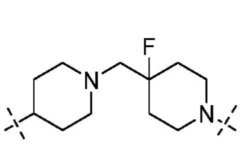 , 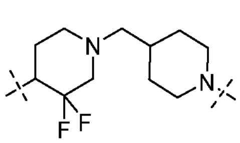 , 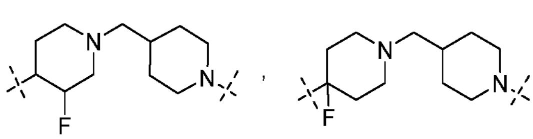

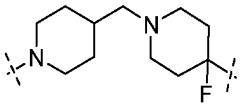, 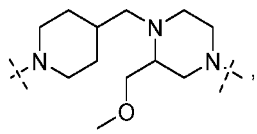, 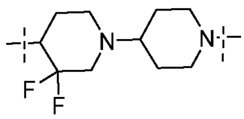, 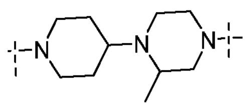,

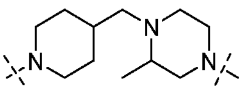, 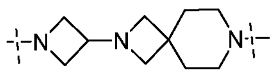, 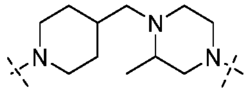, 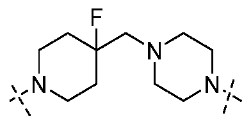,

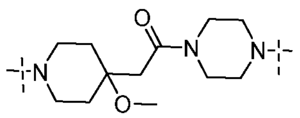, 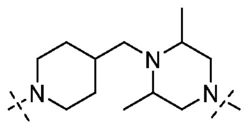, 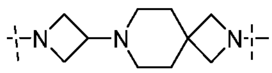, 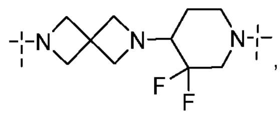,

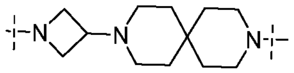, 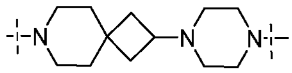, 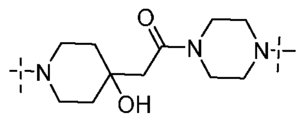, 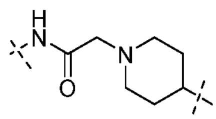,

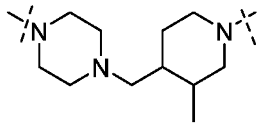, 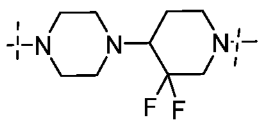, 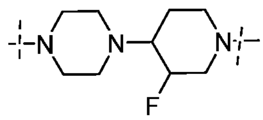, 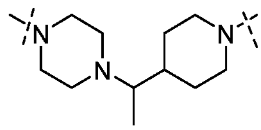,

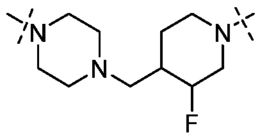, 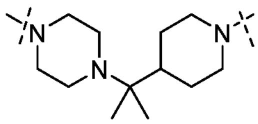, 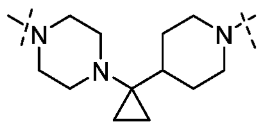, 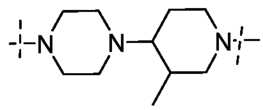,

wherein

 or 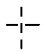

represents a linking site;

D is E3 ubiquitin ligase binding ligand with the following structure,

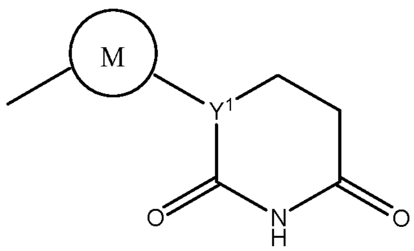 (I-D),

wherein

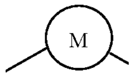

represents an optionally substituted cyclic group;

$Y^1$, for each occurrence, is independently selected from $CT^1$ or N; $T^1$ is selected from hydrogen or deuterium;

L is linked to W and/or D by one or more bonds.

In a second aspect of the first embodiment, W is:

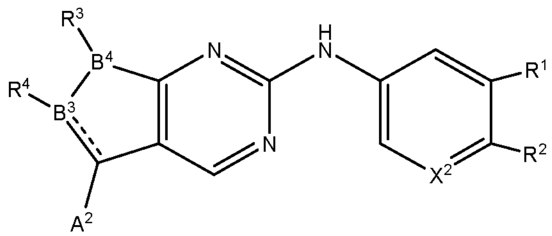

(I-W-II),

wherein:

$A^2$ is selected from O=, halogen, hydroxyl, nitro, cyano, amino, mercapto, $C_1$-$C_6$ alkyl, $C_1$-$C_6$ alkoxy, (mono- and di-$C_1$-$C_6$ alkylamino) $C_0$-$C_4$ alkyl;

$B^3$, for each occurrence, is independently selected from C, CH, CD or N;

$B^4$, for each occurrence, is independently selected from C, CH, CD or N; or

$B^3$-$B^4$ is -C=C-;

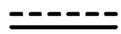

represents a single bond or a double bond;

$X^2$, for each occurrence, is independently selected from $CR^{2'}$ or N;

any one of $R^{2'}$, for each occurrence, is independently selected from absence, hydrogen, deuterium, halogen, hydroxyl, carbonyl, nitro, cyano, amino, mercapto, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^2$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^1$ and $R^2$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl;

any one of $R^1$, $R^2$, $R^1$ and $R^2$ together or the ring systems formed by $R^1$ and $R^2$ together is linked to L groups;

$R^3$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^4$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^3$ and $R^4$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl.

In a third aspect of the first embodiment, W is:

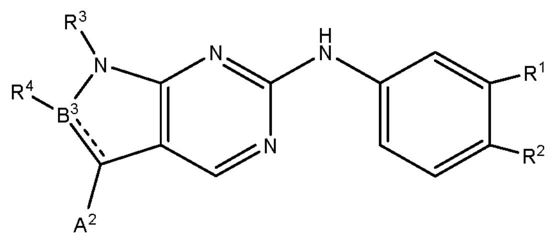

(I-W-III)

$A^2$ is selected from O=, halogen, hydroxyl, nitro, cyano, amino, mercapto, $C_1$-$C_6$ alkyl, $C_1$-$C_6$ alkoxy, (mono- and di-$C_1$-$C_6$ alkylamino) $C_0$-$C_4$ alkyl;

$B^3$, for each occurrence, is independently selected from C, CH, CD or N; alternatively, $R^4B^3$-$NR^3$ is -N=N-;

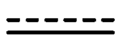

represents a single bond or a double bond;

$R^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^2$, for each occurrence, is independently selected from absence, hydrogen, deuterium, halogen, hydroxyl, carbonyl,

nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^1$ and $R^2$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl;

any one of $R^1$, $R^2$, $R^1$ and $R^2$ together or the ring systems formed by $R^1$ and $R^2$ together is linked to L groups;

$R^3$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^4$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^3$ and $R^4$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl.

In a fourth aspect of the first embodiment, W is:

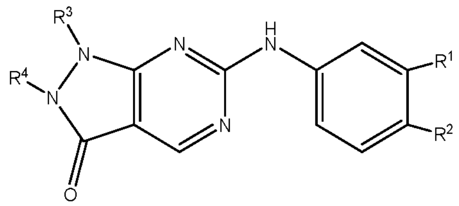

(I-W-IV),

wherein

$R^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^2$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy,

$C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^1$ and $R^2$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl;

any one of $R^1$, $R^2$, $R^1$ and $R^2$ together or the ring systems formed by $R^1$ and $R^2$ together is linked to L groups;

$R^3$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^4$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^3$ and $R^4$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl; or -($R^4$N-N$R^3$)- is -N=N-.

In a fifth aspect of the first embodiment, W is:

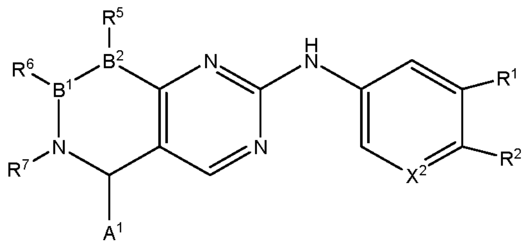

(I-W-V),

wherein

$A^1$ is selected from O=, halogen, hydroxyl, nitro, cyano, amino, mercapto, $C_1$-$C_6$ alkyl, $C_1$-$C_6$ alkoxy, (mono- and di-$C_1$-$C_6$ alkylamino) $C_0$-$C_4$ alkyl;

$B^1$, for each occurrence, is independently selected from C, CH, CD or N;

$B^2$, for each occurrence, is independently selected from C, CH, CD or N; or

$B^1$-$B^2$ represents -(C=C)-, -(N=C)- or-(C =N)-;

$X^2$, for each occurrence, is independently selected from $CR^{2'}$ or N;

$R^{2'}$, for each occurrence, is independently selected from absence, hydrogen, deuterium, halogen, hydroxyl, carbonyl, nitro, cyano, amino, mercapto, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^2$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^1$ and $R^2$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl;

any one of $R^1$, $R^2$, $R^1$ and $R^2$ together or the ring systems formed by $R^1$ and $R^2$ together is linked to L groups;

$R^5$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^6$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^7$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

two of $R^5$, $R^6$ and $R^7$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3, or 4

halogens, hydroxyl, carbonyl, nitro, cyano, amino, mercapto, -COOH, or $C_1$-$C_6$ alkyl.

In a sixth aspect of the first embodiment, W is:

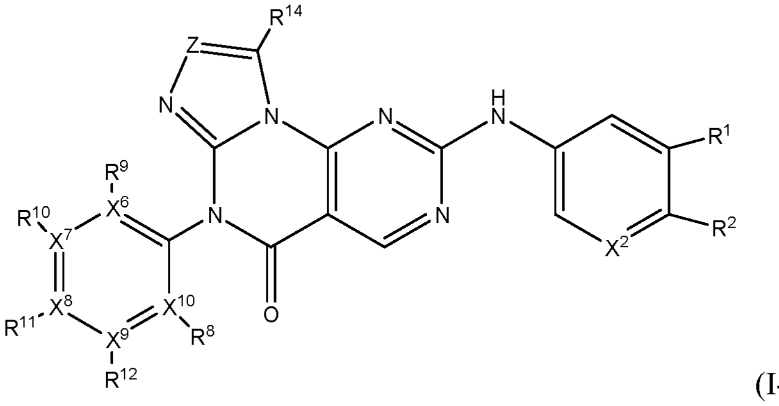

(I-W-VI),

wherein

$X^2$, for each occurrence, is independently selected from $CR^{2'}$ or N;

$R^{2'}$, for each occurrence, is independently selected from absence, hydrogen, deuterium, halogen, hydroxyl, carbonyl, nitro, cyano, amino, mercapto, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^2$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^1$ and $R^2$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl;

any one of $R^1$, $R^2$, $R^1$ and $R^2$ together or the ring systems formed by $R^1$ and $R^2$ together is linked to L groups;

$X^6$, for each occurrence, is independently selected from C or N;

$X^7$, for each occurrence, is independently selected from C or N;

$X^8$, for each occurrence, is independently selected from C or N;

$X^9$, for each occurrence, is independently selected from C or N;

$X^{10}$, for each occurrence, is independently selected from C or N;

any one of $R^8$, $R^9$, $R^{10}$, $R^{11}$, $R^{12}$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

Z, for each occurrence, is independently selected from $CR^{13'}$ or N;

$R^{13'}$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^{14}$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl.

In a seventh aspect of the first embodiment, W is:

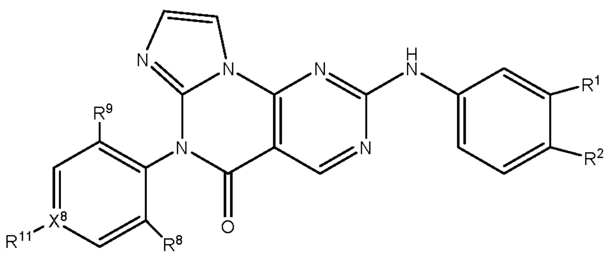

(I-W-VII),

wherein

$R^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^2$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro,

cyano, amino, mercapto, -COOH, or $C_1$-$C_6$ alkyl; or

$R^1$ and $R^2$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl;

any one of $R^1$, $R^2$, $R^1$ and $R^2$ together or the ring systems formed by $R^1$ and $R^2$ together is linked to L groups;

$X^8$, for each occurrence, is independently selected from C or N;

$R^8$, $R^9$, $R^{11}$, for each occurrence, are independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$alkyl, -$C_0$-$C_4$alkyl ($C_3$-$C_7$cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl.

Preferably, W is:

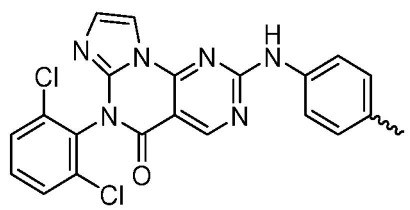

.

In a eighth aspect of the first embodiment, W is:

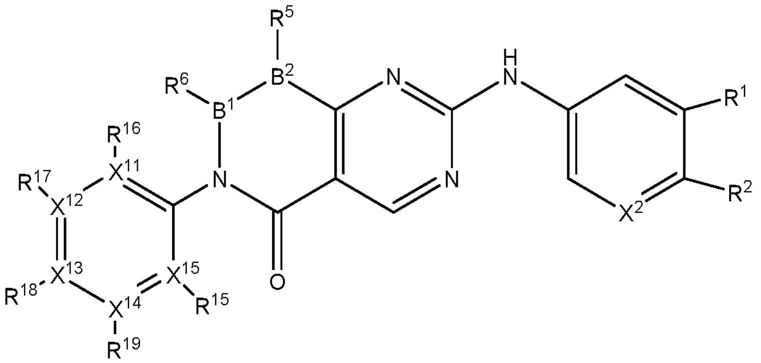

(I-W-X),

$B^1$, for each occurrence, is independently selected from C, CH, CD or N;

$B^2$, for each occurrence, is independently selected from C, CH, CD or N; or

$B^1$-$B^2$ represents -(C=C)-, -(N=C)-, -(C=N)-,-N=N-;

$X^2$, for each occurrence, is independently selected from $CR^{2'}$ or N;

$R^{2'}$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl,

heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^2$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^1$ and $R^2$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl;

any one of $R^1$, $R^2$, $R^1$ and $R^2$ together or the ring systems formed by $R^1$ and $R^2$ together is linked to L groups;

$R^5$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^6$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^5$ and $R^6$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl;

$X^{11}$, for each occurrence, is independently selected from C or N;

$X^{12}$, for each occurrence, is independently selected from C or N;

$X^{13}$, for each occurrence, is independently selected from C or N;

$X^{14}$, for each occurrence, is independently selected from C or N;

$X^{15}$, for each occurrence, is independently selected from C or N;

any one of $R^{15}$, $R^{16}$, $R^{17}$, $R^{18}$, $R^{19}$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl.

In a ninth aspect of the first embodiment, W is:

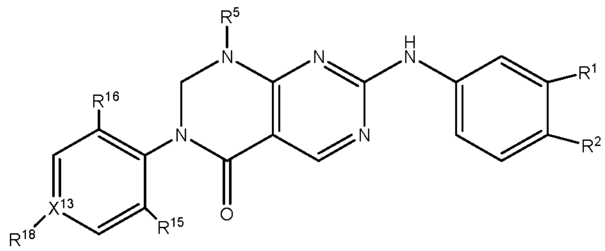

(I-W-XI)

$R^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^2$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^1$ and $R^2$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl;

any one of $R^1$, $R^2$, $R^1$ and $R^2$ together or the ring systems formed by $R^1$ and $R^2$ together is linked to L groups;

$R^5$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$X^{13}$, for each occurrence, is independently selected from C or N;

any one of $R^{15}$, $R^{16}$, and $R^{18}$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl,

nitro, cyano, amino, mercapto, -COOH, or $C_1$-$C_6$ alkyl.

In a tenth aspect of the first embodiment, W is:

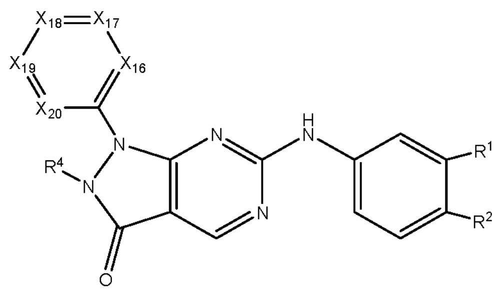

(I-W-XIII),

wherein

$R^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^2$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^1$ and $R^2$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl;

any one of $R^1$, $R^2$, $R^1$ and $R^2$ together or the ring systems formed by $R^1$ and $R^2$ together is linked to L groups;

$R^4$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$X^{16}$, for each occurrence, is independently selected from CH, CD, $CR^{21}$ or N;

$X^{17}$, for each occurrence, is independently selected from CH, CD, $CR^{22}$ or N;

$X^{18}$, for each occurrence, is independently selected from CH, CD, $CR^{23}$ or N;

$X^{19}$, for each occurrence, is independently selected from CH, CD, $CR^{24}$ or N;

$X^{20}$, for each occurrence, is independently selected from CH, CD, $CR^{25}$ or N;

any one of $R^{21}$, $R^{22}$, $R^{23}$, $R^{24}$, $R^{25}$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl.

In a eleventh aspect of the first embodiment, W is:

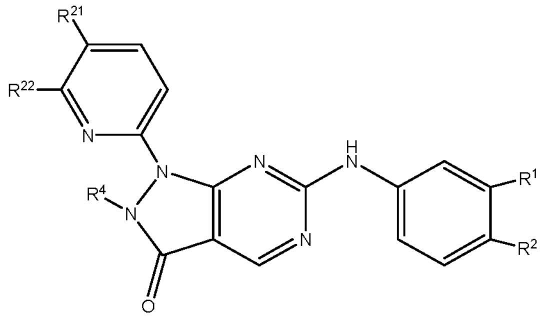

(I-W-XIV)

$R^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^2$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^1$ and $R^2$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl;

any one of $R^1$, $R^2$, $R^1$ and $R^2$ together or the ring systems formed by $R^1$ and $R^2$ together is linked to L groups;

$R^4$, for each occurrence, is independently selected from absence, hydrogen, deuterium, halogen, hydroxyl, carbonyl, nitro, cyano, amino, mercapto, allyl, propenyl, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

any one of $R^{21}$ and $R^{22}$, for each occurrence, is independently selected from absence, hydrogen, deuterium, halogen, hydroxyl, carbonyl, nitro, cyano, amino, mercapto, allyl, propenyl, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$

alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, $C_5$-$C_{10}$ heteroaryl, wherein the cycloalkyl, heterocyclyl, aryl and heteroaryl are optionally substituted with 1, 2, 3 or 4 halogens, hydroxyl, carbonyl, nitro, cyano, amino, mercapto, -COOH, or $C_1$-$C_6$ alkyl optionally substituted with substituents selected from $C_1$-$C_4$alkyl, cyclopropyl, halogen, hydroxyl, carbonyl, nitro; or,

$R^{21}$, $R^{22}$ are joined together with the connected ring atoms to form a five-membered or six-membered ring having 0, 1 or 2 heteroatoms selected from N, O or S, wherein the five-membered or six-membered ring is optionally substituted with substituents selected from $C_1$-$C_4$alkyl, cyclopropyl, halogen, hydroxyl, carbonyl, nitro.

In the twelfth aspect of the first embodiment, W is:

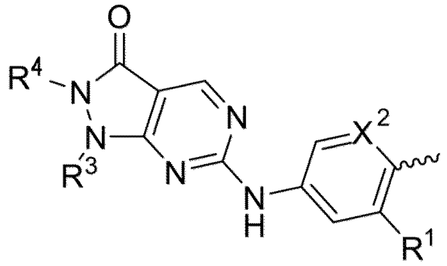

(I-W-XV)

wherein:

$X^2$ is CH or N;

$R^1$ is hydrogen, fluorine, chlorine, methyl, ethyl, methoxy, hydroxymethyl, or linked to L groups;

$R^4$ is selected from allyl, propargyl, methylene cyclopropyl, ethyl, trifluoroethyl, difluoroethyl, isopropyl, cyclopropyl, methylene $C_6$-$C_{12}$ aryl, methylene phenyl, $C_{1-4}$ alkyl, optionally substituted $C_{2-4}$ alkenyl, optionally substituted $C_{2-4}$ alkynyl, optionally substituted $C_{3-6}$ cycloalkyl and optionally substituted $C_{3-6}$ cycloalkyl ($C_{1-4}$ alkyl), wherein the $C_{1-4}$ alkyl, $C_{2-4}$ alkenyl and $C_{2-4}$ alkynyl are optionally independently substituted with one or more substituents selected from halogen, $C_{1-4}$ alkoxy, $C_{1-4}$ haloalkyl, $C_{1-4}$ haloalkoxy, cyano, amino, mono-$C_{1-4}$ alkylamine and di-$C_{1-4}$ alkylamine, and wherein one or more rings of the $C_{3-6}$ cycloalkyl and the $C_{3-6}$ cycloalkyl ($C_{1-4}$ alkyl) are optionally independently substituted with one or more substituents selected from halogen, $C_{1-4}$ alkyl, $C_{1-4}$ alkoxy, $C_{1-4}$ haloalkyl, $C_{1-4}$ haloalkoxy, cyano, amino, mono-$C_{1-4}$ alkylamine and di-$C_{1-4}$ alkylamine;

$R^3$ is selected from:

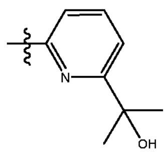 , 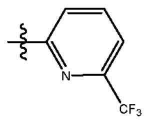 , 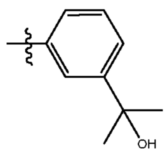 , 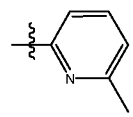 , 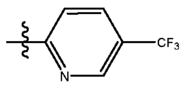 , 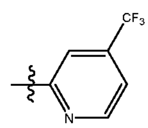 ,

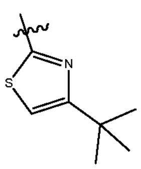 , 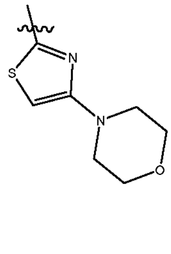 , 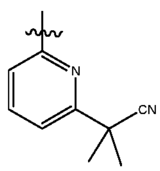 , 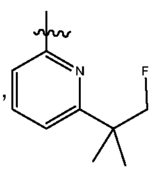 , 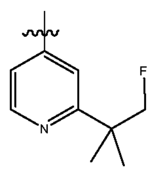 , 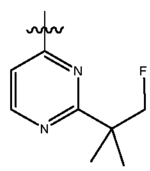 ,

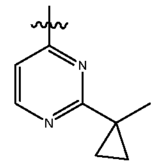, 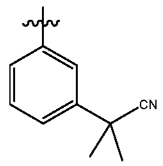, 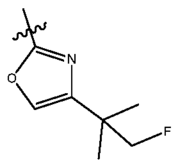, 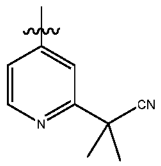, 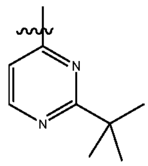, 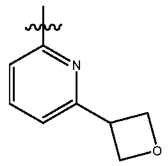,

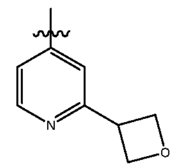, 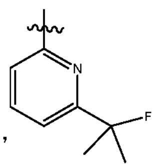, 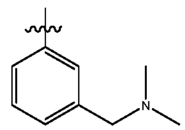, 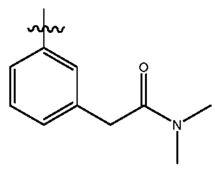, 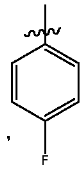, 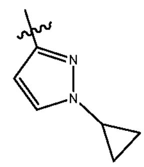,

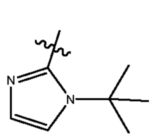, 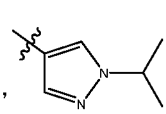, 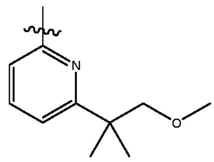, 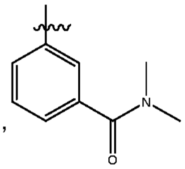, 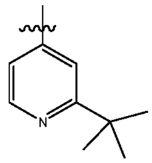, 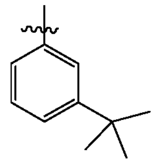,

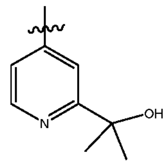, 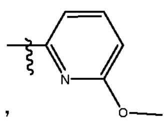, 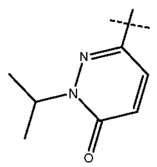

optionally substituted $C_6$-$C_{12}$ aryl or optionally substituted $C_5$-$C_{10}$ heteroaryl, wherein when the aryl or heteroaryl is substituted, the $C_6$-$C_{12}$ aryl and $C_5$-$C_{10}$ heteroaryl are independently substituted with one or more substituents, preferably fluorine, chlorine, methyl, ethyl, methoxy, hydroxymethyl, haloalkyl.

Preferably, W is:

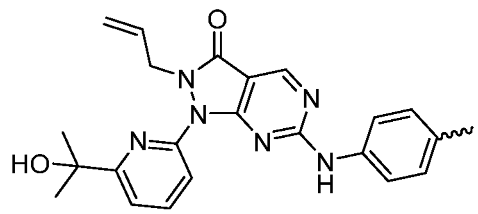

.

In a thirteenth aspect of the first embodiment, W is:

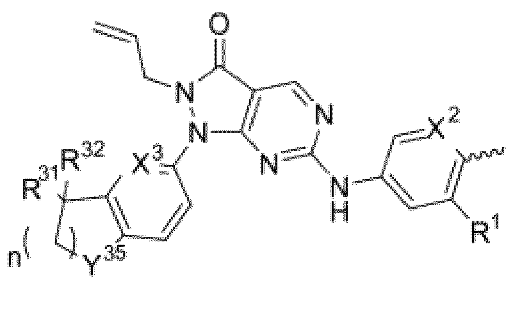

(I-W-XVI),

wherein

$X^2$ is CH or N;

$R^1$ is hydrogen, fluorine, chlorine, methyl, ethyl, methoxy, hydroxymethyl, or linked to L groups;

$R^{31}$ is hydrogen, hydroxyl, fluorine, amino, cyano;

$R^{32}$ is hydrogen, fluorine, methyl, ethyl, fluoroethyl, trifluoromethyl, difluoromethyl, cyclopropyl;

$R^{31}$ and $R^{32}$ are joined together with the connected ring atoms to form ;

$X^3$ is CH, O, N, $NR^{39}$, wherein $R^{39}$ is $C_{1-3}$ alkyl or cycloalkyl;

n is 1 or 2;

$Y^{35}$ is $CH_2$ or O.

Preferably, W is:

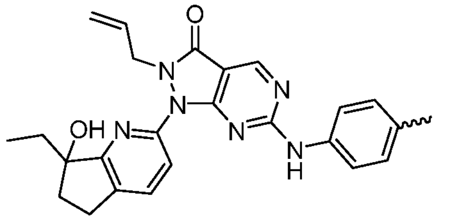

,

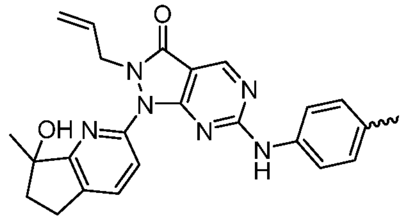

,

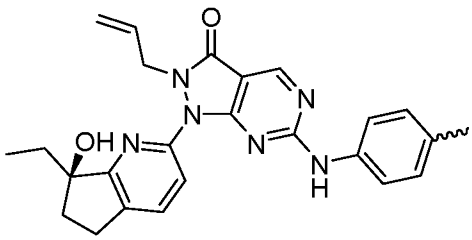

,

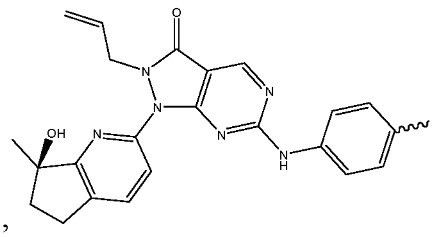

.

In a fourteenth aspect of the first embodiment, W is:

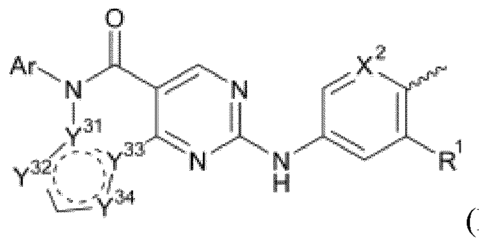

(I-W-XVII)

wherein:

$X^2$ is C or N;

$R^1$ is hydrogen, fluorine, chlorine, methyl, ethyl, methoxy, hydroxymethyl, or linked to L groups;

Ar is phenyl optionally substituted with substituents selected from halogen, methyl or methoxy, $C_5$-$C_{10}$ heteroaryl optionally substituted with substituents selected from halogen, methyl or methoxy;

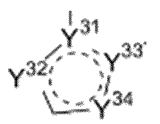

is a nitrogen-containing heteroaromatic ring selected from pyrrolyl, imidazolyl, pyrazolyl, triazolyl, or tetrazolyl.

In a fifteenth aspect of the first embodiment, W is:

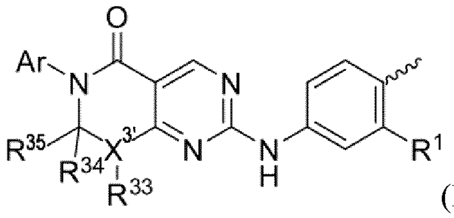

(I-W-XVIII),

wherein

Ar is $C_6$-$C_{12}$ aryl optionally substituted with substituents selected from halogen, methyl or methoxy, $C_5$-$C_{10}$ heteroaryl optionally substituted with substituents selected from halogen, methyl or methoxy;

$R^1$ is hydrogen, fluorine, chlorine, methyl, ethyl, methoxy, hydroxymethyl, or a linking L substituent;

$X^{3'}$is C, CH, CD, O or N;

$R^{33}$ is absent, hydrogen, methyl, fluorine, chlorine;

$R^{34}$ is absent, hydrogen, deuterium, hydroxyl;

$R^{35}$ is absent, hydrogen, deuterium, hydroxyl; or

$R^{34}$ and $R^{35}$ together form =O.

Preferably, W is:

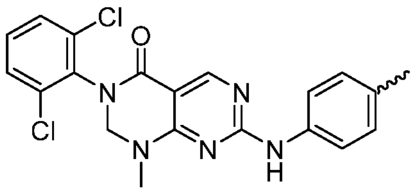

.

In a sixteenth aspect of the first embodiment, W is:

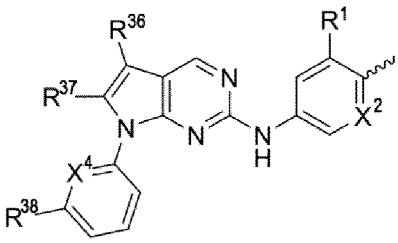

(I-W-XIX),

wherein

$X^2$ is CH orN;

$X^4$ is CH or N;

$R^1$ is selected from hydrogen, fluorine, chlorine, methyl, ethyl, methoxy, hydroxymethyl, or a linking L substituent;

$R_3^6$ is selected from hydrogen, fluorine, cyano, methyl;

$R_3^7$ is selected from hydrogen, -$C_{1-6}$ alkylene-OH, -$C_{1-6}$ alkylene-O-$C_{1-6}$ alkyl, -$C_{1-6}$ alkylene-$NR^aR^b$, -C(=O)$NR^cR^d$, -N=S(=O)($R^e$)($R^f$), -P(=O)($R^g$)($R^h$), -NH-P(=O)($R^i$)($R^j$), pyridonyl, or

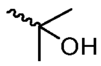,

wherein $R^a$, $R^b$, $R^c$, $R^d$, $R^e$, $R^f$, $R^g$, $R^h$, $R^i$, $R^j$, for each occurrence, are each independently selected from $C_{1-6}$ alkyl, $C_{3-5}$ cycloalkyl;

$R^{38}$ is selected from hydrogen, $C_{1-6}$ alkyl, $C_{3-8}$cycloalkyl, allyl, -$C_{1-6}$ alkylene-OH, or -$C_{1-6}$ alkylene-O-$C_{1-6}$ alkyl.

In a seventeenth aspect of the first embodiment, W is:

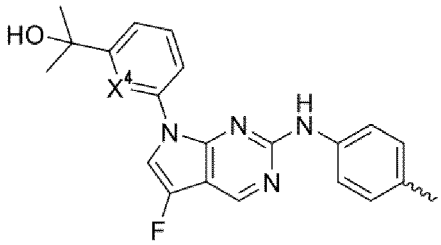
(I-W-XX),

wherein
$X^4$ is CH or N.
In an eighteenth aspect of the first embodiment, W is:

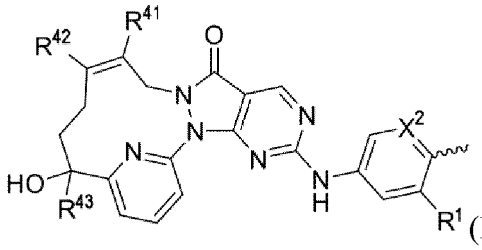
(I-W-XXI),

wherein:

$X^2$ is CH orN;

$R^1$ is selected from hydrogen, fluorine, chlorine, methyl, ethyl, methoxy, hydroxymethyl, or a linking L substituent;

$R^{41}$ is selected from hydrogen, fluorine, or methyl;

$R^{42}$ is selected from hydrogen, fluorine, or methyl;

$R^{43}$ is selected from methyl or ethyl;

Preferably, W is:

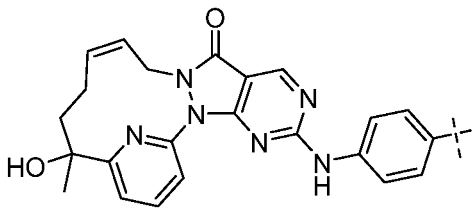
.

In a nineteenth aspect of the first embodiment, D is:

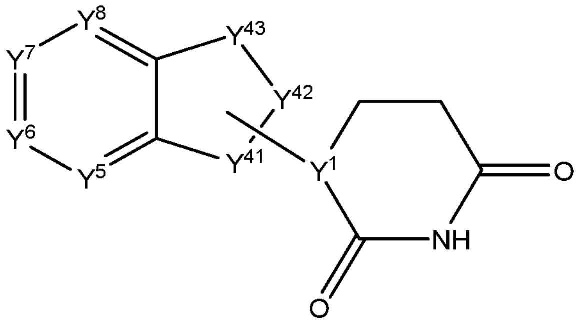

(I-D-II)

$Y^1$, for each occurrence, is independently selected from $CT^1$ or N;

$T^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$Y^{41}$, for each occurrence, is independently selected from C, $CT^{41}T^{41'}$, $NT^{41''}$, C=O, or O;

$Y^{42}$, for each occurrence, is independently selected from C, $CT^{42}T^{42'}$, $NT^{42''}$, C=O, or O;

$Y^{43}$, for each occurrence, is independently selected from C, $CT^{43}T^{43'}$, $NT^{43''}$, C=O, or O; or $Y^{41}$-$Y^{42}$ represents -$T^{41}$(C=C)$T^{42}$-, -(N=C)$T^{42}$-, -$T^{41}$(C=N)- or -(N=N)-, or $Y^{42}$-$Y^{43}$ represents-$T^{42}$(C=C)$T^{43}$-, -(N=C)$T^{43}$-, -$T^{42}$(C=N)- or-(N=N)-;

each of $T^{41}$, $T^{41'}$, $T^{41''}$, $T^{42}$, $T^{42'}$, $T^{42''}$, $T^{43}$, $T^{43}$, $T^{43''}$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$Y^5$, for each occurrence, is independently selected from $CT^5$ or N;

$Y^6$, for each occurrence, is independently selected from $CT^6$ or N;

$Y^7$, for each occurrence, is independently selected from $CT^7$ or N;

$Y^8$, for each occurrence, is independently selected from $CT^8$ or N;

each of $T^5$, $T^6$, $T^7$, $T^8$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy,

$C_2$-$C_6$ alkenyloxy, $C_2$-$C_6$ alkynyloxy, $C_2$-$C_6$ alkanoyl, $C_2$-$C_6$alkylester, $C_1$-$C_6$thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

one or more of $Y^5$, $Y^6$, $Y^7$ and $Y^8$ are linked to L groups.

In a twentieth aspect of the first embodiment, D is:

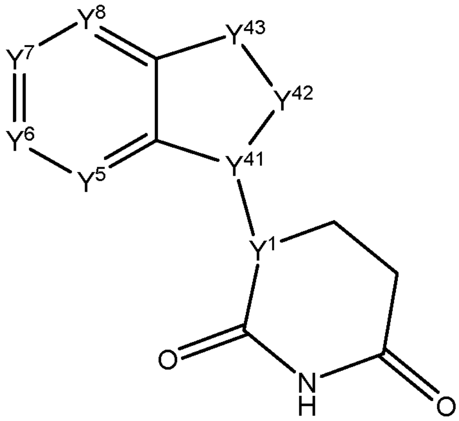
(I-D-III),

wherein

$Y^1$, for each occurrence, is independently selected from $CT^1$ or N;

$T^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$Y^{41}$, for each occurrence, is independently selected from $CT^{41}$ or N;

$Y^{42}$, for each occurrence, is independently selected from $CT^{42}T^{42'}$, $NT^{42'}$, C=O, or O;

$Y^{43}$, for each occurrence, is independently selected from $CT^{43}T^{43'}$, $NT^{43''}$, C=O, or O; or $Y^{41}$-$Y^{42}$ represents -(C=C)$T^{42}$-, -(C=N)-, or $Y^{42}$-$Y^{43}$ represents -$T^{42}$(C=C)$T^{43}$-, -(N=C)$T^{43}$-,-$T^{42}$(C=N)- or-(N=N)-;

each of $T^{41}$, $T^{42}$, $T^{42'}$, $T^{42''}$, $T^{43}$, $T^{43'}$, $T^{43''}$, for each occurrence, is independently selected from absence, hydrogen, deuterium, halogen, hydroxyl, carbonyl, nitro, cyano, amino, mercapto,-COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$Y^5$, for each occurrence, is independently selected from $CT^5$ or N;

$Y^6$, for each occurrence, is independently selected from $CT^6$ or N;

$Y^7$, for each occurrence, is independently selected from $CT^7$ or N;

$Y^8$, for each occurrence, is independently selected from $CT^8$ or N;

each of $T^5$, $T^6$, $T^7$, $T^8$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$alkylester, $C_1$-$C_6$thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

one or more of $Y^5$, $Y^6$, $Y^7$ and $Y^8$ are linked to L groups.

In a 21st aspect of the first embodiment, D is:

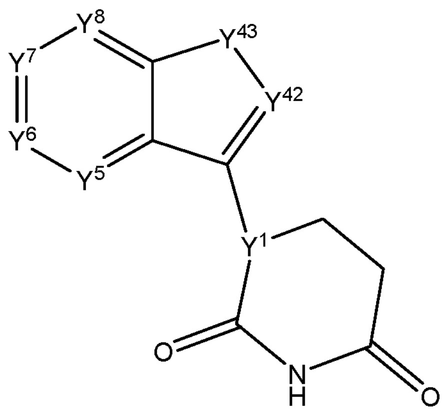

(I-D-IV),

wherein:

$Y^1$, for each occurrence, is independently selected from CH, CD or N;

$Y^{42}$, for each occurrence, is independently selected from CH or N;

$Y^{43}$, for each occurrence, is independently selected from $CH_2$, -NH, O or N-$CH_3$;

$Y^5$, for each occurrence, is independently selected from N; $CT^5$, wherein $T^5$, for each occurrence, is independently selected from absence, hydrogen, fluorine, chlorine, methyl, methoxy;

$Y^6$, for each occurrence, is independently selected from N; $CT^6$, wherein $T^6$, for each occurrence, is independently selected from absence, hydrogen, fluorine, chlorine, methyl, methoxy;

$Y^7$, for each occurrence, is independently selected from N; $CT^7$, wherein $T^7$, for each occurrence, is independently selected from absence, hydrogen, fluorine, chlorine, methyl, methoxy;

$Y^8$, for each occurrence, is independently selected from N; $CT^8$, wherein $T^8$, for each occurrence, is independently selected from absence, hydrogen, fluorine, chlorine, methyl, methoxy;

one or more of $Y^5$, $Y^6$, $Y^7$ and $Y^8$ are linked to L groups.

Preferably, D is:

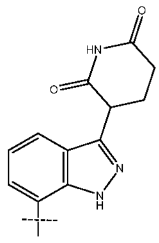 , 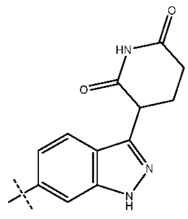 , 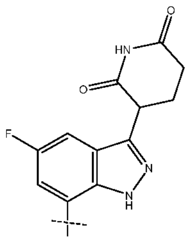 , 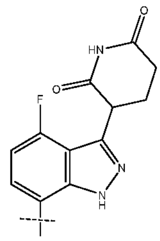 , 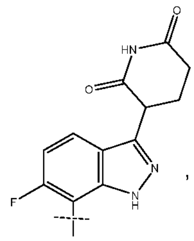 ,

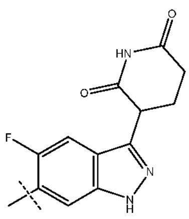 , 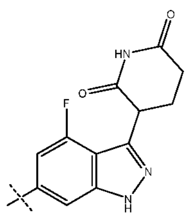 , 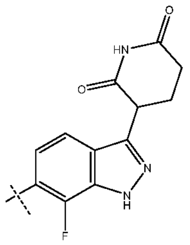 , 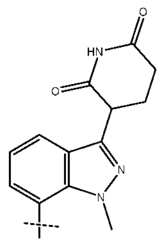 , 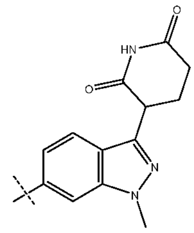 ,

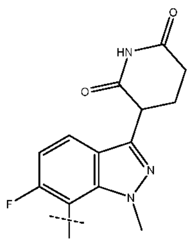 , 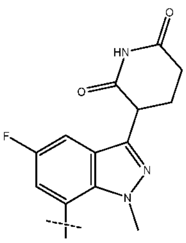 , 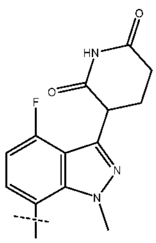 , 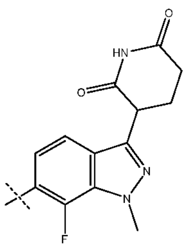 , 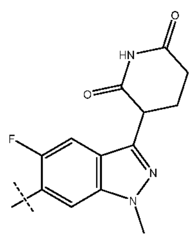 ,

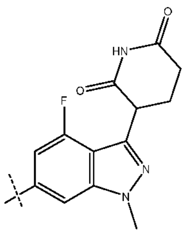 , 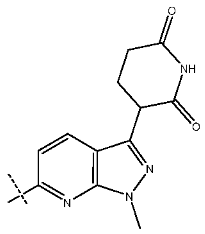 , 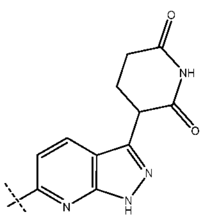 , 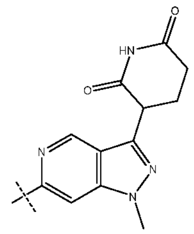 , 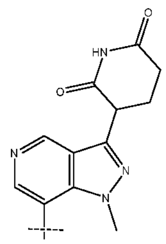 ,

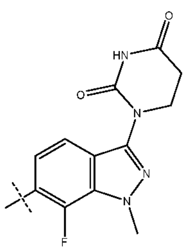 , 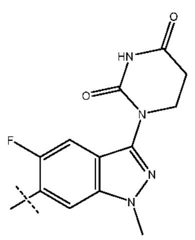 , 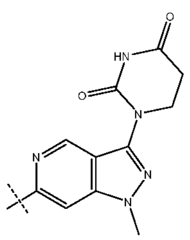 , 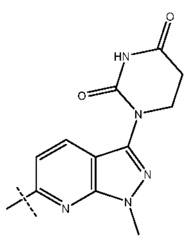 , 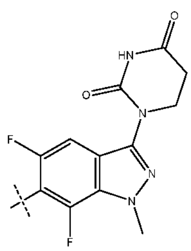 ,

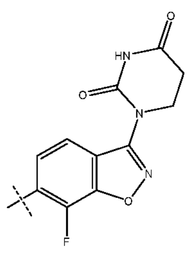 , 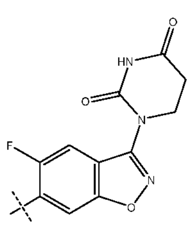

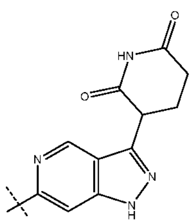 , 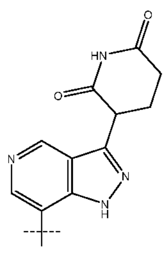 , 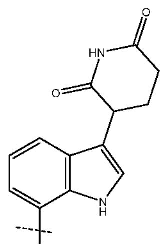 , 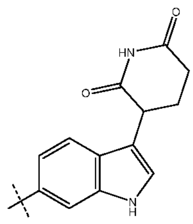 , 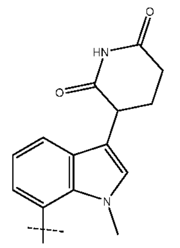 ,

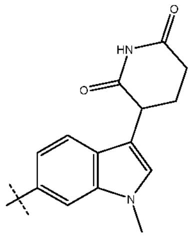 , 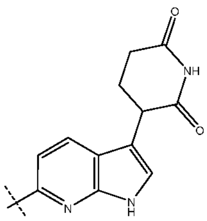 , 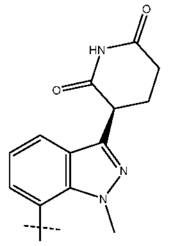 , 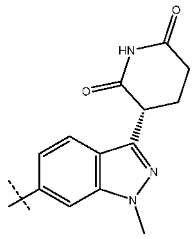 , 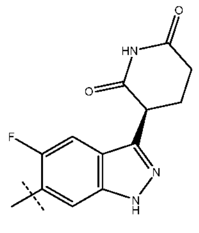 ,

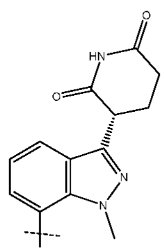 , 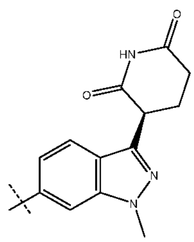 , 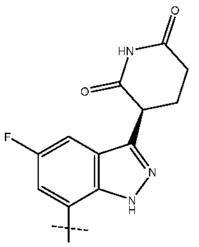 , 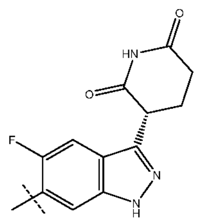 , 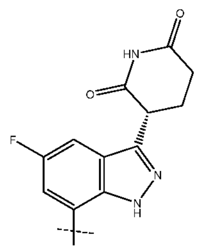 ,

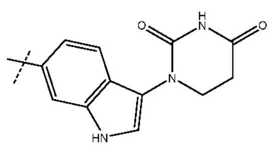 , 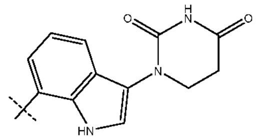 , 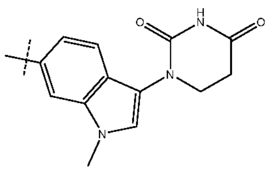 , 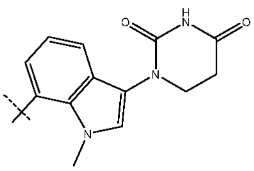 ,

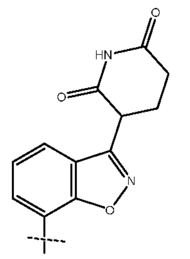 , 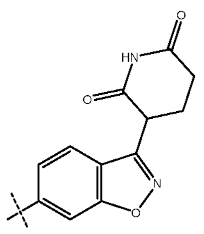 , 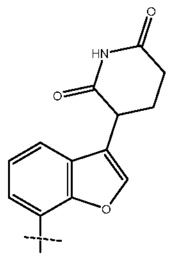 , 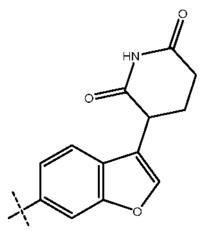 , 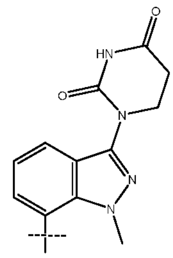 ,

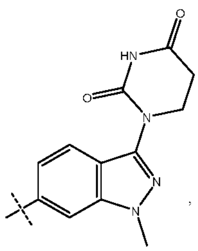 , 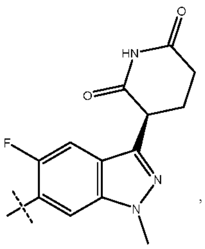 , 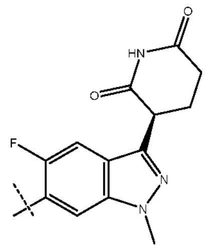 , 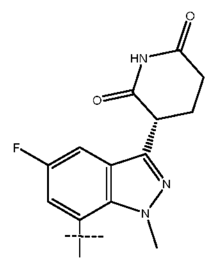 , 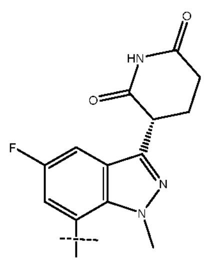

In a 22nd aspect of the first embodiment, D is:

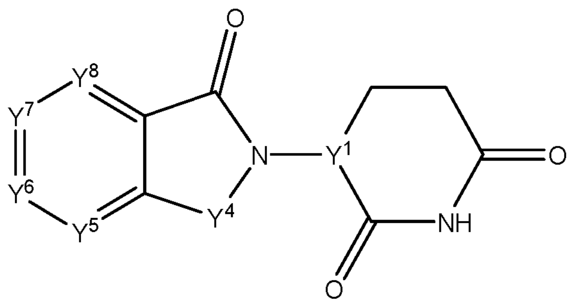

(I-D-V),

wherein

$Y^1$, for each occurrence, is independently selected from $CT^1$ or N;

$T^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$Y^4$, for each occurrence, is independently selected from $CT^4T^{4'}$ or $NT^{4''}$;

each of $T^4$, $T^{4'}$, $T^{4''}$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$alkylester, $C_1$-$C_6$thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$Y^5$, for each occurrence, is independently selected from $CT^5$ or N;

$Y^6$, for each occurrence, is independently selected from $CT^6$ or N;

$Y^7$, for each occurrence, is independently selected from $CT^7$ or N;

$Y^8$, for each occurrence, is independently selected from $CT^8$ or N;

each of $T^5$, $T^6$, $T^7$, $T^8$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$alkylester, $C_1$-$C_6$thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

one or more of $Y^5$, $Y^6$, $Y^7$ and $Y^8$ are linked to L groups.

In a 23rd aspect of the first embodiment, D is:

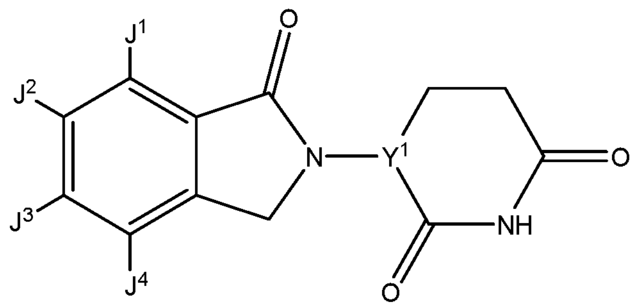

(I-D-VI),

wherein $Y^1$, for each occurrence, is independently selected from CH, CD or N;

$J^1$, for each occurrence, is independently selected from absence, hydrogen, fluorine, chlorine, methyl, methoxy;

$J^2$, for each occurrence, is independently selected from absence, hydrogen, fluorine, chlorine, methyl, methoxy;

$J^3$, for each occurrence, is independently selected from absence, hydrogen, fluorine, chlorine, methyl, methoxy;

$J^4$, for each occurrence, is independently selected from absence, hydrogen, fluorine, chlorine, methyl, methoxy;

one or more of $J^1$, $J^2$, $J^3$ and $J^4$ are linked to L groups.

Preferably, D is:

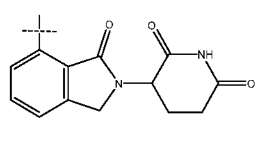 , 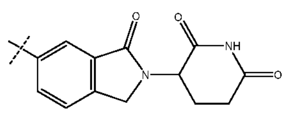 , 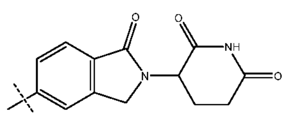 , 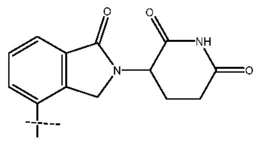 ,

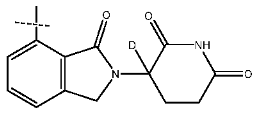 , 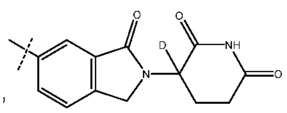 , 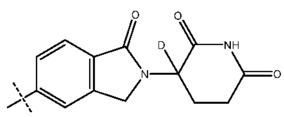 , 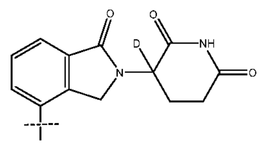 ,

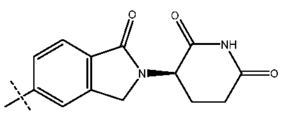 , 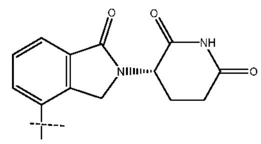 , 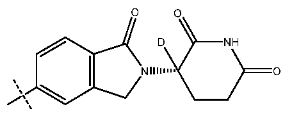 , 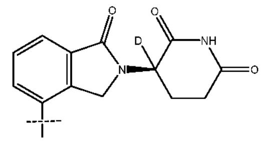 ,

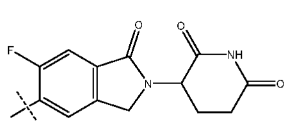 , 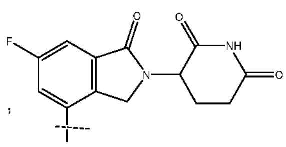

In a 24th aspect of the first embodiment, D is:

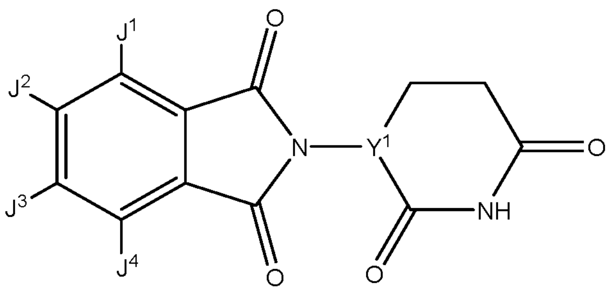

(I-D-VII),

wherein $Y^1$, for each occurrence, is independently selected from CH, CD or N;

$J^1$, for each occurrence, is independently selected from absence, hydrogen, fluorine, chlorine, methyl, methoxy;

$J^2$, for each occurrence, is independently selected from absence, hydrogen, fluorine, chlorine, methyl, methoxy;

$J^3$, for each occurrence, is independently selected from absence, hydrogen, fluorine, chlorine, methyl, methoxy;

$J^4$, for each occurrence, is independently selected from absence, hydrogen, fluorine, chlorine, methyl, methoxy;

one or more of $J^1$, $J^2$, $J^3$ and $J^4$ are linked to L groups.

Preferably, D is a group selected from:

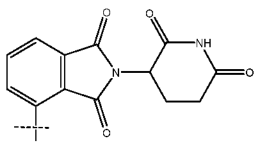 , 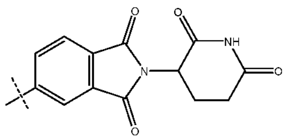 , 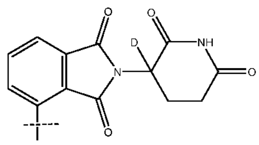 , 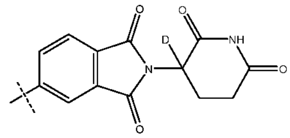 ,

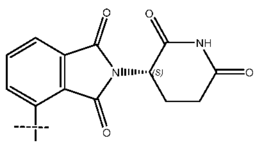 , 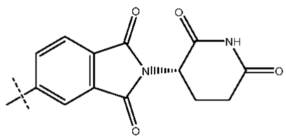 , 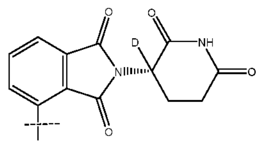 , 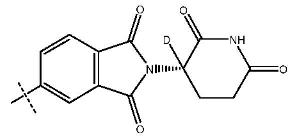 ,

.

In a 25th aspect of the first embodiment, D is:

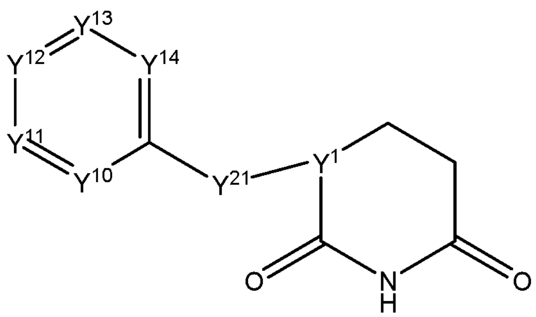

(I-D-VIII),

$Y^1$, for each occurrence, is independently selected from CH, CD or N;

$Y^{21}$ is independently selected from, NH, N-$CH_3$ or O;

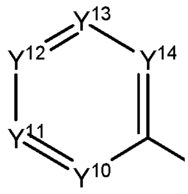

is an aromatic ring or a heteroaromatic ring, wherein

$Y^{10}$, for each occurrence, is independently selected from: absence; O; S; N; NH; $CT^{10}$, wherein $T^{10}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy; $NT^{10'}$, wherein $T^{10'}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;

$Y^{11}$, for each occurrence, is independently selected from absence; O; S; N; NH; $CT^{11}$, wherein $T^{11}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy; $NT^{11'}$, wherein $T^{11'}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;

$Y^{12}$, for each occurrence, is independently selected from absence; O; S; N; NH; $CT^{12}$, wherein $T^{12}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy; $NT^{12'}$ , wherein $T^{12'}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;

$Y^{13}$, for each occurrence, is independently selected from absence; O; S; N; NH; $CT^{13}$, wherein $T^{13}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy; $NT^{13'}$ , wherein $T^{13'}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;

$Y^{14}$, for each occurrence, is independently selected from absence; O; S; N; NH; $CT^{14}$, wherein $T^{14}$,for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy; $NT^{14'}$, wherein $T^{14'}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;

one or more of $Y^{10}$, $Y^{11}$, $Y^{12}$, $Y^{13}$ and $Y^{14}$ are linked to L groups; or

any two adjacent ones of $Y^{10}$, $Y^{11}$, $Y^{12}$, $Y^{13}$ and $Y^{14}$ are joined together with the connected atoms to form an optionally substituted aryl or an optionally substituted heteroaryl, which is linked to L groups by one or more bonds; preferably, the aryl or heteroaryl is selected from phenyl, naphthyl, anthracenyl, indenyl, indanyl, 1,2-dihydronaphthyl, 1,2,3,4-tetrahydronaphthyl, pyridyl, pyrimidinyl, imidazolyl, pyrazolyl, triazolyl, tetrazolyl, furanyl, thienyl, oxazolyl, isoxazolyl, thiazolyl, isothiazolyl, oxadiazolyl, thiadiazolyl, triazinyl, and the substituents thereon are selected from: absence, fluorine, chlorine, methyl, $CH_2F$, $CHF_2$, $CF_3$, $CH_2Cl$, $CHCl_2$, $CCl_3$, methoxy.

Preferably, D is a group selected from:

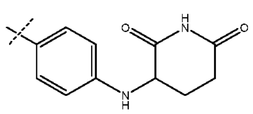 , 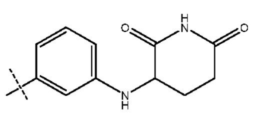 , 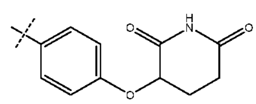 , 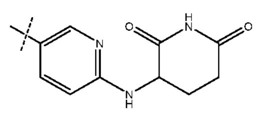 ,

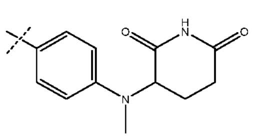 , 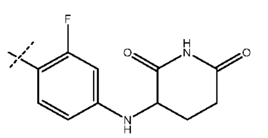 , 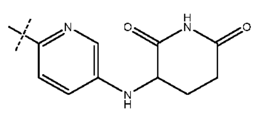 , 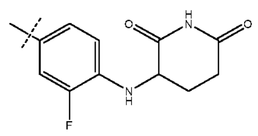 ,

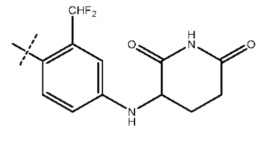 , 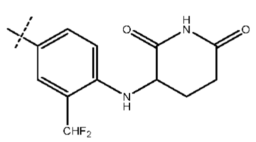 , 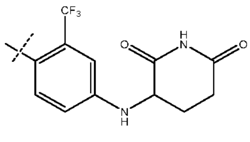 , 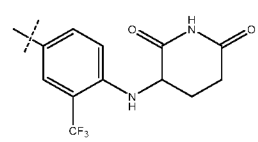 ,

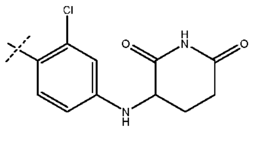 , 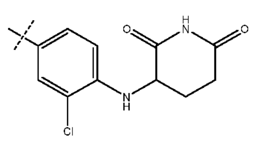 , 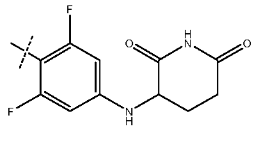 , 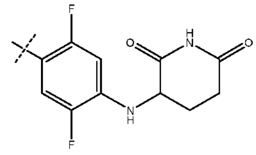 ,

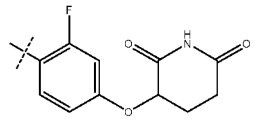 , 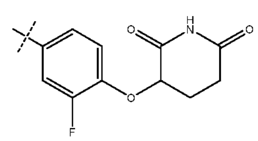 , 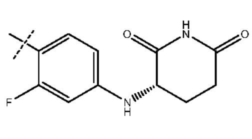 , 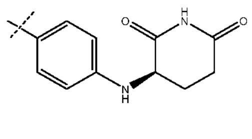 ,

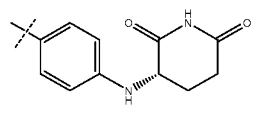 , 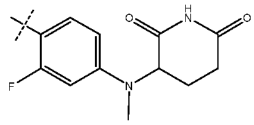 , 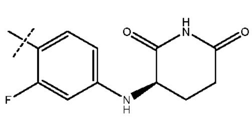 , 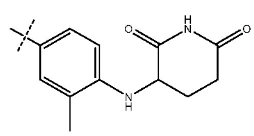 ,

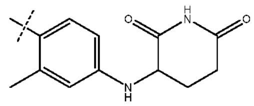 ,

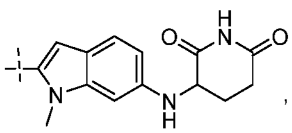 , 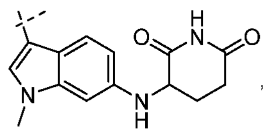 , 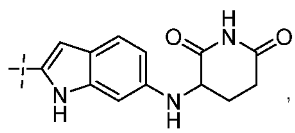 , 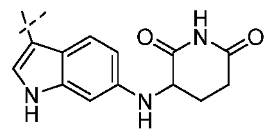 ,

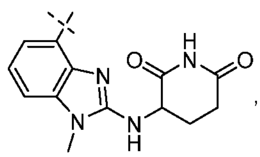 , 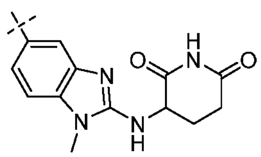 , 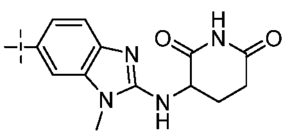 , 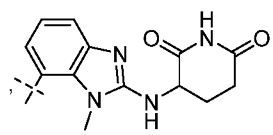 ,

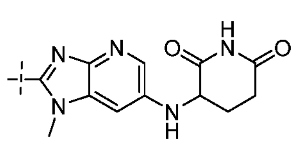 , 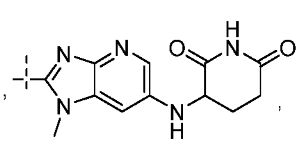 , 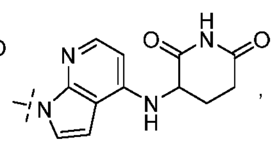 , 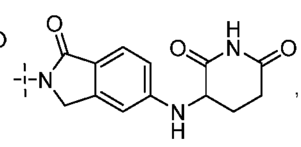 ,

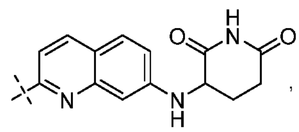 , 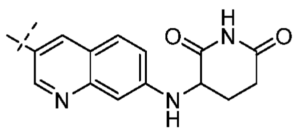 , 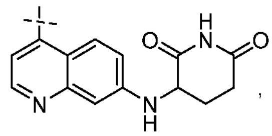 , 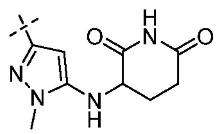 ,

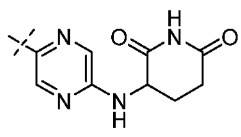 , 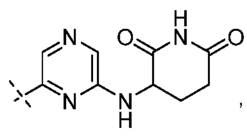 , 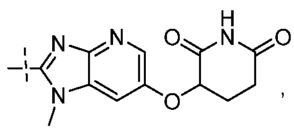 , 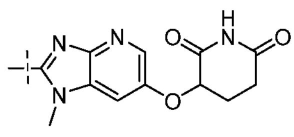 ,

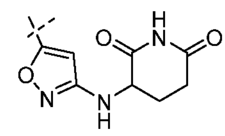 , 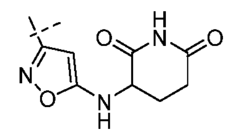 , 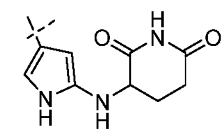 , 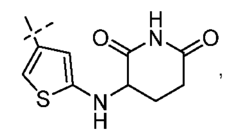 ,

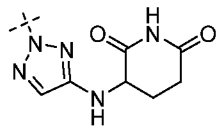 , 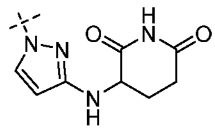 , 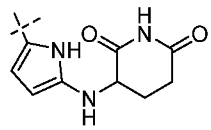 , 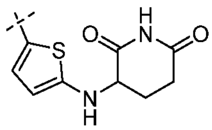 ,

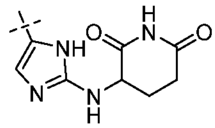 , 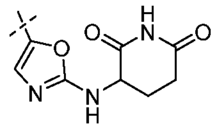 , 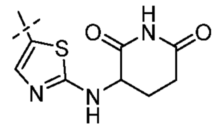 , 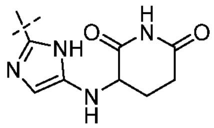 ,

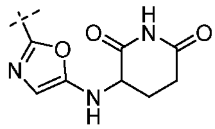 , 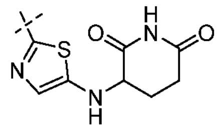 , 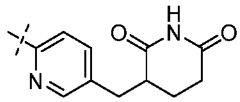 , 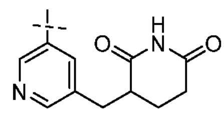 ,

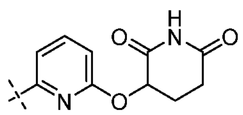 , 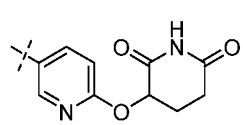 ,

In a 26th aspect of the first embodiment, D is:

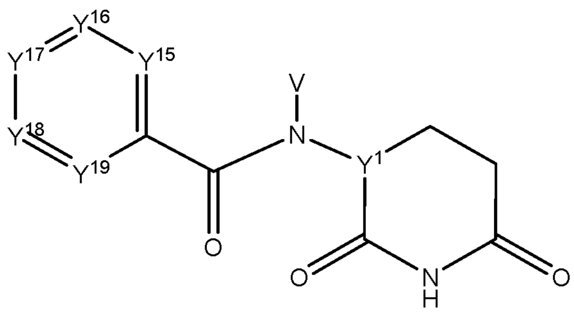 (I-D-IX),

wherein

$Y^1$, for each occurrence, is independently selected from CH, CD or N;

V is hydrogen or methyl;

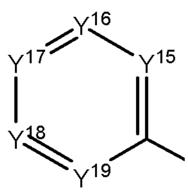

is an aromatic ring or a heteroaromatic ring, wherein

$Y^{15}$, for each occurrence, is independently selected from: absence; O; S; N; NH; $CT^{15}$, wherein $T^{15}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy; $NT^{15'}$, wherein $T^{15'}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;

$Y^{16}$, for each occurrence, is independently selected from: absence; O; S; N; NH; $CT^{16}$, wherein $T^{16}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy; $NT^{16'}$ , wherein $T^{16'}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;

$Y^{17}$, for each occurrence, is independently selected from: absence; O; S; N; NH; $CT^{17}$, wherein $T^{17}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy; $NT^{17'}$, wherein $T^{17'}$, for each occurrence, is independently selected from absence, hydrogen, fluorine, chlorine, methyl, halogen $CH_2F$, $CHF_2$, $CF_3$, $CH_2Cl$, $CHCl_2$, $CCl_3$, methoxy;

$Y^{18}$, for each occurrence, is independently selected from: absence; O; S; N; NH; $CT^{18}$, wherein $T^{18}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy; $NT^{18'}$, wherein $T^{18'}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;

$Y^{19}$, for each occurrence, is independently selected from: absence; O; S; N; NH; $CT^{19}$, wherein $T^{19}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy; $NT^{19'}$, wherein $T^{19'}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;

one or more of $Y^{15}$, $Y^{16}$, $Y^{17}$, $Y^{18}$ and $Y^{19}$ is linked to L groups; or

any two adjacent ones of $Y^{15}$, $Y^{16}$, $Y^{17}$, $Y^{18}$ and $Y^{19}$ are joined together with the connected atom to form an optionally substituted aryl or an optionally substituted heteroaryl, which is linked to L groups by one or more bonds; preferably, the aryl or heteroaryl is selected from phenyl, naphthyl, anthracenyl, indenyl, indanyl, 1,2-dihydronaphthyl, 1,2,3,4-tetrahydronaphthyl, pyridyl, pyrimidinyl, imidazolyl, pyrazolyl, triazolyl, tetrazolyl, furanyl, thienyl, oxazolyl, isoxazolyl, thiazolyl, isothiazolyl, oxadiazolyl, thiadiazolyl, triazinyl, and the substituents thereon are selected from: absence, fluorine, chlorine, methyl, $CH_2F$, $CHF_2$, $CF_3$, $CH_2Cl$, $CHCl_2$, $CCl_3$, methoxy.

Preferably, D is:

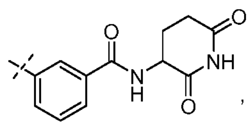, 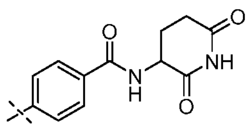, 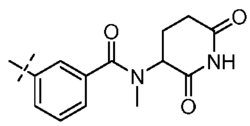, 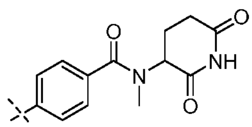,

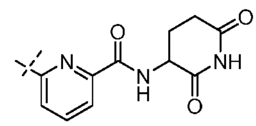, 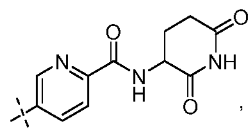, 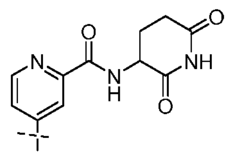, 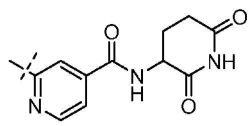,

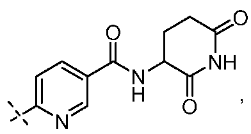, 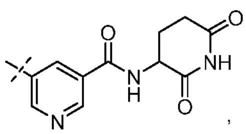, 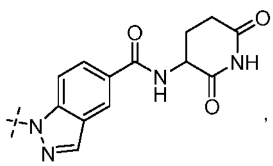, 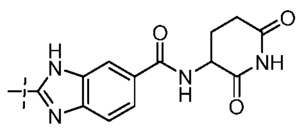,

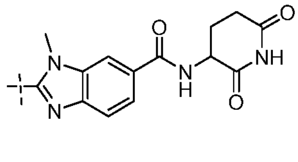

In a 27th aspect of the first embodiment, D is:

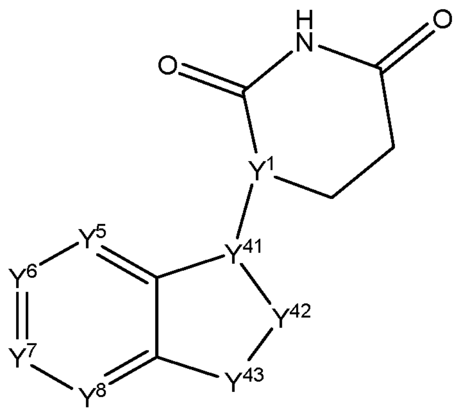

(I-D-X),

wherein

$Y^1$, for each occurrence, is independently selected from CH, CD or N;

$Y^{41}$, for each occurrence, is independently selected from $CT^{41}$ or N;

$T^{41}$, for each occurrence, is independently selected from absence, hydrogen, deuterium, methyl;

$Y^{42}$, for each occurrence, is independently selected from $CH_2$, $CD_2$, CHD, C=O, C-$CH_3$, or 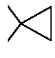;

$Y^{43}$, for each occurrence, is independently selected from $CH_2$, $CD_2$, CHD, C=O, C-$CH_3$, or 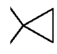; or

$Y^{42}$-$Y^{43}$ is -CH=CH-, -CH=N- or-N=CH-;

$Y^5$, for each occurrence, is independently selected from N; $CT^5$, wherein $T^5$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;

$Y^6$, for each occurrence, is independently selected from N; $CT^6$, wherein $T^6$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;

$Y^7$, for each occurrence, is independently selected from N; $CT^7$, wherein $T^7$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;

$Y^8$, for each occurrence, is independently selected from N; $CT^8$, wherein $T^8$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;

one or more of $Y^5$, $Y^6$, $Y^7$, and $Y^8$ are linked to L groups.

In a 28th aspect of the first embodiment, D is:

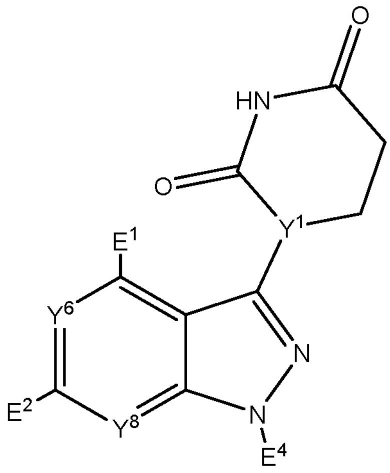

(I-D-XII),

wherein

$Y^1$, for each occurrence, is independently selected from CH, CD or N ;

$Y^6$, for each occurrence, is independently selected from $CT^6$ or N;

$Y^8$, for each occurrence, is independently selected from $CT^8$ or N;

each of $T^6$, $T^8$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;.

each of $E^1$, $E^2$ and $E^4$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;.

one or more of $E^1$, $E^2$, $Y^6$ and $Y^8$ are linked to L groups.

Preferably, D is:

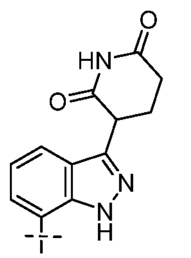 , 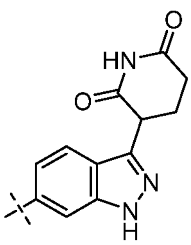 , 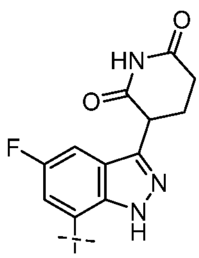 , 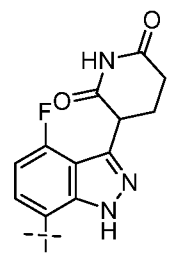 , 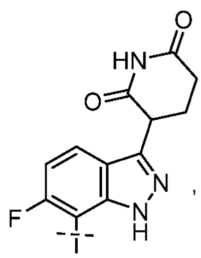 ,

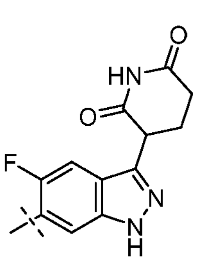 , 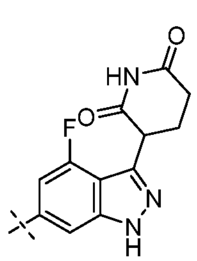 , 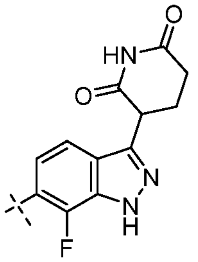 , 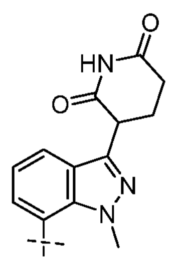 , 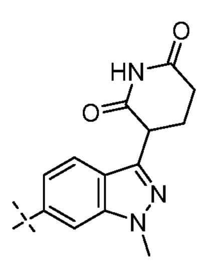 ,

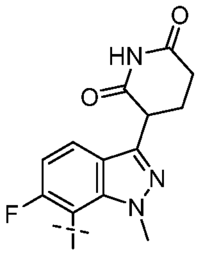 , 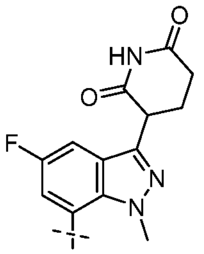 , 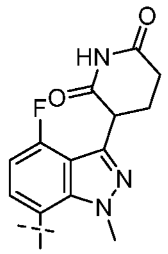 , 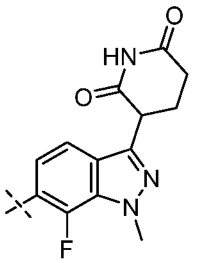 , 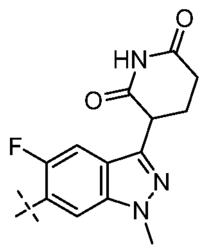 ,

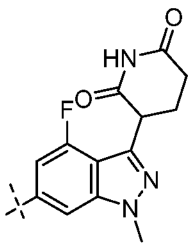 , 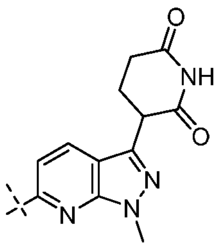 , 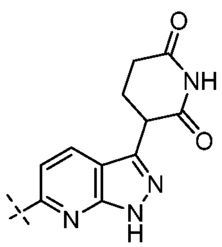 , 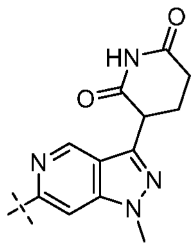 , 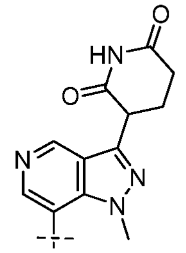 ,

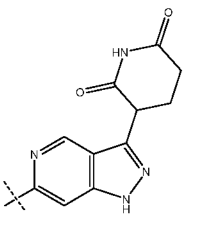 , 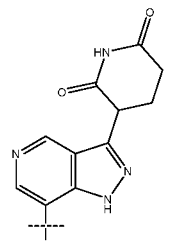 , 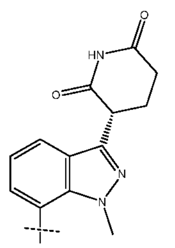 , 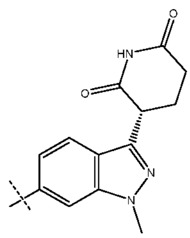 , 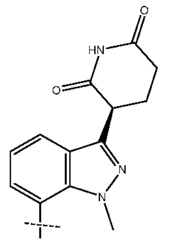 ,

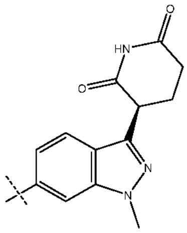 , 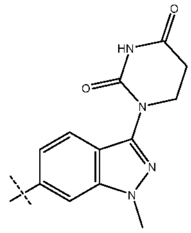 , 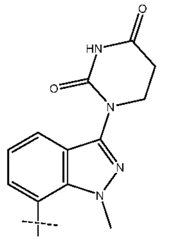 , 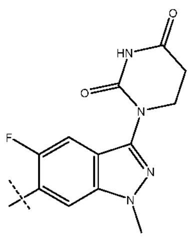 , 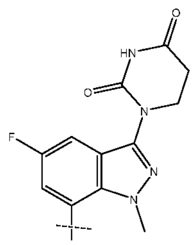

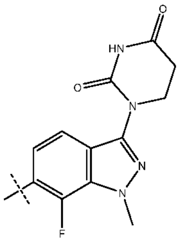 , 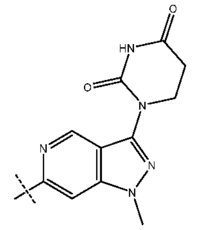 , 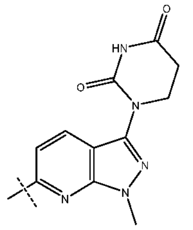 , 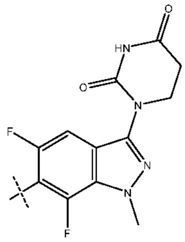 , 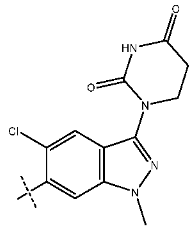 .

In a 29th aspect of the first embodiment, D is:

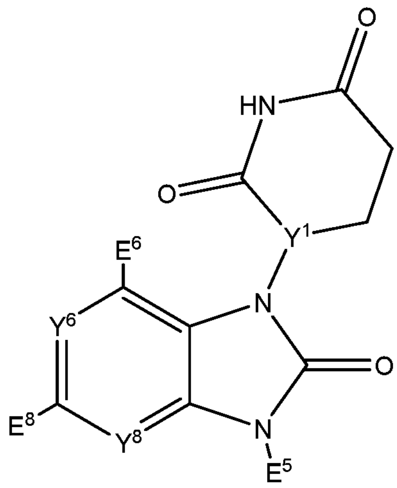

(I-D-XIII),

wherein

$Y^1$, for each occurrence, is independently selected from CH, CD or N ;
$Y^6$, for each occurrence, is independently selected from $CT^6$ or N;
$Y^8$, for each occurrence, is independently selected from $CT^8$ or N;
each of $T^6$, $T^8$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;.
each of $E^5$, $E^6$ and $E^8$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;
one or more of $E^6$, $E^8$, $Y^6$ and $Y^8$ are linked to L groups.

In a 30th aspect of the first embodiment, D is:

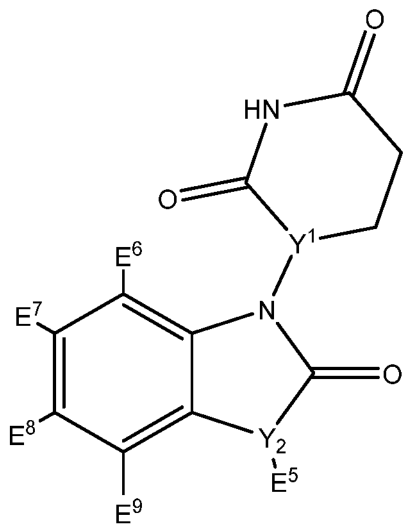

(I-D-XIV)

$Y^1$, for each occurrence, is independently selected from CH, CD or N ;

$Y^2$, for each occurrence, is independently selected from CH, CD, N or oxygen;

$E^5$, for each occurrence, is independently selected from absence, hydrogen, deuterium or methyl;

each of $E^6$, $E^7$, $E^8$ and $E^9$, for each occurrence, is independently selected from absence, hydrogen, deuterium, fluorine, chlorine, methyl, $CH_2F$, $CHF_2$, $CF_3$, methoxy, or cyclopropyl;

one or more of $E^6$, $E^7$, $E^8$ and $E^9$ are linked to L groups.

Preferably, D is:

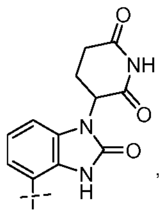 , 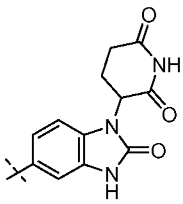 , 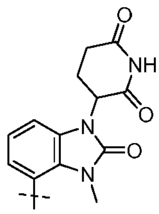 , 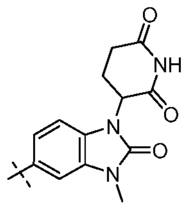 , 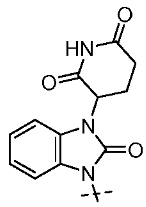 ,

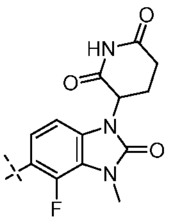 , 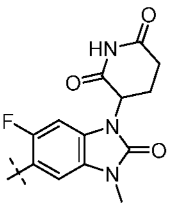 , 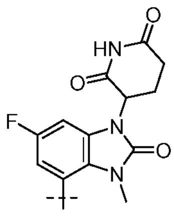 , 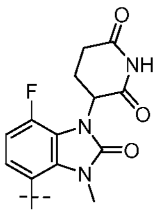 , 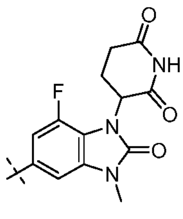 ,

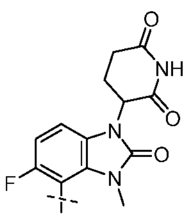 , 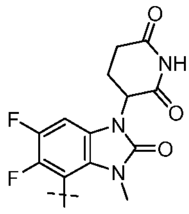 , 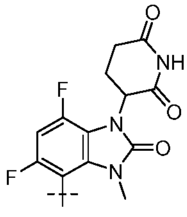 , 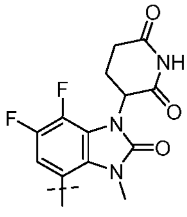 , 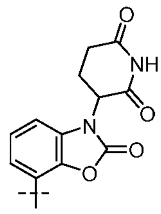 ,

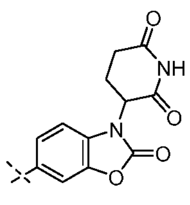 , 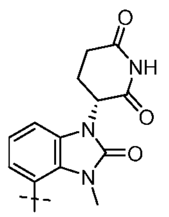 , 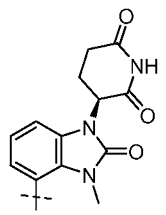

In a 31st aspect of the first embodiment, D is:

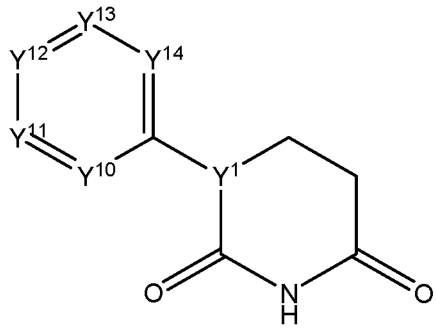

(I-D-XV)

$Y^1$, for each occurrence, is independently selected from CH, CD or N;

$Y^{10}$, for each occurrence, is independently selected from N; $CT^{10}$, wherein $T^{10}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;

$Y^{11}$, for each occurrence, is independently selected from N; $CT^{11}$, wherein $T^{11}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;

$Y^{12}$, for each occurrence, is independently selected from N; $CT^{12}$, wherein $T^{12}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;

$Y^{13}$, for each occurrence, is independently selected from N; $CT^{13}$, wherein $T^{13}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;

$Y^{14}$, for each occurrence, is independently selected from N; $CT^{14}$, wherein $T^{14}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;

one or more of $Y^{10}$, $Y^{11}$, $Y^{12}$, $Y^{13}$ and $Y^{14}$ are linked to L groups.

Preferably, D is a structure selected from:

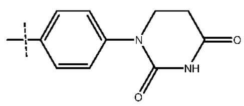 , 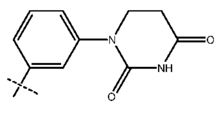 , 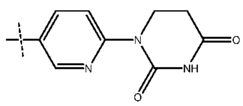 , 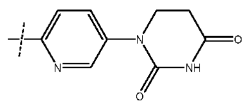 ,

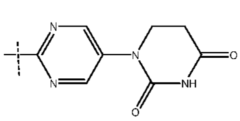 , 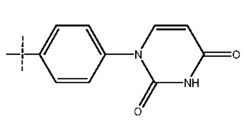 , 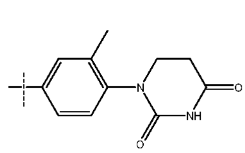 , 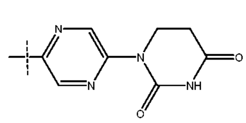 ,

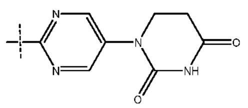 , 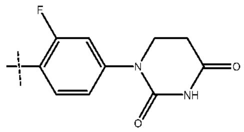 , 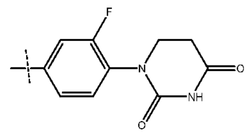 , 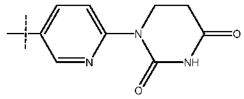 ,

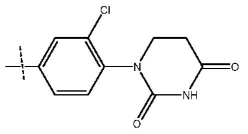 , 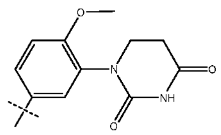

.

In a 32nd aspect of the first embodiment, L is selected from:

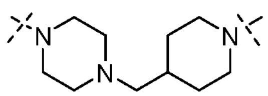 , 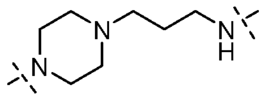 , 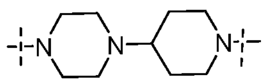 , 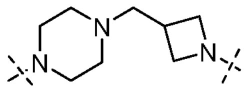 ,

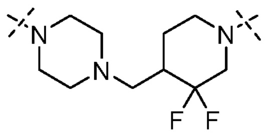 , 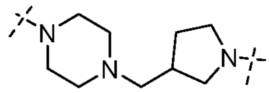 , 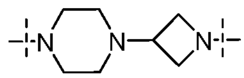 , 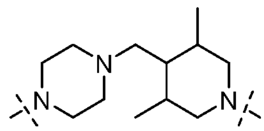 ,

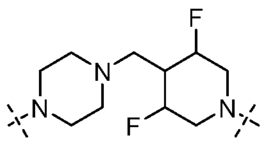 , 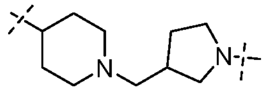 , 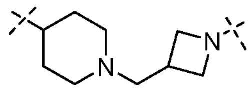 , 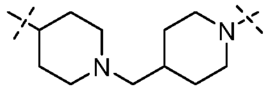 ,

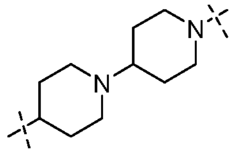 , 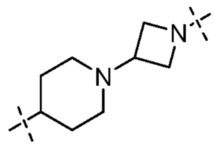 , 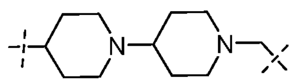 , 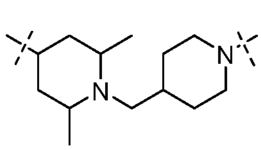 ,

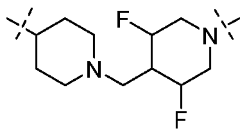 , 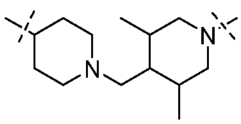 , 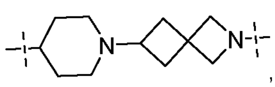 , 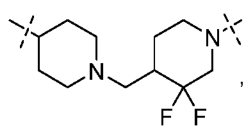 ,

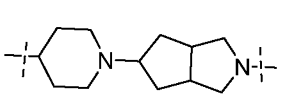 , 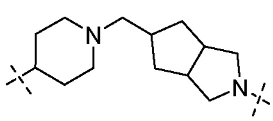 , 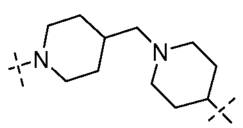 ,

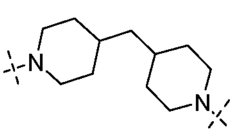 , 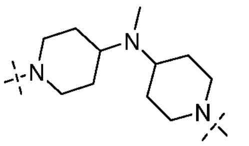 , 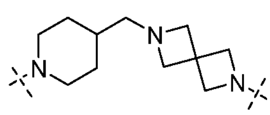 , 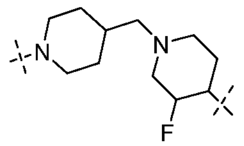 , ,

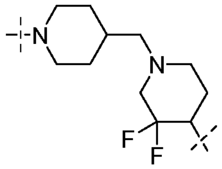 , 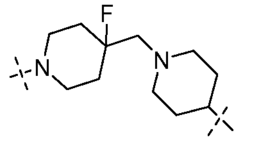 , 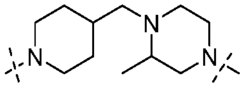 , 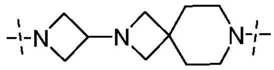 ,

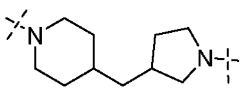 , 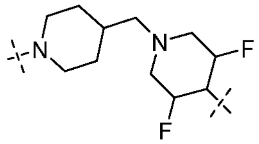 , 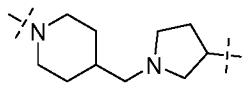 , 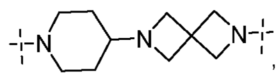 ,

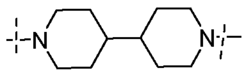 , 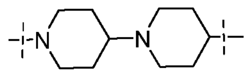 , 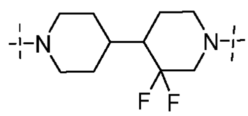 , 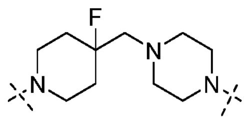 ,

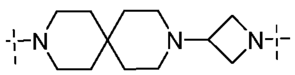 , 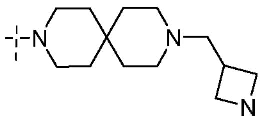 , 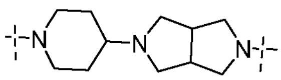 , 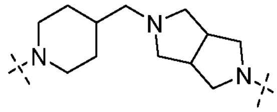 ,

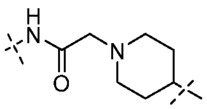 , 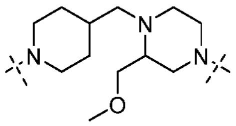 , 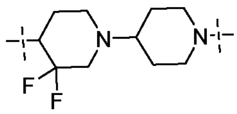 , 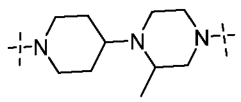 ,

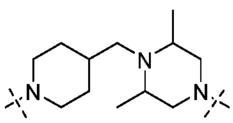 , 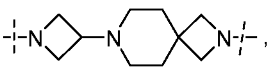 , 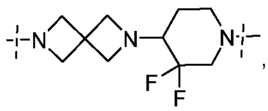 , 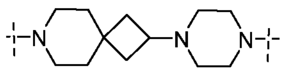 ,

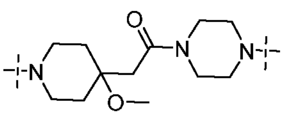 , 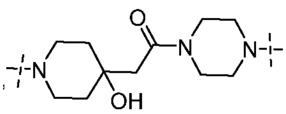 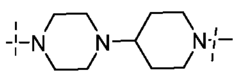 , 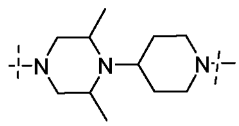

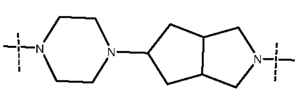 , 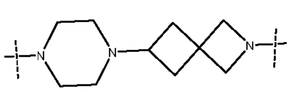 , 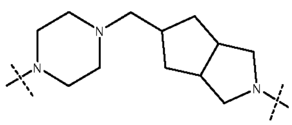

In the second embodiment, the present invention relates to formula (II):

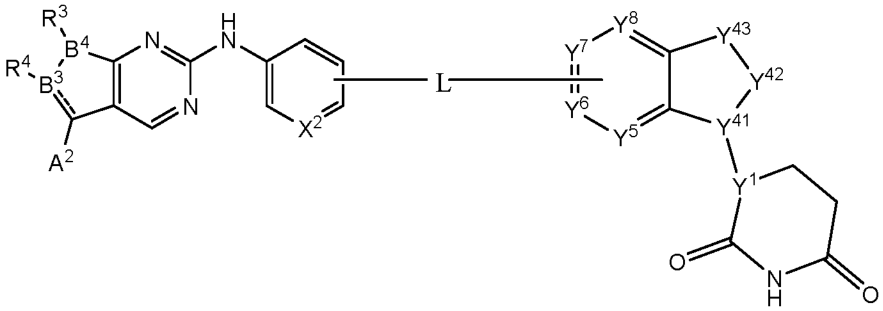

,

the remaining substituents are as defined in the first embodiment and any aspect thereof.

In the third embodiment, the present invention relates to formula (III):

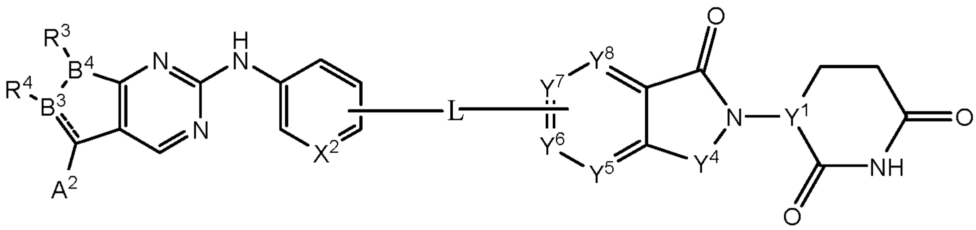

,

the remaining substituents are as defined in the first embodiment and any aspect thereof.

In the fourth embodiment, the present invention relates to formula (IV):

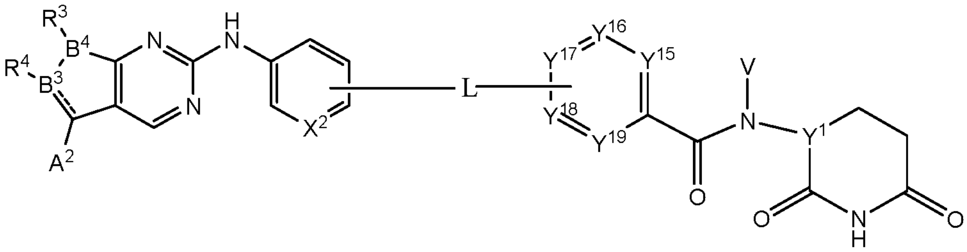

,

the remaining substituents are as defined in the first embodiment and any aspect thereof.

In the fifth embodiment, the present invention relates to formula (V):

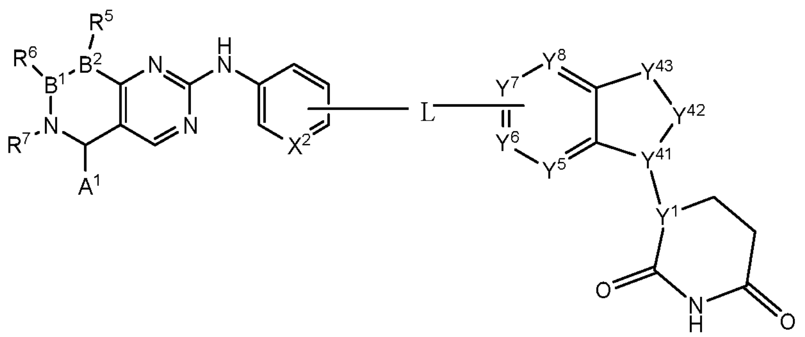

,

the remaining substituents are as defined in the first embodiment and any aspect thereof.

In the sixth embodiment, the present invention relates to formula (VI):

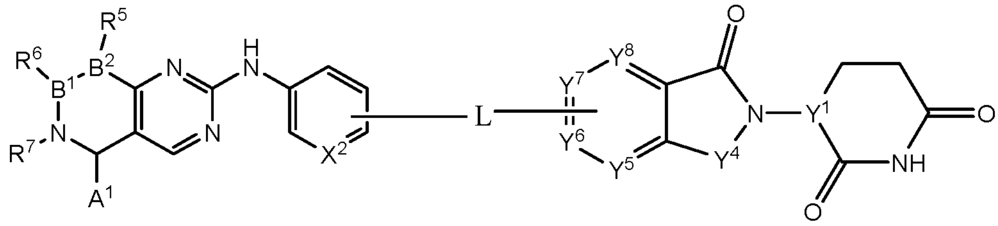

,

the remaining substituents are as defined in the first embodiment and any aspect thereof.

In the seventh embodiment, the present invention relates to formula (VIII):

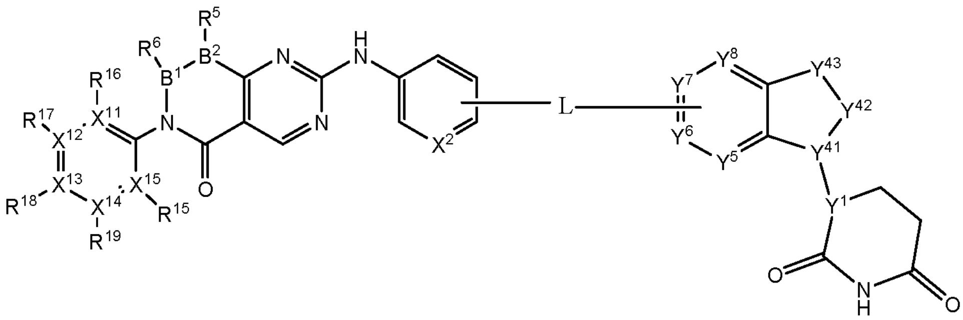

,

the remaining substituents are as defined in the first embodiment and any aspect thereof.

Preferably, $X^{15}$-$R^{15}$ is CH, C or C-Cl; $X^{14}$-$R^{19}$ is CH, C or C-Cl; $X^{13}$-$R^{18}$ is CH, C or C-Cl; $X^{12}$-$R^{17}$ is CH, C or C-Cl; and/or $X^{11}$-$R^{16}$ is CH, C or C-Cl.

Also preferably, $B^1$-$R^6$ are $CH_2$; and/or $B^2$-$R^5$ is NH, $CH_2$ or N-$CH_3$.

Also preferably, $Y^1$ is CH or N.

Also preferably, $Y^5$ is CH or C; $Y^6$ is CH or C; $Y^7$ is CH or C; and/or $Y^8$ is CH or C.

Also preferably, $Y^{41}$ is CH or N; $Y^{42}$ is $CH_2$ or =O; and/or $Y^{43}$ is NH, $CH_2$ or N-$CH_3$.

In the eighth embodiment, the present invention relates to formula (IX):

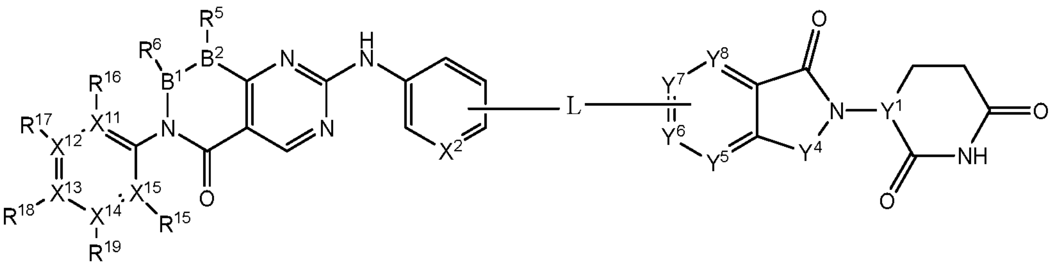

,

the remaining substituents are as defined in the first embodiment and any aspect thereof.

Preferably, $X^{15}$-$R^{15}$ is CH, C or C-Cl; $X^{14}$-$R^{19}$ is CH, C or C-Cl; $X^{13}$-$R^{18}$ is CH, C or C-Cl; $X^{12}$-$R^{17}$ is CH, C or C-Cl; and/or $X^{11}$-$R^{16}$ is CH, C or C-Cl.

Also preferably, $B^1$-$R^6$ are $CH_2$; and/or $B^2$-$R^5$ is NH, $CH_2$ or N-$CH_3$.

Also preferably, $Y^1$ is CH or N.

Also preferably, $Y^5$ is CH or C; $Y^6$ is CH or C; $Y^7$ is CH or C; and/or $Y^8$ is CH or C.

Also preferably, $Y^4$ is $CH_2$ or =O.

In the nineth embodiment, the present invention relates to formula (XIV):

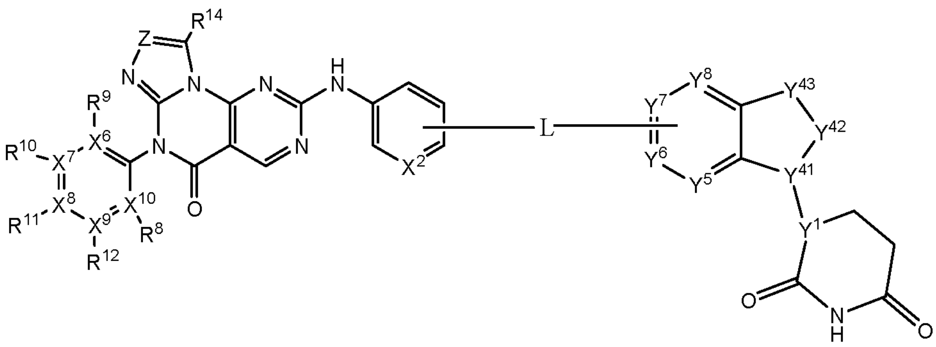

,

the remaining substituents are as defined in the first embodiment and any aspect thereof.

In the tenth embodiment, the present invention relates to formula (XV):

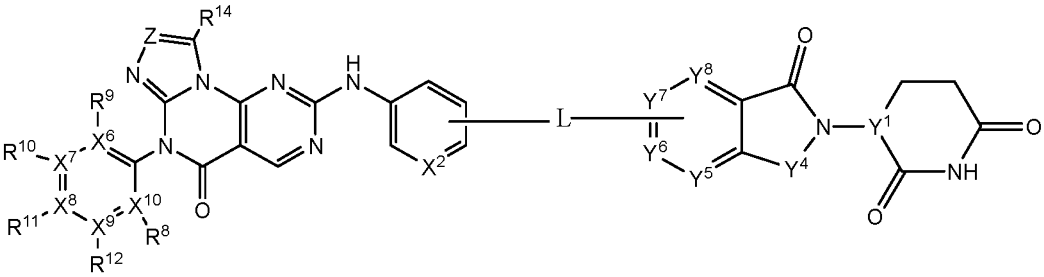

,

the remaining substituents are as defined in the first embodiment and any aspect thereof.

In the eleventh embodiment, the present invention relates to formula (XVII):

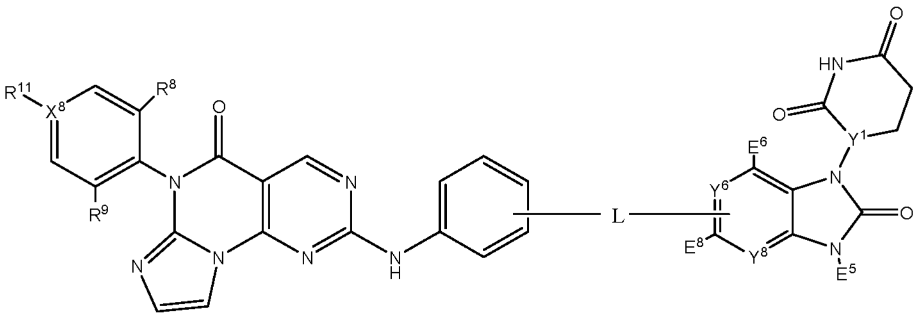

,

the remaining substituents are as defined in the first embodiment and any aspect thereof.

Preferably, $X^8$-$R^{11}$ is CH; $R^8$ is Cl; and/or $R^9$ is Cl.

Also preferably, $E^6$ is H or absent; and/or $E^8$ is H or absent.

Also preferably, $E^5$ is H or $-CH_3$.

Also preferably, $Y^1$ is CH.

Also preferably, $Y^6$ is CH or C; $Y^8$ is CH or C.

In the twelfth embodiment, the present invention relates to formula (XVIII):

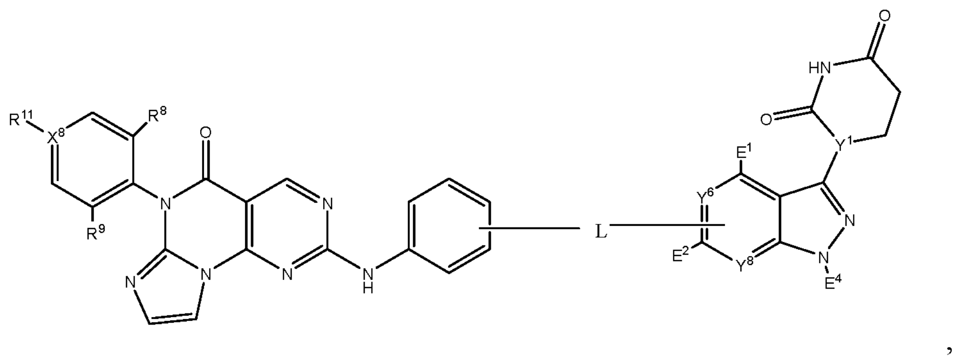

the remaining substituents are as defined in the first embodiment and any aspect thereof.

Preferably, $X^8$-$R^{11}$ is CH; $R^8$ is Cl; and/or $R^9$ is Cl.

Also preferably, $E^1$ is H, F or absent; and/or $E^2$ is H, F or absent.

Also preferably, $E^4$ is H or $-CH_3$.

Also preferably, $Y^1$ is CH, CD or N.

Also preferably, $Y^6$ is CH or C; and/or $Y^8$ is CH, N or C.

In the thirteenth embodiment, the present invention relates to formula (XIX):

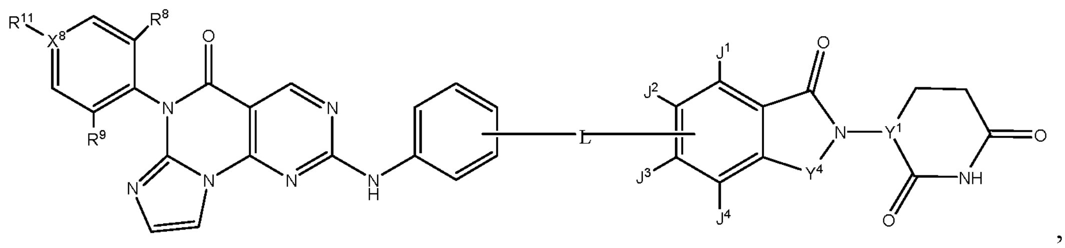

the remaining substituents are as defined in the first embodiment and any aspect thereof.

Preferably, $X^8$-$R^{11}$ is CH; $R^8$ is Cl; and/or $R^9$ is Cl.

Also preferably, $J^1$ is H or absent; $J^2$ is H or absent; $J^3$ is H or absent; and/or $J^4$ is H or absent.

Also preferably, $Y^1$ is CH.

Also preferably, $Y^4$ is $CH_2$ or $N-CH_3$.

In the fourteenth embodiment, the present invention relates to formula (XXIII):

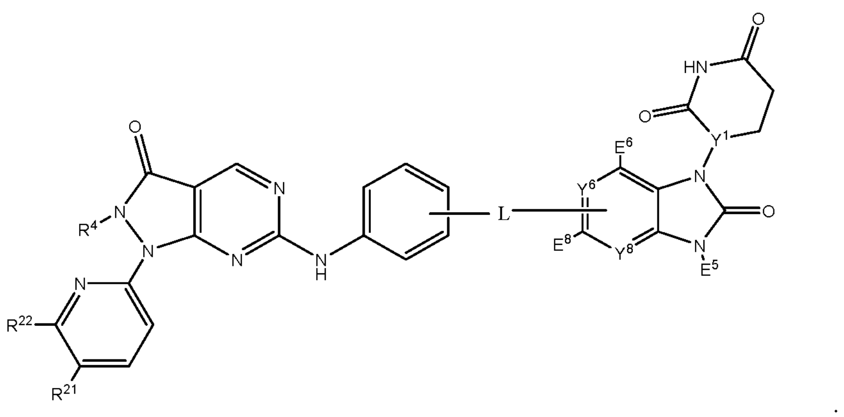

In the fifteenth embodiment, the present invention relates to formula (XXIV):

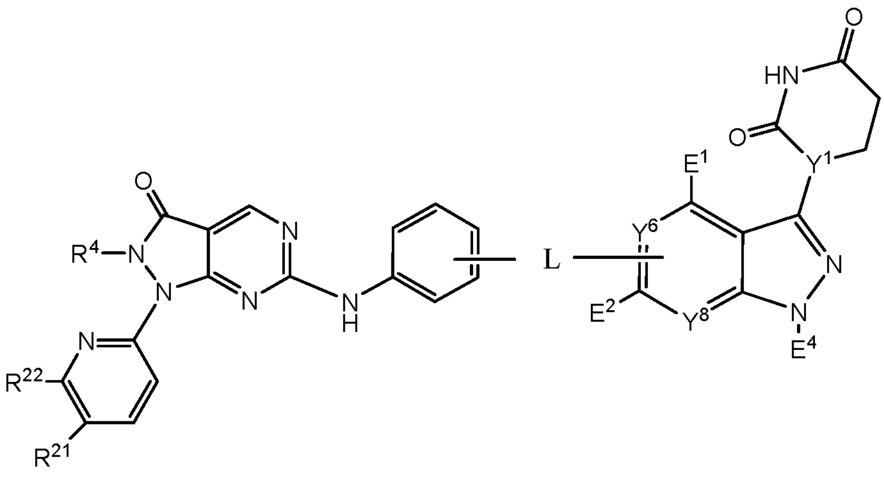

,

the remaining substituents are as defined in the first embodiment and any aspect thereof.

Preferably, $R^4$ is allyl, H or $CH_3$.

Also preferably, $R^{21}$ is CH, C-$CH_3$ or C-Cl; and/or $R^{22}$ is CH, C-$CH_3$ or C-Cl.

Also preferably,

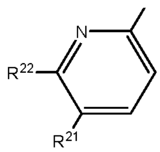

is selected from:

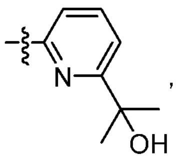 , 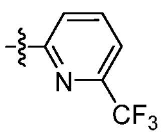 , 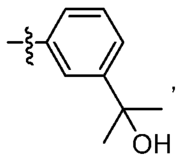 , 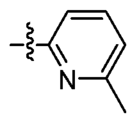 , 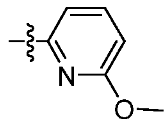 ,

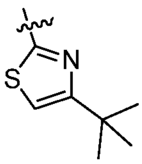 , 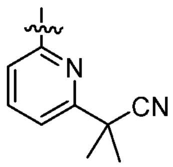 , 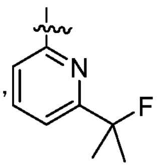 , 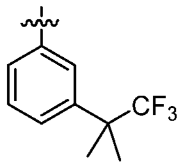 , 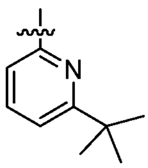 ,

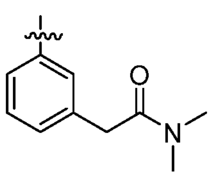 , 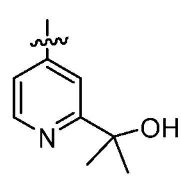 , 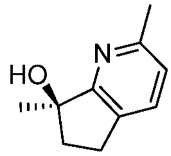 , 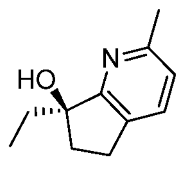 ,

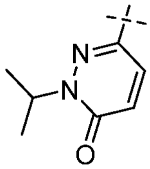

Also preferably, $E^1$ is H, F or absent; and/or $E^2$ is H, F or absent.

Also preferably, $E^4$ is H or $-CH_3$.

Also preferably, $Y^1$ is CH, CD or N.

Also preferably, $Y^6$ is CH or C; and/or $Y^8$ is CH, N or C.

Also preferably, $R^2$ is absent, H, F, chlorine, methyl, ethyl, methoxy, hydroxymethyl, or linked to L groups.

Also preferably, $R^1$ is absent, H, F, chlorine, methyl, ethyl, methoxy, hydroxymethyl, or linked to L groups.

In the sixteenth embodiment, the present invention relates to formula (XXV):

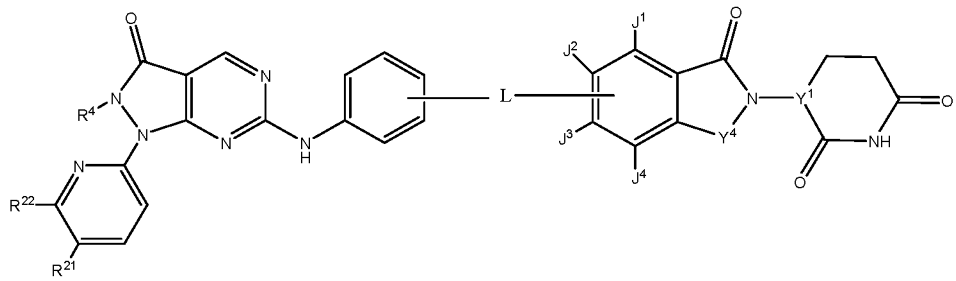

,

the remaining substituents are as defined in the first embodiment and any aspect thereof.

In the seventeenth embodiment, the present invention relates to formula (XXVI):

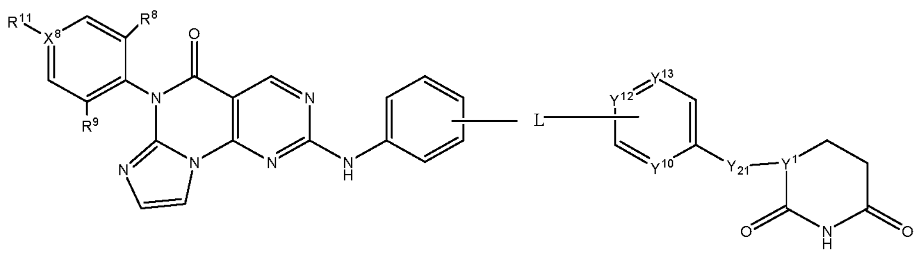

,

the remaining substituents are as defined in the first embodiment and any aspect thereof.

Preferably, $X^8$-$R^{11}$ is CH; $R^8$ is Cl; $R^9$ is Cl.

Also preferably, $Y^{21}$ is NH, O or absent.

Also preferably, $Y^{10}$ is CH or C; $Y^{12}$ is CH or C; and/or $Y^{13}$ is CH or C.

In the eighteenth embodiment, the present invention relates to formula (XXVII):

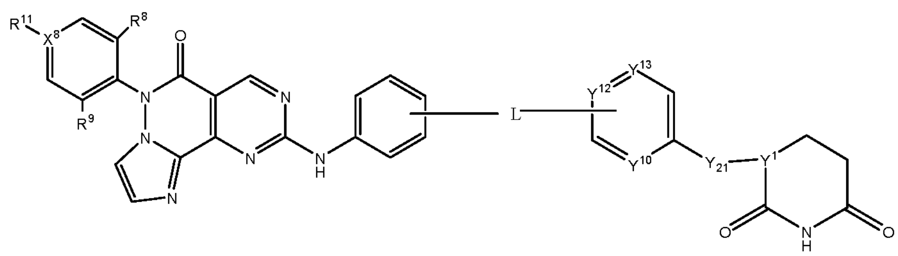

,

the remaining substituents are as defined in the first embodiment and any aspect thereof.

Preferably, $Y^{21}$ is NH, O or absent.

Also preferably, $Y^{10}$ is CH or C; $Y^{12}$ is CH or C; and/or $Y^{13}$ is CH or C.

Also preferably, $Y^1$ is CH or N.

Also preferably, $Y^{10}$ is CH or C; $Y^{12}$ is CH or C; and/or $Y^{13}$ is CH or C.

Also preferably, $X^8$-$R^{11}$ is CH; $R^8$ is Cl; $R^9$ is Cl.

In the nineteenth embodiment, the present invention relates to formula (XXVIII):

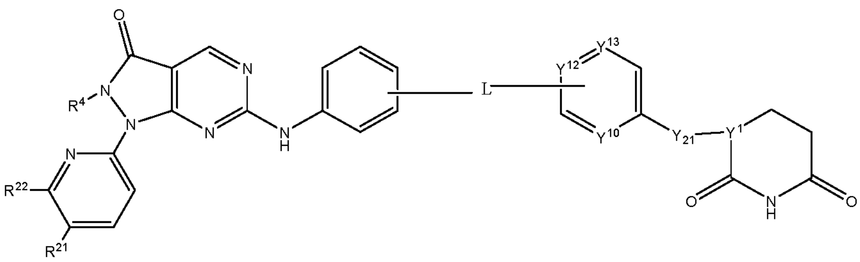

,

the remaining substituents are as defined in the first embodiment and any aspect thereof.

Preferably, $R^4$ is allyl, H or $CH_3$.

Also preferably, $Y^{21}$ is NH, O or absent.

Also preferably, $Y^{10}$ is CH or C; $Y^{12}$ is CH or C; and/or $Y^{13}$ is CH or C.

Also preferably, $R^{21}$ is CH, C-$CH_3$ or C-Cl; and/or $R^{22}$ is CH, C-$CH_3$ or C-Cl.

Also preferably,

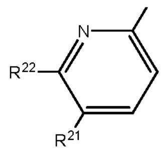

is selected from:

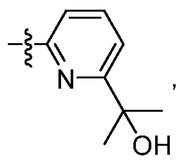 , 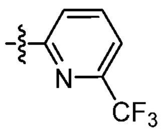 , 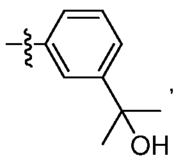 , 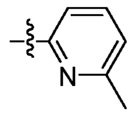 , 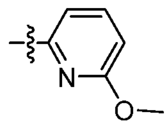 ,

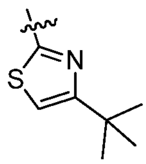 , 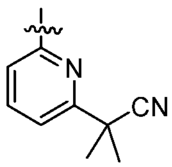 , 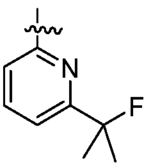 , 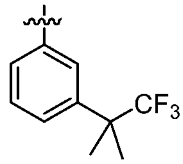 , 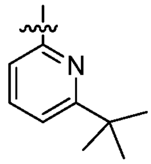 ,

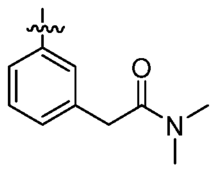 , 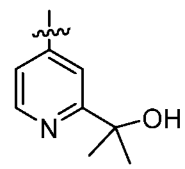 , 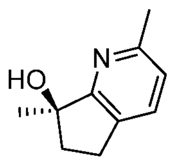 , 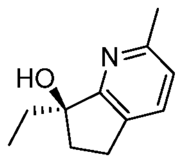 ,

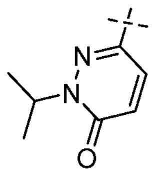

In the twentieth embodiment, the present invention relates to formula (XXIX):

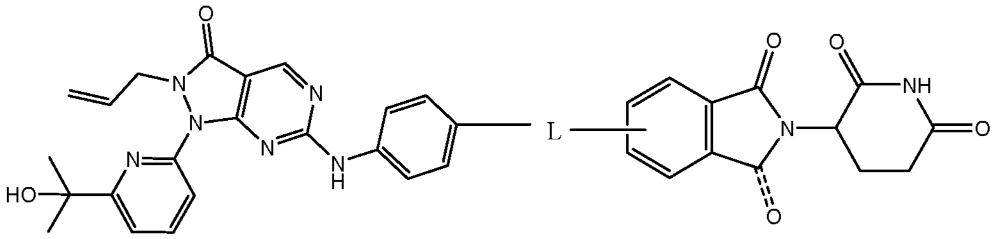

,

wherein:



represents: =O or absence,
the L group is as defined in the first embodiment and any aspect thereof.

Preferably,

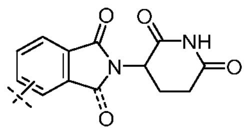

is

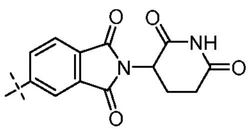

or

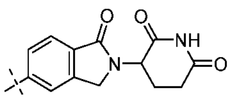

.

In the 21st embodiment, the present invention relates to formula (XXX):

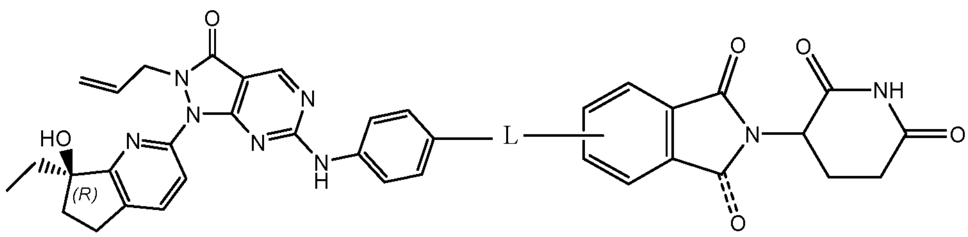

,

wherein:



represents: =O or absence,
the L group is as defined in the first embodiment and any aspect thereof.

Preferably,

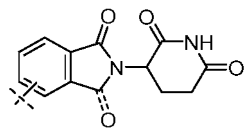

is

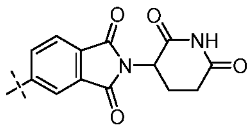
or
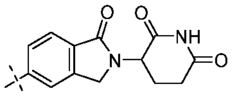
.

In the 22nd embodiment, the present invention relates to formula (XXXI):

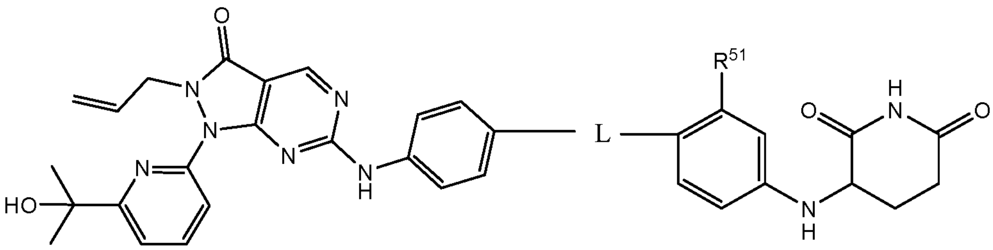
,

wherein:

$R^{51}$, for each occurrence, is independently selected from F, Cl or $CF_3$,
the L group is as defined in the first embodiment and any aspect thereof.

In the 23rd embodiment, the present invention relates to formula (XXXII):

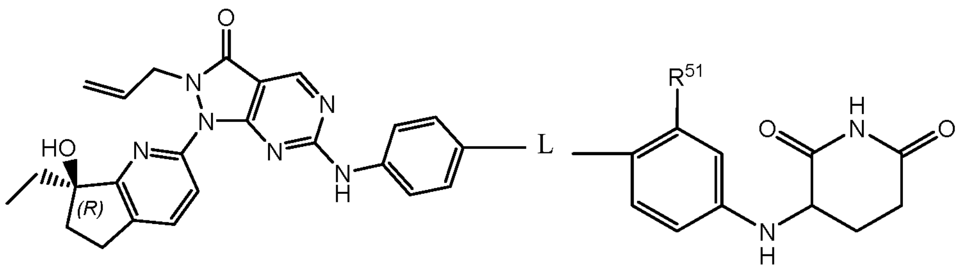
,

wherein:

$R^{51}$, for each occurrence, is independently selected from F, Cl or $CF_3$,
the L group is as defined in the first embodiment and any aspect thereof.

In the 24th embodiment, the present invention relates to formula (XXXIII):

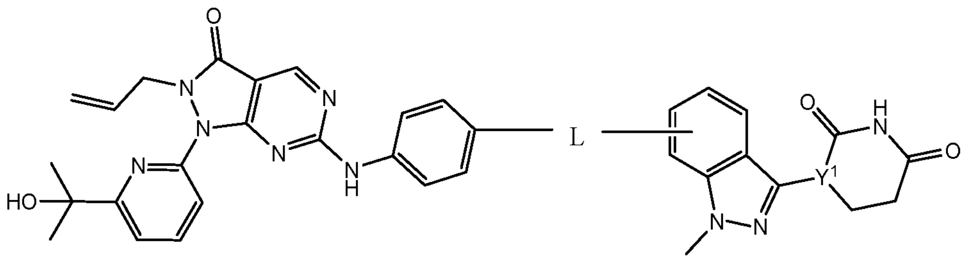

,

wherein:

$Y^1$, for each occurrence, is independently selected from CH, CD or N,
the L group is as defined in the first embodiment and any aspect thereof.

Preferably,

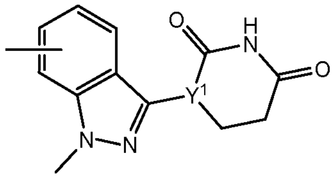

is selected from

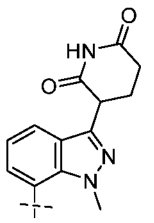

,

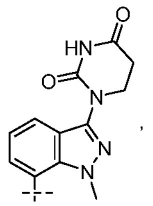

,

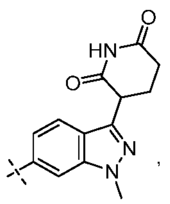

, or

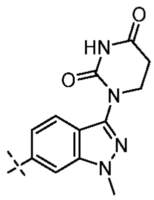

;

In the 25th embodiment, the present invention relates to formula (XXXIV):

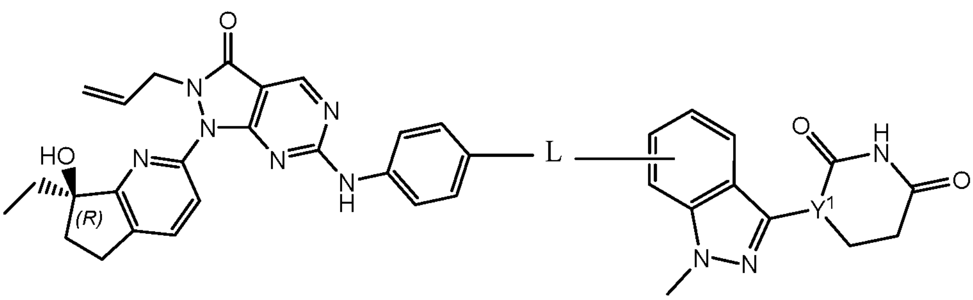

,

wherein:

Y[1], for each occurrence, is independently selected from CH, CD or N,
the L group is as defined in the first embodiment and any aspect thereof.

Preferably,

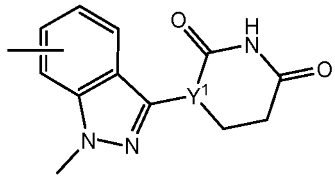

is selected from

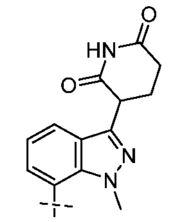
,
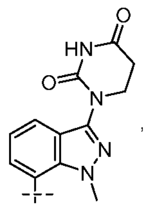
,

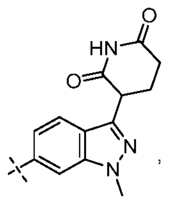
,
or
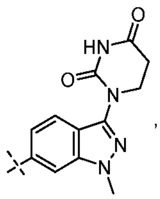
;

In the 26th embodiment, the present invention relates to formula (XXXV):

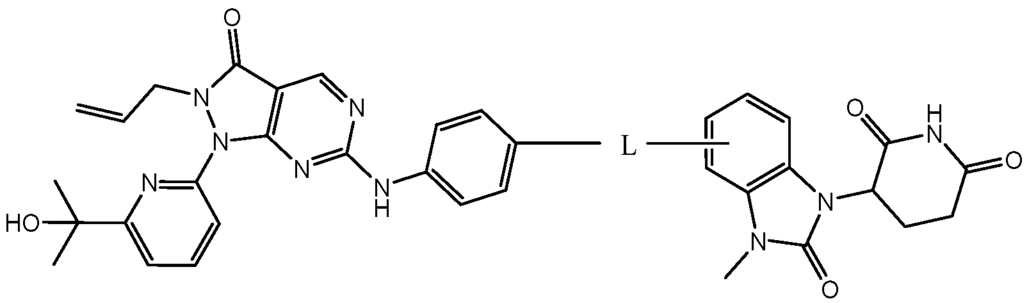
.

Preferably,

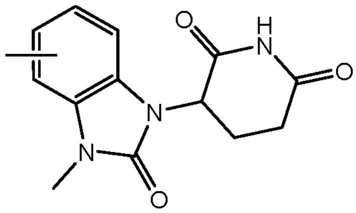

is selected from

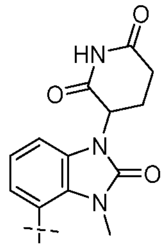, or 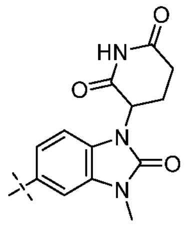.

In the 27th embodiment, the present invention relates to formula (XXXVI):

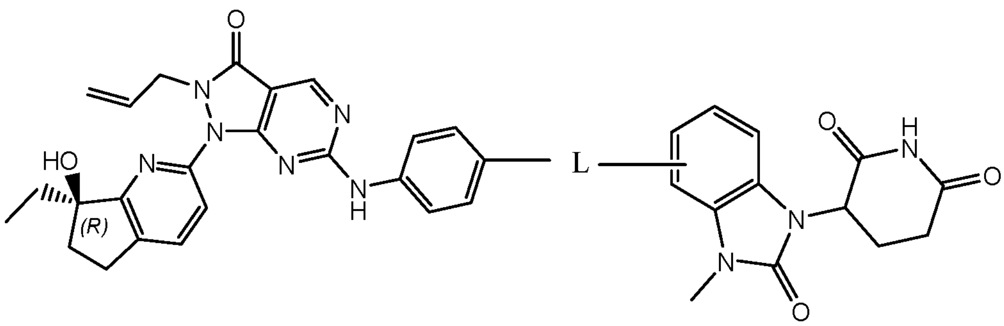,

the L group is as defined in the first embodiment and any aspect thereof.

Preferably,

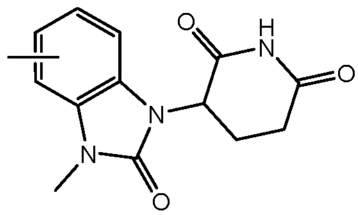

is selected from

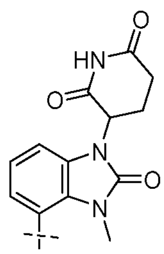, or 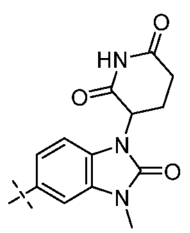.

In the 28th embodiment, the present invention relates to a compound represented by structural formula (XXXXI), or a derivative, pharmaceutically acceptable salt, isomer, solvate, hydrate, adduct, complex or prodrug thereof:

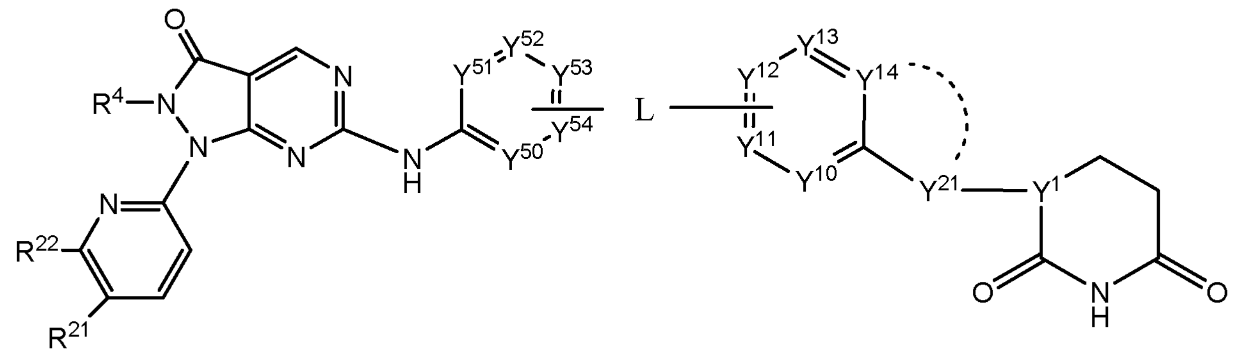

XXXXI,

wherein:

$Y^1$, for each occurrence, is independently selected from CH, CD or N;

$Y^{21}$, for each occurrence, is independently selected from O, C, N, NH, N-$CH_3$, N-$C_2H_5$, N-$C_3H_7$, N-$C_4H_9$;

each of $Y^{10}$, $Y^{11}$, $Y^{12}$, $Y^{13}$ and $Y^{14}$, for each occurrence, is independently selected from N; $CT^{101}$, wherein $T^{101}$, for each occurrence, is independently selected from absence, hydrogen, fluorine, chlorine, methyl, $CH_2F$, $CHF_2$, $CF_3$, $CH_2Cl$, $CHCl_2$, $CCl_3$, ethyl, propyl, isopropyl, butyl, isobutyl, tert-butyl, methoxyl, ethoxyl, propoxyl, isopropoxyl, butoxyl, isobutoxyl, tert-butoxyl, hydroxyl, cyano;

one of $Y^{10}$, $Y^{11}$, $Y^{12}$, $Y^{13}$ and $Y^{14}$ is linked to L groups;

each of $Y^{50}$, $Y^{51}$, $Y^{52}$, $Y^{53}$ and $Y^{54}$, for each occurrence, is independently selected from N; $CT^{102}$, wherein $T^{102}$, for each occurrence, is independently selected from absence, hydrogen, fluorine, chlorine, methyl, $CH_2F$, $CHF_2$, $CF_3$, $CH_2Cl$, $CHCl_2$, $CCl_3$, ethyl, propyl, isopropyl, butyl, isobutyl, tert-butyl, methoxyl, ethoxyl, propoxyl, isopropoxyl, butoxyl, isobutoxyl, tert-butoxyl, hydroxyl, cyano;

one of $Y^{50}$, $Y^{51}$, $Y^{52}$, $Y^{53}$ and $Y^{54}$ are linked to L groups;

$R^4$, for each occurrence, is independently selected from allyl, propargyl, methylene cyclopropyl, ethyl, trifluoroethyl, difluoroethyl, isopropyl, cyclopropyl, methylene $C_6$-$C_{12}$ aryl, methylene phenyl, optionally substituted $C_{1-4}$ alkyl, optionally substituted $C_{2-4}$ alkenyl, optionally substituted $C_{2-4}$ alkynyl, optionally substituted $C_{3-6}$ cycloalkyl and optionally substituted $C_{3-6}$ cycloalkyl ($C_{1-4}$ alkyl), wherein the $C_6$-$C_{12}$ aryl, $C_{1-4}$ alkyl, $C_{2-4}$ alkenyl and $C_{2-4}$ alkynyl are optionally independently substituted with one or more substituents selected from halogen, $C_{1-4}$ alkoxyl, $C_{1-4}$ haloalkyl, $C_{1-4}$ haloalkoxyl, cyano, amino, mono-$C_{1-4}$ alkylamine and di-$C_{1-4}$ alkylamine, and wherein one or more rings of the $C_{3-6}$ cycloalkyl and the $C_{3-6}$ cycloalkyl ($C_{1-4}$ alkyl) are optionally independently substituted with one or more substituents selected from halogen, $C_{1-4}$ alkyl, $C_{1-4}$ alkoxyl, $C_{1-4}$ haloalkyl, $C_{1-4}$ haloalkoxyl, cyano, amino, mono-$C_{1-4}$ alkylamine and di-$C_{1-4}$ alkylamine;

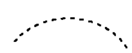

represents absence, -($NR^{81}$)-N=, -($NR^{82}$)-($NR^{83}$)-, -($NR^{84}$)-(C=O)-, or -(C=O)-($NR^{85}$)-, wherein each of $R^{81}$, $R^{82}$, $R^{83}$, $R^{84}$ and $R^{85}$, for each occurrence, is independently selected from hydrogen, methyl, ethyl, propyl, isopropyl, butyl, isobutyl, tert-butyl, methoxyl, ethoxyl, propoxyl, isopropoxyl, butoxyl, isobutoxyl, tert-butoxyl;

each of $R^{21}$ and $R^{22}$, for each occurrence, is independently selected from absence, hydrogen, deuterium, halogen, hydroxyl, carbonyl, nitro, cyano, amino, $C_1$-$C_6$ alkyl, $C_1$-$C_6$ alkoxyl, wherein the alkyl, and alkoxyl are optionally substituted with 1, 2, 3 or 4 halogens, hydroxyl, carbonyl, nitro, cyano, amino,; preferably, each of $R^{21}$ and $R^{22}$, for each occurrence, is independently selected from hydrogen, 2-hydroxyisopropyl; or

$R^{21}$, $R^{22}$ are joined together to form -($CR^{91}R^{92}$)-($CR^{93}R^{94}$)-($CR^{95}R^{96}$)-, wherein each of $R^{91}$, $R^{92}$, $R^{93}$, $R^{94}$, $R^{95}$,

and $R^{96}$, for each occurrence, is independently selected from hydrogen, hydroxyl, methyl, ethyl, propyl; further preferably, $R^{21}$, $R^{22}$ are joined together to form $-(CR^{91}R^{92})-(CH_2)-(CH_2)-$ or $-(CH_2)-(CH_2)-(CR^{95}R^{96})-$, wherein each of $R^{91}$, $R^{92}$, $R^{95}$, and $R^{96}$, for each occurrence, is independently selected from hydroxyl, methyl, ethyl, propyl;

L is a linker and has the following structure;

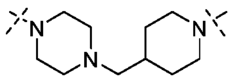, 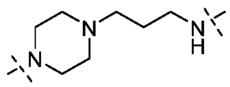, 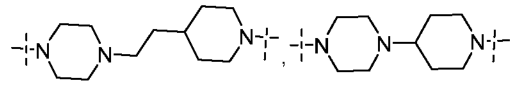,

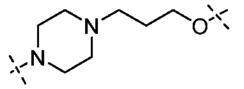, 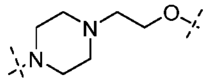, 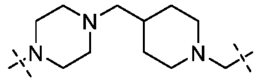, 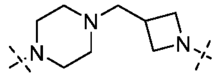,

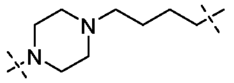, 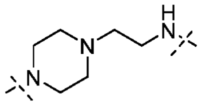, 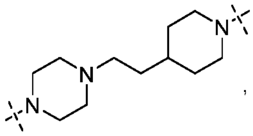, 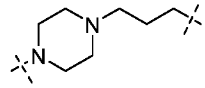,

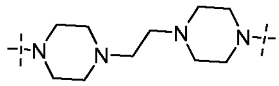, 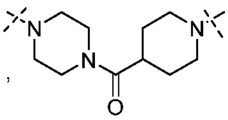, 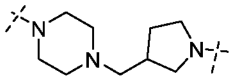, 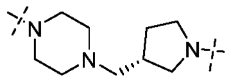,

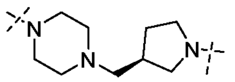, 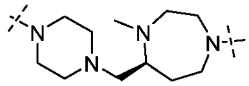, 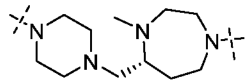, 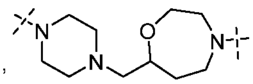,

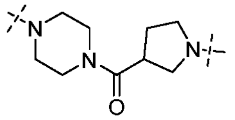, 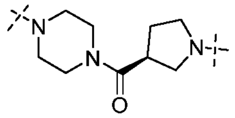, 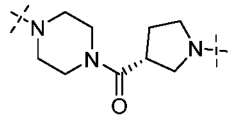, 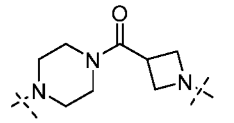,

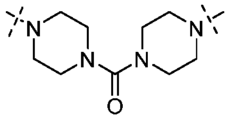, 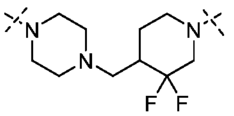, 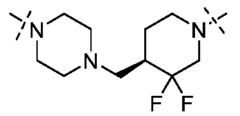, 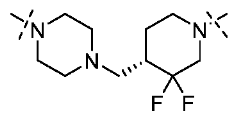,

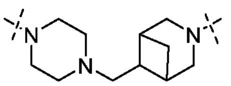, 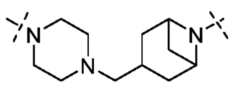, 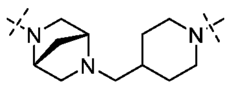, 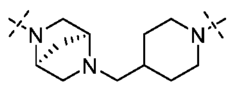,

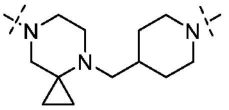, 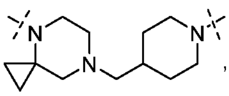, 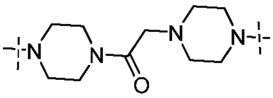, 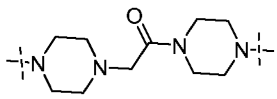,

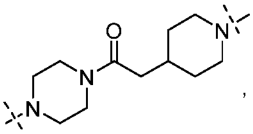, 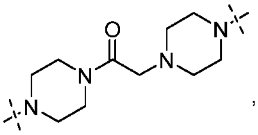, 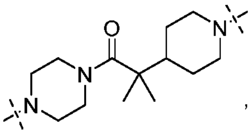, 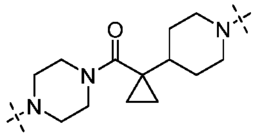,

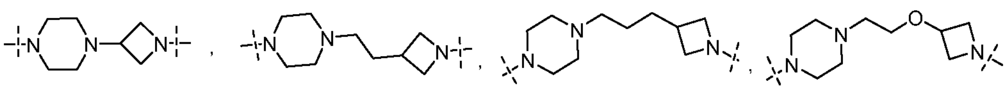,

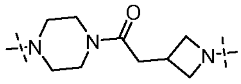, 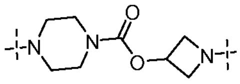, 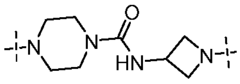, 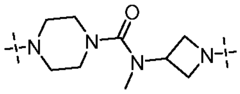,

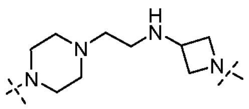, 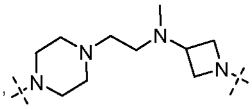, 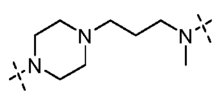, 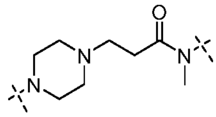,

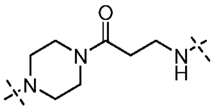, 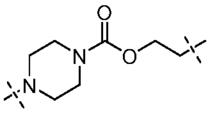, 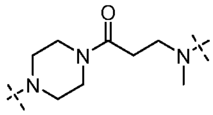, 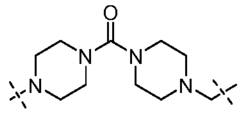,

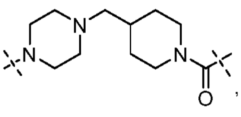, 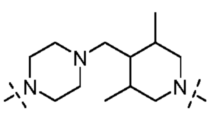, 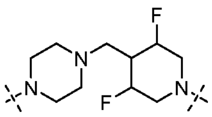, 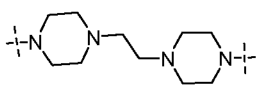,

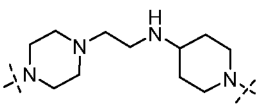, 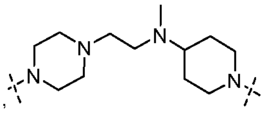, 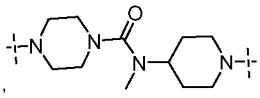, 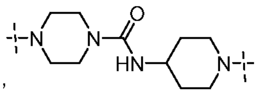,

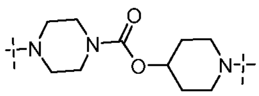, 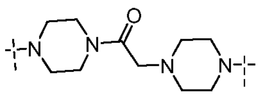, 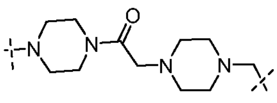, 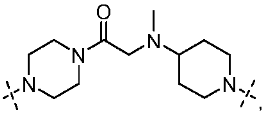,

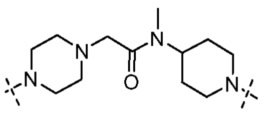, 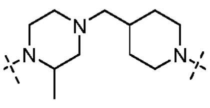, 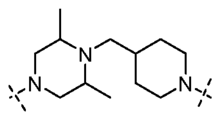, 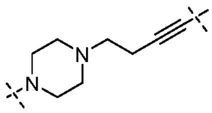,

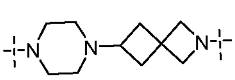, 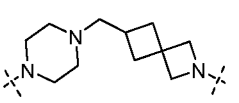, 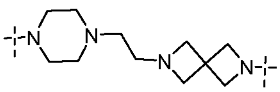, 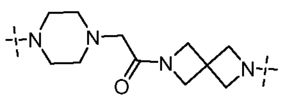,

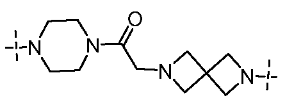, 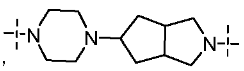, 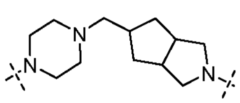, 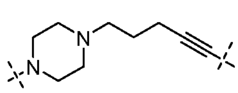,

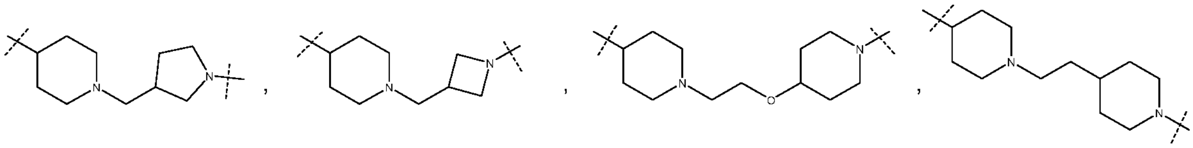

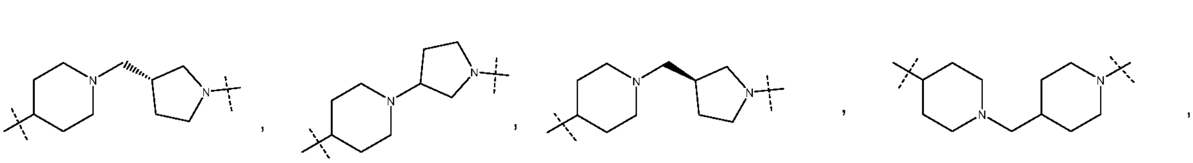

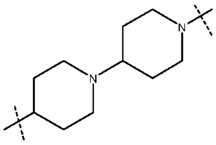 , 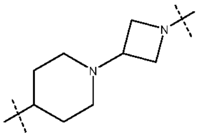 , 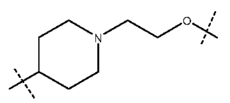 , 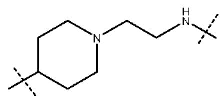

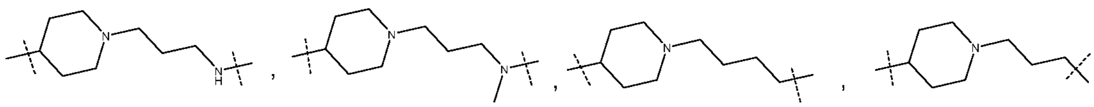

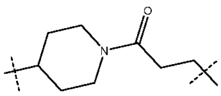 , 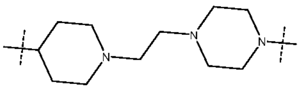 , 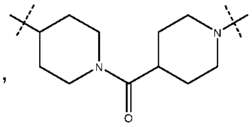 , 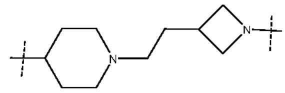 ,

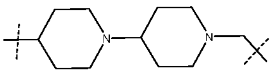 , 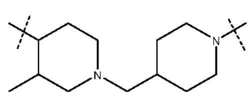 , 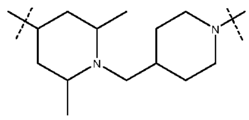 , 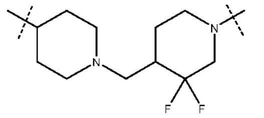 ,

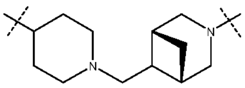 , 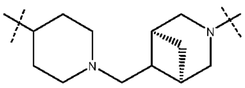 , 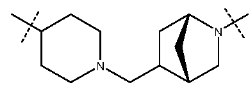 , 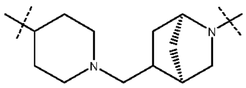 ,

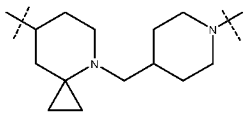 , 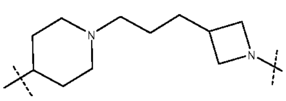 , 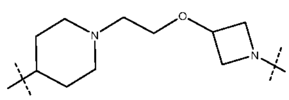 , 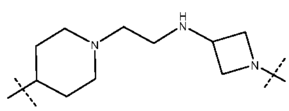 ,

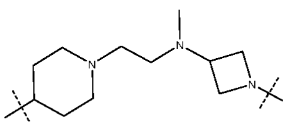 , 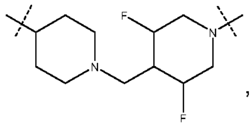 , 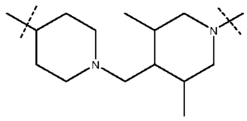 , 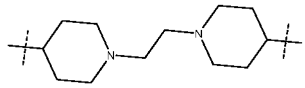 ,

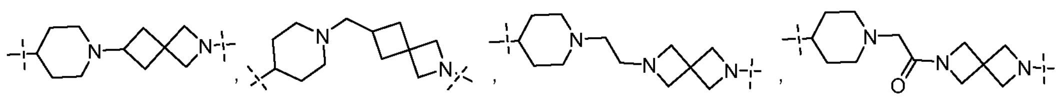

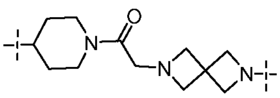 , 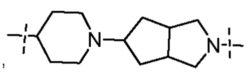 , 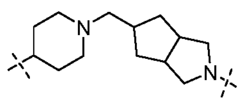 ,

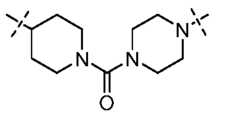 , 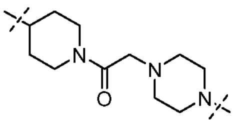 , 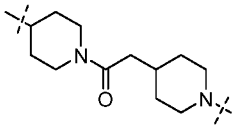 , 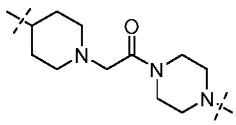 ,

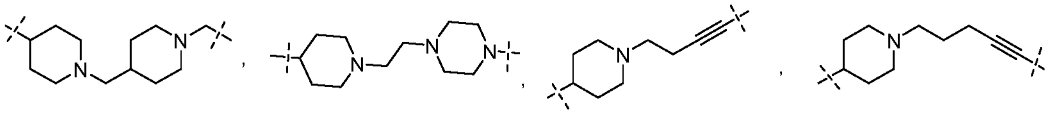

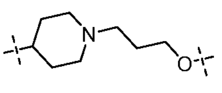, 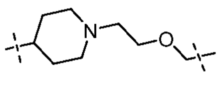, 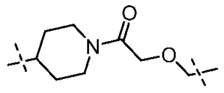, 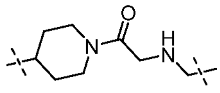,

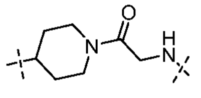, 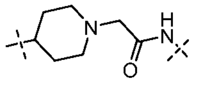, 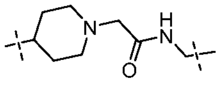, 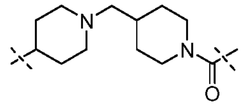,

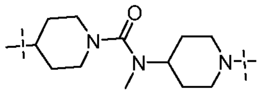, 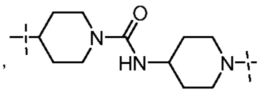, 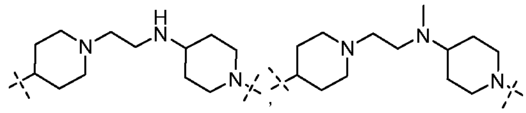,

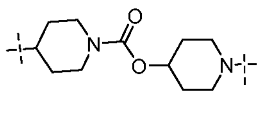, 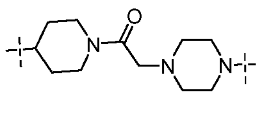, 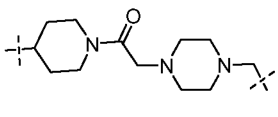, 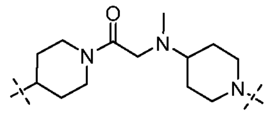,

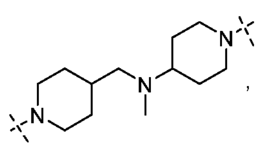, 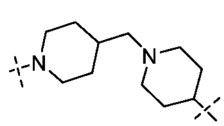, 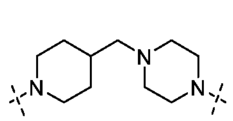, 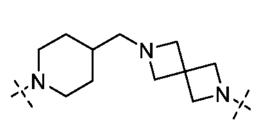,

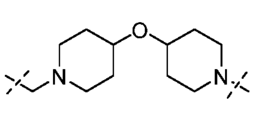, 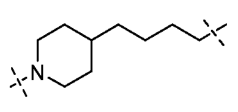, 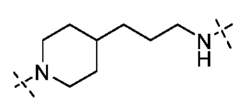, 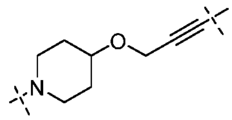,

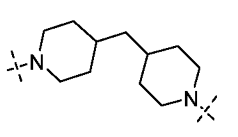, 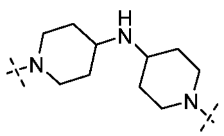, 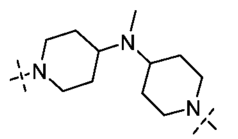, 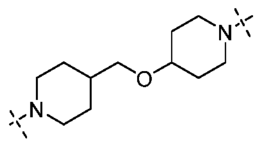,

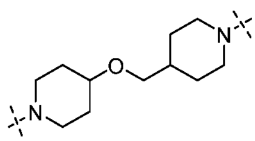, 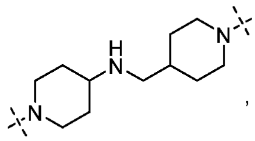, 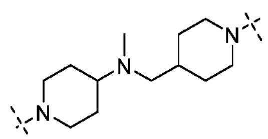, 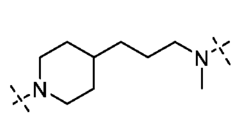,

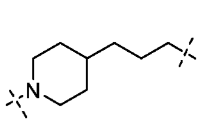, 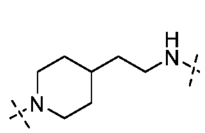, 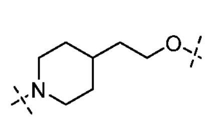, 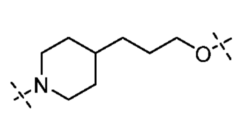,

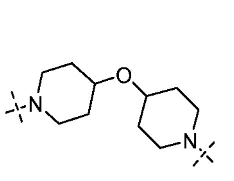, 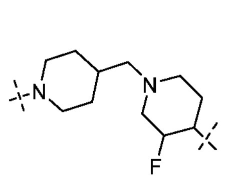, 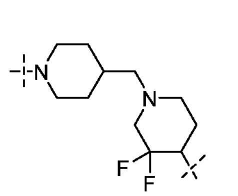, 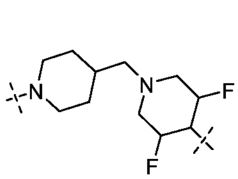

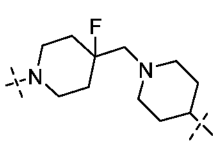 , 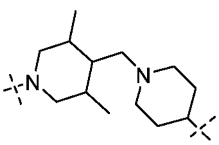 , 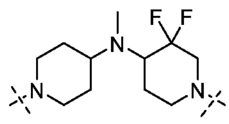 , 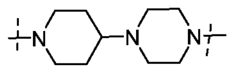 ,

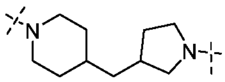 , 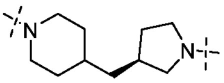 , 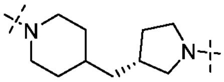 , 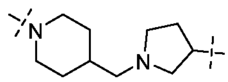

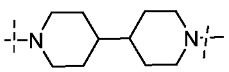 , 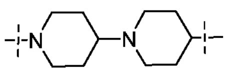 , 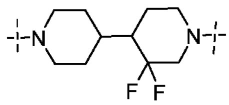 , 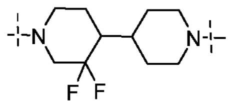

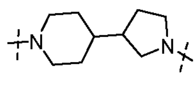 , 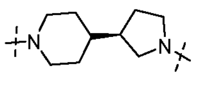 , 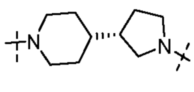 , 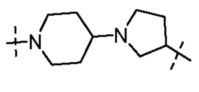 ,

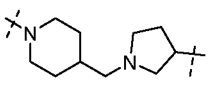 , 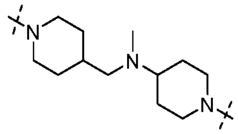 , 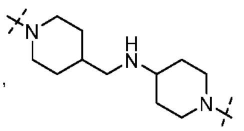 ,

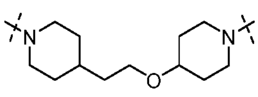 , 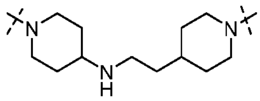 , 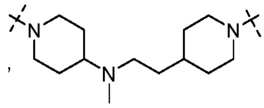 , 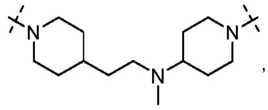 ,

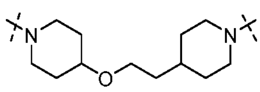 , 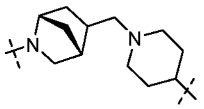 , 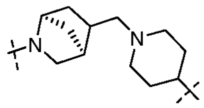 , 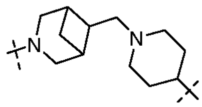 ,

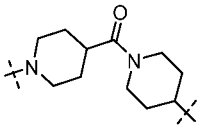 , 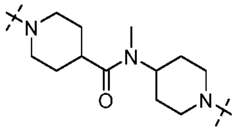 , 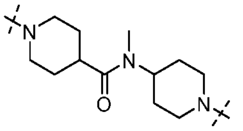 ,

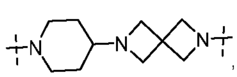 , 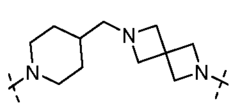 , 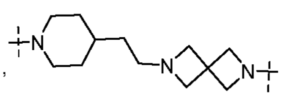 , 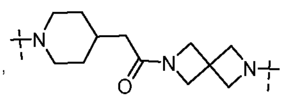 ,

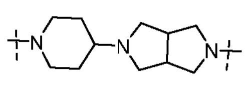 , 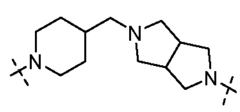 ,

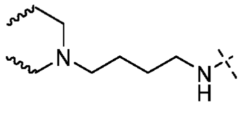 , 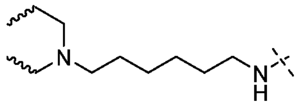 , 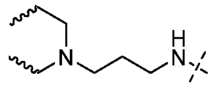 , 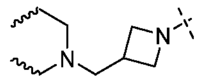 ,

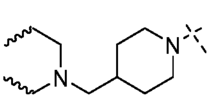 , 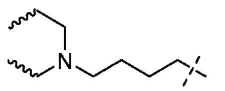 , 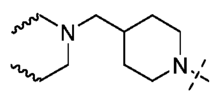 , 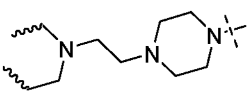 ,

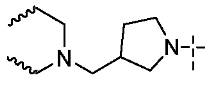 , 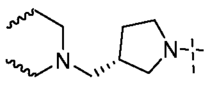 , 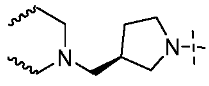 , 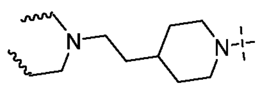 ,

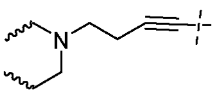 , 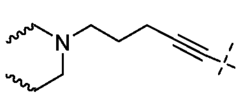 , 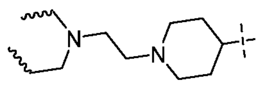 , 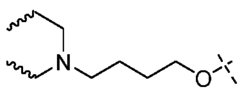 ,

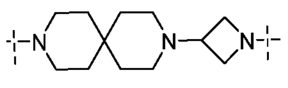 , 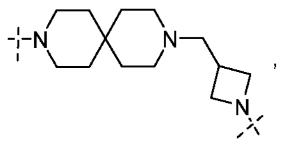 , 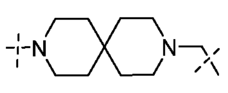 , 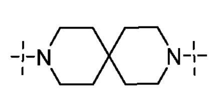 ,

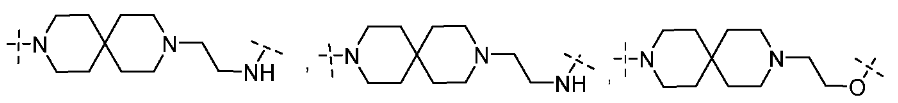 ,

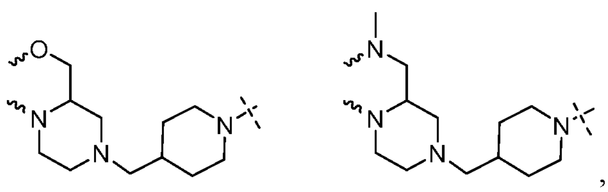 ,

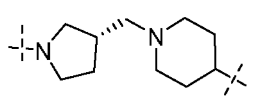 , 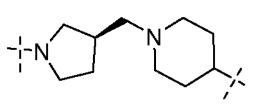 , 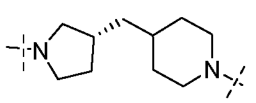 , 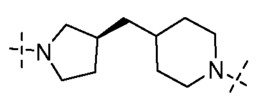 ,

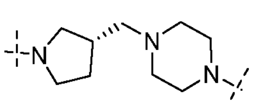 , 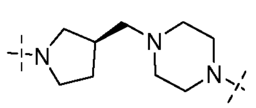 , 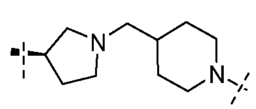 , 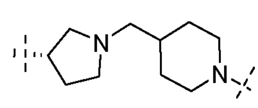 ,

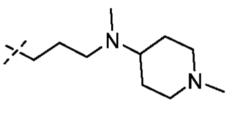 , 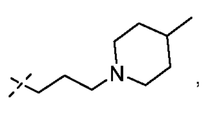 , 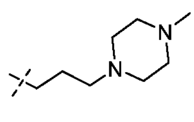 , 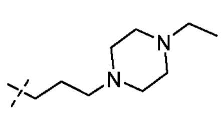 , 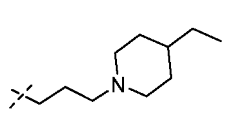 ,

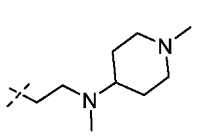 , 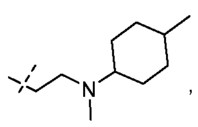 , 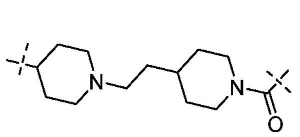 , 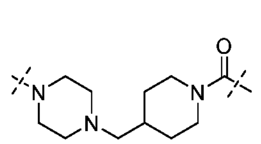 ,

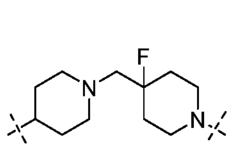 , 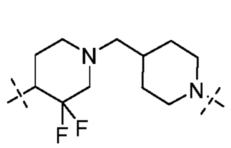 , 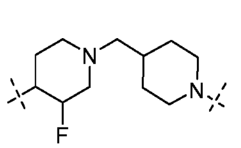 , 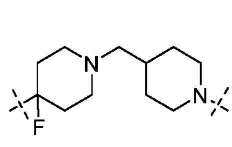

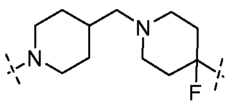 , 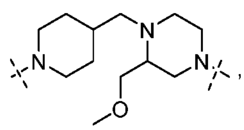 , 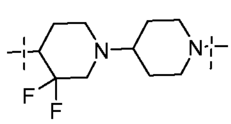 , 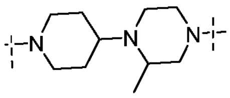 ,

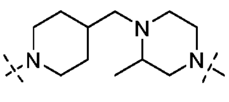 , 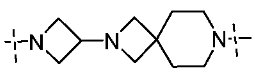 , 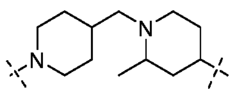 , 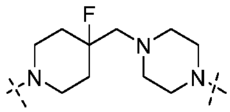 ,

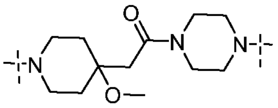 , 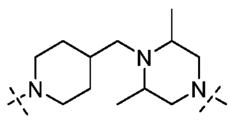 , 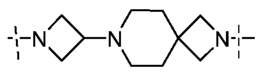 , 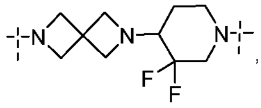 ,

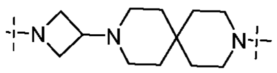 , 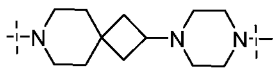 , 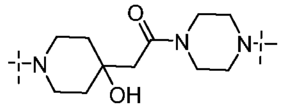 , 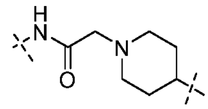

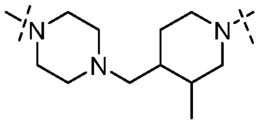 , 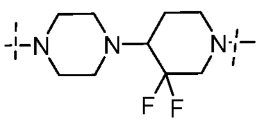 , 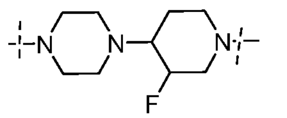 , 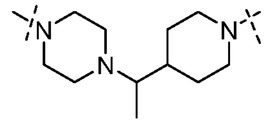 ,

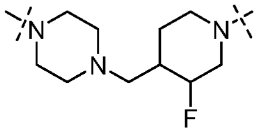 , 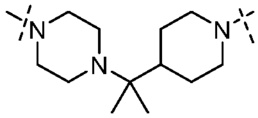 , 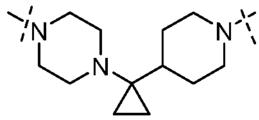 , 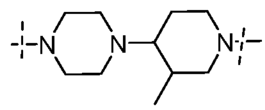 ,

wherein

 or 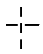

represents a linking site; and wherein the L group is optionally substituted with 1, 2, 3, or 4 substituents selected from halogen, hydroxyl, nitro, cyano, amino, mercapto, COOH, or $C_1$-$C_6$ alkyl.

In a second aspect of the 28th embodiment, the formula (XXXXI) is formula (XXXXII):

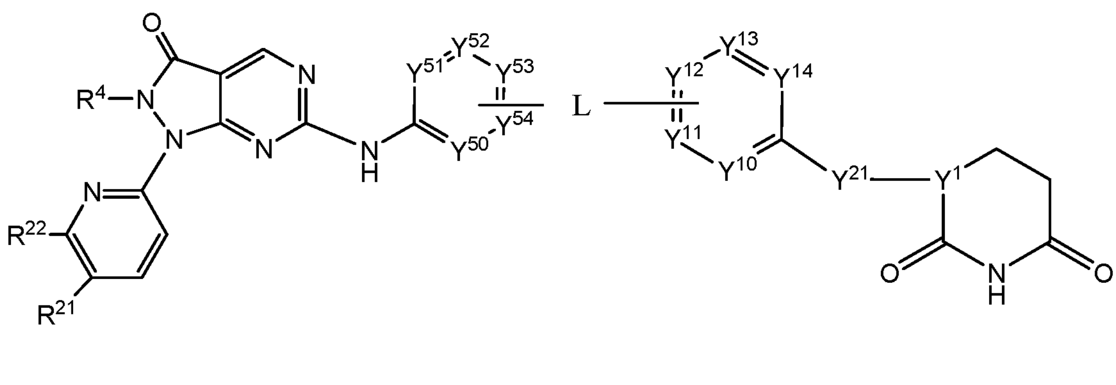

XXXXII

wherein,

$Y^1$, for each occurrence, is independently selected from CH, CD or N;

$Y^{21}$, for each occurrence, is independently selected from NH, N-$CH_3$, N-$C_2H_5$, N-$C_3H_7$, N-$C_4H_9$;

each of $Y^{10}$, $Y^{11}$, $Y^{12}$, $Y^{13}$ and $Y^{14}$, for each occurrence, is independently selected from N; $CT^{101}$, wherein $T^{101}$, for each occurrence, is independently selected from absence, hydrogen, fluorine, chlorine, methyl, $CH_2F$, $CHF_2$, $CF_3$, $CH_2Cl$, $CHCl_2$, $CCl_3$, ethyl, propyl, isopropyl, butyl, isobutyl, tert-butyl, methoxyl, ethoxyl, propoxyl, isopropoxyl, butoxyl, isobutoxyl, tert-butoxyl, hydroxyl, cyano;

one of $Y^{10}$, $Y^{11}$, $Y^{12}$, $Y^{13}$ and $Y^{14}$ is linked to L groups;

each of $Y^{50}$, $Y^{51}$, $Y^{52}$, $Y^{53}$ and $Y^{54}$, for each occurrence, is independently selected from N; $CT^{102}$, wherein $T^{102}$, for each occurrence, is independently selected from absence, hydrogen, fluorine, chlorine, methyl, $CH_2F$, $CHF_2$, $CF_3$, $CH_2Cl$, $CHCl_2$, $CCl_3$, ethyl, propyl, isopropyl, butyl, isobutyl, tert-butyl, methoxyl, ethoxyl, propoxyl, isopropoxyl, butoxyl, isobutoxyl, tert-butoxyl, hydroxyl, cyano;

one or more of $Y^{50}$, $Y^{51}$, $Y^{52}$, $Y^{53}$ and $Y^{54}$ are linked to L groups;

$R^4$, for each occurrence, is independently selected from allyl, propargyl, methylene cyclopropyl, ethyl, trifluoroethyl, difluoroethyl, isopropyl, cyclopropyl;

each of $R^{21}$ and $R^{22}$, for each occurrence, is independently selected from hydrogen, or 2-hydroxyisopropyl; or, $R^{21}$, $R^{22}$ are joined together to form $-(CR^{91}R^{92})-(CH_2)-(CH_2)-$ or $-(CH_2)-(CH_2)-(CR^{95}R^{96})-$, wherein each of $R^{91}$, $R^{92}$, $R^{95}$, and $R^{96}$, for each occurrence, is independently selected from hydrogen, hydroxyl, methyl, ethyl, propyl;

L is a linker and has the following structure;

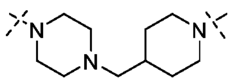 , 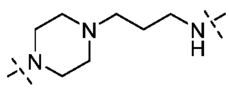 , 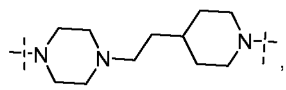 , 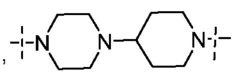 ,

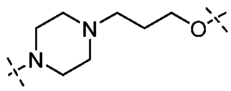 , 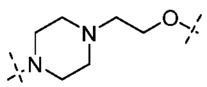 , 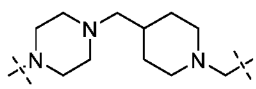 , 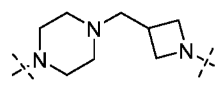 ,

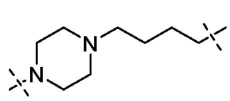 , 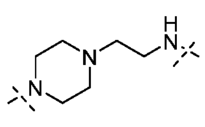 , 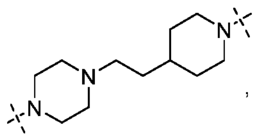 , 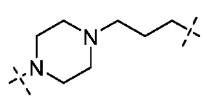 ,

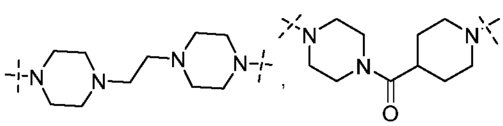 , 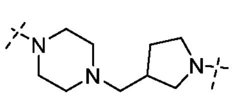 , 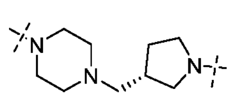 ,

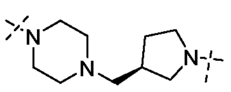 , 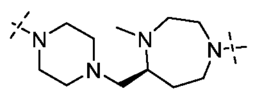 , 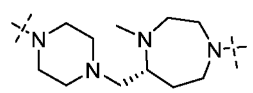 , 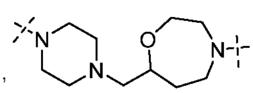 ,

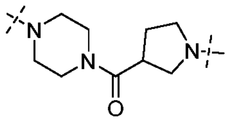 , 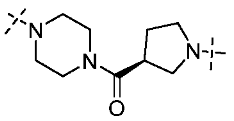 , 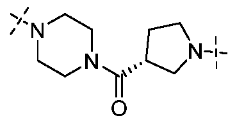 , 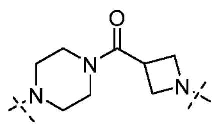 ,

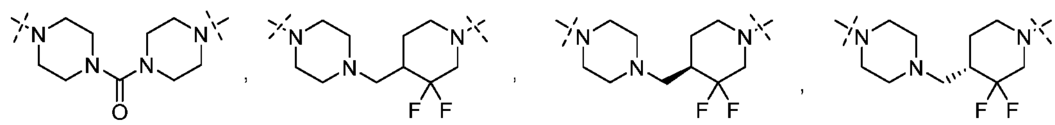
, , , ,

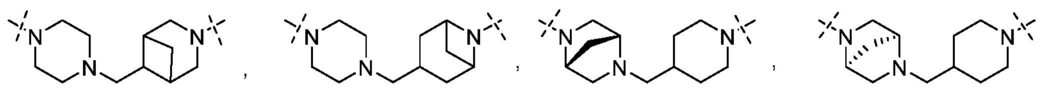
, , , ,

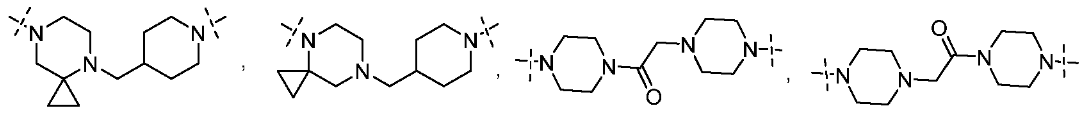
, , , ,

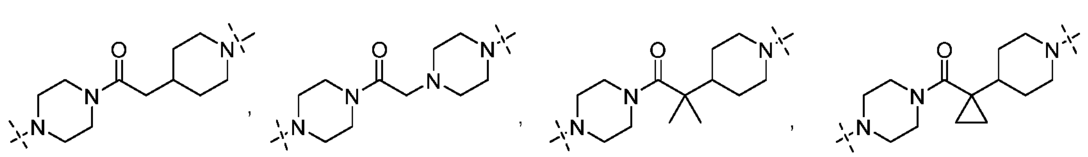
, , , ,

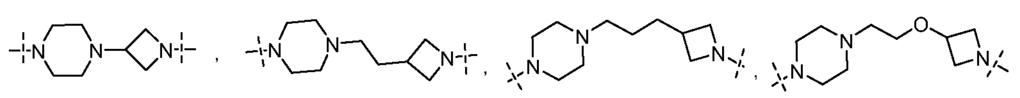
, , , ,

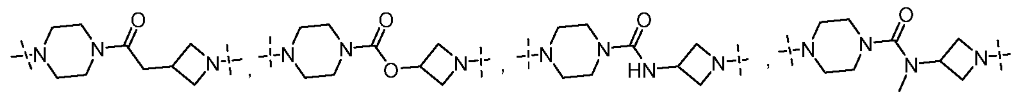
, , , ,

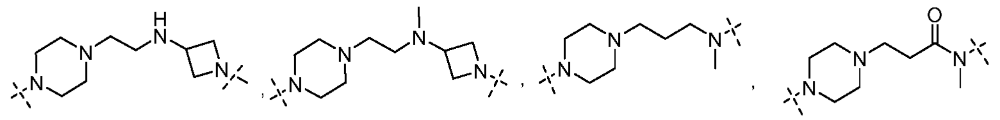
, , , ,

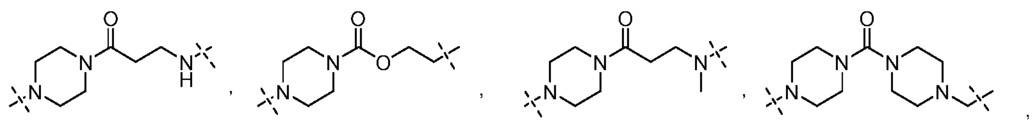
, , , ,

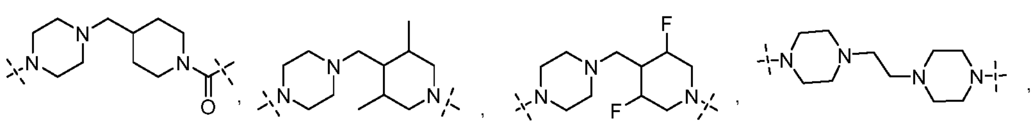
, , , ,

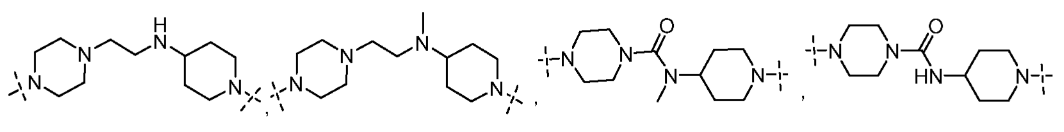
, , , ,

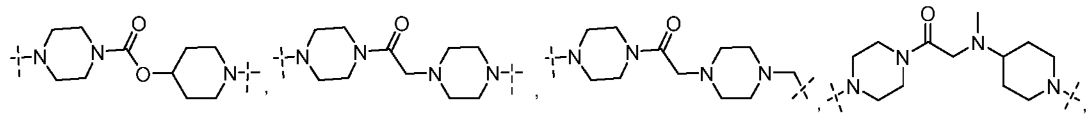
, , , ,

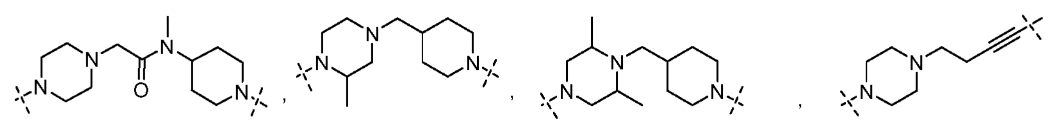
, , , ,

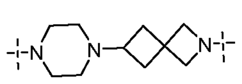 , 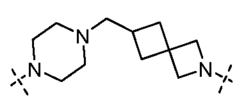 , 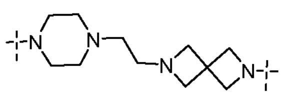 , 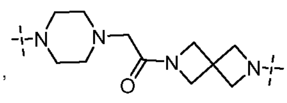 ,

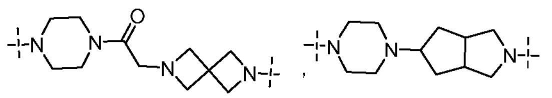 , , 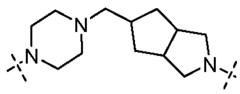 , 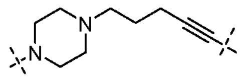 ,

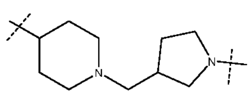 , 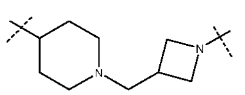 , 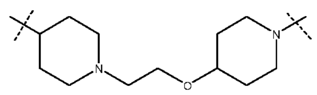 , 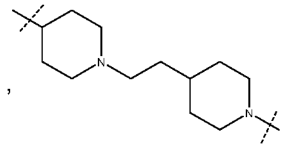

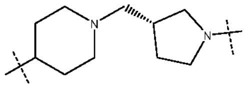 , 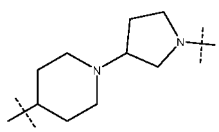 , 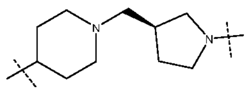 , 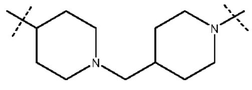 ,

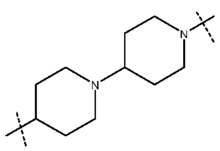 , 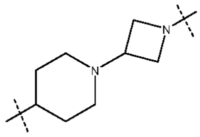 , 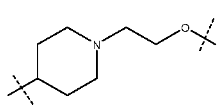 , 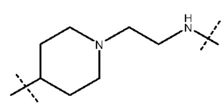

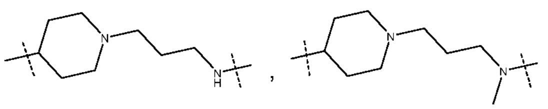 , , 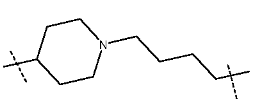 , 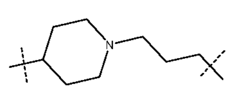 ,

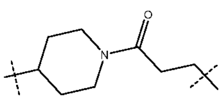 , 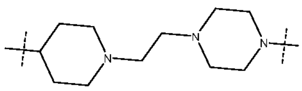 , 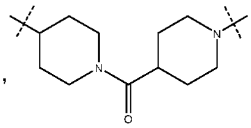 , 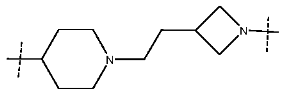 ,

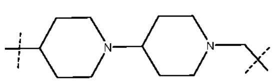 , 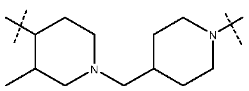 , 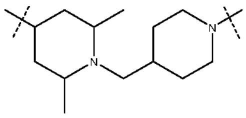 , 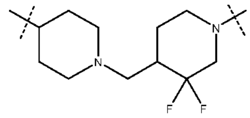 ,

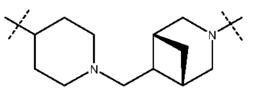 , 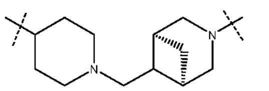 , 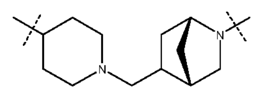 , 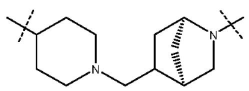 ,

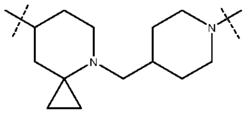 , 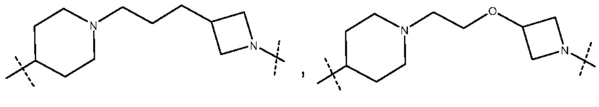 , , 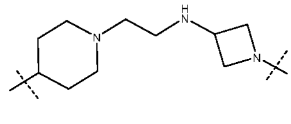 ,

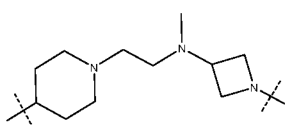 , 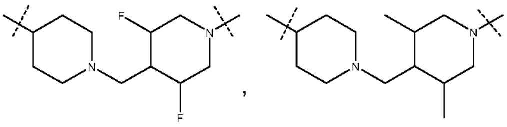 , , 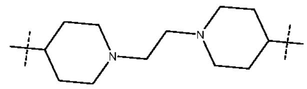 ,

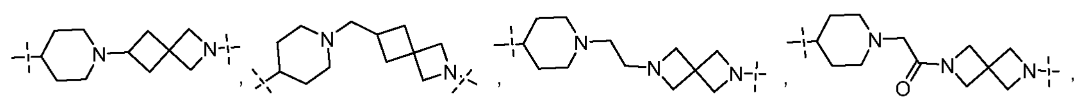

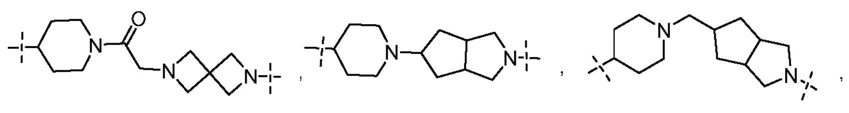

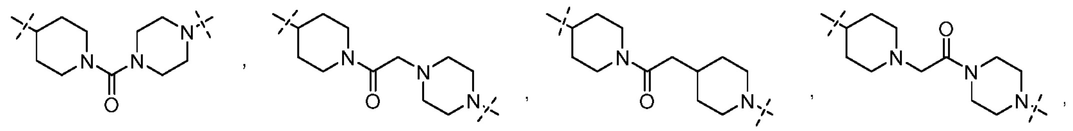

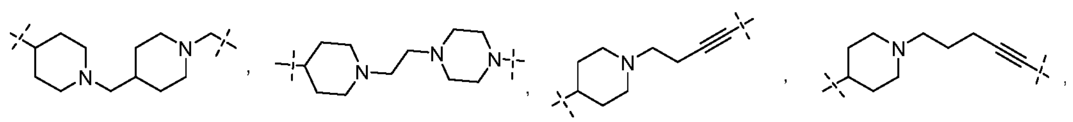

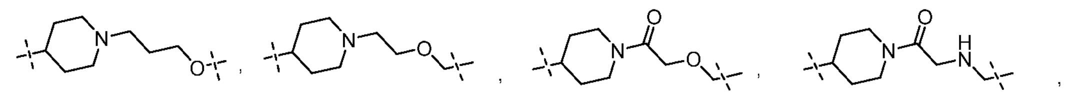

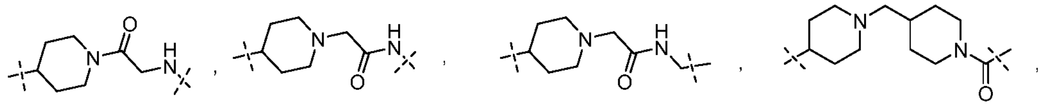

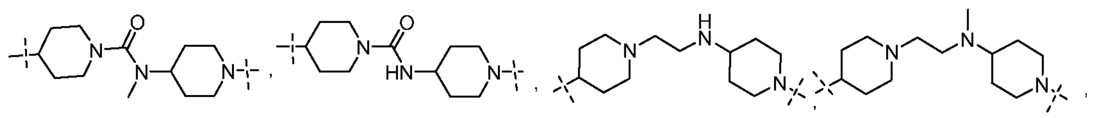

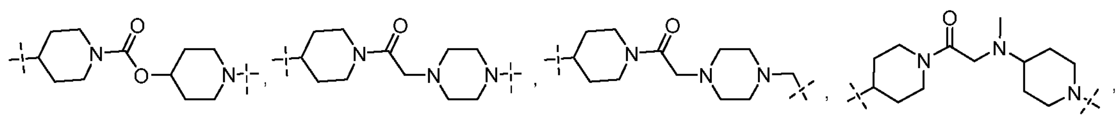

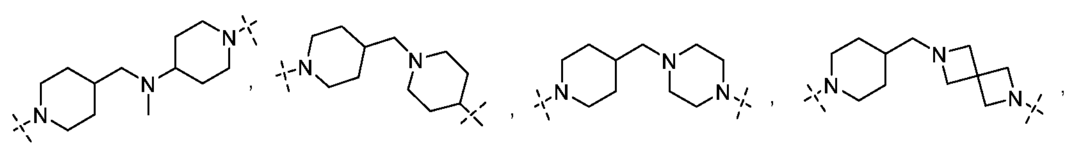

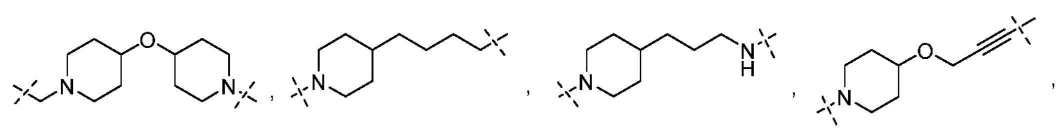

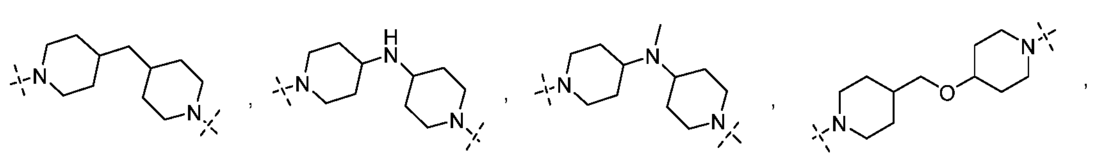

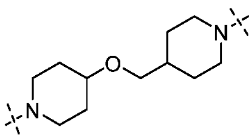 , 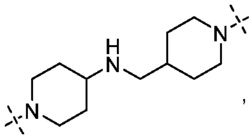 , 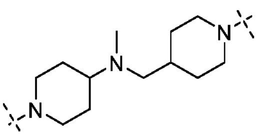 , 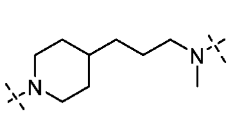 ,

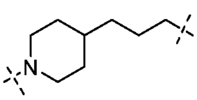 , 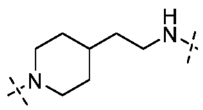 , 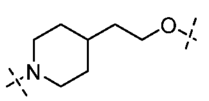 , 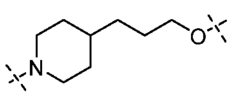 ,

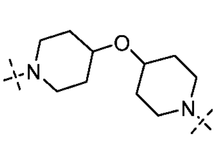 , 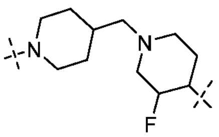 , 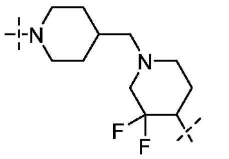 , 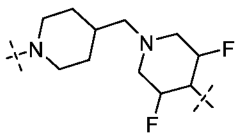

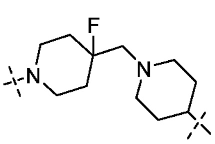 , 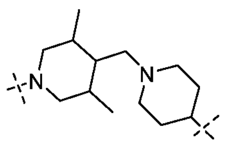 , 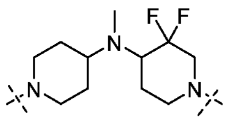 , 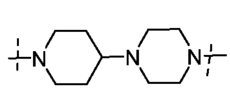 ,

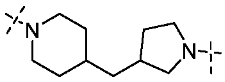 , 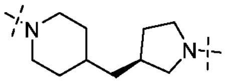 , 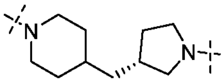 , 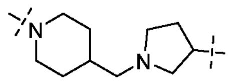

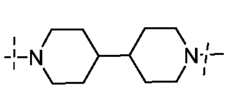 , 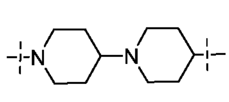 , 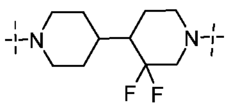 , 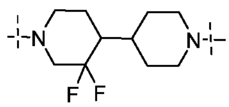

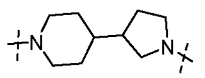 , 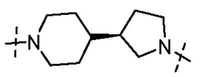 , 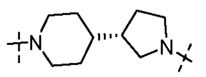 , 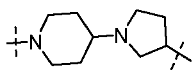 ,

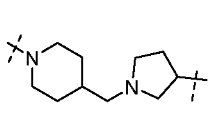 , 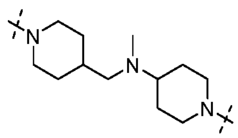 , 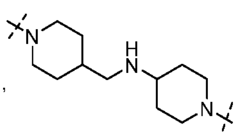 ,

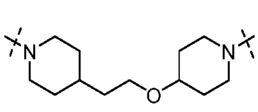 , 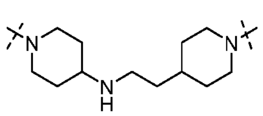 , 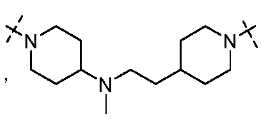 , 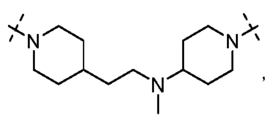 ,

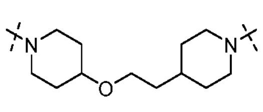 , 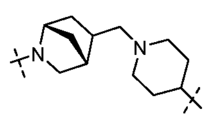 , 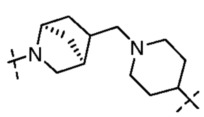 , 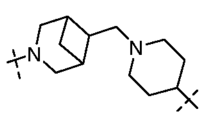 ,

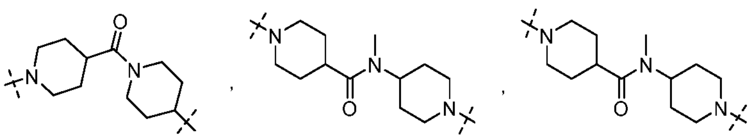 , , ,

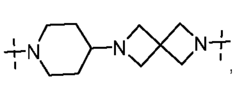, 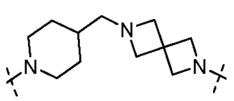, 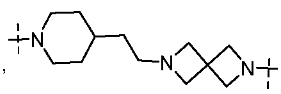, 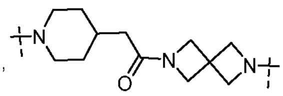,

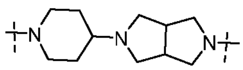, 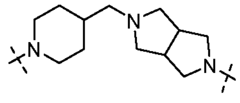,

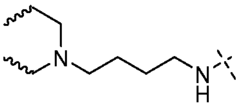, 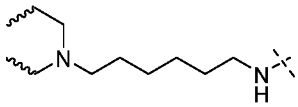, 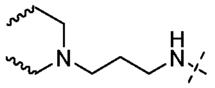, 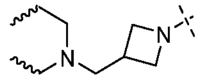,

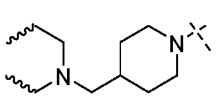, 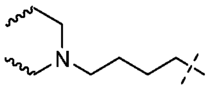, 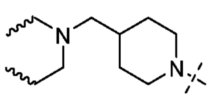, 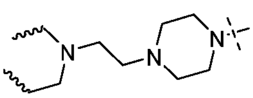,

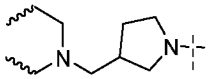, 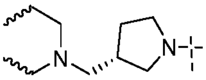, 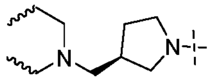, 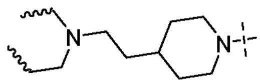,

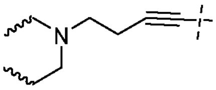, 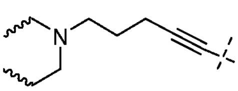, 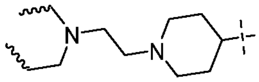, 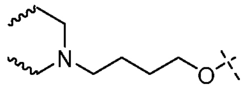,

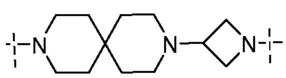, 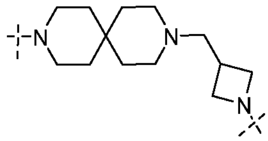, 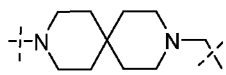, 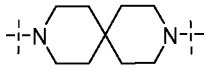,

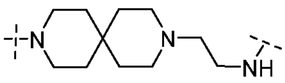, 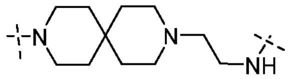, 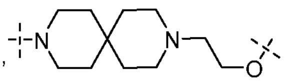,

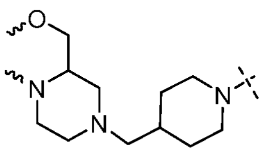 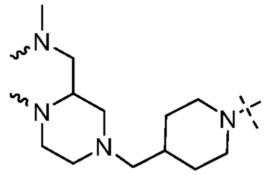,

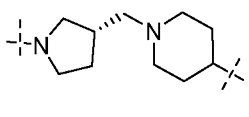, 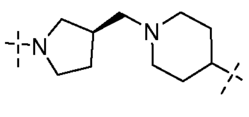, 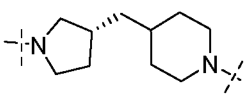, 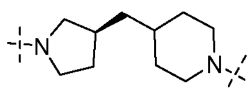,

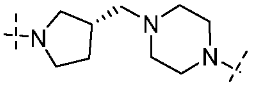, 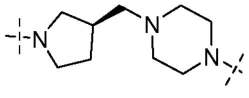, 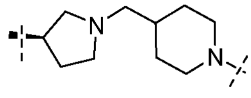, 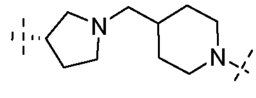,

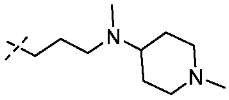, 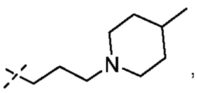, 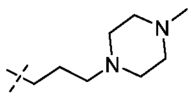, 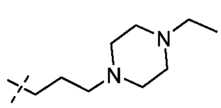, 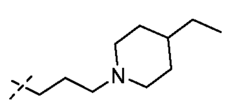,

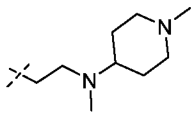, 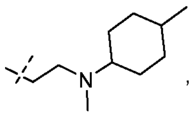, 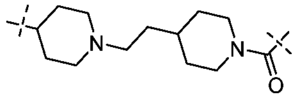, 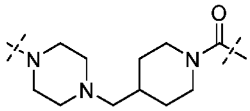,

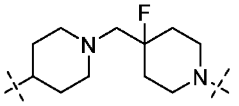, 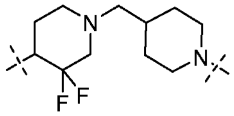, 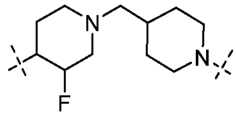, 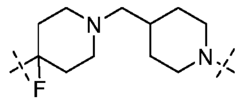

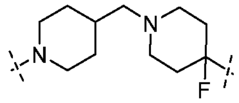, 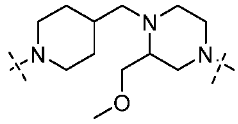, 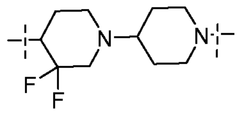, 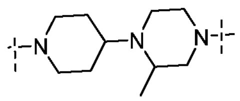,

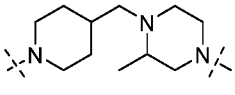, 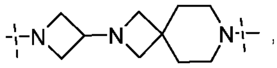, 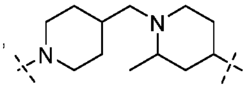, 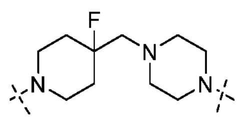,

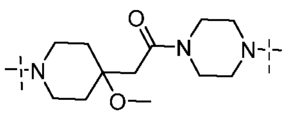, 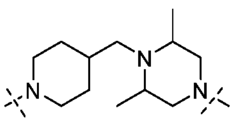, 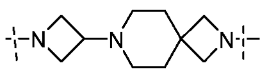, 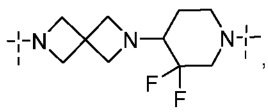,

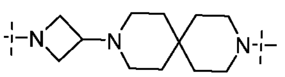, 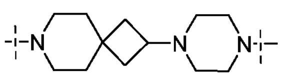, 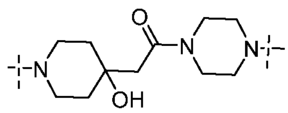, 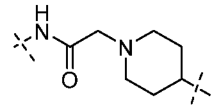

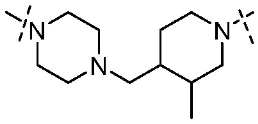, 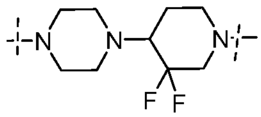, 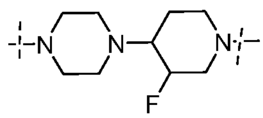, 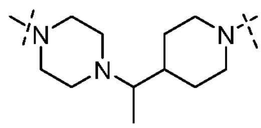,

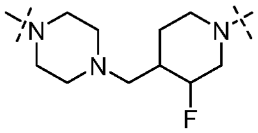, 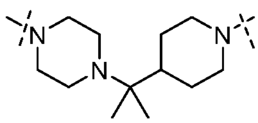, 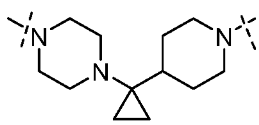, 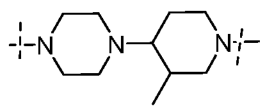,

wherein

 or 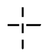

represents a linking site; and wherein the L group is optionally substituted with 1, 2, 3, or 4 substituents independently selected from halogen, hydroxyl, nitro, cyano, amino, mercapto, COOH, or $C_1$-$C_6$ alkyl.

In a third aspect of the 28th embodiment, the formula (XXXXII) is formula (XXXXIII):

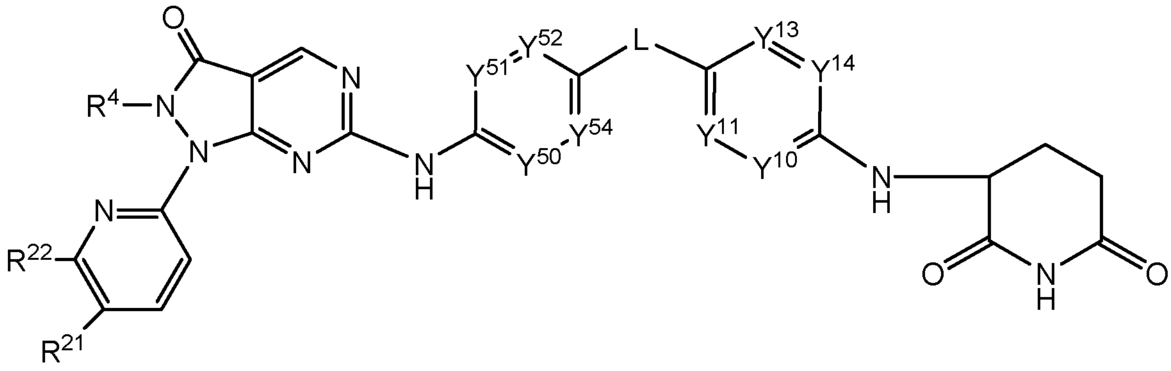

XXXXIII.

In a fourth aspect of the 28th embodiment, the formula (XXXXI) is formula (XXXXIV):

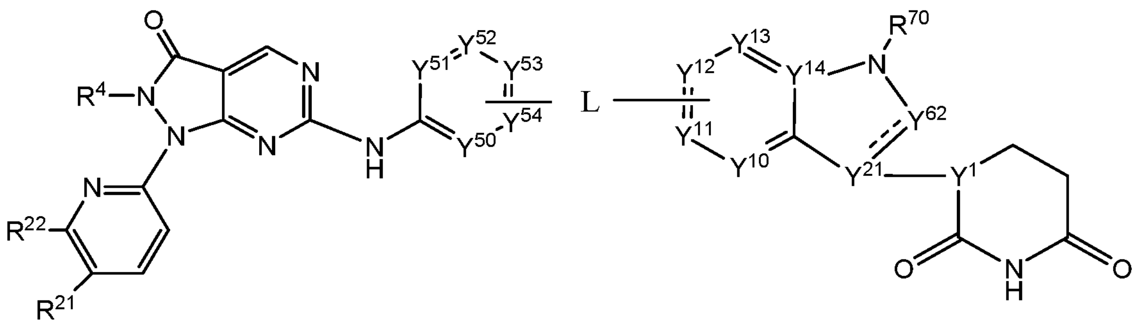

XXXXIV,

wherein,

$Y^1$, for each occurrence, is independently selected from CH, CD or N;

$Y^{21}$, for each occurrence, is independently selected from C, CH, CD, N;

each of $Y^{10}$, $Y^{11}$, $Y^{12}$, $Y^{13}$ and $Y^{14}$, for each occurrence, is independently selected from N; $CT^{101}$, wherein $T^{101}$, for each occurrence, is independently selected from absence, hydrogen, fluorine, chlorine, methyl, $CH_2F$, $CHF_2$, $CF_3$, $CH_2Cl$, $CHCl_2$, $CCl_3$, ethyl, propyl, isopropyl, butyl, isobutyl, tert-butyl, methoxyl, ethoxyl, propoxyl, isopropoxyl, butoxyl, isobutoxyl, tert-butoxyl, hydroxyl, cyano;

one of $Y^{10}$, $Y^{11}$, $Y^{12}$, $Y^{13}$ and $Y^{14}$ is linked to L groups;

each of $Y^{50}$, $Y^{51}$, $Y^{52}$, $Y^{53}$ and $Y^{54}$, for each occurrence, is independently selected from N; $CT^{102}$, wherein $T^{102}$, for each occurrence, is independently selected from absence, hydrogen, fluorine, chlorine, methyl, $CH_2F$, $CHF_2$, $CF_3$, $CH_2Cl$, $CHCl_2$, $CCl_3$, ethyl, propyl, isopropyl, butyl, isobutyl, tert-butyl, methoxyl, ethoxyl, propoxyl, isopropoxyl, butoxyl, isobutoxyl, tert-butoxyl, hydroxyl, cyano;

one or more of $Y^{50}$, $Y^{51}$, $Y^{52}$, $Y^{53}$ and $Y^{54}$ are linked to L groups;

$Y^{62}$, for each occurrence, is independently selected from N, NH, -(C=O)- or $-CH_2-$;

$R^{70}$, for each occurrence, is independently selected from hydrogen, methyl, ethyl or propyl;

------

represents a single bond or a double bond;

$R^4$, for each occurrence, is independently selected from allyl, propargyl, methylene cyclopropyl, ethyl, trifluoroethyl, difluoroethyl, isopropyl, cyclopropyl;

each of $R^{21}$ and $R^{22}$, for each occurrence, is independently selected from hydrogen, or 2-hydroxyisopropyl; or, $R^{21}$, $R^{22}$ are joined together to form $-(CR^{91}R^{92})-(CH_2)-(CH_2)-$ or $-(CH_2)-(CH_2)-(CR^{95}R^{96})-$, wherein each of $R^{91}$, $R^{92}$, $R^{95}$, and $R^{96}$, for each occurrence, is independently selected from hydrogen, hydroxyl, methyl, ethyl, propyl;

L is a linker and has the following structure;

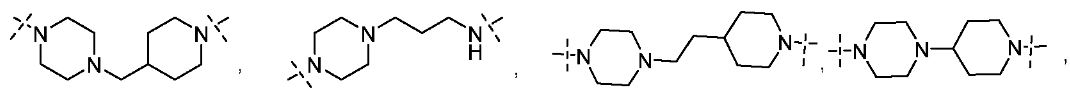
, , , ,

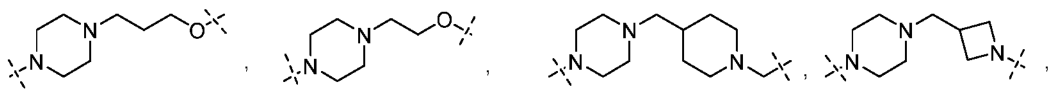
, , , ,

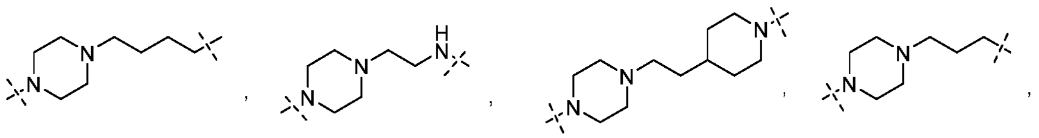
, , , ,

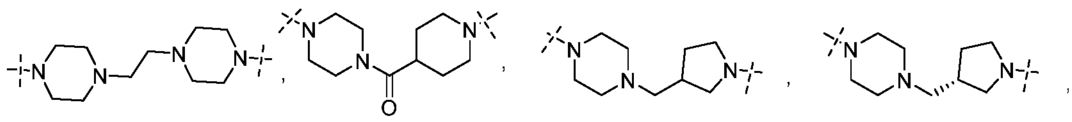
, , , ,

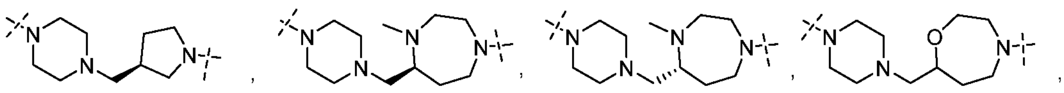
, , , ,

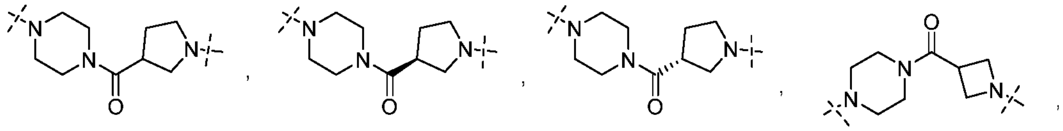
, , , ,

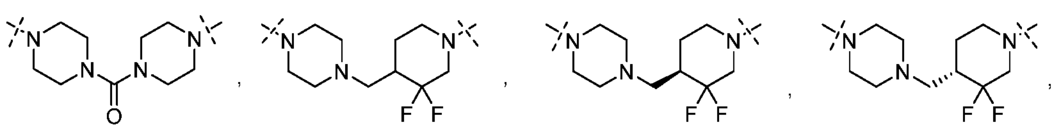
, , , ,

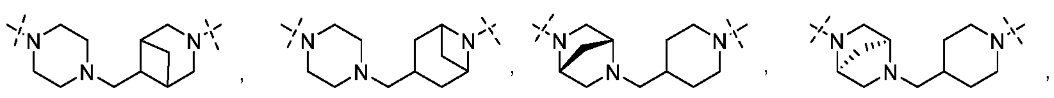
, , , ,

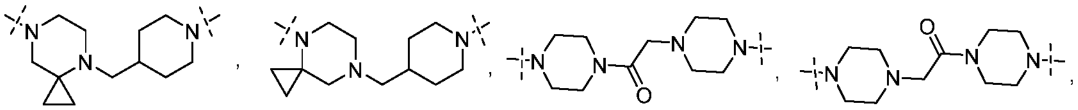
, , , ,

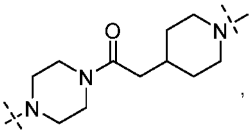, 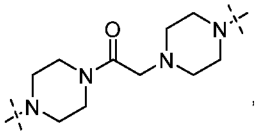, 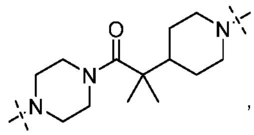, 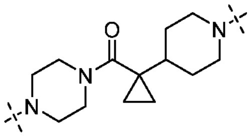,

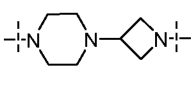, 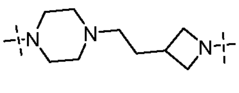, 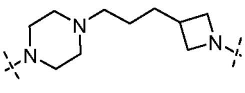, 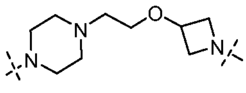,

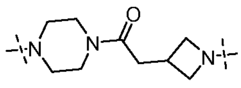, 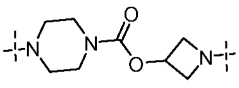, 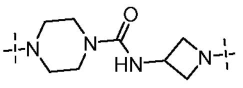, 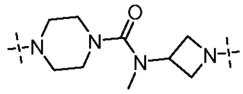,

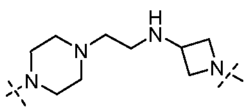, 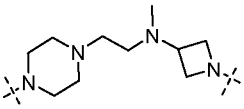, 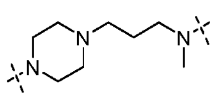, 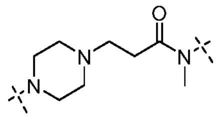,

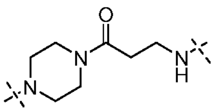, 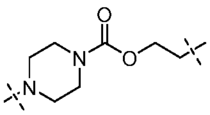, 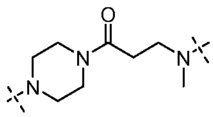, 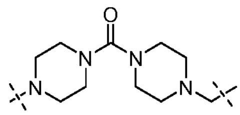,

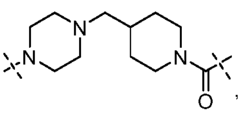, 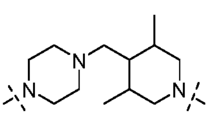, 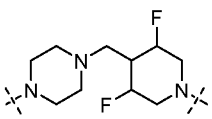, 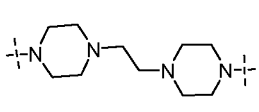,

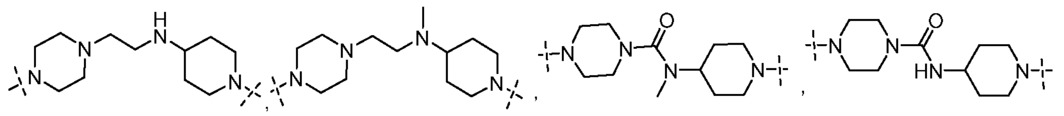,

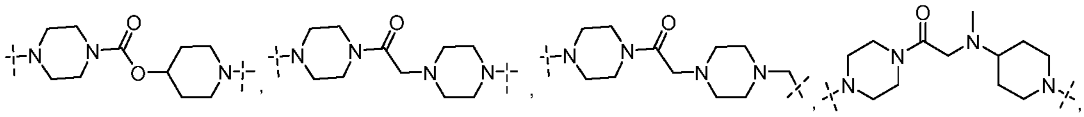,

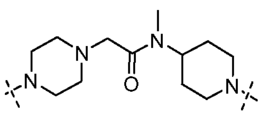, 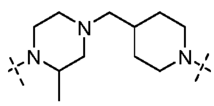, 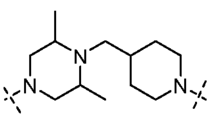, 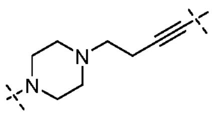,

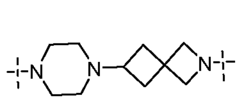, 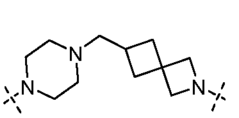, 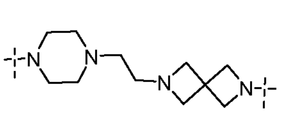, 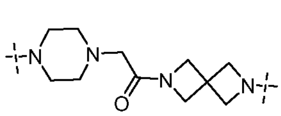,

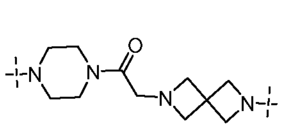, 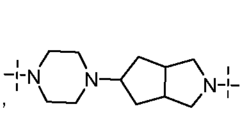, 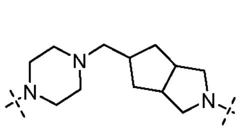, 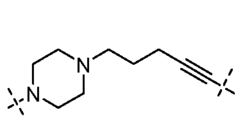,

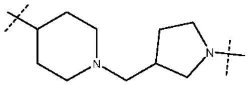 , 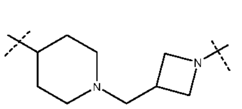 , 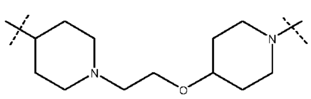 , 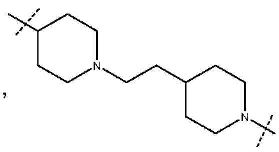

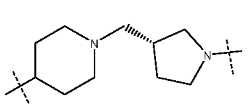 , 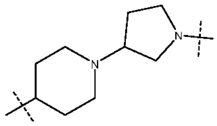 , 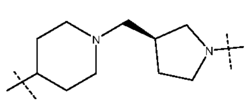 , 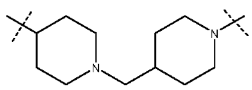 ,

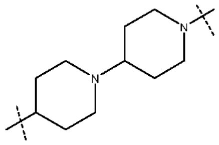 , 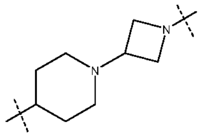 , 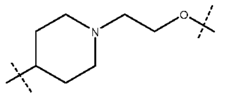 , 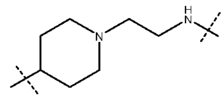

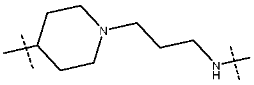 , 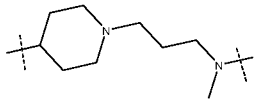 , 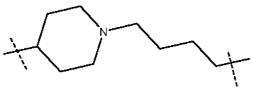 , 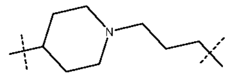 ,

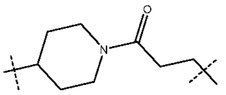 , 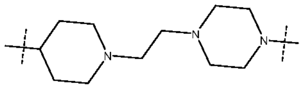 , 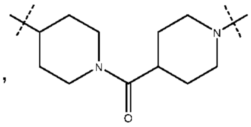 , 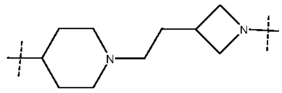 ,

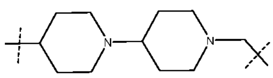 , 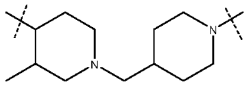 , 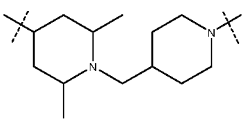 , 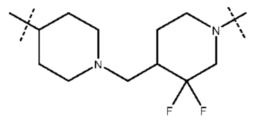 ,

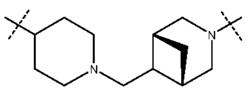 , 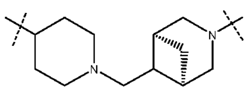 , 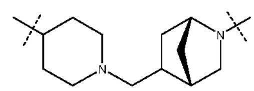 , 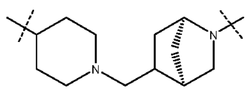 ,

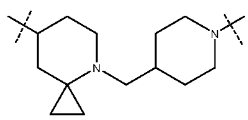 , 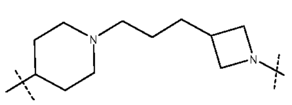 , 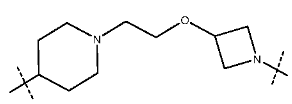 , 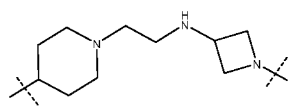

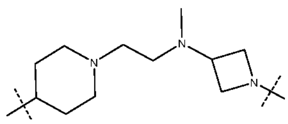 , 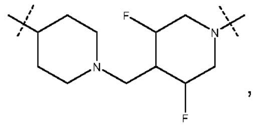 , 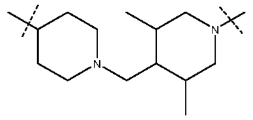 , 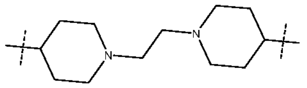 ,

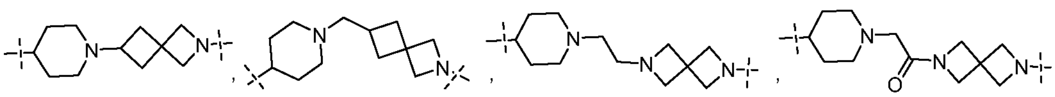 ,

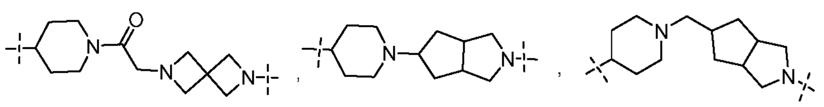 ,

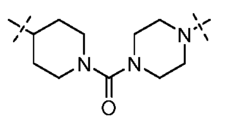 , 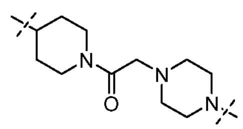 , 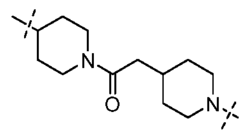 , 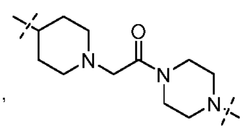 ,

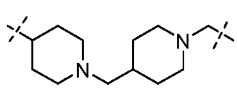 , 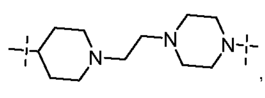 , 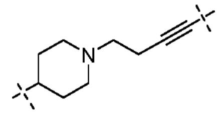 , 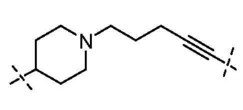 ,

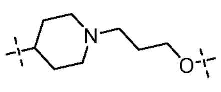 , 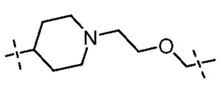 , 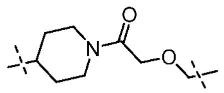 , 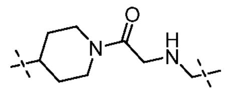 ,

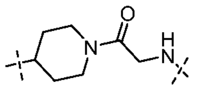 , 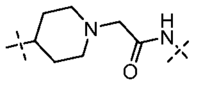 , 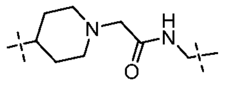 , 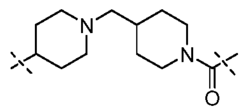 ,

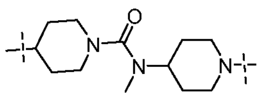 , 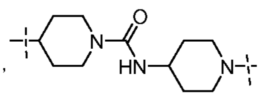 , 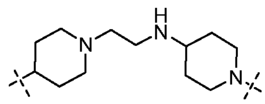 , 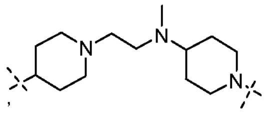 ,

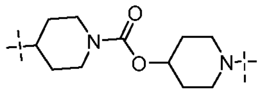 , 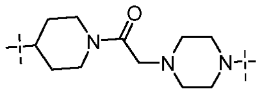 , 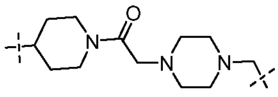 , 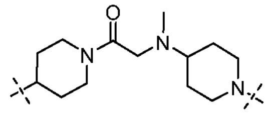 ,

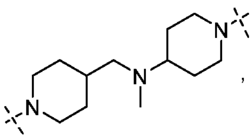 , 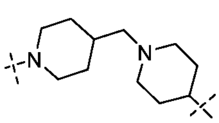 , 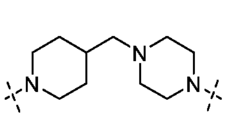 , 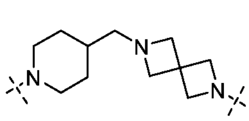 ,

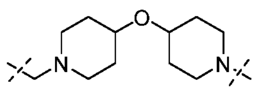 , 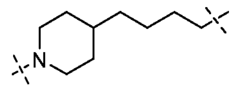 , 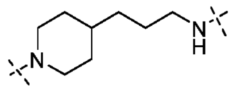 , 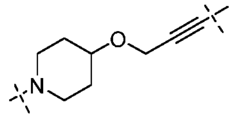 ,

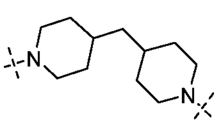 , 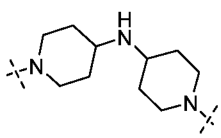 , 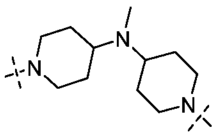 , 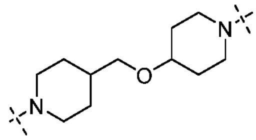 ,

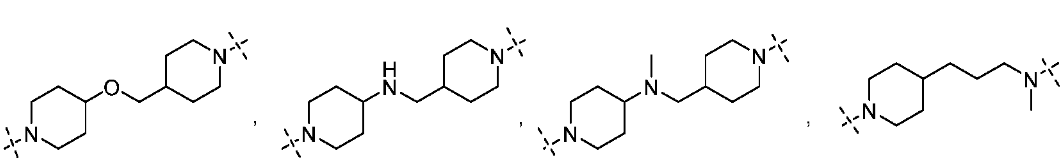 , , , ,

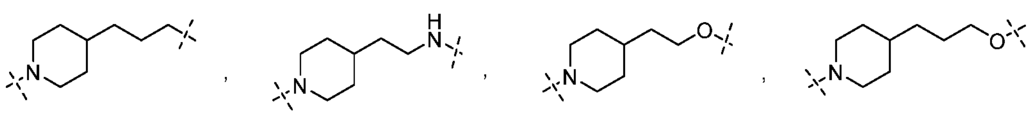 , , , ,

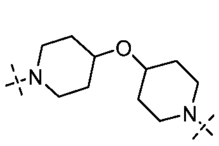 , 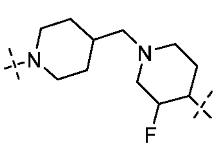 , 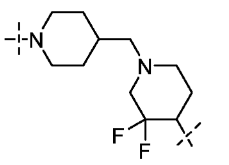 , 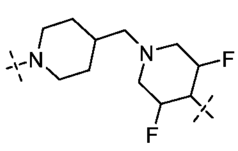

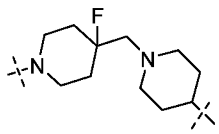 , 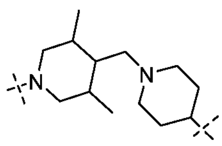 , 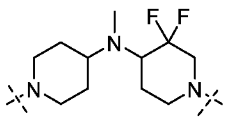 , 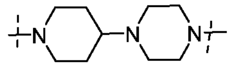 ,

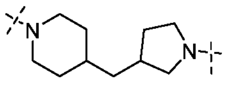 , 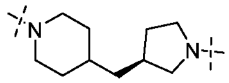 , 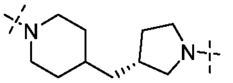 , 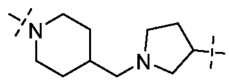

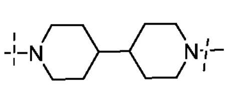 , 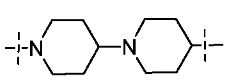 , 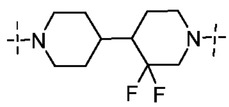 , 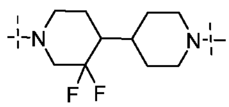

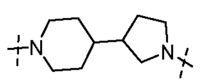 , 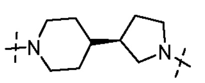 , 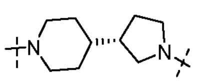 , 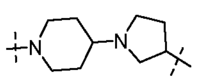 ,

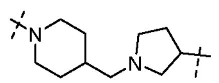 , 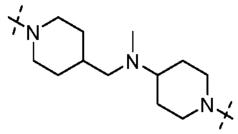 , 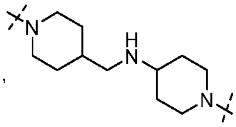 ,

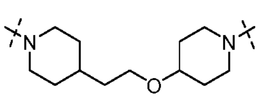 , 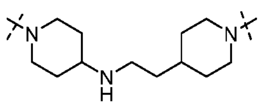 , 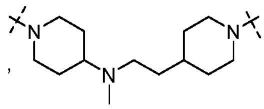 , 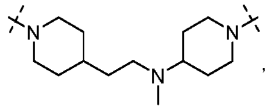 ,

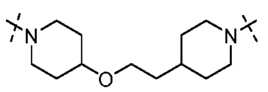 , 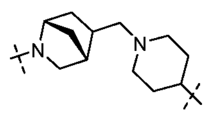 , 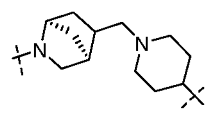 , 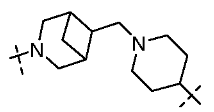 ,

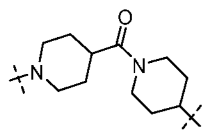 , 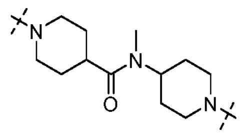 , 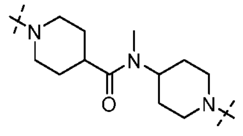 ,

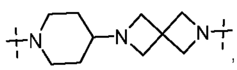 , 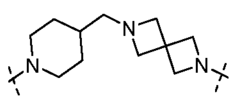 , 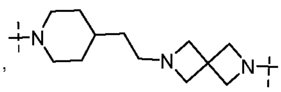 , 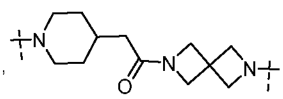 ,

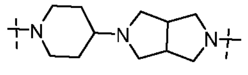 , 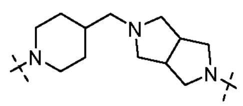 ,

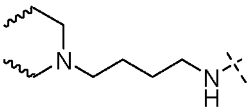, 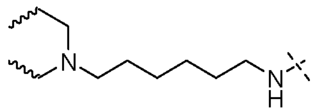, 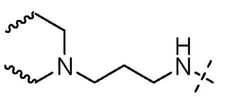, 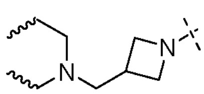,

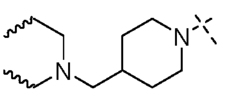, 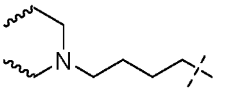, 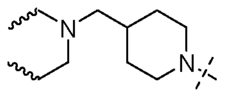, 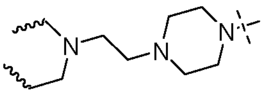,

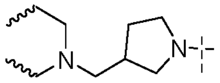, 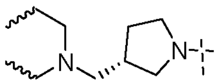, 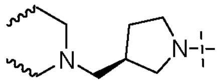, 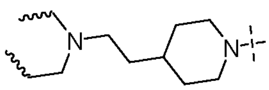,

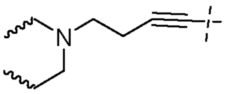, 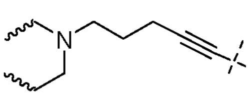, 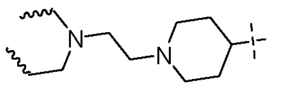, 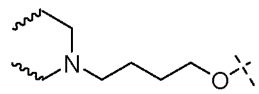,

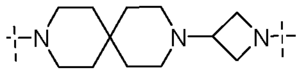, 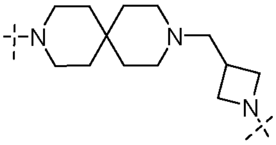, 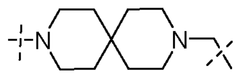, 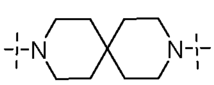,

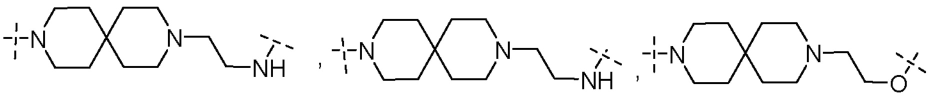

, , ,

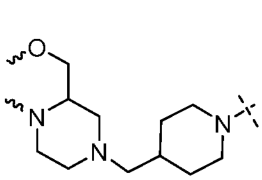 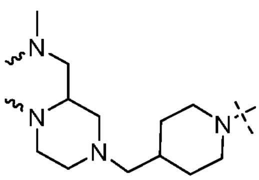,

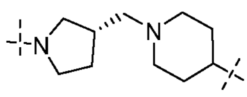, 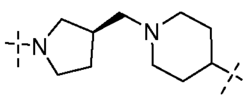, 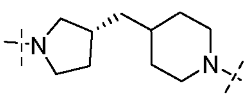, 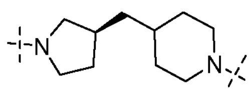,

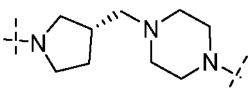, 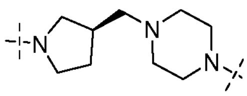, 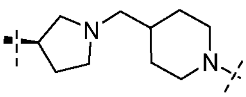, 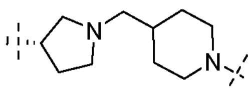,

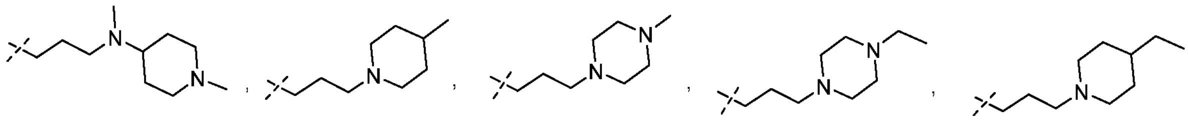

, , , , ,

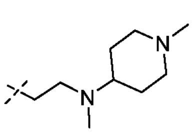, 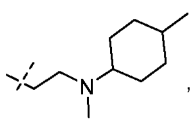, 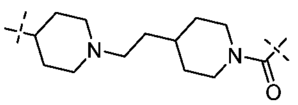, 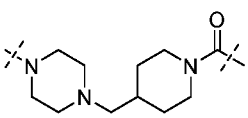,

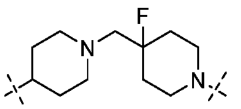 , 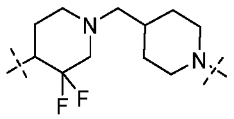 , 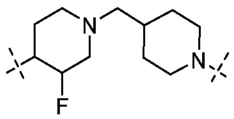 , 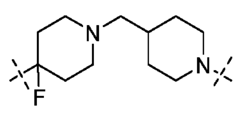 ,

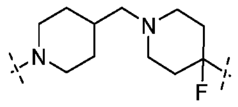 , 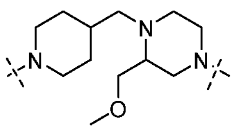 , 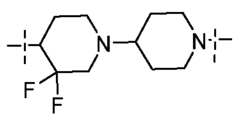 , 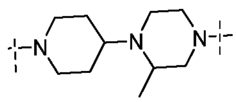 ,

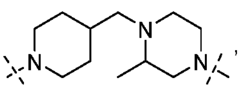 , 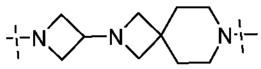 , 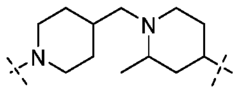 , 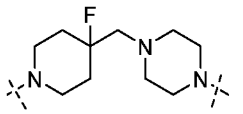 ,

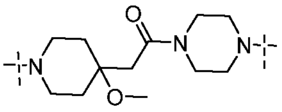 , 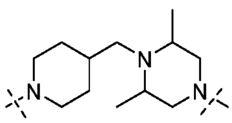 , 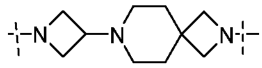 , 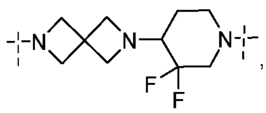 ,

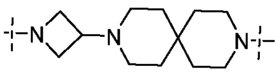 , 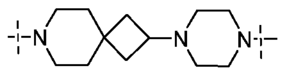 , 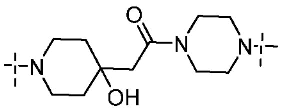 , 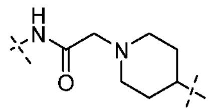

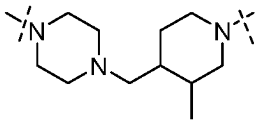 , 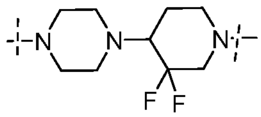 , 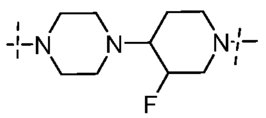 , 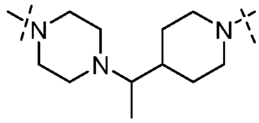 ,

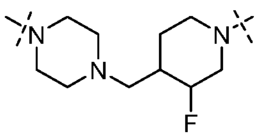 , 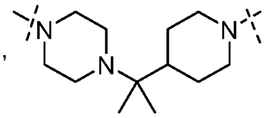 , 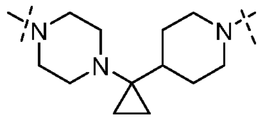 , 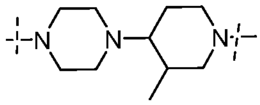 ,

wherein

 or 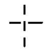

represents a linking site; and wherein the L group is optionally substituted with 1, 2, 3, or 4 substituents independently selected from halogen, hydroxyl, nitro, cyano, amino, mercapto, COOH, or $C_1$-$C_6$ alkyl.

In a fifth aspect of the 28th embodiment, the formula (XXXXIV) is formula (XXXXV) or formula (XXXXVI):

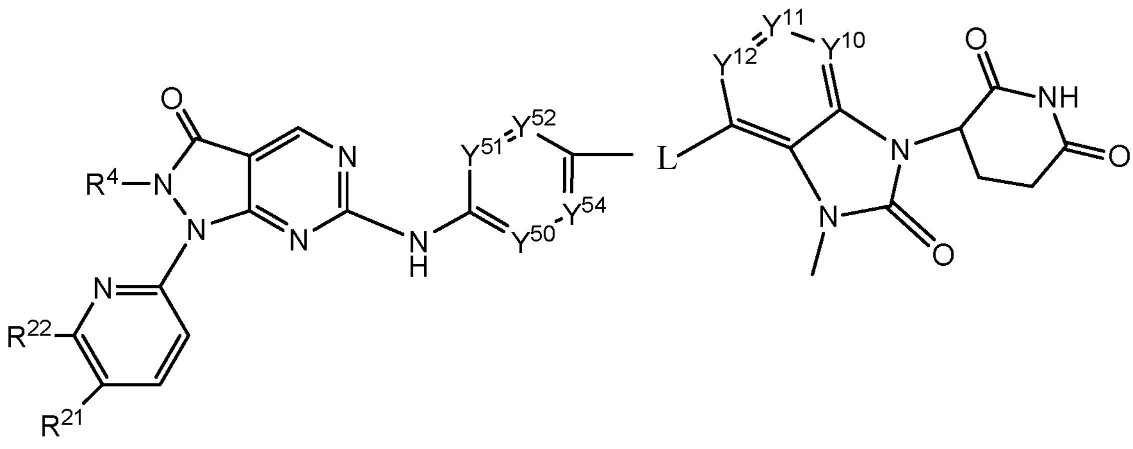

XXXXV;

or

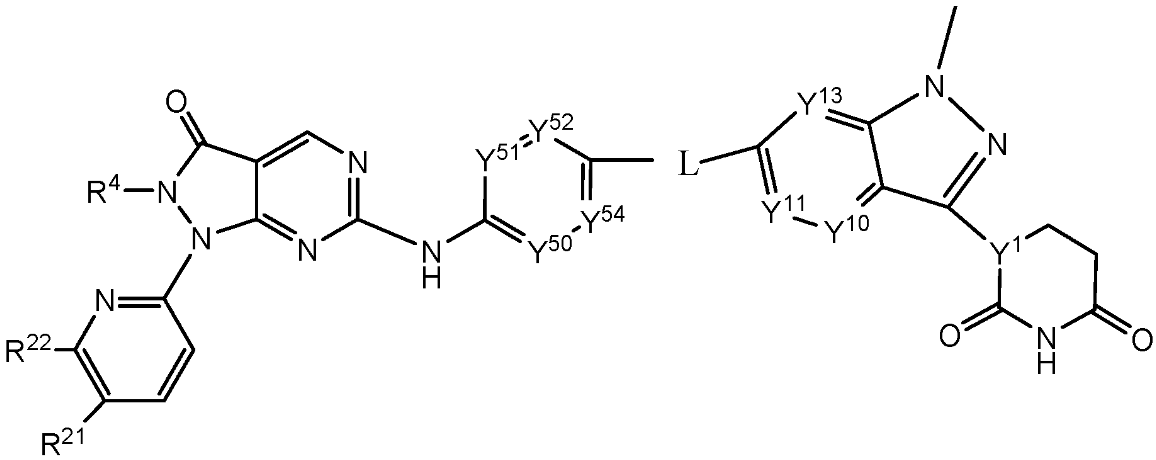

XXXXVI.

In the 29th embodiment, the present application relates to a compound represented by structural formula (XXXXVII), or a derivative, pharmaceutically acceptable salt, isomer, solvate, hydrate, adduct, complex or prodrug thereof:

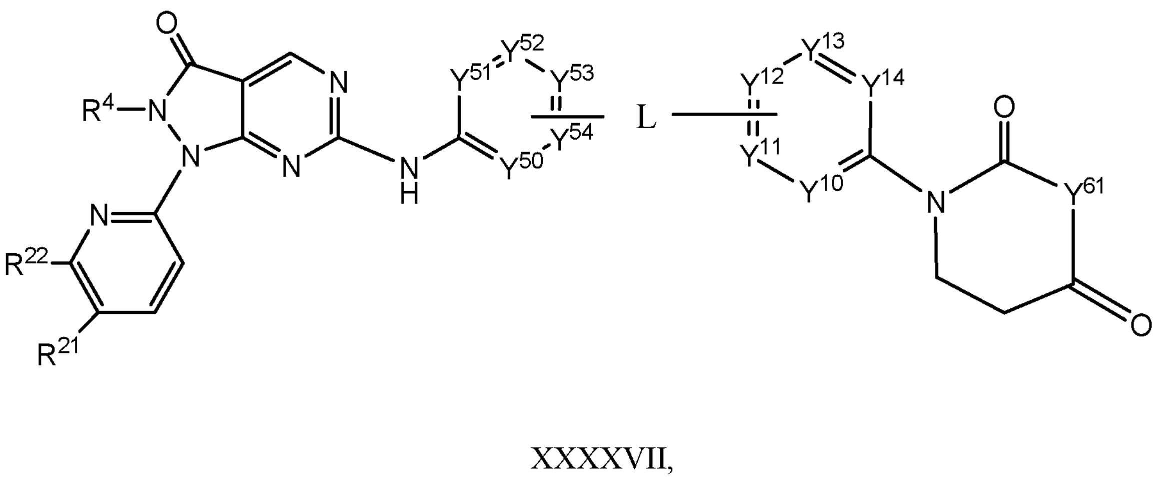

XXXXVII,

wherein,

each of $Y^{10}$, $Y^{11}$, $Y^{12}$, $Y^{13}$ and $Y^{14}$, for each occurrence, is independently selected from N; $CT^{101}$, wherein $T^{101}$, for each occurrence, is independently selected from absence, hydrogen, halogen, $C_1$-$C_6$ alkyl, $C_1$-$C_6$ haloalkyl, $C_1$-$C_6$ alkoxyl, hydroxyl, cyano;

one of $Y^{10}$, $Y^{11}$, $Y^{12}$, $Y^{13}$ and $Y^{14}$ is linked to L groups;

each of $Y^{50}$, $Y^{51}$, $Y^{52}$, $Y^{53}$ and $Y^{54}$, for each occurrence, is independently selected from N; $CT^{102}$, wherein $T^{102}$, for each occurrence, is independently selected from absence, hydrogen, halogen, $C_1$-$C_6$ alkyl, $C_1$-$C_6$ haloalkyl, $C_1$-$C_6$ alkoxyl, hydroxyl, cyano;

one or more of $Y^{50}$, $Y^{51}$, $Y^{52}$, $Y^{53}$ and $Y^{54}$ are linked to L groups;

$R^4$, for each occurrence, is independently selected from allyl, propargyl, methylene cyclopropyl, ethyl, trifluoroethyl, difluoroethyl, isopropyl, cyclopropyl, methylene $C_6$-$C_{12}$ aryl, methylene phenyl, optionally substituted $C_{1-4}$ alkyl, optionally substituted $C_{2-4}$ alkenyl, optionally substituted $C_{2-4}$ alkynyl, optionally substituted $C_{3-6}$ cycloalkyl and optionally substituted $C_{3-6}$ cycloalkyl ($C_{1-4}$ alkyl), wherein the $C_6$-$C_{12}$ aryl, $C_{1-4}$ alkyl, $C_{2-4}$ alkenyl and $C_{2-4}$ alkynyl are optionally independently substituted with one or more substituents selected from halogen, $C_{1-4}$ alkoxyl, $C_{1-4}$ haloalkyl, $C_{1-4}$ haloalkoxyl, cyano, amino, mono-$C_{1-4}$ alkylamine and di-$C_{1-4}$ alkylamine, and wherein one or more rings of the $C_{3-6}$ cycloalkyl and the $C_{3-6}$ cycloalkyl ($C_{1-4}$ alkyl) are optionally independently substituted with one or more substituents selected from halogen, $C_{1-4}$ alkyl, $C_{1-4}$ alkoxyl, $C_{1-4}$ haloalkyl, $C_{1-4}$ haloalkoxyl, cyano, amino, mono-$C_{1-4}$ alkylamine and di-$C_{1-4}$ alkylamine,

$Y^{61}$, for each occurrence, is independently selected from $CH_2$, NH, N($C_1$-$C_6$ alkyl);

each of $R^{21}$ and $R^{22}$, for each occurrence, is independently selected from absence, hydrogen, deuterium, halogen, hydroxyl, carbonyl, nitro, cyano, amino, mercapto, -COOH, $C_1$-$C_6$ alkyl, $C_1$-$C_6$ alkenyl, $C_1$-$C_6$ alkynyl, $C_1$-$C_6$ alkoxyl, wherein the alkyl, alkenyl, alkynyl, alkoxyl are optionally substituted with 1, 2, 3 or 4 halogens, hydroxyl, carbonyl, nitro, cyano, amino, mercapto, -COOH; or

$R^{21}$, $R^{22}$ are joined together to form -($CR^{91}R^{92}$)-($CR^{93}R^{94}$)-($CR^{95}R^{96}$)-, wherein each of $R^{91}$, $R^{92}$, $R^{93}$, $R^{94}$, $R^{95}$, and $R^{96}$, for each occurrence, is independently selected from hydrogen, deuterium, halogen, hydroxyl, carbonyl, nitro, cyano, amino, mercapto, -COOH, $C_1$-$C_6$ alkyl, $C_1$-$C_6$ alkenyl, $C_1$-$C_6$ alkynyl, $C_1$-$C_6$ alkoxyl, wherein the alkyl, alkenyl, alkynyl, alkoxyl are optionally substituted with 1, 2, 3 or 4 halogens, hydroxyl, carbonyl, nitro, cyano, amino, mercapto, -COOH;

L is a linker and has the following structure;

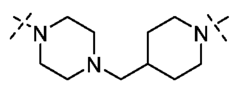, 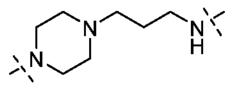, 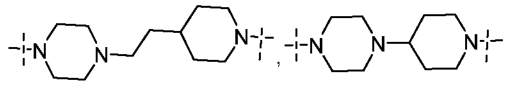,

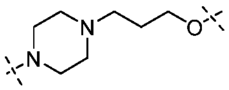, 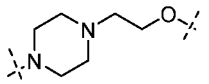, 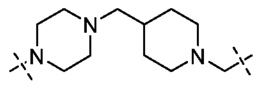, 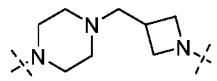,

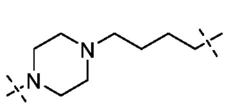, 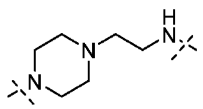, 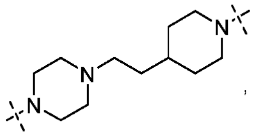, 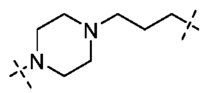,

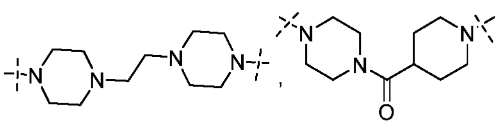, 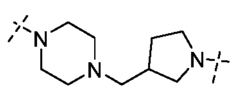, 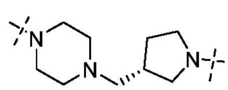,

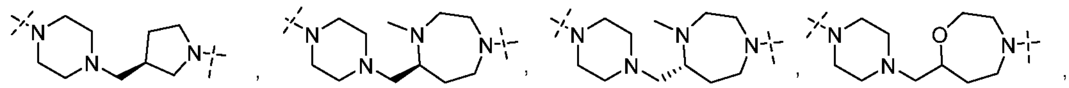

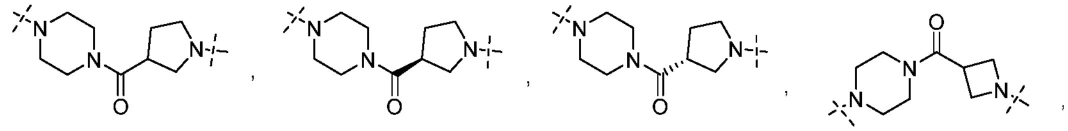

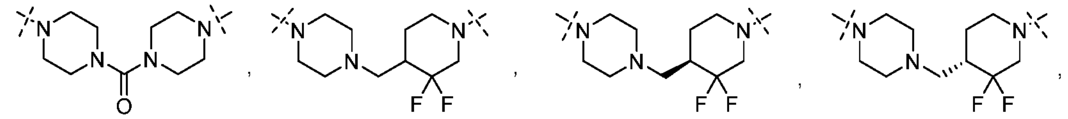

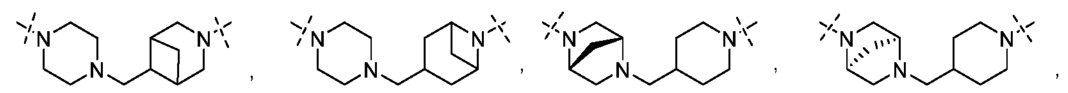

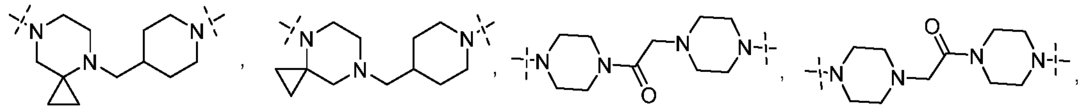

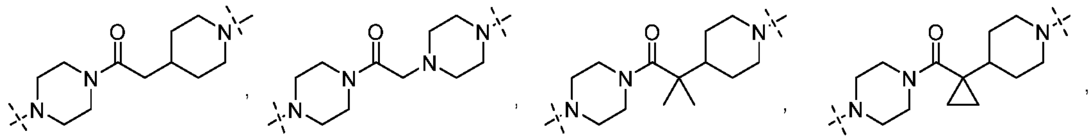

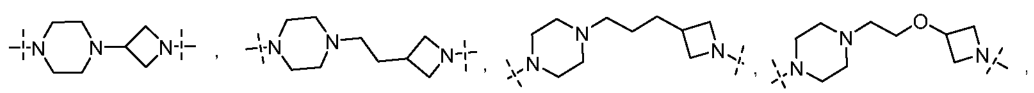

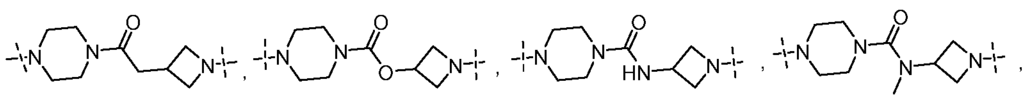

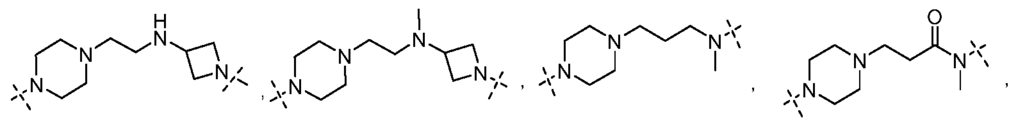

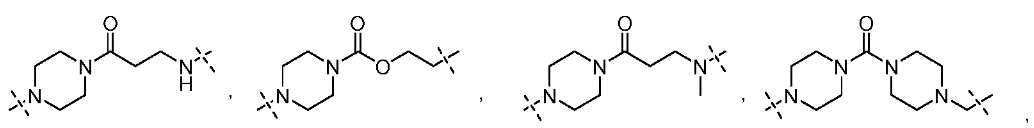

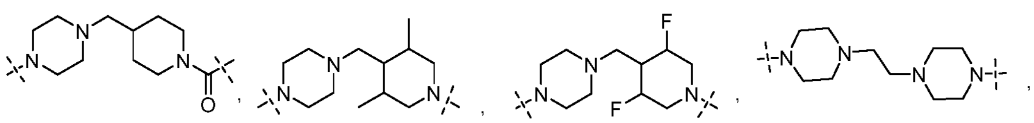

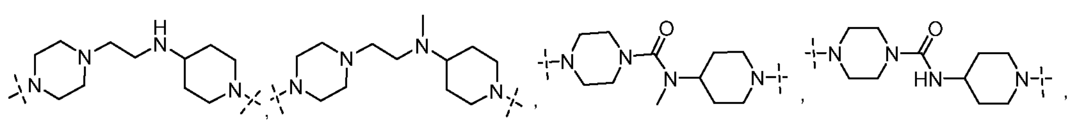

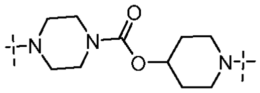, 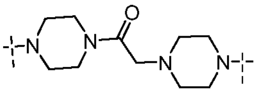, 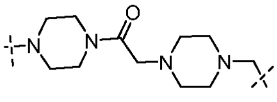, 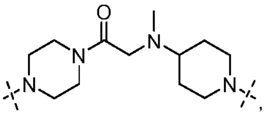,

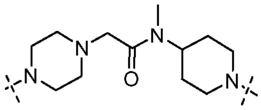, 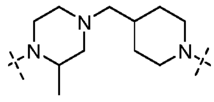, 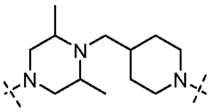, 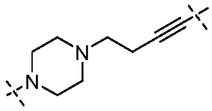,

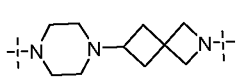, 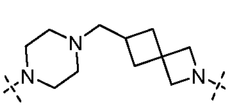, 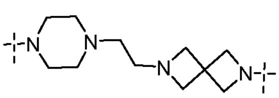, 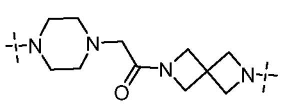,

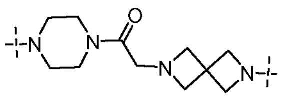, 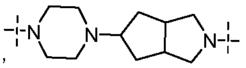, 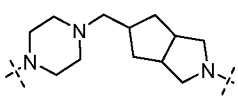, 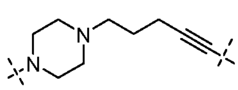,

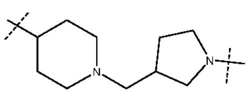, 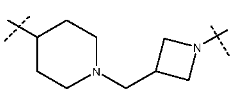, 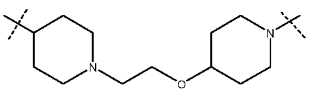, 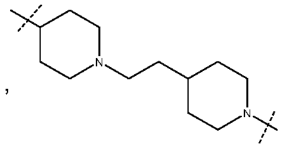

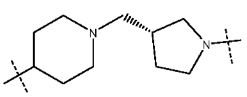, 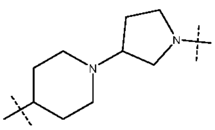, 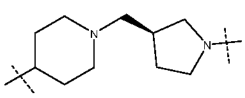, 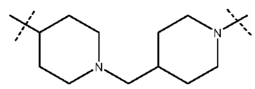,

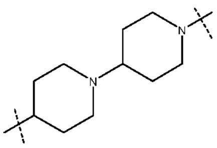, 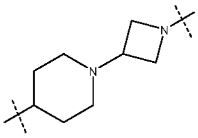, 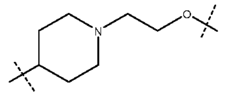, 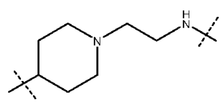

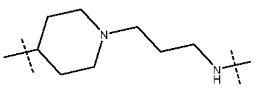, 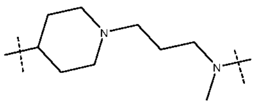, 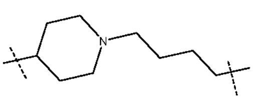, 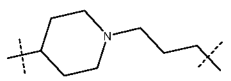,

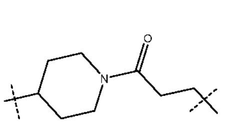, 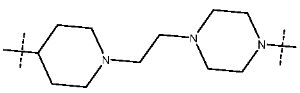, 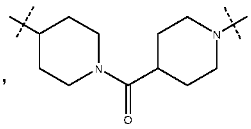, 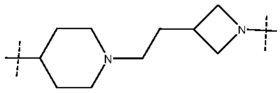,

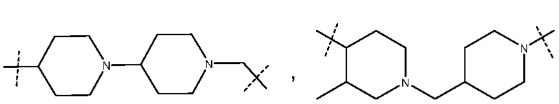, , 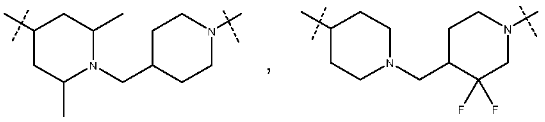, ,

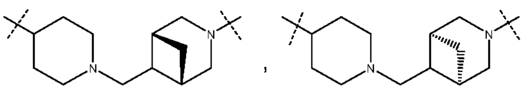, , 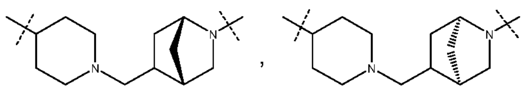, ,

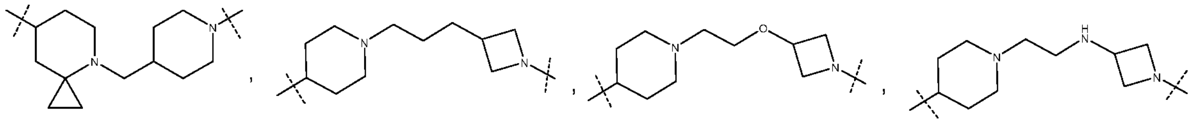
, , ,

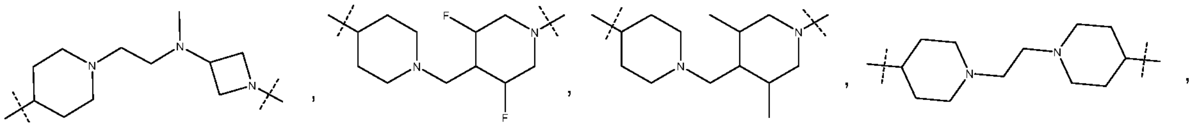
, , , ,

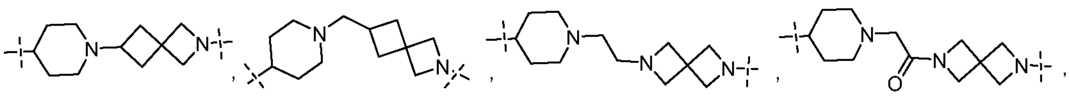
, , , ,

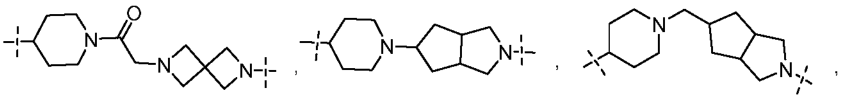
, , ,

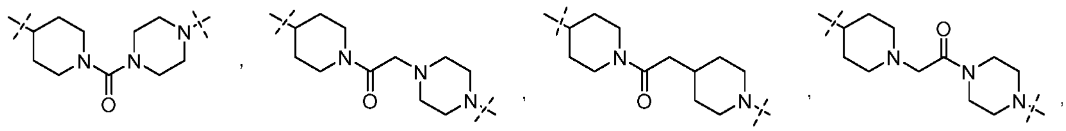
, , , ,

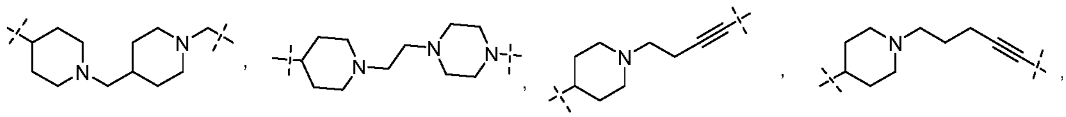
, , , ,

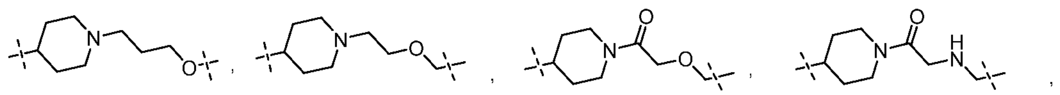
, , , ,

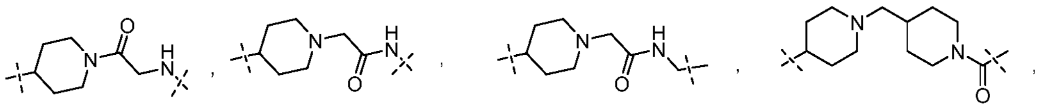
, , , ,

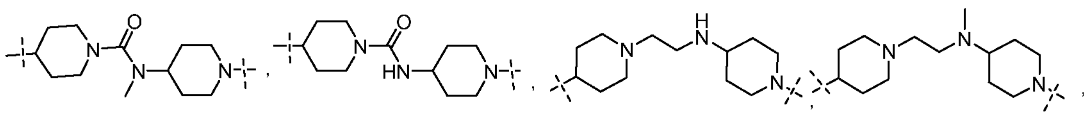
, , , ,

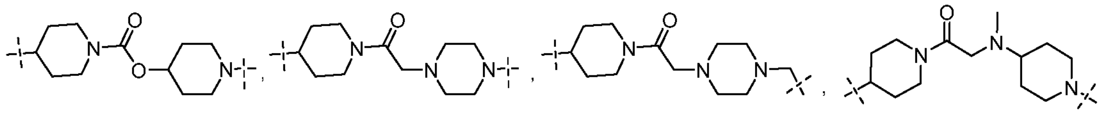
, , , ,

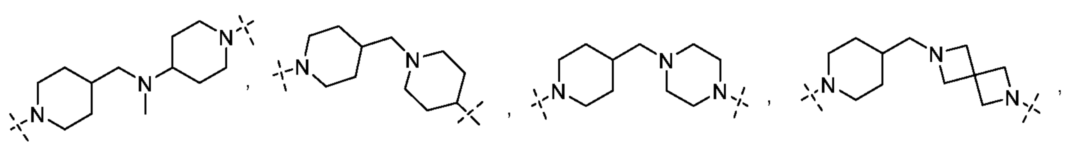
, , , ,

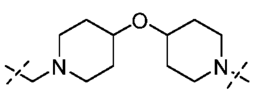, 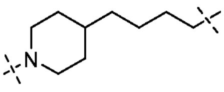, 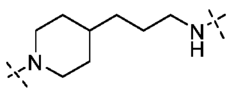, 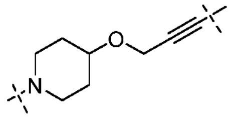,

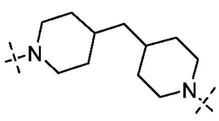, 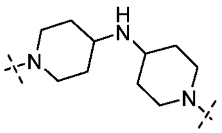, 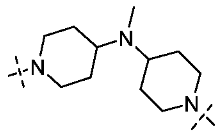, 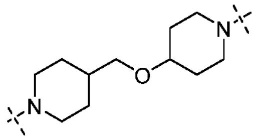,

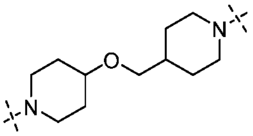, 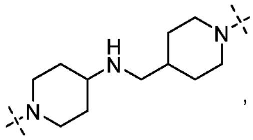, 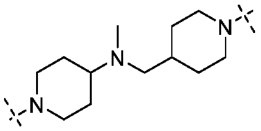, 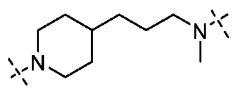,

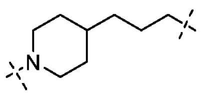, 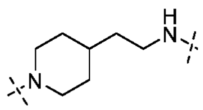, 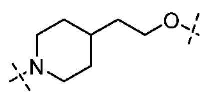, 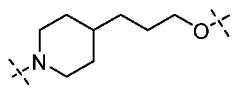,

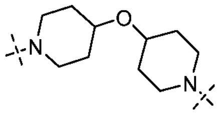, 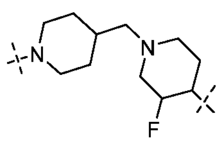, 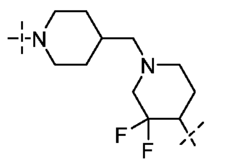, 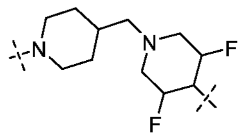,

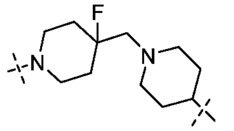, 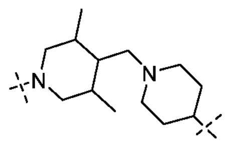, 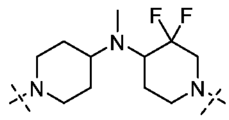, 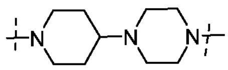,

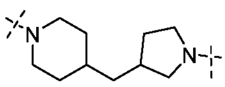, 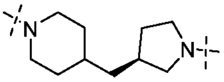, 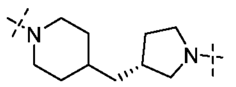, 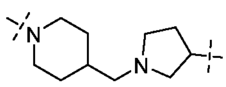,

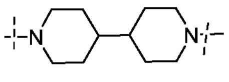, 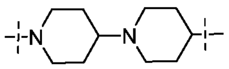, 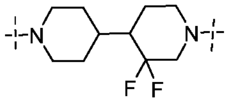, 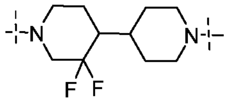,

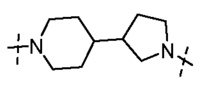, 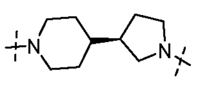, 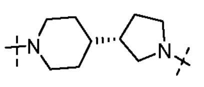, 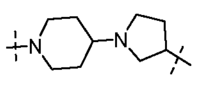,

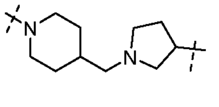, 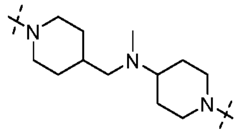, 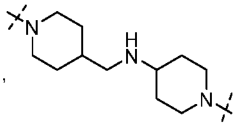,

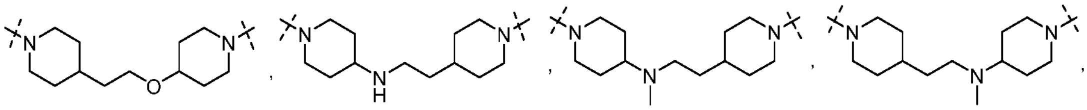

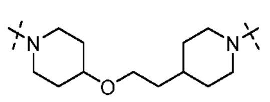, 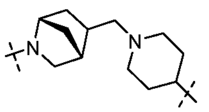, 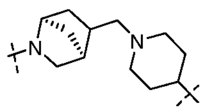, 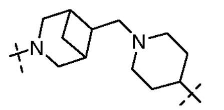,

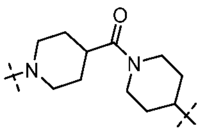, 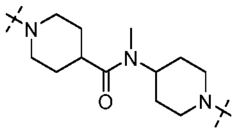, 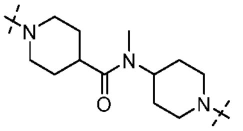,

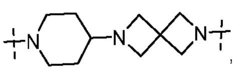, 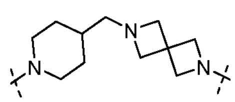, 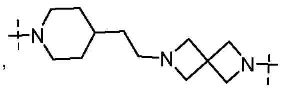, 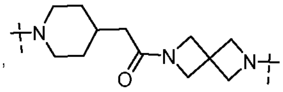,

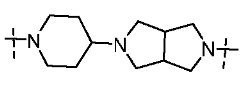, 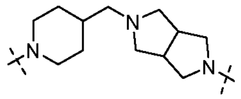,

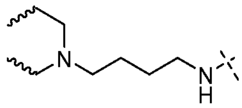, 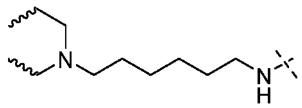, 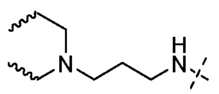, 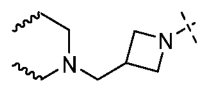,

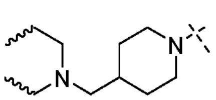, 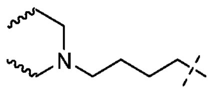, 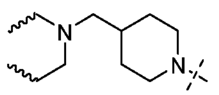, 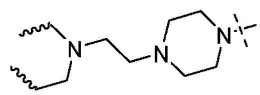,

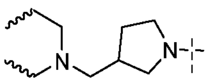, 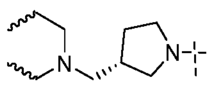, 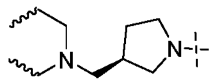, 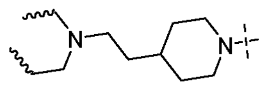,

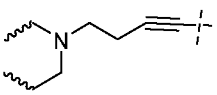, 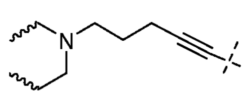, 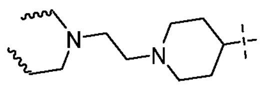, 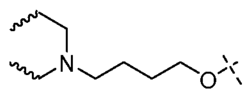,

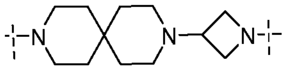, 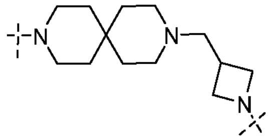, 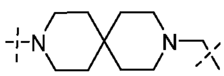, 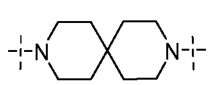,

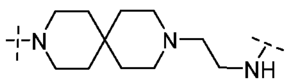, 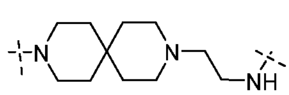, 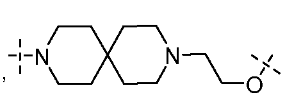,

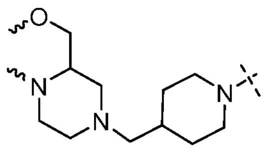 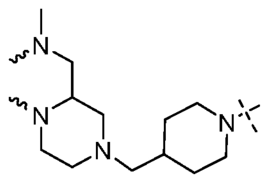,

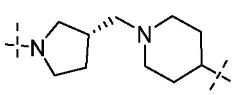 , 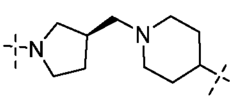 , 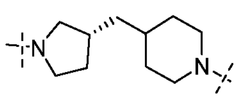 , 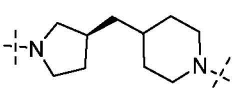 ,

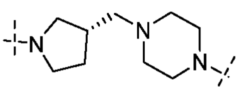 , 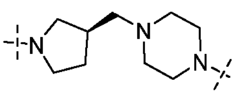 , 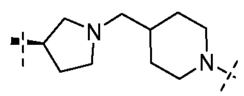 , 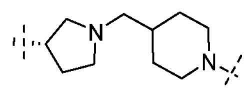 ,

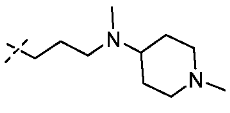 , 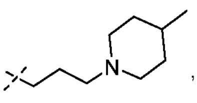 , 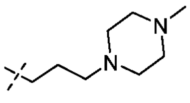 , 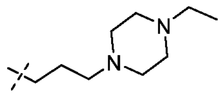 , 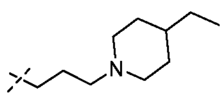 ,

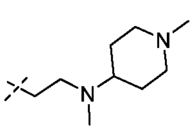 , 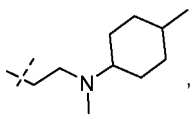 , 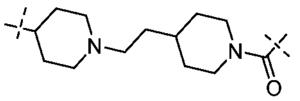 , 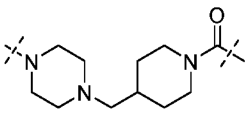 ,

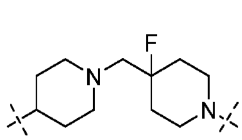 , 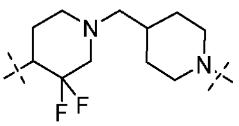 , 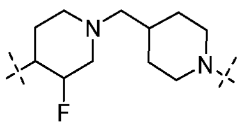 , 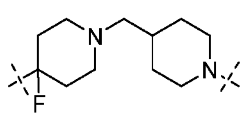

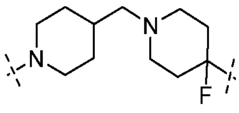 , 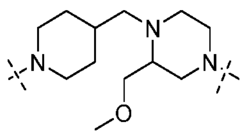 , 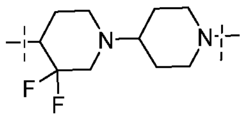 , 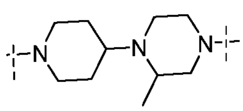 ,

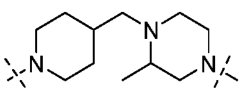 , 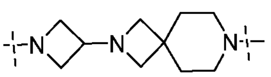 , 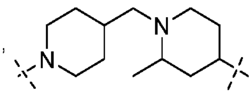 , 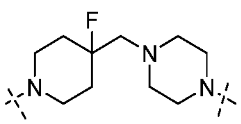 ,

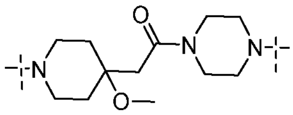 , 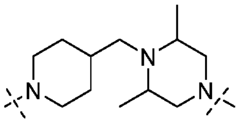 , 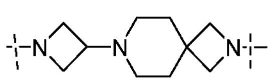 , 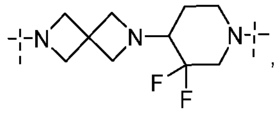 ,

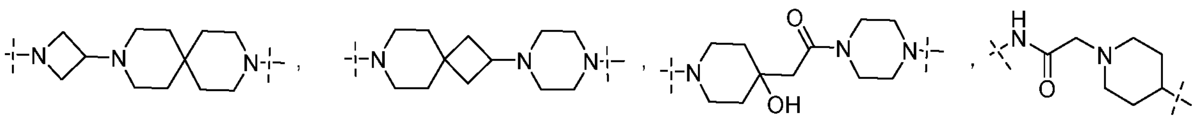 ,

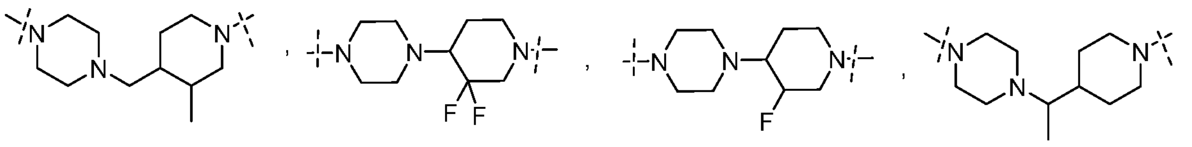 ,

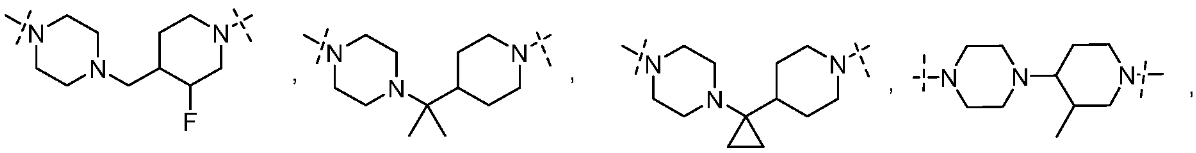 ,

wherein

 or 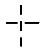

represents a linking site; and wherein the L group is optionally substituted with 1, 2, 3, or 4 substituents independently selected from halogen, hydroxyl, nitro, cyano, amino, mercapto, COOH, or $C_1$-$C_6$ alkyl.

The structure "-L-"does not mean that L must be linked to W and/or D by a single bond. In various aspects of various embodiments of the present application, the link of L to W and/or D may be realized by one or more bonds.

The term "pharmaceutically acceptable salt" refers to pharmaceutically acceptable organic or inorganic salts of the compounds of the present invention. Exemplary salts include, but are not limited to, sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisate, fumarate, gluconate, glucuronate, saccharate, formate, benzoate, glutamate, mesylate, ethanesulfonate, benzenesulfonate, p-toluenesulfonate, pamoate (i.e., 1,1'-methylene-bis-(2-hydroxy-3-naphthoic acid) salt), alkali metal (e.g., sodium and potassium) salts, alkaline earth metal (e.g., magnesium) salts, and ammonium salt. Pharmaceutically acceptable salts may involve the inclusion of another molecule, such as acetate ions, succinate ions, or other counterions. The counterion may be any organic or inorganic moiety that stabilizes the charge on the parent compound. In addition, pharmaceutically acceptable salts may have more than one charged atom in its structure. Examples where multiple charged atoms are part of pharmaceutically acceptable salts may have multiple counterions. Thus, pharmaceutically acceptable salts may have one or more charged atoms and/or one or more counterions.

If the compound of the present invention is a base, the desired pharmaceutically acceptable salt can be prepared by any suitable method available in the art, for example,by treating the free base with an inorganic acid (such as hydrochloric acid, hydrobromic acid, sulfuric acid, nitric acid, methanesulfonic acid, phosphoric acid) or the organic acid (such as acetic acid, maleic acid, succinic acid, mandelic acid, fumaric acid, malonic acid, pyruvic acid, oxalic acid, glycolic acid, salicylic acid, pyranosidic acid (such as glucuronic acid or galacturonic acid), α-hydroxy acid (such as citric acid or tartaric acid), amino acid (such as aspartic acid or glutamic acid), aromatic acid (such as benzoic acid or cinnamic acid), sulfonic acid (such as p-toluenesulfonic acid or ethanesulfonic acid), and the like.)

If the compound of the present invention is an acid, the desired pharmaceutically acceptable salt can be prepared by any suitable method, for example, by treating the free acid with an inorganic or organic base, such as an amine (primary, secondary or tertiary amine), alkali metal hydroxide or alkaline earth metal hydroxide. Illustrative examples of suitable salts include, but are not limited to, organic salts derived from amino acids (such as glycine and arginine), ammonia, primary, secondary and tertiary amines and cyclic amines (such as piperidine, morpholine and piperazine), and inorganic salts derived from sodium, calcium, potassium, magnesium, manganese, iron, copper, zinc, aluminum and lithium.

The term "pharmaceutically acceptable" indicates that the substance or composition must be chemically and/or toxicologically compatible with other ingredients of the formulation and/or mammals treated therewith.

The compounds of the present application may exist in various stereoisomeric forms. The term "stereoisomer" refers to compounds having the same chemical composition and connectivity, but whose atoms have different orientations in space that cannot be interconverted by rotation about a single bond. "Stereoisomer" may include "diastereomer" and "enantiomer". "Diastereomer" refers to a stereoisomer having two or more chiral centers and whose molecules are not mirror images of each other. "Enantiomer" refers to two stereoisomers of a compound, which are non-overlapping mirror images of each other. "R" and "S" represent substituent configurations surrounding one or more chiral atoms. The compounds of the present application can be prepared as individual isomers by chiral synthesis or resolution from mixtures of isomers. The disclosed compounds may have one or more stereocenters, and each stereocenter may independently exist in an R or S configuration. When the absolute stereochemistry of the stereocenter is not determined, the stereochemistry configuration can be designated (*) at the designated center. In one embodiment, the compounds described herein are present in optically active or racemic form. It should be understood that the compounds described herein include racemic, optically active, regioisomeric and stereoisomeric forms or combinations thereof having the therapeutically useful properties described herein. The chiral atoms described herein can be a chiral carbon atom, a chiral nitrogen atom, or a chiral phosphorus atom.

In particular, the compounds disclosed herein may have one or more stereocenters, and each stereocenter may independently exist in an R or S configuration. When the absolute stereochemistry at the stereocenter is not determined, the stereochemical configuration can be marked as "*" at that center, even though the compound itself has been separated as a single stereoisomer and is (non) enantiomeric pure. In other words, in some embodiments, the asterisk "*" is used to indicate that the chiral atom is in a substantially single steric configuration, and its absolute stereochemistry is not determined (even if the bond is drawn in stereochemical form). The actual absolute stereoconfiguration of atoms marked with "*" can be the same or different stereoconfiguration as the stereochemical form drawn. In other words, the atoms marked with "*" can have either an absolute R configuration or an absolute S configuration. The term "substantially" refers to variation in a range of 5%, 2%, or 1% or even 0.1% from the norm. The term "single stereoconfiguration" can be a single R configuration or a single S configuration.

In other embodiments, the stereocenter is marked as "*R" or "*S". When the stereocenter is marked as "*R" or "*S", it means that the absolute stereochemical properties of the stereocenter are not determined (even if the bond is drawn in stereochemical form), even though it is substantially a single stereoconfiguration. The actual absolute stereoconfiguration of the atoms marked with "*R" can be the same or different stereochemical form as the stereochemical form drawn; the actual absolute stereoconfiguration of atoms marked with "*S" can be the same or different stereochemical form as the stereochemical form drawn. In other words, "*R" can be an absolute R configuration or an absolute S configuration. Similarly, "*S" can be an absolute R configuration or an absolute S configuration. "*R" or "*S" can be randomly assigned to that molecule. The stereocenter marked with "*R" can have a single stereoconfiguration that is the same or different from another stereocenter marked with "*S". The stereocenter marked with "*R" can have a single stereoconfiguration that is the same or different from another stereocenter marked with "*R". The stereocenter marked with "*S" can have a single stereoconfiguration that is the same or different from another stereocenter marked with "*S".

Similarly, The stereocenter marked with "*R" can have a single stereoconfiguration that is the same or different from another stereocenter marked with "*". The stereocenter marked with "*S" can have a single stereoconfiguration that is the same or different from another stereocenter marked with "*".

For example, the absolute stereoconfiguration of compound 152-A is one of the following:

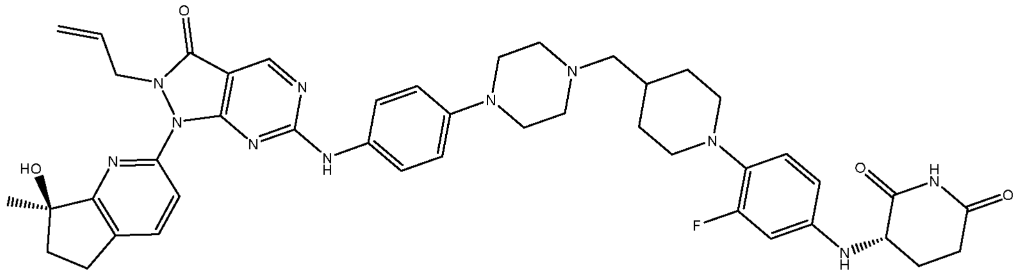
;

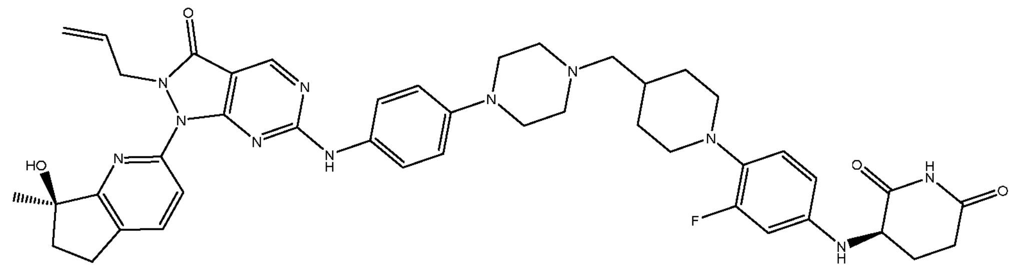
;

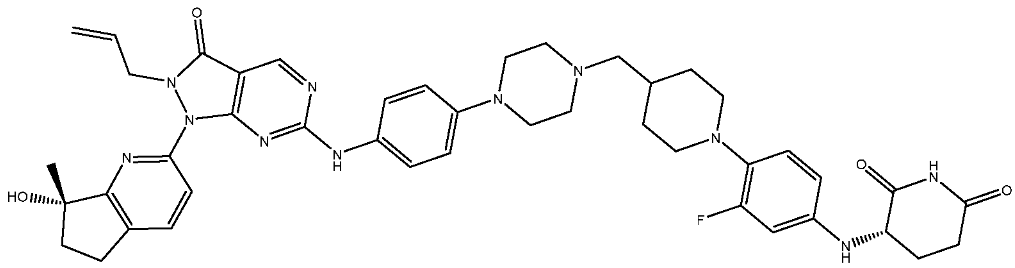
;

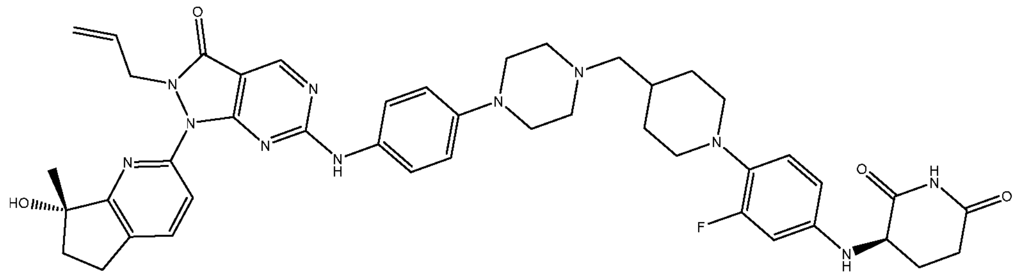

In one embodiment, the compounds described herein contain one or more chiral centers. These compounds can be prepared by any means, including stereoselective synthesis, enantioselective synthesis, or separation of mixtures of enantiomers or diastereomers. Resolution of compounds and their isomers can be achieved by any means, including but not limited to chemical processes, enzymatic processes, fractional crystallization, distillation and chromatography.

Definition

The term "substituted" means that any one or more hydrogen on the designated atom or group is replaced by a moiety selected from the designated group, provided that the designated atom's normal valence is not exceeded.

"Alkyl" is a branched or straight chain saturated aliphatic hydrocarbon group. In one embodiment, the alkyl group contains 1 to about 12 carbon atoms, more typically 1 to about 6 carbon atoms or 1 to about 4 carbon atoms. In one embodiment, the alkyl group contains 1 to about 8 carbon atoms. In certain Examples, the alkyl group is $C_1$-$C_2$, $C_1$-$C_3$, or $C_1$-$C_6$. The specified ranges as used herein indicate an alkyl group having each member of the range described as an independent species. For example, the term $C_1$-$C_6$ alkyl as used herein indicates a straight or branched alkyl group having from 1, 2, 3, 4, 5, or 6 carbon atoms and is intended to mean that each of these is described as an independent species. For example, the term $C_1$-$C_4$alkyl as used herein indicates a straight or branched alkyl group having from 1, 2, 3, or 4 carbon atoms and is intended to mean that each of these is described as an independent species. When $C_0$-$C_n$ alkyl is used herein in conjunction with another group, for example, ($C_3$-$C_7$ cycloalkyl) $C_0$-$C_4$ alkyl, or -$C_0$-$C_4$ alkyl($C_3$-$C_7$ cycloalkyl), the indicated group, in this case cycloalkyl, is either directly bound by a single covalent bond ($C_0$ alkyl), or attached by an alkyl chain in this case 1, 2, 3, or 4 carbon atoms. Alkyls can also be attached via other groups such as heteroatoms as in -O-$C_0$-$C_4$ alkyl ($C_3$-$C_7$ cycloalkyl). Examples of alkyl include, but are not limited to, methyl, ethyl, n-propyl, isopropyl, n-butyl, isobutyl, sec-butyl, t-butyl, n-pentyl, isopentyl, tert-pentyl, neopentyl, n-hexyl, 2-methylpentyl, 3-methylpentyl, 2,2-dimethylbutyl and 2,3-dimethylbutyl. In one embodiment, the alkyl group is optionally substituted as described above.

"Alkenyl" is a branched or straight chain aliphatic hydrocarbon group having one or more carbon-carbon double bonds that may occur at a stable point along the chain. Nonlimiting examples are $C_2$-$C_8$ alkenyl, $C_2$-$C_6$ alkenyl and $C_2$-$C_4$ alkenyl. The specified ranges as used herein indicate an alkenyl group having each member of the range described as an independent species, as described herein for the alkyl moiety. Examples of alkenyl include, but are not limited to, ethenyl, propenyl. In one embodiment, the alkenyl group is optionally substituted as described above.

"Alkynyl" is a branched or straight chain aliphatic hydrocarbon group having one or more carbon-carbon triple bonds that may occur at any stable point along the chain, for example, $C_2$-$C_8$ alkynyl or $C_2$-$C_6$ alkynyl. The specified ranges as used herein indicate an alkynyl group having each member of the range described as an independent species, as described herein for the alkyl moiety. Examples of alkynyl include, but are not limited to, ethynyl, propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl and 5-hexynyl. In one embodiment, the alkynyl group is optionally substituted as described above.

"Alkoxy" is an alkyl group as defined above covalently bound through an oxygen bridge (-O-). Examples of alkoxy include, but are not limited to, methoxy, ethoxy, n-propoxy, i-propoxy, n-butoxy, 2-butoxy, t-butoxy, n-pentoxy, 2-pentoxy, 3-pentoxy, isopentoxy, neopentoxy, n-hexoxy, 2-hexoxy, 3-hexoxy, and 3-methylpentoxy. Similarly an "alkylthio" or a "thioalkyl" group is an alkyl group as defined above with the indicated number of carbon atoms covalently bound through a sulfur bridge (-S-). In one embodiment, the alkoxy group is optionally substituted as describedabove.

"Alkenyloxy" is an alkenyl group as defined above covalently bound to the group it substitutes by an oxygen bridge (-O-).

"Alkanoyl" is an alkyl group as defined above covalently bound through a carbonyl (C=O) bridge. The carbonyl carbon is included in the number of carbons, that is $C_2$ alkanoyl is a $CH_3(C=O)$- group. In one embodiment, the alkanoyl group is optionally substituted as describedabove.

"Alkylester" is an alkyl group as defined covalently bound through an ester linkage. The ester linkage may be in either orientation, e.g., a group of the formula-O(C=O)alkyl or a group of the formula -(C=O)Oalkyl.

"Carbocyclyl", "carbocyclic ring", or "cycloalkyl" is a saturated or partially unsaturated (i.e., not aromatic) group

containing all carbon ring atoms. A carbocyclic group typically contains 1 ring of 3 to 7 carbon atoms or 2 fused rings each containing 3 to 7 carbon atoms. Cycloalkyl substituents may be pendant from a substituted nitrogen or carbon atom, or a substituted carbon atom that may have two substituents can have a cycloalkyl group, which is attached as a spiro group. Examples of carbocyclic rings include cyclohexenyl, cyclohexyl, cyclopentenyl, cyclopentyl, cyclobutenyl, cyclobutyl and cyclopropyl rings. In one embodiment, the carbocyclic ring is optionally substituted as described above. In one embodiment, the cycloalkyl is a partially unsaturated (i.e., not aromatic) group containing all carbon ring atoms.

"Carbocycle-oxy group" is a monocyclic carbocyclic or mono- or bi-cyclic carbocyclic group as described above attached to the group it substitutes by an oxygen-O-linker.

"Haloalkyl" indicates both branched and straight-chain alkyl groups substituted with 1 or more halogen atoms, up to the maximum allowable number of halogen atoms. Examples of haloalkyl include, but are not limited to, trifluoromethyl, monofluoromethyl, difluoromethyl, 2-fluoroethyl, and penta-fluoroethyl.

"Haloalkoxy" indicates a haloalkyl group as defined herein attached through an oxygen bridge (oxygen of an alcohol radical).

"Hydroxyalkyl" is an alkyl group as previously described substituted with at least one hydroxy substituent.

"Aminoalkyl" is an alkyl group as previously described substituted with at least one amino substituent.

"Halo" indicates independently any of fluoro, chloro, bromo, and iodo.

"Aryl" indicates aromatic groups containing only carbon in the aromatic ring or rings. In one embodiment, the aryl groups contain 1 to 3 separate or fused rings and is 6 to about 18 ring atoms (such as 6, 7, 8, 9, 10, 11, 12, 13, 14, 15, 16, 17, or 18 ring atoms), without heteroatoms as ring members. When indicated, such aryl groups may be further substituted with carbon or non-carbon atoms or groups. Such substitution may include fusion to a 5 to 7-membered saturated cyclic group that optionally contains 1 or 2 heteroatoms independently chosen from N, O, and S, to form, for example, a 3,4-methylenedioxyphenyl group. Aryl groups include, for example, phenyl, naphthyl, including 1-naphthyl and 2-naphthyl. In one embodiment, aryl groups are pendant. An example of a pendant ring is a phenyl group substituted with a phenyl group. In one embodiment, the aryl group is optionally substituted as described above. Aryl groups include bicyclic groups comprising an aromatic ring fused to a saturated, partially unsaturated ring or an aromatic carbocyclic or heterocyclic ring. Typical aryl groups include, but are not limited to, those derived from benzene (phenyl), substituted benzene, naphthalene, anthracene, indenyl, indanyl, 1,2-dihydronaphthalene, 1,2,3,4-tetrahydro groups and the like. Preferably, the aryl group is a phenyl group.

The term "heterocyclyl" as used herein refers to a saturated or a partially unsaturated (i.e., having one or more double and/or triple bonds within the ring without aromaticity) carbocyclic radicals of 3 to 18 (such as 3, 4, 5, 6, 7, 8, 9, 10, 11, 12, 13, 14, 15, 16, 17, or 18) ring atoms wherein at least one ring atom is a heteroatom selected from nitrogen, oxygen" phosphorus and sulfur, the remaining ring atoms being C, where one or more ring atoms is optionally substituted independently with one or more substituents described above. A heterocycle may be a monocycle having 3 to 7 ring members (2 to 6 carbon atoms and 1 to 4 heteroatoms selected from N, O, P and S) or a bicycle having 6 to 10 ring members (4 to 9 carbon atoms and 1 to 6 heteroatoms selected from N, O, P and S), for example: a bicyclo [4,5], [5,5], [5,6], or [6,6] system. In one embodiment, the only heteroatom is nitrogen. In one embodiment, the only heteroatom is oxygen. In one embodiment, the only heteroatom is sulfur. Heterocycles are described in Paquette, Leo A.; "Principles of Modern Heterocyclic Chemistry" (W. A. Benjamin, New York, 1968), particularly Chapters 1, 3, 4, 6, 7, and 9; "The Chemistry of Heterocyclic Compounds, A series of Monographs" (John Wiley & Sons, New York, 1950 to present), in particular Chapters 13, 14, 16, 19, and 28; and J. Am. Chem. Soc. (1960) 82:5566. Examples of heterocyclic rings include, but are not limited to, pyrrolidinyl, dihydrofuranyl, tetrahydrothienyl, tetrahydropyranyl, dihydropyranyl, tetrahydrothiopyranyl, piperidino, piperidonyl, morpholino, thiomorpholino, thioxanyl, piperazinyl, homopiperazinyl, azetidinyl, oxetanyl, thietanyl, homopiperidinyl, oxepanyl, thiepanyl, oxazepinyl, diazepinyl, thiazepinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, dihydroisoquinolinyl, tetrahydroisoquinolinyl, pyrazolidinylimidazolinyl, imidazolidinyl, 2-oxa-5-azabicyclo[2.2.2]octane, 3-oxa-8-azabicyclo[3.2.1]octane, 8-oxa-3-azabicyclo[3.2.1]octane, 6-oxa-3-azabicyclo[3.1.1]heptane, 2-oxa-5-azabicyclo[2.2.1]heptane, 3-azabicyco[3.1.0]hexanyl, 3-azabicyclo[4.1.0]heptanyl, azabicyclo[2.2.2]hexanyl, 3H-indolyl, quinolizinyl, N-pyridyl ureas, and pyrrolopyrimidine. Spiro moieties are also included within the scope of this definition. Examples of a heterocyclic group wherein 1 or 2 ring carbon atoms are substituted with oxo (=O) moieties are pyrimidinonyl and 1,1-dioxo-thiomorpholinyl. The heterocycle groups herein are optionally substituted independently with one or more substituents described herein. "Heterocyclyl" includes "heterocycloalkyl". "Heterocycloalkyl" is a saturated ring group. It may have, for example, 1, 2, 3 or 4 heteroatoms independently selected from N, S and O, the remaining ring atoms being carbon. In a typical embodiment, nitrogen is a heteroatom. Monocyclic heterocycloalkyl groups typically have 3 to about 8 ring atoms or 4 to 6 ring atoms. Examples of heterocycloalkyl groups include morpholino, piperazinyl, piperidino, and pyrrolinyl. "Heterocyclyl" may contain 1, 2, 3, 4, or 5 heteroatoms selected from N, O, and S.

"Heterocyclicoxy group" is a monocyclic heterocyclic ring or a bicyclic heterocyclic group as described previously linked to the group it substitutes via an oxygen-O-linker.

"Heteroaryl" can be a stable monocyclic aromatic ring which contains from 1 to 3, or in some embodiments from 1 to 2, heteroatoms chosen from N, O, and S, with remaining ring atoms being carbon, or a stable bicyclic or tricyclic system of 5- to 10-membered (5, 6, 7, 8, 9, 10 membered) aromatic rings containing at least one 1 to 3, or in some embodiments from 1 to 2, heteroatoms chosen from N, O, and S, with remaining ring atoms being carbon. In one embodiment, the only heteroatom is nitrogen. In one embodiment, the only heteroatom is oxygen. In one embodiment, the only heteroatom is sulfur. Monocyclic heteroaryl groups typically have from 5 to 8 ring atoms(5, 6, 7, 8 ring atoms). In some embodiments, bicyclic heteroaryl groups are 9- to 10-membered heteroaryl groups, that is, groups containing 9 or 10 ring atoms wherein one 5- to 7-membered aromatic ring is fused to a second aromatic or nonaromatic ring. When the total number of S and O atoms in the heteroaryl group exceeds 1, these heteroatoms are not adjacent to one another. In one embodiment, the total number of S and O atoms in the heteroaryl group is not more than 2. In another embodiment, the total number of S and O atoms in the aromatic heterocycle is not more than 1. Examples of heteroaryl groups include, but are not limited to, pyridinyl (including, for example, 2-hydroxypyridinyl), furyl, imidazolyl, imidazopyridinyl, pyrimidinyl (including, for example, 4-hydroxypyrimidinyl), pyrazolyl, triazolyl, pyrazinyl, tetrazolyl, furyl, thienyl, isoxazolyl, thiazolyl, oxadiazolyl, oxazolyl, isothiazolyl, pyrrolyl, quinolinyl, isoquinolinyl, tetrahydroisoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, triazolyl, thiadiazolyl, thiadiazolyl, furazanyl, benzofurazanyl, benzothiophenyl, benzothiazolyl, benzoxazolyl, quinazolinyl, quinoxalinyl, naphthyridinyl, tetrahydrofuranyl, and furopyridinyl. Heteroaryl groups are optionally substituted independently with one or more substituents described herein. "Heteroaryloxy" is a heteroaryl group as described bound to the group it substituted via an oxygen-O-linker. "Heteroaryl" may contain 1, 2, 3, 4, or 5 heteroatoms selected from N, O, and S.

In this context,

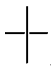,  or 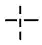

and any indeterminate linking mode may represent a linking site.

The present application also covers bicyclic, tricyclic, tetracyclic, pentacyclic and other polycyclic ring systems formed by one or more monocyclic rings selected from "cycloalkyl", "aryl" and "heteroaryl" in a valence-allowed manner.

The term cyano refers to -CN;

The term CD refers to deuterated carbon.

The compounds or pharmaceutically acceptable salts, isomers, solvates, hydrates, adducts, complexes or prodrugs thereof described herein, are useful in the treatment of proliferative disorders. The present invention also provides the use of the compound or pharmaceutically acceptable salts, isomers, solvates, hydrates, adducts, complexes or prodrugs thereof described herein in the manufacture of a medicament for the treatment of proliferative disorders. The present invention further provides the compounds or pharmaceutically acceptable salts, isomers, solvates, hydrates, adducts, complexes or prodrugs thereof for use in the treatment of proliferative disorders. The application further provides a method of treating proliferative disorders, comprising administering to a subject in need thereof a therapeutically effective amount of the compounds or pharmaceutically acceptable salts, isomers, solvates, hydrates, additions, compounds, complexes or prodrugs thereof.

The term "proliferative disorder" or "cell proliferative disorder" refers to disorders that are associated with some degree of abnormal cell proliferation, whether malignant or benign. In some embodiments, the proliferative disorder is a cancer. In some aspects, the cancer is a solid tumor. In some aspects, the cancer is a hematologic malignancy. The terms "proliferative disorder", "cell proliferative disorder", "cancer," "cancerous," and "tumor" are not mutually exclusive as referred to herein.

The term "cancer" comprises cancerous cells, and/or benign or pre-cancerous cells. Examples of cancer include, but are not limited to, breast cancer, colon cancer, brain cancer, prostate cancer, kidney cancer, pancreatic cancer, ovarian cancer, head and neck cancer, melanoma, colorectal cancer, gastric cancer, squamous cancer, small-cell lung cancer, non small-cell lung cancer, testicular cancer, Merkel cell carcinoma, glioblastoma, neuroblastoma, cancers of lymphatic organs and hematological malignancy including Leukemia (Acute lymphoblastic leukemia (ALL), Acute myelogenous leukemia (AML), Chronic lymphocytic leukemia (CLL), Chronic myelogenous leukemia (CML), Acute monocytic leukemia (AMOL), Hairy cell leukemia (HCL), T-cell prolymphocytic leukemia (T-PLL), Large granular lymphocytic leukemia, Adult T-cell leukemia), Lymphoma (small lymphocytic lymphoma (SLL), Hodgkin’s lymphomas (Nodular sclerosis, Mixed cellularity, Lymphocyte-rich, Lymphocyte depleted or not depleted, and Nodular lymphocyte-predominant Hodgkin lymphoma), Non-Hodgkin’s lymphomas (all subtypes), Chronic lymphocytic leukemia/Small lymphocytic lymphoma, B-cell prolymphocytic leukemia, Lymphoplasmacytic lymphoma (such as Waldenström macroglobulinemia), Splenic marginal zone lymphoma, Plasma cell neoplasms (Plasma cell myeloma, Plasmacytoma, Monoclonal immunoglobulin deposition diseases, Heavy chain diseases), Extranodal marginal zone B cell lymphoma (MALT lymphoma), Nodal marginal zone B cell lymphoma (NMZL), Follicular lymphoma, Mantle cell lymphoma, Diffuse large B cell lymphoma, Mediastinal (thymic) large B cell lymphoma, Intravascular large B cell lymphoma, Primary effusion lymphoma, Burkitt lymphomalleukemia, T cell prolymphocytic leukemia, T cell large granular lymphocytic leukemia, Aggressive NK cell leukemia, Adult T cell leukemia/lymphoma, Extranodal NK/T cell lymphoma (nasal type), Enteropathy-type T cell lymphoma, Hepatosplenic T cell lymphoma, Blastic NK cell lymphoma, Mycosis fungoides / Sezary syndrome, Primary cutaneous CD30-positive T cell lymphoproliferative disorders, Primary cutaneous anaplastic large cell lymphoma, Lymphomatoid papulosis, Angioimmunoblastic T cell lymphoma, Peripheral T cell lymphoma (unspecified), Anaplastic large cell lymphoma), multiple myeloma (plasma cell myeloma or Kahler's disease). The term "cancer" also includes leukemia, carcinoma and sarcoma. Exemplary cancers include brain cancer, breast cancer, cervical cancer, colon cancer, head and neck cancer, liver cancer, kidney cancer, lung cancer, non-small cell lung cancer, melanoma, mesothelioma, ovarian cancer, sarcoma, stomach cancer, uterine cancer and Medulloblastoma. Additional examples include Hodgkin's disease, non-Hodgkin's lymphoma, multiple myeloma, neuroblastoma, ovarian cancer, rhabdomyosarcoma, essential thrombocythemia, primary macroglobulinemia, primary brain tumor, cancer, malignant pancreatic insulinoma, malignant carcinoid, bladder cancer, premalignant skin lesions, testicular cancer, lymphoma, thyroid cancer, neuroblastoma, esophageal cancer, genitourinary tract cancer, malignant hypercalcemia, endometrial cancer, adrenocortical carcinoma, tumors of the endocrine and exocrine pancreas, and prostate cancer. The term "cancer"also includes leukemia, multiple myeloma, lymphoma, liver cancer, stomach cancer, breast cancer, cholangiocarcinoma, pancreatic cancer, lung cancer, colorectal cancer, osteosarcoma, melanoma, human cervical cancer, glioma, nasopharyngeal cancer, laryngeal cancer, esophageal cancer, middle ear tumor, prostate cancer, etc.

"Link to the L group/linked to L groups/linking to L groups" described herein means that the L group is linked to the site indicated by the corresponding variable (eg, a site on a ring, such as a benzene ring atom to which $R^1$ or $R^2$ is attached, $Y^5$, $Y^6$, or $Y^8$ itself, etc., or $R^1$ and/or $R^2$ themselves). Here, the corresponding variable can be present or absent.

In a preferred embodiment, the present application relates to the following diseases: acute lymphoblastic leukemia (ALL), acute myeloid leukemia (AML), multiple myeloma (MM), diffuse large B cell lymphoma (DLBCL), mantle cell lymphoma (MCL), malignant melanoma, gastric cancer, gliomas, ovarian carcinoma, uterine serous carcinoma (USC), colorectal cancer, pancreatic cancer, esophageal cancer, hepatocellular carcinoma, glioblastoma, non-small-cell lung cancer (NSCLC), small-cell lung cancer (SCLC), neuroblastoma, breast cancer, triple negative breast cancer (TNBC), neuroblastoma, head and neck squamous cell carcinoma (HNSCC).

The present invention further provides a pharmaceutical composition comprising the compound or derivatives, pharmaceutically acceptable salts, isomers, solvates, hydrates, adducts, complexes or prodrugs thereof described herein, and a pharmaceutically acceptable carrier. The pharmaceutical composition described herein may be a tablet, a capsule, a granule, a syrup, a suspension, a solution, a dispersion, a slow release preparation for oral or non-oral administration, an intravenous injection preparation, a subcutaneous injection preparation, an inhalation preparation, a transdermal preparation, a rectal or vaginal suppository.

The pharmaceutically acceptable carrier described herein refers to a pharmaceutically acceptable carrier well known to those skilled in the art, and the pharmaceutically acceptable carrier of the present invention includes, but is not limited to, a filler, a wetting agent, a binder, a disintegrating agent, a lubricant, a binder, a glidant, a flavoring agent, a surfactant, a preservative, and the like. Fillers include, but are not limited to, lactose, microcrystalline cellulose, starch, powdered sugar, dextrin, mannitol, calcium sulfate, and the like. Wetting agents and binders include, but are not limited to, sodium carboxymethylcellulose, hydroxypropylcellulose, hydroxypropyl methyl cellulose, gelatin, sucrose, polyvinylpyrrolidone, and the like. Disintegrating agents include, but are not limited to, sodium carboxymethyl starch, crosslinked polyvinylpyrrolidone, croscarmellose sodium, low substituted hydroxypropylcellulose, and the like. Lubricants include, but are not limited to, magnesium stearate, aerosil, talc, hydrogenated vegetable oil, polyethylene glycol, magnesium lauryl sulfate, and the like. Binders include, but are not limited to, gum arabic, alginic acid, calcium carboxymethylcellulose, sodium carboxymethylcellulose, glucose, dextrin, dextrose, ethylcellulose, gelatin, liquid glucose, guar gum, hydroxyethyl cellulose, hydroxypropyl cellulose, hydroxypropyl methyl cellulose, magnesium aluminum silicate, maltodextrin, methyl cellulose, polymethacrylate, polyvinylpyrrolidone, pregelatinized starch, sodium alginate, sorbitol, starch, syrup and tragacanth. Glidants include, but are not limited to, colloidal silica, powdered cellulose, magnesium trisilicate, silica, and talc. Flavoring agents include, but are not limited to, aspartame, stevioside, fructose, glucose, syrup, honey, xylitol, mannitol, lactose, sorbitol, maltitol, glycyrrhizin. Surfactants include, but are not limited to, Tween-80, poloxamer. Preservatives include, but are not limited to, paraben, sodium benzoate, potassium sorbate, and the like.

Methods of preparing various pharmaceutical compositions containing various amounts of active ingredients are known, or will be apparent to those skilled in the art in light of this disclosure, as described in REMINGTON'S PHARMACEUTICAL SCIENCES, Martin, E.W., ed., Mack Publishing Company, 19th ed. (1995). Methods of preparing

the pharmaceutical compositions include incorporation of suitable pharmaceutical excipients, carriers, diluents and the like. The pharmaceutical compositions described herein are made in a known manner, including conventional methods of mixing, dissolving or lyophilizing.

In the pharmaceutical compositions described herein, the amount of active ingredient may vary from about 0.01% to about 99% by weight of a given unit dosage form. In such therapeutically useful pharmaceutical composition formulations, the active ingredient is in an amount such that an effective dosage level can be obtained.

The tablet, capsule and the like described herein may comprise: a binder such as tragacanth, gum arabic, corn starch or gelatin; an excipient such as dicalcium phosphate; a disintegrating agent such as corn starch, potato starch, alginic acid, etc.; a lubricant such as magnesium stearate; and a sweetener such as sucrose, fructose, lactose or aspartame; or a flavoring agent such as mint, wintergreen or cherry flavor. When the unit dosage form is a capsule, it may contain, in addition to materials of the above types, a liquid carrier such as vegetable oil or polyethylene glycol. Various other materials may be present, as a coating, or otherwise alter the physical form of the solid unit dosage form. For example, tablets or capsules may be coated with gelatin, wax, shellac or sugar, etc. The syrup may contain an active ingredient, sucrose or fructose as a sweetener, methylparaben or propylparaben as a preservative, a dye and a flavoring agent such as cherry or orange flavor. Of course, any material used to prepare any unit dosage form should be pharmaceutically acceptable and non-toxic in the amounts employed. In addition, the active ingredient can be incorporated into a slow release formulations and a slow release device.

The active ingredient can also be administered intravenously or intraperitoneally by infusion or injection. An aqueous solution of the active ingredient or a salt thereof can be prepared, optionally with a non-toxic surfactant. Dispersions in glycerol, liquid polyethylene glycol, triacetin and mixtures thereof, and oils can also be prepared. Under ordinary conditions of storage and use, these formulations contain a preservative to prevent the growth of microorganisms.

Pharmaceutical composition dosage forms suitable for injection or infusion may comprise sterile aqueous solution or dispersion or sterile powder comprising an active ingredient suitable for the ready-to-use preparation of a sterile, injectable or infusible solution or dispersion (optionally encapsulated in a liposome). In all cases, the final dosage forms must be sterile, liquid, and stable under the conditions of manufacture and storage. The liquid carrier may be a solvent or liquid dispersion medium including, for example, water, ethanol, polyols (for example, glycerol, propylene glycol, liquid polyethylene glycol, and the like), vegetable oils, non-toxic glycerides, and suitable mixtures thereof. Proper fluidity can be maintained, for example, by liposome formation, by maintaining the desired particle size in the case of dispersion, or by the use of a surfactant. The prevention of microorganisms can be achieved by using various antibacterial and antifungal agents such as parabens, chlorobutanol, phenol, sorbic acid, thimerosal, and the like. In many cases, it will be preferred to include isotonic agents, such as sugars, buffers or sodium chloride. Prolonged absorption of the injectable composition can be brought about by the use of a composition that comprises an absorption delaying agent (for example, aluminum monostearate and gelatin).

Sterile injectable solutions are prepared by combining the active ingredient in a desired amount in a suitable solvent with the various other ingredients listed above, followed by filter sterilization. In the case of sterile powders for the preparation of sterile injectable solutions, the preferred preparation methods are vacuum drying and lyophilization techniques which result in powders of the active ingredient plus any additionally desired ingredients present in the sterile filtration solution.

Useful solid carriers include comminuted solids (e.g., talc, clay, microcrystalline cellulose, silica, alumina, etc.). Useful liquid carriers include water, ethanol or ethylene glycol or a water-ethanol/ethylene glycol mixture, and the pharmaceutical compositions of the present invention may be dissolved or dispersed in the liquid carriers in an effective amount, optionally with the aid of a non-toxic surfactant. Adjuvants (such as fragrances) and additional antimicrobial agents can be added to optimize the properties for a given use.

Thickeners (such as synthetic polymers, fatty acids, fatty acid salts and esters, fatty alcohols, modified celluloses or modified inorganic materials) can also be used with liquid carriers to form coatable pastes, gels, ointments, soaps, etc. that are used directly on the user's skin.

The therapeutically effective amount of the active ingredient will depend not only on the particular salt selected, but also on the administration route, the property of the disease to be treated, and the age and conditions of the patient, and will ultimately depend on the decision of the attending physician or clinician.

The above formulations may be presented in unit dosage form, which is a physically discrete unit containing a unit dose suitable for administration to humans and other mammalian bodies. The unit dosage form may be a capsule or a tablet. The amount of active ingredient in the unit dose may vary or be adjusted between about 0.01 to about 1000 mg or more, depending on the particular treatment involved.

The term "treatment" as used herein generally refers to the acquisition of the desired pharmacological and/or physiological effect. The effect may be prophylactic according to the prevention of the disease or its symptoms in whole or in part; and/or may be therapeutic according to the partial or complete stabilization or cure of the disease and/or the side effect due to the disease. As used herein, "treatment" encompasses any treatment for the disease of a patient, including: (a) prevention of the disease or condition in the patient that may be predisposed to the disease or condition

but has not yet been diagnosed; (b) inhibition of the symptoms of the disease, i.e., preventing its development; or (c) remission of the symptoms of the disease, i.e., causing regression of the disease or symptoms in whole or in part.

The compound described herein, or pharmaceutically acceptable salts, isomers, solvates, hydrates, adducts, complexes or prodrugs thereof, may also be administered in combination with one or more additional therapeutic agents for the treatment of cancers. Such additional therapeutic agents include, but are not limited to, anthracyclines, cyclophosphamide, 5-fluorouracil, cisplatin, and the like.

Unless otherwise stated, percentages, ratios, proportions or parts used in this application are by weight or volume. The amount used in this application is a weight or volume amount. Those skilled in the art can easily determine.

In yet another aspect of the present application, the present application further includes a compound or pharmaceutically acceptable salts, isomers, solvates, hydrates, adducts, complexes or prodrugs thereof obtained by combining any group defined at any variable group of the present application.

The application also includes the following exemplary compounds.

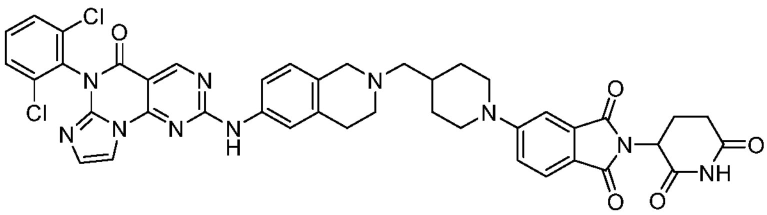

,

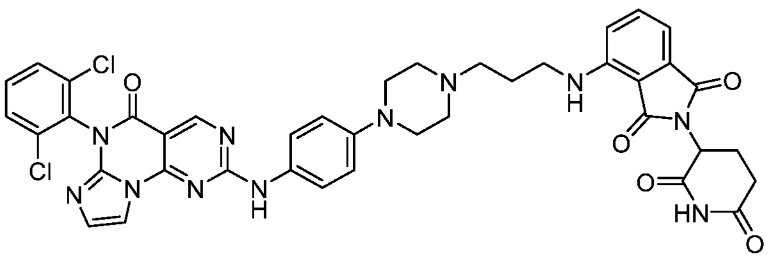

,

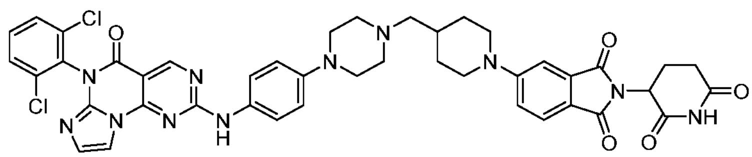

,

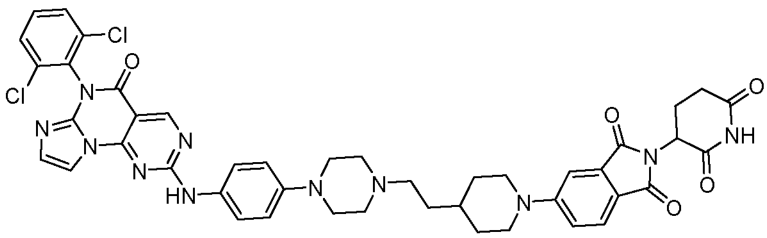

,

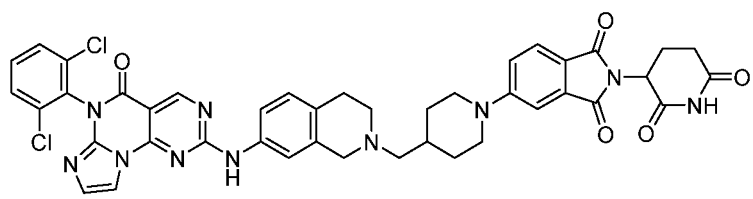

,

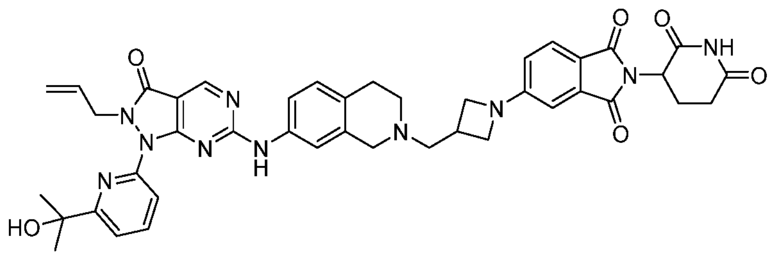

,

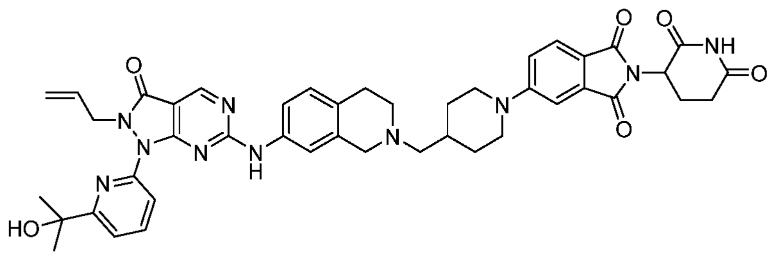

,

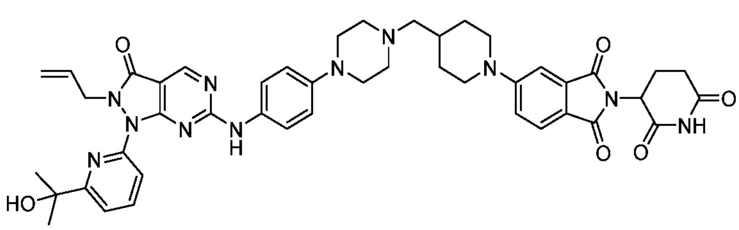

,

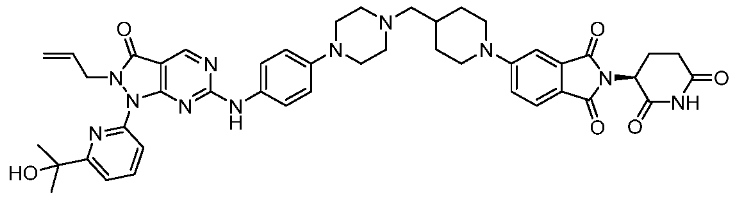

,

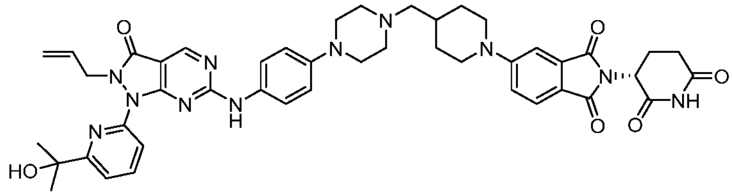

,

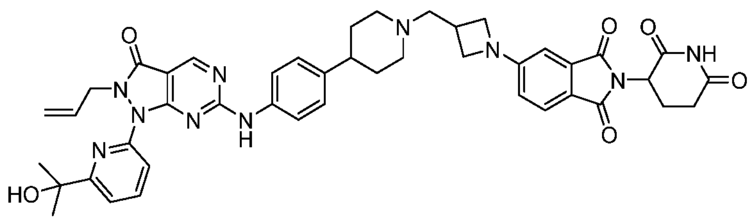

,

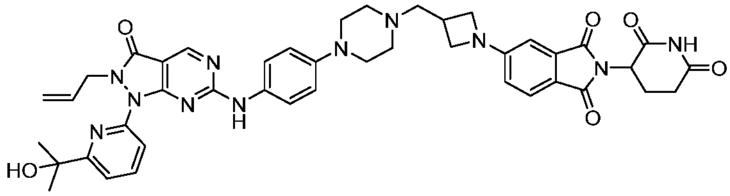
,

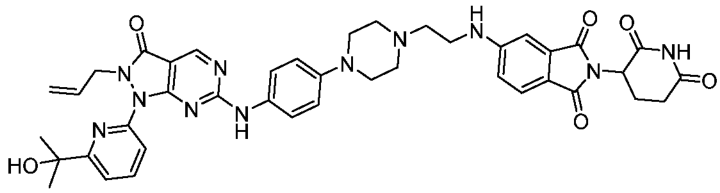
,

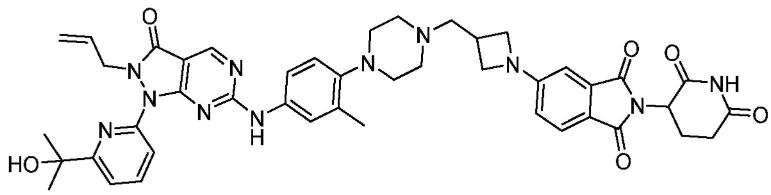
,

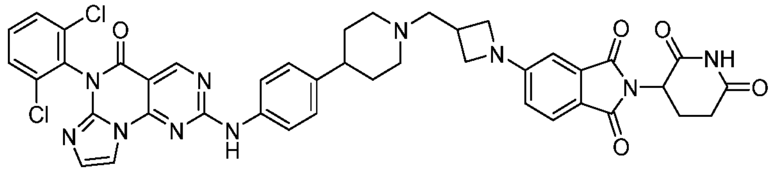
,

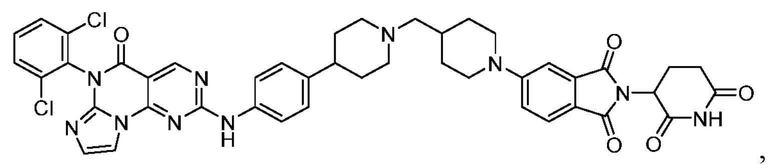
,

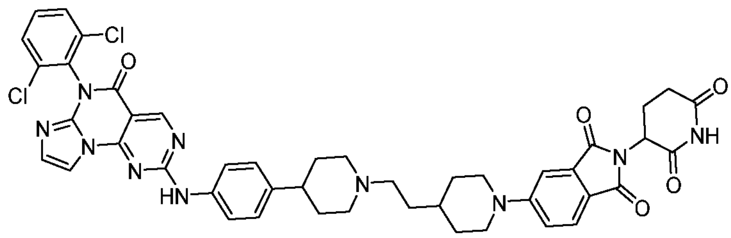
,

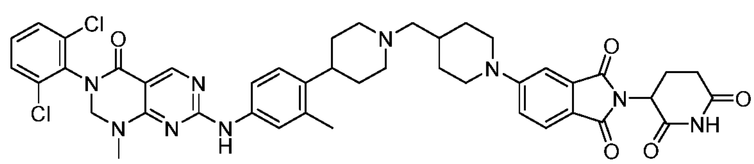
,

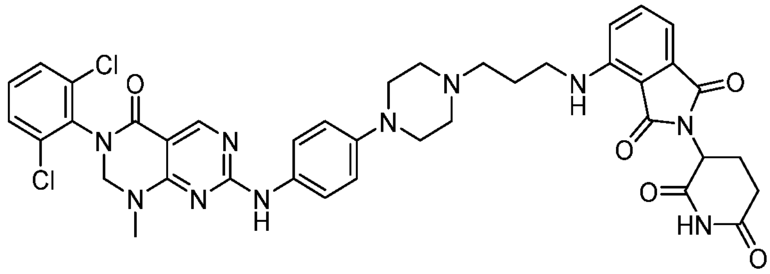,

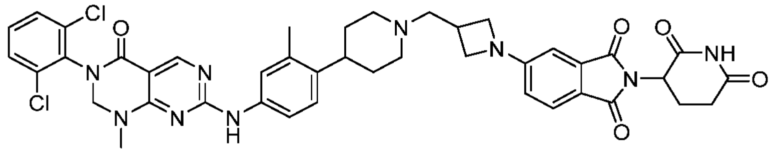,

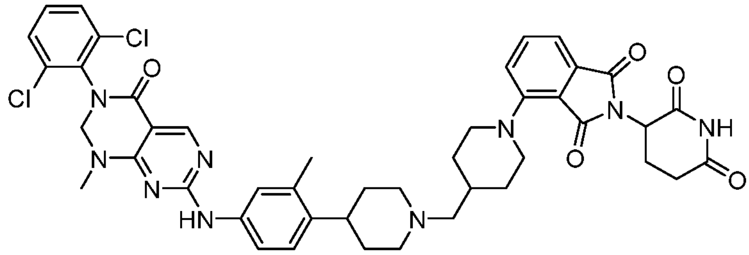,

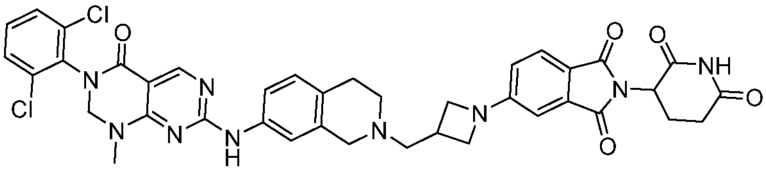,

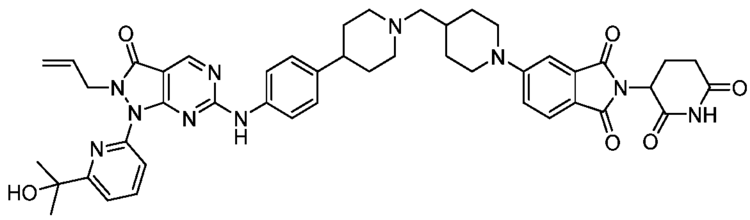,

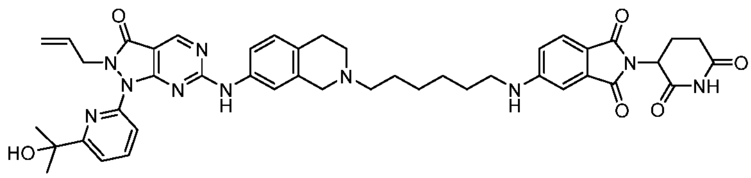,

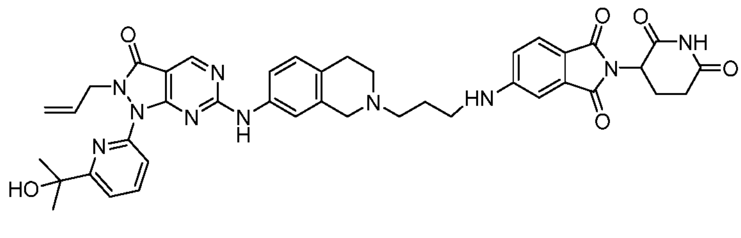,

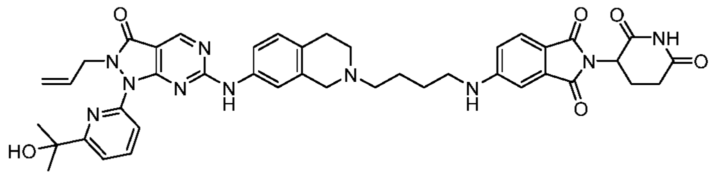

,

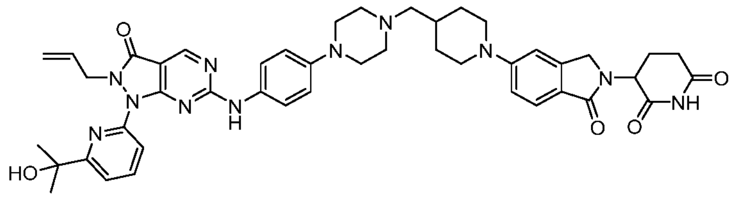

,

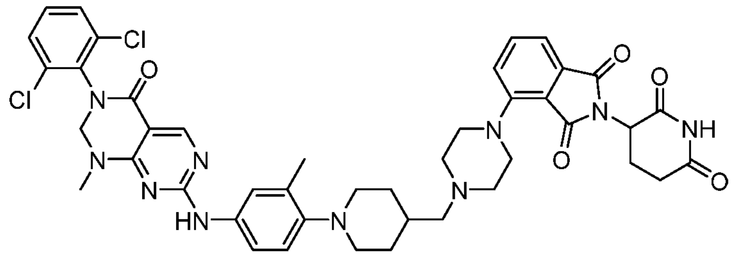

,

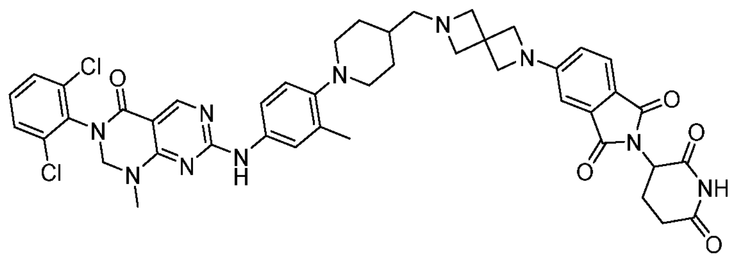

,

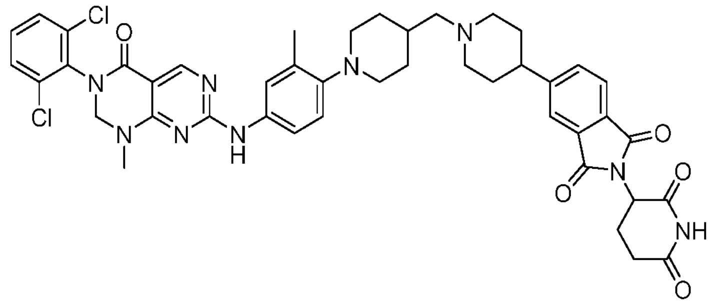

,

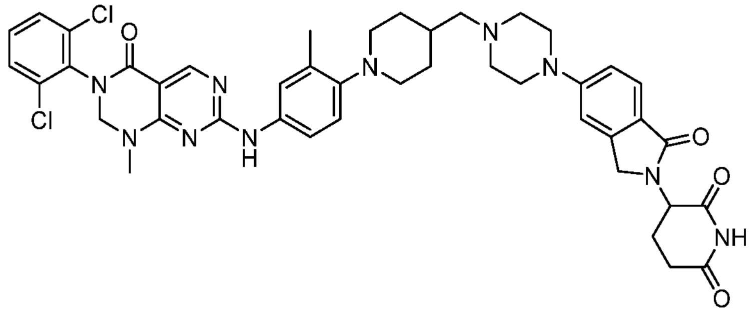

,

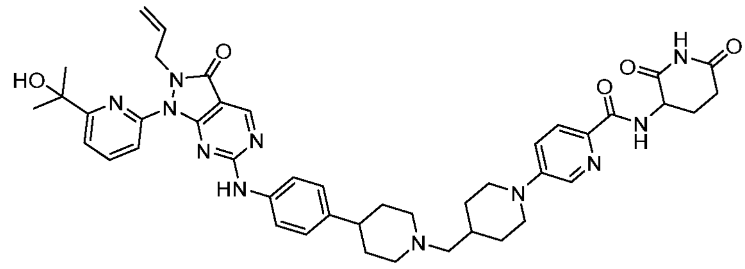

,

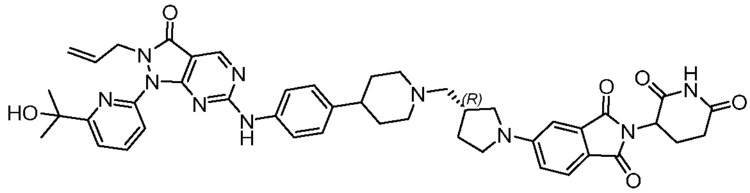

,

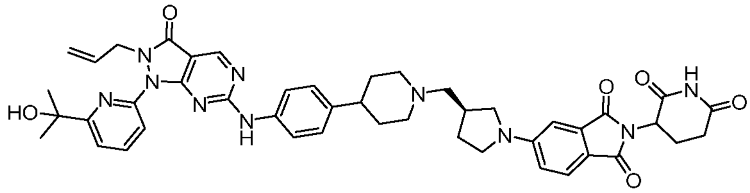

,

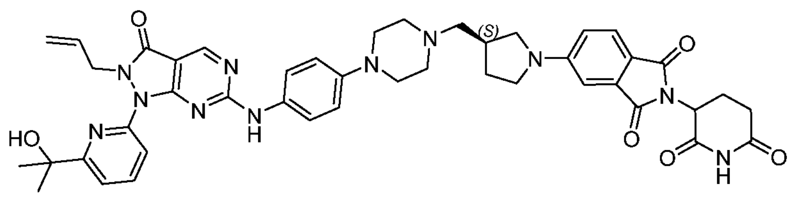

,

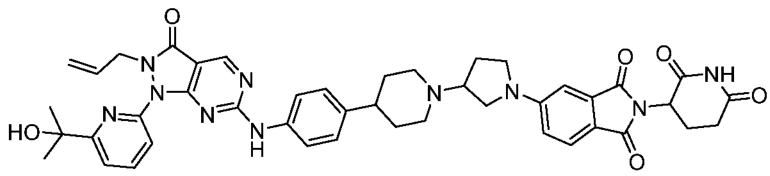

,

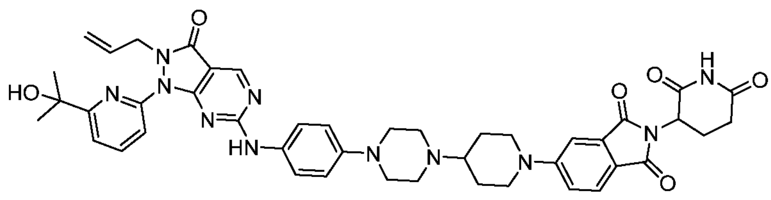

,

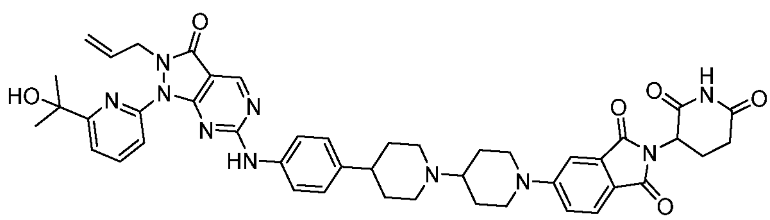

,

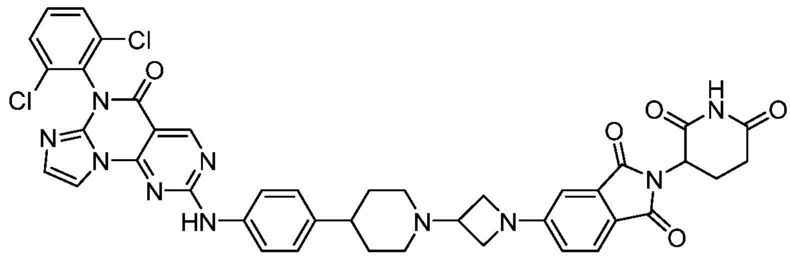

,

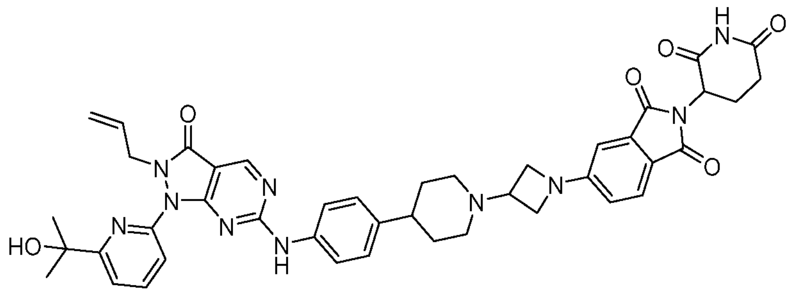

,

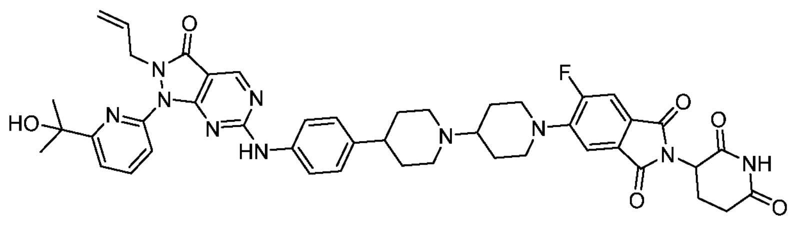

,

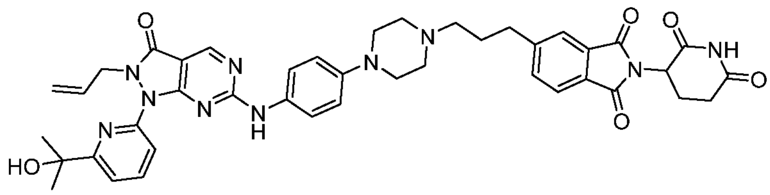

,

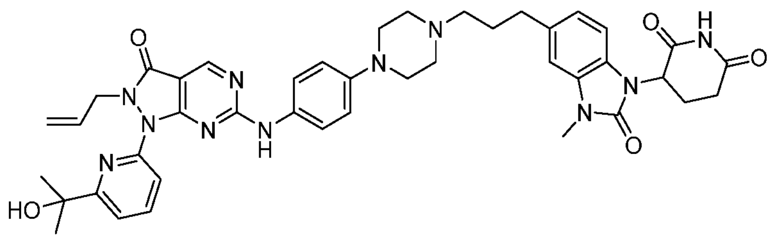

,

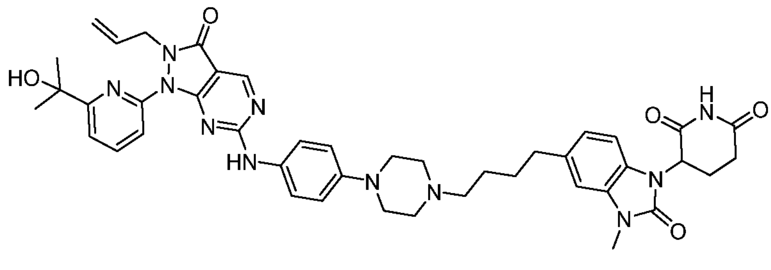

,

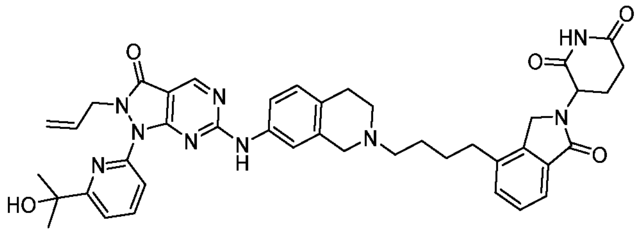

,

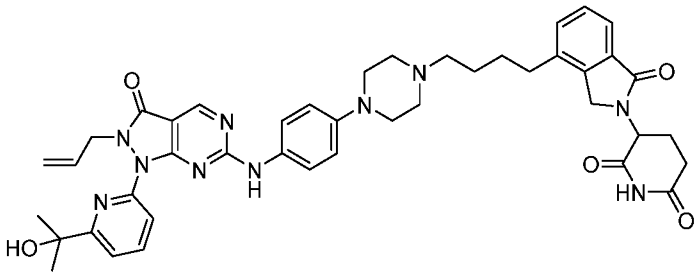

,

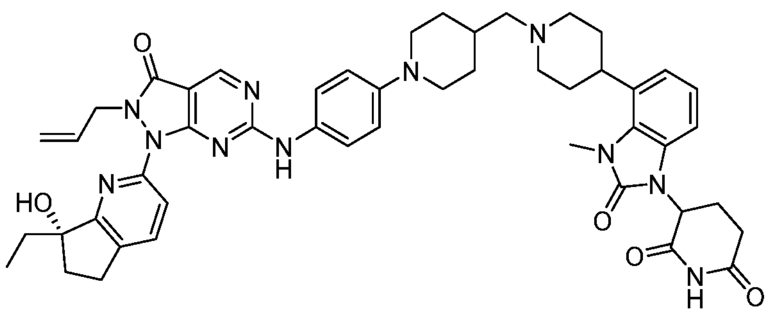

,

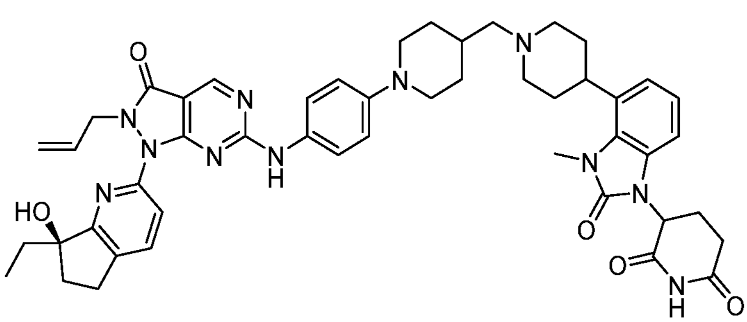

,

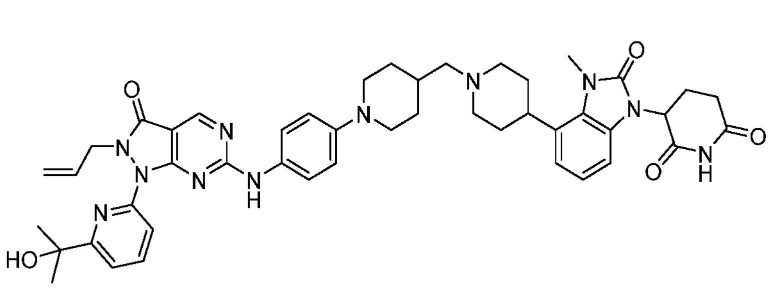

.

,

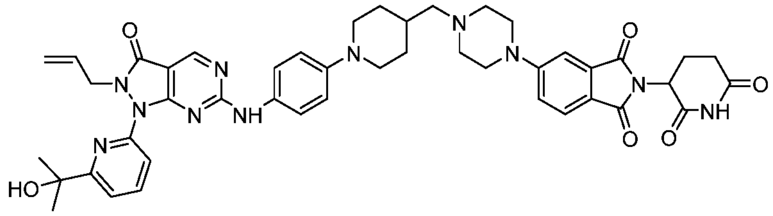

,

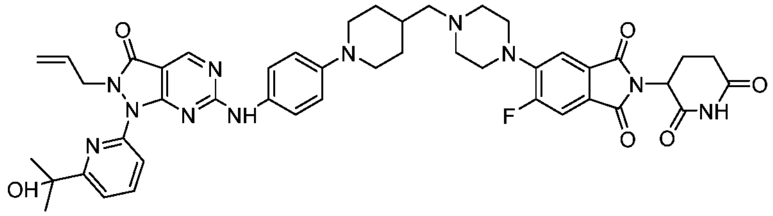

,

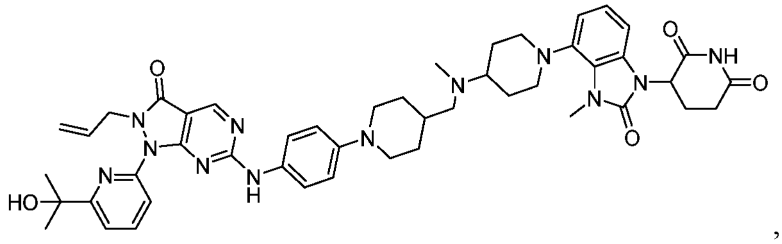

,

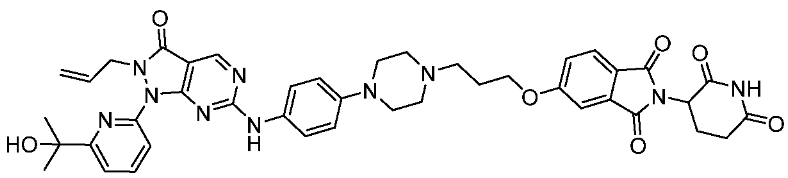

,

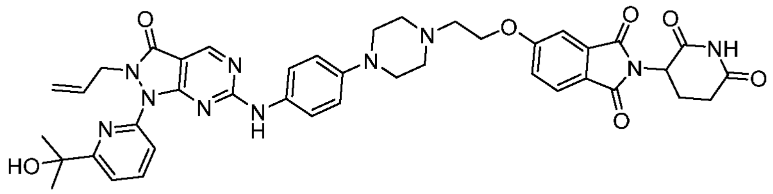

,

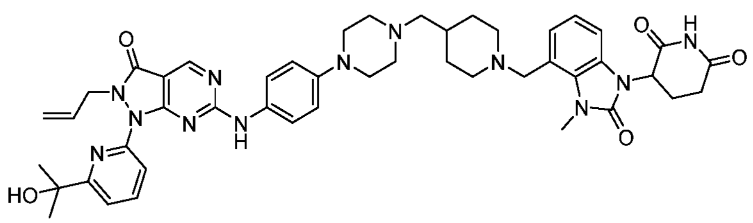

,

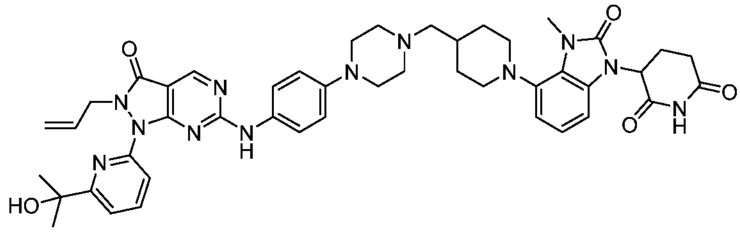

,

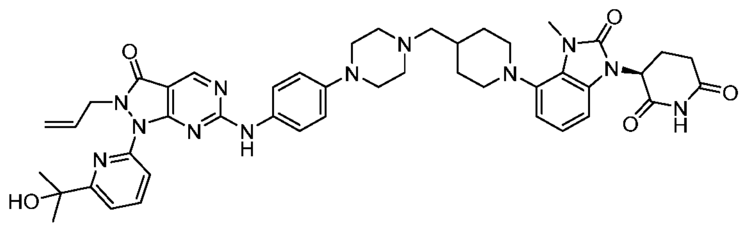

,

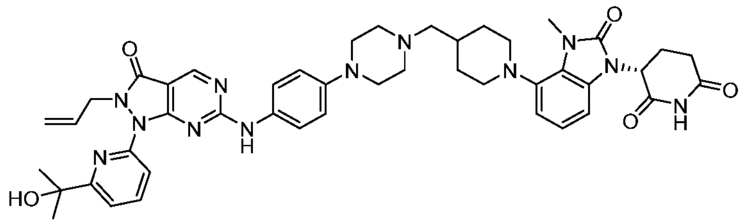

,

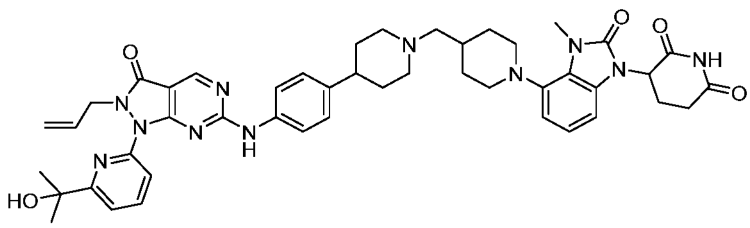

,

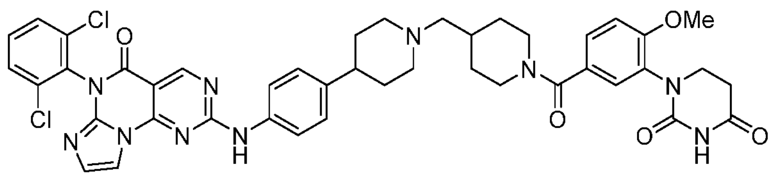

,

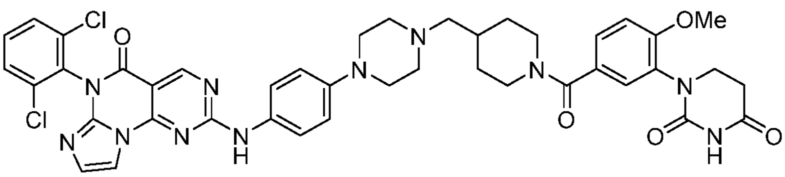

,

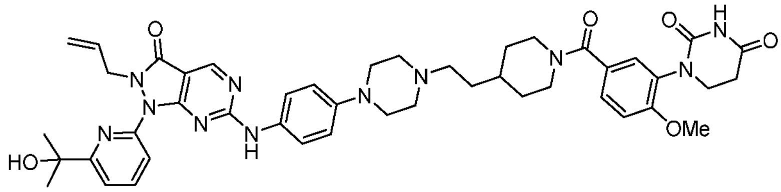

,

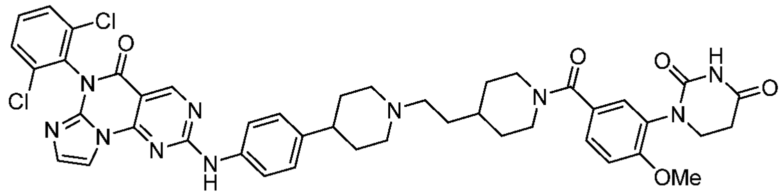

,

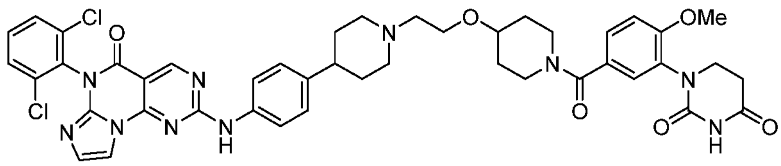

,

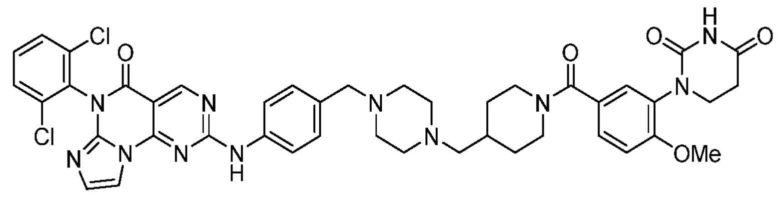

,

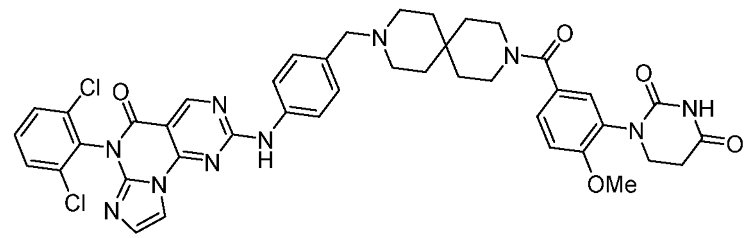

,

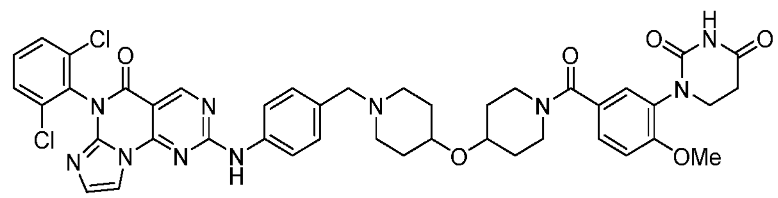

,

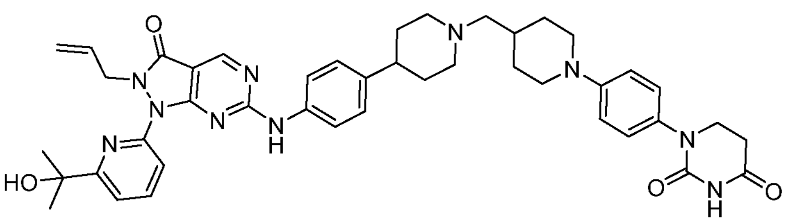

,

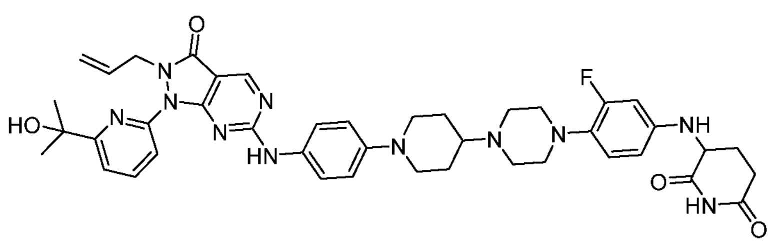

,

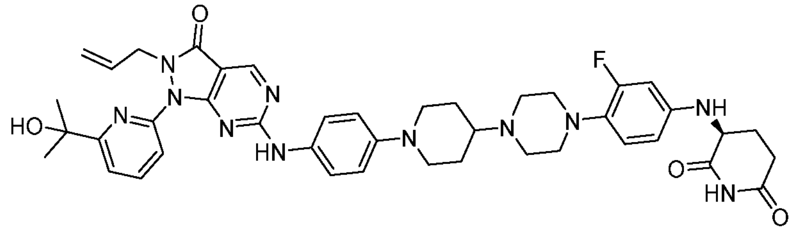,

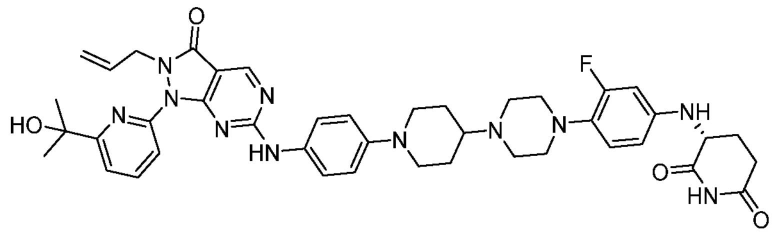,

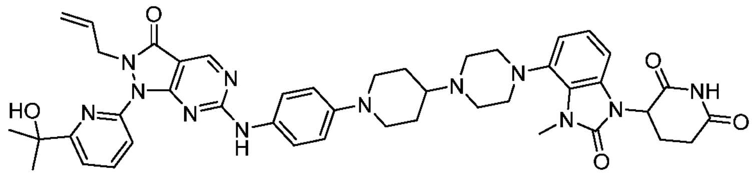,

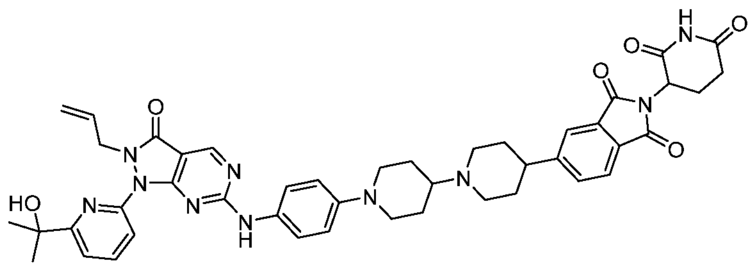,

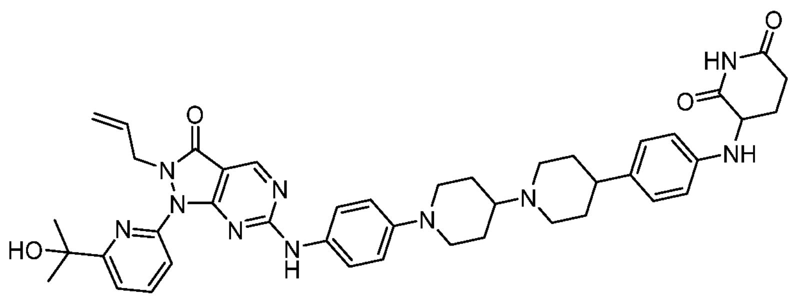,

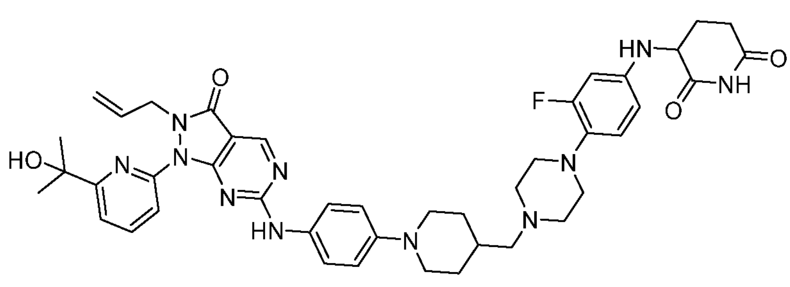,

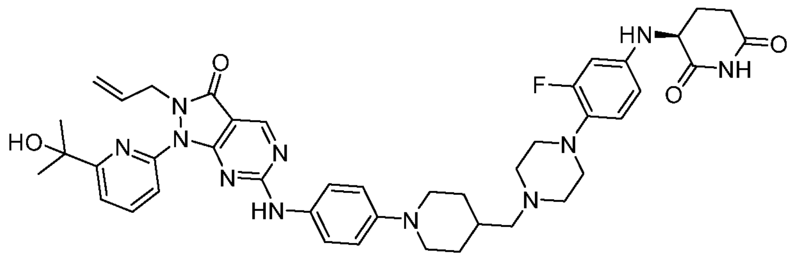

,

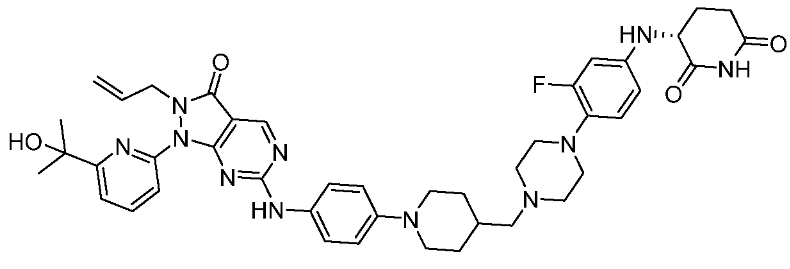

,

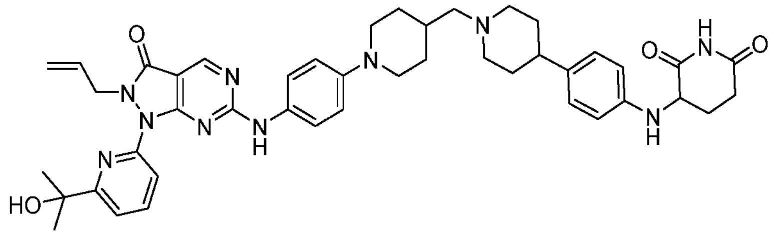

,

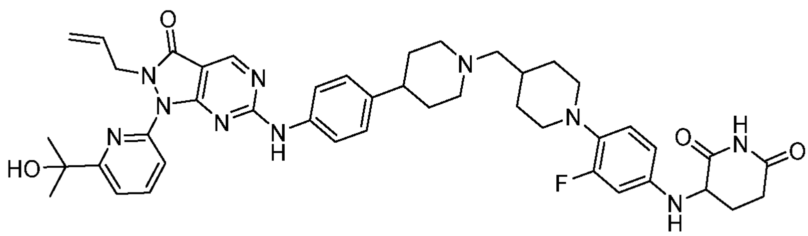

,

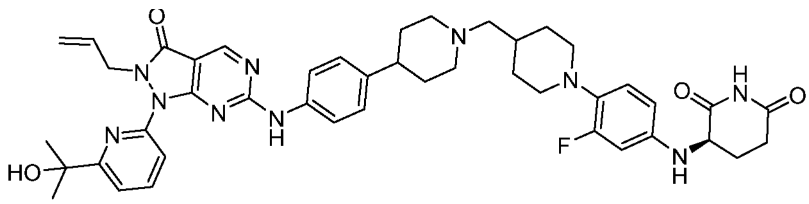

,

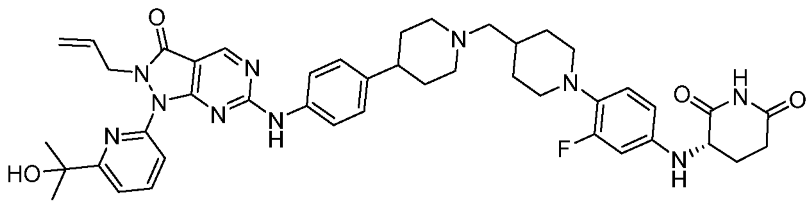

,

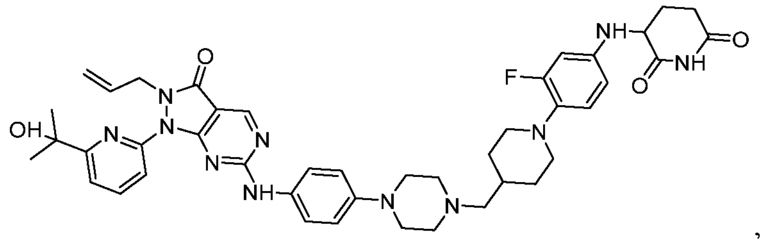

,

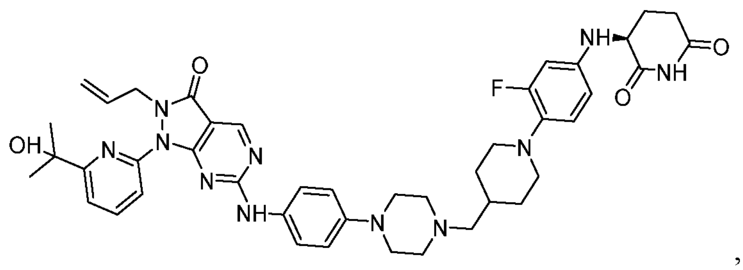

,

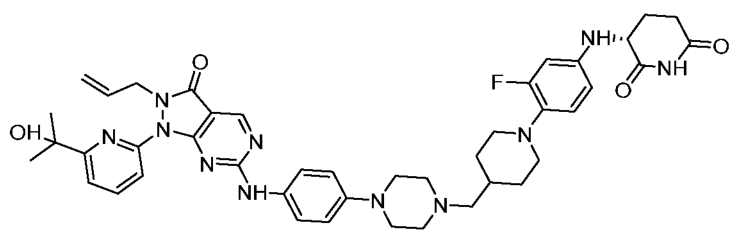

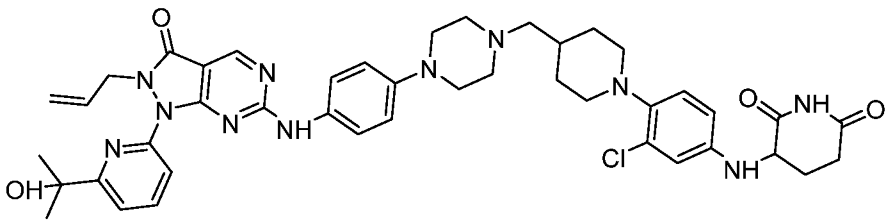

,

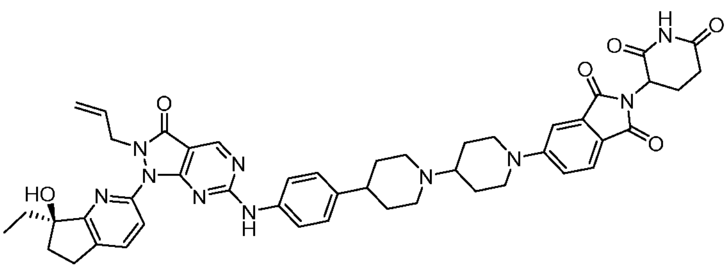

,

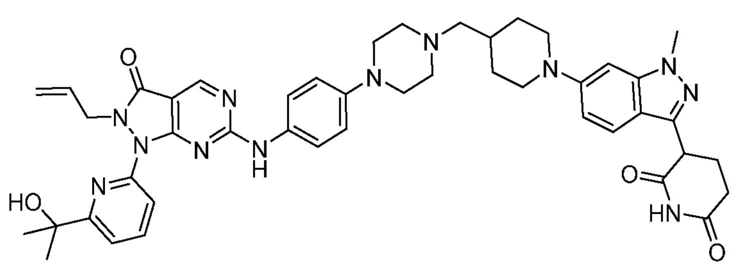

,

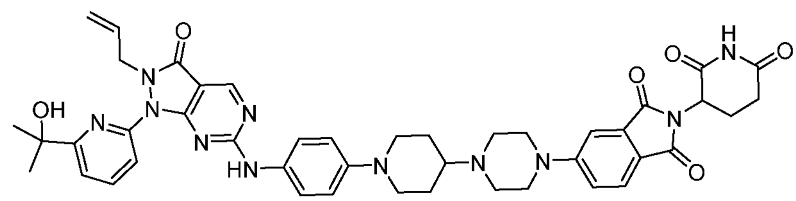

,

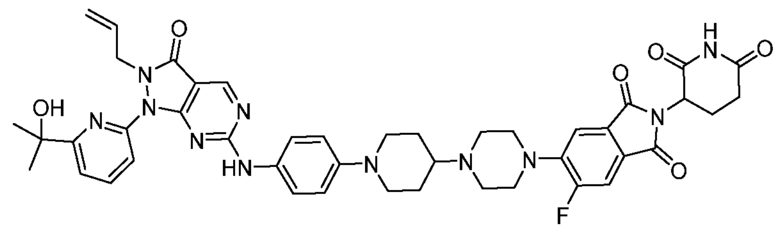

,

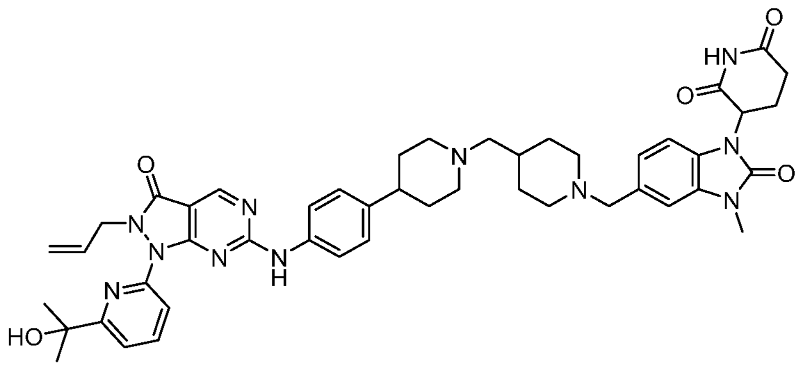

,

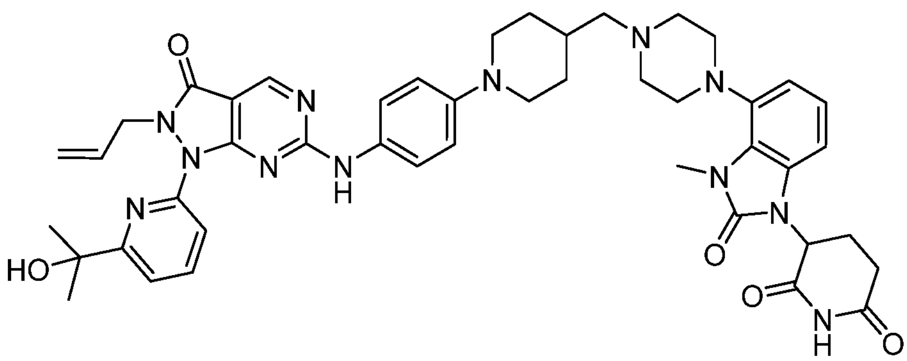

,

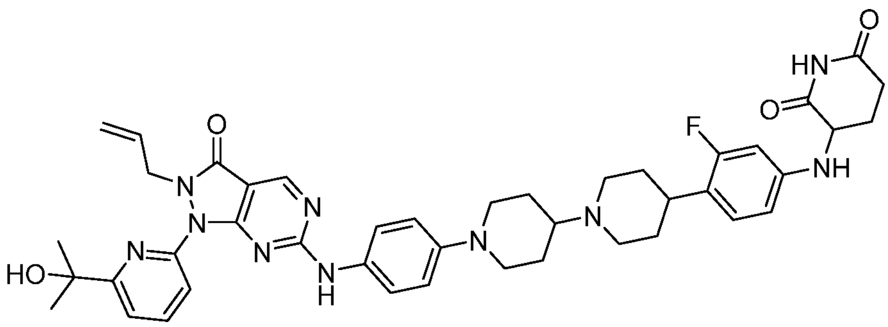

,

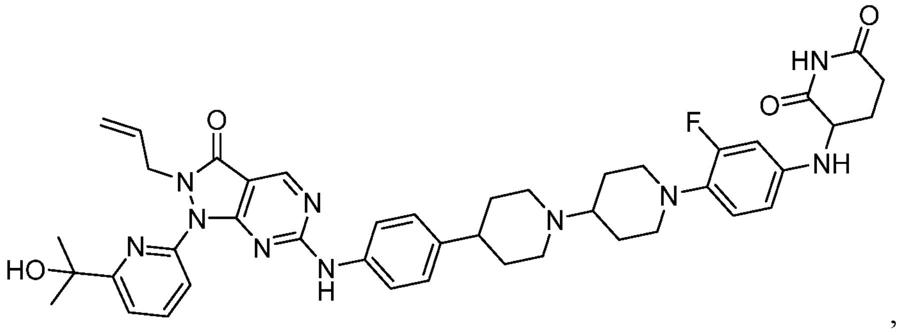

,

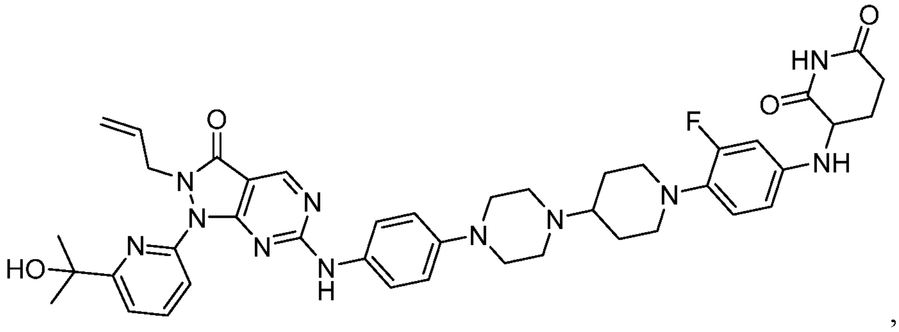

,

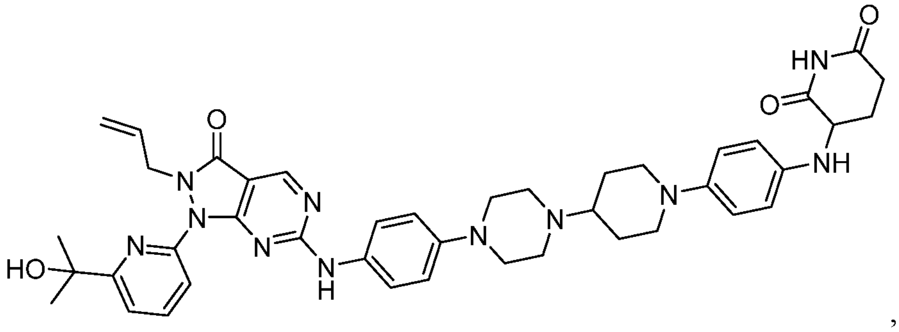

,

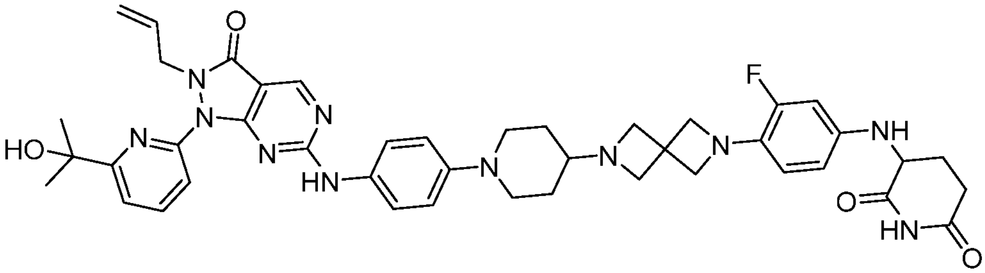

,

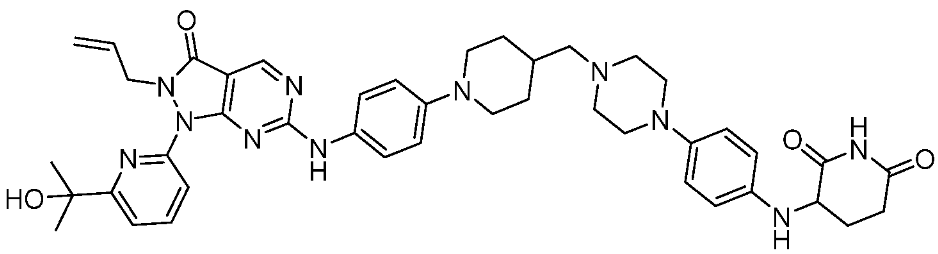

,

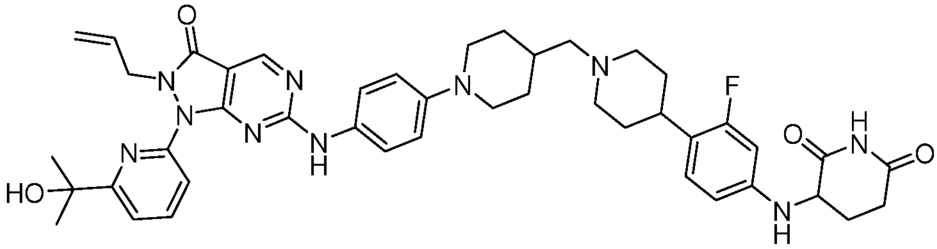

,

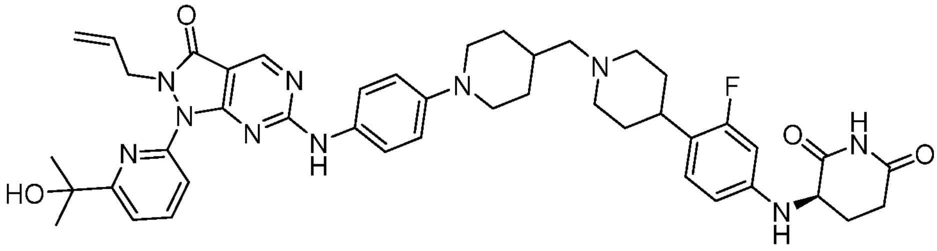

,

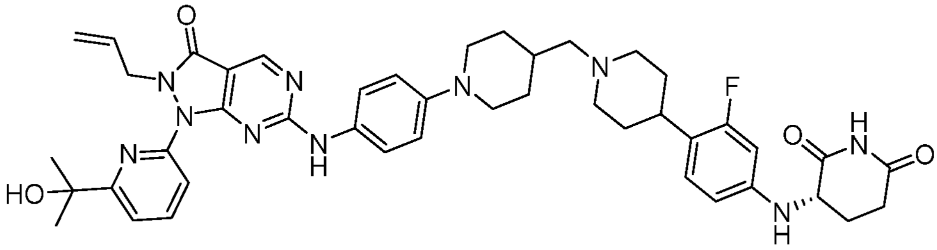

,

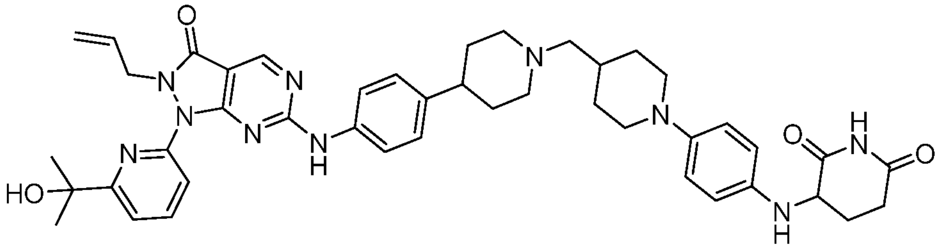

,

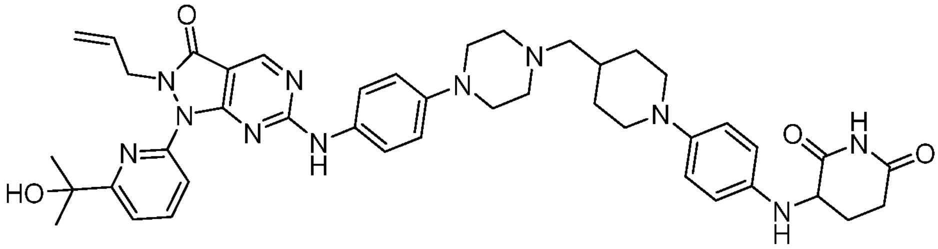

,

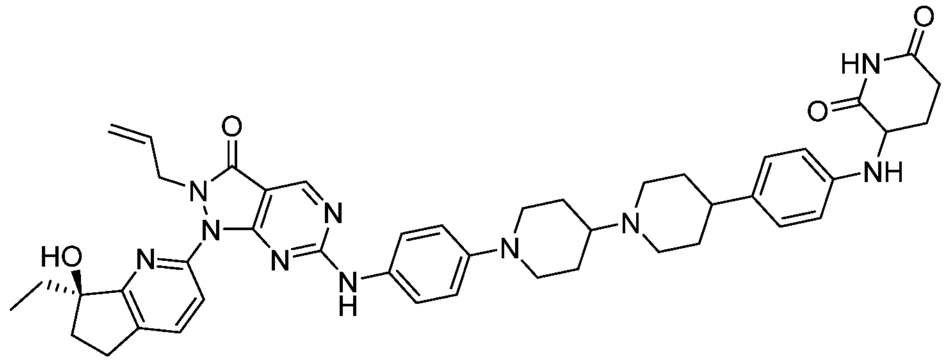

,

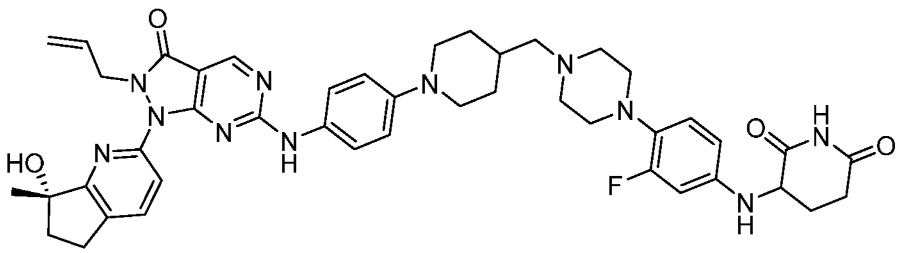

,

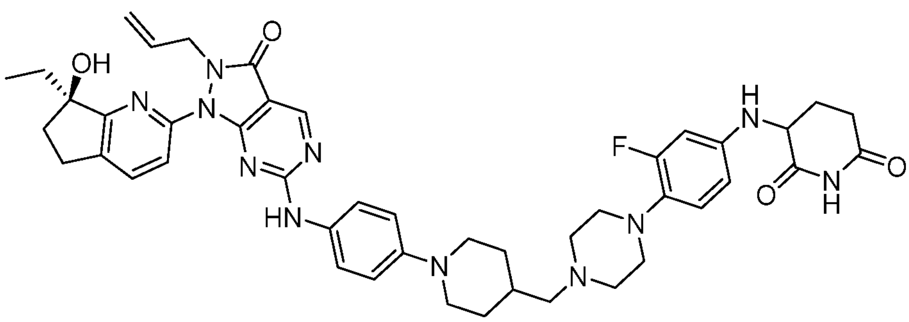

,

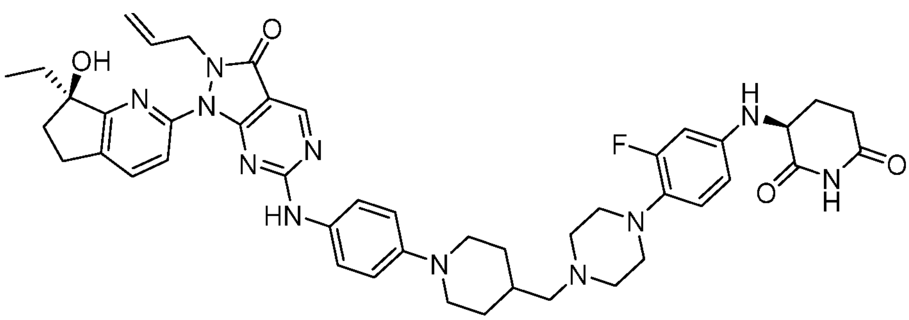

,

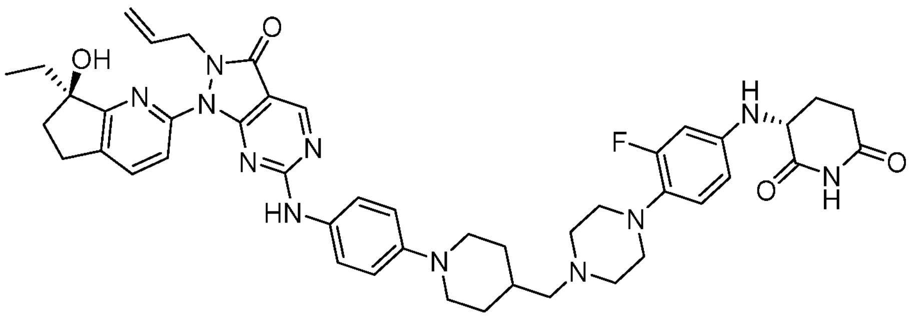

,

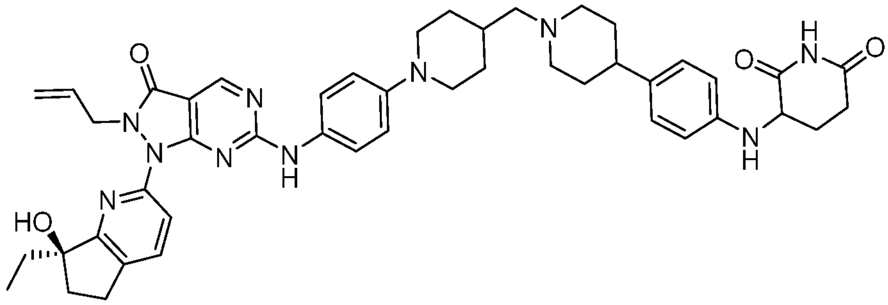

,

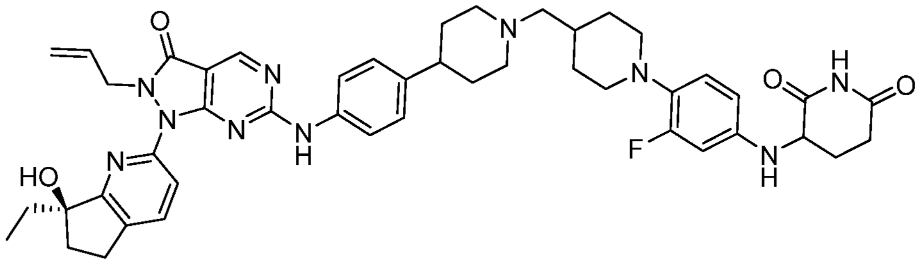

,

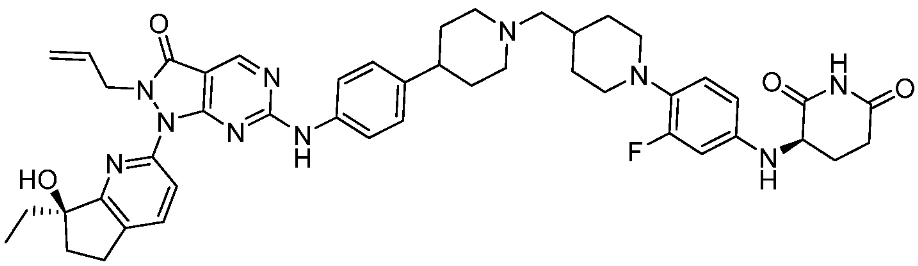

,

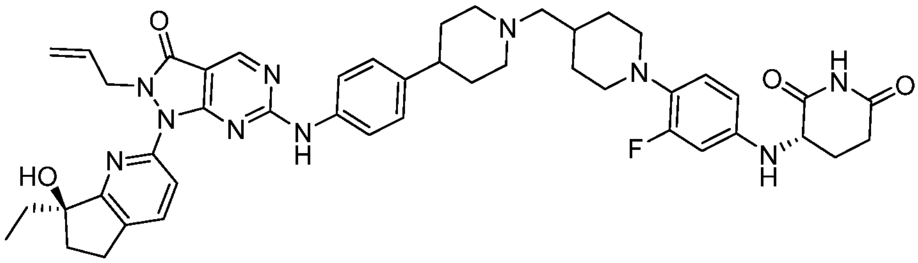

,

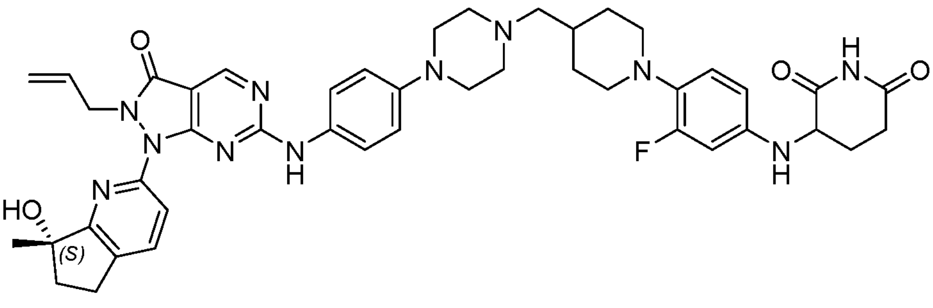

,

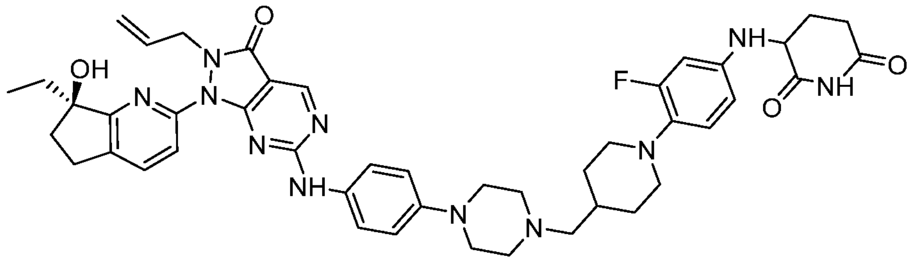

,

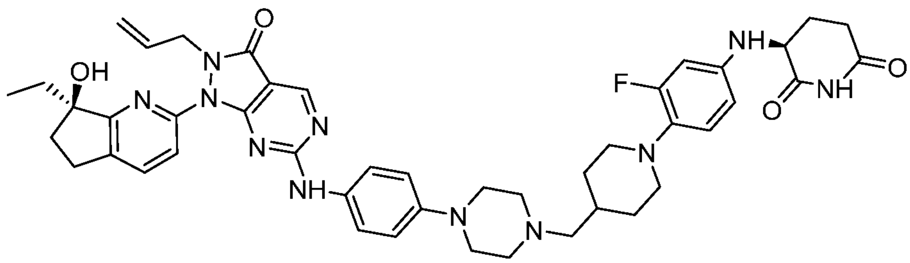

,

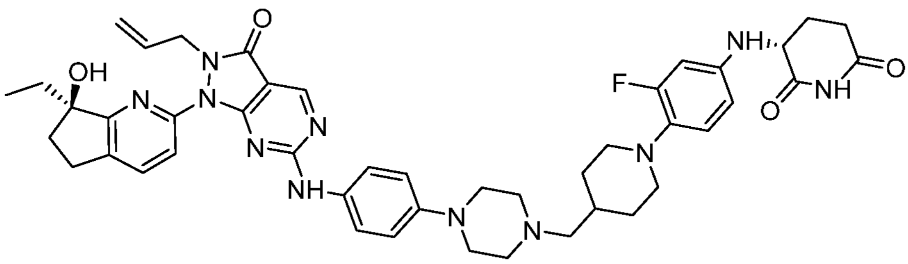

, ,

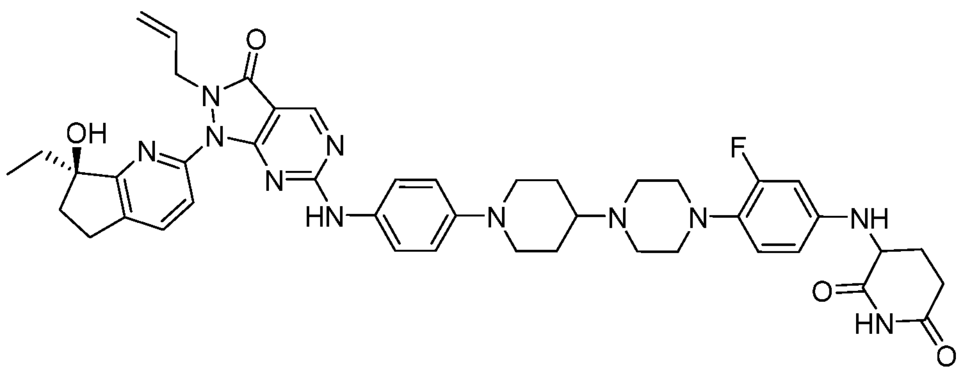

,

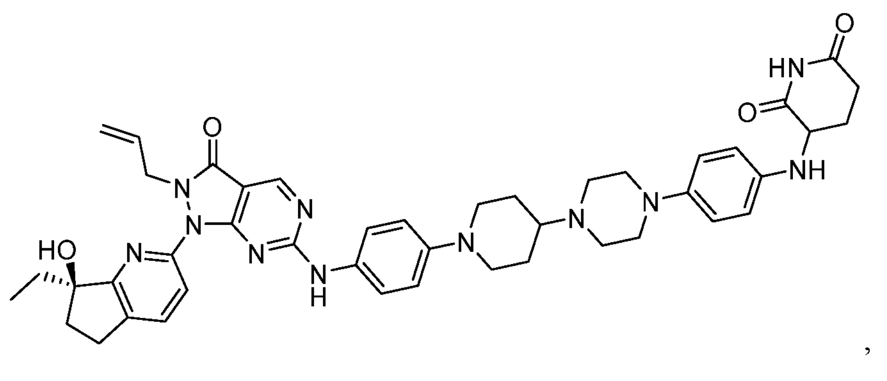

,

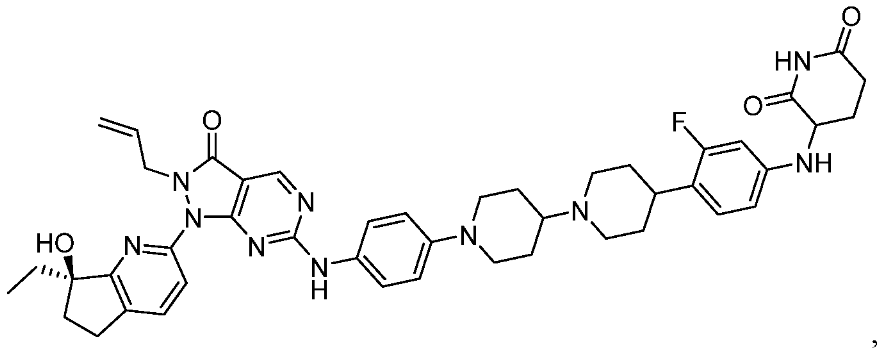

,

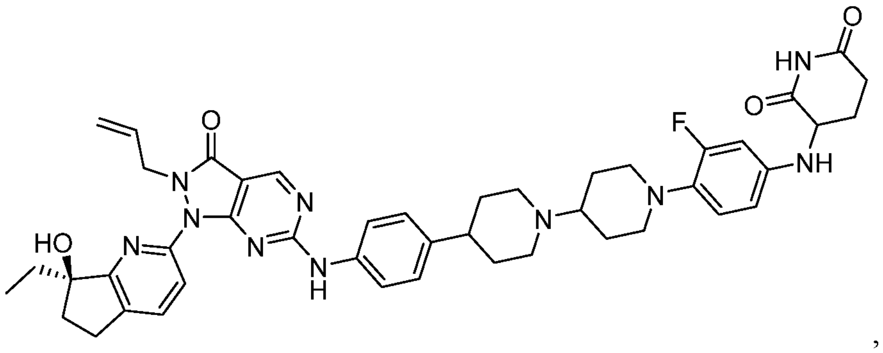

,

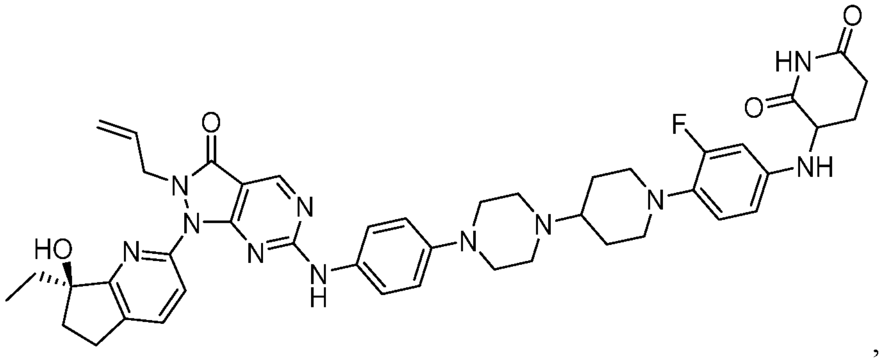

,

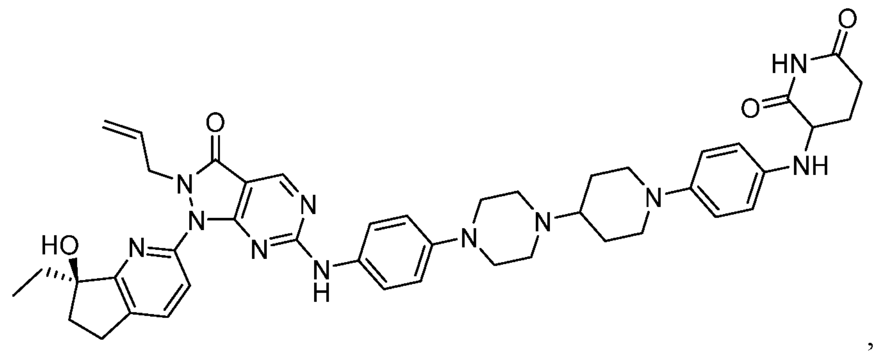

,

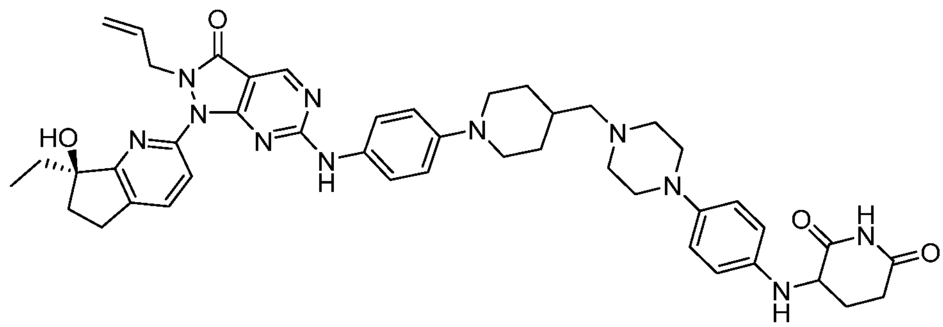

,

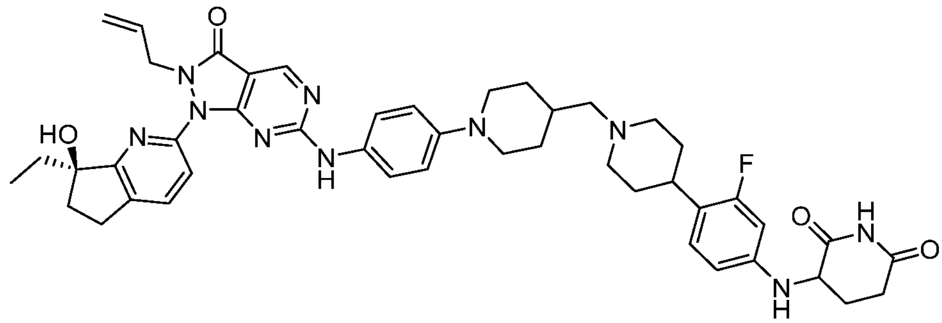

,

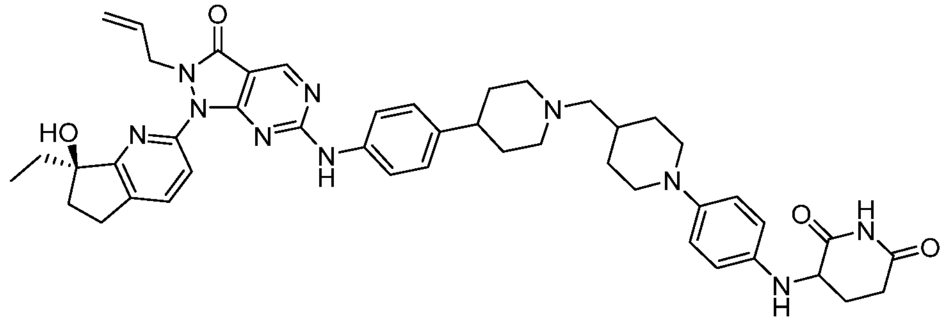

,

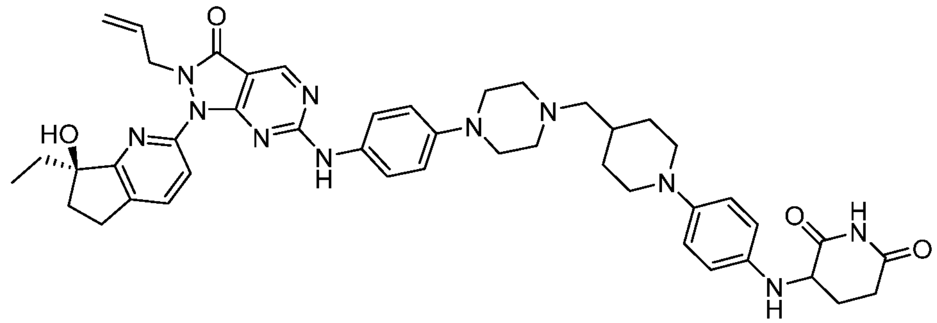

,

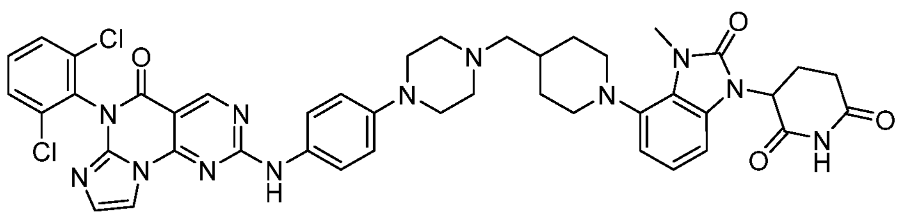

,

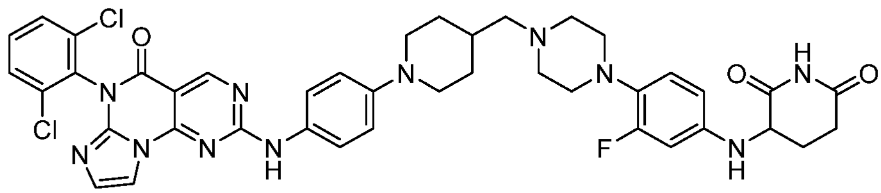

,

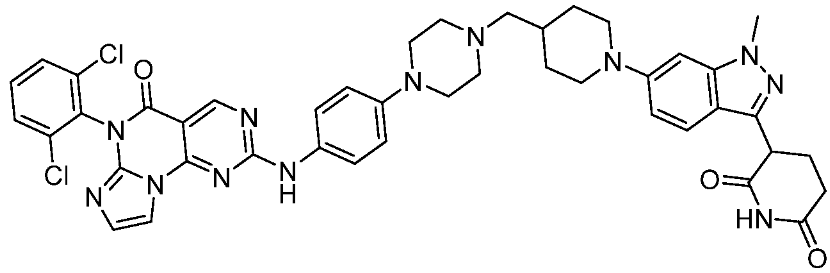

,

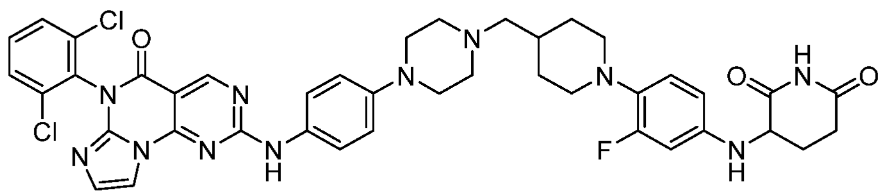

,

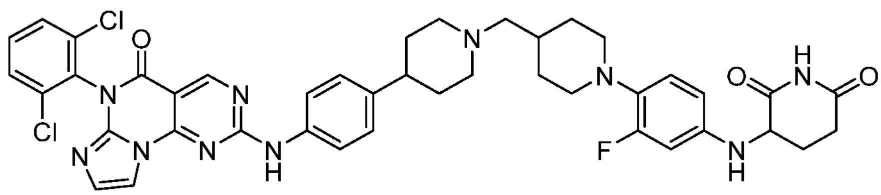

,

,

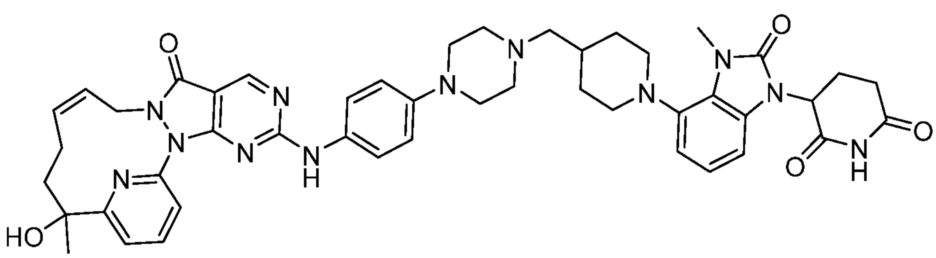

,

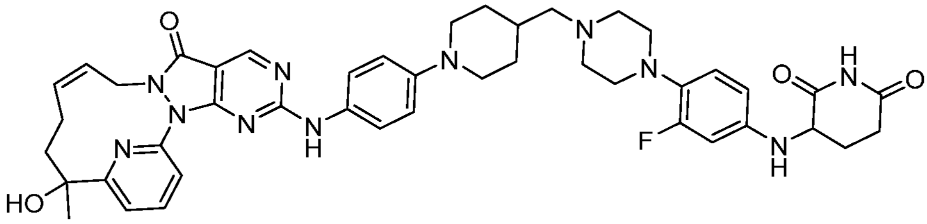

,

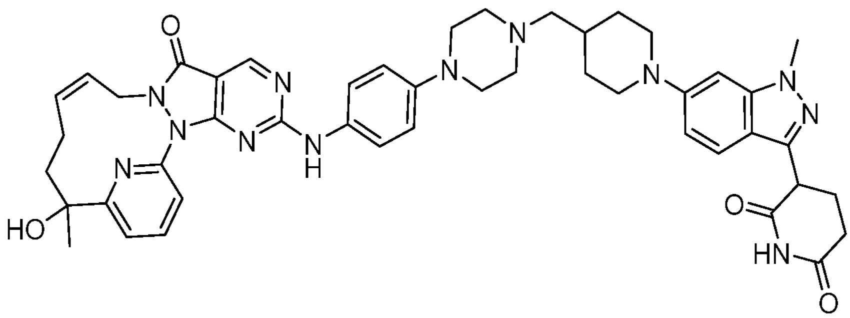,

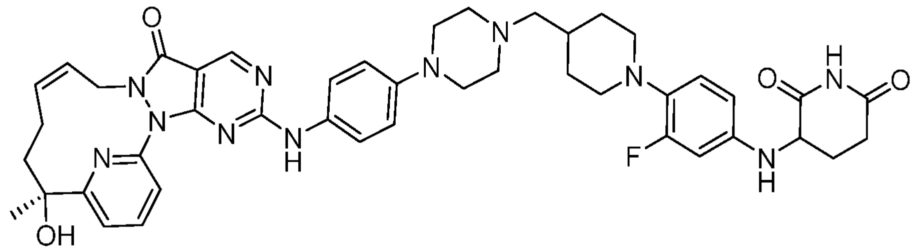,

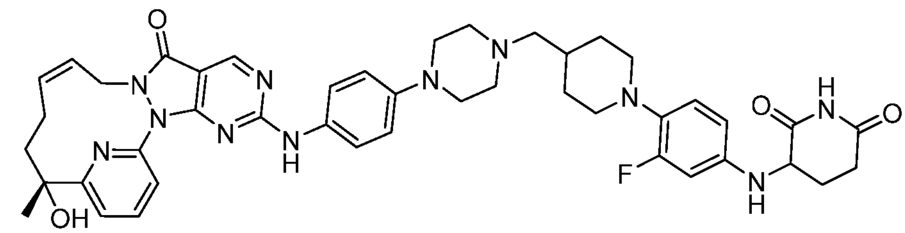,

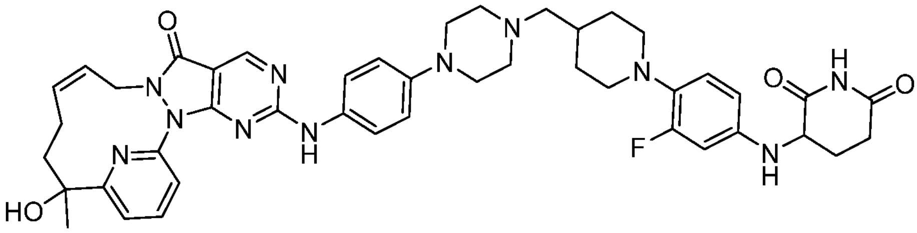,

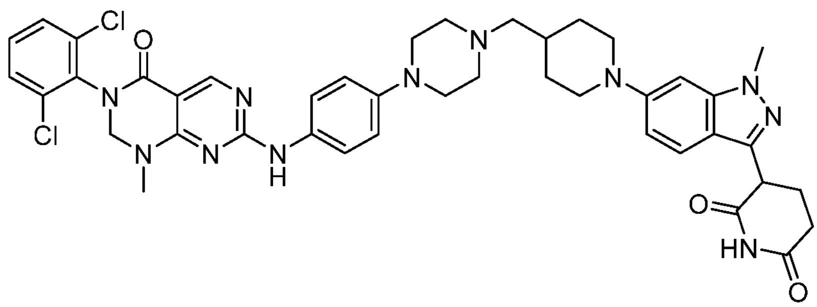,

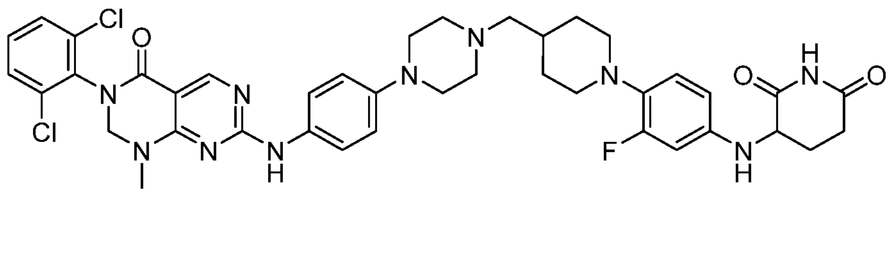,

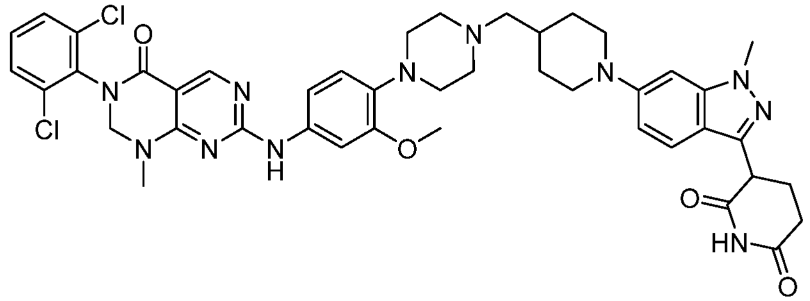

,

,

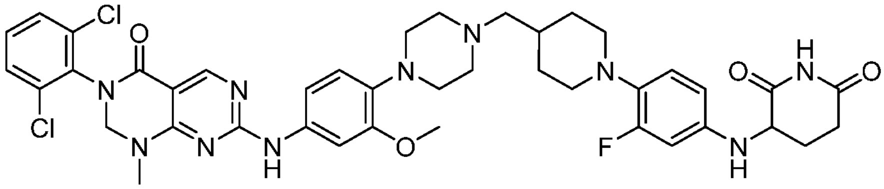

,

,

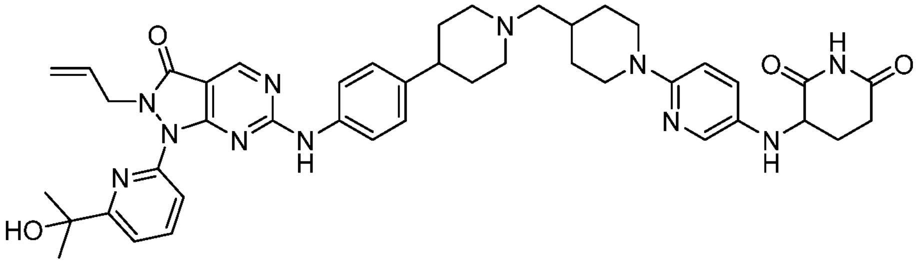

,

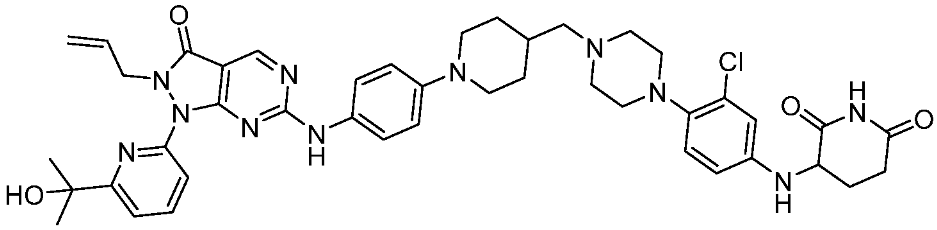

,

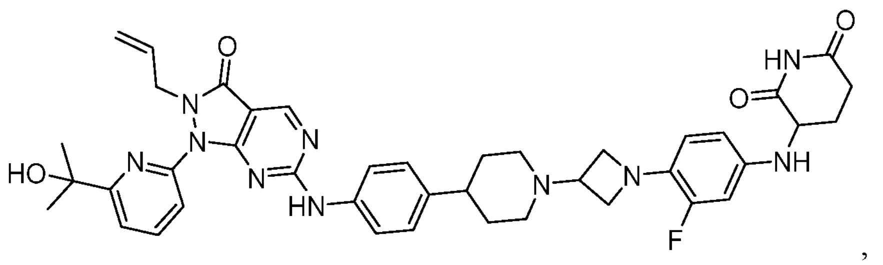

,

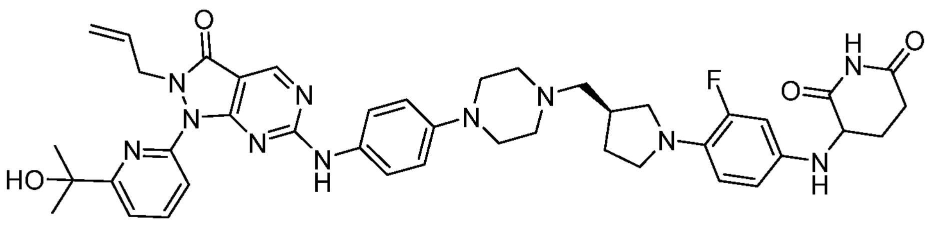

,

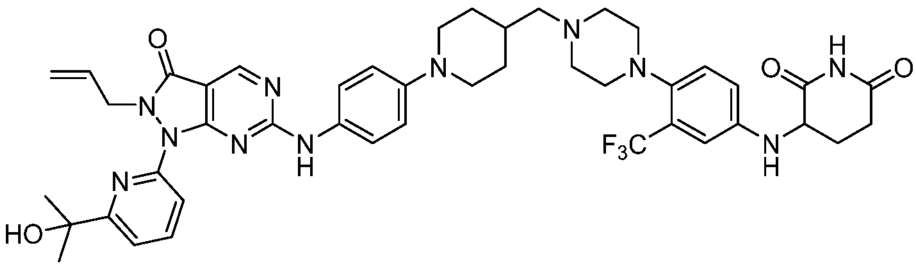

,

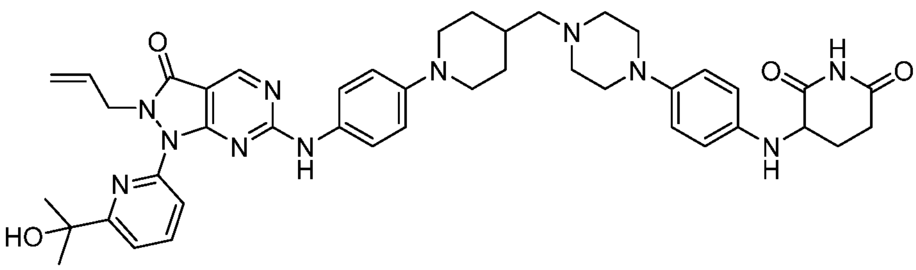

,

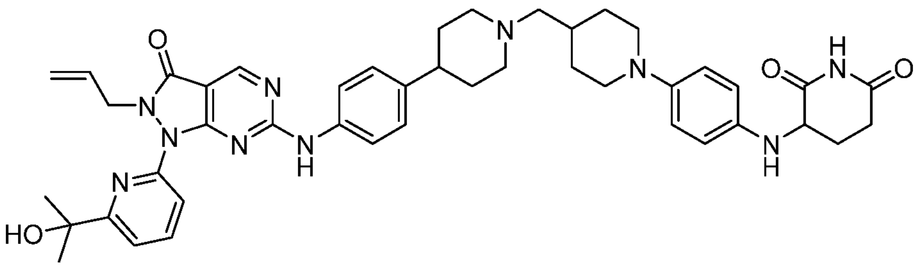

,

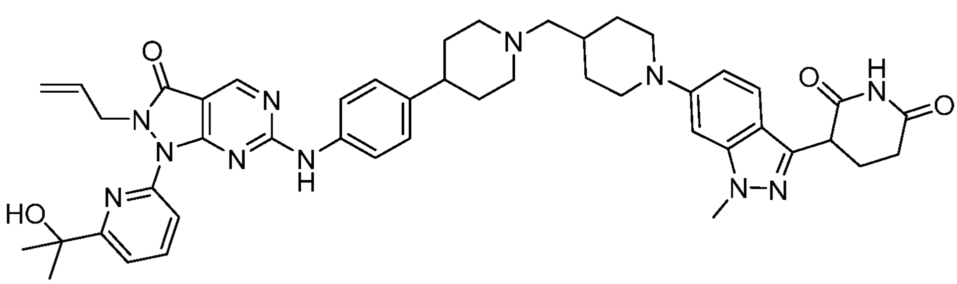

,

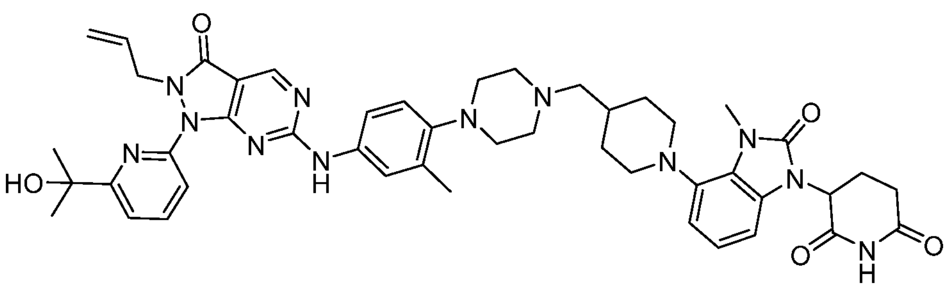

,

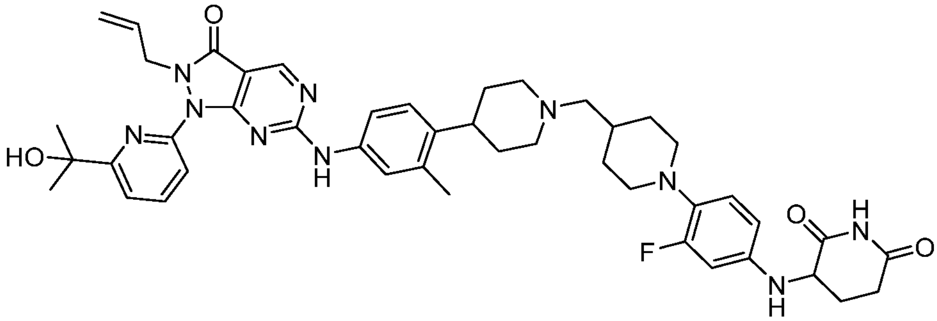

,

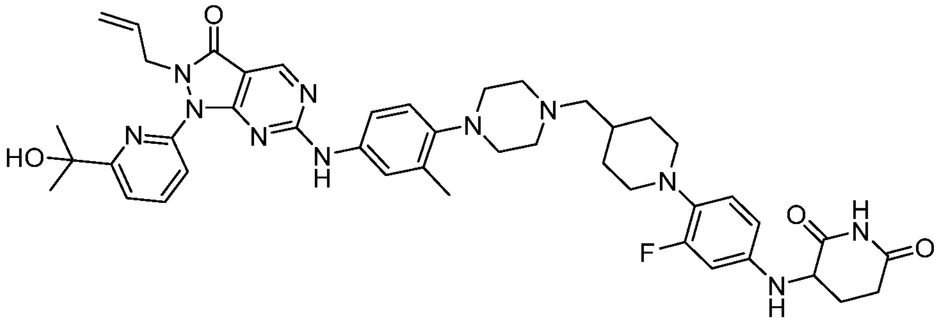

,

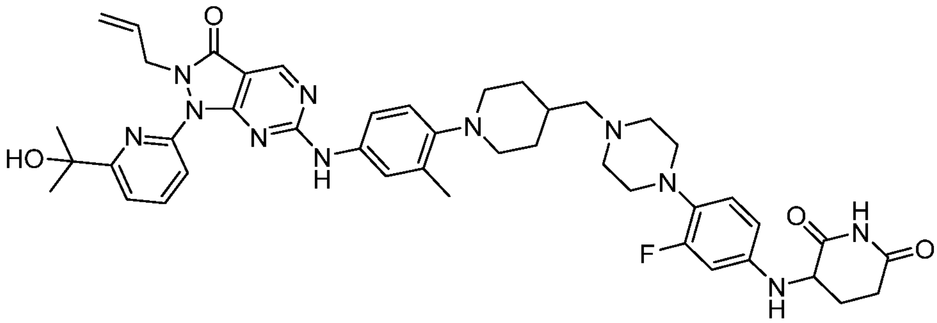

,

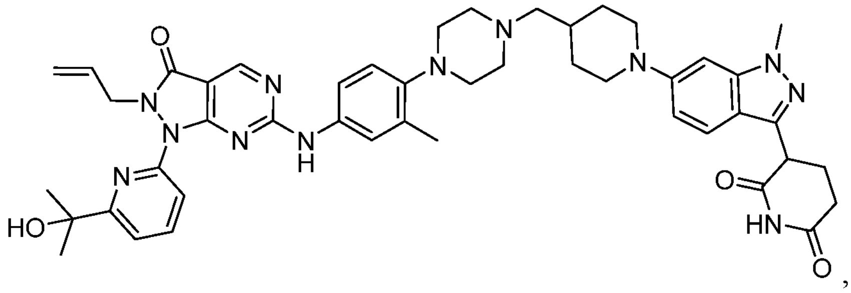

,

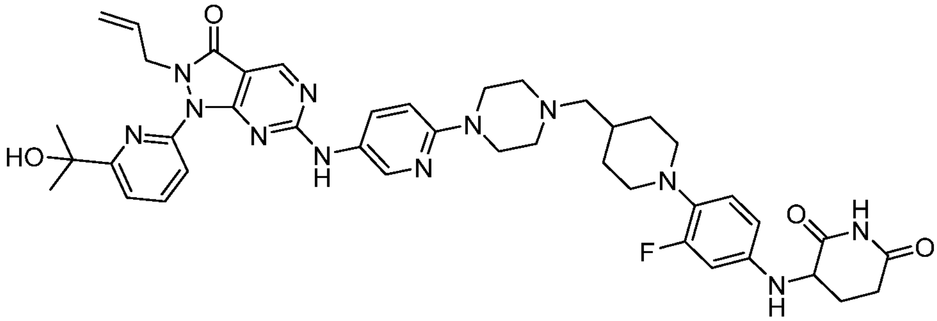

,

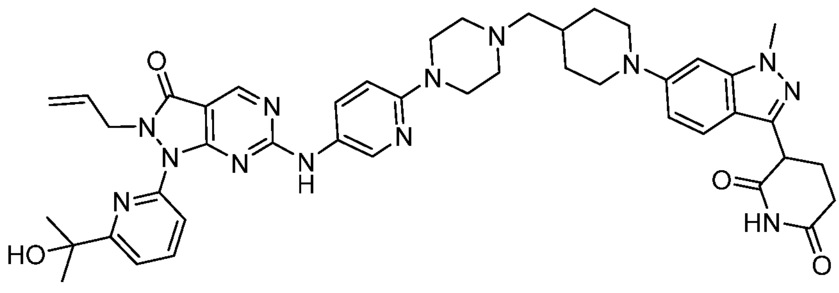

,

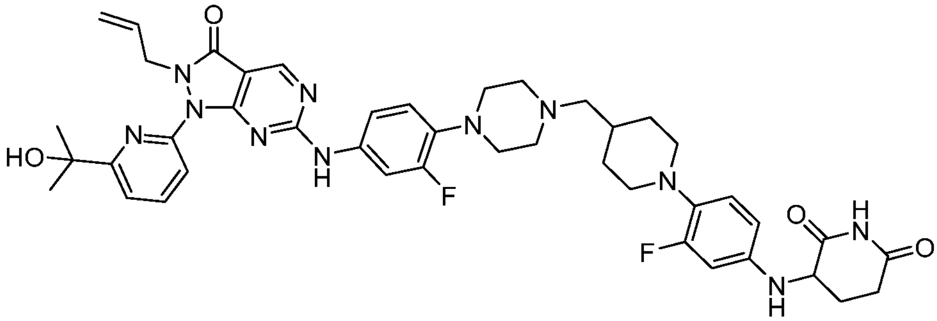

,

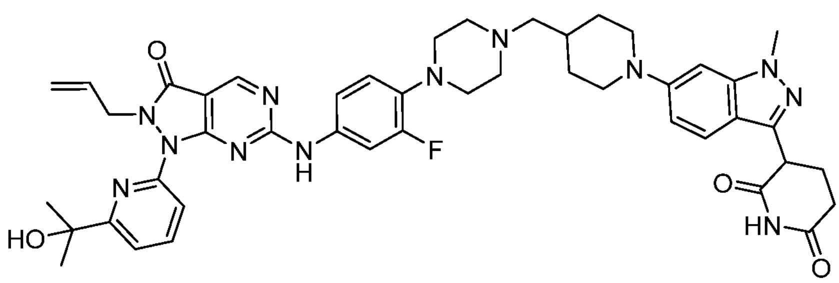

,

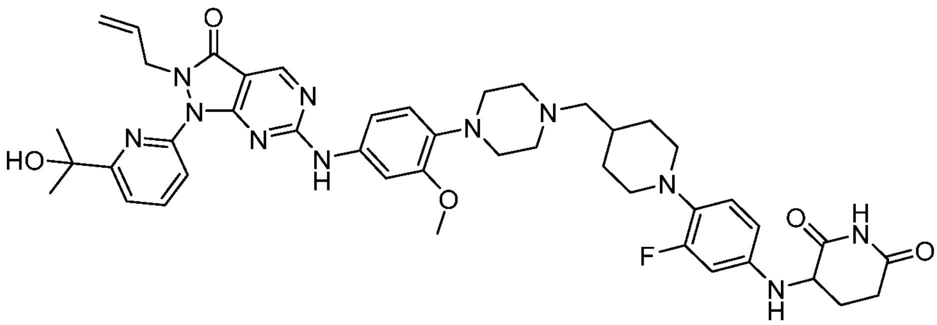

,

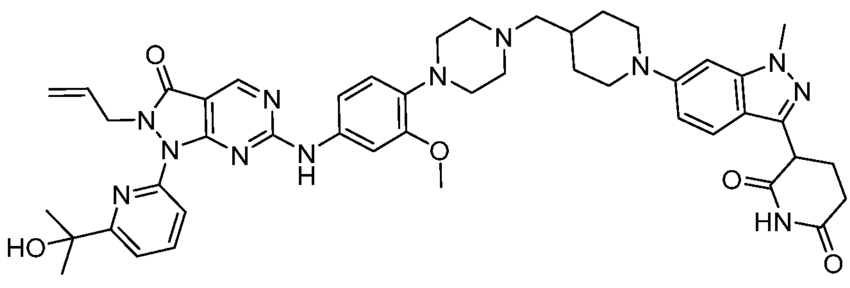

,

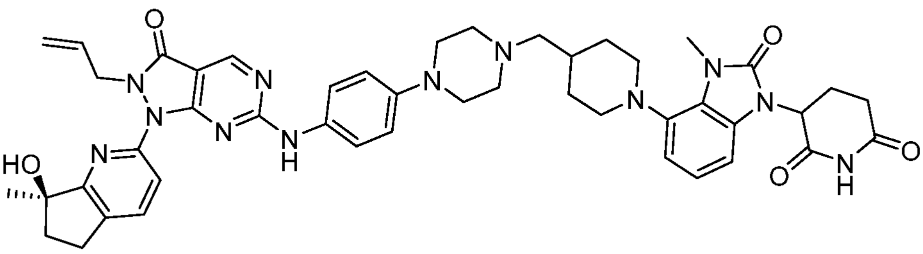

,

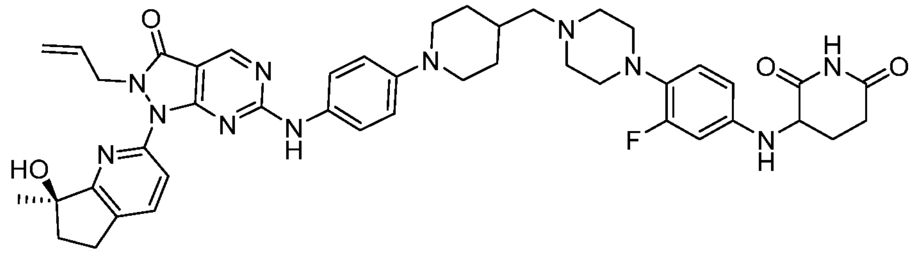

,

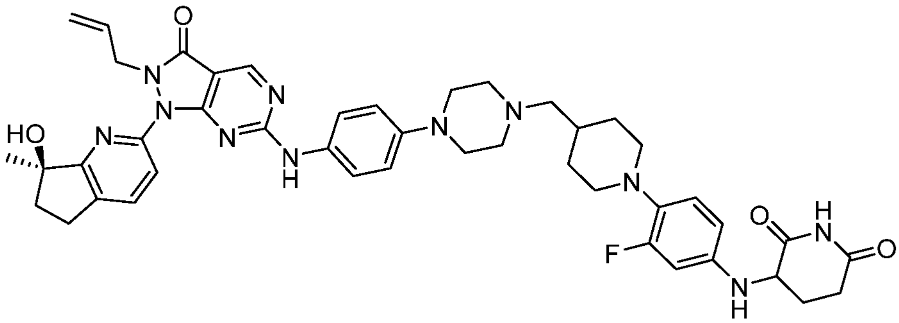

,

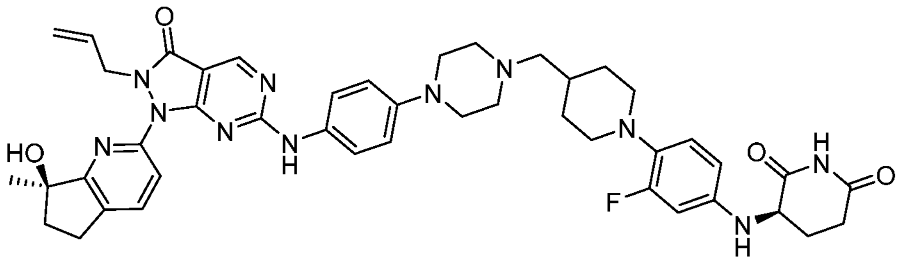

,

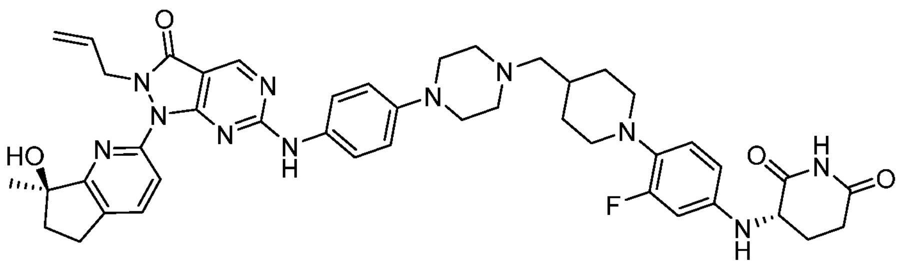

,

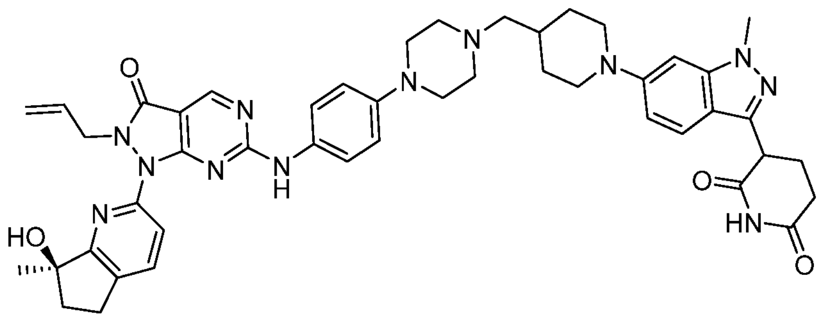

,

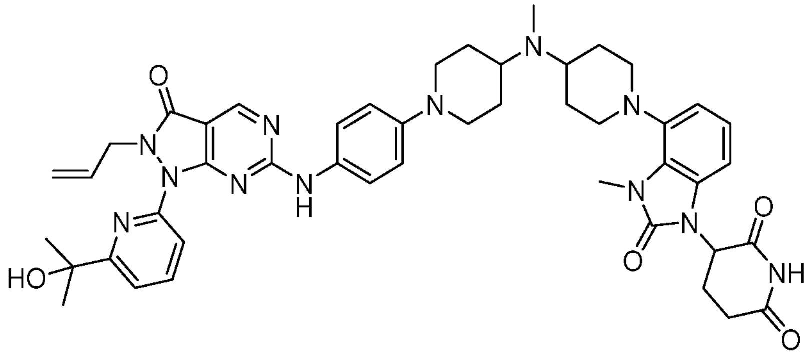

,

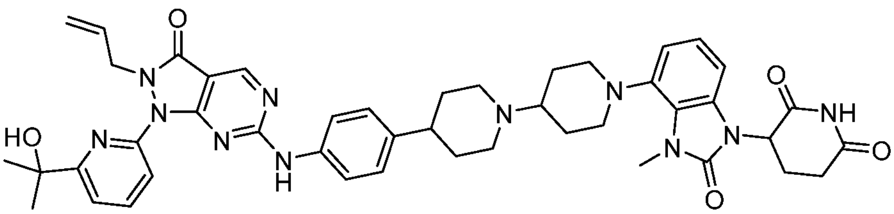

,

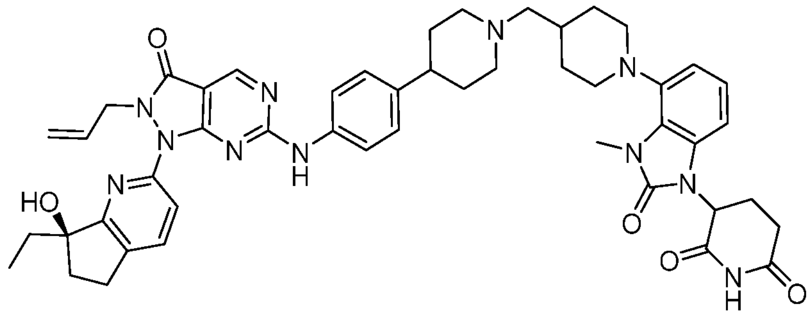

,

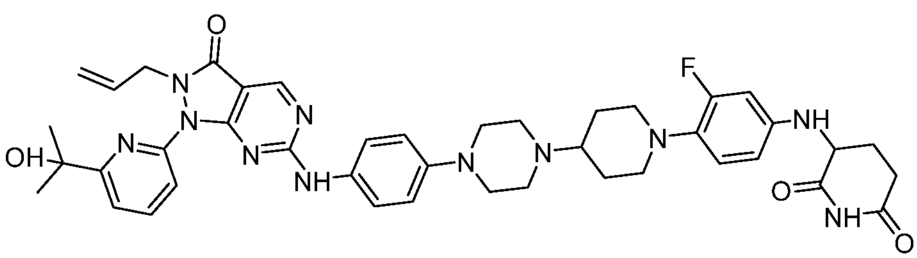

,

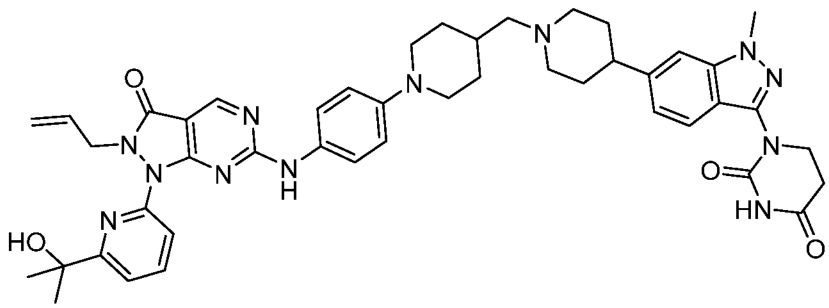

,

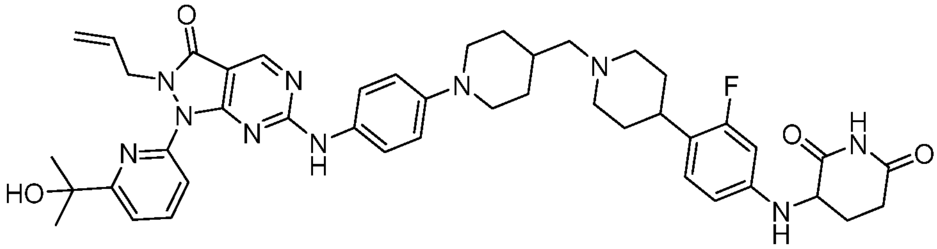

,

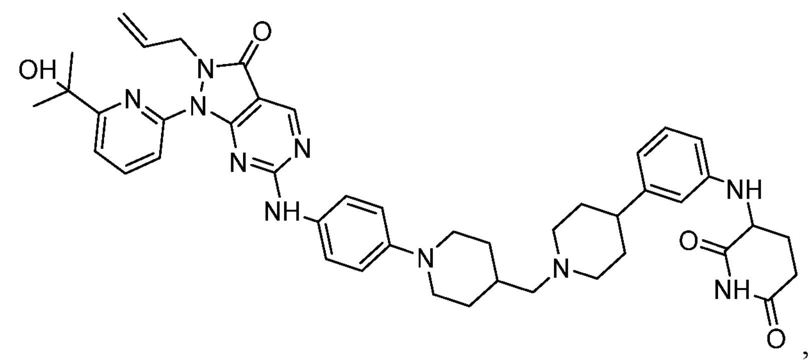

,

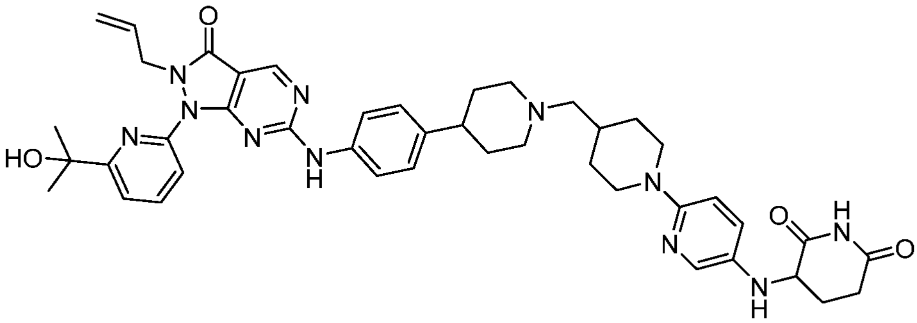

,

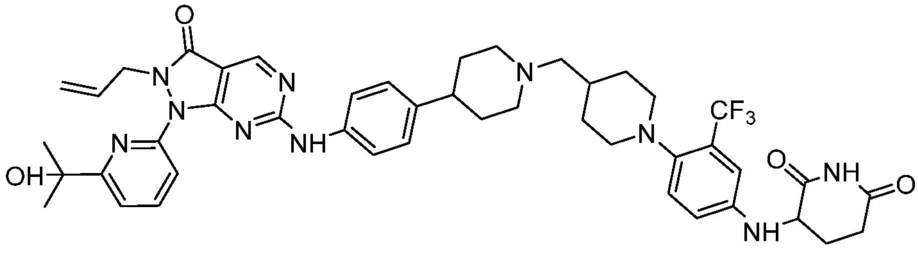

,

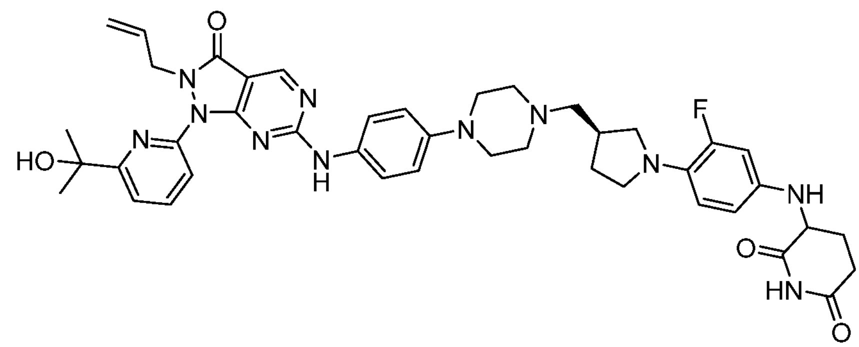

,

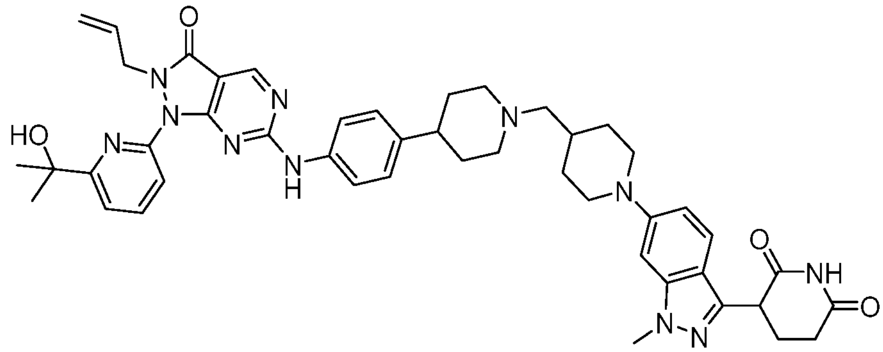

,

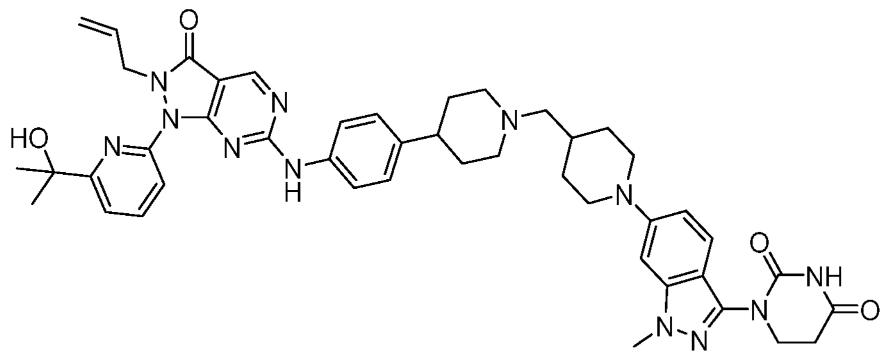

,

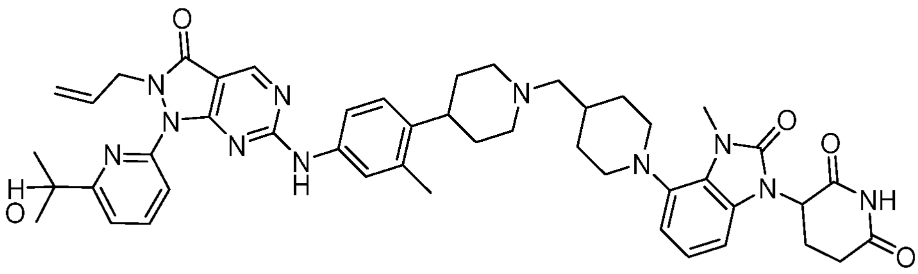

,

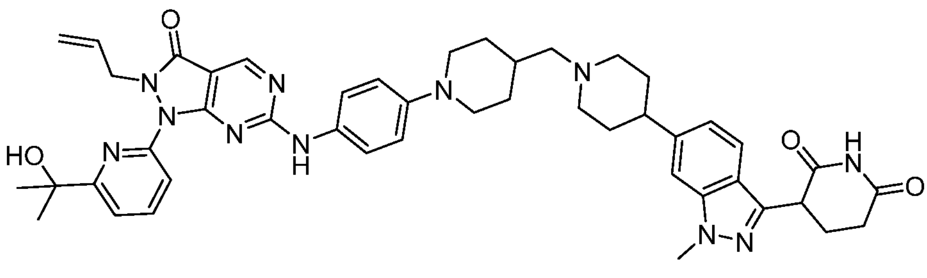

,

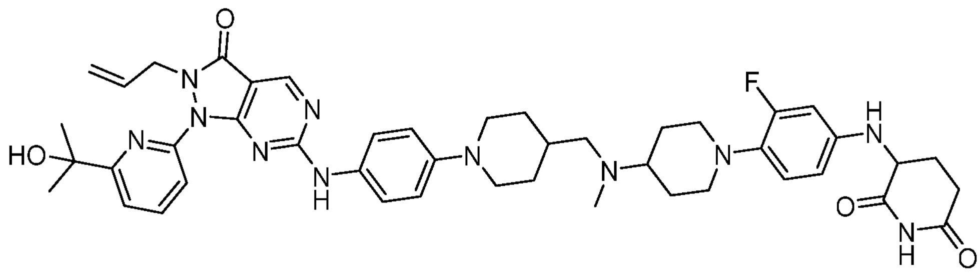

,

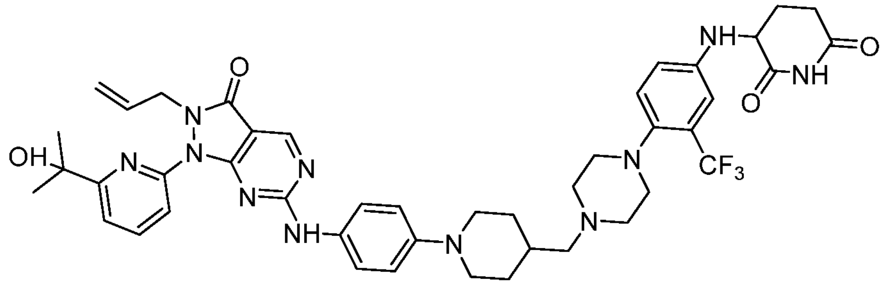

,

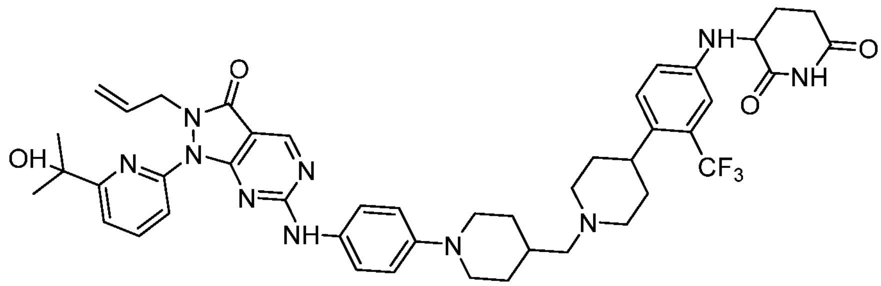

,

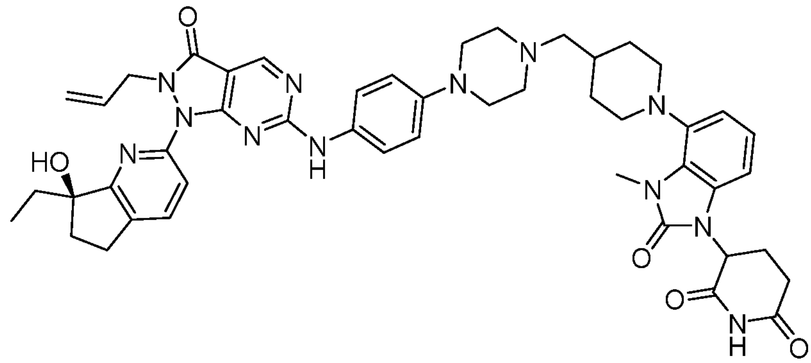

,

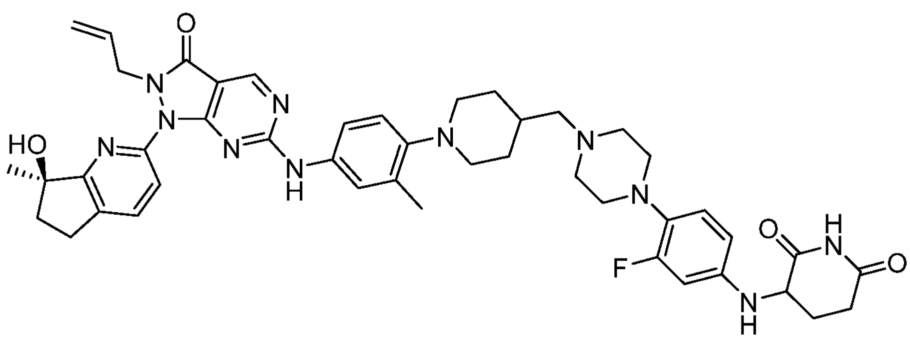

,

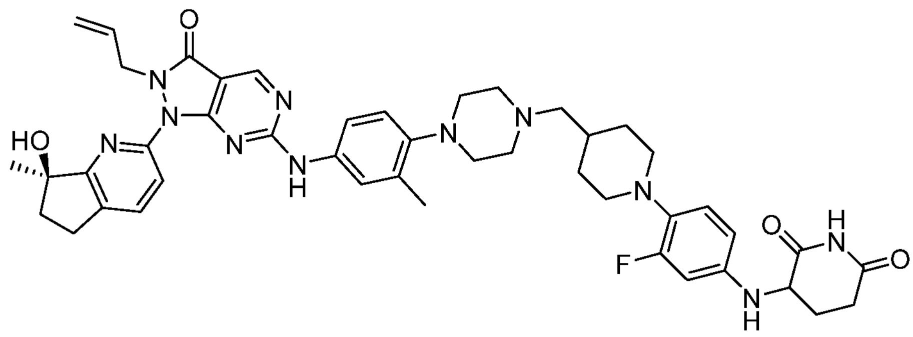

,

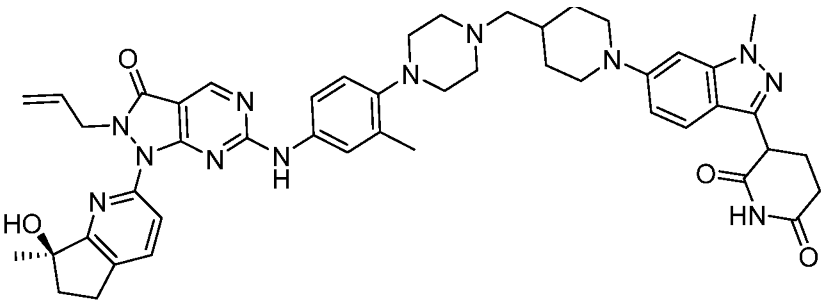

,

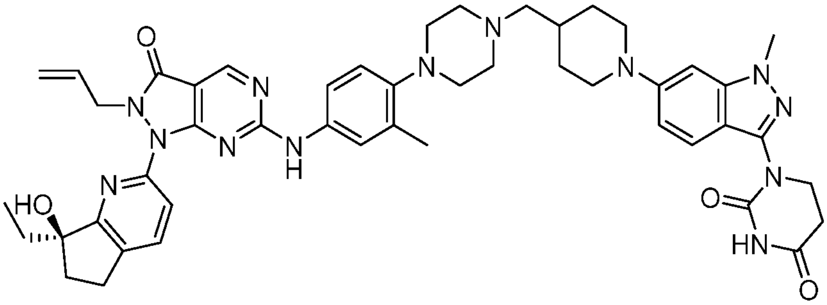

,

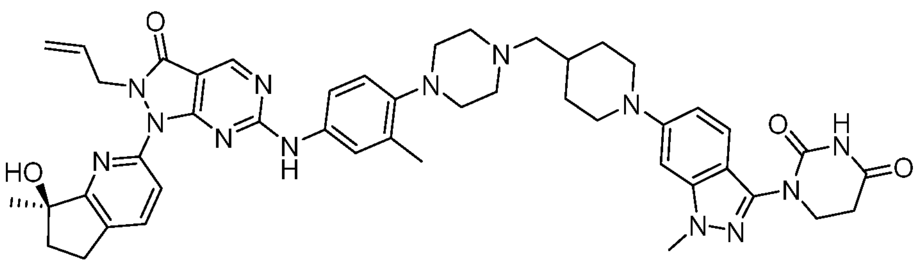

,

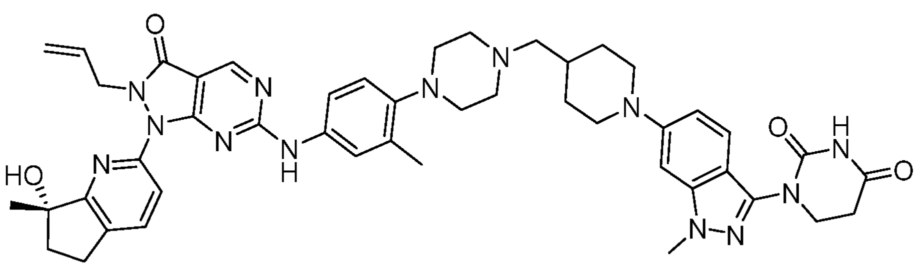

,

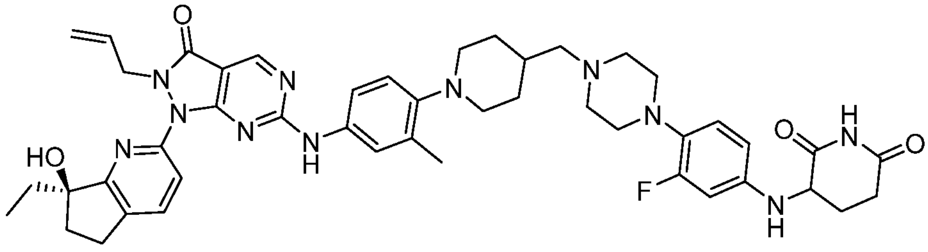

,

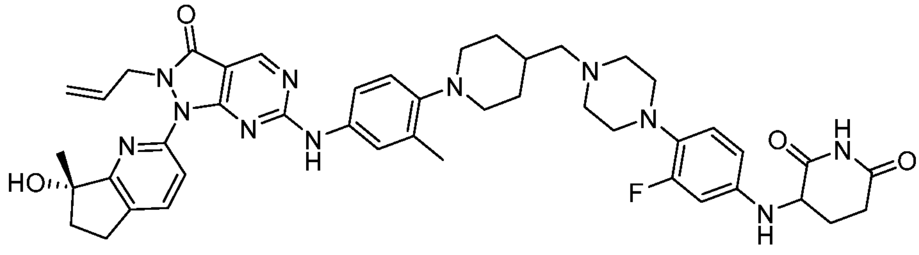

,

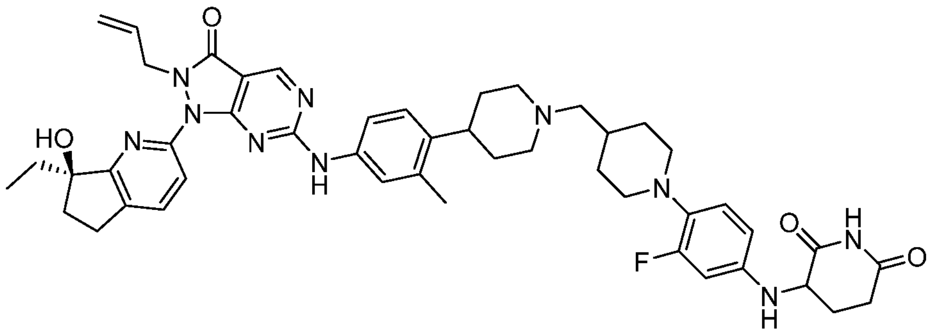

,

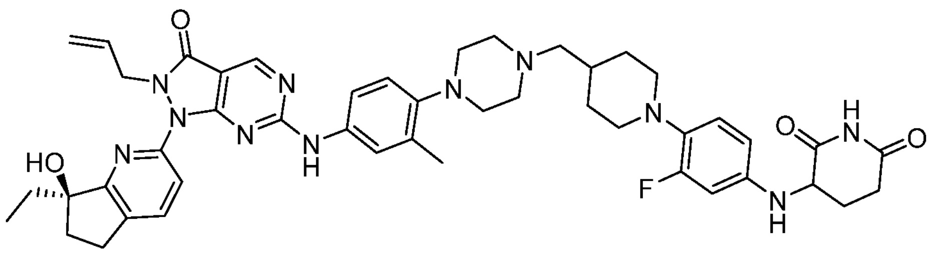

,

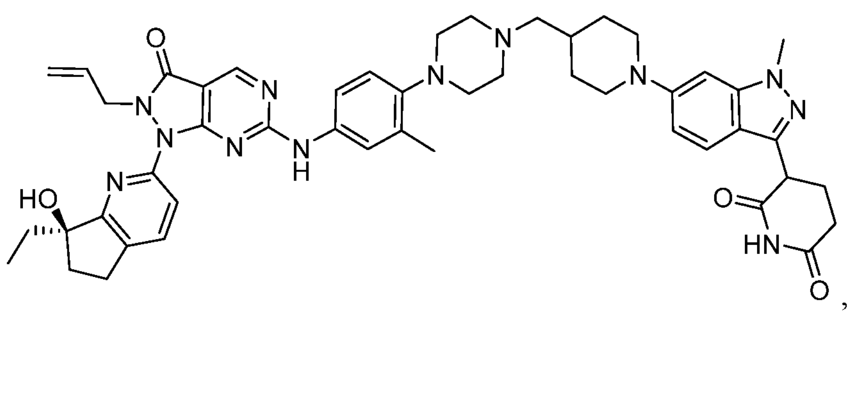

,

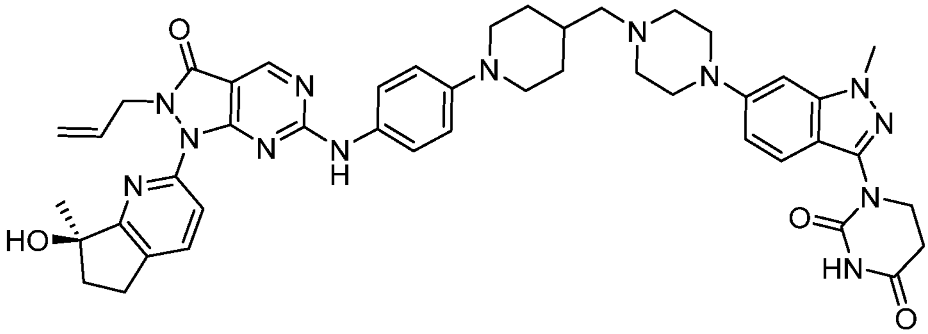

,

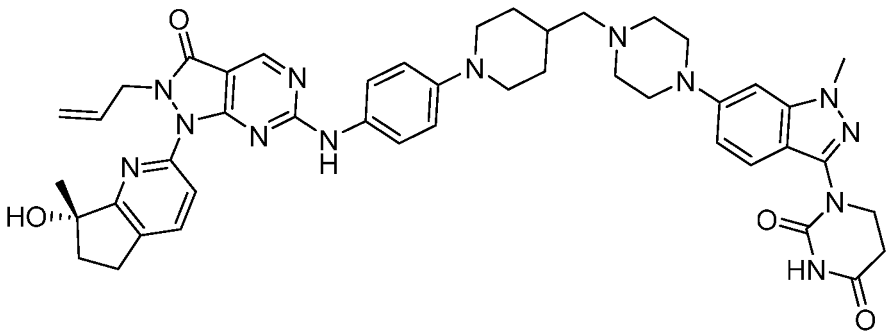

,

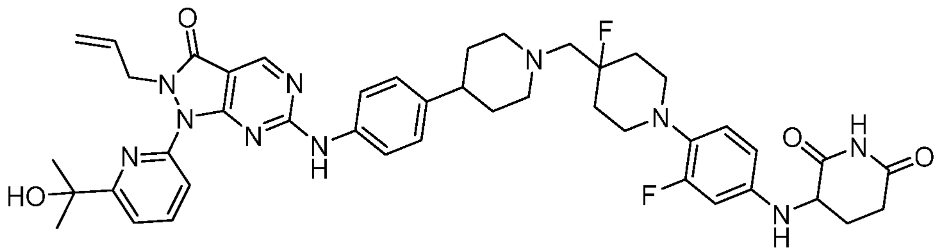

,

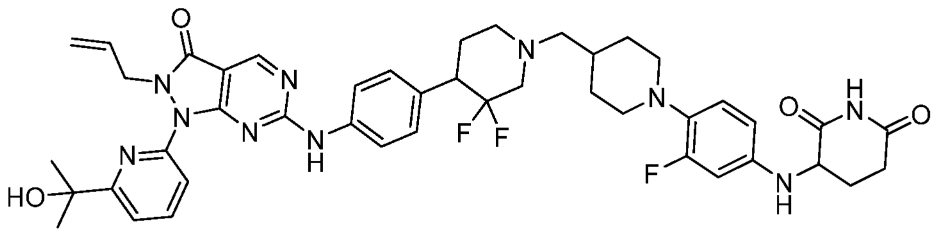

,

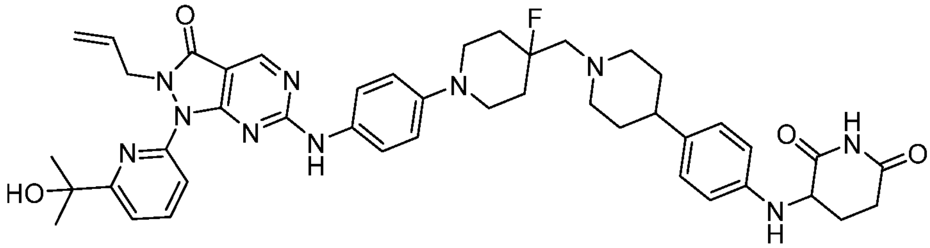

,

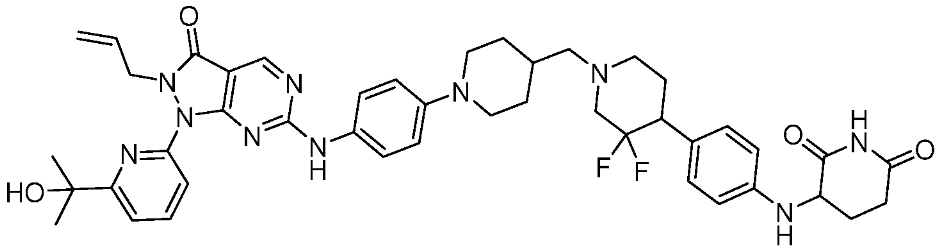
,

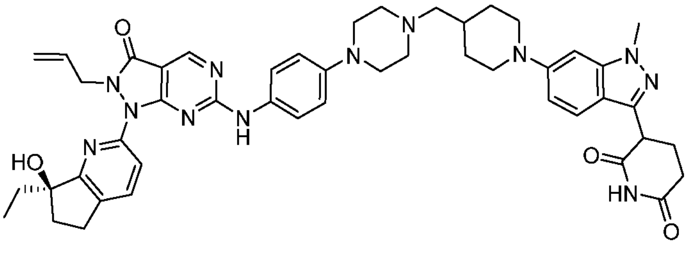
,

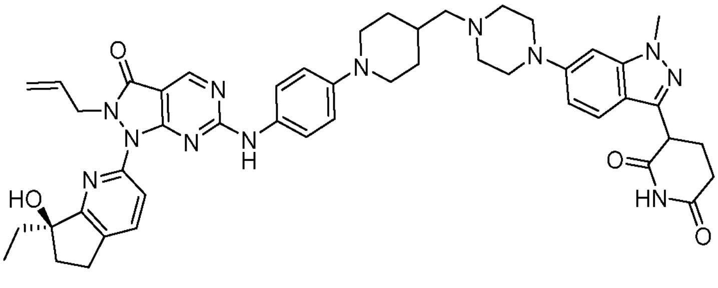
,

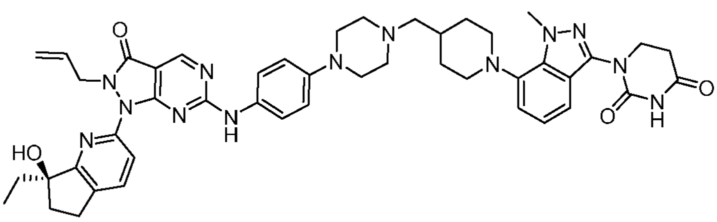
,

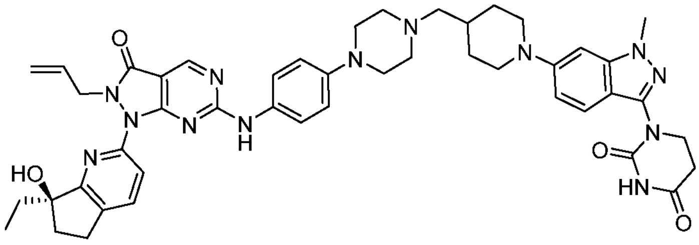
,

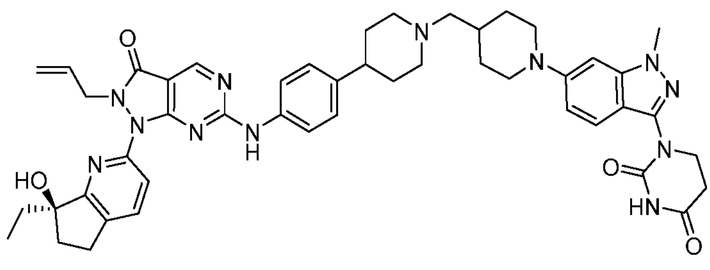

,

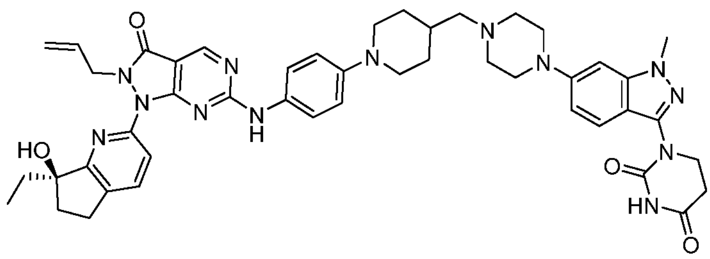

,

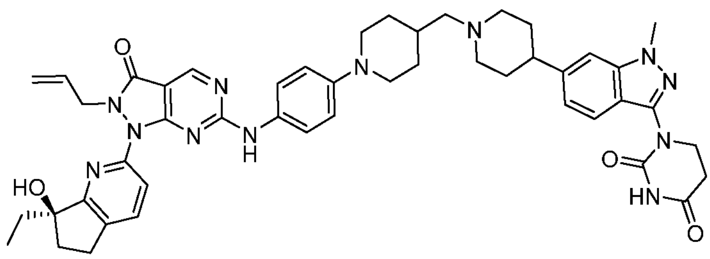

,

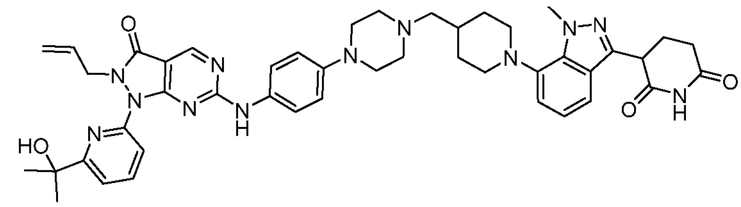

,

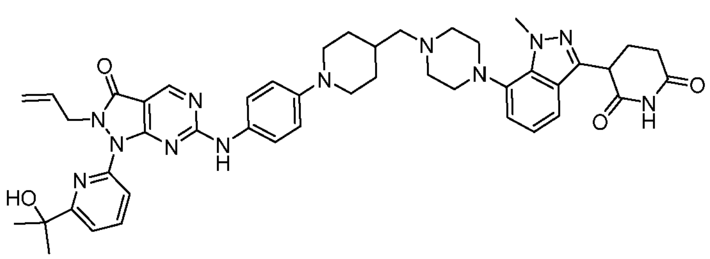

,

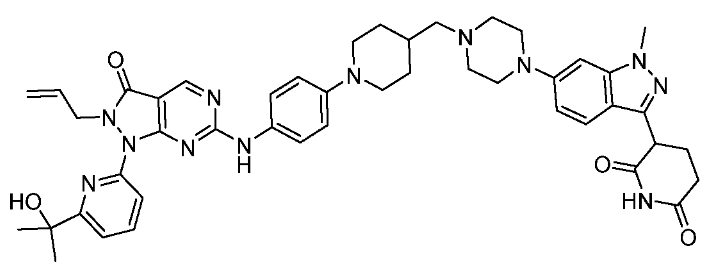

,

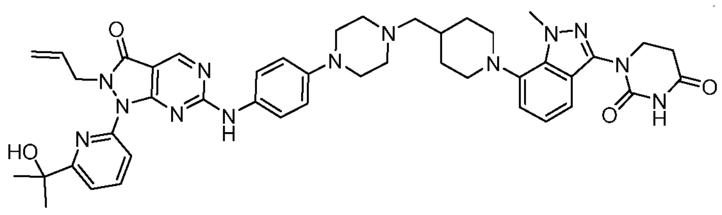

,

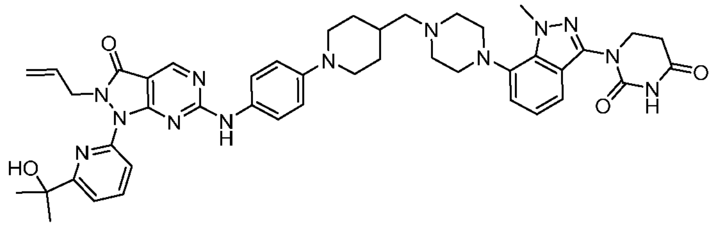

,

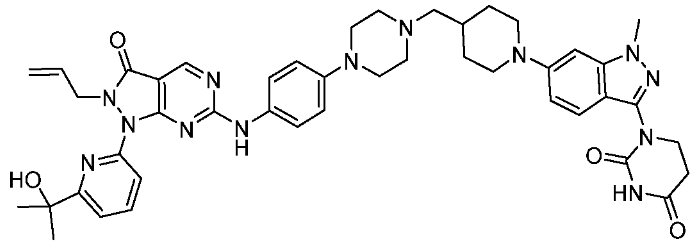

,

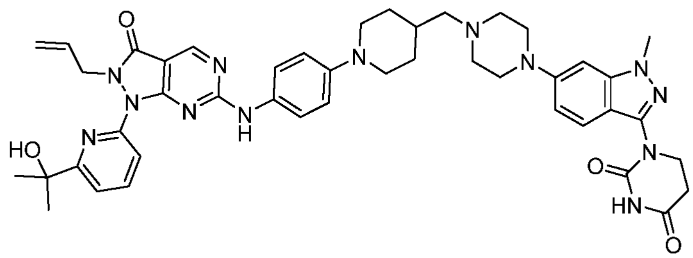

,

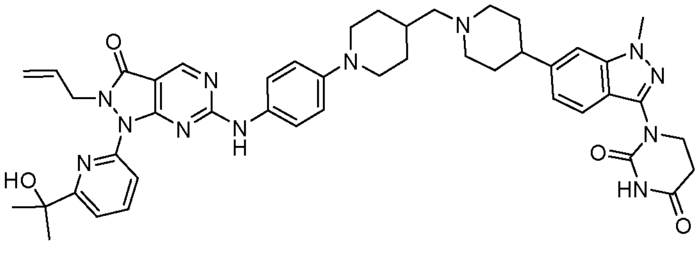

,

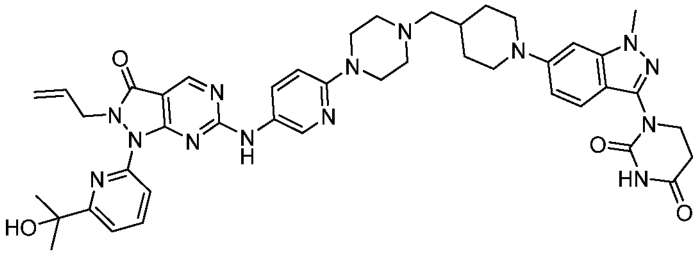

,

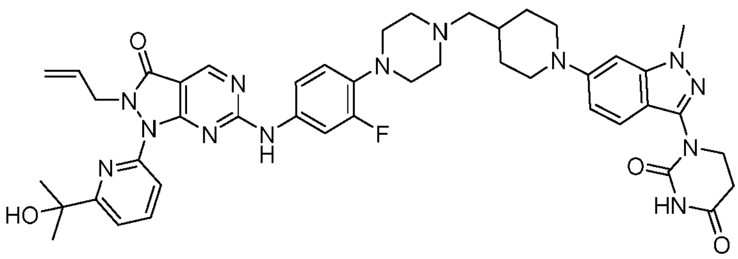

,

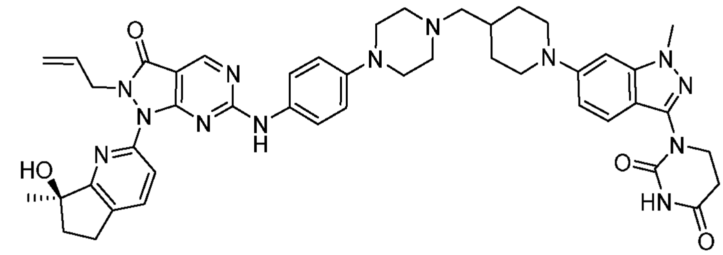

,

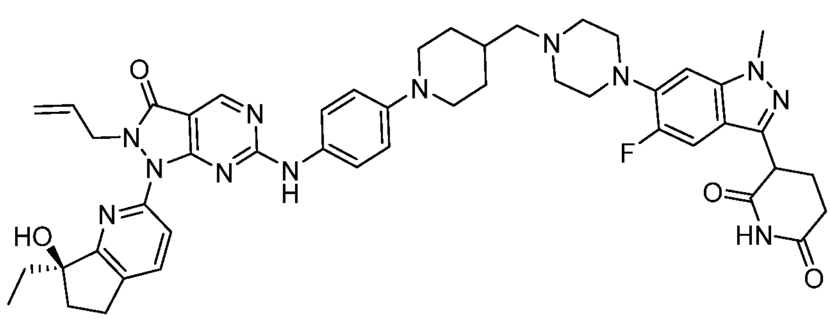

,

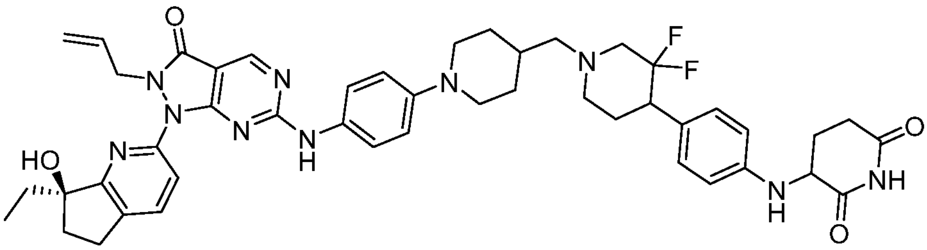

,

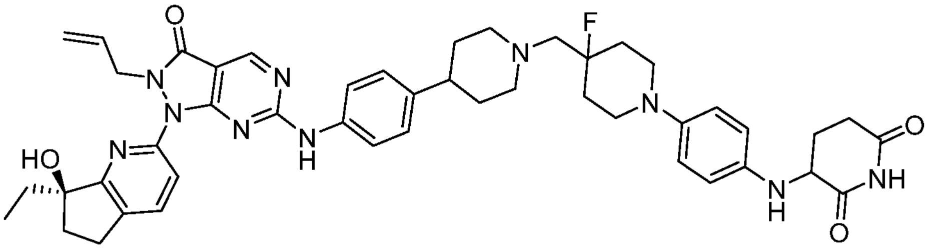

,

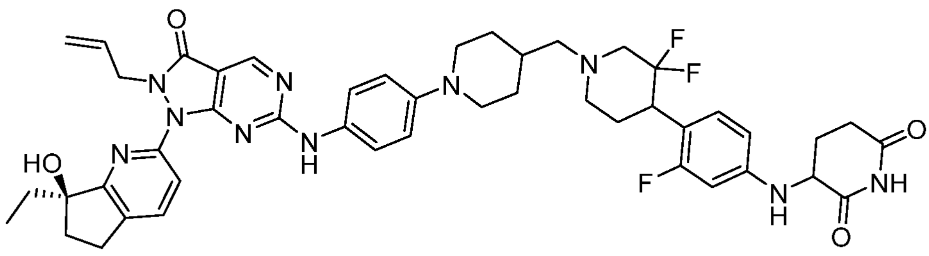

,

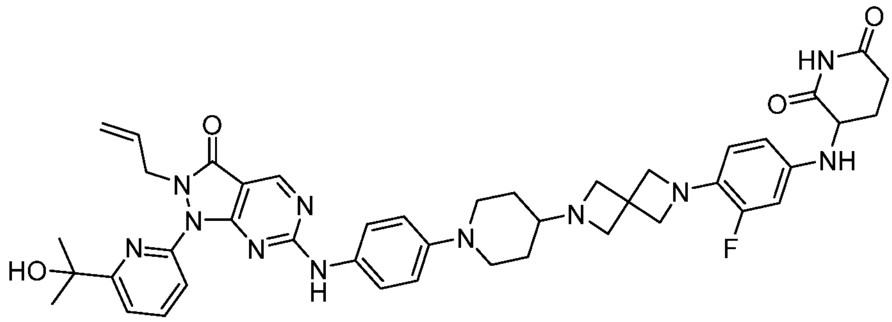

,

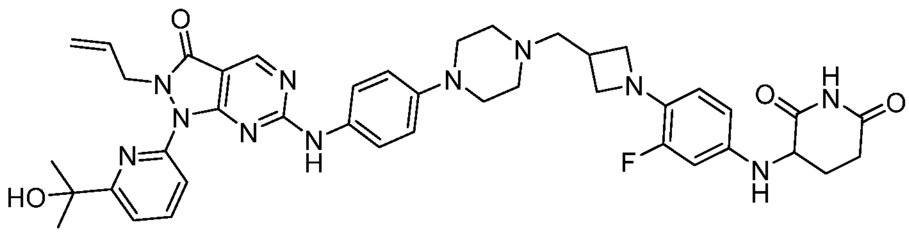

,

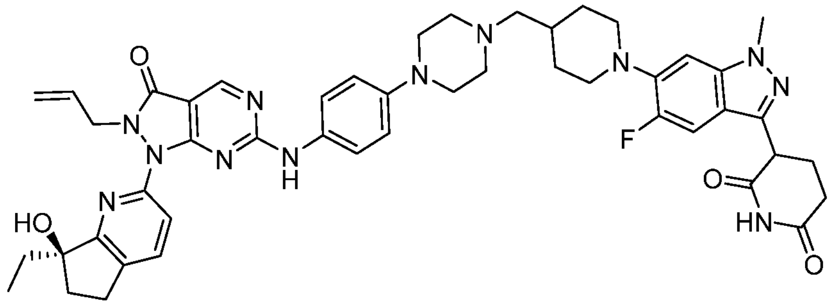

,

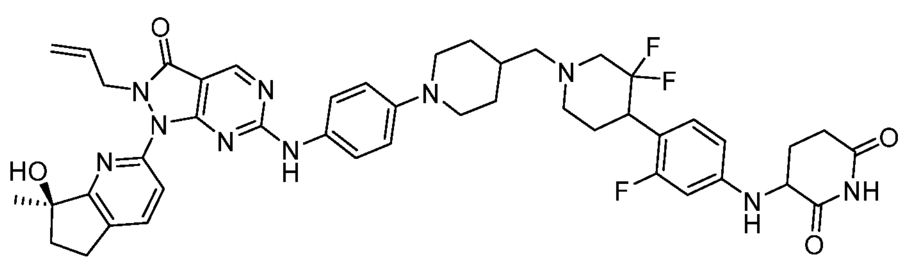

,

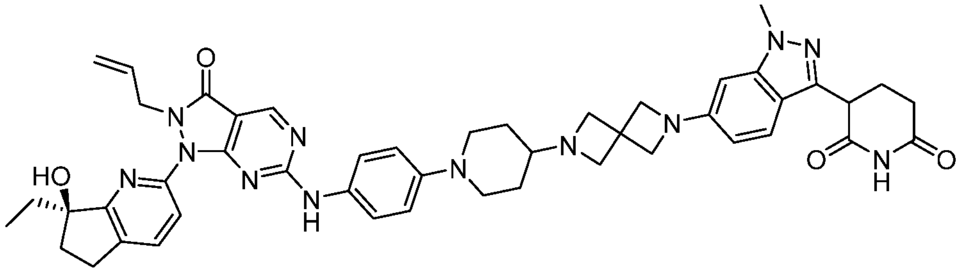

,

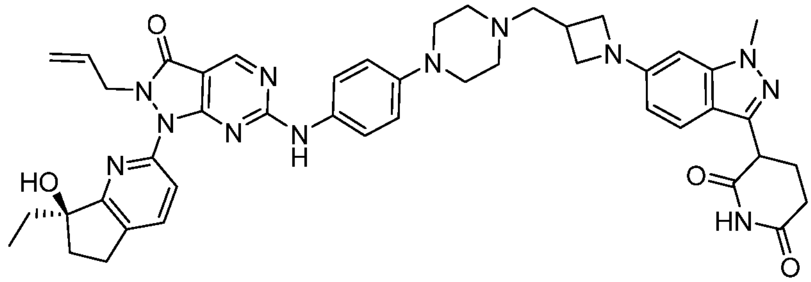

,

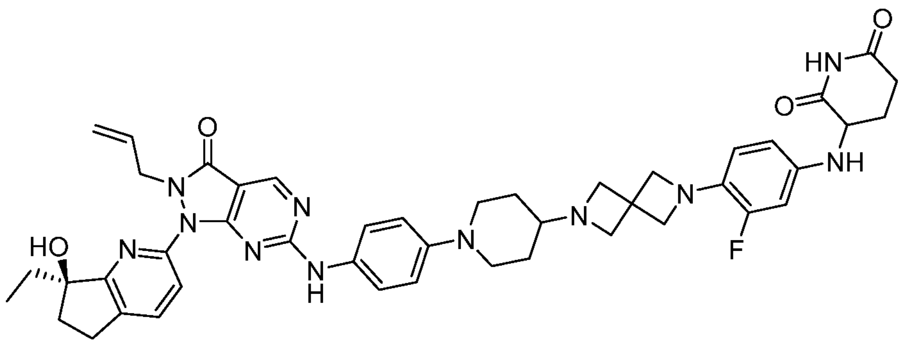

,

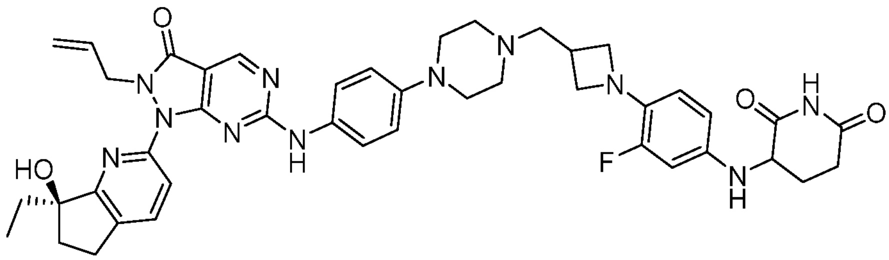

,

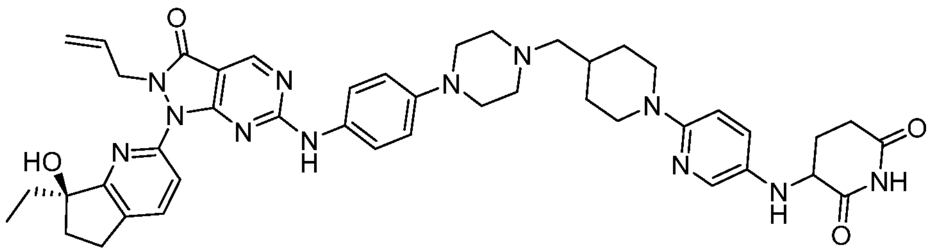

,

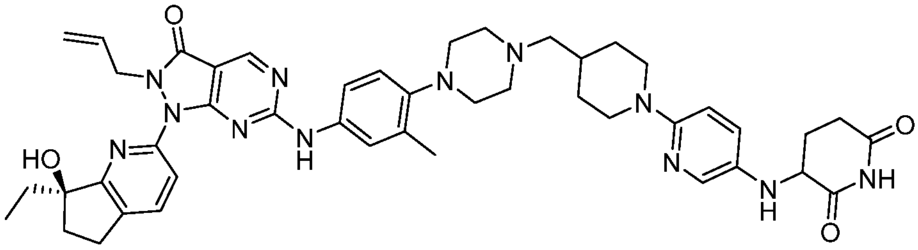
,

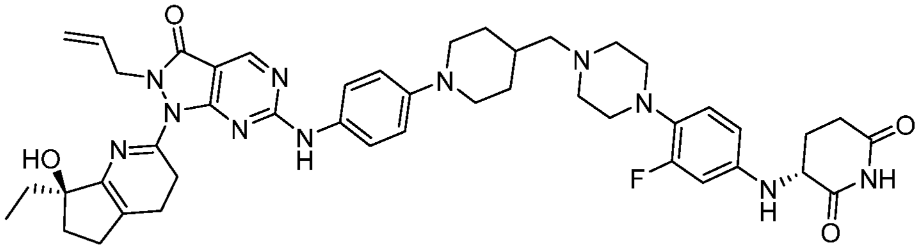
,

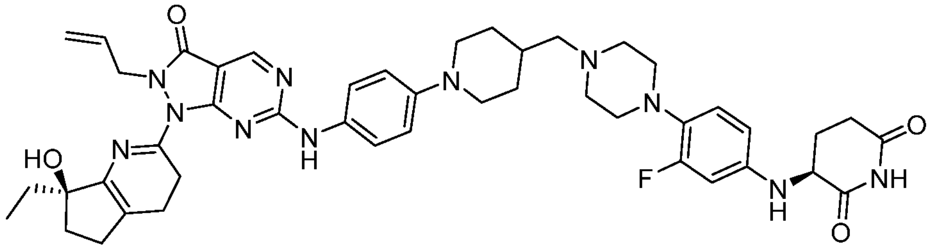
,

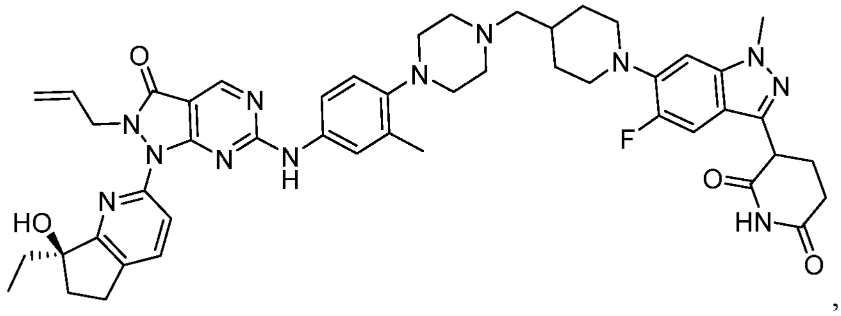
,

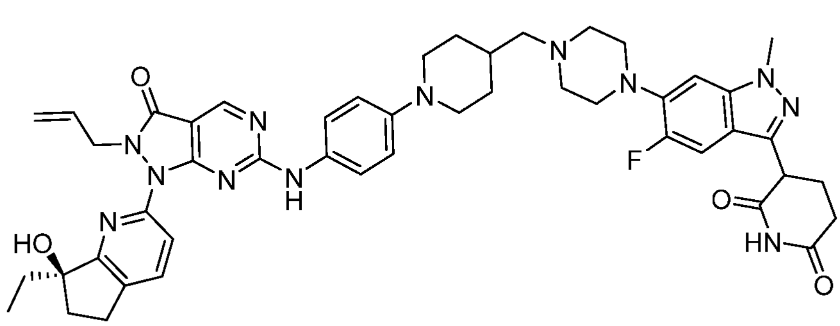
,

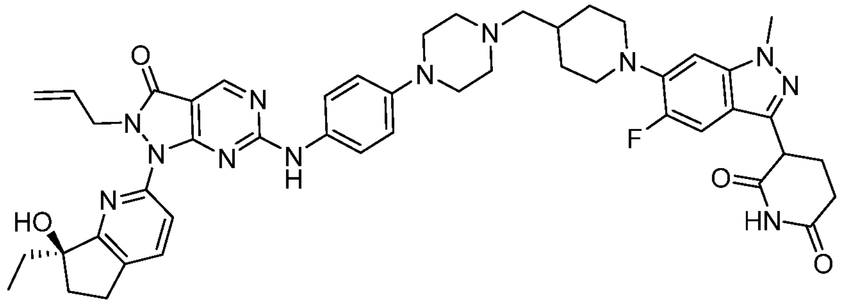

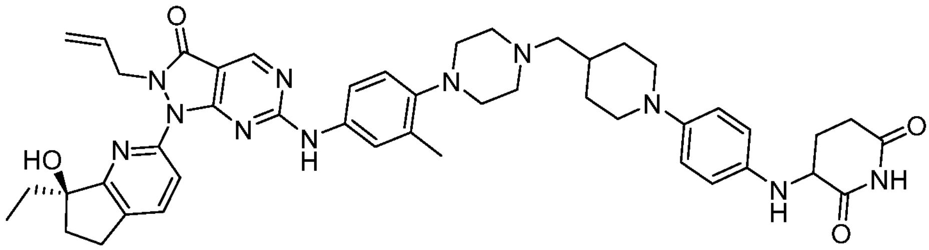

,

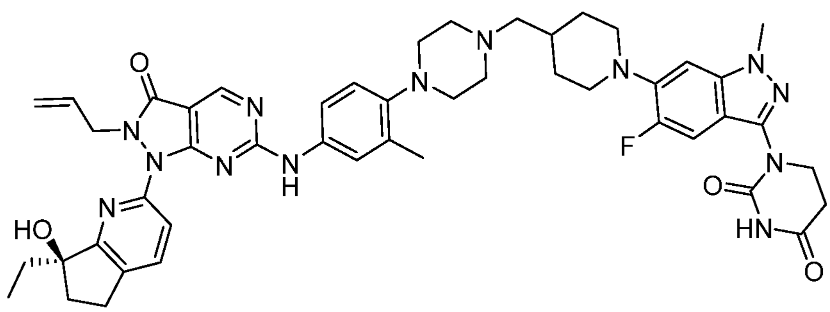

,

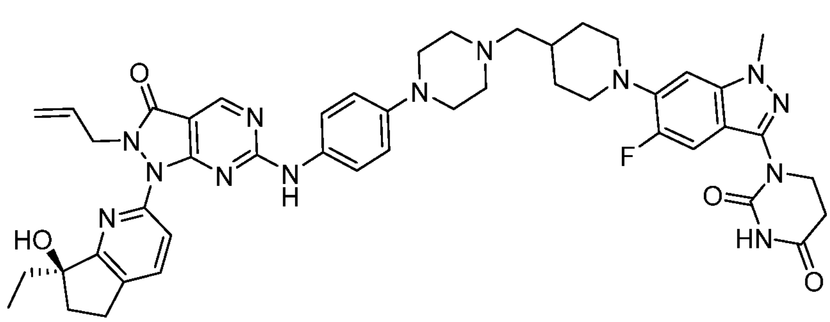

,

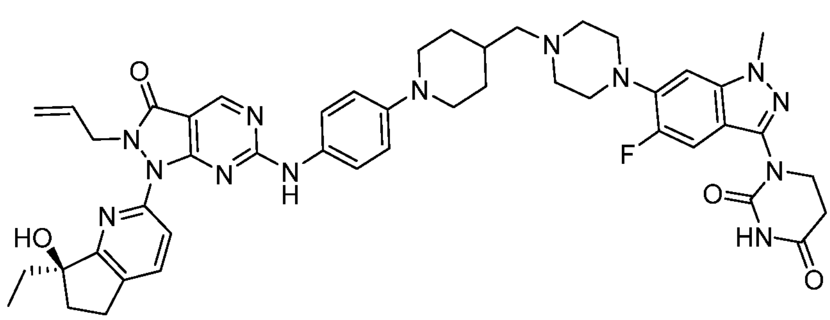

,

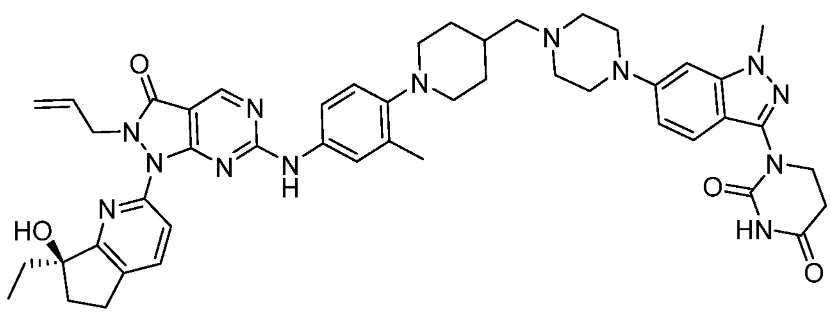

,

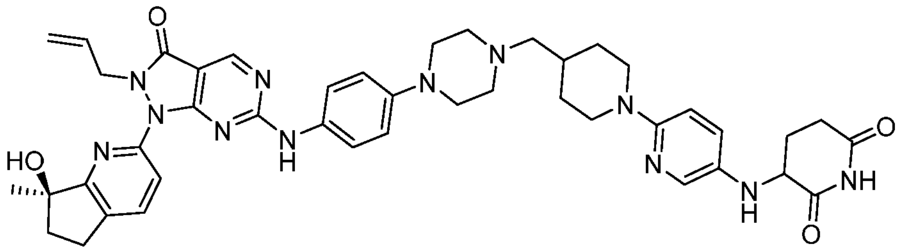

,

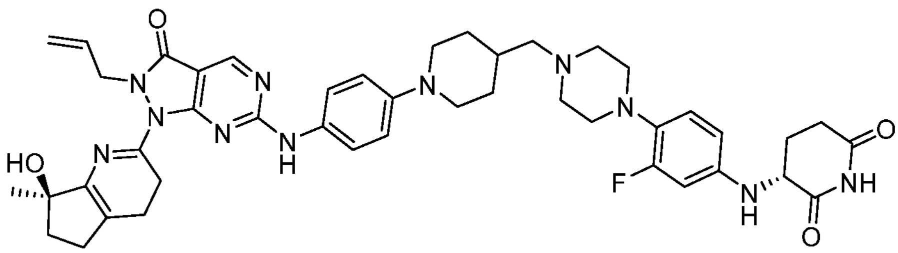

,

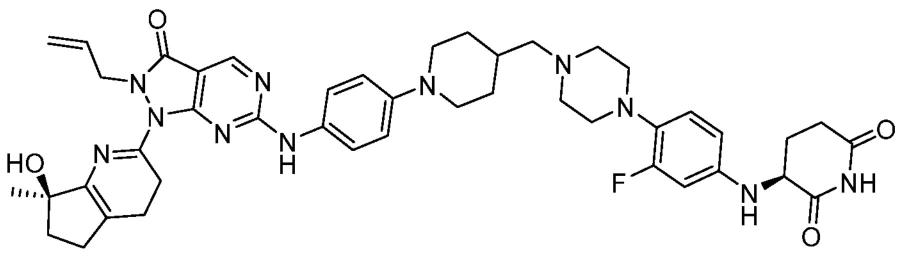

,

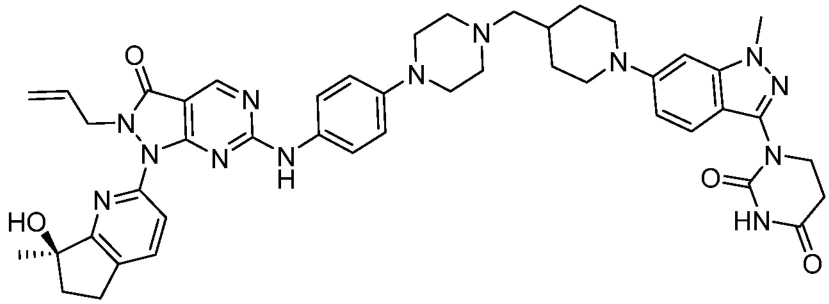

,

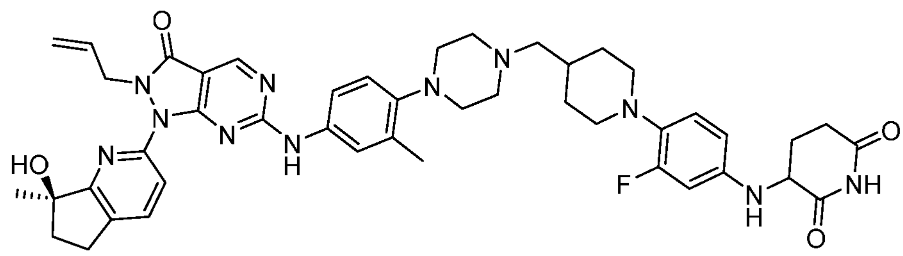

,

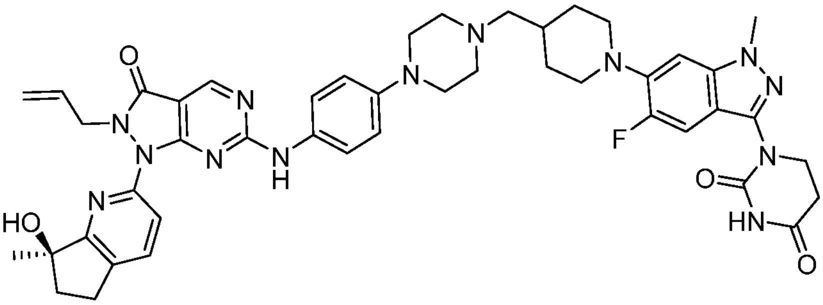

,

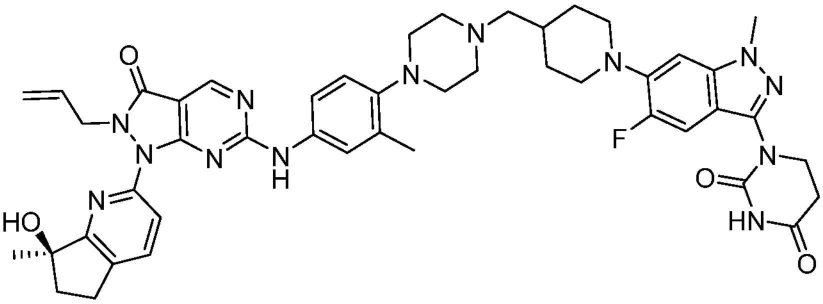

,

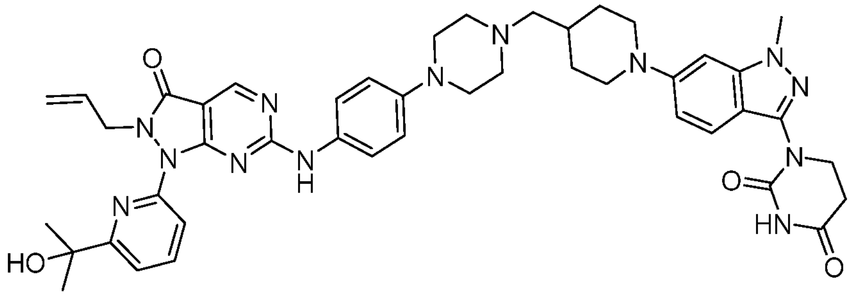

,

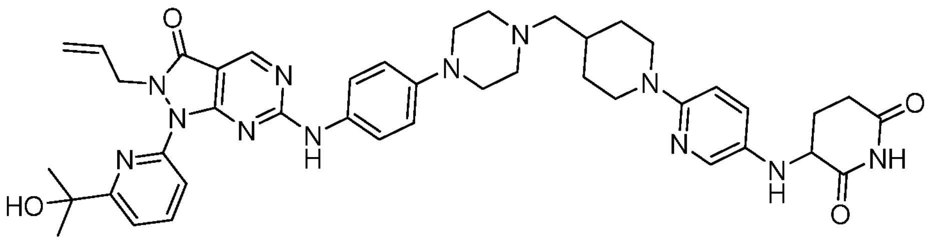

,

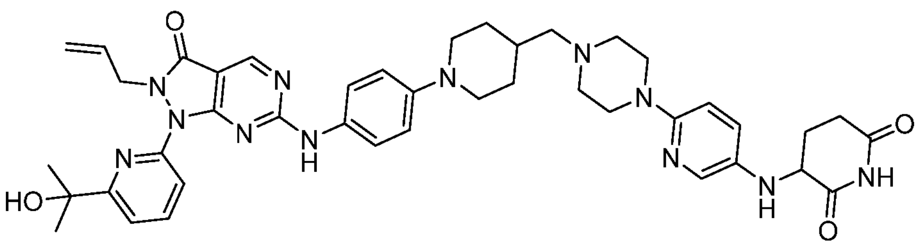

,

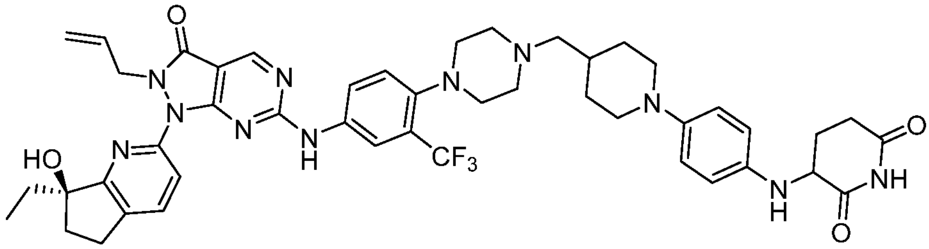

,

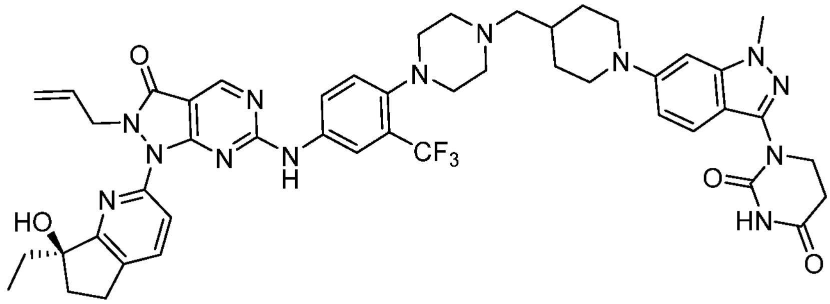

,

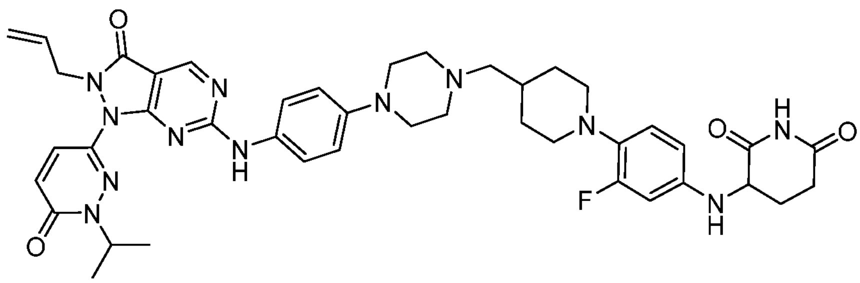

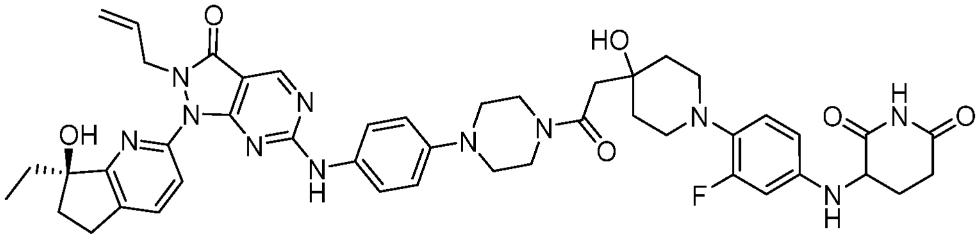

,

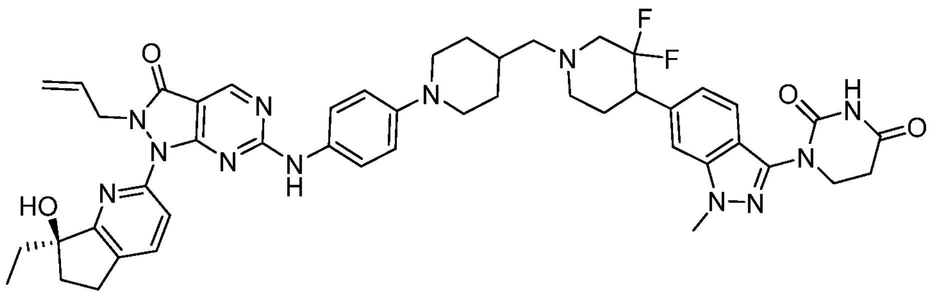

,

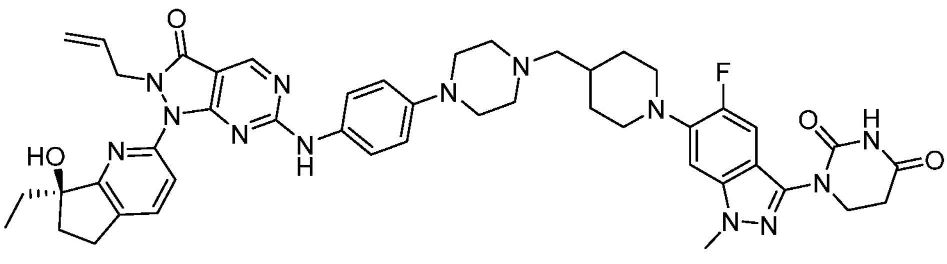

,

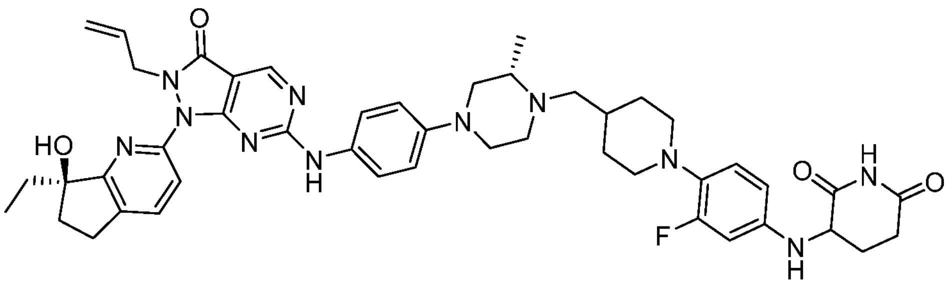

,

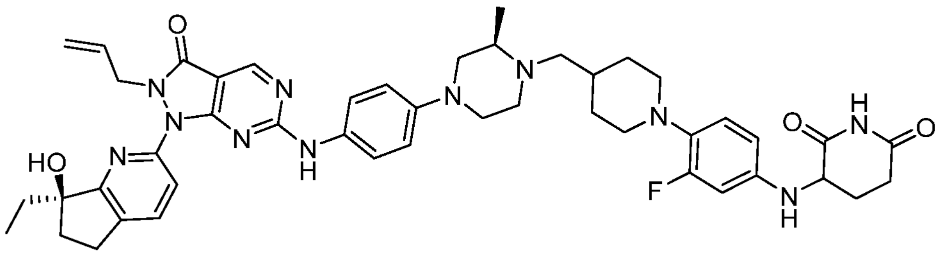

,

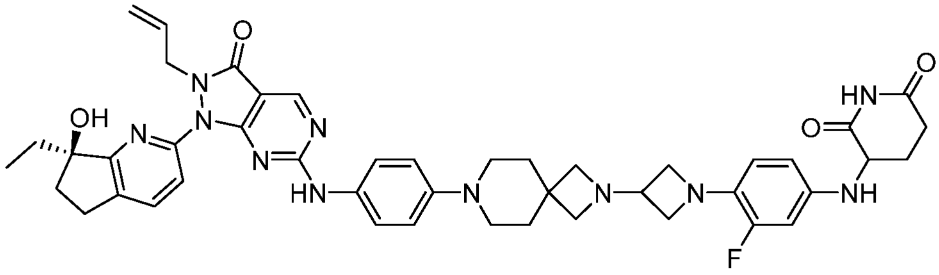

,

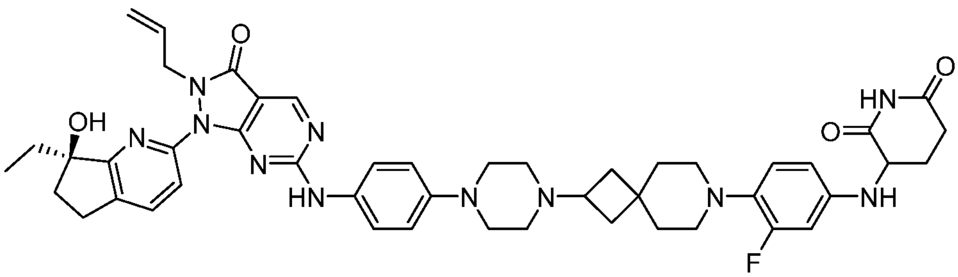

,

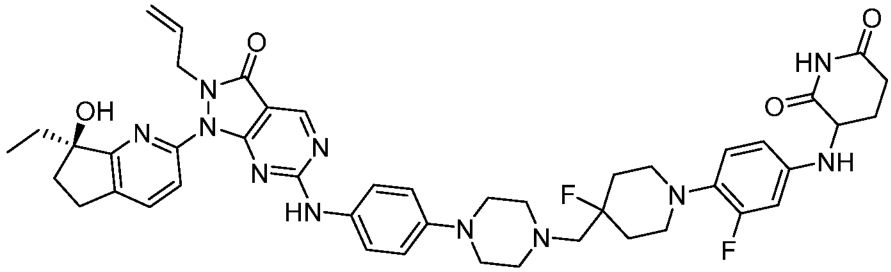

,

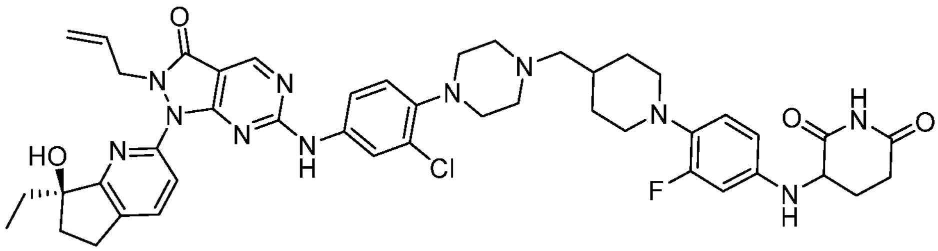

,

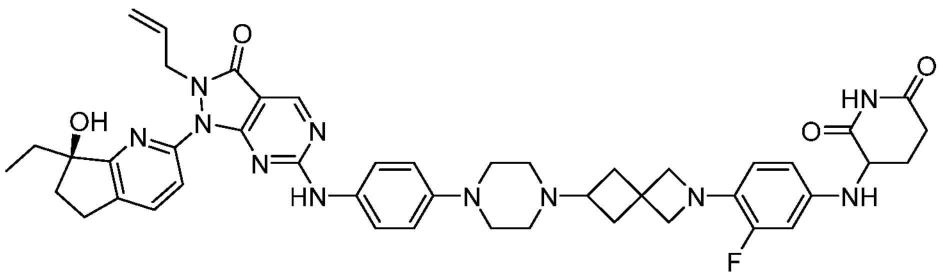

,

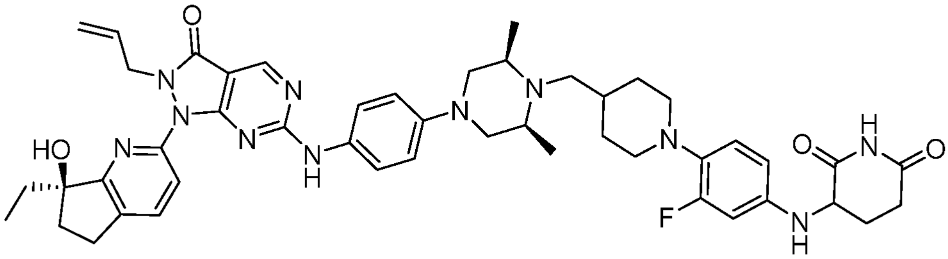

,

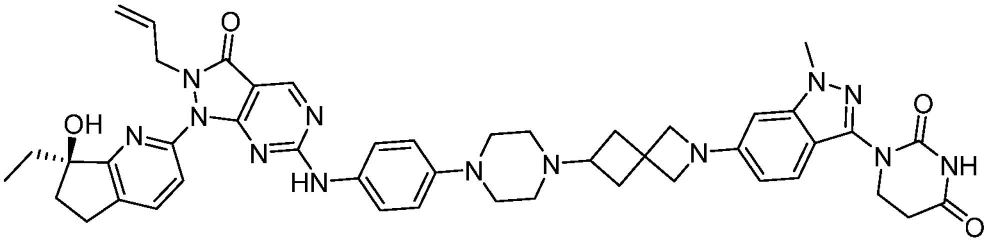,

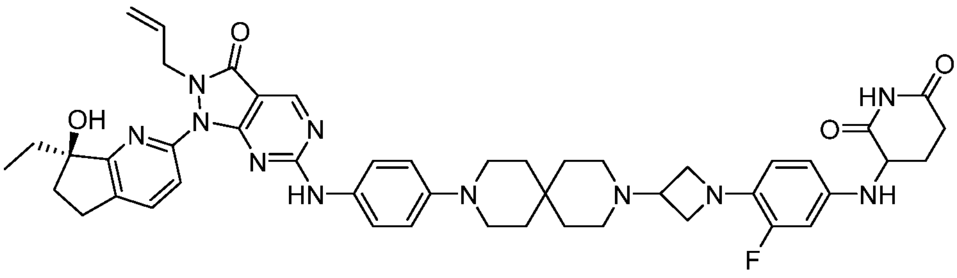,

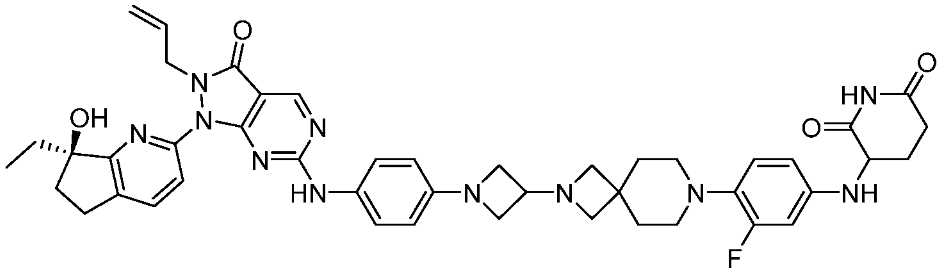,

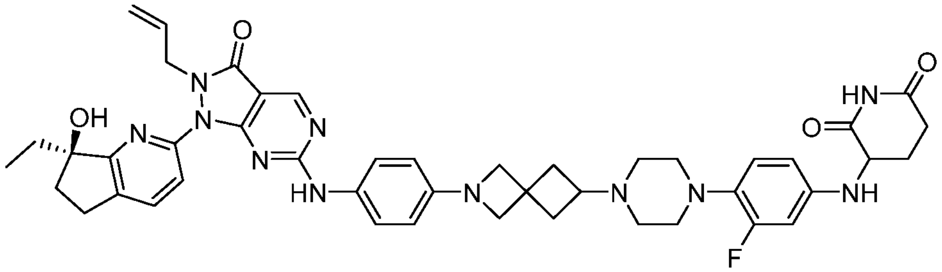,

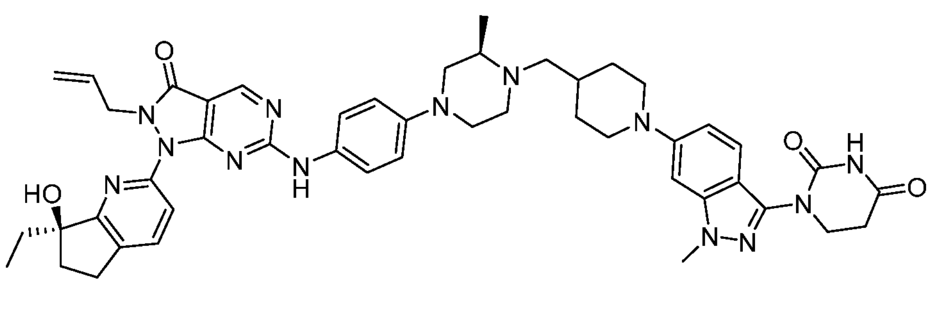,

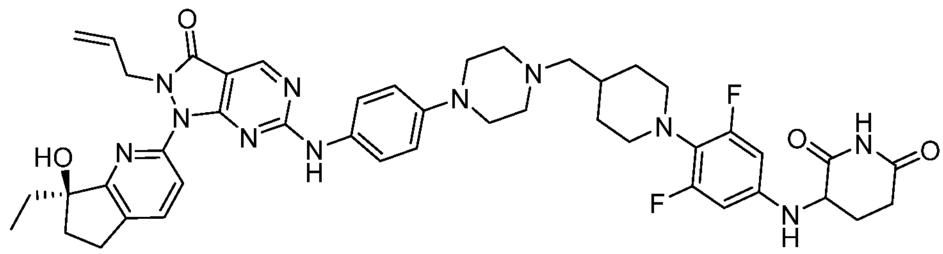

,

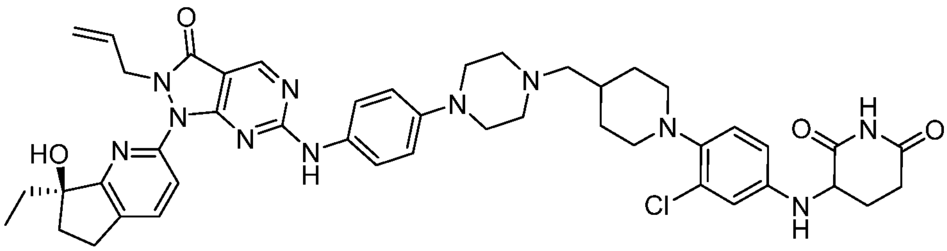

,

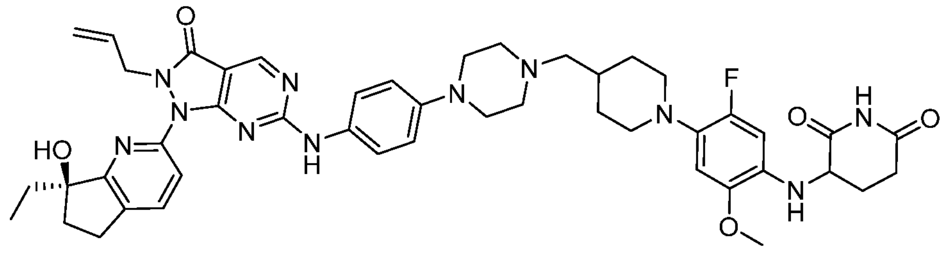

,

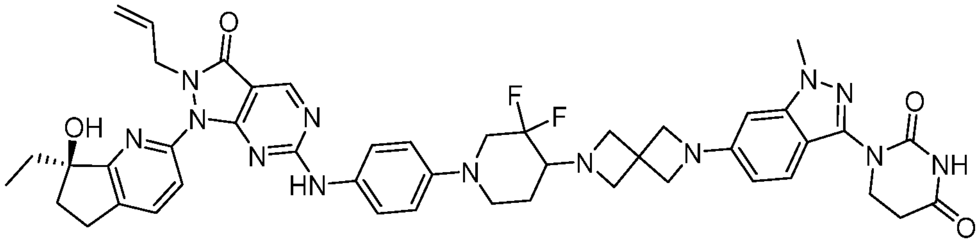

,

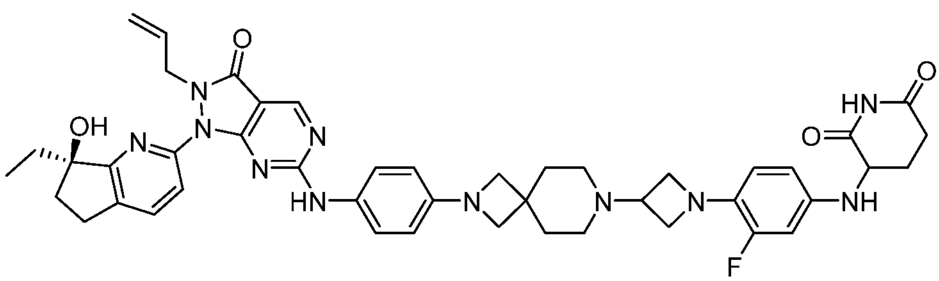

,

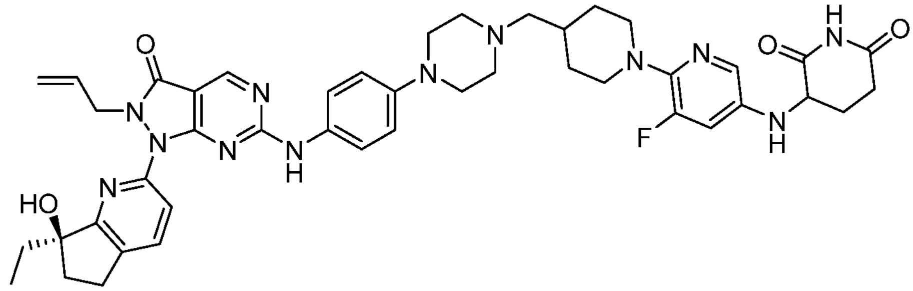

,

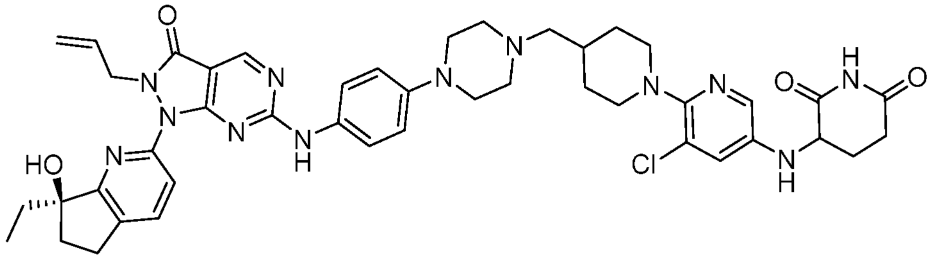

,

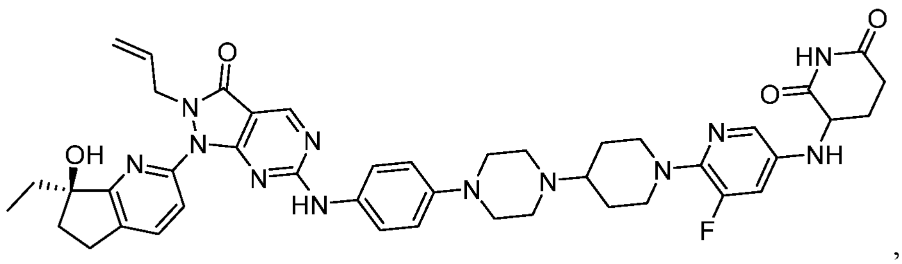

,

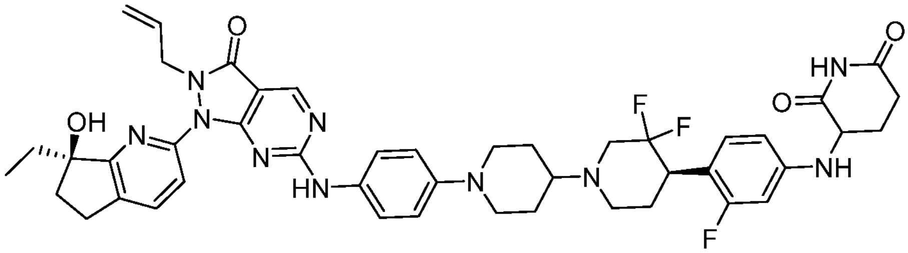

,

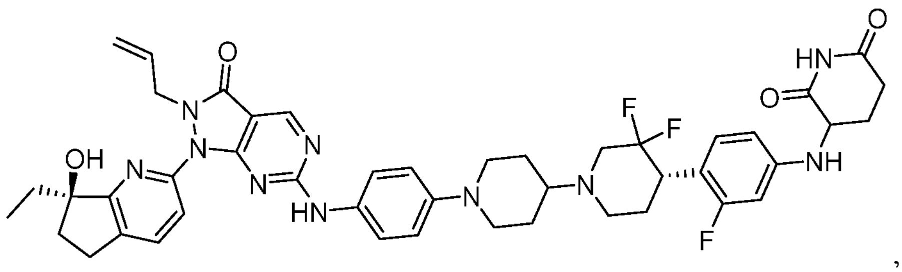

,

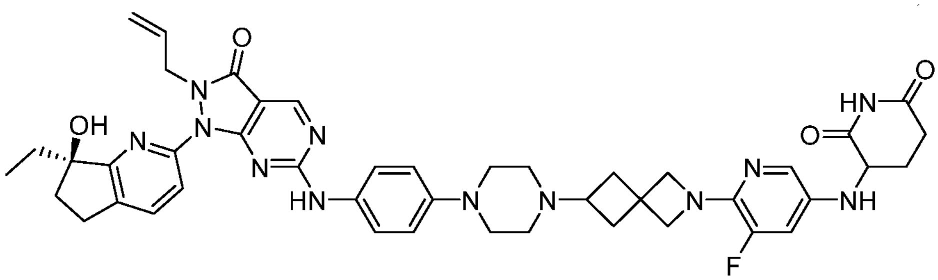

,

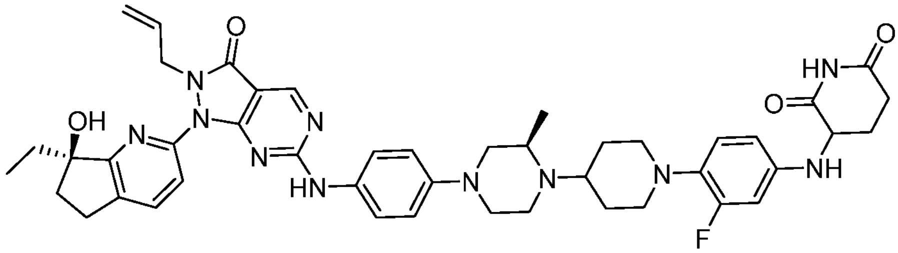

,

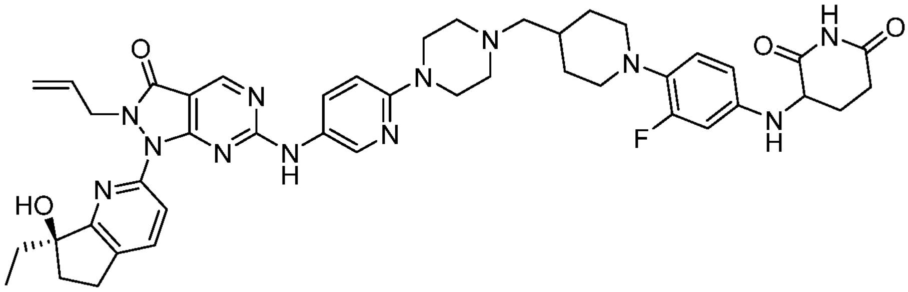

,

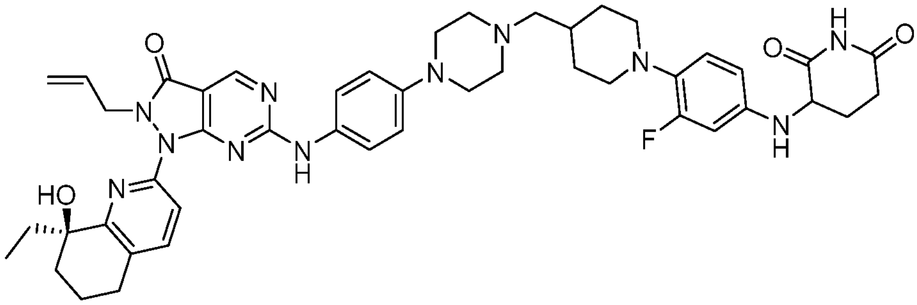

,

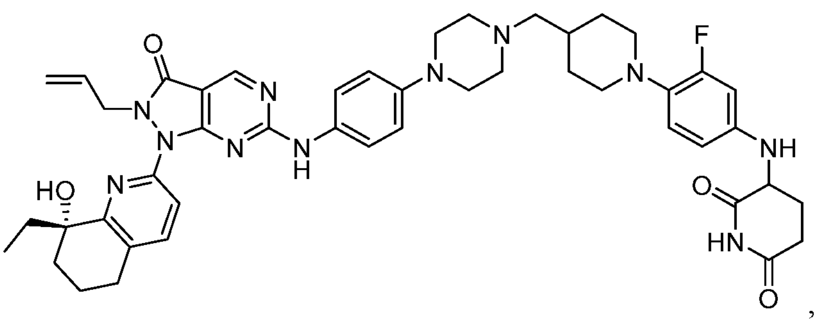

,

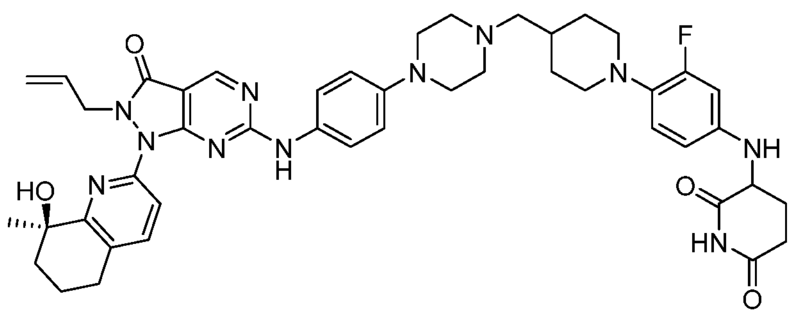

,

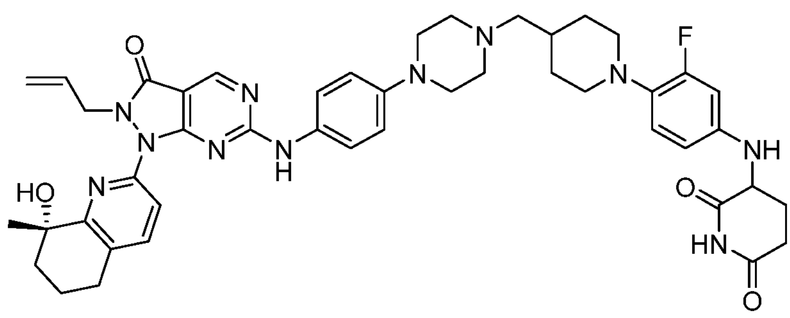

,

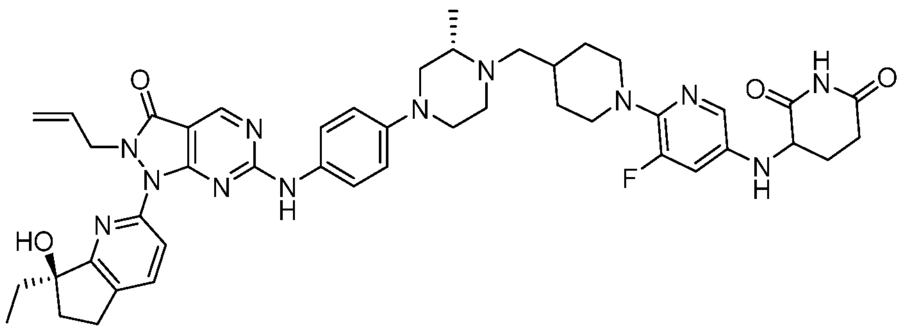

,

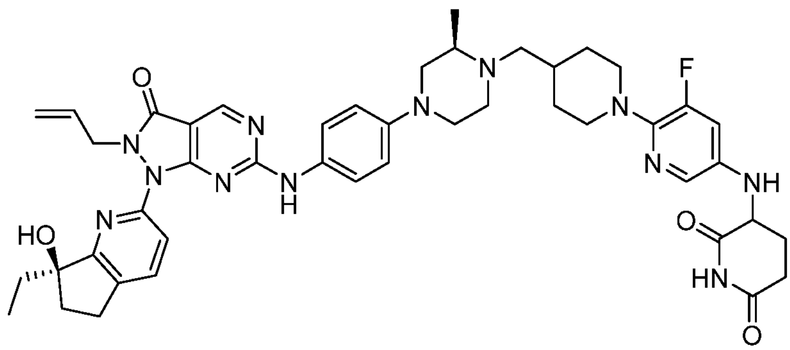

,

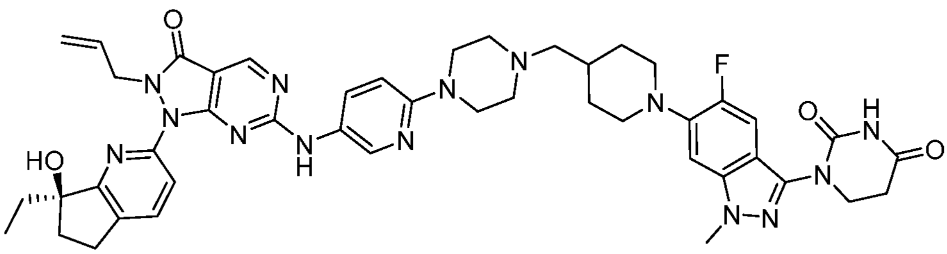

,

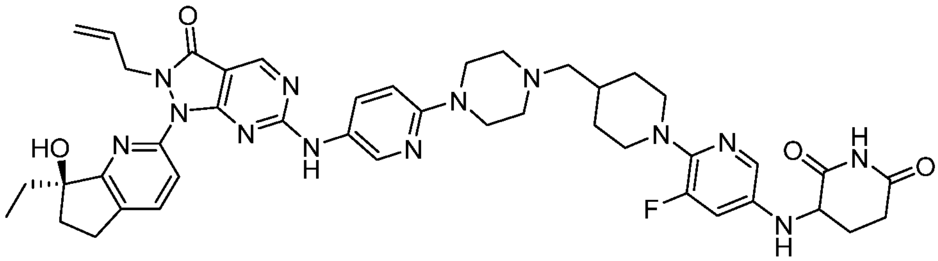

,

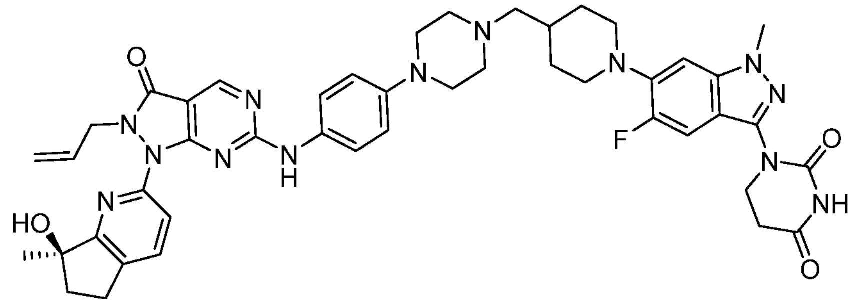

,

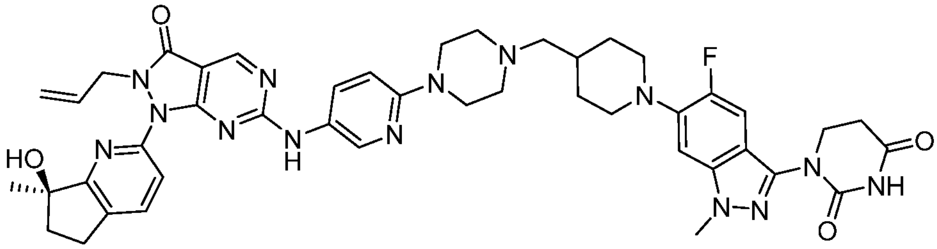

,

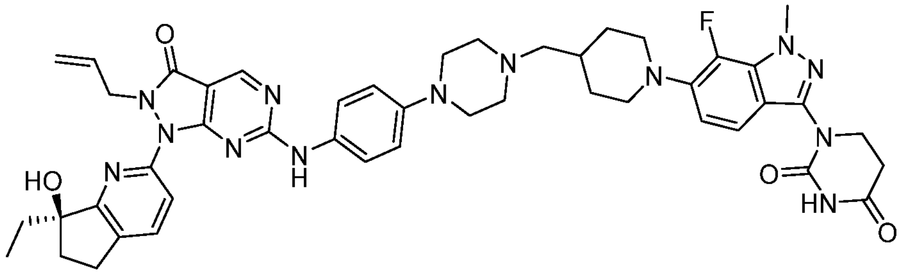

,

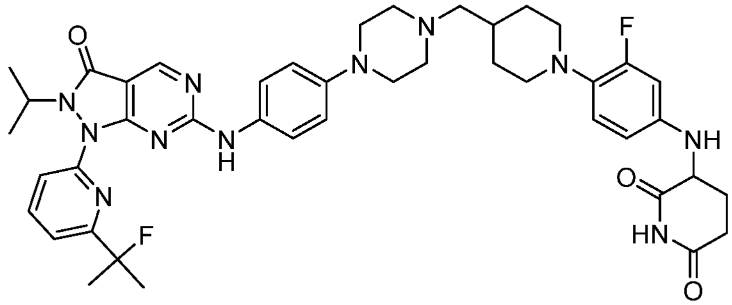

,

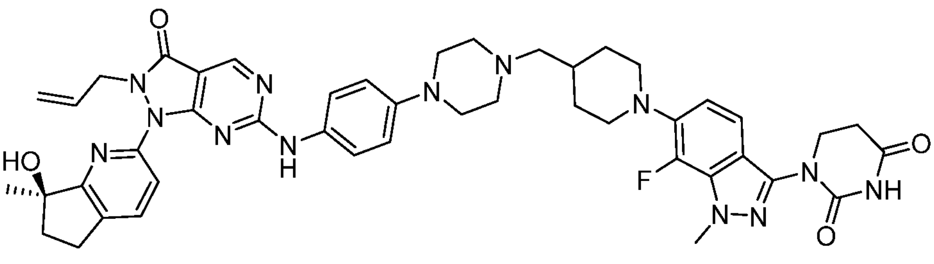
,

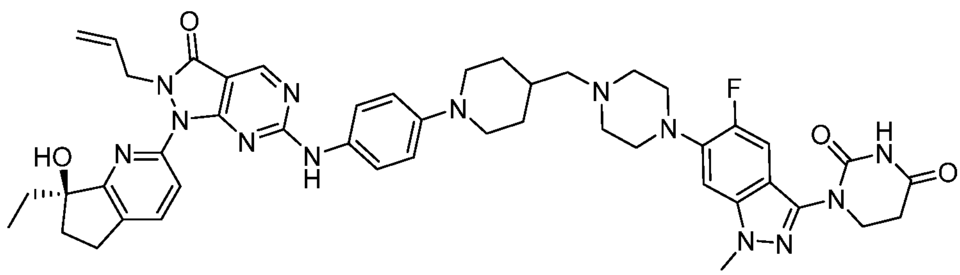
,

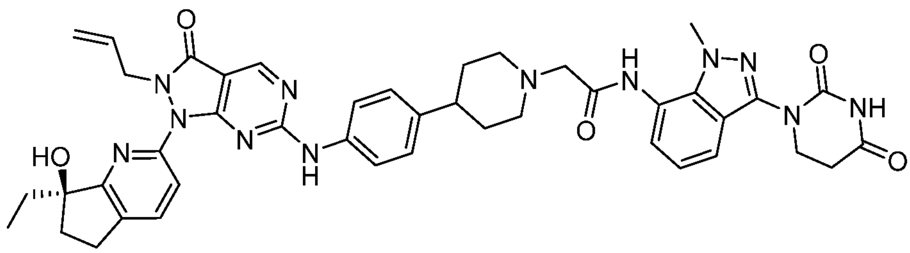
,

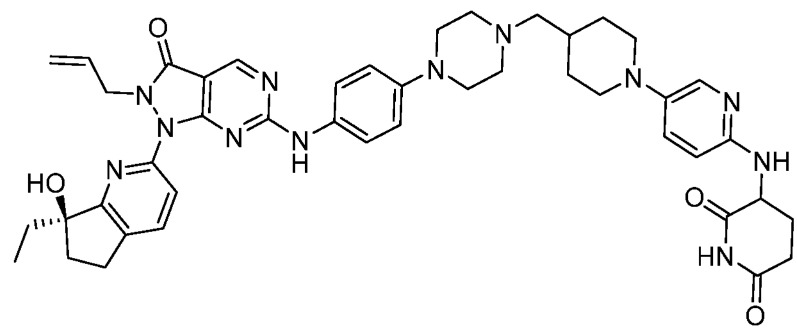
,

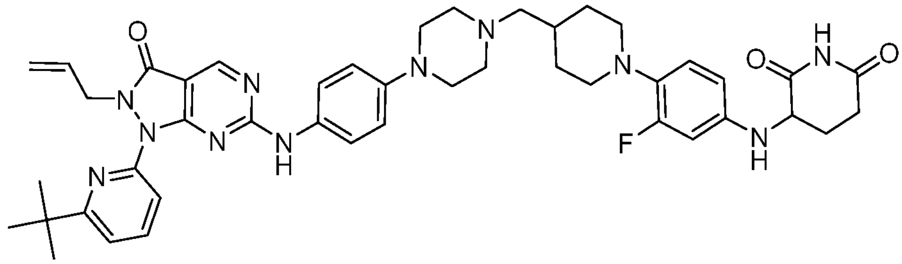
,

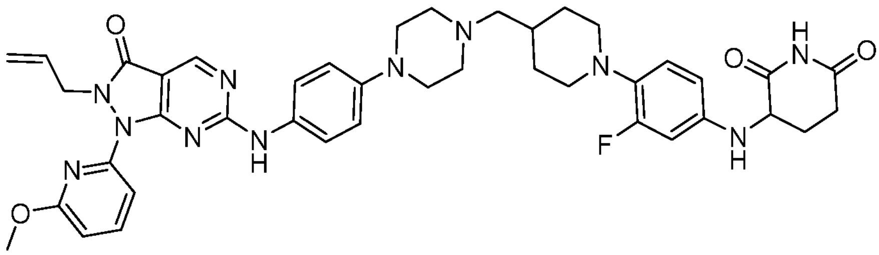

,

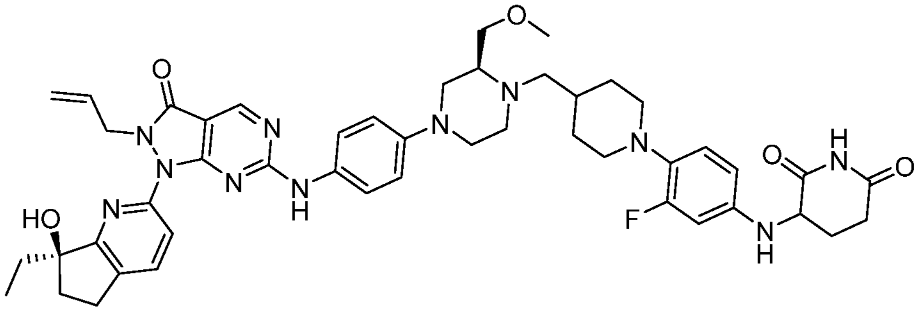

,

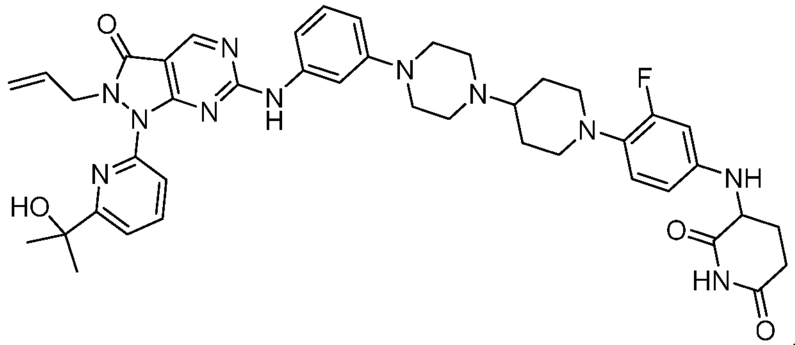

,

Hereinafter, the application will illustrate its beneficial effects by examples. Those skilled in the art will recognize that these examples are illustrative and not limiting. These examples do not limit the scope of this application in any way. The experimental methods described in the following examples are conventional unless otherwise specified. Unless otherwise specified, reagents and materials are commercially available.

The compounds of this invention may be prepared or isolated as described in the following general scheme. Those compounds were synthesized by synthetic and/or semisynthetic methods known to those skilled in the art for analogous compounds and by methods described in detail in the example, herein. All the variable groups applied in these schemes are as defined in the claims.

Scheme **1-1:**

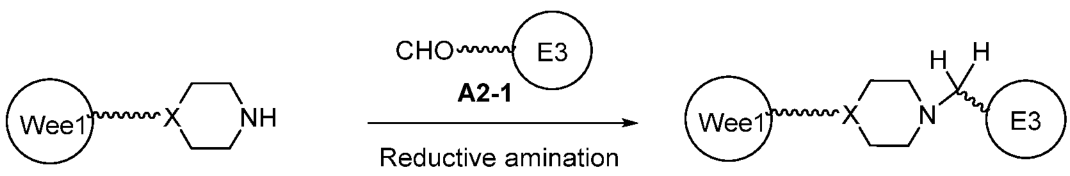


As described in above scheme, the applied compound may be accessed by reductive amination of aldehyde A2-1 with amine A1-1. The reductive amination conditions are generic reductive amination conditions including and not limited to conditions such as sodium cyanoborohydride, sodium triacetylborohydride, sodium borohydride, etc. The X represents a carbon or nitrogen atom, and the wavy line represents the connection of the reactive functional group with other fragments of the compound.

Scheme 1-2:

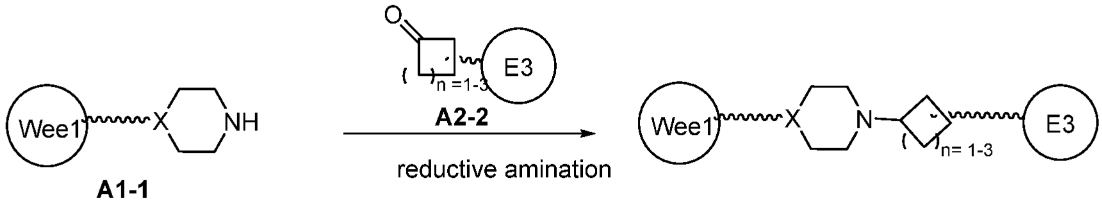


As described in above scheme, the applied compound may be accessed by reductive amination of ketone A2-2 with amine A1-1. The reductive amination conditions are generic reductive amination conditions including and not limited to conditions such as sodium cyanoborohydride, sodium triacetylborohydride, sodium borohydride, etc. The X represents a carbon or nitrogen atom, and the wavy line represents the connection of the reactive functional group with other fragments of the compound.

Scheme 1-3:

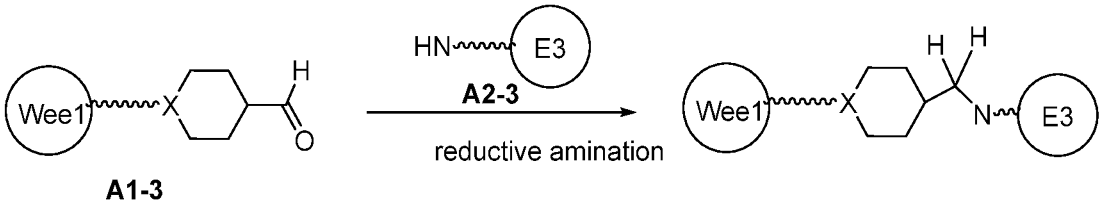


As described in above scheme, the applied compound may be accessed by reductive amination of aldehyde A1-3 with amine A2-3. The reductive amination conditions are generic reductive amination conditions including and not limited to conditions such as sodium cyanoborohydride, sodium triacetylborohydride, sodium borohydride, etc. The X represents a carbon or nitrogen atom, and the wavy line represents the connection of the reactive functional group with other fragments of the compound.

Scheme 1-4:

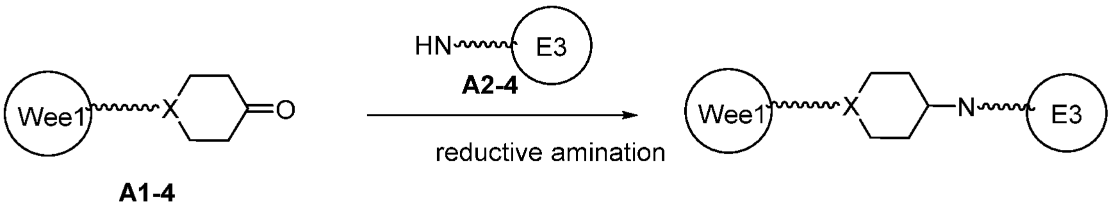


As described in above scheme, the applied compound may be accessed by reductive amination of ketone A1-4 with amine A2-4. The reductive amination conditions are generic reductive amination conditions including and not limited to conditions such as sodium cyanoborohydride, sodium triacetylborohydride, sodium borohydride, etc. The X represents a carbon or nitrogen atom, and the wavy line represents the connection of the reactive functional group with other fragments of the compound.

Scheme 2-1:

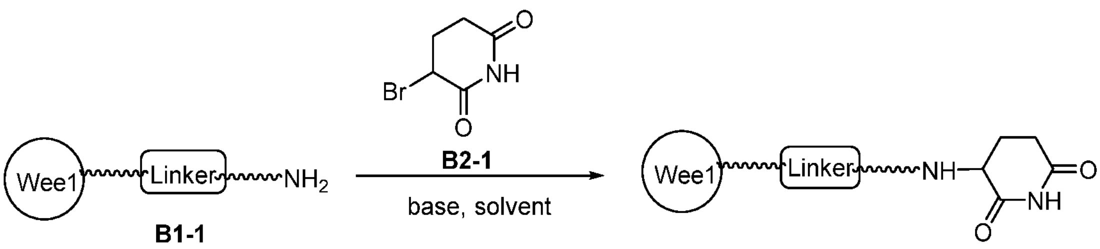


As described in above scheme, the applied compound can be produced by $SN_2$ substitition of bromide B2-1

by amine B1-1 in the presence of a suitable organic base such as DIPEA, $Et_3N$, or other inorganic base such as $K_2CO_3$, $Cs_2CO_3$ in the suitable solvent such as DMF, DMA, DMSO, THF etc. The wavy line represents the connection of the reactive functional group with other fragments of the compound.

Scheme **2-2**:

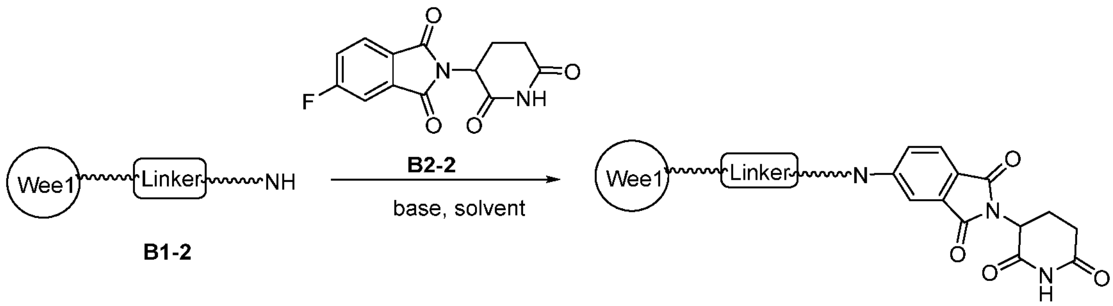


As described in above scheme, the applied compound can be produced by $SN_{Ar}$ substitition of fluoride B2-2 by secondary amine B1-2 in the presence of a suitable organic base such as DIPEA, $Et_3N$, or other inorganic base such as $K_2CO_3$, $Cs_2CO_3$ in the suitable solvent such as DMF, DMA, DMSO, THF etc. The wavy line represents the connection of the reactive functional group with other fragments of the compound.

Scheme **3-1**:

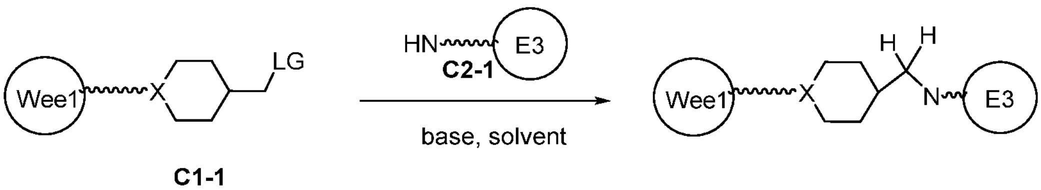


As described in above scheme, the applied compound can be produced by $SN_2$ substitition of intermediate C1-1 with leaving group by secondary amine C2-1 in the presence of a suitable organic base such as DIPEA, $Et_3N$, or other inorganic base such as $K_2CO_3$, $Cs_2CO_3$ in the suitable solvent such as DMF, DMA, DMSO, THF etc. The wavy line represents the connection of the reactive functional group with other fragments of the compound.

Scheme **3-2**:

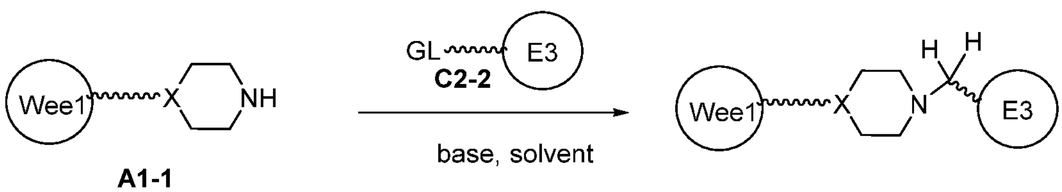


As described in above scheme, the applied compound can be produced by $SN_2$ substitition of intermediate C2-2 with leaving group by secondary amine A1-1 in the presence of a suitable organic base such as DIPEA, $Et_3N$, or other inorganic base such as $K_2CO_3$, $Cs_2CO_3$ in the suitable solvent such as DMF, DMA, DMSO, THF etc. The wavy line represents the connection of the reactive functional group with other fragments of the compound.

Scheme **4-1**:

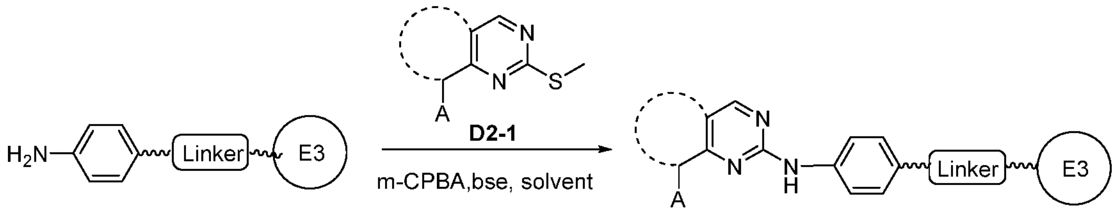


**D1-1**

As described in above scheme, the appled compound can be produced by $SN_2$ substitution of a sulfone intermediate which was formed by oxidization of thioether D2-1 with m-CPBA by aniline D1-1 in the presence of a sitable organic base such as DIPEA, $Et_3N$, or other inorganic base such as $K_2CO_3$, $Cs_2CO_3$ in the suitable solvent such as DMF, DMA, DMSO, THF etc. The wavy line represents the connection of the reactive functional group with other fragments of the compound. Linker presents the fragment of linker.

**Examples**

The compounds of the present application can be synthesized by the following exemplary or the similarly general synthetic methods. Various intermediates may have various substituents described in this application as valence permits.

Abbreviation List

| Number | Abbreviation | Name |
|---|---|---|
| 1. | DCM | dichloromethane |
| 2. | EA | ethyl acetate |
| 3. | DMF | *N*,*N*-Dimethylformamide |
| 4. | DMSO | dimethyl sulfoxide |
| 5. | THF | tetrahydrofuran |
| 6. | MeOH | methanol |
| 7. | EtOH | ethanol |
| 8. | Toluene | methylbenzene |
| 9. | Dioxane | 1,4-Dioxane |
| 10. | Pyridine | Pyridine |
| 11. | MeCN | acetonitrile |
| 12. | NMP | *N*-Methylpyrrolidone |
| 13. | DIEA | *N,N*-Diisopropylethylamine |
| 14. | NaH | Sodium hydride |
| 15. | $POCl_3$ | phosphorus oxychloride |
| 16. | HCl | hydrogen chloride |
| 17. | m-CPBA | 3-chloroperbenzoic acid |
| 18. | $(BOC)_2O$ | di-tert-butyl dicarbonate |
| 19. | $MeNH_2$ | Aminomethane |
| 20. | $POBr_3$ | Phosphorusoxybromide |

(continued)

| Number | Abbreviation | Name |
|---|---|---|
| 21. | $Ac_2O$ | Acetic anhydride |
| 22. | LiOH | Lithium hydroxide |
| 23. | CuI | copper(I) iodide |
| 24. | $K_2CO_3$ | Potassium carbonate |
| 25. | $Et_3N$ | tri ethyl amine |
| 26. | $NaBH_3CN$ | Sodium cyanoborohydride |
| 27. | $NaBH(OAc)_3$ | Sodium triacetoxyborohydride |
| 28. | HOAc | Acetic Acid |
| 29. | $SOCl_2$ | thionyl chloride |
| 30. | $T_3P$ | 2,4,6-Tripropyl-1,3,5,2,4,6-Trioxatriphosphorinane-2,4,6-Trioxide |
| 31. | $NH_4Cl$ | Ammonium chloride |
| 32. | TsCl | Tosyl chloride |
| 33. | KI | Potassium Iodide |
| 34. | $H_2$ | Hydrogen |
| 35. | $Pd(dppf)_2Cl_2$ | 1,1'-Bis(diphenylphosphino)ferrocene-palladium(II) dichloride |
| 36. | Xphos Pd $G_3$ | Methanesulfonato(2-dicyclohexylphosphino-2',4',6'-triipropyl-1,1'-biphenyl)(2'-amino-1,1'-biphenyl-2-yl) palladium(II) |
| 37. | RuPhosPd $G_3$ | Methanesulfonato(2-dicyclohexylphosphino-2',6'-di-i-propoxy-1,1'-biphenyl)(2'-amino-1,1'-biphenyl-2-yl) palladium(II) |
| 38. | $PdPPh_3$ | Tetrakis(triphenyl phosphine)palladium |
| 39. | Pd/C | 10% Palladium on carbon |
| 40. | $Pd(OH)_2$ | Palladium hydroxide |
| 41. | $PtO_2$ | Platinum(IV) oxide |
| 42. | MsCl | Methanesulfonyl chloride |
| 43. | MsOH | Methanesulfonic acid |
| 44. | $NaIO_4$ | Sodium Metaperiodate |
| 45. | $K_2OsO_4$ | Potassium osmate |

**Synthesis of intermediate B 1 and B 2:**

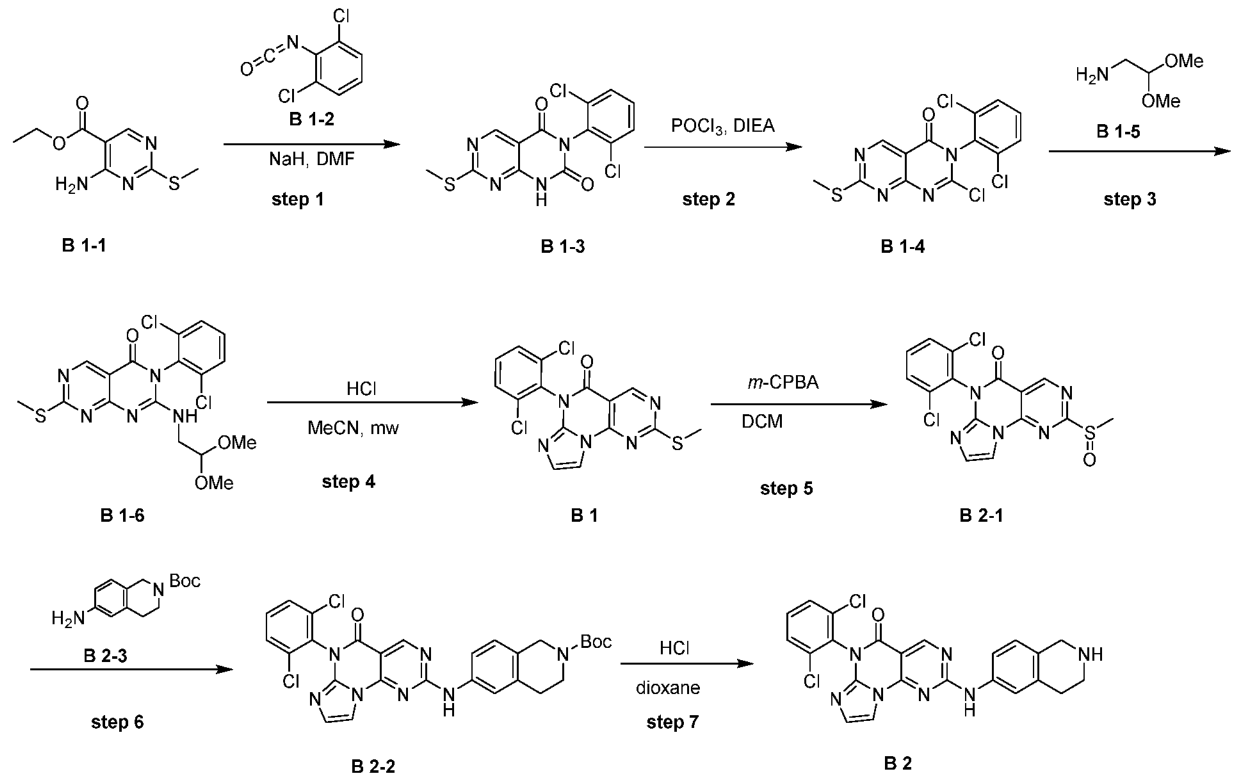


B 1-1 B 1-3 B 1-4 B 1-6 B 1 B 2-1 B 2-2 B 2

**Step 1: synthesis of intermediate B 1-3**

To a solution of **intermediate B 1-1** (8.85 g, 41.49 mmol) in DMF (80 mL) was added 60% NaH in mineral oil (2.83 g, 70.79 mmol) at 0°C. The reaction mixture was stirred for 30 min at 0°C and **intermediate B 1-2** (7.8 g, 41.49 mmol) in DMF (20 mL) was added to the reaction mixture and the reaction mixture was stirred for 2 hours at 0°C under $N_2$ atmospheres. The reaction mixture was poured into ice-water (50 mL), extracted with MTBE (30 mL × 2), the water phase was adjusted pH to 4-5, extracted with EtOAc (50 mL × 2). The organic phase was washed with saturated salt solution (30 mL × 2), dried over $Na_2SO_4$, filtered and concentrated to give a crude product. The crude product was added MeOH (10 mL), and the mixture was stirred for 15 min, filtered to give an **intermediate B 1-3** (9.8 g, 66.50% yield) as a white solid.

$^1$H NMR (400MHz, DMSO-*d*$_6$) $\delta$ 12.91 (s, 1H), 8.98 (s, 1H), 7.67 (d, *J*= 8.0 Hz, 2H), 7.55 - 7.50 (m, 1H), 2.57 (s, 3H)

**Step 2: synthesis of intermediate B 1-4**

A mixture of **intermediate B 1-3** (14.6 g, 41.10 mmol) in $POCl_3$ (82.50 g, 538.05 mmol, 50 mL) and DIEA (55 mL) was stirred at 90°C for 3 hours. The reaction mixture was concentrated by rotary distillation to remove the solvent. The residue was diluted with sat. $NaHCO_3$ (500 mL) and extracted with EtOAc (100 mL × 3). The combined organic layers were washed with sat. $NaHCO_3$ (100 mL × 3), dried over $Na_2SO_4$, filtered and concentrated by rotary distillation to give the residue **intermediate B 1-4** (15.3 g, crude) as a yellow solid. The crude product was used into the next step without further purification.

**Step 3: synthesis of intermediate B 1-6**

A mixture of **intermediate B 1-4** (15.3 g, 40.95 mmol) and **intermediate B 1-5** (8.61 g, 81.90 mmol, 8.92 mL) in MeCN (150 mL) was degassed and purged with $N_2$ for 3 times, and then the mixture was stirred at 80 °C for 1 h under $N_2$ atmosphere. The reaction solution was spin-dried to obtain crude product. To the crude was added PE: EtOAc: DCM (3: 1: 0.2, 50 mL). The reaction mixture was stirred for 1 hour and filtered to give a filter cake, which is **intermediate B** 1-6 (8.2 g, 32.60% yield) as a white solid. The mather lique was purified with chromatography on silica gel (PE: EtOAc=1: 1) to give **intermediate B 1-6** (3.2 g, 17.67% yield) as a yellow solid.

MS (ESI$^+$): m/z 441.95 [M+H]$^+$.

$^1$H NMR (400 MHz, DMSO-*d*$_6$) $\delta$ 8.92 (s, 1H), 7.80 - 7.75 (m, 2H), 7.71 - 7.64 (m, 2H), 4.58 (t, *J*= 5.4 Hz, 1H), 3.47 - 3.41 (m, 2H), 3.33 - 3.27 (m, 6H), 2.58 (s, 3H).

**Step 4: synthesis of intermediate B 1**

**intermediate B 1-6** (700 mg, 1.58 mmol) and HCl (12 M, 132 μL) were taken up into a microwave tube in MeCN (6 mL). The sealed tube was heated at 160 °C for 15 min under microwave. The reaction mixture was added EtOAc (15 mL), stirred for 15 min, filtered to give a crude product, the mather lique was washed with sat. $NaHCO_3$ (5 mL × 2), dried over $Na_2SO_4$, filtered and concentrated in vacuo to give a crude product**intermediate B 1** (580 mg, crude) as a brown solid. The crude product was used into the next step without further purification.

**Step 5: synthesis of intermediate B 2-1**

m-CPBA (268 mg, 1.32 mmol, 85% purity) was added to a mixture consisting of **intermediate B 1** (200 mg, 529 μmol) and DCM (5 mL). The reaction mixture was stirred at 25 °C for 2 hr. The reaction mixture was quenched with aq. $Na_2S_2O_3$ (20 mL) and extracted with DCM (20 mL × 3). The combined organic extracts were dried over anhydrous $Na_2SO_4$, filtered and concentrated to dryness under reduced pressure to give **intermediate B2-**1 (200 mg, 57.6% yield) as a yellow solid.

MS (ESI$^+$): m/z 393.9 [M+H]$^+$.

**Step 6: synthesis of intermediate B 2-2**

A solution of **intermediate B 2-1** (200 mg, 507 μmol), **intermediate B 2-3** (151 mg, 609 μmol) in DMSO (3 mL) was stirred at 120 °C for 8 hr. The reaction solution was spin-dried to obtain crude product. The reaction mixture was quenched by water (10 mL) and extracted with EA (20 mL×3). The combined organic phase was dried over anhydrous $Na_2SO_4$ and filtered and concentrated by rotary distillation to get a crude product. The residue was purified by flash silica gel chromatography (petroleum ether: ethyl acetate = 1: 0 to 0: 1) to give intermidiate **B 2-2** (100 mg, 34.1% yield) as a brown solid.

**Step 7: synthesis of intermediate B 2**

HCl/dioxane (4 M, 3 mL) was added to a mixture consisting of **intermediate B 2-2** (100 mg, 173 μmol) and dioxane (1 mL). The reaction mixture was stirred at 25 °C for 1 hr. The reaction solution was spin-dried to obtain crude product. The residue was purified by prep-HPLC (Calumn: Kromasil 100-5-C18 30×150 mm, Molile Phase A: water (0.01% FA), Molile Phase B: acetonitrile, 25 mL/min, gradient condition form 30% B to 60%) to give **intermediate B 2** (45 mg, 51% yield) as a brown solid.

MS (ESI$^+$): m/z 477.9 [M+H]$^+$.

$^1$H NMR (400 MHz, DMSO-$d_6$) $\delta$ 10.84 (s, 1H), 9.16 (s, 1H), 7.81 - 7.73 (m, 3H), 7.69 - 7.53 (m, 3H), 7.18 - 7.08 (m, 2H), 3.93 (s, 2H), 3.08 - 3.01 (m, 2H), 2.81 (s, 2H).

The following intermediates can be prepared using methods similar to the above synthetic route.

| Intermediate No. | Structure | MS (ESI$^+$): m/z [M+H]$^+$ | $^1$H NMR (400 MHz, DMSO-$d_6$) $\delta$ |
|---|---|---|---|
| B 3A | 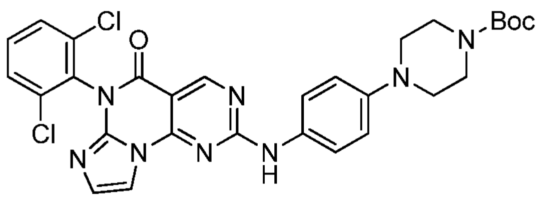 | 607.1 | |
| B 3 | 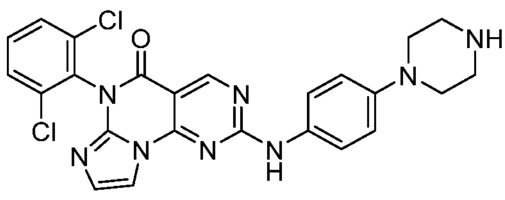 | 507.1 | |

(continued)

| Intermediate No. | Structure | MS (ESI+): m/z [M+H]+ | $^1$H NMR (400 MHz, DMSO-$d_6$) $\delta$ |
|---|---|---|---|
| B 4A | 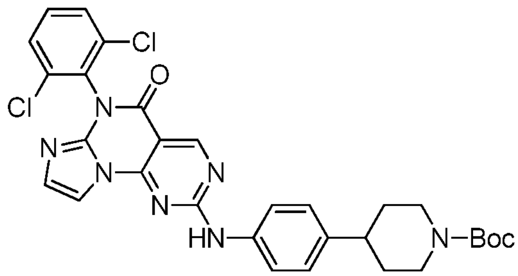 | 606.2 | 10.96 - 10.65 (m, 1H), 9.16 (s, 1H), 7.94 - 7.71 (m, 5H), 7.67 - 7.61 (m, 1H), 7.36 - 7.25 (m, 2H), 7.14 - 7.11 (m, 1H), 4.12 - 4.06 (m, 2H), 2.92 - 2.63 (m, 3H), 1.82 - 1.73 (m, 2H), 1.53 - 1.47 (m, 2H), 1.43 (s, 9H) |
| B 4 | 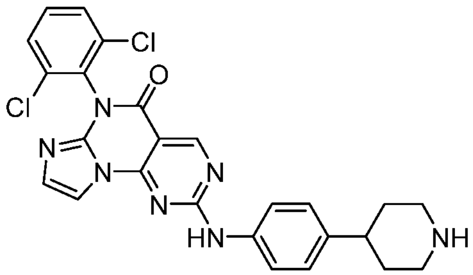 | 506.2 | |
| B 5A | 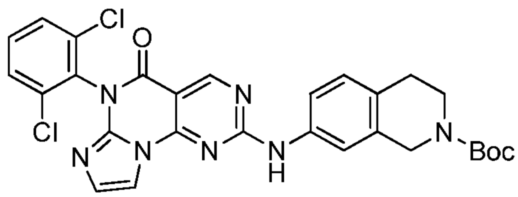 | 579.5 | |
| B 5 | 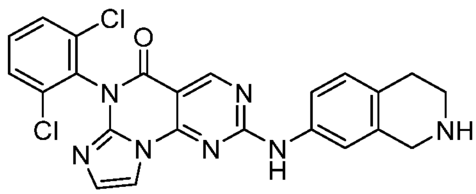 | 478.2 | |

**Synthesis of intermediate B 6 and B 7-B:**

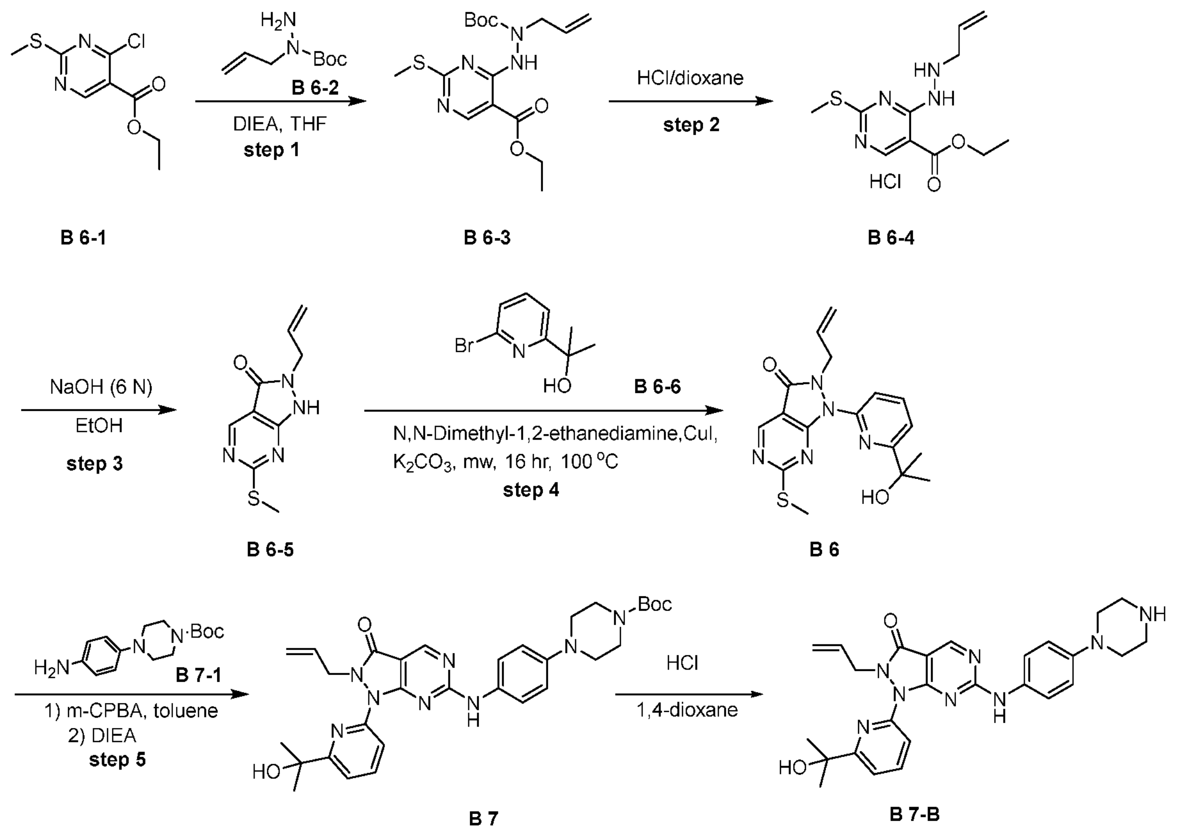


B 6-1 B 6-3 B 6-4

B 6-5 B 6

B 7 B 7-B

**Step 1: synthesis of intermediate B 6-3**

A mixture of intermediate **B 6-1** (6.76 g, 29.0 mmol), **intermediate B 6-2** (5.00 g, 29.0 mmol), DIEA (9.38 g, 72.6 mmol) in THF (50 mL) was stirred at 70 °C for 16 h under $N_2$ atmosphere. The mixture was concentrated by rotary distillation to afford the crude product. The residue was purified by flash silica gel chromatography (Petroleum ether: ethyl acetate = 1: 0 to 10: 1). intermediate **B 6-3** (8.50 g, 73.9% yield) was obtained as a colorless oil.

MS ($ESI^+$): m/z 369.1 $[M+H]^+$.

$^1H$ NMR (400 MHz, DMSO-$d_6$) $\delta$ 9.73 (s, 1H), 8.66 (s, 1H), 5.96 - 5.80 (m, 1H), 5.26 - 5.03 (m, 2H), 4.30 (q, *J* = 7.2 Hz, 2H), 4.16 - 4.06 (m, 2H), 2.46 (s, 3H), 1.44 - 1.20 (m, 12H)..

**Step 2: synthesis of intermediate B 6-4**

To a mixture of **intermediate B 6-3** (8.50 g, 23.07 mmol) in DCM (20 mL) was added HCl/dioxane (4 M, 58 mL). The mixture was stirred at 25 °C for 1 h. The mixture was concentrated by rotary distillation. **intermediate B 6-4** (7.00 g, crude, HCl salt) was obtained as a white solid.

MS ($ESI^+$): m/z 268.7 $[M+H]^+$.

**Step 3: synthesis of intermediate B 6-5**

A mixture of **intermediate B 6-4** (5.00 g, 16.4 mmol, HCl salt) in aq. NaOH (6 M, 21 mL) and EtOH (30 mL) was stirred at 25 °C for 3 h under $N_2$ atmosphere. The reaction mixture was acidified by using 6N HCl solution and EtOH was removed by rotary distillation. The mixture was extracted with DCM 100 mL for three times, washed with brine (150 mL), dried over anhydrous $MgSO_4$, filtered and concentrated by rotary distillation. **Intermediate B 6-5** (3.30 g, crude) was obtained as a yellow solid.

MS ($ESI^+$): m/z 223.06 $[M+H]^+$.

**Step 4: synthesis of intermediate B 6**

**Intermediate B 6-5** (1.10 g, 4.95 mmol), **intermediate B 6-6** (1.18 g, 5.44 mmol), CuI (943 mg, 4.95 mmol), $K_2CO_3$ (1.03 g, 7.42 mmol), and *N,N*-dimethylethane-1,2-diamine (873 mg, 9.90 mmol) in dioxane (10 mL) were heated

at 100 °C for 1 h under microwave. The reaction was repeated for another two times in parallel. The reaction mixture was poured into water (20 mL) and extracted with EA (20 mL) in total for three times. The combined organic phase was washed with water (20 mL), dried over anhydrous $Na_2SO_4$ and concentrated by rotary distillation. The residue was purified by flash silica gel chromatography (petroleum ether: ethyl acetate = 1: 0 to 1: 1). **intermediate B 6** (2.85 g, 53.7% yield) was obtained as a white solid.

MS (ESI$^+$): m/z 358.1 $[M+H]^+$.

$^1$H NMR (400 MHz, DMSO-$d_6$) $\delta$ 9.01 (s, 1H), 8.03 (t, *J* = 8.0 Hz, 1H), 7.79 - 7.73 (m, 1H), 7.69 - 7.60 (m, 1H), 5.75 - 5.59 (m, 1H), 5.39 (s, 1H), 4.98 (dd, *J* = 1.2, 10.4 Hz, 1H), 4.84 - 4.72 (m, 3H), 2.55 (s, 3H), 1.45 (s, 6H).

**Step 5: synthesis of intermediate B 7**

To a solution of **intermediate B 6** (2.00 g, 5.60 mmol) in toluene (10 mL) was added m-CPBA (1.36 g, 6.71 mmol, 85% purity). The mixture was stirred at 25 °C for 1 h. Tert-butyl 4-(4-aminophenyl) piperazine-1-carboxylate (1.86 g, 6.71 mmol) and DIEA (2.89 g, 22.4 mmol, 3.90 mL) was added to the mixture. The mixture was stirred at 25 °C for 12 h. The mixture was quenched with saturated $Na_2S_2O_3$ and extracted with EA (10 mL×2). The combined organic phase was washed with brine (5 mL), dried over anhydrous $Na_2SO_4$, filtered and concentrated by rotary distillation. The residue was purified by flash silica gel chromatography (petroleum ether: ethyl acetate = 1: 0 to 3: 5) to obtained the **intermediate B 7** (2.79 g, 85% yield) as a white solid.

$^1$HNMR (400 MHz, DMSO-$d_6$) $\delta$ 10.28 - 10.11 (m, 1H), 8.83 (s, 1H), 8.13 - 7.97 (m, 1H), 7.81 - 7.71 (m, 1H), 7.64 - 7.55 (m, 3H), 6.95 (d, *J*= 8.8 Hz, 2H), 5.79 - 5.56 (m, 1H), 5.37 (s, 1H), 4.99 (dd, *J*=1.2, 10.3 Hz, 1H), 4.84 - 4.78 (m, 1H), 4.72 - 4.64 (m, 2H), 3.36 - 3.34 (m, 4H), 3.07 - 3.02 (m, 4H), 1.45 (s, 6H), 1.42 (s, 9H).

**Step 6: synthesis of intermediate B 7-B**

To the solution of **intermediate B 7** (100 mg, 170 μmol) in DCM (1 mL) was added HCl / dioxane (4 M, 2 mL), the obtained solution was stirred at 25°C for 1 hour. Then the solution was concentrated to dry to obtained the yellow solid **intermediate B 7-B** (90 mg, HCl salt crude). MS (ESI$^+$): m/z 487 $[M+H]^+$.

The following intermediates were prepared according to the procedures described in the example above using the appropriate starting materials, and the protected intermediates can be deprotected by hydrochloride or trifluoroacetic acid to provide the corresponding amine.

| Intermediate No. | Structure | MS (ESI$^+$): m/z $[M+H]^+$ | $^1$H NMR (400 MHz, DMSO-$d_6$) $\delta$ |
|---|---|---|---|
| B 9A | 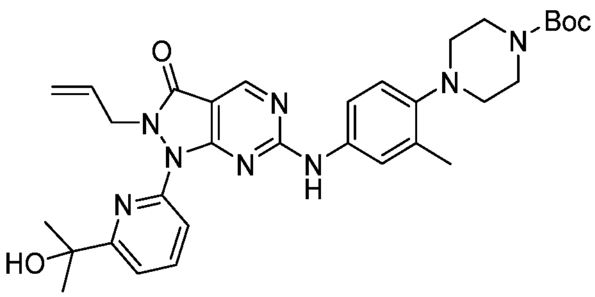 | 601.27 | / |
| B 9 | 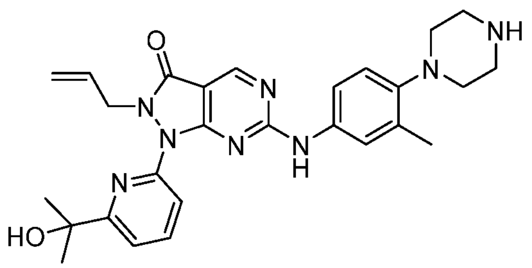 | 501.3 | |

(continued)

| Intermediate No. | Structure | MS (ESI⁺): m/z [M+H]⁺ | $^1$H NMR (400 MHz, DMSO-$d_6$) $\delta$ |
|---|---|---|---|
| B 10A | 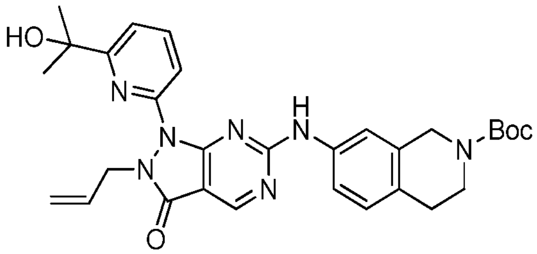 | 558.0 | 10.31 (s, 1H), 8.89 (s, 1H), 8.06 (s, 1H), 7.92 - 7.87 (m, 1H), 7.83 - 7.76 (m, 1H), 7.62 (d, *J* = 7.6 Hz, 1H), 7.42 - 7.31 (m, 1H), 7.14 - 7.05 (m, 1H), 5.70 - 5.59 (m, 1H), 5.37 (s, 1H), 5.04 - 4.96 (m, 1H), 4.88 - 4.77 (m, 1H), 4.72 - 4.64 (m, 2H), 4.50 (s, 2H), 3.58 - 3.52 (m, 2H), 2.77 - 2.68 (m, 2H), 1.46 (s, 9H), 1.39 (s, 6H) |
| B 10 | 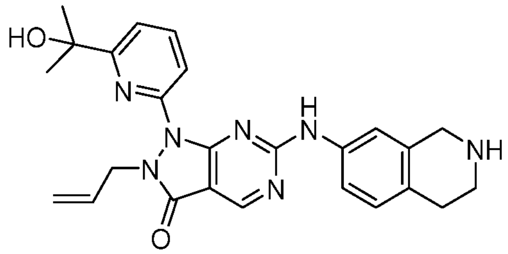 | 458.0 | |
| B 29 | 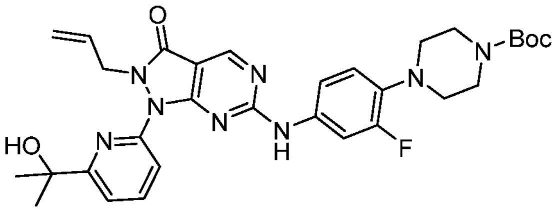 | 604.67 | |
| B 29B | 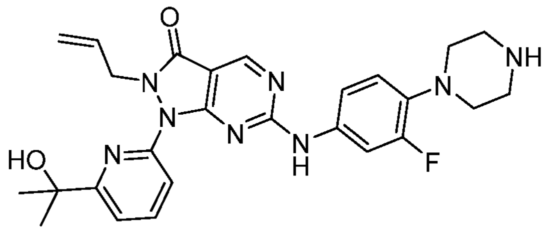 | 504.7 | |
| B 48 | 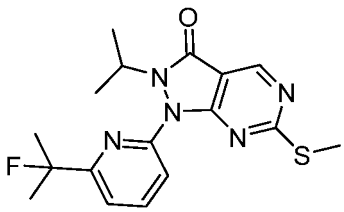 | 362.1 | |
| B 52 | 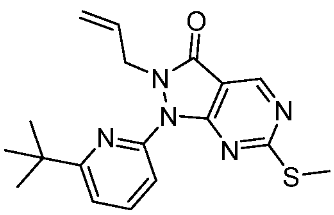 | 356.1 | |

(continued)

| Intermediate No. | Structure | MS (ESI+): m/z [M+H]+ | $^1$H NMR (400 MHz, DMSO-$d_6$) $\delta$ |
|---|---|---|---|
| B 53 | 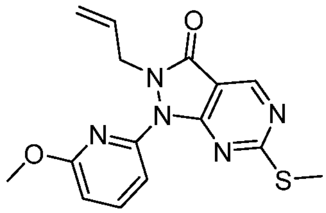 | 329.1 | |
| B 54 | 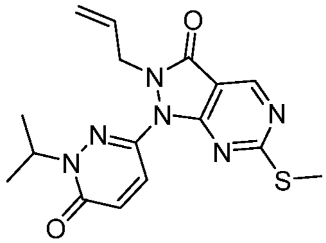 | 359.1 | |

**Synthesis of intermediate B 11:**

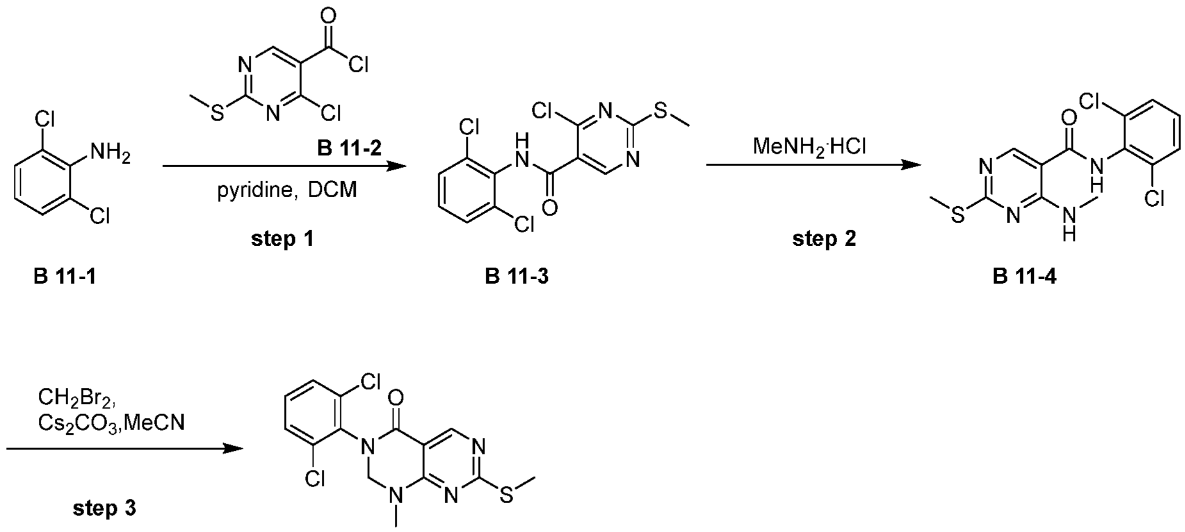


B 11-1

B 11-3

B 11-4

B 11

**Step 1: synthesis of intermediate B 11-3**

To a solution of **intermediate B 11-1** (2g, 12.3 mmol), pyridine (1.22 g, 15.4 mmol, 1.3 mL) in DCM (20 mL) was added **intermediate B 11-2** (2.75 g, 12.3 mmol) under the condition of ice water, the mixture was stirred at room temperature for 48 hours. The reaction mixture was diluted with $H_2O$ (50 mL) and extracted with EA (100 mL). The combined organic layers were washed with 1 N HCl (20 mL), dried over anhydrous $Na_2SO_4$, filtered and concentrated by rotary distillation to give the residue. The residue was triturated with $CH_3CN$ (30 mL) to give the **intermediate B 11-3** (1 g, 2.87 mmol, 23.2% yield) as a white solid.

$^1$H NMR (400 MHz, DMSO-$d_6$) $\delta$ 10.71 (s, 1H), 8.82 (s, 1H), 7.62 (d, $J$= 8.0 Hz, 2H), 7.46 - 7.40 (m, 1H), 2.60

(s, 3H).

**Step 2: synthesis of intermediate B 11-4**

To a solution of **intermediate B 11-3** (500 mg, 1.43 mmol), methanamine hydrochloride (290 mg, 4.30 mmol) in THF (10 mL) was added TEA (435 mg, 4.30 mmol, 600 μL) and the mixture was stirred at room temperature for 48 hours. The reaction mixture was diluted with $H_2O$ (50 mL) and extracted with EA (100 mL). The combined organic layers were dried over anhydrous $Na_2SO_4$, filtered and concentrated by rotary distillation to give the residue. The residue was purified by column chromatography (Petroleum ether: Ethyl acetate=3: 1) to give the **intermediate B 11-4** (320 mg, 49.41% yield).

MS (ESI$^+$): m/z 343.0 [M+H]$^+$.

**Step 3: synthesis of intermediate B 11**

To a solution of **intermediate B 11-4** (100 mg, 291 μmol), dibromomethane (152 mg, 874 μmol, 61 μL), $Cs_2CO_3$ (380 mg, 1.17 mmol) in MeCN (5 mL) was stirred at 80°C for 16 h. The reaction mixture was diluted with $H_2O$ (25 mL) and extracted with EA (50 mL). The combined organic layers were dried over anhydrous $Na_2SO_4$, filtered and concentrated to give the residue. The residue was purified by column chromatography (Petroleum ether: Ethyl acetate=5: 1 to give the **intermediate B 11** (60 mg, 53.59% yield).

MS (ESI$^+$): m/z 355.2 [M+H]$^+$.

**synthesis of intermediate B 12-B**

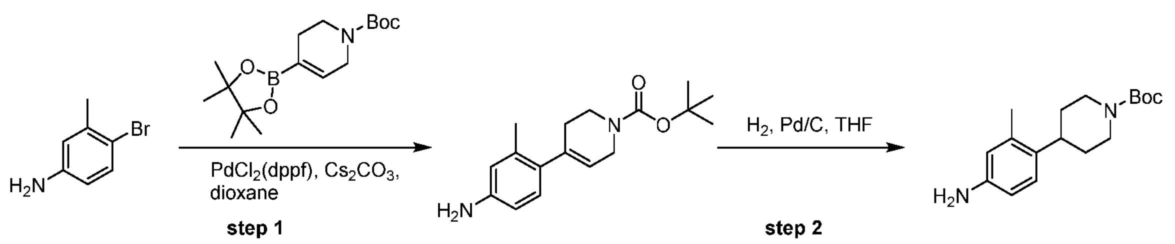


B 12-1 B 12-2 B 12-3

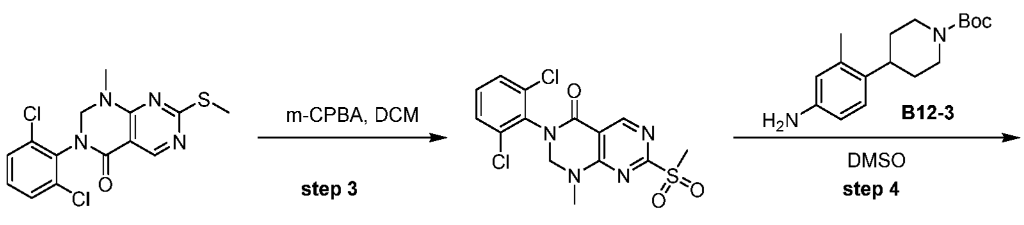


B 11 B 12-4

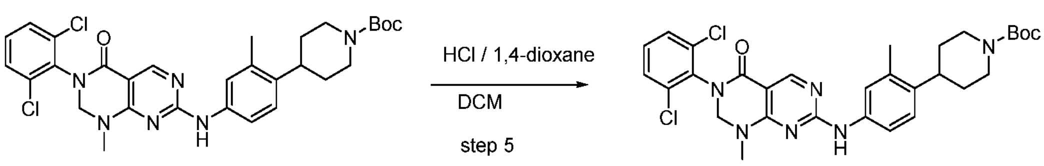


B 12 B 12-B

**Step 1: synthesis of intermediate B 12-2**

A mixture of **intermediate B 12-1** (2 g, 10.8 mmol) and tert-butyl 4-(4,4,5,5-tetramethyl-1,3,2-dioxaborolan-2-yl)-3,6-dihydro-2H-pyridine-1-carboxylate (3.32 g, 10.8 mmol) in $CH_3CN$ (20 mL) and $H_2O$ (4 mL) was added $K_2CO_3$ (4.46 g, 32.3 mmol) and $Pd(dppf)Cl_2$ (787 mg, 1.07 mmol), then stirred at 95 °C for 6 h under $N_2$ atmosphere. The

reaction mixture was filtered and dried by rotary distillation to give the residue. The residue was purified by column chromatography on silica gel (Petroleum ether/Ethyl acetate=1/0 to 5/1) to give an **intermediate B 12-2** (1.02 g, 32.8% yield) as a yellow oil. MS(ESI$^+$):m/z 388.8 [M+H]$^+$.

$^1$H NMR (400MHz, $CDCl_3$) $\delta$ 7.56 (q, *J*= 8.4 Hz, 2H), 4.42 - 4.32 (m, 2H), 2.80 (s, 3H), 1.58 (s, 12H), 1.43 - 1.36 (m, 3H).

**Step 2: synthesis of intermediate B 12-3**

A mixture of **intermediate B 12-2** (500 mg, 1.73 mmol) in THF (10 mL) was added 10% Pd/C (500 mg), degassed and purged with $H_2$ for 3 times. And then the mixture was stirred at 20 °C for 16 h under $H_2$ atmosphere. The reaction mixture was filtered and dried by rotary distillation to give the residue, which is **intermediate B 12-3** (241 mg, 83.6% yield) as a white solid.

$^1$H NMR (400MHz, $CDCl_3$) $\delta$ 6.96 (d, *J*= 7.9 Hz, 1H), 6.59 - 6.47 (m, 2H), 4.38 - 4.04 (m, 2H), 3.53 (br s, 2H), 2.84 - 2.69 (m, 3H), 2.27 (s, 3H), 1.76 - 1.67 (m, 2H), 1.54 - 1.51 (m, 2H), 1.49 (s, 9H).

**Step 3: synthesis of intermediate B 12-4**

A mixture of **intermediate B 11** (100 mg, 282 μmol) in DCM (5 mL) was added 85% m-CPBA (143 mg, 704 μmol) at 0 °C and then the mixture was stirred at 20 °C for 16 h. The reaction mixture was quenched by addition $Na_2S_2O_3$ (10 mL) and $NaHCO_3$ (10 mL) at 0 °C, and then diluted with $H_2O$ (20 mL) and extracted with DCM (20 mL ×2). The combined organic layers were washed with saturated salt solution (10 mL×2), dried over anhydrous $Na_2SO_4$, filtered and concentrated under reduced pressure to give the residue as **intermediate B 12-4** (246 mg, 60.2% yield) as a white solid.

**Step 4: synthesis of intermediate B 12**

A mixture of **intermediate B 12-4** (60 mg, 155 μmol) and **intermediate B 12-3** (45.00 mg, 155 μmol) in DMSO (1 mL) was displaced with $N_2$ for 3 times, and then the mixture was stirred at 120 °C for 16 h under $N_2$ atmosphere. The residue was purified by prep-HPLC to purify the residue. **Intermediate B 12** (19 mg, 20.3% yield) was obtained as a white solid.

$^1$HNMR (400 MHz, DMSO-$d_6$) $\delta$ 11.02 (br s, 1H), 9.54 - 9.41 (m, 1H), 8.13 (br s, 1H), 8.04 (d, *J* = 8.0 Hz, 1H), 7.86 - 7.78 (m, 1H), 7.42 - 7.32 (m, 8H), 7.28 (d, *J* = 6.4 Hz, 2H), 6.51 - 6.37 (m, 1H), 5.18 - 5.11 (m, 1H), 4.57 - 4.48 (m, 1H), 4.44 - 4.36 (m, 1H), 2.96 - 2.87 (m, 1H), 2.60 (d, *J*= 16.4 Hz, 1H), 2.45 - 2.34 (m, 1H), 2.03 (d, *J* = 5.6 Hz, 1H).

**Step 5: synthesis of intermediate B 12-B**

To the solution of intermediate **B 12** (19 mg, 31.8 μmol) in DCM (5 mL) was added HCl / dioxane (4M, 5 mL), the obtained solution was stirred at 20°C under $N_2$ atomosphere for 2 hours. Then the obtained mixture was filtered and concentrated to give a crude HCl salt of intermediate **B 12-B** (10 mg, 42.0% yield) as a white solid, which was used for the next step without further purification.

The following intermediates were prepared by similar methods to the above.

| Intermediate No. | Structure | MS(ESI$^+$): m/z [M+H]$^+$ | $^1$H NMR (400 MHz, DMSO-$d_6$) $\delta$ |
|---|---|---|---|
| B 13 | 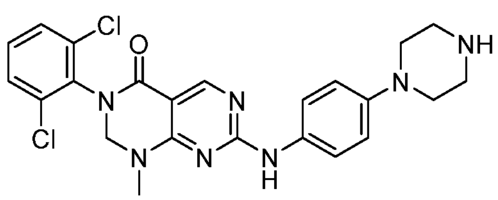 | 584.03 | / |

(continued)

| Intermediate No. | Structure | MS(ESI$^+$): m/z [M+H]$^+$ | $^1$H NMR (400 MHz, DMSO-$d_6$) $\delta$ |
|---|---|---|---|
| B 14 | 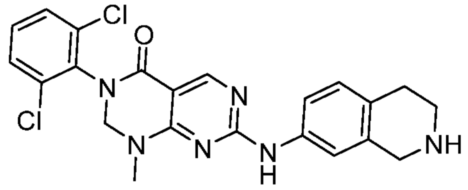 | 556.8 | / |

**Synthesis of intermediate B 30**

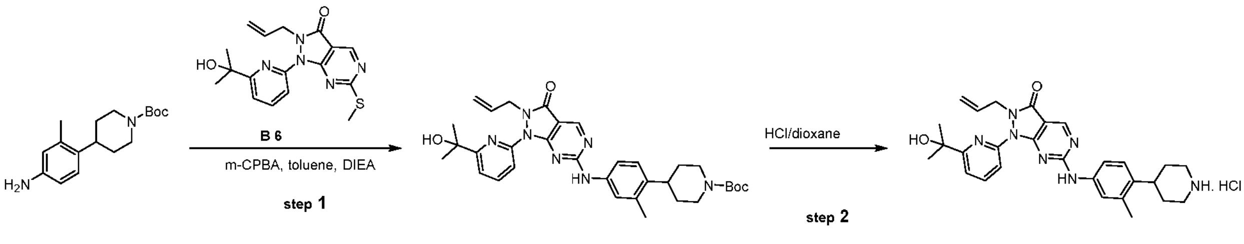


B 12-3 B 30-1 B 30

**Step 1: synthesis of intermediate B 30-1**

To a solution of intermediate **B 6** (246 mg, 689 μmol) in toluene (5 mL) was added m-CPBA (327 mg, 758 μmol) and stirred at 20 °C for 0.5 hours. Then DIEA (267 mg, 2.07 mmol) and intermediate **B 12-3** (200 mg, 689 μmol) was added and stirred at 20 °C for 16 hrs . The reaction mixture was quenched by adding $Na_2S_2O_3$ (20 mL) and $NaHCO_3$ (20 mL) at 0°C, and extracted with DCM 30 mL (10 mL×3). The combined organic layers were washed with water 30 mL (10 mL×3), dried over $Na_2SO_4$, filtered and concentrated under reduced pressure to give a residue. The residue was purified by column chromatography ($SiO_2$, DCM: MeOH = 10: 1) to give intermediate **B 30-1** (287 mg, 69.5% yield) as a yellow solid. LCMS (ESI$^+$): m/z 544.4 [M+H]$^+$

**Step 2: synthesis of intermediate B 30**

To a solution of intermediate **B 30-1** (274 mg, 457 μmol) in DCM (5 mL) was added HCl/dioxane (4 M, 5 mL). The mixture was stirred at 20 °C for 16 hrs. The reaction mixture was filtered and concentrated under reduced pressure to give a residue. Intermediate **B 30** (274 mg, crude, HCl salt) was obtained as a yellow solid, which was used for the next step directly.

The following intermediates were prepared according to the procedures described in the examples above using the appropriate starting materials.

| Intermediate No. | Structure | MS(ESI$^+$): m/z [M+H]$^+$ | $^1$H NMR (400 MHz, DMSO-$d_6$) $\delta$ |
|---|---|---|---|
| B 8A | 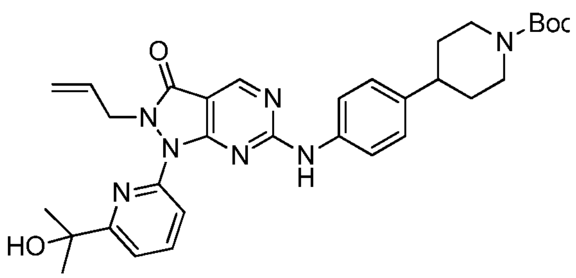 | 586.20 | |

(continued)

| Intermediate No. | Structure | MS(ESI$^+$): m/z $[M+H]^+$ | $^1$H NMR (400 MHz, DMSO-$d_6$) $\delta$ |
|---|---|---|---|
| B 8 | 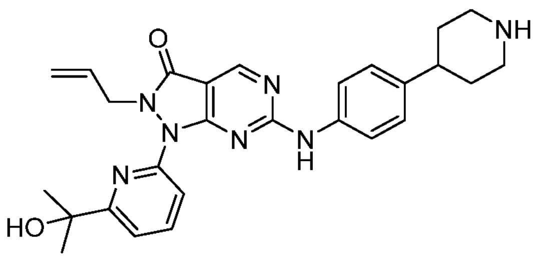 | 486.2 | |

**Synthesis of intermediate B 15-A and B 15-B:**

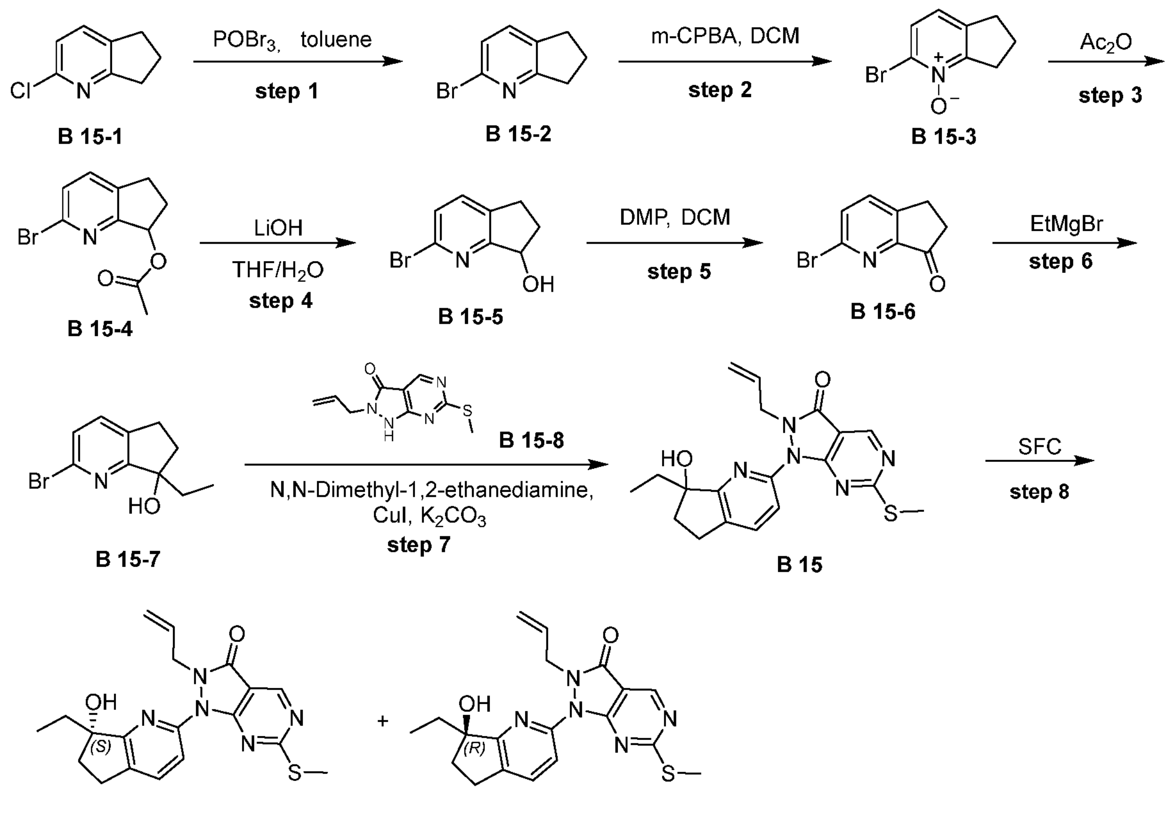


B 15-1, B 15-2, B 15-3, B 15-4, B 15-5, B 15-6, B 15-7, B 15, B 15-A + B 15-B

**Step 1: synthesis of intermediate B 15-2**

To a mixture of **intermediate B 15-1** (4 g, 26.0 mmol) in toluene (40 mL) was added $POBr_3$ (14.9 g, 52.1 mmol). The mixture was stirred at 130 °C for 72 h. The mixture was diluted with $H_2O$ (200 mL) and poured into saturated $NaHCO_3$ (200 mL) dropwise. Then the resulting mixture (pH >7) was stirred at room temprature for 10 min and filtered. The filtrate was extracted with EA (200 mL × 3) and the combined organic layers were dried over anhydrous $Na_2SO_4$, filtered and concentrated by rotary distillation to give the residue. The residue was purified by flash silica gel chromatography (petroleum ether: ethyl acetate = 1: 0 to 5: 1) to give an **intermediate B 15-2** (3.0 g, 52.4% yield) as a white solid.

MS (ESI$^+$): m/z 199.92 $[M+H]^+$.

$^1$H NMR (400 MHz, DMSO-$d_6$) $\delta$ 7.56 (d, $J$ = 8.0 Hz, 1H), 7.33 (d, $J$ = 8.0 Hz, 1H), 2.92 - 2.80 (m, 4H), 2.11 - 2.00 (m, 2H).

**Step 2: synthesis of intermediate B 15-3**

To a mixture of **intermediate B 15-2** (3.44, 17.4 mmol) in DCM (40 mL) was added m-CPBA (14.9 g, 52.1 mmol, 85% purity). The mixture was stirred at 25 °C for 5 h. The reaction mixture was quenched with saturated $NaHCO_3$ (50 mL) and saturated $Na_2S_2O_3$ (50 mL), and extracted with EA (100 mL×2). The combined organic layers were dried over anhydrous $Na_2SO_4$. filtered and concentrated by rotary distillation. The residue was purified by flash silica gel chromatography (petroleum ether: ethyl acetate = 1: 0 to 0: 1) to give **intermediate B 15-3** (3.50 g, 94.1% yield) as a white solid.
$^1$H NMR (400 MHz, $CDCl_3$) $\delta$ 7.46 (d, *J* = 8.0 Hz, 1H), 6.97 (d, *J* = 8.0 Hz, 1H), 3.26 - 3.18 (m, 2H), 3.04 - 2.97 (m, 2H), 2.26 - 2.17 (m, 2H)

**Step 3: synthesis of intermediate B 15-4**

A mixture consisting of **intermediate B 15-3** (3.5 g, 16.4 mmol) and $Ac_2O$ (33 mL, 349 mmol) was stirred at 110 °C for 12 h. The mixture was dried by rotary distillation and diluted with ethyl acetate (100 mL), then washed with saturated $NaHCO_3$ (15 mL × 2) and then with brine (20 mL). The mixture was dried over anhydrous $Na_2SO_4$, filtered and concentrated by rotary distillation to give the residue. The residue was purified by flash chromatography on silica gel (petroleum ether: ethyl acetate = 1: 0 to 4: 1) to give **intermediate B 15-4** (4 g, 82.6% yield) as yellow oil.
MS (ESI$^+$): m/z 257.89 [M+H]$^+$.

**Step 4: synthesis of intermediate B 15-5**

To a solution of **intermediate B 15-4** (6.20 g, 24.2 mmol) in THF (65 mL) and $H_2O$ (65 mL) was added LiOH (2.32 g, 96.8 mmol). The mixture was stirred at 25 °C for 2 hrs. The mixture was poured into water (100 mL) and extracted with ethyl acetate (20 mL×2). The combined organic extract was washed with saturated salt solution (20 mL), dried over anhydrous $Na_2SO_4$. The combined organic layers were filtered and concentrated by rotary distillation to give the residue. The residue was purified by flash chromatography on silica gel (petroleum ether: ethyl acetate = 1: 0 to 3: 1) to give **intermediate B 15-5** (4.50 g, 78.2% yield) as a yellow solid.
$^1$H NMR (400 MHz, $CDCl_3$) $\delta$ 7.45 (d, *J* = 8.0 Hz, 1H), 7.35 (d, *J* = 8.0 Hz, 1H), 5.19 (t, *J* = 6.8 Hz, 1H), 3.07 - 2.94 (m, 1H), 2.85 - 2.73 (m, 2H), 2.63 -2.50 (m, 1H), 2.13 - 2.00 (m, 1H)

**Step 5: synthesis of intermediate B 15-6**

To a solution of **intermediate B 15-5** (4.80 g, 22.4 mmol) in DCM (100 mL) at 0°C was added Dess-Martin periodinane (19.0 g, 44.9 mmol). The mixture was stirred at 25 °C for 2 h. The mixture was filtered and dried by rotary distillation. The residue was purified by flash silica gel chromatography (petroleum ether: ethyl acetate = 1: 0 to 1: 1) to give **intermediate B 15-6** (4.00 g, 75.71% yield) as a white solid.
$^1$H NMR (400 MHz, $CDCl_3$) $\delta$ 7.76 (d, *J* = 8.0 Hz, 1H), 7.64 (d, *J* = 8.0 Hz, 1H), 3.16 - 3.10 (m, 2H), 2.82 - 2.77 (m, 2H)

**Step 6: synthesis of intermediate B 15-7**

To a solution of **intermediate B 15-6** (4 g, 18.86 mmol) in THF (80 mL) was added bromo(ethyl)magnesium (19.0g, 44.9 mmol) at 0 °C. The mixture was stirred at 25 °C for 1 h. The mixture was cooled to 0°C and added dropwise to saturated $NH_4Cl$ (100 mL). The mixture was extracted with ethyl acetate (50 mL×2) and the combined organic extract was washed with saturated salt solution (20 mL), dried over anhydrous $Na_2SO_4$, filtered and dried by rotary distillation. The residue was purified by flash silica gel chromatography (petroleum ether: ethyl acetate = 1: 0 to 3: 1) to give **intermediate B 15-7** (2.20 g, 43.4% yield) as a brown solid.
$^1$H NMR (400 MHz, $CDCl_3$) $\delta$ 7.34 (d, *J* = 8.0 Hz, 1H), 7.25 (d, *J* = 8.0 Hz, 1H), 2.94 - 2.80 (m, 1H), 2.76 - 2.61 (m, 1H), 2.40 - 2.18 (m, 2H), 2.17 - 2.07 (m, 1H), 1.97 - 1.84 (m, 1H), 1.81 - 1.68 (m, 1H), 0.88 (t, *J* = 7.2 Hz, 3H)

**Step 7: synthesis of intermediate B 15**

**Intermediate B 15-7** (1.1 g, 4.54 mmol), **intermediate B 15-8** (1.51 g, 6.82 mmol), CuI (865 mg, 4.54 mmol), $K_2CO_3$ (1.26 g, 9.09 mmol) and *N,N'*-dimethylethane-1,2-diamine (801 mg, 9.09 mmol) were taken up into a microwave tube in 1,4-dioxane (10 mL). The sealed tube was heated at 100 °C for 2 hrs under microwave. The mixture was filtered and dried by rotary distillation to give a crude product, which was purified by flash silica gel chromatography (petroleum ether: ethyl acetate = 1: 0 to 1: 1) to give **intermediate B 15** (0.6g, 32.89% yield) as a white solid. $^1$H NMR (400 MHz, $CDCl_3$) $\delta$ 8.94 (s, 1H), 7.79 - 7.73 (m, 1H), 7.71 - 7.66 (m, 1H), 5.76 - 5.64 (m, 1H), 5.07 - 5.01 (m, 1H), 4.96 - 4.85 (m,

2H), 4.79 - 4.67 (m, 1H), 3.11 - 3.00 (m, 1H), 2.93 - 2.81 (m, 1H), 2.57 (s, 3H), 2.45 - 2.34 (m, 1H), 2.29 - 2.19 (m, 2H), 2.04 - 1.94 (m, 1H), 1.91 - 1.79 (m, 1H), 1.01 - 0.97 (m, 3H)

**Step 8: synthesis of intermediate B 15-A and B 15-B**

The **intermediate B 15** (1.2 g, 3.13 mmol) was separated by prep-SFC (Column: Phenomenex-Cellulose-2 (250mm*50mm, 10μm); Mobile phase: [0.1%$NH_3H_2O$/ETOH]; B%: 45%-). **Intermediate B 15-A** (460 mg, 38.3% yield) as peak **1** was obtained as a yellow solid. **Intermediate B 15-B** (460 mg, 38.3% yield) as peak **2** was obtained as a yellow solid.

**Intermediate B 15-A** optical rotation: $[a]^{20}_{D}$= -46.64, (C=0.1, $CHCl_3$)
Chiral analysis (Column: Cellulose 2 100*4.6mm I.D., 3 μm; Mobile Phase: A:$CO_2$, B: EtOH (0.05%DEA),Isocatic: A/B = 1/1 at 1.0 mL/ min; Temp: 35 °C; Wavelength: 220 nm, $R_T$ = 1.76 min)
$^1$H NMR (400 MHz, DMSO) $\delta$ 9.03 (s, 1H), 7.91 (d, *J* = 8.0 Hz, 1H), 7.70 (d, *J* = 8.0 Hz, 1H), 5.81 - 5.59 (m, 1H), 5.10 (s, 1H), 5.03 - 4.96 (m, 1H), 4.90 - 4.79 (m, 2H), 4.70 - 4.59 (m, 1H), 3.07 - 2.92 (m, 1H), 2.87 - 2.73 (m, 1H), 2.54 (s, 3H), 2.28 - 2.16 (m, 1H), 2.10 - 1.97 (m, 1H), 1.93 - 1.82 (m, 1H), 1.77 - 1.65 (m, 1H), 0.87 (t, *J* = 7.6 Hz, 3H) **Intermediate B 15-B** optical rotation: $[a]^{20}_{D}$ = +48.265, (C=0.1, $CHCl_3$)
Chiral analysis (Column: Cellulose 2 100*4.6mm I.D., 3 μm; Mobile Phase: A:$CO_2$, B: EtOH (0.05%DEA),Isocatic: A/B = 1/1 at 1.0 mL/ min; Temp: 35 °C; Wavelength: 220 nm, $R_T$ = 2.41 min)
$^1$H NMR (400 MHz, DMSO) $\delta$ 9.03 (s, 1H), 7.91 (d, *J* = 8.0 Hz, 1H), 7.70 (d, *J* = 8.0 Hz, 1H), 5.81 - 5.59 (m, 1H), 5.10 (s, 1H), 5.03 - 4.96 (m, 1H), 4.90 - 4.79 (m, 2H), 4.70 - 4.59 (m, 1H), 3.07 - 2.92 (m, 1H), 2.87 - 2.73 (m, 1H), 2.54 (s, 3H), 2.28 - 2.16 (m, 1H), 2.10 - 1.97 (m, 1H), 1.93 - 1.82 (m, 1H), 1.77 - 1.65 (m, 1H), 0.87 (t, *J* = 7.6 Hz, 3H).

**Synthesis of intermediate B 26, intermediate B 26-A and B 26-B**

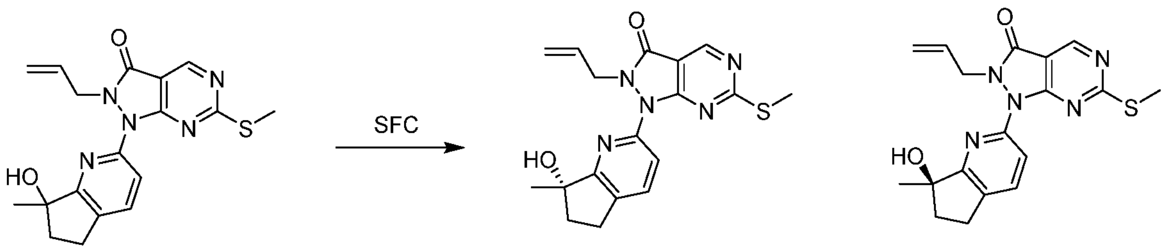


**B 26** **B 26-A** **B 26-B**

Intermediate of **B 26** was synthesis using the method of intermediate **B 15**, which through nucleophilic addition reaction by using methyl Grignard reagent instead of ethyl Grignard reagent.
Intermediate **B 26** (1.10 g, 2.98 mmol) was separated by prep-SFC (Column: Phenomenex-Cellulose-2 (250mm*50mm, 10μM); Mobile phase: [neutral-ETOH]; $CO_2$% : 45%-45%, min). Compound **B 26-A** (500 mg, 45%) as peak **1** was obtained as a yellow solid. Compound **B 26-B** (550 mg, 50%) as peak **2** was obtained as a yellow solid.

**Intermediate B 26-A** :

Chiral analysis (Column: Cellulose 2 100*4.6mm I.D., 3 μm; Mobile Phase: A:$CO_2$, B: EtOH (0.05%DEA),Isocatic: A/B = 6/4 at 1.0 mL/ min; Temp: 35 °C; Wavelength: 220 nm, $R_T$ = 2.16 min)

**Intermediate B 26-B:**

Chiral analysis (Column: Cellulose 2 100*4.6mm I.D., 3 μm; Mobile Phase: A:$CO_2$, B: EtOH (0.05%DEA),Isocatic: A/B = 6/4 at 1.0 mL/ min; Temp: 35 °C; Wavelength: 220 nm, $R_T$ = 2.68 min)
**Intermediate B 49** was synthesis using the method of **intermediate B 15** through starting material of 6,7-Dihydro-5H-quinolin-8-one.

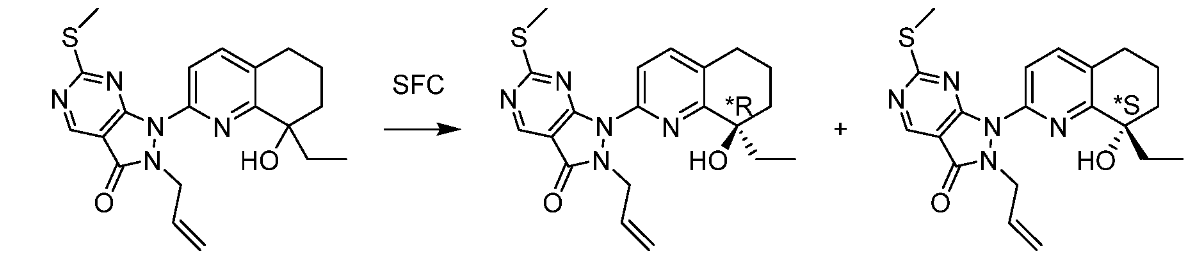


**Intermediate B 49** **Intermediate B 49-A** **Intermediate B 49-B**

**Intermediate B 49** (212 mg, 533.34 umol, 1 eq) was purified by SFC (DAICEL CHIRALPAK IG (250mm*30mm,10um);mobile phase: [Neu-MeOH];B%: 60%-60%,min). the first peak (Rt = 4.63 min) of Compound **B 49-A** (110 mg, 51.9% yield) and the second peak (Rt = 6.59 min) of compound B **49-B** (100 mg, 47.2% yield) was obtained.

**Intermediate B 50** was synthesis using the method of **intermediate B 15** through starting material of 6,7-Dihydro-5H-quinolin-8-one.

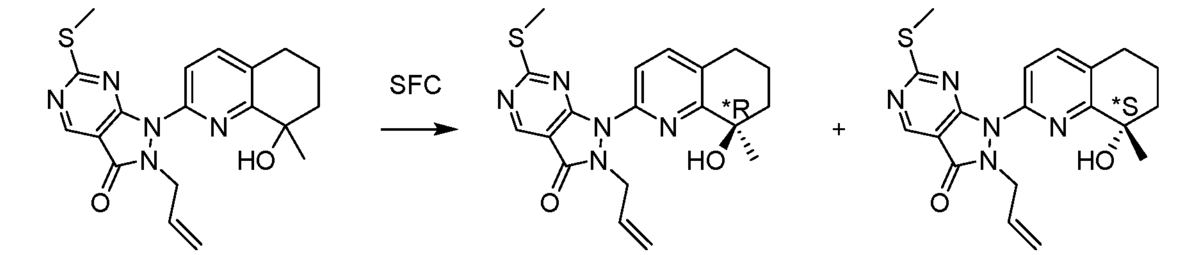


**Intermediate B 50** **Intermediate B 50-A** **Intermediate B 50-B**

**Intermediate B 50** (240 mg, 627 umol) was purified by SFC (DAICEL CHIRALCEL OD-H(250mm*30mm,5um);mobile phase: [Neu-ETOH];B%: 30%-30%,45min). the first peak (Rt = 3.76 min) of Compound **B 50-A** (120 mg, 50.0% yield) and the second peak (Rt = 3.97 min) of compound **B 50-B** (120 mg, 50.0% yield) were obtained.

The following intermediates were prepared according to the procedures described in the example of **intermediate B7-B** using intermediate **B 15-B** or intermediate **B 26-B** as starting materials.

| Intermediate No. | Structure | MS(ESI$^+$): m/z [M+H]$^+$ | $^1$H NMR (400 MHz, DMSO-$d_6$) $\delta$ |
|---|---|---|---|
| B 36 | 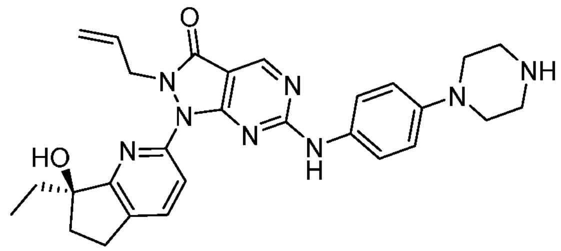 | 513.4 | |
| B 38 | 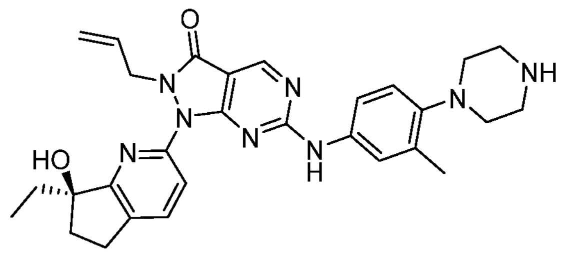 | 527.4 | |

(continued)

| Intermediate No. | Structure | MS(ESI+): m/z [M+H]+ | 1H NMR (400 MHz, DMSO-$d_6$) $\delta$ |
|---|---|---|---|
| B 40 | 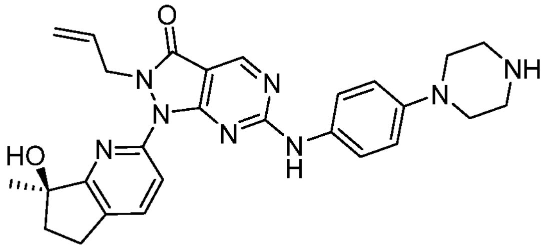 | 499.4 | |

The following intermediates can be prepared through methods described in literature or similar methods as expected .

B17, B18, B19 and B 28 were synthesized using the method from US2019/192668A1, B 20 and B 21 were synthesized using the method from WO2018/102725A1.

| Intermediate No. | Structure | MS (ESI+): m/z [M+H]+ | 1H NMR (400 MHz, DMSO-$d_6$) $\delta$ |
|---|---|---|---|
| B 16 | 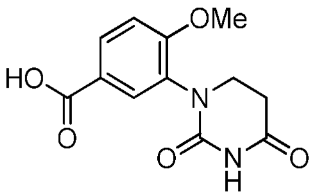 | | 12.77 (br s, 1H), 10.37 (s, 1H), 7.94 - 7.88 (m, 1H), 7.82 (d, $J$ = 2.0 Hz, 1H), 7.21 (d, $J$ = 8.4 Hz, 1H), 3.87 (s, 3H), 3.64 - 3.55 (m, 2H), 2.74 - 2.64 (m, 2H) |
| B 17 | 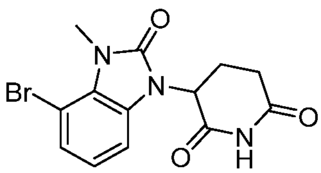 | 338.1 | 11.31 - 11.01 (m, 1H), 7.31 - 7.13 (m, 2H), 7.03 - 6.91 (m, 1H),5.52 - 5.27 (m, 1H), 3.71 - 3.59 (m, 3H), 2.95 - 2.82 (m, 1H), 2.78 - 2.68 (m, 1H), 2.60 (br s, 1H), 2.09 - 1.96 (m, 1H). |
| B 18 | 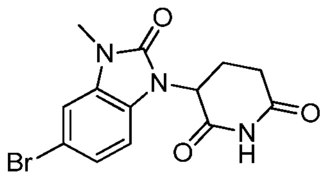 | 337.93 | 11.14 (s, 1H), 7.48 (d, $J$ = 1.6 Hz, 1H), 7.24 - 7.20 (m, 1H), 7.11 (d, $J$ = 8.0 Hz, 1H), 5.43 - 5.33 (m, 1H), 3.34 (s, 3H), 2.95 - 2.82 (m, 1H), 2.76 - 2.58 (m, 2H), 2.07 - 1.97 (m, 1H) |
| B 19 | 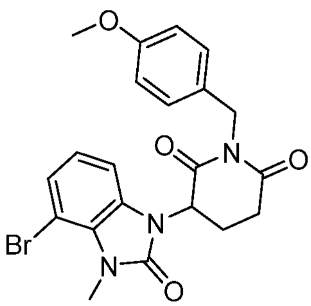 | 459.94 | 7.27 - 7.16 (m, 3H), 7.08 (d, $J$= 7.1 Hz, 1H), 6.98 - 6.91 (m,1H), 6.85 (d, $J$ = 8.6 Hz, 2H), 5.57 (dd, $J$ = 5.3, 13.0 Hz, 1H), 4.78 (q, $J$ = 14.3 Hz, 2H), 3.72 (s,3H), 3.63 (s, 3H), 3.02 (dd, $J$ = 4.5, 13.6 Hz, 1H), 2.86 - 2.66 (m, 2H), 2.11 - 2.01 (m, 1H). |

(continued)

| Intermediate No. | Structure | MS (ESI$^+$): m/z [M+H]$^+$ | $^1$H NMR (400 MHz, DMSO-$d_6$) $\delta$ |
|---|---|---|---|
| B 20 | 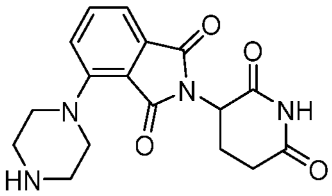 | 343.01 | |
| B 21 | 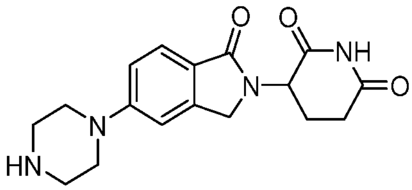 | 328.15 | |
| B 28 | 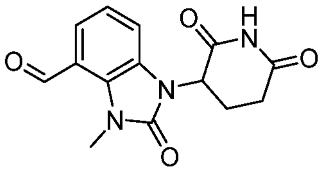 | 288.1 | |
| B 44 | 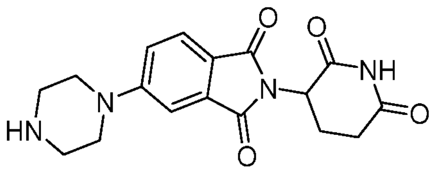 | 343.4 | |
| B 45 | 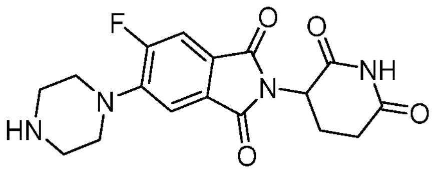 | 361.4 | |

**Synthesis of intermediate 25**

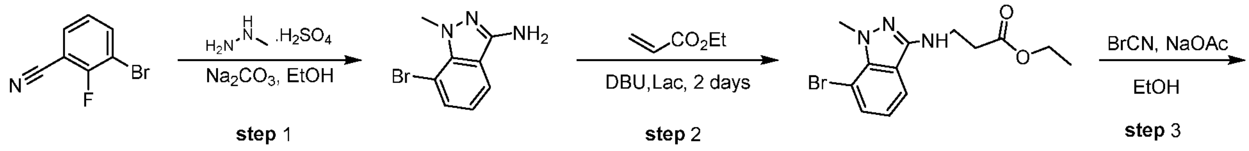


B 25-1 B 25-2 B 25-3

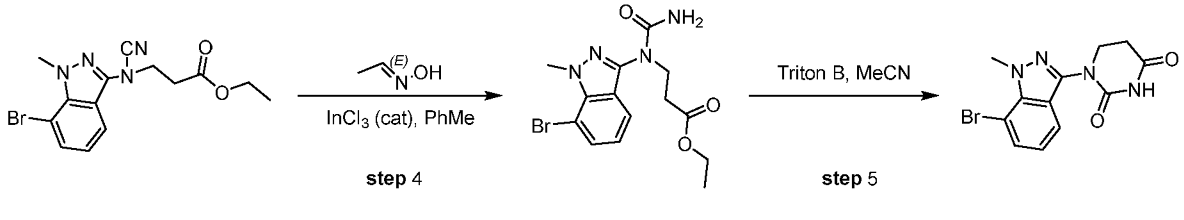


B 25-4 B 25-5 B 25

**Step 1: synthesis of intermediate B 25-2**

A mixture of intermediate **B 25-1** (25 g, 125mmol), methylhydrazine sulfuric acid salt (36.0 g, 250 mmol) and $Na_2CO_3$ (39.7 g, 375 mmol) in EtOH (100 mL) was degassed and purged with $N_2$ for 3 times, and then the mixture was stirred at 120 °C for 16 hrs under $N_2$ atmosphere. The reaction mixture was concentrated under reduced pressure to remove EtOH. The residue was diluted with $H_2O$ (200 mL) and extracted with EtOAc (300 mL) (150 mL * 2). The combined organic layers were washed with brine (200 mL) (100 mL * 2), dried over $Na_2SO_4$, filtered and concentrated under reduced pressure to give a residue. The residue was purified by column chromatography ($SiO_2$, Petroleum ether/Ethyl acetate=3/1 to 1/1) to give intermediate **B 25-2** (14.0 g, 45.6% yield) as a yellow solid. LCMS ($ESI^+$): m/z 228.9 $[M+H]^+$

**Step 2: synthesis of intermediate B 25-3**

A mixture of the DBU (9.16 g, 60.2 mmol, 9.07 mL) and Lac (9.26 g, 60.2 mmol) was stirred at 0°C for 16 hrs. Then the mixture was added intermediate **B 25-2** (17.0 g, 75.0 mmol) and stirred at 80 °C to obtained the solution. Then the ethyl prop-2-enoate (11.3 g, 113 mmol, 12.3 mL) was added in the stirring mixture and stirred at 80°C for 24 hrs. Then the extra ethyl prop-2-enoate (11.3 g, 113 mmol, 12.3 mL) was added in the stirring mixture and stirred at 80°C for 24 hrs. The reaction miture was quenched with sodium hypochlorite (30% aq, 200 mL), diluted with $H_2O$ 200 mL and extracted with EtOAc (500 mL). The combined organic extract was washed with brine (500 mL), dried over $Na_2SO_4$, filtered and concentrated under reduced pressure to give a residue. The residue was purified by column chromatography ($SiO_2$, Petroleum ether/Ethyl acetate=5/1 to 3/1) to give the intermediate **B 25-3** (8.00 g, 30.3% yield) as a yellow solid. LCMS ($ESI^+$): m/z 328.7 $[M+H]^+$.

**Step 3: synthesis of intermediate B 25-4**

To a solution of **intermediate B 25-3** (8.00 g, 24.5 mmol) in EtOH (100 mL) was added NaOAc (4.02 g, 49.1 mmol) and BrCN (8.45 g, 79.8 mmol). The mixture was stirred at 80 °C for 16 hrs. The reaction mixture was concentrated under reduced pressure to remove EtOH. The residue was diluted with $H_2O$ (100 mL) and extracted with EtOAc (100 mL × 2). The combined organic layers were washed with brine (50 mL × 2), dried over $Na_2SO_4$, filtered and concentrated under reduced pressure to give a residue. The residue was purified by column chromatography ($SiO_2$, Petroleum ether/Ethyl acetate=10/1 to 3/1) to give the intermediate **25-4** (5.80 g, 67.3% yield) as a yellow solid. LCMS ($ESI^+$): m/z 352.5 $[M+H]^+$.

**Step 4: synthesis of intermediate B 25-5**

To a solution of intermediate **B 25-4** (5.8 g, 16.5 mmol) in toluene (50 mL) was added (E)-acetaldehyde oxime (2.93 g, 49.5 mmol, 2.99 mL) and $InCl_3$ (365 mg, 1.65 mmol).The mixture was stirred at 110 °C for 1 hr. The reaction mixture was concentrated under reduced pressure to remove toluene. The residue was diluted with $H_2O$ (50 mL) and extracted with EtOAc (80 mL). The combined organic layers were washed with brine (60 mL), dried over $Na_2SO_4$, filtered and concentrated under reduced pressure to give a residue. The residue was purified by column chromatography ($SiO_2$, Petroleum ether/Ethyl acetate=3/1 to 1/1) to give intermediate **25-5** (5.60 g, 81.7% yield) as a yellow solid. LCMS ($ESI^+$):

m/z 371.8 $[M+H]^+$.

**Step 4: synthesis of intermediate B 25**

To a solution of **intermediate B 25-5** (5.6 g, 15.2 mmol) in MeCN (150 mL) was added benzyl(trimethyl)ammonium hydroxide (9.51 g, 22.8 mmol, 10.3 mL, 40% purity). The mixture was stirred at 25 °C for 0.5 hr. The reaction mixture was concentrated under reduced pressure to remove acetonitrile and give a residue. The crude product was triturated with $H_2O$ (50 mL) at 20°C for 5 min, filtered to give a residue which is intermediate **B 25** (3.55 g, 72.4% yield) as a pale yellow solid. LCMS ($ESI^+$): m/z 324.5 $[M+H]^+$.

$^1H$ NMR (400MHz, $CDCl_3$) δ 7.95 - 7.76 (m, 1H), 7.66 - 7.52 (m, 2H), 7.05 - 6.94 (m, 1H), 4.41 - 4.33 (m, 3H), 4.11 - 4.04 (m, 2H), 2.94 - 2.85 (m, 2H).

The following **intermediates B 23** and **B 51** were prepared according to the procedures described in the example of intermediate **B 25** using the different intermediates.

| Intermediate No. | Structure | MS($ESI^+$): m/z $[M+H]^+$ | $^1H$ NMR (400 MHz, DMSO-$d_6$) δ |
|---|---|---|---|
| B 23 | 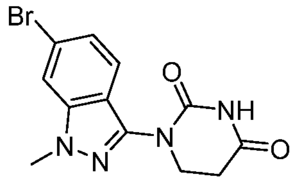 | 324.15 | |
| B 51 | 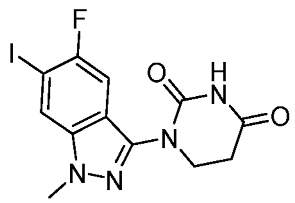 | 389.15 | 10.61 (s, 1H), 8.26 (d, *J* = 4.8 Hz, 1H), 7.50 (d, *J* = 8.8 Hz, 1H), 3.98 (s, 3H), 3.91 (t, *J* = 6.8 Hz, 2H), 2.75 (t, *J* = 6.8 Hz, 2H) |

**Synthesis of intermediate B 46**

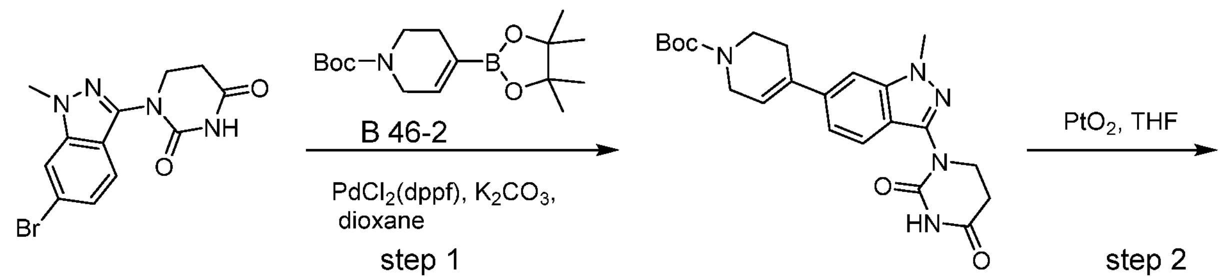


B 23

B 46-3

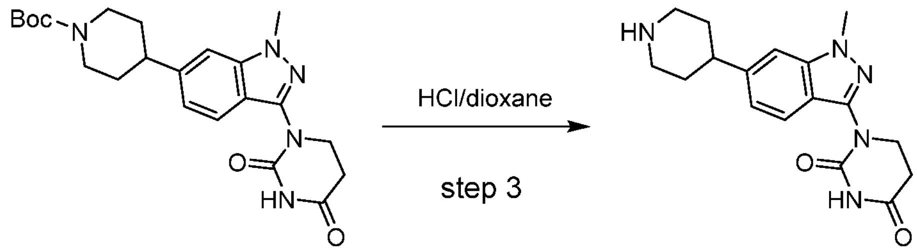


B 46-4

B 46

**Step 1: synthesis of intermediate B 46-3**

A mixture of intermediate **B 23** (250 mg, 774 μmol), **B 46-2** (287 mg, 928 μmol) , $Cs_2CO_3$ (756 mg, 2.32 mmol) and XPhos Pd G3 (131 mg, 155 μmol) in dioxane (1.5 mL) and $H_2O$ (0.06 mL) was degassed and purged with $N_2$ for 3 times, and then the mixture was stirred at 60 °C for 3 hrs under $N_2$ atmosphere. The reaction mixture was dried over $Na_2SO_4$, filtered and concentrated under reduced pressure to give a residue. The residue was purified by column chromatography ($SiO_2$, DCM: MeOH = 1:0 to 10:1) to obtained intermediate **B46-3** (285 mg, 83.12% yield). LCMS (ESI+): m/z 427.2 $[M+H]^+$.

**Step 2: synthesis of intermediate B 46-4**

To a solution of intermediate **B 46-3** (285 mg, 670 μmol) in THF (10 mL) was added $PtO_2$ (250 mg, 1.10 mmol). The mixture was degassed and purged with $H_2$ for 3 times, and then the mixture was stirred at 25 °C for 16 hr under $H_2$ atmosphere. The reaction mixture was filtered and concentrated under reduced pressure to give a residue as intermediate **B 46-4** (240 mg, crude). LCMS (ESI+): m/z 372.3 $[M+H-56]^+$.

**Step 3: synthesis of intermediate B 46**

To a solution of intermediate **B 46-4** (240 mg, 561 μmol) in DCM (2 mL) was added HCl/dioxane (4 M, 2 mL). The mixture was stirred at 25 °C for 0.5 hr. The reaction mixture was concentrated under reduced pressure to give a residue as intermediate **B 46** (265 mg, crude).

Synthesis of intermediate **B 47**

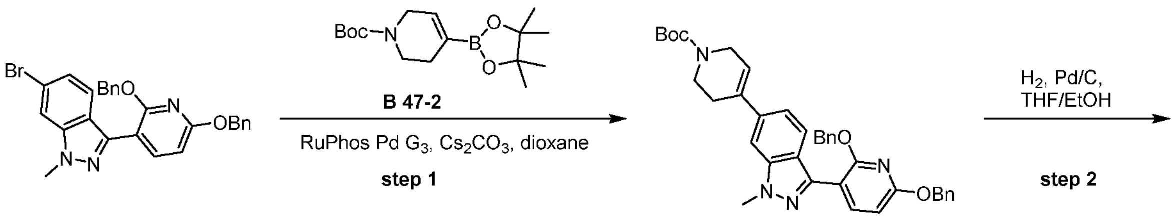


B 47-1 B 47-3

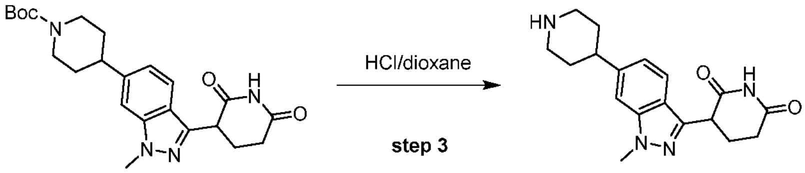


B 47-4 B 47

**Step 1: synthesis of intermediate B 47-3**

To a mixture of intermediate **B 47-1**(300 mg, 560 μmol) and intermediate **B 47-2**(222 mg, 719 μmol) in dioxane (5 mL) and $H_2O$ (0.1 mL) was added $K_2CO_3$ (249 mg, 1.80 mmol) and $Pd(dppf)Cl_2$ (26.3 mg, 36.0 μmol) in one portion at 100°C under $N_2$. The mixture was heated at 100 °C and stirred for 16 hours. The mixture was added $Na_2SO_4$, filtered and washed with DCM (100 mL). The filter liquor was concentrated in vacuo to give a residue. The residue was purified by column chromatography ($SiO_2$, Petroleum ether/Ethyl acetate=1/0 to 1/1)to give the intermediate **B 47-3** (314 mg, 69.5% yield) as colorless oil.

$^1H$ NMR (400 MHz, $CDCl_3$) δ 7.91 (d, *J* = 8 Hz, 1H), 7.67 (d, *J* = 8.8 Hz, 1H), 7.48 - 7.43 (m, 2H), 7.42 - 7.33 (m, 5H), 7.30 - 7.27 (m, 4H), 7.15 - 7.07 (m, 1H), 6.53 (d, *J* = 8 Hz, 1H), 6.14 (br s, 1H), 5.48 (s, 2H), 5.44 - 5.35 (m, 2H), 4.14 (br s, 2H), 4.12 - 4.10 (m, 3H), 3.69 (t, *J* = 5.2 Hz, 2H), 2.63 (br s, 2H), 1.52 (s, 9H).

**Step 2: synthesis of intermediate B 47-4**

To a solution of intermediate **B 47-3** (314 mg, 521 μmol) in THF (10 mL) was added $PtO_2$ (200 mg, 881 μmol) under $N_2$. The suspension was degassed under vacuum and purged with $H_2$ three times. The mixture was stirred under $H_2$ (15 PSi) at 20°C for 48 hours. The reaction mixture was filtered, washed with DCM (80 mL) and the filtrate was concentrated to the crude product. The residue was purified by column chromatography ($SiO_2$, Petroleum ether/Ethyl acetate=1: 0 to 0: 1) to give the intermediate **B 47-4** (65 mg, 25.2% yield) as colorless oil. LCMS ($ESI^+$): m/z 449.3 $[M+23]^+$.

**Step 3: synthesis of intermediate B 47**

To a solution of intermediate **B 47-4** (65 mg, 152 μmol) in DCM (1 mL) was added HCl/dioxane (4 M, 1.5 mL). The mixture was stirred at 25 °C for 5 hrs. The reaction mixture was concentrated under reduced pressure to give the crude product as intermediate **B 47** (60 mg, crude, HCl salt) as a brown solid which was directly used into the next step.

LCMS ($ESI^+$): m/z 327.2 $[M+H]^+$.

**Example 1:**

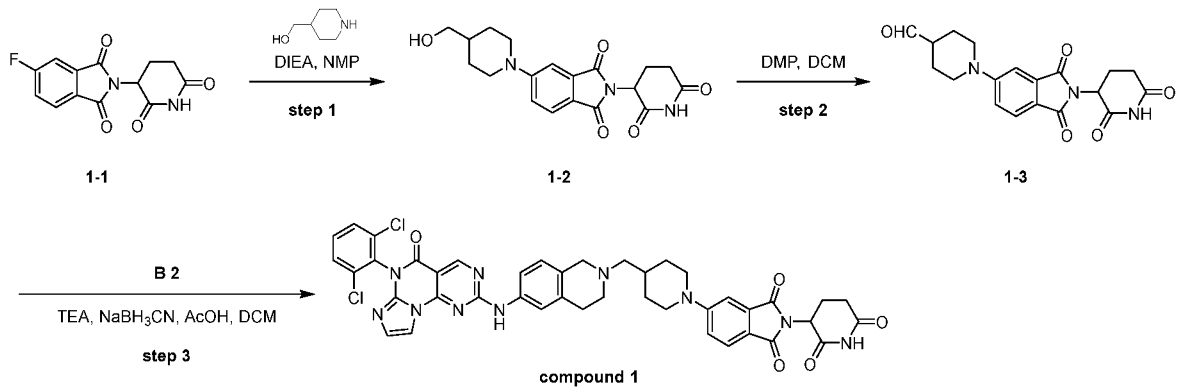


1-1 1-2 1-3

compound 1

**Step 1: synthesis of intermediate 1-2**

DIEA (702 mg, 5.43 mmol) was added to a mixture of intermediate **1-1** (500 mg, 1.81 mmol) and 4-piperidylmethanol (417 mg, 3.62 mmol) in NMP (8 mL). The reaction mixture was stirred at 140 °C under microwave irradiation for 2 hrs. The reaction mixture was quenched with $H_2O$ (20 mL) and extracted with EtOAc (50 mL×3), then separated. The combined organic layers were dried over anhydrous $Na_2SO_4$, filtered and concentrated by rotary distillation to give the residue. The residue was purified by flash silica gel chromatography (dichloromethane: methanol = 1: 0 to 10: 1) to give intermediate **1-2** (500 mg, 66.9% yield) as a yellow solid.

MS (ESI$^+$): m/z 371.8 [M+H]$^+$.

**Step 2: synthesis of intermediate 1-3**

DMP (857 mg, 2.02 mmol) was added to a solution of intermediate **1-2** (500 mg, 1.35 mmol) in DCM (20 mL). The reaction mixture was stirred at 25°C for 2 hrs, then quenched with $NaHCO_3$ (20 mL) and $Na_2SO_3$ (20 mL), extracted, then separated. The combined organic layers were dried over anhydrous $Na_2SO_4$, filtered, then dried by rotary distillation to give residue. The residue was purified by flash silica gel chromatography (dichloromethane: methanol = 1: 0 to 10: 1) to give an **intermediate 1-3** (400 mg, 79.6% yield) as a yellow solid. MS (ESI$^+$): m/z 370.0 [M+H]$^+$.

**Step 3: synthesis of compound 1**

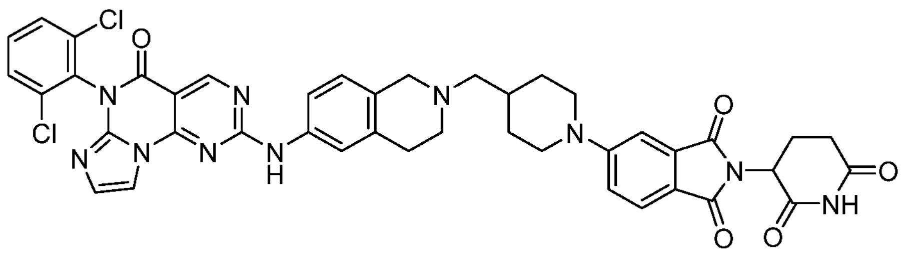

**compound 1**

**Intermediate 1-3** (46.3 mg, 125 μmol) was added to a mixture of **intermediate B-2** (30.0 mg, 62.7 μmol) and TEA (6.35 mg, 62.7 μmol) in DCM (5 mL). The mixture was stirred at 25°C for 10 min. Then AcOH (3.77 mg, 62.72 μmol) was added and the mixture was stirred at 25°C for another 50 min. Finally, $NaBH_3CN$ (7.88 mg, 125.44 μmol) was added to the above mixture and stirred for 1 h. The reaction mixture was concentrated by rotary distillation to give residue. The residue was purified by prep-TLC (DCM: MeOH = 20:1) to give **Compound 1** (3.96 mg, 7.44% yield) as a yellow solid. MS (ESI$^+$): m/z 831.1 [M+H]$^+$.

$^1$HNMR (400 MHz, DMSO-$d_6$) $\delta$ 11.10 (br s, 1H), 10.80 (br s, 1H), 9.15 (s, 1H), 8.35 - 8.29 (m, 2H), 7.85 - 7.73 (m, 3H), 7.68 - 7.62 (m, 2H), 7.61 - 7.55 (m, 1H), 7.36 - 7.30 (m, 1H), 7.28 - 7.20 (m, 1H), 7.11 (d, *J* = 1.6 Hz, 1H), 5.06 (dd, *J* = 5.2, 13.2 Hz, 1H), 4.06 (d, *J* = 13.2 Hz, 2H), 3.62 - 3.57 (m, 2H), 3.00 (t, *J* = 12.0 Hz, 2H), 2.93 - 2.82 (m, 3H), 2.63 - 2.52 (m, 3H), 2.37 - 2.34 (m, 1H), 2.04 - 1.94 (m, 2H), 1.87 - 1.81 (m, 1H), 1.27 - 1.10 (m, 3H), 1.00 - 0.92 (m, 2H).

**Compound 2** to **25** were synthesized in a similar manner to **compound 1** with various intermediates.

| Compound No. | Intermediate No. | MS(ESI+): m/z [M+H]+ | $^1$H NMR (400 MHz, DMSO-$d_6$) $\delta$ |
|---|---|---|---|
| 2 | B 3 | 820.1 | 11.08 (br s, 1H), 10.79 - 10.52 (m, 1H), 9.10 (s, 1H), 7.85 - 7.74 (m, 3H), 7.72 (m, 1H), 7.66 - 7.52 (m, 3H), 7.19 - 7.13 (m, 1H), 7.12 - 7.08 (m, 1H), 7.06 - 6.93 (m, 3H), 6.80 (s, 1H), 5.07 - 4.99 (m, 1H), 3.50 - 3.48 (m, 2H), 3.22 - 3.10 (m, 4H), 2.92 - 2.79 (m, 1H), 2.60 - 2.52 (m, 6H), 2.46 - 2.41 (m, 2H), 2.04 - 1.89 (m, 1H), 1.84 - 1.73 (m, 2H). |
| 3 | B 3 | 860.0 | 11.10 (s, 1H), 10.88 - 10.55 (m, 1H), 9.11 (s, 1H), 7.91 - 7.50 (m, 7H), 7.32 (s, 1H), 7.25 (d, *J* = 8.4 Hz, 1H), 7.11 (s, 1H), 7.08 - 6.93 (m, 2H), 5.07 (dd, *J* = 5.6, 13.2 Hz, 1H), 4.11 - 4.01 (m, 2H), 3.53 - 3.43 (m, 4H), 3.33 - 3.07 (m, 4H), 3.05 - 2.81 (m, 3H), 2.62 - 2.53 (m, 4H), 2.28 - 2.16 (m, 2H), 2.07 - 1.97 (m, 1H), 1.96 - 1.77 (m, 3H) |
| 4 | B 3 | 874.1 | 11.09 (br s, 1H), 10.79 - 10.53 (m, 1H), 9.10 (s, 1H), 7.87 - 7.52 (m, 7H), 7.31 (s, 1H), 7.24 (d, J = 8.8 Hz, 1H), 7.11 (s, 1H), 7.05 - 6.93 (m, 2H), 5.07 (dd, J = 5.2, 12.8 Hz, 1H), 4.05 (d, J = 12.4 Hz, 2H), 3.17 - 3.07 (m, 4H), 3.03 - 2.81 (m, 3H), 2.63 - 2.53 (m, 4H), 2.49 - 2.31 (m, 4H), 2.07 - 1.96 (m, 1H), 1.85 - 1.72 (m, 2H), 1.69 - 1.57 (m, 1H), 1.50 - 1.37 (m, 2H), 1.26 - 1.14 (m, 2H) |
| 5 | B 5 | 831.2 | 11.09 (s, 1H), 10.96 (br s, 1H), 9.17 (s, 1H), 7.89 (s, 1H), 7.81 - 7.60 (m, 6H), 7.60 - 7.47 (m, 1H), 7.37 - 7.27 (m, 2H), 7.18 - 7.09 (m, 1H), 5.11 - 5.02 (m, 1H), 4.71 - 4.30 (m, 1H), 4.16 - 4.06 (m, 2H), 3.15 - 2.98 (m, 5H), 2.94 - 2.81 (m, 1H), 2.64 - 2.51 (m, 5H), 2.41 - 2.26 (m, 1H), 2.13 - 1.77 (m, 3H), 1.38 - 1.13 (m, 3H) |
| 6 | B 10 | 783.3 | 11.09 (s, 1H), 10.13 (br s, 1H), 8.97 - 8.85 (m, 1H), 8.17 - 8.01 (m, 1H), 7.86 - 7.52 (m, 5H), 7.24 (d, J = 8.4 Hz, 1H), 6.84 (s, 1H), 6.75 - 6.66 (m, 1H), 5.77 - 5.58 (m, 1H), 5.38 (br s, 1H), 5.12 - 4.93 (m, 2H), 4.81 (d, J = 17.0 Hz, 1H), 4.73 - 4.64 (m, 2H), 4.57 - 4.33 (m, 2H), 4.32 - 4.17 (m, 2H), 3.98 - 3.84 (m, 2H), 3.80 - 3.60 (m, 2H), 3.14 - 2.98 (m, 3H), 2.95 - 2.79 (m, 2H), 2.69 - 2.56 (m, 3H), 2.05 - 1.95 (m, 1H), 1.46 (s, 6H). |
| 7 | B 10 | 811.3 | 11.10 (br s, 1H), 10.25 (br s, 1H), 8.87 (s, 1H), 8.03 - 7.94 (m, 1H), 7.77 (d, *J* = 8.0 Hz, 1H), 7.69 - 7.56 (m, 3H), 7.38 (d, *J* = 8.0 Hz, 1H), 7.32 (s, 1H), 7.24 (d, *J* = 8.8 Hz, 1H), 7.05 (d, *J* = 8.4 Hz, 1H), 5.75 - 5.59 (m, 1H), 5.40 (br s, 1H), 5.06 (dd, *J* = 5.2, 12.8 Hz, 1H), 4.99 (d, *J* = 10.2 Hz, 1H), 4.81 (d, *J* = 17.2 Hz, 1H), 4.69 (d, *J* = 5.2 Hz, 2H), 4.07 (d, *J* = 12.4 Hz, 2H), 3.09 - 2.90 (m, 4H), 2.88 - 2.82 (m, 1H), 2.80 - 2.74 (m, 2H), 2.69 - 2.58 (m, 3H), 2.47 - 2.31 (m, 3H), 2.04 - 1.98 (m, 1H), 1.88 - 1.81 (m, 2H), 1.46 (s, 6H), 1.27 - 1.12 (m, 3H) |
| 8 | B 7-B | 840.5 | 11.10 (s, 1H), 10.61 (s, 1H), 8.85 (s, 1H), 8.13 - 7.99 (m, 1H), 7.79 - 7.73 (m, 1H), 7.70 - 7.65 (m, 2H), 7.64 - 7.59 (m, 2H), 7.39 - 7.35 (m, 1H), 7.31 - 7.25 (m, 1H), 7.05 - 6.99 (m, 2H), 5.74 - 5.59 (m, 1H), 5.12 - 5.04 (m, 1H), 5.03 - 4.97 (m, 1H), 4.87 - 4.78 (m, 1H), 4.72 - 4.62 (m, 2H), 4.14 - 4.04 (m, 2H), 3.66 - 3.56 (m, 2H), 3.32 - 3.14 (m, 4H), 3.11 - 2.98 (m, 4H), 2.95 - 2.84 (m, 1H), 2.64 - 2.53 (m, 2H), 2.48 - 2.41 (m, 1H), 2.28 - 2.16 (m, 1H), 2.04 - 1.91 (m, 3H), 1.51 - 1.40 (m, 7H), 1.36 - 1.26 (m, 2H) |

(continued)

| Compound No. | Intermediate No. | MS(ESI$^+$): m/z [M+H]$^+$ | $^1$H NMR (400 MHz, DMSO-$d_6$) $\delta$ |
|---|---|---|---|
| 9 | B 8 | 811.5 | 11.08 (s, 1H), 10.25 (s, 1H), 8.87 (s, 1H), 8.11 - 8.02 (m, 1H), 7.78 - 7.72 (m, 1H), 7.69 - 7.61 (m, 4H), 7.26 - 7.19 (m, 2H), 6.82 - 6.76 (m, 1H), 6.68 - 6.63 (m, 1H), 5.74 - 5.61 (m, 1H), 5.35 (s, 1H), 5.09 - 5.02 (m, 1H), 5.02 - 4.96 (m, 1H), 4.86 - 4.78 (m, 1H), 4.71 - 4.65 (m, 2H), 4.18 - 4.12 (m, 2H), 3.74 - 3.67 (m, 2H), 3.07 - 3.01 (m, 1H), 3.00 - 2.95 (m, 2H), 2.91 - 2.83 (m, 1H), 2.65 - 2.58 (m, 3H), 2.46 - 2.44 (m, 2H), 2.09 - 1.98 (m, 3H), 1.76 - 1.63 (m, 4H), 1.46 (s, 6H) |
| 10 | B 7-B | 834.5 [M+Na]$^+$ | 11.08 (br s, 1H), 10.16 (br s, 1H), 8.83 (s, 1H), 8.19 - 7.97 (m, 1H), 7.80 - 7.72 (m, 1H), 7.68 - 7.55 (m, 4H), 6.94 (d, *J* = 8.8 Hz, 2H), 6.79 (s, 1H), 6.68 - 6.61 (m, 1H), 5.73 - 5.60 (m, 1H), 5.33 (s, 1H), 5.11 - 4.95 (m, 2H), 4.88 - 4.79 (m, 1H), 4.74 - 4.60 (m, 2H), 4.23 - 4.12 (m, 2H), 3.78 - 3.69 (m, 2H), 3.14 - 3.09 (m, 4H), 3.07 - 3.04 (m, 1H), 2.95 - 2.79 (m, 1H), 2.69 - 2.66 (m, 6H), 2.46 - 2.42 (m, 2H), 2.07 - 1.93 (m, 1H), 1.47 (s, 6H) |
| 11 | B 7-B | 786.5 | 11.07 (s, 1H), 10.17 (br s, 1H), 8.82 (s, 1H), 8.15 - 7.92 (m, 1H), 7.83 - 7.67 (m, 1H), 7.64 - 7.39 (m, 4H), 7.18 - 7.01 (m, 2H), 6.97 - 6.81 (m, 3H), 5.75 - 5.55 (m, 1H), 5.42 - 5.17 (m, 1H), 5.08 - 4.93 (m, 2H), 4.87 - 4.76 (m, 1H), 4.68 (d, *J* = 5.2 Hz, 2H), 3.22 - 2.99 (m, 6H), 2.94 - 2.81 (m, 1H), 2.64 - 2.53 (m, 7H), 2.41 - 2.36 (m, 1H), 2.05 - 1.94 (m, 1H), 1.46 (s, 6H) |
| 12 | B 9 | 826.6 | 11.08 (s, 1H), 10.21 (br s, 1H), 8.87 (s, 1H), 8.07 - 7.97 (m, 1H), 7.80 - 7.75 (m, 1H), 7.75 - 7.67 (m, 2H), 7.66 - 7.60 (m, 1H), 7.53 - 7.41 (m, 1H), 7.05 (d, *J* = 8.8 Hz, 1H), 6.86 - 6.79 (m, 1H), 6.73 - 6.64 (m, 1H), 5.74 - 5.60 (m, 1H), 5.37 (s, 1H), 5.11 - 4.98 (m, 2H), 4.91 - 4.78 (m, 1H), 4.77 - 4.59 (m, 2H), 4.32 - 4.23 (m, 2H), 3.96 - 3.84 (m, 2H), 3.48 - 3.46 (m, 4H), 3.23 - 3.02 (m, 5H), 3.00 - 2.80 (m, 2H), 2.68 - 2.53 (m, 3H), 2.29 (s, 3H), 2.07 - 1.96 (m, 1H), 1.47 (s, 6H) |
| 13 | B 4 | 831.1 | 11.09 (s, 1H), 10.96 - 10.68 (m, 1H), 9.16 (s, 1H), 7.94 - 7.57 (m, 7H), 7.31 (br s, 2H), 7.13 (s, 1H), 6.80 (d, *J* = 1.6 Hz, 1H), 6.71 - 6.62 (m, 1H), 5.11 - 5.02 (m, 1H), 4.16 (t, *J* = 8.0 Hz, 2H), 3.76 - 3.65 (m, 2H), 3.13 - 2.80 (m, 5H), 2.69 - 2.62 (m, 2H), 2.58 - 2.53 (m, 2H), 2.15 - 1.94 (m, 3H), 1.84 - 1.59 (m, 4H). |
| 14 | B 4 | 859.1 | 11.09 (s, 1H), 10.87 (br s, 1H), 9.15 (s, 1H), 7.94 - 7.57 (m, 7H), 7.39 - 7.19 (m, 4H), 7.12 (s, 1H), 5.07 (dd, *J* = 5.6, 13.2 Hz, 1H), 4.06 (d, *J* = 12.4 Hz, 2H), 3.14 - 2.79 (m, 6H), 2.64 - 2.55 (m, 2H), 2.28 - 1.98 (m, 4H), 1.95 - 1.53 (m, 8H), 1.21 - 1.09 (m, 2H). |
| 15 | B 4 | 873.2 | 11.10 (s, 1H), 10.98 - 10.71 (m, 1H), 9.17 (s, 1H), 8.02 - 7.50 (m, 7H), 7.47 - 7.19 (m, 4H), 7.13 (d, *J* = 1.6 Hz, 1H), 5.14 - 5.00 (m, 1H), 4.08 (d, *J* = 12.0 Hz, 2H), 3.70 - 3.39 (m, 3H), 3.19 - 2.72 (m, 7H), 2.64 - 2.55 (m, 2H), 2.13 - 1.72 (m, 7H), 1.64 (br s, 2H), 1.34 - 1.13 (m, 3H). |
| 16 | B 12-B | 850.2 | 11.10 (s, 1H), 9.89 (br s, 1H), 9.08 (br s, 1H), 8.48 (s, 1H), 7.72 - 7.62 (m, 3H), 7.59 (d, *J* = 8.8 Hz, 1H), 7.52 - 7.45 (m, 1H), 7.42 - 7.34 (m, 1H), 7.32 - 7.24 (m, 1H), 7.11 (d, *J* = 8.8 Hz, 1H), 5.07 (dd, *J* = 5.2, 12.8 Hz, 1H), 4.99 (s, 2H), 4.11 (d, *J* = 12.0 Hz, 2H), 3.56 (br s, 4H), 3.37 - 3.34 (m, 1H), 3.12 (s, 4H), 3.07 - 2.95 (m, 5H), 2.93 - 2.82 (m, 1H), 2.62 - 2.55 (m, 2H), 2.32 (s, 3H), 2.24 - 2.10 (m, 1H), 2.07 - 1.72 (m, 6H), 1.43 - 1.19 (m, 2H). |

(continued)

| Compound No. | Intermediate No. | MS(ESI⁺): m/z [M+H]⁺ | $^1$H NMR (400 MHz, DMSO-$d_6$) $\delta$ |
|---|---|---|---|
| 17 | B 13 | 799.1 | 11.10 (s, 1H), 9.74 (br s, 1H), 8.44 (s, 1H), 8.23 (s, 0.40H), 7.68 - 7.55 (m, 5H), 7.51 - 7.44 (m, 1H), 7.15 (d, *J* = 8.8 Hz, 1H), 7.02 (d, *J* = 7.2 Hz, 1H), 6.90 (d, *J* = 9.2 Hz, 2H), 6.78 (t, *J* = 5.2 Hz, 1H), 5.08 - 5.00 (m, 1H), 4.95 (s, 2H), 3.43 - 3.38 (m, 8H), 3.16 - 3.05 (m, 6H), 2.91 - 2.80 (m, 1H), 2.58 -2.53 (m, 1H), 2.42 (t, *J* = 6.4 Hz, 2H), 2.04 - 1.91 (m, 1H), 1.83 - 1.68 (m, 2H). |
| 18 | B 12-B | 822.2 | 11.08 (s, 1H), 9.79 (s, 1H), 8.47 (s, 1H), 7.59 - 7.69 (m, 4H), 8.0 (d, 1H), 7.45 - 7.52 (m, 1H), 7.15 (d, *J* = 8.8 Hz, 1H), 6.78 (s, 1H), 6.65 ( d, *J* = 8.0 Hz, 1H), 5.05 (t, *J* = 12.8 Hz, 1H), 4.97 (s, 2H) 4.15 (t, *J* = 8.0 Hz, 2H), 3.67 - 3.73 (m, 2H), 3.11 (s, 3H), 2.97 (d, *J* = 11.2 Hz, 2H), 2.89 - 2.84 (m, 1H), 2.54 - 2.55 (m, 2H), 2.42 (s, 1H), 2.28 (s, 3H) 2.07 (d, *J* = 7.6 Hz, 3H), 1.96 - 2.04 (m, 3H), 1.64 (s, 4H). |
| 19 | B 12-B | 849.4 | 11.11 (s, 1H), 10.12 (br s, 1H), 8.24 (s, 1H), 7.75 - 7.62 (m, 3H), 7.57 - 7.45 (m, 1H), 7.39 - 7.02 (m, 5H), 5.21 - 4.95 (m, 3H), 3.71 (br s, 2H), 3.20 - 3.01 (m, 7H), 2.99 - 2.80 (m, 2H), 2.65 - 2.58 (m, 2H), 2.46 - 2.43 (m, 2H), 2.38 (s, 3H), 2.27 (d, *J* = 12.0 Hz, 2H), 2.01 (d, *J* = 12.0 Hz, 5H), 1.88 (d, *J* = 12.0 Hz, 2H), 1.80 - 1.62 (m, 1H), 1.48 (d, *J* = 8.0 Hz, 2H). |
| 20 | B 14 | 780.2 | 11.08 (s, 1H), 9.82 (br s, 1H), 8.47 (s, 1H), 8.37 (br s, 1H), 7.70 - 7.59 (m, 3H), 7.52 - 7.44 (m, 2H), 7.04 (d, *J* = 8.0 Hz, 1H), 6.79 (s, 1H), 6.65 (d, *J* = 8.0 Hz, 1H), 5.08 - 5.02 (m, 1H), 4.97 (s, 2H), 4.22 - 4.09 (m, 2H), 3.78 - 3.70 (m, 2H), 3.57 (br s, 1H), 3.10 (s, 3H), 2.96 - 2.81 (m, 2H), 2.79 - 2.74 (m, 3H), 2.70 (br s, 2H), 2.64 - 2.54 (m, 4H), 2.01 (br s, 1H). |
| 21 | B 8 | 839.6 | 11.09 (s, 1H), 10.26 (br s, 1H), 8.87 (s, 1H), 8.12 - 8.01 (m, 1H), 7.75 (d, *J* = 8.0 Hz, 1H), 7.70 - 7.59 (m, 4H), 7.32 (s, 1H), 7.27 - 7.18 (m, 3H), 5.73 - 5.61 (m, 1H), 5.35 (s, 1H), 5.11 - 5.03 (m, 1H), 4.99 (d, *J* = 12.0 Hz, 1H), 4.86 - 4.77 (m, 1H), 4.68 (d, *J* = 4.0 Hz, 2H), 4.05 (d, *J* = 12.0 Hz, 2H), 3.02 - 2.92 (m, 4H), 2.90 - 2.81 (m, 1H), 2.62 2.57 (m, 1H), 2.43 (br s, 1H), 2.17 (d, *J* = 8.0 Hz, 2H), 2.05 - 1.92 (m, 3H), 1.82 (d, *J* = 12.0 Hz, 3H), 1.77 - 1.55 (m, 5H), 1.46 (s, 6H), 1.16 (d, *J* = 12.0 Hz, 2H). |
| 22 | B 10 | 813.6 | 11.07 (s, 1H), 10.43 - 10.11 (m, 1H), 8.87 (s, 1H), 8.03 - 7.94 (m, 1H), 7.77 (d, *J* = 8.0 Hz, 1H), 7.66 - 7.51 (m, 3H), 7.37 (d, *J* = 8.0 Hz, 1H), 7.17 - 7.07 (m, 1H), 7.04 (d, *J* = 8.0 Hz, 1H), 6.94 (s, 1H), 6.84 (d, *J* = 8.0 Hz, 1H), 5.71 - 5.62 (m, 1H), 5.38 - 5.30 (m, 1H), 5.08 - 4.94 (m, 2H), 4.81 (d, *J* = 16.0 Hz, 1H), 4.68 (d, *J* = 4.0 Hz, 2H), 3.50 (br s, 2H), 3.17 (d, *J* = 8.0 Hz, 1H), 2.93 - 2.80 (m, 1H), 2.74 (br s, 1H), 2.63 (br s, 1H), 2.54 - 2.52 (m, 1H), 2.47 2.44 (m, 2H), 2.05 - 1.93 (m, 2H), 1.62 - 1.56 (m, 2H), 1.49 - 1.35 (m, 11H), 1.23 (br s, 4H). |
| 23 | B 10 | 771.5 | 11.07 (br s, 1H), 10.26 (br s, 1H), 8.87 (s, 1H), 8.38 (s, 2.232H),7.97 (t, *J* = 7.6 Hz, 1H), 7.84 - 7.73 (m, 1H), 7.65 - 7.53 (m, 3H), 7.38 (d, *J* = 8.0 Hz, 1H), 7.22 (br s, 1H), 7.06 (d, *J* = 8.8 Hz, 1H), 6.97 (s, 1H), 6.87 (d, *J* = 8.0 Hz, 1H), 5.66 (d, *J* = 6.4 Hz, 1H), 5.35 (br s, 1H), 5.06 - 4.93 (m, 2H), 4.81 (d, *J* = 17.2 Hz, 1H), 4.68 (d, *J* = 4.4 Hz, 2H), 3.55 (br s, 2H), 3.50 (br s, 2H), 2.92 - 2.81 (m, 1H), 2.79 (br s, 1H), 2.67 (br s, 2H), 2.58 (br s, 3H), 2.55 - 2.53 (m, 1H), 2.43 - 2.37 (m, 1H), 1.98 (d, *J* = 8.4 Hz, 1H), 1.86 (br s, 2H), 1.45 (s, 6H). |

(continued)

| Compound No. | Intermediate No. | MS(ESI$^+$): m/z $[M+H]^+$ | $^1H$ NMR (400 MHz, DMSO-$d_6$) $\delta$ |
|---|---|---|---|
| 24 | B 10 | 785.6 | 11.08 (s, 1H), 10.40 (br s, 1H), 9.83 (br s, 1H), 8.91 (s, 1H), 8.05 (t, *J* = 7.6 Hz, 1H), 7.78 (d, *J* = 8.0 Hz, 1H), 7.70 (br s, 1H), 7.64 (d, *J* = 7.6 Hz, 1H), 7.59 (d, *J* = 8.0 Hz, 2H), 7.25 - 7.16 (m, 2H), 6.99 (s, 1H), 6.89 (d, *J* = 7.2 Hz, 1H), 5.73 - 5.61 (m, 1H), 5.38 (s, 1H), 5.08 - 4.98 (m, 2H), 4.85 - 4.78 (m, 1H), 4.69 (d, *J* = 6.0 Hz, 2H), 4.52 (d, *J* = 12.0 Hz, 1H), 4.35 (br s, 1H), 3.73 (br s, 1H), 3.30 (br s, 6H), 3.13 - 3.01 (m, 1H), 2.96 - 2.79 (m, 1H), 2.60 - 2.54 (m, 2H), 2.05 - 1.96 (m, 1H), 1.88 (br s, 1H), 1.69 (br d, *J* = 6.8 Hz, 2H), 1.46 (s, 6H). |
| 25 | B 7-B | 826.5 | 10.95 (s, 1H), 10.16 (br s, 1H), 8.82 (s, 1H), 8.28 (s, 0.47H), 8.11-7.98 (m, 1H), 7.74 (d, *J* = 0.8 Hz, 1H), 7.64 - 7.46 (m, 4H), 7.11 - 7.00 (m, 2H), 6.92 (d, *J* = 0.8 Hz, 2H), 5.73 - 5.58 (m, 1H), 5.36 (br s, 1H), 5.08 - 4.96 (m, 2H), 4.87 - 4.76 (m, 1H), 4.67 (d, *J* = 0.4 Hz, 2H), 4.36 - 4.27 (m, 1H), 4.24 - 4.14 (m, 1H), 3.88 (d, *J* = 1.2 Hz, 2H), 3.10 (br s, 4H), 2.96 2.77 (m, 3H), 2.60 (br s, 1H), 2.48 - 2.43 (m, 4H), 2.37 (d, *J* = 0.4 Hz, 1H), 2.26 - 2.15 (m, 2H), 2.01 - 1.90 (m, 1H), 1.81 (d, *J* = 1.2 Hz, 3H), 1.46 (s, 6H), 1.26 - 1.15 (m, 2H). |

**Example 26:**

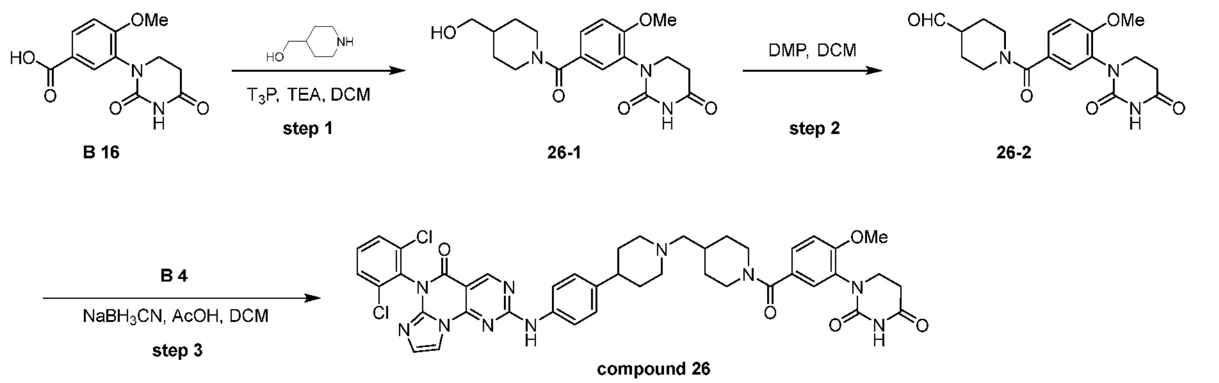


B 16

26-1

26-2

compound 26

**Step 1: synthesis of intermediate 26-1**

**Intermediate B 16** (0.65 g, 2.46 mmol), 4-piperidylmethanol (340 mg, 2.95 mmol) and TEA (747 mg, 7.38 mmol) in DCM (40 mL) were added drops of $T_3P$ (1.88 g, 2.95 mmol). The mixture was stirred at 25 °C for 3 hrs. The mixture was concentrated by rotary distillation to give a crude product. The crude product was purified by flash silica gel chromatography (dichloromethane: methanol = 1: 0 to 9: 1) to give **intermediate 26-1** (0.700 g, 78.0% yield) as a white solid.

MS (ESI$^+$): m/z 362.0 $[M+H]^+$.

**Step 2: synthesis of intermediate 26-2**

To a solution of **intermediate 26-1** (0.700 g, 1.94 mmol) in DCM (10 mL) was added DMP (1.23 g, 2.91 mmol). The mixture was stirred at 25 °C for 1.5 h. The mixture was diluted with DCM (30 mL), washed with saturated $NaHCO_3$ (10 mL $\times$ 2), dried over anhydrous $Na_2SO_4$, filtered and concentrated under reduced pressure to give a crude product, then purified by flash silica gel chromatography (dichloromethane: methanol = 1: 0 to 10: 1) to give **intermediate 26-2** (0.350 g, crude) as a white solid, which was used into the next step without further purification. MS (ESI$^+$): m/z 360.0 $[M+H]^+$.

**Step 3: synthesis of compound 26**

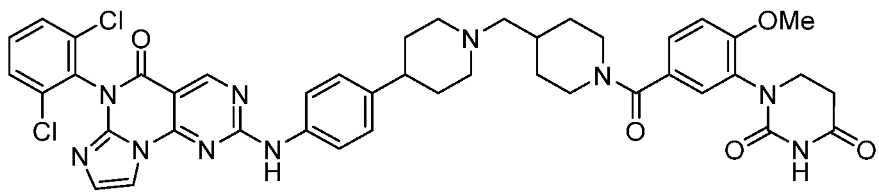

**compound 26**

To a solution of **intermediate B4** (75.0 mg, 138 μmol) in DCM (5 mL) was added TEA (76.9 μL, 553 μmol) to adjust pH to 7 ~ 8, then AcOH (31.6 μL, 553 μmol) was added to adjust pH to 5 ~ 6. **Intermediate 26-2** (75.0 mg, 138 μmol) was added to the above solution and reacted at 25 °C for 2 hours. Finally, $NaBH_3CN$ (17.4 mg, 276 μmol) was added to the mixture and reacted at 25 °C for 1h. The mixture was diluted with DCM (30 mL), washed with saturated $NaHCO_3$ (10 mL × 2). The organic extract was dried over anhydrous $Na_2SO_4$, filtered and concentrated under reduced pressure to give residue. The residue was purified by prep-HPLC (Column: Kromasil 100-5-C18 30*150 mm, Mobile Phase A: water (0.01% FA), Mobile Phase B: acetonitrile, 20 mL/min, gradient condition from 10% B to 50%B) to give **Compound 26** (29.05 mg, 23% yield) as a white solid. MS ($ESI^+$): m/z 849.2 [M+H] +_ $^1$HNMR (400 MHz, DMSO-$d_6$) $\delta$ 10.96 - 10.69 (m, 1H), 10.36 (s, 1H), 9.15 (s, 1H), 7.90 - 7.60 (m, 6H), 7.41 - 7.08 (m, 6H), 3.84 (s, 3H), 3.54 - 3.52 (m, 2H), 3.07 - 2.87 (m, 4H), 2.73 - 2.64 (m, 2H), 2.49 - 2.42 (m, 4H), 2.23 - 2.11 (m, 2H), 2.03 - 1.93 (m, 2H), 1.80 - 1.57 (m, 6H), 1.16 - 0.99 (m, 2H).

**Compound 27** to **30** were synthesized in an analogous manner to **compound 26** with various intermediates.

| Compound No. | Intermediate No. | MS($ESI^+$): m/z $[M+H]^+$ | $^1$H NMR (400 MHz, DMSO-$d_6$) $\delta$ |
|---|---|---|---|
| 27 | B 3 | 850.2 | 10.84 - 10.51 (m, 1H), 10.35 (br s, 1H), 9.10 (s, 1H), 7.85 - 7.68 (m, 4H), 7.67 - 7.51 (m, 2H), 7.40 - 7.30 (m, 2H), 7.15 (d, *J* = 8.4 Hz, 1H), 7.10 (s, 1H), 7.05 - 6.91 (m, 2H), 3.84 (s, 3H), 3.64 - 3.54 (m, 2H), 3.33 - 3.22 (m, 4H), 3.18 - 3.08 (m, 4H), 2.73 - 2.62 (m, 2H), 2.49 - 2.40 (m, 4H), 2.26 - 2.13 (m, 2H), 1.94 - 1.61 (m, 3H), 1.17 - 1.01 (m, 2H). |
| 28 | B 4 | 863.2 | 10.96 - 10.67 (m, 1H), 10.36 (s, 1H), 9.16 (s, 1H), 7.95 - 7.72 (m, 4H), 7.71 - 7.52 (m, 2H), 7.46 - 7.25 (m, 4H), 7.23 - 7.09 (m, 2H), 3.85 (s, 3H), 3.74 - 3.54 (m, 4H), 3.08 - 2.86 (m, 4H), 2.74 - 2.60 (m, 2H), 2.44 - 2.30 (m, 3H), 2.08 - 1.94 (m, 2H), 1.85 - 1.57 (m, 7H), 1.53 - 1.37 (m, 2H), 1.20 - 1.03 (m, 2H) |
| 29 | B 7-B | 866.5 $[M+Na]^+$ | 10.35 (s, 1H), 10.24 - 10.04 (m, 1H), 8.82 (s, 1H), 8.10 - 7.99 (m, 1H), 7.81 - 7.70 (m, 1H), 7.65 - 7.50 (m, 3H), 7.41 - 7.34 (m, 1H), 7.33 - 7.30 (m, 1H), 7.15 (d, *J* = 8.4 Hz, 1H), 6.92 (d, *J* = 8.8 Hz, 2H), 5.73 - 5.59 (m, 1H), 5.39 - 5.27 (m, 1H), 5.03 - 4.94 (m, 1H), 4.87 - 4.77 (m, 1H), 4.74 - 4.61 (m, 2H), 4.55 - 3.92 (m, 2H), 3.84 (s, 3H), 3.60 (t, *J* = 6.4 Hz, 2H), 3.09 (br s, 4H), 2.74 - 2.67 (m, 2H), 2.57 - 2.52 (m, 2H), 2.49 - 2.45 (m, 4H), 2.36 (t, *J* = 7.2 Hz, 2H), 1.84 - 1.53 (m, 3H), 1.49 - 1.37 (m, 8H), 1.19 - 104 (m, 2H). |
| 30 | B 4 | 881.2 $[M+Na]^+$ | 10.32 (s, 1H), 9.45 (s, 1H), 8.16 (s, 0.844 H), 7.68 (d, *J* = 8.0 Hz, 2H), 7.59 - 7.51 (m, 1H), 7.47 - 7.30 (m, 6H), 7.25 (d, *J* = 8.0 Hz, 2H), 7.15 (d, *J*= 8.4 Hz, 1H), 6.92 (s, 1H), 4.28 (s, 2H), 3.84 (s, 3H), 3.59 (d, *J* = 6.4 Hz, 4H), 3.20 - 3.14 (m, 3H), 3.04 (d, *J* = 10.8 Hz, 2H), 2.74 - 2.63 (m, 3H), 2.59 (s, 2H), 2.18 (t, *J* = 10.8 Hz, 2H), 1.84 (br s, 2H), 1.79 - 1.72 (m, 2H), 1.70 - 1.59 (m, 2H), 1.46 (br s, 2H). |

**Example 31: synthesis of compound 31:**

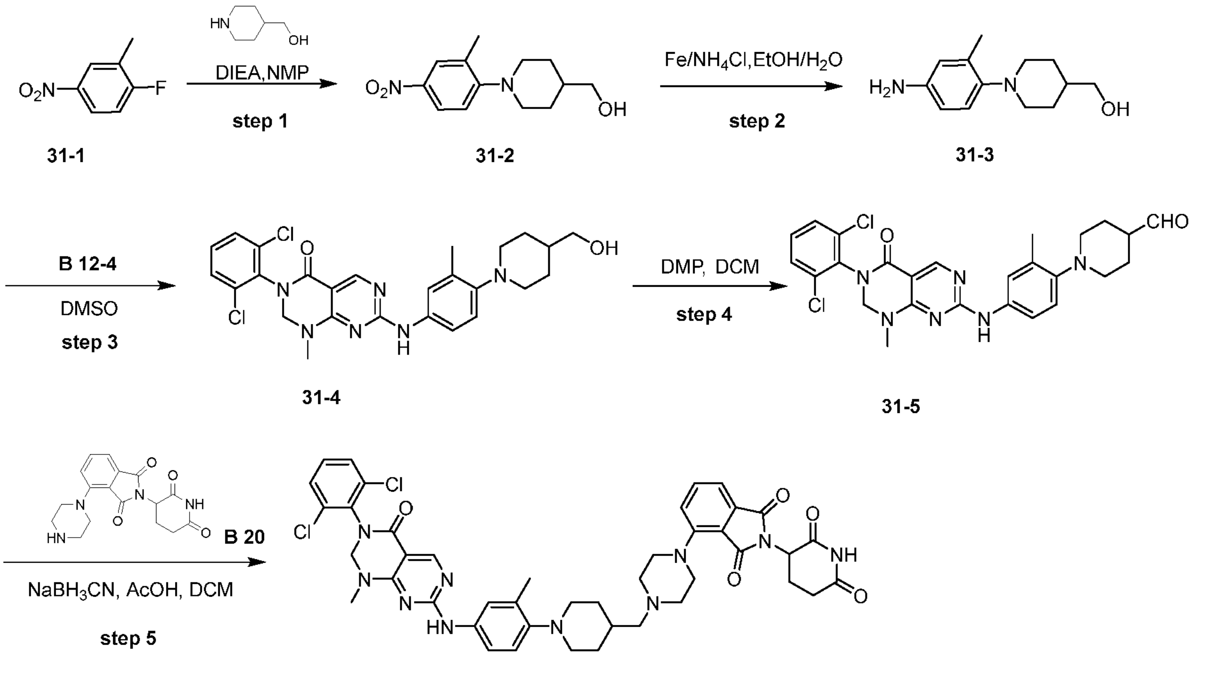


compound 31

**Step 1: synthesis of intermediate 31-2**

To a solution of **intermediate 31-1** (2.00 g, 12.9 mmol) and 4-piperidylmethanol (1.63 g, 14.2 mmol) in NMP (5 mL) was added DIEA (5.00 g, 38.7 mmol). The mixture was reacted at 140°C under microwave irradiation for 2 hrs. The reaction mixture was poured into water (50 mL), and extracted with EA (100 mL × 3). The organic layers were collected and dried over anhydrous $Na_2SO_4$, filtered and concentrated by rotary distillation to give a residue. The residue was purified by flash silica gel chromatography (petroleum ether: ethyl acetate = 1: 0 to 1: 1) to give **intermediate 31-2** (2.70 g, 74.5% yield) as a yellow solid. MS (ESI$^+$): m/z 251.12 [M+H]$^+$.

$^1$HNMR (400 MHz, DMSO-$d_6$) $\delta$ 8.06 - 7.94 (m, 2H), 7.11 (d, $J$ = 8.4 Hz, 1H), 4.54 (t, $J$ = 5.2 Hz, 1H), 3.34 - 3.29 (m, 4H), 2.32 (s, 3H), 2.21 - 2.14 (m,1H), 1.95 - 1.86 (m, 1H), 1.82 - 1.73 (m, 2H), 1.60 - 1.48 (m, 1H), 1.36 - 1.25 (m, 2H).

**Step 2: synthesis of intermediate 31-3**

To a solution of **intermediate 31-2** (1.20 g, 4.79 mmol) in EtOH (30 mL) and $H_2O$ (4 mL) was added $NH_4Cl$ (1.28 g, 24.0 mmol) and iron (1.34 g, 24.0 mmol). The mixture was stirred at 80°C for 2 hrs. Then the mixture was filtered through Celite, and extracted with EA (30 mL × 2). The organic phase was collected and washed with saturated $NaHCO_3$ (20 mL), dried over anhydrous $Na_2SO_4$, filtered and concentrated by rotary distillation to obtain **Intermediate 31-3** (0.83 g, 70.7% yield) as a black liquid.

MS (ESI$^+$): m/z 221.13 [M+H]$^+$.

**Step3: Synthesis of intermediate 31-4**

A mixture of **intermediate B 12-4** (428 mg, 1.15 mmol) and **intermediate 31-3** (305 mg, 1.38 mmol) in DMSO (3 mL) was heated to 120°C and reacted for 16 hrs. The reaction mixture was diluted with DCM (100 mL) and washed with $H_2O$ (50 mL × 2), dried over anhydrous $Na_2SO_4$, filtered and concentrated by rotary distillation to give residue. The residue was purified by flash silica gel chromatography (dichloromethane/methanol=1/0 to 10/1) to give **intermediate 31-4** (260 mg, 33.4% yield) as a yellow solid.

**Step 4: synthesis of intermediate 31-5**

To a solution of **intermediate 31-4** (260 mg, 493 μmol) in DCM (20 mL) was added DMP (314 mg, 740 μmol) slowly. The mixture was reacted at 25 °C for 1 h. The mixture was diluted with DCM (30 mL), washed with saturated $NaHCO_3$ (10 mL×2). The organic extract was dried over anhydrous $Na_2SO_4$, filtered and concentrated under reduced pressure to give a crude product. The crude product was purified by flash silica gel chromatography (dichloromethane: methanol = 1: 0 to 10: 1) to give **intermediate 31-5** (120 mg, 43.6% yield) as a white solid.

MS (ESI+): m/z 525.07 [M+H]+.

**Step 5: synthesis of compound 31**

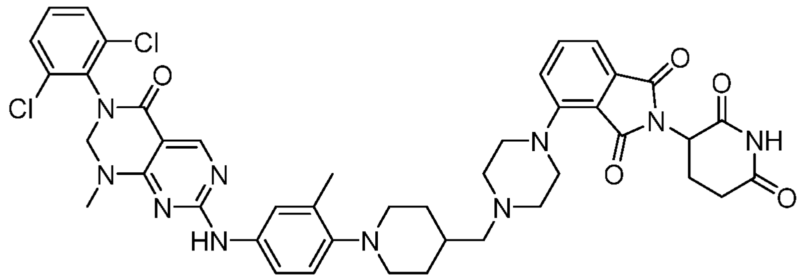

**compound 31**

To a solution of **intermediate B 20** (34.6 mg, 91.4 μmol, HCl) in DCM (5 mL) was added TEA (30.0 mg, 304 μmol) to adjust pH to 7 ~ 8. **Intermediate 31-5** (40.0 mg, 76.1 μmol) and AcOH (4.57 mg, 76.1 μmol) was added to the mixture to adjust pH to 5 ~ 6. The mixture was stirred at 25 °C for 3.5 hrs. $NaBH_3CN$ (9.57 mg, 152 μmol) was added to the mixture and it was reacted for 1.5 hrs. The mixture was concentrated by rotary distillation to gice a residue, which was purified by prep-HPLC (Column: Kromasil 100-5-C18 30*150 mm, Mobile Phase A: water (0.01% TFA), Mobile Phase B: acetonitrile, 20 mL/min, gradient condition from 10%B to 50%B) to give **Compound 31** (30.5 mg, 46.6% yield) as a yellow solid.

MS (ESI+): m/z 851.2 [M+H]+.

$^1$H NMR (400 MHz, DMSO-$d_6$) δ 11.13 (s, 1H), 10.00 - 9.79 (m, 1H), 8.47 (s, 1H), 7.81 - 7.74 (m, 1H), 7.69 - 7.54 (m, 4H), 7.53 - 7.38 (m, 3H), 7.17 - 6.95 (m, 1H), 5.17 - 5.06 (m, 1H), 4.99 (s, 2H), 3.90 - 3.87 (m, 2H), 3.74 - 3.60 (m, 2H), 3.41 - 3.20 (m, 6H), 3.12 (s, 5H), 2.95 - 2.83 (m, 1H), 2.78 - 2.55 (m, 3H), 2.45 - 2.38 (m, 1H), 2.26 (s, 3H), 2.09 - 1.95 (m, 2H), 1.93 - 1.80 (m, 2H), 1.51 - 1.37 (m, 2H).

**Compound 32** to **33** were synthesized in a similar manner to **compound 31** with various intermediates.

| Compound No. | Intermediate No. | MS(ESI+): m/z [M+H]+ | $^1$H NMR (400 MHz, DMSO-$d_6$) δ |
|---|---|---|---|
| 32 | 31-5 | 863.2 | 11.09 (s, 1H), 9.92 - 9.77 (m, 1H), 8.46 (s, 1H), 7.70 - 7.63 (m, 3H), 7.62 - 7.54 (m, 2H), 7.52 - 7.46 (m, 1H), 7.07 - 6.98 (m, 1H), 6.90 - 6.85 (m, 1H), 6.75 - 6.70 (m, 1H), 5.10 - 5.03 (m, 1H), 4.98 (s, 2H), 4.46 - 4.39 (m, 2H), 4.38 - 4.31 (m, 2H), 4.29 (s, 2H), 4.17 (s, 2H), 3.22 - 3.14 (m, 2H), 3.11 (s, 3H), 3.08 - 3.01 (m, 2H), 2.94 - 2.82 (m, 1H), 2.65 - 2.51 (m, 3H), 2.43 - 2.41 (m, 1H), 2.24 (s, 3H), 2.05 - 1.95 (m, 1H), 1.80 - 1.64 (m, 3H), 1.45 - 1.31 (m, 2H). |
| 33 | 31-5 | 850.4 | 11.14 (s, 1H), 9.74 (br s, 1H), 8.46 (s,1H), 8.00 - 7.74 (m, 3H), 7.65 (d, *J* = 8.0 Hz, 2H), 7.62 - 7.52 (m, 2H), 7.52 - 7.42 (m, 1H), 6.98 (d, *J* = 8.4 Hz, 1H), 5.22 - 5.07 (m, 1H), 4.97 (s, 2H), 3.11 (s, 3H), 3.05 - 2.96 (m, 4H), 2.91 - 2.87 (m, 1H), 2.80 - 2.77 (m, 1H), 2.23 (s, 5H), 2.11 - 1.95 (m, 4H), 1.88 - 1.77 (m, 5H), 1.76 - 1.72 (m, 1H), 1.69 (br s, 2H), 1.30 (br s, 4H). |

**Example 34: synthesis of compound 34**

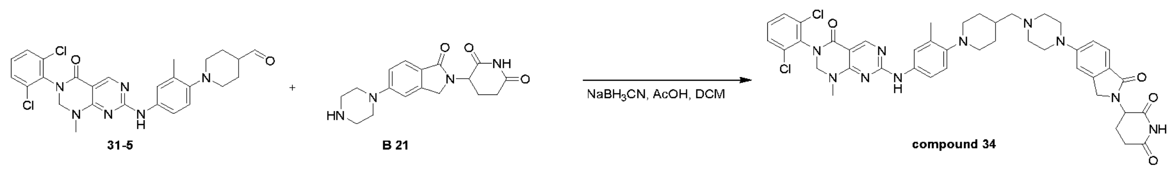


31-5 B 21 compound 34

**Step 1: synthesis of compound 34**

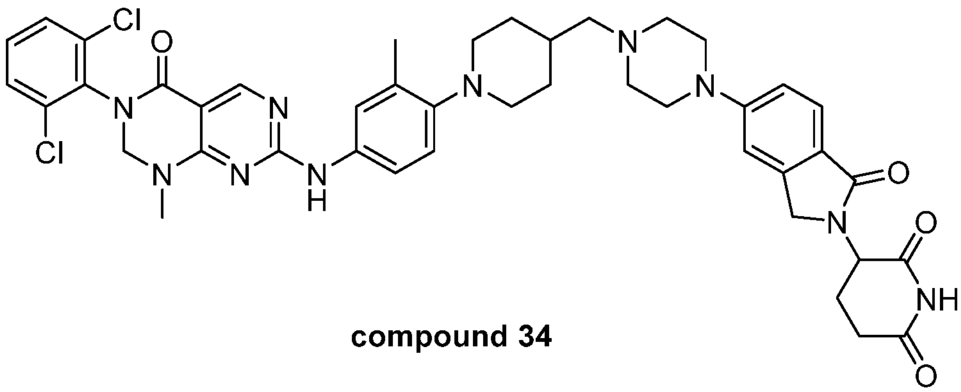

**compound 34**

To a solution of **intermediate B 21** (70.0 mg, 192 μmol, HCl) in DCM (5 mL) was added TEA (92.4 mg, 913 μmol) to adjust pH to 7 ~ 8. **Intermediate 31-5** (120 mg, 228 μmol) and AcOH (13.7 mg, 228 μmol) was added to the above mixture to adjust pH to 5 ~ 6. The mixture was stirred at 25 °C for 2 hrs. Then, $NaBH_3CN$ (21.5 mg, 342 μmol) was added to the mixture and it was stirred at 25°C for 12 hrs. The mixture was concentrated by rotary distillation. The residue was purified by prep-HPLC (Column: Kromasil 100-5-C18 30*150 mm, Mobile Phase A: water (0.01% FA), Mobile Phase B: FA, 20 mL/min, gradient condition from 10%B to 45%) to give **compound 34** (8.50 mg, 4.35% yield) as a white solid.

MS ($ESI^+$): m/z 837.2 $[M+H]^+$.

$^1H$ NMR (400 MHz, DMSO-$d_6$) $\delta$ 10.96 (br s, 1H), 9.73 (s, 1H), 8.45 (s, 1H), 7.67 - 7.61 (m, 2H), 7.59 - 7.43 (m, 4H), 7.11 - 7.03 (m, 2H), 7.00 - 6.93 (m, 1H), 5.10 - 5.00 (m, 1H), 4.96 (s, 2H), 4.39 - 4.14 (m, 2H), 3.35 - 3.30 (m, 4H), 3.10 (s, 3H), 3.04 - 2.97 (m, 2H), 2.94 - 2.84 (m, 1H), 2.64 - 2.51 (m, 7H), 2.39 - 2.31 (m, 1H), 2.28 - 2.19 (m, 5H), 2.02 - 1.90 (m, 1H), 1.87 - 1.76 (m, 2H), 1.73 - 1.58 (m, 1H), 1.33 - 1.23 (m, 2H)

The **compound 56** to **compound 57** were synthesized according to the procedures described for **Compound 34** using the different intermediates.

| Compound No. | Intermediate No. | MS($ESI^+$): m/z $[M+H]^+$ | $^1H$ NMR (400 MHz, DMSO-$d_6$) $\delta$ |
|---|---|---|---|
| 56 | 55-10 B 44 | 862.5 $[M+Na]^+$ | 11.08 (s, 1H), 10.13 (br s, 1H), 8.82 (s, 1H), 8.03 (d, *J* = 8.0 Hz, 1H), 7.75 (d, *J* = 8.0 Hz, 1H), 7.68 (d, *J* = 8.0 Hz, 1H), 7.61 (d, *J* = 8.0 Hz, 1H), 7.55 (br s, 2H), 7.34 (s, 1H), 7.26 (d, *J* = 12.0 Hz, 1H), 6.92 (d, *J* = 8.0 Hz, 2H), 5.76 - 5.58 (m, 1H), 5.32 (s, 1H), 5.11 4.95 (m, 2H), 4.82 (d, *J* = 20.0 Hz, 1H), 4.68 (d, *J* = 4.0 Hz, 2H), 3.64 (d, *J* = 12.0 Hz, 2H), 3.45 (br s, 4H), 2.94 - 2.82 (m, 1H), 2.67 - 2.57 (m, 3H), 2.54 - 2.52 (m, 2H), 2.49 - 2.45 (m, 4H), 2.23 (d, *J* = 8.0 Hz, 2H), 2.06 - 1.97 (m, 1H), 1.83 (d, *J* = 12.0 Hz, 2H), 1.76 - 1.58 (m, 2H), 1.46 (s, 6H). |

(continued)

| Compound No. | Intermediate No. | MS(ESI$^+$): m/z $[M+H]^+$ | $^1H$ NMR (400 MHz, DMSO-$d_6$) $\delta$ |
|---|---|---|---|
| 57 | 55-10 B 45 | 858.6 | 11.11 (s, 1H), 10.12 (br s, 1H), 8.82 (s, 1H), 8.03 (d, *J* = 8.0 Hz, 1H), 7.78 - 7.70 (m, 2H), 7.63 - 7.51 (m, 3H), 7.46 (d, *J* = 8.0 Hz, 1H), 6.92 (d, *J* = 8.0 Hz, 2H), 5.72 - 5.60 (m, 1H), 5.33 (s, 1H), 5.15 - 5.07 (m, 1H), 4.99 (d, *J* = 8.0 Hz, 1H), 4.82 (d, *J* = 16.0 Hz, 1H), 4.67 (d, *J* = 4.0 Hz, 2H), 3.64 (d, *J*= 12.0 Hz, 2H), 3.32 - 3.30 (m, 4H), 2.93 - 2.86 (m, 1H), 2.64 - 2.60 (m, 2H), 2.49 - 2.47 (m, 4H), 2.24 (d, *J* = 4.0 Hz, 2H), 2.11 - 1.93 (m, 2H), 1.82 (d, *J* = 12.0 Hz, 2H), 1.77 - 1.56 (m, 2H), 1.46 (s, 6H), 1.31 - 1.24 (m, 2H). |

**Example 35: compound 35**

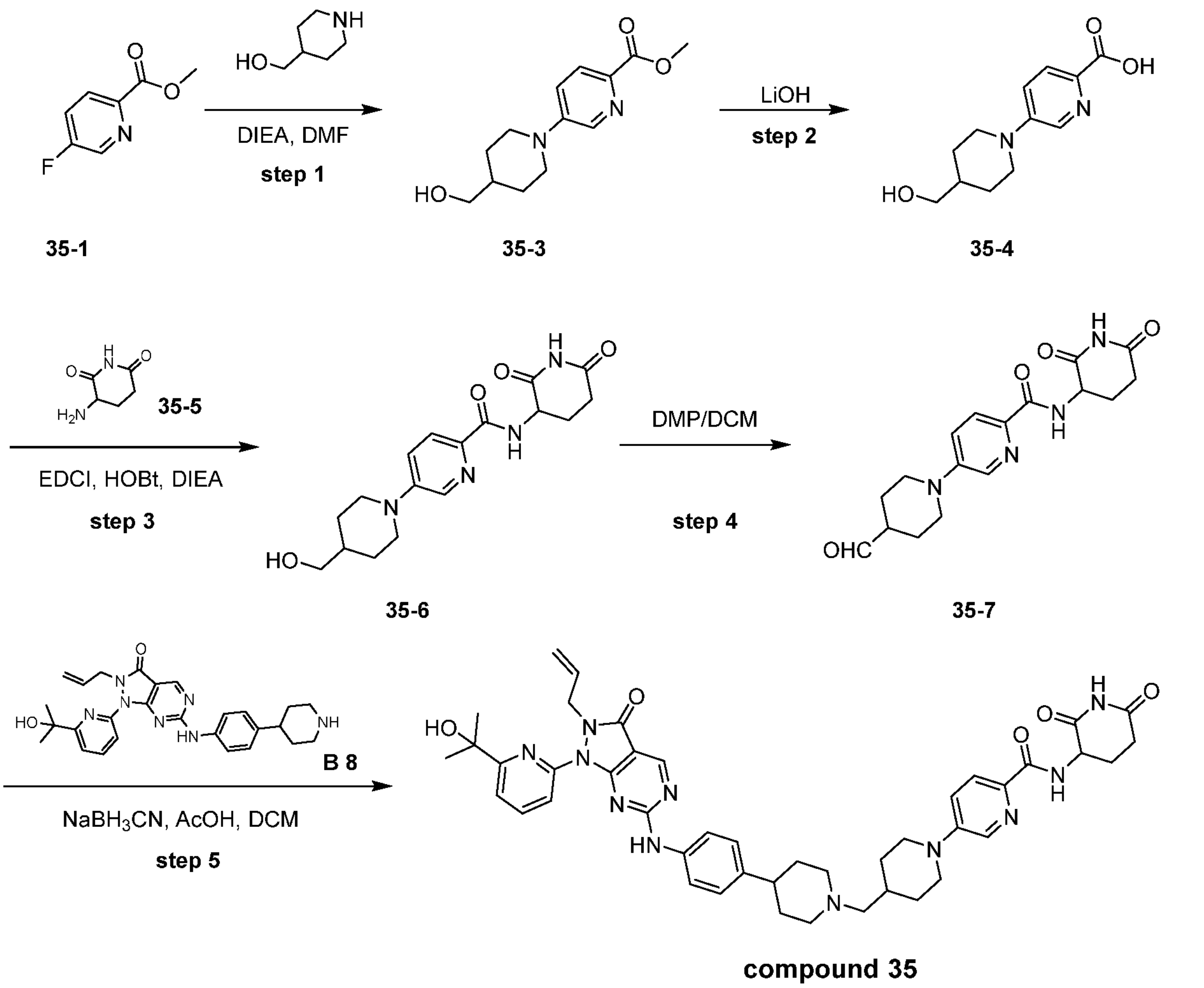


**35-1** **35-3** **35-4** **35-6** **35-7**

**compound 35**

**Step 1: synthesis of intermediate 35-3**

DIEA (6.67 g, 51.57 mmol) was added to a mixture of 4-piperidylmethanol (1.47 g, 9.67 mmol) and **intermediate 35-1** (1g, 6.45 mmol) in DMF (10 mL). The reaction mixture was stirred at 100°C for 12 hrs. The reaction mixture was quenched with saturated NaCl (50 mL) and then extracted with EA (50 mL × 3). The combined organic layers were washed with saturated salt solution (30 mL), separared, dried over anhydrous $Na_2SO_4$, filtered and concentrated by rotary distillation to give the residue. The residue was purified by flash silica gel chromatography (Petroleum ether: ethyl

acetate=1: 0 to 1: 2) to give **35-3** (1.30 g, 80.5% yield) as a white solid.

MS (ESI+): m/z 250.8 [M+H]+.

**Step 2: synthesis of intermediate 35-4**

LiOH•$H_2O$ (327 mg, 7.79 mmol) was added to a solution of **intermediate 35-3** (1.30 g, 5.19 mmol) in $H_2O$ (5 mL) and THF (5 mL). The reaction mixture was sitrred at 25°C for 2 hrs. The reaction mixture was neutralized by HCl (2 M) until pH to ~3. The mixture was concentrated by rotary distillation to give **intermediate 35-4** (1.20 g, 96.8% yield) as a white solid.

MS (ESI+): m/z 237.12 [M+H]+.

**Step 3: synthesis of intermediate 35-6**

EDCI (609 mg, 3.17 mmol) was added to a mixture of **intermediate 35-4** (500 mg, 2.12 mmol), **intermediate 35-5** (418 mg, 2.54 mmol), DIEA (1.37 g, 10.58 mmol) and HOBt (572 mg, 4.23 mmol) in DMF (10 mL). The reaction mixture was stirred at 25°C for 2 hrs. The reaction mixture was concentrated by rotary distillation to give residue. The residue was purified by prep-HPLC (Column: Kromasil 100-5-C18 30*150 mm, Mobile Phase A: water (0.01% FA), Mobile Phase B: acetonitrile, 20 mL/min, gradient condition from 25% B to 55%) to give **intermediate 35-6** (150 mg, 19.9% yield) as a white solid.

MS (ESI+): m/z 347.08 [M+H]+.

**Step 4: synthesis of intermediate 35-7**

DMP (122 mg, 289 μmol) was added to a solution of **intermediate 35-6** (50.0 mg, 144 μmol) in DCM (5 mL). The reaction mixture was stirred at 25°C for 1 h. The reaction mixture was concentrated by rotary distillation to give the residue. The residue was purified by flash silica gel chromatography (dichloromethane: methanol = 1: 0 to 10: 1) to give **intermediate 35-7** (50.0 mg, 74.4% yield) as a yellow oil.

MS (ESI+): m/z 345.08 [M+H]+.

**Step 5: synthesis of compound 35**

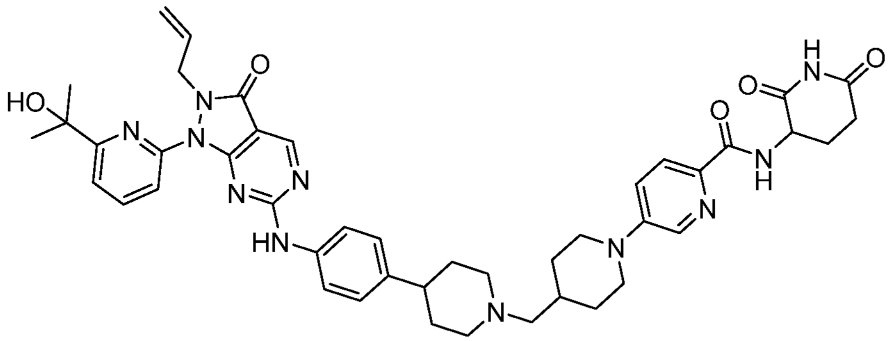

**compound 35**

AcOH (8.72 mg, 145 μmol) was added to a mixture of **intermediate 35-7** (50.0 mg, 145 μmol) and **intermediate B 8** (70.5 mg, 145 μmol) in DCM (5 mL). The mixture was stirred for 1 h. Then $NaBH_3CN$ (18.3 mg, 290 μmol) was added to the mixture and stirred at 25 °C for 1 h. The reaction mixture was quenched with $H_2O$ (10 mL) and extracted with DCM (10 mL×3), separated. The combined organic layers were dried over anhydrous $Na_2SO_4$, filtered and concentrated by rotary distillation to give the residue. The residue was purified by prep-HPLC (Column: Kromasil 100-5-C18 30× 150 mm, Mobile Phase A: water (0.01% FA), Mobile Phase B: acetonitrile, 20 mL/min, gradient condition from 10% B to 45%) to give **compound 35** (8.24 mg, 6.94% yield) as a white solid.

MS (ESI+): m/z 814.6 [M+H]+.

$^1H$ NMR (400 MHz, DMSO-$d_6$) $\delta$ 10.86 (s, 1H), 10.26 (s, 1H), 8.88 (s, 1H), 8.77 - 8.66 (m, 1H), 8.35 - 8.29 (m, 1H), 8.15 - 8.00 (m, 1H), 7.90 - 7.82 (m, 1H), 7.79 - 7.73 (m, 1H), 7.72 - 7.57 (m, 3H), 7.47 - 7.32 (m, 1H), 7.27 - 7.13 (m, 2H), 5.76 - 5.58 (m, 1H), 5.00 (d, $J$ = 10.0 Hz, 1H), 4.88 - 4.79 (m, 1H), 4.79 - 4.72 (m, 1H), 4.72 - 4.63 (m, 2H), 4.04

- 3.87 (m, 2H), 3.26 - 3.19 (m, 1H), 3.12 - 2.70 (m, 6H), 2.26 - 2.11 (m, 3H), 2.05 - 1.95 (m, 3H), 1.87 - 1.62 (m, 7H), 1.47 (s, 6H), 1.26 - 1.14 (m, 2H).

**Example 36: compound 36**

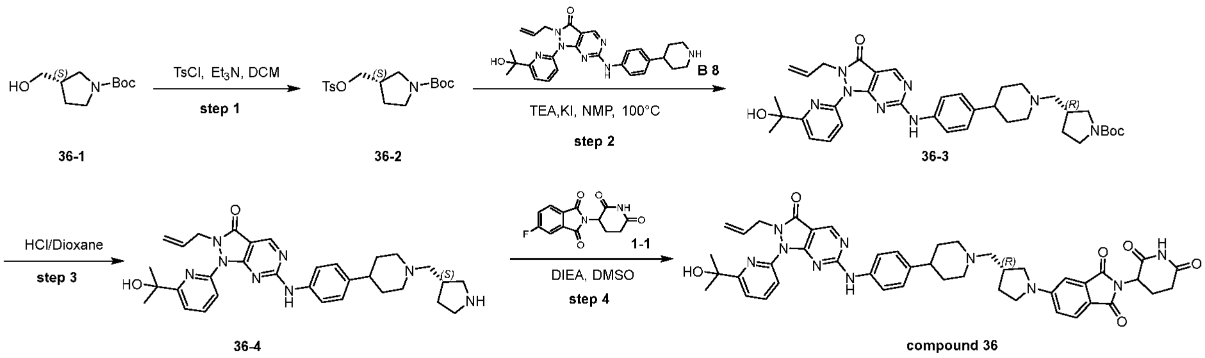


**Step 1: synthesis of intermediate 36-2**

To a solution of **intermediate 36-1** (2.00 g, 9.94 mmol) TEA (3.02 g, 29.8 mmol) in DCM (10 mL) was added 4-methylbenzenesulfonyl chloride (2.84 g, 14.9 mmol) in batches. The mixture was stirred at 25°C for 2 hrs. The mixture was concentrated by rotary distillation. The residue was purified by flash silica gel chromatography (petroleum ether: ethyl acetate = 1: 0 to 1: 1) to give **intermediate36-2** (2.78 g, 73.2% yield) as a white solid. MS (ESI$^+$): m/z 378.1 [M+Na]$^+$.

**Step 2: synthesis of intermediate 36-3**

**Intermediate B 8** (140 mg, 268 μmol, HCl), **intermediate 36-2** (143 mg, 402 μmol) and TEA (81.4 mg, 805 μmol) in ACN (2 mL) were added KI (134 mg, 805 μmol). The mixture was reacted at 100 °C for 2 hrs under microwave, then concentrated to give the residue. The residue was purified by flash silica gel chromatography (petroleum ether: ethyl acetate = 1:0 to 0:1).to give **intermediate 36-3** (150 mg, 64.4% yield) as a white solid. MS (ESI$^+$): m/z 669.5 [M+H]$^+$.

**Step 3: synthesis of intermediate 36-4**

To a mixture of **intermediate 36-3** (150 mg, 224 μmol) in DCM (2 mL) was added HCl/dioxane (4M, 4 mL) and stirred at 25 °C for 1 h. The mixture was concentrated by rotary distillation to give **intermediate 36-4** (130 mg, crude, HCl salt) as a yellow solid. MS (ESI$^+$): m/z 569.24

[M+H]$^+$.

**Step 4: Synthesis of compound 36**

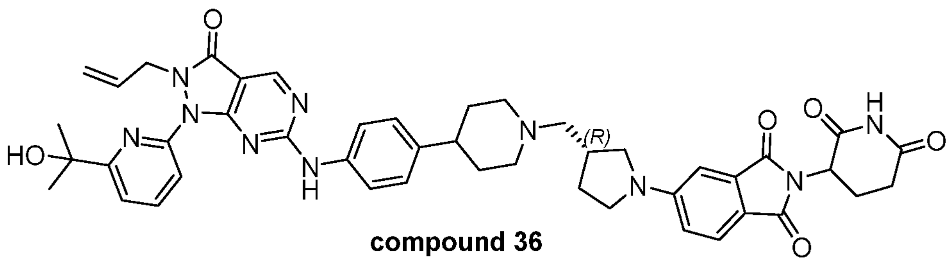

**compound 36**

**Intermediate 36-4** (65.0 mg, 107 μmol, HCl salt), DIEA (41.7 mg, 322 μmol), **intermediate 1-1** (32.6 mg, 118 μmol) in DMSO (1 mL) was reacted at 110 °C for 1 h under microwave. The mixture was concentrated by rotary distillation to give the residue. The residue was purified by prep-HPLC (Column: Kromasil 100-5-C18 30*150 mm, Mobile Phase

A: water (0.01% FA), Mobile Phase B: FA, 20 mL/min, gradient condition from 15% B to 55%B). to give **compound 36** (10.0 mg, 11.1% yield) as a yellow solid.

MS (ESI+): m/z 825.4 $[M+H]^+$.

$^1H$ NMR (400 MHz, DMSO-$d_6$) $\delta$ 11.07 (s, 1H), 10.25 (s, 1H), 8.87 (s, 1H), 8.10 - 8.00 (m, 1H), 7.83 - 7.71 (m, 1H), 7.69 - 7.56 (m, 4H), 7.25 - 7.18 (m, 2H), 6.92 - 6.89 (m, 1H), 6.86 - 6.78 (m, 1H), 5.72 - 5.61 (m, 1H), 5.34 (s, 1H), 5.09 - 5.02 (m, 1H), 5.01 - 4.96 (m, 1H), 4.85 - 4.78 (m, 1H), 4.71 - 4.64 (m, 2H), 3.61 - 3.55 (m, 1H), 3.53 - 3.47 (m, 1H), 3.19 - 3.12 (m, 1H), 3.08 - 2.97 (m, 2H), 2.92 - 2.83 (m, 1H), 2.65 - 2.58 (m, 2H), 2.55 - 2.54 (m, 1H), 2.46 - 2.35 (m, 4H), 2.21 - 2.10 (m, 1H), 2.06 - 1.95 (m, 3H), 1.82 - 1.60 (m, 5H), 1.46 (s, 6H).

## Example 37: Compound 37

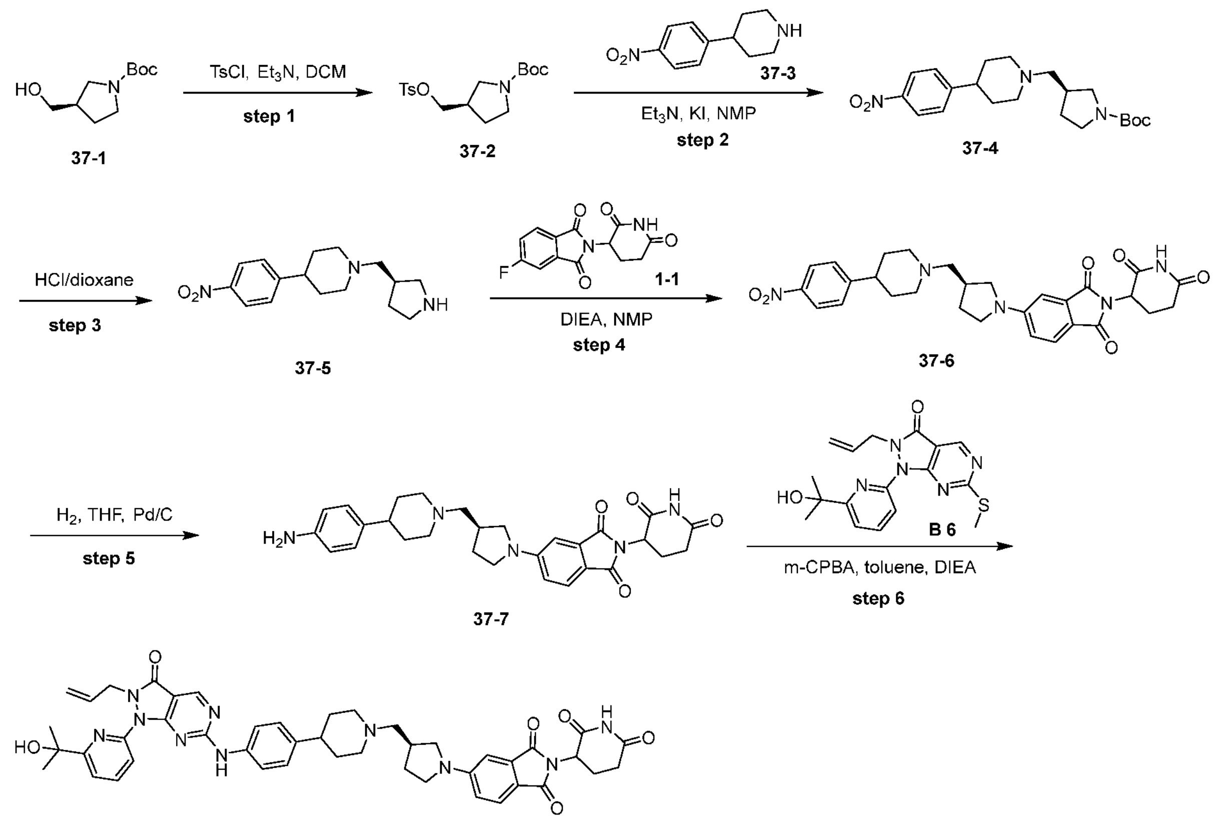


compound 37

### Step 1: Synthesis of compound 37-2

**Intermediate 37-1** (2 g, 9.94 mmol), 4-methylbenzenesulfonyl chloride (2.8g, 14.9 mmol) DMAP (364 mg, 2.98 mmol) , $Et_3N$ (2 g, 19.87 mmol, 2.8 mL) in DCM (20 mL) was reacted at 20°C for 16 hrs. The reaction mixture was concentrated by rotary distillation to give the residue. The residue was purified by column chromatography (petroleum ether/ethyl acetate=5/1 to 3/1) to give **intermediate 37-2** (4.2 g, crude) as a white solid.

MS (ESI+): m/z 355.7 $[M+H]^+$.

### Step 2: Synthesis of compound 37-4

**Intermediate 37-2** (2g, 5.63 mmol), **intermediate 37-3** (387 mg, 1.88 mmol), $Et_3N$ (1.9g, 18.76 mmol, 2.6 mL), KI (934mg, 5.63mmol) in NMP (1 mL) was reacted at 110°C for 2 hrs under microwave. The reaction mixture was diluted with $H_2O$ (80 mL) and extracted with DCM (500 mL) in total for twice. The combined organic layers were washed with NaCl (150 mL), dried over anhydrous $Na_2SO_4$, and concentrated by rotary distillation the to give the residue. The residue was purified by column chromatography (Petroleum ether/Ethyl acetate=1/0 to 3/1) to give **intermediate 37-4** (400 mg, 42.5% yield) as a yellow liquid.

MS (ESI+): m/z 390.4 [M+H]+.

**Step 3: Synthesis of intermediate 37-5**

A mixture of **intermediate 37-4** (200 mg, 513.50 μmol) in DCM (5 mL) was added HCl/dioxane (4 M, 5 mL) and reacted at 20 °C for 1 h. The reaction mixture was concentrated by rotary distillation to give the **intermediate 37-5** (300mg, crude) as a yellow solid, which was directly used into the next step.
MS (ESI+): m/z 290.1 [M+H]+.

**Step 4: Synthesis of intermediate 37-6**

A mixture of **intermediate 37-5** (200mg, 691μmol), **intermediate 1-1** (210 mg, 760 μmol), DIEA (268mg, 2.07 mmol, 361 μL) in DMSO (1 mL) was reacted at 20 °C for 2 hrs. The reaction mixture was diluted with $H_2O$ (30 mL) and extracted with EA (150 mL) in total for three times. The combined organic layers were washed with saturated salt solution (50 mL), dried over $Na_2SO_4$, filtered and concentrated by rotary distillation to give the residue. The residue was purified by column chromatography (Dichloromethane: Methanol=1/0 to 20/1) to give **intermediate 37-6** (100 mg, 26.0% yield) as a yellow solid.
MS (ESI+): m/z 546.2 [M+H]+.

**Step 5: Synthesis of intermediate 37-7**

A mixture of **intermediate 37-6** (100 mg, 183 μmol), Pd/C (300 mg, 10%) in THF (3 mL) was reacted under $H_2$ atmosphere at 20°C for 2 h. The reaction mixture was filtered and concentrated by rotary distillation to give the residue, which was purified by column chromatography (Dichloromethane: Methanol=1/0 to 20/1) to give **intermediate 37-7** (30 mg, 31.7% yield) as a yellow solid.
MS (ESI+): m/z 516.2 [M+H]+.

**Step 6: Synthesis of compound 37**

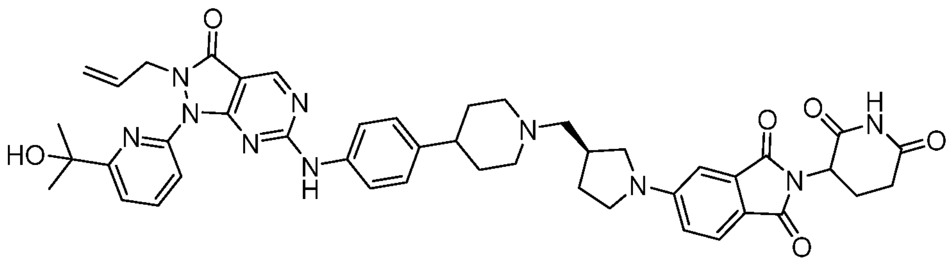

**compound 37**

To a solution of **intermediate B 6** (20 mg, 55.96 μmol) in toluene (3 mL) was added m-CPBA (18.8 mg, 87.3 μmol, 80%). After addition, the mixture was reacted at 20°C for 0.5 hr and then added **intermediate 37-7** (30 mg, 58.2 μmol) and DIEA (22.6 mg, 175 μmol, 30 μL). The mixture was reacted at 20 °C for 16 hrs. The reaction mixture was quenched by $Na_2S_2O_3$ (10 mL) and $NaHCO_3$ (10 mL), and then extracted with EA (60 mL) in total for two times. The combined organic layers were dried over anhydrous $Na_2SO_4$, filtered and concentrated by rotary distillation to give the residue. The residue was purified by prep-HPLC (acetonitrile / water (0.1%TFA)), and the crude was further purified by prep-TLC (DCM: MeOH = 10:1) to give **compound 37** (4 mg, 8.15% yield) as a yellow solid.
MS (ESI+): m/z 825.6 [M+H]+.
$^1H$ NMR (400MHz, DMSO-$d_6$) δ 11.08 (s, 1H), 10.26 (br s, 1H), 8.88 (s, 1H), 8.16 - 7.97 (m, 1H), 7.76 (d, *J* = 8.0 Hz, 1H), 7.71 - 7.59 (m, 4H), 7.23 (d, *J* = 12.0 Hz, 2H), 6.91 (s, 1H), 6.83 (d, *J* = 8.0 Hz, 1H), 5.75 - 5.60 (m, 1H), 5.35 (s, 1H), 5.11 - 4.96 (m, 2H), 4.82 (d, *J* = 16.0 Hz, 1H), 4.72 - 4.62 (m, 2H), 3.61 - 3.54 (m, 1H), 3.44 - 3.38 (m, 2H), 3.20 - 3.12 (m, 1H), 3.10 - 2.95 (m, 2H), 2.94- 2.82 (m, 1H), 2.58 (d, *J* = 20.0 Hz, 2H), 2.38 (d, *J* = 8.0 Hz, 2H), 2.16 (d, *J* = 8.0 Hz, 2H), 2.09 1.90 (m, 4H), 1.83 - 1.62 (m, 5H), 1.47 - 1.43 (s, 6H)._
**Compound 38** was synthesized in an analogous manner of **compound 37** with different intermediate.

| Compound No. | Intermediate No. | MS(ESI+): m/z [M+H]+ | $^1$H NMR (400 MHz, DMSO-$d_6$) $\delta$ |
|---|---|---|---|
| 38 | B 7-B | 826.5 | 11.02 (s, 1H), 10.22 - 9.85 (m, 1H), 8.77 (s, 1H), 7.99 (s, 1H), 7.69 (d, *J* = 8.0 Hz, 1H), 7.62 - 7.41 (m, 4H), 6.92 - 6.82 (m, 3H), 6.76 (d, *J* = 8.8 Hz, 1H), 5.73 - 5.50 (m, 1H), 5.34 - 5.23 (m, 1H), 5.10 - 4.87 (m, 2H), 4.76 (d, *J* = 17.2 Hz, 1H), 4.62 (d, *J* = 5.2 Hz, 2H), 3.53 (d, *J* = 10.4 Hz, 1H), 3.44 (d, *J* = 7.6 Hz, 1H), 3.15 - 3.10 (m, 1H), 3.07(s, 4H), 2.89 - 2.73 (m, 1H), 2.61 - 2.58 (m, 1H), 2.58 - 2.52 (m, 1H), 2.50 (s, 2H), 2.48 (s, 4H),2.36 (d, *J* = 7.6 Hz, 2H), 2.14 - 2.08 (m, 1H), 2.00 - 1.86 (m, 1H), 1.78 - 1.65 (m, 1H), 1.40 (s, 6H). |

**Example 39: compound 39**

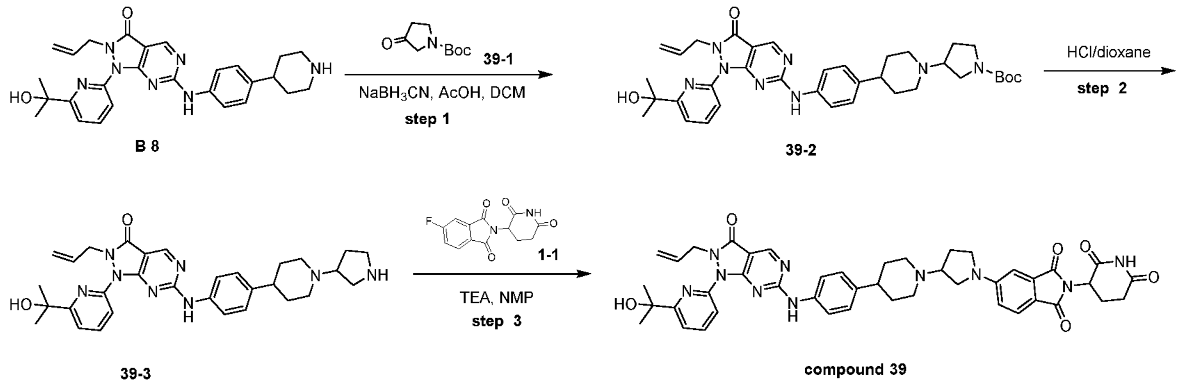

B 8, 39-2, 39-3, compound 39

**Step 1: Synthesis of compound 39-2**

To a solution **intermediate B 8** (80.0 mg, 153 μmol, HCl) in MeOH (2 mL) was added TEA (62.0 mg, 613 μmol), and pH is about 7 ~ 8. **Intermediate 39-1** (114 mg, 613 μmol) and AcOH (9.20 mg, 153 μmol) was added to the above mixture and pH is about 5 ~ 6. The mixture was stirred at 25 °C for 2 hrs. Then $NaBH_3CN$ (14.4 mg, 230 μmol) was added to the mixture and it was stirred at 25°C for 12 hrs. The mixture was quenched with $NH_4Cl$ (10 mL) and extracted with DCM (20 mL × 2). The combined organic layers were washed with saturated salt solution (20 mL), dried over anhydrous $Na_2SO_4$, filtered and concentrated by rotary distillation. The residue was purified by flash silica gel chromatography (Petroleum ether: ethyl acetate = 1:0 to 0:1) to give **intermediate 39-2** (100 mg, 97.7% yield) as a yellow solid.
MS (ESI+): m/z 655.0 [M+H]+.

**Step 2: Synthesis of intermediate 39-3**

To a solution of **intermediate 39-2** (100 mg, 153 μmol) in DCM (1 mL) was added HCl/dioxane (4 M, 2 mL). The mixture was stirred at 25°C for 1 h. The mixture was concentrated by rotary distillation to give **intermediate 39-3** (85.0 mg, crude, HCl salt) as a yellow solid.
MS (ESI+): m/z 555.21 [M+H]+.

**Step 3: Synthesis of compound 39**

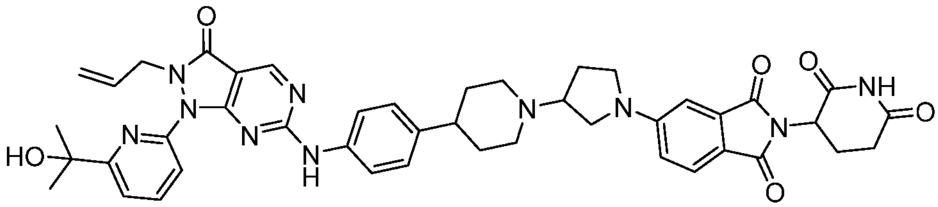

**compound 39**

**Intermediate 39-3** (85.0 mg, 144 μmol, HCl), **intermediate 1-1** (43.7 mg, 158 μmol) and TEA (43.7 mg, 431 μmol) in NMP (2 mL) were reacted at 110 °C for 1 h under microwave. The mixture was concentrated to give the residue. The residue was purified by prep-HPLC (Column: Kromasil 100-5-C18 30*150 mm, Mobile Phase A: water (0.01% FA), Mobile Phase B: ACN, 20 mL/min, gradient condition from 10% B to 50%) to give **intermediate 39** (10.0 mg, 8.25% yield) as a white solid.

MS ($ESI^+$): m/z 811.5 $[M+H]^+$.

$^1H$ NMR (400 MHz, DMSO-$d_6$) $\delta$ 11.08 (s, 1H), 10.27 (s, 1H), 8.87 (s, 1H), 8.10 - 7.99 (m, 1H), 7.79 - 7.72 (m, 1H), 7.71 - 7.59 (m, 4H), 7.27 - 7.19 (m, 2H), 6.99 - 6.96 (m, 1H), 6.89 - 6.81 (m, 1H), 5.73 - 5.61 (m, 1H), 5.38 - 5.31 (m, 1H), 5.19 - 5.04 (m, 1H), 5.02 - 4.96 (m, 1H), 4.86 - 4.78 (m, 1H), 4.73 - 4.64 (m, 2H), 3.77 - 3.69 (m, 1H), 3.62 - 3.55 (m, 1H), 3.14 - 3.04 (m, 2H), 3.03 - 2.96 (m, 1H), 2.93 - 2.84 (m, 1H), 2.60 - 2.56 (m, 1H), 2.45 - 2.41 (m, 2H), 2.32 - 2.24 (m, 2H), 2.18 - 2.04 (m, 3H), 2.03 - 1.98 (m, 1H), 1.92 - 1.84 (m, 1H), 1.79 - 1.64 (m, 4H), 1.46 (s, 6H).

**Compound 40** to **44** were synthesized in an analogous manner of **compound 39,** via various intermediates.

| Compound No. | Intermediate No. | MS($ESI^+$): m/z $[M+H]^+$ | $^1H$ NMR (400 MHz, DMSO-$d_6$) $\delta$ |
|---|---|---|---|
| 40 | B7 | 826.5 | 11.09 (s, 1H), 10.17 (s, 1H), 8.83 (s, 1H), 8.04 (d, *J* = 6.4 Hz, 1H), 7.81 - 7.51 (m, 5H), 7.43 - 7.24 (m, 2H), 6.95 (s, 2H), 5.76 - 5.58 (m, 1H), 5.36 - 5.34 (m, 1H), 5.08 (dd, *J* = 5.2, 13.2 Hz, 1H), 5.00 (d, *J* = 10.4 Hz, 1H), 4.83 (d, *J* = 17.2 Hz, 1H), 4.68 (d, *J* = 4.0 Hz, 2H), 4.31 - 4.00 (m, 2H), 3.07 - 3.25 (m, 6H), 3.00 (t, *J* = 12.4 Hz, 3H), 2.93 - 2.86 (m, 3H), 2.62 - 2.59 (m, 1H), 2.46 - 2.45 (m, 1H), 2.09 - 1.99 (m, 2H), 1.48 - 1.44 (s, 7H), 1.22 - 1.11 (m, 2H). |
| 41 | B 8 | 800.82 | 11.09 (s, 1H), 10.17 (s, 1H), 8.83 (s, 1H), 8.04 (d, *J* = 6.4 Hz, 1H), 7.81 - 7.51 (m, 5H), 7.43 - 7.24 (m, 2H), 6.95 (s, 2H), 5.76 - 5.58 (m, 1H), 5.36 - 5.34 (m, 1H), 5.08 (dd, *J* = 5.2, 13.2 Hz, 1H), 5.00 (d, *J* = 10.4 Hz, 1H), 4.83 (d, *J* = 17.2 Hz, 1H), 4.68 (d, *J* = 4.0 Hz, 2H), 4.31 - 4.00 (m, 2H), 3.07 - 3.25 (m, 6H), 3.00 (t, *J* = 12.4 Hz, 3H), 2.93 - 2.86 (m, 3H), 2.62 - 2.59 (m, 1H), 2.46 - 2.45 (m, 1H), 2.09 - 1.99 (m, 2H), 1.48 - 1.44 (s, 7H), 1.22 - 1.11 (m, 2H). |
| 42 | B 4 | 817.1 | 11.10 (br s, 1H), 10.94 - 10.67 (m, 1H), 9.16 (s, 1H), 7.91 - 7.72 (m, 5H), 7.69 - 7.61 (m, 2H), 7.39 - 7.24 (m, 2H), 7.12 (s,1H), 6.81 (s, 1H), 6.71 - 6.62 (m, 1H), 5.12 - 5.01 (m, 1H), 4.19 - 4.09 (m, 2H), 3.92 - 3.82 (m, 2H), 3.00 - 2.94 (m, 2H), 2.90 - 2.85 (m, 1H), 2.61 - 2.52 (m, 4H), 2.10 - 1.90 (m, 3H), 1.88 - 1.57 (m, 4H). |
| 43 | B 8 | 797.6 | 11.09 (s, 1H), 10.26 (br s, 1H), 8.87 (s, 1H), 8.05 (t, *J* = 7.6 Hz, 1H), 7.75 (d, *J* = 8.0 Hz, 1H), 7.70 - 7.59 (m, 4H), 7.22 (d, *J* = 8.8 Hz, 2H), 6.81 (d, *J* = 2.0 Hz, 1H), 6.71 - 6.64 (m, 1H), 5.78 - 5.60 (m, 1H), 5.36 (s, 1H), 5.10 - 5.02 (m, 1H), 4.99 (d, *J* = 10.0 Hz, 1H), 4.82 (d, *J* = 16.8 Hz, 1H), 4.68 (d, *J* = 5.6 Hz, 2H), 4.14 (t, *J* = 7.6 Hz, 2H), 3.91 - 3.81 (m, 2H), 3.00 - 2.80 (m, 3H), 2.63 - 2.53 (m, 3H), 2.45 (br s, 1H), 2.09 - 1.90 (m, 3H), 1.78 (d, *J* = 10.8 Hz, 2H), 1.73 - 1.57 (m, 2H), 1.52 - 1.38 (m, 6H) |

(continued)

| Compound No. | Intermediate No. | MS(ESI+): m/z [M+H]+ | $^1$H NMR (400 MHz, DMSO-$d_6$) $\delta$ |
|---|---|---|---|
| 44 | B 8 | 843.6 | 11.09 (s, 1H), 10.26 (br s, 1H), 8.87 (s, 1H), 8.05 (t, *J* = 7.6 Hz, 1H), 7.75 (d, *J* = 8.0 Hz, 1H), 7.70 - 7.59 (m, 4H), 7.22 (d, *J* = 8.8 Hz, 2H), 6.81 (d, *J* = 2.0 Hz, 1H), 6.71 - 6.64 (m, 1H), 5.78 - 5.60 (m, 1H), 5.36 (s, 1H), 5.10 - 5.02 (m, 1H), 4.99 (d, *J* = 10.0 Hz, 1H), 4.82 (d, *J* = 16.8 Hz, 1H), 4.68 (d, *J* = 5.6 Hz, 2H), 4.14 (t, *J* = 7.6 Hz, 2H), 3.91 - 3.81 (m, 2H), 3.00 - 2.80 (m, 3H), 2.63 - 2.53 (m, 3H), 2.45 (br s, 1H), 2.09 - 1.90 (m, 3H), 1.78 (d, *J* = 10.8 Hz, 2H), 1.73 - 1.57 (m, 2H), 1.52 - 1.38 (m, 6H). |

**Example 45: compound 45**

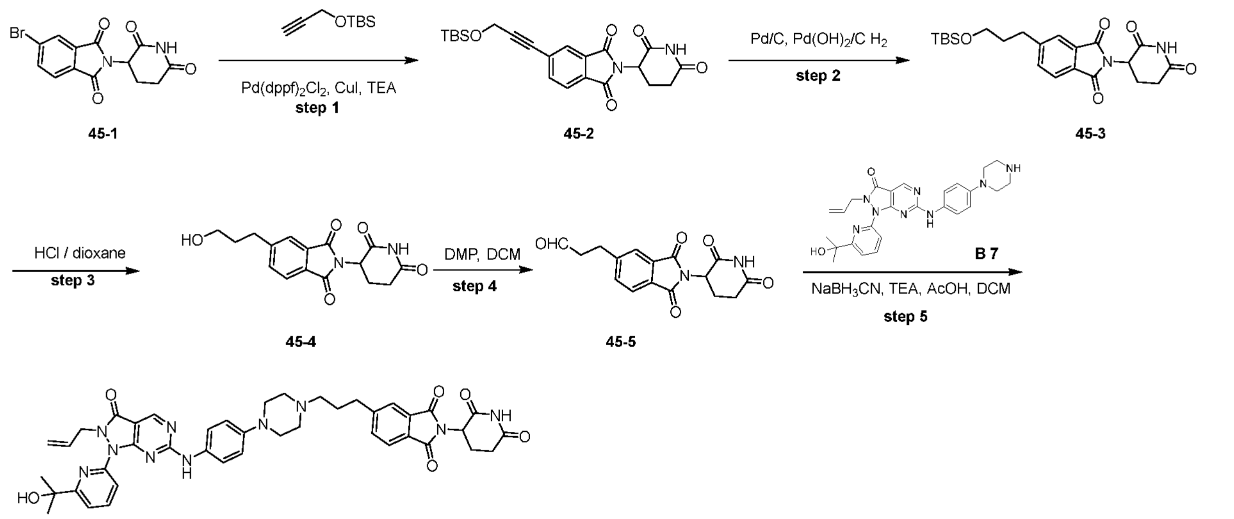


compound 45

**Step 1: synthesis of intermediate 45-2**

To a solution of **intermediate 45-1** (1.00 g, 2.97 mmol), tert-butyldimethyl(prop-2-yn-1-yloxy)silane (1.01 g, 5.93 mmol), TEA (300 mg, 2.97 mmol), CuI (113 mg, 593 $\mu$mol) in DMSO (20 mL) was stirred and degassed for 3 times, and $Pd(PPh_3)_4$ (343 mg, 297 $\mu$mol) was added, then the mixture was stirred at 85 °C for 1 h under $N_2$ atmosphere. The mixture was diluted with ethyl acetate (30 mL) and $H_2O$ (40 mL), and then extracted with EtOAc (30×2 mL). The combined organic layers were washed with saturated salt water for 2 times, dried over anhydrous $Na_2SO_4$, filtered and concentrated under reduced pressure to give the residue. The residue was purified by flash silica gel chromatography (Petroleum ether: ethyl acetate = 1: 0 to 3: 1) to give **intermediate 45-2** (1.18 g, 92.3% yield) as a brown soild.

$^1$H NMR (400 MHz, DMSO-$d_6$) $\delta$ 11.14 (s, 1H), 8.17 - 7.65 (m, 3H), 5.16 (dd, *J* = 5.2, 12.8 Hz, 1H), 4.75 - 4.38 (m, 2H), 3.02 - 2.77 (m, 1H), 2.64 - 2.55 (m, 2H), 2.14 - 2.02 (m, 1H), 0.90 (s, 9H), 0.15 (s, 6H).

**Step 2: synthesis of intermediate 45-3**

To a solution of **intermediate 45-2** (500 mg, 1.17 mmol) in THF (10 mL) was added wet Pd/C (200 mg, 606.28 $\mu$mol, 10% purity) and $Pd(OH)_2/C$ (200 mg, 142 $\mu$mol, 20% purity). The mixture was stirred at 25°C for 12 hrs under $H_2$ atmosphere. The mixture was filtered and concentrated by rotary distillation to give **intermediate 45-3** (380 mg, 75.3% yield) as a yellow oil.

$^1$H NMR (400 MHz, DMSO-$d_6$) $\delta$ 11.12 (s, 1H), 7.83 (d, *J* = 7.8 Hz, 1H), 7.76 (s, 1H), 7.70 (d, *J* = 7.8 Hz, 1H), 5.13 (s, 1H), 3.67 - 3.52 (m, 2H), 2.97 - 2.77 (m, 3H), 2.65 - 2.52 (m, 2H), 2.10 - 2.01 (m, 1H), 1.87 - 1.70 (m, 2H), 0.92 - 0.82 (m, 9H), 0.03 (s, 6H).

**Step 3: synthesis of intermediate 45-4**

To a solution of **intermediate 45-3** (200 mg, 465 μmol) in DCM (8 mL) was added HCl/dioxane (12 mL, 4M). The mixture was sirred at 25°C for 1 h. The mixture was concentrated by rotary distillation to give **intermediate 45-4** (120 mg, 81.7% yield) as a yellow soild.

MS (ESI+): m/z 317.03 $[M+H]^+$.

**Step 4: synthesis of intermediate 45-5**

To a solution of **intermediate 45-4** (120 mg, 379 μmol) in DCM (4 mL) was added DMP (241 mg, 569 μmol). The mixture was stirred at 25°C for 1 h under $N_2$ atmosphere. The mixture was added saturated $Na_2S_2O_3$ (6 ml) and extracted with DCM (8 mL). The combined organic layers were washed with saturated salt solution (10 mL×2), dried over anhydrous $Na_2SO_4$, filtered and concentrated under reduced pressure to give the residue. The residue was purified by flash silica gel chromatography (Petroleum ether: ethyl acetate = 1: 0 to 0: 1) to give **intermediate 45-5** (102 mg, 85.6% yield) as a colorless oil.

$^1H$ NMR (400 MHz, DMSO-$d_6$) δ 11.12 (s, 1H), 9.71 (s, 1H), 7.86 - 7.79 (m, 2H), 7.77 - 7.70 (m, 1H), 5.18 - 5.08 (m, 1H), 3.12 - 3.01 (m, 2H), 2.94 - 2.78 (m, 3H), 2.59 (s, 2H), 2.11 - 2.00 (m, 1H).

**Step 5: synthesis of compound 45**

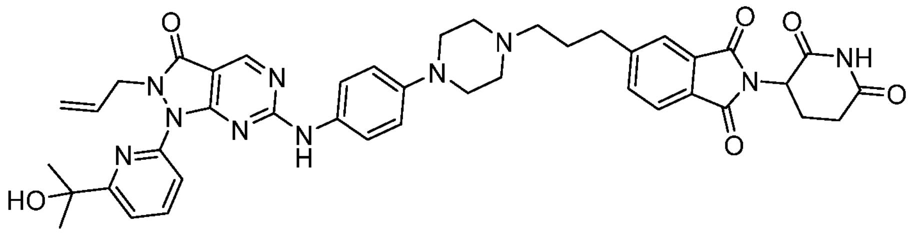

**compound 45**

To a solution of **intermediate B 7-B** (136 mg, 260 μmol, HCl) in DCM (6 mL) was added TEA (98.5 mg, 974 μmol) to adjust pH to 7 ~ 8. Then **intermediate 45-5** (102 mg, 325 μmol) and AcOH (39.0 mg, 649 μmol) was added to the above mixture to adjust pH to 5 ~ 6. The mixture was stirred at 25 °C for 12 hrs under $N_2$ atmospher. Then $NaBH_3CN$ (24.5 mg, 389 μmol) was added and the mixture was stirred at 25 °C for 1 h under $N_2$ atmospher. The mixture was diluted with DCM (16 mL) and $H_2O$ (20 mL), then extracted with DCM (30 mL×2) and the combined organic layers were washed with saturated salt solution (10 mL×2), dried over anhydrous $Na_2SO_4$, filtered and concentrated under reduced pressure to give the residue. The residue was purified by prep-HPLC (Column: Kromasil 100-5-C18 30*150 mm, Mobile Phase A: water (0.01% FA), Mobile Phase B: acetonitrile, 20 mL/min, gradient condition from 40% B to 60%) to give **intermediate 45** (36.0 mg, 13.4% yield) as a white soild.

MS (ESI+): m/z 785.5$[M+H]^+$.

$^1H$ NMR (400 MHz, DMSO-$d_6$) δ 11.15 (s, 1H), 10.65 - 9.97 (m, 1H), 8.85 (s, 1H), 8.11 - 7.99 (m, 1H), 7.94 - 7.88 (m, 2H), 7.86 - 7.42 (m, 5H), 7.01 (d, *J* = 8.8 Hz, 2H), 5.75 - 5.56 (m, 1H), 5.16 (dd, *J* = 5.6, 12.8 Hz, 1H), 5.05 - 4.95 (m, 1H), 4.88 - 4.76 (m, 1H), 4.73 - 4.60 (m, 2H), 3.83 - 3.72 (m, 2H), 3.66- 3.52 (m, 2H), 3.19 - 3.00 (m, 6H), 2.97 - 2.80 (m, 3H), 2.65 - 2.53 (m, 2H), 2.22 - 2.02 (m, 3H), 1.52 - 1.40 (m, 6H)

**Compound 46** to **51** were synthesized in an analogous manner of **compound 45,** via various intermediates.

| Compound No. | Intermediate No. | MS(ESI+): m/z $[M+H]^+$ | $^1H$ NMR (400 MHz, DMSO-$d_6$) δ |
|---|---|---|---|
| 46 | B 7-B | 786.6 | 11.18 (s, 1H), 10.84 (s, 1H), 8.91 (s, 1H), 8.11 (t, *J* = 7.6 Hz, 1H), 7.81 (d, *J* = 8.0 Hz, 1H), 7.68 (d, *J* = 7.6 Hz, 3H), 7.23 - 6.97 (m, 5H), 5.79 - 5.67 (m, 1H), 5.49 - 5.38 (m, 1H), 5.06 (d, *J* = 10.0 Hz, 1H), 4.89 (d, *J* = 17.2 Hz, 1H), 4.75 (d, *J* = 5.6 Hz, 2H), 3.79 - 3.72 (m, 2H), 3.64 - 3.63 (m, 2H), 3.42 (s, 3H), 3.24 - 3.12 (m, 6H), 3.02 - 2.93 (m, 1H), 2.85 - 2.70 (m, 4H), 2.23 - 2.12 (m, 2H), 2.11 - 2.03 (m, 1H), 1.53 (s, 6H). |

(continued)

| Compound No. | Intermediate No. | MS(ESI$^+$): m/z [M+H]$^+$ | $^1$H NMR (400 MHz, DMSO-$d_6$) $\delta$ |
|---|---|---|---|
| 47 | B 7-B | 800.6 | 11.09 (s, 1H), 10.15 (s, 1H), 8.83 (s, 1H), 8.04 (d, *J* = 8.0 Hz, 1H), 7.75 (d, *J* = 8.0 Hz, 1H), 7.65 - 7.50 (m, 3H), 7.09 - 6.98 (m, 2H), 6.95 - 6.86 (m, 3H), 5.72 - 5.58 (m, 1H), 5.39 - 5.28 (m, 2H), 5.00 (d, *J* = 10.4 Hz, 1H), 4.83 (d, *J* = 17.2 Hz, 1H), 4.68 (d, *J* = 5.2 Hz, 2H), 3.47 - 3.43 (m, 3H), 3.11 - 3.02 (m, 6H), 2.95 - 2.85 (m, 1H), 2.76 - 2.69 (m, 1H), 2.69 - 2.66 (m, 1H), 2.64 - 2.59 (m, 1H), 2.48 - 2.40 (m, 4H), 2.38 - 2.35 (m, 1H), 2.05 - 1.98 (m, 1H), 1.68 - 1.58 (m, 2H), 1.55 - 1.48 (m, 2H), 1.47 (s, 6H). |
| 48 | B 7-B | 786.6 | 11.10 (s, 1H), 10.17 (br s, 1H), 8.82 (s, 1H), 8.31 (s, 0.649 H), 8.05 (br s, 1H), 7.75 (d, *J* = 8.0 Hz, 1H), 7.60 (d, *J* = 7.6 Hz, 3H), 7.05 - 6.80 (m, 5H), 5.72 - 5.56 (m, 1H), 5.44 - 5.24 (m, 2H), 4.99 (d, *J* = 10.8 Hz, 1H), 4.82 (d, *J* = 18.0 Hz, 1H), 4.68 (m, *J* = 5.2 Hz, 2H), 3.58 (s, 3H), 3.09 (br s, 6H), 3.00 - 2.90 (m, 3H), 2.90 - 2.82 (m, 1H), 2.62 (br s, 1H), 2.43 - 2.39 (m, 4H), 2.04 - 1.91 (m, 1H), 1.88 - 1.74 (m, 2H), 1.46 (s, 6H). |
| 49 | B 10 | 756.5 | 10.99 (s, 1H), 10.23 (br s, 1H), 8.87 (s, 1H), 8.02 - 7.92 (m, 1H), 7.76 (d, *J* = 8.0 Hz, 1H), 7.64 - 7.53 (m, 3H), 7.51 - 7.42 (m, 2H), 7.41 - 7.33 (m, 1H), 7.03 (d, *J* = 8.4 Hz, 1H), 5.73 - 5.59 (m, 1H), 5.35 (s, 1H), 5.12 - 5.05 (m, 1H), 4.99 (d, *J* = 10.0 Hz, 1H), 4.81 (d, *J* = 17.2 Hz, 1H), 4.72 - 4.64 (m, 2H), 4.50 - 4.26 (m, 2H), 3.45 - 3.42 (m, 2H), 2.98 - 2.83 (m, 1H), 2.78 - 2.68 (m, 6H), 2.01 - 1.88 (m, 1H), 1.71 - 1.58 (m, 4H), 1.45 (s, 6H), 1.34 (d, *J* = 6.0 Hz, 1H), 1.27 - 1.19 (m, 3H) |
| 50 | B 7 | 785.5 | 11.01 (s, 1H), 10.30 - 10.04 (m, 1H), 8.82 (s, 1H), 8.14 - 7.97 (m, 1H), 7.88 - 7.70 (m, 1H), 7.66 - 7.52 (m, 4H), 7.49 - 7.42 (m, 2H), 7.00 - 6.83 (m, 2H), 5.79 - 5.57 (m, 1H), 5.39 - 5.29 (m, 1H), 5.22 - 5.06 (m, 1H), 5.05 - 4.92 (m, 1H), 4.88 - 4.75 (m, 1H), 4.73 - 4.63 (m, 2H), 4.54 - 4.26 (m, 2H), 3.12 - 3.01 (m, 4H), 2.96 - 2.90 (m, 1H), 2.72 - 2.68 (m, 2H), 2.64 - 2.61 (m, 2H), 2.57 - 2.55 (m, 4H), 2.41 - 2.34 (m, 2H), 2.06 - 1.96 (m, 1H), 1.71 - 1.59 (m, 2H), 1.56 - 1.50 (m, 2H), 1.46 (s, 6H). |
| 51 | B 7 | 822.5 [M+Na]$^+$ | 11.09 (s, 1H), 10.29 - 9.89 (m, 1H), 8.86 (s, 1H), 8.29 - 8.20 (m,0.621H), 8.12 - 7.96 (m, 1H), 7.75 (d, *J* = 8.0 Hz, 1H), 7.66 - 7.40 (m, 3H), 7.01 - 6.79 (m, 5H), 5.73 - 5.60 (m, 1H), 5.44 - 5.26 (m, 2H), 4.99 (d, *J* = 10.0 Hz, 1H), 4.87 - 4.78 (m, 1H), 4.74 - 4.62 (m, 2H), 3.58 (s, 3H), 3.09 (br s, 4H), 2.94 - 2.84 (m, 3H), 2.76 - 2.71 (m, 1H), 2.66 - 2.59 (m, 4H), 2.46 - 2.44 (m, 1H), 2.41 - 2.36 (m, 2H), 2.02 - 1.93 (m, 1H), 1.72 - 1.57 (m, 4H), 1.46(s, 6H). |

**Example 52: compound 52**

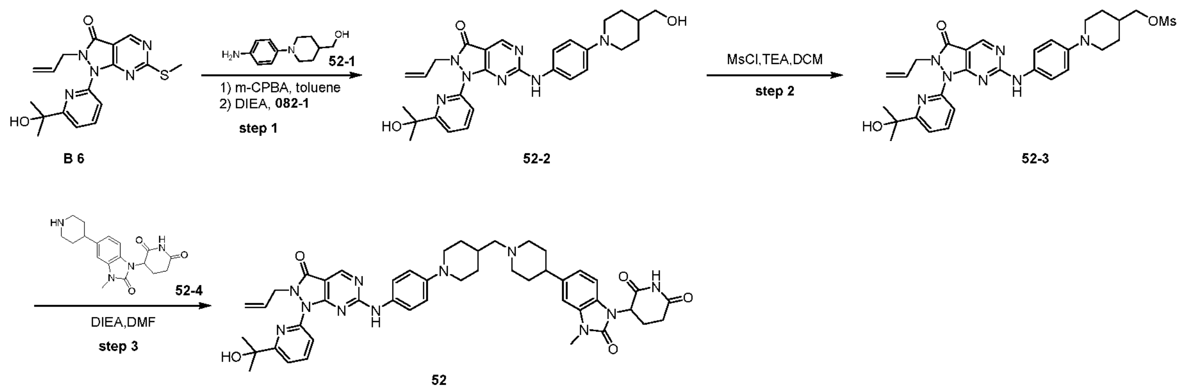


B 6 52-2 52-3

52

### Step 1: Synthesis of intermediate 52-2

To a solution of **intermediate B 6** (200 mg, 560 μmol) in toluene (3 mL) was added m-CPBA (136 mg, 671 μmol, 85% purity). The mixture was stirred at 25°C for 1 h. **Intermediate 52-1** (139 mg, 671 μmol) and DIEA (289 mg, 2.24 mmol) was added. The mixture was stirred at room tempreture for 3 hrs. The reaction mixture was quenched by $Na_2S_2O_3$ (10 mL, 1M), and then extracted with EtOAc (10 mL × 3). The combined organic layers were washed with saturated salt solution (10 mL × 2), dried over anhydrous $Na_2SO_4$, filtered and concentrated by rotary distillation to give the residue. The residue was purified by column chromatography (Petroleum ether:ethyl acetate = 1:0 to 1:1) to give **intermediate 52-2** (230 mg, 71.8% yield) as a yellow solid.

MS (ESI$^+$): m/z 516.19 [M+H]$^+$.

$^1$H NMR (400 MHz, DMSO-$d_6$) $\delta$ 10.15 (s, 1H), 8.82 (s, 1H), 8.15 - 7.97 (m, 1H), 7.82 - 7.70 (m, 1H), 7.63 - 7.47 (m, 3H), 7.09 - 6.83 (m, 2H), 5.74 - 5.53 (m, 1H), 5.36 (s, 1H), 5.08 - 4.93 (m, 1H), 4.88 - 4.77 (m, 1H), 4.74 - 4.59 (m, 2H), 4.57 - 4.48 (m, 1H), 3.73 - 3.56 (m, 2H), 3.31 - 3.29 (m, 2H), 2.63 - 2.58 (m, 2H), 1.80 - 1.70 (m, 2H), 1.50 - 1.42 (m, 7H), 1.29 - 1.21 (m, 2H).

### Step 2: Synthesis of intermediate 52-3

MsCl (190 mg, 1.66 mmol) was added to a solution of **intermediate 52-2** (122 mg, 237 μmol), TEA (71.8 mg, 710 μmol) in DCM (5 mL). The reaction mixture was reacted at 25°C for 1 h. The reaction mixture was poured into $H_2O$ (20 mL) and extracted with DCM (30 mL × 2). The organic layer was collected and dried over anhydrous $Na_2SO_4$, filtered and concentrated by rotary distillation to give the **intermedaite 52-3** (130 mg, 70.7% yield) as a green solid.
$^1$H NMR (400 MHz, DMSO-$d_6$) $\delta$ 10.16 (s, 1H), 8.82 (s, 1H), 8.17 - 7.98 (m, 1H), 7.81 - 7.70 (m, 1H), 7.62 - 7.52 (m, 3H), 6.96 - 6.85 (m, 2H), 5.73 - 5.59 (m, 1H), 5.36 (s, 1H), 5.05 - 4.94 (m, 1H), 4.88 - 4.79 (m, 1H), 4.76 - 4.61 (m, 2H), 4.16 - 4.04 (m, 2H), 3.72 - 3.64 (m, 2H), 3.19 - 3.17 (m, 3H), 2.67 - 2.60 (m, 2H), 1.81 - 1.75 (m, 2H), 1.50 - 1.43 (m, 7H), 1.42 - 1.33 (m, 2H)

### Step 3: Synthesis of compound 52

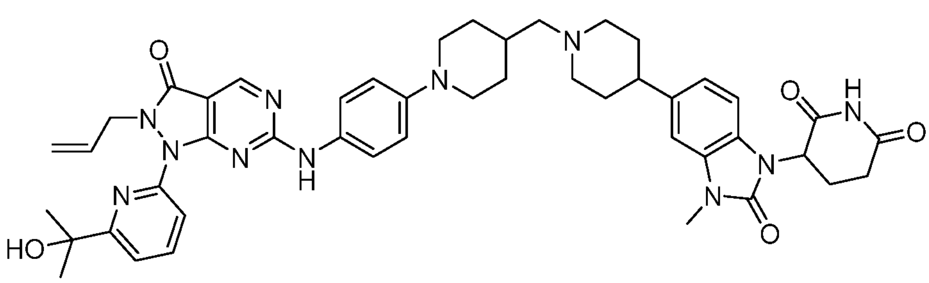

**compound 52**

**Intermediate 52-3** (130 mg, 153 μmol), **intermediate 52-4** (52.5 mg, 153 μmol) and DIEA (59.4 mg,460 μmol) in DMF (3 mL) was reacted at 100°C for 1 h under MW. The reaction mixture was poured into $H_2O$ (20 mL) and extracted with DCM (30 mL × 2). The organic layer was collected and dried over anhydrous $Na_2SO_4$, filtered and dried by rotaty

distillation to give the residue. The residue was purified by prep-HPLC (Calumn:Kromasil 100-5-C18 30*150 mm, Mobile Phase A: water (0.01% FA), Mobile Phase B: acetonitrile, 20 mL/min, gradient condition form 20% B to 50%) to give **compound 52** (10.0 mg, 15.5% yield) as a yellow solid. MS (ESI$^+$): m/z 840.6 $[M+H]^+$.

$^1$H NMR (400 MHz, DMSO-$d_6$) $\delta$ 11.09 (s, 1H), 10.05 (s, 1H), 8.86 - 8.68 (m, 1H), 8.08 - 7.95 (m, 1H), 7.90 - 7.19 (m, 5H), 7.16 - 6.85 (m, 3H), 6.54 - 6.47(m, 1H), 5.72 - 5.59 (m, 1H), 5.33 (dd, *J* = 5.2, 12.8 Hz, 1H), 5.05 - 4.94 (m, 1H), 4.88 - 4.76 (m, 1H), 4.74 - 4.60 (m, 2H), 3.32 (s, 3H), 3.05 - 3.00 (m, 2H), 2.93 - 2.82 (m, 3H), 2.67 - 2.61 (m, 2H), 2.46 - 2.25 (m, 5H), 2.18 -1.94 (m, 5H), 1.81 -1.58 (m, 7H), 1.45 (s, 6H).

**Compound 53** to **54** were synthesized in an analogous manner of **compound 52,** via various intermediates.

| Compound No. | Intermediate No. | MS(ESI$^+$ ): m/z $[M+H]^+$ | $^1$H NMR (400 MHz, DMSO-$d_6$) $\delta$ |
|---|---|---|---|
| 53 | B 15-A | 866.6 | 11.17 - 11.06 (m, 1H), 10.55 - 9.87 (m, 1H), 8.97 - 8.70 (m, 1H), 8.04 - 7.76 (m, 1H), 7.75 - 7.36 (m, 3H), 7.30 - 6.44 (m, 5H), 5.75 - 5.56 (m, 1H), 5.46 - 5.33 (m, 1H), 5.04 - 4.93 (m, 1H), 4.90 - 4.70 (m, 2H), 4.65 - 4.47 (m, 1H), 3.62 (s, 3H), 3.33 - 3.08 (m, 8H), 3.03 - 2.69 (m, 5H), 2.65 - 2.52 (m, 3H), 2.39 - 2.34 (m, 1H), 2.25 - 2.15 (m, 2H), 2.15 - 1.80 (m, 9H), 1.76 - 1.57 (m, 2H), 0.87 (t, *J* = 7.6 Hz, 3H). |
| 54 | B 15-B | 866.6 | 11.12 (s, 1H), 10.53 - 9.89 (m, 1H), 8.98 - 8.66 (m, 1H), 8.04 - 7.81 (m, 1H), 7.77 - 7.35 (m, 3H), 7.33 - 6.46 (m, 5H), 5.74 -5.59 (m, 1H), 5.48 - 5.32 (m, 1H), 5.05 - 4.96 (m, 1H), 4.90 - 4.67 (m, 2H), 4.64 - 4.44 (m, 1H), 3.66 - 3.63 (m, 3H), 3.27 - 3.10 (m, 8H), 3.05 - 2.82 (m, 5H),2.81 - 2.69 (m, 3H), 2.39 - 2.35 (m, 1H), 2.24 - 2.16 (m, 2H), 2.08 - 1.78 (m, 9H), 1.76 - 1.59 (m, 2H), 0.87 (t,J= 7.6 Hz, 3H) |

**Example 55: compound 55**

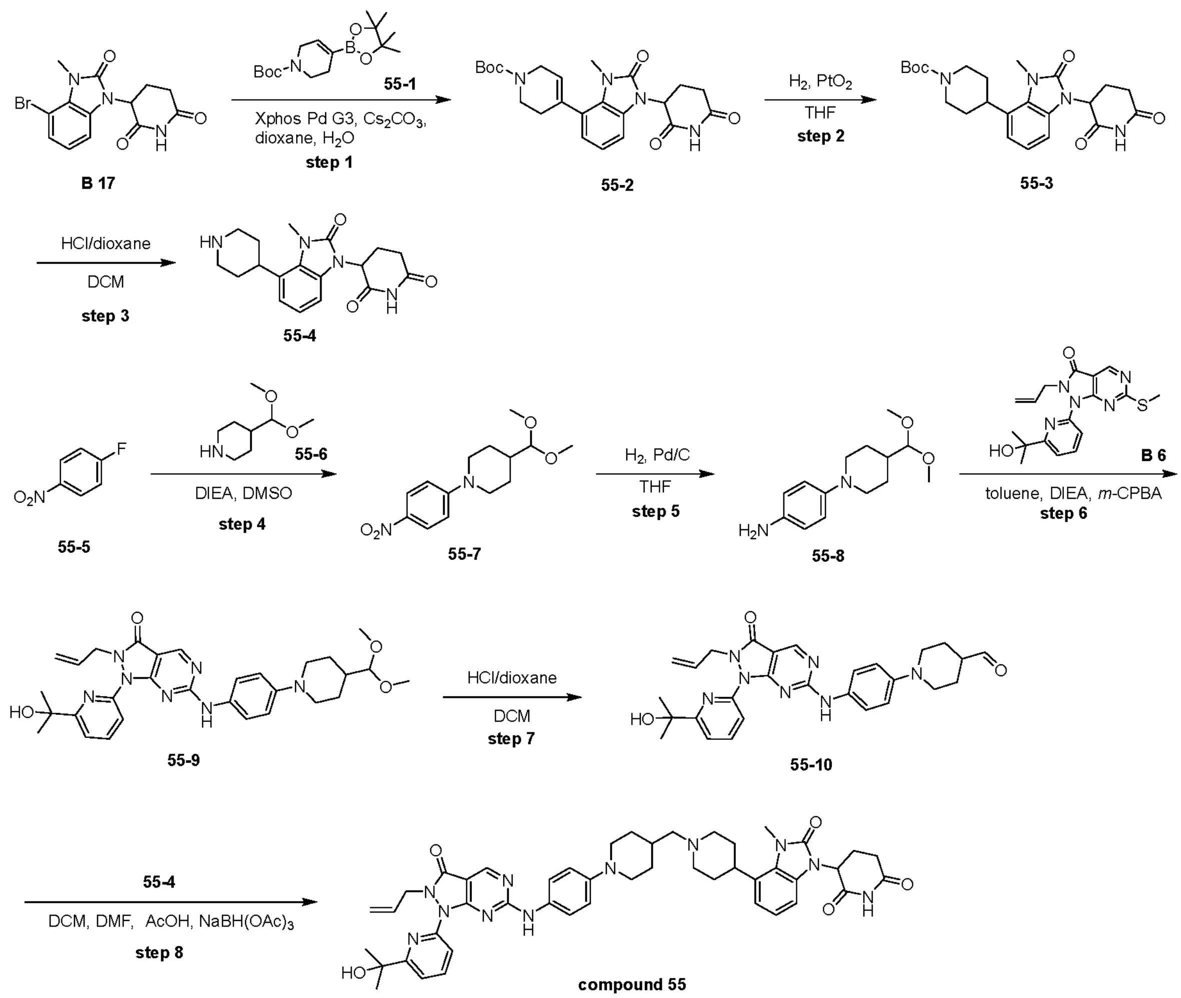


B 17, 55-2, 55-3, 55-4, 55-5, 55-7, 55-8, 55-9, 55-10, compound 55

**Step 1: Synthesis of intermediate 55-2**

To a solution of **intermediate B 17** (250 mg, 793 μmol) and **intermediate 55-1** (297 mg, 961 μmol) in dioxane (5 mL) and $H_2O$ (0.2 mL) was added $Cs_2CO_3$ (723 mg, 2.22 mmol) and XPhos Pd G3 (125 mg, 148 μmol), then the mixture was displaced with $N_2$ for 3 times, and then the mixture was stirred at 60 °C for 3 h under $N_2$ atmosphere. The reaction mixture was dried over anhydrous $Na_2SO_4$ and filtered. The filtrate was concentrated by rotary distillation to give the residue. The residue was purified by column chromatography (DCM: MeOH=1: 0 to 20: 1). **Intermediate 55-2** (322 mg, 85.03% yield) was obtained as a white solid.

MS (ESI$^+$): m/z 384.8 [M+H-56]$^+$.

**Step 2: Synthesis of intermediate 55-3**

To a solution of **intermediate 55-2** (322 mg, 629 μmol) in THF (15 mL) was added $PtO_2$ (500 mg, 2.20 mmol). The reaction mixture was then degassed and purged with $H_2$ for 3 times, and then the mixture was stirred at 20 °C for 32 h under $H_2$ atmosphere. The reaction mixture was filtered and concentrated by rotary distillation to give the residue. **Intermediate 55-3** (324 mg, crude) was obtained as a white solid.

MS (ESI$^+$): m/z 387.0 [M+H-56]$^+$.

**Step 3: Synthesis of intermediate 55-4**

To a solution of **intermediate 55-3** (324 mg, 732 μmol) in DCM (5 mL) was added HCl/dioxane (4 M, 4 mL). The mixture was stirred at 20 °C for 1 h. The reaction mixture was concentrated by rotary distillation to give the residue. **Intermediate 55-4** (302 mg, crude) was obtained as a white solid.

**Step 4: Synthesis of intermediate 55-7**

A solution of **intermediate 55-5** (1.0 g, 7.09 mmol), **intermediate 55-6** (1.13 g, 7.09 mmol) in DMSO (5 mL) was added DIEA (4.58 g, 35.4 mmol, 6.17 mL) and stirred at 90°C for 1 h. The reaction mixture was seprated with EA (20 mL) and water (20 mL). The organic layer was washed with saturated NaCl (30 mL), dried over anhydrous $Na_2SO_4$, concentrated by rotaty destination to give the residue. The residue was purified by column chromatography (Petroleum ether/ethyl acetate=3/1). **Intermediate 55-7** (1.50 g, 75.5% yield) was obtained as a yellow solid.

**Step 5: Synthesis of intermediate 55-8**

A sulotion of **intermediate 55-7** (1.5 g, 5.35 mmol) in $C_4H_8O$ (20 mL) was added Pd/C (1 g, 10% purity) and was exchanged with $H_2$ for 3 times, then stirred at 30°C under $H_2$ for 1 hour. The reaction mixture was filtered and concentrated by rotaty distillation to give the **intermediate 55-8** (1.35 g, crude) as a brown solid.

**Step 6: Synthesis of intermediate 55-9**

To a solution of **intermediate B 6** (100 mg, 280 μmol) in toluene (3 mL) was added m-CPBA (62.5 mg, 308 μmol) and stirred at 30°C for 0.5 h and then DIEA (72.3 mg, 560 μmol, 98 μL) and **intermediate 55-8** (77.0 mg, 308 μmol) were added. The resulting mixture was stirred at 30°C for 10 hours. The reaction mixture was added EA (50 mL) and saturated $Na_2S_2O_3$ (30 mL), separated. The organic layer was washed with saturated NaCl (30 mL), dried over anhydrous $Na_2SO_4$ and concentrated by rotaty distillation to give the residue. The residue was purified by column chromatography (Petroleum ether/ethyl acetate=1/2) to give **intermediate 55-9** (160 mg, 98.9% yield) as a yellow solid.

MS (ESI$^+$): m/z 560.0 [M+H]$^+$.

**Step 7: Synthesis of intermediate 55-10**

A solution of **intermediate 55-9** (60 mg, 107 μmol,) and HCl/dioxane (4 M, 2 mL) was stirred at 30°C for 1 hour. The reaction mixture was concentrated by rotaty distillation to give the residue. **Intermediate 55-10** (65.0 mg, crude) was obtained as a yellow solid and used for the next step directly.

**Step 8: Synthesis of compound 55**

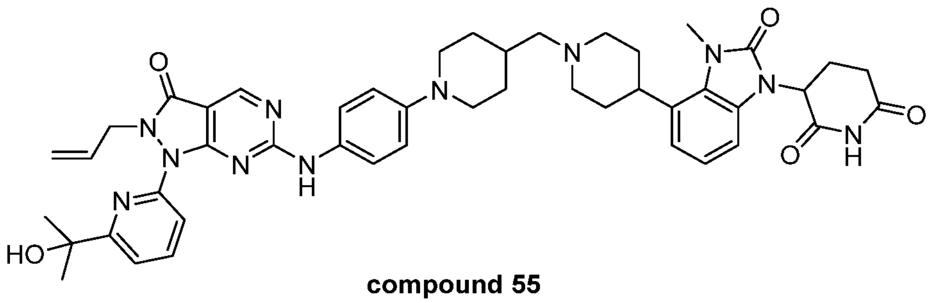

**compound 55**

A solution of **intermediate 55-10** (50 mg, 132 μmol), **intermediate 55-4** (65 mg, 118 μmol) and DMF (1.5 mL) in DCM (4 mL) was added $Et_3N$ (120 mg, 1.18 mmol, 164 μL) and stirred at 30 °C for 0.5 h. Then AcOH (70.6 mg, 1.18 mmol, 67.6 μL) was added and the reaction mixture was stirred at 30°C for 0.5 h. Then $NaBH(OAc)_3$ (150 mg, 709 μmol) was added and the reaction mixture was stirred at 30°C for 16 hours. The reaction mixture was seprated with DCM (10 mL) and water (10 mL). The organic layer was dried over anhydrous $Na_2SO_4$, filtered and concentrated by rotaty distillation to give the residue which was purified by prep-HPLC (acetonitrile/water (2% FA)) to give **compound 55** (30.0 mg, 28.9% yield) as a yellow solid.

MS (ESI$^+$): m/z 840.6 [M+H]$^+$.

$^1$H NMR (400 MHz, DMSO-$d_6$) $\delta$ 11.09 (s, 1H), 10.12 (br s, 1H), 8.82 (s, 1H), 8.22 (s, 0.476 H), 8.04 (br s, 1H), 7.82 - 7.69 (m, $J$ = 8.4 Hz, 1H), 7.68 - 7.45 (m, 3H), 7.10 - 6.85 (m, 5H), 5.73 - 5.59 (m, 1H), 5.43 - 5.24 (m, 2H), 5.07 - 4.94 (m, $J$ = 10.4 Hz, 1H), 4.82 (d, $J$ = 17.2 Hz, 1H), 4.73 - 4.59 (m, $J$ = 4.8 Hz, 2H), 3.64 (d, $J$ = 12.0 Hz, 2H), 3.58 (s, 3H), 2.99 (d, $J$=10.8 Hz, 2H), 2.93 - 2.81 (m, 1H), 2.76 - 2.57 (m, 4H), 2.45 - 2.38 (m, 1H), 2.28 - 2.17 (m, $J$ = 6.4 Hz, 2H), 2.14 - 1.93 (m, 3H), 1.86 - 1.62 (m, 7H), 1.46 (s, 6H), 1.31 - 1.15 (m, 2H).

The **compound 95** to **96** were synthesized according to the procedures described for **compound 55** using the different intermediates.

| Compound No. | Intermedite No. | MS(ESI+): m/z [M+H]+ | $^1$H NMR (400 MHz, DMSO-$d_6$) $\delta$ |
|---|---|---|---|
| 95 | B 46 | 825.6 | 10.58 (s, 1H), 10.18 (br s, 1H), 8.83 (s, 1H), 8.05 (br s, 1H), 7.79 - 7.73 (m, 1H), 7.67 - 7.50 (m, 4H), 7.44 (br s, 1H), 7.08 - 7.03 (m, 1H), 6.98 - 6.82 (m, 2H), 5.72 - 5.58 (m, 1H), 5.35 (s, 1H), 4.99 (d, *J* = 9.2 Hz, 1H), 4.86 - 4.78 (m, 1H), 4.69 (br s, 2H), 3.98 (s, 4H), 3.94 - 3.87 (m, 2H), 3.67 (br s, 3H), 3.09 (br s, 3H), 2.78 - 2.72 (m, 2H), 2.66 - 2.61 (m, 1H), 2.46 - 2.43 (m, 2H), 2.06 (br s, 3H), 1.93 - 1.61 (m, 5H), 1.46 (s, 6H), 1.35 (br s, 2H). |
| 96 | B 47 | 824.5 | 10.89 (s, 1H), 10.25 - 10.05 (m, 1H), 8.82 (s, 1H), 8.11 - 7.95 (m, 1H), 7.75 (d, *J* = 8.0 Hz, 1H), 7.67 - 7.52 (m, 4H), 7.46 (s, 1H), 7.05 (d, *J* = 8.4 Hz, 1H), 6.93 (d, *J* = 8.8 Hz, 2H), 5.75 - 5.58 (m, 1H), 5.34 (s, 1H), 4.99 (d, *J* = 10.4 Hz, 1H), 4.82 (d, *J* = 17.2 Hz, 1H), 4.68 (d, *J* = 5.2 Hz, 2H), 4.42 - 4.27 (m, 1H), 3.97 (s, 3H), 3.65 (d, *J* = 12.0 Hz, 2H), 3.32 (br s, 3H), 3.11 - 2.89 (m, 2H), 2.70 - 2.62 (m, 3H), 2.40 - 2.35 (m, 1H), 2.26 (d, *J* = 9.6 Hz, 1H), 2.21 - 2.14 (m, 1H), 2.12 - 1.95 (m, 2H), 1.81 (br s, 6H), 1.75 - 1.65 (m, 1H), 1.46 (s, 6H), 1.32 - 1.24 (m, 2H). |

**Example 58: compound 58**

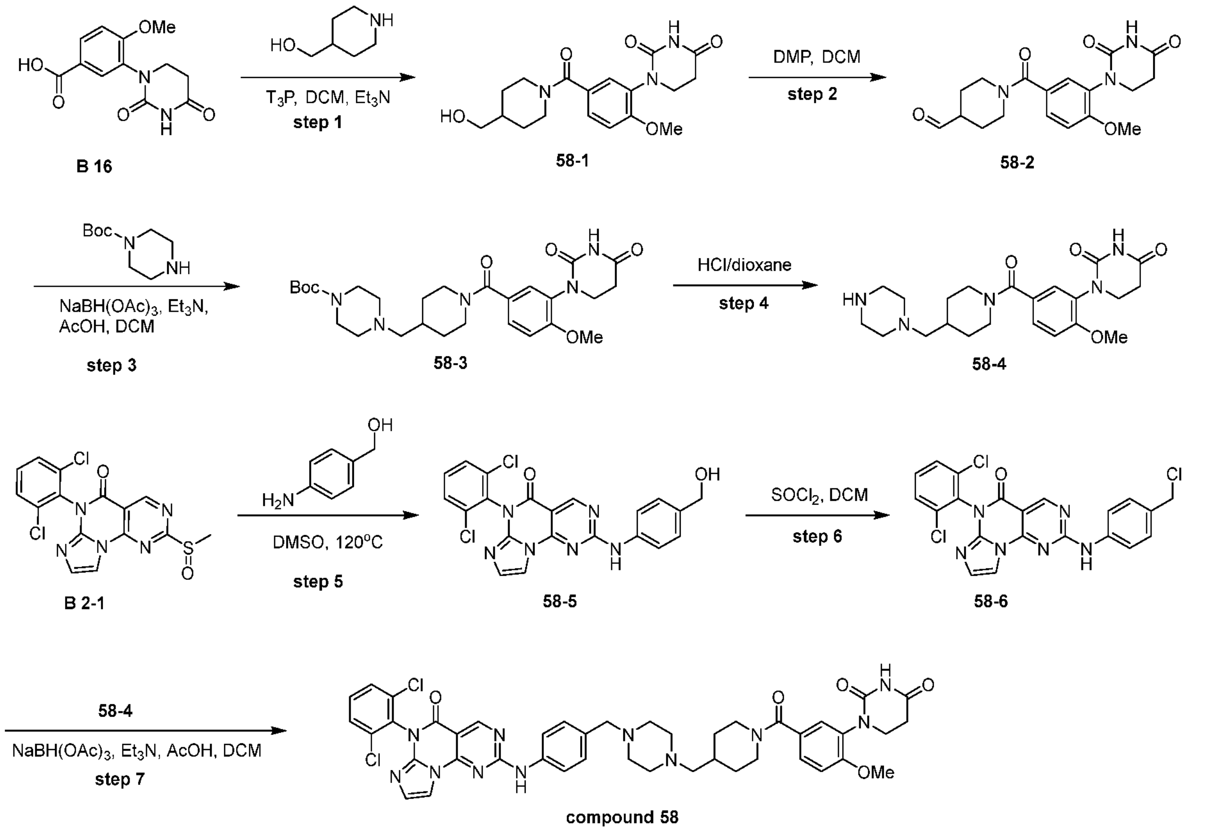


**Step1: Synthesis of compound 58-1**

A mixture of **intermediate B 16** (500 mg, 1.89 mmol), 3-Aminopiperidine-2,6-dione (492 mg, 2.99 mmol, HCl),

TEA (957 mg, 9.46 mmol, 1.3 mL), $T_3P$ (2.4 g, 3.78 mmol, 2.3 mL, 50% purity) in DCM (5 mL) was stirred at 20°C for 6 hrs. The reaction mixture was diluted with $H_2O$ (30 mL) and extracted with Dichloromethane: Methanol=10:1 (50 mL × 4). The combined organic layers were seprated and dried over anhydrous $Na_2SO_4$, filtered and concentrated under reduced pressure to give **intermediate 58-1** (200 mg, 14.6% yield) as a yellow solid.

MS ($ESI^+$): m/z 362.1 $[M+H]^+$.

**Step 2: Synthesis of intermediate 58-2**

A mixture of **intermediate 58-1** (300 mg, 830.12 μmol) and DMP (704 mg, 1.66 mmol, 514.00 μL) in DCM (5 mL) was stirred at 20 °C for 1 h. The reaction mixture was diluted with $Na_2S_2O_3$:$NaHCO_3$=1:1 (100 mL) and extracted with DCM (30 mL). The combined organic layers were seprated, dried over anhydrous $Na_2SO_4$, and concentrated by rotary distillation to give the residue. **Intermediate 58-2** (280 mg, 91.7% yield) was obtained as a yellow liquid.

MS ($ESI^+$): m/z 360.1 $[M+H]^+$.

**Step 3: Synthesis of intermediate 58-3**

To a mixture of **intermediate 58-2** (200 mg, 557 μmol) and tert-butyl piperazine-1-carboxylate (103 mg, 557 μmol) in DCM (2 mL) was added AcOH (67 mg, 1.11 mmol), the mixture was stirred for 1 hour, then added $NaBH(OAc)_3$ (354 mg, 1.67 mmol), and then the mixture was stirred at 20 °C for 16 hrs. The reaction mixture was added $H_2O$ (20 mL) and DCM (50 mL). The combined organic layers were washed dried over $Na_2SO_4$, filtered and concentrated by rotary distillation to give the residue. The residue was purified by column chromatography ($SiO_2$, Dichloromethane: Methanol=1/0 to 10/1). **Intermediate 58-3** (150 mg, 45.0% yield) was obtained as a yellow solid.

MS ($ESI^+$): m/z 529.9 $[M+H]^+$.

**Step 4: Synthesis of intermediate 58-4**

A mixture of **intermediate 58-3** (150 mg, 283.22 μmol) and HCl/dioxane (4 M, 10 mL) in DCM (3 mL) was stirred at 20 °C for 1 h. The reaction mixture was concentrated by rotary distillation to give **intermediate 58-4** (150 mg,crude) as a gray solid, and the crude product was used into the next step.

MS ($ESI^+$): m/z 430.1 $[M+H]^+$.

**Step 5: Synthesis of intermediate 58-5**

A mixture of **intermediate B 2-1** (1.6 g, 4.06 mmol and (4-aminophenyl)methanol (800 mg, 6.50 mmol) in DMSO (8 mL) was reacted at 120°C for 3 hours.The reaction mixture was concentrated to give the **intermediate 58-5** (330mg, 12.6 yield) as a brown solid.

MS ($ESI^+$): m/z 454.96 $[M+H]^+$.

**Step 6: Synthesis of intermediate 58-6**

A mixture of **intermediate 58-5** (30 mg, 66.2 μmol) and $SOCl_2$ (40 mg, 331 μmol, 24.0 μL) in DCM (3 mL) was reacted at 20 °C for 1 h. The reaction mixture was dried by rotary distillation to give **intermediate 58-6** (31 mg, 99.29% yield) as a yellow solid.

**Step 7: Synthesis of compound 58**

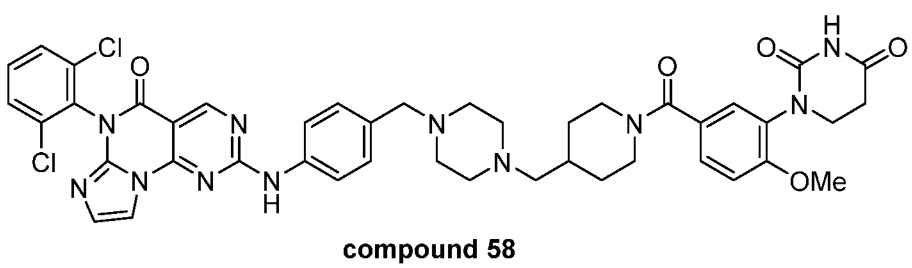

**compound 58**

A mixture of **intermediate 58-4** (30 mg, 63.60 μmol), **intermediate 58-6** (30 mg, 59.71 μmol, HCl) and $K_2CO_3$ (41.26 mg, 299 μmol) in DMF (3 mL) was degassed and purged with $N_2$ for 3 times, and then the mixture was stirred at 20 °C for 16 hrs under $N_2$ atmosphere. The reaction mixture was filtered and dried by rotary distillation, then purified by prep-HPLC (acetonitrile/water (0.1%TFA)) to give **compound 58** (2.43 mg, 4.66% yield) as a white solid. MS (ESI$^+$): m/z 864.2 [M+H]$^+$.

$^1$H NMR (400MHz, DMSO-$d_6$) $\delta$ 11.22 - 10.81 (m, 1H), 10.36 (s, 1H), 9.20 (s, 1H), 8.95 (br s, 1H), 7.90 (br s, 2H), 7.83 - 7.72 (m, 2H), 7.70 - 7.59 (m, 1H), 7.48 (d, *J* = 8.0 Hz, 1H), 7.42 - 7.29 (m, 3H), 7.24 - 7.08 (m, 2H), 4.40 (br s, 1H), 4.02 (br s, 2H), 3.85 (s, 3H), 3.88 - 3.82 (m, 1H), 3.62 3.57 (m, 4H), 3.27 (br s, 5H), 2.74 - 2.60 (m, 5H), 2.33 (br s, 1H), 1.95 (br s, 1H), 1.76 (br s, 2H), 1.25 - 1.03 (m, 3H).

**Compound 59** to **60** were synthesized in an analogous manner of **compound 58** with various intermediates.

| Compou nd No. | Intermed iate No. | MS(ESI$^+$): m/z [M+H]$^+$ | $^1$H NMR (400 MHz, DMSO-$d_6$) $\delta$ |
|---|---|---|---|
| 59 | 58-6 | 835.2 | 10.98 - 10.78 (m, 1H), 10.35 (s, 1H), 9.16 (s, 1H), 7.97 - 7.74 (m, 5H), 7.67 - 7.60 (m, 1H), 7.41 - 7.27 (m, 4H), 7.19 - 7.05 (m, 2H), 3.83 (s, 3H), 3.57 (br s, 2H), 3.47 - 3.44 (m, 8H), 2.67 (br s, 2H), 2.36 (br s, 2H), 1.50 (br s, 4H), 1.42 (br s, 4H). |
| 60 | 58-6 | 865.1 | 10.88 (br s, 1H), 10.34 (s, 1H), 9.16 (s, 1H), 7.95 - 7.59 (m, 6H), 7.43 -7.25 (m, 4H), 7.19 - 7.06 (m, 2H), 3.84 (s, 3H), 3.67 (d, *J* = 8.0 Hz, 1H), 3.61 - 3.56 (m, 2H), 3.49 - 3.42 (m, 6H), 3.27 - 3.13 (m, 1H), 2.75 - 2.59 (m, 4H), 2.08 (t, *J* = 9.6 Hz, 2H), 1.78 (br s, 4H), 1.52 - 1.33 (m, 4H) |

## Example 61: compound 61

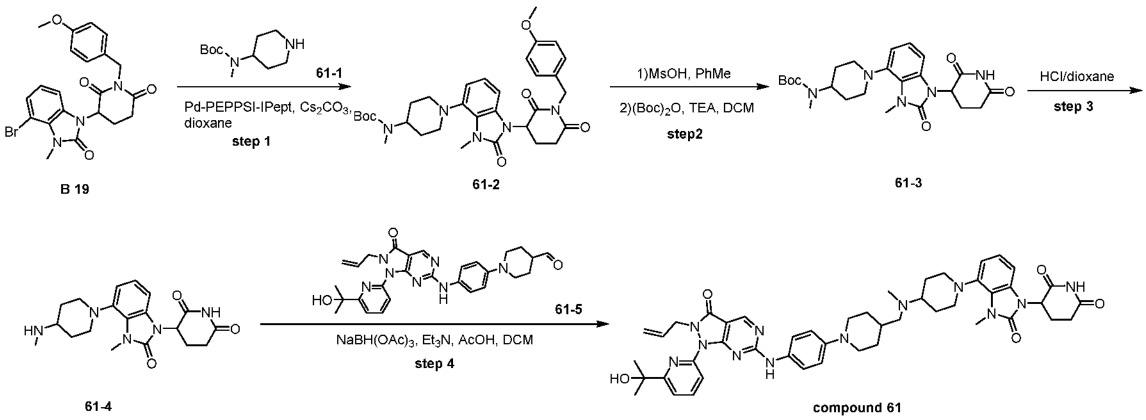

B 19
61-2
61-3
61-4
compound 61

### Step 1: Synthesis of intermediate 61-2

A mixture of **intermediate 61-1** (304 mg, 1.42 mmol), **intermediate B 19** (500 mg, 1.09 mmol) and $Cs_2CO_3$ (1.07 g, 3.27 mmol) in dioxane (5 mL) and toluene (5 mL) was added Pd-PEPPSI-IPentCl (53.1 mg, 54.6 μmol), and purged with $N_2$ for 3 times, then stirred at 100 °C for 16 hrs. The reaction mixture was diluted with $CH_2Cl_2$, filtered and concentrated by rotary distillation, then purified by column chromatography (Petroleum ether/Ethyl acetate=1/0 to 0/1) to give **intermediate 61-2** (330 mg, 39.4% yield) was obtained as colorless oil.

MS (ESI$^+$): m/z 592.2 [M+H]$^+$.

### Step 2: Synthesis of intermediate 61-3

To a mixture of **intermediate 61-2** (390 mg, 659 μmol) in toluene (0.94 mL) was added MsOH (1.27 g, 13.2 mmol, 938 μL) and degassed and purged with $N_2$ for 3 times, and then the mixture was stirred at 120°C for 2 hrs and stirred at 0°C for 15min. Then the reaction was added TEA (2.67 g, 26.4 mmol, 3.67 mL) and $(Boc)_2O$ (1.44 g, 6.59

mmol) at 0°C and stirred at 0°C for 15min. The reaction mixture was diluted with DCM (30 mL) and water (50 mL). Then the organic phase was separated, and the water phase was extracted with DCM (30 mL × 3). The combined organic layers were dried over anhydrous $Na_2SO_4$, filtered and concentrated by rotary distillation to give the residue. The residue was purified by column chromatography (DCM/MeOH=1/0 to 20/1) to give **intermediate 61-3** (267 mg, 53.3% yield) as colorless oil. MS (ESI$^+$): m/z 472.3 $[M+H]^+$.

**Step 3: Synthesis of intermediate 61-4**

To a solution of **intermediate 61-3** (267 mg, 566 μmol) in DCM (1 mL) was added HCl/dioxane (4 M, 2.33 mL). The mixture was stirred at 30°C for 1 h. The reaction mixture was concentrated by rotary distillation to give the **intermediate 61-4** (239 mg, HCl salt) as a yellow solid, which was used into the next step directly.

**Step 4: Synthesis of compound 61**

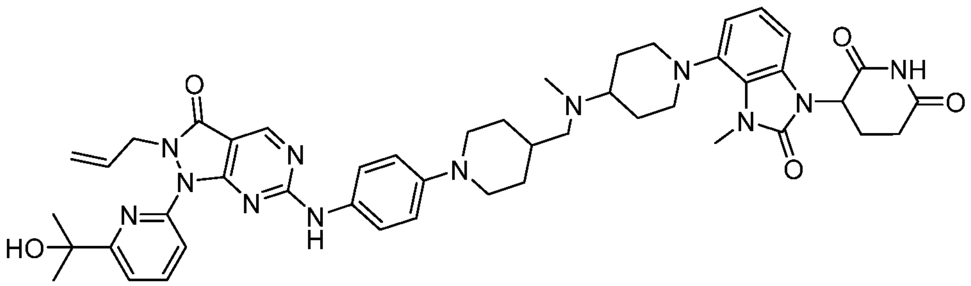

**compound 61**

To a mixture of **intermediate 55-10** (30 mg, 58.4 μmol) and **intermediate 61-4** (23.8 mg, 58.4 μmol, HCl) in DCM (3 mL) was added TEA (17.7 mg, 175 μmol, 24 μL) and stirred at 25°C for 30 min, then added AcOH (10.5 mg, 175 μmol, 10.0 μL) and stirred at 25°C for 30 min. The reaction was added $NaBH(OAc)_3$ (37.1 mg, 175 μmol) and stirred at 25°C for 16 hrs. The reaction mixture was concentrated by rotary distillation and purified by prep-HPLC (acetonitrile/ water (0.1%TFA condition)) to give the residue. The residue was further purified by prep-HPLC (acetonitrile/ water (2%FA condition)) to give **compound 61** (3 mg, 5.5% yield) as a yellow solid.

MS (ESI$^+$): m/z 869.6 $[M+H]^+$
$^1$H NMR (400 MHz, DMSO-$d_6$) $\delta$ 11.25 - 10.93 (m, 1H), 10.25 - 9.93 (m, 1H), 8.81 (s, 1H), 8.36 (s,1.07H),8.04 (t, *J* = 8.0 Hz, 1H), 7.75 (d, *J* = 6.8 Hz, 1H), 7.63 - 7.40 (m, 3H), 7.02 - 6.79 (m, 5H), 5.76- 5.60 (m, 1H), 5.45 - 5.24 (m, 2H), 4.99 (d, *J* = 10.0 Hz, 1H), 4.88 - 4.80 (m, 1H), 4.75 - 4.60 (m, 2H),3.63 (s, 5H), 3.16 - 3.12 (m, 2H), 2.89 - 2.85 (m, 1H), 2.74 - 2.70 (m, 3H), 2.64 - 2.60 (m, 4H), 2.36 -2.31 (m, 2H), 2.25 (s, 3H), 2.04 - 1.94 (m, 1H), 1.88 - 1.74 (m, 4H), 1.71 - 1.53 (m, 3H), 1.46 (s, 6H),1.31 - 1.16 (m, 2H).

The **compound 90, 98** and **99** were synthesized according to the procedures described for **compound 61** using the different intermediates.

| Compound No. | Intermediate No. | MS(ESI$^+$): m/z $[M+H]^+$ | $^1$H NMR (400 MHz, DMSO-$d_6$) $\delta$ |
|---|---|---|---|
| 90 | 55-10; | 863.5 $[M+Na]^+$ | 11.10 (s, 1H), 10.24 - 10.03 (m, 1H), 8.82 (s, 1H), 8.10 -7.96 (m, 1H), 7.75 (d, *J* = 7.6 Hz, 1H), 7.65 - 7.43 (m, 3H), 7.05 - 6.85 (m, 5H), 5.76 - 5.55 (m, 1H), 5.42 - 5.25 (m, 2H), 4.99 (d, *J* = 10.4 Hz, 1H), 4.87 - 4.78 (m, 1H), 4.74 - 4.60 (m, 2H), 3.71 - 3.57 (m, 5H), 3.09 - 2.80 (m, 6H), 2.75 - 2.58 (m, 4H), 2.48 - 2.40 (m, 1H), 2.33 - 2.11 (m, 4H), 2.05 - 1.93 (m, 1H), 1.82 (d, *J* = 12.0 Hz, 2H), 1.74 - 1.61 (m, 1H), 1.46 (s, 6H), 1.31 - 1.16 (m, 2H). |

(continued)

| Compound No. | Intermediate No. | MS(ESI$^+$): m/z $[M+H]^+$ | $^1H$ NMR (400 MHz, DMSO-$d_6$) $\delta$ |
|---|---|---|---|
| 98 | B 6 | 855.6 | 11.10 (s, 1H), 10.15 (br s, 1H), 8.82 (s, 1H), 8.33 (s, 1.226H), 8.16 - 7.93 (m, 1H), 7.75 (d, *J* = 7.2 Hz, 1H), 7.63 - 7.39 (m, 3H), 7.08 - 6.78 (m, 5H), 5.72 - 5.57 (m, 1H), 5.45 - 5.22 (m, 2H), 4.99 (d, *J* = 10.0 Hz, 1H), 4.82 (d, *J* = 17.2 Hz, 1H), 4.68 (d, *J* = 4.0 Hz, 2H), 3.74 - 3.66 (m, 2H), 3.64 (s, 3H), 3.14 (d, *J* = 10.8 Hz, 2H), 2.95 - 2.84 (m, 1H), 2.79 - 2.64 (m, 7H), 2.44 (br s, 1H), 2.24 (s, 3H), 2.06 - 1.95 (m, 1H), 1.88 - 1.67 (m, 6H), 1.67 - 1.56 (m, 2H), 1.46 (s, 6H). |
| 99 | B 8 | 826.6 | 11.11 (s, 1H), 10.26 (s, 1H), 8.88 (s, 1H), 8.07 (t, *J* = 7.6 Hz, 1H), 7.76 (d, *J* = 8.0 Hz, 1H), 7.72 - 7.56 (m, 3H), 7.22 (d, *J* = 8.4 Hz, 2H), 7.04 - 6.82 (m, 3H), 5.67-5.72 (m, 1H), 5.41 - 5.32 (m, 2H), 5.04 - 4.97 (m, 1H), 4.88 - 4.79 (m, 1H), 4.69 (d, *J* = 5.6 Hz, 2H), 3.64 (s, 3H), 3.21 - 3.12 (m, 2H), 3.12 - 2.98 (m, 2H), 2.95 - 2.84 (m, 1H), 2.79 - 2.59 (m, 4H), 2.48 - 2.41 (m, 2H), 2.40 - 2.28 (m, 2H), 2.04 - 1.97 (m, 1H), 1.95 - 1.85 (m, 2H), 1.84 - 1.57 (m, 6H), 1.47 (s, 6H). |

**Example 62: compound 62**

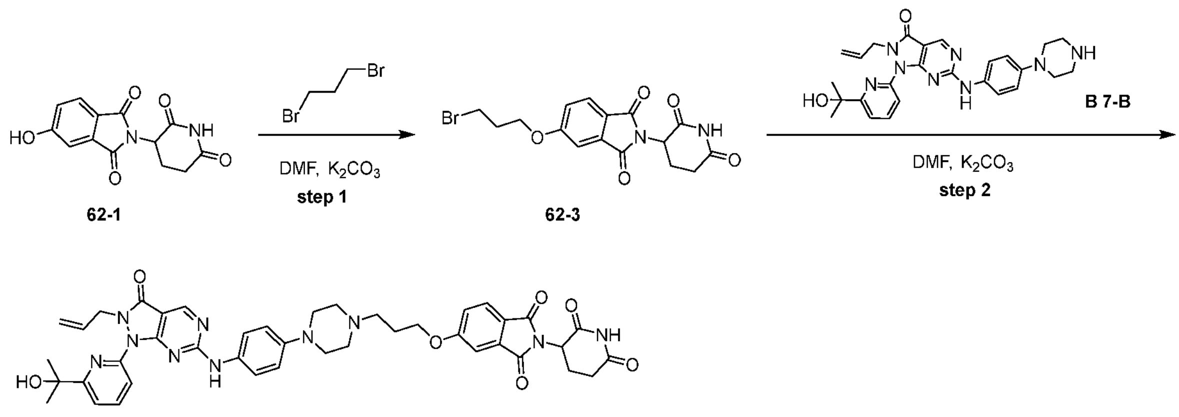


compound 62

**Step 1: Synthesis of intermediate 62-3**

To a solution of **intermediate 62-1** (500 mg, 1.82 mmol) and 1,3-dibromopropane (1.84 g, 9.12 mmol) in DMF (2 mL) was added $K_2CO_3$ (252 mg, 1.82 mmol) .The mixture was stirred at 50°C for 16 hrs. Then water (20 mL) and EA (20 mL) was added to the mixture. The organic phase was separated, dried over anhydrous $Na_2SO_4$, filtered and concentrated under reduced pressure to give the residue. The residue was purified by column chromatography (Petroleum ether/Ethyl acetate=3/1 to 2/1) to give **intermediate 62-3** (480 mg, 26.7% yield) as a colourless oil. MS (ESI$^+$): m/z 395.20 $[M+H]^+$.

**Step 2: Synthesis of compound 62**

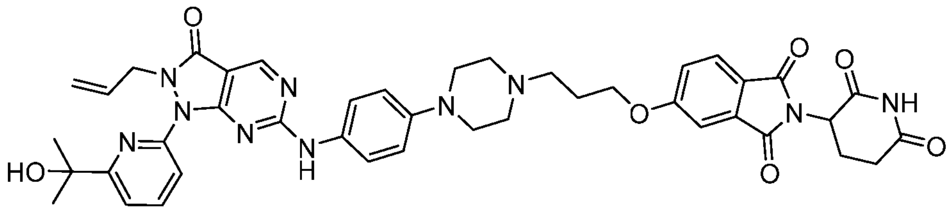

**compound 62**

To a solution of **intermediate 62-3** (75.56 mg, 191 μmol) and **intermediate B 7-B** (100 mg, 191 μmol) in DMF (0.5 mL) was added $K_2CO_3$ (252 mg, 1.82 mmol) . The mixture was stirred at 60°C for 16 hrs. The reaction mixture was added water (20 mL) and EtOAc (20 mL), then the organic layer separated, dried over anhydrous $Na_2SO_4$, filtered and concentrated under reduced pressure to give the residue. The residue was purified by prep-HPLC (TFA condition) and lyophilized to give a yellow solid. The yellow solid was diluted with DCM (5 mL) and washed with $NaHCO_3$ (3 mL). The organic layer was dried over anhedrous $Na_2SO_4$, filtered and concentrated, then purified by chromatography on silica gel (DCM: MeOH=10: 1, 4 g silica gel column) to give **compound 62** (12 mg, 14.83 μmol, 7.76% yield) as a yellow solid.

MS (ESI+): m/z 395.20 [M+H]+.

$^1H$ NMR (400 MHz, DMSO-$d_6$) δ 11.13 (s, 1H), 10.16 (s, 1H), 8.83 (s, 1H), 8.05 (s, 1H), 7.85 (d,J = 8.4 Hz, 1H), 7.75 (d, *J* = 8.0 Hz, 1H), 7.61 (d, *J* = 8.0 Hz, 3H), 7.46 (d, *J* = 2.0 Hz, 1H), 7.38 (d,*J* = 8.4 Hz, 1H), 6.93 (d, *J* = 8.0 Hz, 2H), 5.73 - 5.61 (m, 1H), 5.35 (s, 1H), 5.13 (dd, *J* = 5.6, 13.2 Hz, 1H), 4.99 (d, *J* = 10.0 Hz, 1H), 4.82 (d, *J* = 17.2 Hz, 1H), 4.68 (d, *J* = 4.4 Hz, 2H), 4.26 (t, *J* = 6.0 Hz, 2H), 3.39 (s, 1H), 3.30 - 3.29 (m, 1H), 3.12 (s, 4H), 2.97 - 2.82 (m, 1H), 2.64 - 2.54 (m, 5H),2.15 - 1.88 (m, 4H), 1.46 (s, 6H).

**Compound 63** is similar to **compound 62,** and is obtained by synthesizing different intermediates.

| Compound No. | Intermediate No. | MS(ESI+): m/z [M+H]+ | $^1H$ NMR (400 MHz, DMSO-$d_6$) δ |
|---|---|---|---|
| 63 | B 7-B | 787.5 | 11.12 (s, 1H), 10.15 (br s, 1H), 8.82 (s, 1H), 8.32 (s, 0.209H), 8.11 -7.98 (m, 1H), 7.84 (d, *J* = 8.4 Hz, 1H), 7.75 (d, *J* = 8.0 Hz, 1H), 7.64 - 7.52 (m, 3H), 7.50 (d, *J* = 2.4 Hz,1H), 7.39 (dd, *J* = 2.4, 8.4 Hz, 1H), 6.93 (d, *J* = 9.2 Hz, 2H), 5.71 - 5.59 (m, 1H), 5.33 (s, 1H), 5.12 (dd, *J* = 5.2, 11.2 Hz, 1H), 4.99 (d, *J* = 10.4 Hz, 1H), 4.82 (d, *J* = 17.2 Hz, 1H), 4.68 (d, *J* = 5.2 Hz, 2H), 4.35 (t, *J* = 5.6 Hz, 2H), 3.29 - 3.27 (m, 2H), 3.12 (br s, 4H), 2.87 (d, *J* = 11.6 Hz, 1H), 2.81 (t, *J* = 5.2 Hz, 2H), 2.53 - 2.52 (m, 4H), 2.09 - 2.01 (m, 1H), 1.46 (s, 6H). |

**Example 64: compound 64**

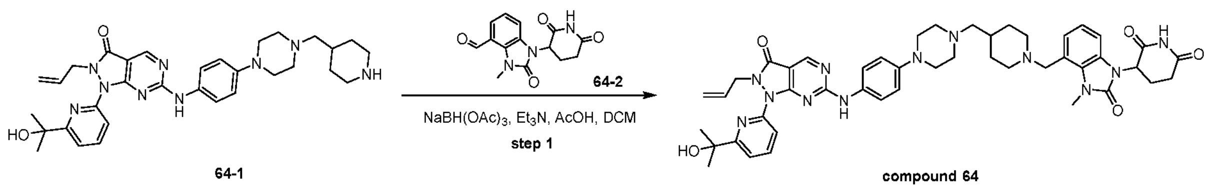


**Step 1: Synthesis of compound 64**

To a solution of **intermediate 64-1** (82.0 mg, 132.28 μmol) and **intermediate 64-2** (38.0 mg, 132.28 μmol) in DCM (10 mL) was added TEA (53.5 mg,529.12 μmol) and stirred at 25°C for 30 min, then AcOH (23.8 mg, 396.84 μmol) was added and stirred at 25°C for 30 min. The reaction mixture was added $NaBH_3CN$ (84.1 mg, 397 μmol) and stirred at 25°C for 16 hrs. The reaction mixture was added water (20 mL) and EtOAc (20 mL), then the organic layer was separated, dried over anhydrous $Na_2SO_4$, filtered and concentrated under reduced pressure to give the residue. The residue was purified by prep-HPLC (FA condition) to give **compound 64** (3 mg, 3.51 μmol, 2.65% yield) as a yellow solid.

MS (ESI+): m/z 877.6 [M+Na]+.

$^1$H NMR (400 MHz, DMSO-$d_6$) $\delta$ 11.11 (s, 1H), 10.38 - 9.93 (m, 1H), 8.84 (s, 1H), 8.13 - 7.99 (m,1H), 7.76 (d, $J$ = 4.0 Hz, 1H), 7.61 (d, $J$ = 8.0 Hz, 3H), 7.31 - 6.86 (m, 5H), 5.78 - 5.59 (m, 1H), 5.46 - 5.28 (m, 2H), 5.00 (d, $J$ = 8.0 Hz, 1H), 4.83 (d, $J$ = 16.0 Hz, 1H), 4.68 (d, $J$ = 4.0 Hz, 2H), 3.72 - 3.62 (m, 4H), 3.31 - 3.29 (m, 3H), 3.09 (br s, 4H), 2.94 - 2.81 (m, 2H), 2.76 - 2.69 (m, 1H), 2.57 - 2.53(m, 6H), 2.45 - 2.36 (m, 2H), 2.08 - 1.93 (m, 4H), 1.71 (br s, 2H), 1.47 (s, 6H).

**Example 65: compound 65**

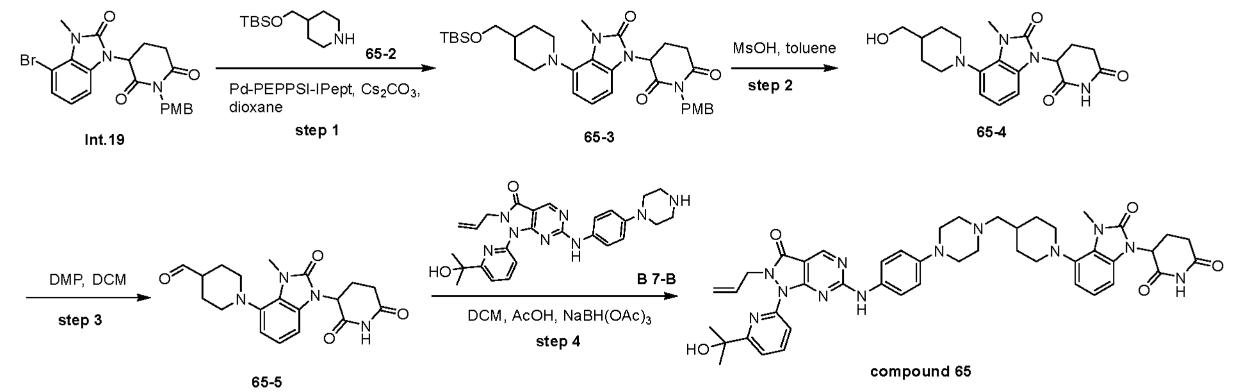


**Step 1: Synthesis of intermediate 65-3**

A mixture of **intermediate 65-2** (325 mg, 1.42 mmol), **intermediate 19** (500 mg, 1.09 mmol) and $Cs_2CO_3$ (1.07 g, 3.27 mmol) in dioxane (8 mL) and toluene (8 mL) was added Pd-PEPPSI-IPentCl (53.1 mg, 54.6 $\mu$mol) , then degassed and purged with $N_2$ for 3 times, and then stirred at 100 °C for 16 hrs. The reaction was diluted with DCM, filtered and dried by rotary distillation to give the residue. The residue was purified by column chromatography (Petroleum ether/Ethyl acetate=1/0 to 3/1) to give **intermediate 65-3** (95 mg, 12.9% yield) as colorless oil.

$^1$H NMR (400 MHz, DMSO-$d_6$) $\delta$ 7.27 - 7.15 (m, 2H), 6.95 - 6.88 (m, 2H), 6.87 - 6.83 (m, 2H), 6.81 - 6.70 (m, 1H), 5.58 - 5.42 (m, 1H), 4.92 - 4.51 (m, 2H), 3.72 (s, 3H), 3.61 (s, 3H), 3.55 - 3.43 (m, 2H), 3.21 - 2.98 (m, 3H), 2.88 - 2.63 (m, 4H), 2.10 - 2.01 (m, 1H), 1.86 - 1.70 (m, 2H), 1.63 - 1.50 (m, 1H), 1.46 - 1.31 (m, 2H), 0.88 (s, 9H), 0.08 - 0.04 (m, 6H).

**Step 2: Synthesis of intermediate 65-4**

To a mixture of **intermediate 65-3** (110 mg, 181 $\mu$mol) in toluene (2 mL) was added MsOH (1.35 g, 14.1 mmol, 1 mL) and degassed and purged with $N_2$ for 3 times, and then stirred at 120 °C for 1.5 hrs. The reaction mixture was added additional TEA (3 mL) at 0 °C and purified by prep-HPLC (neutral condition) to give the residue. The residue was added DCM (10 mL) and adjusted to pH 8 with aq. sat. $NaHCO_3$. Then the mixture was added $H_2O$ (5 mL), the organic phase was separated. The aqueous phase was extracted with DCM (10 mL × 3).The combined organic phase dried over anhydrous $Na_2SO_4$, filtered and concentrated by rotary distillation to give **intermediate 65-4** (31 mg, 38.6% yield) as colorless oil.

MS (ESI$^+$): m/z 373.2 [M+H]$^+$.

**Step 3: Synthesis of intermediate 65-5**

To a solution of **intermediate 65-4** (31 mg, 83.2 $\mu$mol) in DCM (2 mL) and DMSO (0.1 mL) was added DMP (70.6 mg, 166 $\mu$mol). The mixture was stirred at 25°C for 30 min. The reaction mixture was quenched by DCM, then quenched with $Na_2S_2O_3$ (5 mL) and $NaHCO_3$ (5 mL), and extracted with DCM (15 mL × 3). The combined organic layers were dried over anhydrous $Na_2SO_4$, filtered and concentrated by rotary distillation to give the **intermediate 65-5** (30 mg, crude) as colorless oil, which was used into the next step directly.

**Step 4: synthesis of compound 65**

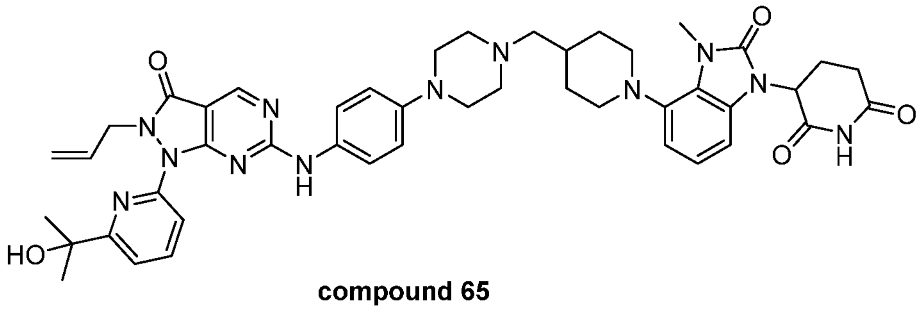

**compound 65**

To a mixture of **intermediate 65-5** (30 mg, 81.0 μmol) and **intermediate B 7-B** (42.4 mg, 81.0 μmol, HCl) in DCM (3 mL) was added TEA (24.6 mg, 243 μmol, 33.8 μL) and stirred at 25 °C for 30 min, then added AcOH (14.6 mg, 243 μmol) and stirred at 25 °C for 30 min. The reaction was added $NaBH(OAc)_3$ (51.5 mg, 243 μmol) and stirred at 25°C for 16 hours. The reaction mixture was concentrated by rotary distillation to give the residue. The residue was purified by HPLC (acetonitrile / water (2% FA)). Then the residue was added MeCN (3 mL) to triturate it to give **intermediate 65** (16 mg, 22.9% yield) as a yellow solid.

MS (ESI$^+$): m/z 841.6 [M+H]$^+$
$^1$H NMR (400 MHz, DMSO-$d_6$) δ 11.09 (s, 1H), 10.29 - 10.06 (m, 1H), 8.82 (s, 1H), 8.15 - 7.98 (m, 1H), 7.82 - 7.69 (m, 1H), 7.68 - 7.44 (m, 3H), 7.05 - 6.79 (m, 5H), 5.74 - 5.58 (m, 1H), 5.40 - 5.27 (m, 2H), 5.05 - 4.94 (m, 1H), 4.89 - 4.78 (m, 1H), 4.75 - 4.61 (m, 2H), 3.63 (s, 3H), 3.11 (br s, 5H), 2.95 - 2.83 (m, 1H), 2.76 - 2.62 (m, 4H), 2.59 (br s, 1H), 2.56 - 2.52 (m, 4H), 2.31 - 2.19 (m, 2H), 2.07 - 1.93 (m, 1H), 1.90 - 1.79 (m, 2H), 1.77 - 1.62 (m, 1H), 1.46 (s, 6H), 1.40 - 1.28 (m, 2H).

The **compound 66** and **compound 97** were synthesized according to the procedures described for **compound 65** using the different intermediates.

| Compound No. | Intermediate No. | MS(ESI$^+$): m/z [M+H]$^+$ | $^1$H NMR (400 MHz, DMSO-$d_6$) δ |
|---|---|---|---|
| 66 | B 8 | 862.5 [M+Na]$^+$ | 11.10 (s, 1H), 10.26 (br s, 1H), 8.87 (s, 1H), 8.19 (s, 1.072 H),8.07 (t, J=7.6 Hz, 1H), 7.75 (d,J=8.0 Hz, 1H), 7.64 (dd,J=7.6, 15.6 Hz, 3H), 7.21 (d,J=8.4 Hz,2H), 7.01 - 6.84 (m, 3H), 5.75 - 5.57 (m, 1H), 5.35 (dd,J=5.2, 12.4 Hz, 2H), 4.99 (d,J=10.2 Hz,1H), 4.90 - 4.78 (m, 1H), 4.68 (d,J=5.2 Hz, 2H), 3.63 (s, 3H), 3.18 - 3.12 (m, 2H), 3.01 - 2.94 (m,2H), 2.93 - 2.84 (m, 1H), 2.77 - 2.69 (m, 2H), 2.67 - 2.57 (m, 2H), 2.43 (br s, 1H), 2.23 (br s, 2H),2.08 - 1.93 (m, 3H), 1.84 (d,J=12.0 Hz, 2H), 1.78 - 1.59 (m, 5H), 1.46 (s, 5H), 1.39 - 1.26 (m, 2H) |
| 97 | B 30 | 854.6 | 11.11 (s, 1H), 10.21 (br s, 1H), 8.87 (s, 1H), 8.23 (br s, 0.838H), 8.05 (t, *J* = 8.0Hz, 1H), 7.79 (d, *J* = 8.0 Hz, 1H), 7.63 (d, *J* = 7.6 Hz, 2H), 7.46 (d, *J* = 8.0 Hz, 1H), 7.19 (d, *J* = 8.4 Hz, 1H),7.02 - 6.83 (m, 3H), 5.72 - 5.61 (m, 1H), 5.40 - 5.29 (m, 2H), 4.99 (d, *J* = 10.4 Hz, 1H), 4.82 (d, *J* = 17.6 Hz, 1H), 4.69 (d, *J* = 4.0 Hz, 2H), 3.64 (s, 3H), 3.14 (d, *J* = 8.0 Hz, 2H), 2.99 (d, *J* = 9.2 Hz, 2H), 2.94 - 2.87(m, 1H), 2.71 (br s, 2H), 2.66 - 2.58 (m, 2H), 2.43 (br s, 1H), 2.31 (s, 3H), 2.24 (br s, 2H), 2.00 (d, *J* = 6.0Hz, 3H), 1.86 (d, *J* = 12.0 Hz, 2H), 1.66 (br s, 5H), 1.46 (s, 6H), 1.40 - 1.27 (m, 2H). |

**Example 67: compound 67**

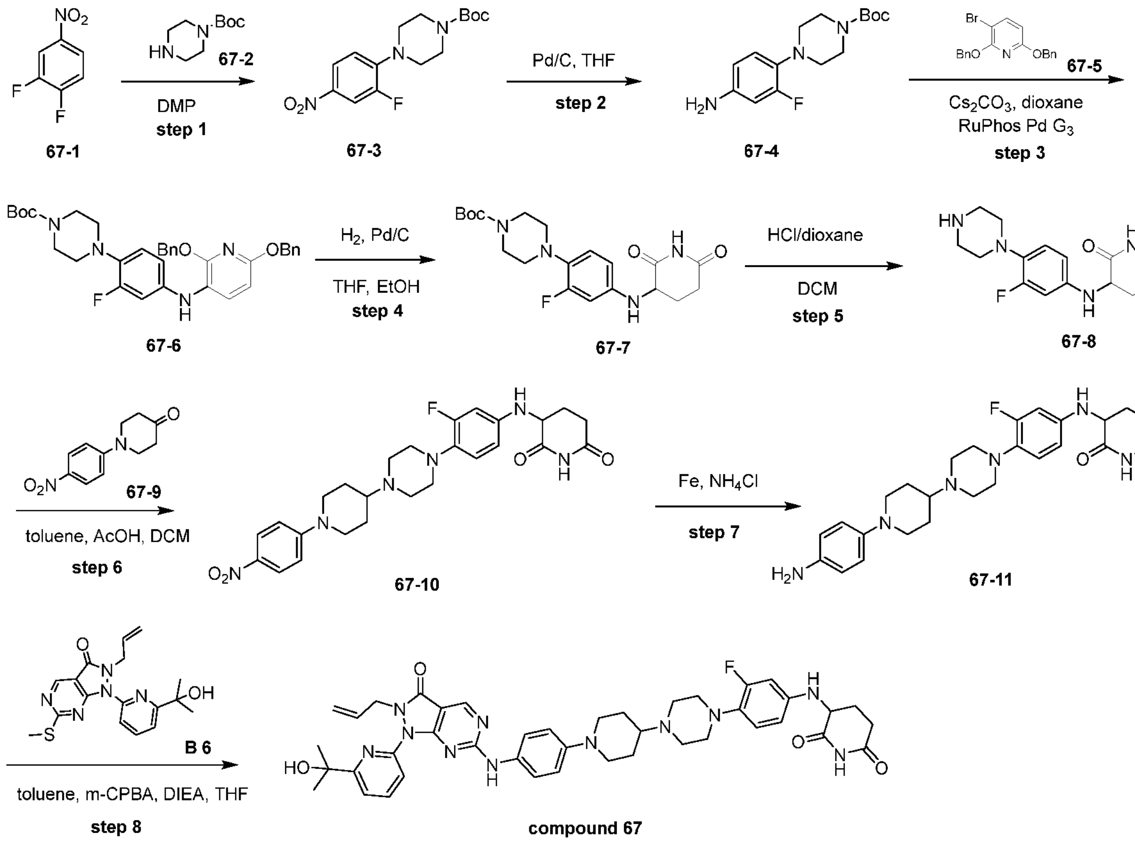


**Step 1: Synthesis of intermediate 67-3**

To a solution of **intermediate 67-1** (3 g, 18.9 mmol) and **intermediate 67-2** (5.27 g, 28.3 mmol) in NMP (10 mL) was stirred at 60°C for 3 hrs. The reaction mixture was partitioned between EtOAc (100 mL) and water (50 mL). The combined organic layers were washed with saturated saturated salt solution, dried over anhydrous $Na_2SO_4$, filtered and concentrated by rotary distillation to give **intermediate 67-3** (6.01 g, crude) as a white solid. The crude product was used into the next step directly.

MS (ESI$^+$): m/z 269.8 [M+H-56]$^+$.

**Step 2: Synthesis of intermediate 67-4**

To a solution of **intermediate 67-3** (6.01 g, 18.5 mmol) in THF (60 mL) was added Pd/C (2 g, 10% purity) then degassed and purged with $H_2$ for 3 times, and then the mixture was stirred at 25 °C for 16 hr under $H_2$ atmosphere. The reaction mixture was filtered and concentrated by rotary distillation to give **intermediate 67-4** (5.66 g, crude) as a white solid. The crude product was used into the next step directly.

MS (ESI$^+$): m/z 240.0 [M-56+H]$^+$.

**Step 3: Synthesis of intermediate 67-6**

To a solution of **intermediate 67-4** (1 g, 3.39 mmol) and **intermediate 67-5** (1.25 g, 3.39 mmol) in 1,4-dioxane (20 mL) was added $Cs_2CO_3$ (3.31 g, 10.2 mmol) and Xphos Pd G3(141.6 mg, 169 μmol) and then the mixture was stirred at 100 °C for 16 hrs. The reaction mixture was partitioned between EtOAc (100 mL) and water (100 mL). The organic phase was washed with saturated saturated salt solution (30 mL × 2), dried over anhydrous $Na_2SO_4$, filtered and concentrated by rotary distillation to give the residue. The residue was purified by column chromatography (PE/EA=20/1 to 3/1) to give **intermediate 67-6** (908 mg, 94% yield) as a white solid.

MS (ESI$^+$): m/z 528.9 [M+H-56]$^+$.

**Step 4: Synthesis of intermediate 67-7**

To a solution of **intermediate 67-6** (908 mg, 1.55 mmol) in THF (10 mL) and EtOH (10 mL) was added Pd/C

(800 mg, 10% purity) and then the mixture was stirred at 25°C for 16 hrs under $H_2$ atmosphere. The reaction mixture was filtered and concentrated by rotary distillation to give the residue. The residue was purified by column chromatography (PE/EA =5/1 to 1/1) to give **intermediate 67-7** (324 mg, 51.3% yield) as a white solid.

MS (ESI+): m/z 350.9 [M-56]+.

**Step 5: Synthesis of intermediate 67-8**

To a solution of **intermediate 67-7** (324 mg, 797 μmol) in DCM (5 mL) was added HCl/dioxane (4 M, 5 mL). The mixture was stirred at 25 °C for 1 h. The reaction mixture was concentrated by rotary distillation to give **intermediate 67-8** (242 mg, crude) as a white solid.

**Step 6: Synthesis of intermediate 67-10**

To a mixture of **intermediate 67-8** (50.0 mg, 146 μmol), **intermediate 67-9** (48.2 mg, 219 μmol) in DCM (2 mL) was added $Et_3N$ (44.3 mg, 438 μmol, 60.9 μL) and the reaction mixture was stirred at 20 °C for 0.5 h. Then the mixture was added AcOH (44.28 mg, 438 μmol, 25.0 μL) and stirred at 20 °C for 0.5 h. Then the mixture was added the $NaBH_3CN$ (18.3 mg, 292 μmol) and stirred at 20°C for 16 hrs. The reaction mixture was partitioned between DCM (50 mL) and water (50 mL). The organic phase was dried over anhydrous $Na_2SO_4$, filtered and concentrated by rotary distillation to give the residue. The residue was purified by column chromatography (Dichloromethane/methanol =1: 0 to 10: 1) to give **intermediate 67-10** (56 mg, 61.7% yield) as a yellow solid.

MS (ESI+): m/z 510.5 [M].

**Step 7: Synthesis of intermediate 67-11**

To a solution of **intermediate 67-10** (56 mg, 110 μmol) in THF (1 mL) and saturated $NH_4Cl$ (0.3 mL) was added iron (61 mg, 1.10 mmol), and the mixture was stirred at 80°C for 1 h. The reaction mixture was filtered and concentrated by rotary distillation to give **intermediate 67-11** (60.0 mg, crude) as a gray solid.

**Step 8: Synthesis of compound 67**

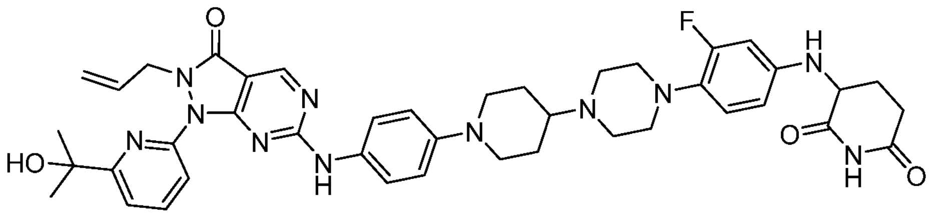

**compound 67**

To a solution of **intermediate 6** (60 mg, 125 μmol) in toluene (2 mL) was added m-CPBA (27.9 mg, 137 μmol). The mixture was stirred at 25 °C for 0.5 h. Then the mixture was added DIEA (32.3 mg, 250 μmol, 43.5 μL) and **intermediate 67-11** (44.6 mg, 125 μmol ) and stirred at 25 °C for 16 hrs. The reaction mixture was quenched by addition aqueous $Na_2S_2O_3$ and $NaHCO_3$ (1:1, 50 mL) at 20°C, and then extracted with EtOAc (50 mL). The aeparated combined organic layers were washed with saturated saturated salt solution, dried over anhydrous $Na_2SO_4$, filtered and concentrated by rotary distillation to give the residue. The residue was purified by HPLC (acetonitrile /water (2% FA)) to give compound **67** (1.00 mg, 1.01% yield) as a yellow solid.

MS (ESI+): m/z 790.5 [M+H]+.

$^1H$ NMR (400MHz, DMSO-$d_6$) $\delta$ 10.80 (s, 1H), 10.23 - 10.11 (m, 1H), 8.83 (s, 1H), 8.38 (s, 1.897H), 8.06 (br s, 1H), 7.75 (d, *J* = 8.4 Hz, 1H), 7.68 - 7.49 (m, 3H), 6.95 (d, *J* = 8.6 Hz, 2H), 6.83 (t, *J* = 9.6 Hz, 1H), 6.52 (d, *J* = 16.0 Hz, 1H), 6.43 (d, *J* = 8.4 Hz, 1H), 5.82 (d, *J* = 7.6 Hz, 1H), 5.73 - 5.59 (m, 1H), 5.37 (s, 1H), 5.00 (d, *J* = 11.2 Hz, 1H), 4.86 - 4.77 (m, *J* = 16.8 Hz, 1H), 4.73 - 4.61 (m, *J* = 5.2 Hz, 2H), 4.29 - 4.21 (m, 1H), 3.70 (d, *J* = 11.2 Hz, 2H), 2.86 (br s, 4H), 2.64 (br s, 4H), 2.59 (d, *J* = 4.4 Hz, 2H), 2.12 - 2.04 (m, 2H), 1.94 - 1.83 (m, 4H), 1.55 (d, *J* = 8.4 Hz, 2H), 1.47 (s, 6H).

The **compound 68** to **compound 70,** and **compound 123, 154** and **155** were synthesized according to the procedures described for **compound 67** using the different intermediates.

| Compound No. | Itermediate No. | MS(ESI$^+$): m/z [M+H]$^+$ | $^1$H NMR (400 MHz, DMSO-$d_6$) $\delta$ |
|---|---|---|---|
| 68 | B 6 | 827.5 | 11.11 (s, 1H), 10.34 - 10.06 (m, 1H), 8.83 (s, 1H), 8.19 - 7.95 (m, 1H), 7.82 - 7.69 (m, 1H), 7.65 - 7.45 (m, 3H), 7.11 - 6.81 (m, 5H), 5.76 - 5.60 (m, 1H), 5.46 - 5.27 (m, 2H), 5.00 (d, *J* = 10.0 Hz, 1H), 4.82 (d, *J* = 16.4 Hz, 1H), 4.68 (d, *J* = 5.2 Hz, 2H), 3.76 - 3.69 (m, 2H), 3.64 (s, 3H), 3.06 - 2.82 (m, 7H), 2.71 - 2.63 (m, 4H), 2.46 - 2.39 (m, 2H), 2.06 - 1.96 (m, 2H), 1.95 - 1.85 (m, 2H), 1.66 - 1.54 (m, 2H), 1.46 (s, 6H). |
| 69 | B 6 | 827.5 | 11.13 (s, 1H), 10.17 (s, 1H), 8.82 (s, 1H), 8.05 (br s, 1H), 7.90- 7.74 (m, 4H), 7.63 - 7.52 (m, 2H), 7.42 - 7.13 (m, 1H), 7.04 - 6.86 (m, 2H), 5.76 - 5.59 (m,1H), 5.34 (s, 1H), 5.14 (dd, *J* = 5.2, 12.8 Hz, 1H), 4.99 (d, *J* = 10.4 Hz, 1H), 4.82 (d, *J* = 17.2 Hz,1H), 4.73 - 4.64 (m, 2H), 3.78 - 3.65 (m, 2H), 3.05 (br s, 2H), 2.93 - 2.84 (m, 1H), 2.80 - 2.70(m, 1H), 2.65 - 2.55 (m, 4H), 2.33 (br s, 2H), 2.12 - 1.98 (m, 2H), 1.90 - 1.78 (m, 4H), 1.77 -1.66 (m, 2H), 1.66 - 1.55 (m, 2H), 1.47 - 1.45 (m, 6H). |
| 70 | B 6 | 771.6 | 10.87 - 10.70 (m, 1H), 10.35 - 9.98 (m, 1H), 8.82 (s, 1H), 8.31 (s, 1H), 8.17 - 7.95 (m, 1H), 7.79 - 7.70 (m, 1H), 7.66 - 7.47 (m, 3H), 7.02 - 6.85 (m, 4H), 6.65 - 6.47 (m, 2H), 5.67 - 5.63 (m, 1H), 5.35 (br s, 1H), 5.02 - 4.92 (m, 1H), 4.86 - 4.77 (m, 1H), 4.74 - 4.64 (m, 2H), 4.31 - 4.21 (m, 1H), 3.79 - 3.58 (m, 2H), 3.04 - 2.94 (m, 2H), 2.78 - 2.69 (m, 1H), 2.66 - 2.58 (m, 4H), 2.45 - 2.35 (m, 2H), 2.29 - 2.20 (m, 2H), 2.14 - 2.05 (m, 1H), 1.87 - 1.82 (m, 2H), 1.76 - 1.65 (m, 2H), 1.62 - 1.50 (m, 4H), 1.46 (s, 6H). |
| 123 | B 6 | 802.5 | 10.77 (s, 1H), 10.16 (br s, 1H), 8.83 (s, 1H), 8.05 (br s, 1H), 7.75 (d, *J* = 7.6 Hz, 1H), 7.64 - 7.49 (m, 3H), 6.92 (d, *J* = 8.8 Hz, 2H), 6.52 - 6.33(m, 3H), 5.75 - 5.50 (m, 2H), 5.35 (s, 1H), 4.99 (d, *J* = 10.4 Hz, 1H), 4.86 - 4.77 (m, 1H), 4.68 (d, *J* = 5.0 Hz,2H), 4.24 - 4.14 (m, 1H), 3.79 (s, 4H), 3.55 (br s, 2H), 3.53 - 3.49 (m, 2H), 3.52 - 3.48(m, 1H), 2.79 - 2.63 (m, 4H), 2.58 (d, *J* = 4.2 Hz, 1H), 2.54 (br s, 1H), 2.21 (br s, 1H), 2.16 - 2.00 (m, 1H), 1.88 - 1.78 (m, 1H), 1.73 (br d, *J* = 11.1 Hz, 2H), 1.46 (s, 6H), 1.27 (br d, *J* = 12.2 Hz, 2H) |
| 154 | B 15-B | 828.5 | 10.78 (s, 1H), 10.15 (br s, 1H), 8.82 (s, 1H), 7.94 (br s, 1H), 7.69 (d, *J* = 8.0 Hz, 1H), 7.57 (br s, 2H), 6.91 (d, *J* = 8.8 Hz, 2H), 6.53 - 6.29 (m, 4H), 5.72 - 5.61 (m, 1H), 5.57 (d, *J* = 7.6 Hz, 1H), 5.08 (s, 1H), 4.99 (d, *J* = 10.4 Hz, 1H), 4.90 - 4.72 (m, 2H), 4.57 (br s, 1H), 4.19 (br t, *J* = 11.8 Hz, 1H), 3.77 (s, 4H), 3.49 (br s, 4H), 2.96 (d, *J* = 8.8 Hz, 1H), 2.85 - 2.70 (m, 4H), 2.69 - 2.57 (m, 4H), 2.25 - 2.15 (m, 1H), 2.07 (br d, *J* = 8.8 Hz, 2H), 2.03 - 1.96 (m, 2H), 1.93 - 1.84 (m, 2H), 1.72 - 1.68 (m, 2H), 0.87 (br t, *J* = 7.2 Hz, 3H) |
| 155 | B 15-B | | 10.79 (s, 1H), 10.15 (br s, 1H), 8.82 (s, 1H), 7.94 (br s, 1H), 7.70 (d, *J* = 7.9 Hz, 1H), 7.58 (br s, 2H), 6.92 (d, *J* = 8.8 Hz, 2H), 6.88 - 6.78 (m, 1H), 6.59 - 6.46 (m, 1H), 6.44 - 6.37 (m, 1H), 5.84 - 5.76 (m, 1H), 5.72 - 5.59 (m, 1H), 5.07 (s, 1H), 4.99 (d, *J* = 9.6 Hz, 1H), 4.85 (d, *J* = 17.6 Hz, 1H), 4.76 (br s, 1H), 4.58 (br s, 1H), 4.25 (s, 1H), 3.18 - 3.14 (m, 2H), 3.10 (br s, 4H), 2.97 (d, *J* = 15.6 Hz, 1H), 2.79 (d, *J* = 8.8 Hz, 1H), 2.73 (s, 1H), 2.67 (br s, 6H), 2.61 - 2.58 (m, 2H), 2.25 - 2.17 (m, 1H), 2.09 - 2.00 (m, 2H), 1.91 - 1.81 (m, 4H), 1.76 - 1.67 (m, 1H), 1.58 (d, *J*=9.2 Hz, 2H), 0.90 - 0.84 (m, 3H) |

**Example 71: compound 71**

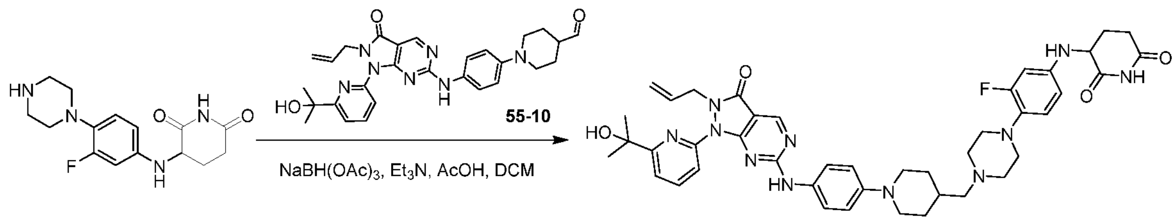


67-8

55-10

compound 71

**Step 1: synthesis of compound 71**

To a solution of **intermediate67-8** (100 mg, 292 μmol) and **intermediate 55-10** (160 mg, 292 μmol) in DCM (2 mL) was added $Et_3N$ (88.6 mg, 875 μmol, 122 μL), the mixture was stirred at 25°C for 0.5 h. Then the mixture was added AcOH (52.6 mg, 875 μmol, 50 μL) and stirred at 25°C for 0.5 h. Then the mixture was added $NaBH(OAc)_3$ (124 mg, 583 μmol) and stirred at 25°C for 16 hrs. The reaction mixture was partitioned between DCM (10 mL) and $H_2O$ (10 mL). The organic phase was dried over anhydrous $Na_2SO_4$, filtered and concentrated by rotary distillation to give the residue. The residue was purified by HPLC (acetonitrile / water (2%FA)) to give **compound 71** (8.98 mg, 3.64% yield) as a yellow solid.

MS (ESI$^+$): m/z 804.5 $[M+H]^+$.

$^1$H NMR (400 MHz, DMSO-$d_6$) δ 10.78 (s, 1H), 10.13 (br s, 1H), 8.81 (s, 1H), 8.32 (s, 0.337 H), 8.04 (br s, 1H), 7.74 (d, *J* = 7.2 Hz, 1H), 7.64 - 7.47 (m, 3H), 6.97 - 6.89 (m, 2H), 6.87 - 6.77 (m, 1H), 6.56 - 6.48 (m, 1H), 6.46 - 6.38 (m, 1H), 5.80 (d, *J* = 7.6 Hz, 1H), 5.73 - 5.58 (m, 1H), 5.35 (s, 1H), 4.99 (d, *J* = 10.0 Hz, 1H), 4.82 (d, *J* = 18.0 Hz, 1H), 4.67 (d, *J* = 5.2 Hz, 2H), 4.30 - 4.20 (m, 1H), 3.66 - 3.61 (m, 2H), 2.86 (br s, 4H), 2.76 - 2.67 (m, 1H), 2.65 - 2.57 (m, 2H), 2.48 - 2.46 (m, 5H), 2.21 (d, *J* = 6.8 Hz, 2H), 2.12 - 2.00 (m, 1H), 1.89 - 1.76 (m, 3H), 1.66 (br s, 1H), 1.46 (s, 6H), 1.30 - 1.24 (m, 2H).

The **compound 72, 91, 92, 94, 140** and **147** were synthesized according to the procedures described for **compound 71** using the different intermediates.

| Compound No. | Intermediate No. | MS(ESI$^+$): m/z $[M+H]^+$ | $^1$H NMR (400 MHz, DMSO-$d_6$) δ |
|---|---|---|---|
| **72** | 71-2 | 785.6 | 10.87 - 10.76 (m, 1H), 10.52 (br s, 1H), 10.35 - 10.06 (m, 1H), 8.94 (s, 1H), 8.10 - 8.02 (m, 1H), 7.94 - 7.85 (m, 2H), 7.84 - 7.70 (m, 3H), 7.65 (d, *J* = 4.0 Hz, 1H), 7.14 - 6.97 (m, 2H), 6.76 - 6.64 (m, 2H), 5.74 - 5.60 (m, 1H), 5.04 - 4.95 (m, 1H), 4.86 - 4.78 (m, 1H), 4.74 - 4.62 (m, 2H), 4.37 - 4.26 (m, 1H), 3.42 - 3.31 (m, 7H), 3.11 - 2.97 (m, 3H), 2.84 - 2.54 (m, 4H), 2.24 - 2.04 (m, 5H), 2.02 - 1.80 (m, 5H), 1.46 (s, 6H) |
| **91** | 55-10 | 832.6 | 10.78 (s, 1 H), 10.14 (s, 1 H), 8.81 (s, 1 H), 8.14 (s, 1 H), 8.04 (s, 1 H), 7.74 (d, *J* = 8.00 Hz, 1 H), 7.48 - 7.63 (m, 3 H), 6.92 (d, *J* = 8.80 Hz, 2 H), 6.82 (t, *J* = 9.20 Hz, 1 H), 6.45 - 6.54 (m, 1 H), 6.40 (d, *J* = 8.80 Hz, 1 H), 5.79 (d, *J* = 7.60 Hz, 1 H), 5.59 - 5.72 (m, 1 H), 5.33 (s, 1 H), 4.98 (d, *J* = 10.00 Hz, 1 H), 4.82 (d, J=16.00 Hz, 1 H), 4.67 (d, *J* = 4.80 Hz, 2 H), 4.19 - 4.29 (m, 1 H), 3.63 (d, *J* = 11.60 Hz, 2 H), 3.16 (d, *J* = 10.80 Hz, 2 H), 2.69 - 2.79 (m, 1 H), 2.55 - 2.64 (m, 3 H), 2.42 (s, 1 H), 2.32 (s, 2 H), 2.25 (s, 3 H), 2.07 (d, *J* = 8.40 Hz, 1 H), 1.69 - 1.90 (m, 5 H), 1.58 (d, *J* = 9.60 Hz, 4 H), 1.45 (s, 6 H), 1.23 (s, 2 H). |
| **92** | 55-10 | 808.6 $[M+Na]^+$ | 10.78 (s, 1H), 10.17 (br s, 1H), 8.83 (s, 1H), 8.12 - 8.00 (m, 1H), 7.75 (d, *J* = 7.2 Hz, 1H), 7.62 - 7.53 (m, 3H), 6.93 (d, *J* = 8.4 Hz, 2H), 6.76 (d, *J* = 8.8 Hz, 2H), 6.61 (d, *J* = 8.8 Hz, 2H), 5.71 - 5.61 (m, 1H), 5.40 (d, *J* = 7.2 Hz, 1H), 5.35 (s, 1H), 5.01 - 4.97 (m, 1H), 4.86 - 4.78 (m, 1H), 4.71 - 4.66 (m, 2H), 4.26 - 4.15 (m, 1H), 3.68 - 3.59 (m, 2H), 3.43 - 3.37 (m, 4H), 3.00 - 2.90 (m, 4H), 2.78 - 2.66 (m, 2H), 2.62 - 2.56 (m, 4H), 2.24 - 2.19 (m, 2H), 2.13 - 2.06 (m, 1H), 1.90 - 1.77 (m, 4H), 1.46 (s, 6H) . |

(continued)

| Compound No. | Intermediate No. | MS(ESI$^+$): m/z [M+H]$^+$ | $^1$H NMR (400 MHz, DMSO-$d_6$) $\delta$ |
|---|---|---|---|
| **94** | B 2-1 | 824.4 | 10.79 (s, 1H), 10.75 - 10.53 (m, 1H), 9.09 (s, 1H), 8.14 (s, 0.566H), 7.84 (br s, 1H), 7.80 - 7.57 (m, 6H), 7.13 - 6.95 (m, 3H), 6.88 - 6.80 (m, 1H), 6.56 - 6.47 (m, 1H), 6.46 - 6.38 (m, 1H), 5.86 - 5.74 (m, 1H), 4.67 - 4.35 (m, 1H), 4.31 - 4.16 (m, 1H), 4.04 - 3.84 (m, 1H), 3.68 (br s, 2H), 2.87 (br s, 4H), 2.78 - 2.72 (m, 2H), 2.42 - 2.37 (m, 4H), 2.25 - 2.17 (m, 2H), 2.12 - 1.96 (m, 2H), 1.87 - 1.76 (m, 3H), 1.69 (br s, 1H). |
| **140** | B 46 | 856.6 | 10.82 (s, 1H), 10.23 (s, 1H), 7.98 - 7.94 (m, 1H), 7.79 (d, *J* = 8.0 Hz, 2H), 7.65 - 7.50 (m, 5H), 7.31 (d, *J* = 8.0 Hz, 1H), 6.98 - 6.93 (m, 2H), 5.67 - 5.55 (m, 1H), 4.60 - 4.96 (m, 1H), 4.85 - 4.80 (m, 1H), 4.48 - 4.41 (m, 3H), 4.38 - 4.32 (m, 2H), 3.59 - 3.56 (m, 5H), 3.41 - 3.85 (m, 1H), 3.23 - 3.19 (m, 2H), 3.10 - 3.08 (m, 2H), 2.95 - 2.92 (m, 2H), 2.80 - 2.71 (m, 1H), 2.59 - 2.57 (m, 1H), 2.42 - 2.36 (m, 1H), 2.23 - 2.18 (m, 2H), 2.12 - 1.85 (m, 4H), 1.45 (s, 6H). |
| **147** | B 46 | 819.37 | 10.79 (s, 1H), 10.29 - 10.02 (m, 1H), 8.82 (s, 1H), 8.05 (s, 1H), 7.75 (d, *J* = 8.4 Hz, 1H),7.64 - 7.54 (m, 2H), 7.05 - 6.88 (m, 3H), 6.76 (s, 1H), 6.61 (d,*J* = 8.8 Hz, 1H), 5.88 (d, *J* = 8.4 Hz, 1H), 5.74 - 5.63 (m,1H), 5.34 (s, 1H), 4.99 (d, *J* = 10.0 Hz, 1H), 4.82 (d, *J* = 17.2 Hz, 1H), 4.68 (s, 2H), 4.30 (s, 1H), 3.64 (d, *J* = 12.0 Hz, 2H), 2.92 - 2.77 (m, 4H), 2.70 - 2.59 (m, 5H), 2.33 (s, 2H), 2.27 - 2.17 (m, 2H), 2.12 - 1.99 (m, 2H), 2.07 (d,*J* = 8.4 Hz, 1H),1.93 - 1.76 (m, 3H), 1.67 (s, 1H), 1.46 (s, 6H), 1.23 (s, 2H). |

**Example 73: compound 73**

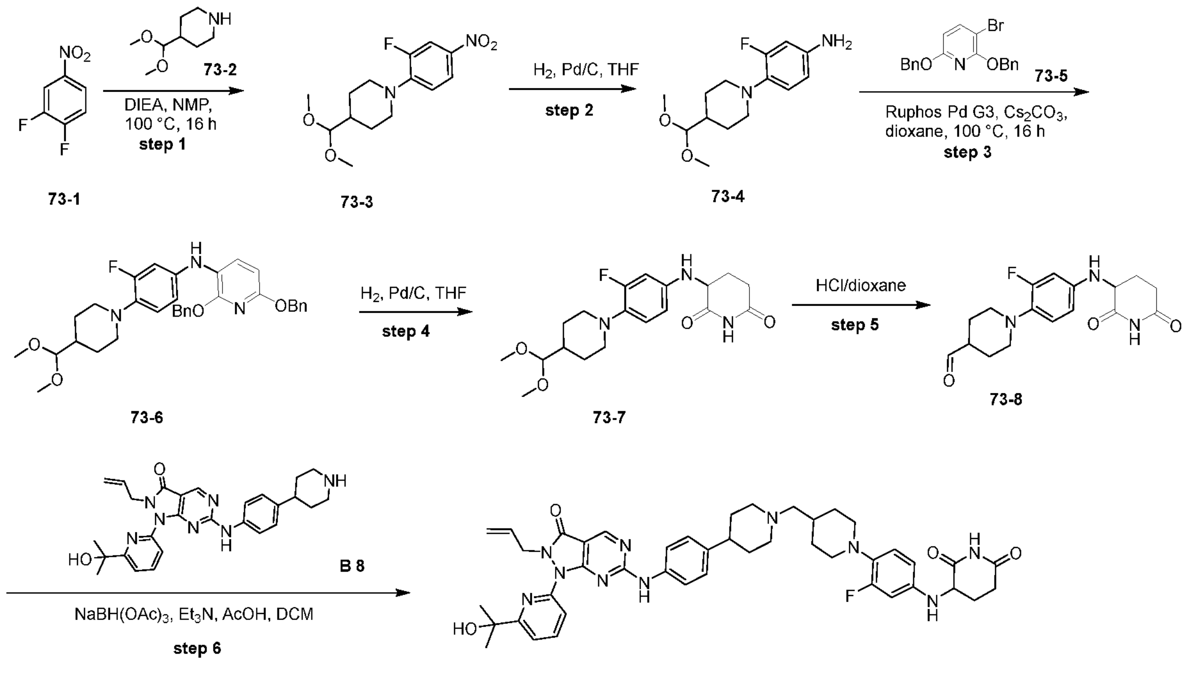


compound 73

**Step 1: synthesis of intermediate 73-3**

To a solution of **intermediate 73-2** (3 g, 18.84 mmol,) and **intermediate 73-1** (4.50 g, 28.26 mmol) in DMSO (10 mL). The mixture was stirred at 90 °C for 16 hrs. The reaction mixture was partitioned between EA (20 mL) and water (20 mL). The organic phase was separated, dried over anhydrous $Na_2SO_4$, filtered and concentrated under reduced pressure to give the residue. The residue was purified by column chromatography (DCM: MeOH = 10:1). **Intermediate 73-3** (6.93 g, crude) was obtained as a white solid, which is used for the next step directly.
MS ($ESI^+$): m/z 298.31 $[M+H]^+$.

**Step 2: Synthesis of intermediate 73-4**

To a solution of **intermediate 73-3** (6.39 g, 21.4 mmol) in THF (30 mL) was added Pd/C (4 g, 10% purity) The mixture was stirred at 20 °C for 16 hrs under $H_2$ atomosphere. The reaction mixture was filtered and concentrated under reduced pressure to give **intermediate 73-4** (7.42 g, crude) as a red solid which was used to the next step directly. MS ($ESI^+$): m/z 268.33 $[M+H]^+$.

**Step 3: Synthesis of intermediate 73-6**

A mixture of **intermediate 73-4** (2.63 g, 9.80 mmol) and **intermediate 73-5** (3.63 g, 9.80 mmol) in 1,4-dioxane (30 mL) was added $Cs_2CO_3$ (9.58 g, 29.41 mmol) and [2-(2-aminophenyl)phenyl]-methylsulfonyloxy-palladium- dicyclohexyl-[2-(2,6-diisopropoxyphenyl)phenyl]phosphane (410 mg, 490 μmol ) and then the mixture was stirred at 100 °C for 16 hrs under $N_2$ atmosphere. The reaction mixture was partitioned between EA (20 mL) and water (20 mL). The organic phase was separated, dried over anhydrous $Na_2SO_4$, filtered and concentrated under reduced pressure to give the residue. The residue was purified by column chromatography (Petroleum ether/Ethyl acetate=5/1 to 3/1). **Intermediate 73-6** (3.4 g, 62.2% yield) was obtained as a red solid.
MS ($ESI^+$): m/z 557.65 $[M+H]^+$.

**Step 4: Synthesis of intermediate 73-7**

To a solution of **intermediate 73-6** (3.39 g, 6.08 mmol) in THF (20 mL) was added Pd/C (2 g, 10% purity) and purged with $H_2$ for 3 times, and then the mixture was stirred at 20 °C for 16 hrs under $H_2$ atmosphere. The reaction mixture was filtered and concentrated under reduced pressure to give the residue. The residue was purified by column chromatography (Petroleum ether/Ethyl acetate=3/1 to 0/1) to give **intermediate 73-7** (699 mg, 30.00% yield) as a pale green solid.
MS ($ESI^+$): m/z 379.42 $[M+H]^+$.

**Step 5: Synthesis of intermediate 73-8**

To a solution of **intermediate 73-7** (293 mg, 772 μmol) in DCM (5 mL) was added HCl/dioxane (5 mL). The mixture was stirred at 20°C for 3 hrs. The reaction mixture was filtered and concentrated under reduced pressure to give **intermediate 73-8** (350 mg, crude) as a yellow solid, which was used for the next step directly.
MS ($ESI^+$): m/z 333.365 $[M+H]^+$.

**Step 6: Synthesis of compound 73**

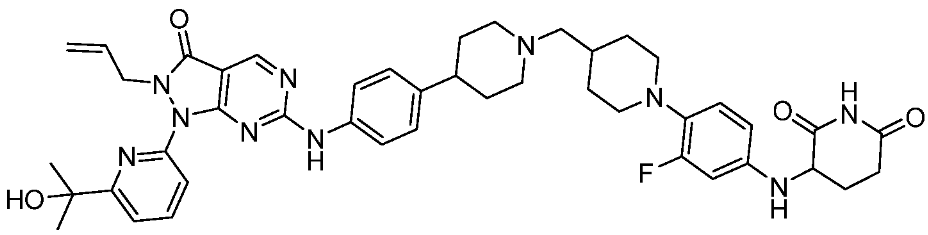

**compound 73**

To a solution of **intermediate 73-8** (80.0 mg, 216 μmol, HCl) and **intermediate B 8** (113 mg, 216 μmol, HCl

salt) in DCM (2 mL) was added TEA (87.6 mg, 865 μmol) and the mixture was stirred at 25°C for 30 min, then AcOH (38.97 mg, 648.97 μmol,) was added and pH was maintain between5 to 6. The reaction mixture was stirred at 25°C for 30 min,then was added $NaBH(OAc)_3$ (138 mg, 649 μmol) and stirred at 25 °C for 16 hrs. The reaction mixture was filtered and concentrated under reduced pressure to give the residue. The residue was purified by prep-HPLC (FA condition) to give **compound 73** (81 mg, 46.6% yield) as a white solid.

MS ($ESI^+$): m/z 803.6 $[M+H]^+$.

$^1H$ NMR (400MHz, DMSO-$d_6$) $\delta$ 10.79 (s, 1H), 10.27 (br s, 1H), 8.87 (s, 1H), 8.20 (s, 1.07H), 8.07 (t, *J* = 7.6 Hz, 1H), 7.86 - 7.52 (m, 4H), 7.21 (d, *J* = 8.4 Hz, 2H), 6.83 (t, *J* = 9.6 Hz, 1H), 6.50 (d, *J* = 14.8 Hz, 1H), 6.41 (d, *J* = 8.4 Hz, 1H), 5.79 (d, *J* = 7.6 Hz, 1H), 5.73 - 5.64 (m, 1H), 5.35 (br s, 1H), 4.99 (d, *J* = 10.4 Hz, 1H), 4.82 (d, *J* = 17.2 Hz, 1H), 4.68 (d, *J* = 5.6 Hz, 2H), 4.32 - 4.17 (m, 1H), 3.12 (d, *J* = 11.2 Hz, 2H), 2.96 (d, *J* = 10.4 Hz, 2H), 2.81 - 2.63 (m, 2H), 2.54 (br s, 2H), 2.46 - 2.41 (m, 1H), 2.21 (d, *J* = 6.8 Hz, 2H), 2.08 (s, 1H), 1.98 (t, *J* = 10.8 Hz, 2H), 1.90 - 1.81 (m, 1H), 1.80 - 1.69 (m, 4H), 1.62 (d, *J* = 9.2 Hz, 2H), 1.46 (s, 6H), 1.32 - 1.16 (m, 2H).

The **compound 74, compound 83** to **compound 88,** and **compound 109, 122, 125** and **158** were synthesized according to the procedures described for **compound 73** using the different intermediates.

| Compound No. | Intermediate No. | MS($ESI^+$): m/z $[M+H]^+$ | $^1H$ NMR (400 MHz, DMSO-$d_6$) $\delta$ |
|---|---|---|---|
| 74 | B 7 | 826.6 $[M+Na]^+$ | 10.79 (s, 1H), 10.17 (br s, 1H), 8.83 (s, 1H), 8.28 (s, 1H), 8.05 (br s,1H), 7.75 (d, *J* = 7.6 Hz, 1H), 7.60 (d, *J* = 7.6 Hz, 3H), 6.92 (d, *J* = 8.8 Hz, 2H), 6.83 (t, *J* = 9.2 Hz, 1H),6.49 (d, *J* = 14.8 Hz, 1H), 6.44 - 6.38 (m, 1H), 5.80 (d, *J* = 8.0 Hz, 1H), 5.73 - 5.58 (m, 1H), 5.35 (br s,1H), 4.99 (d, *J* = 9.2 Hz, 1H), 4.82 (d, *J* = 16.0 Hz, 1H), 4.68 (d, *J* = 4.6 Hz, 2H), 4.32 - 4.16 (m, 1H),3.10 (br s, 6H), 2.78 - 2.68 (m, 1H), 2.58 (br s, 2H), 2.55 (br s, 4H), 2.23 (d, *J* = 6.8 Hz, 2H), 2.08 (td,*J* = 4.2, 8.4 Hz, 1H), 1.91 - 1.74 (m, 3H), 1.62 (br s, 1H), 1.46 (s, 6H), 1.32 - 1.20 (m, 2H). |
| 83 | B 3 | 824.4 | 10.79 (s, 2H), 10.20 - 9.06 (m, 1H), 8.16 (br s, 0.279H), 8.02 - 7.52 (m, 6H), 7.16 - 6.79 (m, 4H), 6.58 - 6.34 (m, 2H), 5.80 (d, J = 7.6 Hz, 1H), 4.32 - 4.15 (m, 1H), 3.12 (d, J = 9.8 Hz, 6H), 2.78 - 2.71 (m, 1H), 2.59 (br s, 2H), 2.47 - 2.41 (m, 5H), 2.23 (br s, 2H), 2.08 (d, J = 8.8 Hz, 1H), 1.90 - 1.72 (m, 3H), 1.64 (br s, 1H), 1.26 (d, J = 17.6 Hz, 2H). |
| 84 | B 8 | 786.6 | 10.79 (s, 1H), 10.28 (s, 1H), 8.88 (s, 1H), 8.35 (s, 1H), 8.08 (s, 1H),7.76 (d, J = 7.6 Hz, 1H), 7.70 - 7.60 (m, 4H), 7.22 (d, J = 8.4 Hz, 2H), 7.07 - 6.99 (m, 1H), 6.68 (d, J = 8.8 Hz, 1H), 5.76 - 5.61 (m, 1H), 5.38 (d, J = 7.6 Hz, 2H), 5.00 (d, J = 9.6 Hz, 1H), 4.82 (d, J = 18 Hz, 1H), 4.69 (d, J = 5.2 Hz, 2H), 4.20 (br s, 1H), 4.01 (d, J = 12.8 Hz, 2H), 2.95 (d, J = 10.4 Hz, 2H),2.78 - 2.71 (m, 1H), 2.65 - 2.57 (m, 1H), 2.20 - 2.07 (m, 4H), 2.04 - |
| 85 | B 8 | 785.6 | 10.79 (s, 1H), 10.33 (br s, 1H), 8.99 - 8.84 (m, 1H), 8.14 (s, 0.413H), 8.11 - 7.94 (m, 1H), 7.76 (d, J = 7.6 Hz, 1H), 7.69 ( d, J = 7.2 Hz, 2H), 7.63 (d, J = 7.6 Hz, 1H), 7.22( d, J=7.6 Hz, 2H), 6.77 ( d, J = 8.8 Hz, 2H), 6.60 (d, J = 8.8 Hz, 2H), 5.79 - 5.59 (m, 1H), 5.47 - 5.31 (m, 2H), 4.99 (d, J = 9.6 Hz, 1H), 4.81 ( d, J = 16.4 Hz, 1H), 4.68 ( d, J = 5.6 Hz, 2H), 4.30 - 4.13 (m, 1H), 3.29 (br s, 2H), 2.79 - 2.71 (m, 1H), 2.62 -2.52 (m, 5H), 2.44 (br s, 5H), 2.10 (d, J = 4.4, 13.2Hz, 1H), 1.99 - 1.71 (m, 8H), 1.46 (s, 6H),1.31 (d, J = 10.4 Hz, 2H). |
| 86 | B 7-B | 786.6 | 10.77 (s, 1H), 10.18 (s, 1H), 8.83 (s, 1H), 8.28 (s, 1H), 8.06 (s, 1H), 7.76 (d, J = 8.0 Hz, 1H), 7.61 (d, J = 7.6 Hz, 3H), 6.93 (d, J = 9.2 Hz, 2H), 6.76 (d, J = 8.8 Hz, 2H), 6.60 (d, J = 8.8 Hz, 2H), 5.73 - 5.61 (m, 1H), 5.40 - 5.30 (m, 2H), 5.00 (d, J = 9.6 Hz,1H), 4.83 (d, J = 16.0 Hz, 1H), 4.68 (d, J = 5.6 Hz, 2H), 4.20 (d, J = 7.2 Hz, 1H), 3.11 (s, 4H), 2.78 - 2.68(m, 2H), 2.62 - 2.57 (m, 1H), 2.46 - 2.40 (m, 4H), 2.36 - 2.31 (m, 2H), 2.23 (d, J = 7.2 Hz, 2H), 2.15 - 2.05 (m, 1H), 1.90 - 1.74 (m, 3H), 1.62 (s, 1H), 1.46 (s, 6H), 1.29 - 1.19 (m, 2H). |

(continued)

| Compound No. | Intermediate No. | MS(ESI+): m/z [M+H]+ | $^1$H NMR (400 MHz, DMSO-$d_6$) $\delta$ |
|---|---|---|---|
| 87 | B 7-B | 809.6 | 10.79 (s, 1H), 10.18 (br s, 1H), 8.83 (s, 1H), 8.15 (s, 0.419H), 8.05 (br s, 1H), 7.78 - 7.72 (m, 1H), 7.69 (br s, 1H), 7.66 - 7.40 (m, 4H), 7.07 - 7.00 (m, 1H), 6.97 - 6.87 (m, 1H), 6.97 - 6.87 (m, 1H), 6.72 - 6.64 (m, 1H), 5.73 - 5.59 (m, 1H), 5.42 - 5.31 (m, 2H), 5.03 - 4.95 (m, 1H), 4.86 - 4.77 (m, 1H), 4.68 (br s, 2H), 4.20 (br s, 1H), 4.07 - 3.96 (m, 2H), 3.11 (br s, 4H), 2.78 - 2.66 (m, 2H), 2.63 - 2.57 (m, 2H), 2.63 - 2.54 (m, 1H), 2.26 - 2.17 (m, 2H), 2.16 - 2.02 (m, 2H), 1.97 - 1.61 (m, 5H), 1.46 (s, 6H), 1.17 - 1.09 (m, 1H), 1.21 - 104 (m, 1H). |
| 88 | B 7-B | 820.6 | 10.78 (s, 1H), 10.16 (br s, 1H), 8.82 (s, 1H), 8.05 (br s, 1H), 7.75 (d, J = 7.6 Hz, 1H), 7.60 (d, J = 7.6 Hz, 3H), 6.93 (t, J = 8 Hz, 3H), 6.75 (d, J =2.4 Hz, 1H), 6.60 (dd, J = 2.4, 8.8 Hz, 1H), 5.84 (d, J = 8.0 Hz, 1H), 5.74 - 5.59 (m, 1H), 5.33 (br s, 1H), 4.99 (d, J = 10.4 Hz, 1H), 4.82 (d, J = 17.2 Hz, 1H), 4.68 (d, J = 4.8 Hz, 2H), 4.39 - 4.20 (m, 1H), 3.17 - 3.00 (m, 6H), 2.82 - 2.70 (m, 1H), 2.58 (br s, 2H), 2.49 - 2.44 (m, 5H), 2.24 (d, J = 7.2 Hz, 2H), 2.13 - 2.00 (m, 1H), 1.92 - 1.75 (m, 3H), 1.64 (br s, 1H), 1.46 (s, 6H), 1.32 - 1.23 (m, 2H) . |
| 109 | 73-8; 108-5 | 829.6 | 10.80 (s, 1H), 10.26 (br s, 1H), 8.88 (s, 1H), 7.95 (br s, 1H), 7.77 - 7.58 (m, 3H), 7.21 (d, *J* = 8.4 Hz, 2H), 6.84 (t, *J* = 9.2 Hz, 1H), 6.50 (d, *J* = 15.2 Hz, 1H), 6.41 (d, *J* = 8.4 Hz, 1H), 5.81 (d, *J* = 7.6 Hz, 1H), 5.76 - 5.60 (m, 1H), 5.10 (s, 1H), 5.00 (d, *J* = 10.0 Hz, 1H), 4.89 - 4.69 (m, 2H), 4.64 - 4.49 (m, 1H), 4.26 (br s, 1H), 3.12 (d, *J* = 10.8 Hz, 2H), 3.03 - 2.88 (m, 1H), 2.83 - 2.70 (m, 2H), 2.60 - 2.53 (m, 4H), 2.41 (br s, 4H), 2.27 - 2.13 (m, 3H), 2.12 - 1.53 (m, 12H), 1.35 - 1.25 (m, 2H), 0.87 (t, *J* = 7.2 Hz, 3H). |
| 122 | B 7-B | 776.4 | 10.77 (s, 1H), 10.24 (s, 1H), 8.82 (s, 1H), 8.14 - 7.97 (m, 1H), 7.80 - 7.72 (m, 1H), 7.68 - 7.51 (m, 3H), 6.92 (d, *J* = 9.2 Hz, 2H), 6.54 - 6.45 (m, 1H), 6.44 - 6.33 (m, 2H), 5.73 - 5.59 (m, 1H), 5.54 (d, *J* = 7.6 Hz, 1H), 5.36 - 5.26 (m, 1H), 5.03 - 4.94 (m, 1H), 4.88 - 4.77 (m, 1H), 4.75 - 4.63 (m, 2H), 4.27 - 4.12 (m, 1H), 3.88 (t, *J* = 6.8 Hz, 2H), 3.09 (br s, 4H), 2.96 - 2.82 (m, 1H), 2.64 - 2.58 (m, 7H), 2.12 - 2.04 (m, 1H), 2.03 - 1.93 (m, 1H), 1.87 - 1.77 (m, 1H), 1.46 (s, 6H), 1.30 - 1.26 (m, 2H) |
| 125 | B 29B | 822.5 | 10.79 (s, 1H), 10.38 (br s, 1H), 8.89 (s, 1H), 8.22 (br s, 1H), 8.03 (t, *J* = 7.9 Hz, 1H), 7.75 (d, *J* = 8.0 Hz, 2H), 7.64 (d, *J* = 7.6 Hz, 1H), 7.37 (d, *J* = 8.7 Hz, 1H), 7.08 - 6.95 (m, 1H), 6.83 (t, *J* = 9.3 Hz, 1H), 6.57 - 6.36 (m, 2H), 5.80 (d, *J* = 7.6 Hz, 1H), 5.73 - 5.49 (m, 1H), 5.36 (br s, 1H), 4.99 (d, *J* = 10.1 Hz, 1H), 4.82 (d, *J* = 16.9 Hz, 1H), 4.69 (d, *J* = 5.4 Hz, 2H), 4.30 -4.21 (m, 1H), 3.12 (d, *J* = 11.1 Hz, 2H), 2.98 (br s, 4H), 2.80 - 2.66 (m, 1H), 2.62 - 2.54 (m, 2H),2.50 - 2.48 (m, 5H), 2.23 (d, *J* = 6.7 Hz, 2H), 2.13 - 2.03 (m, 1H), 1.93 - 1.72 (m, 3H), 1.61 (br s,1H), 1.46 (s, 6H), 1.33 - 1.19 (m, 2H) |
| 158 | B 54 | 805.5 | 10.81 (s, 1H), 10.28 (s, 1H), 8.85 (s, 1H), 7.90 (m, 1H),7.51 (m, 2H), 7.24 (s, 1H), 6.92 (m, 2H), 6.87 - 6.80 (m, 1H), 6.50 (m, 1H), 6.41 (m, 1H), 5.81 (m, 1H), 5.77 - 5.64 (m, 1H), 5.22 - 5.06 (m, 2H),5.02 (m, 1H), 4.49 (br s, 2H), 4.26 (m, 1H), 3.10 (br s, 6H), 2.86 - 2.66 (m, 1H), 2.56 (m, 6H), 2.23 (m, 2H), 2.06 (m, 1H), 1.92 - 1.74 (m, 4H),1.62 (br s, 1H), 1.30 (m, 6H), 1.24 (br s, 2H). |

**Example 75: compound 75**

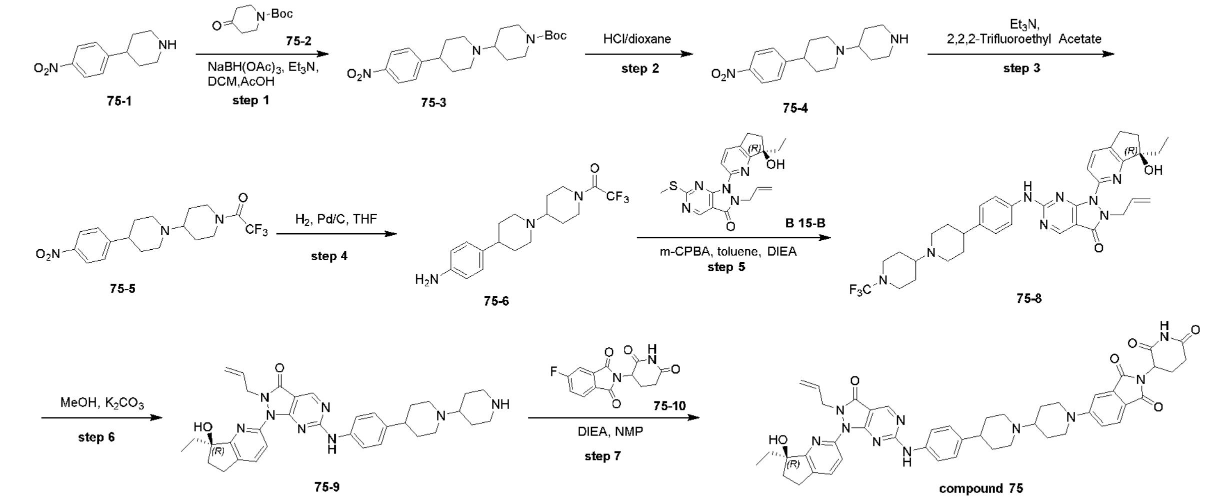


**Step 1: Synthesis of intermediate 75-3**

To a solution of **intermediate 75-1** (300 mg, 1.45 mmol) and **intermediate 75-2** (434.74 mg, 2.18 mmol) in DCM (5 mL) was added $Et_3N$ (441.57 mg, 4.36 mmol) and stirred at 25 °C for 30 min. Then AcOH (349.41 mg, 5.82 mmol) was added to the mixture. The reaction mixure was stirred at 25 °C for 30 min and then $NaBH_3CN$ (182.82 mg, 2.91 mmol) was added . The mixture was stirred at 25°C for 16 hrs. Then the reaction mixture was partitioned between EA (20 mL) and water (20 mL). The organic phase was separated, dried over anhydrous $Na_2SO_4$, filtered and concentrated under reduced pressure to give the residue. The residue was purified by column chromatography (Petroleum ether/Ethyl acetate=5/1 to 3/1). **Intermediate 75-3** (675 mg, crude) was obtained as a yellow oil and was used into the next step directly. MS ($ESI^+$): m/z 389.49 $[M+H]^+$.

**Step 2: Synthesis of intermediate 75-4**

To a solution of **intermediate 75-3** (675 mg, 1.73 mmol) in DCM (5 mL) was added HCl/dioxane (5 mL). The mixture was stirred at 20 °C for 2 hrs. The reaction mixture was filtered and concentrated under reduced pressure to give **intermediate 75-4** (861 mg, crude) as a white solid, which is used for the next step directly. MS ($ESI^+$): m/z 289.37 $[M+H]^+$.

**Step 3: Synthesis of intermediate 75-5**

To a solution of **intermediate 75-4** (861 mg, 2.98 mmol) in ethyl 2,2,2-trifluoroacetate (5 mL) was added $Et_3N$ (903.24 mg, 8.93 mmol). The mixture was stirred at 80 °C for 16 hrs. The reaction mixture was partitioned between EA (20 mL) and water (20 mL). The organic phase was separated, dried over anhydrous $Na_2SO_4$, filtered and concentrated under reduced pressure to give the residue. The residue was purified by column chromatography (DCM: MeOH = 20: 1). **Intermediate 75-5** (358 mg, crude) was obtained as a yellow solid and used for the next step.

MS ($ESI^+$): m/z 385.38 $[M+H]^+$.

**Step 4: Synthesis of intermediate 75-6**

To a solution of **intermediate 75-5** (358.3 mg, 929.73 μmol) in THF (10 mL) was added Pd/C (300 mg, 5% purity). The mixture was stirred at 20°C for 16 hrs under $H_2$ atmosphere. The reaction mixture was filtered and concentrated under reduced pressure to give **intermediate 75-6** (121 mg, crude) as black oil, which is used for the next step directly.

MS ($ESI^+$): m/z 355.40 $[M+H]^+$.

**Step 5: Synthesis of intermediate 75-8**

To a solution of **intermediate 75-6** (68.66 mg, 179.06 μmol) in toluene (1 mL) was added m-CPBA (123.60 mg, 286 μmol) and the mixture was stirred at 20 °C for 30 min, then DIEA (69.43 mg, 537.17 μmol) and **intermediate B 15-B** (70 mg, 196.96 μmol) was added to the mixture and the obtained mixture was stirred at 20°C for 16 hrs. The reaction mixture was quenched by addition $Na_2S_2O_3$ (10 mL) and $NaHCO_3$ (10 mL) at 0°C, partitioned between EA (30 mL) and water (30 mL), dried over anhydrous $Na_2SO_4$, filtered and concentrated under reduced pressure to give the residue. The residue was purified by column chromatography (DCM: MeOH = 10:1). **Intermediate 75-8** (149 mg, crude) was obtained as a yellow solid, which is used for next step directly.

MS (ESI$^+$): m/z 690.76 [M+H]$^+$.

**Step 6: Synthesis of intermediate 75-9**

To a solution of **intermediate 75-8** (149 mg, 216 μmol) in MeOH (5 mL) was added $K_2CO_3$ (89.44 mg, 647.12 μmol). The mixture was stirred at 25°C for 2 hrs. The reaction mixture was partitioned between EA (20 mL) and water (20 mL). The organic phase was separated, dried over anhydrous $Na_2SO_4$, filtered and concentrated under reduced pressure to give the residue. The residue was purified by column chromatography (DCM: MeOH= 10: 1). **Compound 75-9** (120 mg, crude) was filtered and concentrated *in vacuo* to obtained as a yellow solid, which is used for next step directly.

MS (ESI$^+$): m/z 594.75 [M+H]$^+$.

**Step 7: Synthesis of compound 75**

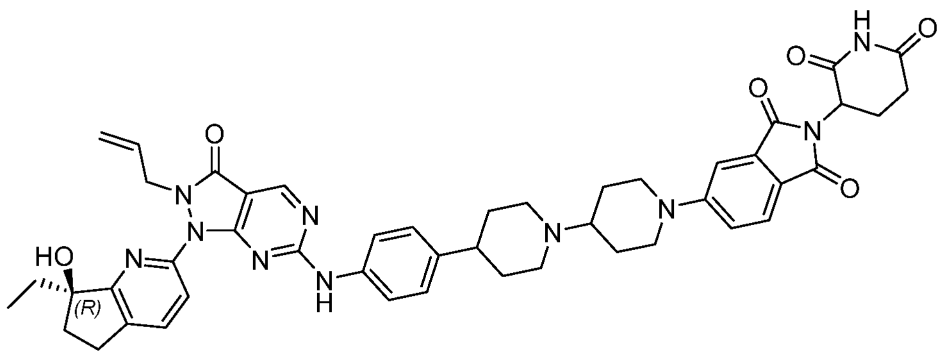

**compound 75**

To a solution of **intermediate 1-1** (83.6 mg, 303 μmol) and **intermediate 75-9** (120 mg, 202 μmol) in NMP (5 mL) was added DIEA (78.2 mg, 605 μmol). The mixture was stirred at 100°C for 16 hrs. The reaction mixture was partitioned between EA (10 mL) and water (10 mL). The organic phase was separated, dried over anhydrous $Na_2SO_4$, filtered and concentrated under reduced pressure to give the residue. The residue was purified by HPLC (acetonitrile / water (0.1%TFA condition)) to give a yellow solid. The yellow solid was diluted with DCM (5 mL) and washed with $NaHCO_3$ (3 mL). The organic layer was dried over anhydrous $Na_2SO_4$, filtered and concentrated, and then purified by combi flash (DCM: MeOH=10: 1, 4 g silica gel column) to give **compound 75** (19 mg, 10.5 % yield) as a yellow solid.

MS (ESI$^+$): m/z 851.6 [M+H]$^+$.

$^1$H NMR (400MHz, DMSO-$d_6$) 11.10 (s, 1H), 10.43 - 10.10 (m, 1H), 8.87 (s, 1H), 7.94 (d, $J$ = 7.6Hz, 1H), 7.75 - 7.60 (m, 4H), 7.39 - 7.31 (m, 1H), 7.27 (d, $J$ = 7.2 Hz, 1H), 7.20 (d, $J$ = 8.4 Hz, 2H),5.81 - 5.56 (m, 1H), 5.11 - 4.95 (m, 3H), 4.85 (d, $J$ = 18.4 Hz, 1H), 4.76 (d, $J$ = 12.8 Hz, 1H), 4.63 -4.50 (m, 1H), 4.12 (d, $J$ = 11.6 Hz, 2H), 3.06 - 2.93 (m, 5H), 2.90 - 2.72 (m, 3H), 2.59 (d, $J$ = 16.0 Hz,2H), 2.32 - 2.15 (m, 3H), 2.08 - 1.97 (m, 2H), 1.96 - 1.83 (m, 3H), 1.83 - 1.44 (m, 8H), 0.87 (t, $J$ = 7.6 Hz, 3H).

**Example 76: compound 76**

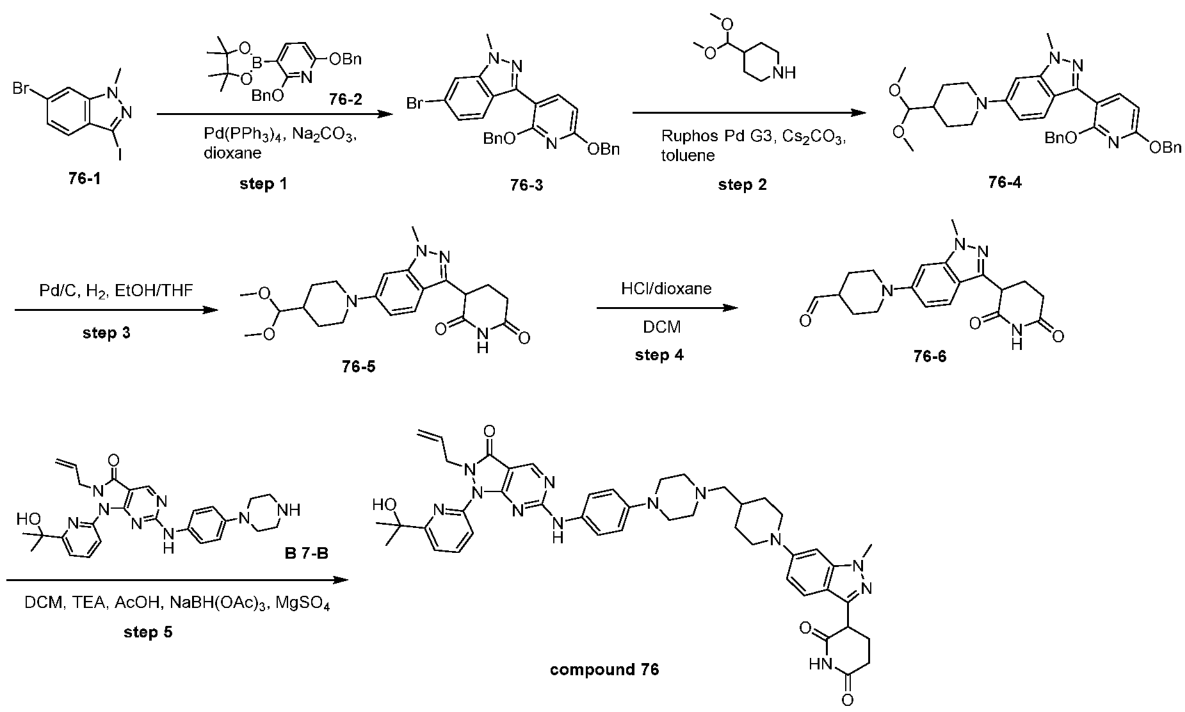


### Step 1: Synthesis of intermediate 76-3

To a solution of **intermediate 76-1** (1 g, 2.97 mmol), and **intermediate 76-2** (1.24 g, 2.97 mmol) in 1,4-dioxane (10 mL) and $H_2O$ (1 mL) was added $Pd(PPh_3)_4$ (343 mg, 297 μmol) and $Na_2CO_3$ (944 mg, 8.90 mmol), then degassed and purged with $N_2$ for 3 times, and then the mixture was stirred at 100°C for 16 hrs under $N_2$ atmosphere. The reaction mixture was partitioned between EA (50 mL) and $H_2O$ (50 mL). The organic phase was washed with saturated saturated salt solution, dried over anhydrous $Na_2SO_4$, filtered and concentrated by rotary distillation to give the residue. The residue was purified by column chromatography (PE/EA=1/0 to 5/1) to give **intermediate 76-3** (946 mg, 52.9% yield) as a yellow solid.

MS ($ESI^+$): m/z 502.0 $[M+H]^+$.

### Step 2: Synthesis of intermediate 76-4

To a solution of **intermediate 76-3** (300 mg, 600 μmol) and 4-(dimethoxymethyl)piperidine (191 mg, 1.20 mmol) in toluene (5 mL) was added $Cs_2CO_3$ (586 mg, 1.80 mmol) and RuPhos Pd G3 (50.1 mg, 60.0 μmol), then degassed and purged with $N_2$ for 3 times, and then the mixture was stirred at 100°C for 16 hrs under $N_2$ atmosphere. The reaction mixture was concentrated by rotary distillation, and diluted with EA (40 mL) and extracted with water (50 mL). The organic layers were dried over anhydrous $Na_2SO_4$, concentrated by rotary distillation to give the residue. The residue was purified by column chromatography (PE/EA=5/1 to 1/1) to give **intermediate76-4** (201 mg, 55.0% yield) as a white solid.

MS ($ESI^+$): m/z 579.3 $[M+H]^+$.

### Step 3: Synthesis of intermediate 76-5

To a solution of **intermediate 76-4** (201 mg, 347 μmol) in THF (2 mL) and EtOH (2 mL) was added Pd/C (200 mg, 10% purity), then degassed and purged with $H_2$ for 3 times, and then the mixture was stirred at 20°C for 16 hrs under $H_2$ atmosphere. The reaction mixture was filtered and concentrated by rotary distillation to give the residue. The residue was purified by column chromatography (PE/EA=5/1 to 1/1) to give **intermediate 76-5** (82 mg, 58.9% yield) as a white solid.

MS ($ESI^+$): m/z 401.3 $[M+H]^+$.

### Step 4: Synthesis of intermediate 76-6

To a solution of **intermediate 76-5** (82 mg, 205 μmol) in DCM (1 mL) was added HCl/dioxane (4 M, 2 mL) and the mixture was stirred at 25°C for 16 hrs. The reaction mixture was filtered and concentrated by rotary distillation to

give **intermediate 76-6** (113 mg, crude) as a white solid, which is used for next step directly.

**Step 5: Synthesis of compound 76**

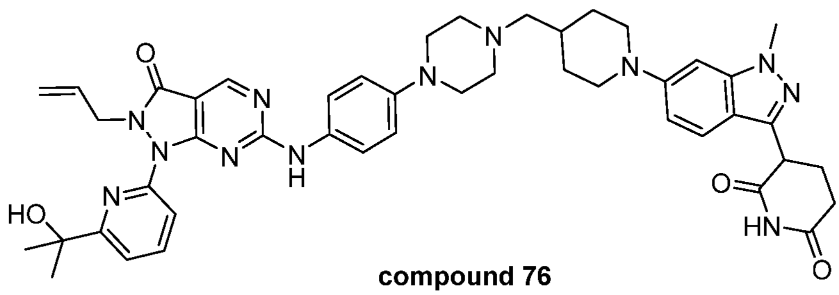

**compound 76**

To the solution of **intermediate 76-6** (50 mg, 128 μmol) and **intermediate B 7-B** (80.3 mg, 154 μmol) in DCM (2 mL) was added $Et_3N$ (25.9 mg, 256 μmol, 36 μL), and the obtained solution was stirred at 25°C for 0.5 h, then AcOH (15.4 mg, 256 μmol, 15 μL) was added and stirred another 0.5 h at 25°C. Then $NaBH(OAc)_3$ (81.3 mg, 384 μmol) was added, and the mixture was stirred at 25°C for 16 hrs. The mixture was concentrated by rotaty distillation and the residue was purified by HPLC (acetonitrile / water (2% FA)) to obtain **compound 76** as a yellow solid (45 mg, 41.4%).

MS (ESI$^+$): m/z 825.6 [M+H]$^+$.

$^1$H NMR (400MHz, DMSO-$d_6$) $\delta$ 10.85 (s, 1H), 10.26 - 10.00 (m, 1H), 8.82 (s, 1H), 8.27 (s, 0.336 H), 8.04 (br s, 1H), 7.75 (d, *J* = 8.4 Hz, 1H), 7.60 (d, *J* = 7.6 Hz, 3H), 7.47 (d, *J* = 8.8 Hz, 1H), 6.99 - 6.87 (m, 3H), 6.83 (s, 1H), 5.72 - 5.58 (m, 1H), 5.33 (s, 1H), 4.99 (d, *J* = 10.4 Hz, 1H), 4.82 (d, *J* = 18.4 Hz, 1H), 4.72 - 4.63 (m, 2H), 4.29 - 4.22 (m, 1H), 3.88 (s, 3H), 3.84 - 3.73 (m, 2H), 3.11 (br s, 4H), 2.81 - 2.69 (m, 2H), 2.64 - 2.58 (m, 1H), 2.55 - 2.52 (m, 4H), 2.42 (br s, 1H), 2.31 - 2.20 (m, 3H), 2.19 - 2.11 (m, 1H), 1.83 (d, *J* = 12.4 Hz, 3H), 1.46 (s, 6H), 1.34 - 1.20 (m, 2H).

The **compound 111, 117** and **compound 144** were synthesized according to the procedures described for **compound 76** using the different intermediates.

| Compound No. | Intermediate No. | MS(ESI$^+$): m/z [M+H]$^+$ | $^1$H NMR (400 MHz, DMSO-$d_6$) $\delta$ |
|---|---|---|---|
| 111 | B 8 | 824.6 | 10.86 (s, 1H), 10.27 (br s, 1H), 8.88 (s, 1H), 8.13 (s, 0.274H), 8.10 - 8.01 (m, 1H), 7.76 (d, *J* = 8.4 Hz, 1H), 7.73 - 7.65 (m, 2H), 7.63 (d, *J* = 7.6 Hz, 1H), 7.49 (d, *J* = 8.8 Hz, 1H), 7.27 - 7.18 (m, 2H), 6.96 - 6.89 (m, 1H), 6.84 (s, 1H), 5.73 - 5.61 (m, 1H), 5.35 (s, 1H), 5.00 (d, *J* = 10.0 Hz, 1H), 4.87 - 4.78 (m, 1H), 4.73 - 4.64 (m, 2H), 4.29 - 4.21 (m, 1H), 3.89 (s, 3H), 3.86 - 3.77 (m, 2H), 3.24 (br s, 2H), 2.81 - 2.70 (m, 2H), 2.64 - 2.56 (m, 3H), 2.40 (br s, 1H), 2.32 - 2.22 (m, 2H), 2.21 - 2.11 (m, 2H), 1.87 (br s, 2H), 1.84 (br s, 6H), 1.46 (s, 6H), 1.35 - 1.28 (m, 2H) |
| 117 | B 7-B | 825.5 | 10.89 (s, 1H), 10.17 (br s, 1H), 8.83 (s, 1H), 8.13 (s, 0.46 H), 8.05 (br s, 1H), 7.75 (d, *J* = 8.0 Hz, 1H), 7.61 (br d, *J* = 8.0 Hz, 3H), 7.42 - 7.33 (m, 1H), 7.07 - 6.88 (m, 4H), 5.75 - 5.58 (m, 1H), 5.33 (s, 1H), 4.99 (br d, *J* = 8.0 Hz, 1H), 4.82 (br d, *J* = 16.0 Hz, 1H), 4.69 (br s, 2H), 4.37 - 4.30 (m, 1H), 4.25 (s, 3H), 3.30 - 2.89 (m, 8H), 2.70 (br s, 2H), 2.62 (br d, *J* = 4.0 Hz, 2H), 2.58 - 2.53 (m, 2H), 2.32 (br d, *J* = 8.0 Hz, 3H), 2.22 - 2.10 (m, 1H), 1.90 (br d, *J* = 12.0 Hz, 2H), 1.78 (br s, 1H), 1.46 (s, 8H).. |

(continued)

| Compound No. | Intermediate No. | MS(ESI$^+$): m/z $[M+H]^+$ | $^1$H NMR (400 MHz, DMSO-$d_6$) $\delta$ |
|---|---|---|---|
| 144 | **55-10** | 825.5 | 10.89 (s, 1H), 10.14 (br s, 1H), 8.82 (s, 1H), 8.20 (br s, 0.241H), 8.05 (br s, 1H), 7.75 (d, *J* = 8.0 Hz, 1H), 7.64 - 7.51 (m, 3H), 7.39 (d, *J* = 8.0 Hz, 1H), 7.08 - 6.99 (m, 2H), 6.93 (d, *J* = 8.0 Hz, 2H), 5.73 - 5.59 (m, 1H), 5.33 (s, 1H), 4.99 (d, *J* = 8.0 Hz, 1H), 4.82 (d, *J* = 16.0 Hz, 1H), 4.68 (d, *J* = 4.0 Hz, 2H), 4.38 - 4.30 (m, 1H), 4.25 (s, 3H), 3.65 (d, *J* = 12.0 Hz, 2H), 3.25 - 2.77 (m, 8H), 2.67 - 2.59 (m, 3H), 2.36 - 2.23 (m, 4H), 2.20 - 2.13 (m, 1H), 1.84 (d, *J* = 12.0 Hz, 2H), 1.71 (br s, 1H), 1.46 (s, 6H), 1.32 - 1.20 (m, 2H). |

**Example 77: compound 77**

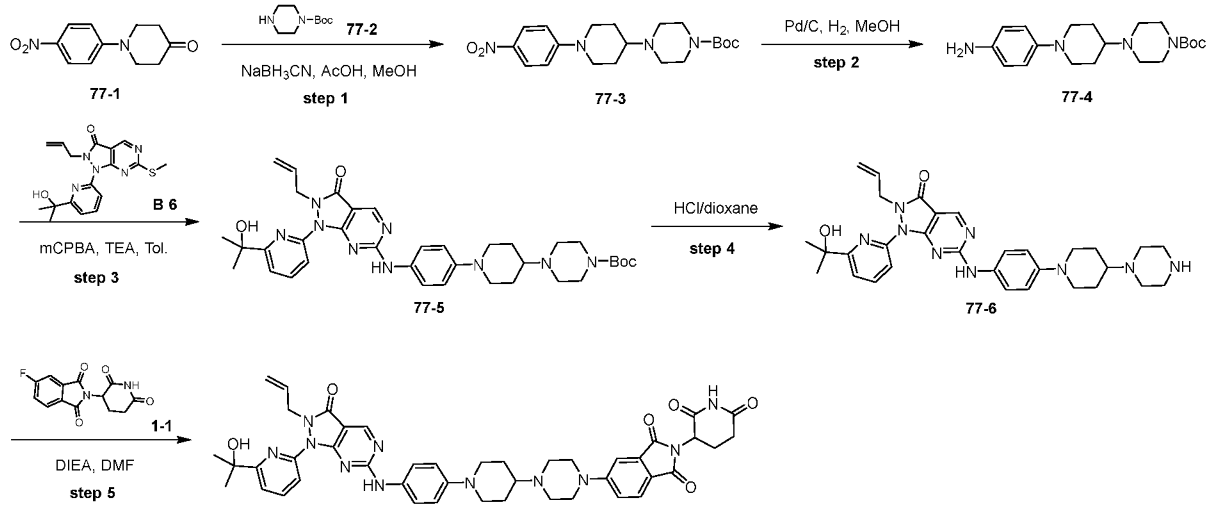


compound 77

**Step 1: Synthesis of intermediate 77-3**

To a solution of **intermediate 77-1** (500 mg, 2.27 mmol) and **intermediate 77-2** (443 mg, 2.27 mmol) in MeOH (10 mL) was added AcOH (130 μL, 2.27 mmol). The mixture was stirred at 25°C for 2 hrs. Then $NaBH_3CN$ (214 mg, 3.41 mmol) was added and the mixture was stirred at 25°C for 2 hrs. The mixture was added saturated $NaHCO_3$ (20 mL) and extracted with ethyl acetate (40 mL×3). The organic phase was collected and dried over anhydrous $Na_2SO_4$, filtered and concentrated by rotary distillation to give the resiue. The residue was purified by flash silica gel chromatography (Petroleum ether: ethyl acetate=1:0 to 65:35) to give **intermediate77-3** (280 mg, 31.6% yield) as yellow solid.

MS (ESI$^+$): m/z 391.14 $[M+H]^+$.

$^1$H NMR (400 MHz, DMSO-$d_6$) $\delta$ 8.03 (d, *J* = 9.6 Hz, 2H), 7.01 (d, *J* = 9.6 Hz, 2H), 4.15 - 3.99 (m, 2H), 3.30 - 3.24 (m, 4H), 3.02 - 2.90 (m, 2H), 2.57 - 2.52 (m, 1H), 2.45 - 2.38 (m, 4H), 1.88 - 1.78 (m, 2H), 1.49 - 1.35 (m, 11H).

**Step 2: Synthesis of intermediate 77-4**

To a mixture of **intermediate 77-3** (500 mg, 1.28 mmol) in MeOH (10 mL) was added wet Pd/C (50 mg, 10% purity). The mixture was stirred under $H_2$ (15 psi) at 25°C for 1 h. The reaction mixture was filtered and concentrated by rotary distillation to give **intermediate77-4** (480 mg, crude) as a brown solid.

**Step 3: Synthesis of intermediate 77-5**

To a solution of **intermediate B 6** (262 mg, 733 μmol) in toluene (10 mL) was added m-CPBA (298 mg, 1.47

mmol, 85% purity). The mixture was stirred at 25°C for 1 h. **Intermediate 77-4** (317 mg, 879 μmol) and DIEA (379 mg, 2.93 mmol) was added to the above mixture. Then the mixture was stirred at 25°C for 12 hrs. The reaction mixture was quenched with saturated $NaHCO_3$ (5 mL) and saturated $Na_2S_2O_3$ (5 mL), and extracted with EA (20 mL×2). The organic layers were collected and dried over anhydrous $Na_2SO_4$, filtered and concentrated by rotary distillation to give the crude product. The crude product was purified by flash silica gel chromatography (dichloromethane: methanol = 1:0 to 10:1) to give **intermediate77-5** (480 mg, 98% yield) as a yellow soild.

MS (ESI$^+$): m/z 669.9 [M+H]$^+$.

**Step 4: Synthesis of intermediate 77-6**

A solution of **intermediate 77-5** (480 mg, 717 μmol) in DCM (4 mL) was added HCl/dioxane (4 M, 10 mL). The reaction mixture was stirred at 25°C for 2 hrs. The reaction mixture was concentrated by rotary distillation to give **intermediate 77-6** (408 mg, 93.9% yield, HCl salt) as a white solid.

MS (ESI$^+$): m/z 569.6 [M+H]$^+$.

**Step 5: Synthesis of compound 77**

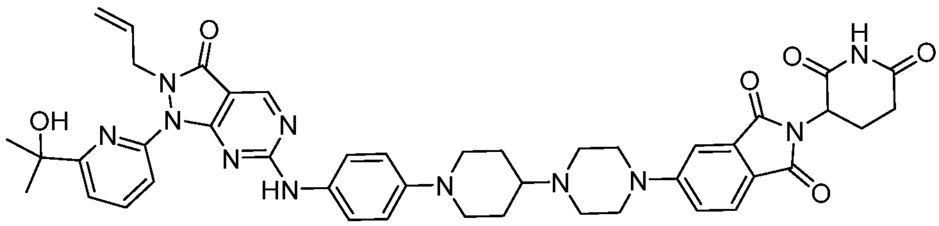

**compound 77**

To a solution of **intermediate77-6** (200 mg, 352 μmol) in DMF (4 mL) was added **intermediate 1-1** (97.0 mg, 351 μmol) andDIEA (183 μL, 1.05 mmol). The mixture was stirred at 100°C for 2 hrs under microwave. The mixture was diluted with DCM (20 mL), washed with $H_2O$ (28 mL) and the combined organic layers were collected and dried over anhydrous $Na_2SO_4$, filtered and concentrated by rotary distillation to give the residue. The residue was purified by prep-HPLC (Column: Kromasil 100-5-C18 30*150 mm, Mobile Phase A: water (0.01% FA), Mobile Phase B: acetonitrile, 20 mL/min, gradient condition from 30% B to 70% B) to give **compound 77** (25.36 mg, 8.66% yield) as a yellow solid.

MS (ESI$^+$): m/z 826.5 [M+H]$^+$.

$^1$H NMR (400 MHz, DMSO-$d_6$) $\delta$ 11.10 (s, 1H), 10.27 - 10.10 (m, 1H), 8.83 (s, 1H), 8.14 - 8.00 (m, 1H), 7.80 - 7.74 (m, 1H), 7.70 - 7.67 (m, 1H), 7.64 - 7.51 (m, 3H), 7.35 (s, 1H), 7.31 - 7.24 (m, 1H), 7.01 - 6.90 (m, 2H), 5.75 - 5.59 (m, 1H), 5.35 (s, 1H), 5.08 (dd, *J* = 5.2, 12.8 Hz, 1H), 5.01 - 4.97 (m, 1H), 4.88 - 4.77 (m, 1H), 4.71 - 4.62 (m, 2H), 3.77 - 3.67 (m, 2H), 3.45 - 3.43 (m, 4H), 2.95 - 2.82 (m, 1H), 2.70 - 2.55 (m, 8H), 2.42 - 2.36 (m, 1H), 2.06 - 1.96 (m, 1H), 1.94 - 1.84 (m, 2H), 1.62 - 1.52 (m, 2H), 1.46 (s, 6H)

The **Compound 78** was synthesized according to the similar procedures described for **Compound 77.**

| Compound No. | Intermediate No. | MS(ESI$^+$): m/z [M+H]$^+$ | $^1$H NMR (400 MHz, DMSO-$d_6$) $\delta$ |
|---|---|---|---|
| 78 | 77-6 | 844.5 | 11.12 (s, 1H), 10.27 - 10.06 (m, 1H), 8.82 (s, 1H), 8.06 (s, 1H), 7.82 - 7.71 (m, 2H), 7.66 - 7.50 (m, 3H), 7.49 - 7.41 (m, 1H), 6.94 (d, J = 8.8 Hz, 2H), 5.75 - 5.59 (m, 1H), 5.43 - 5.27 (m, 1H), 5.11 (dd, J = 5.2, 12.8 Hz, 1H), 5.03 - 4.96 (m, 1H), 4.88 - 4.77 (m, 1H), 4.74 - 4.63 (m, 2H), 3.81 - 3.61 (m, 2H), 3.27 - 3.23 (m, 4H), 2.97 - 2.81 (m, 1H), 2.74 - 2.52 (m, 8H), 2.45 - 2.35 (m, 1H), 2.07 - 1.99 (m, 1H), 1.95 - 1.83 (m, 2H), 1.64 - 1.50 (m, 2H), 1.46 (s, 6H). |

**Example 79: compound 79**

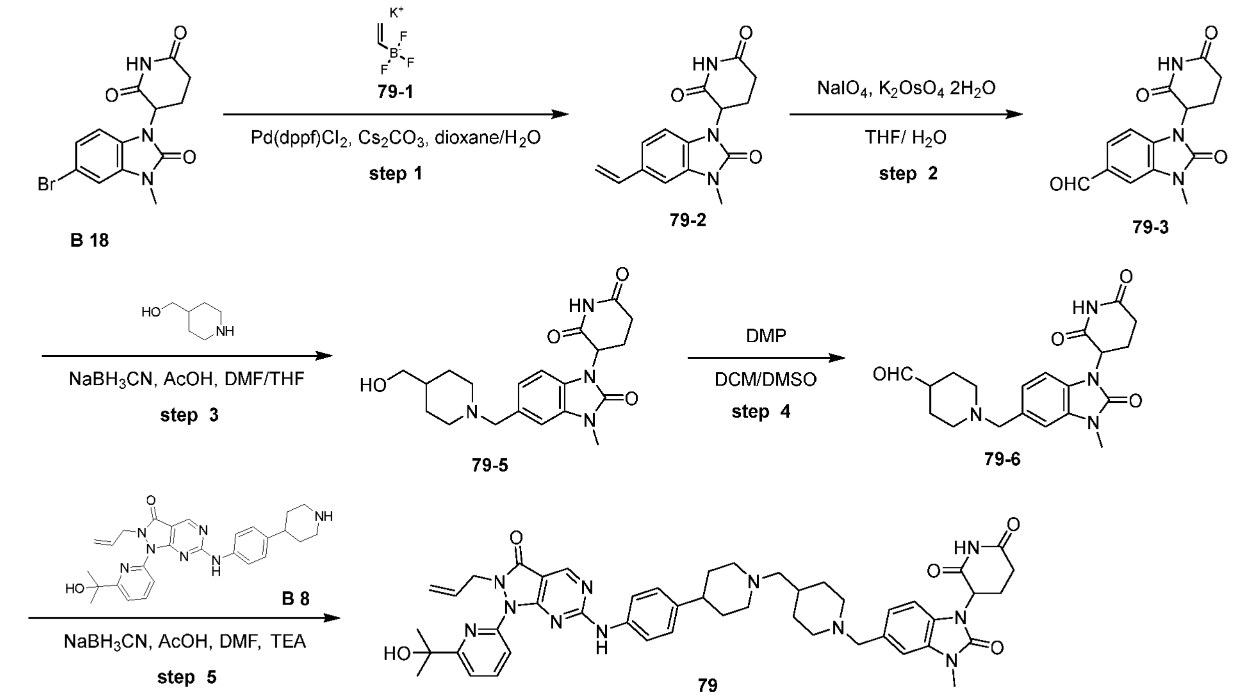


**Step 1: Synthesis of intermediate 79-2**

Pd(dppf)$Cl_2$ (216 mg, 296 μmol) was added to a mixture of **intermediate B 18** (1 g, 2.96 mmol), **intermediate 79-1** (1.19 g, 8.87 mmol) and $Cs_2CO_3$ (1.93 g, 5.91 mmol) in dioxane (5 mL) and $H_2O$ (1 mL). The mixture was degassed and purged with $N_2$ for 5 minutes, and then the sealed tube was reacted at 80°C for 1 h under microwave. The reaction mixture was concentrated by rotary distillation to give thr residue. The residue was purified by flash silica gel chromatography (Petroleum ether: ethyl acetate = 1:1 to 0:1). **Intermediate 79-2** (750 mg, 72.0% yield) was obtained as a brown solid.

MS ($ESI^+$): m/z 286.07 $[M+H]^+$.

**Step 2: Synthesis of intermediate 79-3**

$NaIO_4$ (2.25 g, 10.5 mmol) in $H_2O$ (10 mL) was added to a solution of **intermediate 79-2** (750 mg, 2.63 mmol) in THF (50 mL). The mixture was stirred at 0°C for 10 min. $K_2O_SO_4$ (48.4 mg, 131 μmol) was added to the above mixture and stirred at 25°C for 50 min. The reaction mixture was quenched with saturated $Na_2SO_3$ (50 mL) and then extracted with DCM (50 mL×3). The combined organic layers were dried over anhydrous $Na_2SO_4$, filtered and concentrated by rotary distillation to give the residue. The residue was purified by flash silica gel chromatography (Petroleum ether:ethyl acetate = 10:0 to 0:1). **Intermediate 79-3** (550 mg, 30% yield) was obtained as a brown solid.

MS ($ESI^+$): m/z 288.04 $[M+H]^+$.

**Step 3: Synthesis of intermediate 79-5**

AcOH (20.9 mg, 20.0 μL) was added to a mixture of **intermediate 79-3** (100 mg, 348 μmol) and 4-piperidylmethanol (60.1 mg, 522 μmol) in DMF (1 mL) and THF (2 mL). The reaction mixture was stirred at 80°C for 0.5 h. Then $NaBH_3CN$ (43.8 mg, 696 μmol) was added to the mixture at 25°C and stirred at 25°C for 12 hrs. The reaction mixture was quenched with saturated NaCl (10 mL) and then extracted with DCM (20 mL×3). The aqueous phase was concentrated by rotary distillation to give the residue. The residue was purified by HPLC (Column: Kromasil 100-5-C18 30*150 mm, Mobile Phase A: water (0.01% FA), Mobile Phase B: acetonitrile, 20 mL/min, gradient condition from 5% B to 35%) to give **intermedaite 79-5** (4.69 mg, 16% yield) as a white soild.

MS ($ESI^+$): m/z 387.3 $[M+H]^+$.

**Step 4: Synthesis of intermediate 79-6**

DMP (22.0 mg, 51.8 μmol) was added to a solution of **intermediate 79-5** (10.0 mg, 25.9 μmol) in DMSO (0.1 mL) and DCM (1 mL) at 0°C. The reaction mixture was stirred at 20°C for 2 hrs. The reaction mixture was quenched with saturated $NaHCO_3$ (0.5 mL), filtered and concentrated by rotary distillation to give **intermediate 79-6** (10 mg, crude), which was used for the next step without further purification.

MS ($ESI^+$): m/z 385.07 $[M+H]^+$.

**Step 5: Synthesis of compound 79**

TEA (5.04 mg, 49.8 μmol) was added to a mixture of **intermediate B 8** (13.0 mg, 24.9 μmol, HCl) and **intermediate 79-6** (9.57 mg, 24.9 μmol) in DMF (5 mL). The mixture was stirred at 25°C for 0.5 h. Then AcOH (1.50 mg, 24.9 μmol, 1.42 μL) was added to the above mixture and stirred for another 0.5 h. Finally, $NaBH_3CN$ (3.13 mg, 49.8 μmol) was added to the mixture and reacted at 25°C for 2 hrs. The reaction mixture was quenched with saturated $NaHCO_3$ (0.5 mL), filtered and concentrated by rotary distillation to give the residue. The residue was purified by HPLC (Column: Kromasil 100-5-C18 30*150 mm, Mobile Phase A: water (0.01% FA), Mobile Phase B: acetonitrile, 20 mL/min, gradient condition from 10% B to 50%B) to give compound 79 (4.69 mg, 22% yield) as a white solid.

MS ($ESI^+$): m/z 854.6 $[M+H]^+$.

$^1H$ NMR (400 MHz, DMSO-$d_6$) δ 11.12 (br s, 1H), 10.27 (br s, 1H), 8.88 (s, 1H), 8.12 - 7.94 (m, 1H), 7.82 - 7.72 (m, 1H), 7.70 - 7.56 (m, 3H), 7.20 (d, *J* = 8.4 Hz, 2H), 7.11 (s, 1H), 7.07 - 7.01 (m, 1H), 6.99 - 6.88 (m, 1H), 5.78 - 5.58 (m, 1H), 5.55 - 5.16 (m, 2H), 5.03 - 4.96 (m, 1H), 4.88 - 4.78 (m, 1H), 4.75 - 4.62 (m, 2H), 3.48 - 3.47 (m, 2H), 3.34 (s, 3H), 2.98 - 2.80 (m, 6H), 2.77 - 2.70 (m, 1H), 2.65 - 2.59 (m, 1H), 2.44 - 2.39 (m, 1H), 2.17 - 2.09 (m, 2H), 2.05 - 1.90 (m, 5H), 1.74 - 1.61 (m, 5H), 1.53 - 1.49 (m, 1H), 1.46 (s, 6H), 1.18 - 1.05 (m, 2H).

**Example 80: synthesis of compound 80**

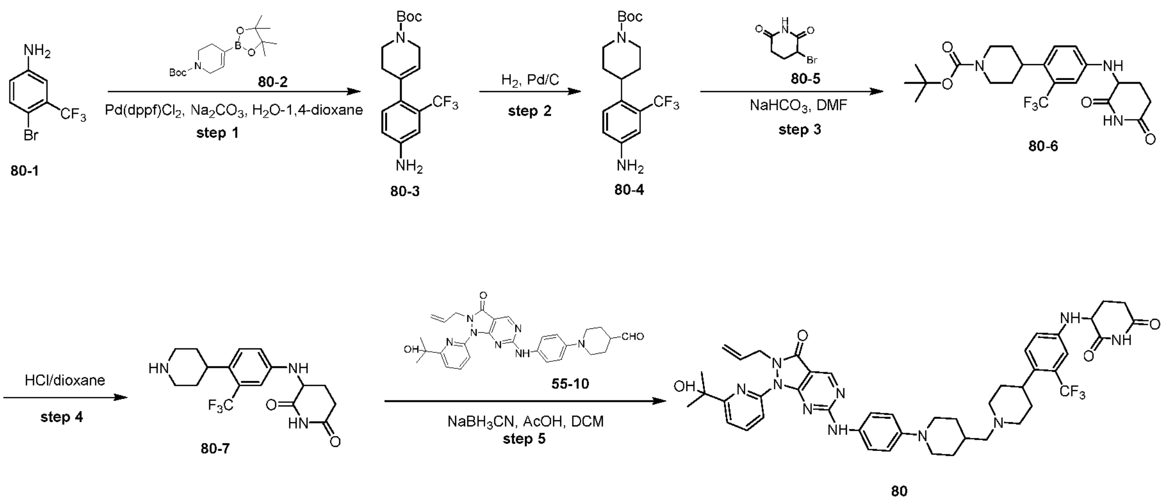


**Step 1: synthesis of intermediate 80-3**

To a mixture of **80-1** (5 g, 20.8 mmol), **80-2** (7.73 g, 25.0 mmol), $Cs_2CO_3$ (20.36 g, 62.49 mmol) in dioxane (30 mL) and water (6 mL) was added $Pd(dppf)Cl_2$ (1.52 g, 2.08 mmol) under $N_2$. The result mixture was stirred at 90°C for 8 hrs. The mixture was dried over with $Na_2SO_4$ filtered and concentrated under reduced pressure to give a residue. The residue was purified by flash silica gel chromatography (Petroleum ether: ethyl acetate = 1:1 to 50:50) to give **intermediate 80-3** (6.5 g, 91.1% yield) as a yellow solid.

LCMS ($ESI^+$): m/z 287.8 $[M-56]^+$.

$^1H$ NMR (400MHz, DMSO-$d_6$) δ 6.94 (d, *J* = 8.4 Hz, 1H), 6.86 - 6.84 (m, 1H), 6.75 - 6.71 (m, 1H), 5.54 - 5.43 (m, 3H), 3.88 (br s., 2H), 3.50 - 3.44 (m, 2H), 2.23 - 2.16 (m, 2H), 1.41 (s, 9H).

**Step 2: synthesis of intermediate 80-4**

To a mixture of **80-3** (2.00 g, 5.84 mmol) in EtOH (30 mL) was added Pd/C (500 mg, 10%). The suspension was degassed under vacuum and purged with $H_2$ three times. The mixture was stirred under $H_2$ (15 psi) at 25°C for 20 hours. The reaction mixture was filtered and concentrated under reduced pressure to give the **intermediate 80-4** (1.8 g, crude) as a brown solid, and was used into the next step directly.

$^1H$ NMR (400 MHz, DMSO-$d_6$) δ 7.17 (d, *J* = 8.4 Hz, 1H), 6.82 (d, *J* = 2.4 Hz, 1H), 6.78 - 6.73 (m, 1H), 5.34 (s, 2H), 2.82 - 2.65 (m, 4H), 2.47 - 2.41 (m, 1H), 1.61 - 1.45 (m, 4H), 1.41 (s, 9H).

**Step 3: synthesis of intermediate 80-6**

To a solution of **80-4** (1 g, 2.90 mmol) and **80-5** (1.12 g, 5.81 mmol) in DMF (10 mL) was added $NaHCO_3$ (731.83 mg, 8.71 mmol) The mixture was stirred at 80 °C for 8 hrs . The reaction mixture was added water 20 mL at 25°C, and then extracted with EtOAc (30 mL × 2). The combined organic layers were dried over $Na_2SO_4$, filtered and concentrated under reduced pressure to give a residue. The residue was purified by flash silica gel chromatography to give **intermediate 80-6** (400 mg, 30.2% yield) as a green oil.

LCMS ($ESI^+$): m/z 358.3 $[M-100+H]^+$.

$^1H$ NMR (400 MHz, DMSO-$d_6$) δ 10.80 (s, 1H), 7.27 (d, *J* = 8.4 Hz, 1H), 6.96 - 6.83 (m, 2H), 6.24 (d, *J* = 8.0 Hz, 1H), 4.45 - 4.36 (m, 1H), 4.15 - 4.00 (m, 2H), 2.97 - 2.90 (m, 2H), 2.84 - 2.77 (m, 2H), 2.59 - 2.55 (m, 1H), 2.13 - 2.03 (m, 1H), 1.96 - 1.87 (m, 1H), 1.61 - 1.48 (m, 4H), 1.41 (s, 9H).

**Step 4: synthesis of intermediate 80-7**

To a solution of **80-6** (400 mg, 878.21 μmol) in DCM (2 mL) was added HCl/dioxane (4 M, 10 mL). The mixture was stirred at 25°C for 1 hr. The reaction mixture was concentrated to dryness under reduced pressure to give **intermediate 80-7** (300 mg, 87.2% yield) as a white solid. The crude product was used into the next step without further purification.

LCMS ($ESI^+$): m/z 356.4 $[M+H]^+$.

**Step 5: synthesis of compound 80**

To a solution of **80-7** (70 mg, 179 μmol, HCl) and intermediate **55-10** (91.8 mg, 179 μmol) in DCM (4 mL) was added TEA (54.2 mg, 536 μmol, 75 μL) and AcOH (10.73 mg, 179 μmol, 10.2 μL), the mixture was stirred at 25°C for 1 hr. Then $NaBH_3CN$ (16.8 mg, 268 μmol) was added and the obtained mixture was further stirred at 25 °C for 3 hrs. The reaction mixture was concentrated under reduced pressure to give a residue. The residue was purified by prep-HPLC (acetonitrile / water, (2%FA)) to give the crude, which was further purified by prep-TLC (SiO2, DCM: MeOH = 10:1) to give **compound 80** (12.93 mg, 8.15% yield) as a yellow solid. LCMS ($ESI^+$): m/z 853.6 $[M+H]^+$.

$^1H$ NMR (400 MHz, DMSO-$d_6$) δ 10.80 (s, 1H), 10.14 (br s, 1H), 8.82 (s, 1H), 8.09 - 8.00 (m, 1H), 7.78 - 7.72 (m, 1H), 7.63 - 7.52 (m, 3H), 7.33 - 7.27 (m, 1H), 6.95 - 6.87 (m, 4H), 6.26 - 6.18 (m, 1H), 5.73 - 5.60 (m, 1H), 5.33 (s, 1H), 5.02 - 4.97 (m, 1H), 4.86 - 4.79 (m, 1H), 4.72 - 4.64 (m, 2H), 4.45 - 4.37 (m, 1H), 3.69 - 3.60 (m, 2H), 3.07 - 2.88 (m, 2H), 2.79 - 2.69 (m, 1H), 2.64 - 2.56 (m, 3H), 2.45 - 2.35 (m, 1H), 2.29 - 2.13 (m, 2H), 2.10 - 2.04 (m, 1H), 1.96 - 1.87 (m, 2H), 1.86 - 1.78 (m, 3H), 1.76 - 1.58 (m, 5H), 1.46 (s, 6H), 1.27 - 1.22 (m, 2H).

The **compound 81, 82** and **compound 145** were synthesized according to the procedures described for **compound 77** using the different intermediates.

| Compound No. | Intermediate No. | MS (ESI$^+$): m/z [M+H]$^+$ | $^1$H NMR (400 MHz, DMSO-$d_6$) $\delta$ |
|---|---|---|---|
| 81 | **55-10** | 803.6 | 10.81 (s, 1H), 10.16 (br s, 1H), 8.93 - 8.70 (m, 1H), 8.28 (s, 0.508H), 8.05 (br s, 1H), 7.75 (d, *J* = 8.0 Hz, 1H), 7.65 - 7.46 (m, 3H), 7.10 - 6.77 (m, 3H), 6.52 - 6.33 (m, 2H), 6.01 (d, *J* = 8.0 Hz, 1H), 5.80 - 5.55 (m, 1H), 5.38 (br s, 1H), 4.99 (d, *J* = 10.4 Hz, 1H), 4.82 (d, *J* = 17.2 Hz, 1H), 4.68 (d, *J* = 5.2 Hz, 2H), 4.36 - 4.26 (m, 1H), 3.65 (br s, 2H), 2.94 (d, *J* = 10.4 Hz, 2H), 2.76 - 2.67 (m, 1H), 2.66 - 2.55 (m, 4H), 2.19 (d, *J* = 6.8 Hz, 2H), 2.13 - 2.03 (m, 1H), 2.03 - 1.92 (m, 2H), 1.91 - 1.75 (m, 3H), 1.65 (br s, 5H), 1.46 (s, 6H), 1.30 - 1.10 (m, 2H). |
| 82 | **B 6** | 789.5 | 10.82 (s, 1H), 10.20 (br s, 1H), 8.83 (s, 1H), 8.06 (br s, 1H), 7.75 (d, *J* = 8.0 Hz, 1H), 7.61 (d, *J* = 7.6 Hz, 3H), 6.97 (d, *J* = 8.0 Hz, 3H), 6.55 - 6.36 (m, 2H), 6.11 (d, *J* = 6.0 Hz, 1H), 5.78 - 5.56 (m, 1H), 5.37 (s, 1H), 4.99 (d, *J* = 10.4 Hz, 1H), 4.81 (d, *J* = 17.2 Hz, 1H), 4.69 (br s, 2H), 4.36 - 4.25 (m, 1H), 3.79 (br s, 2H), 3.17 - 2.86 (m, 3H), 2.78 - 2.62 (m, 4H), 2.59 (d, *J* = 3.6 Hz, 1H), 2.45 (br s, 1H), 2.24 - 1.61 (m, 11H), 1.46 (s, 6H). |
| 145 | **55-10** | 785.5 | 10.79 (s, 1H), 10.15 (br s, 1H), 8.82 (s, 1H), 8.04 (br s, 1H), 7.75 (d, *J* = 8.0 Hz, 1H), 7.65 - 7.48 (m, 3H), 7.03 - 6.96 (m, 1H), 6.92 (d, *J* = 8.0 Hz, 2H), 6.56 (s, 1H), 6.53-6.42 (m, 2H), 5.75 (d, *J* = 8.0 Hz, 1H), 5.71 - 5.60 (m, 1H), 5.34 (s, 1H), 4.99 (d, *J* = 8.0 Hz, 1H), 4.82 (d, *J* = 16.0 Hz, 1H), 4.68 (d, *J* = 4.0 Hz, 2H), 4.38 - 4.29 (m, 1H), 3.64 (d, *J* = 12.0 Hz, 2H), 2.97 (br s, 2H), 2.82 - 2.70 (m, 1H), 2.65 - 2.56 (m, 3H), 2.47 - 2.43 (m, 1H), 2.20 (br s, 2H), 2.13 - 2.06 (m, 1H), 1.97 (br s, 1H), 1.92 - 1.76 (m, 4H), 1.70 (br s, 5H), 1.46 (s, 6H), 1.23 (br s, 2H) |

**Example 89: synthesis of compound 89**

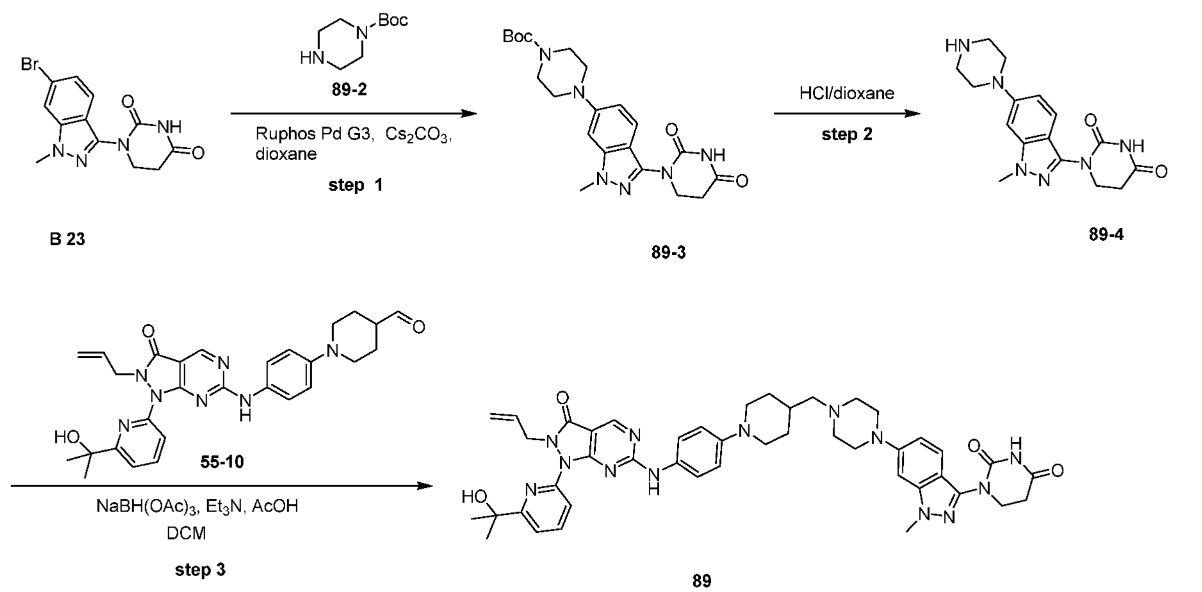


**Step 1: synthesis of intermediate 89-3**

A mixture of **intermediate B 23** (100 mg, 309 μmol) , **intermediate 89-2** (86.5 mg, 464 μmol) , RuPhos Pd G3 (51.7 mg, 61.8 μmol) , $Cs_2CO_3$ (302 mg, 928 μmol) in dioxane (1 mL) was stirred at 100°C for 2 hrs under $N_2$

atmosphere. The reaction mixture was filtered and concentrated under reduced pressure to give a residue. The residue was purified by chromatography on silica gel (DCM: MeOH =10: 1) to give **intermediate 89-3** (91 mg, 54.9% yield) as a red solid.

LCMS (ESI+): m/z 429.4 [M+H]+.

**Step 2: synthesis of intermediate 89-4**

To a solution of **intermediate 89-3** (81 mg, 189 μmol) in DCM (2 mL) was added HCl/dioxane (4M, 2 mL) . The mixture was stirred at 20°C for 2 hrs. The reaction mixture was concentrated under reduced pressure to give a residue to give **intermediate 89-4** (71 mg, crude) as a white solid which was used into next step directly.

LCMS (ESI+): m/z 329.3 [M+H]+.

**Step 3: synthesis of compound 89**

To a solution of **intermediate 89-4**(71.0mg,216μmol) and **intermediate 55-10** (111 mg, 216 μmol) in DCM (5 mL) was added TEA (109 mg, 1.08 mmol, 150 uL) and AcOH (64.9 mg, 1.08 mmol, 62 uL) and was stirred at 25°C for 0.5 hour. Then the mixture was added $NaBH(OAc)_3$ (137 mg, 648 μmol) and stirred at 20 °C for another 16 hrs. The reaction mixture was diluted with DCM (10 mL) and water (10 mL), and the organic layer was dried over $Na_2SO_4$, filtered and concentrated in vacuo to give the residue. The residue was purified by prep-HPLC (acetonitrile / water (2% FA)) to give **compound 89** (20 mg, 11.1% yield) as a white solid. LCMS (ESI+): m/z 848.6 [M+H]+.

$^1$H NMR (400MHz, DMSO-$d_6$) δ 10.52 (s, 1 H), 10.15 (s, 1 H), 8.82 (s, 1 H), 8.16 (s, 0.172H), 8.05 (s, 1 H), 7.75 (d, *J* = 7.20 Hz, 1 H), 7.50 - 7.65 (m, 3 H), 7.46 (d, *J* = 9.20 Hz, 1 H), 6.93 (d, *J* = 8.80 Hz, 3 H), 6.83 (s, 1 H), 5.57 - 5.76 (m, 1 H), 5.33 (s, 1 H), 4.99 (d, *J* = 10.00 Hz, 1 H), 4.82 (d, *J* = 17.6 Hz, 1 H), 4.68 (d, *J* = 6.00 Hz, 2 H), 3.86 - 3.92 (m, 6 H), 3.65 (d, *J* = 10.80 Hz, 2 H), 3.31 (s, 2 H), 3.24 (s, 5 H), 2.73 (t, *J* = 6.80 Hz, 2 H), 2.59 - 2.65 (m, 2 H), 2.25 (d, *J* = 7.20 Hz, 2 H), 1.83 (d, *J* = 11.20 Hz, 2 H), 1.71 (s, 1 H), 1.46 (s, 6 H), 1.23 (s, 2 H).

**Example 93: synthesis of compound 93**

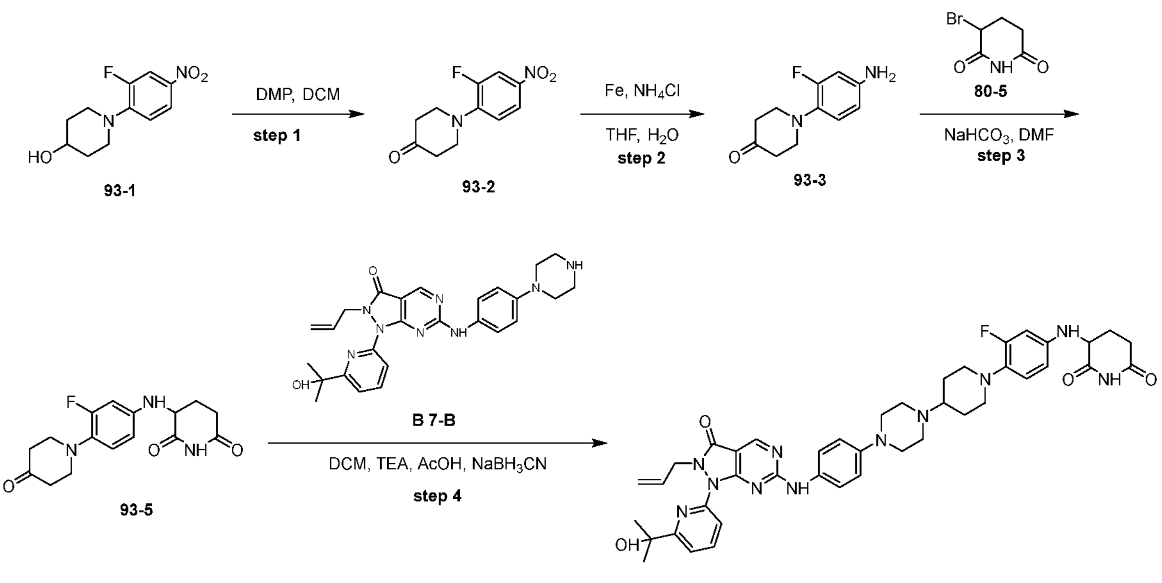


93

**Step 1: synthesis of intermediate 93-2**

To a solution of **intermediate 93-1** (1 g, 4.16 mmol) in DCM (5 mL) was added DMP (2.65 g, 6.24 mmol) and the mixture was stirred at 25°C for 3 hrs. The reaction mixture was quenched by addition aqueous $NaHCO_3$ and $Na_2S_2O_3$ (1:1) 10 mL at 25°C, and then diluted with $H_2O$ (20 mL) and extracted with EtOAc (40 mL × 2). The combined organic layers were dried over $Na_2SO_4$, filtered and concentrated under reduced pressure to give the **intermediate 93-2** (956 mg, crude) as a yellow solid which was used into the next step directly.

**Step 2: synthesis of intermediate 93-3**

To a solution of **intermediate 93-2** (500 mg, 2.10 mmol) in THF (5 mL) was added saturated aqueous $NH_4Cl$ (1 mL) and Fe (1.17 g, 21.0 mmol). The mixture was stirred at 80 °C for 1 hr.

The reaction mixture was dried over $Na_2SO_4$, filtered, washed with EtOAc and concentrated under reduced pressure to give a residue. The residue was purified by column chromatography ($SiO_2$, Petroleum ether/Ethyl acetate=3/1 to 1/1) to give the **intermediate 93-3** (256 mg, 28.6% yield) as a yellow solid.

**Step 3: synthesis of intermediate 93-5**

To a solution of **intermediate 93-3** (256 mg, 1.23 mmol) and **intermediate 80-5** (708 mg, 3.69 mmol) in DMF (5 mL) was added $NaHCO_3$ (310 mg, 3.69 mmol).The mixture was stirred at 70 °C for 3 hrs. The reaction mixture was partitioned between EtOAc (100 mL) and $H_2O$ (100 mL). The organic phase was separated, washed with brine, dried over $Na_2SO_4$, filtered and concentrated under reduced pressure to give a residue. The residue was purified by column chromatography ($SiO_2$, Petroleum ether/Ethyl acetate=3/1 to 1/1) to give the **intermediate 93-5** (151 mg, 38.5% yield) as a yellow solid. LCMS ($ESI^+$): m/z 320.2 $[M+H]^+$.

**Step 4: synthesis of compound 93**

To a solution of **intermediate 93-5** (151 mg, 424 μmol) and **intermediate B 7-B** (165 mg, 315 μmol) in DCM (5 mL) was added TEA (95.7 mg, 946 μmol, 132 uL), the mixture was stirred at 25°C for 0.5 hr. Then the mixture was added AcOH (56.8 mg, 946 μmol, 54 uL) and stirred at 25°C for 0.5 hr. Then the mixture was added $NaBH(OAc)_3$ (134 mg, 630 μmol) and stirred at 25°C for 16 hrs. The reaction mixture was concentrated under reduced pressure to give a residue. The residue was purified by prep-HPLC (acetonitrile / water (2%FA)) to give **compound 93** (10.55 mg, 4.12% yield) as a yellow solid.

LCMS ($ESI^+$): m/z 790.6 $[M+H]^+$.

$^1H$ NMR (400MHz, DMSO-$d_6$) δ 10.80 (s, 1H), 10.18 (br s, 1H), 8.83 (s, 1H), 8.14 (s, 0.706H), 8.06 (br s, 1H), 7.82 - 7.71 (m, 1H), 7.66 - 7.46 (m, 3H), 7.00 - 6.89 (m, 2H), 6.88 - 6.78 (m, 1H), 6.55 - 6.46 (m, 1H), 6.45 - 6.36 (m, 1H), 5.85 - 5.76 (m, 1H), 5.72 - 5.59 (m, 1H), 5.35 (s, 1H), 4.99 (d, *J* = 9.6 Hz, 1H), 4.86 - 4.77 (m, 1H), 4.73 - 4.59 (m, 2H), 4.30 - 4.19 (m, 1H), 3.22 - 3.15 (m, 2H), 3.11 (br s, 4H), 2.80 - 2.72 (m, 1H), 2.68 (br s, 4H), 2.63 - 2.56 (m, 3H), 2.33 (br s, 1H), 2.13 - 2.03 (m, 1H), 1.91 - 1.78 (m, 3H), 1.66 - 1.52 (m, 2H), 1.46 (s, 6H).

The compound **148** was synthesized according to the procedures described for compound **93** using the different intermediates.

| Compound No. | Intermediate No. | MS($ESI^+$): m/z $[M+H]^+$ | $^1H$ NMR (400 MHz, DMSO-$d_6$) δ |
|---|---|---|---|
| 148 | B 8 | 760.36 | 10.78 (s, 1H), 10.27 (s, 1H), 8.87 (s, 1H), 8.23 - 7.94 (m, 2H), 7.75 (d,*J* = 8.0 Hz, 1H), 7.66 (d, *J* = 8.0 Hz, 2H), 7.61 (d, *J* = 8.0 Hz, 1H), 7.21 (d, *J* = 8.4 Hz, 2H), 6.55 - 6.46 (m, 1H), 6.45 - 6.36 (m, 2H), 5.73 - 5.61 (m, 1H), 5.57 (d, *J* = 7.6 Hz,1H), 5.34 (s, 1H), 4.99 (d, *J* = 10.0 Hz, 1H), 4.82 (d,*J* = 17.2 Hz, 1H), 4.68 (d, *J* = 5.6 Hz, 2H), 4.21 (s, 1H), 3.90 (s, 2H), 3.48 (s, 2H), 2.90 (s, 2H), 2.75 - 2.64 (m, 1H), 2.56 - 2.52 (m, 2H), 2.08 (s, 1H), 1.91 (s, 3H),1.75 (d, *J* = 11.1 Hz, 2H), 1.69 - 1.56 (m, 2H), 1.46 (s, 6H) |

**Example 100-A and 100-B: synthesis of compound 100-A** and **compound 100-B**

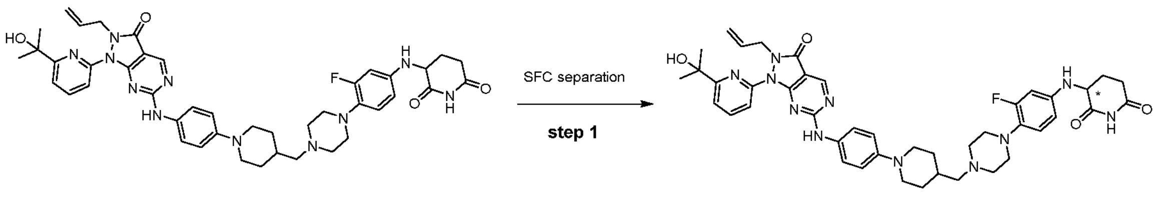


compound 71

compound 100-A (enantiomer A)
compound 100-B (enantiomer B)

**Step 1: synthesis of compound 100-A and compound 100-B**

The compound **71** (12 mg, 3.13 mmol) was separated by prep-SFC (Column: DAICEL CHIRALPAK IE (250mm*30mm,10μm); Mobile phase: [IPA-ACN]; $CO_2$%: 45%-45%). Compound **100-A** (1.18 mg, 9.83% yield) as peak **1** was obtained as a yellow solid. Compound **100-B** (0.76 mg, 6.33% yield) as peak **2** was obtained as a yellow solid.

Compound **100-A:**
Chiral analysis (Column: chiralpak IE-3, 100*4.6mm I.D., 3 μm; Mobile Phase: A:isopropanol (0.05%DEA), B: acetonitrile,Isocatic: A/B = 1/1 at 1.0 mL/ min; Temp: 35 °C; Wavelength: 254 nm, $R_T$ = 1.87 min).
Compound **100-B:**
Chiral analysis (Column: chiralpak IE-3, 100*4.6mm I.D., 3 μm; Mobile Phase: A:isopropanol (0.05%DEA), B: acetonitrile,Isocatic: A/B = 1/1 at 1.0 mL/ min; Temp: 35 °C; Wavelength: 254 nm, $R_T$ = 2.43 min).

| Compound No. | MS(ESI$^+$): m/z [M+H]$^+$ | MS(ESI$^+$): m/z [M+H]$^+$ |
|---|---|---|
| 100-A | 804.6 | 10.79 (s, 1H), 10.15 (br s, 1H), 8.82 (s, 1H), 8.05 (br s, 1H), 7.81 - 7.67 (m, 1H), 7.64 - 7.41 (m, 3H), 6.96 - 6.89 (m, 2H), 6.87 - 6.80 (m, 1H), 6.54 - 6.47 (m, 1H), 6.45 - 6.39 (m, 1H), 5.82 (d, *J* = 7.6 Hz, 1H), 5.71 - 5.60 (m, 1H), 5.35 (s, 1H), 4.99 (d, *J* = 10.0 Hz, 1H), 4.85 - 4.78 (m, 1H), 4.68 (br s, 2H), 4.30 - 4.21 (m, 1H), 3.67 - 3.60 (m, 2H), 2.86 (br s, 4H), 2.78 - 2.69 (m, 1H), 2.62 - 2.57 (m, 2H), 2.46 - 2.40 (m, 5H), 2.25 - 2.18 (m, 2H), 2.12 - 2.04 (m, 1H), 1.87 - 1.77 (m, 3H), 1.67 (br s, 1H), 1.46 (s, 6H), 1.23 (br s, 2H). |
| 100-B | 804.6 | 10.80 (s, 1H), 10.16 (br s, 1H), 8.82 (s, 1H), 8.05 (br s, 1H), 7.82 - 7.70 (m, 1H), 7.65 - 7.47 (m, 3H), 6.98 - 6.89 (m, 2H), 6.87 - 6.80 (m, 1H), 6.55 - 6.48 (m, 1H), 6.44 - 6.39 (m, 1H), 5.82 (d, *J* = 7.6 Hz, 1H), 5.71 - 5.60 (m, 1H), 5.35 (s, 1H), 4.99 (d, *J* = 9.2 Hz, 1H), 4.86 - 4.78 (m, 1H), 4.72 - 4.63 (m, 2H), 4.30 - 4.21 (m, 1H), 3.69 - 3.59 (m, 2H), 2.86 (br s, 4H), 2.78 - 2.69 (m, 1H), 2.62 - 2.56 (m, 2H), 2.42 (br s, 5H), 2.26 - 2.18 (m, 2H), 2.12 - 2.03 (m, 1H), 1.88 - 1.76 (m, 3H), 1.66 (br s, 1H), 1.46 (s, 6H), 1.23 (br s, 2H). |

**Example 102-A and 102-B: synthesis of compound 102-A and comound 102-B**

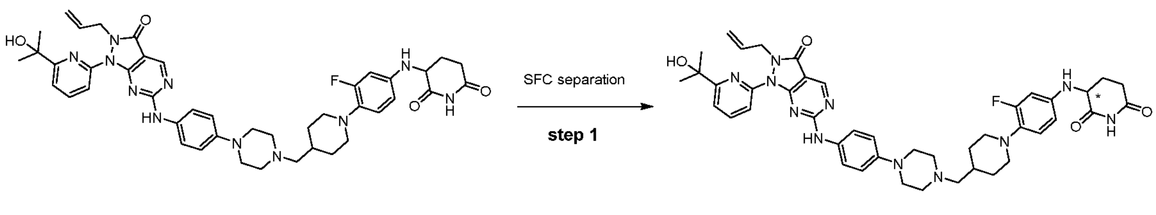


compound 74

compound 102-A (enantiomer A)
compound 102-B (enantiomer B)

**Step 1: synthesis of compound 102-A and compound 102-B**

The compound **74** (15.20 mg, 18.91 μmol) was separated by prep-SFC (Column: DAICEL CHIRALPAK IE (250mm*30mm,10μM); Mobile phase: [IPA-ACN]; $CO_2$%: 45%-45%. Compound **102-A** (2.10 mg, 13.82 %) as peak **1** was obtained as a yellow solid. Compound **102-B** (2.20 mg, 14.47 %) as peak **2** was obtained as a yellow solid.

Compound **102-A:**

Chiral analysis (Column: chiralpak IF-3, 100*4.6mm I.D., 3 μm; Mobile Phase: A:isopropanol (0.05%DEA), B: acetonitrile,Isocatic: A/B = 1/1 at 1.0 mL/ min; Temp: 35 °C; Wavelength: 254 nm, $R_t$ = 3.35 min).

Compound **102-B:**

Chiral analysis (Column: chiralpak IE-3, 100*4.6mm I.D., 3 μm; Mobile Phase: A:isopropanol (0.05%DEA), B: acetonitrile,Isocatic: A/B = 1/1 at 1.0 mL/ min; Temp: 35 °C; Wavelength: 254 nm, $R_t$ = 5.89 min).

| Compound No. | MS(ESI$^+$): m/z [M+H]$^+$ | MS(ESI$^+$): m/z [M+H]$^+$ |
|---|---|---|
| 102-A | 826.6 [M+Na]$^+$ | 10.79 (s, 1H), 10.17 (br s, 1H), 8.83 (s, 1H), 8.05 (br s, 1H), 7.80 -7.50 (m, 4H), 6.98 - 6.76 (m, 3H), 6.50 (d, *J* = 14.8 Hz, 1H), 6.41 (d, *J* = 8.0 Hz, 1H), 5.79 (d, *J* = 7.2Hz, 1H), 5.73 - 5.58 (m, 1H), 5.34 (s, 1H), 4.99 (d, *J* = 9.6 Hz, 1H), 4.82 (d, *J* = 17.6 Hz, 1H), 4.69(s, 2H), 4.26 (br s, 1H), 3.11 (br s, 6H), 2.73 (br s, 1H), 2.59 (br s, 2H), 2.46 - 2.36 (m, 5H), 2.23(br s, 2H), 2.07 (br s, 1H), 1.95 - 1.70 (m, 3H), 1.63 (br s, 1H), 1.46 (s, 6H), 1.24 (br s, 2H). |
| 102-B | 826.6 [M+Na]$^+$ | 10.79 (s, 1H), 10.18 (br s, 1H), 8.84 - 8.79 (m, 1H), 8.05 (br s, 1H),7.80 - 7.51 (m, 4H), 6.93 (d, *J* = 8.8 Hz, 2H), 6.88 - 6.80 (m, 1H), 6.50 (d, *J* = 15.2 Hz, 1H), 6.42 (d, *J* = 8.0 Hz, 1H), 5.79 (d, *J* = 7.6 Hz, 1H), 5.74 - 5.59 (m, 1H), 5.34 (s, 1H), 4.99 (d, *J* = 10.4 Hz, 1H), 4.82 (d, *J* = 17.2 Hz, 1H), 4.68 (d, *J* = 4.4 Hz, 2H), 4.32 - 4.20 (m, 1H), 3.11 (br s, 6H), 2.80 - 2.70(m, 1H), 2.59 (br s, 2H), 2.43 (br s, 5H), 2.24 ( d, *J* = 4.8 Hz, 2H), 2.14 - 2.04 (m, 1H), 1.91 -1.75 (m, 3H), 1.62 (br s, 1H), 1.46 (s, 6H), 1.24 (br s, 2H). |

**Example 104: synthesis of compound 104**

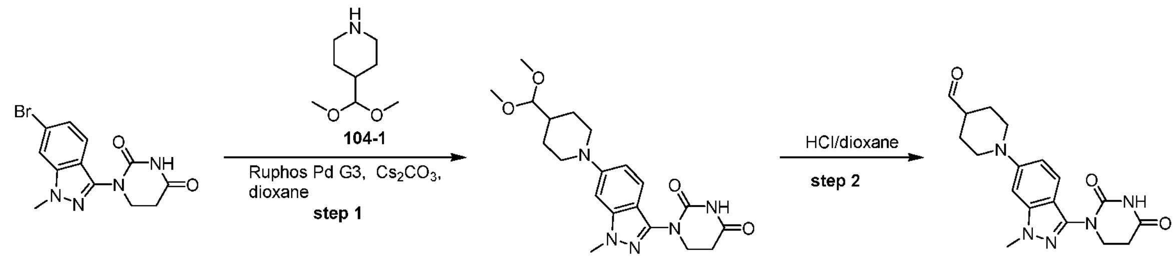


B 23 104-2 104-3

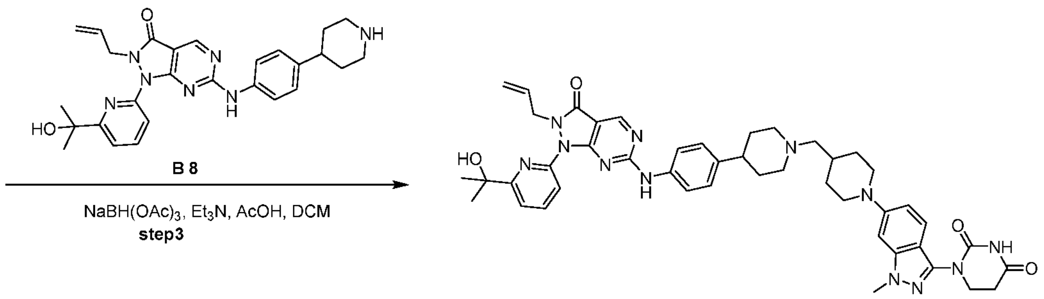


104

**Step 1: synthesis of intermediate 104-2**

A mixture of **intermediate B 23** (500 mg, 1.55 mmol), **intermediate 104-1** (320 mg, 2.01 mmol), RuPhos Pd G3 (388 mg, 464 μmol), $Cs_2CO_3$ (1.51 g, 4.64 mmol) in dioxane (5 mL) was stirred at 100°C for 2 hrs under $N_2$ atmosphere. The reaction mixture was filtered and concentrated under reduced pressure to give a residue. The residue was purified by column chromatography ($SiO_2$, Petroleum ether/Ethyl acetate=3/1 to 1/1) to give the **intermediate 104-2** (160 mg, 17.8% yield) as a light yellow solid.

LCMS (ESI+): m/z 402.5 $[M+H]^+$.

**Step 2: synthesis of intermediate 104-3**

A mixture of **intermediate 104-2** (160 mg, 399 μmol), HCl/dioxane (4 M, 3 mL) in DCM (5 mL) was stirred at 20 °C for 1 hr. The reaction mixture was concentrated under reduced pressure to give the **intermediate 104-3** (160 mg, crude) as a brown solid. The crude product was used into the next step without further purification.

LCMS (ESI+): m/z 356.3 $[M+H]^+$.

**Step 3: synthesis of compound 104**

To a solution of **intermediate 104-3** (80 mg, 225 μmol) and **intermediate B 8** (109 mg, 225 μmol) in DCM (5 mL) was added dropwise $Et_3N$ (68.3 mg, 675 μmol) and AcOH (27 mg, 450 μmol). After addition, the mixture was stirred at this temperature for 30 min, and then $NaBH(OAc)_3$ (143 mg, 675 μmol) was added dropwise. The resulting mixture was stirred at 20°C for 16 hrs. The reaction mixture was filtered and concentrated under reduced pressure to give a residue. The residue was purified by prep-HPLC (acetonitrile / water (2% FA) to give **compound 104** (13 mg, 6.79% yield) as a pink solid.

LCMS (ESI+): m/z 825.6 $[M+H]^+$.

$^1$HNMR (400MHz, DMSO-d6) δ 10.50 (s, 1H), 10.24 (br s, 1H), 8.87 (s, 1H), 8.23 (s, 0.858FA), 8.10 - 8.03 (m, 1H), 7.75 (d, *J* = 8.0 Hz, 1H), 7.69 - 7.59 (m, 3H), 7.43 (d, *J* = 8.0 Hz, 1H), 7.22 (d, *J* = 12.0 Hz, 2H), 6.91 (d, *J* = 8.0 Hz, 1H), 6.80 (s, 1H), 5.72 - 5.61 (m, 1H), 5.36 (br s, 1H), 4.99 (d, *J* = 12.0 Hz, 1H), 4.82 (d, *J* = 16.0 Hz, 1H), 4.68 (d, *J* = 8.0 Hz, 2H), 3.92 - 3.84 (m, 5H), 3.80 (d, *J* = 12.0 Hz, 2H), 2.97 (d, *J* = 8.0 Hz, 2H), 2.77 - 2.70 (m, 4H), 2.46 (br s, 1H), 2.21 (d, *J* = 8.0 Hz, 2H), 2.04 - 1.93 (m, 2H), 1.83 (d, *J* = 12.0 Hz, 2H), 1.77 - 1.70 (m, 3H), 1.69 - 1.60 (m, 2H), 1.46 (s, 6H), 1.33 - 1.19 (m, 2H).

The **compound 105, 117, 124, 129** and **compound 143** were synthesized according to the procedures described for **compound 104** using the different intermediates.

| Compound No. | Intermediate No. | MS(ESI+): m/z $[M+H]^+$ | $^1H$ NMR (400 MHz, DMSO-$d_6$) $\delta$ |
|---|---|---|---|
| 105 | B 7-B | 826.6 | 10.51 (s, 1H), 10.16 (br s, 1H), 8.83 (s, 1H), 8.29 (s, 0.568H), 8.05 (br s, 1H), 7.75 (d, *J* = 8.0 Hz, 1H), 7.61 (d, *J* = 8.0 Hz, 3H), 7.43 (d, *J* = 8.0 Hz, 1H), 6.97 - 6.88 (m, 3H), 6.81 (s, 1H), 5.74 - 5.57 (m, 1H), 5.33 (br s, 1H), 4.99 (d, *J* = 8.0 Hz, 1H), 4.82 (d, *J* = 16.0 Hz, 1H), 4.68 (d, *J* = 8.0 Hz, 2H), 3.91 - 3.87 (m, 5H), 3.81 (d, *J* = 16.0 Hz, 2H), 3.12 (br s, 4H), 2.80 - 2.71 (m, 4H), 2.52 - 2.51 (m, 4H), 2.24 (d, *J* = 8.0 Hz, 2H), 1.84 (d, *J* = 12.0 Hz, 2H), 1.77 (br s, 1H), 1.46 (s, 6H), 1.34 - 1.21 (m, 2H). |
| 117 | B 7-B B-24 | 825.5 | 10.89 (s, 1H), 10.17 (br s, 1H), 8.83 (s, 1H),8.05 (br s, 1H), 7.75 (d, *J* = 8.0 Hz, 1H), 7.61 (br d, *J* = 8.0 Hz, 3H), 7.42 - 7.33 (m, 1H), 7.07 - 6.88 (m, 4H), 5.75 - 5.58 (m, 1H), 5.33 (s, 1H), 4.99 (br d, *J* = 8.0 Hz, 1H), 4.82 (br d, *J* = 16.0 Hz, 1H), 4.69 (br s, 2H), 4.37 - 4.30 (m, 1H), 4.25 (s, 3H), 3.30 - 2.89 (m, 8H), 2.70 (br s, 2H), 2.62 (br d,*J* = 4.0 Hz, 2H), 2.58 - 2.53 (m, 2H), 2.32 (br d, *J* = 8.0 Hz, 3H), 2.22 - 2.10 (m, 1H), 1.90 (br d, *J* = 12.0 Hz, 2H), 1.78 (br s, 1H), 1.46 (s, 8H) |
| 124 | B 36; B-24 | 852.5 | 10.55 (s, 1H), 10.15 (br s, 1H), 8.82 (s, 1H), 7.93 (br s, 1H), 7.70 (d, *J* = 8.0 Hz, 1H), 7.59 (br s, 2H), 7.36 - 7.21 (m, 1H), 7.11 - 6.86 (m, 4H), 5.77 - 5.58 (m, 1H), 5.06 (s,1H), 4.99 (d, *J* = 10.0 Hz, 1H), 4.91 - 4.66 (m, 2H), 4.64 - 4.46 (m, 1H), 4.24 (s, 3H), 3.88 (t, *J* = 6.4Hz, 2H), 3.12 (br s, 4H), 3.03 - 2.91 (m, 1H), 2.87 - 2.67 (m, 5H), 2.58 - 2.54 (m, 4H), 2.43 (br s,1H), 2.31 (d, *J* = 14.4 Hz, 2H), 2.24 - 2.14 (m, 1H), 2.08 - 1.96 (m, 1H), 1.90 (d, *J* = 13.2 Hz, 3H), 1.80-1.61 (m, 2H), 1.40 (d, *J* = 11.2 Hz, 2H), 0.87 (t, *J* = 7.2 Hz, 3H) |
| 129 | B 38; 104-3 | 866.5 | 10.52 (s, 1H), 10.17 (br s, 1H), 8.85 (s, 1H), 7.90 (br s, 1H), 7.72 (d, *J* = 8.4 Hz, 2H), 7.44 (d, *J* = 8.4 Hz, 2H), 7.09 - 6.76 (m,3H), 5.75 - 5.62 (m, 1H), 5.08 (s, 1H), 5.03 - 4.98 (m, 1H), 4.89 - 4.82 (m, 1H), 4.81 - 4.70 (m, 1H), 4.62 - 4.50 (m, 1H), 3.92 - 3.87 (m, 5H), 3.83 (br s, 2H),3.15 - 3.05 (m, 3H), 3.01 - 2.92 (m, 1H), 2.89 - 2.78 (m, 4H), 2.78 - 2.70 (m, 4H), 2.58 - 2.53 (m, 4H), 2.29 - 2.16 (m, 5H), 2.07 - 1.98 (m, 1H), 1.93 - 1.81(m, 3H), 1.75 - 1.65 (m, 1H), 1.33 - 1.24 (m, 2H), 0.93 - 0.78 (m, 3H) |
| 143 | B 7-B; B 25 | 826.5 | 10.56 (br s, 1H), 10.17 (br s, 1H), 8.83 (s, 1H), 8.22 (br s, 0.343 H), 8.05 (br s, 1H), 7.85 - 7.71 (m, 1H), 7.68 - 7.46 (m, 3H), 7.28 (br s, 1H), 7.11 - 6.84 (m, 4H), 5.73 - 5.58 (m, 1H), 5.34 (br s, 1H), 5.04 - 4.93 (m, 1H), 4.85 - 4.77 (m, 1H), 4.68 (br s, 2H), 4.24 (s, 3H), 3.88 (br s, 2H), 3.30 - 3.21 (m, 4H), 3.11 (br s, 4H), 2.81 - 2.63 (m, 4H), 2.54 (br s, 2H), 2.35 - 2.21 (m, 1H), 2.29 (br s, 1H), 1.96 - 1.83 (m, 2H), 1.73 (br s, 1H), 1.46 (s, 6H), 1.42 - 1.32 (m, 2H). |

**Example 106: synthesis of compound 106**

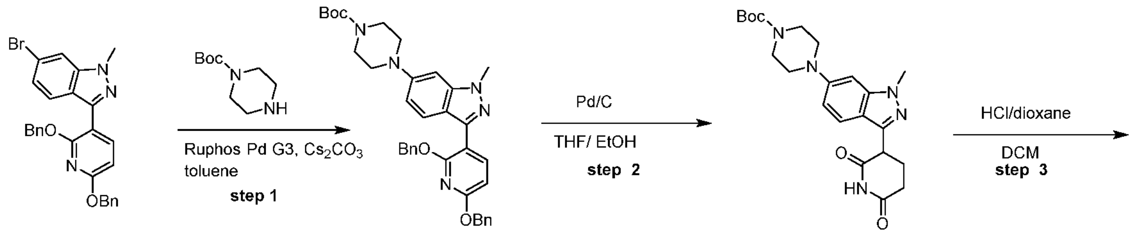


106-1 106-2 106-3

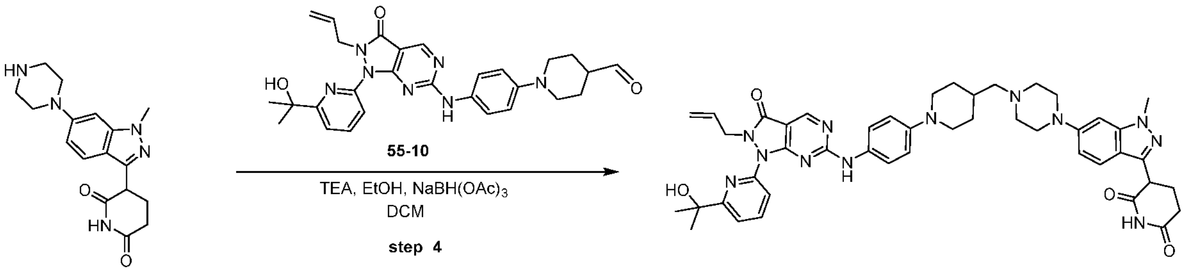


106-4 106

**Step 1: synthesis of intermediate 106-2**

A mixture of **intermediate 106-1** (500 mg, 999 μmol), tert-butyl piperazine-1-carboxylate (372 mg, 2.00 mmol), $Cs_2CO_3$ (977 mg, 3.00 mmol) and [2-(2-aminophenyl)phenyl]-methylsulfonyloxy-palladium;dicyclohexyl-[2-(2,6-diisopropoxyphenyl)phenyl]phosphane (84 mg, 99.9 μmol) in toluene (5 mL) was degassed and purged with $N_2$ for 3 times, and then the mixture was stirred at 100 °C for 16 hrs under $N_2$ atmosphere. The reaction mixture was partitioned between EtOAc (50 mL) and $H_2O$ (50 mL). The organic phase was separated, washed with brine, dried over $Na_2SO_4$, filtered and concentrated under reduced pressure to give a residue. The residue was purified by column chromatography ($SiO_2$, Petroleum ether/Ethyl acetate=5/1 to 1/1) to give **intermediate 106-2** (302 mg, 40.42% yield) as a yellow solid. LCMS ($ESI^+$): m/z 606.5 $[M+H]^+$.

**Step 2: synthesis of intermediate 106-3**

To a solution of **intermediate 106-2** (302 mg, 499 μmol), Pd/C (300 mg, 10% purity) in THF (10 mL) and EtOH (10 mL) was degassed and purged with $H_2$ for 3 times, and then the mixture was stirred at 25°C for 16 hrs under $H_2$ atmosphere. The reaction mixture was filtered and concentrated under reduced pressure to give a residue. The residue was purified by column chromatography ($SiO_2$, Petroleum ether/Ethyl acetate=1/1 to 0/1) to give intermediate **106-3** (118 mg, 53.7% yield) as a white solid. LCMS ($ESI^+$): m/z 428.2 $[M+H]^+$.

**Step 3: synthesis of intermediate 106-4**

To a solution of **intermediate 106-3** (118 mg, 276 μmol) in DCM (2 mL) was added HCl/dioxane (4 M, 2 mL).The mixture was stirred at 20°C for 0.5 hr. The reaction mixture was filtered and concentrated under reduced pressure to give the **intermediate 106-4** (115 mg, crude) as a white solid, which was used into next step directly.

**Step 4: synthesis of comound 106**

To a solution of **intermediate 106-4** (115 mg, 316 μmol) and **intermediate 55-10** (174 mg, 316 μmol) in DCM (5 mL) was added TEA (96 mg, 948 μmol, 132 uL) and stirred at 25°C for 0.5 hr. Then the mixture was added AcOH (43.7 mg, 727 μmol, 41.6 uL) and stirred at 25°C for 0.5 hr. Then the mixture was added $NaBH(OAc)_3$ (134 mg, 632 μmol) and stirred at 25°C for 16 hrs. The reaction mixture was concentrated under reduced pressure to give a residue. The residue was purified by prep-HPLC (acetonitrile / water (2%FA)) to give **compound 106** (65 mg, 23.71% yield) as a yellow solid. LCMS ($ESI^+$): m/z 825.5 $[M+H]^+$.

$^1H$ NMR (400MHz, DMSO-$d_6$) δ 10.87 (s, 1H), 10.16 (br s, 1H), 8.82 (s, 1H), 8.17 (s, 0.460H), 8.05 (br s, 1H), 7.79 - 7.71 (m, 1H), 7.63 - 7.53 (m, 3H), 7.50 (d, *J* = 8.8 Hz, 1H), 6.93 (d, *J* = 8.8 Hz, 3H), 6.85 (s, 1H), 5.72 - 5.59 (m, 1H), 5.35 (br s, 1H), 4.99 (d, *J* = 10.4 Hz, 1H), 4.86 - 4.78 (m, 1H), 4.72 - 4.61 (m, 2H), 4.30 - 4.23 (m, 1H), 3.89 (s, 3H), 3.69 - 3.60 (m, 2H), 3.23 (br s, 3H), 2.70 - 2.59 (m, 4H), 2.54 (br s, 5H), 2.34 - 2.28 (m, 1H), 2.27 - 2.21 (m, 2H), 2.20 -

2.10 (m, 1H), 1.88 - 1.77 (m, 2H), 1.70 (br s, 1H), 1.46 (s, 6H), 1.31 - 1.19 (m, 2H).

The **compound 118** was synthesized according to the procedures described for **compound 106** using thedifferent intermediates.

| Compound No. | Intermediate No. | MS(ESI$^+$): m/z $[M+H]^+$ | $^1H$ NMR (400 MHz, DMSO-$d_6$) $\delta$ |
|---|---|---|---|
| 118 | 55-10; B 25 | 826.5 | 10.56 (s, 1H), 10.15 (br s, 1H), 8.82 (s, 1H), 8.14 (s, 0.569H), 8.05 (br s, 1H), 7.79 - 7.71 (m, 1H), 7.64 - 7.48 (m, 3H), 7.33 - 7.28 (m, 1H), 7.10 - 6.99 (m, 2H), 6.97 -6.88 (m, 2H), 5.72 - 5.60 (m, 1H), 5.34 (s, 1H), 5.02 - 4.96 (m, 1H), 4.86 - 4.78 (m, 1H), 4.72 -4.62 (m, 2H), 4.24 (s, 3H), 3.92 - 3.85 (m, 2H), 3.71 - 3.60 (m, 2H), 3.53 - 3.37 (m, 4H), 3.27 (br s,4H), 3.21 - 3.07 (m, 2H), 2.90 (br s, 2H), 2.79 - 2.73 (m, 2H), 1.89 - 1.79 (m, 2H), 1.71 (br s, 1H),1.46 (s, 6H), 1.33 - 1.25 (m, 2H) |

**Example 107: synthesis of compound 107**

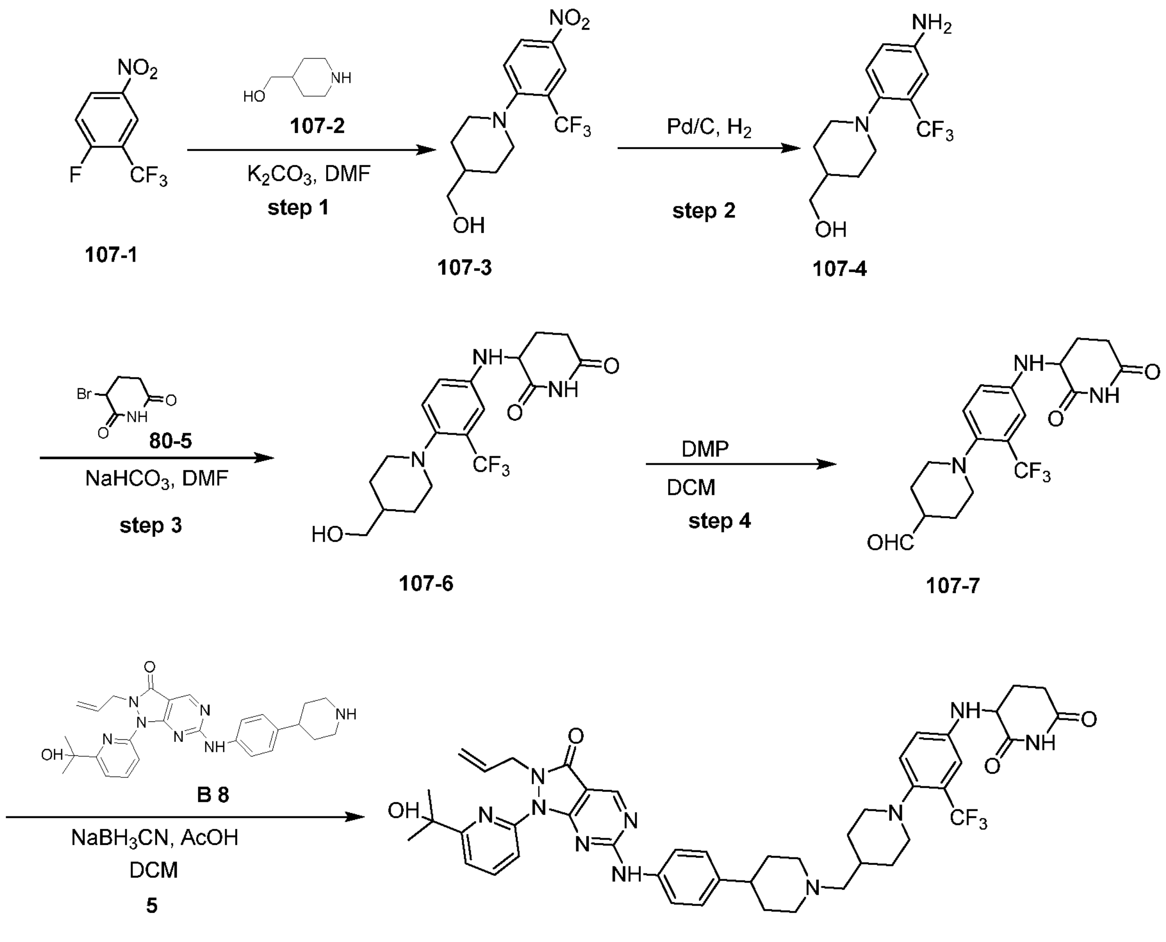


107

**Step 1: synthesis of intermediate 107-3**

To a solution of **107-1** (5 g, 23.91 mmol) and **107-2** (2.75 g, 23.91 mmol) in DMF (10 mL) was added $K_2CO_3$ (9.91 g, 71.74 mmol) and the mixture was stirred at 80 °C for 3 hrs under $N_2$. The mixture was poured into water (30

mL) and extracted with ethyl acetate (40 mL × 2). The combined organic extracts were washed with brine (20 mL), dried over anhydrous $Na_2SO_4$ filtered and concentrated under reduced pressure to give a residue, which was purified by flash silica gel chromatography (ISCO®; 20 g SepaFlash® Silica Flash Column, Eluent of 0~50% Ethyl acetate/Petroleum ethergradient at 60 mL/min) to afford the title intermediate **107-3** (7.1 g, 97.6% yield) as a yellow solid
$^1$H NMR (400 MHz, $CDCl_3$) δ 8.51 - 8.48 (m, 1H), 8.32 - 8.26 (m, 1H), 7.26 - 7.22 (m, 1H), 3.62 - 3.54 (m, 2H), 3.48 - 3.39 (m, 2H), 2.93 - 2.90 (m, 1H), 2.88 - 2.84 (m, 1H), 1.92 - 1.81 (m, 2H), 1.72 - 1.66 (m, 1H), 1.53 - 1.41 (m, 2H).

**Step 2: synthesis of intermediate 107-4**

To a solution of **107-3** (3 g, 9.86 mmol) in EtOAc (20 mL) was added Pd/C (0.325 g, 985.98 μmol, 10%) and the mixture was stirred at 25°C for 12 hr under $H_2$ (15 psi) . The mixture was filtered and concentrated under reduced pressure to afford **intermediate 107-4** (2.5 g, 92.44% yield) as a yellow solid.
LCMS (ESI$^+$): m/z 275.9[M+H]$^+$.

**Step 3: synthesis of intermediate 107-6**

To a solution of **107-4** (2.5 g, 9.11 mmol) and intermediate **80-5** (5.25 g, 27.34 mmol) in DMF (18 mL) was added $NaHCO_3$ (4.59 g, 54.69 mmol) and the mixture was stirred at 70 °C for 12 hrs. The mixture was poured into water (100 mL) and extracted with ethyl acetate (40 mL × 2). The combined organic extracts were washed with brine (80 mL), dried over anhydrous $Na_2SO_4$, filtered and concentrated under reduced pressure to afford the crude product. The crude product was purified by flash silica gel chromatography ( Ethyl acetate/Petroleum ethergradient = 0/1 to 1/1) to afford the title **intermediate 107-6** (1.72 g, crude) as a yellow solid.

**Step 4: synthesis of intermediate 107-7**

To a solution of intermediate **107-6** (300 mg, 778.45 μmol) in DCM (10 mL) was added DMP (660.35 mg, 1.56 mmol) and the mixture was stirred at 25 °C for 10 min. The mixture was poured into aq. $Na_2S_2O_3$ (Sat. 6 mL) and $NaHCO_3$ (Sat. 8mL) and extracted with ethyl acetate (6 mL x 2). The combined organic extracts were washed with brine (20 mL), dried over anhydrous $Na_2SO_4$, filtered and concentrated under reduced pressure to afford the intermediate **107-7** (170 mg, crude) as a blue solid. The residue was used for the next step reaction directly.

**Step 5: synthesis of compound 107**

To a solution of intermediate **107-7** (40 mg, 104.34 μmol) and intermediate **B 8** (42.86 mg, 82.10 μmol) in DCM (8 mL) was added acetic acid (66.34 mg, 313.02 μmol) and the mixture was stirred at 25 °C for 12 hrs, then sodium triacetoxyboranuide (66.34 mg, 313.02 μmol, 3 eq) was added to the mixture and the mixture was stirred at 25 °C for 1.5 hrs. The mixture was concentrated under reduced pressure to afford the crude product. The residue was purified by prep-HPLC (acetonitrile / water (0.1%TFA)) to give the desired **compound 107** (18 mg, 18.87% yield) as white solids.
LCMS (ESI$^+$): m/z 853.5 [M+H]$^+$.
$^1$H NMR (400 MHz, DMSO-$d_6$) δ 10.81 (s, 1H), 10.38 - 10.21 (m, 1H), 8.89 -8.87 (m, 1H), 8.09 - 7.96 (m, 1H), 7.78 - 7.74 (m, 1H), 7.73 - 7.68 (m, 2H), 7.66 - 7.61 (m, 1H), 7.32 - 7.27 (m, 1H), 7.25 - 7.19 (m, 2H), 6.95 - 6.92 (m, 1H), 6.92 - 6.87 (m, 1H), 5.76 - 5.60 (m, 1H), 5.02 - 4.98 (m, 1H), 4.88 - 4.77 (m,1H), 4.73 - 4.64 (m, 2H), 4.42 - 4.32 (m, 1H), 3.10 - 2.99 (m, 4H), 2.88 - 2.53 (m, 8H), 2.23 - 1.79 (m, 11H), 1.47 (s, 6H), 1.40 - 1.32 (m, 2H).

**Example 108: synthesis of compound 108**

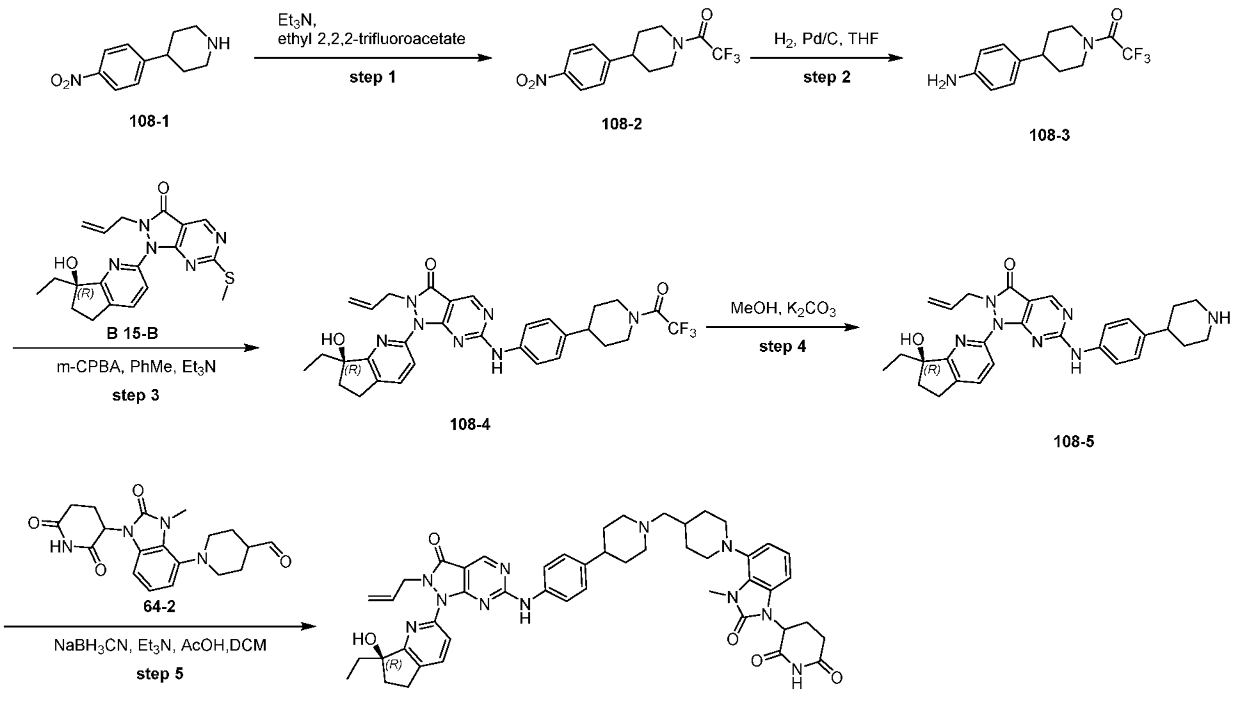


108-1 108-2 108-3

B 15-B

108-4 108-5

64-2

108

**Step 1: synthesis of intermediate 108-2**

To a solution of **intermediate 108-1** (500 mg, 2.42 mmol) in ethyl 2,2,2-trifluoroacetate (8 mL) was added TEA (491 mg, 4.85 mmol). The mixture was stirred at 80°C for 16 hrs. The reaction mixture was partitioned between EA (30 mL) and $H_2O$ (20 mL). The organic phase was separated, washed with saturated NaCl (10 mL), dried over $Na_2SO_4$, filtered and concentrated under reduced pressure to give the **intermediate 108-2** (750 mg, crude) as brown oil.

**Step 2: synthesis of intermediate 108-3**

To a solution of **intermediate 108-2** (750 mg, 2.48 mmol,) in THF (8 mL) was added Pd/C (500 mg, 5% purity) under $N_2$. The suspension was degassed under vacuum and purged with $H_2$ three times. The mixture was stirred under $H_2$ (15 PSi) at 30°C for 16 hours. The reaction mixture was filtered, washed with DCM (50 mL) and the filtrate was concentrated to the crude product **intermediate 108-3** (670 mg, crude) as a white solid, which was directly used in next step.

**Step 3: synthesis of intermediate 108-4**

To a colorless solution of **intermediate B 15-B** (40.0 mg, 104 μmol) in toluene (3 mL) was added m-CPBA (23.3 mg, 115 μmol, 85% purity) and stirred at 20°C for 30 min. Then $Et_3N$ (21.5 mg, 212 μmol) and **intermediate 108-3** (31.2 mg, 115 μmol) was added. The reaction mixture was stirred at 20°C for 16 hours. The reaction mixture was concentrated to give a residue. The residue was purified by prep-TLC (DCM: MeOH=10:1) to give the intermediate **108-4** (45.0 mg, 71.0% yield) as a yellow oil.

**Step 4: synthesis of intermediate 108-5**

A white suspension of intermediate **108-4** (45.0 mg, 74.1 μmol) , $K_2CO_3$ (15.35 mg, 111 μmol) and MeOH (2 mL) was stirred at 30°C for 16 hours. The reaction mixture was diuted with DCM (30 mL) and washed with water (10 mL). The orgnic layer was dried over $Na_2SO_4$, filtered and concenrtated to give the **intermediate 108-5** (24.0 mg, crude) as a yellow solid which was used into next step directly.

LCMS ($ESI^+$): m/z 512.4 $[M+H]^+$.

**Step 5: synthesis of compound 108**

A mixture of **intermediate 108-5** (24.0 mg, 46.9 μmol) , **intermediate 64-2** (17.4 mg, 46.9 μmol) , $Et_3N$ (14.24 mg, 140.7 μmol) in DCM (2 mL) was stirred at 20°C for 30 min and then AcOH (10.0 mg, 166.52 μmol) was added. The reaction mixtue was stirred at 20°C for 30 min and then $NaBH_3CN$ (5.90 mg, 93.8 μmol) was added. The reaction mixture was stirred at 20°C for 16 hours. The reaction mixture was diluted with DCM (30 mL) and washed with water (30 mL). The organic layer was dried over $Na_2SO_4$, filtered and concentrated to give a residue. The residue was purified by prep-TLC (DCM: MeOH=10: 1) to give **compound 108** (7 mg, 16% yield) as a white solid. LCMS (ESI$^+$): m/z 866.6[M+H]$^+$.

$^1$H NMR (400 MHz, DMSO-$d_6$) δ 11.12 (s, 1H), 10.29 (br s, 1H), 9.19 - 8.93 (m, 1H), 8.88 (s, 1H), 7.92 (br s, 1H), 7.70 (d, *J* = 8.0 Hz, 3H), 7.21 (d, *J* = 8.4 Hz, 2H), 7.09 - 6.82 (m, 3H), 5.75 - 5.60 (m, 1H), 5.36 (dd, *J* = 5.2, 12.4 Hz, 1H), 5.09 (s, 1H), 5.00 (d, *J* = 10.4 Hz, 1H), 4.85 (d, *J* = 17.6 Hz, 1H), 4.80 - 4.69 (m, 1H), 4.56 (dd, *J* = 5.6, 15.6 Hz, 1H), 3.64 (s, 5H), 3.15 (br s, 4H), 3.01 - 2.84 (m, 3H), 2.83 - 2.68 (m, 4H), 2.44 (d, *J* = 8.8 Hz, 1H), 2.27 - 2.16 (m, 2H), 2.09 - 1.94 (m, 5H), 1.93 - 1.82 (m, 4H), 1.80 - 1.63 (m, 2H), 1.45 (br s, 2H), 0.87 (t, *J* = 7.6 Hz, 3H).

**Example 110: synthesis of compound 110, compound 150-A** and **compound 150-B**

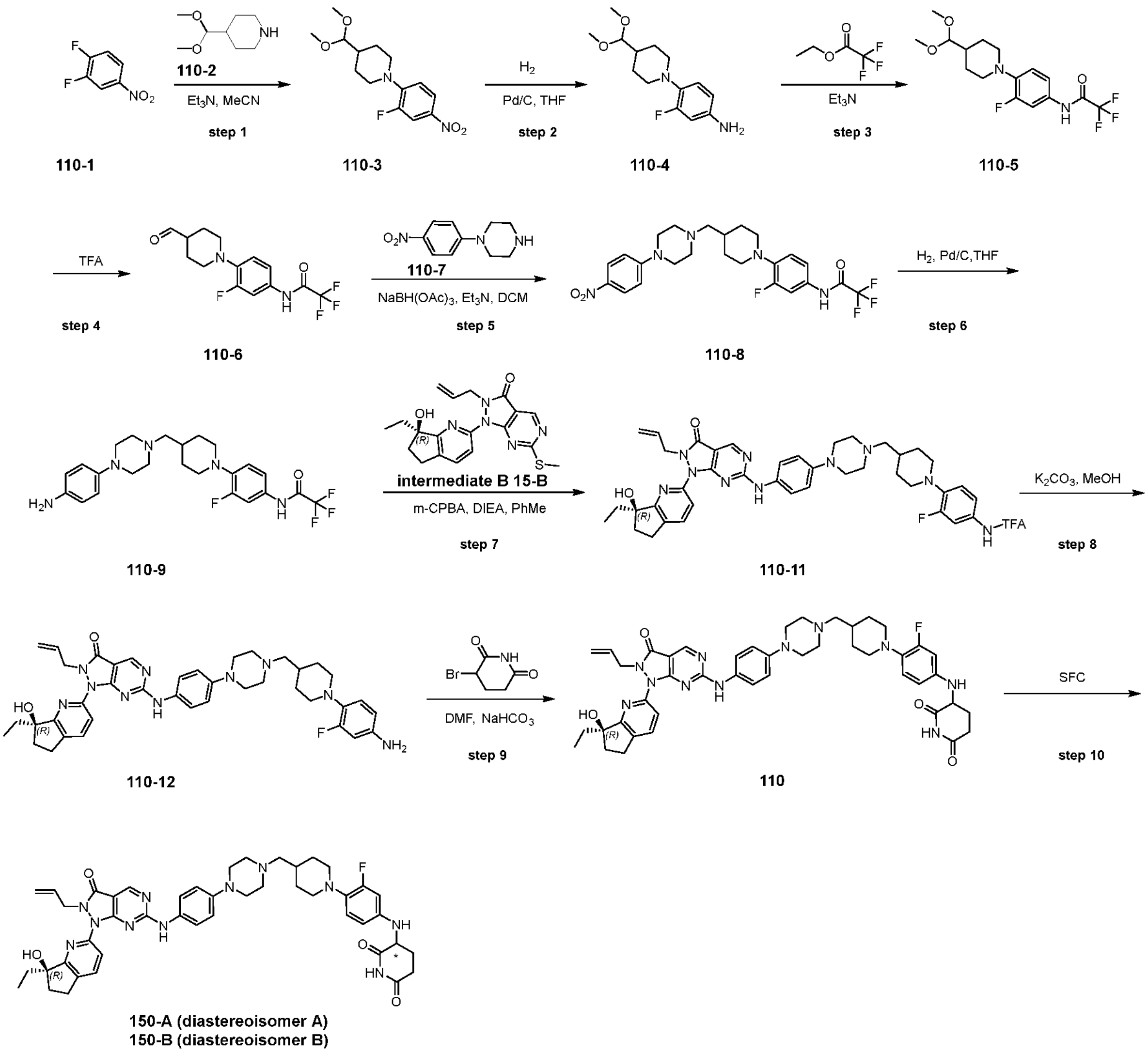


**Step 1: synthesis of intermedite 110-3**

To a solution of **intermediate 110-1** (10 g, 62.9 mmol) and **intermediate 110-2** (15.0 g, 94.3 mmol) in MeCN (100 mL) was added TEA (12.7 g, 126 mmol). The mixture was stirred at 90 °C for 16 hrs. The reaction mixture was

concentrated under reduced pressure to give a residue. The residue was purified by column chromatography ($SiO_2$, Petroleum ether/Ethyl acetate=1/0 to 10/1) to give the **intermediate 110-3** (17.8 g, 85.4% yield) as an orange solid.

$^1$H NMR (400 MHz, chloroform-*d*) 7.97 (m, 1H), 7.89 (m, 1H), 6.91 (t, *J* = 8.8 Hz, 1H), 4.10 (d, *J* = 6.8 Hz, 1H), 3.79 - 3.70 (m, 2H), 3.39 (s, 6H), 2.85 (m, 2H), 1.92 - 1.77 (m, 3H), 1.58 - 1.42 (m, 2H).

**Step 2: synthesis of intermedite 110-4**

To a solution of **intermediate 110-3** (17.8 g, 59.7 mmol) in THF (200 mL) was added Pd/C (10%, 4 g) under $N_2$ atmosphere. The suspension was degassed and purged with $H_2$ for 3 times. The mixture was stirred under $H_2$ (15 Psi) at 15 °C for 16 hrs. The reaction mixture was filtered, wahed with DCM(200 mL) and concentrated under reduced pressure to give the **intermediate 110-4** (15.8 g, crude) was obtained as an orange solid.

**Step 3: synthesis of intermedite 110-5**

To a solution of **intermediate 110-4** (15.8 g, 58.9 mmol) in ethyl 2,2,2-trifluoroacetate (102 g, 717 mmol) was added TEA (32.4 g, 320 mmol). The mixture was stirred at 80 °C for 16 hrs. The reaction mixture was concentrated under reduced pressure to give a residue. The crude product was triturated with (PE: EA=3:1, 50 mL), filtered and dried to give the **intermediate 110-5** (20.5 g, 86.0% yield) as a white solid.

$^1$H NMR (400 MHz, $CDCl_3$) 8.02 (br s, 1H), 7.42 (d, *J* = 13.2 Hz, 1H), 7.18 (d, *J* = 8.8 Hz, 1H), 6.94 (t, *J* = 8.4 Hz, 1H), 4.15 - 4.05 (m, 1H), 3.46 (d, *J* = 10.8 Hz, 2H), 3.38 (d, *J* = 1.2 Hz, 6H), 2.63 (t, *J* = 12 Hz, 2H), 1.85 (d, *J* = 12.8 Hz, 2H), 1.53 (m, 2H), 1.36 - 1.25 (m, 1H).

**Step 4: synthesis of intermedite 110-6**

A solution of **intermediate 110-5** (20.5 g, 56.3 mmol) in TFA (100 mL) was stirred at 50 °C for 1.5 hrs. The reaction mixture was concentrated under reduced pressure to give the **intermediate 110-6** (37 g, 3.0 eq TFA salt) as brown oil. The crude product was used into the next step without further purification. LCMS (ESI$^+$): m/z 319.2 $[M+H]^+$.

**Step 5: synthesis of intermedite 110-8**

To a mixture of **intermediate 110-6** (37 g, 56.0 mmol, 3TFA) and **intermediate 110-7** (11.6 g, 56.0 mmol) in DCM (200 mL) was added TEA (56.7 g, 560 mmol) at 25°C and stirred at 25 °C for 30 min, then AcOH (42.0 g, 699mmol) was added and stirred for 0.5hr. The reaction mixture was added $NaBH(OAc)_3$ (35.6 g, 168 mmol, 3 eq) at 25°C and stirred at 25 °C for 16 hours. The reaction mixture was diluted with water (200 mL) and DCM (200 mL), and extracted with DCM for three times. The combined organic layers were washed with sat. aq. NaCl (100 mL), dried over $Na_2SO_4$, filtered and concentrated under reduced pressure to give a residue. The residue was purified by column chromatography ($SiO_2$, DCM: MeOH =1/0 to 10/1) to give the **intermediate 110-8** (13.6 g, 39.5% yield) as a yellow solid.

LCMS (ESI$^+$): m/z 509.6 $[M+H]^+$.

$^1$H NMR (400 MHz, DMSO-$d_6$) $\delta$ 11.25 (s, 1H), 8.14 - 7.96 (m, 2H), 7.49 (d, *J* = 14.2 Hz, 1H), 7.39 (d, *J* = 8.8 Hz, 1H), 7.15 - 6.91 (m, 3H), 3.45 (br s, 4H), 3.33 - 3.31 (m, 4H), 2.65 (t, *J* = 11.2 Hz, 2H), 2.58 - 2.52 (m, 2H), 2.23 (d, *J* = 6.8 Hz, 2H), 1.81 (d, *J* = 12.0 Hz, 2H), 1.68 (br s, 1H), 1.31 - 1.20 (m, 2H).

**Step 6: synthesis of intermedite 110-9**

To a solution of **intermediate 110-8** (1 g, 1.96 mmol) in THF (20 mL) was added Pd/C (10%, 500 mg) under $N_2$ atmosphere. The suspension was degassed and purged with $H_2$ for 3 times. The mixture was stirred under $H_2$ (15 Psi) at 20 °C for 16 hrs. The reaction mixture was filtered, washed with (DCM: MeOH=10:1, 200 mL) and concentrated under reduced pressure to give a residue. The residue was purified by column chromatography ($SiO_2$, DCM: MeOH = 1:0 to 10:1) to give the **intermediate 110-9** (624 mg, 61.7% yield) as a gray white solid. LCMS (ESI$^+$): m/z 480.9 $[M+H]^+$.

**Step 7: synthesis of intermedite 110-11**

A solution of intermediate **B 15-B** (300 mg, 782 $\mu$mol) in toluene (6 mL) was added m-CPBA (186 mg, 916 $\mu$mol, 85% purity) at 0°C and stirred at 3 mins. Then the reaction mixture was added DIEA (202 mg, 1.56 mmol) and **intermediate 110-9** (394 mg, 822 $\mu$mol) at 15°C and the mixture was stirred at 15°C for 16 hours. The reaction mixture was quenched by addition $Na_2S_2O_3$ (15 mL) and $NaHCO_3$ (15 mL) at 15 °C, and then extracted with DCM (30 mL $\times$ 3). The combined organic layers were washed with aqueous NaCl (15 mL), dried over $Na_2SO_4$, filtered and concentrated

under reduced pressure to give a residue. The residue was purified by column chromatography ($SiO_2$, DCM/MeOH = 1:0 to 20:1) to give the **intermediate 110-11** (596 mg, 84.1% yield) as a yellow solid. LCMS ($ESI^+$): m/z 816.4 $[M+H]^+$.

**Step 8: synthesis of intermedite 110-12**

To a solution of intermediate **110-11** (596 mg, 731 μmol) in MeOH (12 mL) and $H_2O$ (4 mL) was added $K_2CO_3$ (1.52 g, 11.0 mmol) .The mixture was stirred at 40°C for 16 hrs. The reaction mixture was diluted with $H_2O$ (8 mL) and extracted with DCM 120 mL (40 mL × 3). The combined organic layers were dried over $Na_2SO_4$, filtered and concentrated under reduced pressure to give a residue. The residue was purified by column chromatography ($SiO_2$, DCM/MeOH=1/0 to 10/1) to give the **intermediate 110-12** (362 mg, 68.85% yield) as a yellow solid. LCMS ($ESI^+$): m/z 719.2 $[M+H]^+$.

**Step 9: synthesis of intermedite 110**

To a solution of **intermediate 110-12** (362 mg, 504 μmol) and 3-bromopiperidine-2,6-dione (1.45 g, 7.55 mmol) in DMF (8 mL) was added $NaHCO_3$ (635 mg, 7.55 mmol). The mixture was stirred at 70 °C for 16 hrs. The reaction mixture was partitioned between $H_2O$ (30 mL) and EA (30 mL) and extracted with EA two times. The organic phase was combined, and washed with sat. aq. NaCl (15 mL × 2), dried over $Na_2SO_4$, filtered and concentrated under reduced pressure to give a residue. The residue was purified by column chromatography ($SiO_2$, DCM: MeOH=1/0 to 10/1). The obtained crude product was triturated with MeCN/$H_2O$=1:1 (8 mL) and filtered to give a cake of **compound 110** (210 mg, 48.7% yield) as a yellow solid. LCMS ($ESI^+$): m/z 852.5 $[M+Na]^+$.

$^1H$ NMR (400 MHz, DMSO-$d_6$) δ 10.79 (s, 1H), 10.29 - 10.05 (m, 1H), 8.82 (s, 1H), 7.93 (br s, 1H), 7.70 (d, *J* = 8.0 Hz, 1H), 7.59 (br s, 2H), 6.92 (d, *J* = 8.8 Hz, 2H), 6.83 (t, *J* = 9.2 Hz, 1H), 6.49 (d, *J* = 14.8 Hz, 1H), 6.44 - 6.36 (m, 1H), 5.86 - 5.74 (m, 1H), 5.71 - 5.59 (m, 1H), 5.07 (s, 1H), 4.99 (d, *J* = 10.2 Hz, 1H), 4.92 - 4.67 (m, 2H), 4.64 - 4.49 (m, 1H), 4.31 - 4.18 (m, 1H), 3.10 (br s, 6H), 3.01 - 2.93 (m, 1H), 2.83 - 2.65 (m, 4H), 2.60 - 2.53 (m, 5H), 2.27 - 2.15 (m, 3H), 2.11 - 1.97 (m, 2H), 1.94 - 1.75 (m, 4H), 1.73 - 1.55 (m, 2H), 1.31 - 1.20 (m, 2H), 0.87 (t, *J* = 7.2 Hz, 3H).

**Step 10: synthesis of compound 150-A and compound 150-B**

The diastereoisomers of **compound 110** (50 mg, 60.2 μmol) was separated by prep-SFC (Column: DAICEL CHIRALPAK ID (250mm*30mm,10μm); Mobile phase: [MeOH-ACN]; $CO_2$%: 50%-50% at 50 mL/min. Compound **150-A** (18 mg, 36%) as peak **1** was obtained as a yellow solid.

**Compound 150-B** (20 mg, 40%) as peak **2** was obtained as a yellow solid.

**Compound 150-A:**

Chiral analysis (Column: chiralpak IE-3, 100*4.6mm I.D., 3 μm; Mobile Phase: A:methol (0.05%DEA), B: acetonitrile,Isocatic: A/B = 1/1 at 1.0 mL/ min; Temp: 35 °C; Wavelength: 254 nm, $R_t$ = 3.66 min).

LCMS ($ESI^+$): m/z 830.4 $[M+H]^+$.

$^1H$ NMR (400 MHz, DMSO-$d_6$) δ 10.80 (s, 1H), 10.25 - 10.06 (m, 1H), 8.82 (s, 1H), 8.02 - 7.85 (m, 1H), 7.70 (d, *J* = 8.4 Hz, 1H), 7.63 - 7.42 (m, 2H), 7.01 - 6.88 (m, 2H), 6.86 - 6.74 (m, 1H), 6.56 - 6.46 (m, 1H), 6.41 (d, *J* = 8.8 Hz, 1H), 5.87 - 5.74 (m, 1H), 5.72 - 5.57 (m, 1H), 5.08 (s, 1H), 4.99 (d, *J* = 10.4 Hz, 1H), 4.91 - 4.69 (m, 2H), 4.64 - 4.48 (m, 1H), 4.33 - 4.17 (m, 1H), 3.10 (br s, 6H), 3.00 - 2.93 (m, 1H), 2.81 - 2.66 (m, 4H), 2.62 - 2.54 (m, 5H), 2.26 - 2.16 (m, 3H), 2.10 - 1.98 (m, 2H), 1.94 - 1.76 (m, 4H), 1.73 - 1.58 (m, 2H), 1.30 - 1.18 (m, 2H), 0.87 (t, *J* = *7.2* Hz, 3H)

**Compound 150-B:**

Chiral analysis (Column: chiralpak IE-3, 100*4.6mm I.D., 3 μm; Mobile Phase: A:methol (0.05%DEA), B: acetonitrile,Isocatic: A/B = 1/1 at 1.0 mL/ min; Temp: 35 °C; Wavelength: 254 nm, $R_t$ = 5.92 min).

LCMS ($ESI^+$): m/z 830.4 $[M+H]^+$.

$^1H$ NMR (400 MHz, DMSO-$d_6$) δ 10.80 (s, 1H), 10.25 - 10.06 (m, 1H), 8.86 - 8.77 (m, 1H), 8.02 - 7.86 (m, 1H), 7.73 - 7.66 (m, 1H), 7.64 - 7.48 (m, 2H), 6.92 (d, *J* = 8.4 Hz, 2H), 6.83 (t, *J* = 9.2 Hz, 1H), 6.55 - 6.44 (m, 1H), 6.41 (d, *J* = 9.2 Hz, 1H), 5.84 - 5.75 (m, 1H), 5.72 - 5.60 (m, 1H), 5.08 (s, 1H), 5.02 - 4.95 (m, 1H), 4.91 - 4.71 (m, 2H), 4.63 - 4.49 (m, 1H), 4.31 - 4.19 (m, 1H), 3.10 (br s, 6H), 3.00 - 2.94 (m, 1H), 2.84 - 2.69 (m, 4H), 2.56 (d, *J* = 11.6 Hz, 5H), 2.27 - 2.19 (m, 3H), 2.10 - 1.99 (m, 2H), 1.90 - 1.76 (m, 4H), 1.73 - 1.60 (m, 2H), 1.28 - 1.21 (m, 2H), 0.87 (t, *J* = 7.2 Hz, 3H)

The **compound 112, compound 152-A** and **compound 152-B** were synthesized according to the procedures described for **compound 110** using the appropriated intermediates.

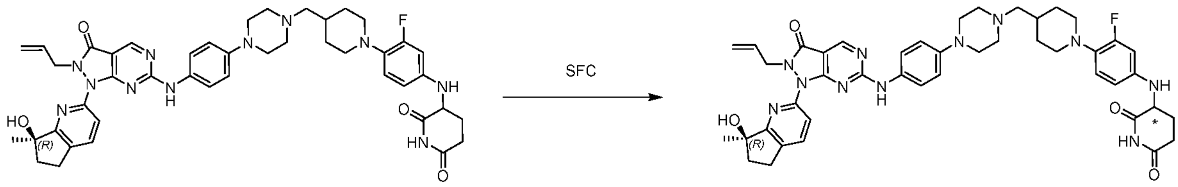


compound 112

152-A (diastereoisomer A)
152-B (diastereoisomer B)

The compound **112** (40 mg, 49.02 μmol) was separated by prep-SFC (Column: DAICEL CHIRALPAK ID (250mm*30mm,10μM); Mobile phase: [MeOH-ACN]; $CO_2$%: 50%-50%. **Compound 152-A** (15 mg, 37.5%) as peak **1** was obtained as a yellow solid. **Compound 152-B** (15 mg, 37.5%) as peak **2** was obtained as a yellow solid.

Compound **152-A:**

Chiral analysis (Column: chiralpak IE-3, 100*4.6mm I.D., 3 μm; Mobile Phase: A:methanol (0.05%DEA), B: acetonitrile,Isocatic: A/B = 1/1 at 1.0 mL/ min; Temp: 35 °C; Wavelength: 254 nm, $R_t$ = 3.43 min).

Compound **152-B:**

Chiral analysis (Column: chiralpak IE-3, 100*4.6mm I.D., 3 μm; Mobile Phase: A:methanol (0.05%DEA), B: acetonitrile,Isocatic: A/B = 1/1 at 1.0 mL/ min; Temp: 35 °C; Wavelength: 254 nm, $R_t$ = 5.41 min).

The following compounds were synthesized according to the procedures described for **compound 110** using the appropriated intermediates.

| Compound No. | Intermediate No. | MS(ESI+): m/z [M+H]+ | $^1$H NMR (400 MHz, DMSO-$d_6$) $\delta$ |
|---|---|---|---|
| 112 | B 26-B | 816.5 | 10.77 (s, 1H), 10.13 (br s, 1H), 8.82 (s, 1H), 7.92 (br s, 1H), 7.69 (d, *J* = 8.0 Hz, 1H), 7.58 (br s, 2H), 6.92 (d, *J* = 8.8 Hz, 2H), 6.83 (t, *J* = 9.6 Hz, 1H), 6.55 - 6.45 (m, 1H), 6.44 - 6.35 (m, 1H), 5.78 (d, *J* = 7.6 Hz, 1H), 5.73 - 5.59 (m, 1H), 5.18 (s, 1H), 4.99 (d, *J* = 10.4 Hz, 1H), 4.86 (d, *J* = 17.2 Hz, 1H), 4.73 (br s, 1H), 4.62 (br s, 1H), 4.33 - 4.20 (m, 1H), 3.10 (br s, 5H), 3.02 - 2.90 (m, 1H), 2.87 - 2.67 (m, 2H), 2.61 - 2.54 (m, 3H), 2.49 (br s, 5H), 2.23 (d, *J*=7.2 Hz, 2H), 2.16 - 2.04 (m, 3H), 1.94 - 1.74 (m, 3H), 1.62 (br s, 1H), 1.45 (s, 3H), 1.36 - 1.19 (m, 2H). |
| 113 | B 26-A 73-8 | 816.5 | 10.78 (s, 1 H), 10.14 (s, 1 H), 8.82 (s, 1 H), 7.93 ( d, *J* = 6.00 Hz, 1 H), 7.69 (d, *J* = 8.40 Hz, 1 H), 7.64 - 7.46 (m, 2 H), 6.92 (s, 2 H), 6.83 (t, *J* = 9.60 Hz, 1 H), 6.54 - 6.46 (m, 1 H), 6.41 (d, *J* = 8.40 Hz, 1 H), 5.79 (d, *J* = 7.60 Hz, 1 H), 5.73-5.57 (m, 1 H), 5.19 (s, 1 H), 4.99 (d, *J* = 10.00 Hz, 1 H), 4.86 (d, *J* = 17.20 Hz, 1 H), 4.72 (s, 1 H), 4.62 (s, 1 H), 4.32-4.20 (m, 1 H), 3.19- 3.12 (m, 2 H), 3.10 (s, 4 H), 3.01-2.91 (m, 1 H), 2.84-2.66 (m, 2 H), 2.57 (d, *J* = 13.20 Hz, 6 H), 2.48-2.41 (m, 1 H), 2.24 (s, 2 H), 2.17-2.04 (m, 3 H), 1.88-1.73 (m, 3 H), 1.62 (s, 1 H), 1.45 (s, 3 H), 1.28 (s, 2 H). |
| 152-A | | 838.4 [M+Na]+ | $^1$H NMR (400MHz, DMSO- $d_6$) $\delta$ 10.79 (s, 1H), 10.14 (br s, 1H), 8.82 (s, 1H), 7.93 (br s, 1H), 7.69 (d, *J*=8.0 Hz, 1H), 7.59 (br s, 2H), 6.92 (d, *J*=8.0 Hz, 2H), 6.88 - 6.77 (m, 1H), 6.50 (d, *J*=12.0 Hz, 1H), 6.41 (d, *J*=8.0 Hz, 1H), 5.79 (d, *J*=8.0 Hz, 1H), 5.74 - 5.60 (m, 1H), 5.19 (s, 1H), 4.99 (d, *J*=8.0 Hz, 1H), 4.86 (d, *J*=16.0 Hz, 1H), 4.79 - 4.50 (m, 2H), 4.31 - 4.19 (m, 1H), 3.10 (br s, 6H), 3.03 - 2.88 (m, 1H), 2.86 - 2.66 (m, 3H), 2.57 (d, *J*=16.0 Hz, 6H), 2.23 (d, *J*=8.0 Hz, 2H), 2.17 2.02 (m, 3H), 1.92 - 1.72 (m, 3H), 1.63 (br s, 1H), 1.45 (s, 3H), 1.26 (d, *J*=12.0 Hz, 2H) |

(continued)

| Compound No. | Intermediate No. | MS(ESI$^+$): m/z [M+H]$^+$ | $^1$H NMR (400 MHz, DMSO-$d_6$) $\delta$ |
|---|---|---|---|
| 152-B | | 816.5 | $^1$H NMR (400MHz, DMSO- $d_6$) $\delta$ 10.79 (s, 1H), 10.16 (br s, 1H), 8.82 (s, 1H), 7.92 (br s, 1H), 7.69 (d, $J$=8.0 Hz, 1H), 7.59 (br s, 2H), 6.92 (d, $J$=8.0 Hz, 2H), 6.87 - 6.77 (m, 1H), 6.55 - 6.37 (m, 2H), 5.79 (d, $J$=8.0 Hz, 1H), 5.73 - 5.57 (m, 1H), 5.19 (s, 1H), 4.99 (d, $J$=8.0 Hz, 1H), 4.85 (d, $J$=20.0 Hz, 1H), 4.73 (br s, 1H), 4.62 (br s, 1H), 4.25 (br s, 1H), 3.10 (br s, 6H), 3.02 - 2.90 (m, 2H), 2.85 - 2.65 (m, 2H), 2.57 (d, $J$=12.0 Hz, 6H), 2.23 (d, $J$=8.0 Hz, 2H), 2.16 - 2.05 (m, 3H), 1.89 - 1.74 (m, 3H), 1.60 (br s, 1H), 1.45 (s, 3H), 1.26 (d, $J$=16.0 Hz, 2H) |
| 119 | B 15-B | 830.5 | $^1$H NMR (400 MHz, DMSO-$d_6$) $\delta$ 10.79 (s, 1H), 10.12 (br s, 1H), 8.82 (s, 1H), 7.92 (br s, 1H), 7.69 (d, $J$ = 8.0 Hz, 1H), 7.57 (br s, 2H), 6.92 (d, $J$ = 8.4 Hz, 2H), 6.84 (t, $J$ = 9.2 Hz, 1H), 6.51 (d, $J$ = 16.4 Hz, 1H), 6.43 (d, $J$ = 8.8 Hz, 1H), 5.81 (d, $J$ = 5.6 Hz, 1H), 5.73 - 5.57 (m, 1H), 5.06 (s, 1H), 5.00 (d, $J$ = 10.2 Hz, 1H), 4.85 (d, $J$ = 17.2 Hz, 1H), 4.75 (br s, 1H), 4.58 (br s, 1H), 4.36 - 4.18 (m, 1H), 3.68 - 3.59 (m, 2H), 3.02 - 2.93 (m, 1H), 2.93 - 2.80 (m, 4H), 2.80 - 2.72 (m, 1H), 2.72 - 2.65 (m, 1H), 2.60 (d, $J$ = 10.2 Hz, 2H), 2.49 - 2.44 (m, 5H), 2.26 - 2.15 (m, 3H), 2.11 - 1.96 (m, 2H), 1.92 - 1.76 (m, 4H), 1.75 - 1.61 (m, 2H), 1.33 - 1.24 (m, 2H), 0.87 (t, $J$ = 7.2 Hz, 3H). |
| 120 | B 26 | 838.5 [M+Na]$^+$ | $^1$H NMR (400MHz, DMSO-$d_6$) $\delta$ 10.78 (s, 1 H), 10.12 (s, 1 H), 8.82 (s, 1 H), 7.92 (s, 1 H), 7.69 (d, $J$ = 7.60 Hz, 1 H), 7.63 - 7.43 (m, 2 H), 6.92 (d, $J$ = 8.40 Hz, 2H), 6.83 (t, $J$ = 9.20 Hz, 1 H), 6.51 (d, $J$ = 14.80 Hz, 1 H), 6.42 (d, $J$ = 8.40 Hz, 1 H), 5.81 (d, $J$ = 8.00 Hz, 1 H), 5.74- 5.56 (m, 1 H), 5.18 (s, 1H), 4.99 (d, $J$ = 9.60 Hz, 1 H), 4.86 (d, $J$ = 16.80 Hz, 1 H), 4.72 (s, 1 H), 4.62 (s, 1 H), 4.30 - 4.20 (m, 1 H), 3.63 (d, $J$ = 11.60 Hz, 2H), 3.06 - 2.92 (m, 1 H), 2.91 - 2.72 (m, 5 H), 2.61 (s, 4 H), 2.47 - 2.42 (m, 4 H), 2.21 (d, $J$ = 7.20 Hz, 2 H), 2.17 - 2.4 (m, 3 H), 1.92- 1.77 (m, 3 H), 1.67 (s, 1 H), 1.45 (s, 3 H), 1.26 -1.20 (m, 2 H). |
| 121 | B 26 | 838.5 [M+Na]$^+$ | $^1$H NMR (400MHz, DMSO-$d_6$) 810.78 (s, 1H), 10.12 (br s, 1H), 8.82 (s, 1H), 7.92 (br s, 1H), 7.69 (d, $J$ = 8.0 Hz, 1H), 7.57 (br s, 2H),6.92 (d, $J$ = 8.8 Hz, 2H), 6.83 (t, $J$ = 9.6 Hz, 1H), 6.55 - 6.48 (m, 1H), 6.44 - 6.39 (m, 1H), 5.81 (d, $J$ = 8.0 Hz, 1H),5.72 - 5.62 (m, 1H), 5.18 (s, 1H), 4.99 (d, $J$ = 10.0 Hz, 1H), 4.86 (d, $J$ = 16.0 Hz, 1H), 4.73 (br s, 1H),4.62 (br s, 1H), 4.30 - 4.22 (m, 1H), 3.63 (d, $J$ = 11.2 Hz, 2H), 3.03 - 2.92 (m, 1H), 2.91 - 2.78 (m,5H), 2.65 - 2.53 (m, 4H), 2.47 - 2.35 (m, 4H), 2.21 (d, $J$ = 6.8 Hz, 2H), 2.13 (t, $J$ = 7.2 Hz, 3H), 1.87-1.76 (m, 3H), 1.66 (br s, 1H), 1.45 (s, 3H), 1.26 - 1.21 (m, 2H). |
| 123 | B 6 | 802.5 | $^1$H NMR (400MHz, DMSO-$d_6$) $\delta$ 10.77 (s, 1H), 10.16 (br s, 1H), 8.83 (s, 1H), 8.16 (s, 0.542H),8.05 (br s, 1H), 7.75 (d, $J$ = 7.6 Hz, 1H), 7.64 - 7.49 (m, 3H), 6.92 (d, $J$ = 8.8 Hz, 2H), 6.52 - 6.33(m, 3H), 5.75 - 5.50 (m, 2H), 5.35 (s, 1H), 4.99 (d, $J$ = 10.4 Hz, 1H), 4.86 - 4.77 (m, 1H), 4.68 (d, $J$ = 4.8 Hz, 2H), 4.24 - 4.14 (m, 1H), 3.79 (s, 4H), 3.55 (br s, 2H), 3.53 - 3.49 (m, 2H),2.79 - 2.63 (m, 4H), 2.58 (d, $J$ = 4.4 Hz, 1H), 2.54 (br s, 1H), 2.21 (br s, 1H), 2.16 - 2.00(m, 1H), 1.88 - 1.78 (m, 1H), 1.73 (d, $J$= 11.2 Hz, 2H), 1.46 (s, 6H), 1.27 (d, $J$ = 12.4 Hz, 2H). |

(continued)

| Compound No. | Intermediate No. | MS(ESI$^+$): m/z $[M+H]^+$ | $^1$H NMR (400 MHz, DMSO-$d_6$) $\delta$ |
|---|---|---|---|
| 128 | B 15-B | 866.5 $[M+Na]^+$ | 10.78 (s, 1H), 10.42 - 9.92 (m, 1H), 8.84 (s, 1H), 7.91 (d, *J* = 7.6 Hz, 1H), 7.80 - 7.55 (m, 2H), 7.43 (d, *J* = 8.8Hz, 1H), 7.00 (d, *J* = 8.8 Hz, 1H), 6.83 (t, *J* = 9.6 Hz, 1H), 6.58 - 6.29 (m, 2H), 5.89 - 5.52 (m, 2H), 5.07 (s, 1H), 4.99 (d, *J* = 10.4 Hz, 1H), 4.84 (d, *J* = 17.2 Hz, 1H), 4.76 (d, *J* = 14.0 Hz, 1H), 4.56 (d, *J* = 10.0 Hz, 1H), 4.37 - 4.14 (m, 1H), 3.12 (d, *J* = 10.0 Hz, 2H), 2.96 (dd, *J* = 7.6, 14.8 Hz, 1H), 2.88 - 2.70 (m, 6H), 2.57 (br s, 7H), 2.24 (br s, 6H), 2.13 - 2.00 (m, 2H), 1.93 - 1.75 (m, 4H), 1.72 - 1.52 (m, 2H), 1.28 - 1.17 (m, 2H), 0.86 (t, *J* = 7.2 Hz, 3H). |
| 130 | B 26-B | 852.5 $[M+Na]^+$ | $^1$H NMR (400 MHz, DMSO-$d_6$) $\delta$ 10.78 (s, 1H), 10.30 - 10.00 (m, 1H), 8.96 - 8.73 (m, 1H), 7.91 (d, *J* = 8.0 Hz, 1H), 7.77 - 7.57 (m, 2H), 7.43 (d, *J* = 8.8 Hz, 1H), 7.00 (d, *J* = 8.4 Hz, 1H), 6.83 (t, *J* = 9.2 Hz, 1H), 6.56 - 6.34 (m, 2H), 5.83 - 5.74 (m, 1H), 5.73 - 5.61 (m, 1H), 5.19 (s, 1H), 4.99 (d, *J* = 10.4 Hz, 1H), 4.91 - 4.82 (m, 1H), 4.80 - 4.67 (m, 1H), 4.65 - 4.52 (m, 1H), 4.33 - 4.16 (m, 1H), 3.12 (d, *J* = 10.4 Hz, 2H), 3.04 - 2.93 (m, 1H), 2.89 - 2.77 (m, 5H), 2.75 - 2.64 (m, 2H), 2.58 (br s, 6H), 2.24 (s, 5H), 2.17 - 2.04 (m, 3H), 1.90 - 1.75 (m, 3H), 1.69 - 1.54 (m, 1H), 1.45 (s, 3H), 1.28 (br s, 2H). |
| 131 | B 26-A | 852.5 $[M+Na]^+$ | $^1$H NMR (400MHz, DMSO-$d_6$) $\delta$ 10.79 (s, 1H), 10.16 (s, 1H), 8.85 (s, 1H), 7.91 (d, *J* = 8.0 Hz, 1H),7.76 - 7.58 (m, 2H), 7.44 (d, *J* = 8.8 Hz, 1H), 7.01 (d, *J* = 8.8 Hz, 1H), 6.84 (t, *J* = 9.2 Hz, 1H), 6.55 -6.37 (m, 2H), 5.79 (d, *J* = 7.2 Hz, 1H), 5.74 - 5.61 (m, 1H), 5.20 (s, 1H), 5.00 (d, *J* = 10.4 Hz, 1H),4.86 (d, *J* = 17.2 Hz, 1H), 4.72 (br s, 1H), 4.60 (d, *J* = 10.8 Hz, 1H), 4.26 (t, *J* = 11.6 Hz, 1H), 3.13 (d, *J* = 10.4 Hz, 2H), 3.03 - 2.92 (m, 1H), 2.83 (br s, 5H), 2.76 - 2.69 (m, 2H), 2.58 (br s, 6H), 2.25 (s,5H), 2.16 - 2.05 (m, 3H), 1.90 - 1.75 (m, 3H), 1.62 (br s, 1H), 1.45 (s, 3H), 1.24 (br s, 2H). |
| 132 | B 26-B | 830.5 | $^1$H NMR (400MHz, DMSO-$d_6$) $\delta$ 10.79 (s, 1H), 10.29 - 9.96 (m, 1H), 8.84 (s, 1H), 7.90 (d, *J* = 8.0 Hz, 1H), 7.83 - 7.54 (m, 2H), 7.42 (d, *J* = 8.4 Hz, 1H), 6.97 (d, *J* = 8.8 Hz, 1H), 6.83 (t, *J* = 10.0 Hz, 1H), 6.61 - 6.36 (m, 2H), 5.89 - 5.61 (m, 2H), 5.27 - 5.14 (m, 1H), 4.99 (d, *J* = 9.2 Hz, 1H), 4.85 (d, *J* = 17.2 Hz, 1H), 4.73 (d, *J* = 12.4 Hz, 1H), 4.65 - 4.55 (m, 1H), 4.26 (br s, 1H), 3.08 - 2.92 (m, 4H), 2.91 - 2.78 (m, 4H), 2.78 - 2.68 (m, 2H), 2.64 - 2.53 (m, 5H), 2.24 (s, 5H), 2.17 - 2.05 (m, 3H), 1.91 - 1.74 (m, 3H), 1.65 (br s, 1H), 1.45 (s, 3H), 1.35 - 1.17 (m, 3H). |
| 133 | B 26-A | 830.5 | $^1$H NMR (400MHz, DMSO-$d_6$) $\delta$10.79 (br s, 1H), 10.30 - 10.06 (m, 1H), 8.84 (s, 1H), 7.90 (d, *J* = 7.2 Hz, 1H), 7.71 (d, *J* = 8.0 Hz, 2H), 7.41 (d, *J* = 8.4 Hz, 1H), 6.97 (d, *J* = 8.4 Hz, 1H), 6.83 (t, *J* = 9.2 Hz, 1H), 6.51 (d, *J* = 14.8 Hz, 1H), 6.42 (d, *J* = 8.4 Hz, 1H), 5.85 - 5.62 (m, 2H), 5.32 (s,1H), 5.20 (s, 1H), 4.99 (d, *J* = 10.4 Hz, 1H), 4.85 (d, *J* = 18.0 Hz, 1H), 4.63 (s, 1H), 4.26 (s, 1H),3.05 - 2.94 (m, 4H), 2.86 (br s, 4H), 2.82 - 2.68 (m, 2H), 2.63 - 2.56 (m, 5H), 2.23 (s, 5H), 2.17- 2.08 (m, 3H), 2.03 - 1.94 (m, 3H), 1.65 (br s, 1H), 1.45 (s, 3H), 1.36 - 1.28 (m, 3H). |

(continued)

| Compound No. | Intermediate No. | MS(ESI+): m/z [M+H]+ | $^1$H NMR (400 MHz, DMSO-$d_6$) $\delta$ |
|---|---|---|---|
| 134 | B 15-B | 844.5 [M+Na]+ | $^1$H NMR (400 MHz, DMSO-$d_6$) $\delta$ 10.79 (s, 1H), 10.30 - 9.97 (m, 1H), 8.84 (s, 1H), 7.90 (d, *J* = 8.0 Hz, 1H), 7.78 - 7.57 (m, 2H), 7.48 - 7.32 (m, 1H), 6.97 (d, *J* = 8.4 Hz, 1H), 6.83 (t, *J* = 9.2 Hz, 1H), 6.59 - 6.37 (m, 2H), 5.87 - 5.77 (m, 1H), 5.75 - 5.59 (m, 1H), 5.07 (s, 1H), 4.99 (d, *J* = 10.0 Hz, 1H), 4.90 - 4.68 (m, 2H), 4.63 - 4.47 (m, 1H), 4.34 - 4.16 (m, 1H), 3.08 - 2.93 (m, 4H), 2.92 - 2.68 (m, 7H), 2.64 - 2.54 (m, 4H), 2.34 - 2.15 (m, 7H), 2.12 - 1.98 (m, 2H), 1.94 - 1.78 (m, 4H), 1.73 - 1.55 (m, 2H), 1.37-1.19 (m, 2H), 0.87 (t, *J* = 7.2 Hz, 3H). |
| 136 | B 15-B | 835.5 [M+Na]+ | $^1$H NMR (400MHz, DMSO-$d_6$) $\delta$ 10.77 (s, 1H), 10.14 (br s, 1H), 8.82 (s, 1H), 7.92 (br s, 1H), 7.74 -7.65 (m, 2H), 7.58 (br s, 2H), 7.03 (d, *J* = 8.0 Hz, 1H), 6.92 (d, *J* = 8.8 Hz, 2H), 6.68 (d, *J* = 8.8 Hz, 1H),5.72 - 5.61 (m, 1H), 5.37 (d, *J* = 6.4 Hz, 1H), 5.06 (s, 1H), 4.99 (d, *J* = 10.0 Hz, 1H), 4.85 (d, *J* = 17.6Hz, 1H), 4.75 (br s, 1H), 4.58 (br s, 1H), 4.19 (br s, 1H), 4.01 (d, *J* = 11.2 Hz, 2H), 3.10 (br s, 4H), 3.01- 2.90 (m, 1H), 2.83 - 2.71 (m, 2H), 2.64 - 2.54 (m, 7H), 2.20 (br s, 3H), 2.10 (d, *J* = 8.8 Hz, 1H), 2.02(d, *J* = 6.4 Hz, 1H), 1.95 - 1.82 (m, 2H), 1.81 - 1.66 (m, 4H), 1.14 (br s, 2H), 0.90 - 0.85 (m, 3H). |
| 156 | B 15-B | 828.4 | 10.77 (s, 1H), 10.22 - 10.01 (m, 1H), 8.82 (s, 1H), 7.94 (m, 1H), 7.70 (m, 1H), 7.57 (br s, 2H), 6.92 (m, 2H), 6.53 - 6.46 (m,1H), 6.43 - 6.34 (m, 2H), 5.75 - 5.62 (m, 1H), 5.57 (m, 1H), 5.06 (s, 1H), 5.04 - 4.97 (m, 1H), 4.86 (m, 1H), 4.75 (br s, 1H), 4.57 (m, 1H), 4.24 - 4.15 (m, 1H), 3.79 (br s, 4H), 3.51 (br s, 2H), 3.33 - 3.32 (m, 2H), 3.04 - 2.92 (m, 1H), 2.83 - 2.77 (m, 1H), 2.71 - 2.66(m, 3H), 2.58 (m, 1H), 2.49 - 2.43 (m, 5H), 2.25 - 2.16 (m, 1H), 2.14 - 1.97 (m, 3H),1.90 - 1.80 (m, 2H), 1.77 - 1.66 (m, 2H), 0.88 (m, 3H). |
| 157 | B 15-B | 816.3 | 10.78 (s, 1H), 10.13 (br s, 1H), 8.83 (s, 1H), 8.00 - 7.89 (m, 1H), 7.70 (d, *J* = 8.0 Hz, 1H), 7.59 (br s, 2H), 6.98 - 6.90 (m, 2H), 6.88 - 6.80 (m, 1H), 6.56 - 6.48 (m, 1H), 6.45 - 6.38 (m, 1H), 5.80 (d, *J* = 7.6 Hz, 1H), 5.74 - 5.61 (m, 1H), 5.05 (s, 1H), 5.00 (d, *J* = 9.6 Hz, 1H), 4.91 - 4.82 (m, 1H), 4.75 (br s, 1H), 4.63 - 4.51 (m, 1H), 4.30 - 4.21 (m, 1H), 3.22 - 3.16 (m, 2H), 3.11 (br s, 4H), 3.03 - 2.94 (m, 1H), 2.83 - 2.72 (m, 1H), 2.68 (br s, 4H), 2.57 (d, *J* = 12.8 Hz, 3H), 2.25 - 2.17 (m, 1H), 2.12 - 2.00 (m, 2H), 1.94 - 1.79 (m, 5H), 1.77 - 1.67 (m, 1H), 1.66 - 1.50 (m, 3H), 0.91 - 0.85 (m, 3H). |
| 162 | B 15-B | 844.4 | 10.78 (s, 1H), 10.30 - 10.00 (m, 1H), 8.82 (s, 1H), 7.92 (br s, 1H), 7.69 (d, *J* = 8.4 Hz, 1H), 7.62 - 7.44 (m, 2H), 6.92 (d, *J* = 8.8 Hz, 2H), 6.87 - 6.76 (m, 1H), 6.57 - 6.45 (m, 1H), 6.43 - 6.36 (m, 1H), 5.78 (d, *J* = 7.6 Hz, 1H), 5.73 - 5.56 (m, 1H), 5.06 (s, 1H), 4.99 (d, *J* = 11.2 Hz, 1H), 4.85 (d, *J* = 16.4 Hz, 1H), 4.74 (br s, 1H), 4.57 (d, *J* = 12.0 Hz, 1H), 4.33 - 4.18 (m, 1H), 3.38 (d, *J* = 8.4 Hz, 2H), 3.12 (d, *J* = 9.6 Hz, 2H), 3.02 - 2.88 (m, 2H), 2.84 - 2.75 (m, 2H), 2.74 - 2.68 (m, 1H), 2.57 (d, *J* = 9.2 Hz, 3H), 2.30 - 2.16 (m, 3H), 2.12 - 1.96 (m, 4H), 1.95 - 1.78 (m, 4H), 1.70 (dd, *J* = 6.8, 14.0 Hz, 2H), 1.58 (br s, 1H), 1.32 - 1.25 (m, 1H), 1.22 - 116 (m, 1H), 1.07 (d, *J* = 5.6 Hz, 3H), 0.87 (t, *J* = 7.6 Hz, 3H). |

(continued)

| Compound No. | Intermediate No. | MS(ESI$^+$): m/z [M+H]$^+$ | $^1$H NMR (400 MHz, DMSO-$d_6$) $\delta$ |
|---|---|---|---|
| 163 | B 15-B | 844.4 | 10.77 (s, 1H), 10.29 - 9.95 (m, 1H), 8.82 (s, 1H), 7.92 (br s, 1H), 7.70 (d, *J* = 8.4 Hz, 1H), 7.59 (br s, 1H), 6.96 - 6.80 (m, 3H), 6.62 - 6.46 (m, 1H), 6.45 - 6.39 (m, 1H), 5.81 - 5.75 (m, 1H), 5.73 - 5.61 (m, 1H), 5.07 - 4.97 (m, 2H), 4.90 - 4.82 (m, 1H), 4.75 (br s, 1H), 4.59 (br s, 1H), 4.29 - 4.20 (m, 1H), 3.39 (br s, 1H), 3.30 (s, 4H), 3.17 - 3.07 (m, 2H), 3.04 - 2.89 (m, 2H), 2.86 - 2.71 (m, 3H), 2.59 (br s, 4H), 2.32 - 2.15 (m, 2H), 2.13 - 1.97 (m, 3H), 1.95 - 1.80 (m, 3H), 1.80 - 1.64 (m, 2H), 1.58 (br s, 1H), 1.36 - 1.25 (m, 1H), 1.21 (br s, 1H), 1.14 - 1.01 (m, 3H), 0.95 - 0.80 (m, 3H). |
| 164 | | 867.2 [M+Na]$^+$ | 10.78 (s, 1H), 10.23 - 10.04 (m, 1H), 8.82 (s, 1H), 7.98 - 7.90 (m, 1H), 7.72 - 7.66 (m, 1H), 7.58 (s, 2H), 6.97 - 6.87 (m, 2H), 6.58 - 6.41 (m, 3H), 5.70 - 5.64 (m, 1H), 5.36 - 5.30 (m, 1H), 5.08 (s, 1H), 5.03 - 4.95 (m, 1H), 4.90 - 4.68 (m, 2H), 4.63 - 4.51 (m, 1H), 4.23 (s, 1H), 4.08 - 3.99 (m, 4H), 3.86 (s, 2H), 3.67 - 3.59 (m, 2H), 3.14 - 3.02 (m, 4H), 2.85 - 2.72 (m, 1H), 2.27 - 2.15 (m, 1H), 2.08 (s, 2H), 2.02 - 1.97 (m, 4H), 1.92 - 1.81 (m, 4H), 1.75 - 1.68 (m, 1H), 1.51 - 1.39 (m, 2H), 0.87 - 0.85 (m, 3H). |
| 165 | B 15-B | 856.3 | 10.77 (s, 1H), 10.12 (s, 1H), 8.82 (s, 1H), 7.92 (s, 1H), 7.69 (d, *J* = 8.0 Hz, 1H), 7.57 (s, 1H), 6.92 (d, *J* = 8.6 Hz, 2H), 6.82 (t, *J* = 9.2 Hz, 1H), 6.49 (d, *J* = 15.6 Hz, 1H), 6.40 (d, *J* = 7.6 Hz, 1H), 5.78 (d, *J* = 7.6 Hz, 1H), 5.73 - 5.56 (m, 1H), 5.13 - 4.95 (m, 2H), 4.90 - 4.69 (m, 2H), 4.58 (s, 1H), 4.25 (s, 1H), 3.10 (s, 4H), 3.02 - 2.91 (m, 2H), 2.78 (s, 4H), 2.71 (d, *J* = 6.8 Hz, 4H), 2.39 (s, 4H), 2.33 (s, 1H), 2.24 - 2.17 (m, 1H), 2.00 (d, *J* = 8.4 Hz, 2H), 1.92 - 1.82 (m, 2H), 1.68 (s, 2H), 1.59 (s, 4H), 1.26 - 1.21 (m, 2H), 0.87 (t, *J* = 7.2 Hz, 3H) |
| 166 | B 15-B | 848.3 | 10.79 (s, 1H), 10.15 (s, 1H), 8.82 (s, 1H), 7.93 (s, 1H), 7.69 (d, *J* = 8.0 Hz, 1H), 7.58 (s, 2H), 6.96 - 6.83 (m, 3H), 6.55 - 6.46 (m, 1H), 6.43 (s, 1H), 5.82 (s, 1H), 5.67 (s, 1H), 5.07 (s, 1H), 5.03 - 4.94 (m, 1H), 4.90 - 4.79 (m, 1H), 4.75 (s, 1H), 4.58 (s, 1H), 4.26 (s, 1H), 3.48 (s, 2H), 3.31 - 3.27 (m, 2H), 3.10 (s, 4H), 2.95 (s, 2H), 2.81 (s, 2H), 2.66 (s, 4H), 2.60 - 2.54 (m, 2H), 2.19 (s, 1H), 2.06 (s, 1H), 2.03 - 1.81 (m, 6H), 1.81 - 1.65 (m, 2H), 0.87 (s, 3H). |
| 167 | B 15-B | 864.3 | 10.77 (s, 1H), 10.46 - 10.21 (m, 1H), 8.89 (s, 1H), 8.06 (br s, 1H), 7.93 (d, *J* = 8.0 Hz, 1H), 7.69 (d, *J* = 8.0 Hz, 1H), 7.56 - 7.48 (m, 1H), 7.14 (d, *J* = 8.8 Hz, 1H), 6.83 (t, *J* = 9.6 Hz, 1H), 6.49 (d, *J* = 2.0, 14.8 Hz, 1H), 6.41 (d, *J* = 8.8 Hz, 1H), 5.78 (d, *J* = 7.6 Hz, 1H), 5.73 - 5.60 (m, 1H), 5.07 (s, 1H), 4.99 (d, *J* = 9.6 Hz, 1H), 4.89 - 4.72 (m, 2H), 4.57 (d, *J* = 6.4, 16.0 Hz, 1H), 4.31 - 4.19 (m, 1H), 3.11 (d, *J* = 11.2 Hz, 2H), 3.02 - 2.87 (m, 5H), 2.83 - 2.71 (m, 2H), 2.62 - 2.53 (m, 5H), 2.29 - 2.14 (m, 4H), 2.11 - 1.99 (m, 2H), 1.92 - 1.84 (m, 2H), 1.84 - 1.74 (m, 3H), 1.70 (d, *J* = 7.2, 13.6 Hz, 1H), 1.61 (s, 1H), 1.30 - 1.26 (m, 2H), 0.86 (t, *J* = 7.6 Hz, 3H). |

(continued)

| Compound No. | Intermediate No. | MS(ESI$^+$): m/z [M+H]$^+$ | $^1$H NMR (400 MHz, DMSO-$d_6$) $\delta$ |
|---|---|---|---|
| 168 | B 15-B | 850.3 [M+Na]$^+$ | 10.76 (s, 1H), 10.13 (s, 1H), 8.82 (s, 1H), 7.92 (br s, 1H), 7.69 (d, *J* = 8.0 Hz, 1H), 7.58 (br s, 2H), 6.92 (d, *J* = 8.4 Hz, 2H), 6.48 (d, *J* = 14.8 Hz, 1H), 6.43 - 6.27 (m, 2H), 5.76 - 5.62 (m, 1H), 5.54 (d, *J* = 7.2 Hz, 1H), 5.05 (s, 1H), 4.99 (d, *J* = 10.4 Hz, 1H), 4.85 (d, *J* = 17.2 Hz, 1H), 4.73 (br s, 1H), 4.65 - 4.49 (m, 1H), 4.19 (br s, 1H), 3.75 (s, 2H), 3.63 (s, 2H), 3.09 (br s, 4H), 3.00 - 2.92 (m, 1H), 2.83 - 2.70 (m, 2H), 2.60 - 2.55 (m, 2H), 2.39 (br s, 4H), 2.31 - 2.26 (m, 2H), 2.24 - 2.16 (m, 1H), 2.11 -1.96 (m, 4H), 1.94 - 1.78 (m, 2H), 1.76 - 1.61 (m, 1H), 0.87 (t, *J* = 7.2 Hz, 3H) |
| 169 | B 15-B | 858.3 | 10.78 (s, 1H), 10.21 - 10.01 (m, 1H), 8.82 (s, 1H), 7.99 - 7.86 (m, 1H), 7.69 (d, *J* = 8.4 Hz, 1H), 7.62 - 7.42 (m, 2H), 6.91 (d, *J* = 8.8 Hz, 2H), 6.83 (t, *J* = 9.2 Hz, 1H), 6.54 - 6.46 (m, 1H), 6.44 - 6.38 (m, 1H), 5.78 (d, *J* = 7.6 Hz, 1H), 5.73 - 5.60 (m, 1H), 5.06 (s, 1H), 5.03 - 4.96 (m, 1H), 4.90 - 4.81 (m, 1H), 4.80 - 4.69 (m, 1H), 4.62 - 4.51 (m, 1H), 4.31 - 4.19 (m, 1H), 3.50 - 3.43 (m, 2H), 3.41 - 3.38 (m, 2H), 3.18 - 3.07 (m, 2H), 3.04 - 2.91 (m, 1H), 2.82 - 2.66 (m, 2H), 2.63 - 2.54 (m, 3H), 2.42 - 2.29 (m, 4H), 2.26 - 2.15 (m, 1H), 2.13 - 1.96 (m, 2H), 1.95 - 1.77 (m, 4H), 1.76 - 1.63 (m, 1H), 1.49 - 1.36 (m, 1H), 1.31 - 1.23 (m, 2H), 1.10 (d, *J* = 6.0 Hz, 6H), 0.87 (t, *J* = 7.2 Hz, 3H). |
| 171 | B 15-B | 856.4 | 10.77 (s, 1H), 10.12 (br s, 1H), 8.82 (s, 1H), 7.93 (br s, 1H), 7.69 (d, *J* = 6.8 Hz, 1H), 7.58 (br s, 2H), 6.92 (d, *J* = 6.8 Hz, 2H), 6.49 (d, *J* = 12.8 Hz, 1H), 6.43 - 6.34 (m, 2H), 5.73 - 5.61 (m, 1H), 5.56 (d, *J* = 6.0 Hz, 1H), 5.06 (s, 1H), 5.00 (d, *J* = 8.0 Hz, 1H), 4.85 (d, *J* = 14.0 Hz,1H), 4.75 (br s, 1H), 4.58 (br s, 1H), 4.24 - 4.15 (m, 1H), 3.87 (t, *J* = 4.8 Hz, 2H), 3.43 (d, *J* = 5.6 Hz, 2H), 3.16 (d, *J* = 4.4 Hz, 1H), 3.08 (br s, 4H), 3.02 -2.93 (m, 1H), 2.82 - 2.68 (m, 2H), 2.60 - 2.53 (m, 1H), 2.31 - 2.23 (m, 4H), 2.22 - 2.16 (m, 1H), 2.12 - 2.05 (m, 1H), 2.05 - 1.98 (m, 1H), 1.92 - 1.86 (m, 1H),1.85 - 1.77 (m, 1H), 1.75 - 1.66 (m, 1H), 1.55 (br s, 4H), 1.47 (br s, 4H), 0.87 (t, *J* = 6.0 Hz, 3H) |
| 172 | B 15-B | 828.3 | 10.75 (br s, 1H), 10.06 (br s, 1H), 8.79 (s, 1H), 7.94 - 7.82 (m, 1H), 7.68 (d, *J* = 8.0 Hz, 1H), 7.61 - 7.41 (m, 2H), 6.60 - 6.54 (m, 1H), 6.53 - 6.48 (m, 1H), 6.44 - 6.28 (m, 3H), 5.71 - 5.61 (m, 1H), 5.54 - 5.49 (m, 1H), 5.05 (s, 1H), 5.02 - 4.96 (m, 1H), 4.89 - 4.81 (m, 1H), 4.75 (br s, 1H), 4.57 (br s, 1H), 4.23 - 4.15 (m, 1H), 3.92 - 3.84 (m, 2H), 3.62 (br s, 2H), 3.24 - 3.17 (m, 2H), 3.16 - 3.12 (m, 1H), 3.07 - 3.03 (m, 1H), 3.00 - 2.90 (m, 1H), 2.81 - 2.67 (m, 2H), 2.66 - 2.61 (m, 1H), 2.60 - 2.53 (m, 3H), 2.47 - 2.43 (m, 1H), 2.23 - 2.16 (m, 1H), 2.11 - 2.05 (m, 1H), 2.04 - 1.95 (m, 2H), 1.94 - 1.87 (m, 2H), 1.86 - 1.74 (m, 4H), 1.73 - 1.66 (m, 1H), 0.88 - 0.85 (m, 3H) |
| 173 | B 15-B | 828.3 | 10.77 (br s, 1H), 10.04 (br s, 1H), 8.79 (s, 1H), 7.88 (d, *J* = 8.4 Hz, 1H), 7.68 (d, *J* = 8.4 Hz, 1H), 7.50 (br s, 2H), 6.83 (t, *J* = 9.2 Hz, 1H), 6.50 (d, *J* = 14.8 Hz, 1H), 6.45 - 6.34 (m, 3H), 5.80 (d, *J* = 7.2 Hz, 1H), 5.72 - 5.60 (m, 1H), 5.04 (s, 1H), 4.99 (d, *J* = 10.4 Hz, 1H), 4.85 (d, *J* = 17.2 Hz, 1H), 4.74 (br s, 1H), 4.57 (br s, 1H), 4.30 - 4.20 (m, 1H), 3.81 (s, 2H), 3.69 (s, 2H), 3.30 (br s, 4H), 3.02 - 2.91 (m, 1H), 2.85 (br s, 3H), 2.77 (d, *J* = 5.6 Hz, 1H), 2.67 (d, *J* = 2.0 Hz, 1H), 2.38 (br s, 3H), 2.30 (br s, 2H), 2.23 - 2.15 (m, 1H), 2.13 - 2.07 (m, 1H), 2.05 - 1.97 (m, 3H), 1.91 - 1.83 (m, 2H), 1.76 - 1.64 (m, 1H), 0.87 (t, *J* = 7.2 Hz, 3H). |

(continued)

| Compound No. | Intermediate No. | MS(ESI+): m/z [M+H]+ | $^1$H NMR (400 MHz, DMSO-$d_6$) $\delta$ |
|---|---|---|---|
| 175 | B 15-B | 848.9 | 10.81 (s, 1H), 10.12 (s, 1H), 8.85 - 8.77 (m, 1H), 7.92 (s, 1H), 7.69(d, *J* = 8.0 Hz, 2H), 7.59 (s, 2H), 6.92 (d, *J* = 8.4 Hz, 2H), 6.31 (d, *J* = 12.0 Hz, 2H), 6.22 (d, *J* = 8.4 Hz,1H), 5.72 - 5.60 (m, 1H), 5.05 (s, 1H), 4.99 (d, *J* = 10.4 Hz, 1H), 4.85 (d, *J* = 17.6 Hz, 1H), 4.74 (s,1H), 4.59 (s, 1H), 4.31 (s, 1H), 3.10 (br s, 4H), 2.94 (br s, 5H), 2.81 - 2.73 (m, 1H), 2.58 (br s, 5H), 2.22 (d, *J* = 6.4 Hz, 2H), 2.19 (s, 1H), 2.07 - 1.97 (m, 2H), 1.91 - 1.82 (m, 2H), 1.74 (d, *J* = 8.8 Hz, 2H), 1.66 - 1.55 (m, 2H), 1.34 (s, 2H), 0.90 - 0.85 (m, 3H). |
| 176 | B 15-B | 846.2 | 10.77 (s, 1H), 10.26 - 9.99 (m, 1H), 8.82 (s, 1H), 8.01 - 7.84 (m, 1H), 7.69 (d, *J* = 8.0 Hz, 1H), 7.59 (s, 2H), 6.93 (t, *J* = 8.4 Hz, 3H), 6.75 (d, *J* = 2.4 Hz, 1H), 6.63 - 6.57 (m, 1H), 5.84 (d, *J* = 8.0 Hz, 1H), 5.72 - 5.60 (m, 1H), 5.05 (s, 1H), 4.99 (d, *J* = 9.2 Hz, 1H), 4.85 (d, *J* = 17.6 Hz, 1H), 4.74 (s, 1H), 4.58 (s, 1H), 4.33 -4.24 (m, 1H), 3.17 - 3.02 (m, 7H), 3.02 - 2.93 (m, 1H), 2.82 - 2.75 (m, 2H), 2.58 (s, 6H), 2.24 (d, *J* = 6.8 Hz, 2H), 2.20 (d, *J* = 5.2 Hz, 1H), 2.09 - 1.97(m, 2H), 1.93 - 1.83 (m, 2H), 1.79 (d, *J* = 11.6 Hz, 2H), 1.74 - 1.58 (m, 2H), 1.36 - 1.25 (m, 2H),0.87 (t, *J* = 7.6 Hz, 3H). |
| 177 | B 15-B | 860.3 | 10.85 (br s, 1H), 10.14 (br s, 1H), 8.82 (s, 1H), 7.93 (br s, 1H), 7.69(m, 1H), 7.58 (br s, 2H), 6.92 (m, 2H), 6.64 - 6.49 (m, 2H), 5.73 - 5.60 (m, 1H),5.14 - 4.95 (m, 3H), 4.91 - 4.68 (m, 2H), 4.62 - 4.48 (m, 1H), 4.32 - 4.18 (m, 1H), 3.78 (s, 3H), 3.20 -3.05 (m, 6H), 3.03 - 2.91 (m, 1H), 2.85 - 2.72 (m, 2H), 2.63 (m, 2H), 2.55 (br s, 5H), 2.28 - 2.09 (m, 4H), 2.07 - 1.97 (m, 1H), 1.95 - 1.84 (m, 2H), 1.83 - 1.75 (m, 2H), 1.73 - 1.57 (m, 2H),1.37 - 1.24 (m, 2H), 0.87 (t, *J* = 7.2 Hz, 3H). |
| 179 | B 15-B | 828.3 | 10.76 (br s, 1H), 10.05 (br s, 1H), 8.80 (s, 1H), 7.94 - 7.85 (m, 1H), 7.69 (d, *J* = 8.4 Hz, 1H), 7.52 (br s, 2H), 6.54 - 6.47 (m, 1H), 6.45 - 6.36 (m, 4H), 5.73 - 5.61 (m, 1H), 5.58 - 5.52 (m, 1H), 5.05 (s, 1H), 5.02 - 4.96 (m, 1H), 4.89 - 4.82 (m, 1H), 4.74 (br s, 1H), 4.58 (br s, 1H), 4.21 (br s, 1H), 3.87 (br s, 2H), 3.53 (s, 4H), 3.46 (br s, 2H), 3.23 - 3.08 (m, 2H), 3.03 - 2.92 (m, 1H), 2.83 - 2.71 (m, 2H), 2.59 (br s, 2H), 2.30 - 2.18 (m, 4H), 2.12 - 1.99 (m, 2H), 1.93 - 1.80 (m, 2H), 1.77 - 1.70 (m, 4H), 0.90 - 0.85 (m, 3H). |
| 180 | B 15-B | 416.4 [1/2M+H]+ | 10.80 (s, 1H), 10.22 - 10.01 (m, 1H), 8.82 (s, 1H), 8.01 - 7.84 (m, 1H), 7.70 (d, *J* = 8.0 Hz, 1H), 7.56 (br s, 3H), 7.02 - 6.95 (m, 1H), 6.92 (d, *J* = 8.8 Hz, 2H), 5.89 (d, *J* = 7.6 Hz, 1H), 5.76 - 5.57 (m, 1H), 5.04 (s, 1H), 4.99 (d, *J* = 9.6 Hz, 1H), 4.90 - 4.82 (m, 1H), 4.81 - 4.69 (m, 1H), 4.63 - 4.50 (m, 1H), 4.33 - 4.21 (m, 1H), 3.57 - 3.45 (m, 2H), 3.10 (br s, 4H), 3.02 - 2.92 (m, 1H), 2.84 - 2.75 (m, 1H), 2.72 - 2.63 (m, 3H), 2.57 - 2.53 (m, 1H), 2.49 - 2.43 (m, 4H), 2.27 - 2.18 (m, 3H), 2.15 - 2.06 (m, 1H), 2.04 - 1.94 (m, 1H), 1.94 - 1.84 (m, 2H), 1.83 - 1.75 (m, 2H), 1.73 - 1.63 (m, 2H), 1.29 - 1.23 (m, 2H), 0.87 (t, *J* = 7.2 Hz, 3H). |
| 181 | B 15-B | 869.2 [M+Na]+ | 10.80 (s, 1H), 10.28 - 9.96 (m, 1H), 8.82 (s, 1H), 7.92 (br s, 1H), 7.80 - 7.65 (m, 3H), 7.58 (br s, 2H), 7.20 (d, *J*=4.0 Hz, 1H), 6.92 (d, *J*=8.0 Hz, 2H), 6.00 (d, *J*=8.0 Hz, 1H), 5.75 - 5.56 (m, 1H), 5.08 - 4.96 (m, 2H), 4.85 (d, *J*=20.0 Hz, 1H), 4.74 (br s, 1H), 4.60 (br s, 1H), 4.34 (br s, 1H), 3.10 (br s, 4H), 3.02 - 2.92 (m, 1H), 2.84 - 2.71 (m, 2H), 2.67 - 2.64 (m, 2H), 2.61 (d, *J*=12.0 Hz, 2H), 2.55 - 2.53 (m, 4H), 2.28 - 2.16 (m, 3H), 2.13 - 1.97 (m, 2H), 1.95 1.85 (m, 2H), 1.80 (d, *J*=12.0 Hz, 2H), 1.74 - 1.62 (m, 2H), 131-124 (m, 2H), 0.89 - 0.85 (m, 3H). |

(continued)

| Compound No. | Intermediate No. | MS(ESI$^+$): m/z [M+H]$^+$ | $^1$H NMR (400 MHz, DMSO-$d_6$) $\delta$ |
|---|---|---|---|
| 182 | B 15-B | 817.4 | 10.81 (s, 1H), 10.14 (s, 1H), 8.82 (s, 1H), 7.93 (s, 1H), 7.70 (d, *J* = 8.2 Hz, 1H), 7.57 (s, 3H), 7.00 (d, *J = 14.8* Hz, 1H), 6.92 (d, *J* = 9.2 Hz, 2H), 5.92 (d, *J* = 8.0 Hz, 1H), 5.74-5.60 (m, 1H), 5.06 (s, 1H), 5.00 (d, *J* = 10.4 Hz, 1H), 4.86 (d, *J* = 17.2 Hz, 1H), 4.75 (s, 1H), 4.59 (s, 1H), 4.29 (d, *J*= 5.2 Hz, 1H), 3.60 (d, *J* = 11.6 Hz, 2H), 3.10 (s, 4H), 3.03 - 2.92 (m, 1H), 2.83 - 2.76 (m, 1H), 2.72 - 2.65 (m, 7H), 2.59 - 2.55 (m, 1H), 2.43 - 2.35 (m, 1H), 2.26-2.16 (m, 1H), 2.15 - 2.07 (m, 1H), 2.06 - 1.97 (m, 1H), 1.91 - 1.83 (m, 4H), 1.76 - 1.66 (m, 1H), 1.56 (d, *J* = 10.0 Hz, 2H), 0.87 (t, *J* = 7.2 Hz, 3H) |
| 183 | B 15-B | 851.2 | 10.81 (s, 1H), 10.13 (s, 1H), 8.82 (s, 1H), 7.95 (s, 1H), 7.70 (d, *J* = 8.0 Hz, 1H), 7.59 (s, 2H), 7.09 (s, 1H), 6.94 (d, *J* = 8.4 Hz, 2H), 6.53 - 6.43 (m, 2H), 6.17 (d, *J* = 6.8 Hz, 1H), 5.69 (d, *J* = 6.8 Hz, 1H), 5.06 (s, 1H), 4.99 (d, *J* = 10.6 Hz, 1H), 4.85 (d, *J* = 16.8 Hz, 2H), 4.59 (s, 1H), 4.44 - 4.39 (m, 1H), 3.69 (s, 2H), 3.43 - 3.41 (m, 1H), 3.28 (s, 4H), 3.00 (s, 1H), 2.74 (s, 2H), 2.62 (d, *J* = 12.4 Hz, 2H), 2.39 (s, 2H), 1.99 (d, *J* = 6.8 Hz, 4H), 1.86 (s, 2H), 1.71 (s, 2H), 1.58 (s, 2H), 1.43 (s, 2H), 0.87 (s, 3H) |
| 184 | B 15-B | 851.2 | 10.81 (s, 1H), 10.29 - 9.98 (m, 1H), 8.82 (s, 1H), 7.94 (s, 1H), 7.73 - 7.68 (m, 1H), 7.59 (s, 2H), 7.16 - 7.05 (m, 1H), 7.01 - 6.88 (m, 2H), 6.51 - 6.44 (m, 1H), 6.21 - 6.13 (m, 1H), 5.74 - 5.60 (m, 1H), 5.06 (s, 1H), 5.03 - 4.96 (m, 1H), 4.92 - 4.69 (m, 2H), 4.64 - 4.50 (m, 1H), 4.39 - 4.29 (m, 1H), 3.76 - 3.66 (m, 3H), 3.27 - 3.11 (m, 1H), 3.06 - 2.90 (m, 1H), 2.83 - 2.70 (m, 2H), 2.69 - 2.58 (m, 5H), 2.43 - 2.31 (m, 2H), 2.25 - 2.15 (m, 1H), 2.13 - 1.97 (m, 3H), 1.94 - 1.79 (m, 4H), 1.75 - 1.66 (m, 2H), 1.58 (br s, 2H), 1.23 (s, 1H), 0.91 - 0.84 (m, 3H). |
| 185 | B 15-B | 829.3 | 10.79 (s, 1H), 10.12 (br s, 1H), 8.82 (s, 1H), 7.97 - 7.88 (m, 1H), 7.70 (d, *J* = 8.0 Hz, 1H), 7.58 (br s, 2H), 7.49 (s, 1H), 7.04 - 6.86 (m, 3H), 5.70 (d, *J*=9.2 Hz, 1H), 5.67 - 5.62 (m, 1H), 5.05 (s, 1H), 5.00 (d, *J* = 10.0 Hz, 1H), 4.90 - 4.82 (m, 1H), 4.75 (br s, 1H), 4.64 - 4.52 (m, 1H), 4.28 - 4.20 (m, 1H), 3.91 (s, 2H), 3.79 (s, 2H), 3.09 (br s, 4H), 3.03 - 2.93 (m, 1H), 2.83 - 2.74 (m, 1H), 2.73 - 2.66 (m, 2H), 2.62 - 2.58 (m, 1H), 2.40 (br s, 4H), 2.33 - 2.27 (m, 2H), 2.25 - 2.17 (m, 1H), 2.15 - 2.07 (m, 1H), 2.06 - 1.97 (m, 3H), 1.94 - 1.83 (m, 2H), 1.79 - 1.65 (m, 1H), 0.91 - 0.85 (m, 3H) |
| 186 | B 15-B | 830.2 | 10.78 (s, 1H), 10.12 (br s, 1H), 8.82 (s, 1H), 7.93 (br s, 1H), 7.70 (d, *J* = 8.0 Hz, 1H), 7.58 (br s, 2H), 6.94 - 6.89 (m, 2H), 6.88 - 6.81 (m, 1H), 6.54 - 6.46 (m, 1H), 6.44 - 6.38 (m, 1H), 5.83 - 5.77 (m, 1H), 5.73 - 5.60 (m, 1H), 5.05 (s, 1H), 5.02 - 4.97 (m, 1H), 4.90 - 4.82 (m, 1H), 4.75 (br s, 1H), 4.61 - 4.51 (m, 1H), 4.26 (br s, 1H), 3.42 - 3.38 (m, 2H), 3.21 - 3.14 (m, 2H), 3.02 - 2.93 (m, 2H), 2.93 - 2.87 (m, 1H), 2.86 - 2.72 (m, 5H), 2.70 (br s, 1H), 2.61 - 2.56 (m, 2H), 2.25 - 2.16 (m, 1H), 2.15 - 1.97 (m, 3H), 1.94 - 1.79 (m, 3H), 1.79 - 1.67 (m, 2H), 1.66 - 1.59 (m, 1H), 1.56 - 1.47 (m, 1H), 1.15 - 1.05 (m, 3H), 0.87 (t, *J* = 7.2 Hz, 3H). |

(continued)

| Compound No. | Intermediate No. | MS(ESI$^+$): m/z [M+H]$^+$ | $^1$H NMR (400 MHz, DMSO-$d_6$) $\delta$ |
|---|---|---|---|
| 187 | B 15-B | 831.3 | 10.79 (s, 1H), 10.16 (s, 1H), 8.83 (s, 1H), 8.35 (s, 1H),7.97 (s, 1H), 7.87 (d, *J* = 8.4 Hz, 1H), 7.64 (s, 1H), 6.85 (d, *J* = 8.0 Hz, 2H), 6.49 (d, *J* = 14.4 Hz,1H), 6.41 (d, *J* = 7.2 Hz, 1H), 5.79 (d, *J* = 6.0 Hz, 1H), 5.66 (d, *J* = 5.6 Hz, 1H), 5.08 (s, 1H), 4.99 (d, *J* = 8.8 Hz, 1H), 4.85 (d, *J* = 17.6 Hz, 1H), 4.73 (s, 1H), 4.57 (s, 1H), 4.25 (s, 1H), 3.42 (s,3H), 3.12 (d, *J* = 9.2 Hz, 2H), 3.02 - 2.92 (m, 1H), 2.85 - 2.66 (m, 3H), 2.57 (d, *J* = 12.8 Hz, 3H), 2.46(s, 3H), 2.21 (s, 3H), 2.12 - 1.98 (m, 3H), 1.86 (s, 2H), 1.78 (d, *J* = 12.0 Hz, 2H), 1.69 (d, *J* = 6.0 Hz, 1H), 1.45 (s, 1H), 1.32 - 1.26 (m, 2H), 0.86 (br s, 3H). |
| 188 | B 49-A | 843.3 | 10.79 (s, 1H), 10.14 (s, 1H), 8.82 (s, 1H), 7.78 (s, 1H), 7.73 - 7.66 (m, 1H), 7.58 (s, 2H), 6.93 (d, *J* = 8.0 Hz, 2H), 6.83 (t, *J* = 8.8 Hz, 1H), 6.49 (d, *J* = 14.4 Hz, 1H), 6.41 (d, *J* = 9.2 Hz, 1H), 5.79 (d, *J* = 8.0 Hz, 1H), 5.65 (d, *J* = 6.0 Hz, 1H), 4.99 (d, *J* = 9.6 Hz, 1H), 4.86 (d, *J* = 16.8 Hz, 2H), 4.75 (s, 1H), 4.59 (d, *J* = 13.6 Hz, 1H), 4.25-4.17 (m, 1H), 3.10 (s, 6H), 2.81-2.64 (m, 4H), 2.57 (d, *J* = 14.0 Hz, 7H), 2.22 (s, 2H), 2.07 (s, 1H), 1.96 - 1.70 (m, 8H), 1.62 (s, 1H), 1.26 (d, *J* = 11.2 Hz, 2H), 0.78 (br s, 3H). |
| 190 | B 50-A | 830.2 | 10.79 (s, 1H), 10.13 (s, 1H), 8.82 (s, 1H), 7.77 (s, 1H), 7.73 - 7.68 (m, 1H), 7.58 (s, 2H), 6.93 (d, *J* = 8.4 Hz, 2H), 6.84 (s, 1H), 6.50 (d, *J* = 14.4 Hz, 1H), 6.42 (d, *J* = 8.8 Hz, 1H), 5.79 (d, *J* = 6.8 Hz, 1H), 5.73 - 5.55 (m, 1H), 4.99 (d, *J* = 10.4 Hz, 1H), 4.91 (s, 1H), 4.86 (d, *J* = 16.8 Hz, 2H), 4.65 (d, *J* = 12.8 Hz, 1H), 4.25 (s, 1H), 3.11 (s, 6H), 2.91 - 2.67 (m, 3H), 2.57 (d, *J* = 12.0 Hz, 6H), 2.23 (d, *J* = 6.8 Hz, 2H), 2.08 (s, 1H), 2.02 - 1.88 (m, 3H), 1.81 (t, *J* = 12.5 Hz, 4H), 1.72 (s, 1H), 1.62 (s, 1H), 1.50 (s, 3H), 1.27 (s, 2H) |
| 191 | B 50-B | 830.2 | 10.78 (br s, 1H), 10.11 (s, 1H), 8.82 (br s, 1H), 7.82 - 7.66 (m, 2H), 7.58 (br s, 2H), 6.94 (br s, 2H), 6.84 (br s, 1H), 6.56 - 6.46 (m, 1H), 6.42 (br s, 1H), 5.79 (br s, 1H), 5.67 (br s, 1H), 5.03 - 4.95 (m, 1H), 4.93 - 4.82 (m, 3H), 4.67 (br s, 1H), 4.25 (br s, 1H), 3.11 (br s, 6H), 2.90 - 2.67 (m, 3H), 2.63 - 2.56 (m, 6H), 2.23 (br s, 2H), 2.07 (br s, 1H), 2.02 - 1.56 (m, 9H), 1.50 (br s, 3H), 1.27 (br s, 2H) |
| 192 | B 15-B | 845.3 | 10.82 (s, 1H), 10.15 (s, 1H), 8.82 (s, 1H), 7.93 (s, 1H), 7.70 (s, 1H), 7.56 (s, 3H), 7.03 - 6.82 (m, 3H), 5.90 (s, 1H), 5.67 (s, 1H), 5.07 (s, 1H), 5.03 - 4.95 (m, 1H), 4.90 - 4.68 (m, 2H), 4.58 (s, 1H), 4.28 (s, 1H), 3.50 (s, 2H), 2.95 (s, 4H), 2.85 - 2.57 (m, 9H), 2.25 (s, 1H), 2.31 - 2.15 (m, 1H), 2.14 - 1.95 (m, 4H), 1.88 (s, 4H), 1.69 (s, 3H), 106 (s, 3H), 0.87 (s, 3H). |
| 193 | B 15-B | 867.3 [M+Na]$^+$ | 10.82 (br s, 1H), 10.22 - 10.05 (m, 1H), 8.82 (s, 1H), 8.00 - 7.86 (m, 1H), 7.73 - 7.66 (m, 1H), 7.56 (br s, 2H), 7.03 - 6.87 (m, 3H), 5.91 (d, *J* = 7.6 Hz, 1H), 5.74 - 5.59 (m, 1H), 5.07 (s, 1H), 4.99 (d, *J* = 10.0 Hz, 1H), 4.90 - 4.70 (m, 2H), 4.63 - 4.50 (m, 1H), 4.35 - 4.22 (m, 1H), 3.51 (d, *J* = 11.6 Hz, 2H), 3.45 - 3.40 (m, 2H), 3.03 - 2.91 (m, 2H), 2.84 - 2.64 (m, 5H), 2.63 - 2.53 (m, 3H), 2.48 - 2.39 (m, 2H), 2.31 - 2.15 (m, 2H), 2.13 - 1.96 (m, 3H), 1.94 - 1.82 (m, 3H), 1.76 - 1.60 (m, 3H), 1.32 - 1.17 (m, 2H), 1.10-1.02 (m, 3H), 0.87 (t, *J* = 6.8 Hz, 3H). |

(continued)

| Compound No. | Intermediate No. | MS(ESI$^+$): m/z [M+H]$^+$ | $^1$H NMR (400 MHz, DMSO-$d_6$) $\delta$ |
|---|---|---|---|
| 195 | B 15-B | 832.3 | 10.82 (br s, 1H), 10.16 (br s, 1H), 8.83 (br s, 1H), 8.36 (br s, 1H), 7.97 (br s, 1H), 7.87 (m, 1H), 7.65 (br s, 1H), 7.56 (s, 1H), 6.99 (m, 1H), 6.90 - 6.77 (m, 1H), 5.91 (m, 1H), 5.74 - 5.60 (m, 1H), 5.08 (s, 1H), 5.00 (m, 1H), 4.85 (m, 1H), 4.73 (br s, 1H), 4.63 - 4.48 (m, 1H), 4.29 (br s, 1H), 3.52 (m, 4H), 3.30 (br s, 2H), 2.96 (m, 1H), 2.84 - 2.66 (m, 4H), 2.60 (br s, 1H), 2.46 (br s, 4H), 2.21 (br s, 3H), 2.14 - 2.06 (m, 1H), 2.05 - 1.96 (m, 1H), 1.95 - 1.84 (m, 2H), 1.79 (m, 2H), 1.73 - 1.64 (m, 2H), 1.23 (br s, 2H), 0.87 (br s, 3H). |
| 199 | B 48 | 808.6 | 10.78 (s, 1H), 10.15 (s, 1H), 8.76 (s, 1H), 8.15 (s, 1H), 7.85 (d, *J* = 8.4 Hz, 1H), 7.52 (d, *J* = 8.0 Hz, 3H), 6.92 (d, *J* = 8.0 Hz, 2H), 6.84 (t, *J* = 9.2 Hz, 1H), 6.56 - 6.47 (m, 1H),6.42 (d, *J* = 8.4 Hz, 1H), 5.79 (d, *J* = 8.0 Hz, 1H), 4.25 (s, 1H), 4.18 - 4.02 (m, 1H), 3.11 (s, 6H), 2.78 -2.67 (m, 1H), 2.57 (d, *J* = 12.0 Hz, 6H), 2.24 (s, 2H), 2.08 (d, *J* = 8.0 Hz, 1H), 2.04 - 1.97 (m, 1H),1.89 - 1.75 (m, 4H), 1.70 (s, 3H), 1.64 (s, 3H), 1.37 (d, *J* = 6.0 Hz, 6H), 1.30 - 1.26 (m, 2H). |
| 203 | B 15-B | 813.3 | 10.74 (s, 1H), 10.15 (br s, 1H), 8.82 (s, 1H), 7.93 (br s, 1H), 7.78 - 7.47 (m, 4H), 7.22 (d, *J* = 9.2 Hz, 1H), 6.92 (d, *J* = 8.0 Hz, 2H), 6.54 (d, *J* = 8.0 Hz, 1H), 6.39 (d, *J* = 8.0 Hz, 1H), 5.73 - 5.57 (m, 1H), 5.10 - 4.94 (m, 2H), 4.91 - 4.69 (m, 2H), 4.69 - 4.48 (m, 2H), 3.29 - 3.21 (m, 1H), 3.10 (br s, 4H), 3.02 - 2.89 (m, 1H), 2.82 - 2.68 (m, 2H), 2.55 (br s, 5H), 2.28 - 2.15 (m, 3H), 2.13 - 1.93 (m, 4H), 1.92 - 1.84 (m, 2H), 1.80 (d, *J* = 12.0 Hz, 2H), 1.74 - 1.67 (m, 1H), 1.62 (br s, 1H), 1.54 - 1.35 (m, 1H), 1.31 - 1.18 (m, 2H), 0.94 - 0.79 (m, 3H). |
| 204 | | 802.3 | 10.78 (s, 1H), 10.15 (br s, 1H), 8.82 (s, 1H), 8.34 (s, 0.139HCOOH), 8.09 - 7.93 (m, 1H), 7.73 (d, *J* = 8.0 Hz, 1H), 7.57 (br s, 2H), 7.38 (d, *J* = 8.0 Hz, 1H), 6.92 (d, *J* = 8.0 Hz, 2H), 6.87 - 6.78 (m, 1H), 6.55 - 6.33 (m, 2H), 5.79 (d, *J* = 8.0 Hz, 1H), 5.72 - 5.60 (m, 1H), 4.98 (d, *J* = 12.0 Hz, 1H), 4.81 (d, *J* = 16.0 Hz, 1H), 4.71 (d, *J* = 4.0 Hz, 2H), 4.25 (br s, 1H), 3.27 (br s, 1H), 3.10 (br s, 6H), 2.79 - 2.68 (m, 1H), 2.58 (br s, 2H), 2.55 (br s, 4H), 2.23 (d, *J* = 8.0 Hz, 2H), 2.08 (d, *J* = 8.0 Hz, 1H), 1.85 (br s, 1H), 1.78 (d, *J* = 12.0 Hz, 2H), 1.62 (br s, 1H), 1.33 (s, 9H), 1.26 (d, *J* = 9.6 Hz, 2H) |
| 205 | | 776.2 | 10.78 (s, 1H), 10.18 (br s, 1H), 8.82 (s, 1H), 7.97 (br s, 1H), 7.57 (br s, 2H), 7.45 (m, 1H), 6.92 (m, 2H),6.87 - 6.76 (m, 2H), 6.50 (m, 1H), 6.41 (m, 1H), 5.79 (m, 1H), 5.74 - 5.62 (m, 1H), 5.03 (m, 1H), 4.93 (m, 1H), 4.66 (br s, 2H), 4.25 (br s, 1H), 3.88 (s, 3H), 3.10 (br s, 6H), 2.80 - 2.67 (m, 1H), 2.59 (br s, 2H), 2.55 - 2.52 (m, 5H), 2.23 (m, 2H), 2.08(m, 1H), 1.88 (m, 1H), 1.78 (m, 2H), 1.62 (br s, 1H), 1.33 - 1.26 (m, 2H). |
| 206 | B 15-B | 874.3 | 10.78 (br s, 1H), 10.14 (br s, 1H), 8.82 (s, 1H), 7.91 (br s, 1H), 7.74 - 7.63 (m, 1H), 7.58 (br s, 2H), 6.98 - 6.77 (m, 3H), 6.61 - 6.45 (m, 1H), 6.45 - 6.36 (m, 1H), 5.82 - 5.74 (m, 1H), 5.73 - 5.60 (m, 1H), 5.07 (s, 1H), 5.04 - 4.94 (m, 1H), 4.90 - 4.80 (m, 1H), 4.74 (br s, 1H), 4.63 - 4.49 (m, 1H), 4.25 (br s, 1H), 3.59 - 3.50 (m, 1H), 3.28 (br s, 3H), 3.24 (br s, 2H), 3.16 - 3.05 (m, 3H), 3.03 - 2.85 (m, 4H), 2.83 - 2.71 (m, 3H), 2.59 (br s, 2H), 2.57 - 2.52 (m, 2H), 2.19 (br s, 2H), 2.13 - 1.96 (m, 2H), 1.95 - 1.77 (m, 4H), 1.71 (br s, 2H), 1.55 (br s, 1H), 1.27 (br s, 2H), 0.87 (t, *J* = 6.8 Hz, 3H). |

**Example 114: synthesis of compound 114**

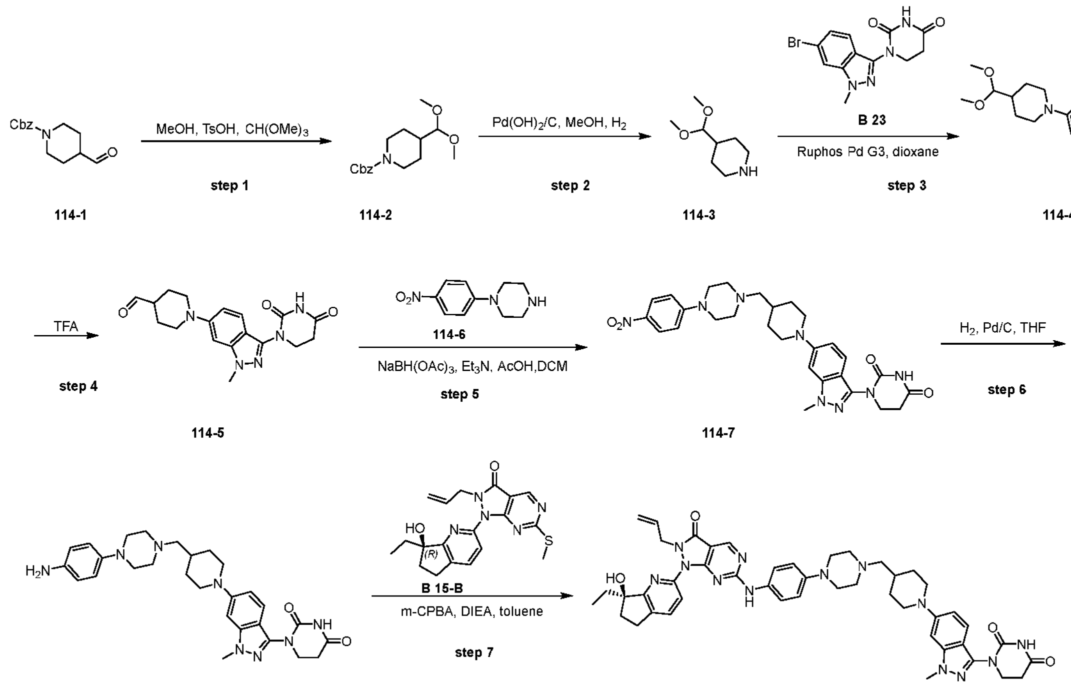


114-1 114-2 114-3 114-4 114-5 114-7 114-8 114

**Step 1: synthesis of intermediate 114-2**

To a solution of intermediate **114-1** (250 g, 1.01 mol) and trimethoxymethane (318.36 g, 3.0 mol, 328.88 mL) in MeOH (1 L) was added TsOH (8.70 g, 50.55 mmol). The mixture was stirred at 20 °C for 16 hrs . Then aq. NaHCO3 (150 mL) was added to the mixture,, and the reaction mixture was partitioned between water (500 mL) and EA (1000 mL). The organic phase was separated, dried over $Na_2SO_4$, filtered and concentrated under reduced pressure to give a crude of **intermediate 114-2** (320 g) as a yellow oil, which was used into the next step directly.

**Step 2: synthesis of intermediate 114-3**

To a solution of **intermediate 114-2** (320 g, 1.09 mol) in MeOH (600 mL) was added $Pd(OH)_2/C$ (7.5 g, 10% purity). The mixture was stirred at 40 °C under $H_2$ (40 Psi) for 16 hrs. The reaction mixture was filtered and the filtrate was concentrated to give a crude of **intermediate 114-3** (170 g, crude) as a yellow oil, which was used into the next step directly.

**Step 3: synthesis of intermediate 114-4**

A brown suspension of **intermediate B 23** (6.30 g, 19.5 mmol) , **intermediate 114-3** (4.04 g, 25.3 mmol), $Cs_2CO_3$ (19.1 g, 58.5 mmol), RuPhos Pd G3 (4.08 g, 4.87 mmol) in dioxane (120 mL) was degassed with $N_2$ for 3 times and stirred at 100°C for 6 hours under $N_2$. The reacion mixture was diluted with DCM (100 mL) and filtered. The filtrate was concentrated to give a residue. The residue was purified by combi flash (DCM:MeOH=1:0 to 20:1, 80 g silica gel column) to give **intermediate 114-4** (3.9 g, 49.8% yield) as a yellow solid.

**Step 4: synthesis of intermediate 114-5**

A solution of **intermediate 114-4** (3.9 g, 6.08 mmol) in TFA (50 mL) was stirred at 50°C for 30 min. The reaction mixture was concentrated to give a crude of intermediate **114-5** (4.70 g, TFA salt) as a red oil which was used into the next step directly.
LCMS (ESI+): m/z 356.0 $[M+H]^+$.

**Step 5: synthesis of intermediate 114-7**

A yellow suspension of **intermediate 114-5** (4.70 g, 10.0 mmol, TFA), **intermediate 114-6** (2.70 g, 13.0 mmol), $Et_3N$ (7.09 g, 70.1 mmol) in DCM (100 mL) was stirred at 20°C for 30 min. Then AcOH (3.01 g, 50.1 mmol) was added and the mixture was stirred at 20°C for 1 hour. Then $NaBH(OAc)_3$ (8.49 g, 40.1 mmol) was added and the reaction mixture was stirred at 20°C for 1 hour. The reaction mixture was diluted with DCM (100 mL) and washed with water (100 mL). The organic layer was separated and dried over $Na_2SO_4$, filtered and concentrated to give a residue. The residue was purified by combi flash (DCM: MeOH=1: 0 to 20: 1). **Intermediate 114-7** (4.90g, 89.5% yield) was obtained as a yellow solid.

**Step 6: synthesis of intermediate 114-8**

A suspension of **intermediate 114-7** (3.0 g, 5.49 mmol) and Pd/C (2.0 g, 10% purity) in THF (100 mL) was stirred at 20°C under $H_2$ (15 Psi) for 16 hours. The black reaction mixture was filtered and the filtrate was concentrated to give a residue. The residue was purified by combi flash (DCM:MeOH=20:1 to 10:1, 20 g silica gel column) to give the **intermediate 114-8** (1.70 g, 59.96% yield) as a brown solid.

**Step 7: synthesis of compound 114**

To a solution of **intermediate B15-B** (550 mg, 1.06 mmol) in toluene (3 mL) was added m-CPBA (303 mg, 1.49 mmol, 85% purity) and the obtained mixture was stirred at 20°C for 30 min. Then DIEA (275 mg, 2.13 mmol), THF (3 mL) and **intermediate 114-8** (420 mg, 1.10 mmol) was added . The reaction mixture was stirred at 20°C for 16 hours. The reaction mixture was quenched by $Na_2S_2O_3$ (10 mL) and extrated with DCM (40 mL), dried over $Na_2SO_4$, filtered and concentrated to give a residue. The residue was purified by chromatography on silica gel (EA: MeOH=10: 1) to give the **compound 114** (650 mg, 72% yield) as a pale yellow solid.

LCMS ($ESI^+$): m/z 852.4 $[M+H]^+$.

$^1H$ NMR (400 MHz, DMSO-$d_6$) $\delta$ 10.51 (s, 1H), 10.14 (br s, 1H), 8.82 (s, 1H), 7.93 (br s, 1H), 7.70 (d, *J* = 8.0 Hz, 1H), 7.59 (br s, 2H), 7.43 (d, *J* = 8.8 Hz, 1H), 6.98 - 6.84 (m, 3H), 6.81 (s, 1H), 5.76 - 5.56 (m, 1H), 5.06 (s, 1H), 4.99 (d, *J* = 10.0 Hz, 1H), 4.85 (d, *J*= 17.2 Hz, 1H), 4.75 (br s, 1H), 4.66 - 4.48 (m, 1H), 3.96 - 3.86 (m, 5H), 3.81 (d, *J* = 12.4 Hz, 2H), 3.11 (br s, 4H), 3.02 - 2.90 (m, 1H), 2.82 - 2.71 (m, 5H), 2.62 - 2.54 (m, 4H), 2.28 - 2.18 (m, 3H), 2.09 - 1.97 (m, 1H), 1.93 - 1.81 (m, 3H), 1.79 - 1.61 (m, 2H), 1.31 - 1.21 (m, 2H), 0.87 (t, *J* = 7.6 Hz, 3H)

The following compounds were synthesized according to the procedures described for **compound 114** using the different intermediates.

| Compound No. | Intermediate No. | MS($ESI^+$): m/z $[M+H]^+$ | $^1H$ NMR (400 MHz, DMSO-$d_6$) $\delta$ |
|---|---|---|---|
| 115 | B 26-B; B 40 | 838.5 $[M+H]^+$ | 10.51 (br s, 1H), 10.14 (br s, 1H), 8.82 (br s, 1H), 7.93 (br s, 1H), 7.75 - 7.39 (m, 4H), 7.03 - 6.74 (m, 4H), 5.68 (br s, 1H), 5.19 (br s, 1H), 5.08 - 4.95 (m, 1H), 4.92 - 4.81 (m, 1H), 4.79 - 4.53 (m, 2H), 3.97 - 3.75 (m, 7H), 3.11 (br s, 4H), 3.03 - 2.90 (m, 2H), 2.86 - 2.69 (m, 6H), 2.29 - 2.06 (m, 5H), 1.94 - 1.68 (m, 4H), 1.45 (br s, 3H), 1.23 (br s, 2H). |
| 137 | B 26-B | 852.5 $[M+H]^+$ | 10.51 (s, 1H), 10.16 (br s, 1H), 8.85 (s, 1H), 7.91 (d, *J*=8.0 Hz, 1H), 7.76 - 7.58 (m, 2H), 7.43 (d, *J*=8.0 Hz, 2H), 7.01 (d, *J* = 8.0 Hz, 1H), 6.91 (d, *J* = 8.0 Hz, 1H), 6.81 (s, 1H), 5.76 - 5.58 (m, 1H), 5.20 (s, 1H), 4.99 (d, *J* = 8.0 Hz, 1H), 4.85 (d, *J* = 16.0 Hz, 1H), 4.72 (br s,1H), 4.60 (d, *J* = 12.0 Hz, 1H), 3.94 - 3.85 (m, 5H), 3.81 (d, *J* = 8.0 Hz, 2H), 3.02 - 2.92 (m, 1H), 2.83 (br s, 4H), 2.78 - 2.69 (m, 4H), 2.44 (br s, 5H), 2.25 (s, 5H), 2.17 - 2.09 (m, 2H), 1.89 - 1.69 (m, 3H), 1.45 (s, 3H), 1.31 - 1.20 (m, 2H) |

(continued)

| Compound No. | Intermediate No. | MS(ESI$^+$): m/z [M+H]$^+$ | $^1$H NMR (400 MHz, DMSO-$d_6$) $\delta$ |
|---|---|---|---|
| 138 | B 26-A | 852.5 [M+H]$^+$ | 10.51 (s, 1H), 10.24 - 10.09 (m, 1H), 8.85 (s, 1H), 7.91 (d, *J* = 8.0 Hz, 1H), 7.79 - 7.60 (m, 2H), 7.43 (d, *J* = 8.8 Hz, 2H), 7.08 - 6.87 (m, 2H), 6.81 (s, 1H), 5.78 - 5.58 (m, 1H), 5.20 (s, 1H), 4.99 (d, *J* = 10.0 Hz, 1H), 4.90 - 4.83 (m, 1H), 4.80 - 4.69 (m, 1H), 4.67 - 4.51 (m, 1H), 3.93 - 3.85 (m, 5H), 3.85 - 3.76 (m, 2H), 3.02 - 2.92 (m, 1H), 2.89 - 2.81 (m, 4H), 2.77 - 2.71 (m, 4H), 2.43 (br s, 5H), 2.31 - 2.19 (m, 5H), 2.13 (t, *J* = 6.8 Hz, 2H), 1.90 - 1.70 (m, 3H), 1.45 (s, 3H), 1.26 - 1.20 (m, 2H). |
| 160 | B 15-B | 887.3 | 10.56 (s, 1H), 10.12 (br s, 1H), 8.82 (s, 1H), 7.93 (d, *J* = 6.4 Hz,1H), 7.70 (d, *J* = 8.4 Hz, 1H), 7.63 - 7.51 (m, 4H), 7.09 (d, *J* = 8.4 Hz, 1H), 6.93 (d, *J* = 8.8 Hz, 2H), 5.67 (d, *J* = 6.4 Hz, 1H), 5.05 (s, 1H), 5.00 (d, *J* = 10.4 Hz, 1H), 4.86 (d, *J* = 17.2 Hz, 1H), 4.75 (br s, 1H), 4.63 - 4.43 (m, 1H), 4.00 (s, 3H), 3.92 (t, *J* = 6.8 Hz, 2H), 3.65 (d, *J* = 8.8 Hz, 2H), 3.28 -3.15 (m, 2H), 3.10 - 2.91 (m, 2H), 2.83 - 2.72 (m, 3H), 2.66 - 2.58 (m, 2H), 2.42 - 2.32 (m, 3H),2.29 - 2.16 (m, 3H), 2.07 - 1.97 (m, 1H), 1.94 - 1.78 (m, 4H), 1.76 - 1.61 (m, 2H), 131-109 (m, 2H), 0.87 (t, *J* = 7.2 Hz, 3H) |
| 161 | B 15-B | 870.4 | 10.53 (s, 1H), 10.26 - 9.97 (m, 1H), 8.82 (s, 1H), 8.01 - 7.83 (m, 1H), 7.70 (d, *J* = 8.0 Hz, 1H), 7.64 - 7.50 (m, 2H), 7.34 (d, *J* = 12.8 Hz, 1H), 7.09 (d, *J* = 7.2 Hz, 1H), 6.93 (d, *J* = 10.0 Hz, 2H), 5.75 - 5.58 (m, 1H), 5.05 (s, 1H), 4.99 (d, *J* = 10.4 Hz, 1H), 4.90 - 4.82 (m, 1H), 4.81 -4.69 (m, 1H), 4.63 - 4.49 (m, 1H), 3.94 (s, 3H), 3.89 (t, *J* = 6.8 Hz, 2H), 3.45 (d, *J* = 11.2 Hz, 2H), 3.12 (br s, 4H), 3.04 - 2.89 (m, 2H), 2.84 - 2.69 (m, 6H), 2.60 - 2.58 (m, 1 H), 2.30 (br s, 2H), 2.24 - 2.13 (m, 2H), 2.07 - 1.96 (m, 1H), 1.92 (br s, 3H), 1.77 - 1.66 (m, 2H), 1.42 - 1.28 (m, 2H), 0.87 (t, *J* = 7.2 Hz, 3H). |
| 170 | B 15-B | 850.3 | 10.29 - 10.01 (m, 1H), 8.82 (s, 1H), 7.97 - 7.86 (m, 1H), 7.77 - 7.65 (m, 1H), 7.64 - 7.53 (m, 2H), 7.46 - 7.38 (m, 1H), 7.29 - 7.15 (m, 1H), 6.93 (d, *J* = 7.6 Hz, 2H), 6.80 - 6.60 (m, 2H), 6.42 - 6.24 (m, 2H), 5.73 - 5.60 (m, 1H), 5.37 - 5.27 (m, 1H), 5.12 - 5.05 (m, 1H), 5.04 - 4.96 (m, 1H), 4.92 - 4.82 (m, 1H), 4.80 - 4.68 (m, 1H), 4.65 - 4.54 (m, 1H), 4.50 - 4.38 (m, 1H), 3.92 (br s, 2H), 3.84 (s, 3H), 3.82 - 3.79 (m, 2H), 3.10 (br s, 4H), 3.03 - 2.91 (m, 1H), 2.84 - 2.74 (m, 2H), 2.41 (br s, 4H), 2.23 - 2.15 (m, 1H), 2.07 (br s, 2H), 2.04 - 2.02 (m, 1H), 1.91 - 1.86 (m, 1H), 1.75 - 1.70 (m, 1H), 1.29 (br s, 2H), 0.87 (br s, 3H). |
| 174 | B 15-B | 866.3 | 10.51 (s, 1H), 10.13 (s, 1H), 8.82 (s, 1H), 7.94 (s, 1H), 7.74 - 7.65 (m, 1H), 7.58 (s, 2H), 7.47 - 7.35 (m, *J* = 8.8 Hz, 1H),6.91 (s, 3H), 6.81 (s, 1H), 5.79 - 5.57 (m, 1H), 5.06 (s, 1H), 5.02 - 4.96 (m, 1H), 4.90 - 4.81 (m, 1H), 4.76 (s, 1H), 4.58 (s, 1H), 3.88 (s, 3H), 3.80 (s, 2H), 2.95 (s, 4H), 2.84 - 2.71 (m, 7H), 2.61 - 2.55 (m, 2H), 2.28 (s, 1H), 2.20 (s, 2H), 2.05 - 1.98 (m, 2H), 1.90 (s, 2H), 1.73 (s, 4H), 1.36 - 1.24 (m, 2H), 1.09 -1.02 (m, 3H), 0.90 - 0.82 (m, 3H) |
| 178 | B 15-B | 887.2 | 10.48 (s, 1H), 10.15 (br s, 1H), 8.83 (s, 1H), 7.96 (br s, 1H), 7.70 (d, *J* = 8.0 Hz, 1H), 7.60 (br s, 2H), 7.42 (d, *J* = 8.0 Hz, 1H), 6.97 (d, *J* = 8.0 Hz, 2H), 6.40 - 6.28 (m, 2H), 5.74 - 5.60 (m, 1H), 5.05 (s, 1H), 5.00 (d, *J* = 8.0 Hz, 1H), 4.86 (d, *J* = 16.0 Hz, 1H), 4.74 (br s, 1H), 4.57 (d, *J* = 8.0 Hz, 1H), 3.97 (s, 4H), 3.90 - 3.85 (m, 2H), 3.84 (s, 3H), 3.54 - 3.42 (m, 6H), 3.08 (br s, 1H), 3.03 - 2.93 (m, 1H), 2.85 - 2.78 (m, 1H), 2.76 - 2.69 (m, 4H), 2.26 - 2.15 (m, 1H), 2.08 - 1.96 (m, 1H), 1.96 - 1.79 (m, 2H), 1.75 - 1.61 (m, 2H), 0.91 - 0.84 (m, 3H) |

(continued)

| Compound No. | Intermediate No. | MS(ESI$^+$): m/z [M+H]$^+$ | $^1$H NMR (400 MHz, DMSO-$d_6$) $\delta$ |
|---|---|---|---|
| 194 | B 15-B | 871.2 | 10.53 (s, 1H), 10.14 (br s, 1H), 8.82 (s, 1H), 8.42 - 8.26 (m, 1H), 8.16 - 7.76 (m, 2H), 7.70 - 7.52 (m, 1H), 7.33 (d, *J* = 13.2 Hz, 1H), 7.08 (d, *J* = 7.2 Hz, 1H), 6.86 (d, *J*= 8.0 Hz, 1H), 5.66 (d, *J*= 6.4 Hz, 1H), 5.07 (s, 1H), 4.99 (d, *J* = 9.6 Hz, 1H), 4.85 (d, *J* = 16.8 Hz, 1H), 4.78 - 4.65 (m, 1H), 4.64 - 4.46 (m, 1H), 3.94 (s, 3H), 3.89 (br s, 2H), 3.44 - 3.44 (m, 2H), 3.31 - 3.30 (m, 4H), 2.98 - 2.92 (m, 1H), 2.79 (br s, 1H), 2.74 (t, *J*=6.8 Hz, 4H), 2.48 - 2.41 (m, 4H), 2.30 - 2.23 (m, 2H), 2.22 - 2.12 (m, 1H), 2.07 - 1.95 (m, 1H), 1.93 - 1.82 (m, 3H), 1.80 - 1.66 (m, 2H), 1.42 - 1.30 (m, 2H), 0.86 (t, *J* = 7.2 Hz, 3H). |
| 196 | B 26-B | 856.2 | 10.53 (s, 1H), 10.14 (br s, 1H), 8.82 (s, 1H), 7.93 (br s, 1H), 7.69 (d, *J* = 8.0 Hz, 1H), 7.59 (br s, 2H), 7.34 (d, *J* = 12.8 Hz, 1H), 7.09 (d, *J* = 6.4 Hz, 1H), 6.93 (d, *J* = 8.4 Hz, 2H), 5.77 - 5.50 (m, 1H), 5.19 (s, 1H), 4.99 (d, *J* = 10.4 Hz, 1H), 4.86 (*d, J* = 17.2 Hz, 1H), 4.72 (br s, 1H), 4.63 (br s, 1H), 3.94 (s, 3H), 3.89 (t, *J* = 6.8 Hz, 2H), 3.49 - 3.41 (m, 2H), 3.12 (br s, 4H), 3.02 - 2.93 (m, 1H), 2.84 - 2.78 (m, 1H), 2.74 (t, *J* = 6.8 Hz, 4H), 2.57 - 2.53 (m, 4H), 2.27 (d, *J* = 6.8 Hz, 2H), 2.13 (t, *J* = 7.2 Hz, 2H), 1.86 (d, *J* = 11.2 Hz, 2H), 1.78 - 1.67 (m, 1H), 1.45 (s, 3H), 1.40 - 1.30 (m, 2H). |
| 197 | B 26-B | 857.2 | 10.54 (br s, 1H), 10.16 (br s, 1H), 8.83 (s, 1H), 8.35 (br s, 1H), 7.97 (br s, 1H), 7.87 (d, *J* = 9.2 Hz, 1H), 7.64 (br s, 1H), 7.33 (d, *J* = 12.8 Hz, 1H), 7.15 - 7.00 (m, 1H), 6.87 (br s, 1H), 5.76 - 5.55 (m, 1H), 5.21 (s, 1H), 4.99 (d, *J* = 9.2 Hz, 1H), 4.85 (d, *J* = 16.8 Hz, 1H), 4.78 - 4.53 (m, 2H), 3.94 (s, 3H), 3.89 (s, 2H), 3.31 (br s, 4H), 3.11 - 2.90 (m, 1H), 2.86 - 2.67 (m, 6H), 2.62 - 2.51 (m, 4H), 2.45 - 2.37 (m, 1H), 2.26 (br s, 2H), 2.13 (br s, 2H), 1.87 (d, *J* = 11.2 Hz, 2H), 1.74 (br s, 1H), 1.45 (br s, 3H), 1.40 - 1.29 (m, 2H). |
| 198 | B 15-B | 853.8 | 10.57 (s, 1H), 10.13 (s, 1H), 8.82 (s, 1H), 7.92 (s, 1H), 7.70 (d, *J* = 8.0 Hz, 1H), 7.58 (s, 2H), 7.34 (d, *J* = 8.4 Hz, 1H), 6.93 (d, *J* = 8.0 Hz, 3H), 5.74 - 5.60 (m, 1H),5.06 (s, 1H), 5.00 (d, *J* = 9.6 Hz,1H), 4.85 (d, *J* = 17.6 Hz, 1H), 4.75 (s, 1H), 4.57 (d, *J* = 10.0 Hz,1H), 4.05 (s, 3H), 3.91 - 3.88 (m, 2H), 3.11 (s, 4H), 3.00 - 2.94 (m, 1H), 2.88 - 2.77 (m, 4H), 2.75(s, 2H), 2.59 - 2.53 (m, 4H), 2.25 (s, 2H), 2.22 - 2.15 (m, 1H), 2.02 (d, *J* = 5.2 Hz, 1H), 1.93 -1.79 (m, 4H), 1.77 - 1.66 (m, 2H), 1.32 (d, *J* = 11.2 Hz, 2H), 0.87 (t, *J* = 7.2 Hz, 3H) |
| 200 | B 26-B | 856.7 | 10.57 (s, 1H), 10.11 (br s, 1H), 8.82 (s, 1H), 7.92 (br s, 1H), 7.69 (d, *J* = 8.0 Hz, 1H), 7.58 (br s, 2H), 7.33 (d, *J* = 8.8 Hz, 1H), 6.97 - 6.86 (m, 3H), 5.73 - 5.60 (m, 1H), 5.20 (s, 1H), 4.99 (d, *J* = 10.0 Hz, 1H), 4.85 (d, *J* = 16.8 Hz, 1H), 4.71 (br s, 1H), 4.61 (br s, 1H), 4.05 (s, 3H), 3.89 (t, *J*=6.4 Hz, 4H), 3.11 (br s, 4H), 3.02 - 2.93 (m, 1H), 2.85 - 2.78 (m, 3H), 2.74 (t, *J*=6.4 Hz, 2H), 2.57 - 2.52 (m, 4H), 2.26 (br s, 2H), 2.19 - 2.09 (m, 2H), 1.84 (d, *J* = 10.8 Hz, 2H), 1.74 (br s, 1H), 1.45 (s, 3H), 1.39 - 1.29 (m, 2H). |

(continued)

| Compound No. | Intermediate No. | MS(ESI$^+$): m/z [M+H]$^+$ | $^1$H NMR (400 MHz, DMSO-$d_6$) $\delta$ |
|---|---|---|---|
| 201 | B 15-B | 870.3 | 10.53 (s, 1H), 10.11 (s, 1H), 8.82 (s, 1H), 7.92 (s, 1H), 7.69 (d, *J* = 7.6 Hz, 1H), 7.57 (s, 2H), 7.36 (d, *J* = 12.4 Hz, 1H), 7.11 (d, *J* = 6.8 Hz, 1H), 6.92 (d, *J* = 8.8 Hz,2H), 5.71 - 5.61 (m, 1H), 5.06 (s, 1H), 4.99 (d, *J* = 9.6 Hz, 1H), 4.85 (d, *J* = 17.6 Hz, 1H), 4.74 (s, 1H), 4.58 (s, 1H), 3.94 (s, 3H), 3.89 (t, *J* = 6.5 Hz, 2H), 3.65 (d, *J* = 9.6 Hz, 2H), 3.09 (s, 4H), 3.01 - 2.92 (m, 1H), 2.80 (s, 1H), 2.74 (t, *J* = 6.4 Hz, 2H), 2.63 (s, 2H), 2.57 (s, 4H), 2.26(d, *J* = 5.6 Hz, 2H), 2.19 (d, *J* = 7.2 Hz, 1H), 2.02 (d, *J* = 5.2 Hz, 1H), 1.91 - 1.79 (m, 3H), 1.75 - 1.66(m, 2H), 1.29 - 1.24 (m, 2H), 0.87 (t, *J* = 7.2 Hz, 3H). |
| 202 | B 15-B | 811.3 | 10.58 (s, 1H), 10.27 (s, 1H), 9.96 (s, 1H), 8.87 (s, 1H), 7.92 (m, 1H), 7.75 - 7.64 (m, 3H), 7.51 (m, 1H), 7.34(m, 1H), 7.24 (m, 2H), 7.14 - 7.03 (m, 1H), 5.75 - 5.58 (m, 1H), 5.32 (s, 1H), 5.07 (s, 1H), 5.00 (m, 1H), 4.85 (m, 1H),4.80 - 4.70 (m, 1H), 4.62 - 4.51 (m, 1H), 4.16 (s, 3H), 3.94 - 3.86 (m, 2H), 3.08 (m, 2H), 2.97 (m, 1H), 2.78 - 2.75 (m, 2H), 2.33 (br s, 2H),2.20 (br s, 1H), 2.03 - 1.98 (m, 3H), 1.89 - 1.77 (m, 5H), 1.70 (m, 1H), 1.45 (br s, 1H), 0.87 (br s, 3H) |

**Example 116: synthesis of compound 116**

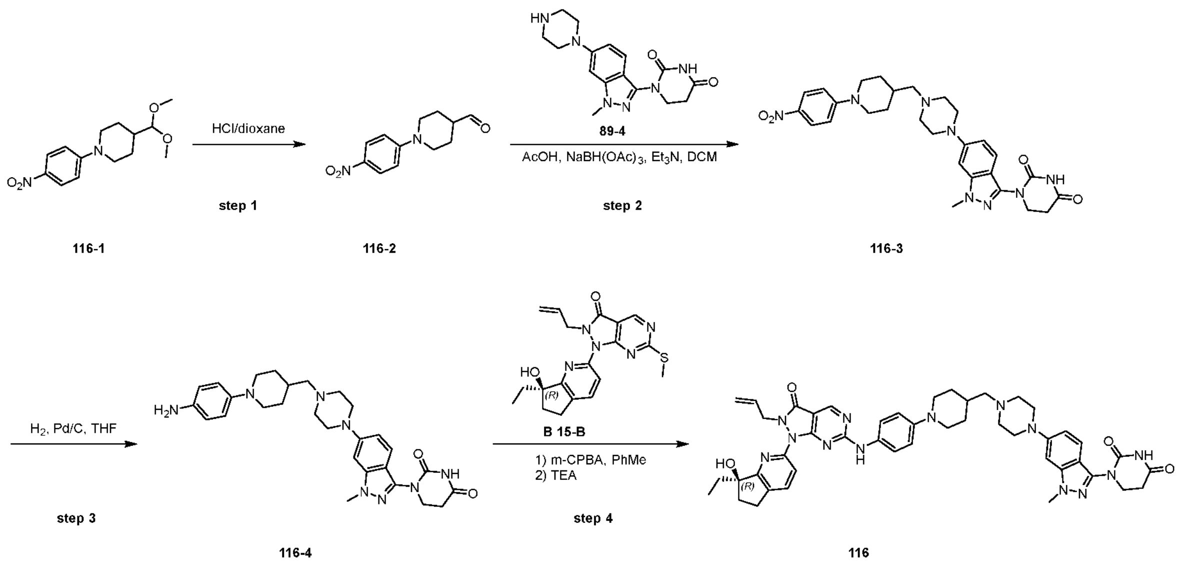


**Step 1: synthesis of intermediate 116-2**

To a solution of **intermediate 116-1** (1.36 g, 4.85 mmol) in DCM (15 mL) was added HCl/dioxane (4 M, 20 mL). The mixture was stirred at 20 °C for 16 hrs . The reaction mixture was concentrated under reduced pressure to give a crude of **intermediate 116-2** (1.5 g ) as a yellow solid which was used into the next step directly.

LCMS (ESI$^+$): m/z 234.9 [M+H]$^+$.

**Step 2: synthesis of intermediate 116-3**

A mixture of **intermediate 89-4** (200 mg, 548 μmol) , intermediate **116-2** (222 mg, 822 μmol) in DCM (5 mL) was added TEA (277 mg, 2.74 mmol) and HOAc (165 mg, 2.74 mmol). After addition, the mixture was stirred at this

temperature for 30 min. Then $NaBH(OAc)_3$ (349 mg, 1.64 mmol) was added and the resulting mixture was stirred at 20°C for 16 hrs. The reaction mixture was diluted with $H_2O$ (50 mL) and extracted with EA (50 mL × 2). The combined organic layers were dried over $Na_2SO_4$, filtered and concentrated under reduced pressure to give a residue. The residue was purified by prep-TLC ($SiO_2$, DCM: MeOH = 10: 1) to give the **intermediate 116-3** (150 mg, 48.5% yield) as a yellow solid.

LCMS ($ESI^+$): m/z 547.1 $[M+H]^+$.

**Step 3: synthesis of intermediate 116-4**

A mixture of **intermediate 116-3** (150 mg, 274 μmol) , 10% Pd/C (150 mg) in THF (10 mL) was was stirred at 20 °C for 16 hrs under $H_2$ atmosphere (15 Psi). The reaction mixture was filtered and concentrated under reduced pressure to give a crude of **intermediate 116-4** (130 mg, crude) as a white solid which was used into the nexst step directly.

LCMS ($ESI^+$): m/z 517.1 $[M+H]^+$.

**Step 4: synthesis of compound 116**

To a colorless solution of **intermediate B15-B** (50.0 mg, 130 μmol) in toluene (10 mL) was added m-CPBA (29 mg, 143 μmol) and stirred at 20°C for 30 min. Then $Et_3N$ (26.4 mg, 261 μmol) and **intermediate 116-4** (67.3 mg, 130 μmol) was added. The reaction mixture was stirred at 20°C for 16 hours. The reaction mixture was filtered and concentrated under reduced pressure to give a residue. The residue was purified by prep-TLC ($SiO_2$, DCM: MeOH = 10:1) To give the **compound 116** (23 mg, 14.5% yield) as a yellow solid.

LCMS ($ESI^+$): m/z 852.5 $[M+H]^+$.

$^1H$ NMR (400MHz, DMSO-$d_6$) δ 10.52 (s, 1H), 10.13 (br s, 1H), 8.82 (s, 1H), 7.99 - 7.85 (m, 1H), 7.69 (d, *J* = 8.0 Hz, 1H), 7.57 (br s, 2H), 7.46 (d, *J* = 8.0 Hz, 1H), 6.93 (d, *J* = 12.0 Hz, 3H), 6.83 (s, 1H), 5.74 - 5.58 (m, 1H), 5.06 (s, 1H), 4.99 (d, *J* = 12.0 Hz, 1H), 4.85 (d, *J* = 16.0 Hz, 1H), 4.75 (s, 1H), 4.56 (d, *J* = 12.0 Hz, 1H), 3.95 - 3.84 (m, 5H), 3.65 (d, *J* = 12.0 Hz, 2H), 3.28 - 3.19 (m, 4H), 3.03 - 2.91 (m, 1H), 2.84 - 2.76 (m, 1H), 2.75 - 2.70 (m, 2H), 2.65 - 2.58 (m, 2H), 2.54 (br s, 4H), 2.30 - 2.15 (m, 3H), 2.07 - 1.96 (m, 1H), 1.94 - 1.78 (m, 3H), 1.77 - 1.62 (m, 2H), 1.25 (d, *J*= 16.0 Hz, 2H), 0.91 - 0.82 (m, 3H).

The **compound 126, 127** and **compound 135** were synthesized according to the procedures described for **compound 116** using the different intermediates.

| Compound No. | Intermediate No. | MS ($ESI^+$): m/z $[M+H]^+$ | $^1H$ NMR (400 MHz, DMSO-$d_6$) δ |
|---|---|---|---|
| 126 | B 26-A | 838.5 | 10.52 (s, 1H), 10.14 (s, 1H), 8.82 (s, 1H), 7.91 (d, *J* = 18.80 Hz, 1H), 7.69 (d, *J* = 8.40 Hz, 1H), 7.57 (s, 1H), 7.46 (d, *J* = 8.40 Hz, 1H), 6.93 (d, *J* = 8.80 Hz, 4H), 6.83 (s, 1H), 5.61 - 5.76 (m, 1H), 5.19 (s, 1H) 4.99 (d, *J* = 10.80 Hz, 1H), 4.86 (d, *J* = 16.40 Hz, 1H), 4.73 (s, 1H), 4.62 (s, 1H), 3.89 (s, 5H), 3.63 (s, 2H), 3.63 - 3.69 (m, 2H), 3.25 (d, *J* = 5.20 Hz, 4H), 2.93 - 3.02 (m, 1H), 2.76 - 2.77 (m, 1H), 2.73 (t, *J* = 6.42 Hz, 3H), 2.59 - 2.65 (m, 2H), 2.53 - 2.58 (m, 4H), 2.24 (s, 2H), 2.13 (t, *J* = 6.70 Hz, 2H), 1.95 - 2.03 (m, 1H) 1.83 (d, *J* = 12.00 Hz, 2H), 1.72 (s, 1H), 1.45 (s, 3H), 1.26 - 1.30 (m, 2H) |
| 127 | B 26-B | 838.5 | 10.13 (br s, 1H), 8.82 (s, 1H), 7.93 (br s, 1H), 7.69 (d, *J* = 8.0 Hz, 1H), 7.57 (br s, 1H), 7.49 - 7.42 (m, 1H), 7.10 - 6.86 (m, 4H), 6.83 (br s, 1H), 5.73 - 5.62(m, 1H), 5.19 (s, 1H), 5.04 - 4.95 (m, 1H), 4.91 - 4.81 (m, 1H), 4.78 - 4.56 (m, 2H), 3.92 - 3.88 (m,5H), 3.69 - 3.60 (m, 2H), 3.24 (br s, 4H), 3.04 - 2.92 (m, 1H), 2.86 - 2.77 (m, 1H), 2.76 - 2.71 (m,2H), 2.65 - 2.59 (m, 2H), 2.55 (br s, 4H), 2.25 (br s, 2H), 2.17 - 2.08 (m, 2H), 1.88 - 1.78 (m, 2H),1.72 (br s, 1H), 1.45 (s, 2H), 1.43 (s, 1H), 1.26 (br s, 2H) |

(continued)

| Compound No. | Intermediate No. | MS (ESI+): m/z $[M+H]^+$ | $^1H$ NMR (400 MHz, DMSO-$d_6$) $\delta$ |
|---|---|---|---|
| 135 | B 15-B | 866.5 | 10.52 (s, 1H), 10.16 (br s, 1H), 8.84 (s, 1H), 7.90 (d, *J* = 8.0 Hz,1H), 7.72 (d, *J* = 8.0 Hz, 2H), 7.49 - 7.38 (m, 2H), 7.01 - 6.89 (m, 2H), 6.83 (s, 1H), 5.74 - 5.59 (m,1H), 5.07 (s, 1H), 5.00 (d, *J* = 12.0 Hz, 1H), 4.90 - 4.67 (m, 2H), 4.57 (d, *J* = 16.0 Hz, 1H), 3.91 -3.87 (m, 5H), 3.24 (br s, 4H), 3.02 (d, *J* = 12.0 Hz, 3H), 2.84 - 2.69 (m, 3H), 2.57 - 2.54 (m, 6H),2.28 (br d, *J* = 8.0 Hz, 2H), 2.25 - 2.21 (m, 3H), 2.20 (d, *J* = 4.0 Hz, 1H), 2.06 - 1.98 (m, 1H), 1.93 -1.80 (m, 3H), 1.74 - 1.62 (m, 2H), 1.30 (d, *J* = 8.0 Hz, 2H), 0.89 - 0.84 (m, 3H) |

**Example 139: synthesis of compound 139**

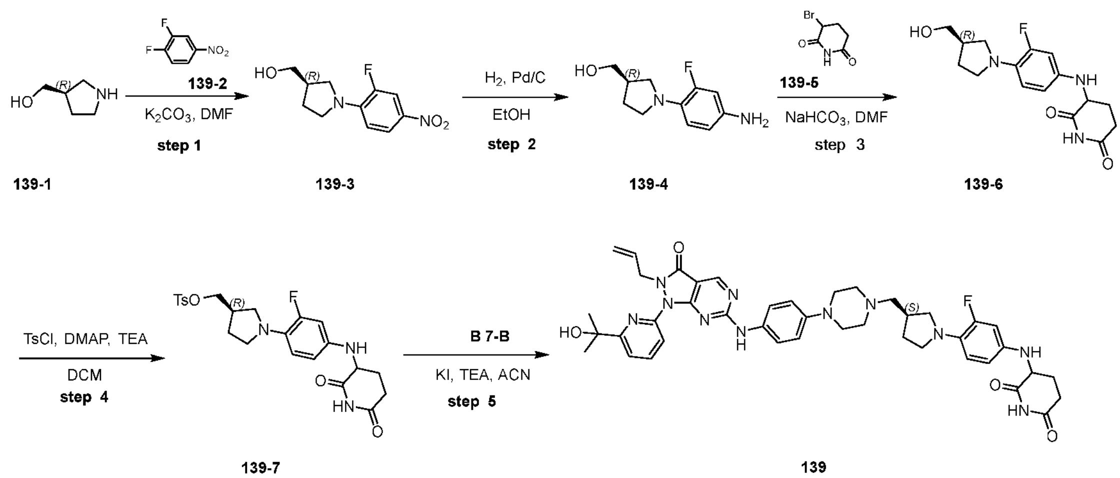


**Step 1: synthesis of intermediate 139-3**

To a solution of **intermediate 139-1** (2.00 g, 19.8 mmol) and **intermediate 139-2** (3.15 g, 19.8 mmol) in DMF (10 mL) was added $K_2CO_3$ (5.47 g, 39.6 mmol). The mixture was stirred at 70 °C for 2 hrs. The reaction mixture was poured into water (20 mL) at 25 °C, and then extracted with EtOAc (20 mL × 3). The combined organic layers were dried over $Na_2SO_4$, filtered and concentrated under reduced pressure to give a residue. The residue was purified by flash silica gel chromatography (Ethyl acetate/Petroleum = 0/1 to 1/1) to give the **intermediate 139-3** (4.5 g, 94.7% yield) as a yellow oil.

$^1H$ NMR (400MHz, DMSO-$d_6$) $\delta$ 7.94 - 7.86 (m, 2H), 6.83 - 6.64 (m, 1H), 4.80 (t, *J*= 5.2 Hz, 1H), 3.68 - 3.44 (m, 4H), 3.39 - 3.32 (m, 2H), 2.46 - 2.31 (m, 1H), 2.09 - 1.99 (m, 1H), 1.79 - 1.66 (m, 1H).

**Step 2: synthesis of intermediate 139-4**

To a mixture of **intermediate 139-3** (4.5 g, 18.73 mmol) in EtOH (50 mL) was added Pd/C (450 mg, 10% purity). The suspension was degassed under vacuum and purged with $H_2$ three times. The mixture was stirred under $H_2$ (15 psi) at 25°C for 1 hour. The reaction mixture was filtered and concentrated under reduced pressure to give a crude of **intermediate 139-4** (4.50 g) as a white solid.

**Step 3: synthesis of intermediate 139-6**

To a solution of **intermediate 139-4** (1.00 g, 4.76 mmol) and **intermediate 139-5** (1.83 g, 9.51 mmol) in DMF

(10 mL) was added $NaHCO_3$ (1.20 g, 14.3 mmol). The mixture was stirred at 80 °C for 8 hrs. The mixture were poured into water (60 mL), extracted with EA (40 mL × 3), dried over $Na_2SO_4$, filtered and concentrated under reduced pressure to give a residue. The residue was purified by flash silica gel chromatography (ethyl acetate/petroleum= 0/1 to 3/1) to give the **intermediate 139-6** (800 mg, 52.3% yield) as a purple solid.

$^1$H NMR (400MHz, DMSO-$d_6$) $\delta$ 10.95 - 10.70 (m, 1H), 6.61 (t, *J* = 9.6 Hz, 1H), 6.51 (dd, *J* = 2.4, 15.6 Hz, 1H), 6.40 (dd, *J* = 2.0, 8.4 Hz, 1H), 5.57 (d, *J* = 7.6 Hz, 1H), 4.66 (t, *J* = 5.2 Hz, 1H), 4.30 - 4.14 (m, 1H), 3.44 - 3.37 (m, 2H), 3.20 - 3.02 (m, 3H), 2.97 - 2.90 (m, 1H), 2.73 - 2.65 (m, 1H), 2.61 - 2.54 (m, 1H), 2.38 - 2.26 (m, 1H), 2.15 - 2.05 (m, 1H), 1.98 - 1.74 (m, 2H), 1.64 - 1.49 (m, 1H).

**Step 4: synthesis of intermediate 139-7**

To a solution of **intermediate 139-6** (400 mg, 1.24 mmol) in DCM (5 mL) was added TEA (315 mg, 3.11 mmol), DMAP (15.2 mg, 124 μmol) and 4-methylbenzenesulfonyl chloride (119 mg, 622 μmol). The mixture was stirred at 25 °C for 4 hrs. The reaction mixture was diluted with DCM (20 mL) and washed with water (20 mL * 2), The organic layer was dried over $Na_2SO_4$ and filtered, concentrated under reduce pressure to give a residue. The residue was purified by flash silica gel chromatography (ethyl acetate/petroleum= 0/1 to 1/1) to give the **intermediate 139-7** (300 mg, 50.68% yield) as a white solid.

MS (ESI$^+$): m/z 475.6 [M+H]$^+$.

**Step 5: synthesis of compound 139**

To a solution of **intermediate 139-7** (200 mg, 420.58 μmol) and **intermediate B7-B** (205 mg, 391 μmol, HCl salt) in $CH_3CN$ (6 mL) was added KI (140 mg, 841 μmol) and TEA (213 mg, 2.10 mmol). The mixture was stirred at 90°C for 2 hrs. The mixture was concentrated under reduced pressure to give a residue. The residue was purified by flash silica gel chromatography (DCM /MeOH = 1/0 to 15/1) and the crude was further purified by prep-HPLC (Calumn:Kromasil 100-5-C18 30*150 mm, Mobile Phase A: water (0.01% FA), Mobile Phase B: acetonitrile, 20 mL/min, gradient condition form 15% B to 55%) to give compound 139 (30.1 mg, 8.88% yield) as a white solid.

MS (ESI$^+$): m/z 790.5 [M+H]$^+$.

$^1$H NMR (400MHz, DMSO-$d_6$) $\delta$10.78 (s, 1H), 10.31 - 9.98 (m, 1H), 8.83 (s, 1H), 8.13 - 7.99 (m, 1H), 7.82 - 7.71 (m, 1H), 7.66 - 7.49 (m, 3H), 6.98 - 6.86 (m, 2H), 6.67 - 6.57 (m, 1H), 6.55 - 6.47 (m, 1H), 6.42 - 6.35 (m, 1H), 5.72 - 5.60 (m, 1H), 5.59 - 5.54 (m, 1H), 5.40 - 5.28 (m, 1H), 5.03 - 4.95 (m, 1H), 4.86 - 4.77 (m, 1H), 4.73 - 4.63 (m, 2H), 4.26 - 4.16 (m, 1H), 3.23 - 3.21 (m, 1H), 3.15 - 3.08 (m, 6H), 2.95 - 2.89 (m, 1H), 2.76 - 2.67 (m, 1H), 2.61 - 2.52 (m, 5H), 2.44 - 2.32 (m, 3H), 2.13 - 1.98 (m, 2H), 1.88 - 1.77 (m, 1H), 1.65 - 1.53 (m, 1H), 1.46 (s, 6H).

**Example 141: synthesis of compound 141**

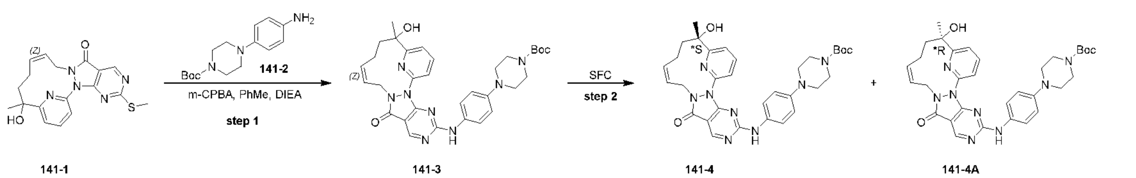


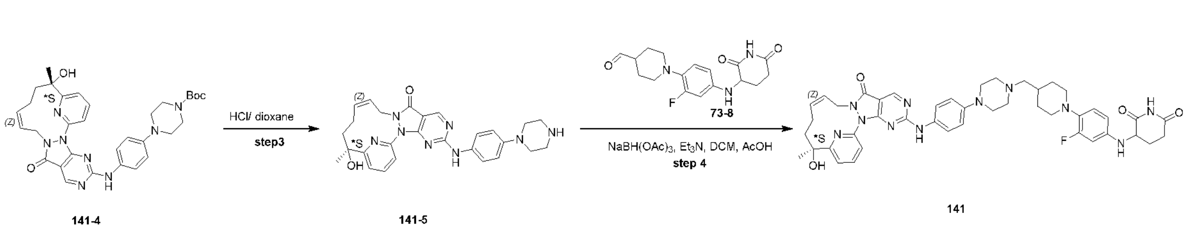


**Step 1: synthesis of intermediate 141-3**

To a solution of **intermediate 141-1** (100 mg, 271 μmol) in toluene (4 mL) was added m-CPBA (60.5 mg, 298 μmol, 85% purity) and the reaction stirred at 20 °C for 0.5 hr. Then DIEA (105 mg,812 μmol) and **intermediate 141-2** (82.6 mg, 298 μmol) was added. The mixture was stirred at 20 °C for 16 hrs. The reaction mixture was quenched by

addition $Na_2S_2O_3$ (20 mL) and $NaHCO_3$ (20 mL) and extracted with EA (10 mL × 3). The combined organic layers were washed with water 30 mL (10 mL × 3), dried over $Na_2SO_4$, filtered and concentrated under reduced pressure to give a residue. The residue was purified by column chromatography ($SiO_2$, DCM: MeOH =10: 1) to give the **intermediate 141-3** (120 mg, 74.1% yield) as a yellow solid, which was used into the next step. LCMS (ESI$^+$): m/z 598.69 [M+H]$^+$

**Step 2: synthesis of intermediate 141-4**

Diastereoisomers of compound **141-3** (95 mg, 158.7 μmol) was separated by SFC (column: DAICEL CHIRALPAK IF (250mm*30mm,10μM); mobile phase: [IPA-ACN];B%: 50%-50%, 45 mL/min). Peak 1 (Rt = 1.13 min) as **compound 141-4** (40 mg, 42.11% yield) was obtained as a pale yellow solid and Peak 2 (Rt = 2.01 min) as **compound 141-4A** (40 mg, 42.11% yield) was obtained as a pale yellow solid. The absolute stereochemistry was arbitrarily assigned for **compound 141-4** and **compound 141-4A**.

**Step 3: synthesis of intermediate 141-5**

To a solution of compound **141-4** (40.0 mg, 66.8 μmol) in DCM (7 mL) was added HCl/dioxane (4 M, 2 mL). The mixture was stirred at 25°C for 16 hrs. The reaction mixture was filtered and concentrated under reduced pressure to give a crude of compound 141-5 (40 mg, crude) as a green solid which was used into the next step directly. LCMS (ESI$^+$): m/z 498.58 [M+H]$^+$

**Step 4: synthesis of compound 141**

To a mixture of **compound 141-5** (40.0 mg, 80.2 μmol) and **intermediate 73-8** (26.7 mg, 80.2 μmol) in DCM (4 mL) was added TEA(56.8 mg, 562 μmol) at 20°C and stirred at 20 °C for 30 min, then added AcOH (14.5 mg, 241 μmol) and stirred for 1 hr. The reaction was added $NaBH(OAc)_3$ (51.0 mg, 241 μmol) stirred at 25 °C for 16 hours. The reaction mixture was partitioned between water (20 mL) and DCM (40 mL). The organic phase was separated dried over $Na_2SO_4$, filtered and concentrated under reduced pressure to give a residue. The residue was purified by prep-HPLC (acetonitrile/water (2%FA)) and lyophilized to give **compound 141** (30 mg, 45.8% yield) as a white solid.

LCMS (ESI$^+$): m/z 815.94 [M+H]$^+$.

$^1$H NMR (400MHz, DMSO-$d_6$) δ 10.78 (s, 1H), 10.07 (br s, 1H), 8.82 (s, 1H), 8.18 (br s, 0.531H),8.11 - 7.92 (m, 1H), 7.75 (d, *J* = 8 Hz, 1H), 7.57 (t, *J* = 7.2 Hz, 3H), 6.94 - 6.78 (m, 3H), 6.54 -6.37 (m, 2H), 5.78 (d, *J* = 7.6 Hz, 1H), 5.31 (br s, 3H), 4.45 (d, *J* = 4.5 Hz, 2H), 4.29 - 4.19 (m, 1H),3.13 (br s, 1H), 3.09 (br s, 4H), 2.79 - 2.70 (m, 1H), 2.56 (d, *J* = 12.4 Hz, 3H), 2.50 - 2.47 (m, 4H),2.22 (d, *J* = 6.7 Hz, 3H), 2.12 - 1.98 (m, 2H), 1.98 - 1.82 (m, 2H), 1.81 - 1.67 (m, 4H), 1.62 (br s,1H), 1.55 (s, 3H), 1.32 - 1.17 (m, 2H).

The compound **142** was synthesized according to the procedures described for compound **141** using the different intermediates.

| Compound No. | Intermediate No. | MS(ESI$^+$): m/z [M+H]$^+$ | $^1$H NMR (400 MHz, DMSO-$d_6$) δ |
|---|---|---|---|
| 142 | 141-4A | 816.5 | 10.78 (s, 1H), 10.07 (br s, 1H), 8.82 (s, 1H), 8.22 (s, 1.806 H), 8.06 - 7.98 (m, 1H), 7.76 (d, *J* = 8.0 Hz, 1H), 7.62 - 7.53 (m, 3H), 6.95 - 6.88 (m, 2H), 6.86 - 6.79 (m, 1H), 6.53 - 6.47 (m, 1H), 6.43 - 6.38 (m, 1H), 5.83 - 5.75 (m, 1H), 5.33 (br s, 3H), 4.54 - 4.38 (m, 2H), 4.30 - 4.21 (m, 1H), 3.13 (br s, 2H), 3.09 (br s, 4H), 2.78 - 2.71 (m, 1H), 2.60 - 2.54 (m, 3H), 2.50 - 2.45 (m, 4H), 2.27 - 2.16 (m, 3H), 2.12 - 2.03 (m, 2H), 1.98 - 1.84 (m, 2H), 1.80 - 1.72 (m, 3H), 1.62 (br s, 1H), 1.56 (s, 3H), 1.31 - 1.19 (m, 2H) |

**Example 146: synthesis of compound 146**

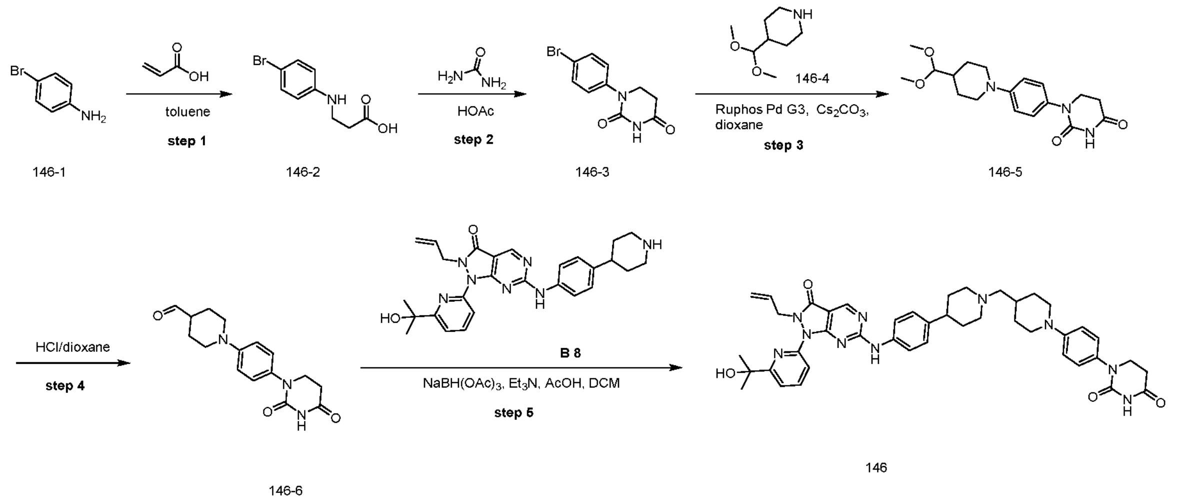


**Step 1: synthesis of intermediate 146-2**

A solution of 4-bromoaniline **146-1** (10 g, 58.1 mmol) and acrylic acid (6.28 g, 87.2 mmol, 6.0 mL) in toluene (100 mL) was stirred at 100 °C for 8 hrs. The reaction mixture was concentrated under reduced pressure to give a crude of **intermediate 146-2** (15 g, crude) as black oil.

**Step 2: synthesis of intermediate 146-3**

A solution of intermediate **146-2** (14.5 g, 59.4 mmol) and urea (21.4 g, 356 mmol) in AcOH (100 mL) was stirred at 120 °C for 16 hrs. The reaction mixture was concentrated under reduced pressure to remove AcOH. The residue was diluted with $NaHCO_3$ (100 mL) and extracted with EA (100 mL × 3). The combined organic layers were dried over $Na_2SO_4$, filtered and concentrated under reduced pressure to give a residue. The crude was purified by chromatography on silca gel (petroleum / EA = 1/0 to 3/1) to give the **intermediate 146-3** (3.69 g, 12.34 mmol, 20.8% yield) as a white solid.
$^1$HNMR (400MHz, DMSO-$d_6$) δ 10.63 - 10.31 (m, 1 H), 7.67 - 7.47 (m, 2 H), 7.35-7.25 (m, 2 H), 5.91 (s, 1 H), 3.79 (t, *J* = 6.8 Hz, 2 H), 2.71 (t, *J* = 6.8 Hz, 2 H).

**Step 3: synthesis of intermediate 146-5**

A mixture of **intermediate 146-3** (500 mg, 1.86 mmol) , **intermediate 146-4** (295 mg, 1.86 mmol), RuPhos Pd G3 (155 mg, 185 μmol), $Cs_2CO_3$ (1.82 g, 5.57 mmol) in dioxane (10 mL) was stirred at 100 °C for 2 hrs under $N_2$ atmosphere. The reaction mixture was filtered and concentrated under reduced pressure to give a residue. The residue was purified by column chromatography ($SiO_2$, DCM/MeOH = 10/1) to give the **intermediate 146-5** (34 mg, 4.21% yield) as a red solid.
LCMS (ESI$^+$): m/z 185.7 $[M+H]^+$.

**Step 4: synthesis of intermediate 146-6**

To a solution of **intermediate 146-5** (30 mg, 86.3 μmol) in DCM (1 mL) was added HCl/dioxane (5 mL) The mixture was stirred at 20 °C for 1 hr. The reaction mixture was concentrated under reduced pressure to give a crude of **intermediate 146-6** (45 mg, crude) as white solid which was used into the next step directly.
LCMS (ESI$^+$): m/z 320.4 $[M+H]^+$.

**Step 5: synthesis of compound 146**

To a solution of **intermediate 146-6** (45 mg,149 μmol) and **intermediate B 8** (72.5 mg, 149 μmol) in DCM (5 mL) was added TEA (75.6 mg, 747 μmol, 104 μL) and the miture was stirred at 25°C for 0.5 hour. Then AcOH (44.8 mg, 747 μmol, 43 μL) was added and stirred at 25°C for another 0.5 hour. Then the obtained mixture was added $NaBH(OAc)_3$ (95.0 mg, 448 μmol) and stirred at 25 °C for 16 hrs . The reaction mixture was diluted with DCM (10 mL) and water (10 mL), and the organic layer was dried over Na2SO4, filtered and concentrated to give to give a residue, which was purified by prep-HPLC to give the **compound 146** (20 mg, 16.0% yield) as a yellow solid.
LCMS (ESI$^+$): m/z 793.5 $[M+Na]^+$.

$^1$H NMR (400MHz, DMSO-$d_6$) δ 10.28 (s, 1 H), 8.88 (s, 1 H), 8.14 (s, 0.672H), 8.06 (t, *J* = 7.60 Hz, 1 H), 7.59 - 7.81 (m, 4 H), 7.22 (d, *J* = 8.80 Hz, 2 H), 7.13 (d, *J* = 8.80 Hz, 2 H), 6.93 (d, *J* = 8.80 Hz, 2 H), 5.60 - 5.74 (m, 1 H), 5.36 (s, 1 H), 4.99 (d, *J* = 10.80 Hz, 1 H), 4.82 (d, *J* = 17.20 Hz, 1 H), 4.68 (d, *J* = 5.60 Hz, 2 H), 3.64 - 3.74 (m, 4 H), 3.46 (s, 4 H), 3.28 (s, 1 H), 3.03 (d, *J* = 9.20 Hz, 2 H), 2.66 (d, *J* = 3.60 Hz, 2 H), 2.42 (s, 1 H), 2.29 (s, 2 H), 2.07 (s, 2 H), 1.62 - 1.86 (m, 7 H), 1.46 (s, 6 H).

**Example 149: synthesis of compound 149**

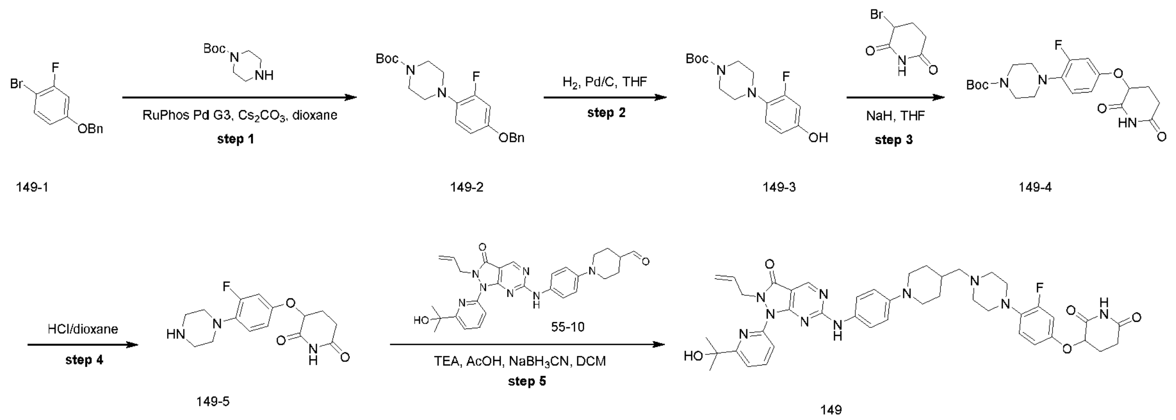


149-1 149-2 149-3 149-4 149-5 55-10 149

**Step 1: synthesis of intermediate 149-2**

A mixture of **intermediate 149-1** (3 g, 10.7 mmol) , tert-butyl piperazine-1-carboxylate (2.38 g, 12.8 mmol) , RuPhos Pd $G_3$ (446 mg, 534 μmol) , $Cs_2CO_3$ (10.4 g, 32.0 mmol) in dioxane (40 mL) was degassed and purged with $N_2$ for 3 times, and then the mixture was stirred at 100 °C for 16 hrs under $N_2$ atmosphere. The mixture was filtered and washed with DCM (80 mL). Then the filtrate was concentrated under reduced pressure to give a residue. The residue was purified by column chromatography ($SiO_2$, Petroleum ether/Ethyl acetate=1/0 to 1/1) to give the **intermediate 149-2** (1.14 g, 24.9% yield) as a pale yellow solid.

LCMS (ESI$^+$): m/z 307.3 [M+H]$^+$.

$^1$H NMR (400 MHz, CHLOROFORM-*d*) δ 7.44 - 7.37 (m, 4H), 7.36 - 7.32 (m, 1H), 6.88 (t, *J* = 9.2 Hz, 1H), 6.79 - 6.65 (m, 2H), 5.02 (s, 2H), 3.64 - 3.54 (m, 4H), 3.01 - 2.91 (m, 4H), 1.49 (s, 9H).

**Step 2: synthesis of intermediate 149-3**

To a solution of **intermediate 149-2** (1.14 g, 2.95 mmol) in THF (15 mL) and EtOH (15 mL) was added Pd/C (1 g, 10% purity) under $N_2$. The suspension was degassed under vacuum and purged with $H_2$ three times. The mixture was stirred under $H_2$ (15 PSi) at 20°C for 16 hours. The reaction mixture was filtered, washed with DCM (100 mL) and the filterate was concentrated to the crude product. The crude product was triturated with MeCN (10 mL) and filted to give a cake of **intermediate 149-3** (774 mg, 79.7% yield) as a white solid.

LCMS (ESI$^+$): m/z 297.3 [M+H]$^+$.

$^1$H NMR (400 MHz, DMSO-$d_6$) δ 9.47 (br s, 1H), 6.89 (t, *J* = 9.2 Hz, 1H), 6.61 - 6.46 (m, 2H), 3.43 (br s, 4H), 2.83 - 2.76 (m, 4H), 1.41 (s, 9H).

**Step 3: synthesis of intermediate 149-4**

A mixture of **intermediate 149-3** (200 mg, 675 μmol) in DMF (5 mL) was added NaH (67.5 mg, 1.69 mmol, 60% purity) at 0°C under $N_2$. The reaction was stirred at 0°C for 30 mins. 3-bromopiperidine-2,6-dione (194 mg, 1.01 mmol) in DMF (0.5 mL) was added to the reaction at 0°C under $N_2$. The reaction was stirred at 0°C-20°C for 16 hrs. The reaction mixture was quenched by addition $NH_4Cl$ (10 mL) at 25 °C, and then diluted with $H_2O$ (10 mL) and extracted with EA (30 mL) and extracted with EA two times. The combined organic layers were washed with sat. aq. NaCl (15 mL × 2), dried over $Na_2SO_4$, filtered and concentrated under reduced pressure to give a residue. The residue was purified by column chromatography ($SiO_2$, Petroleum ether/Ethyl acetate=1/0 to 1/1) to give the **intermediate 149-4** (160 mg, 48.9% yield) as colorless oil.

LCMS ($ESI^+$): m/z 308.1 $[M\text{-}Boc+H]^+$.

$^1H$ NMR (400 MHz, CHLOROFORM-*d*) δ 7.92 - 7.69 (m, 1H), 6.94 - 6.74 (m, 3H), 4.78 (m, 1H), 3.66 - 3.52 (m, 4H), 2.94 (br s, 5H), 2.75 - 2.63 (m, 1H), 2.38 - 2.27 (m, 2H), 1.49 (s, 9H).

**Step 4: synthesis of intermediate 149-5**

To a solution of intermediate **149-4** (160 mg, 393 μmol) in DCM (1 mL) was added HCl/dioxane (4 M, 2 mL). The mixture was stirred at 25 °C for 16 hrs. The reaction mixture was concentrated under reduced pressure to give the crude product of intermediate **149-5** (132 mg, crude, HCl salt) as a brown solid.

LCMS ($ESI^+$): m/z 308.1 $[M+H]^+$.

**Step 5: synthesis of compound 149**

To a mixture of intermediate **55-10** (144 mg, 262 μmol) and intermediate **149-5** (60 mg, 175 μmol, HCl salt) in DCM (3 mL) and DMF (1 mL) was added TEA (106 mg, 1.05 mmol) at 25°C and stirred at 25 °C for 30 min, then AcOH (99.6 mg, 1.66 mmol) was added and stirred for 16 hrs. The reaction mixture was added $NaBH(OAc)_3$ (111 mg, 524 μmol) at 25°C and stirred at 25 °C for 16 hours. The reaction mixture was diluted with $H_2O$ (10 mL) and extracted with DCM (15 mL × 3). The combined organic layers were dried over $Na_2SO_4$, filtered and concentrated under reduced pressure to give a residue. The residue was purified by prep-HPLC (acetonitrile / water (2% FA)) to give **compound 149** (53 mg, 36% yield) as a yellow solid. LCMS ($ESI^+$): m/z 805.5 $[M+H]^+$.

1H NMR (400 MHz, DMSO-$d_6$) δ 10.93 (br s, 1H), 10.26 - 10.00 (m, 1H), 8.82 (s, 1H), 8.04 (br s, 1H), 7.83 - 7.70 (m, 1H), 7.66 - 7.48 (m, 3H), 7.04 - 6.85 (m, 4H), 6.78 (d, *J* = 8.4 Hz, 1H), 5.73 - 5.59 (m, 1H), 5.51 - 5.23 (m, 1H), 5.18 - 5.08 (m, 1H), 4.99 (d, *J* = 10.4 Hz, 1H), 4.82 (d, *J* = 17.2 Hz, 1H), 4.73 - 4.60 (m, 2H), 3.70 - 3.60 (m, 2H), 2.93 (br s, 4H), 2.70 - 2.58 (m, 4H), 2.42 (br s, 4H), 2.27 - 2.08 (m, 4H), 1.81 (d, *J* = 12.0 Hz, 2H), 1.72 - 1.56 (m, 1H), 1.46 (s, 6H), 1.33 - 1.14 (m, 2H).

**Example 159: synthesis of compound 159**

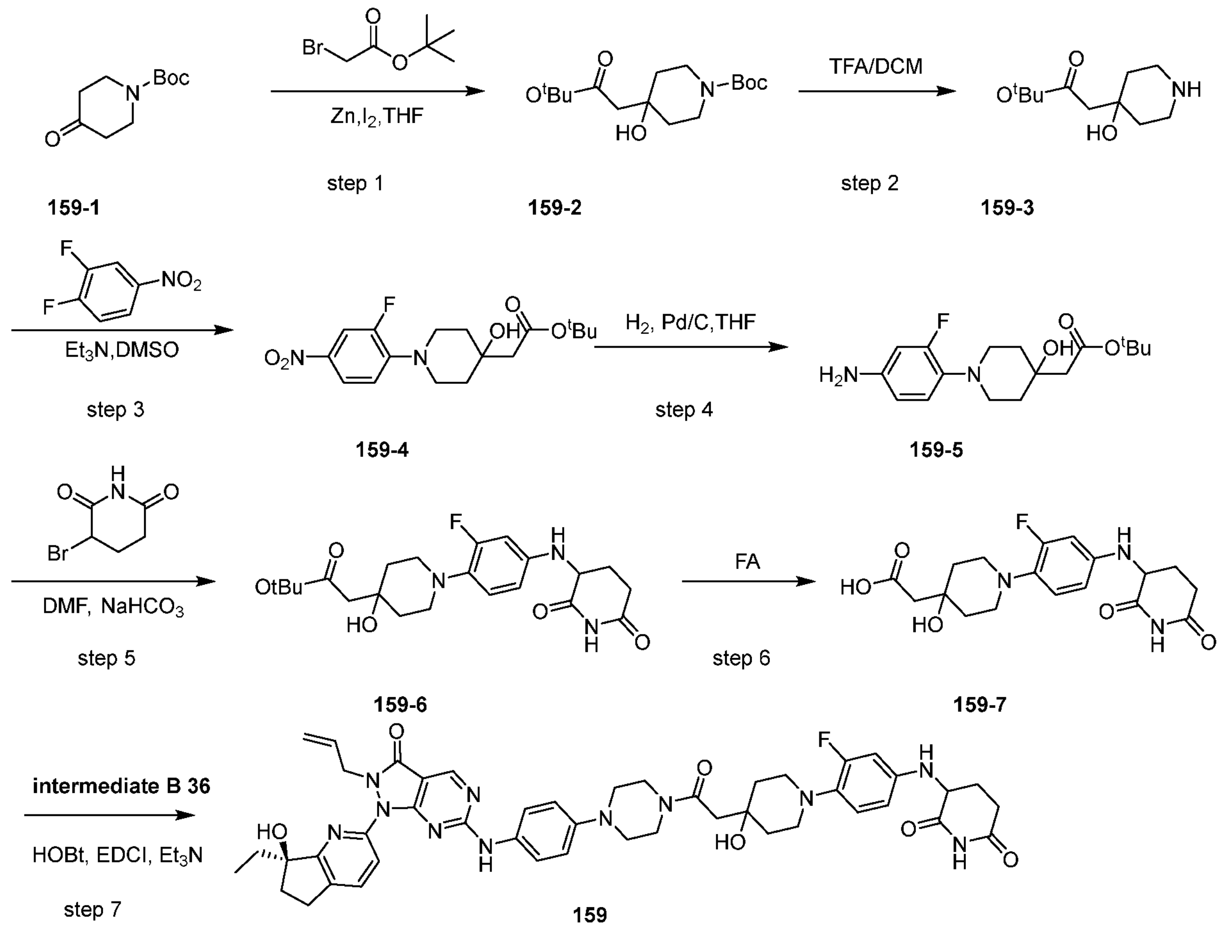

159-1 159-2 159-3 159-4 159-5 159-6 159-7 159

**Step 1: synthesis of intermediate 159-2**

To a solution of Zn (10.0 g, 153 mmol) in THF (50 mL) was added $I_2$ (6.35 g, 25.0 mmol) at 20°C, then tert-butyl 2-bromoacetate (6.42 g, 32.93 mmol) was added to the mixture. Then the solution of intermediate **159-1** (5 g, 25.09 mmol) in THF (20 mL) was added dropwise to the above mixture at 0°C. Then the reaction mixture was stirred at 100°C for 16 hours. The reaction mixture was quenched by addition $NaHCO_3$ (70 mL), and then diluted with EA (70 mL) and extracted with EA (60 mL × 3), the combined organic layer was dried over $Na_2SO_4$, filtered and concentrated under reduced pressure to give a residue. The residue was purified by column chromatography ($SiO_2$, Petroleum ether/Ethyl acetate=2/1 to 0/1) to give the **intermediate 159-2**(6.81 g, crude) as a yellow solid, which was used into the next step directly.

**Step 2: synthesis of intermediate 159-3**

To a solution of **intermediate 159-2** (6.81 g, 21.6 mmol) in DCM (40 mL) was added TFA (12.3 g, 108 mmol).The mixture was stirred at 20 °C for 1 hr. The reaction mixture was filtered and concentrated under reduced pressure to give a crude of **intermediate 159-3** (10.0 g, crude) as a red oil, which was used into the next step directly.

**Step 3: synthesis of intermediate 159-4**

To a solution of **intermediate 159-3** (10 g, 30.4 mmol) and 1,2-difluoro-4-nitro-benzene (4.83 g, 30.4 mmol) in DMSO (40 mL) was added TEA (6.15 g, 60.7 mmol).The mixture was stirred at 80 °C for 16 hr. The reaction mixture was partitioned between water (60 mL) and EA ( 20 mL × 3). The organic phase was separated dried over $Na_2SO_4$, filtered and concentrated under reduced pressure to give a residue.The residue was purified by column chromatography ($SiO_2$, Petroleum ether/Ethyl acetate=5/1 to 3/1) to give the **intermediate 159-4** (6.81 g, 63.3%) as a yellow solid. MS ($ESI^+$): m/z 355.3

**Step 4: synthesis of intermediate 159-5**

To a solution of **intermediate 159-4** (6.75 g, 19.1 mmol) in THF (40 mL) was added Pd/C (10%, 5 g). The suspension was degassed and purged with $H_2$ for 3 times. The mixture was stirred under $H_2$ (15 Psi) at 20 °C for 16 hrs.The reaction mixture was filtered and concentrated under reduced pressure to give a crude of intermediate **159-5** (7.21 g, crude).

**Step 5: synthesis of intermediate 159-6**

A mixture of intermediate **159-5** (7.21 g, 22.2 mmol) , 3-bromopiperidine-2,6-dione (6.40 g, 33.3 mmol) , $NaHCO_3$ (5.60 g, 66.7 mmol) in DMF (20 mL) was degassed and purged with $N_2$ for 3 times, and then the mixture was stirred at 80 °C for 16 hrs under $N_2$ atmosphere. The reaction mixture was partitioned between water (10 mL) and DCM (90 mL). The organic phase was separated and dried over $Na_2SO_4$, filtered and concentrated under reduced pressure to give a residue. The residue was purified by column chromatography ($SiO_2$, Petroleum ether/Ethyl acetate=3/1 to 1/1)to give the **intermediate 159-6** (7.46 g, 60.9% yield) as a green solid.
MS (ESI$^+$): m/z 436.5 [M+H]$^+$.

**Step 6: synthesis of intermediate 159-7**

A solution of **intermediate 159-6** (500 mg, 1.15 mmol) in FA (1.15 mmol, 10 mL) was stirred at 50 °C for 2 hrs. The reaction mixture was concentrated under reduced pressure to give a crude of the **intermediate 159-7** (6.81 g crude) as a black solid, which was used into the next step directly.

**Step 7: synthesis of compound 159**

To a mixture of intermediate **159-7** (50 mg, 118 umol)and intermediate **3** (60.25 mg, 117.53 umol) in DCM (2 mL) was added HOBt (23.82 mg, 176.30 umol) and EDCI (33.80 mg, 176.30 umol). The obtained mixture was stirred at 20°C for 0.5 hr. Then $Et_3N$ (35.68 mg, 352.60 umol) was added to the mixture and the obtained mixture was stirred at 25 °C for 16 hours. The reaction mixture was partitioned between water (2 mL) and DCM (20 mL). The organic phase was separated and dried over $Na_2SO_4$, filtered and concentrated under reduced pressure to give a residue. The residue was purified by column chromatography ($SiO_2$, DCM: MeOH=1/0 to 10/1) to give compound **159** (8 mg, 7.63% yield) as a white solid. MS (ESI$^+$): m/z 874.4[M+H]$^+$
$^1$H NMR (400MHz, DMSO-$d_6$)10.79 (br s, 1H), 10.19 (br s, 1H), 8.83 (s, 1H), 7.93 (br s, 1H), 7.69(m, 1H), 7.61 (br s, 2H), 6.96 (m, 2H), 6.85 (m, 1H), 6.49 (m, 1H), 6.41 (m, 1H), 5.79 (m, 1H), 5.72 - 5.60 (m, 1H), 5.08 (s, 1H), 4.99 (m, 1H), 4.90 - 4.71 (m, 3H), 4.57 (m, 1H), 4.30 - 4.19 (m, 1H), 3.68 (m, 4H), 3.59 (br s, 1H), 3.10 (m, 4H), 3.01 - 2.70 (m, 7H), 2.59 - 2.56 (m, 2H), 2.25 -2.15 (m, 1H), 2.12 - 1.95 (m, 3H), 1.93 - 1.81 (m, 2H), 1.78 - 1.64 (m, 4H), 0.86 (m, 3H).

**Exemplary Compounds of the Present Invention**

**Table 1**

| compound | Structure |
|---|---|
| 1 | 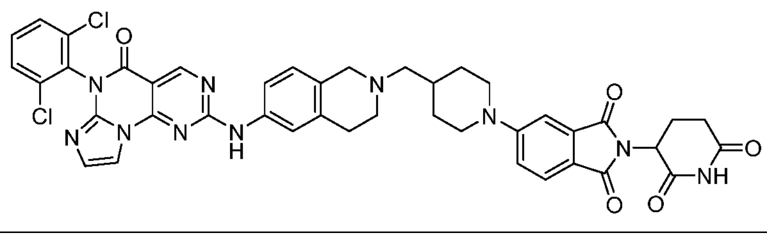 |

(continued)

| compound | Structure |
|---|---|
| 2 | 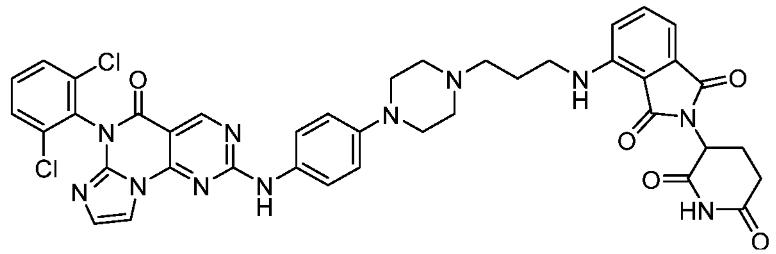 |
| 3 | 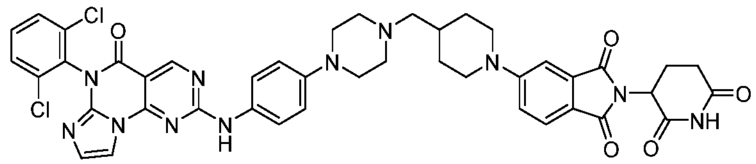 |
| 4 | 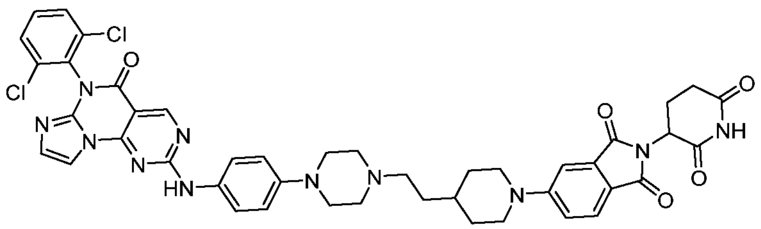 |
| 5 | 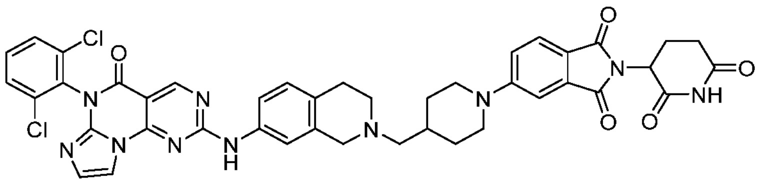 |
| 6 | 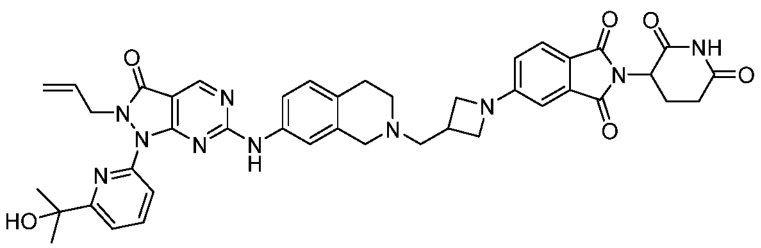 |
| 7 | 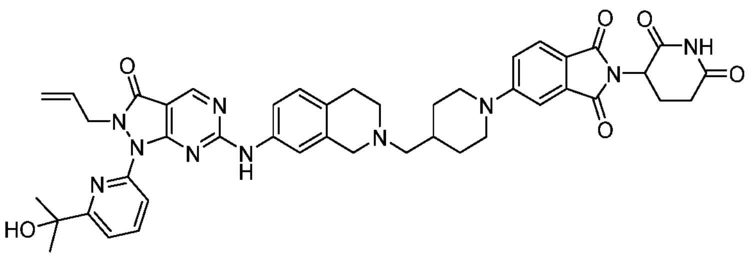 |
| 8 | 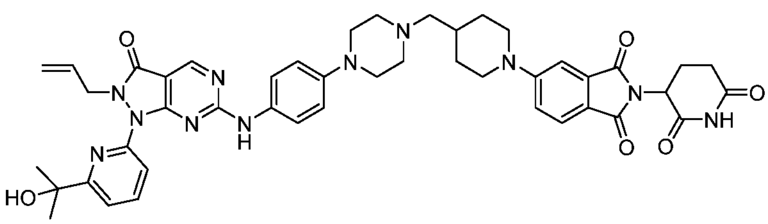 |

(continued)

| compound | Structure |
|---|---|
| 9 | 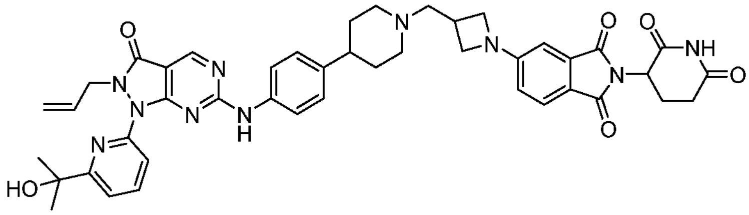 |
| 10 | 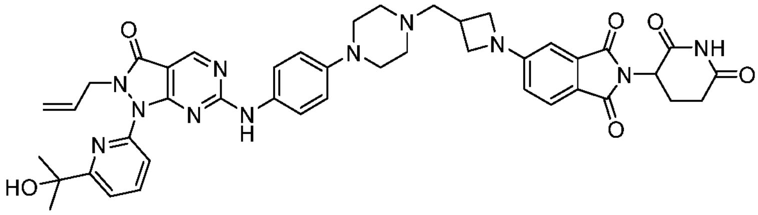 |
| 11 | 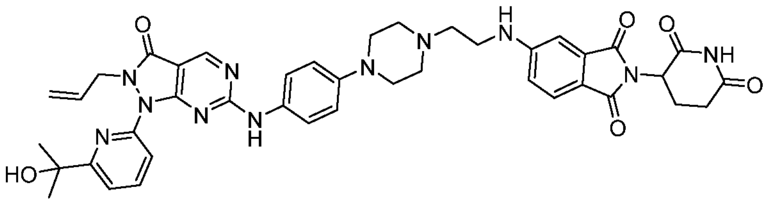 |
| 12 | 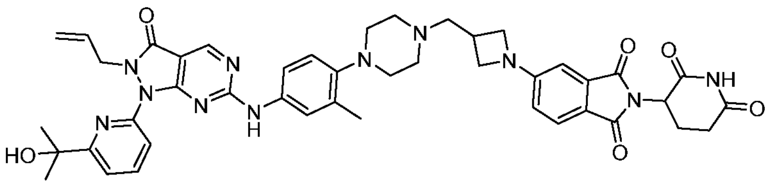 |
| 13 | 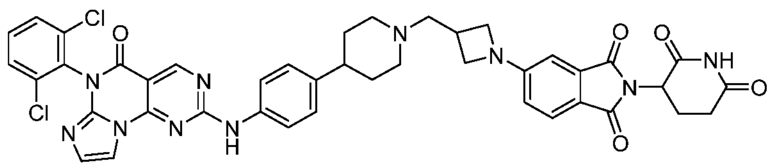 |
| 14 | 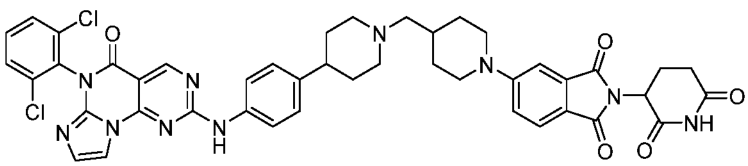 |
| 15 | 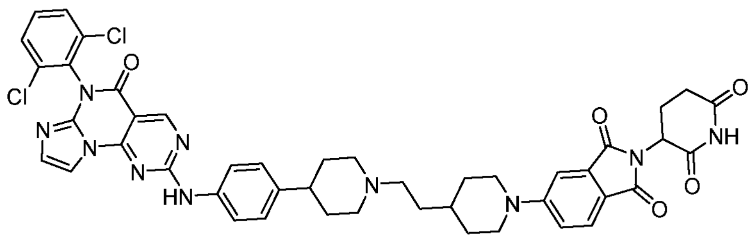 |

(continued)

| compound | Structure |
|---|---|
| 16 | 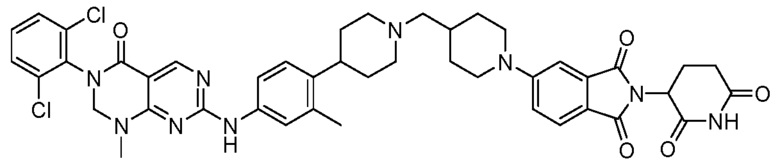 |
| 17 | 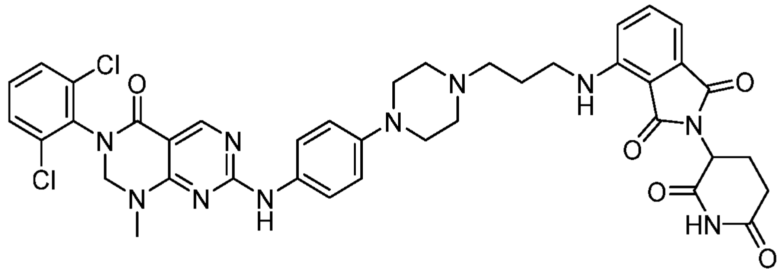 |
| 18 | 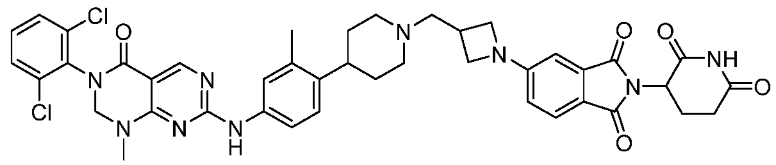 |
| 19 | 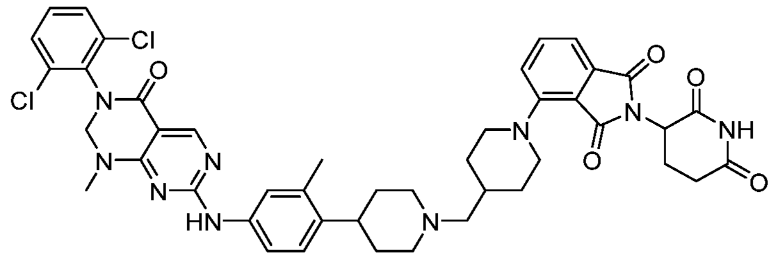 |
| 20 | 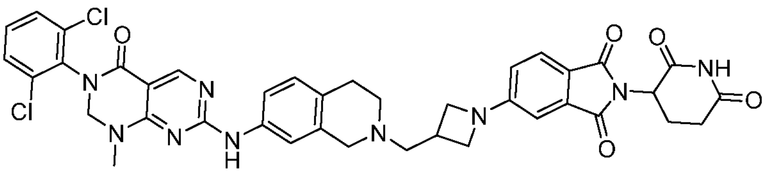 |
| 21 | 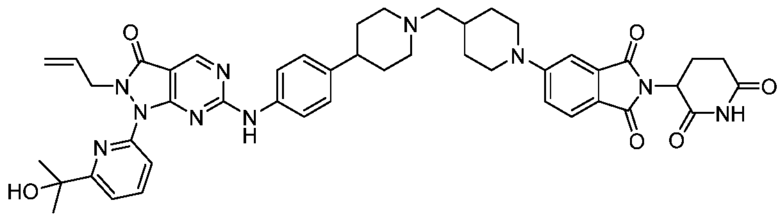 |
| 22 | 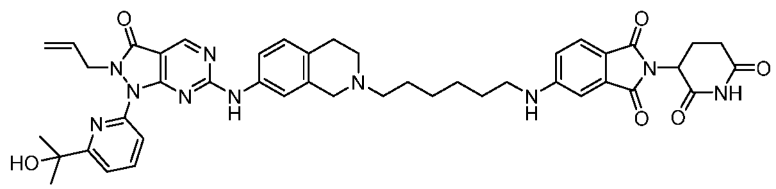 |

(continued)

| compound | Structure |
|---|---|
| 23 | 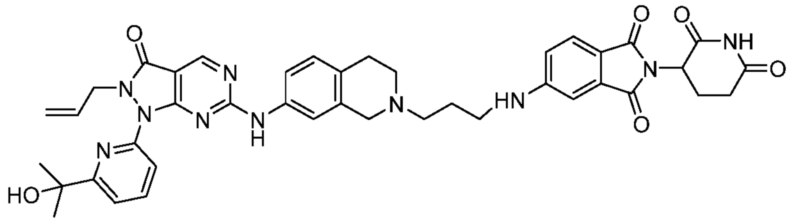 |
| 24 | 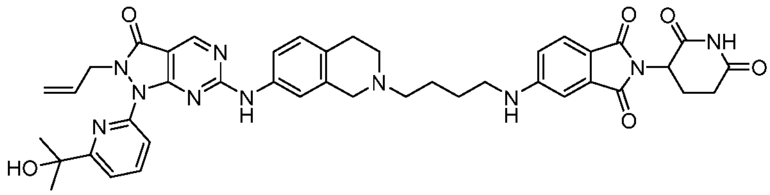 |
| 25 | 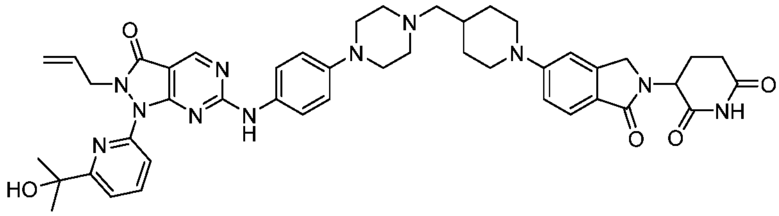 |
| 26 | 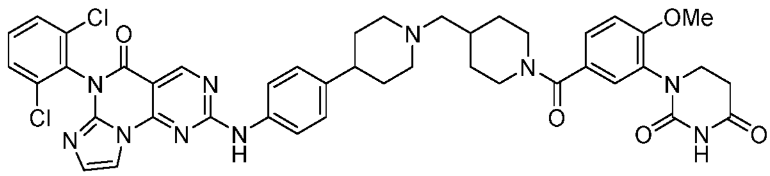 |
| 27 | 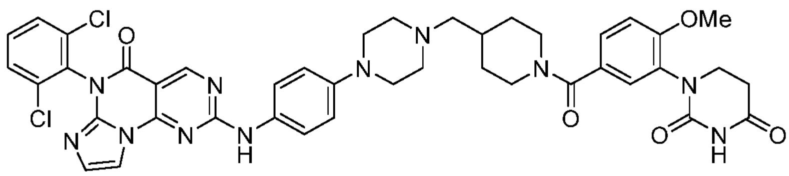 |
| 28 | 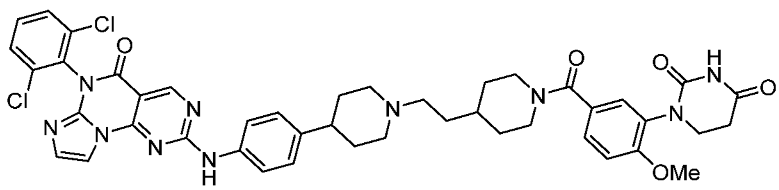 |
| 29 | 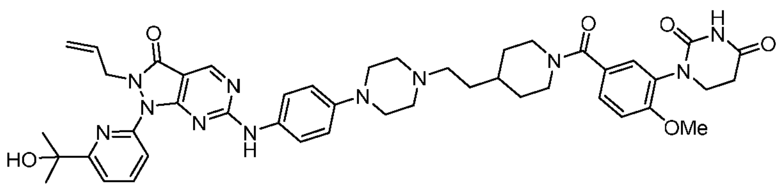 |
| 30 | 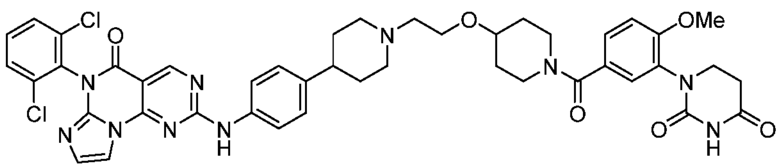 |

(continued)

| compound | Structure |
|---|---|
| 31 | 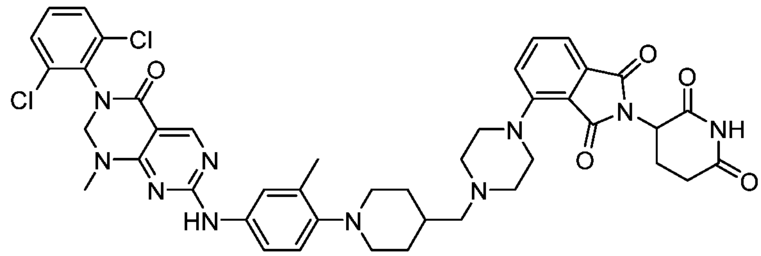 |
| 32 | 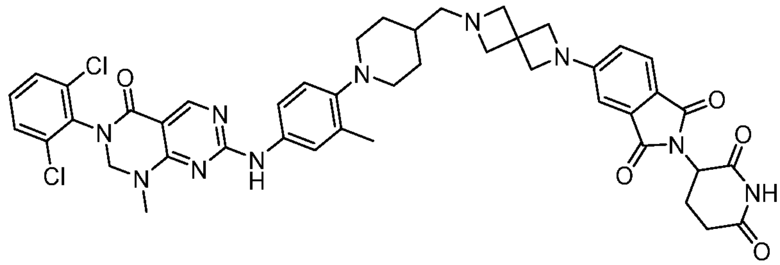 |
| 33 | 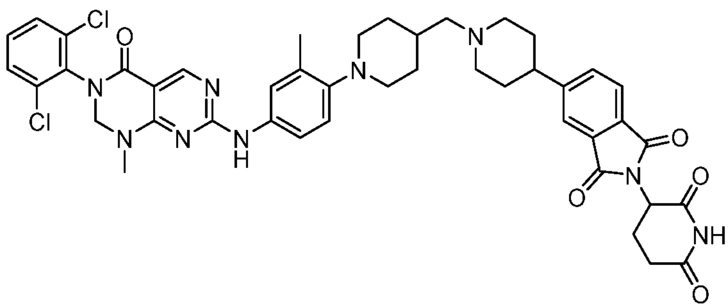 |
| 34 | 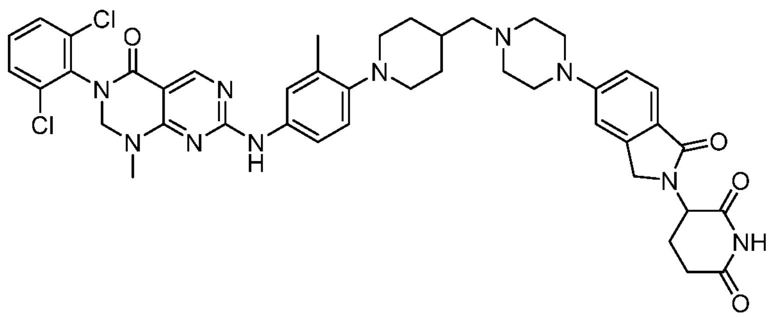 |
| 35 | 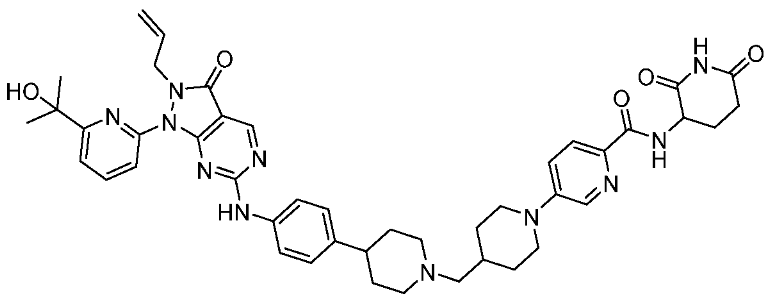 |

(continued)

| compound | Structure |
|---|---|
| 36 | 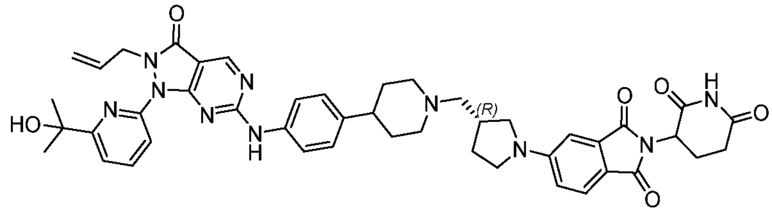 |
| 37 | 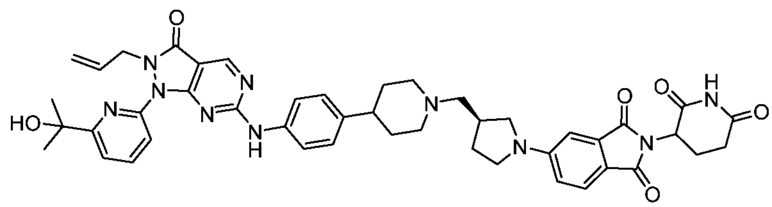 |
| 38 | 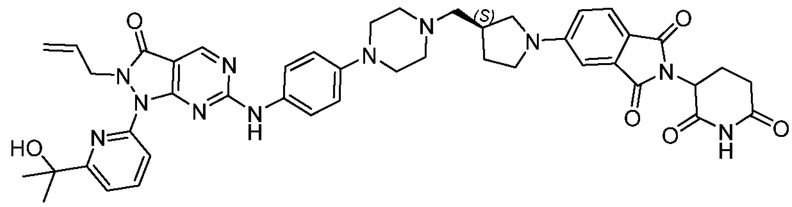 |
| 39 | 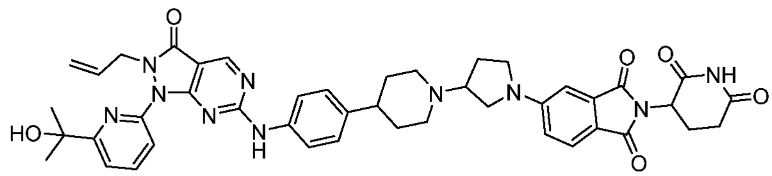 |
| 40 | 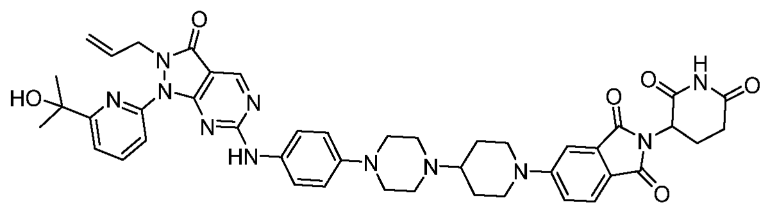 |
| 41 | 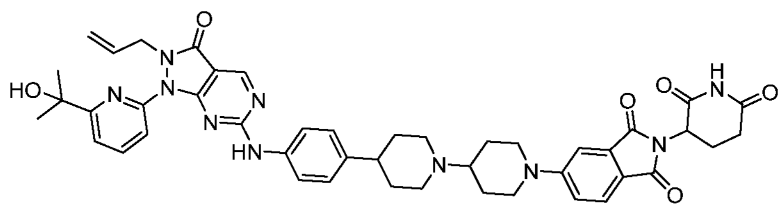 |
| 42 | 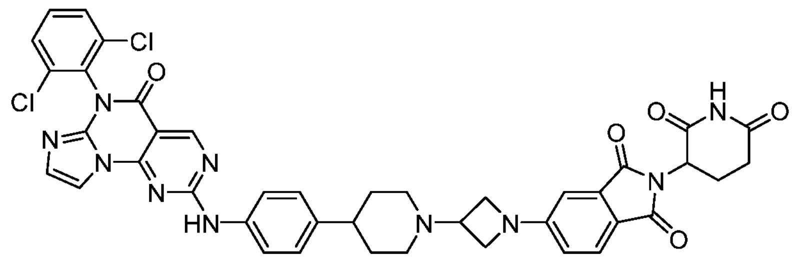 |

(continued)

| compound | Structure |
|---|---|
| 43 | 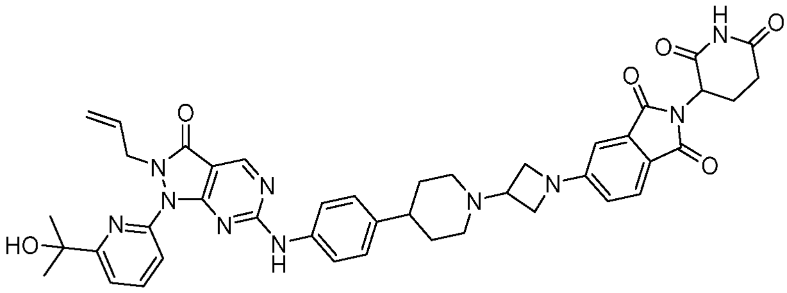 |
| 44 | 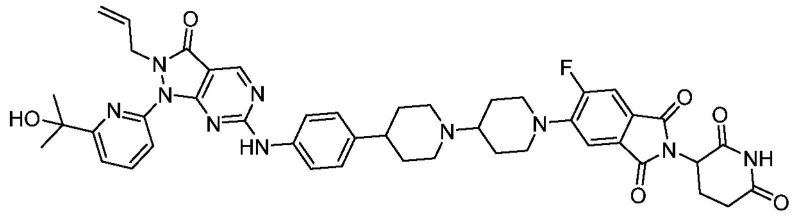 |
| 45 | 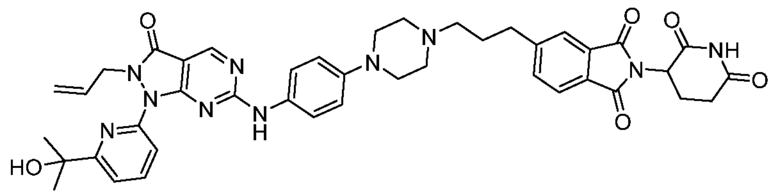 |
| 46 | 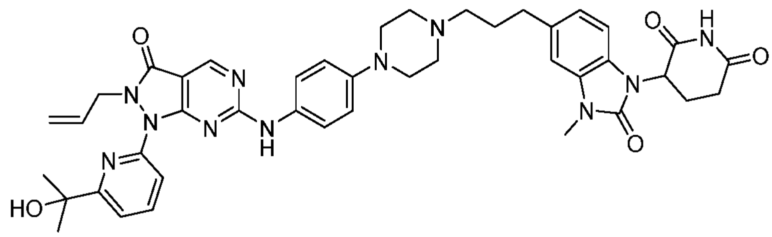 |
| 47 | 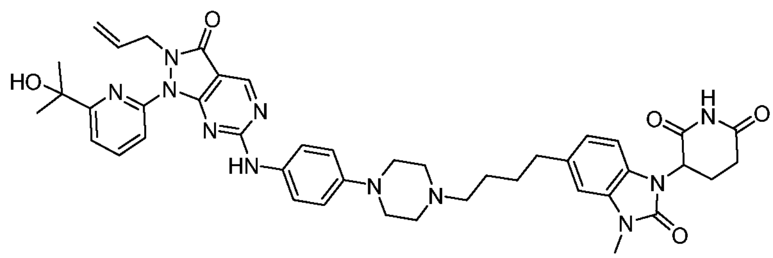 |
| 48 | 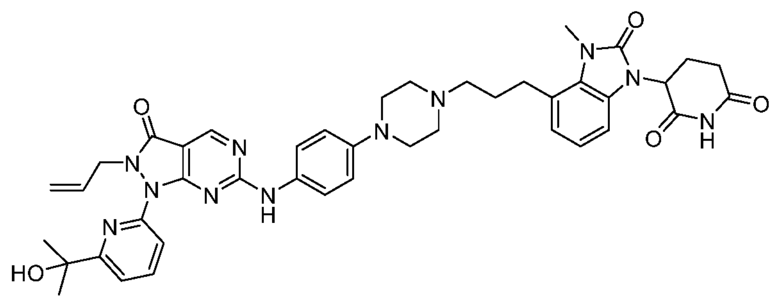 |

(continued)

| compound | Structure |
|---|---|
| 49 | 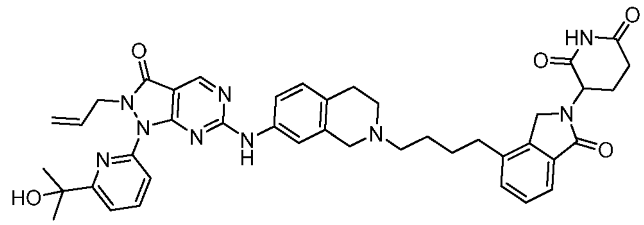 |
| 50 | 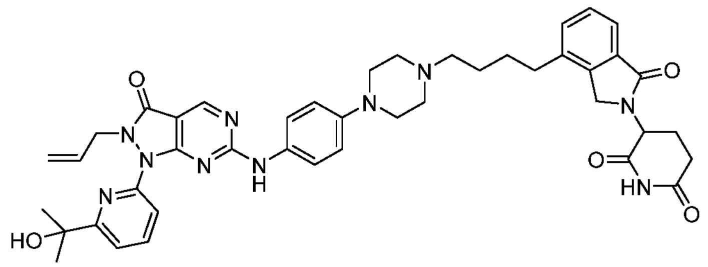 |
| 51 | 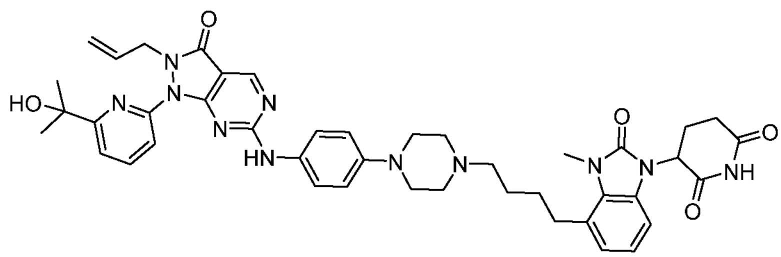 |
| 52 | 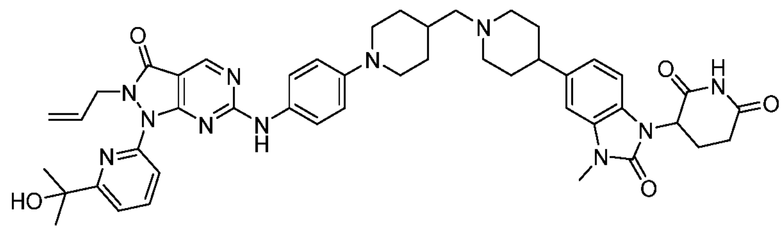 |
| 53 | 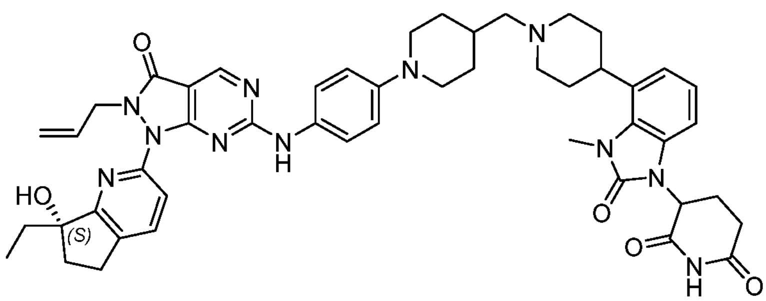 |
| 54 | 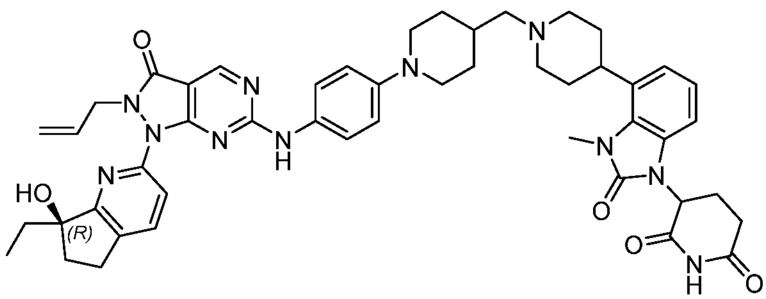 |

(continued)

| compound | Structure |
|---|---|
| 55 | 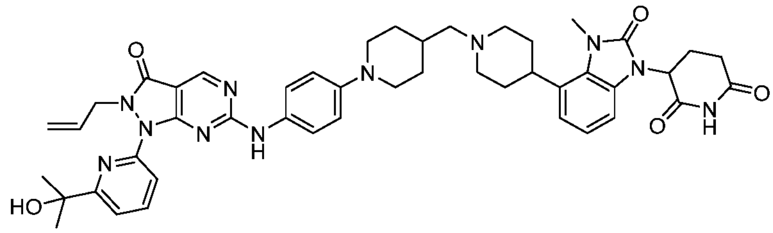 |
| 56 | 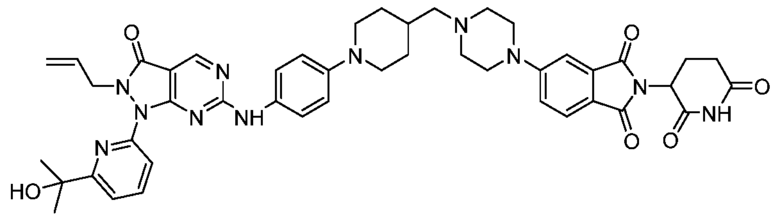 |
| 57 | 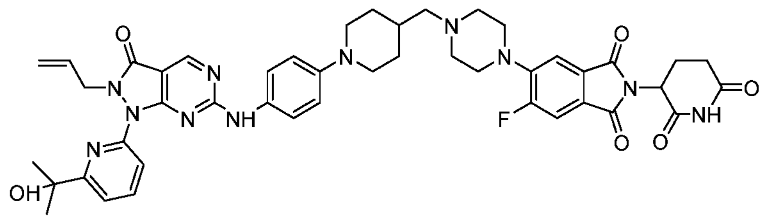 |
| 58 | 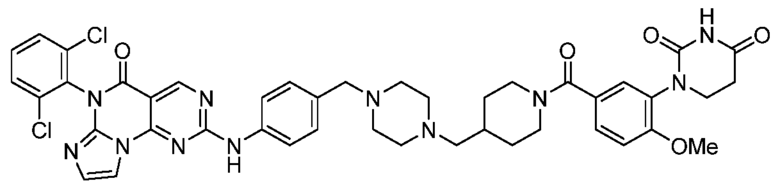 |
| 59 | 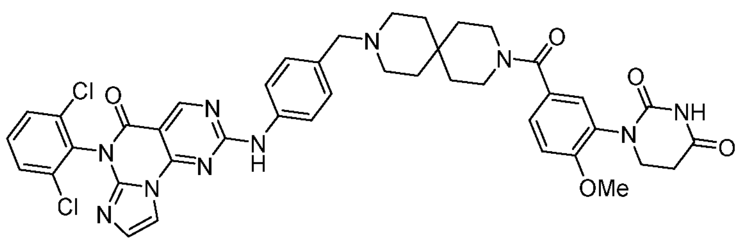 |
| 60 | 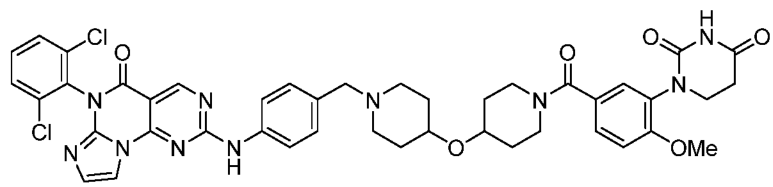 |
| 61 | 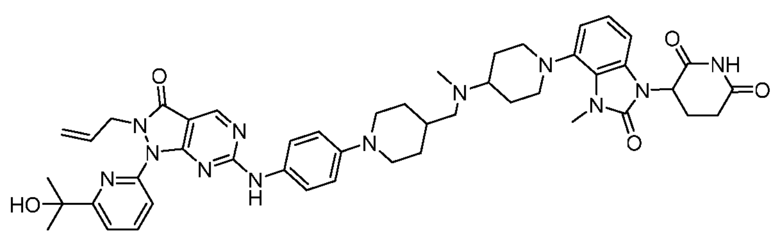 |

(continued)

| compound | Structure |
|---|---|
| 62 | 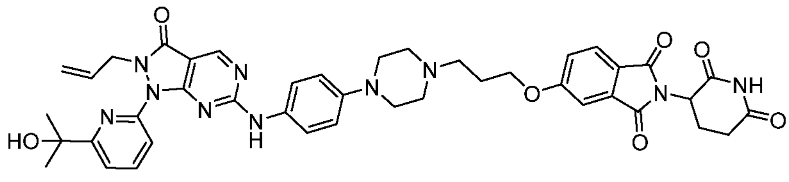 |
| 63 | 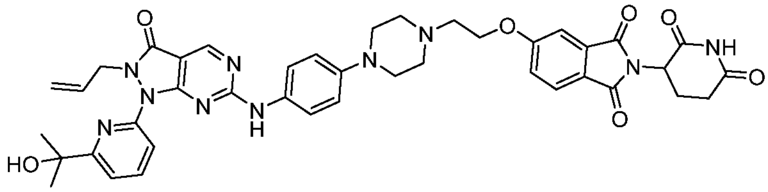 |
| 64 | 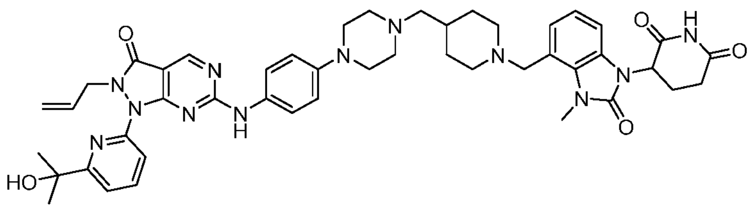 |
| 65 | 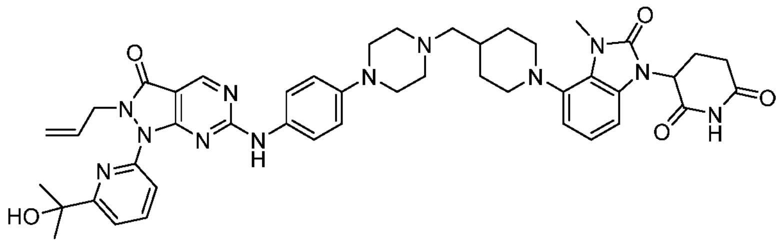 |
| 66 | 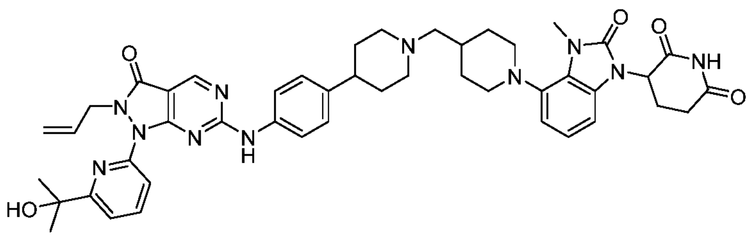 |
| 67 | 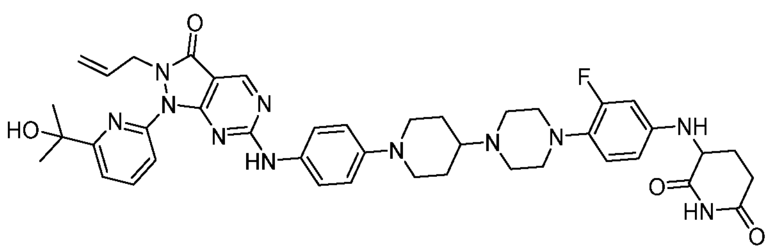 |
| 68 | 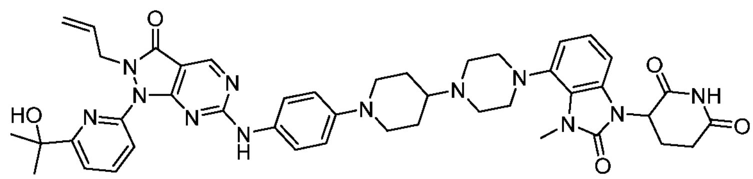 |

(continued)

| compound | Structure |
|---|---|
| 69 | 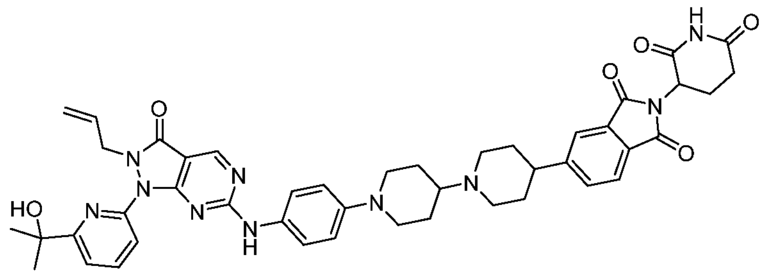 |
| 70 | 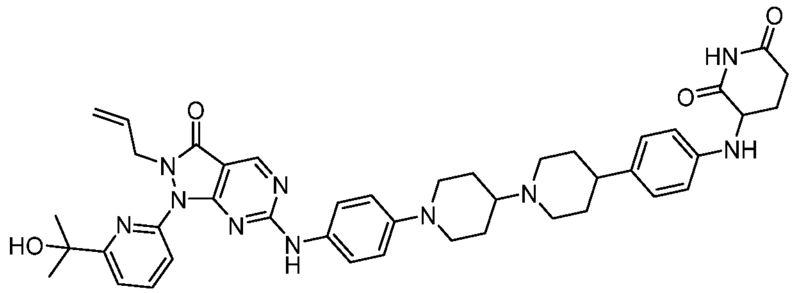 |
| 71 | 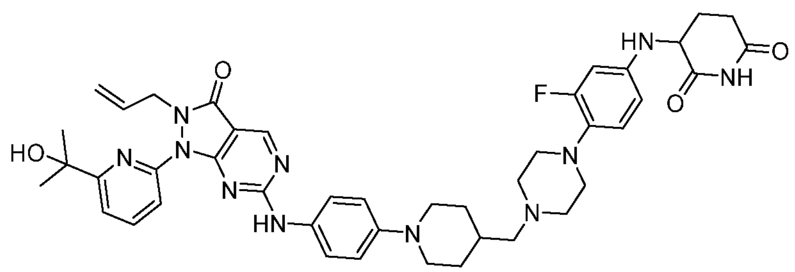 |
| 72 | 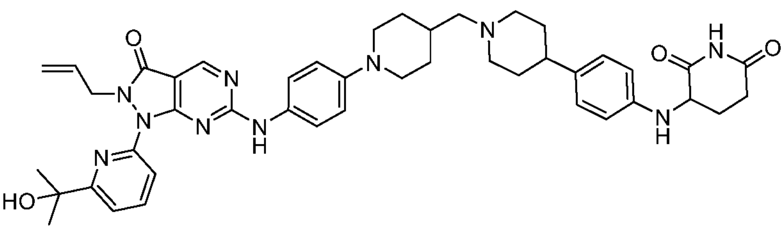 |
| 73 | 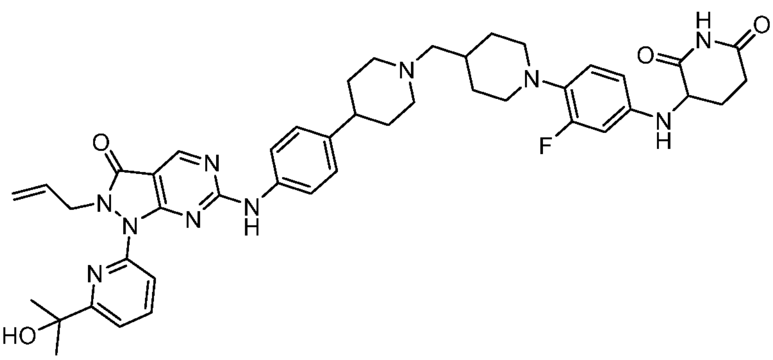 |

(continued)

| compound | Structure |
|---|---|
| 74 | 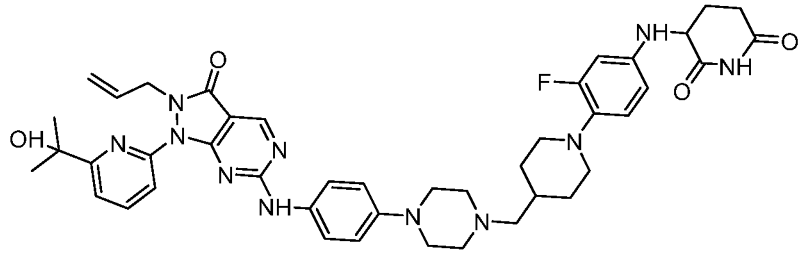 |
| 75 | 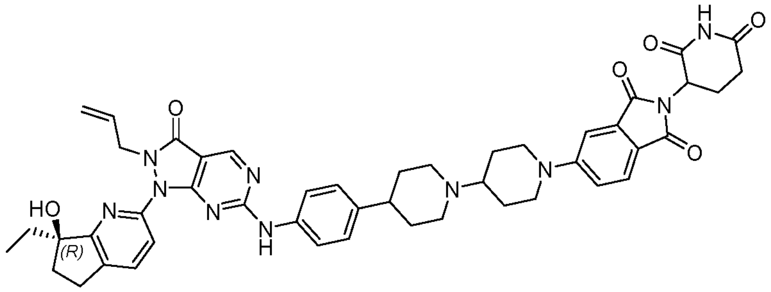 |
| 76 | 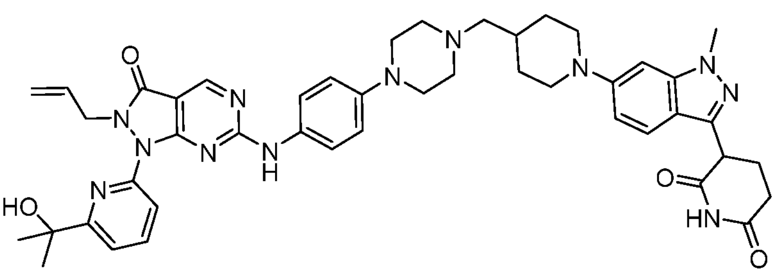 |
| 77 | 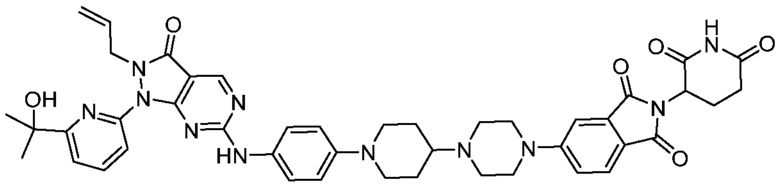 |
| 78 | 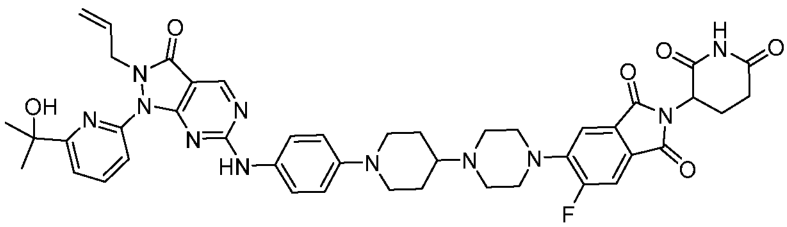 |

(continued)

| compound | Structure |
|---|---|
| 79 | 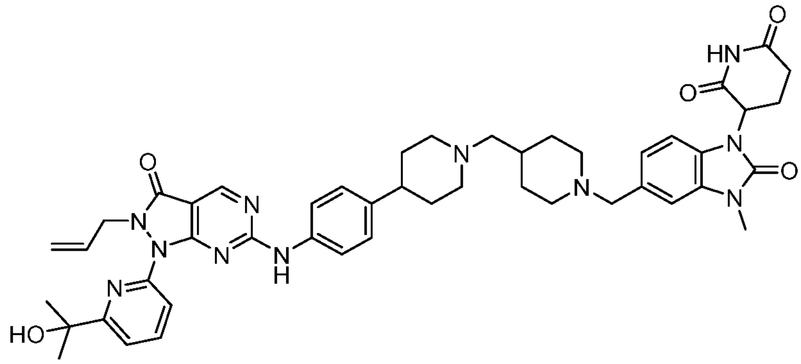 |
| 80 | 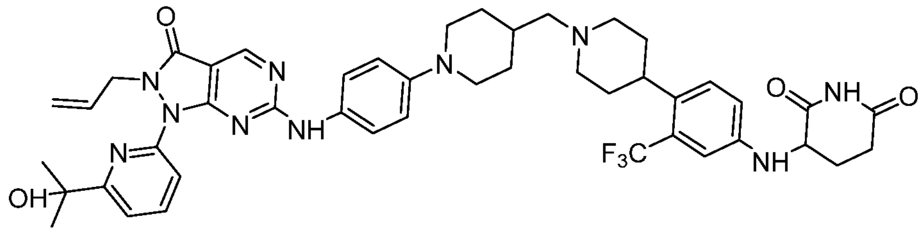 |
| 81 | 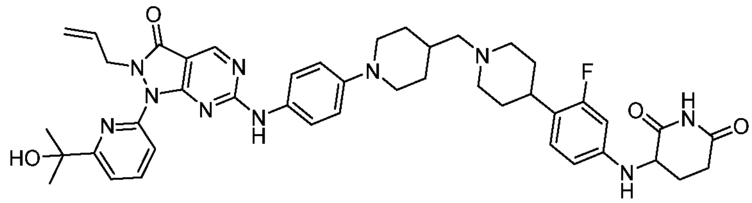 |
| 82 | 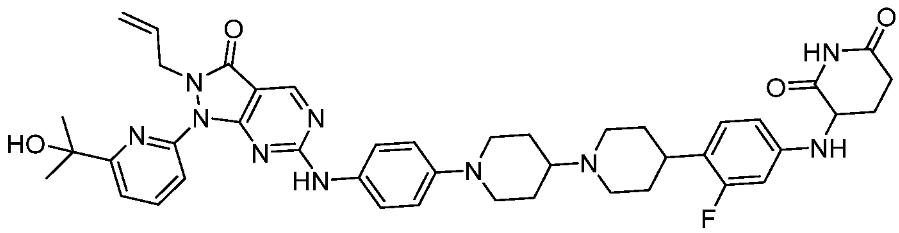 |
| 83 | 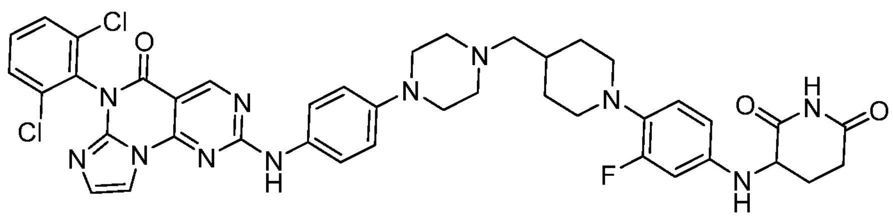 |
| 84 | 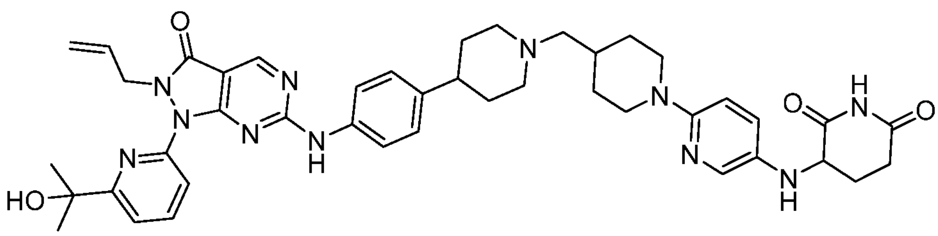 |

(continued)

| compound | Structure |
|---|---|
| 85 | 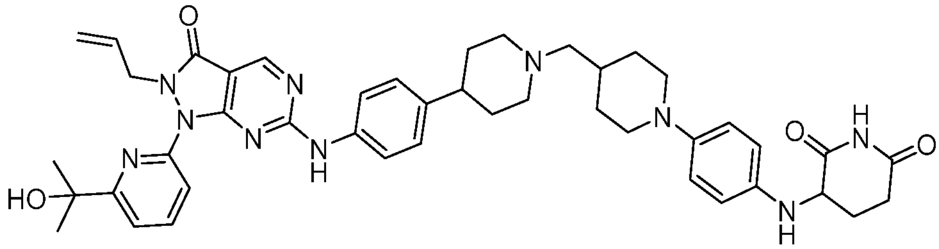 |
| 86 | 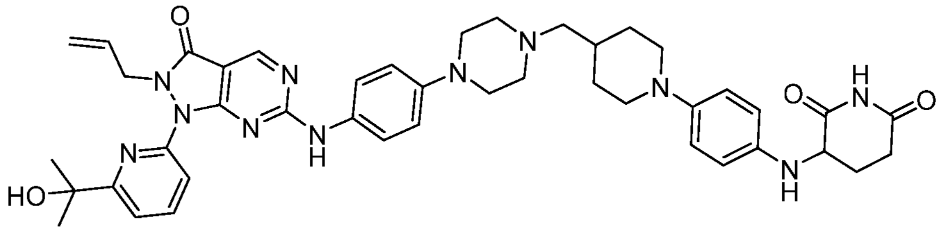 |
| 87 | 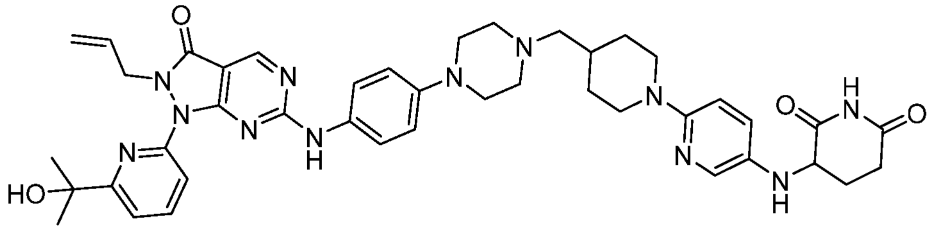 |
| 88 | 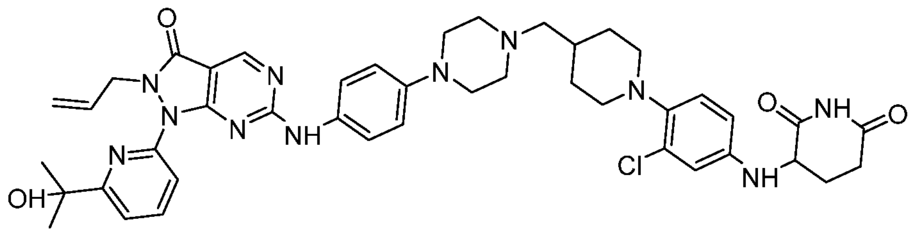 |
| 89 | 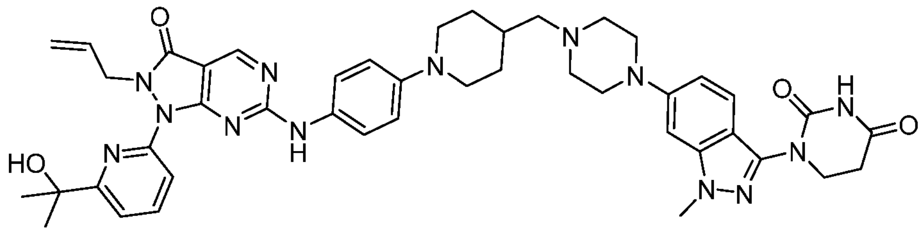 |
| 90 | 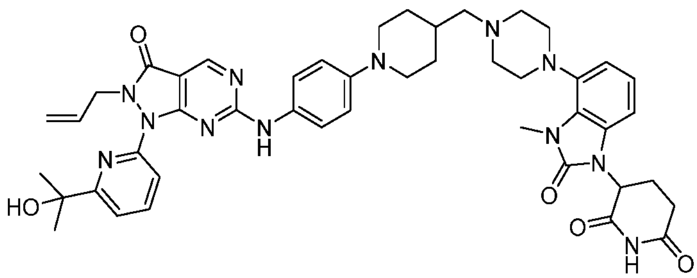 |

(continued)

| compound | Structure |
| --- | --- |
| 91 | 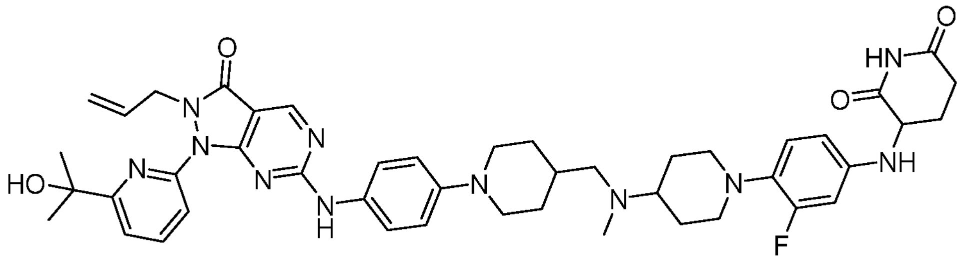 |
| 92 | 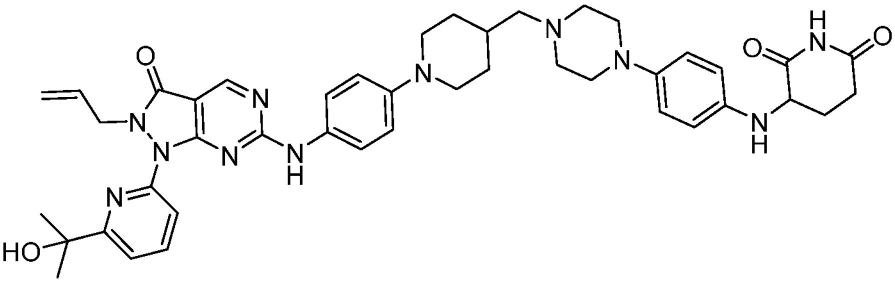 |
| 93 | 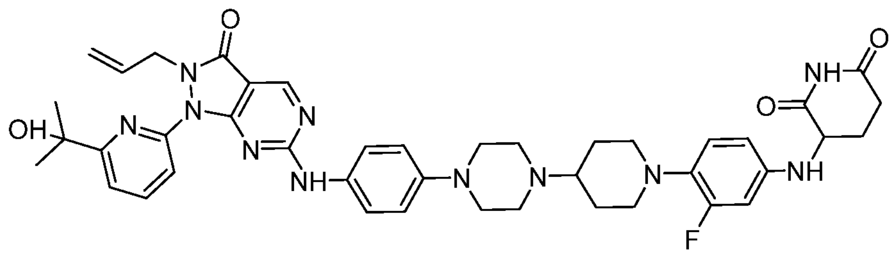 |
| 94 | 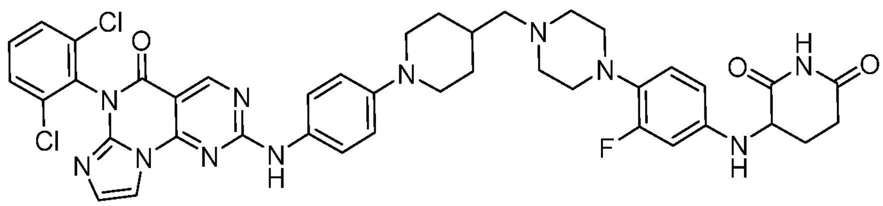 |
| 95 | 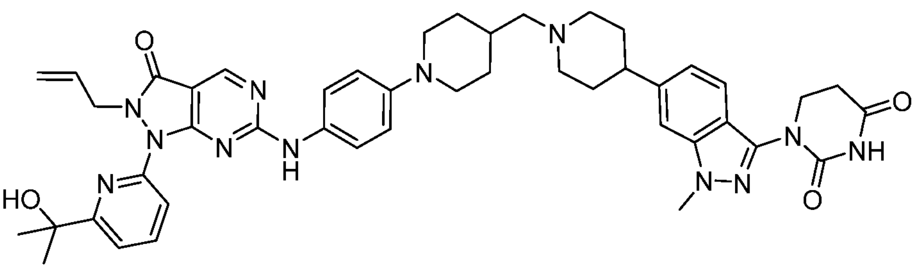 |
| 96 | 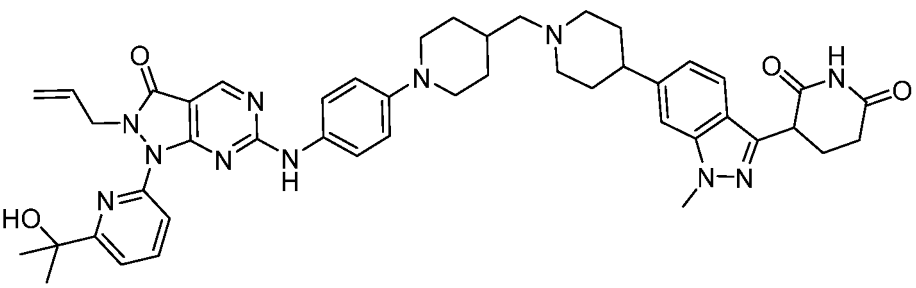 |

(continued)

| compound | Structure |
|---|---|
| 97 | 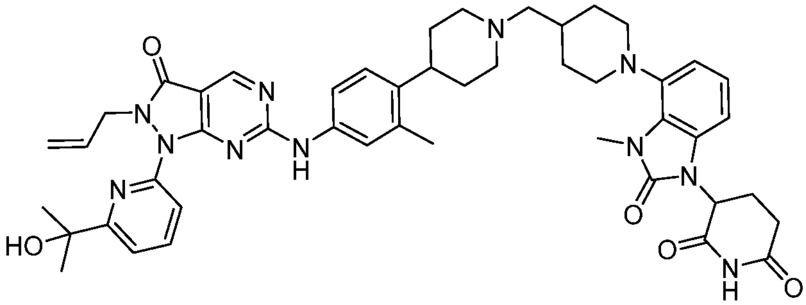 |
| 98 | 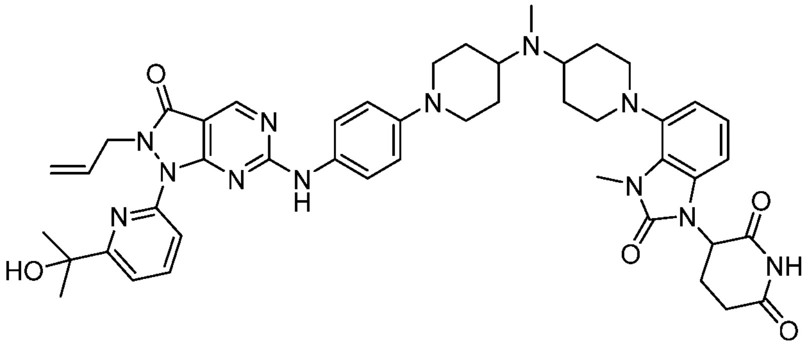 |
| 99 | 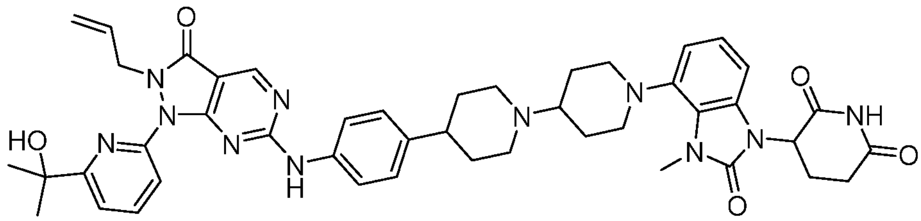 |
| 100-A | 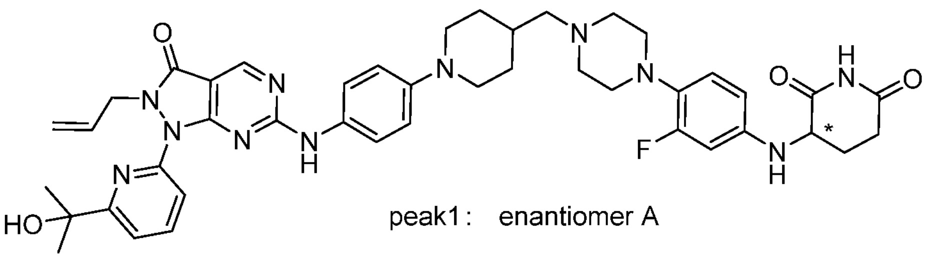 peak1: enantiomer A |
| 100-B | 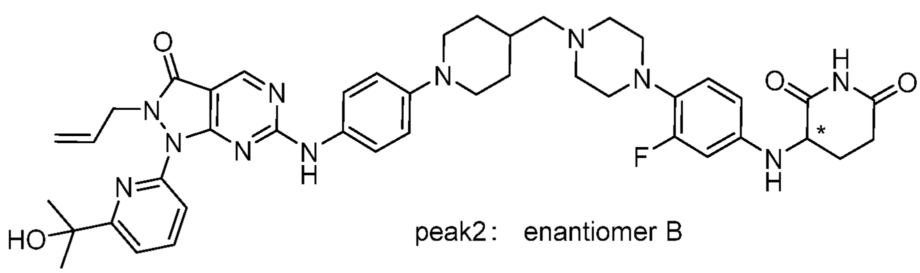 peak2: enantiomer B |

(continued)

| compound | Structure |
|---|---|
| 102-A | 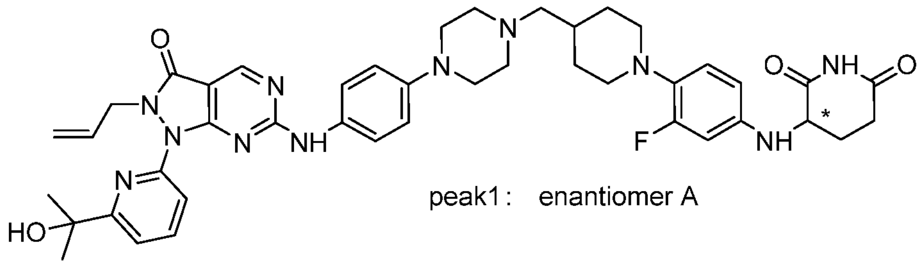 peak1： enantiomer A |
| 102-B | 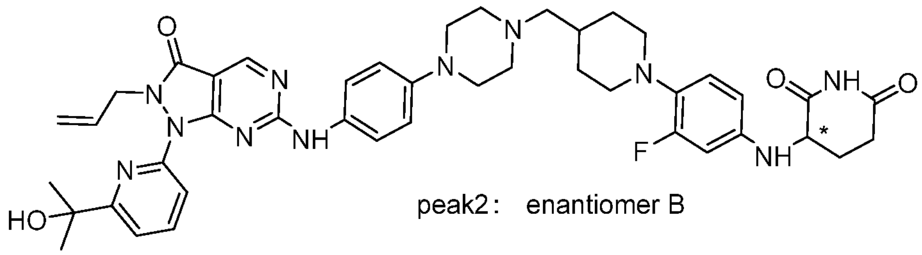 peak2： enantiomer B |
| 104 | 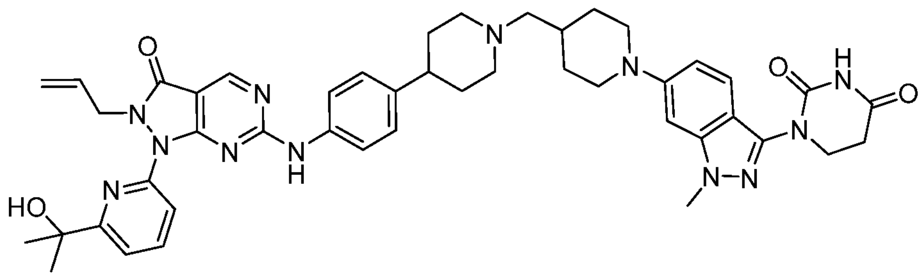 |
| 105 | 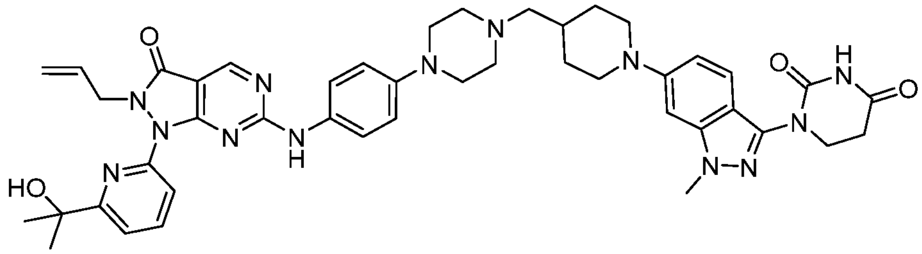 |
| 106 | 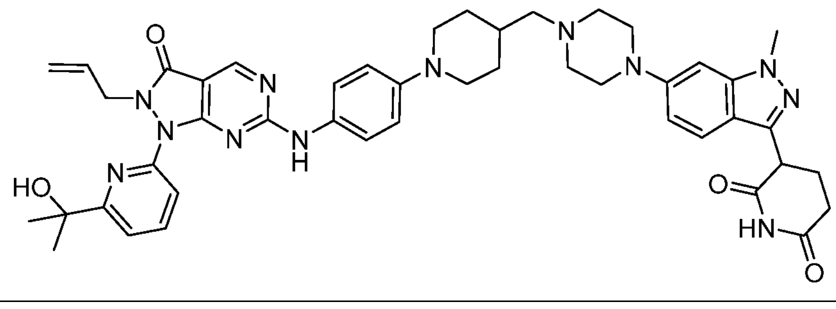 |
| 107 | 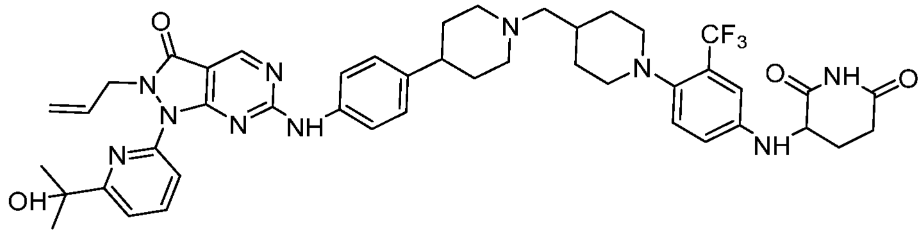 |

(continued)

| compound | Structure |
|---|---|
| 108 | 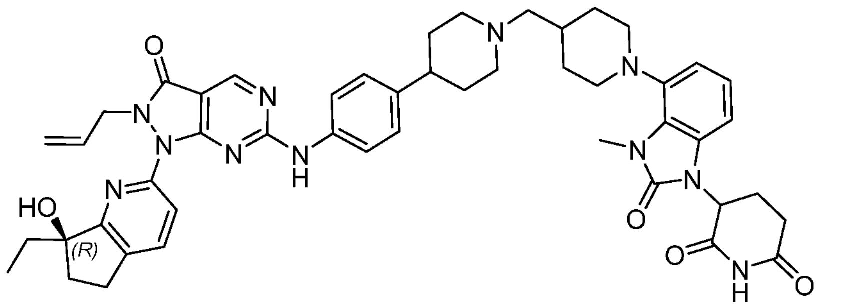 |
| 109 | 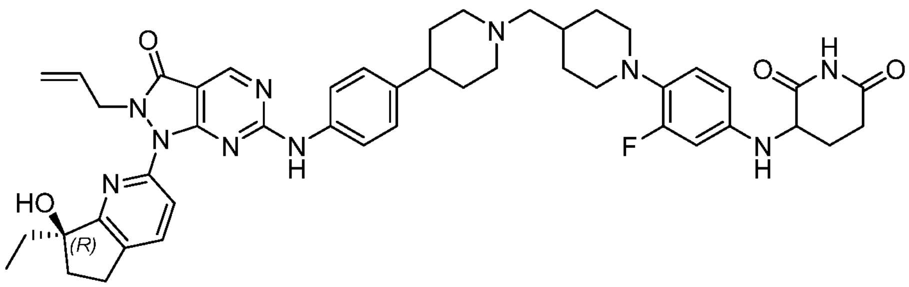 |
| 110 | 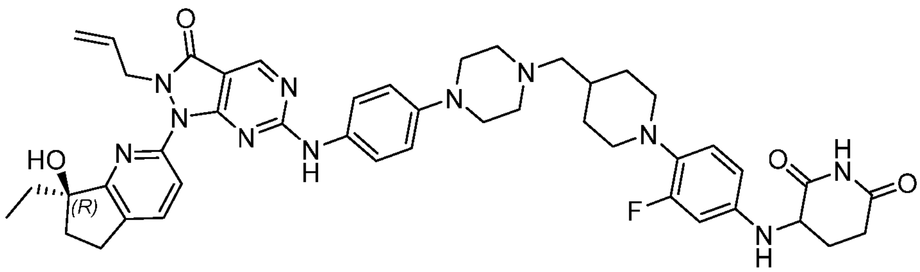 |
| 111 | 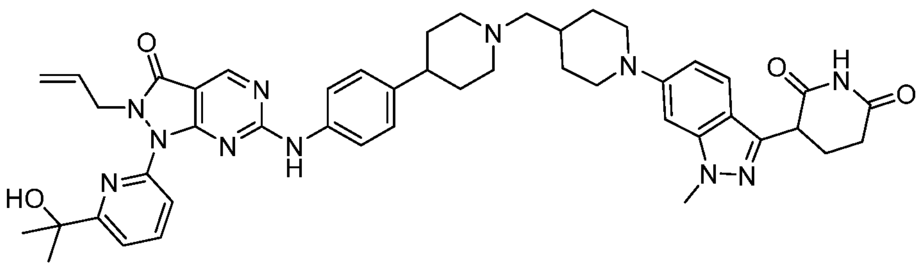 |
| 112 | 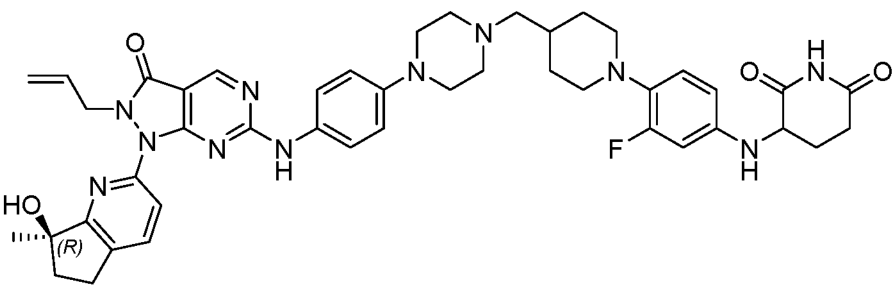 |

(continued)

| compound | Structure |
|---|---|
| 113 | 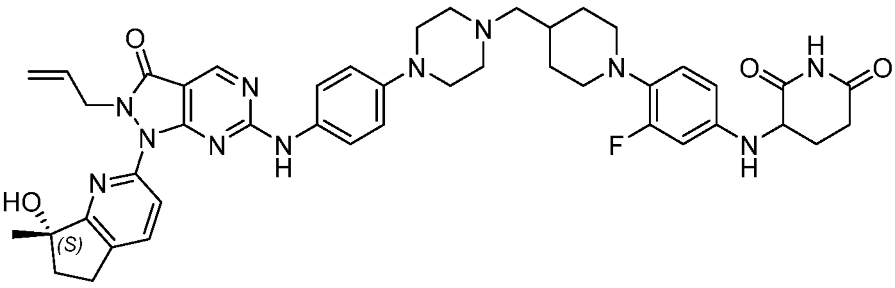 |
| 114 | 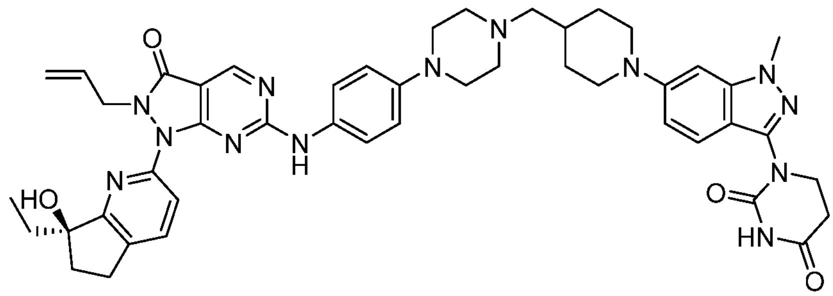 |
| 115 | 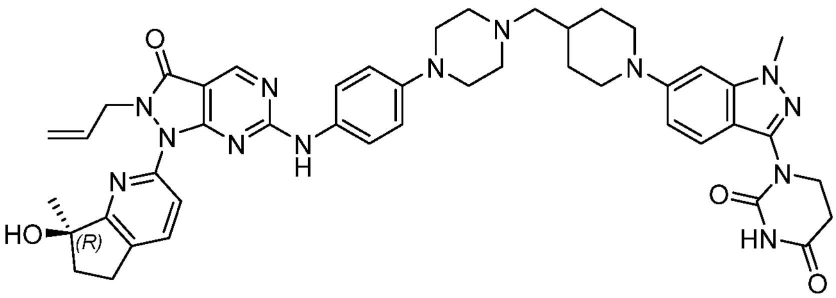 |
| 116 | 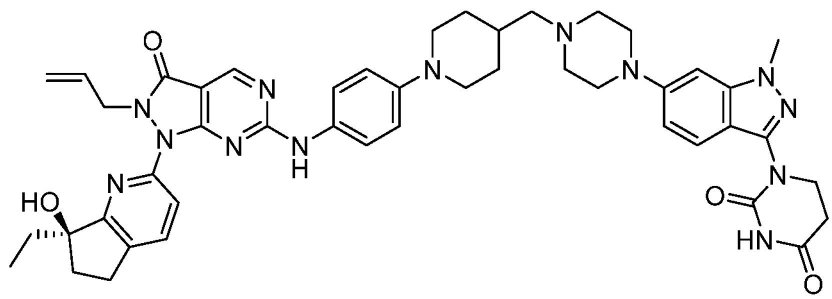 |
| 117 | 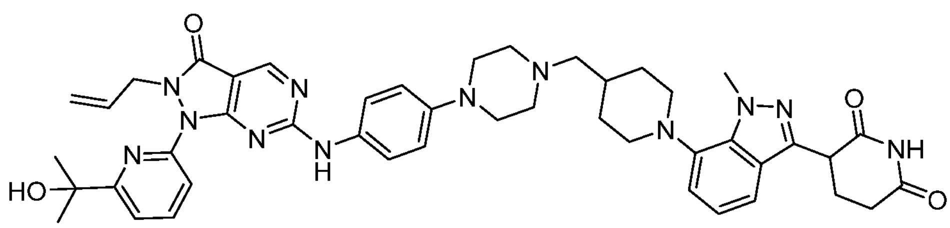 |

(continued)

| compound | Structure |
|---|---|
| 118 | 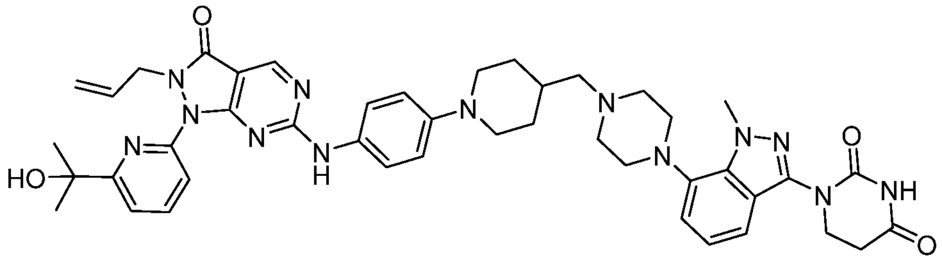 |
| 119 | 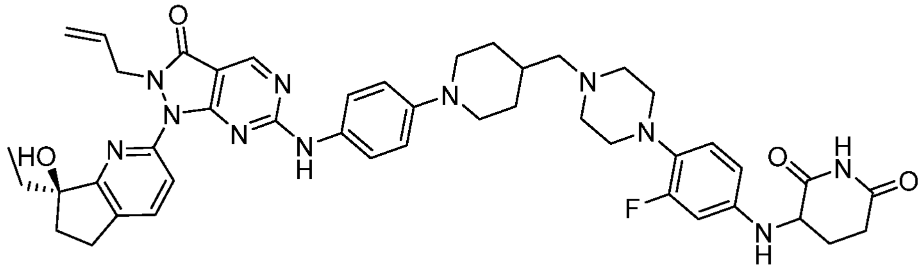 |
| 120 | 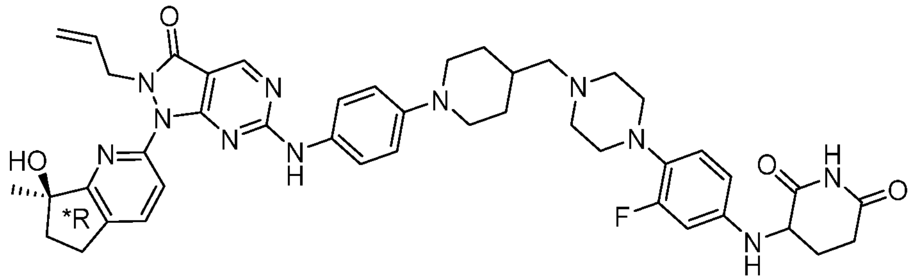 |
| 121 | 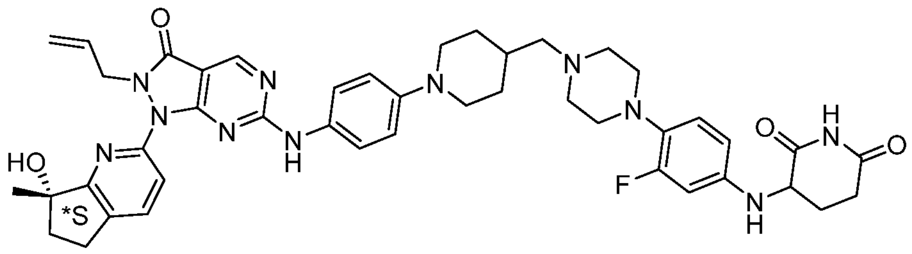 |
| 122 | 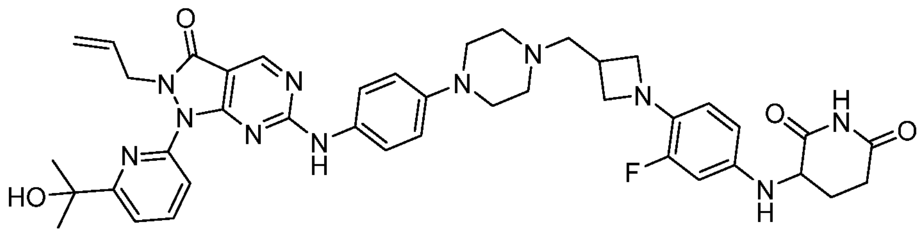 |
| 123 | 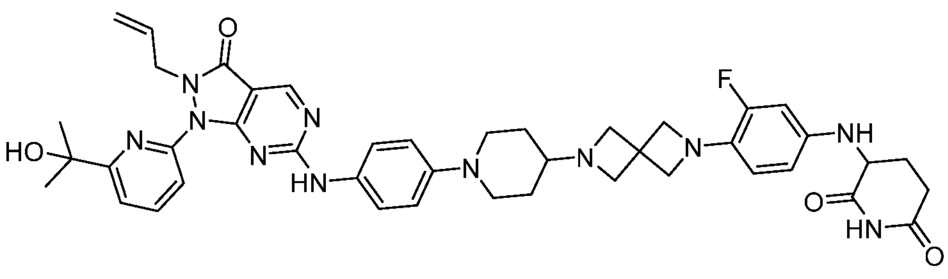 |

(continued)

| compound | Structure |
|---|---|
| 124 | 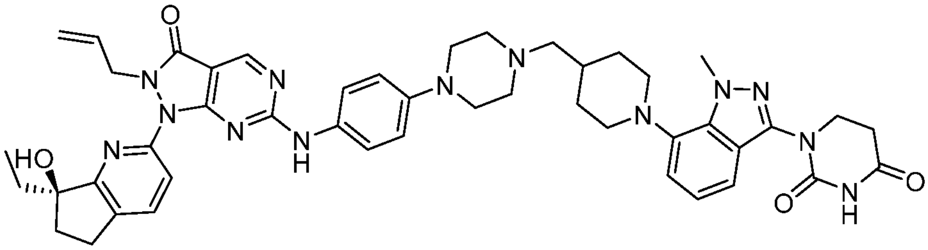 |
| 125 | 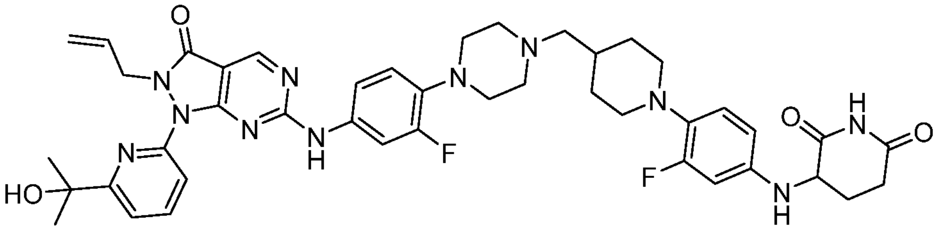 |
| 126 | 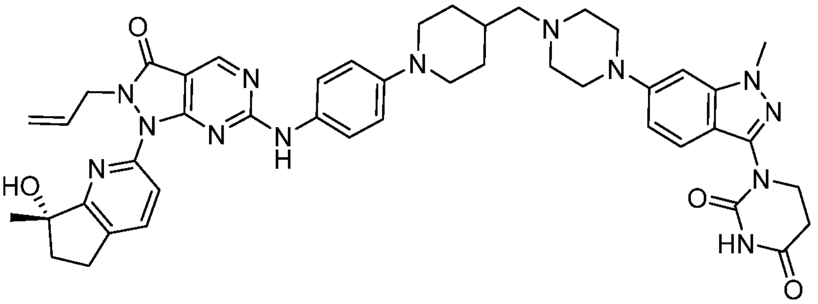 |
| 127 | 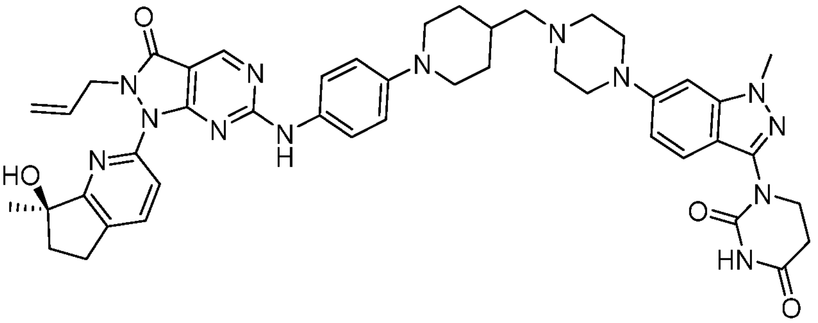 |
| 128 | 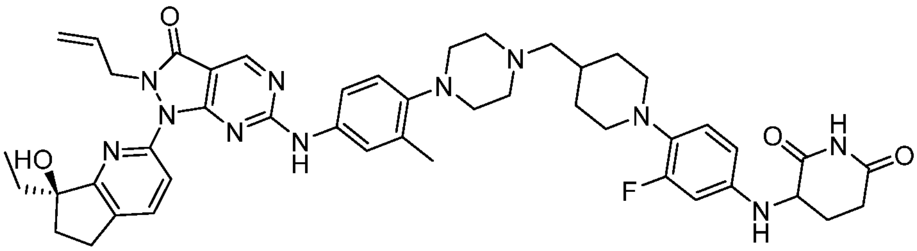 |

(continued)

| compound | Structure |
|---|---|
| 129 | 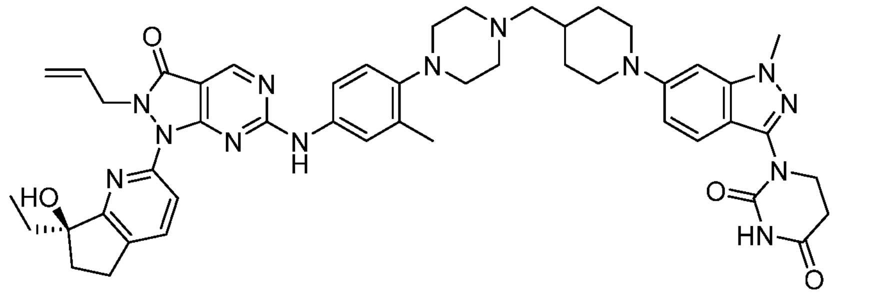 |
| 130 | 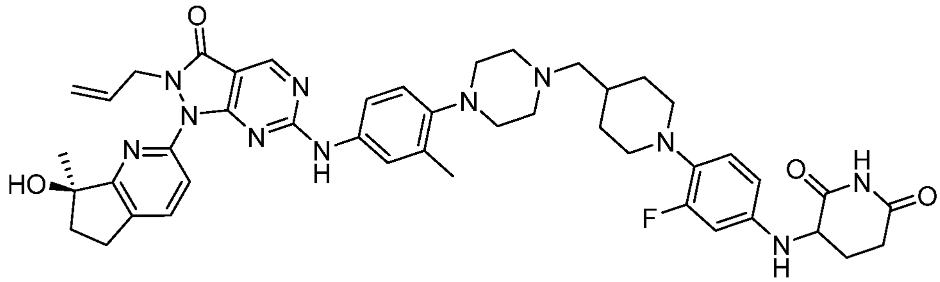 |
| 131 | 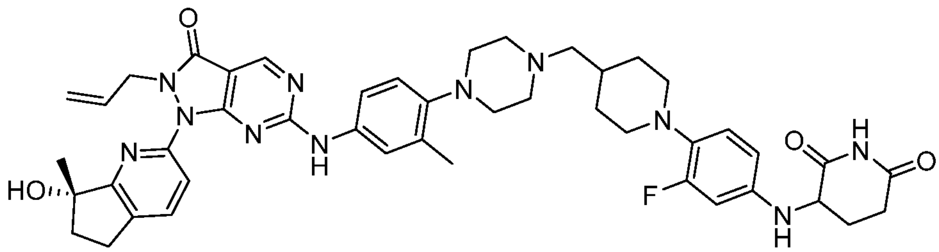 |
| 132 | 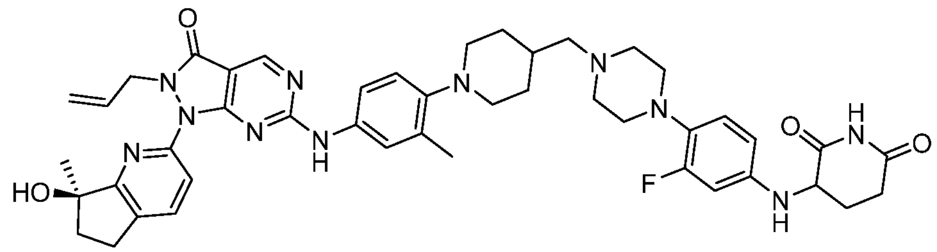 |
| 133 | 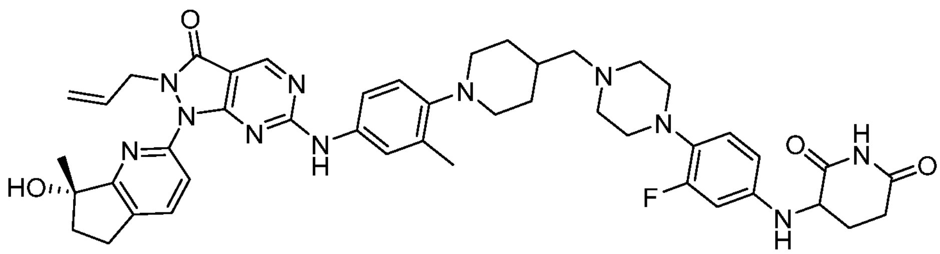 |

(continued)

| compound | Structure |
|---|---|
| 134 | 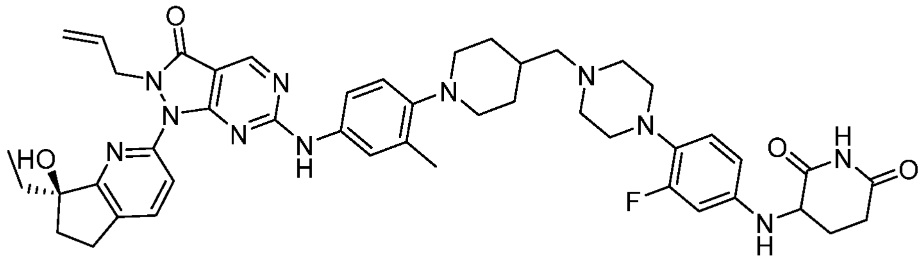 |
| 135 | 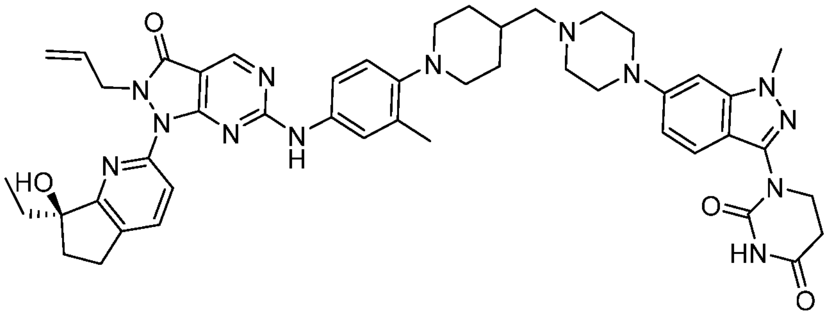 |
| 136 | 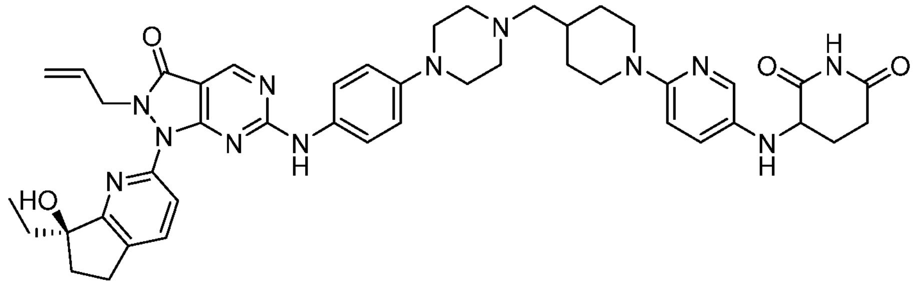 |
| 137 | 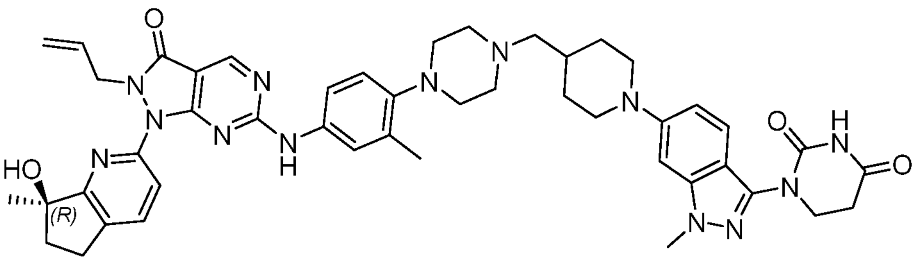 |
| 138 | 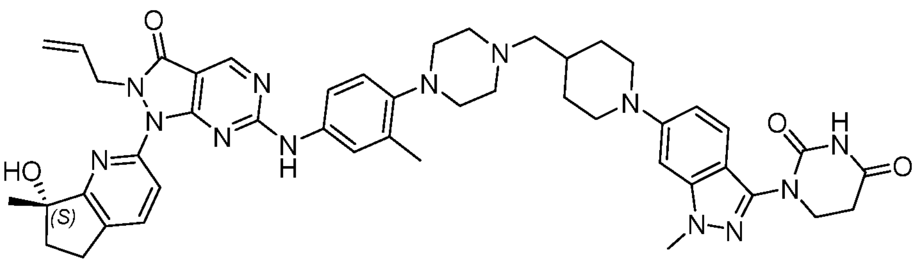 |

(continued)

| compound | Structure |
|---|---|
| 139 | 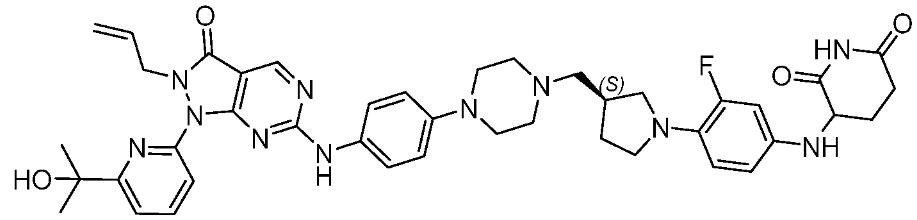 |
| 140 | 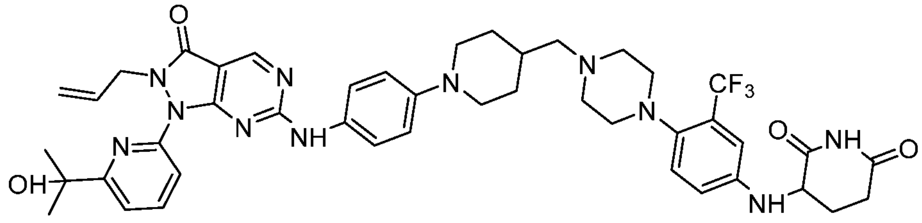 |
| 141 | 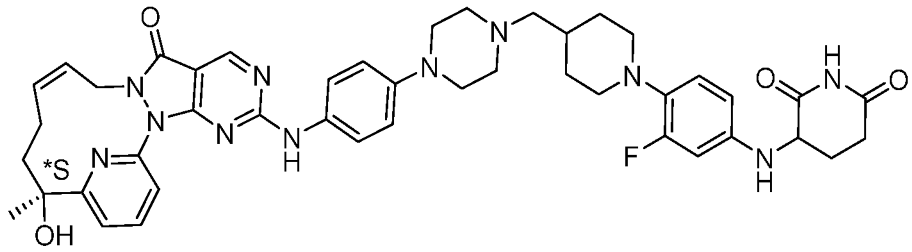 |
| 142 | 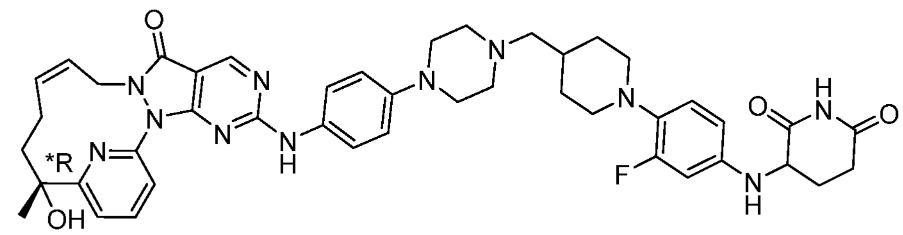 |
| 143 | 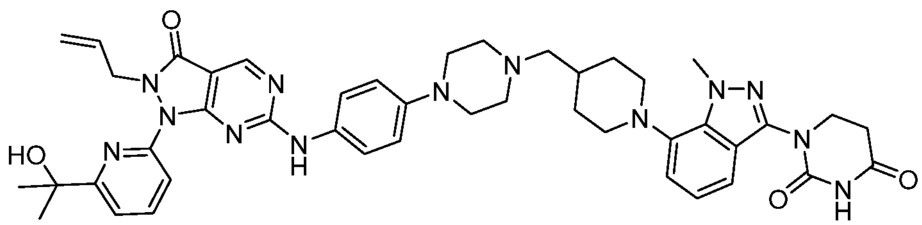 |
| 144 | 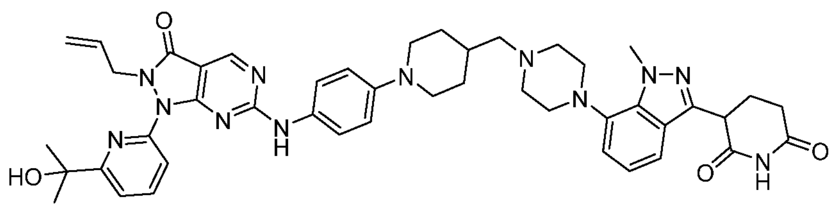 |

(continued)

| compound | Structure |
|---|---|
| 145 | 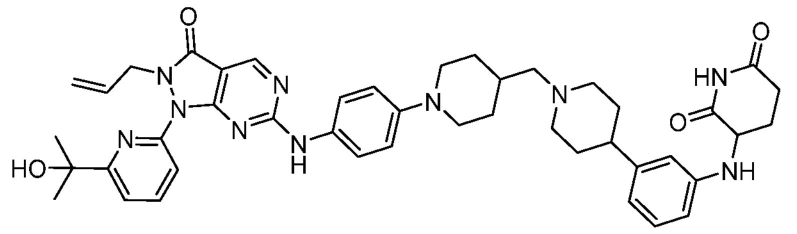 |
| 146 | 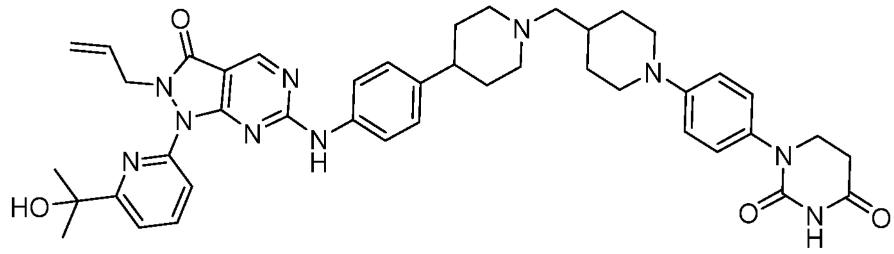 |
| 147 | 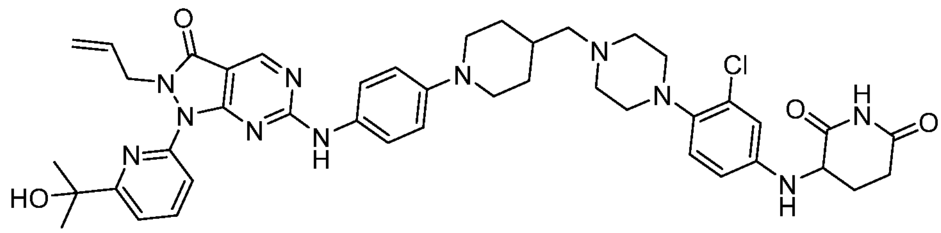 |
| 148 | 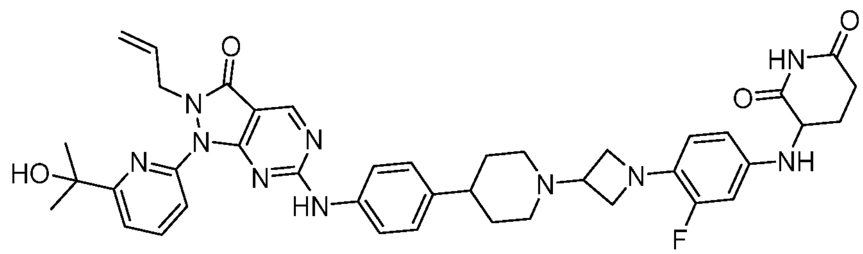 |
| 149 | 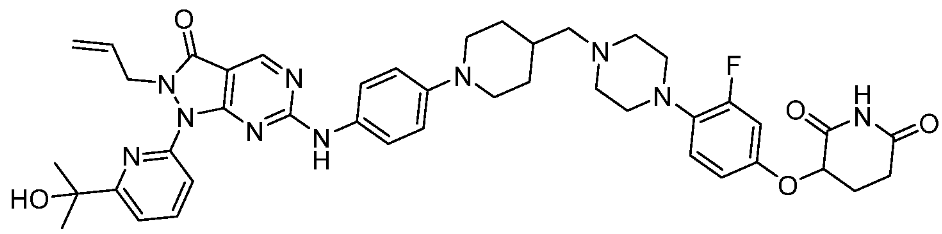 |
| 150-A | 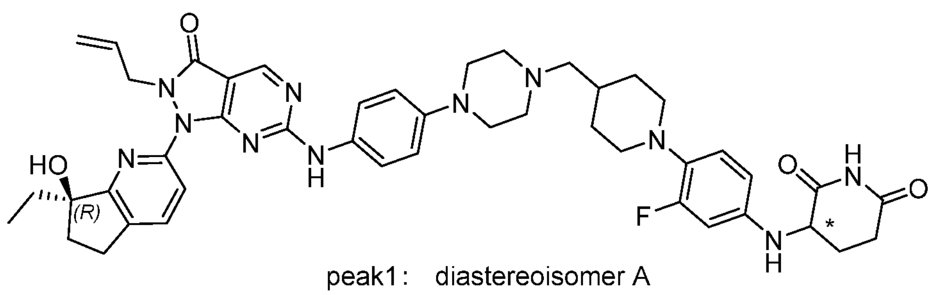 peak1: diastereoisomer A |

(continued)

| compound | Structure |
|---|---|
| 150-B | 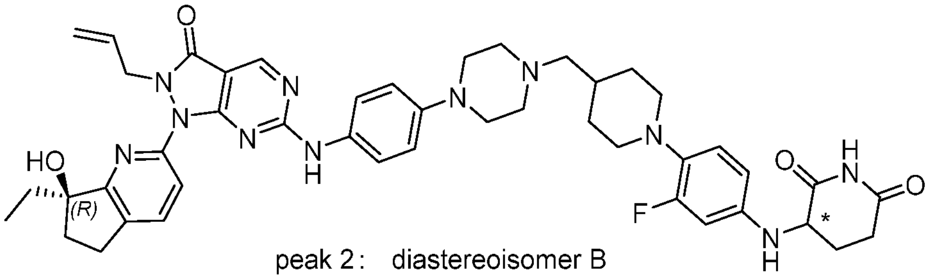 peak 2: diastereoisomer B |
| 152-A | 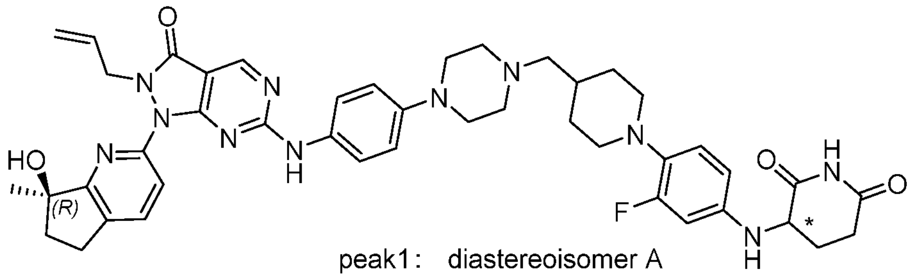 peak1: diastereoisomer A |
| 152-B | 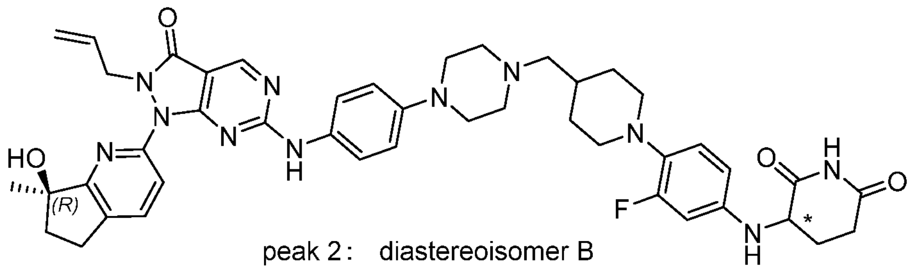 peak 2: diastereoisomer B |
| 154 | 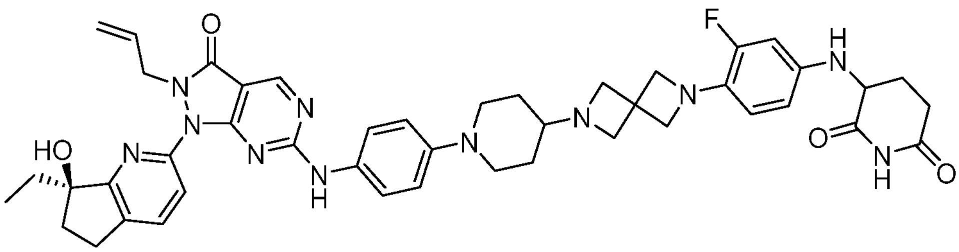 |
| 155 | 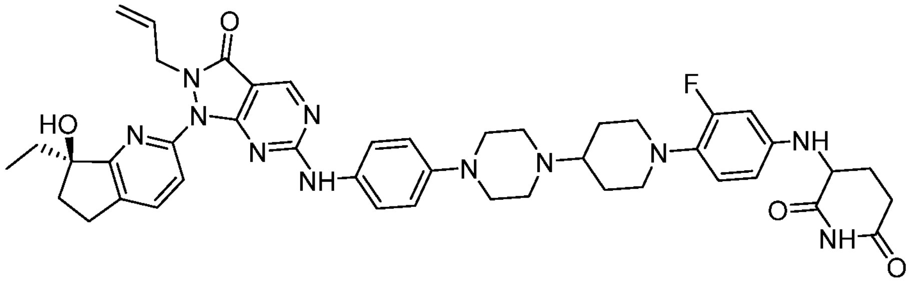 |
| 156 | 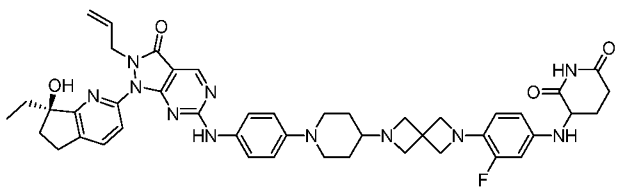 |

(continued)

| compound | Structure |
|---|---|
| 157 | 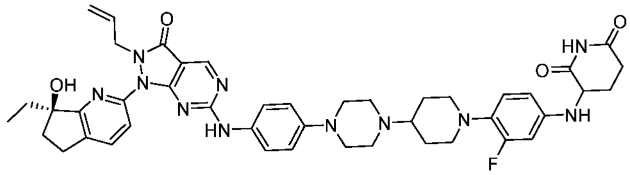 |
| 158 | 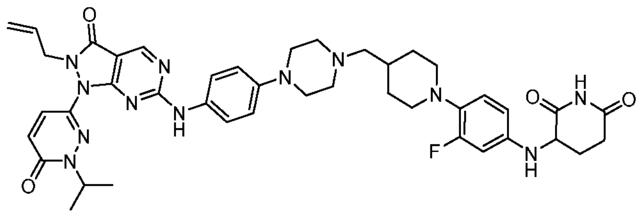 |
| 159 | 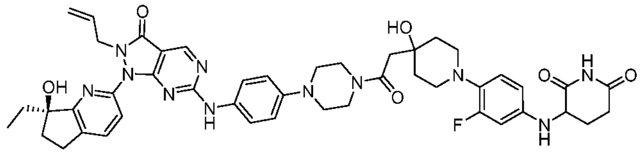 |
| 160 | 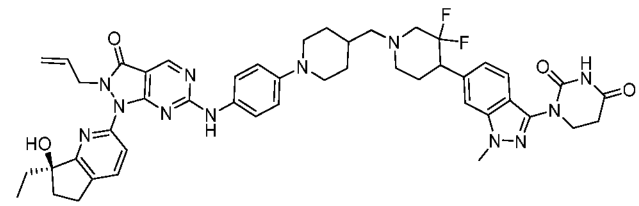 |
| 161 | 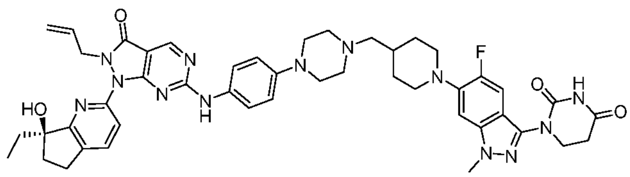 |
| 162 | 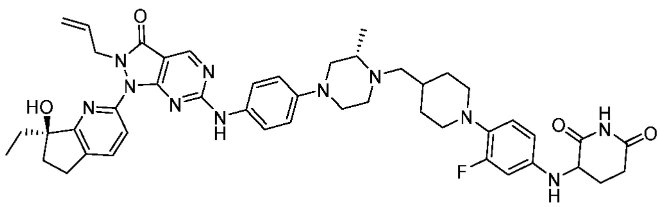 |
| 163 | 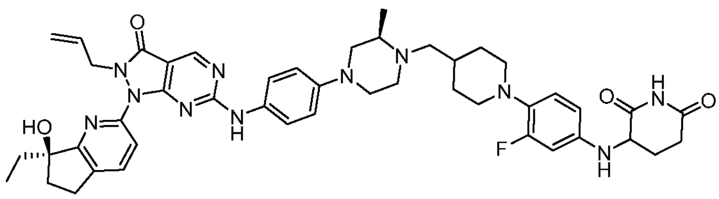 |

(continued)

| compound | Structure |
|---|---|
| 164 | 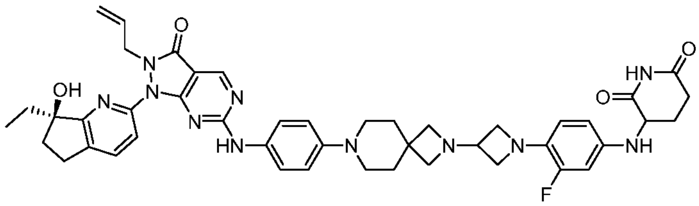 |
| 165 | 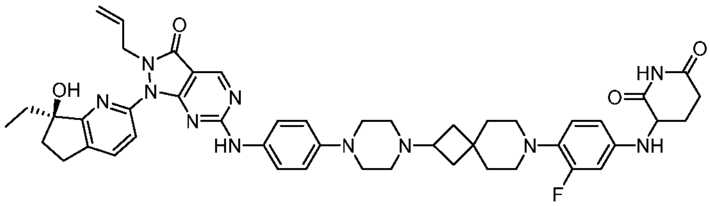 |
| 166 | 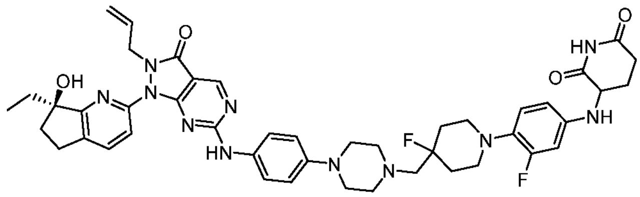 |
| 167 | 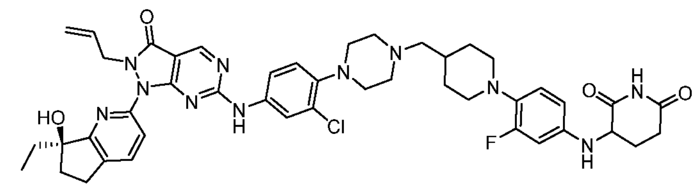 |
| 168 | 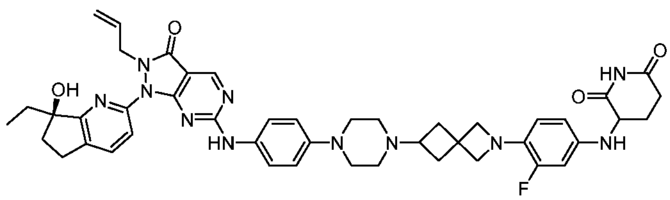 |
| 169 | 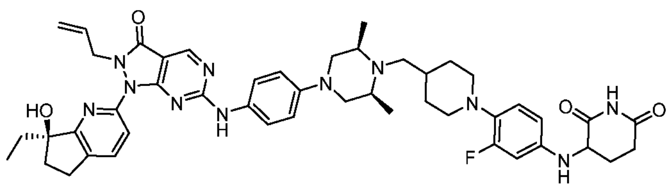 |
| 170 | 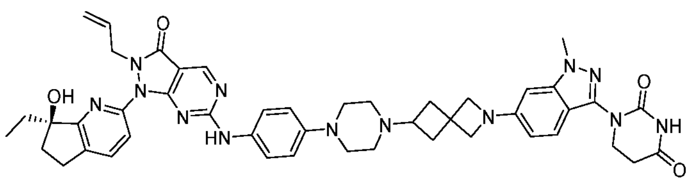 |

(continued)

| compound | Structure |
|---|---|
| 171 | 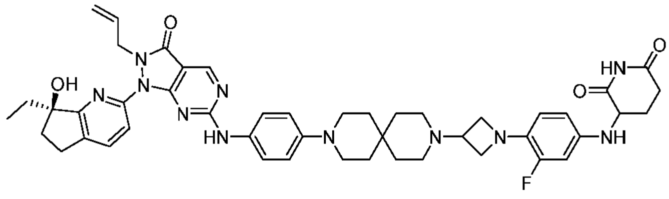 |
| 172 | 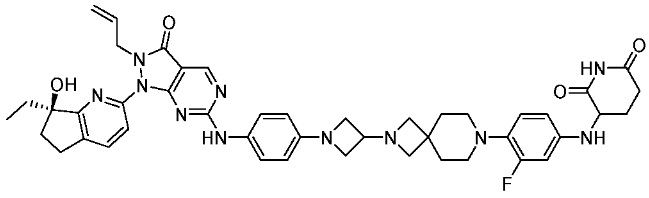 |
| 173 | 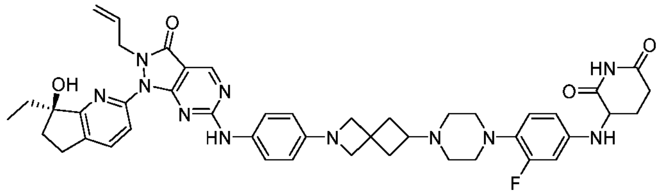 |
| 174 | 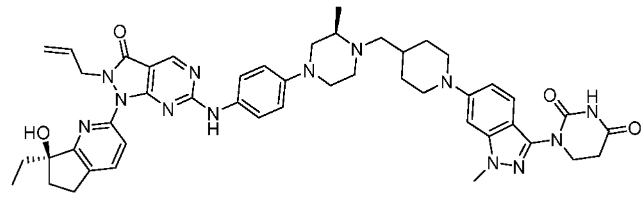 |
| 175 | 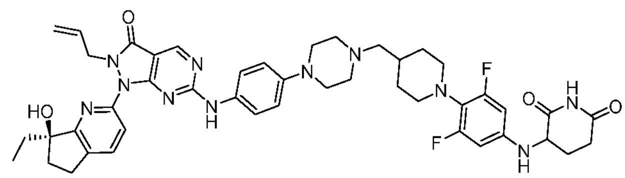 |
| 176 | 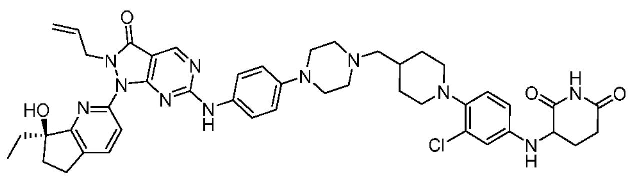 |
| 177 | 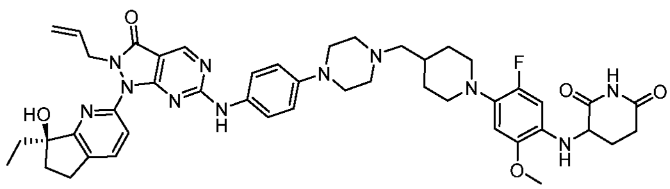 |
| 178 | 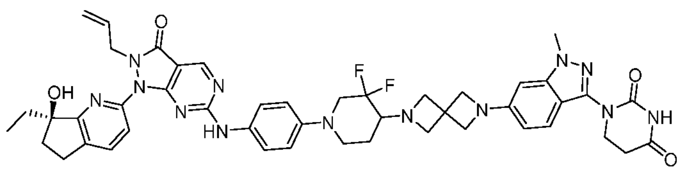 |

(continued)

| compound | Structure |
|---|---|
| 179 | 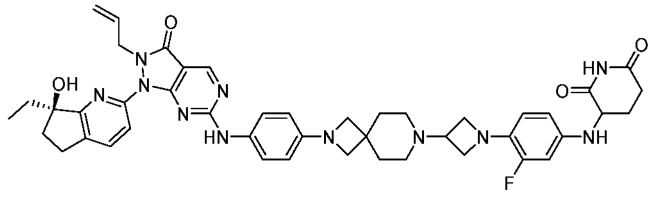 |
| 180 | 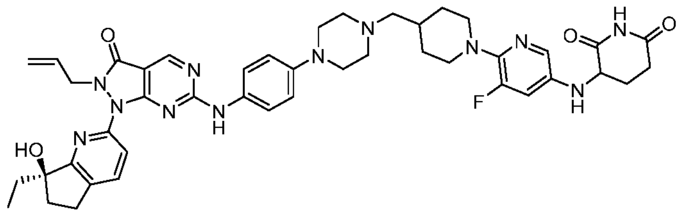 |
| 181 | 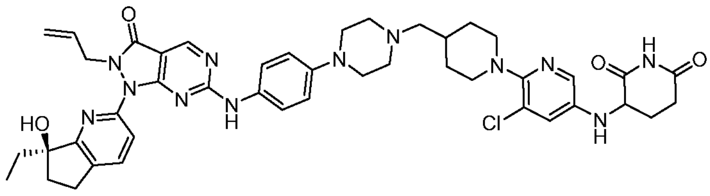 |
| 182 | 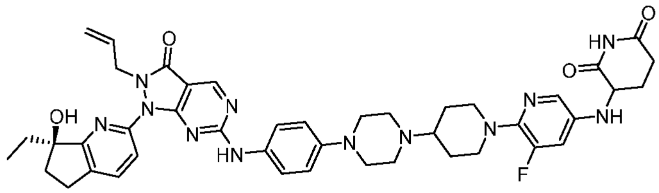 |
| 183 | 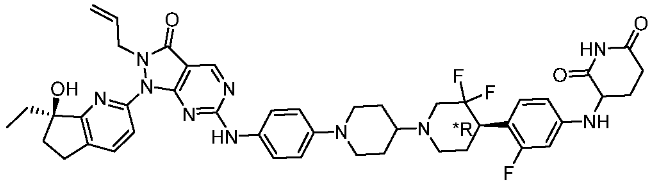 |
| 184 | 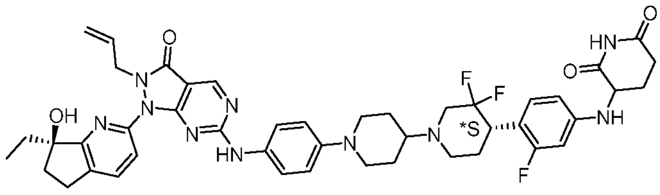 |
| 185 | 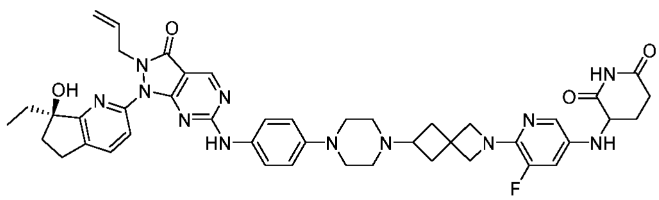 |

(continued)

| compound | Structure |
|---|---|
| 186 | 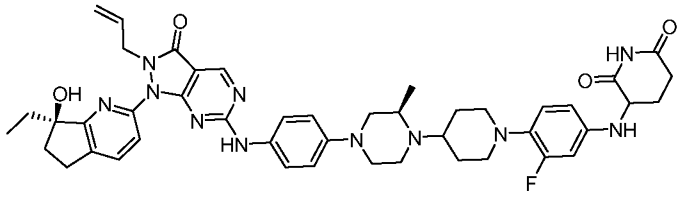 |
| 187 | 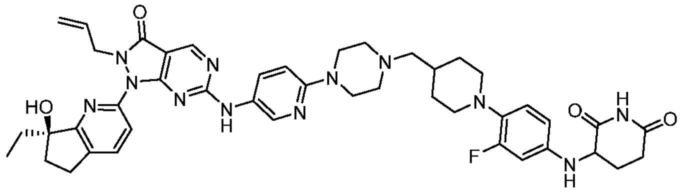 |
| 188 | 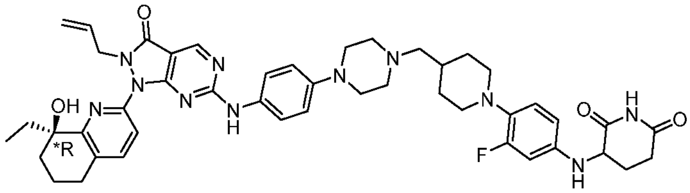 |
| 190 | 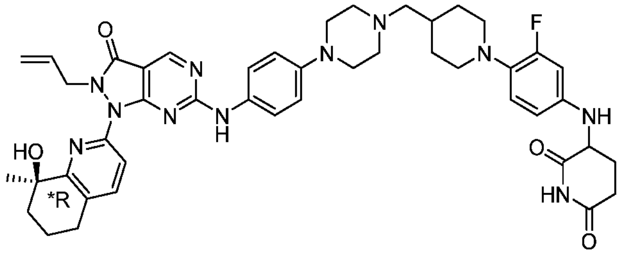 |
| 191 | 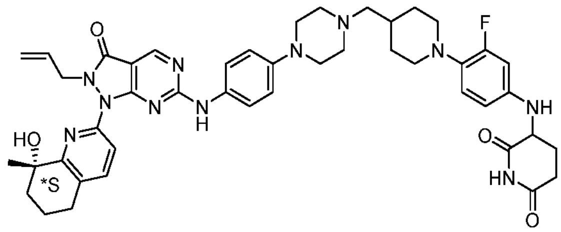 |
| 192 | 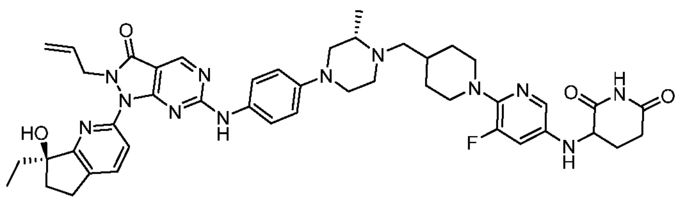 |
| 193 | 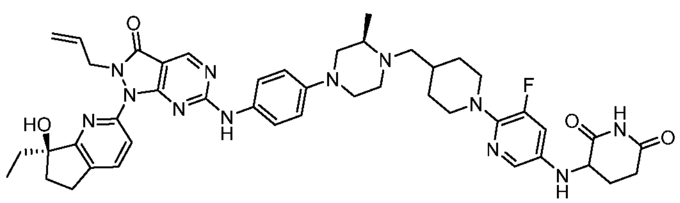 |

(continued)

| compound | Structure |
|---|---|
| 194 | 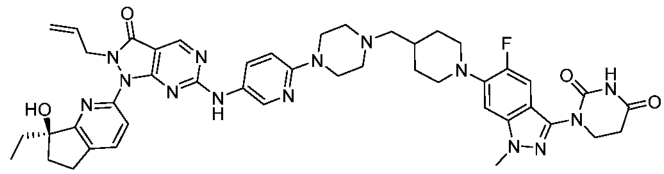 |
| 195 | 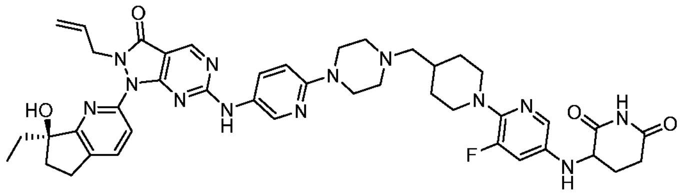 |
| 196 | 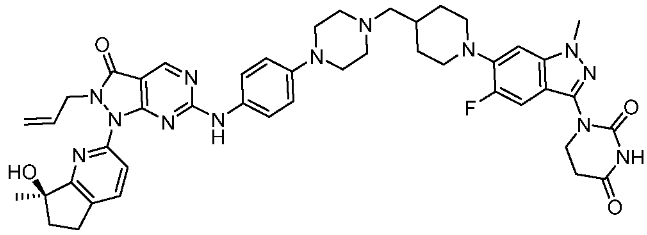 |
| 197 | 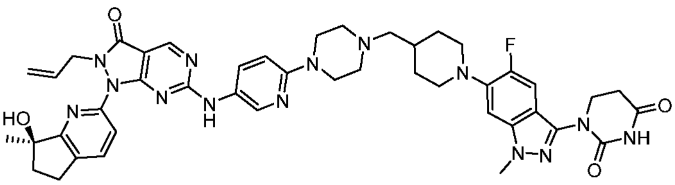 |
| 198 | 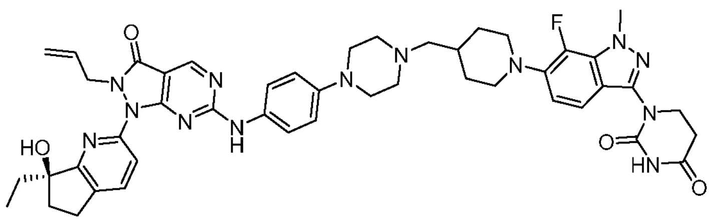 |
| 199 | 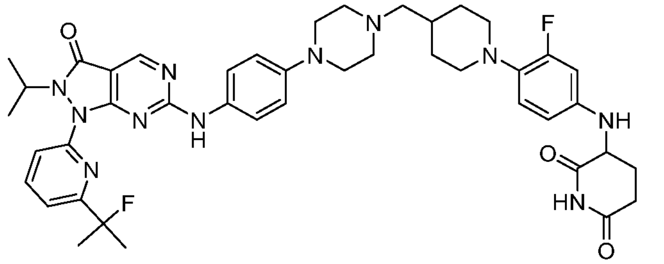 |
| 200 | 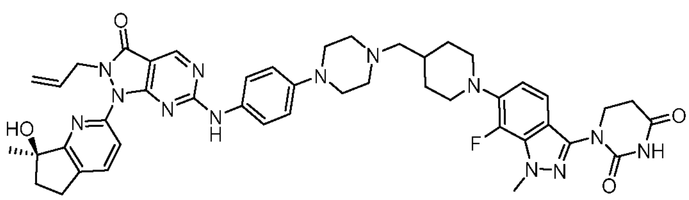 |

(continued)

| compound | Structure |
|---|---|
| 201 | 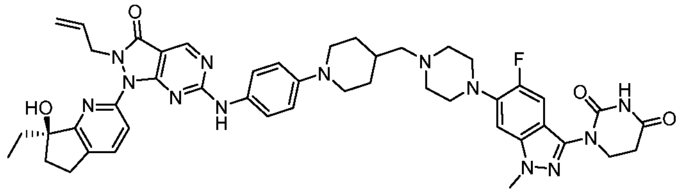 |
| 202 | 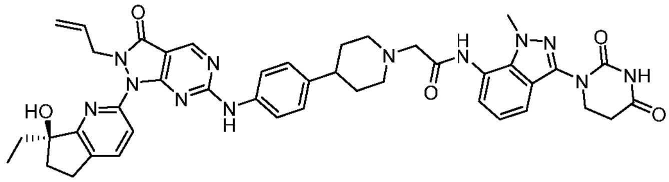 |
| 203 | 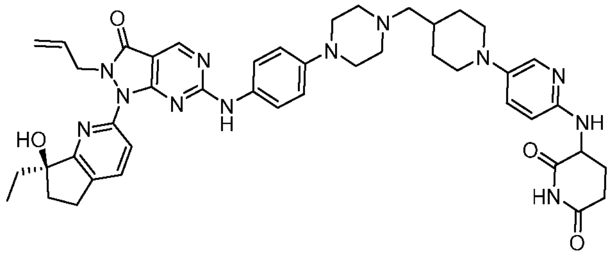 |
| 204 | 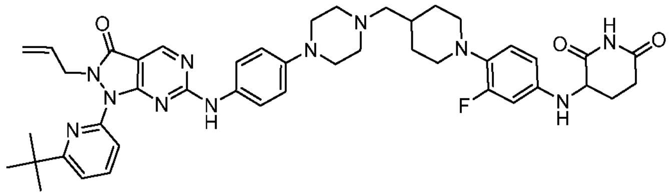 |
| 205 | 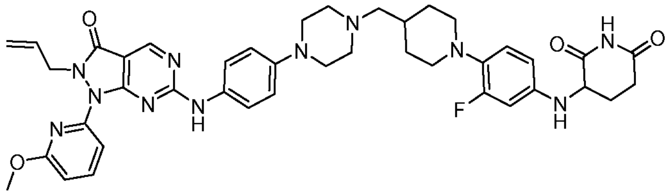 |
| 206 | 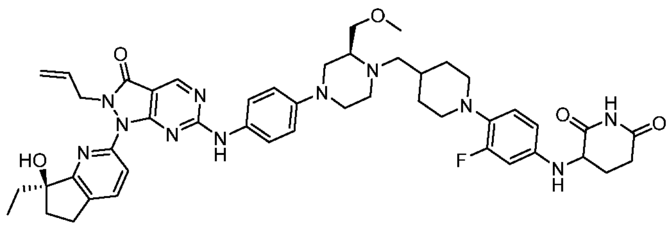 |

**Example 300:**

**Wee1 protein degradation** assay **(HiBiT** assay)

The following is an example of an assay that can be used to determine Wee1 degradation activity of compounds in the HT-1080 cell line. HT-1080 cell line was purchased from ATCC (Cat# CC-121), which was then engineered to stably express wild-type CRBN, GSPT (Δ1-138)/G575N mutant and HiBiT tagged Wee1. Cells were cultured in DMEM medium supplemented with 10% heat-inactivated FBS. For Wee1 degradation assay, about 10,000 engineered HT-1080 cells in 35 μl culture medium were seeded into 384-well plates (Cat# 3764, Coming), pre-spotted with compounds

using the Echo 650 liquid handler (Beckman). The half-log dose-response-curve (DRC) typically has 10 points with the highest concentration at 10 μM, and the lowest concentration at 0.316 nM. Assay plates were incubated at 37°C with 5% $CO_2$ for 20 hours. Wee1 degradation was then assessed using the Nano-Glo HiBiT Lytic Detection Reagent according to the manufacturer's instructions. Luminescence was measured on a Pherastar microplate reader (BMG Labtech). The data was processed using Collaborative Drug Discovery Vault and DMSO control was used as the reference value to calculate degradation values for each treated sample (% compared to DMSO control). A four Parameter Logistic Model was used to determine the compound's $EC_{50}$ and $DC_{50}$, using the following equation:

$$y = (A+ ((B-A)/ (1+ ((C/x)AD))))$$

A = $Y_{min}$ (lowest Wee1 level normalized to DMSO control in response to compound treatment, as determined by curve fit)

B = $Y_{max}$ (highest value, Wee1 value in DMSO control)

C = $EC_{50}$

D = Hill Slope

x = compound concentration

$EC_{50}$ = the concentration of compound when y = ($Y_{max}$-$Y_{min}$)/2

$DC_{50}$ = the concentration of the sample when y = 50% of DMSO control (50% Wee1 degradation)

y = Wee1 protein level normalized to DMSO control

$$D_{max} = (1- Y_{min}/Y_{max}) * 100\%$$

$D_{max}$ represented the maximum percentage of Wee 1 degradation that could be achieved by a compound at its highest treatment concentration.

**NCI-H1048 cell proliferation assay (CTG assay)**

The following is an example of an assay that can be used to determine the anti-proliferative activity of Wee1 PROTACs in a small cell lung cancer cell line, NCI-H1048 (ATCC Cat# CRL-5853). This cell line was cultured in RPMI-1640 medium supplemented with 10% FBS. About 1,200 NCI-H1048 cells in 50 μL of culture medium were seeded into a 384-well plate (Cat# 3764, Corning), pre-spotted with compounds using the Echo 650 liquid handler (Beckman). The dose-response-curve (DRC) typically has 10-points with a highest concentration at 10 μM, and half-log diluted to as low as 0.316 nM, plus a DMSO control. Assay plates were incubated at 37 °C with 5% $CO_2$ for 120 hours. After that, 20 μL of the CellTiter-Glo (CTG) Luminescent Cell Viability Assay Reagent (Cat# G7573, Promega) was added to each well and incubated at room temperature for 30 minutes. Luminescence was then measured with Pherastar microplate reader (BMG Labtech). All percentage of cell growth inhibition curves were processed using Collaborative Drug Discovery Vault.

Cell viability reads in compound-treated wells were normalized to that of DMSO control and expressed as Percent of Control (PoC, y). A four Parameter Logistic Model was used to determine the compound's $EC_{50}$ and $DC_{50}$, using the following equation:

$$y = (A+ ((B-A)/ (1+ ((C/x)AD))))$$

A = $Y_{min}$ (lowest cell viability read normalized to DMSO control in response to compound treatment, as determined by curve fit)

B = $Y_{max}$ (cell viability read in DMSO control)

C = $EC_{50}$

D = Hill Slope

x = compound concentration

$EC_{50}$ = the concentration of compound when y = $(Y_{max}-Y_{min})/2$

$IC_{50}$ = the concentration of the compound when y = 50% of DMSO control

y = cell viability read normalized to DMSO control

Table 2. The Wee1 degradation activities and the anti-proliferative activities of representative compounds.

"+++" represents $DC_{50} \leq 100$ nM in the HiBiT assay; "++" represents 100 nM < $DC_{50} \leq 1$ μM in the HiBiT assay; "+" represents $DC_{50} > 1$ μM in the HiBiT assay.
"A" represents $D_{max} \geq 75\%$ in the HiBiT assay; "B" represents $50\% \leq D_{max} < 75\%$ in the HiBiT assay; "C" represents $D_{max} < 50\%$ in the HiBiT assay.
"***" represents $IC_{50} \leq 25$ nM in the CTG assay in H1048 cell line; "**" represents 25 nM < $IC_{50} \leq 100$ nM in the CTG assay in H1048 cell line; "*" represents $IC_{50} > 100$ nM in the CTG assay in H1048 cell line.

| Compound No. | HiBiT Wee1: $DC_{50}$ | HiBiT Wee1: $D_{max}$ | CTG cellular assay _H1048: $IC_{50}$ |
|---|---|---|---|
| 1 | + | C | |
| 2 | +++ | B | ** |
| 3 | +++ | B | ** |
| 4 | + | C | |
| 5 | + | C | |
| 6 | + | C | * |
| 7 | + | C | * |
| 8 | +++ | B | ** |
| 9 | +++ | B | ** |
| 10 | +++ | B | ** |
| 11 | +++ | B | |
| 12 | +++ | B | |
| 13 | +++ | B | |
| 14 | +++ | B | ** |
| 15 | ++ | B | |
| 16 | + | C | * |
| 17 | + | C | * |
| 18 | + | C | * |
| 19 | + | C | |
| 20 | + | C | |
| 21 | +++ | B | ** |
| 22 | + | C | |
| 23 | + | C | |
| 24 | + | C | |

(continued)

| "+++" represents $DC_{50} \leq 100$ nM in the HiBiT assay; "++" represents 100 nM < $DC_{50} \leq 1$ μM in the HiBiT assay; "+" represents $DC_{50} > 1$ μM in the HiBiT assay.<br>"A" represents $D_{max} \geq 75\%$ in the HiBiT assay; "B" represents 50% ≤ $D_{max}$ < 75% in the HiBiT assay; "C" represents $D_{max}$ < 50% in the HiBiT assay.<br>"***" represents $IC_{50} \leq 25$ nM in the CTG assay in H1048 cell line; "**" represents 25 nM < $IC_{50} \leq 100$ nM in the CTG assay in H1048 cell line; "*" represents $IC_{50} > 100$ nM in the CTG assay in H1048 cell line. | | | |
|---|---|---|---|
| Compound No. | HiBiT Wee1: $DC_{50}$ | HiBiT Wee1: $D_{max}$ | CTG cellular assay _H1048: $IC_{50}$ |
| 25 | +++ | B | |
| 26 | + | C | |
| 27 | + | C | |
| 28 | +++ | B | |
| 29 | + | C | |
| 30 | + | C | |
| 31 | + | C | |
| 32 | ++ | B | |
| 33 | + | C | |
| 34 | + | C | |
| 35 | + | C | |
| 36 | +++ | B | |
| 37 | +++ | B | ** |
| 38 | +++ | B | |
| 39 | ++ | B | |
| 40 | +++ | B | |
| 41 | +++ | B | |
| 42 | + | B | |
| 43 | +++ | A | |
| 44 | +++ | B | |
| 45 | +++ | A | |
| 46 | +++ | B | ** |
| 47 | ++ | B | *** |
| 48 | +++ | B | ** |
| 49 | + | C | |
| 50 | +++ | B | |
| 51 | +++ | B | *** |
| 52 | +++ | B | *** |
| 53 | + | C | |
| 54 | +++ | B | ** |
| 55 | +++ | B | *** |
| 56 | +++ | B | |

(continued)

| "+++" represents $DC_{50} \leq 100$ nM in the HiBiT assay; "++" represents 100 nM < $DC_{50} \leq 1$ μM in the HiBiT assay; "+" represents $DC_{50} > 1$ μM in the HiBiT assay.<br>"A" represents $D_{max} \geq 75\%$ in the HiBiT assay; "B" represents 50% ≤ $D_{max}$ < 75% in the HiBiT assay; "C" represents $D_{max}$ < 50% in the HiBiT assay.<br>"***" represents $IC_{50} \leq 25$ nM in the CTG assay in H1048 cell line; "**" represents 25 nM < $IC_{50} \leq 100$ nM in the CTG assay in H1048 cell line; "*" represents $IC_{50}$ > 100 nM in the CTG assay in H1048 cell line. | | | |
|---|---|---|---|
| Compound No. | HiBiT Wee1: $DC_{50}$ | HiBiT Wee1: $D_{max}$ | CTG cellular assay _H1048: $IC_{50}$ |
| 57 | +++ | B | |
| 58 | + | C | |
| 59 | + | C | |
| 60 | + | C | |
| 61 | +++ | B | *** |
| 62 | +++ | A | |
| 63 | +++ | B | |
| 64 | +++ | B | |
| 65 | +++ | B | *** |
| 66 | +++ | B | *** |
| 67 | +++ | A | *** |
| 68 | +++ | B | |
| 69 | + | C | |
| 70 | +++ | A | *** |
| 71 | +++ | A | *** |
| 72 | +++ | A | *** |
| 73 | +++ | A | |
| 74 | +++ | A | |
| 75 | +++ | B | |
| 76 | +++ | B | *** |
| 77 | +++ | B | |
| 78 | ++ | B | |
| 79 | +++ | B | *** |
| 80 | +++ | A | *** |
| 81 | +++ | A | *** |
| 82 | +++ | A | *** |
| 83 | +++ | A | |
| 84 | +++ | B | |
| 85 | +++ | B | *** |
| 86 | +++ | B | *** |
| 87 | +++ | A | *** |
| 88 | +++ | A | |

(continued)

| "+++" represents $DC_{50} \leq 100$ nM in the HiBiT assay; "++" represents 100 nM < $DC_{50} \leq 1$ μM in the HiBiT assay; "+" represents $DC_{50}$ > 1 μM in the HiBiT assay.<br>"A" represents $D_{max} \geq 75\%$ in the HiBiT assay; "B" represents 50% ≤ $D_{max}$ < 75% in the HiBiT assay; "C" represents $D_{max}$ < 50% in the HiBiT assay.<br>"***" represents $IC_{50} \leq 25$ nM in the CTG assay in H1048 cell line; "**" represents 25 nM < $IC_{50} \leq 100$ nM in the CTG assay in H1048 cell line; "*" represents $IC_{50}$ > 100 nM in the CTG assay in H1048 cell line. | | | |
|---|---|---|---|
| Compound No. | HiBiT Wee1: $DC_{50}$ | HiBiT Wee1: $D_{max}$ | CTG cellular assay _H1048: $IC_{50}$ |
| 89 | +++ | A | *** |
| 90 | +++ | A | |
| 91 | +++ | A | *** |
| 92 | +++ | A | *** |
| 93 | +++ | A | *** |
| 94 | +++ | A | |
| 95 | +++ | A | *** |
| 96 | +++ | B | *** |
| 97 | | B | *** |
| 98 | +++ | B | |
| 99 | +++ | B | |
| 100-A | +++ | A | *** |
| 100-B | +++ | A | *** |
| 102-A | +++ | A | |
| 102-B | +++ | A | |
| 104 | +++ | A | *** |
| 105 | +++ | A | *** |
| 106 | +++ | A | *** |
| 107 | +++ | A | |
| 108 | +++ | B | *** |
| 109 | +++ | B | *** |
| 110 | +++ | A | *** |
| 111 | +++ | B | *** |
| 112 | +++ | A | |
| 113 | +++ | A | |
| 114 | +++ | A | *** |
| 115 | +++ | A | *** |
| 116 | +++ | A | *** |
| 117 | +++ | A | *** |
| 118 | ++ | B | |
| 119 | +++ | A | *** |
| 120 | +++ | B | |

(continued)

| "+++" represents $DC_{50} \leq 100$ nM in the HiBiT assay; "++" represents 100 nM < $DC_{50} \leq 1$ μM in the HiBiT assay; "+" represents $DC_{50}$ > 1 μM in the HiBiT assay.<br>"A" represents $D_{max} \geq 75\%$ in the HiBiT assay; "B" represents 50% ≤ $D_{max}$ < 75% in the HiBiT assay; "C" represents $D_{max}$ < 50% in the HiBiT assay.<br>"***" represents $IC_{50} \leq 25$ nM in the CTG assay in H1048 cell line; "**" represents 25 nM < $IC_{50} \leq 100$ nM in the CTG assay in H1048 cell line; "*" represents $IC_{50}$ > 100 nM in the CTG assay in H1048 cell line. | | | |
|---|---|---|---|
| Compound No. | HiBiT Wee1: $DC_{50}$ | HiBiT Wee1: $D_{max}$ | CTG cellular assay _H1048: $IC_{50}$ |
| 121 | +++ | A | *** |
| 122 | +++ | A | |
| 123 | +++ | A | *** |
| 124 | +++ | B | |
| 125 | +++ | A | ** |
| 126 | +++ | B | |
| 127 | +++ | A | |
| 128 | +++ | A | |
| 129 | +++ | B | *** |
| 130 | +++ | A | |
| 131 | +++ | A | |
| 132 | +++ | A | |
| 133 | +++ | A | |
| 134 | +++ | A | |
| 135 | +++ | A | |
| 136 | +++ | A | |
| 137 | +++ | A | *** |
| 138 | +++ | B | |
| 139 | +++ | A | |
| 140 | ++ | A | |
| 141 | +++ | A | *** |
| 142 | +++ | B | |
| 143 | +++ | B | |
| 144 | +++ | A | *** |
| 145 | ++ | B | |
| 146 | + | C | |
| 147 | +++ | A | |
| 148 | +++ | A | |
| 149 | +++ | A | |
| 150-A | +++ | A | *** |
| 150-B | +++ | A | *** |
| 152-A | +++ | A | *** |

(continued)

| "+++" represents $DC_{50} \leq 100$ nM in the HiBiT assay; "++" represents 100 nM < $DC_{50} \leq 1$ μM in the HiBiT assay; "+" represents $DC_{50} > 1$ μM in the HiBiT assay.<br>"A" represents $D_{max} \geq 75\%$ in the HiBiT assay; "B" represents 50% ≤ $D_{max}$ < 75% in the HiBiT assay; "C" represents $D_{max}$ < 50% in the HiBiT assay.<br>"***" represents $IC_{50} \leq 25$ nM in the CTG assay in H1048 cell line; "**" represents 25 nM < $IC_{50} \leq 100$ nM in the CTG assay in H1048 cell line; "*" represents $IC_{50} > 100$ nM in the CTG assay in H1048 cell line. | | | |
|---|---|---|---|
| Compound No. | HiBiT Wee1: $DC_{50}$ | HiBiT Wee1: $D_{max}$ | CTG cellular assay _H1048: $IC_{50}$ |
| 152-B | +++ | A | *** |
| 154 | +++ | A | |
| 155 | +++ | A | |
| 156 | +++ | A | *** |
| 157 | +++ | A | *** |
| 158 | +++ | A | *** |
| 159 | +++ | B | |
| 160 | +++ | B | |
| 161 | +++ | A | *** |
| 162 | +++ | A | *** |
| 163 | +++ | A | *** |
| 164 | +++ | A | |
| 165 | +++ | A | |
| 166 | +++ | A | ** |
| 167 | +++ | A | |
| 168 | +++ | A | *** |
| 169 | +++ | A | *** |
| 170 | +++ | A | |
| 171 | +++ | A | |
| 172 | +++ | B | |
| 173 | +++ | A | *** |
| 174 | +++ | B | *** |
| 175 | +++ | A | |
| 176 | +++ | A | *** |
| 177 | +++ | A | |
| 178 | +++ | B | |
| 179 | +++ | A | *** |
| 180 | +++ | A | *** |
| 181 | +++ | A | *** |
| 182 | +++ | A | *** |
| 183 | +++ | A | |
| 184 | +++ | A | |

(continued)

| "+++" represents $DC_{50}$ ≤ 100 nM in the HiBiT assay; "++" represents 100 nM < $DC_{50}$ ≤ 1 μM in the HiBiT assay; "+" represents $DC_{50}$ > 1 μM in the HiBiT assay.<br>"A" represents $D_{max}$ ≥ 75% in the HiBiT assay; "B" represents 50% ≤ $D_{max}$ < 75% in the HiBiT assay; "C" represents $D_{max}$ < 50% in the HiBiT assay.<br>"***" represents $IC_{50}$ ≤25 nM in the CTG assay in H1048 cell line; "**" represents 25 nM < $IC_{50}$ ≤ 100 nM in the CTG assay in H1048 cell line; "*" represents $IC_{50}$ > 100 nM in the CTG assay in H1048 cell line. | | | |
|---|---|---|---|
| Compound No. | HiBiT Wee1: $DC_{50}$ | HiBiT Wee1: $D_{max}$ | CTG cellular assay _H1048: $IC_{50}$ |
| 185 | +++ | A | *** |
| 186 | +++ | A | |
| 187 | +++ | A | *** |
| 188 | +++ | A | |
| 190 | +++ | A | |
| 191 | +++ | A | |
| 192 | +++ | A | *** |
| 193 | +++ | A | *** |
| 194 | +++ | A | *** |
| 195 | +++ | A | |
| 196 | +++ | A | *** |
| 197 | +++ | A | *** |
| 198 | +++ | A | *** |
| 199 | +++ | A | ** |
| 200 | +++ | A | *** |
| 201 | +++ | A | |
| 202 | + | C | |
| 203 | +++ | B | |
| 204 | +++ | A | |
| 205 | +++ | A | |
| 206 | +++ | A | |

Representive compounds shown in Table 2 exhibited excellent Wee1 degration activities and excellent anti-proliferative activities in H1048 cell line.

Example 301: Determination of Kinetic Solubility

Preparation of the sample, shaking and filtration

An aliquot of 30 μL stock solution (10 mM) was placed in order into their proper 96-well rack. 970 μL of PBS was added into each vial of the cap-less solubility sample plate. The assay was performed in duplicate. In each vial, a stir stick was added and sealed with a molded PTFE/Silicone plug. Then the solubility sample plate was transferred to the Eppendorf Thermomixer Comfort plate shaker, shaking at 25°C at 1100 rpm for 2 hours. Plugs were removed after 2 hours and the stir sticks were removed using a big magnet. Then the samples were transferred from the solubility sample plate into the filter plate. All the samples were filtered using the vacuum manifold. An aliquot of 5 μL filtrate was mixed with 5μL DMSO and 490 μL of a mixture of $H_2O$ and acetonitrile (v:v, 1:1) containing internal standard. A certain

proportion of ultrapure water was used to dilute according to the peak shape. The dilution factor was changed according to the solubility values and the LC-MS/MS signals.

Preparation of 3 μM Standards

An aliquot of 6 μL stock solution (10 mM in DMSO) was transferred from standard (STD) stock plate into a new plate, and then 194 μL of DMSO was added to get a STD working solution of 300 μM. Then 5 μL of 300 μM working solution was mixed with 5μE of DMSO and 490 μL of a mixture of $H_2O$ and acetonitrile (v:v, 1:1) containing internal standard and the final STD concentration was 3 μM. The STD concentration were changed according to the LC-MS/MS signals.

The sample plate was put into the autosampler and the test compound concentration was determined by LC-MS/MS.

Table 3 Kinetic solubility data of the present invented compounds.

| | Compound 55 | Compound 66 | Compound 61 | Compound 110 |
|---|---|---|---|---|
| Kinetic solubility (μM) pH 3.0 | 194 | 252 | 299 | 233 |
| Kinetic solubility (μM) pH 5.0 | 55.7 | 148 | 203 | 132 |

The compounds of the present invention showed good kinetic solubility.

**Example 302: Permeability study for inventive compounds**

MDCKII (Madin-Darby Canine Kidney cell line, wild type) cell was obtained from the Netherlands Cancer Institute, which was used for P-gp substrates or inhibitors screening and predicting the permeability of compounds with high efflux barriers such as duodenum, blood-brain barrier, hepatocyte nucleus and renal unit. The MDCKII cells with 10 to 22 passages were used for the permeability investigation.

MDCKII cells were cultured using Minimum Essential Media (MEM) at 37°C, 5% $CO_2$ and 95% relative humidity. The cells were seeded in Transwell plate with $5.45\times10^5$ cells/$cm^2$. Then the cells were incubated in $CO_2$ Incubator for 4-7 days for transport studies.

Preparation of MEM cell culture medium:

The complete medium was prepared by adding 10% FBS (fetal bovine serum) and 1% glutamax-I to 500ml MEM.

The information of reagent is shown below:

| Reagent | Sponsor | Cat. No. | Lot. No. |
|---|---|---|---|
| Fetal Bovine Serum | VWR | 76294-180 | 234D19 |
| MEM Medium | Gibco | 10370-021 | 2187093 |
| GlutaMax-I | Gibco | 35050-061 | 2186982 |

The dose concentrations of test compounds and control compounds were 1 μM. The bidirectional (A to B and B to A) permeability was evaluated. All incubations are performed in duplicate.

The regents were pre-incubated at 37±1°C for 30 minutes. To determine the transport rate from apical to basolateral direction, 125 μL working solution (1 μM) was added to the Transwell insert (apical compartment), and 50 μL sample was immediately transferred from the apical compartment to a new 96-well plate as the initial donor sample (A-B) and mixed with 200 μL of acetonitrile or methanol containing IS. And 235 μL HBSS (10 mM HEPES, pH 7.4) was added to the receiver plate (basolateral compartment). To determine the transport rate from basolateral to apical direction, 285 μL working solution (1 μM) was added to the receiver plate (basolateral compartment), and 50 μL sample was immediately transferred from the basolateral compartment to a new 96-well plate as the initial donor sample (B-A) and mixed with 200 μL of acetonitrile or methanol containing IS. And 75 μL HBSS (10 mM HEPES, pH 7.4) was added to the Transwell insert (apical compartment). Then the plate was incubated at 37±°C with 5% $CO_2$ and 95% relative humidity for 120 minutes.

At the end of incubation, 50 μL samples from donor and receiver sides were transferred into a new 96-well plate. 200 μL of ice-cold acetonitrile or methanol containing appropriate internal standards (IS) was mixed with the samples. After vortex for 10 minutes, the samples are centrifuged at 3220 g for 40 minutes. An aliquot of 100 μL of the supernatant was mixed with appropriate volume of ultra-pure water and was analyzed by LC-MS/MS. All incubations

are performed in duplicate.

The integrity of MDCKII cells were checked by Lucifer Yellow Rejection Assay after transport assay. The plate(s) were incubated with Lucifer Yellow solution at 37°C for 30 minutes. The Lucifer Yellow fluorescence (to monitor monolayer integrity) was measured in a fluorescence plate reader at 480 nm excitation and 530 nm emission.

Test and reference compounds (metoprolol and digoxin) were semi-quantitatively analyzed, and the peak area ratio of the analyte and the internal standard was used to evaluate the concentration of the compounds.

Table 5 Permeability ($10^{-6}$ cm/s) data of representative compounds of the present invention.

| | **Compound 41** | **Compound 55** | **Compound 65** | **Compound 110** |
|---|---|---|---|---|
| A to B | 0.52 | 2.48 | 1.35 | 2.37 |
| B to A | 1.41 | 7.09 | 1.27 | 2.11 |
| Efflux ratio | 2.73 | 2.88 | 0.94 | 0.89 |

The compounds of the present invention showed good permeability.

**Example 303: Pharmacokinetic study in mice.**

Objective: the objective of this study was to determine the pharmacokinetics of test compound following intravenous (IV) and oral (PO) administration to male CD1 mice. Materials: Male CD1 mouse (20-30g, 6-8 weeks, Zhejiang Vital River Laboratory Animal Technology Co., Ltd.).

The CD1 mice were administered with the test article intravenously via tail vein at 2 mg/kg or orally at 10 mg/kg. The animals had free access to food and water. After dose administration, blood was collected from the dorsal metatarsal vein at 5 min (0.0833 h, IV only), 15 min (0.25 h), 30 min (0.5 h), 60 min (1h), 2h, 4h, 6h, 8h and 24h. Blood samples were placed in an anticoagulant tube with EDTA-K2, mixed adequately and centrifuged at 4°C, 4000 g for 5 minutes to get the plasma. The plasma samples were stored at -75±15°C until analyzed by LC-MS/MS. The PK parameters were calculated using non-compartmental model by Phoenix WinNonlin 6.1 (Linear/log trapezoidal).

Table 6 The mouse PK data of representative compounds of the present invention

| Parameter | IV (2 mg/kg) | | | PO (10 mg/kg) | | | |
|---|---|---|---|---|---|---|---|
| | CL (mL/ min/kg) | T1/2 (h) | AUC (0-inf) (h*ng/mL) | Cmax (ng/ mL) | T1/2 (h) | AUC (0-inf) (h*ng/mL) | F (%) |
| **Compound 41** | 8.13 | 3.38 | 4100 | 578 | 2.54 | 5040 | 24.6 |
| **Compound 55** | 4.78 | 5.70 | 6975 | 902 | 5.2 | 13551 | 38.9 |
| **Compound 66** | 1.25 | 7.00 | 26728 | 1880 | 7.2 | 31572 | 23.6 |
| **Compound 71** | 4.95 | 2.79 | 6731 | 2730 | 3.0 | 25377 | 75.4 |
| **Compound 72** | 29.5 | 2.81 | 1129 | 550 | 2.85 | 4528 | 80.2 |
| **Compound 110** | 3.24 | 3.03 | 10851 | 4210 | 2.95 | 53179 | 106 |

The compounds of the present invention showed good oral exposure, low clearance rate and high bioavailability.

## Claims

1. A compound represented by structural formula (I), or a derivative, pharmaceutically acceptable salt, isomer, solvate, hydrate, adduct, complex or prodrug thereof:

W-L-D (I),

wherein:

W is a Wee1 kinase binding ligand with the following structure:

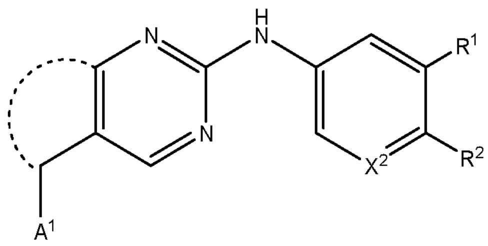

,

wherein:

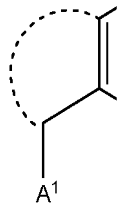

represents an optionally substituted cyclic group;

$A^1$ is selected from O=, halogen, hydroxyl, nitro, cyano, amino, mercapto, $C_1$-$C_6$ alkyl, $C_1$-$C_6$ alkoxy, (mono- and di-$C_1$-$C_6$ alkylamino) $C_0$-$C_4$ alkyl;

$X^2$, for each occurrence, is independently selected from $CR^{2'}$ or N;

$R^{2'}$, for each occurrence, is independently selected from absence, hydrogen, deuterium, halogen, hydroxyl, carbonyl, nitro, cyano, amino, mercapto, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^2$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^1$ and $R^2$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl;

any one of $R^1$, $R^2$, $R^1$ and $R^2$ together or the ring systems formed by $R^1$ and $R^2$ together is linked to L groups;

L is a linker and has the following structure;

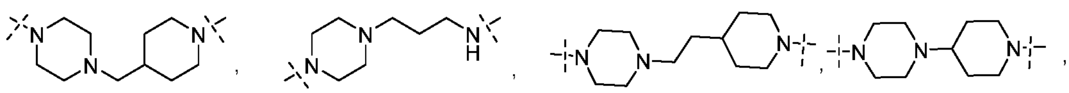
, , , ,

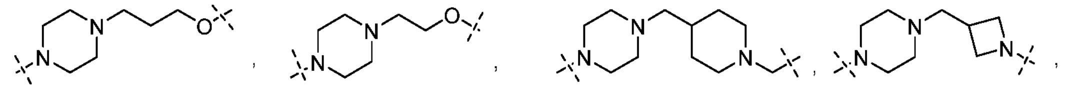
, , , ,

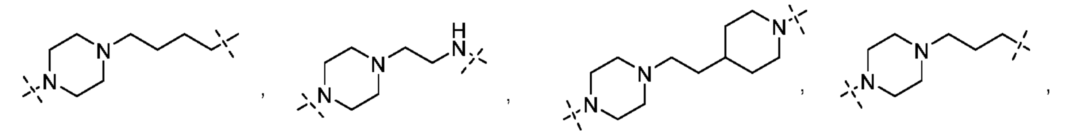
, , , ,

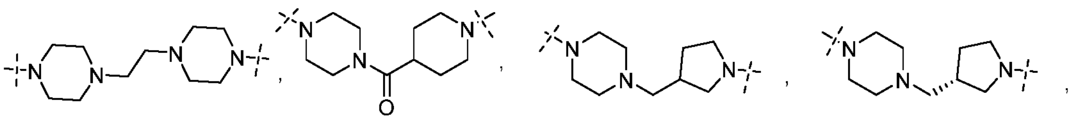
, , , ,

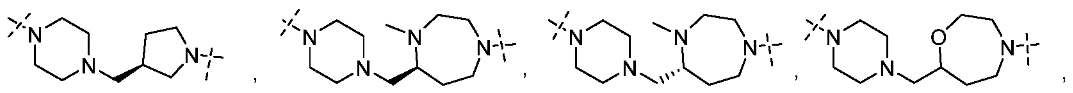
, , , ,

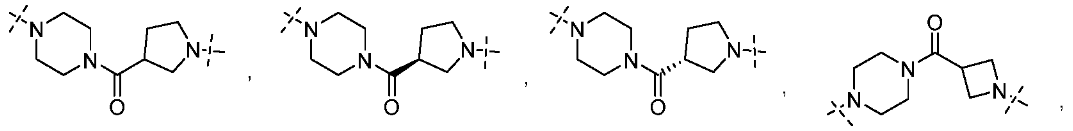
, , , ,

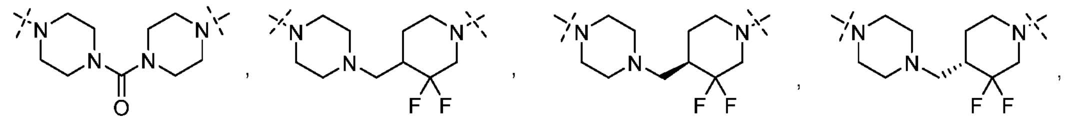
, , , ,

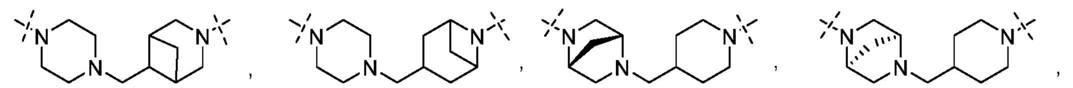
, , , ,

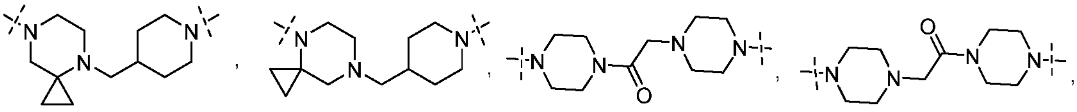
, , , ,

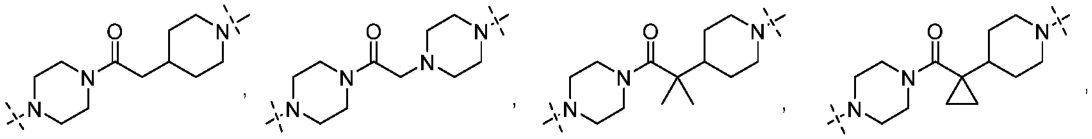
, , , ,

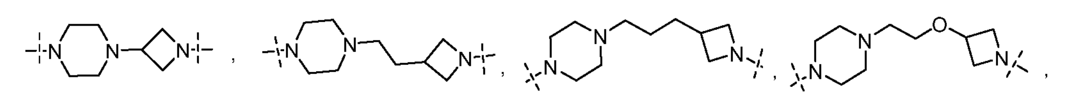
, , , ,

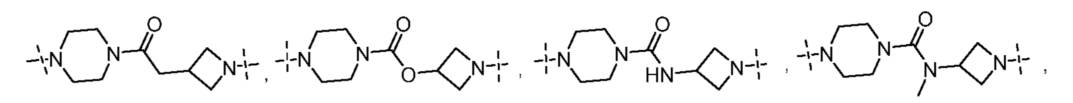
, , , ,

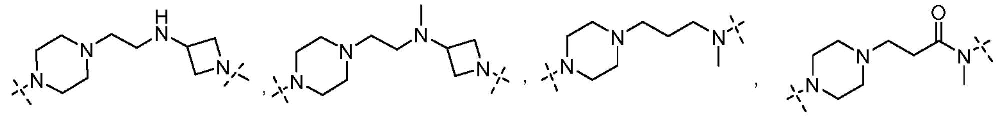
, , , ,

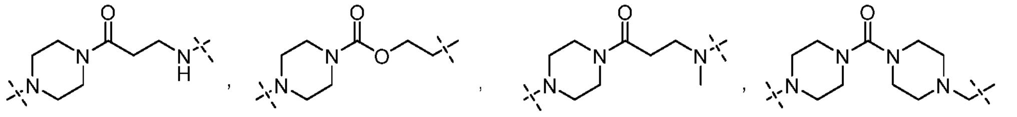
, , , ,

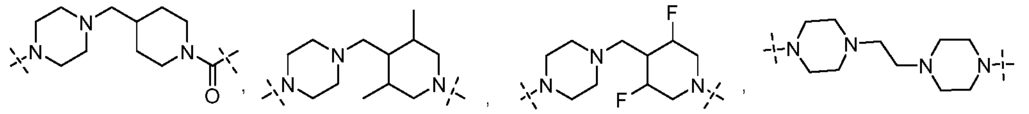
, , , ,

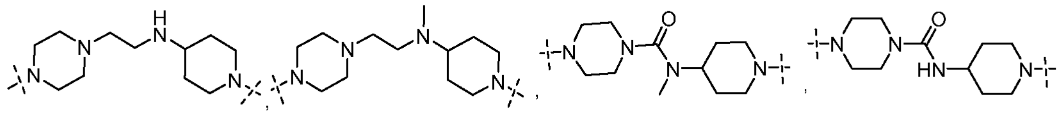
, , , ,

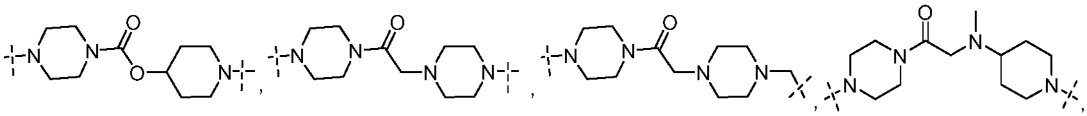
, , , ,

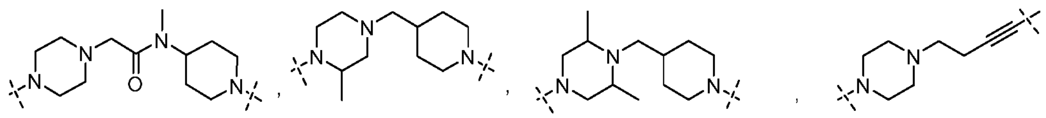
, , , ,

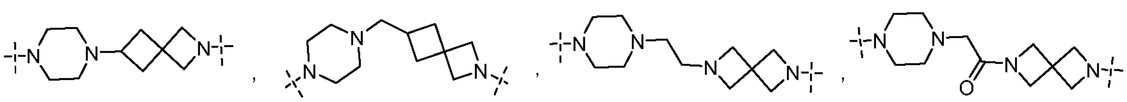
, , , ,

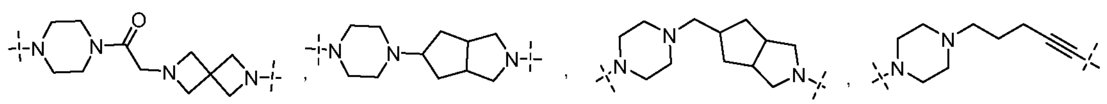
, , , ,

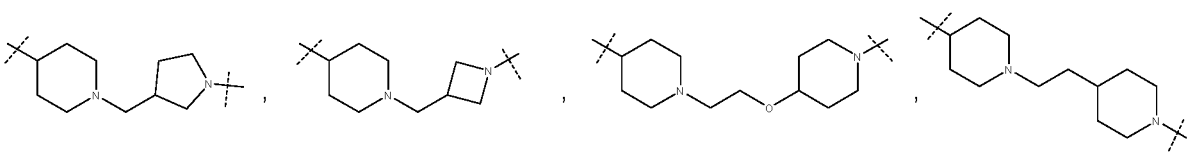
, , ,

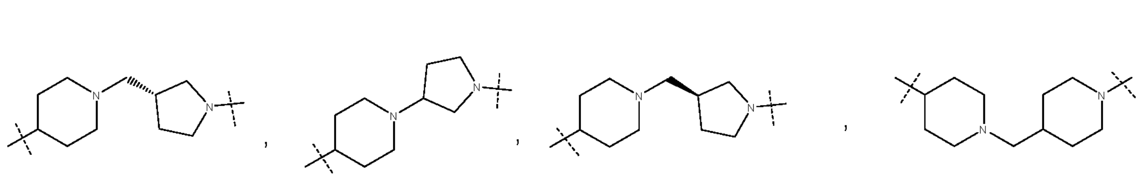
, , , ,

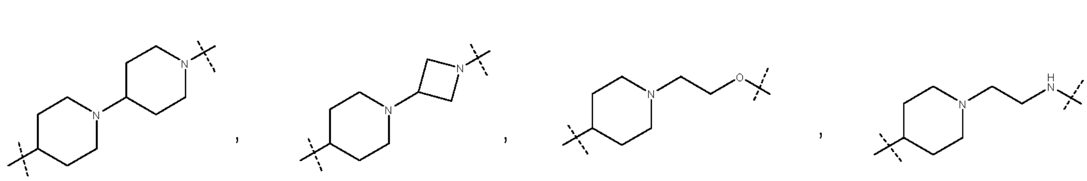
, , , ,

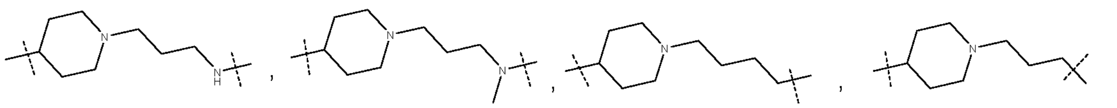
, , , ,

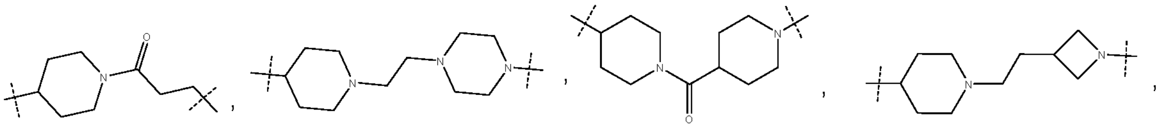
, , , ,

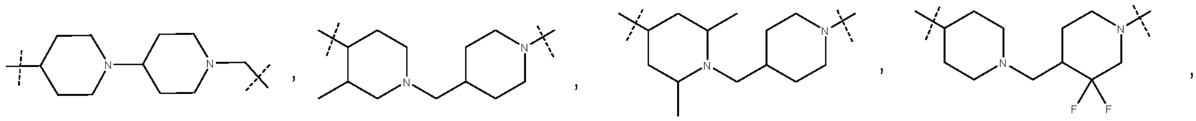
, , , ,

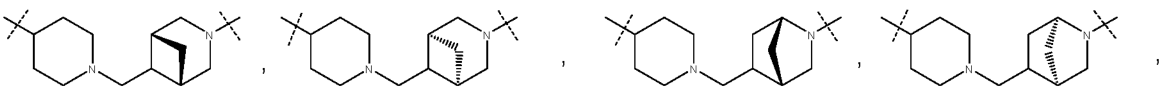
, , , ,

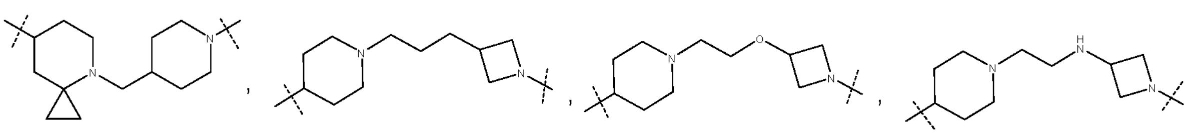
, , , ,

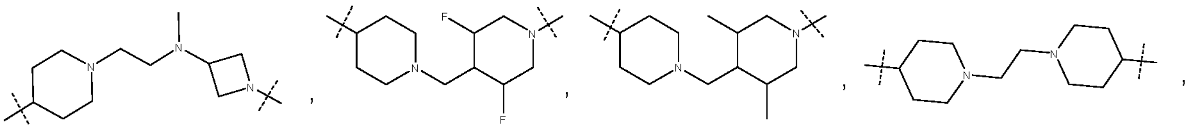
, , , ,

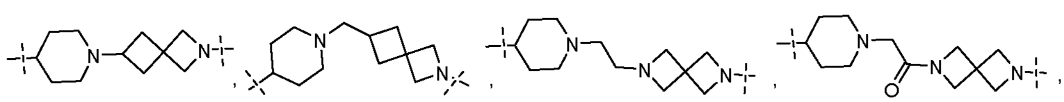
, , , ,

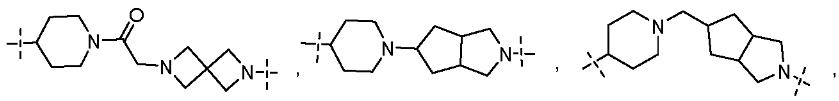
, , ,

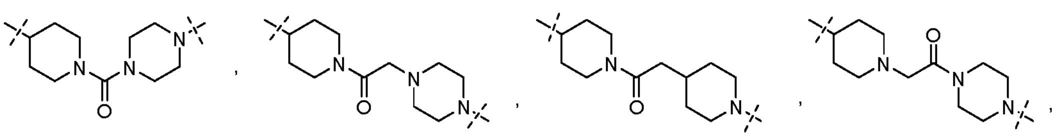
, , , ,

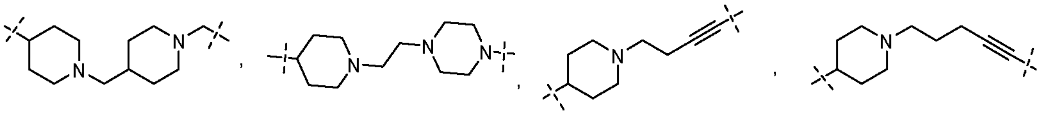
, , , ,

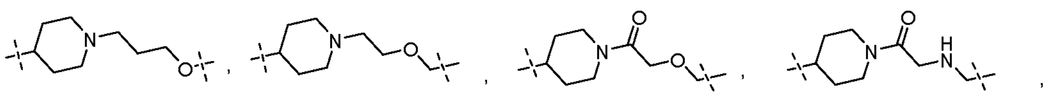
, , , ,

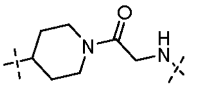, 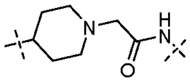, 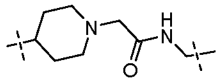, 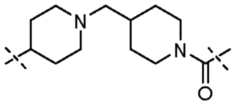,

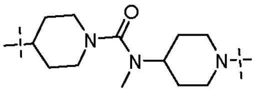, 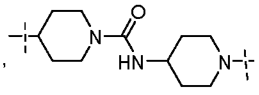, 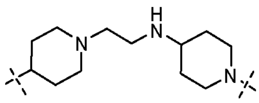, 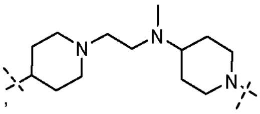,

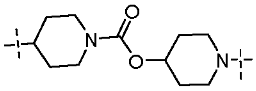, 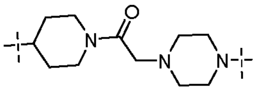, 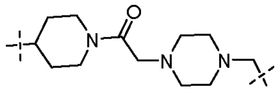, 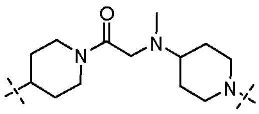,

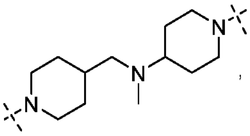, 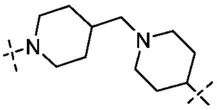, 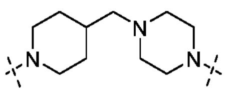, 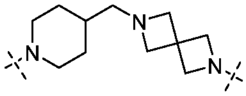,

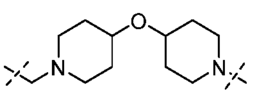, 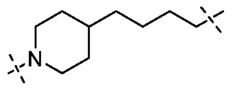, 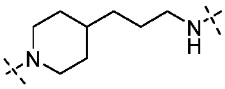, 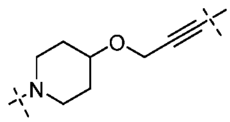,

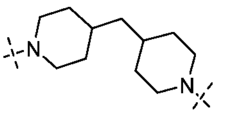, 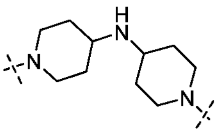, 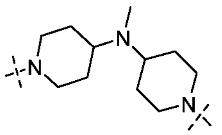, 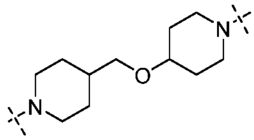,

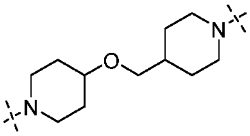, 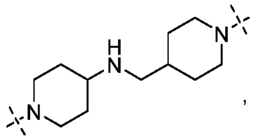, 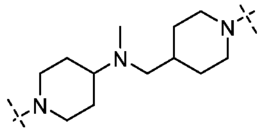, 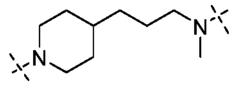,

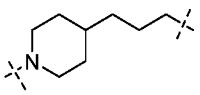, 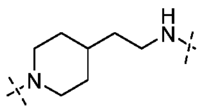, 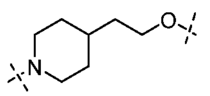, 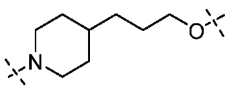,

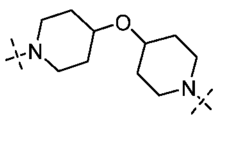, 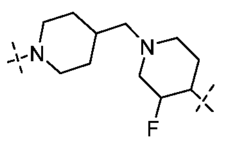, 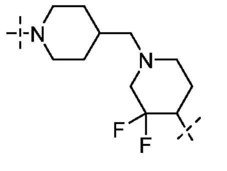, 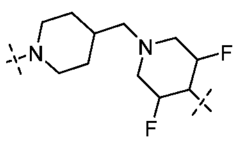,

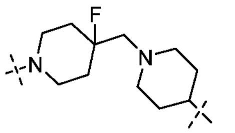, 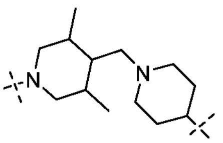, 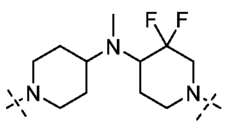,

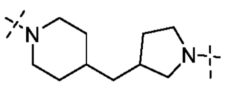, 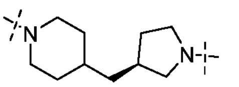, 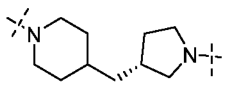, 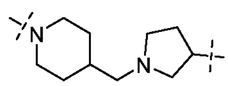

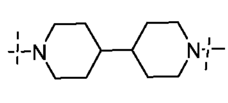, 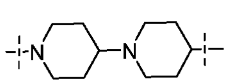, 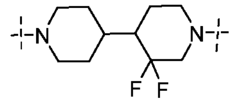, 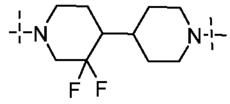

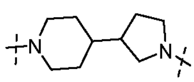, 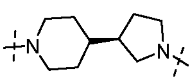, 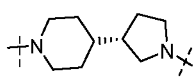, 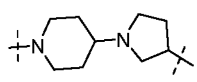,

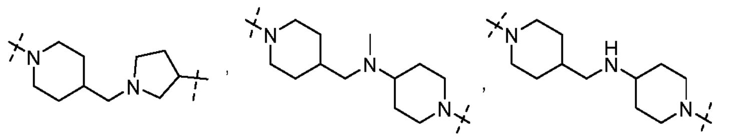

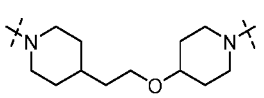, 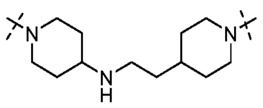, 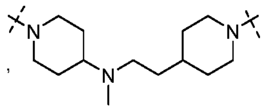, 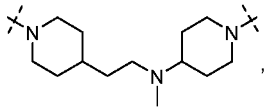,

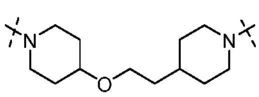, 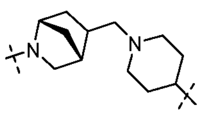, 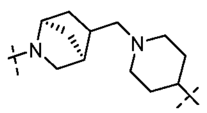, 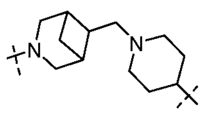,

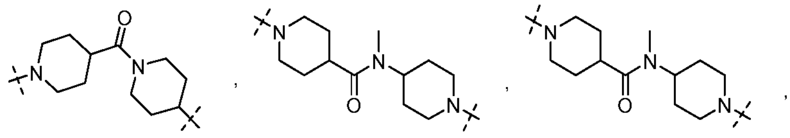

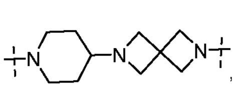, 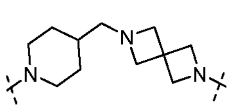, 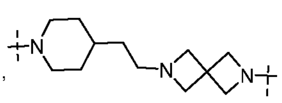, 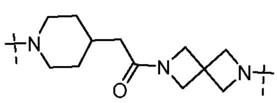,

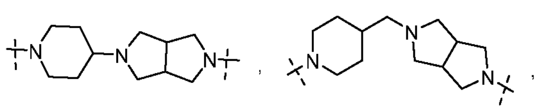

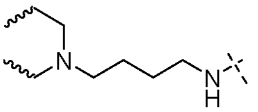, 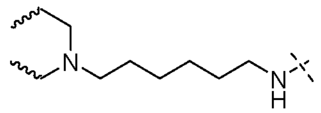, 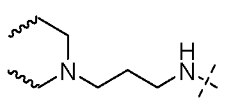, 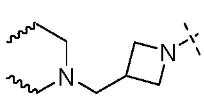,

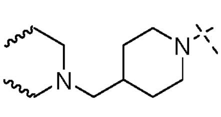, 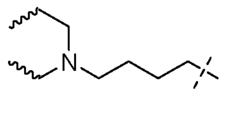, 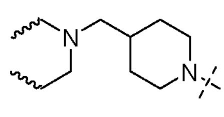, 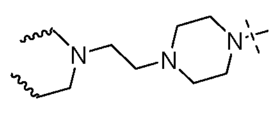,

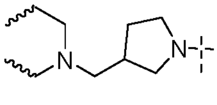, 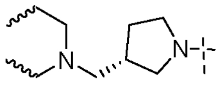, 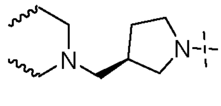, 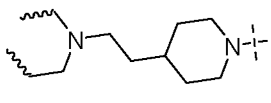,

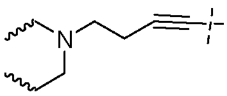 , 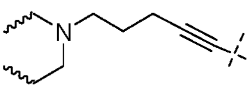 , 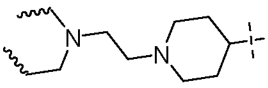 , 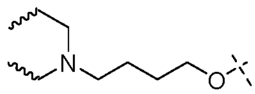 ,

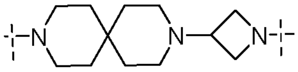 , 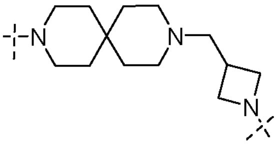 , 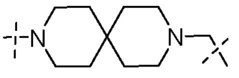 , 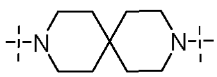 ,

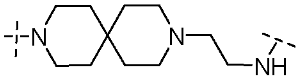 , 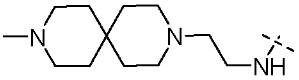 , 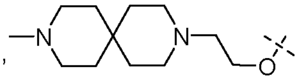 ,

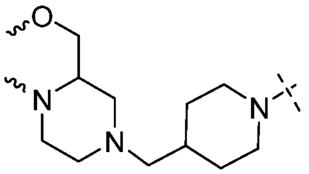 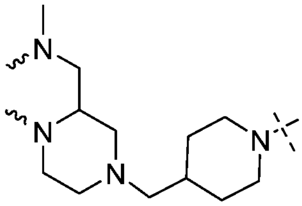 ,

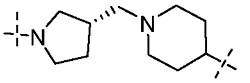 , 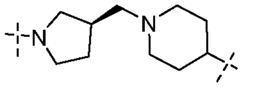 , 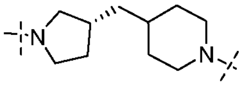 , 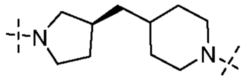 ,

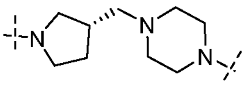 , 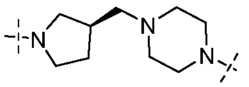 , 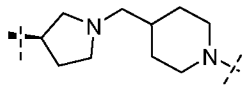 , 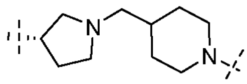 ,

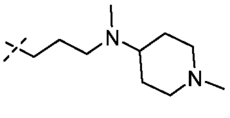 , 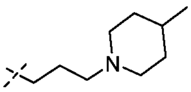 , 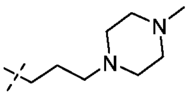 , 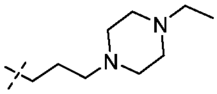 , 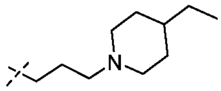 ,

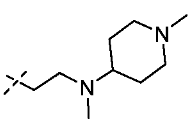 , 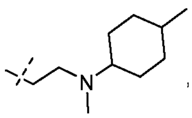 , 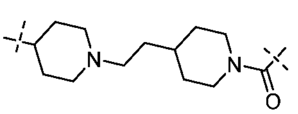 , 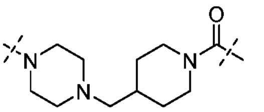 ,

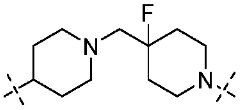 , 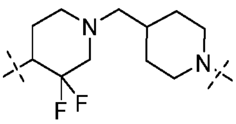 , 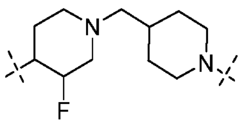 , 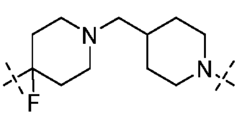 ,

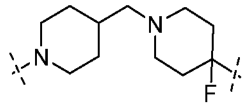 , 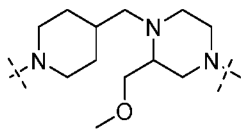 , 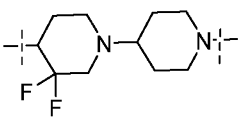 , 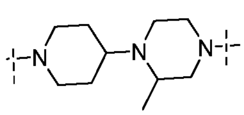 ,

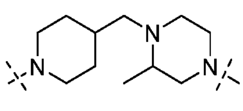, 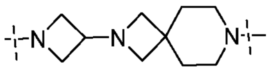, 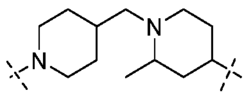, 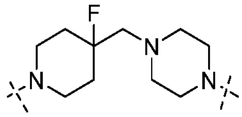,

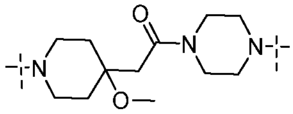, 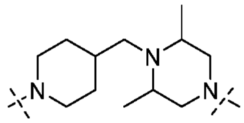, 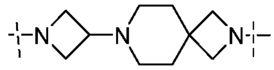, 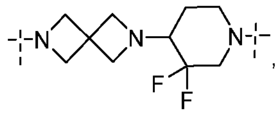,

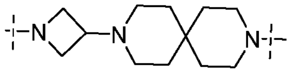, 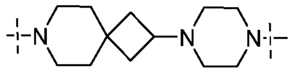, 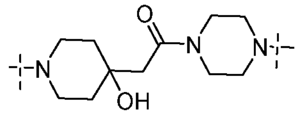, 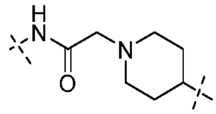

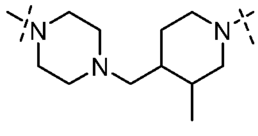, 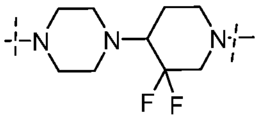, 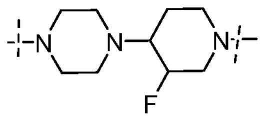, 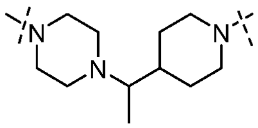,

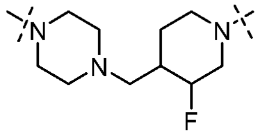, 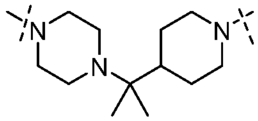, 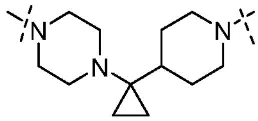, 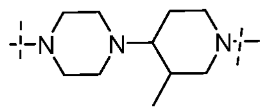,

wherein

 or 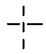

represents a linking site;

D is E3 ubiquitin ligase binding ligand with the following structure,

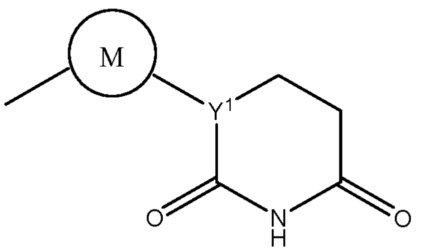, wherein 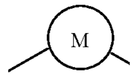

represents an optionally substituted cyclic group;

$Y^1$, for each occurrence, is independently selected from $CT^1$ or N; $T^1$ is selected from hydrogen or deuterium;

L is linked to W and/or D by one or more bonds.

**2.** The compound according to any one of the preceding claims, W is:

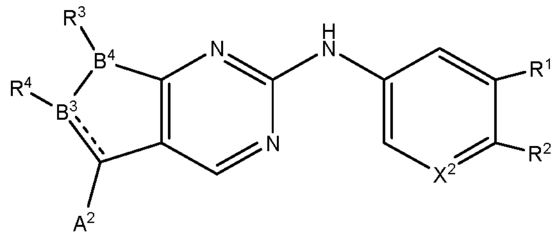

,

wherein:

$A^2$ is selected from O=, halogen, hydroxyl, nitro, cyano, amino, mercapto, $C_1$-$C_6$ alkyl, $C_1$-$C_6$ alkoxy, (mono- and di-$C_1$-$C_6$ alkylamino) $C_0$-$C_4$ alkyl;
$B^3$, for each occurrence, is independently selected from C, CH, CD or N;
$B^4$, for each occurrence, is independently selected from C, CH, CD or N; or
$B^3$-$B^4$ is -C=C-;

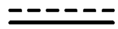

represents a single bond or a double bond;
$X^2$, for each occurrence, is independently selected from $CR^{2'}$ or N;
any one of $R^{2'}$, for each occurrence, is independently selected from absence, hydrogen, deuterium, halogen, hydroxyl, carbonyl, nitro, cyano, amino, mercapto, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;
$R^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;
$R^2$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or
$R^1$ and $R^2$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl;
any one of $R^1$, $R^2$, $R^1$ and $R^2$ together or the ring systems formed by $R^1$ and $R^2$ together is linked to L groups;
$R^3$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;
$R^4$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl,

wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or
$R^3$ and $R^4$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl.

**3.** The compound according to any one of the preceding claims, W is:

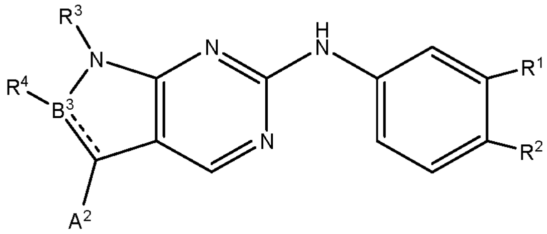

$A^2$ is selected from O=, halogen, hydroxyl, nitro, cyano, amino, mercapto, $C_1$-$C_6$ alkyl, $C_1$-$C_6$ alkoxy, (mono- and di-$C_1$-$C_6$ alkylamino) $C_0$-$C_4$ alkyl;
$B^3$, for each occurrence, is independently selected from C, CH, CD or N; alternatively, $R^4B^3$-$NR^3$ is -N=N-;

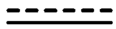

represents a single bond or a double bond;
$R^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;
$R^2$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or
$R^1$ and $R^2$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl;
any one of $R^1$, $R^2$, $R^1$ and $R^2$ together or the ring systems formed by $R^1$ and $R^2$ together is linked to L groups;
$R^3$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;
$R^4$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester,
$C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl,
(mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^3$ and $R^4$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl.

**4.** The compound according to any one of the preceding claims, wherein W is:

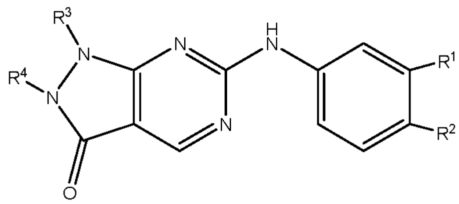

,

wherein

$R^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^2$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^1$ and $R^2$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl;

any one of $R^1$, $R^2$, $R^1$ and $R^2$ together or the ring systems formed by $R^1$ and $R^2$ together is linked to L groups;

$R^3$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^4$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^3$ and $R^4$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl; or

-($R^4$N-N$R^3$)- is -N=N-.

**5.** The compound according to any one of the preceding claims, wherein W is:

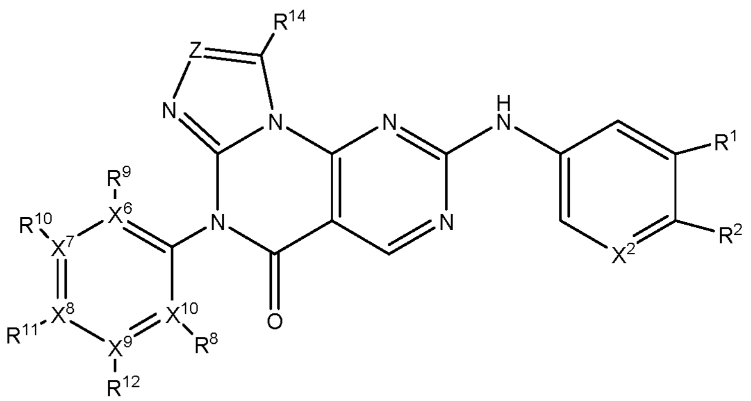

,

wherein

$X^2$, for each occurrence, is independently selected from $CR^{2'}$ or N;

$R^{2'}$, for each occurrence, is independently selected from absence, hydrogen, deuterium, halogen, hydroxyl, carbonyl, nitro, cyano, amino, mercapto, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^2$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^1$ and $R^2$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl;

any one of $R^1$, $R^2$, $R^1$ and $R^2$ together or the ring systems formed by $R^1$ and $R^2$ together is linked to L groups;

$X^6$, for each occurrence, is independently selected from C or N;

$X^7$, for each occurrence, is independently selected from C or N;

$X^8$, for each occurrence, is independently selected from C or N;

$X^9$, for each occurrence, is independently selected from C or N;

$X^{10}$, for each occurrence, is independently selected from C or N;

any one of $R^8$, $R^9$, $R^{10}$, $R^{11}$, $R^{12}$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

Z, for each occurrence, is independently selected from $CR^{13'}$ or N;

$R^{13'}$, for each occurrence, is independently selected from absence, hydrogen, deuterium, halogen, hydroxyl,

carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^{14}$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl.

**6.** The compound according to any one of the preceding claims, wherein W is:

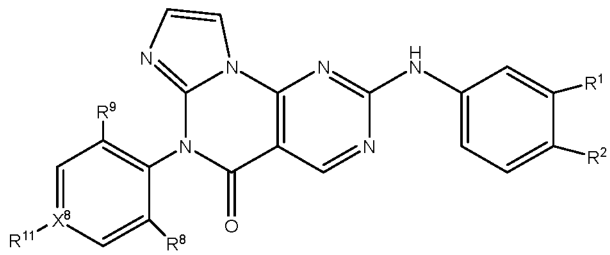

,

wherein

$R^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^2$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^1$ and $R^2$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl;

any one of $R^1$, $R^2$, $R^1$ and $R^2$ together or the ring systems formed by $R^1$ and $R^2$ together is linked to L groups;

$X^8$, for each occurrence, is independently selected from C or N;

$R^8$, $R^9$, $R^{11}$, for each occurrence, are independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl.

**7.** The compound according to any one of the preceding claims, wherein W is:

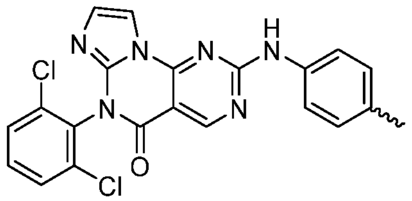

**8.** The compound according to any one of the preceding claims, wherein W is:

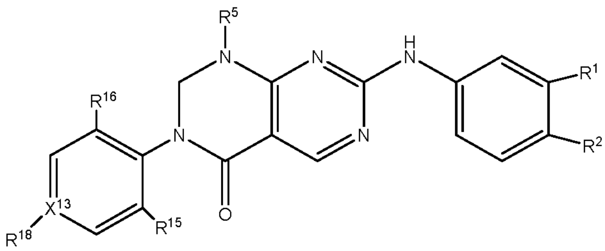

$R^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^2$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^1$ and $R^2$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl;

any one of $R^1$, $R^2$, $R^1$ and $R^2$ together or the ring systems formed by $R^1$ and $R^2$ together is linked to L groups;

$R^5$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$X^{13}$, for each occurrence, is independently selected from C or N;

any one of $R^{15}$, $R^{16}$, and $R^{18}$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl.

**9.** The compound according to any one of the preceding claims, wherein W is:

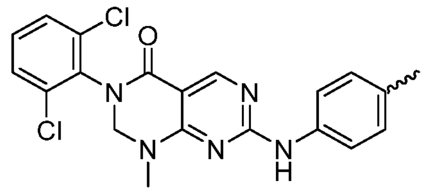

.

**10.** The compound according to any one of the preceding claims, wherein W is:

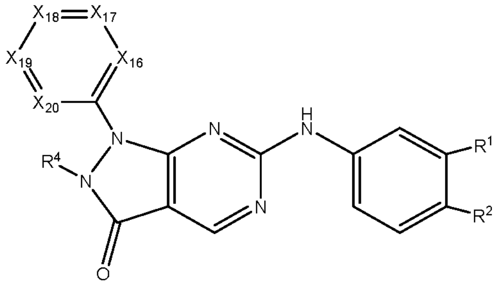

,

wherein

$R^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^2$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^1$ and $R^2$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl;

any one of $R^1$, $R^2$, $R^1$ and $R^2$ together or the ring systems formed by $R^1$ and $R^2$ together is linked to L groups;

$R^4$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$X^{16}$, for each occurrence, is independently selected from CH, CD, $CR^{21}$ or N;

$X^{17}$, for each occurrence, is independently selected from CH, CD, $CR^{22}$ or N;

$X^{18}$, for each occurrence, is independently selected from CH, CD, $CR^{23}$ or N;

$X^{19}$, for each occurrence, is independently selected from CH, CD, $CR^{24}$ or N;

$X^{20}$, for each occurrence, is independently selected from CH, CD, $CR^{25}$ or N;

any one of $R^{21}$, $R^{22}$, $R^{23}$, $R^{24}$, $R^{25}$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylami-

no) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl.

**11.** The compound according to any one of the preceding claims, wherein W is:

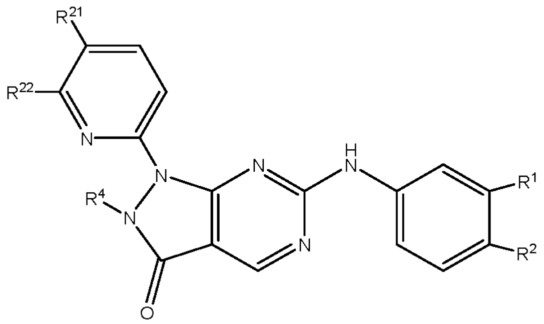

$R^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

$R^2$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl; or

$R^1$ and $R^2$ are joined together with the connected ring atoms to form a monocyclic, bicyclic or tricyclic saturated or unsaturated ring system with 5-14 ring atoms, wherein 0, 1, 2, 3 or 4 are heteroatoms selected from N, O and S, with remaining ring atoms being carbon, and the ring system is optionally substituted with 1, 2, 3 or 4 halogens, hydroxyl, carbonyl, nitro, cyano, amino, mercapto, -COOH, or $C_1$-$C_6$ alkyl;

any one of $R^1$, $R^2$, $R^1$ and $R^2$ together or the ring systems formed by $R^1$ and $R^2$ together is linked to L groups;

$R^4$, for each occurrence, is independently selected from absence, hydrogen, deuterium, halogen, hydroxyl, carbonyl, nitro, cyano, amino, mercapto, allyl, propenyl, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;

any one of $R^{21}$ and $R^{22}$, for each occurrence, is independently selected from absence, hydrogen, deuterium, halogen, hydroxyl, carbonyl, nitro, cyano, amino, mercapto, allyl, propenyl, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, $C_5$-$C_{10}$ heteroaryl, wherein the cycloalkyl, heterocyclyl, aryl and heteroaryl are optionally substituted with 1, 2, 3 or 4 halogens, hydroxyl, carbonyl, nitro, cyano, amino, mercapto, -COOH, or $C_1$-$C_6$ alkyl optionally substituted with substituents selected from $C_1$-$C_4$ alkyl, cyclopropyl, halogen, hydroxyl, carbonyl, nitro; or,

$R^{21}$, $R^{22}$ are joined together with the connected ring atoms to form a five-membered or six-membered ring having 0, 1 or 2 heteroatoms selected from N, O or S, wherein the five-membered or six-membered ring is optionally substituted with substituents selected from $C_1$-$C_4$ alkyl, cyclopropyl, halogen, hydroxyl, carbonyl, nitro.

**12.** The compound according to any one of the preceding claims, wherein W is:

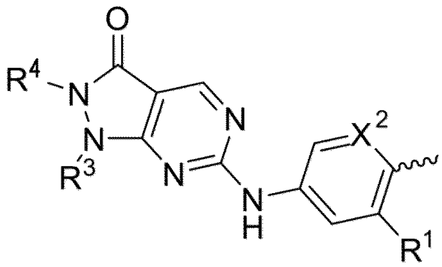

wherein:

$X^2$ is CH or N;
$R^1$ is hydrogen, fluorine, chlorine, methyl, ethyl, methoxy, hydroxymethyl, or linked to L groups;
$R^4$ is selected from allyl, propargyl, methylene cyclopropyl, ethyl, trifluoroethyl, difluoroethyl, isopropyl, cyclopropyl, methylene $C_6$-$C_{12}$ aryl, methylene phenyl, $C_{1-4}$ alkyl, optionally substituted $C_{2-4}$ alkenyl, optionally substituted $C_{2-4}$ alkynyl, optionally substituted $C_{3-6}$ cycloalkyl and optionally substituted $C_{3-6}$ cycloalkyl ($C_{1-4}$ alkyl), wherein the $C_{1-4}$ alkyl, $C_{2-4}$ alkenyl and $C_{2-4}$ alkynyl are optionally independently substituted with one or more substituents selected from halogen, $C_{1-4}$ alkoxy, $C_{1-4}$ haloalkyl, $C_{1-4}$ haloalkoxy, cyano, amino, mono-$C_{1-4}$ alkylamine and di-$C_{1-4}$ alkylamine, and wherein one or more rings of the $C_{3-6}$ cycloalkyl and the $C_{3-6}$ cycloalkyl ($C_{1-4}$ alkyl) are optionally independently substituted with one or more substituents selected from halogen, $C_{1-4}$ alkyl, $C_{1-4}$ alkoxy, $C_{1-4}$ haloalkyl, $C_{1-4}$ haloalkoxy, cyano, amino, mono-$C_{1-4}$ alkylamine and di-$C_{1-4}$ alkylamine;
$R^3$ is selected from:

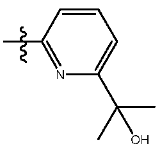 , 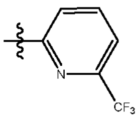 , 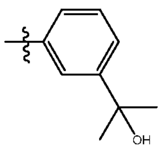 , 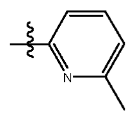 , 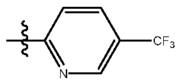 , 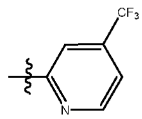 ,

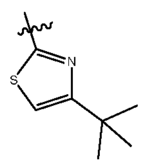 , 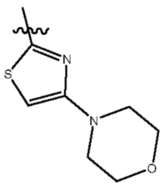 , 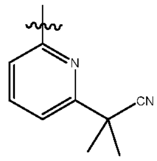 , 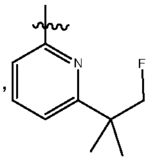 , 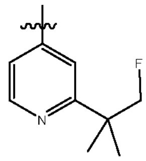 , 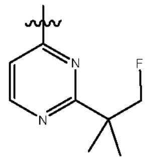 ,

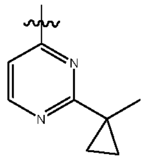 , 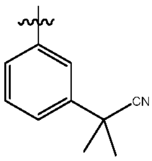 , 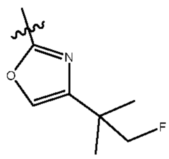 , 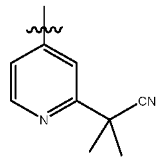 , 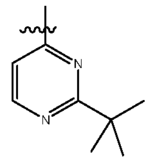 , 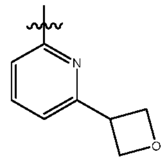 ,

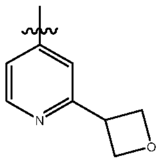 , 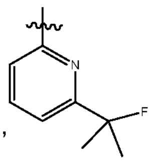 , 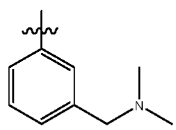 , 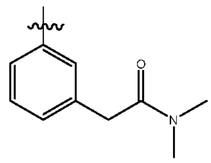 , 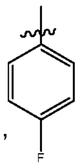 , 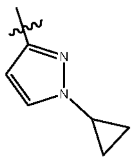 ,

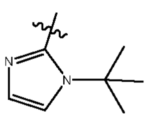 , 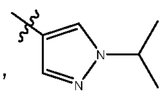 , 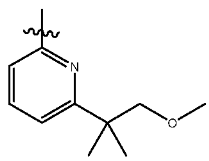 , 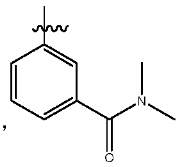 , 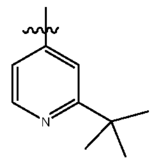 , 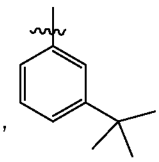 ,

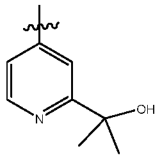 , 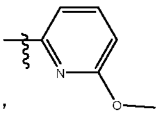 , 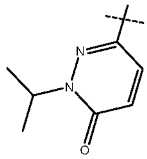 ;

optionally substituted $C_6$-$C_{12}$ aryl or optionally substituted $C_5$-$C_{10}$ heteroaryl, wherein when the aryl or heteroaryl is substituted, the $C_6$-$C_{12}$ aryl and $C_5$-$C_{10}$ heteroaryl are independently substituted with one or more substituents, preferably fluorine, chlorine, methyl, ethyl, methoxy, hydroxymethyl, haloalkyl.

**13.** The compound according to any one of the preceding claims, wherein W is:

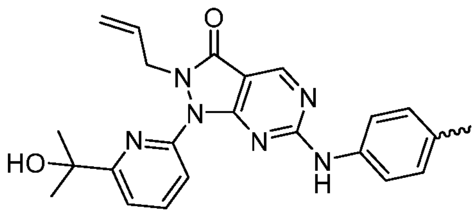
.

**14.** The compound according to any one of the preceding claims, wherein W is:

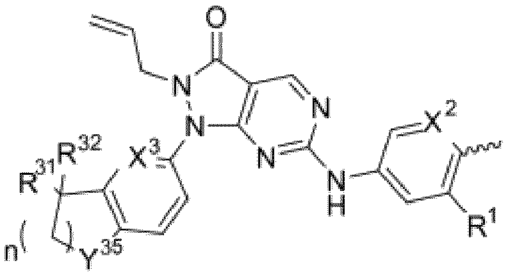
,

wherein

$X^2$ is CH orN;
$R^1$ is hydrogen, fluorine, chlorine, methyl, ethyl, methoxy, hydroxymethyl, or linked to L groups;
$R^{31}$ is hydrogen, hydroxyl, fluorine, amino, cyano;
$R^{32}$ is hydrogen, fluorine, methyl, ethyl, fluoroethyl, trifluoromethyl, difluoromethyl, cyclopropyl;
$R^{31}$ and $R^{32}$ are joined together with the connected ring atoms to form

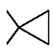 ;

$X^3$ is CH, O, N, $NR^{39}$, wherein $R^{39}$ is $C_{1-3}$ alkyl or cycloalkyl;
n is 1 or 2;
$Y^{35}$ is $CH_2$ or O.

**15.** The compound according to any one of the preceding claims, wherein W is:

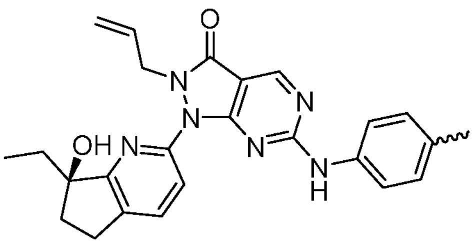
,
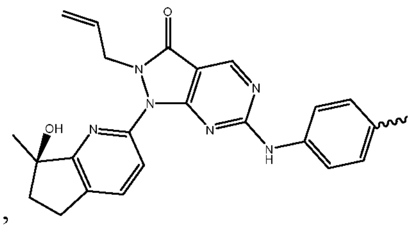
.

**16.** The compound according to any one of the preceding claims, wherein W is:

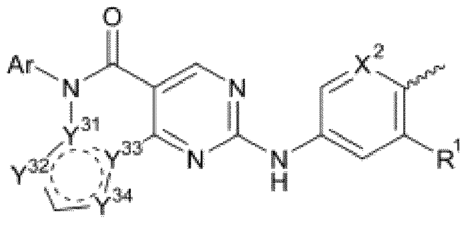

wherein:

$X^2$ is C or N;
$R^1$ is hydrogen, fluorine, chlorine, methyl, ethyl, methoxy, hydroxymethyl, or linked to L groups;
Ar is phenyl optionally substituted with substituents selected from halogen, methyl or methoxy, $C_5$-$C_{10}$ heteroaryl optionally substituted with substituents selected from halogen, methyl or methoxy;

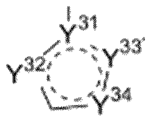

is a nitrogen-containing heteroaromatic ring selected from pyrrolyl, imidazolyl, pyrazolyl, triazolyl, or tetrazolyl.

**17.** The compound according to any one of the preceding claims, wherein W is:

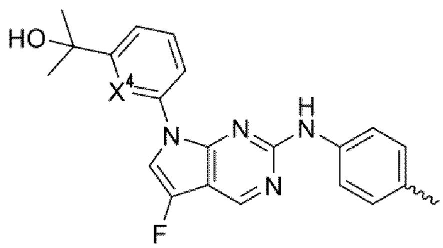
,

wherein
$X^4$ is CH or N.

**18.** The compound according to any one of the preceding claims, wherein W is:

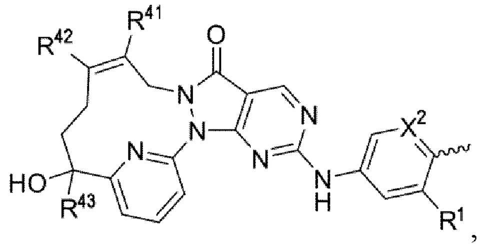

wherein:

$X^2$ is CH orN;
$R^1$ is selected from hydrogen, fluorine, chlorine, methyl, ethyl, methoxy, hydroxymethyl, or a linking L substituent;
$R^{41}$ is selected from hydrogen, fluorine, or methyl;
$R^{42}$ is selected from hydrogen, fluorine, or methyl;
$R^{43}$ is selected from methyl or ethyl;

**19.** The compound according to any one of the preceding claims, wherein W is:

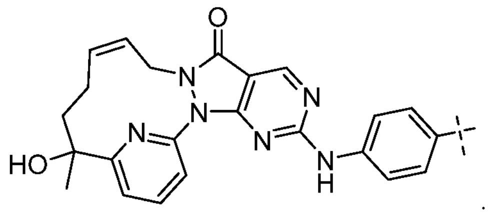

**20.** The compound according to any one of the preceding claims, wherein D is:

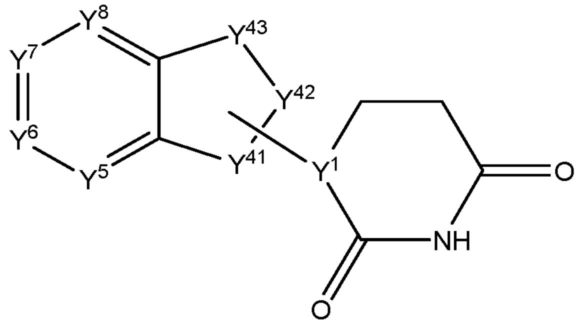

$Y^1$, for each occurrence, is independently selected from $CT^1$ or N;
$T^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;
$Y^{41}$, for each occurrence, is independently selected from C, $CT^{41}T^{41'}$, $NT^{41''}$, C=O, or O;
$Y^{42}$, for each occurrence, is independently selected from C, $CT^{42}T^{42'}$, $NT^{42''}$, C=O, or O;
$Y^{43}$, for each occurrence, is independently selected from C, $CT^{43}T^{43'}$, $NT^{43''}$ , C=O, or O; or $Y^{41}$-$Y^{42}$ represents -$T^{41}$(C=C)$T^{42}$-, -(N=C)$T^{42}$-, -$T^{41}$(C=N)- or -(N=N)-, or $Y^{42}$-$Y^{43}$ represents - $T^{42}$(C=C)$T^{43}$-, -(N=C)$T^{43}$-, -$T^{42}$(C=N)- or-(N=N)-;
each of $T^{41}$, $T^{41'}$, $T^{41''}$, $T^{42}$, $T^{42}$, $T^{42''}$ , $T^{43}$, $T^{43}$, $T^{43''}$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester,

$C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;
$Y^5$, for each occurrence, is independently selected from $CT^5$ or N;
$Y^6$, for each occurrence, is independently selected from $CT^6$ or N;
$Y^7$, for each occurrence, is independently selected from $CT^7$ or N;
$Y^8$, for each occurrence, is independently selected from $CT^8$ or N;
each of $T^5$, $T^6$, $T^7$, $T^8$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$alkylester, $C_1$-$C_6$thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;
one or more of $Y^5$, $Y^6$, $Y^7$ and $Y^8$ are linked to L groups.

**21.** The compound according to any one of the preceding claims, wherein D is:

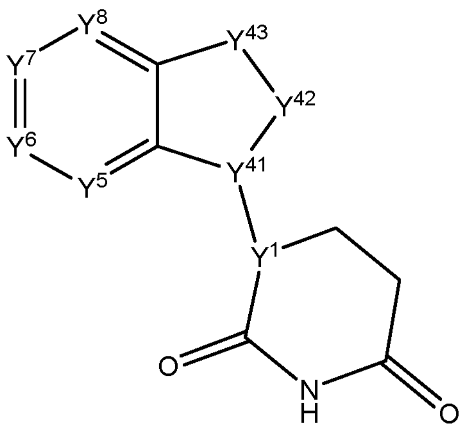

,

wherein

$Y^1$, for each occurrence, is independently selected from $CT^1$ or N;
$T^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;
$Y^{41}$, for each occurrence, is independently selected from $CT^{41}$ or N;
$Y^{42}$, for each occurrence, is independently selected from $CT^{42}T^{42'}$, $NT^{42''}$, C=O, or O;
$Y^{43}$, for each occurrence, is independently selected from $CT^{43}T^{43'}$, $NT^{43''}$, C=O, or O; or
$Y^{41}$-$Y^{42}$ represents -(C=C)$T^{42}$-, -(C=N)-, or $Y^{42}$-$Y^{43}$ represents -$T^{42}$(C=C)$T^{43}$-, -(N=C)$T^{43}$-, - $T^{42}$(C=N)- or-(N=N)-;
Each of $T^{41}$, $T^{42}$, $T^{42'}$, $T^{42''}$, $T^{43}$, $T^{43}$, $T^{43''}$, for each occurrence, is independently selected from absence, hydrogen, deuterium, halogen, hydroxyl, carbonyl, nitro, cyano, amino, mercapto, - COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;
$Y^5$, for each occurrence, is independently selected from $CT^5$ or N;

$Y^6$, for each occurrence, is independently selected from $CT^6$ or N;
$Y^7$, for each occurrence, is independently selected from $CT^7$ or N;
$Y^8$, for each occurrence, is independently selected from $CT^8$ or N;
each of $T^5$, $T^6$, $T^7$, $T^8$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;
one or more of $Y^5$, $Y^6$, $Y^7$ and $Y^8$ are linked to L groups.

**22.** The compound according to any one of the preceding claims, wherein D is:

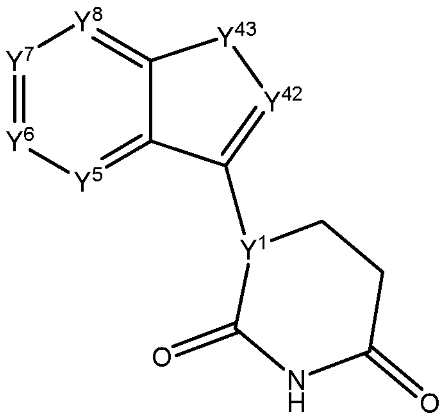

,

wherein:

$Y^1$, for each occurrence, is independently selected from CH, CD or N;
$Y^{42}$, for each occurrence, is independently selected from CH or N;
$Y^{43}$, for each occurrence, is independently selected from $CH_2$, -NH, O or N-$CH_3$;
$Y^5$, for each occurrence, is independently selected from N; $CT^5$, wherein $T^5$, for each occurrence, is independently selected from absence, hydrogen, fluorine, chlorine, methyl, methoxy;
$Y^6$, for each occurrence, is independently selected from N; $CT^6$, wherein $T^6$, for each occurrence, is independently selected from absence, hydrogen, fluorine, chlorine, methyl, methoxy;
$Y^7$, for each occurrence, is independently selected from N; $CT^7$, wherein $T^7$, for each occurrence, is independently selected from absence, hydrogen, fluorine, chlorine, methyl, methoxy;
$Y^8$, for each occurrence, is independently selected from N; $CT^8$, wherein $T^8$, for each occurrence, is independently selected from absence, hydrogen, fluorine, chlorine, methyl, methoxy;
one or more of $Y^5$, $Y^6$, $Y^7$ and $Y^8$ are linked to L groups.

**23.** The compound according to any one of the preceding claims, wherein D is:

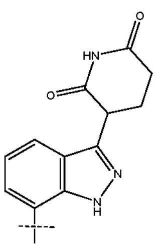 , 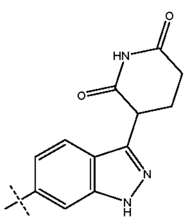 , 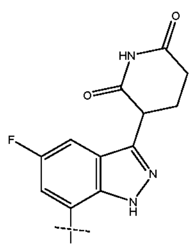 , 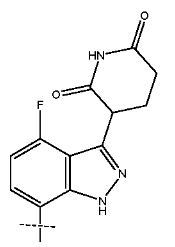 , 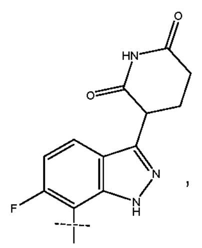 ,

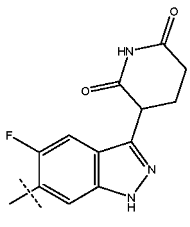, 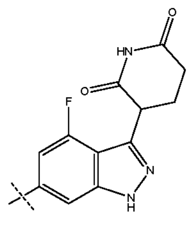, 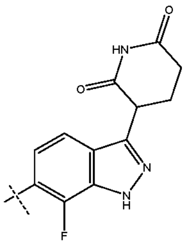, 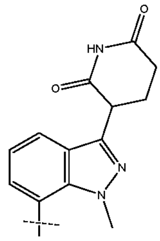, 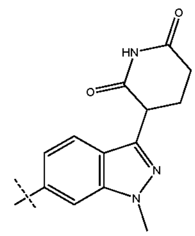,

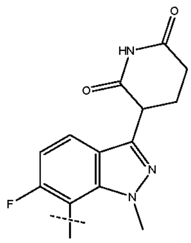, 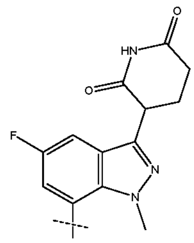, 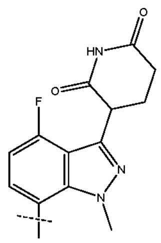, 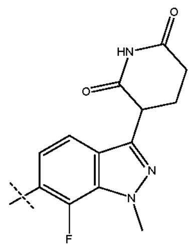, 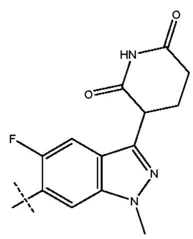,

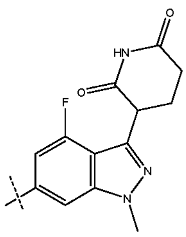, 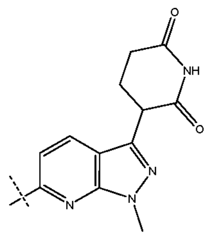, 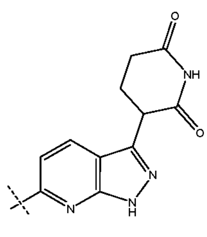, 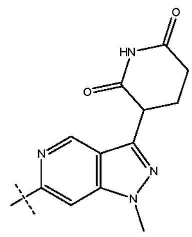, 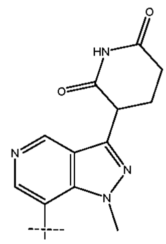,

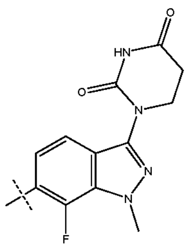, 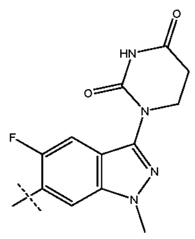, 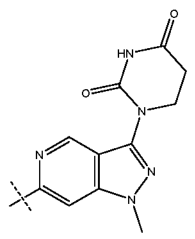, 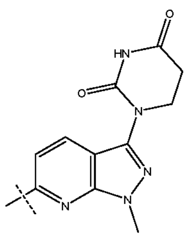, 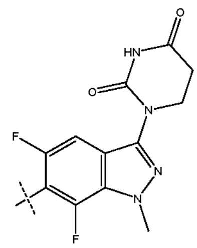,

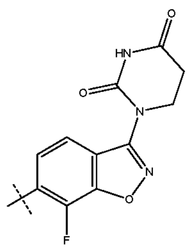, 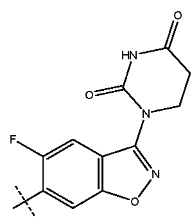

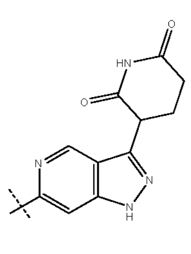, 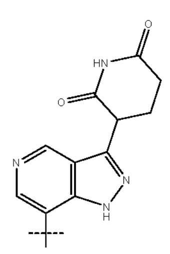, 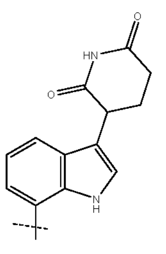, 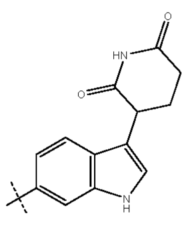, 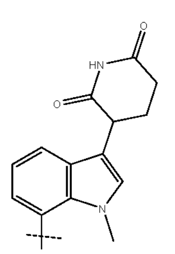,

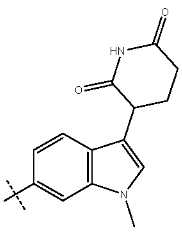, 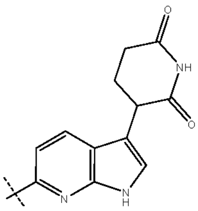, 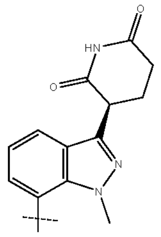, 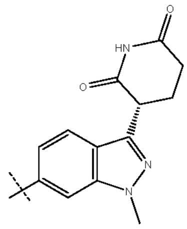, 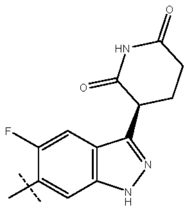,

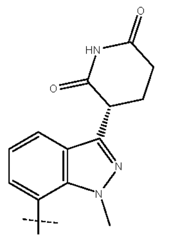, 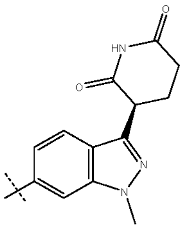, 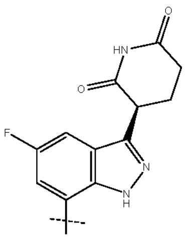, 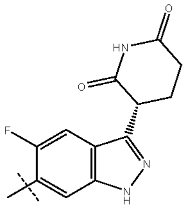, 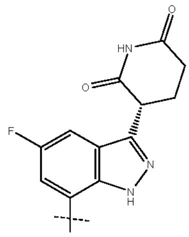,

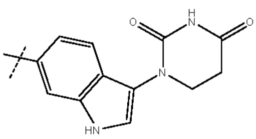, 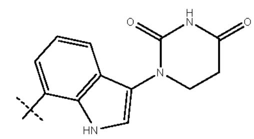, 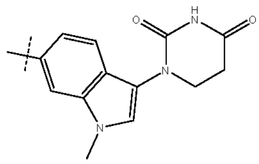, 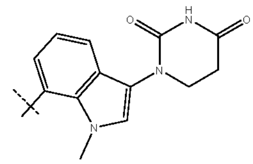,

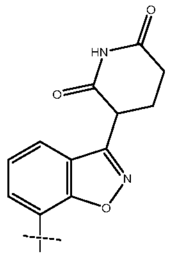, 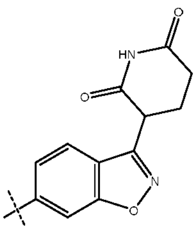, 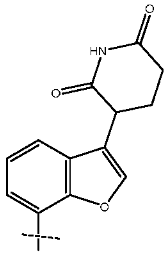, 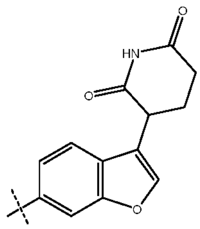, 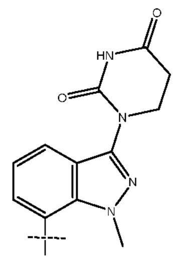,

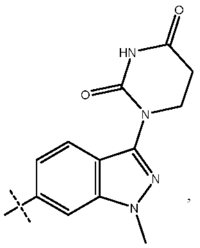, 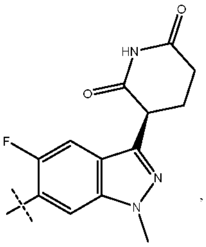, 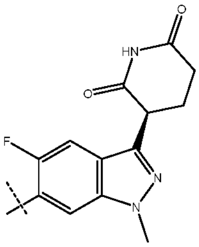, 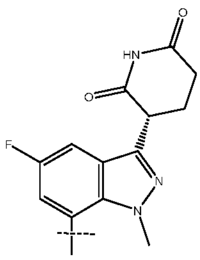, 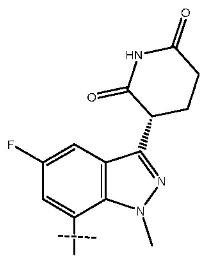

.

**24.** The compound according to any one of the preceding claims, wherein D is:

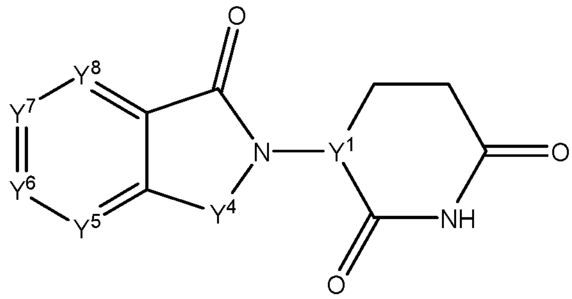

,

wherein

$Y^1$, for each occurrence, is independently selected from $CT^1$ or N;
$T^1$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$ alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;
$Y^4$, for each occurrence, is independently selected from $CT^4T^{4'}$ or $NT^{4''}$;
each of $T^4$, $T^{4'}$, $T^{4''}$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;
$Y^5$, for each occurrence, is independently selected from $CT^5$ or N;
$Y^6$, for each occurrence, is independently selected from $CT^6$ or N;
$Y^7$, for each occurrence, is independently selected from $CT^7$ or N;
$Y^8$, for each occurrence, is independently selected from $CT^8$ or N;
each of $T^5$, $T^6$, $T^7$, $T^8$, for each occurrence, is independently selected from absence, hydrogen, deuterium, halogen, hydroxyl, carbonyl, nitro, cyano, amino, mercapto, -COOH, $C_1$-$C_6$ alkyl,
$C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;
one or more of $Y^5$, $Y^6$, $Y^7$ and $Y^8$ are linked to L groups.

**25.** The compound according to any one of the preceding claims, wherein D is:

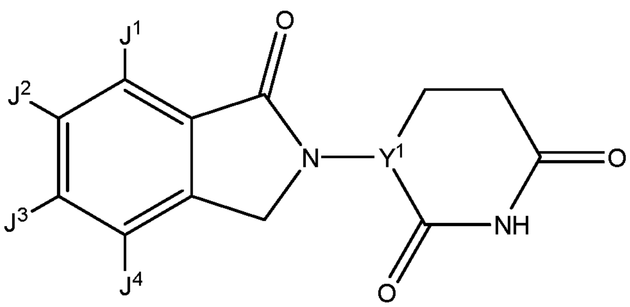

,

wherein $Y^1$, for each occurrence, is independently selected from CH, CD or N;
$J^1$, for each occurrence, is independently selected from absence, hydrogen, fluorine, chlorine, methyl, methoxy;
$J^2$, for each occurrence, is independently selected from absence, hydrogen, fluorine, chlorine, methyl, methoxy;
$J^3$, for each occurrence, is independently selected from absence, hydrogen, fluorine, chlorine, methyl, methoxy;
$J^4$, for each occurrence, is independently selected from absence, hydrogen, fluorine, chlorine, methyl, methoxy;
one or more of $J^1$, $J^2$, $J^3$ and $J^4$ are linked to L groups.

**26.** The compound according to any one of the preceding claims, wherein D is:

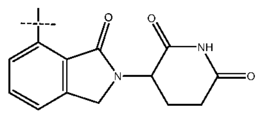 , 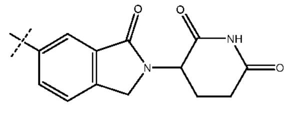 , 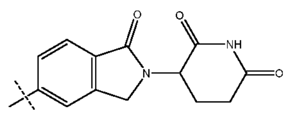 , 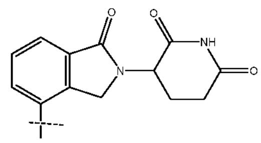 ,

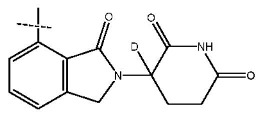 , 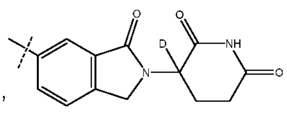 , 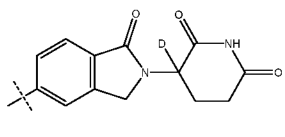 , 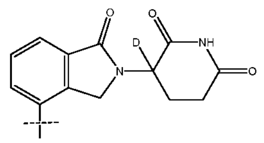 ,

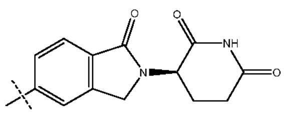 , 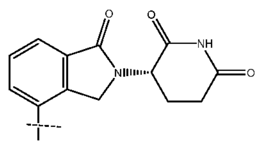 , 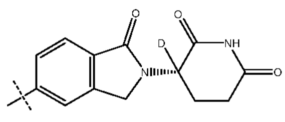 , 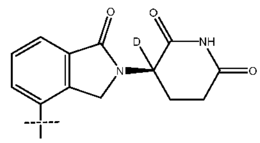 ,

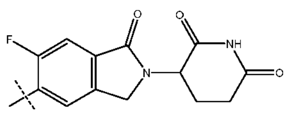 , 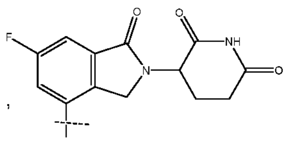

**27.** The compound according to any one of the preceding claims, wherein D is:

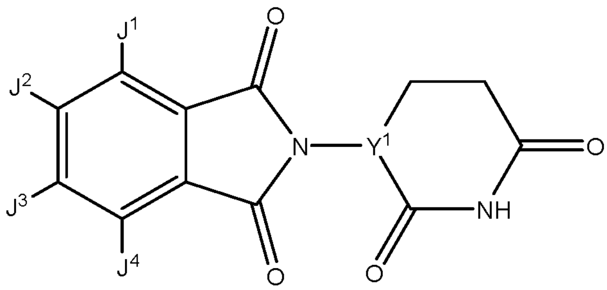

,

wherein $Y^1$, for each occurrence, is independently selected from CH, CD or N;
$J^1$, for each occurrence, is independently selected from absence, hydrogen, fluorine, chlorine, methyl, methoxy;
$J^2$, for each occurrence, is independently selected from absence, hydrogen, fluorine, chlorine, methyl, methoxy;
$J^3$, for each occurrence, is independently selected from absence, hydrogen, fluorine, chlorine, methyl, methoxy;
$J^4$, for each occurrence, is independently selected from absence, hydrogen, fluorine, chlorine, methyl, methoxy;
one or more of $J^1$, $J^2$, $J^3$ and $J^4$ are linked to L groups.

**28.** The compound according to any one of the preceding claims, wherein D is a group selected from:

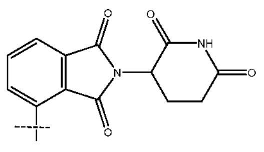 , 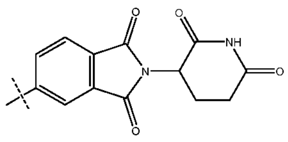 , 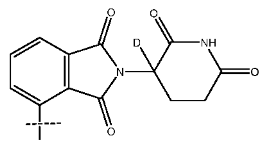 , 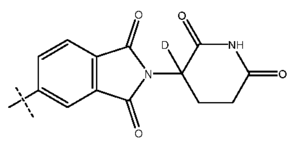 ,

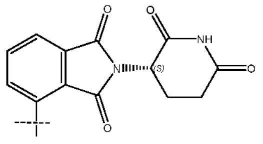 , 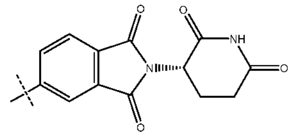 , 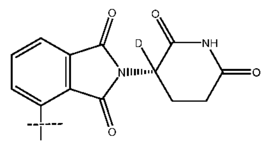 , 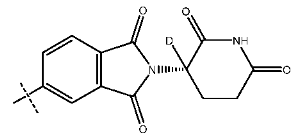 ,

.

**29.** The compound according to any one of the preceding claims, wherein D is:

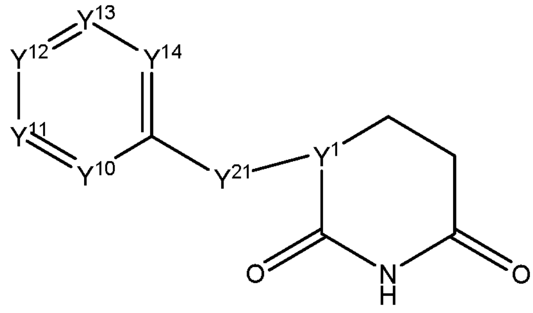

,

$Y^1$, for each occurrence, is independently selected from CH, CD or N;
$Y^{21}$ is independently selected from NH, N-$CH_3$ or O;

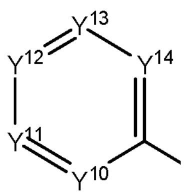

is an aromatic ring or a heteroaromatic ring, wherein
$Y^{10}$, for each occurrence, is independently selected from: absence; O; S; N; NH; $CT^{10}$, wherein $T^{10}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy; $NT^{10'}$, wherein $T^{10'}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;
$Y^{11}$, for each occurrence, is independently selected from absence; O; S; N; NH; $CT^{11}$, wherein $T^{11}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy; $NT^{11'}$, wherein $T^{11'}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;
$Y^{12}$, for each occurrence, is independently selected from absence; O; S; N; NH; $CT^{12}$, wherein $T^{12}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy; $NT^{12'}$, wherein $T^{12'}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;
$Y^{13}$, for each occurrence, is independently selected from absence; O; S; N; NH; $CT^{13}$, wherein $T^{13}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy; $NT^{13'}$, wherein $T^{13'}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;
$Y^{14}$, for each occurrence, is independently selected from absence; O; S; N; NH; $CT^{14}$, wherein $T^{14}$,for each occurrence, is independently selected from absence, hydrogen, fluorine, chlorine, methyl, $CH_2F$, $CHF_2$, $CF_3$,

$CH_2Cl$, $CHCl_2$, $CCl_3$, methoxy; $NT^{14'}$, wherein $T^{14'}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;
one or more of $Y^{10}$, $Y^{11}$, $Y^{12}$, $Y^{13}$ and $Y^{14}$ are linked to L groups; or
any two adjacent ones of $Y^{10}$, $Y^{11}$, $Y^{12}$, $Y^{13}$ and $Y^{14}$ are joined together with the connected atoms to form an optionally substituted aryl or an optionally substituted heteroaryl, which is linked to L groups by one or more bonds; preferably, the aryl or heteroaryl is selected from phenyl, naphthyl, anthracenyl, indenyl, indanyl, 1,2-dihydronaphthyl, 1,2,3,4-tetrahydronaphthyl, pyridyl, pyrimidinyl, imidazolyl, pyrazolyl, triazolyl, tetrazolyl, furanyl, thienyl, oxazolyl, isoxazolyl, thiazolyl, isothiazolyl, oxadiazolyl, thiadiazolyl, triazinyl, and the substituents thereon are selected from: absence, fluorine, chlorine, methyl, $CH_2F$, $CHF_2$, $CF_3$, $CH_2Cl$, $CHCl_2$, $CCl_3$, methoxy.

**30.** The compound according to any one of the preceding claims, wherein D is a group selected from:

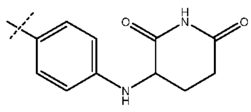 , 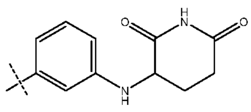 , 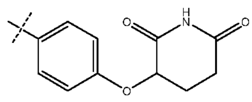 , 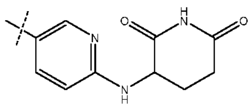 ,

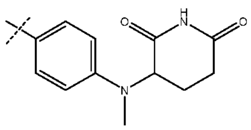 , 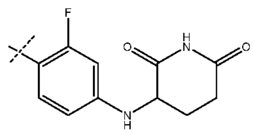 , 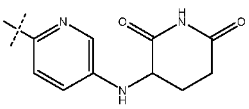 , 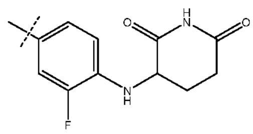 ,

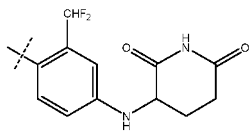 , 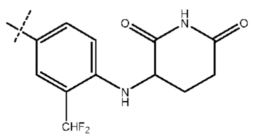 , 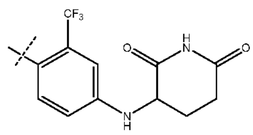 , 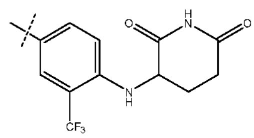 ,

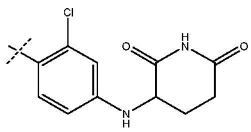 , 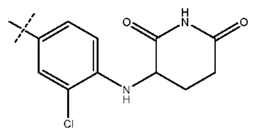 , 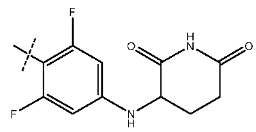 , 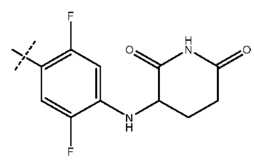 ,

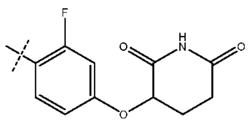 , 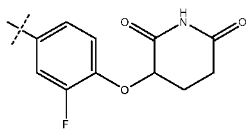 , 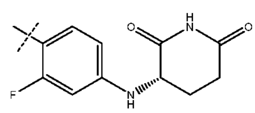 , 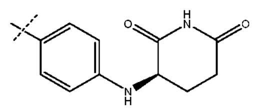 ,

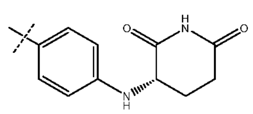 , 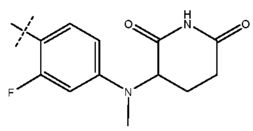 , 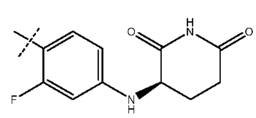 , 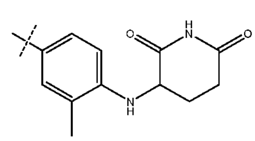 ,

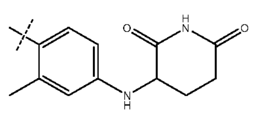 ,

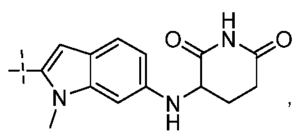 , 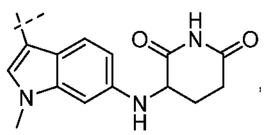 , 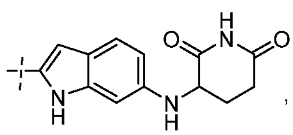 , 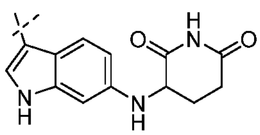 ,

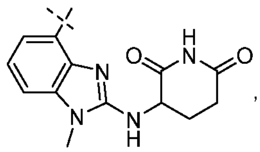 , 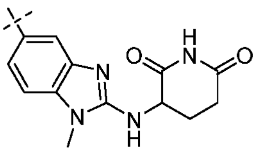 , 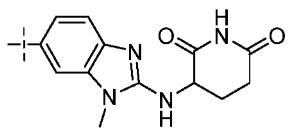 , 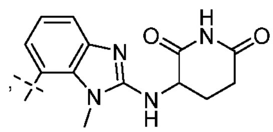 ,

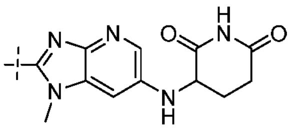 , 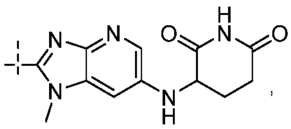 , 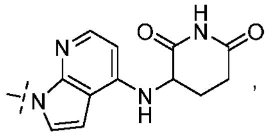 , 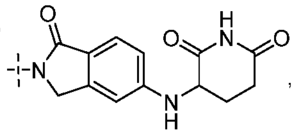 ,

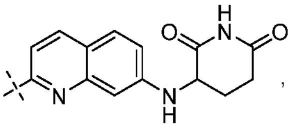 , 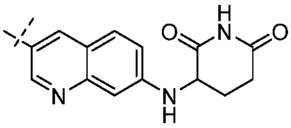 , 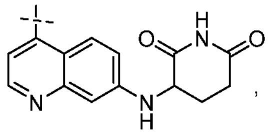 , 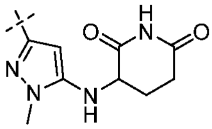 ,

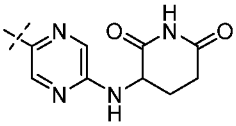 , 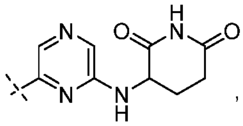 , 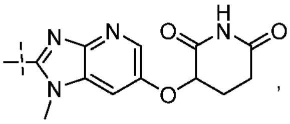 , 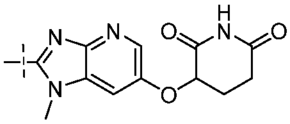 ,

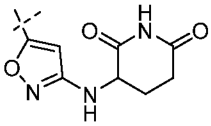 , 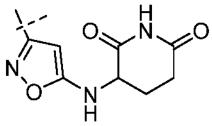 , 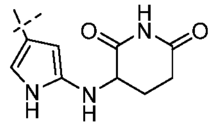 , 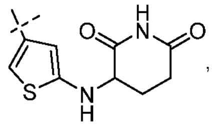 ,

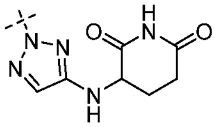 , 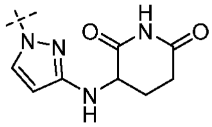 , 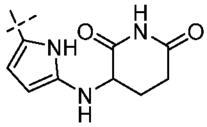 , 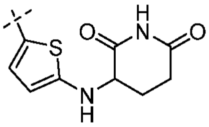 ,

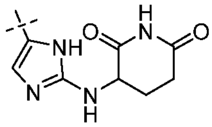 , 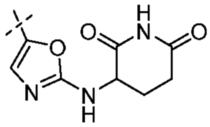 , 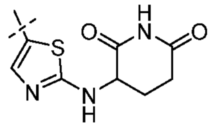 , 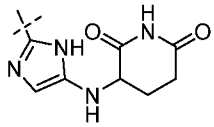 ,

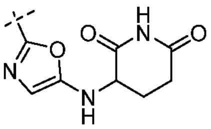 , 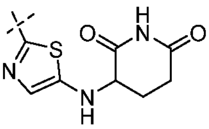 , 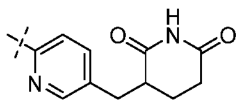 , 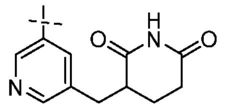 ,

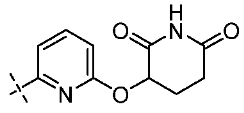 , 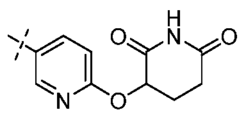 ,

**31.** The compound according to any one of the preceding claims, wherein D is:

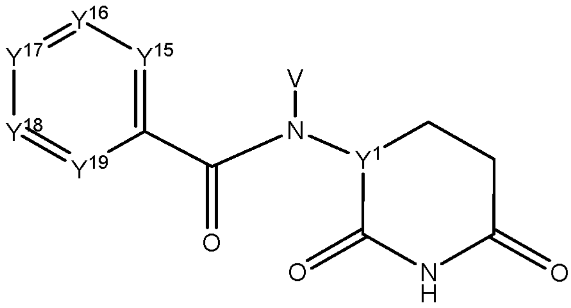

,

wherein

$Y^1$, for each occurrence, is independently selected from CH, CD or N;
V is hydrogen or methyl;

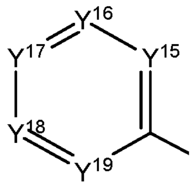

is an aromatic ring or a heteroaromatic ring, wherein
$Y^{15}$, for each occurrence, is independently selected from: absence; O; S; N; NH; $CT^{15}$, wherein $T^{15}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy; $NT^{15'}$, wherein $T^{15'}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;
$Y^{16}$, for each occurrence, is independently selected from: absence; O; S; N; NH; $CT^{16}$, wherein $T^{16}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy; $NT^{16'}$, wherein $T^{16'}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;
$Y^{17}$, for each occurrence, is independently selected from: absence; O; S; N; NH; $CT^{17}$, wherein $T^{17}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy; $NT^{17'}$, wherein $T^{17'}$, for each occurrence, is independently selected from absence, hydrogen, fluorine, chlorine, methyl, halogen $CH_2F$, $CHF_2$, $CF_3$, $CH_2Cl$, $CHCl_2$, $CCl_3$, methoxy;
$Y^{18}$, for each occurrence, is independently selected from: absence; O; S; N; NH; $CT^{18}$, wherein $T^{18}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy; $NT^{18'}$, wherein $T^{18'}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;
$Y^{19}$, for each occurrence, is independently selected from: absence; O; S; N; NH; $CT^{19}$, wherein $T^{19}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy; $NT^{19'}$, wherein $T^{19'}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;
one or more of $Y^{15}$, $Y^{16}$, $Y^{17}$, $Y^{18}$ and $Y^{19}$ are linked to L groups; or
any two adjacent ones of $Y^{15}$, $Y^{16}$, $Y^{17}$, $Y^{18}$ and $Y^{19}$ are joined together with the connected atom to form an optionally substituted aryl or an optionally substituted heteroaryl, which is linked to L groups by one or more bonds; preferably, the aryl or heteroaryl is selected from phenyl, naphthyl, anthracenyl, indenyl, indanyl, 1,2-dihydronaphthyl, 1,2,3,4- tetrahydronaphthyl, pyridyl, pyrimidinyl, imidazolyl, pyrazolyl, triazolyl, tetrazolyl, furanyl, thienyl, oxazolyl, isoxazolyl, thiazolyl, isothiazolyl, oxadiazolyl, thiadiazolyl, triazinyl, and the substituents thereon are selected from: absence, fluorine, chlorine, methyl, $CH_2F$, $CHF_2$, $CF_3$, $CH_2Cl$, $CHCl_2$, $CCl_3$, methoxy.

**32.** The compound according to any one of the preceding claims, wherein D is:

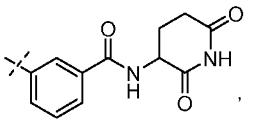 , 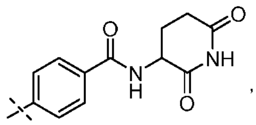 , 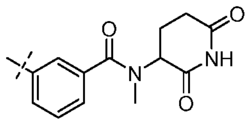 , 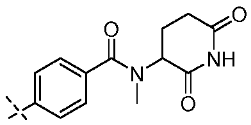 ,

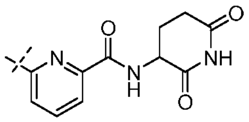, 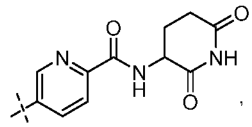, 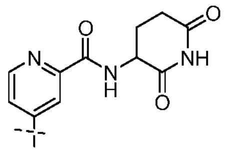, 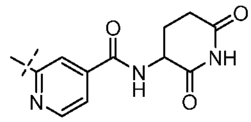,

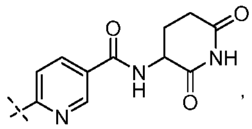, 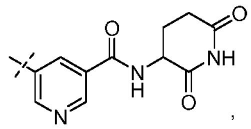, 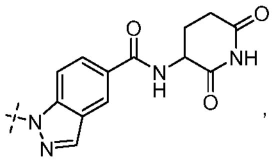, 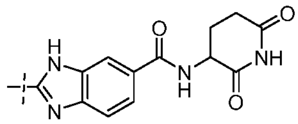,

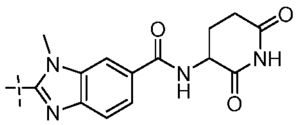

**33.** The compound according to any one of the preceding claims, wherein D is:

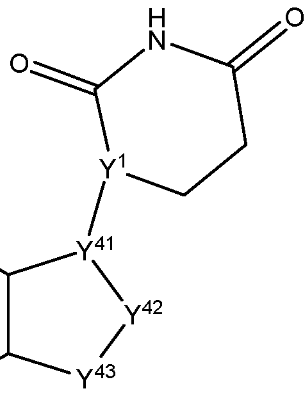,

wherein

$Y^1$, for each occurrence, is independently selected from CH, CD or N;
$Y^{41}$, for each occurrence, is independently selected from $CT^{41}$ or N;
$T^{41}$, for each occurrence, is independently selected from absence, hydrogen, deuterium, methyl;

$Y^{42}$, for each occurrence, is independently selected from $CH_2$, $CD_2$, CHD, C=O, C-$CH_3$, or 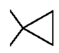;

$Y^{43}$, for each occurrence, is independently selected from $CH_2$, $CD_2$, CHD, C=O, C-$CH_3$, or 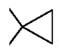; or
$Y^{42}$-$Y^{43}$ is -CH=CH-, -CH=N- or -N=CH-;
$Y^5$, for each occurrence, is independently selected from N; $CT^5$, wherein $T^5$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;
$Y^6$, for each occurrence, is independently selected from N; $CT^6$, wherein $T^6$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;
$Y^7$, for each occurrence, is independently selected from N; $CT^7$, wherein $T^7$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;
$Y^8$, for each occurrence, is independently selected from N; $CT^8$, wherein $T^8$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;
one or more of $Y^5$, $Y^6$, $Y^7$ and $Y^8$ are linked to L groups.

**34.** The compound according to any one of the preceding claims, wherein D is:

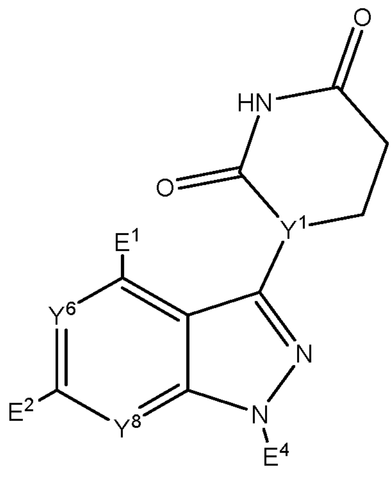

,

wherein

$Y^1$, for each occurrence, is independently selected from CH, CD or N ;
$Y^6$, for each occurrence, is independently selected from $CT^6$ or N;
$Y^8$, for each occurrence, is independently selected from $CT^8$ or N;
each of $T^6$, $T^8$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;
each of $E^1$, $E^2$ and $E^4$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;
one or more of $E^1$, $E^2$, $Y^6$ and $Y^8$ are linked to L groups.

**35.** The compound according to any one of the preceding claims, wherein D is:

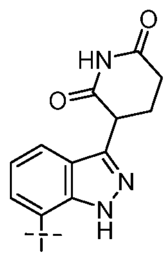 , 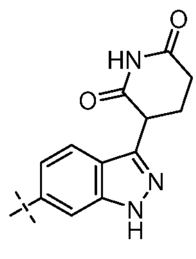 , 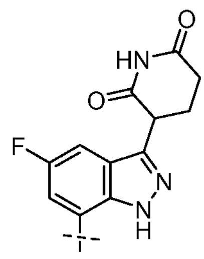 , 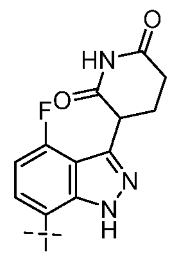 , 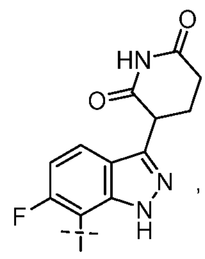 ,

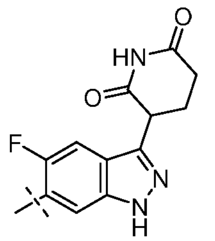 , 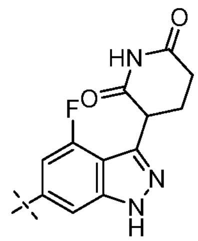 , 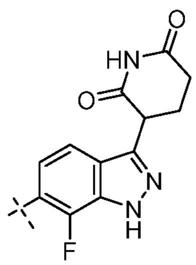 , 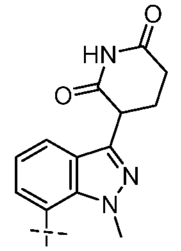 , 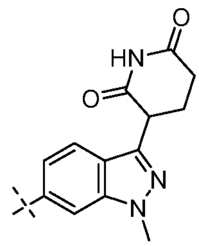 ,

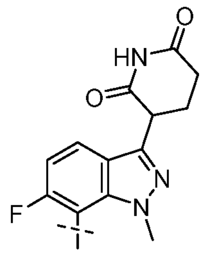 , 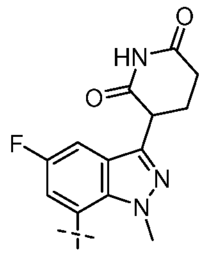 , 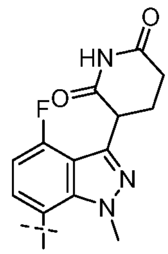 , 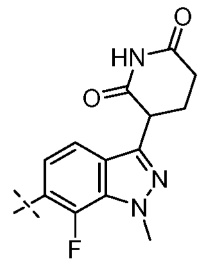 , 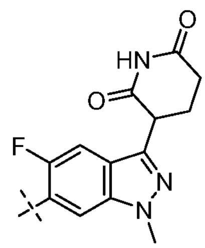 ,

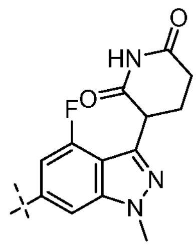 , 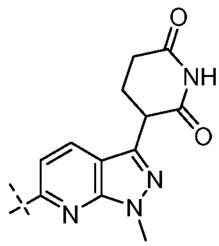 , 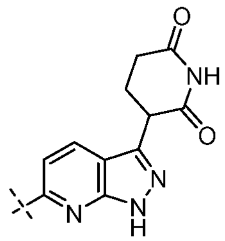 , 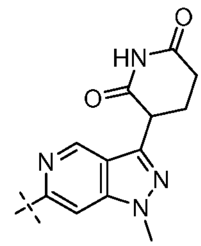 , 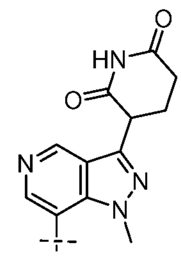 ,

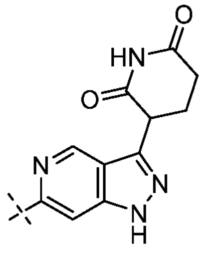 , 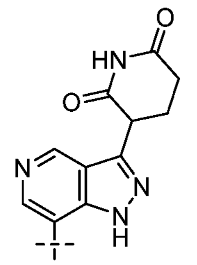 , 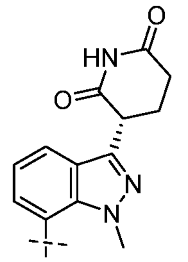 , 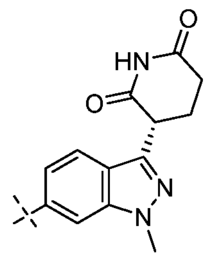 , 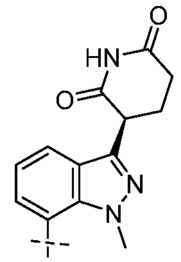 ,

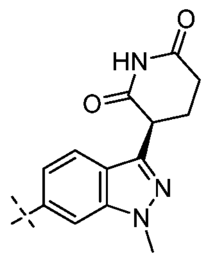 , 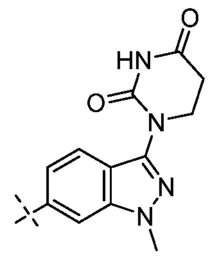 , 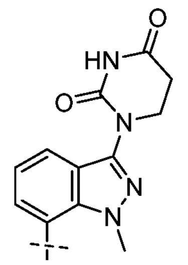 , 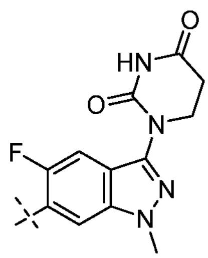 , 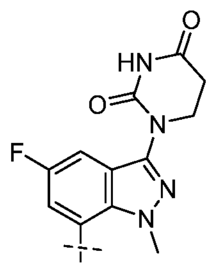

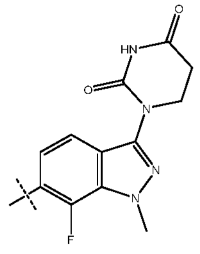, 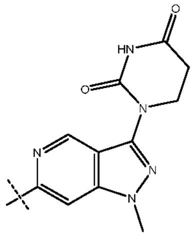, 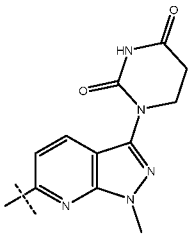, 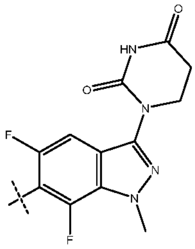, 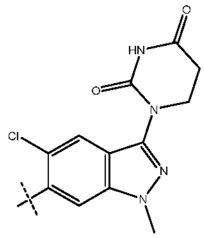.

**36.** The compound according to any one of the preceding claims, wherein D is:

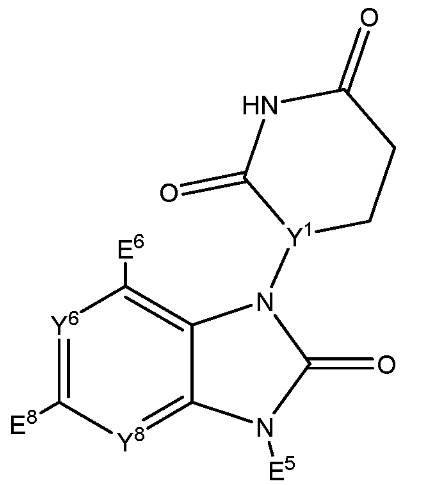

,

wherein

$Y^1$, for each occurrence, is independently selected from CH, CD or N;
$Y^6$, for each occurrence, is independently selected from $CT^6$ or N;
$Y^8$, for each occurrence, is independently selected from $CT^8$ or N;
each of $T^6$, $T^8$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;
each of $E^5$, $E^6$ and $E^8$, for each occurrence, is independently selected from absence, hydrogen, deuterium, halogen, hydroxyl, carbonyl, nitro, cyano, amino, mercapto, -COOH, $C_1$-$C_6$ alkyl, $C_2$-$C_6$ alkenyl, $C_2$-$C_6$ alkynyl, $C_1$-$C_6$ alkoxy, $C_2$-$C_6$ alkenyloxy, $C_2$-$C_6$ alkynyloxy, $C_2$-$C_6$ alkanoyl, $C_2$-$C_6$alkylester, $C_1$-$C_6$ thioalkyl, $C_1$-$C_6$ haloalkyl, $C_1$-$C_6$ haloalkoxy, hydroxy $C_1$-$C_6$ alkyl, amino $C_1$-$C_6$ alkyl, (mono- and di-$C_1$-$C_6$ alkylamino) $C_0$-$C_4$ alkyl, -$C_0$-$C_4$ alkyl ($C_3$-$C_7$ cycloalkyl), -O-$C_0$-$C_4$ alkyl ($C_3$-$C_7$ cycloalkyl), $C_3$-$C_{12}$ heterocyclyl, $C_6$-$C_{12}$ aryl, and $C_5$-$C_{10}$ heteroaryl, wherein the cycloalkyl, heterocyclyl, aryl, and heteroaryl are optionally substituted with 1, 2, 3, or 4 halogens, hydroxyl, carbonyl, nitro, cyano, amino, mercapto, -COOH, or $C_1$-$C_6$ alkyl;
one or more of $E^6$, $E^8$, $Y^6$ and $Y^8$ are linked to L groups.

**37.** The compound according to any one of the preceding claims, wherein D is:

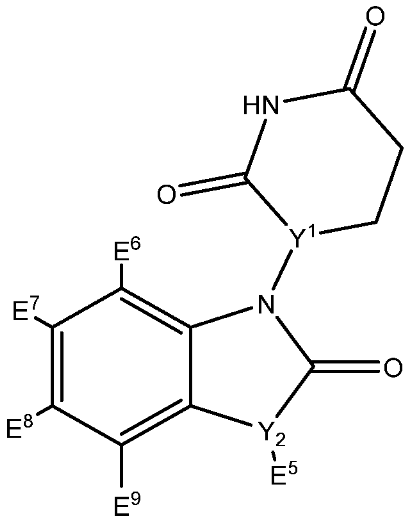

,

wherein

$Y^1$, for each occurrence, is independently selected from CH, CD or N;
$Y^2$, for each occurrence, is independently selected from CH, CD, N or oxygen;
$E^5$, for each occurrence, is independently selected from absence, hydrogen, deuterium or methyl;
each of $E^6$, $E^7$, $E^8$ and $E^9$, for each occurrence, is independently selected from absence, hydrogen, deuterium, fluorine, chlorine, methyl, $CH_2F$, $CHF_2$, $CF_3$, methoxy, or cyclopropyl;
one or more of $E^6$, $E^7$, $E^8$ and $E^9$ are linked to L groups.

**38.** The compound according to any one of the preceding claims, wherein D is:

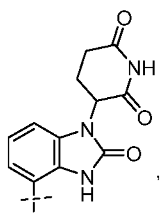 , 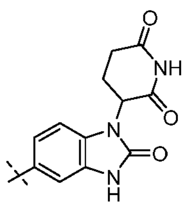 , 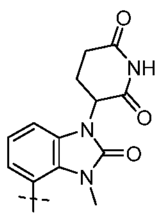 , 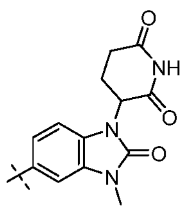 , 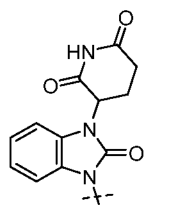 ,

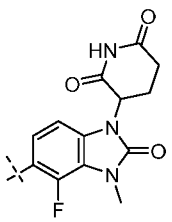 , 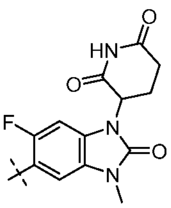 , 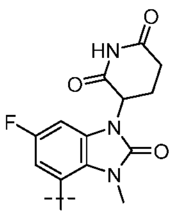 , 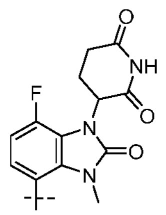 , 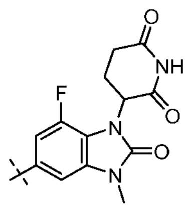 ,

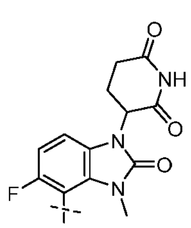 , 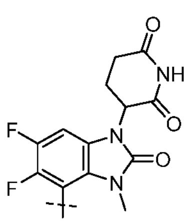 , 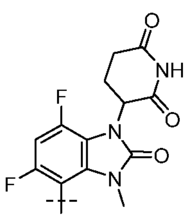 , 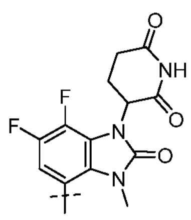 , 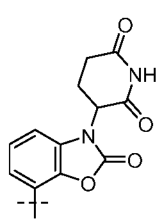 ,

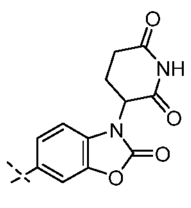, 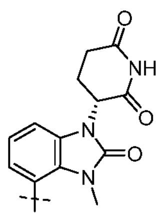, 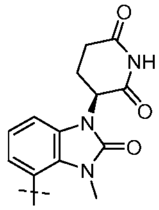

**39.** The compound according to any one of the preceding claims, wherein D is:

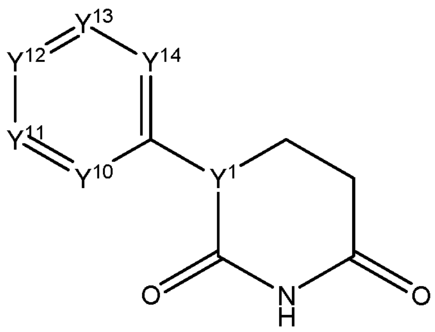

$Y^1$, for each occurrence, is independently selected from CH, CD or N;
$Y^{10}$, for each occurrence, is independently selected from N; $CT^{10}$, wherein $T^{10}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;
$Y^{11}$, for each occurrence, is independently selected from N; $CT^{11}$, wherein $T^{11}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;
$Y^{12}$, for each occurrence, is independently selected from N; $CT^{12}$, wherein $T^{12}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;
$Y^{13}$, for each occurrence, is independently selected from N; $CT^{13}$, wherein $T^{13}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;
$Y^{14}$, for each occurrence, is independently selected from N; $CT^{14}$, wherein $T^{14}$, for each occurrence, is independently selected from absence, hydrogen, fluorine, chlorine, methyl, $CH_2F$, $CHF_2$, $CF_3$, $CH_2Cl$, $CHCl_2$, $CCl_3$, methoxy;
one or more of $Y^{10}$, $Y^{11}$, $Y^{12}$, $Y^{13}$ and $Y^{14}$ are linked to L groups.

**40.** The compound according to any one of the preceding claims, wherein D is a structure selected from:

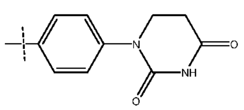, 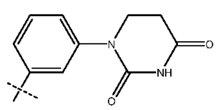, 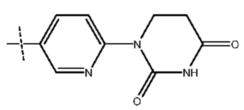, 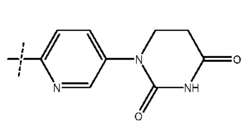,

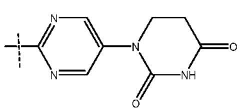, 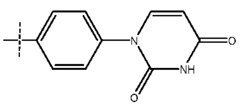, 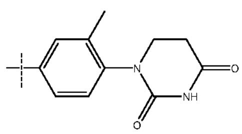, 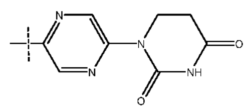,

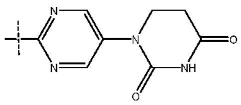 , 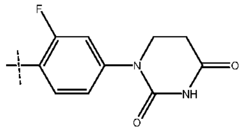 , 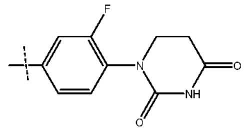 , 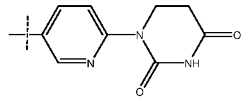 ,

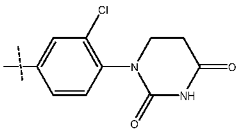 , 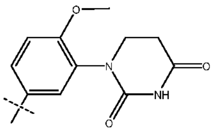

.

**41.** The compound according to any one of the preceding claims, wherein L is selected from:

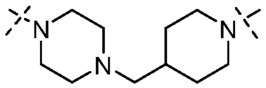 , 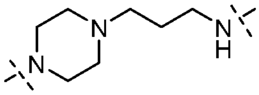 , 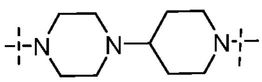 , 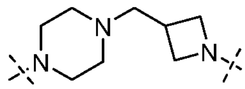 ,

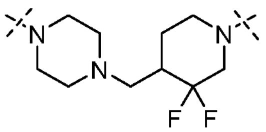 , 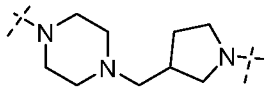 , 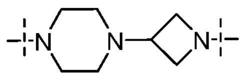 , 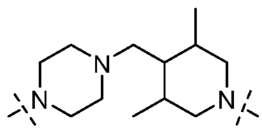 ,

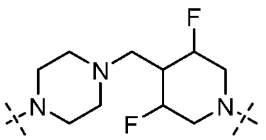 , 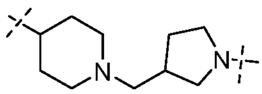 , 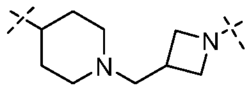 , 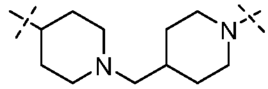 ,

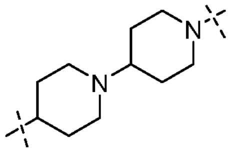 , 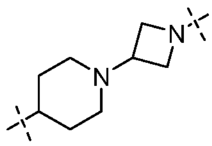 , 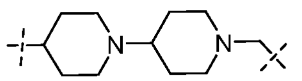 , 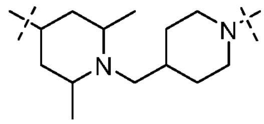 ,

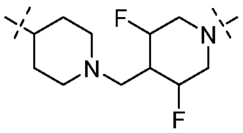 , 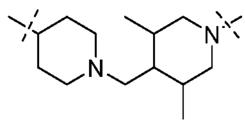 , 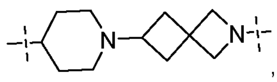 , 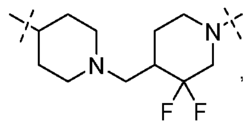 ,

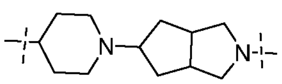 , 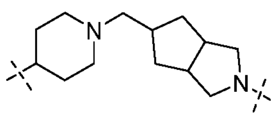 , 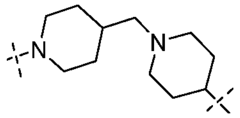 , 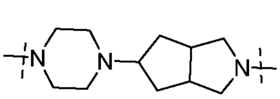 ,

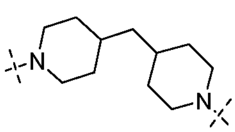 , 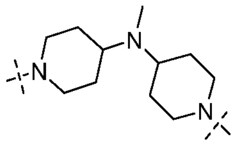 , 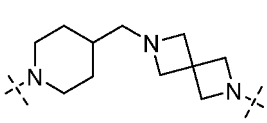 , 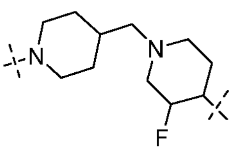 ,

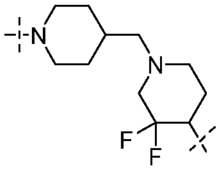 , 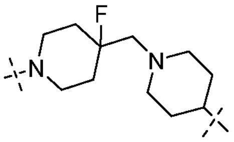 , 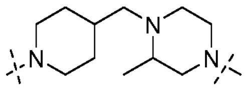 , 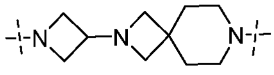 ,

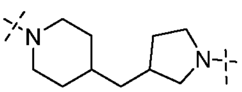 , 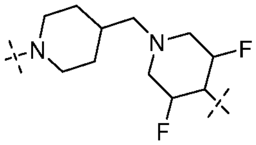 , 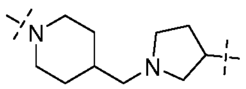 , 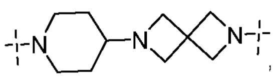 ,

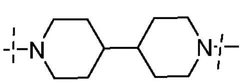 , 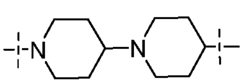 , 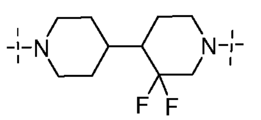 , 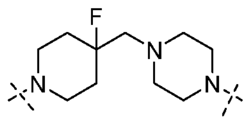 ,

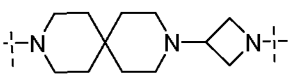 , 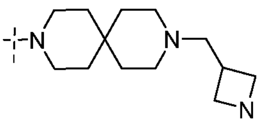 , 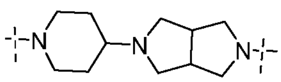 , 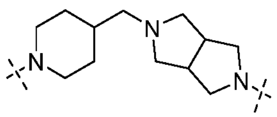 ,

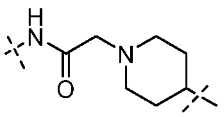 , 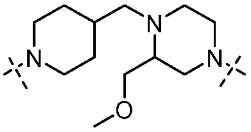 , 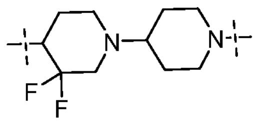 , 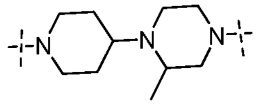 ,

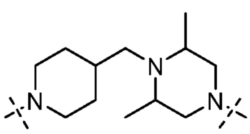 , 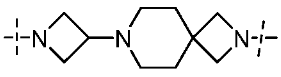 , 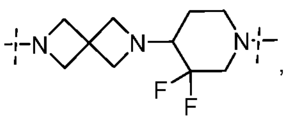 , 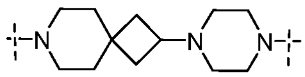 ,

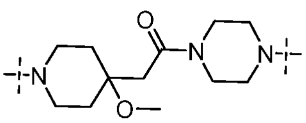 , 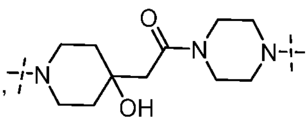 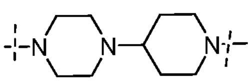 , 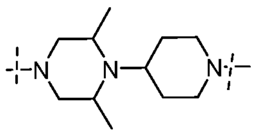

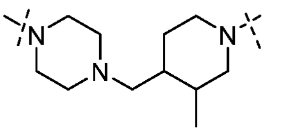 , 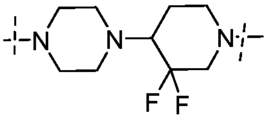 , 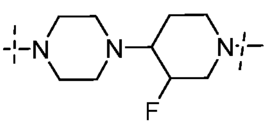 , 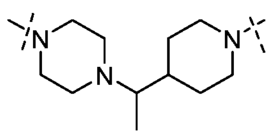 ,

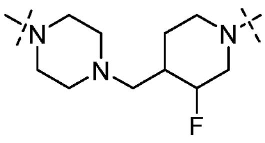 , 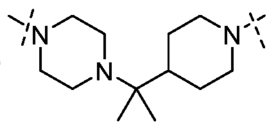 , 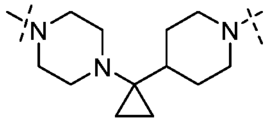 , 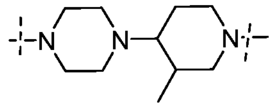 ,

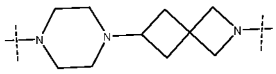 , 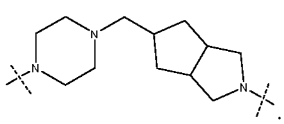 .

**42.** The compound according to any one of the preceding claims, wherein formula (I) is formula (II):

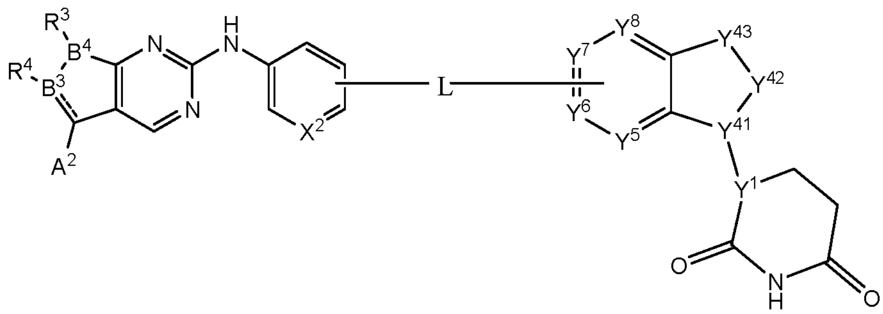 .

**43.** The compound according to any one of the preceding claims, wherein formula (I) is formula (III):

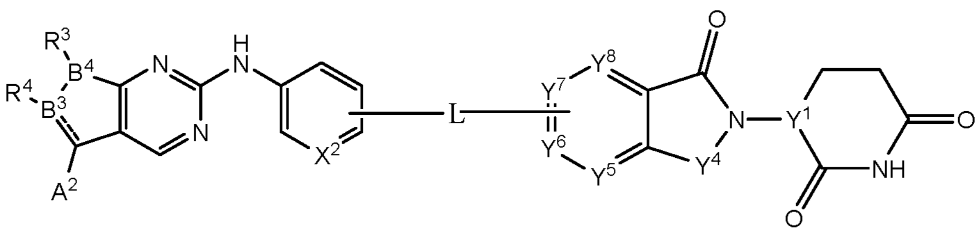 .

**44.** The compound according to any one of the preceding claims, wherein formula (I) is formula (IV):

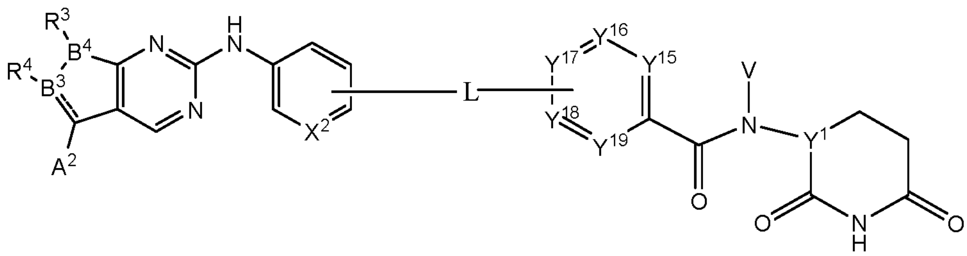 .

**45.** The compound according to any one of the preceding claims, wherein formula (I) is formula (V):

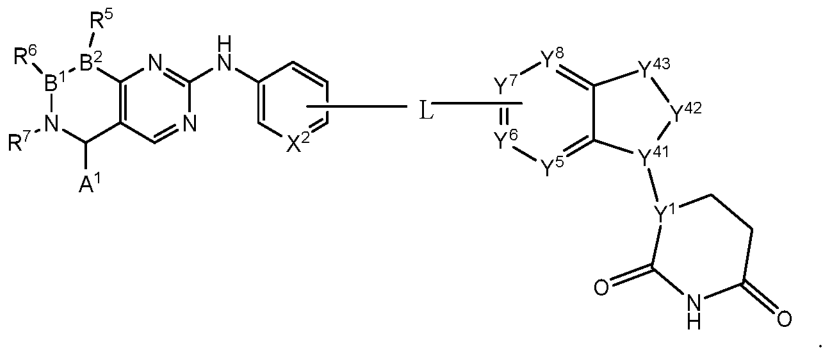

46. The compound according to any one of the preceding claims, wherein formula (I) is formula (VI):

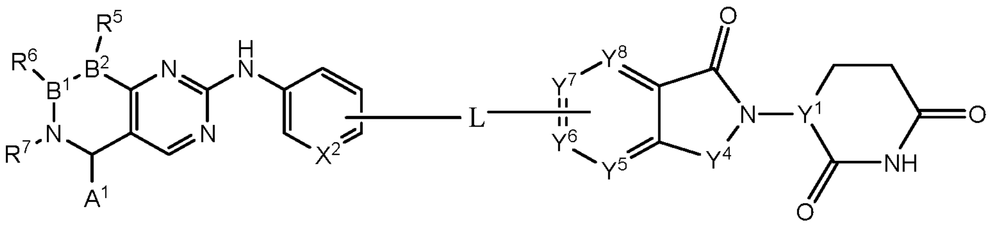

47. The compound according to any one of the preceding claims, wherein formula (I) is formula (VIII):

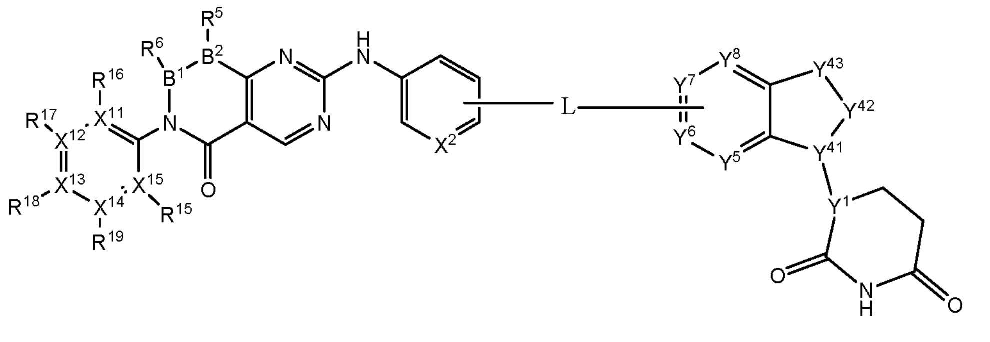

48. The compound according to any one of the preceding claims, wherein formula (I) is formula (IX):

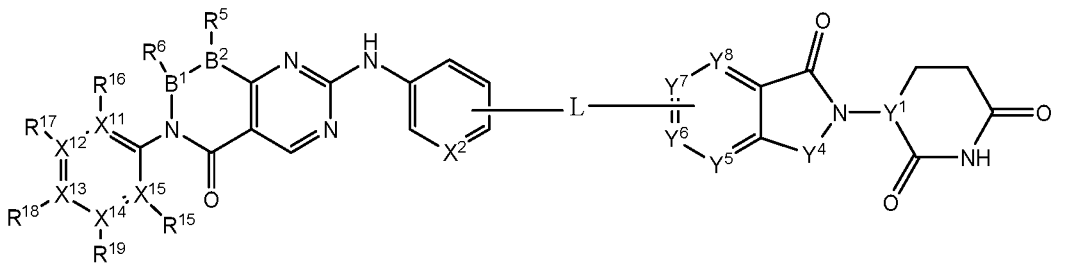

49. The compound according to any one of the preceding claims, wherein formula (I) is formula (XIV):

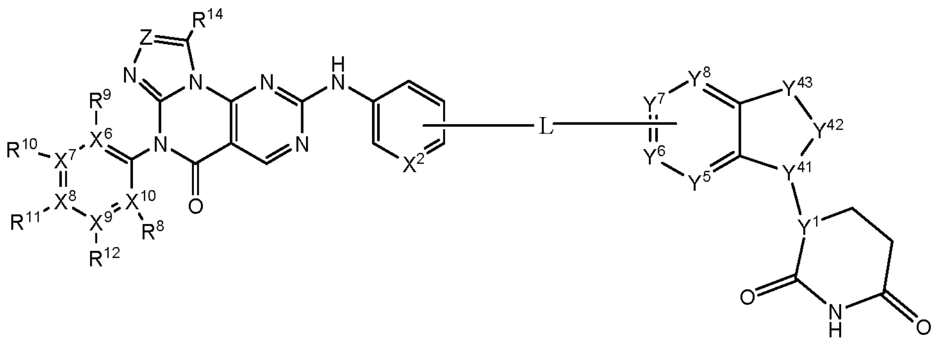

.

**50.** The compound according to any one of the preceding claims, wherein formula (I) is formula (XV):

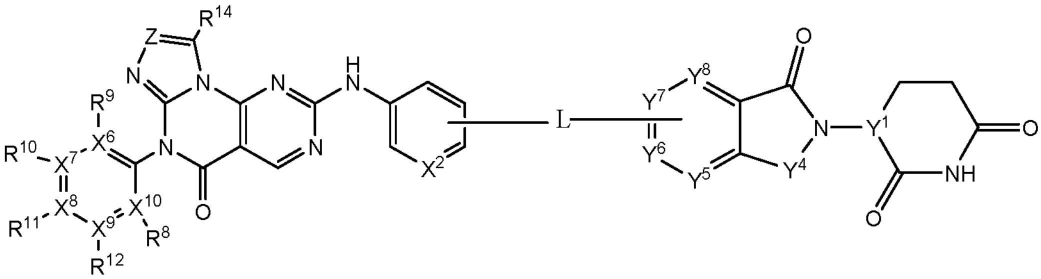

.

**51.** The compound according to any one of the preceding claims, wherein formula (I) is formula(XVII):

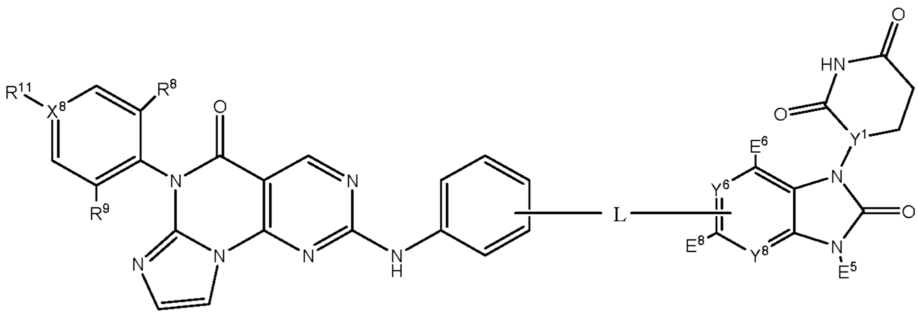

**52.** The compound according to any one of the preceding claims, wherein formula (I) is formula (XVIII):

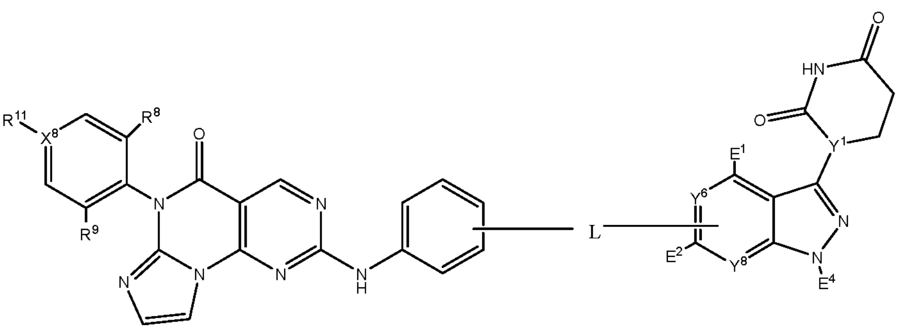

.

**53.** The compound according to any one of the preceding claims, wherein formula (I) is formula (XIX) :

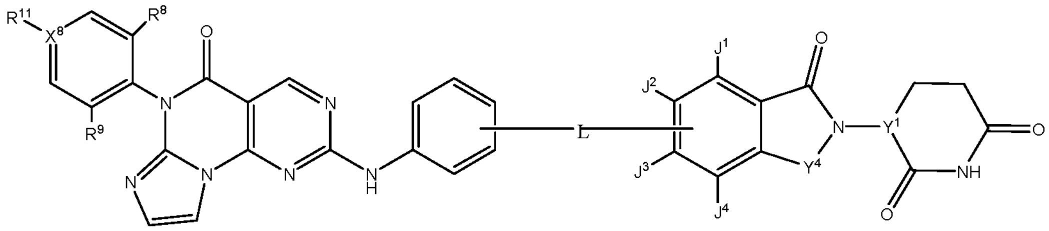

54. The compound according to any one of the preceding claims, wherein formula (I) is formula (XXIII):

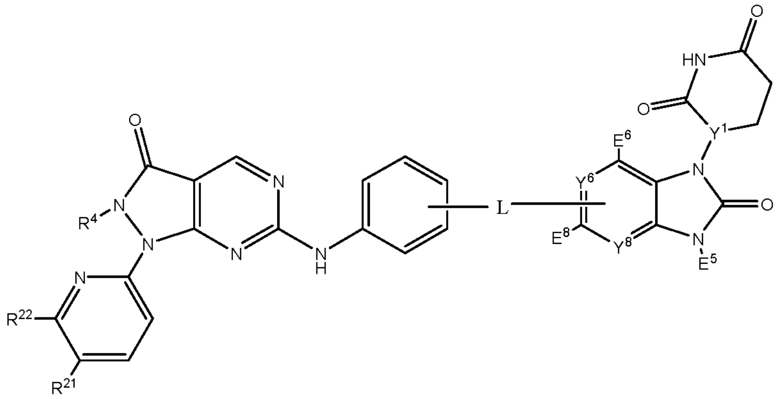

55. The compound according to any one of the preceding claims, wherein formula (I) is formula (XXIV):

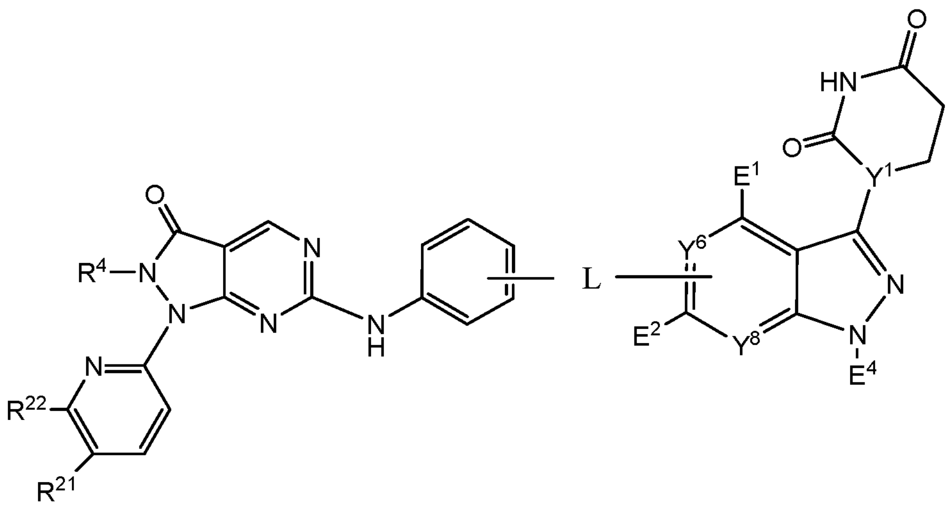

56. The compound according to any one of the preceding claims, wherein formula (I) is formula (XXV):

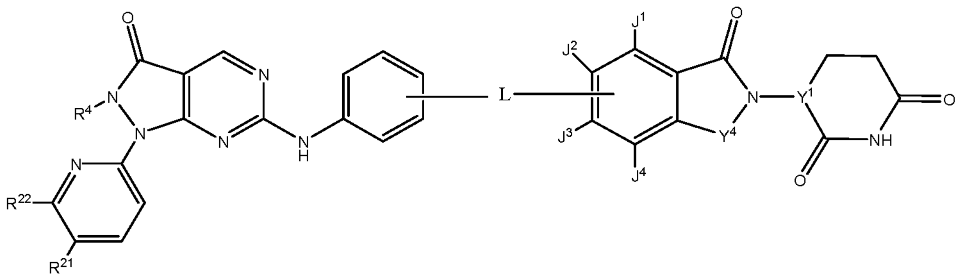

**57.** The compound according to any one of the preceding claims, wherein formula (I) is formula (XXVI):

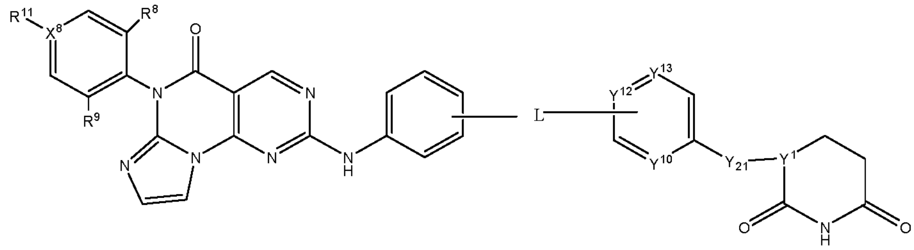

**58.** The compound according to any one of the preceding claims, wherein formula (I) is formula (XXVIII):

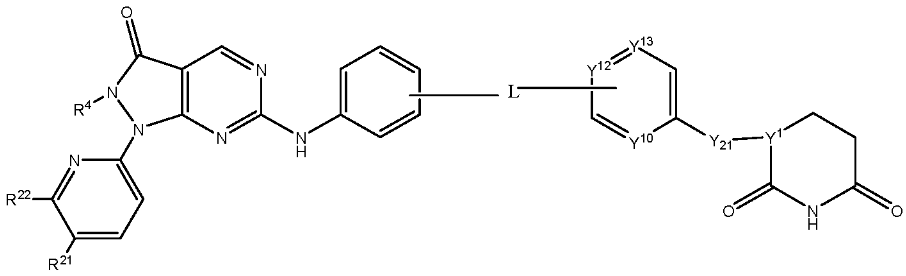

**59.** The compound according to any one of the preceding claims, wherein formula (I) is formula (XXIX):

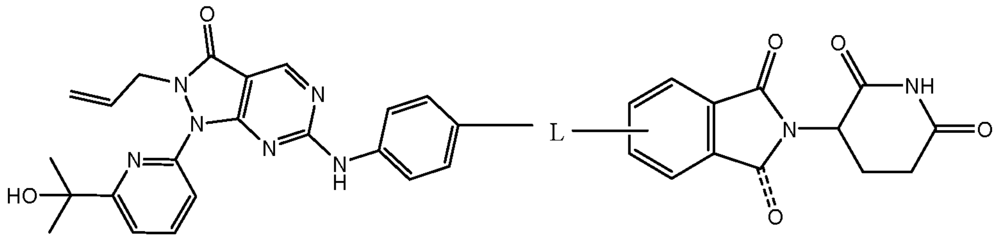

,

wherein:



represents: =O or absence.

**60.** The compound according to any one of the preceding claims, wherein formula (I) is formula (XXX):

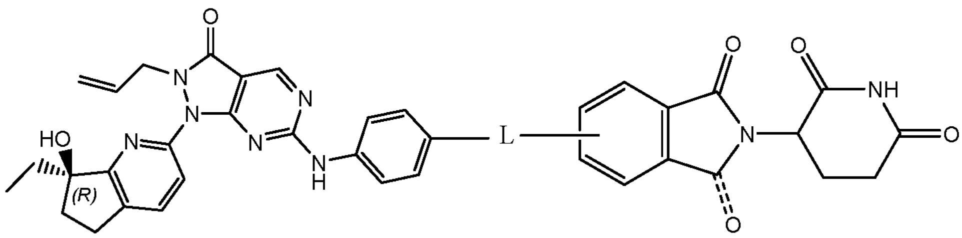

,

wherein:

represents: =O or absence.

**61.** The compound according to any one of the preceding claims, wherein formula (I) is formula (XXXI):

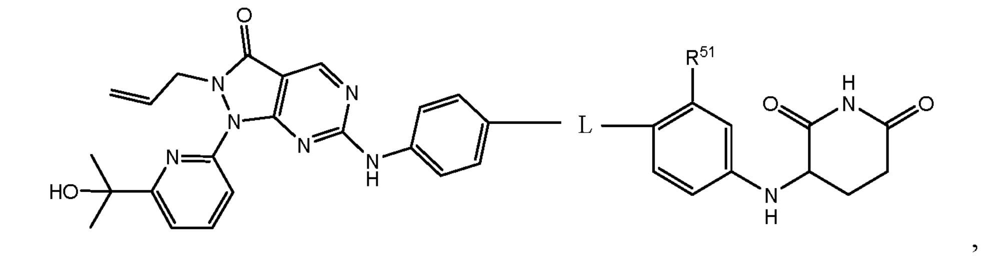

wherein:
$R^{51}$, for each occurrence, is independently selected from F, Cl or $CF_3$.

**62.** The compound according to any one of the preceding claims, wherein formula (I) is formula (XXXII) :

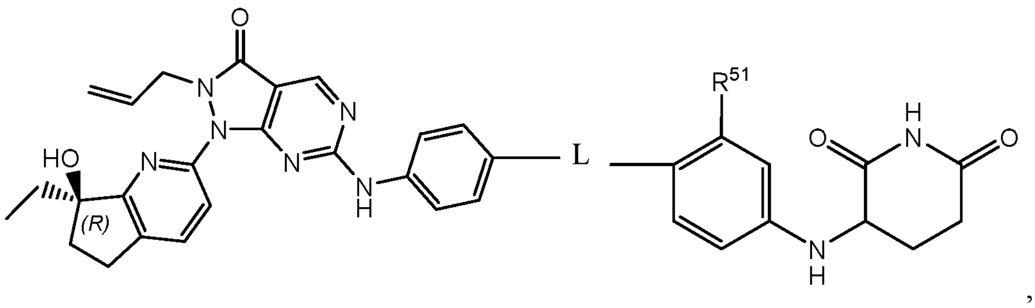

wherein:
$R^{51}$, for each occurrence, is independently selected from F, Cl or $CF_3$.

**63.** The compound according to any one of the preceding claims, wherein formula (I) is formula (XXXIII):

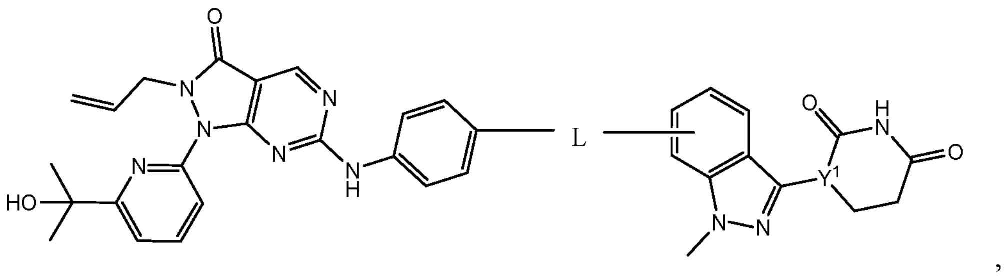

wherein:
$Y^1$, for each occurrence, is independently selected from CH, CD or N.

**64.** The compound according to any one of the preceding claims, wherein formula (I) is formula (XXXIV):

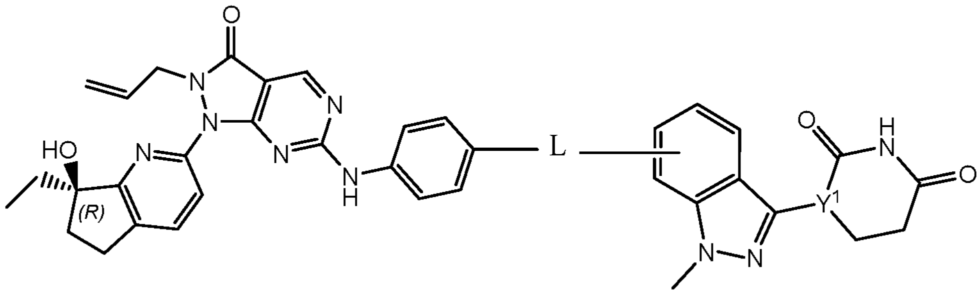

,

wherein:
$Y^1$, for each occurrence, is independently selected from CH, CD or N.

**65.** The compound according to any one of the preceding claims, wherein formula (I) is formula (XXXV):

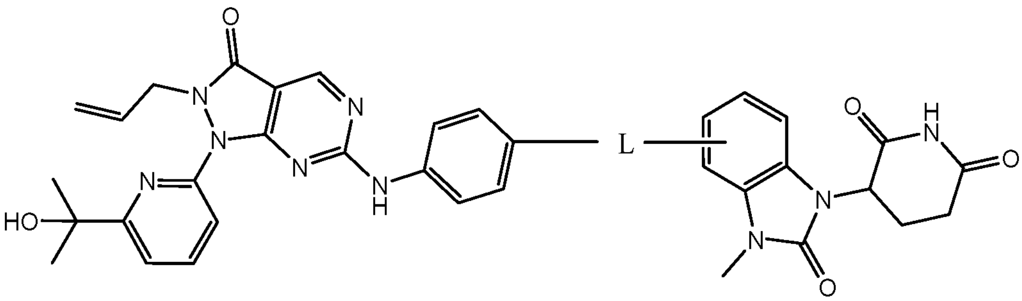

.

**66.** The compound according to any one of the preceding claims, wherein formula (I) is formula (XXXVI):

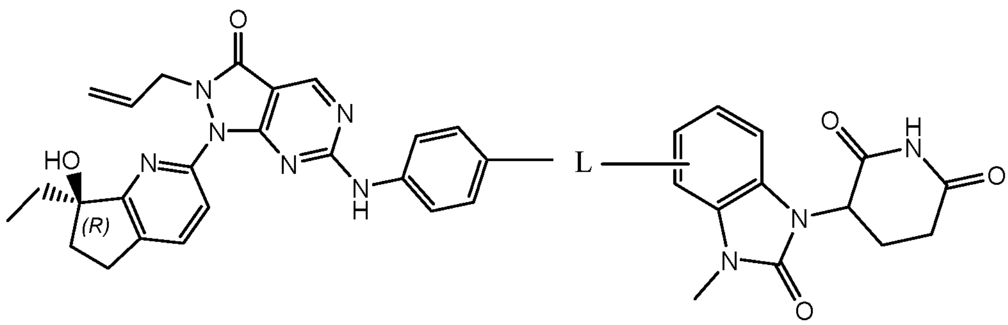

.

**67.** A compound represented by structural formula (XXXXI), or a derivative, pharmaceutically acceptable salt, isomer, solvate, hydrate, adduct, complex or prodrug thereof:

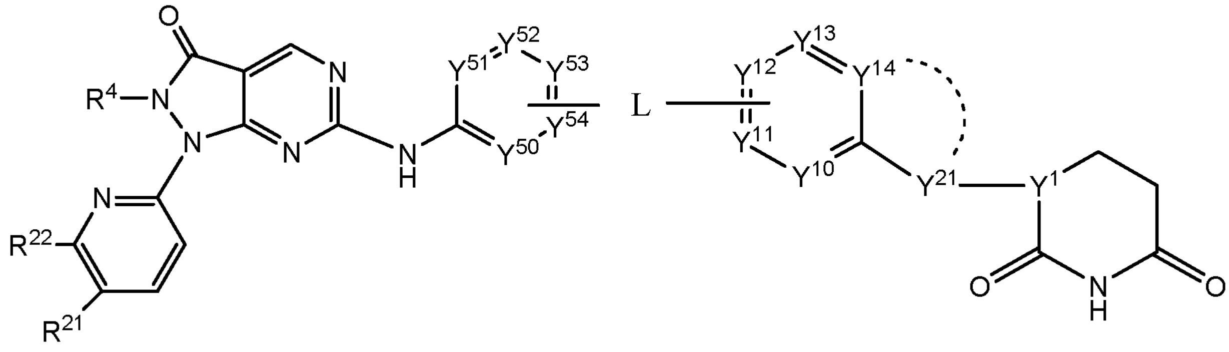

XXXXI,

wherein:

$Y^1$, for each occurrence, is independently selected from CH, CD or N;
$Y^{21}$, for each occurrence, is independently selected from C, O, N, NH, N-$CH_3$, N-$C_2H_5$, N-$C_3H_7$, N-$C_4H_9$;
each of $Y^{10}$, $Y^{11}$, $Y^{12}$, $Y^{13}$ and $Y^{14}$, for each occurrence, is independently selected from N; $CT^{101}$, wherein $T^{101}$, for each occurrence, is independently selected from absence, hydrogen, fluorine, chlorine, methyl, $CH_2F$, $CHF_2$, $CF_3$, $CH_2Cl$, $CHCl_2$, $CCl_3$, ethyl, propyl, isopropyl, butyl, isobutyl, tert-butyl, methoxyl, ethoxyl, propoxyl, isopropoxyl, butoxyl, isobutoxyl, tert-butoxyl, hydroxyl, cyano;
one of $Y^{10}$, $Y^{11}$, $Y^{12}$, $Y^{13}$ and $Y^{14}$ is linked to L groups;
each of $Y^{50}$, $Y^{51}$, $Y^{52}$, $Y^{53}$ and $Y^{54}$, for each occurrence, is independently selected from N; $CT^{102}$, wherein $T^{102}$, for each occurrence, is independently selected from absence, hydrogen, fluorine, chlorine, methyl, $CH_2F$, $CHF_2$, $CF_3$, $CH_2Cl$, $CHCl_2$, $CCl_3$, ethyl, propyl, isopropyl, butyl, isobutyl, tert-butyl, methoxyl, ethoxyl, propoxyl, isopropoxyl, butoxyl, isobutoxyl, tert-butoxyl, hydroxyl, cyano;
one or more of $Y^{50}$, $Y^{51}$, $Y^{52}$, $Y^{53}$ and $Y^{54}$ are linked to L groups;
$R^4$, for each occurrence, is independently selected from allyl, propargyl, methylene cyclopropyl, ethyl, trifluoroethyl, difluoroethyl, isopropyl, cyclopropyl, methylene $C_6$-$C_{12}$ aryl, methylene phenyl, optionally substituted $C_{1-4}$ alkyl, optionally substituted $C_{2-4}$ alkenyl, optionally substituted $C_{2-4}$ alkynyl, optionally substituted $C_{3-6}$ cycloalkyl and optionally substituted $C_{3-6}$ cycloalkyl ($C_{1-4}$ alkyl), wherein the $C_6$-$C_{12}$ aryl, $C_{1-4}$ alkyl, $C_{2-4}$ alkenyl and $C_{2-4}$ alkynyl are optionally independently substituted with one or more substituents selected from halogen, $C_{1-4}$ alkoxyl, $C_{1-4}$ haloalkyl, $C_{1-4}$ haloalkoxyl, cyano, amino, mono-$C_{1-4}$ alkylamine and di-$C_{1-4}$ alkylamine, and wherein one or more rings of the $C_{3-6}$ cycloalkyl and the $C_{3-6}$ cycloalkyl ($C_{1-4}$ alkyl) are optionally independently substituted with one or more substituents selected from halogen, $C_{1-4}$ alkyl, $C_{1-4}$ alkoxyl, $C_{1-4}$ haloalkyl, $C_{1-4}$ haloalkoxyl, cyano, amino, mono-$C_{1-4}$ alkylamine and di-$C_{1-4}$ alkylamine;

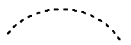 represents absence, -($NR^{81}$)-N=, -($NR^{82}$)-($NR^{83}$)-, -($NR^{84}$)-(C=O)-, or -(C=O)-($NR^{85}$)-, wherein each of $R^{81}$, $R^{82}$, $R^{83}$, $R^{84}$ and $R^{85}$, for each occurrence, is independently selected from hydrogen, methyl, ethyl, propyl, isopropyl, butyl, isobutyl, tert-butyl, methoxyl, ethoxyl, propoxyl, isopropoxyl, butoxyl, isobutoxyl, tert-butoxyl;
each of $R^{21}$ and $R^{22}$, for each occurrence, is independently selected from absence, hydrogen, deuterium, halogen, hydroxyl, carbonyl, nitro, cyano, amino, mercapto, allyl, propenyl, $C_1$-$C_6$ alkyl, $C_1$-$C_6$ alkoxyl, wherein the alkyl, and alkoxyl are optionally substituted with 1, 2, 3 or 4 halogens, hydroxyl, carbonyl, nitro, cyano, amino, mercapto; preferably, each of $R^{21}$ and $R^{22}$, for each occurrence, is independently selected from hydrogen, 2-hydroxyisopropyl; or
$R^{21}$, $R^{22}$ are joined together to form -($CR^{91}R^{92}$)-($CR^{93}R^{94}$)-($CR^{95}R^{96}$)-, wherein each of $R^{91}$, $R^{92}$, $R^{93}$, $R^{94}$, $R^{95}$, and $R^{96}$, for each occurrence, is independently selected from hydrogen, deuterium, fluorine, chlorine, bromine, hydroxyl, carbonyl, nitro, cyano, amino, mercapto, allyl, propenyl, methyl, ethyl, propyl, isopropyl, butyl, isobutyl, tert-butyl, methoxyl, ethoxyl, propoxyl, isopropoxyl, butoxyl, isobutoxyl, tert-butoxyl; preferably, each of $R^{91}$, $R^{92}$, $R^{93}$, $R^{94}$, $R^{95}$, and $R^{96}$, for each occurrence, is independently selected from hydrogen, hydroxyl, methyl, ethyl, propyl; further preferably, $R^{21}$, $R^{22}$ are joined together to form -($CR^{91}R^{92}$)-($CH_2$)-($CH_2$)- or -($CH_2$)-($CH_2$)-($CR^{95}R^{96}$)-, wherein each of $R^{91}$, $R^{92}$, $R^{95}$, and $R^{96}$, for each occurrence, is independently selected from hydroxyl, methyl, ethyl, propyl;
L is a linker and has the following structure:

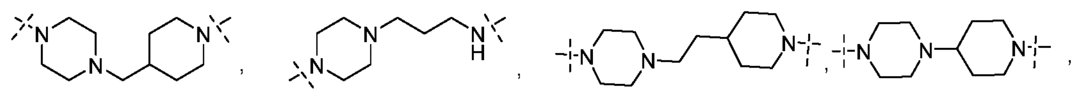
, , , ,

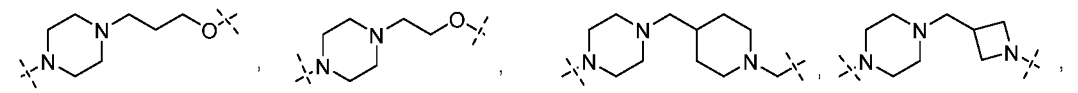
, , , ,

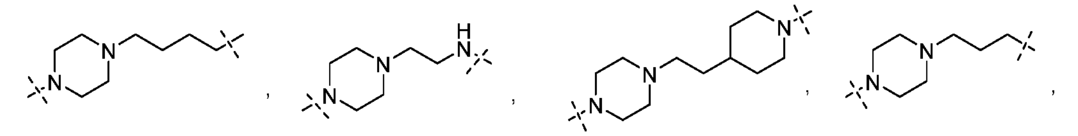
, , , ,

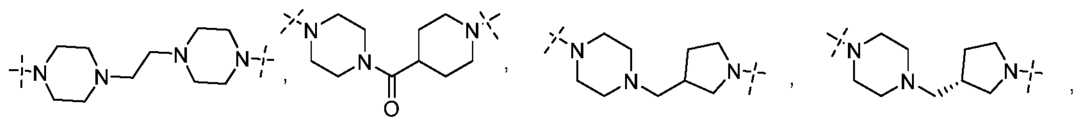
, , , ,

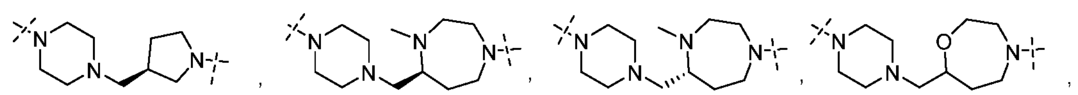
, , , ,

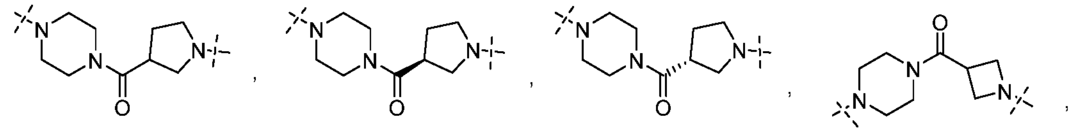
, , , ,

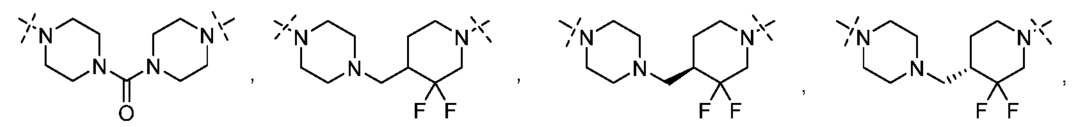
, , , ,

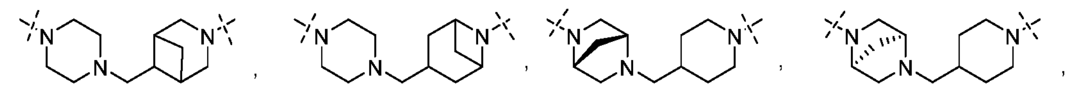
, , , ,

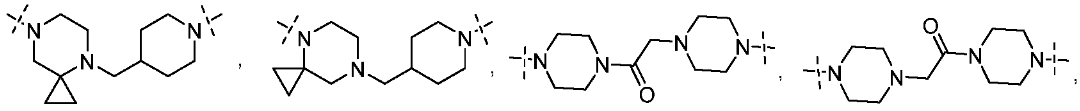
, , , ,

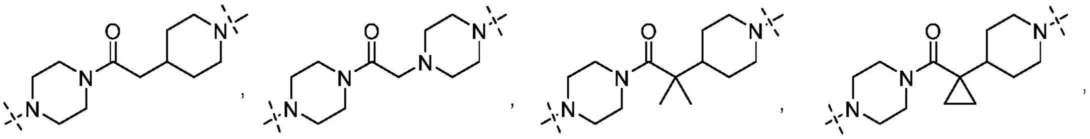
, , , ,

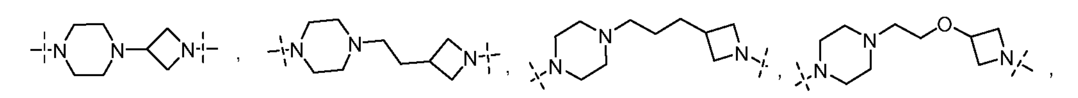
, , , ,

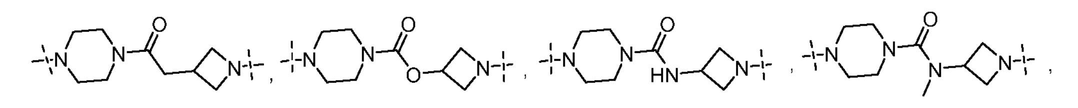
, , , ,

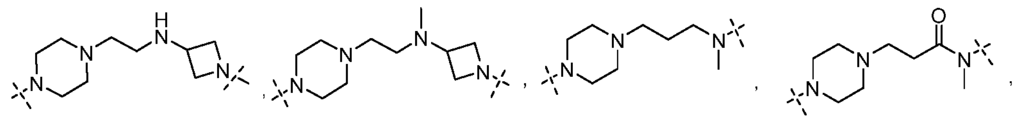
, , , ,

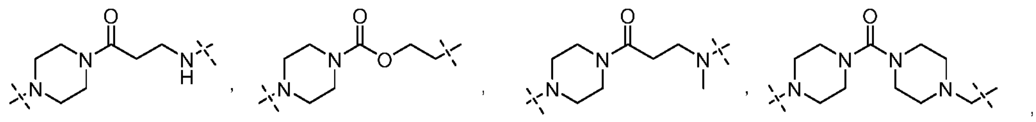
, , , ,

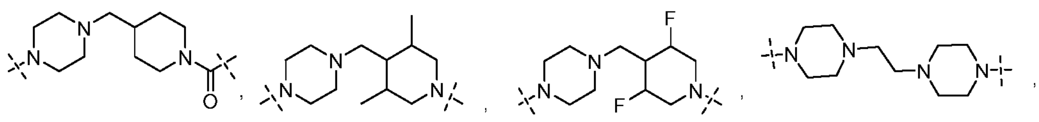
, , , ,

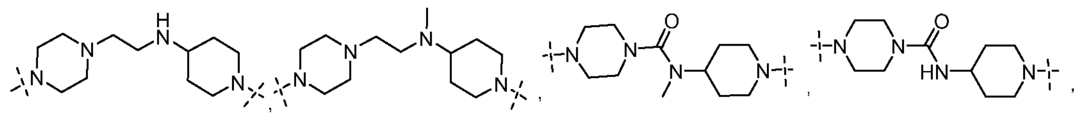
, , , ,

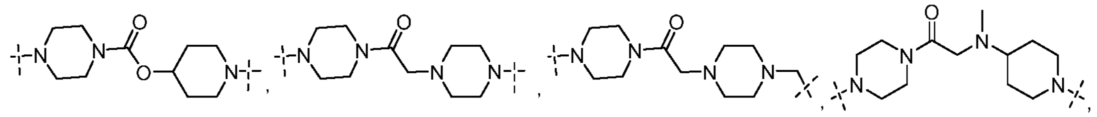
, , , ,

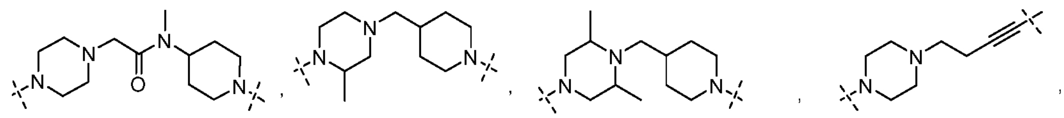
, , , ,

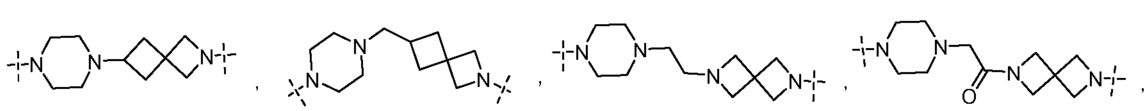
, , , ,

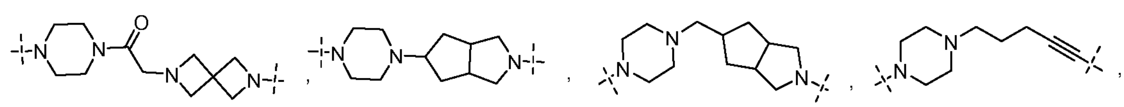
, , , ,

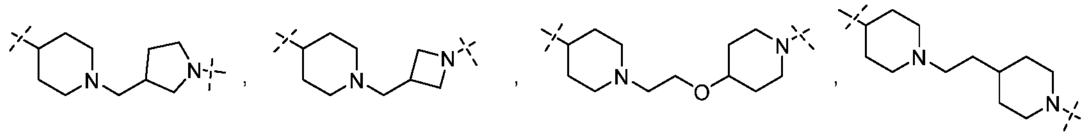
, , ,

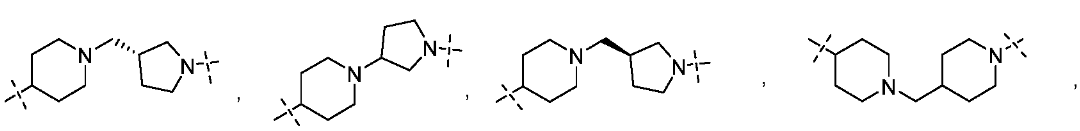
, , , ,

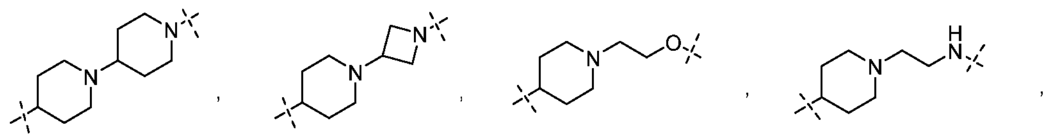
, , , ,

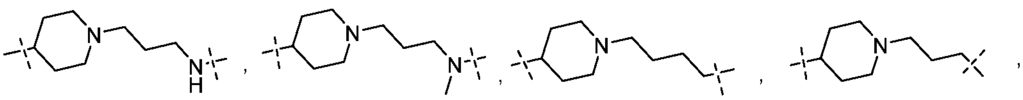

, , , ,

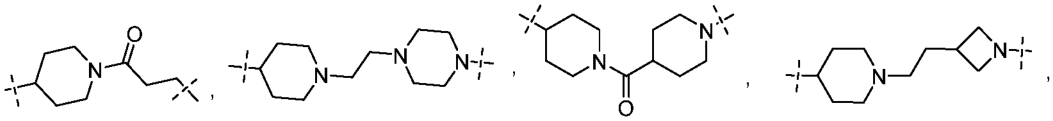

, , , ,

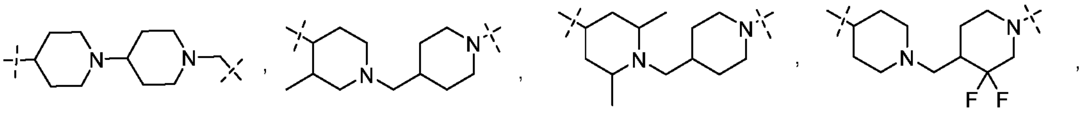

, , , ,

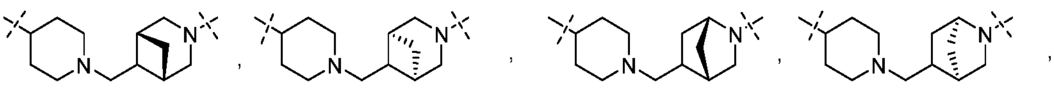

, , , ,

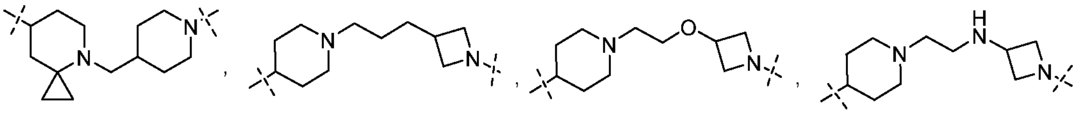

, , , ,

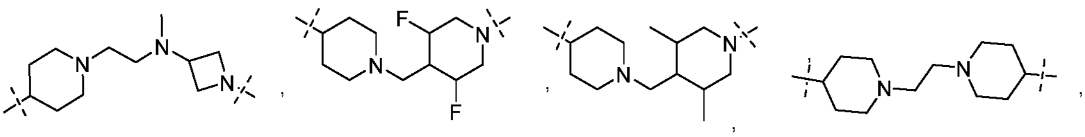

, , , ,

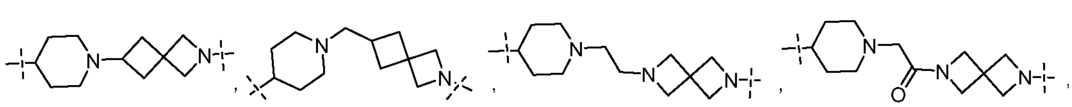

, , , ,

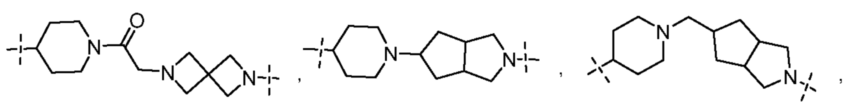

, , ,

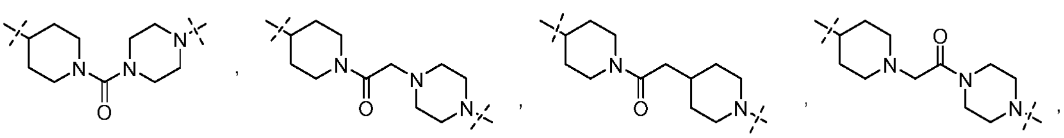

, , , ,

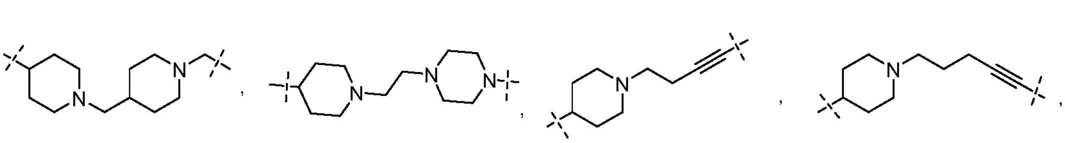

, , , ,

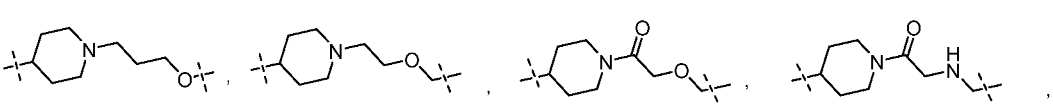

, , , ,

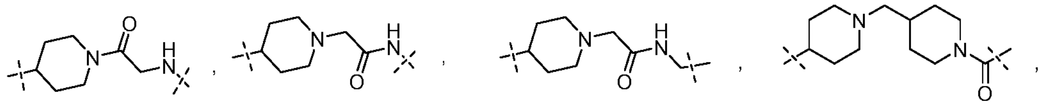

, , , ,

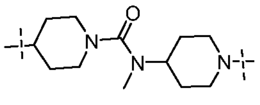 , 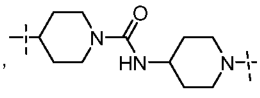 , 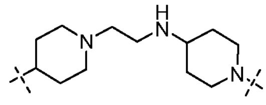 , 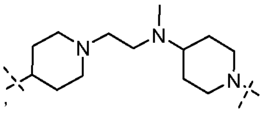 ,

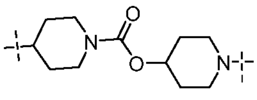 , 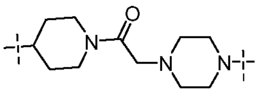 , 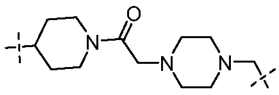 , 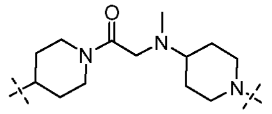 ,

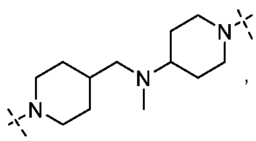 , 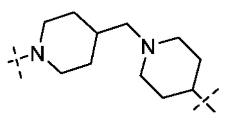 , 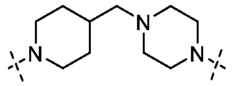 , 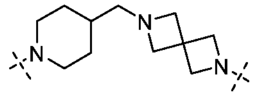 ,

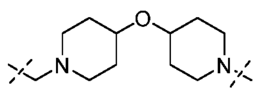 , 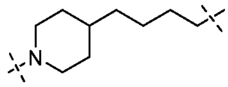 , 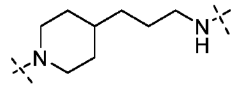 , 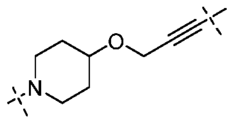 ,

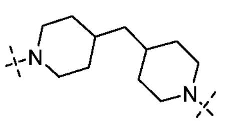 , 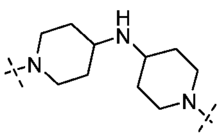 , 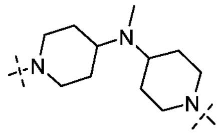 , 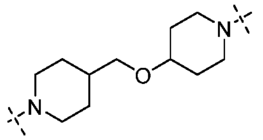 ,

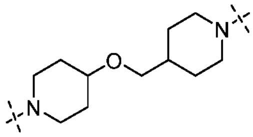 , 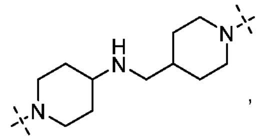 , 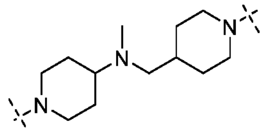 , 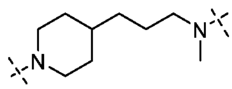 ,

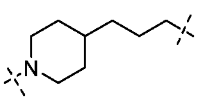 , 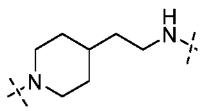 , 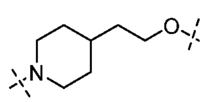 , 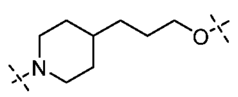 ,

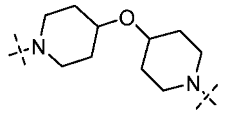 , 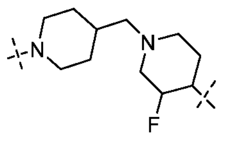 , 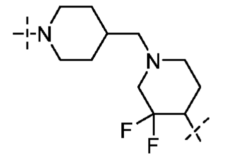 , 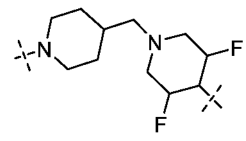 ,

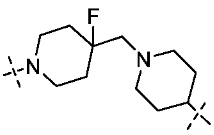 , 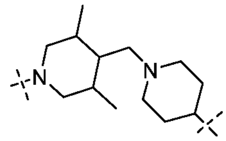 , 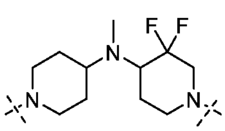 , 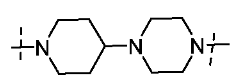 ,

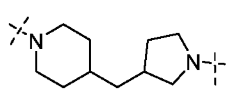 , 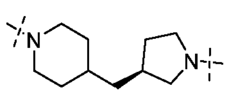 , 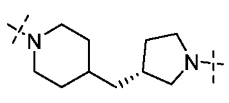 , 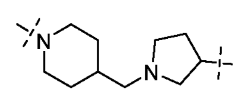

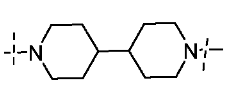 , 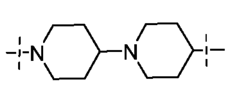 , 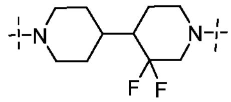 , 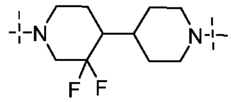

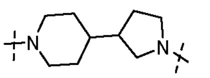 , 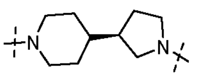 , 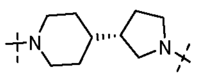 , 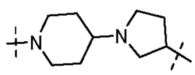 ,

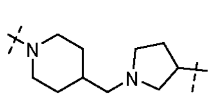 , 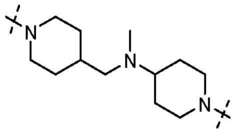 , 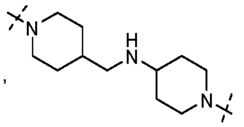 ,

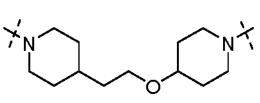 , 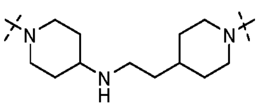 , 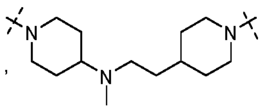 , 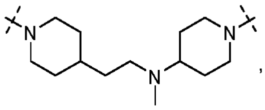 ,

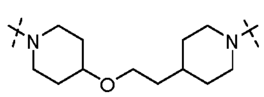 , 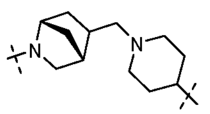 , 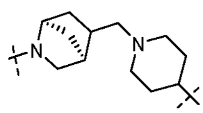 , 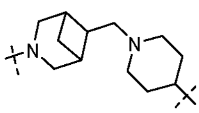 ,

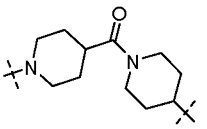 , 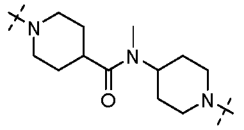 , 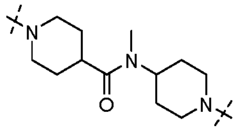 ,

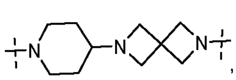 , 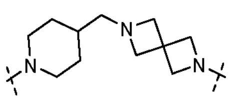 , 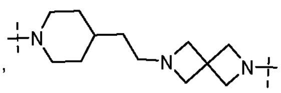 , 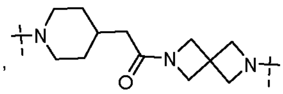 ,

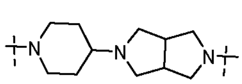 , 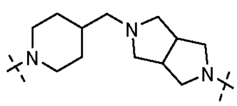 ,

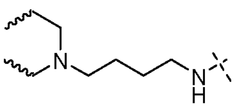 , 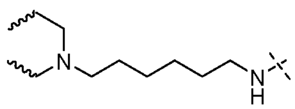 , 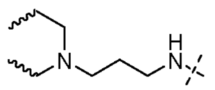 , 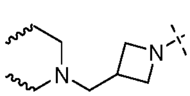 ,

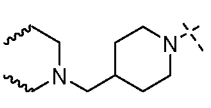 , 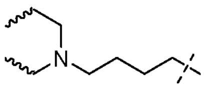 , 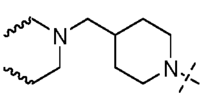 , 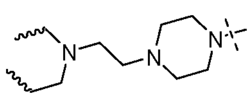 ,

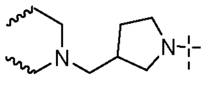 , 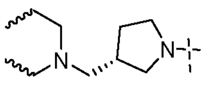 , 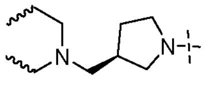 , 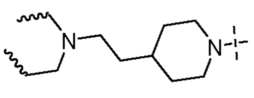 ,

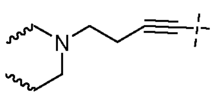 , 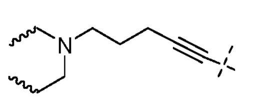 , 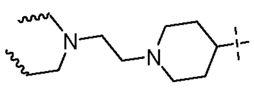 , 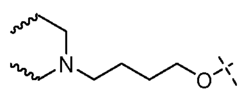 ,

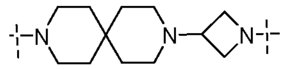 , 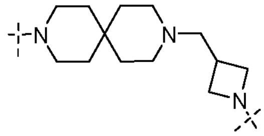 , 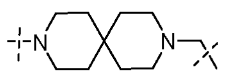 , 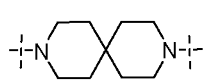 ,

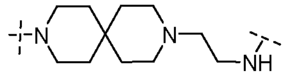 , 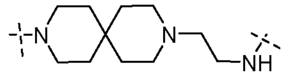 , 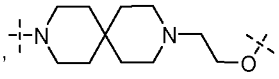 ,

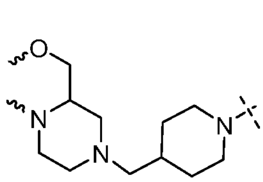 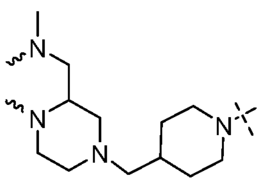 ,

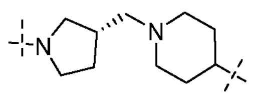 , 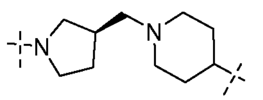 , 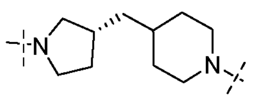 , 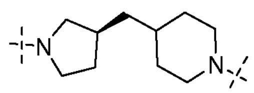 ,

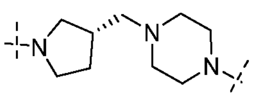 , 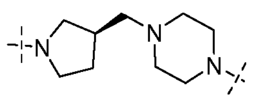 , 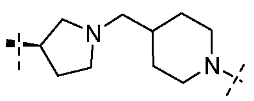 , 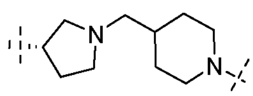 ,

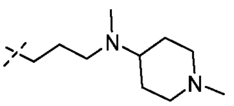 , 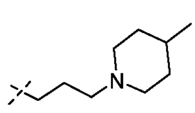 , 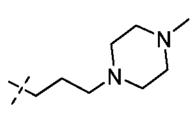 , 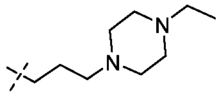 , 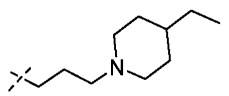 ,

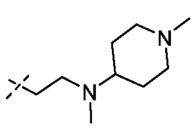 , 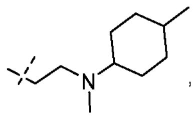 , 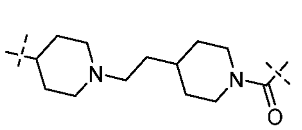 , 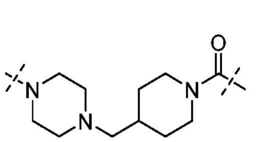 ,

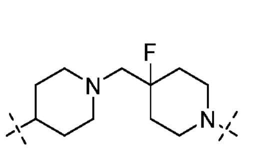 , 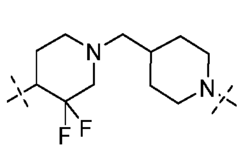 , 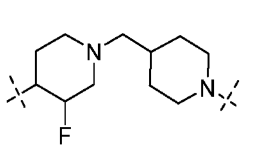 , 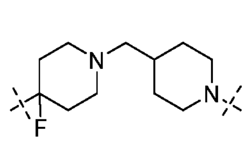 ,

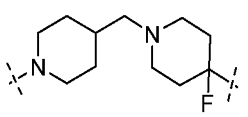 , 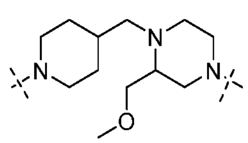 , 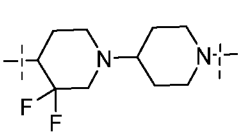 , 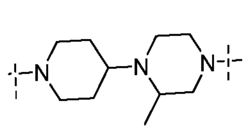 ,

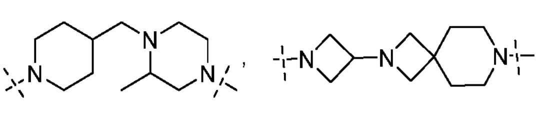 , , 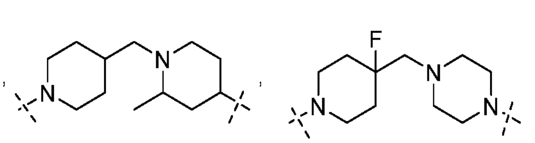 , ,

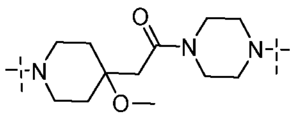 , 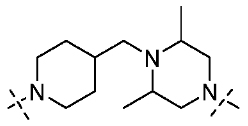 , 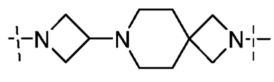 , 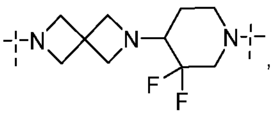 ,

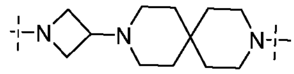 , 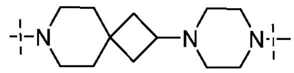 , 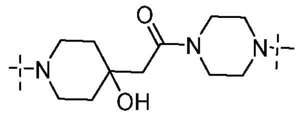 , 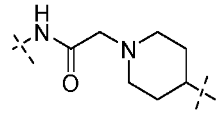 ,

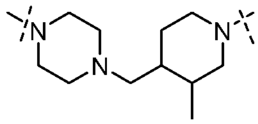 , 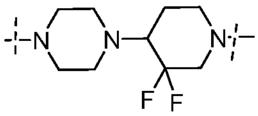 , 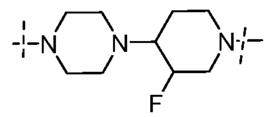 , 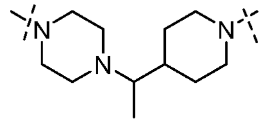 ,

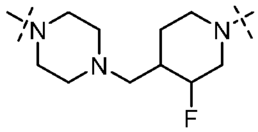 , 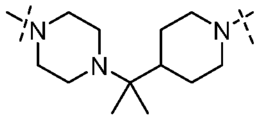 , 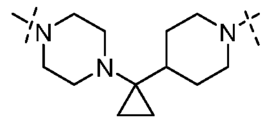 , 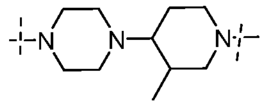 ,

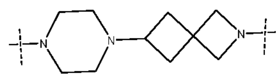 , 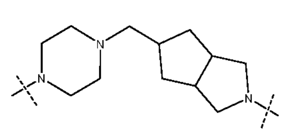

wherein

 or 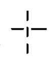

represents a linking site; and wherein the L group is optionally substituted with 1, 2, 3, or 4 substituents selected from halogen, hydroxyl, nitro, cyano, amino, mercapto, COOH, or $C_1$-$C_6$ alkyl.

**68.** The compound according to claim 67, wherein formula (XXXXI) is formula (XXXXII):

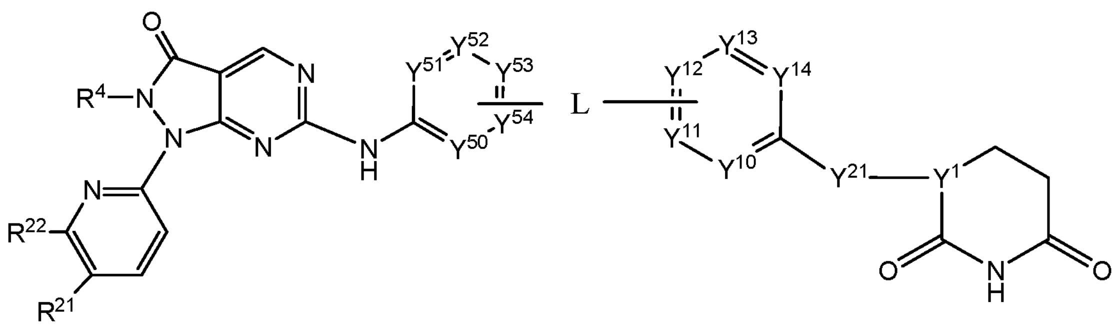

XXXXII,

wherein,

$Y^1$, for each occurrence, is independently selected from CH, CD or N;

$Y^{21}$, for each occurrence, is independently selected from NH, N-$CH_3$, N-$C_2H_5$, N-$C_3H_7$, N-$C_4H_9$; each of $Y^{10}$, $Y^{11}$, $Y^{12}$, $Y^{13}$ and $Y^{14}$, for each occurrence, is independently selected from N; $CT^{101}$, wherein $T^{101}$, for each occurrence, is independently selected from absence, hydrogen, fluorine, chlorine, methyl, $CH_2F$, $CHF_2$, $CF_3$,

$CH_2Cl$, $CHCl_2$, $CCl_3$, ethyl, propyl, isopropyl, butyl, isobutyl, tert-butyl, methoxyl, ethoxyl, propoxyl, isopropoxyl, butoxyl, isobutoxyl, tert-butoxyl, hydroxyl, cyano;

one of $Y^{10}$, $Y^{11}$, $Y^{12}$, $Y^{13}$ and $Y^{14}$ is linked to L groups;

each of $Y^{50}$, $Y^{51}$, $Y^{52}$, $Y^{53}$ and $Y^{54}$, for each occurrence, is independently selected from N; $CT^{102}$, wherein $T^{102}$, for each occurrence, is independently selected from absence, hydrogen, fluorine, chlorine, methyl, $CH_2F$, $CHF_2$, $CF_3$, $CH_2Cl$, $CHCl_2$, $CCl_3$, ethyl, propyl, isopropyl, butyl, isobutyl, tert-butyl, methoxyl, ethoxyl, propoxyl, isopropoxyl, butoxyl, isobutoxyl, tert-butoxyl, hydroxyl, cyano,;

one or more of $Y^{50}$, $Y^{51}$, $Y^{52}$, $Y^{53}$ and $Y^{54}$ are linked to L groups;

$R^4$, for each occurrence, is independently selected from allyl, propargyl, methylene cyclopropyl, ethyl, trifluoroethyl, difluoroethyl, isopropyl, cyclopropyl;

each of $R^{21}$ and $R^{22}$, for each occurrence, is independently selected from hydrogen, or 2-hydroxyisopropyl; or, $R^{21}$, $R^{22}$ are joined together to form $-(CR^{91}R^{92})-(CH_2)-(CH_2)-$ or $-(CH_2)-(CH_2)-(CR^{95}R^{96})-$, wherein each of $R^{91}$, $R^{92}$, $R^{95}$, and $R^{96}$, for each occurrence, is independently selected from hydrogen, hydroxyl, methyl, ethyl, propyl;

L is a linker and has the following structure:

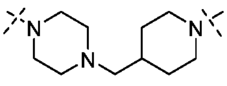, 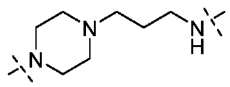, 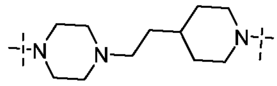, 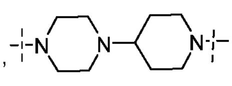,

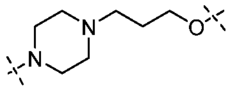, 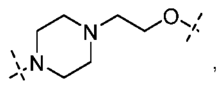, 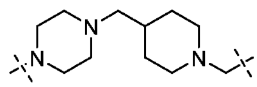, 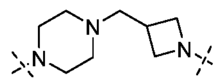,

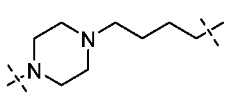, 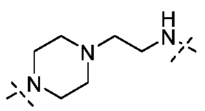, 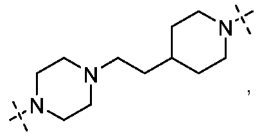, 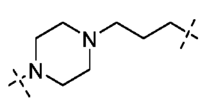,

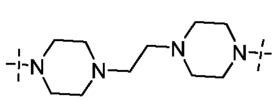, 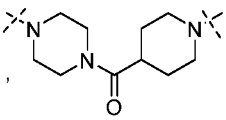, 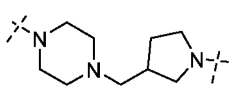, 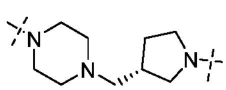,

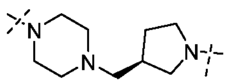, 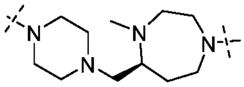, 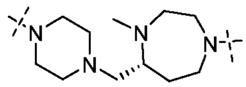, 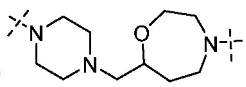,

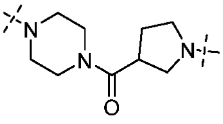, 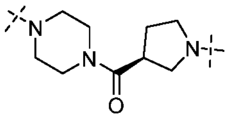, 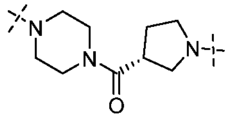, 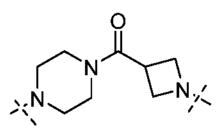,

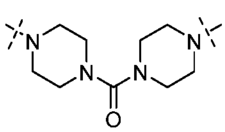, 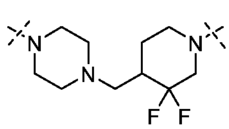, 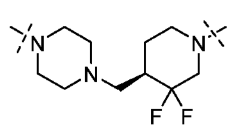, 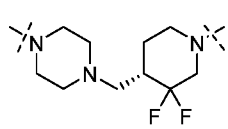,

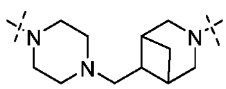, 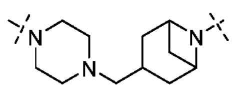, 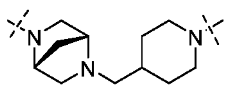, 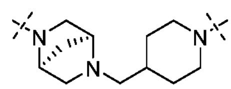,

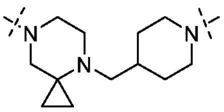, 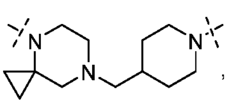, 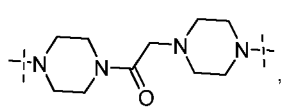, 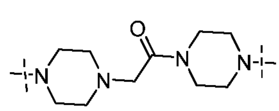,

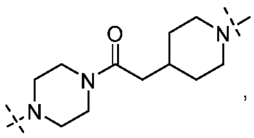, 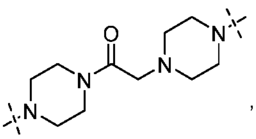, 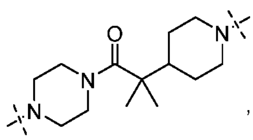, 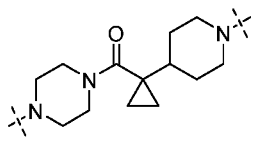,

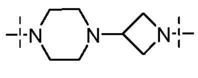, 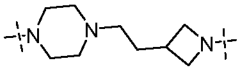, 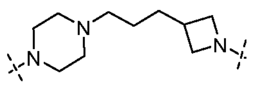, 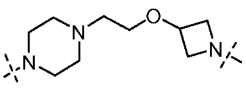,

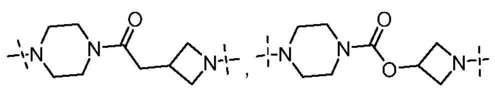, 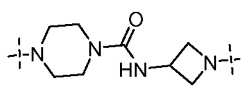, 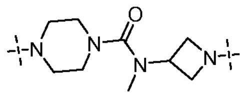,

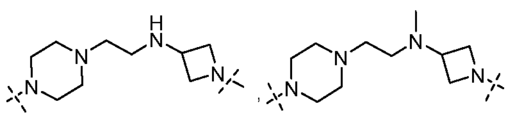, 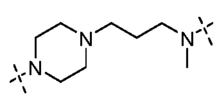, 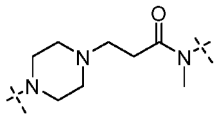,

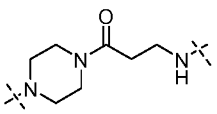, 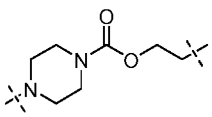, 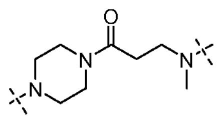, 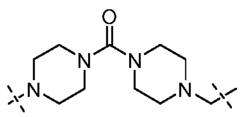,

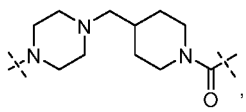, 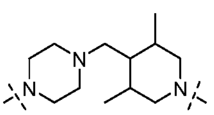, 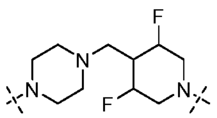, 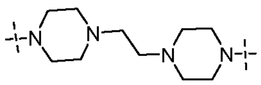,

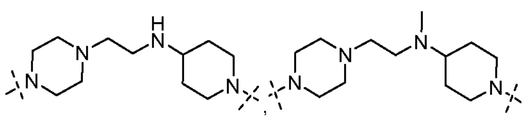, 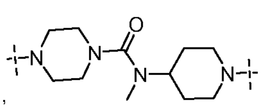, 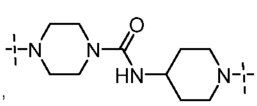,

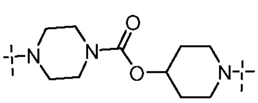, 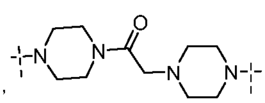, 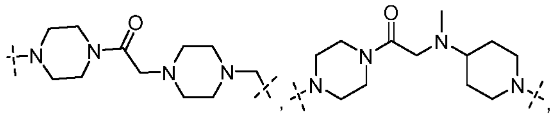,

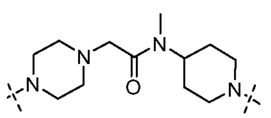, 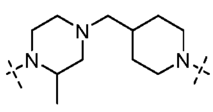, 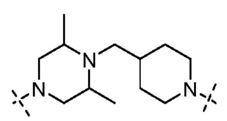, 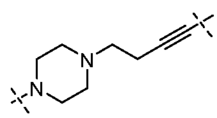,

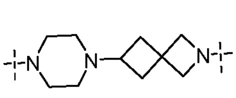, 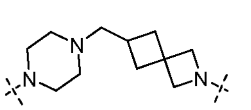, 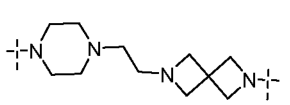, 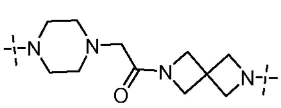,

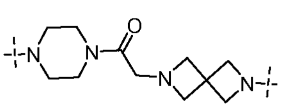, 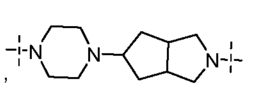, 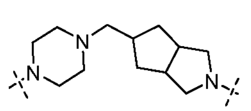, 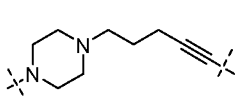,

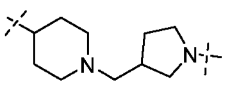, 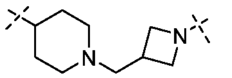, 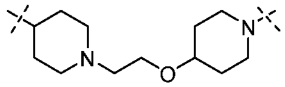, 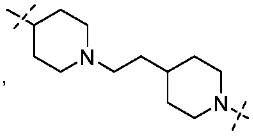

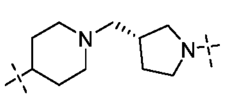, 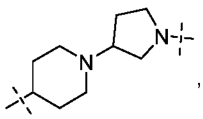, 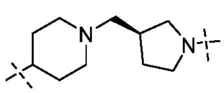, 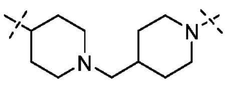,

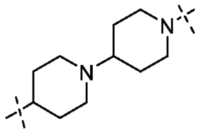, 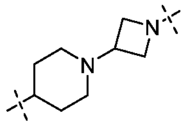, 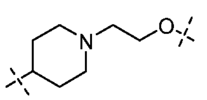, 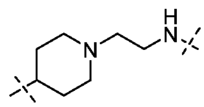,

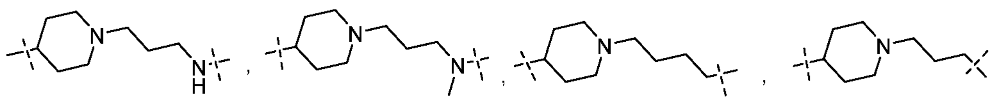 , , , ,

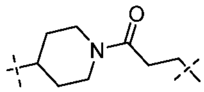, 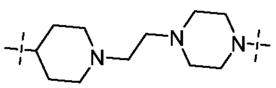, 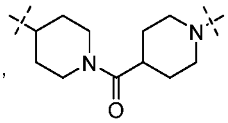, 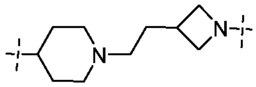,

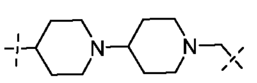, 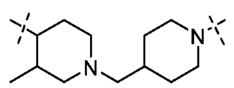, 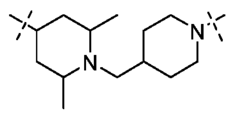, 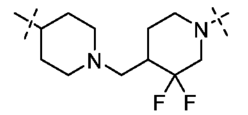,

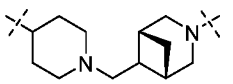, 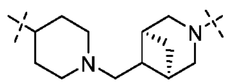, 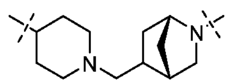, 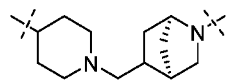,

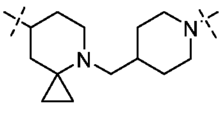, 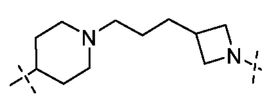, 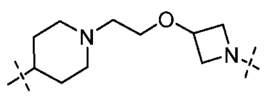, 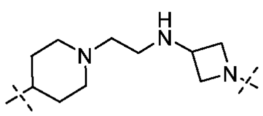,

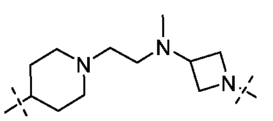, 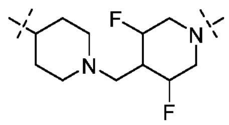, 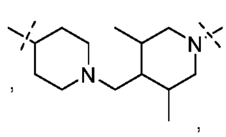, 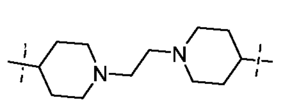,

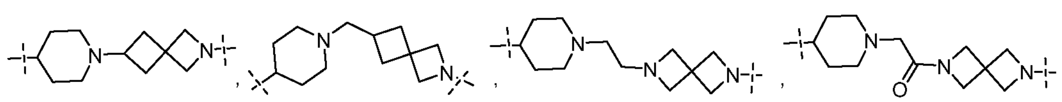 , , , ,

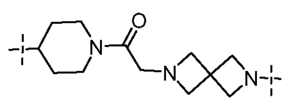, 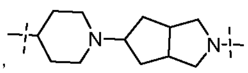, 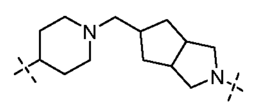,

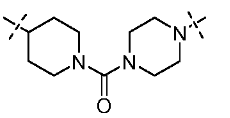, 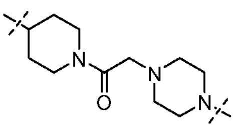, 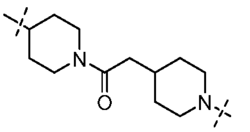, 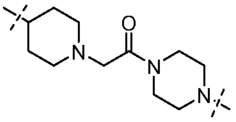,

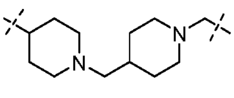, 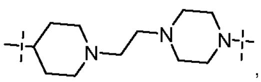, 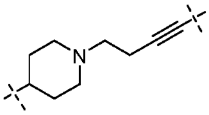, 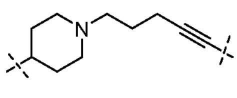,

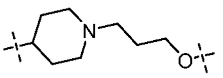, 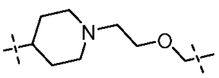, 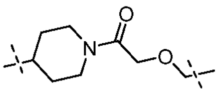, 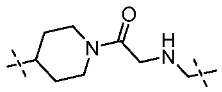,

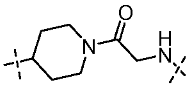, 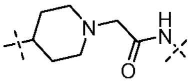, 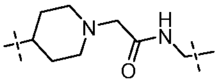, 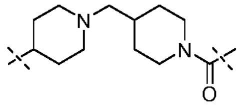,

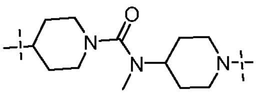, 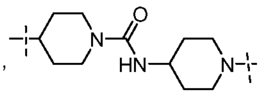, 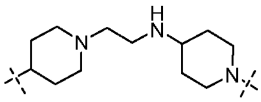, 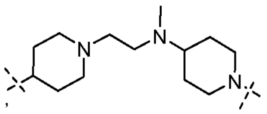,

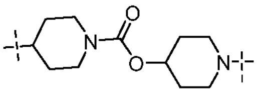, 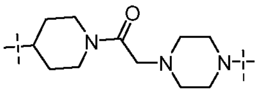, 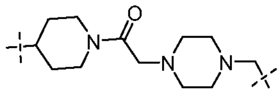, 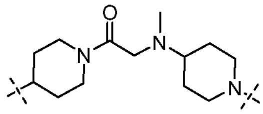,

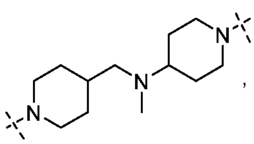, 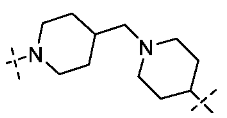, 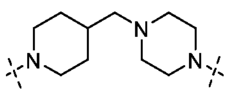, 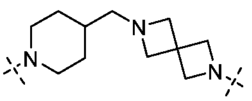,

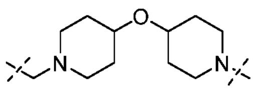, 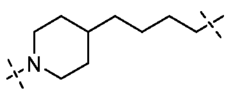, 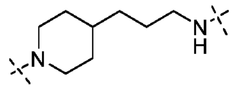, 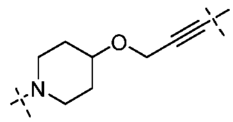,

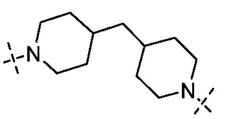, 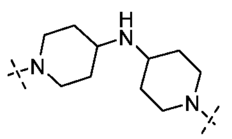, 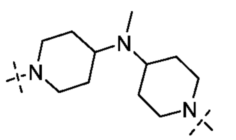, 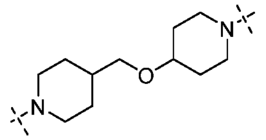,

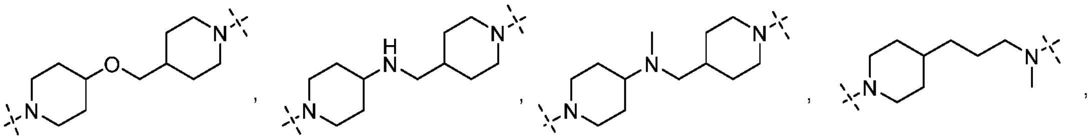

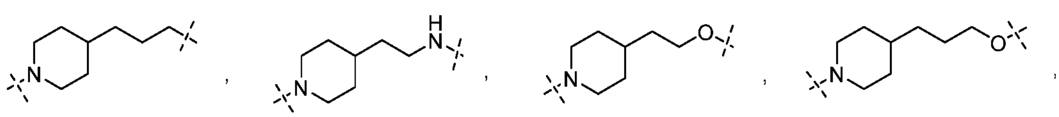

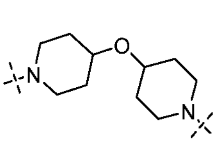 , 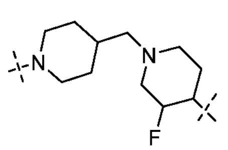 , 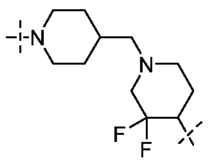 , 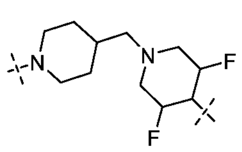

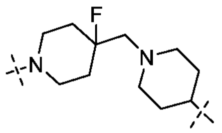 , 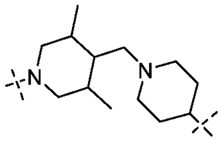 , 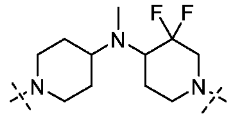 , 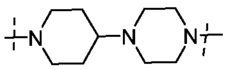 ,

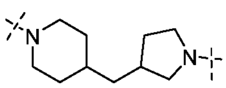 , 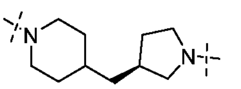 , 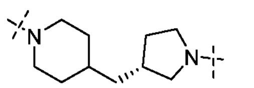 , 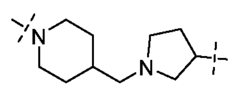

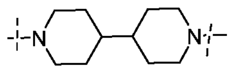 , 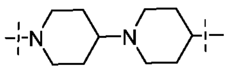 , 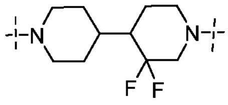 , 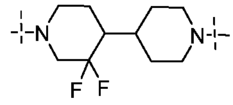

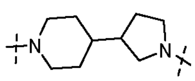 , 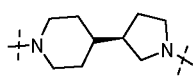 , 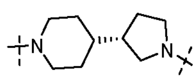 , 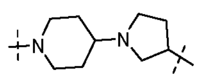 ,

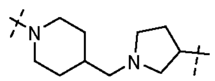 , 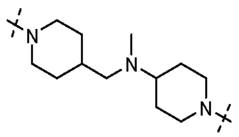 , 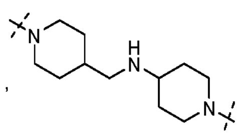 ,

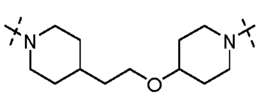 , 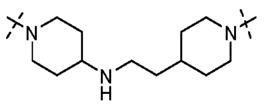 , 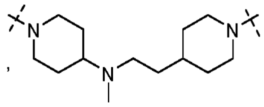 , 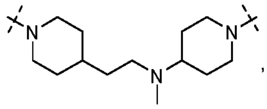 ,

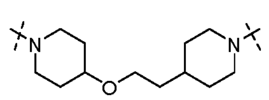 , 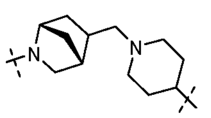 , 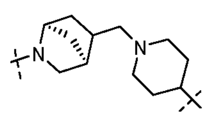 , 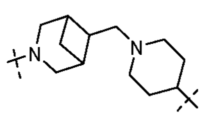 ,

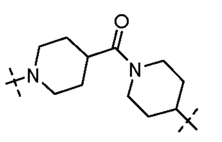 , 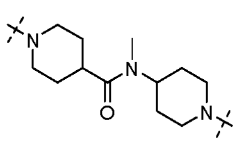 , 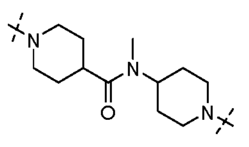 ,

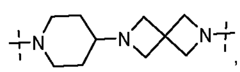 , 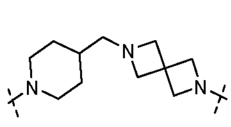 , 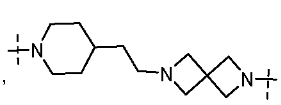 , 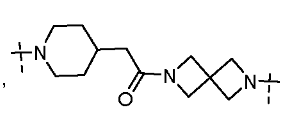 ,

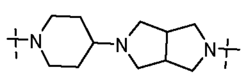 , 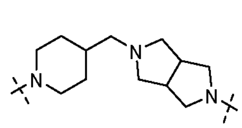 ,

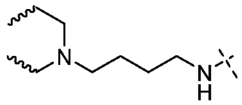 , 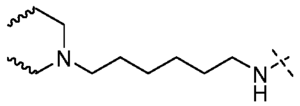 , 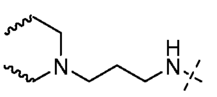 , 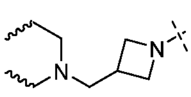 ,

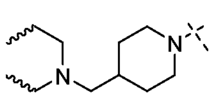 , 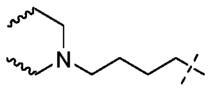 , 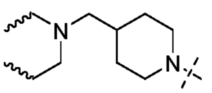 , 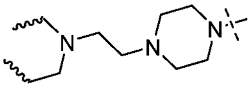 ,

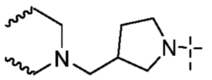 , 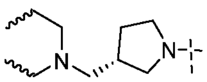 , 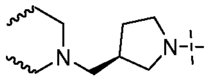 , 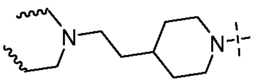 ,

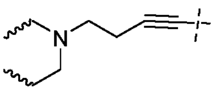 , 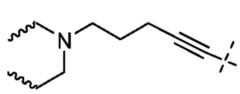 , 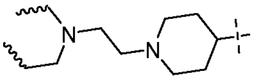 , 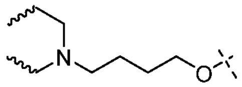 ,

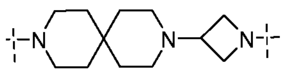 , 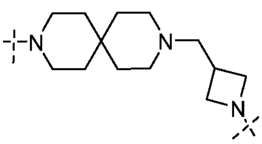 , 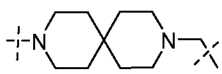 , 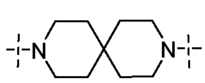 ,

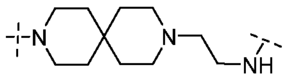 , 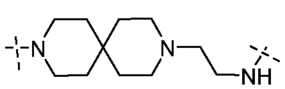 , 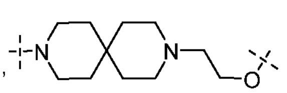 ,

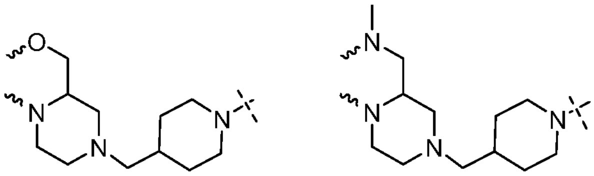 ,

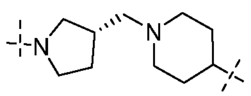 , 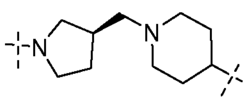 , 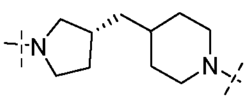 , 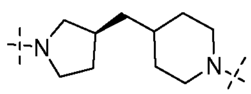 ,

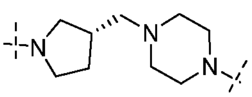 , 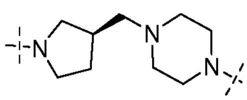 , 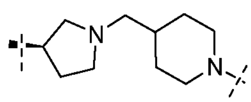 , 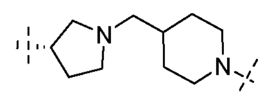 ,

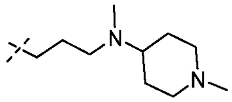 , 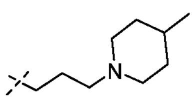 , 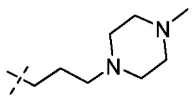 , 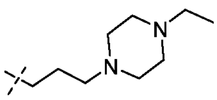 , 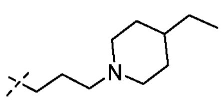 ,

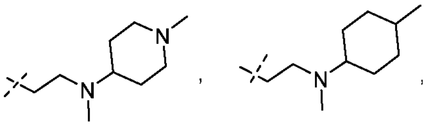 , 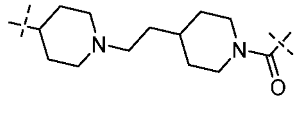 , 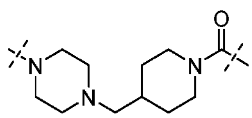 ,

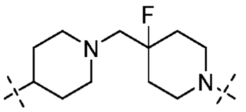, 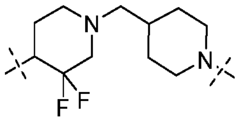, 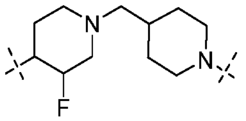, 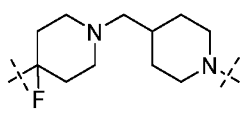

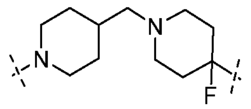, 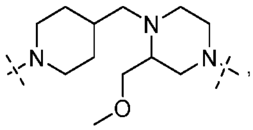, 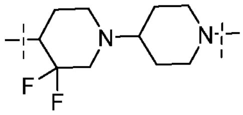, 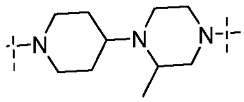,

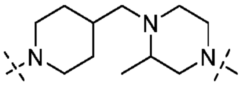, 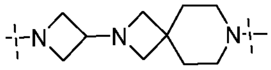, 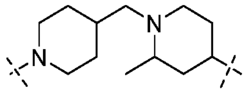, 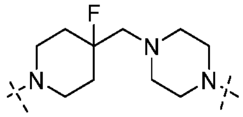,

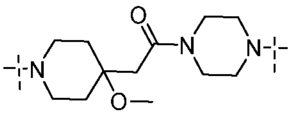, 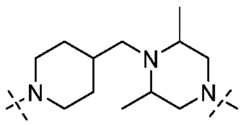, 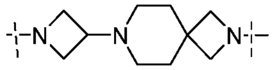, 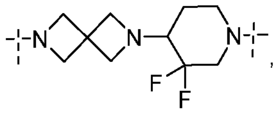,

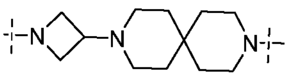, 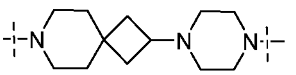, 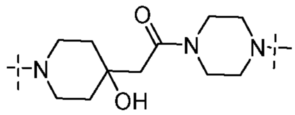, 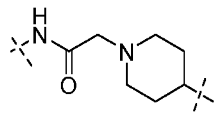

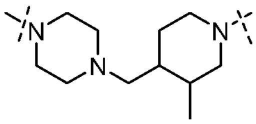, 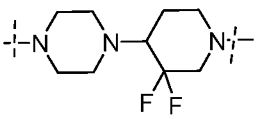, 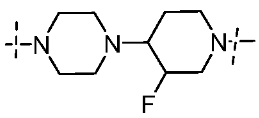, 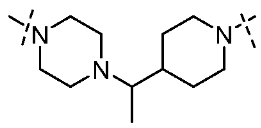,

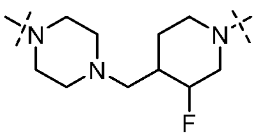, 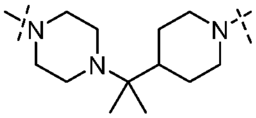, 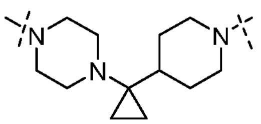, 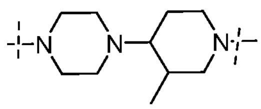,

wherein

 or 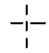

represents a linking site; and wherein the L group is optionally substituted with 1, 2, 3, or 4 substituents independently selected from halogen, hydroxyl, nitro, cyano, amino, mercapto, or $C_1$-$C_6$ alkyl.

**69.** The compound according to claim 68, wherein formula (XXXXII) is formula (XXXXIII):

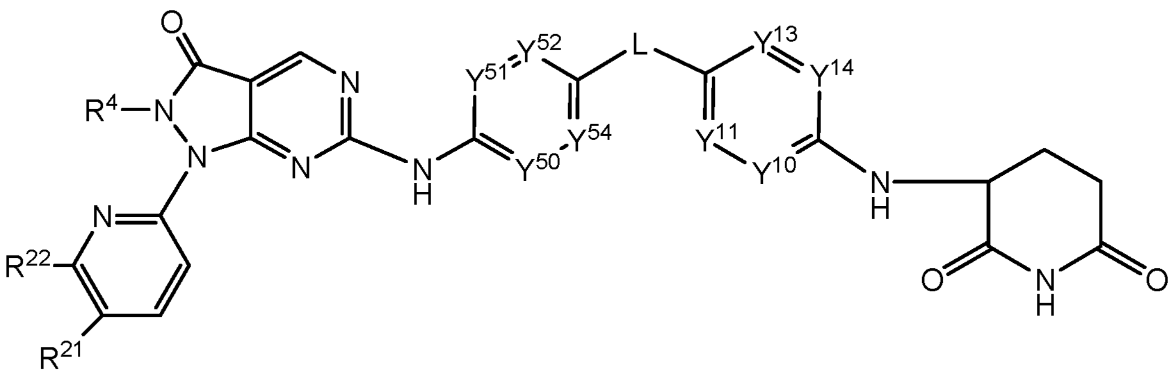

XXXXIII.

**70.** The compound according to claim 67, wherein formula (XXXXI) is formula (XXXXIV):

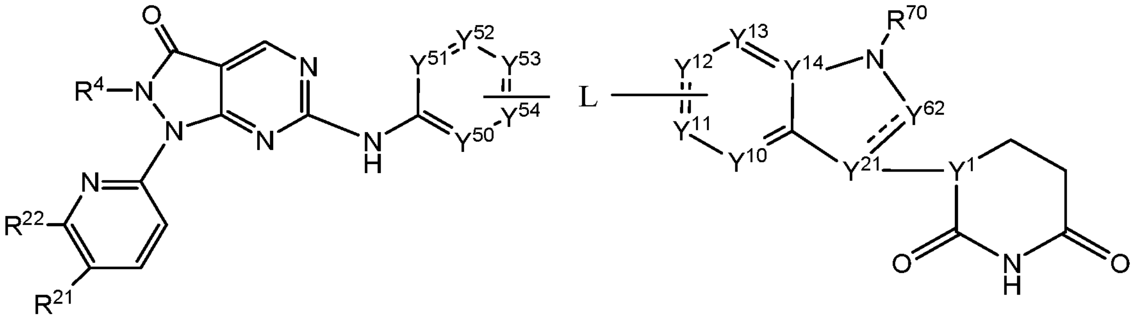

XXXXIV,

wherein,

$Y^1$, for each occurrence, is independently selected from CH, CD or N;
$Y^{21}$, for each occurrence, is independently selected from C, CH, CD, N;
each of $Y^{10}$, $Y^{11}$, $Y^{12}$, $Y^{13}$ and $Y^{14}$, for each occurrence, is independently selected from N; $CT^{101}$, wherein $T^{101}$, for each occurrence, is independently selected from absence, hydrogen, fluorine, chlorine, methyl, $CH_2F$, $CHF_2$, $CF_3$, $CH_2Cl$, $CHCl_2$, $CCl_3$, ethyl, propyl, isopropyl, butyl, isobutyl, tert-butyl, methoxyl, ethoxyl, propoxyl, isopropoxyl, butoxyl, isobutoxyl, tert-butoxyl, hydroxyl, cyano;
one of $Y^{10}$, $Y^{11}$, $Y^{12}$, $Y^{13}$ and $Y^{14}$ is linked to L groups;
each of $Y^{50}$, $Y^{51}$, $Y^{52}$, $Y^{53}$ and $Y^{54}$, for each occurrence, is independently selected from N; $CT^{102}$, wherein $T^{102}$, for each occurrence, is independently selected from absence, hydrogen, fluorine, chlorine, methyl, $CH_2F$, $CHF_2$, $CF_3$, $CH_2Cl$, $CHCl_2$, $CCl_3$, ethyl, propyl, isopropyl, butyl, isobutyl, tert-butyl, methoxyl, ethoxyl, propoxyl, isopropoxyl, butoxyl, isobutoxyl, tert-butoxyl, hydroxyl, cyano;
one or more of $Y^{50}$, $Y^{51}$, $Y^{52}$, $Y^{53}$ and $Y^{54}$ are linked to L groups;
$Y^{62}$, for each occurrence, is independently selected from N, NH, -(C=O)- or $-CH_2-$;
$R^{70}$, for each occurrence, is independently selected from hydrogen, methyl, ethyl or propyl;
- - - - - - represents a single bond or a double bond;
$R^4$, for each occurrence, is independently selected from allyl, propargyl, methylene cyclopropyl, ethyl, trifluoroethyl, difluoroethyl, isopropyl, cyclopropyl;
each of $R^{21}$ and $R^{22}$, for each occurrence, is independently selected from hydrogen, or 2-hydroxyisopropyl; or, $R^{21}$, $R^{22}$ are joined together to form $-(CR^{91}R^{92})-(CH_2)-(CH_2)-$ or $-(CH_2)-(CH_2)-(CR^{95}R^{96})-$, wherein each of $R^{91}$, $R^{92}$, $R^{95}$, and $R^{96}$, for each occurrence, is independently selected from hydrogen, hydroxyl, methyl, ethyl, propyl;
L is a linker and has the following structure:

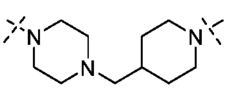 , 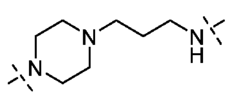 , 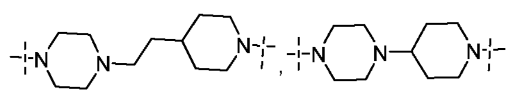 ,

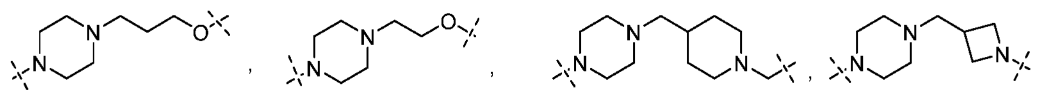
, , , ,

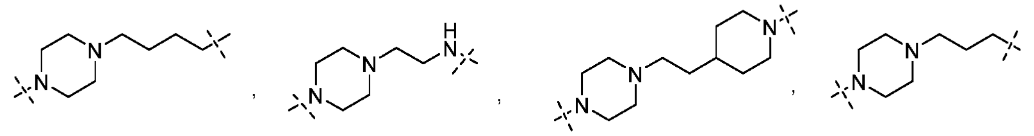
, , , ,

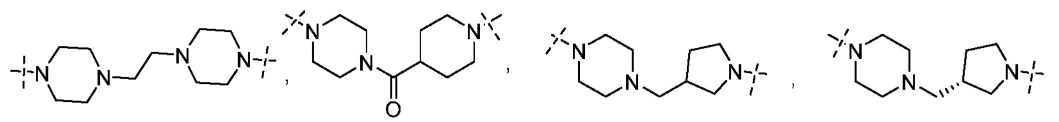
, , , ,

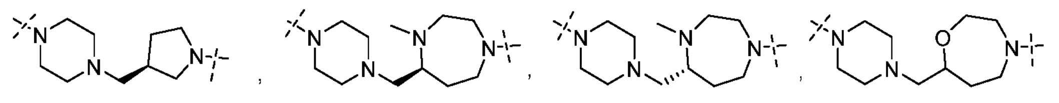
, , , ,

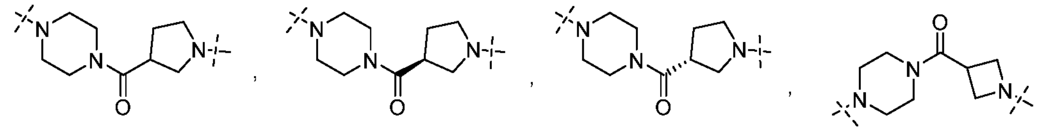
, , , ,

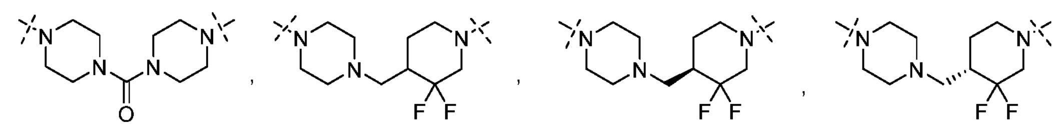
, , , ,

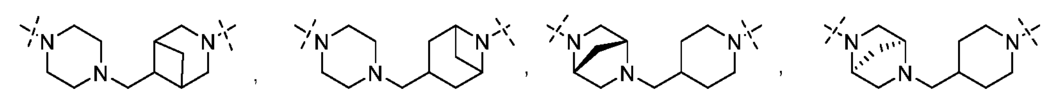
, , , ,

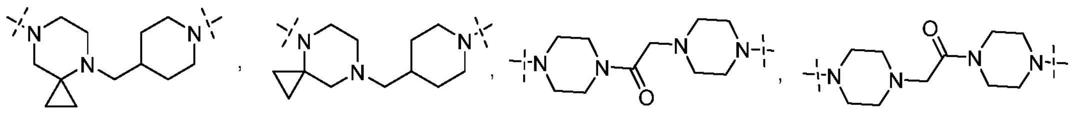
, , , ,

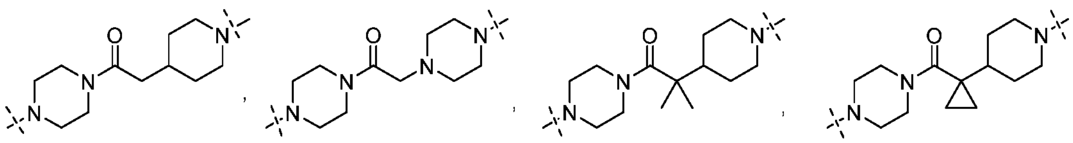
, , , ,

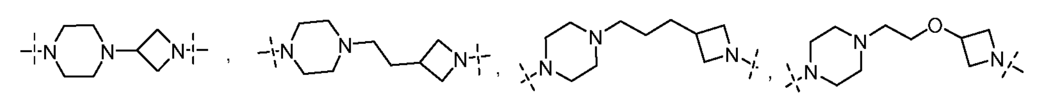
, , , ,

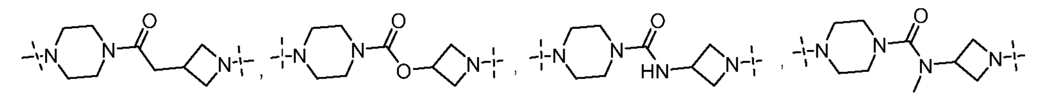
, , , ,

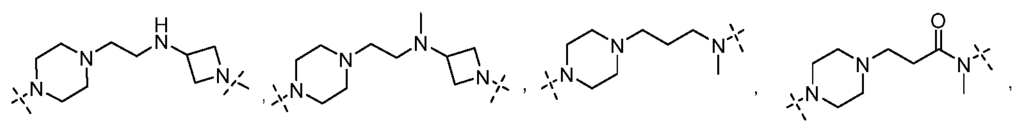
, , , ,

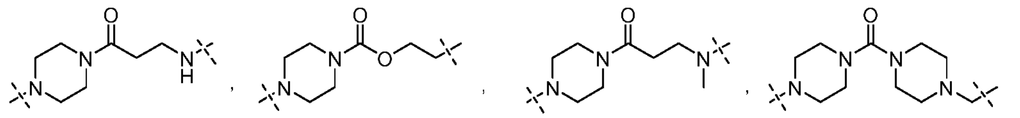

, , , ,

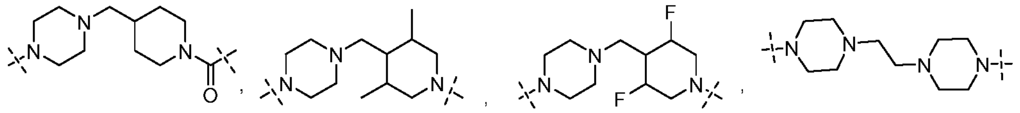

, , , ,

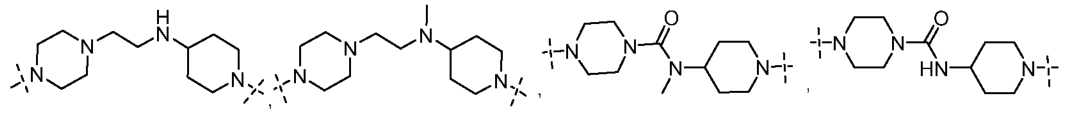

, , , ,

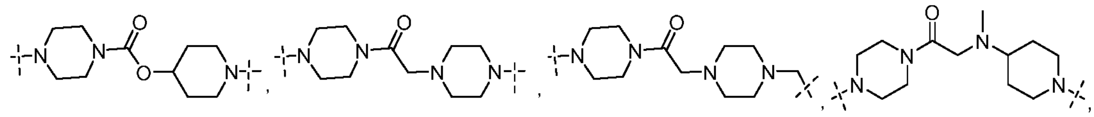

, , , ,

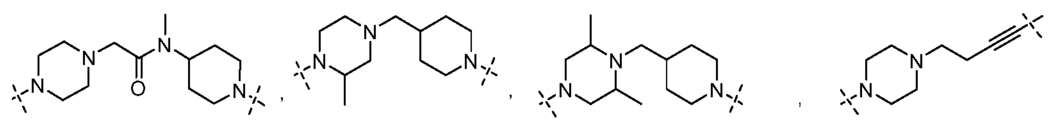

, , , ,

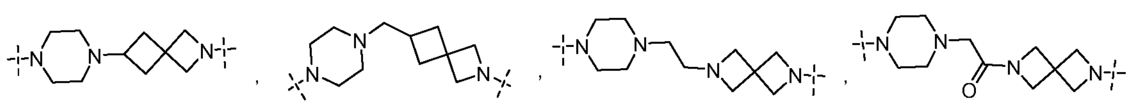

, , , ,

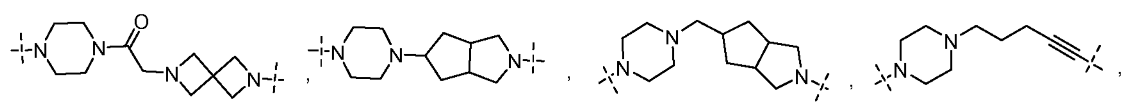

, , , ,

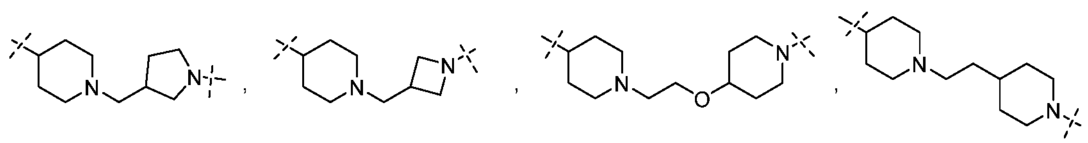

, , , ,

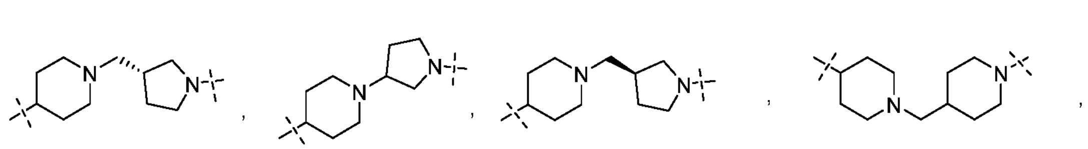

, , , ,

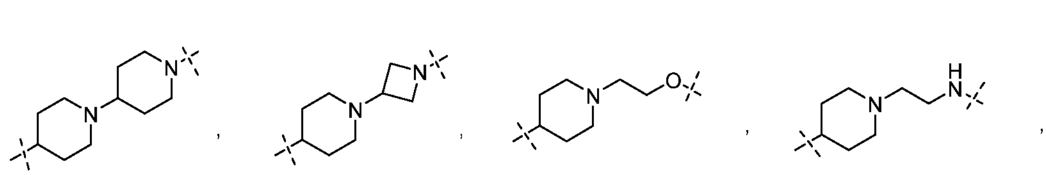

, , , ,

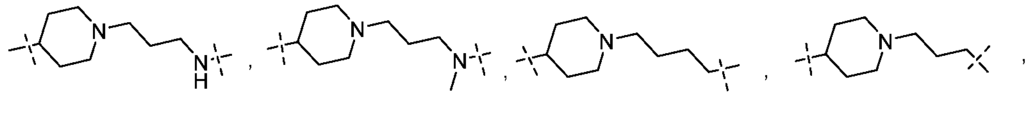

, , , ,

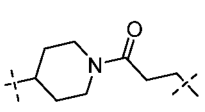, 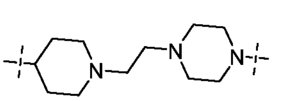, 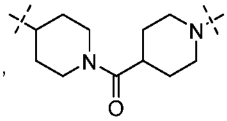, 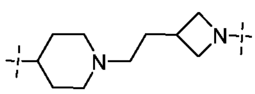,

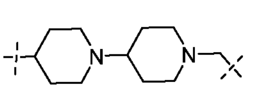, 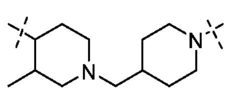, 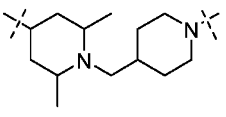, 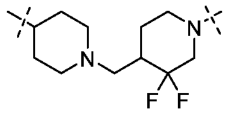,

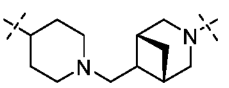, 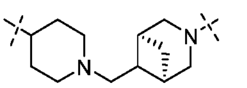, 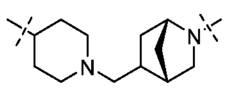, 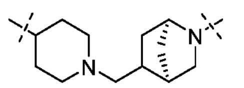,

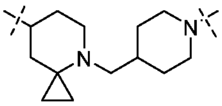, 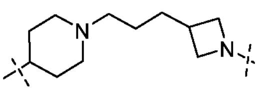, 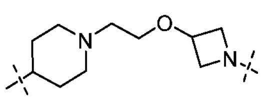, 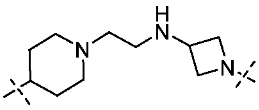,

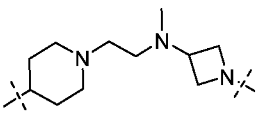, 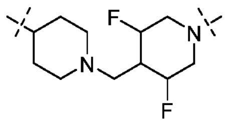, 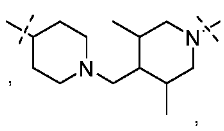, 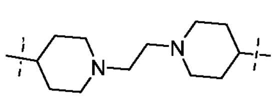,

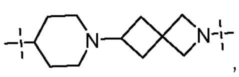, 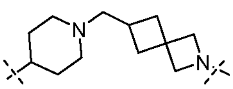, 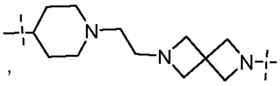, 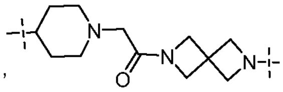,

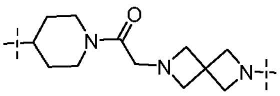, 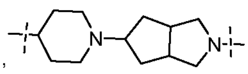, 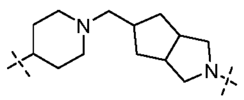,

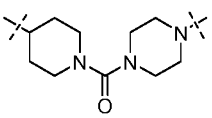, 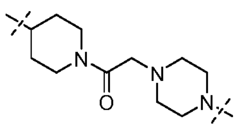, 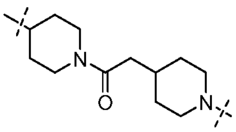, 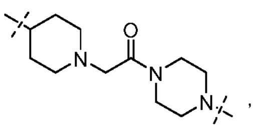,

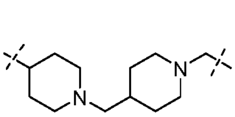, 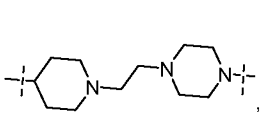, 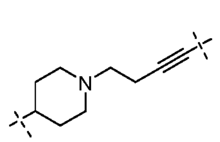, 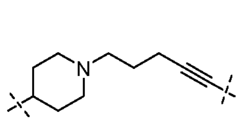,

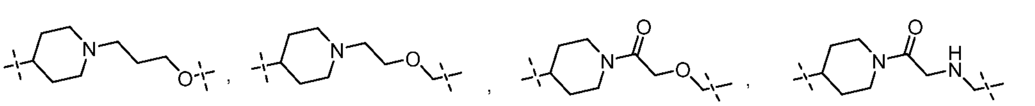,

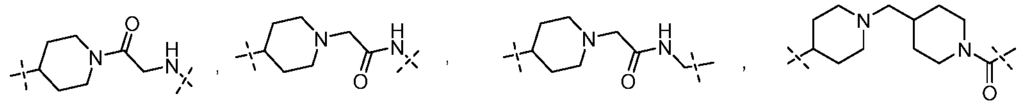,

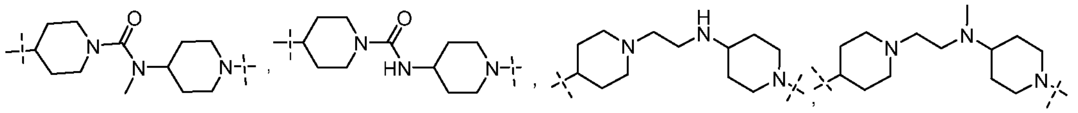,

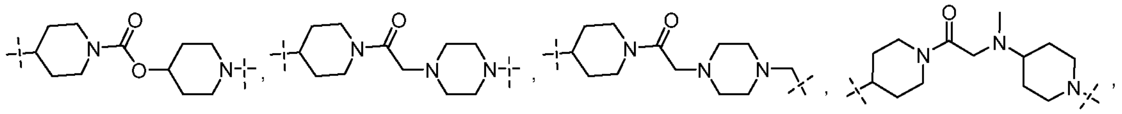
, , , ,

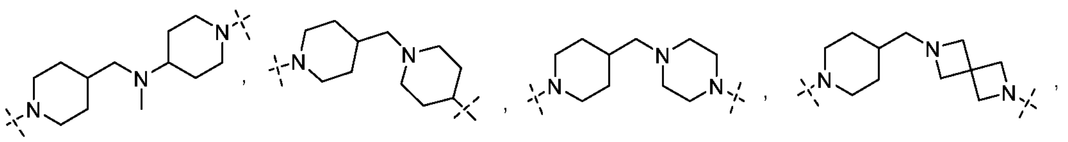
, , , ,

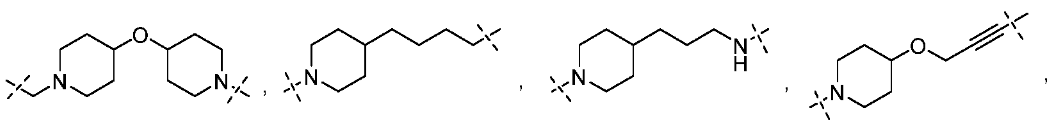
, , , ,

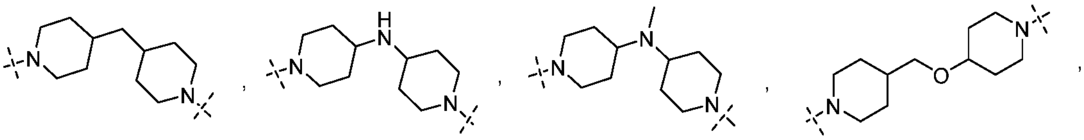
, , , ,

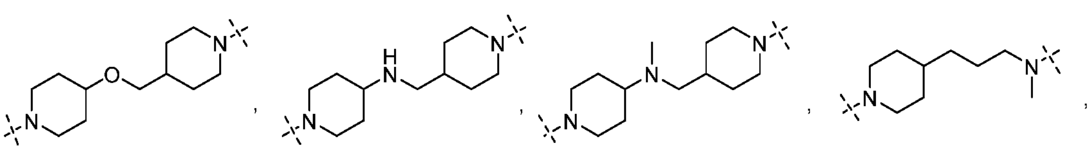
, , , ,

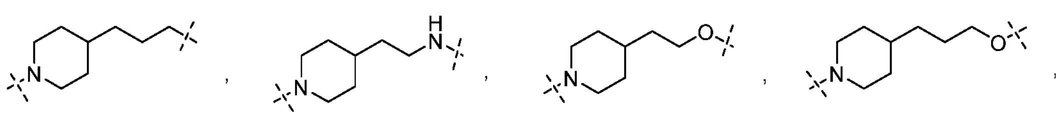
, , , ,

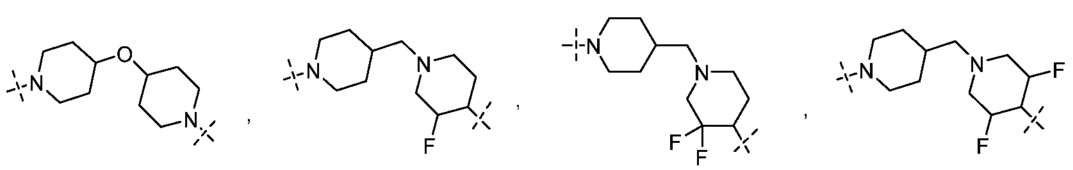
, , ,

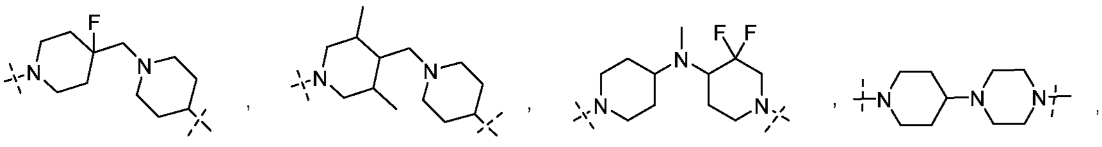
, , , ,

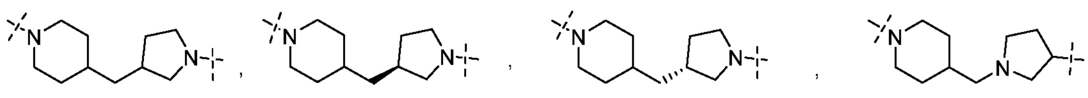
, , ,

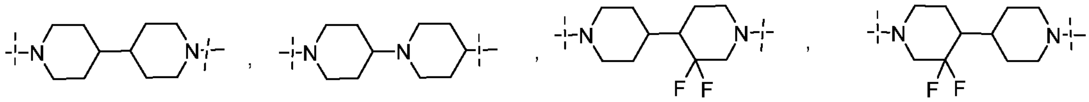
, , ,

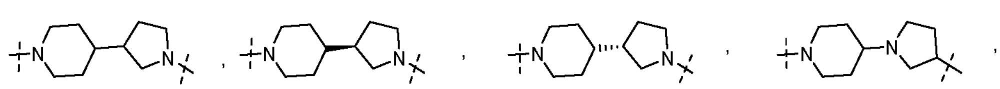
, , , ,

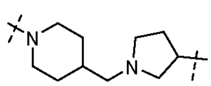 , 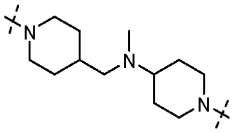 , 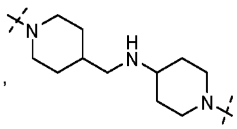 ,

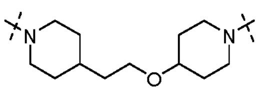 , 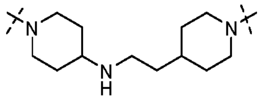 , 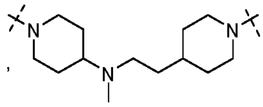 , 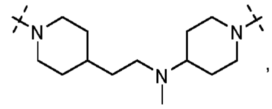 ,

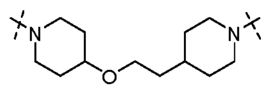 , 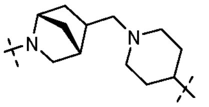 , 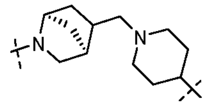 , 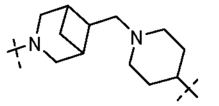 ,

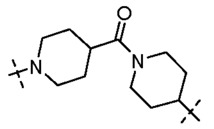 , 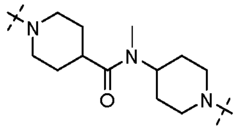 , 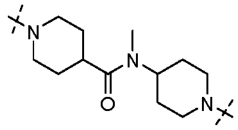 ,

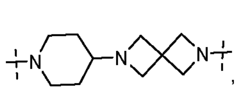 , 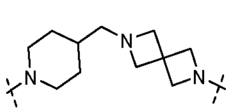 , 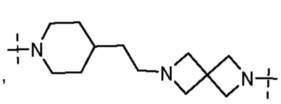 , 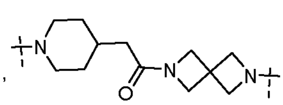 ,

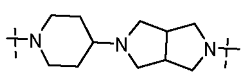 , 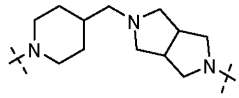 ,

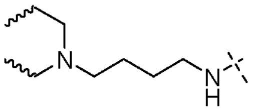 , 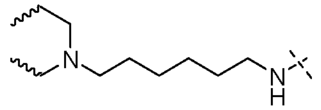 , 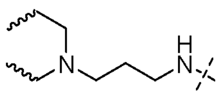 , 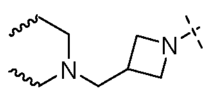 ,

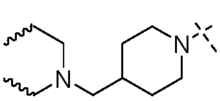 , 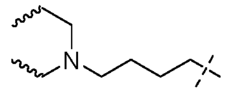 , 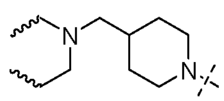 , 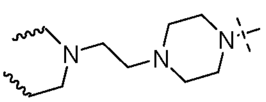 ,

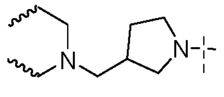 , 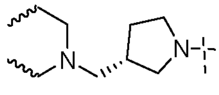 , 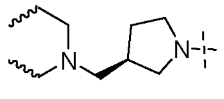 , 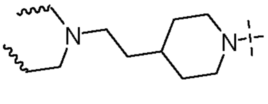 ,

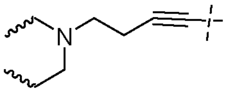 , 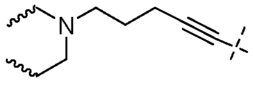 , 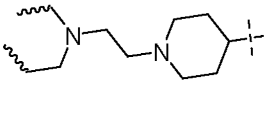 , 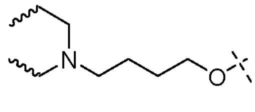 ,

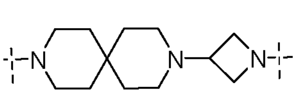 , 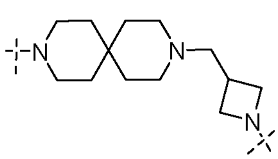 , 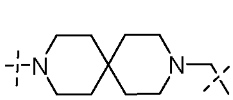 , 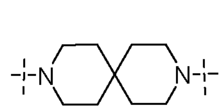 ,

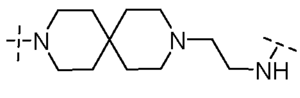 , 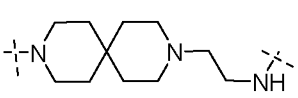 , 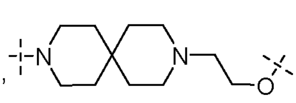 ,

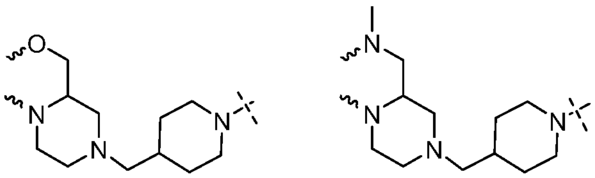 ,

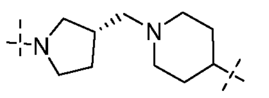 , 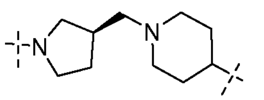 , 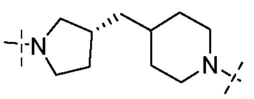 , 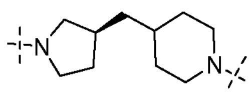 ,

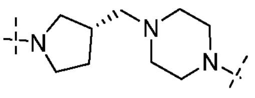 , 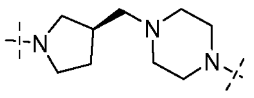 , 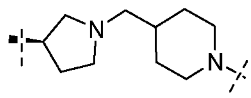 , 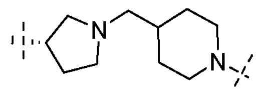 ,

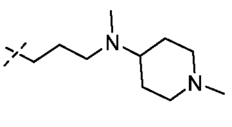 , 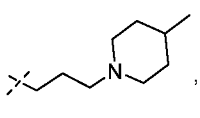 , 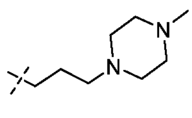 , 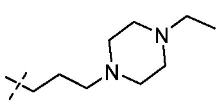 , 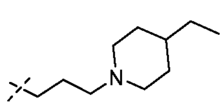 ,

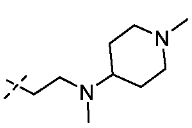 , 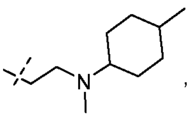 , 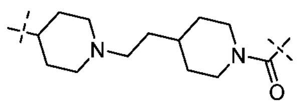 , 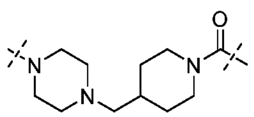 ,

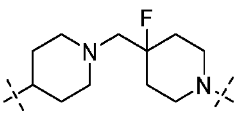 , 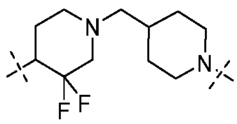 , 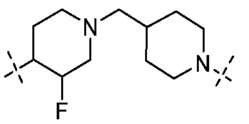 , 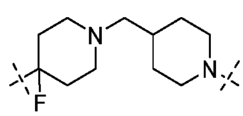

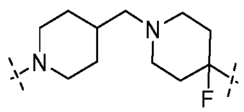 , 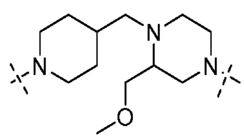 , 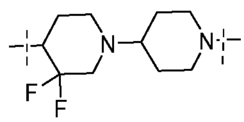 , 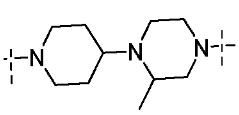 ,

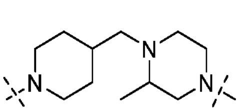 , 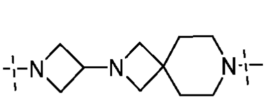 , 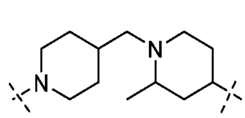 , 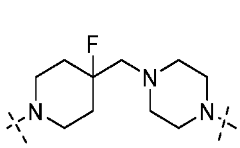 ,

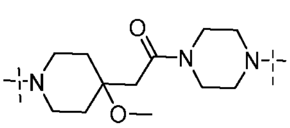 , 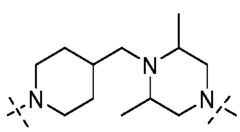 , 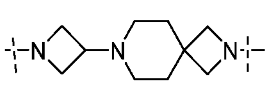 , 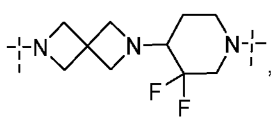 ,

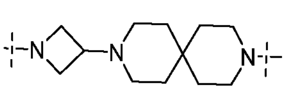 , 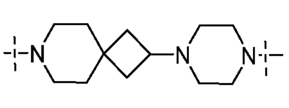 , 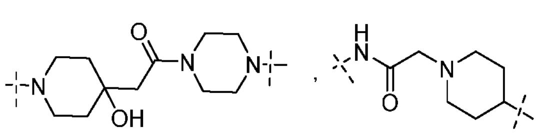

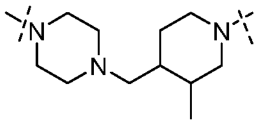 , 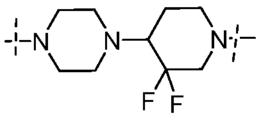 , 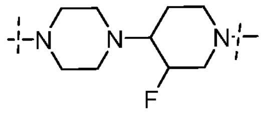 , 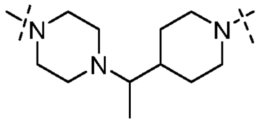 ,

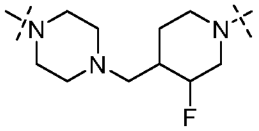 , 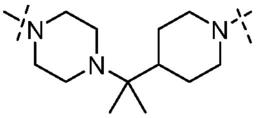 , 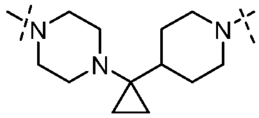 , 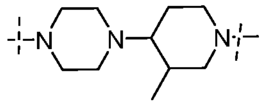 ,

wherein

 or 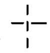

represents a linking site; and wherein the L group is optionally substituted with 1, 2, 3, or 4 substituents independently selected from halogen, hydroxyl, nitro, cyano, amino, mercapto, COOH, or $C_1$-$C_6$ alkyl.

**71.** The compound according to claim 70, wherein formula (XXXXIV) is formula (XXXXV) or formula (XXXXVI):

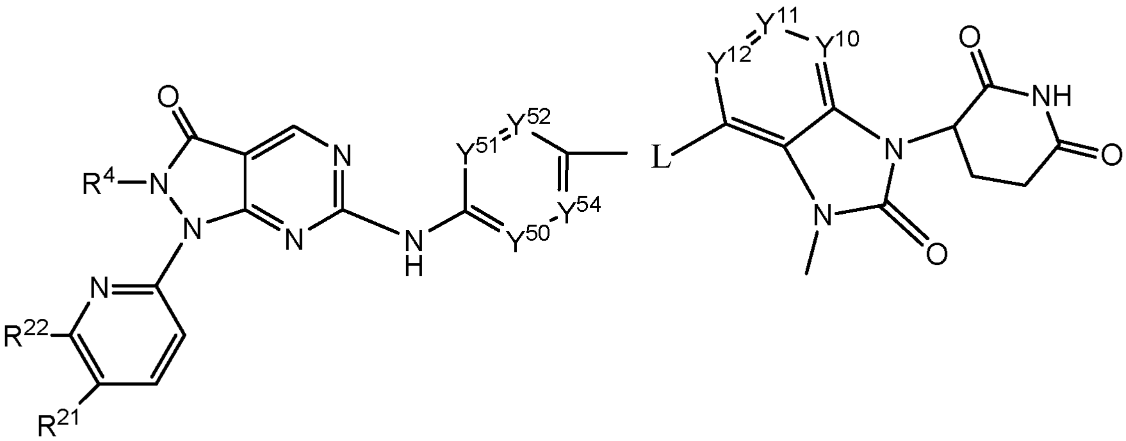

XXXXV;

or

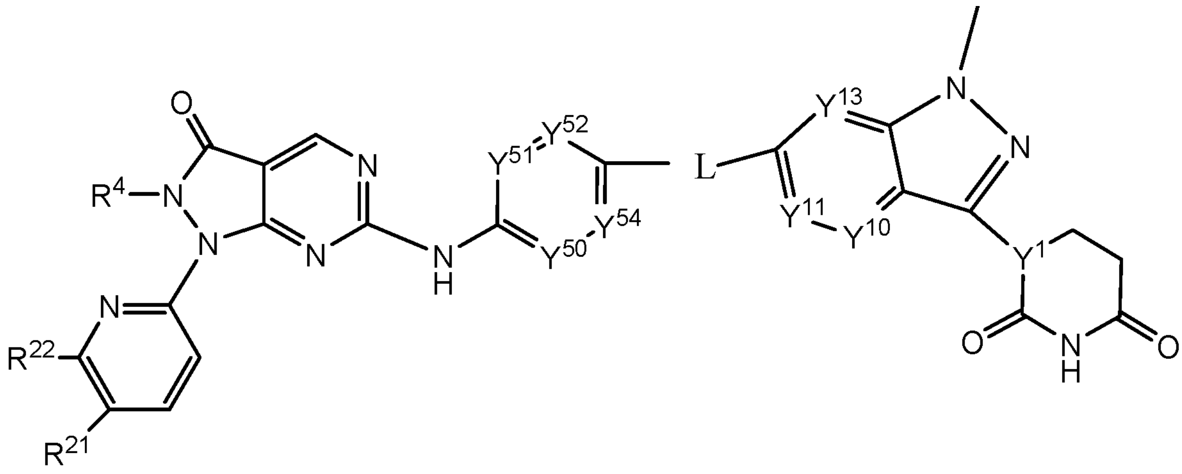

XXXXVI.

**72.** A compound represented by structural formula (XXXXVII), or a derivative, pharmaceutically acceptable salt, isomer, solvate, hydrate, adduct, complex or prodrug thereof:

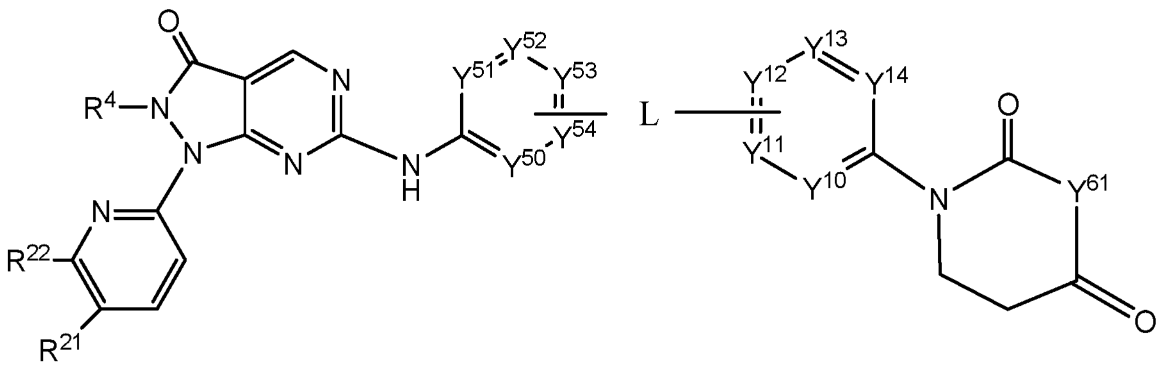

XXXXVII,

wherein,

each of $Y^{10}$, $Y^{11}$, $Y^{12}$, $Y^{13}$ and $Y^{14}$, for each occurrence, is independently selected from N; $CT^{101}$, wherein $T^{101}$, for each occurrence, is independently selected from absence, hydrogen, halogen, $C_1$-$C_6$ alkyl, $C_1$-$C_6$ haloalkyl, $C_1$-$C_6$ alkoxyl, hydroxyl, cyano;

one of $Y^{10}$, $Y^{11}$, $Y^{12}$, $Y^{13}$ and $Y^{14}$ is linked to L groups;

each of $Y^{50}$, $Y^{51}$, $Y^{52}$, $Y^{53}$ and $Y^{54}$, for each occurrence, is independently selected from N; $CT^{102}$, wherein $T^{102}$, for each occurrence, is independently selected from absence, hydrogen, halogen, $C_1$-$C_6$ alkyl, $C_1$-$C_6$ haloalkyl, $C_1$-$C_6$ alkoxyl, hydroxyl, cyano,;

one or more of $Y^{50}$, $Y^{51}$, $Y^{52}$, $Y^{53}$ and $Y^{54}$ are linked to L groups;

$R^4$, for each occurrence, is independently selected from allyl, propargyl, methylene cyclopropyl, ethyl, trifluoroethyl, difluoroethyl, isopropyl, cyclopropyl, methylene $C_6$-$C_{12}$ aryl, methylene phenyl, optionally substituted $C_{1-4}$ alkyl, optionally substituted $C_{2-4}$ alkenyl, optionally substituted $C_{2-4}$ alkynyl, optionally substituted $C_{3-6}$ cycloalkyl and optionally substituted $C_{3-6}$ cycloalkyl ($C_{1-4}$ alkyl), wherein the $C_6$-$C_{12}$ aryl, $C_{1-4}$ alkyl, $C_{2-4}$ alkenyl and $C_{2-4}$ alkynyl are optionally independently substituted with one or more substituents selected from halogen, $C_{1-4}$ alkoxyl, $C_{1-4}$ haloalkyl, $C_{1-4}$ haloalkoxyl, cyano, amino, mono-$C_{1-4}$ alkylamine and di-$C_{1-4}$ alkylamine, and wherein one or more rings of the $C_{3-6}$ cycloalkyl and the $C_{3-6}$ cycloalkyl ($C_{1-4}$ alkyl) are optionally independently substituted with one or more substituents selected from halogen, $C_{1-4}$ alkyl, $C_{1-4}$ alkoxyl, $C_{1-4}$ haloalkyl, $C_{1-4}$ haloalkoxyl, cyano, amino, mono-$C_{1-4}$ alkylamine and di-$C_{1-4}$ alkylamine;

$Y^{61}$, for each occurrence, is independently selected from $CH_2$, NH, N($C_1$-$C_6$ alkyl);
each of $R^{21}$ and $R^{22}$, for each occurrence, is independently selected from absence, hydrogen, deuterium, halogen, hydroxyl, carbonyl, nitro, cyano, amino, mercapto, -COOH, $C_1$-$C_6$ alkyl, $C_1$-$C_6$ alkenyl, $C_1$-$C_6$ alkynyl, $C_1$-$C_6$ alkoxyl, wherein the alkyl, alkenyl, alkynyl, alkoxyl are optionally substituted with 1, 2, 3 or 4 halogens, hydroxyl, carbonyl, nitro, cyano, amino, mercapto, -COOH; or
$R^{21}$, $R^{22}$ are joined together to form $-(CR^{91}R^{92})-(CR^{93}R^{94})-(CR^{95}R^{96})-$, wherein each of $R^{91}$, $R^{92}$, $R^{93}$, $R^{94}$, $R^{95}$, and $R^{96}$, for each occurrence, is independently selected from hydrogen, deuterium, halogen, hydroxyl, carbonyl, nitro, cyano, amino, mercapto, -COOH, $C_1$-$C_6$ alkyl, $C_1$-$C_6$ alkenyl, $C_1$-$C_6$ alkynyl, $C_1$-$C_6$ alkoxyl, wherein the alkyl, alkenyl, alkynyl, alkoxyl are optionally substituted with 1, 2, 3 or 4 halogens, hydroxyl, carbonyl, nitro, cyano, amino, mercapto, -COOH;
L is a linker and has the following structure:

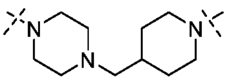, 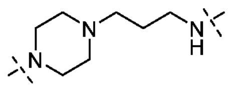, 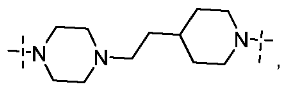, 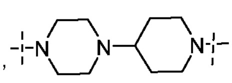,

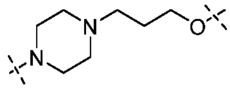, 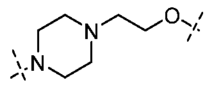, 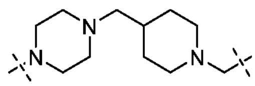, 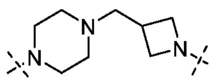,

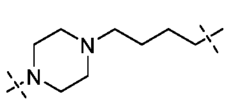, 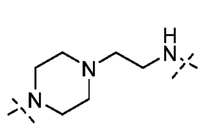, 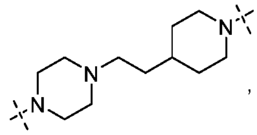, 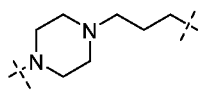,

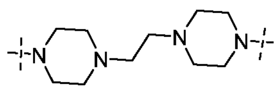, 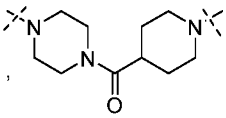, 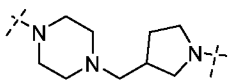, 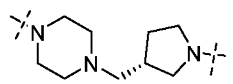,

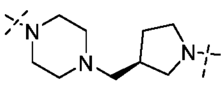, 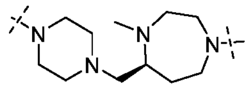, 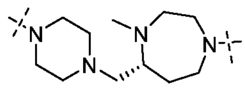, 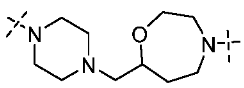,

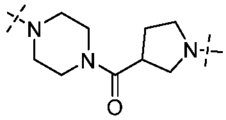, 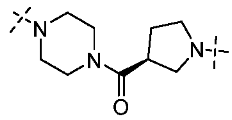, 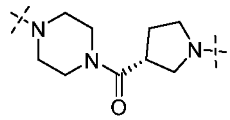, 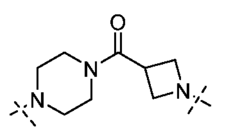,

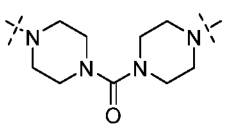, 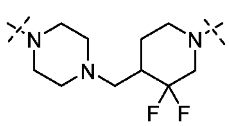, 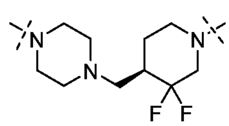, 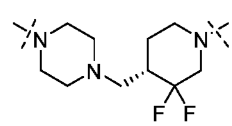,

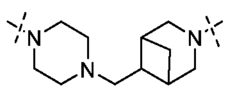, 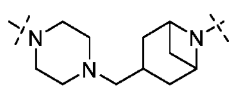, 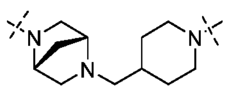, 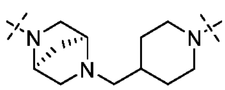,

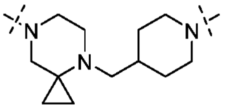, 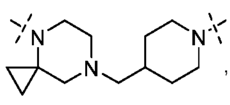, 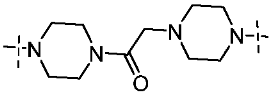, 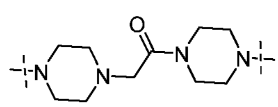,

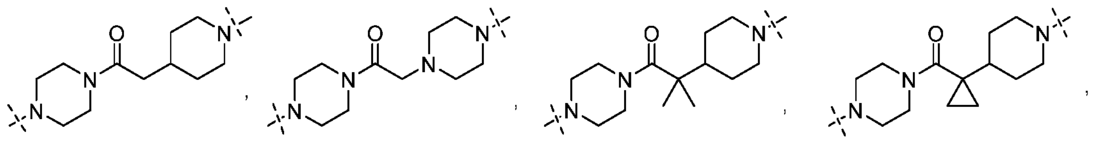
, , , ,

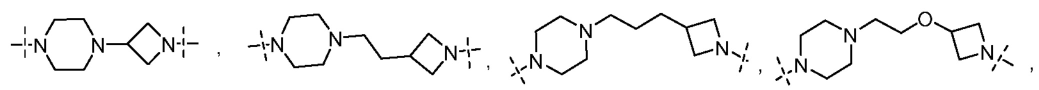
, , , ,

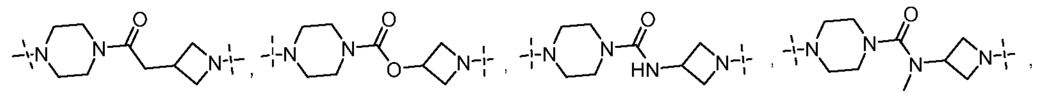
, , , ,

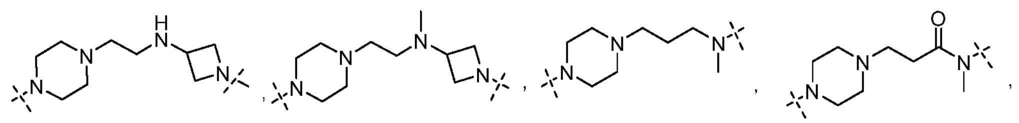
, , , ,

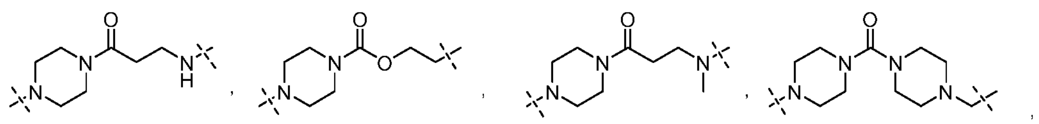
, , , ,

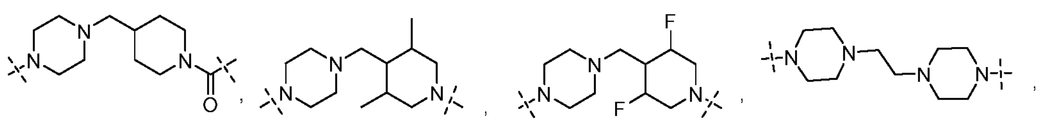
, , , ,

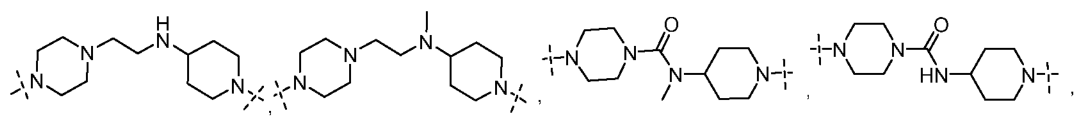
, , , ,

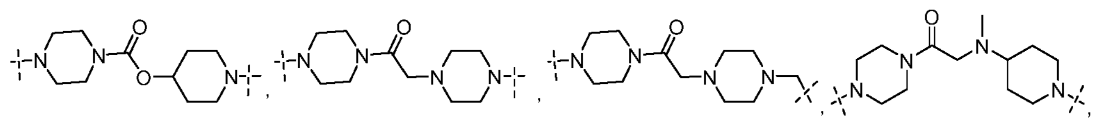
, , , ,

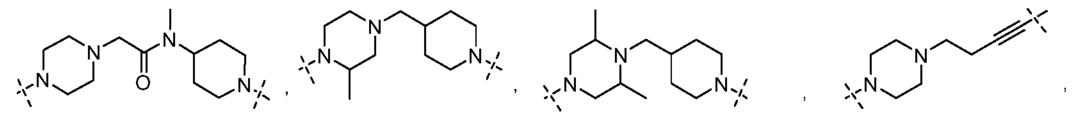
, , , ,

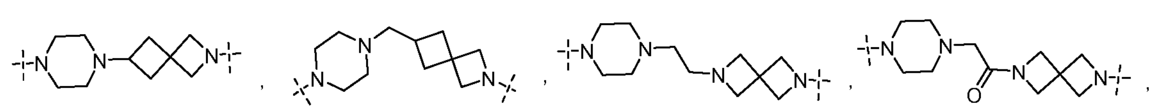
, , , ,

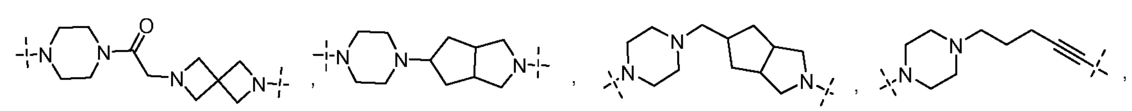
, , , ,

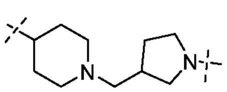 , 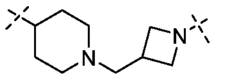 , 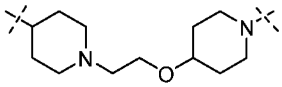 , 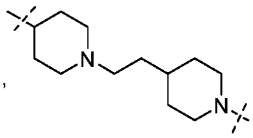

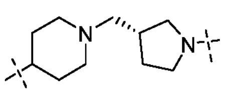 , 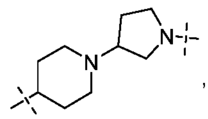 , 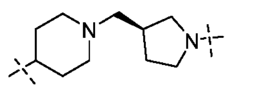 , 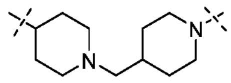 ,

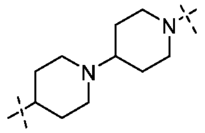 , 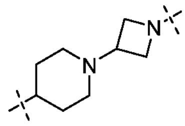 , 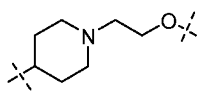 , 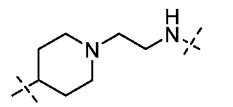 ,

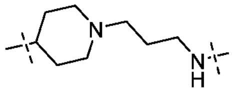 , 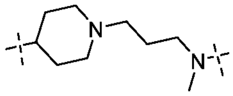 , 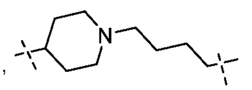 , 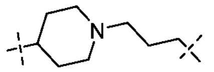 ,

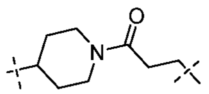 , 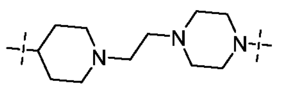 , 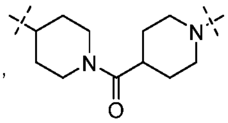 , 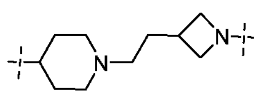 ,

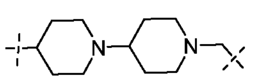 , 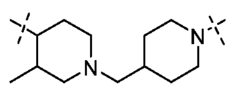 , 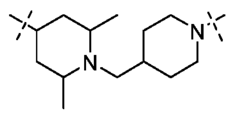 , 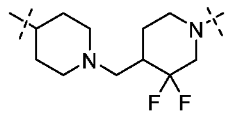 ,

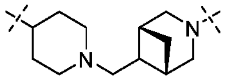 , 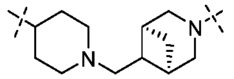 , 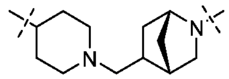 , 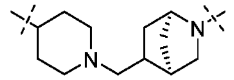 ,

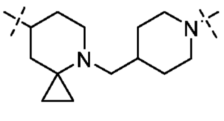 , 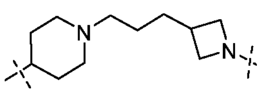 , 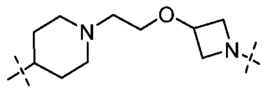 , 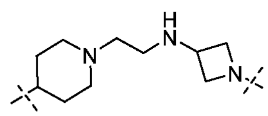

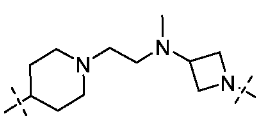 , 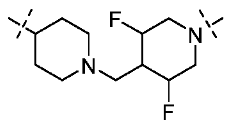 , 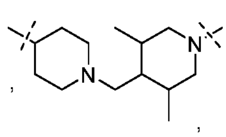 , 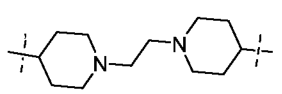 ,

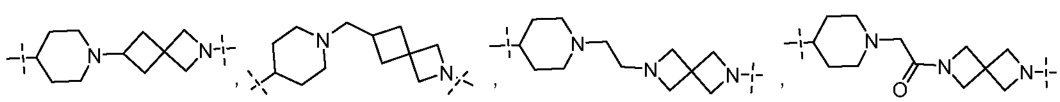 , , , ,

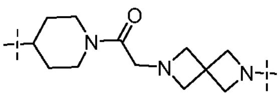 , 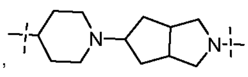 , 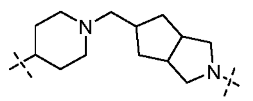 ,

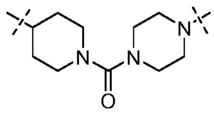 , 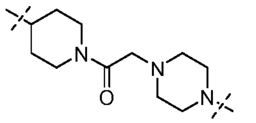 , 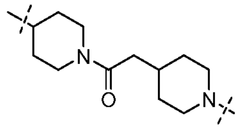 , 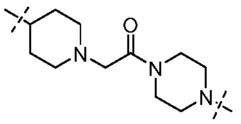 ,

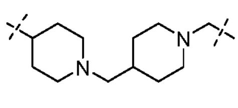 , 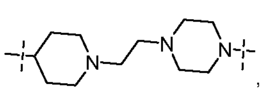 , 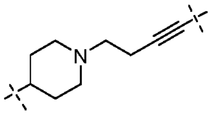 , 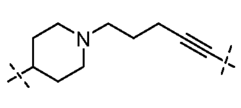 ,

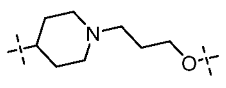 , 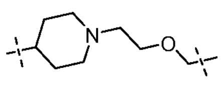 , 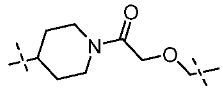 , 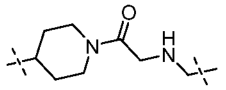 ,

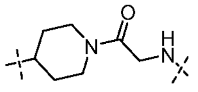 , 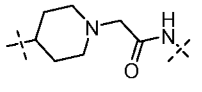 , 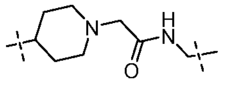 , 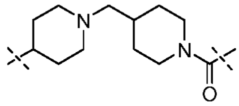 ,

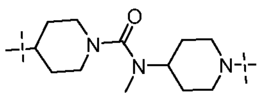 , 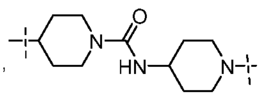 , 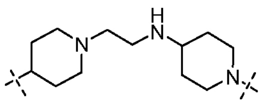 , 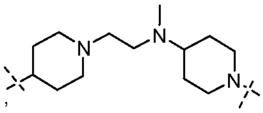 ,

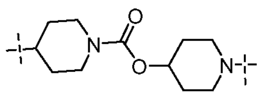 , 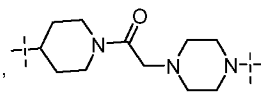 , 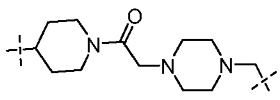 , 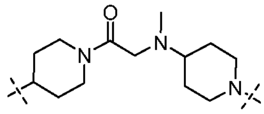 ,

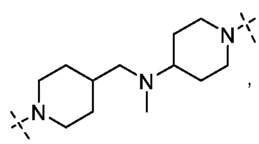 , 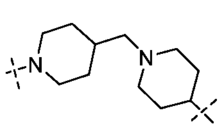 , 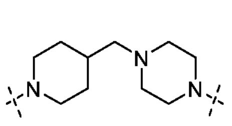 , 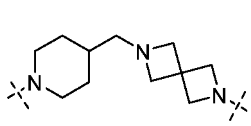 ,

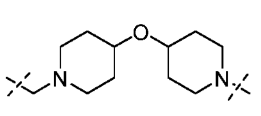 , 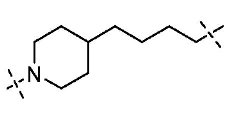 , 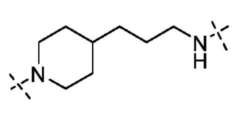 , 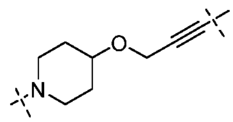 ,

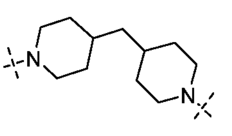 , 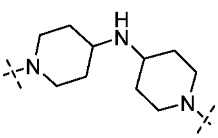 , 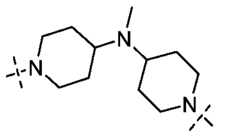 , 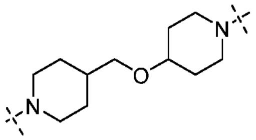 ,

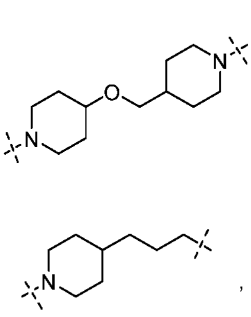 , 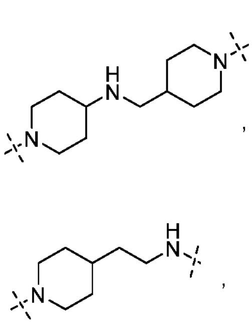 , 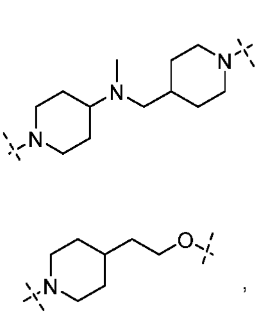 , 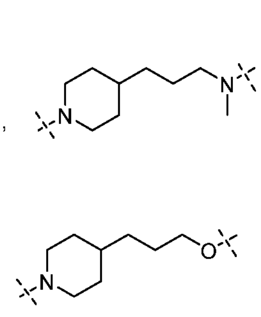 ,

, , , ,

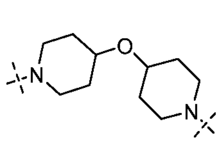 , 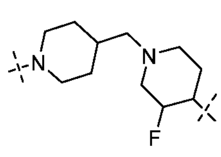 , 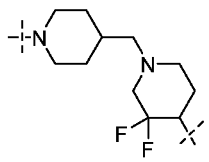 , 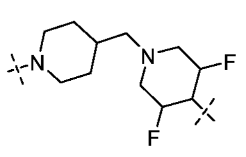

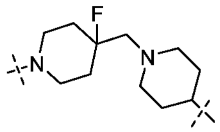 , 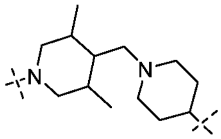 , 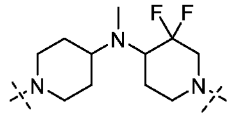 , 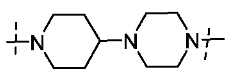 ,

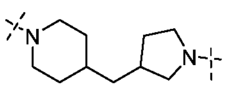 , 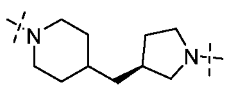 , 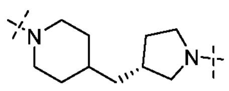 , 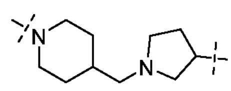

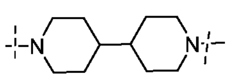 , 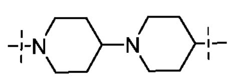 , 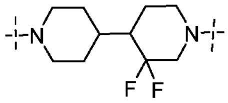 , 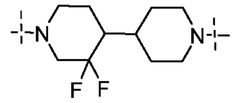

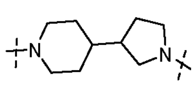 , 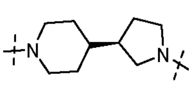 , 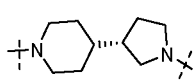 , 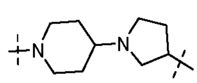 ,

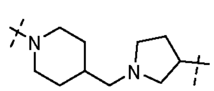 , 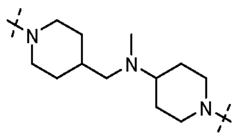 , 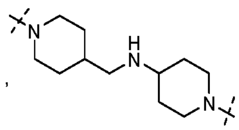 ,

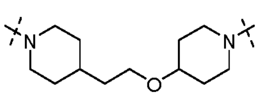 , 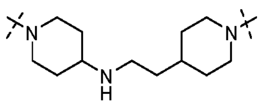 , 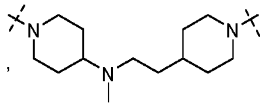 , 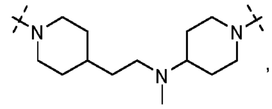 ,

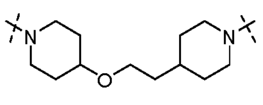 , 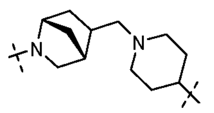 , 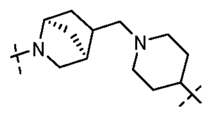 , 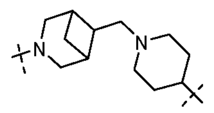 ,

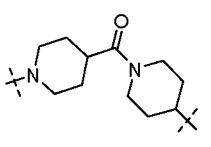 , 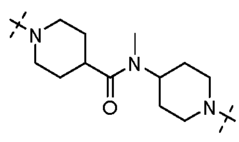 , 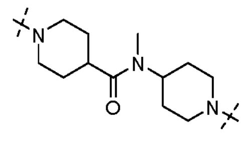 ,

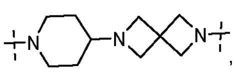 , 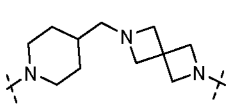 , 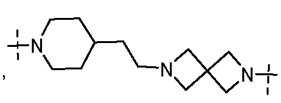 , 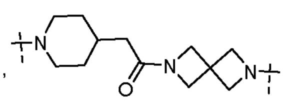 ,

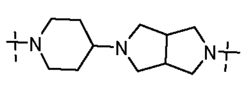 , 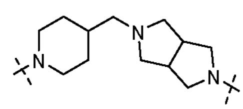 ,

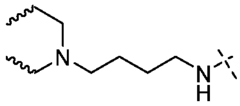 , 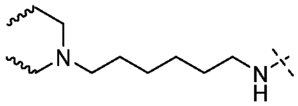 , 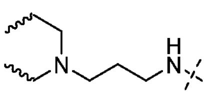 , 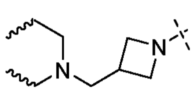 ,

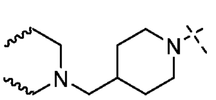 , 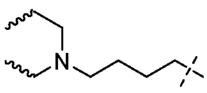 , 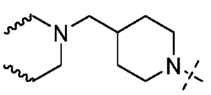 , 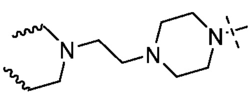 ,

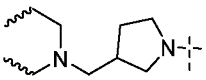 , 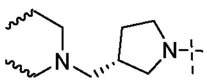 , 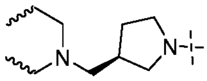 , 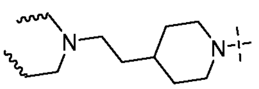 ,

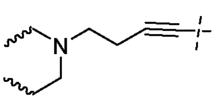 , 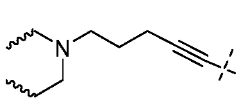 , 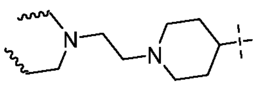 , 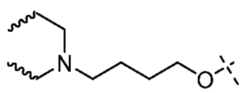 ,

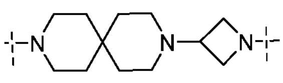 , 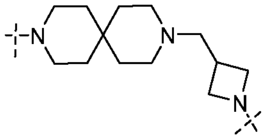 , 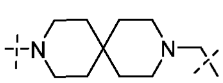 , 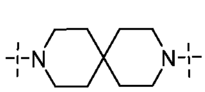 ,

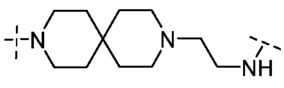 , 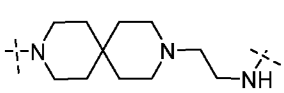 , 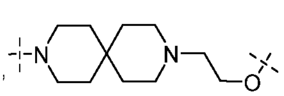 ,

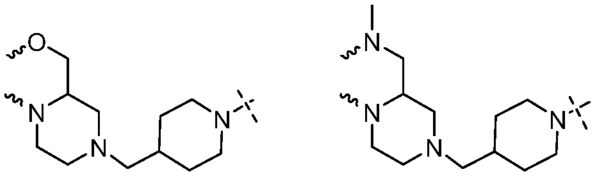 ,

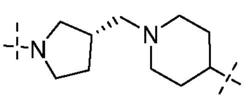 , 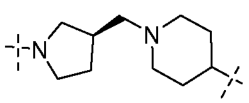 , 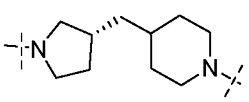 , 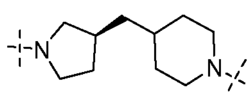 ,

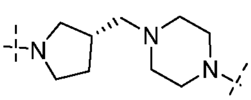 , 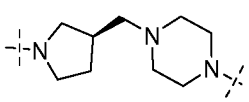 , 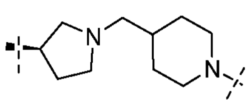 , 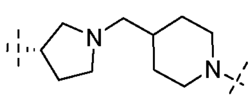 ,

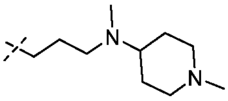 , 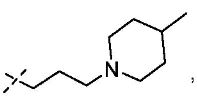 , 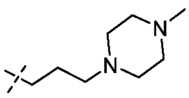 , 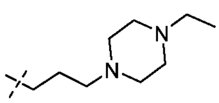 , 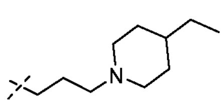 ,

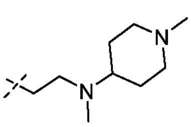 , 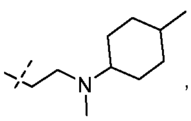 , 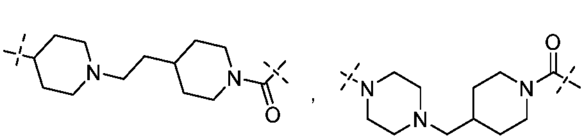 ,

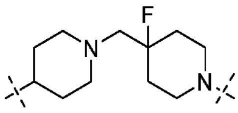, 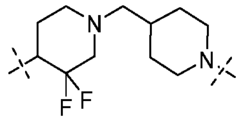, 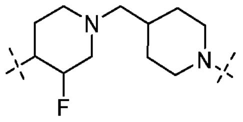, 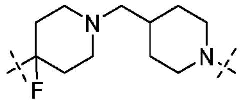

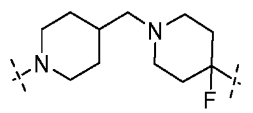, 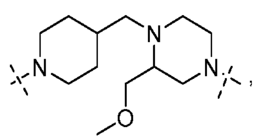, 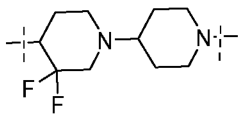, 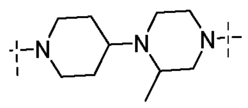,

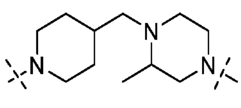, 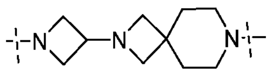, 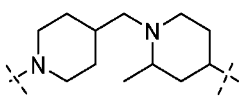, 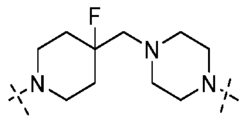,

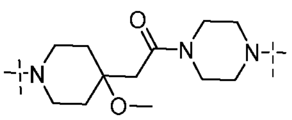, 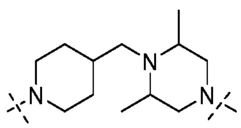, 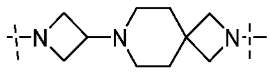, 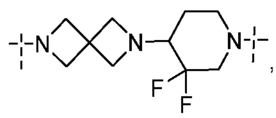,

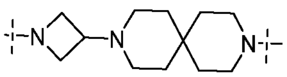, 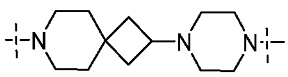, 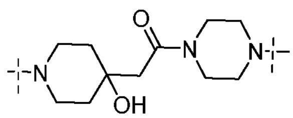, 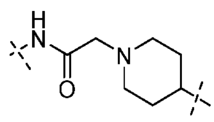

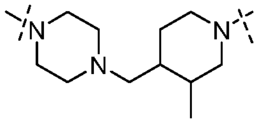, 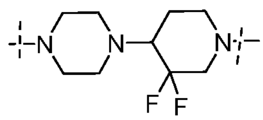, 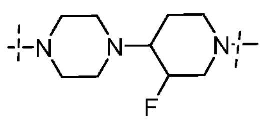, 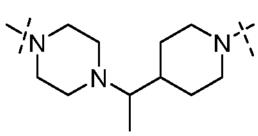,

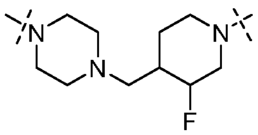, 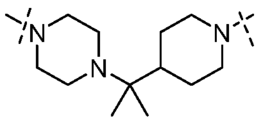, 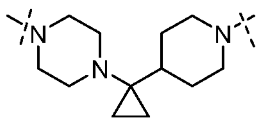, 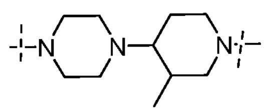,

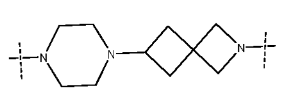, 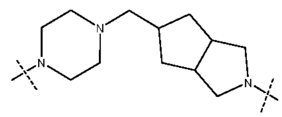

wherein

 or 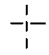

represents a linking site; and wherein the L group is optionally substituted with 1, 2, 3, or 4 substituents independently selected from halogen, hydroxyl, nitro, cyano, amino, mercapto, COOH, or $C_1$-$C_6$ alkyl.

**73.** A compound, or a derivative, pharmaceutically acceptable salt, isomer, solvate, hydrate, adduct, complex or prodrug thereof, having a structure selected from:

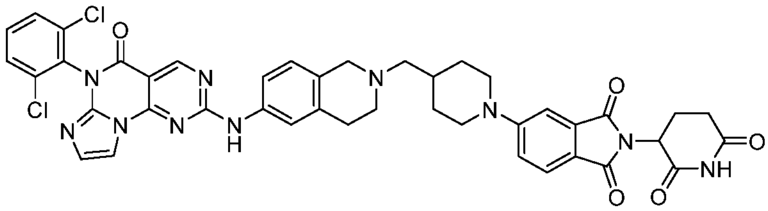

,

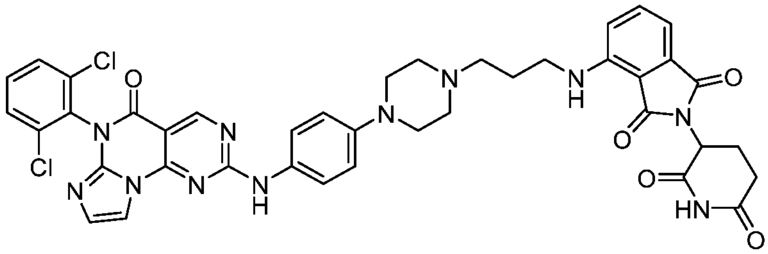

,

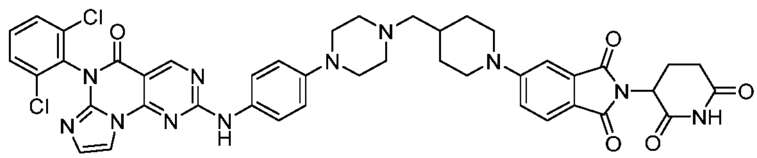

,

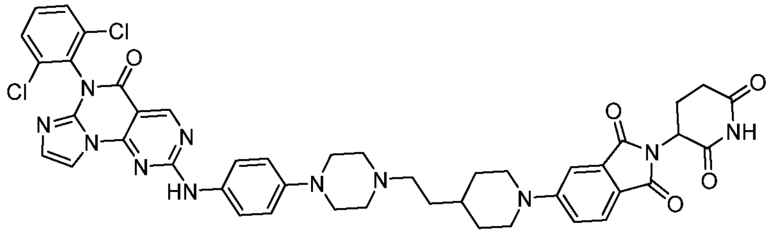

,

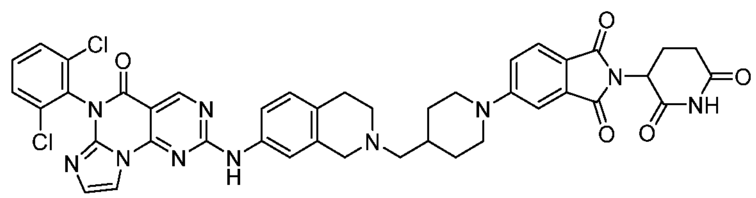

,

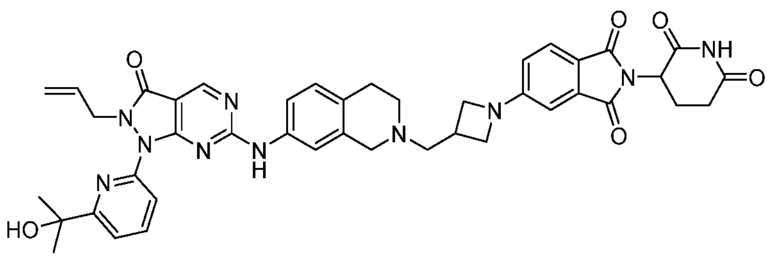

,

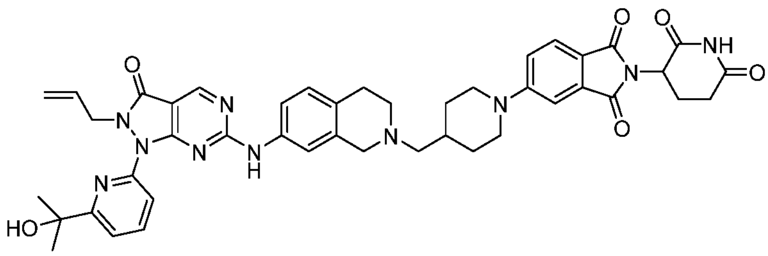

,

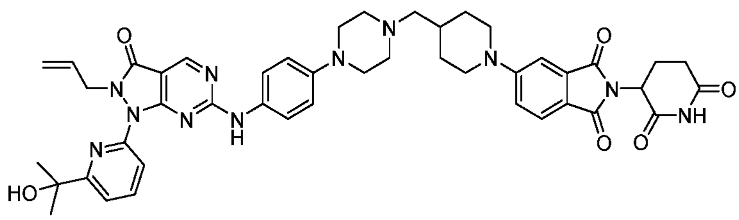

,

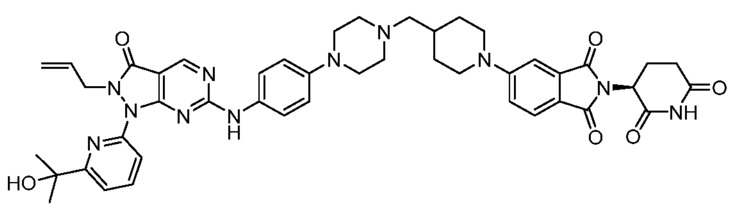

,

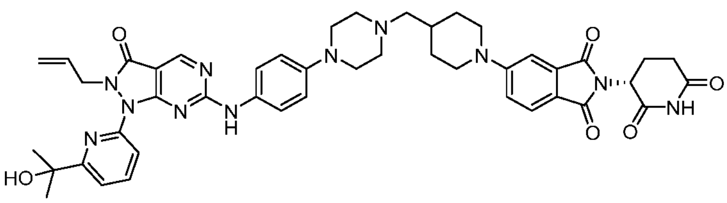

,

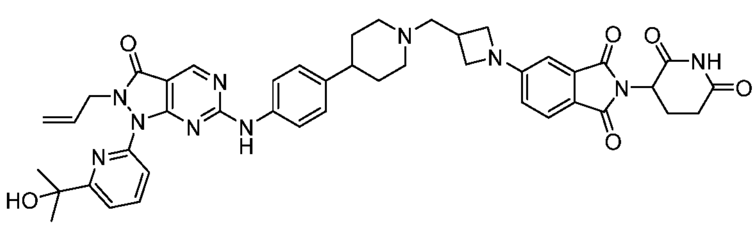

,

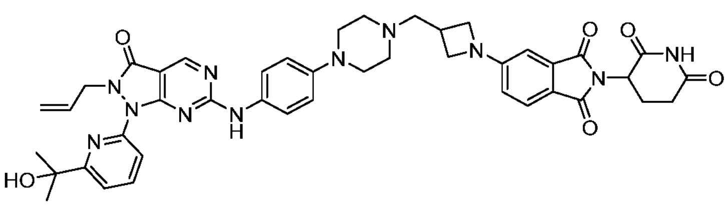

,

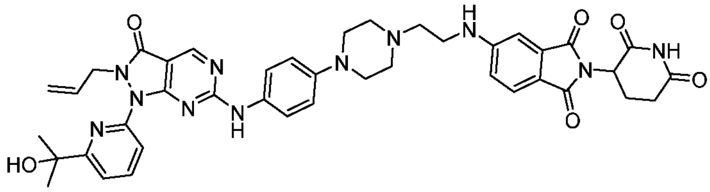

,

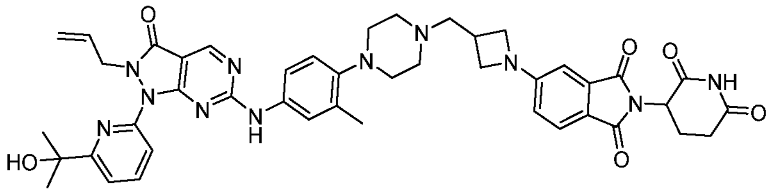

,

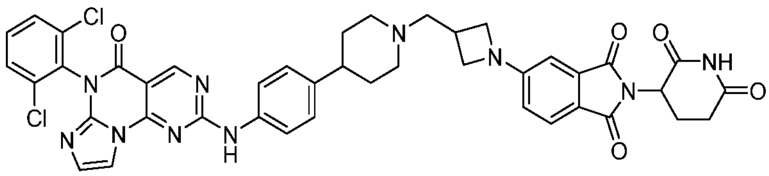

,

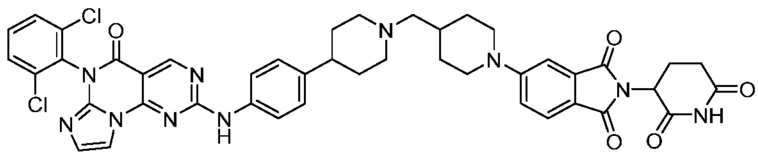

,

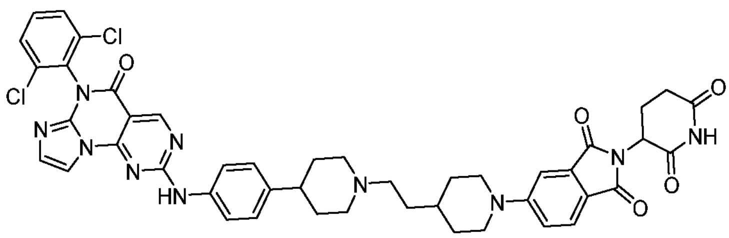

,

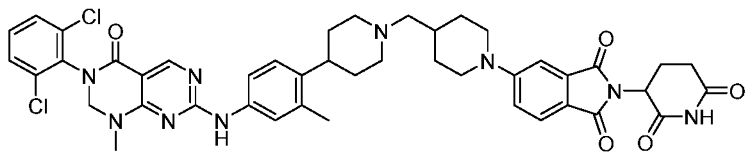

,

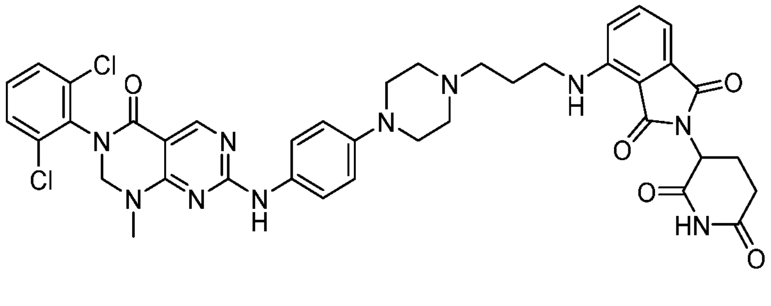

,

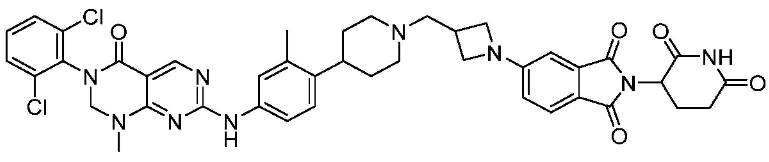

,

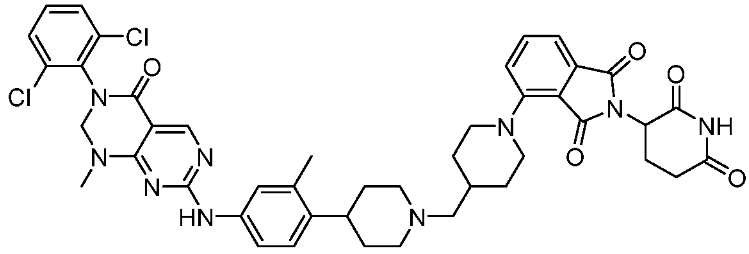

,

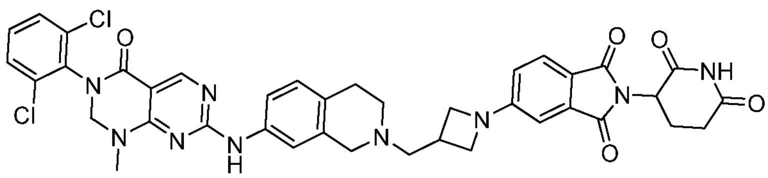

,

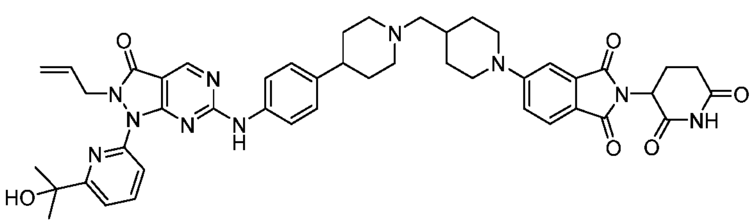

,

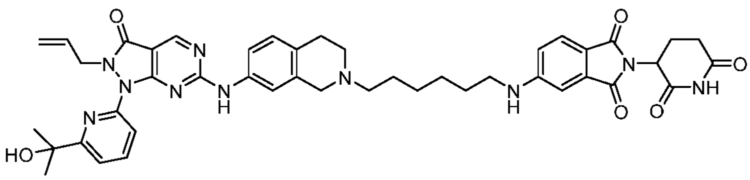

,

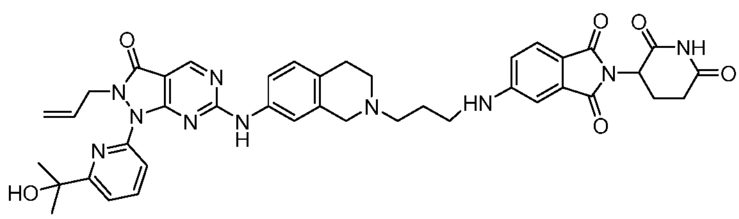

,

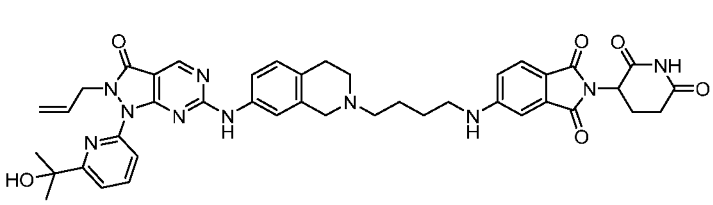

,

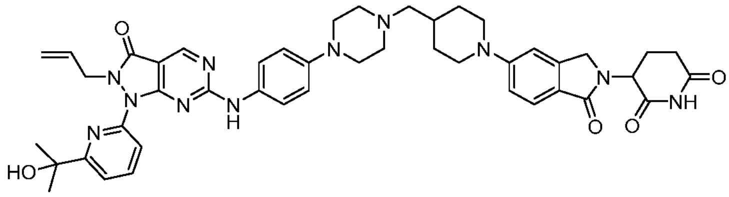

,

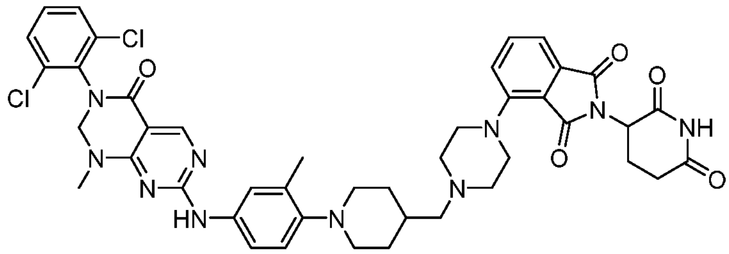

,

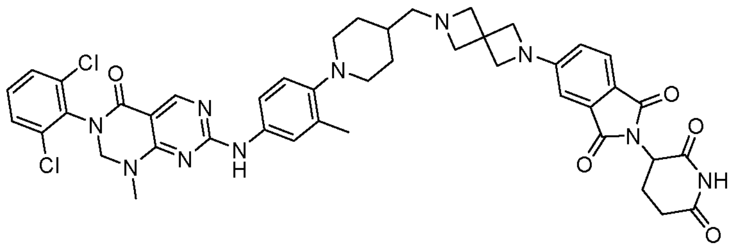

,

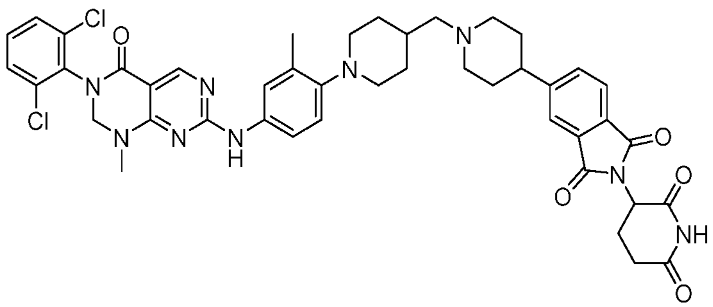

,

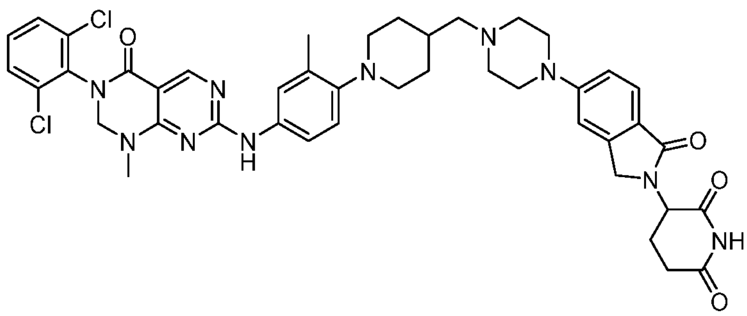

,

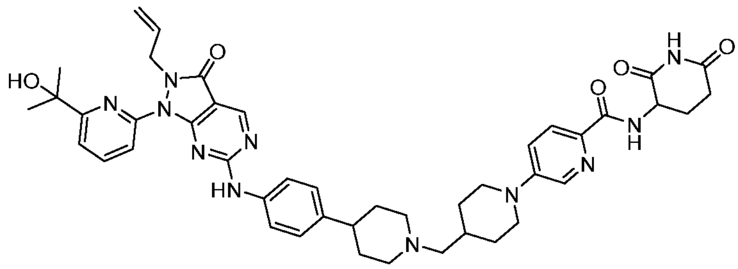

,

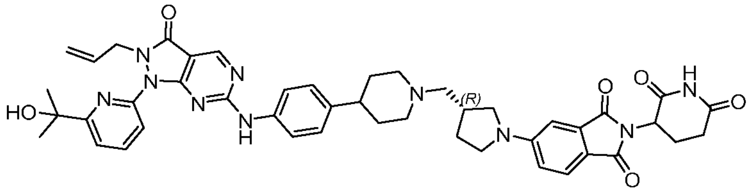

,

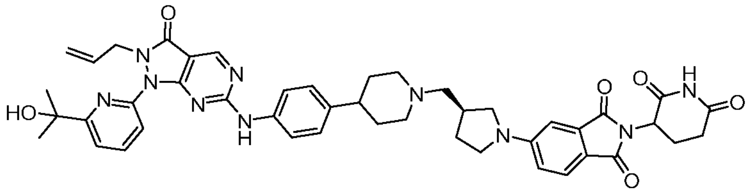

,

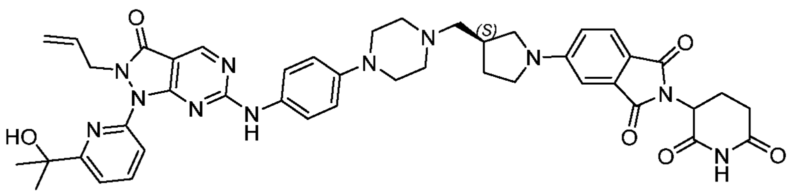

,

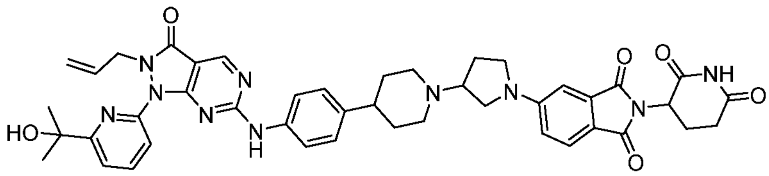

,

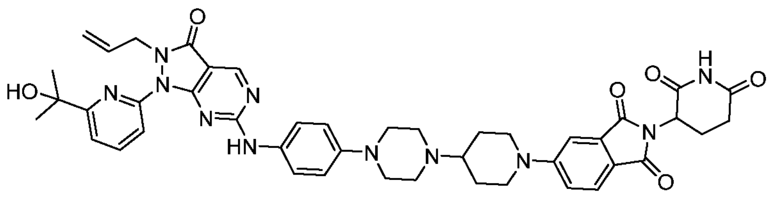

,

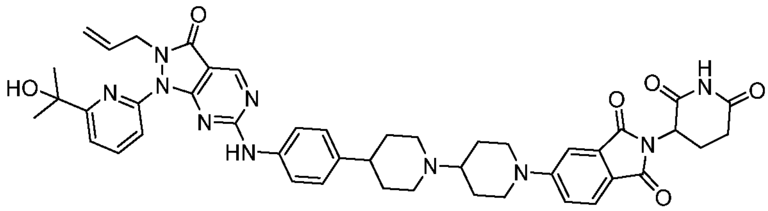

,

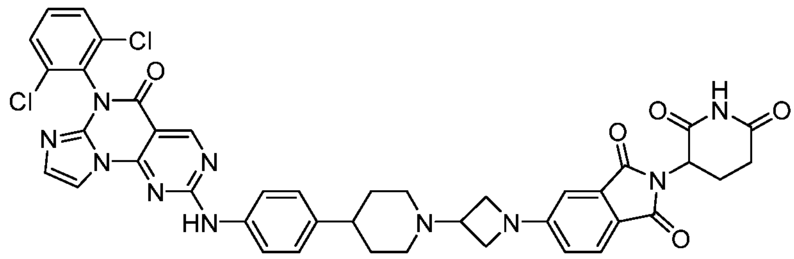
,

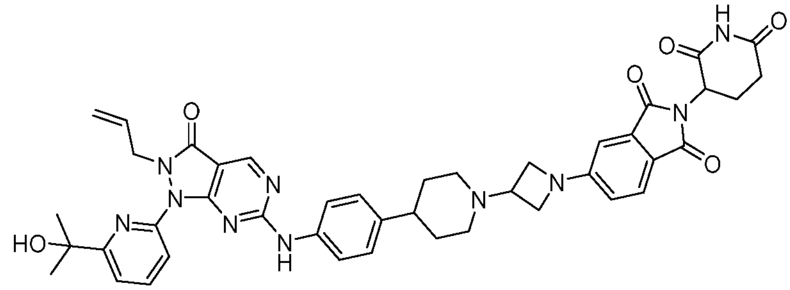
,

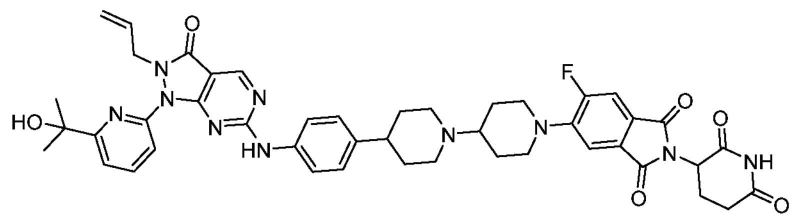
,

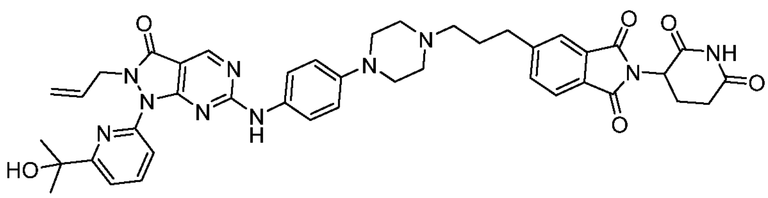
,

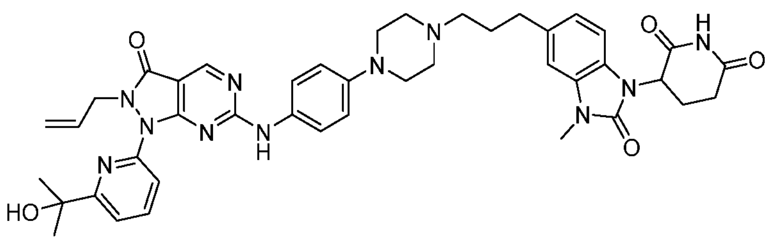
,

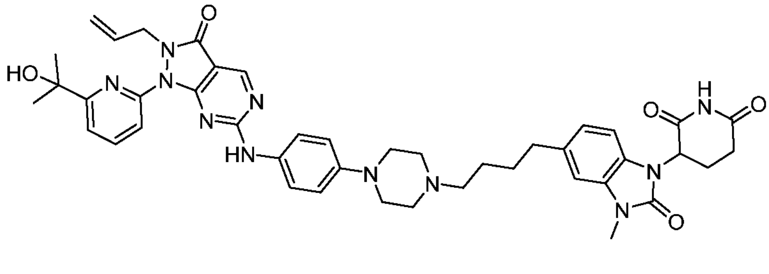
,

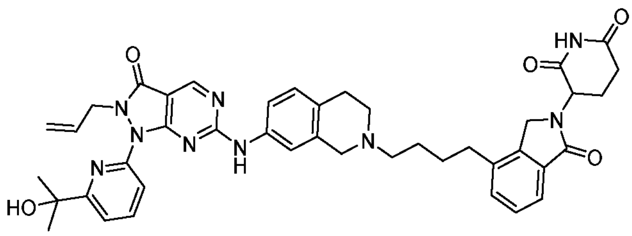

,

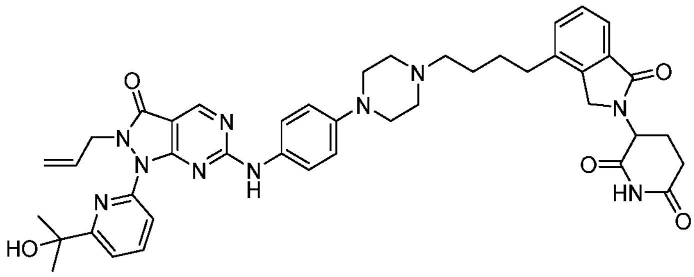

,

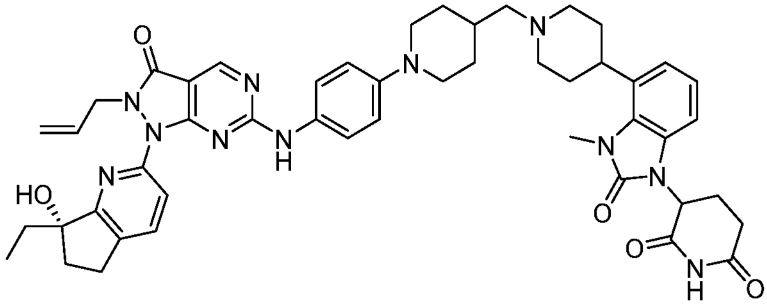

,

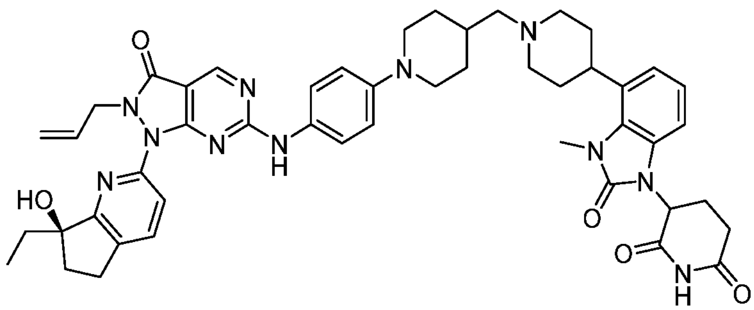

,

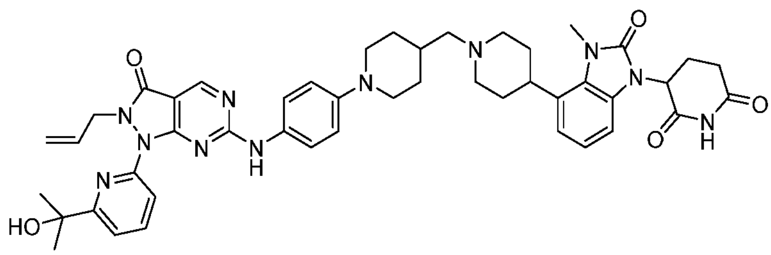

,

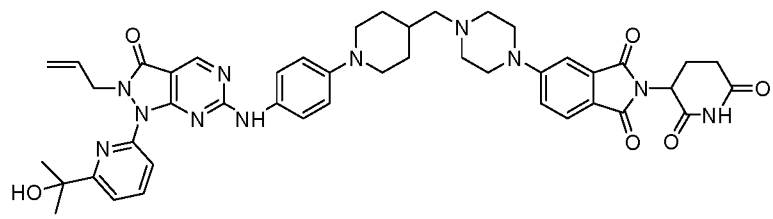

,

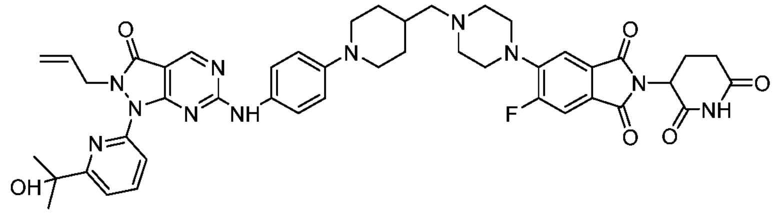

,

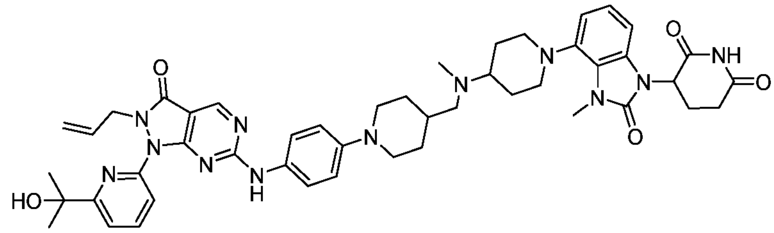

,

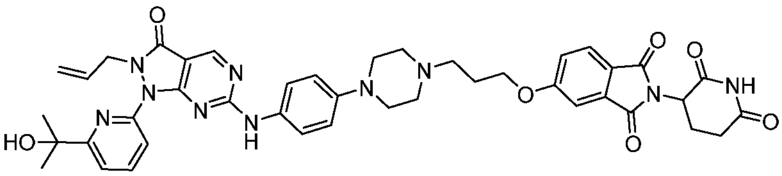

,

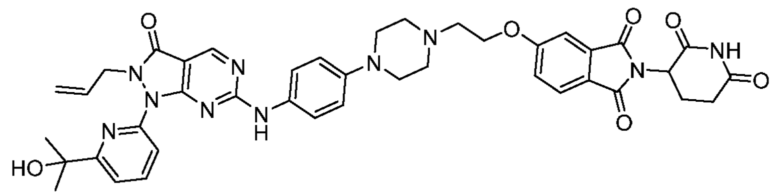

,

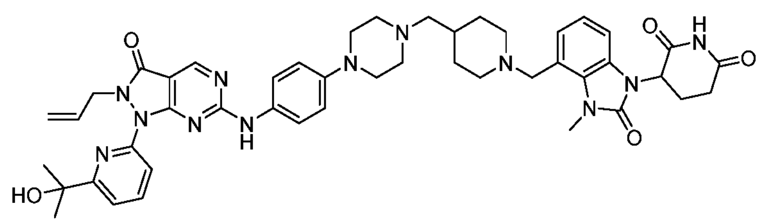

,

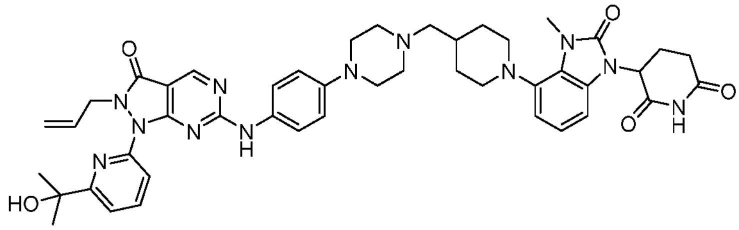

,

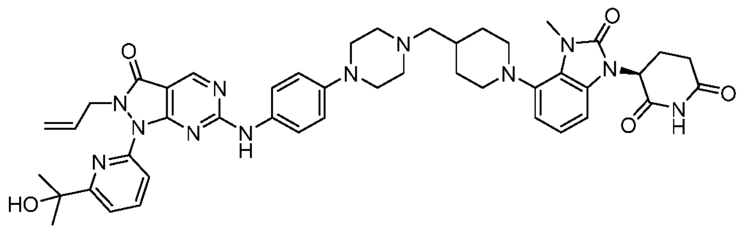

,

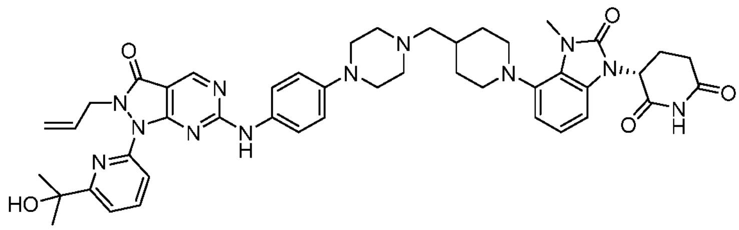

,

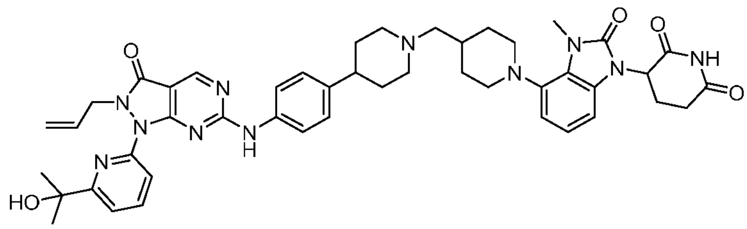

,

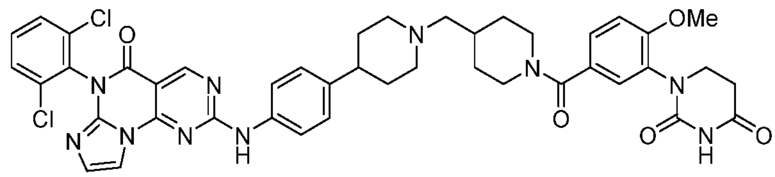

,

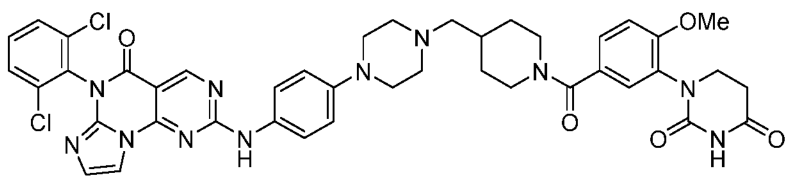

,

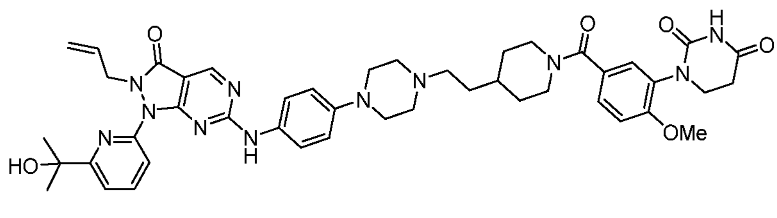

,

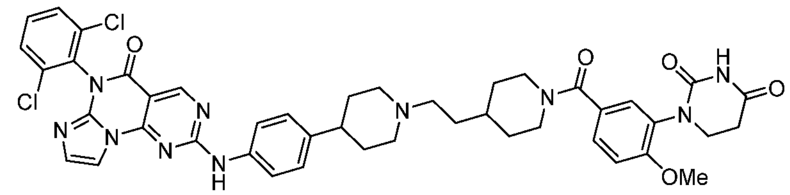
,

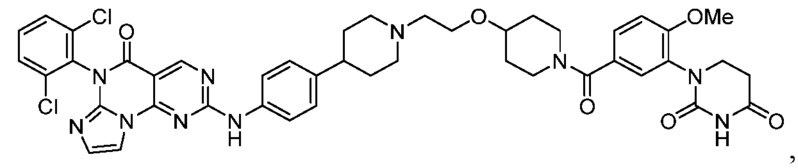
,

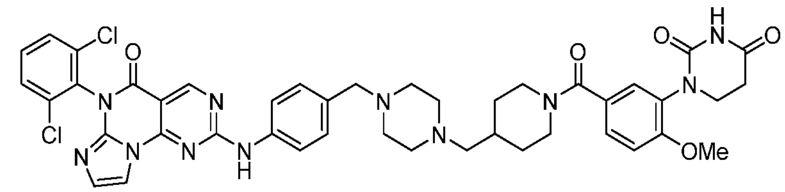
,

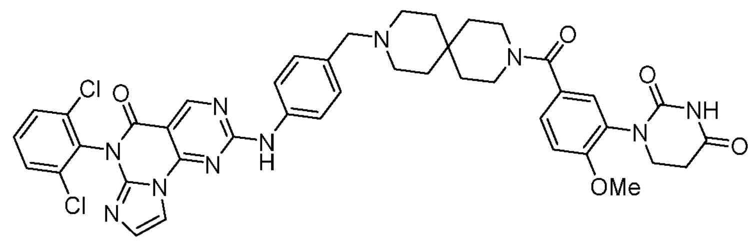
,

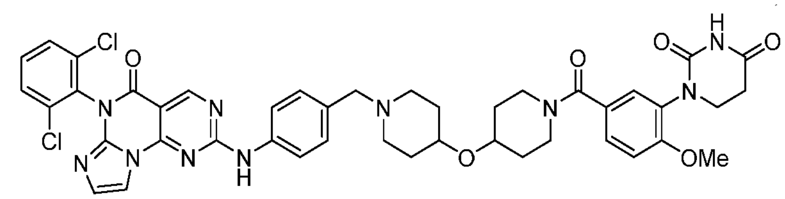
,

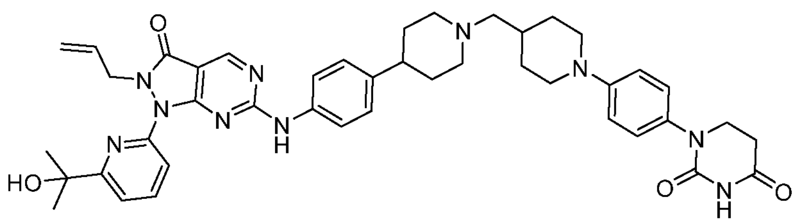
,

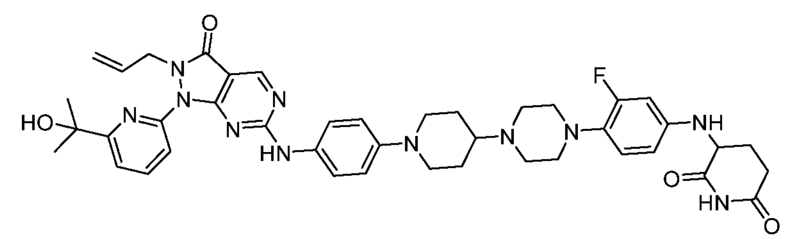
,

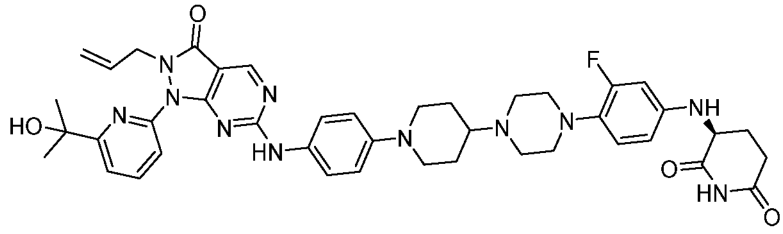

,

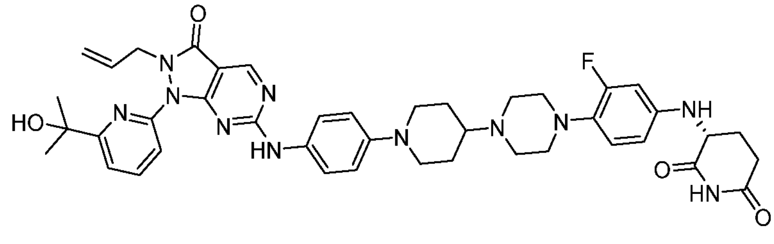

,

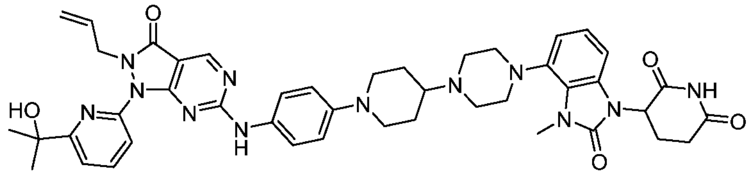

,

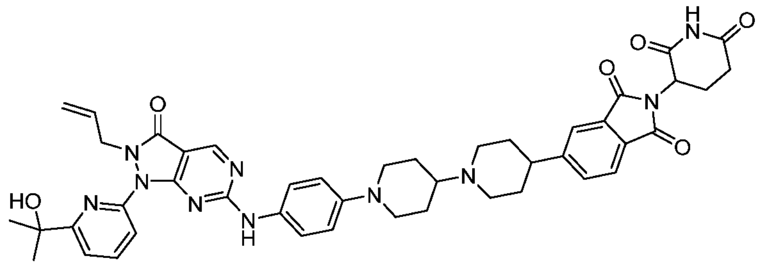

,

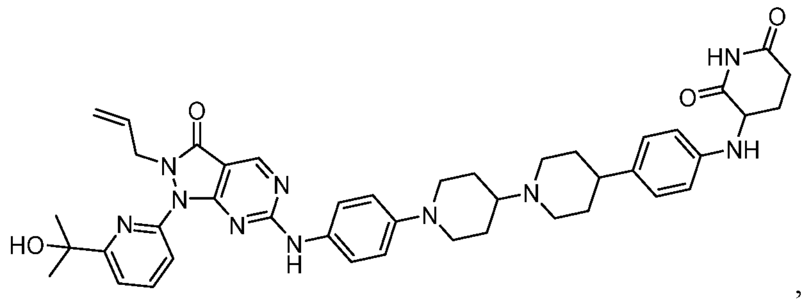

,

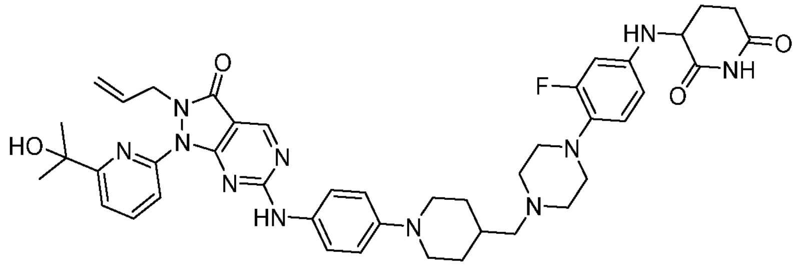

,

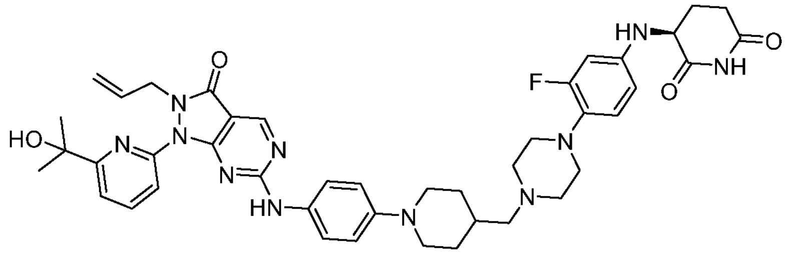

,

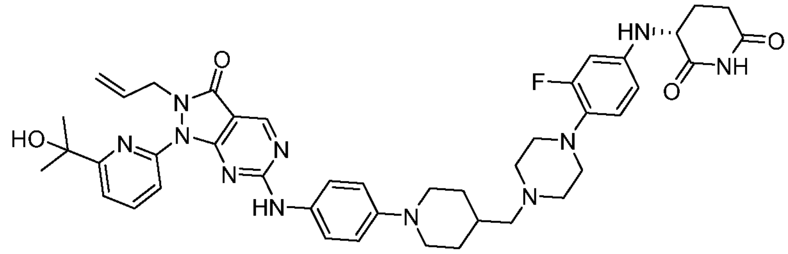

,

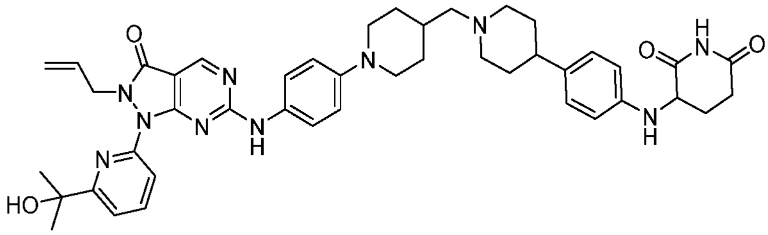

,

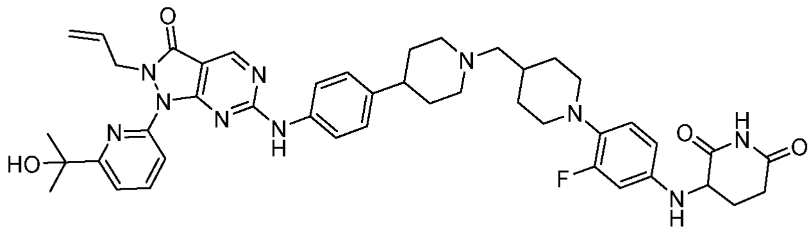

,

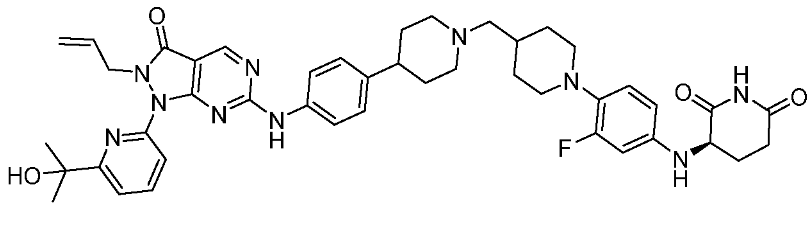

,

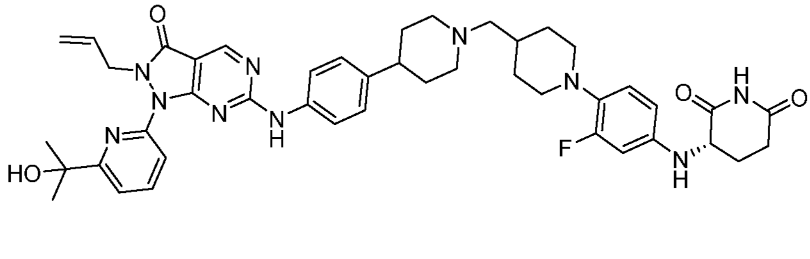

,

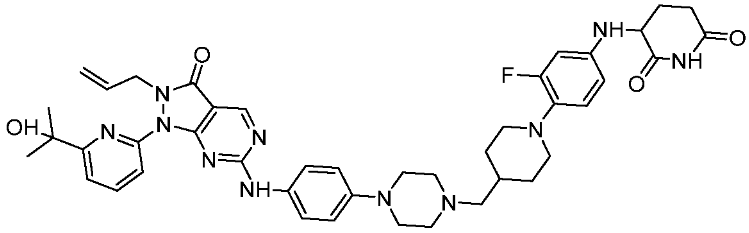

,

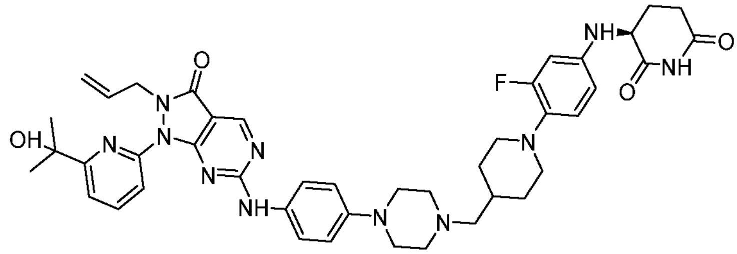

,

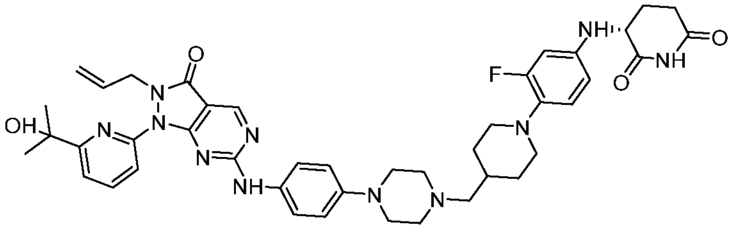

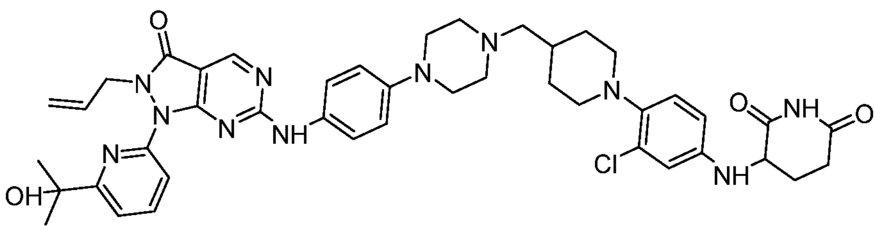

,

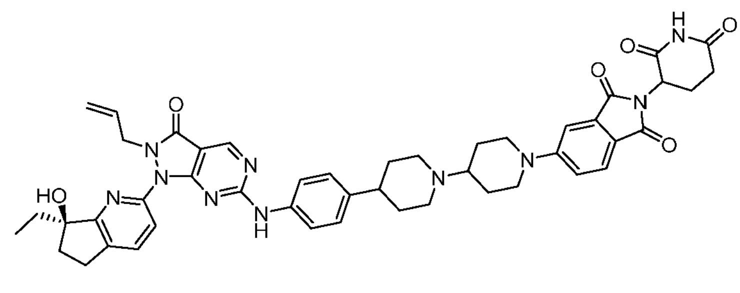

,

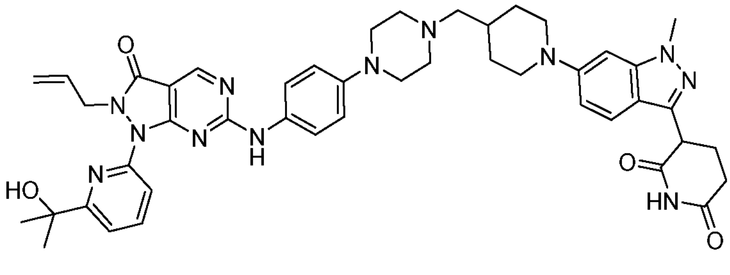

,

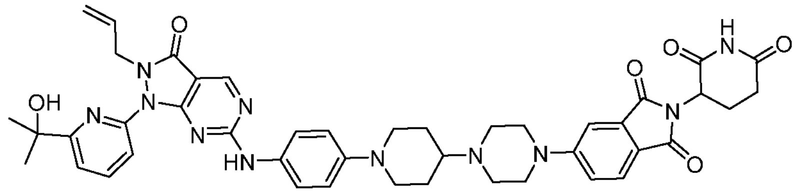

,

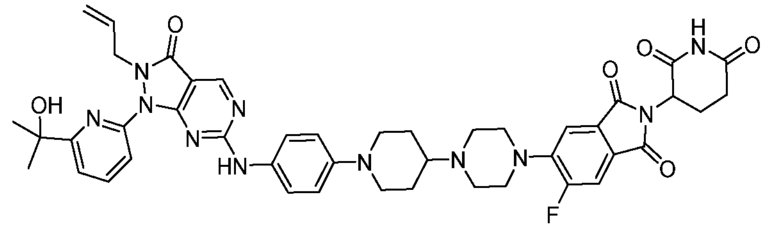

,

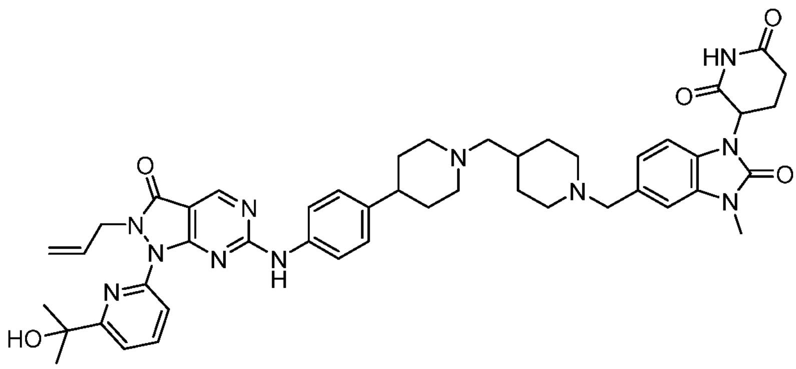

,

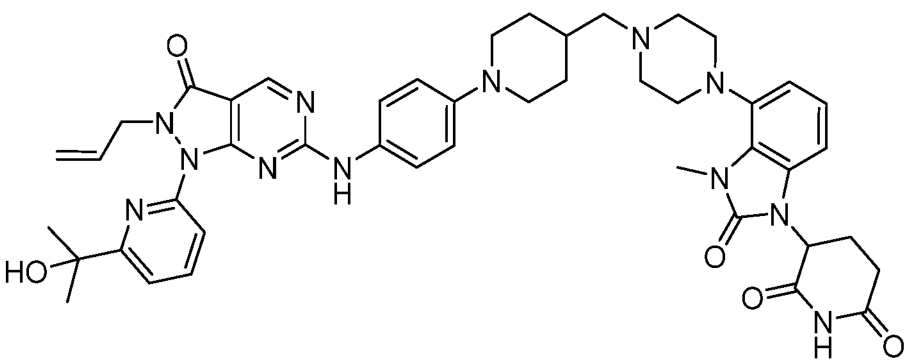

,

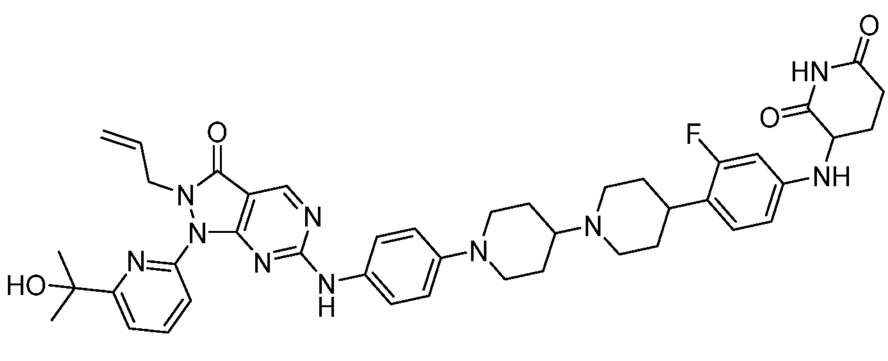

,

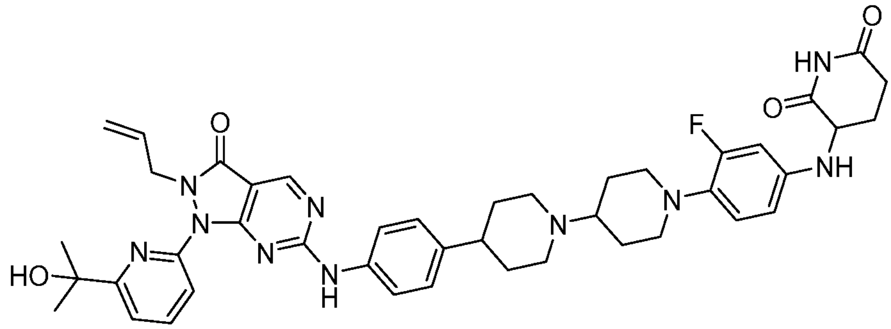

,

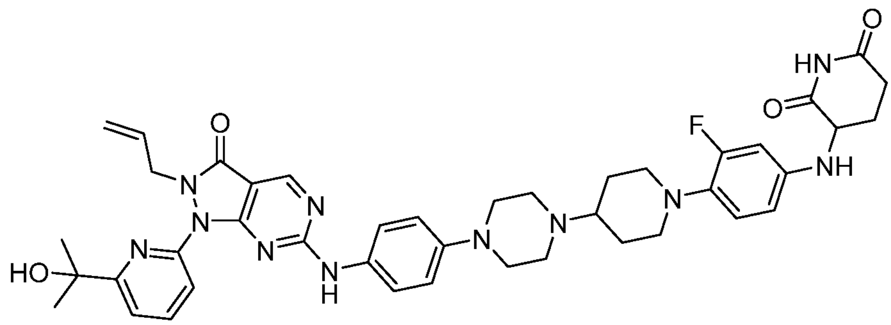

,

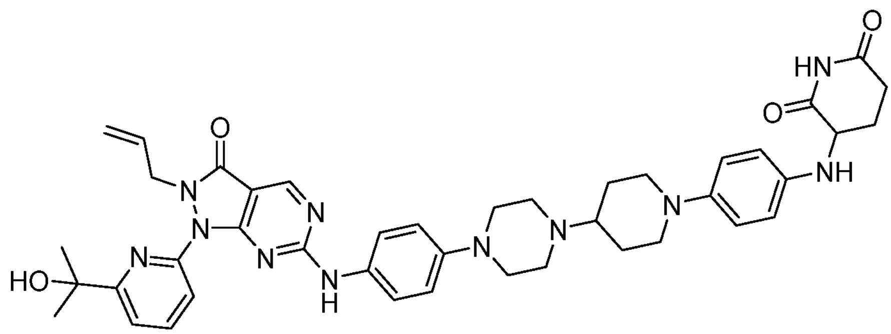

,

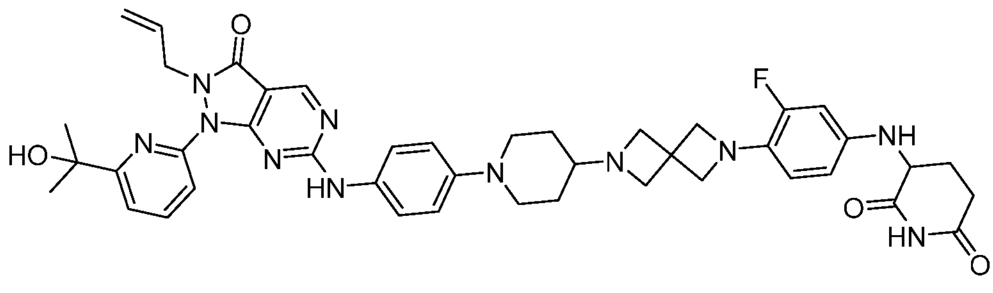

,

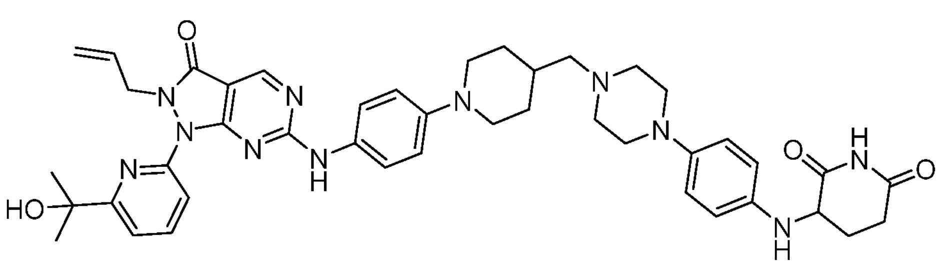

,

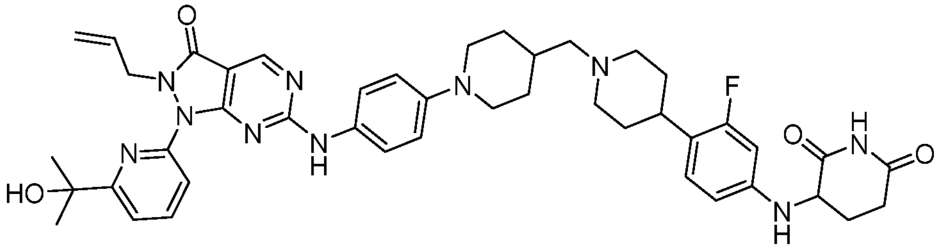

,

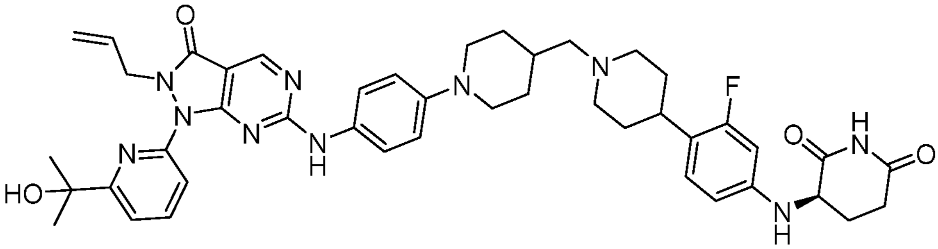

,

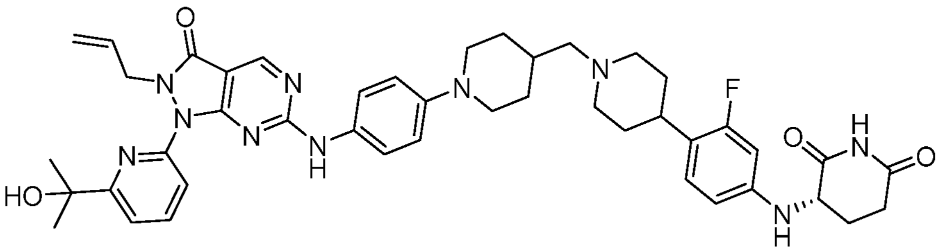

,

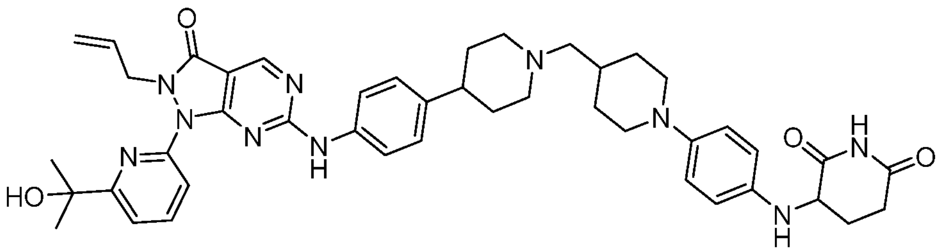

,

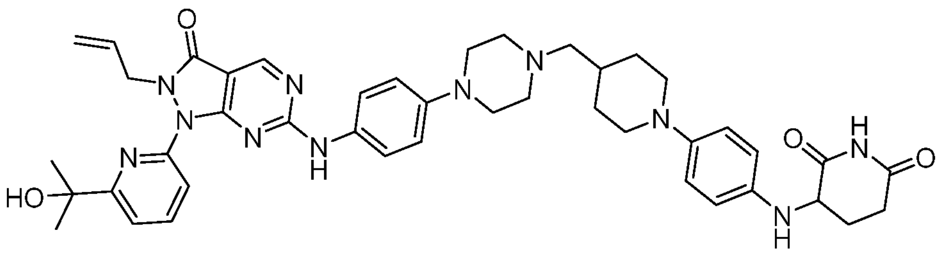

,

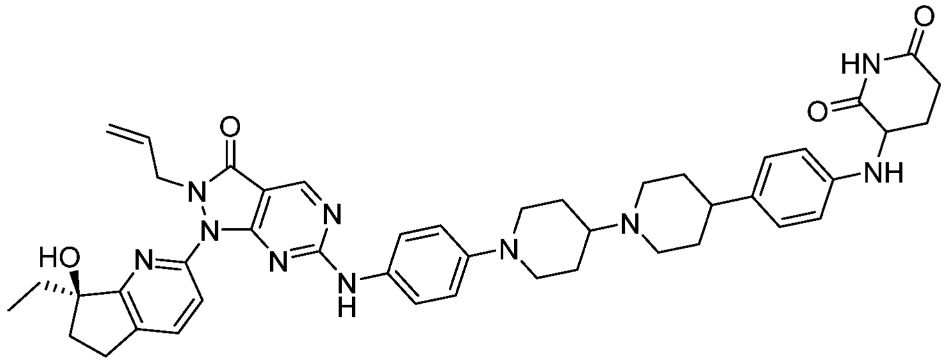

,

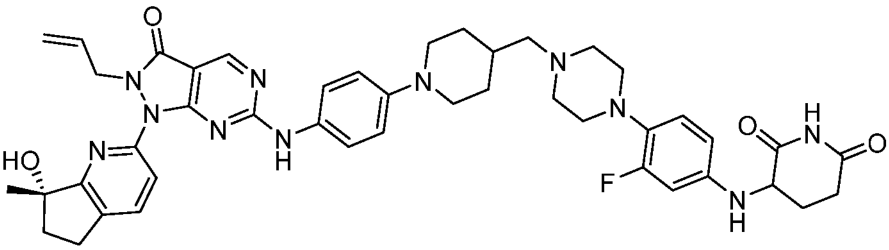

,

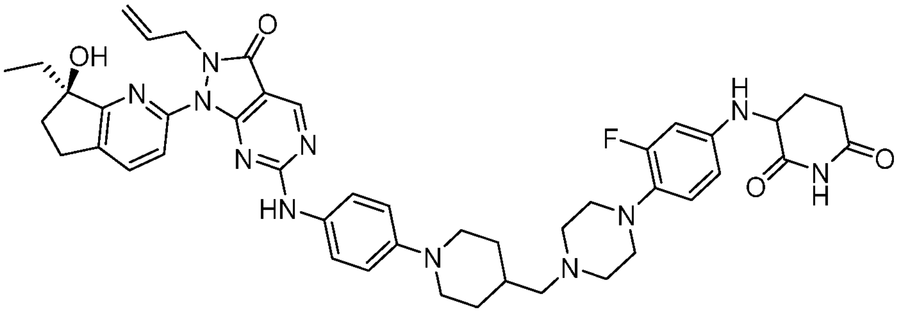

,

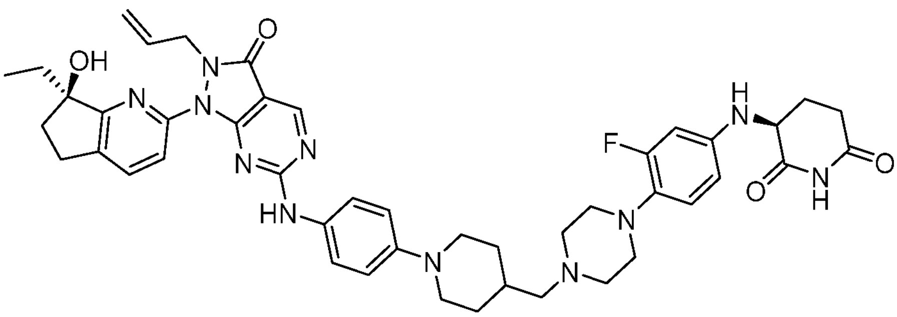

,

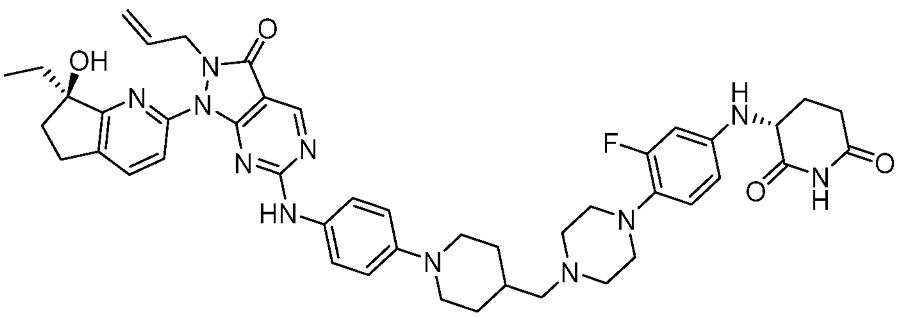

,

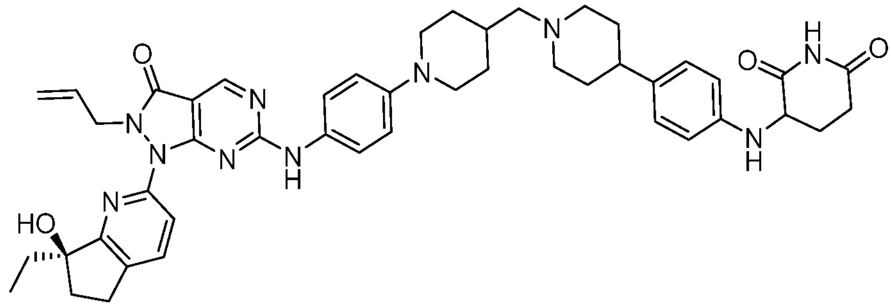

,

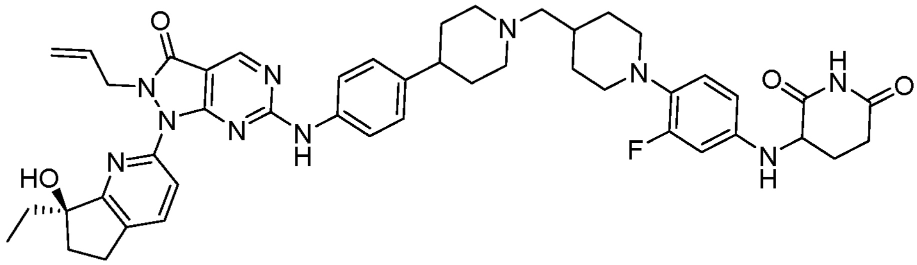

,

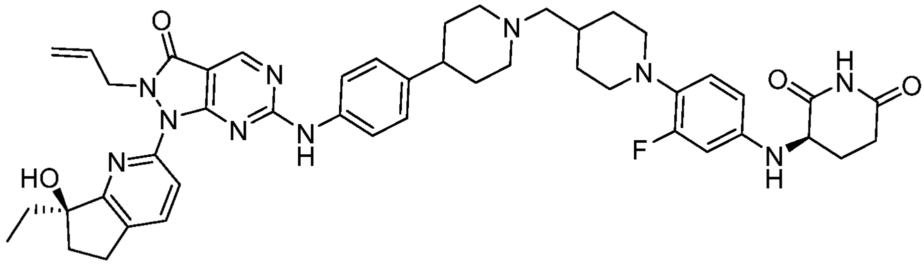

,

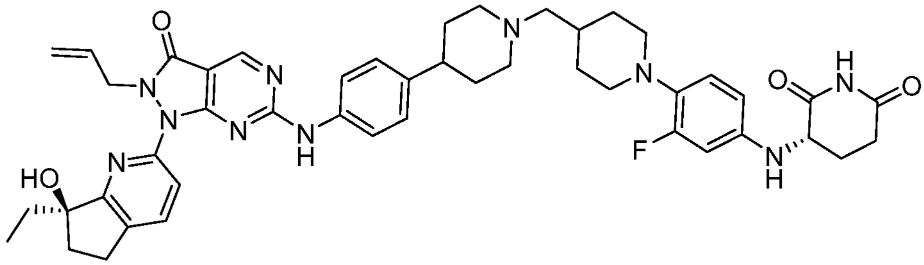

,

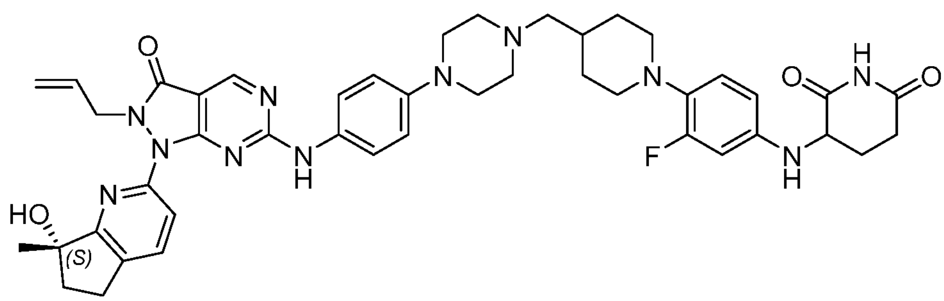

,

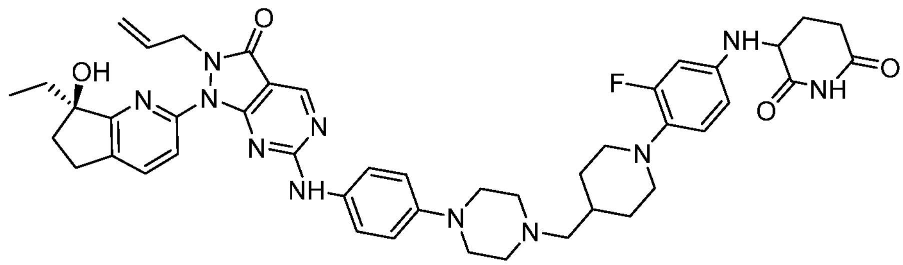

,

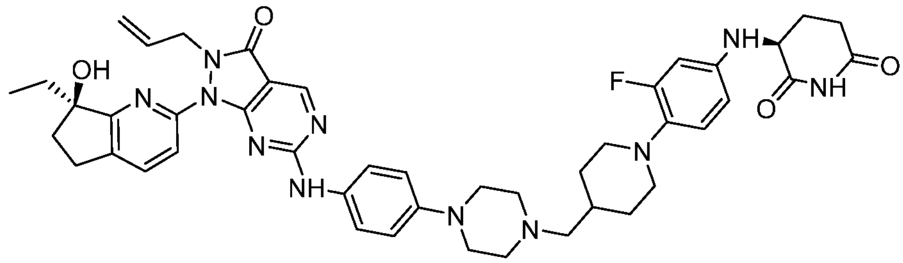

,

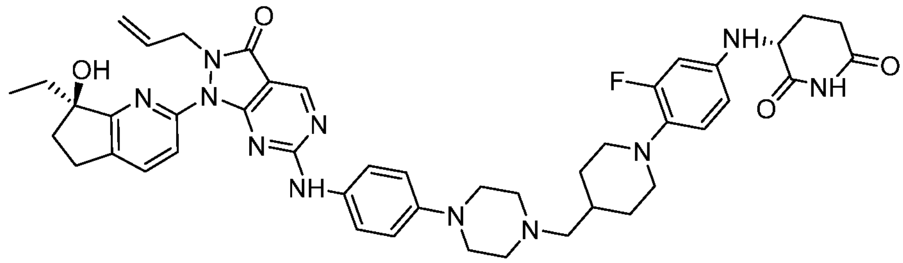

,

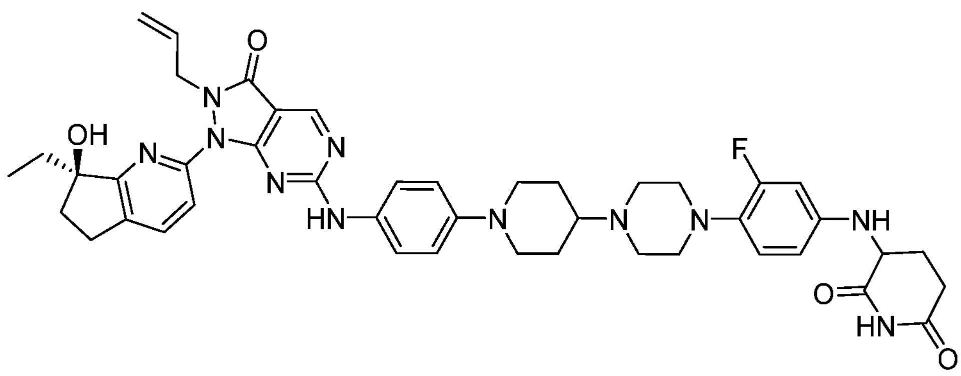

,

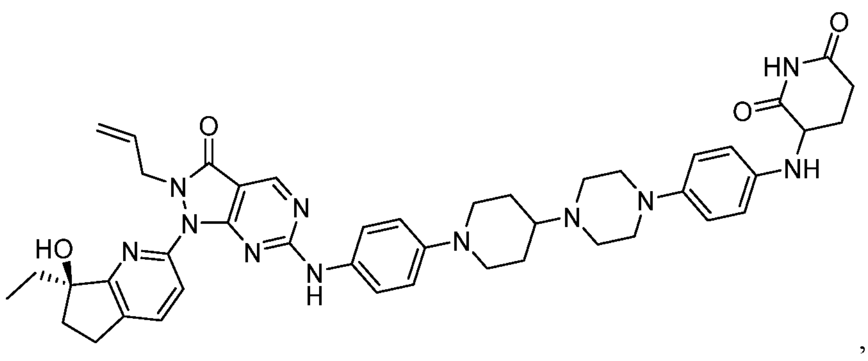

,

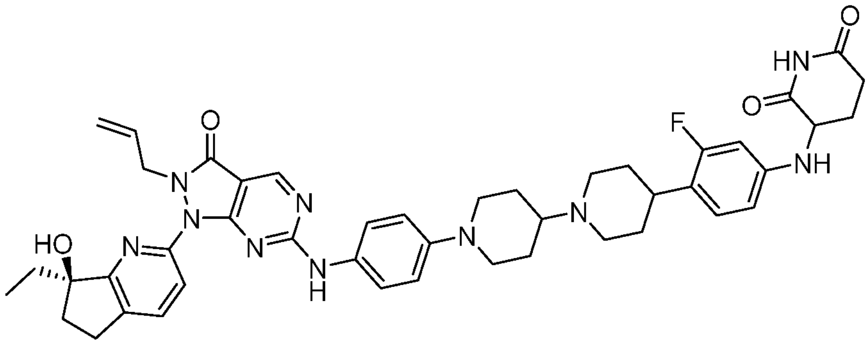

,

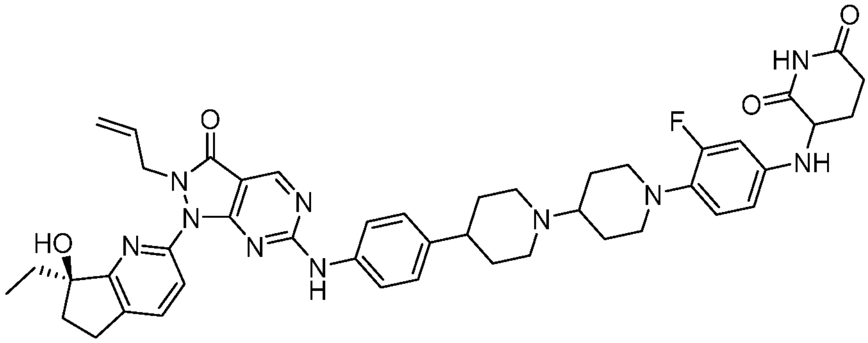

,

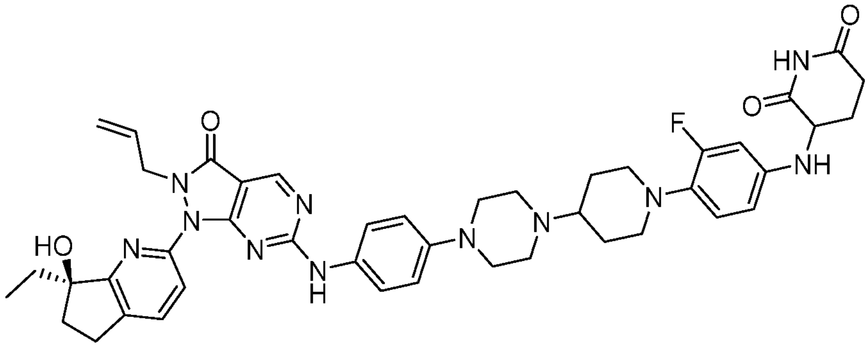

,

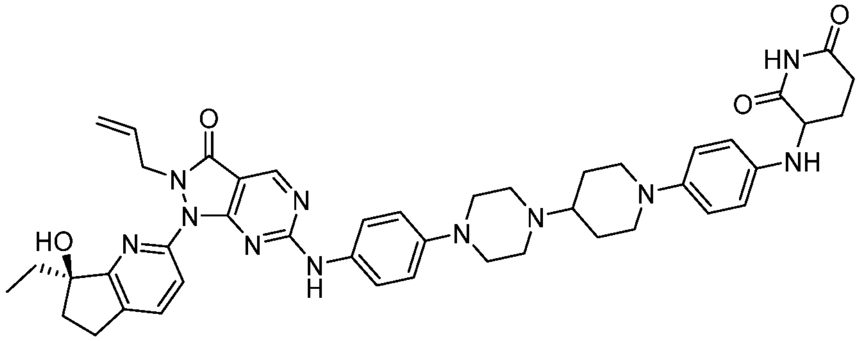

,

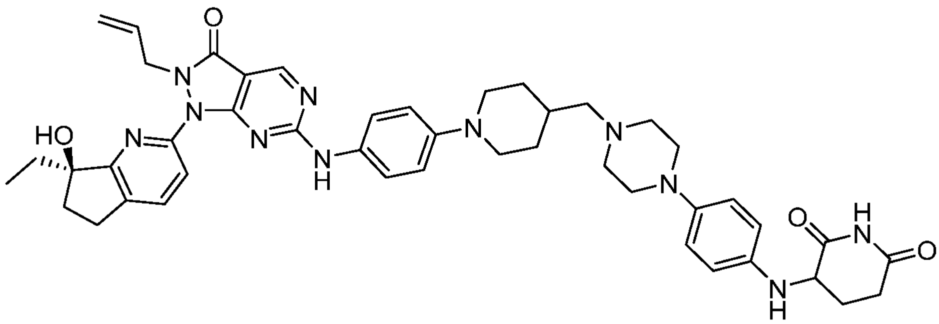

,

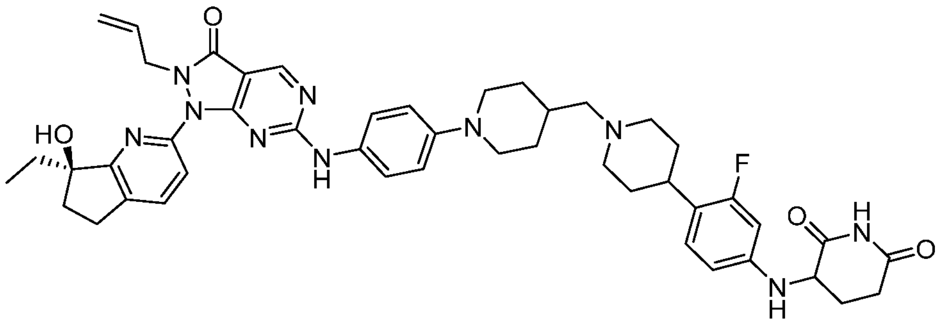

,

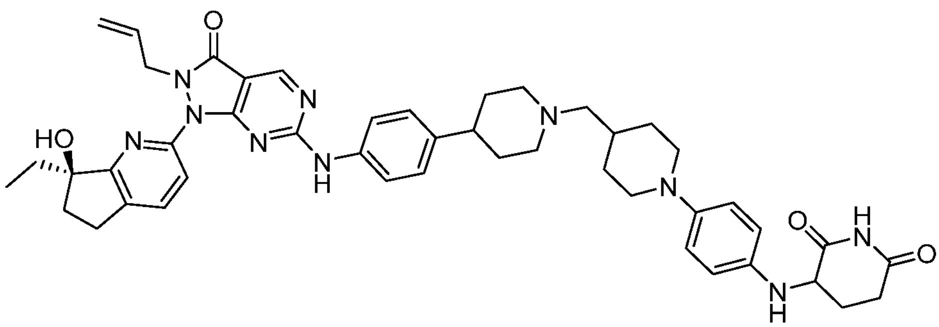

,

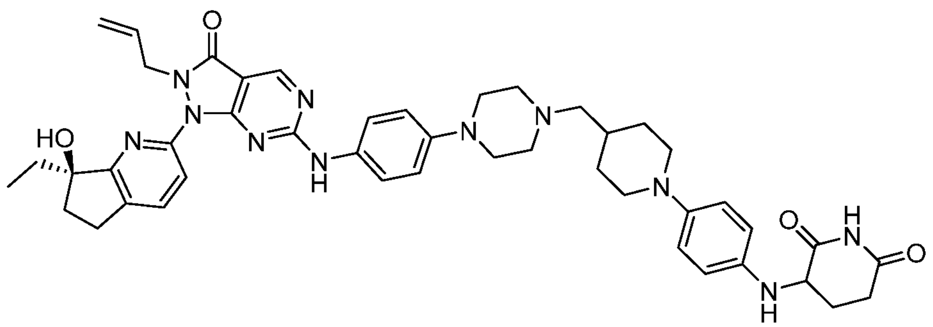

,

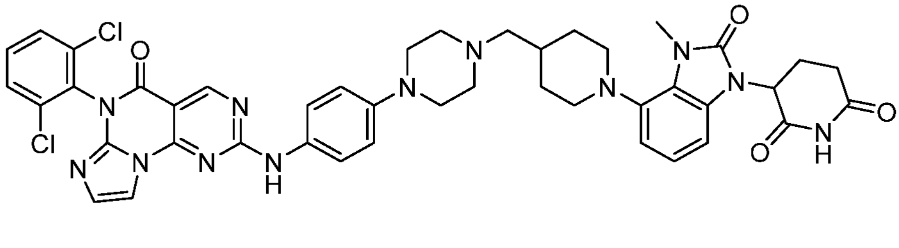

,

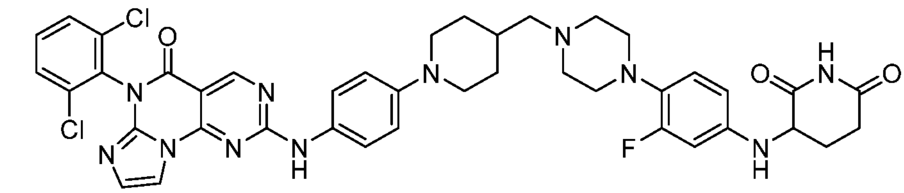

,

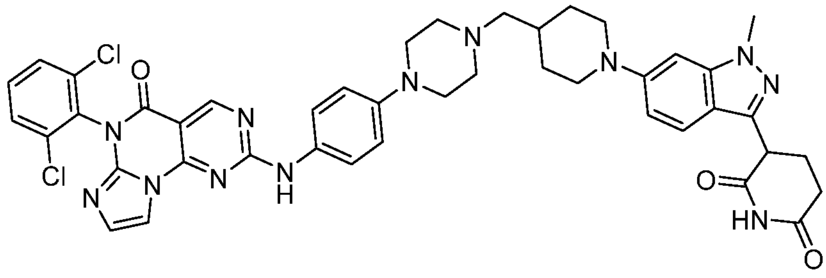

,

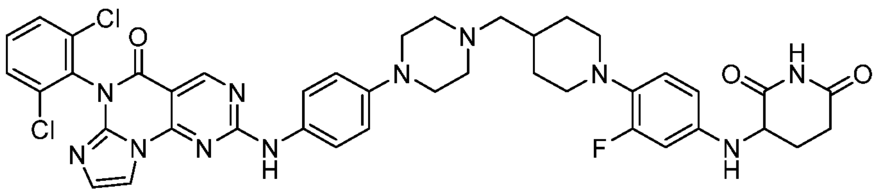

,

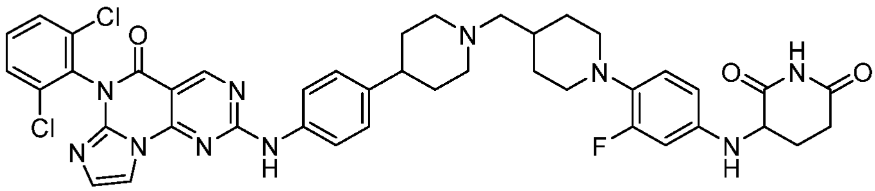

,

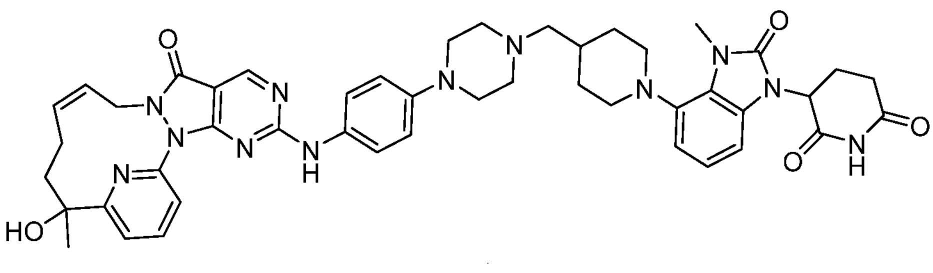

,

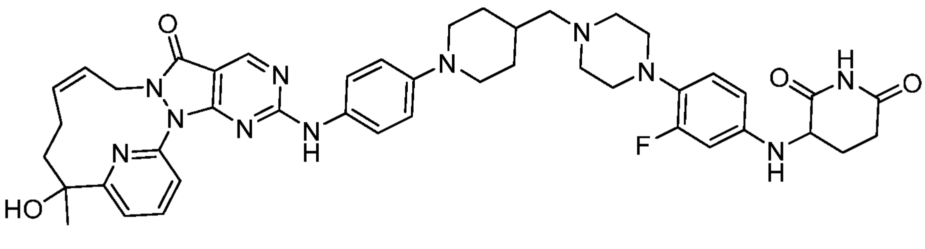

,

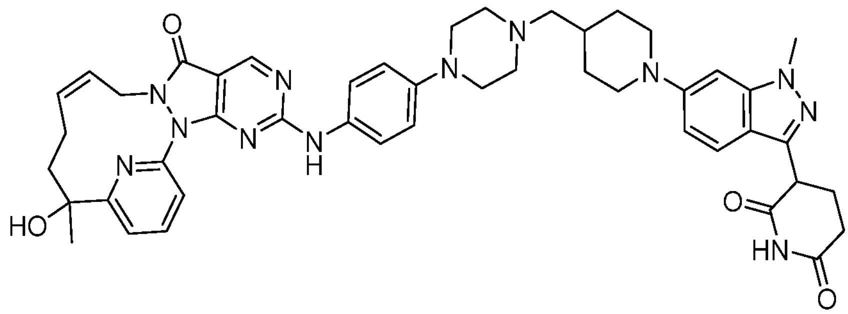

,

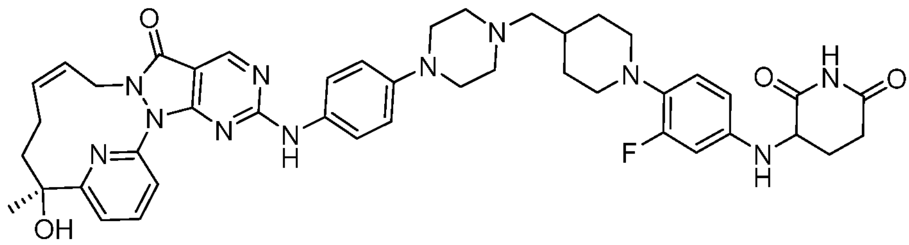

,

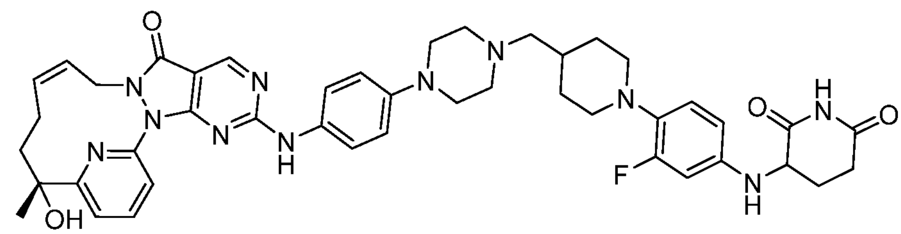

,

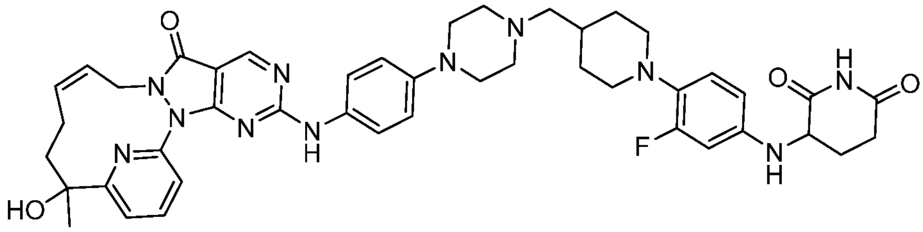

,

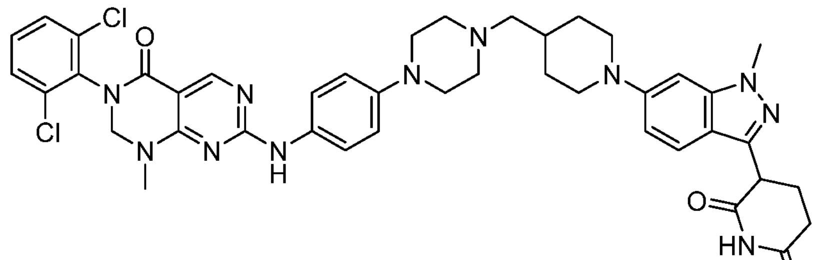

,

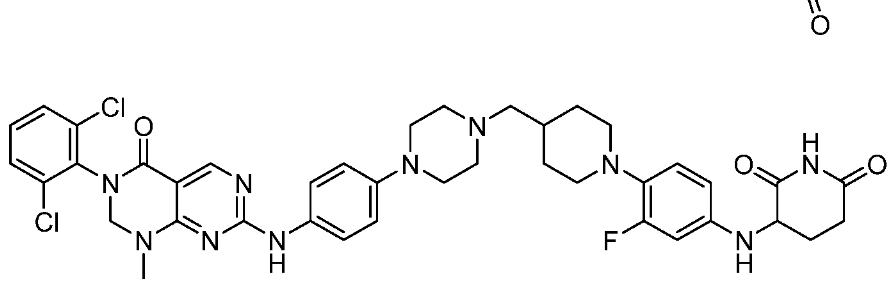

,

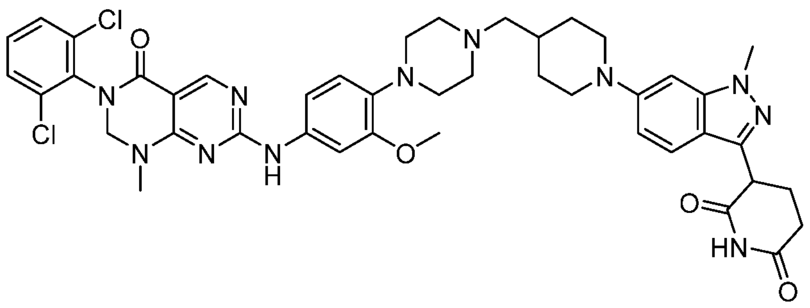

,

,

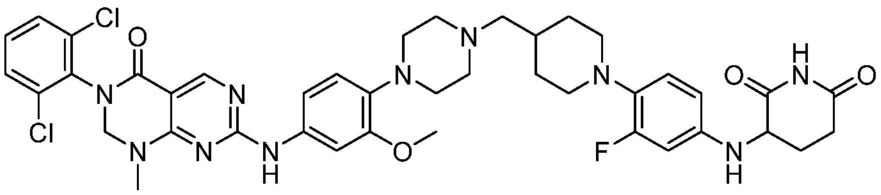

,

,

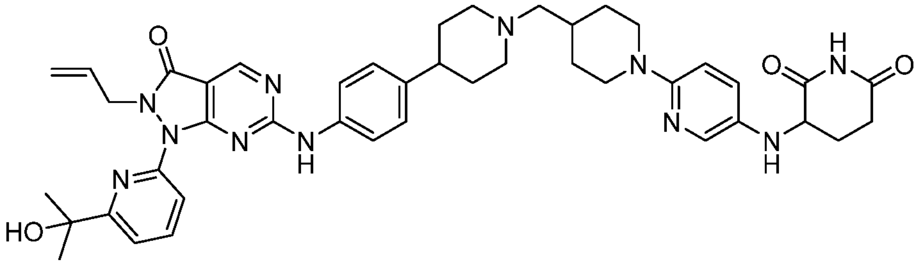

,

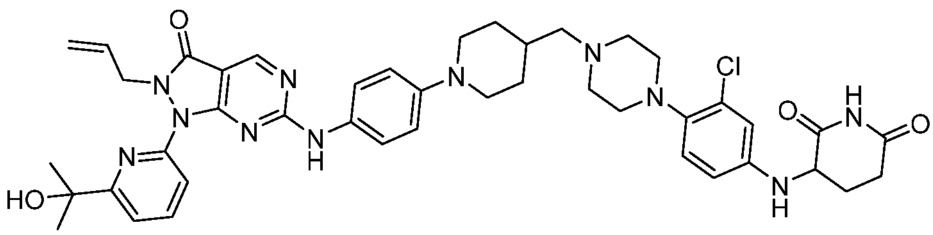

,

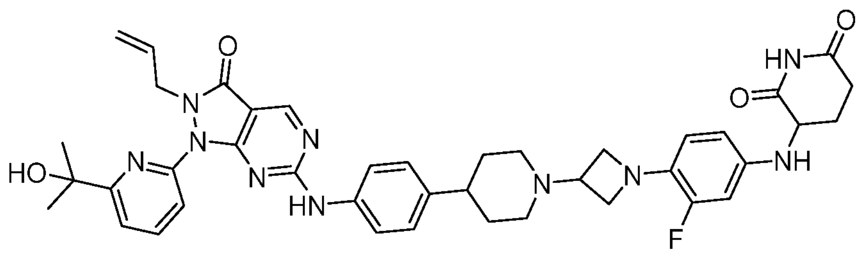

,

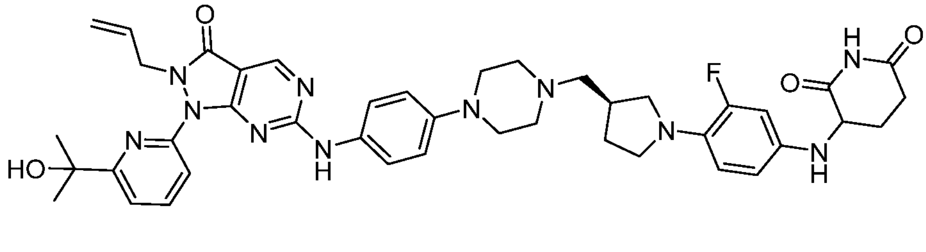

,

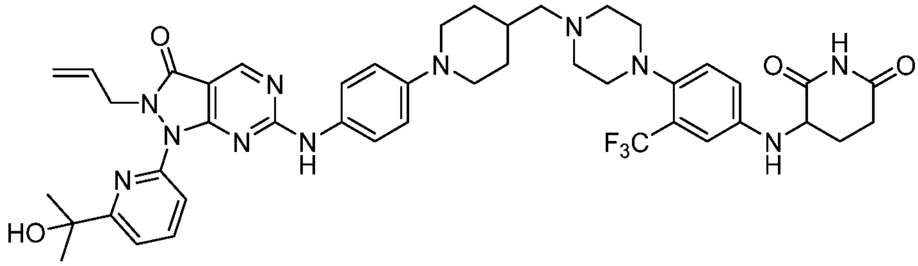

,

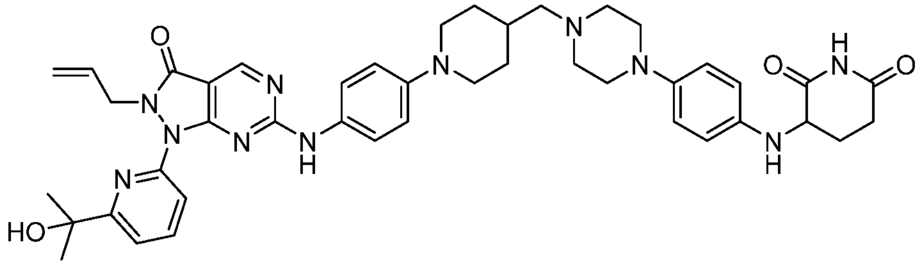

,

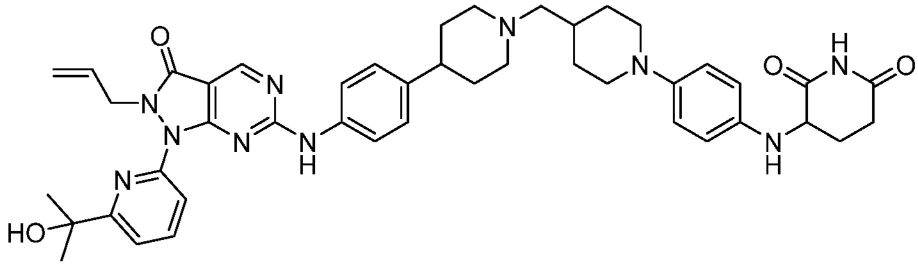

,

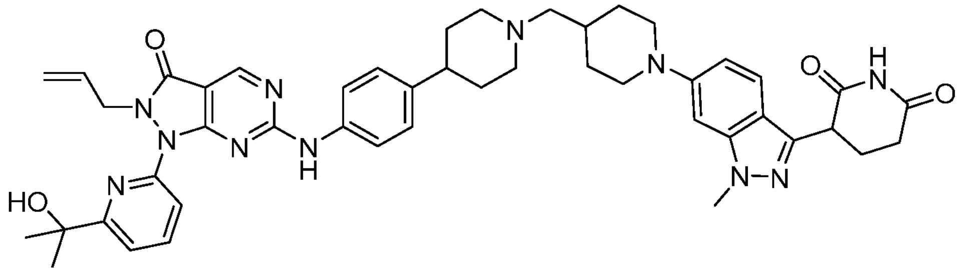

,

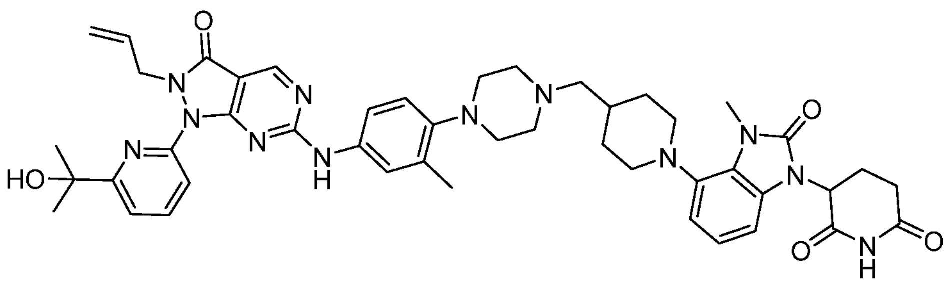

,

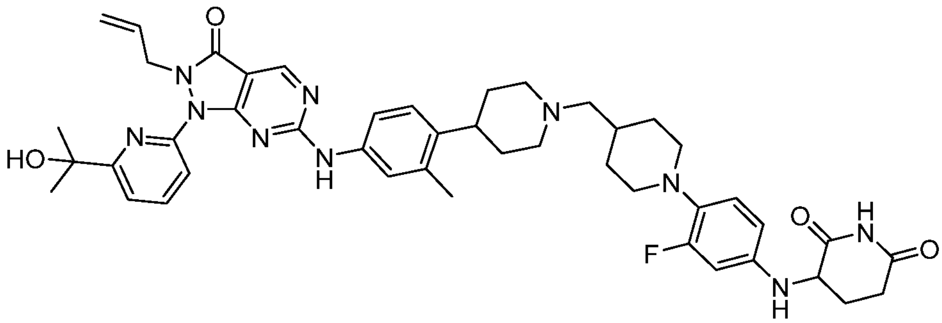

,

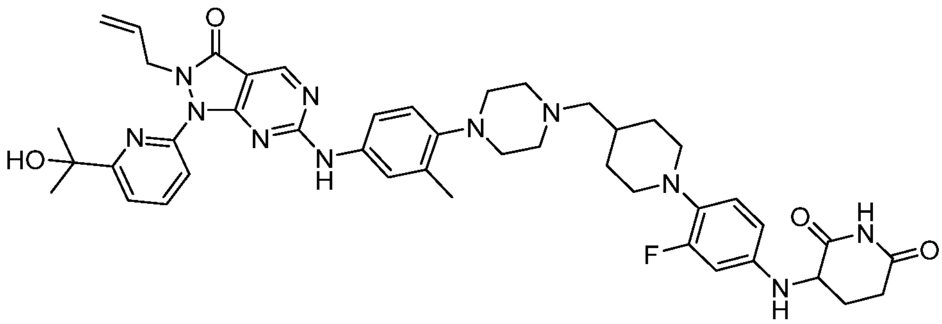

,

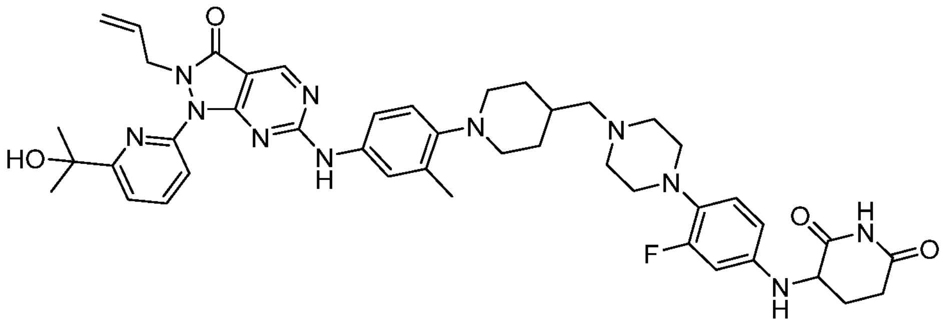

,

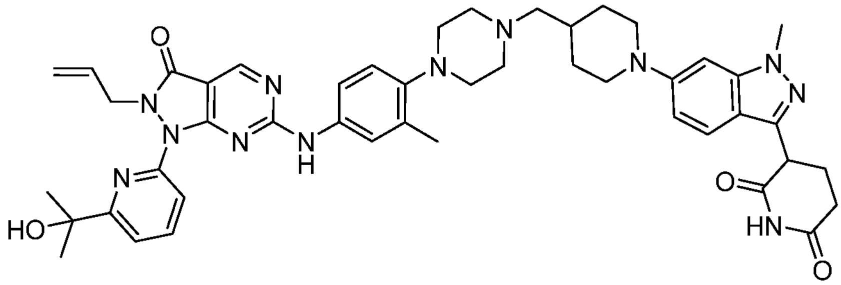

,

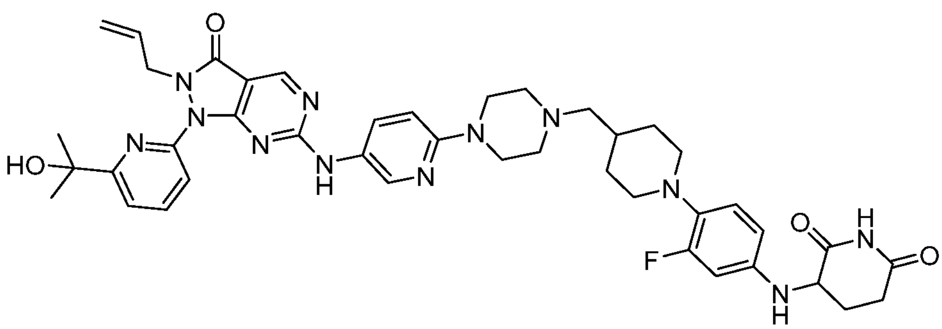

,

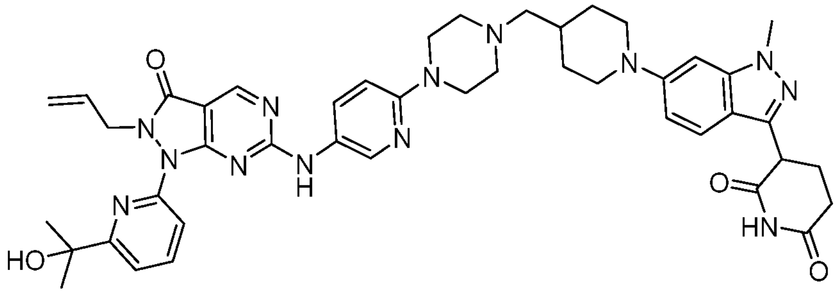

,

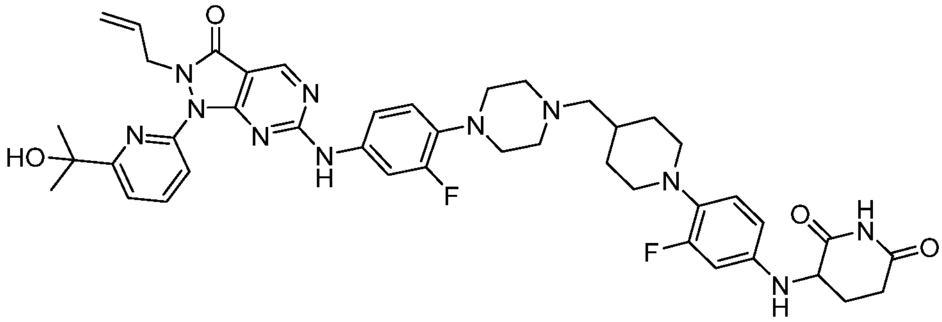

,

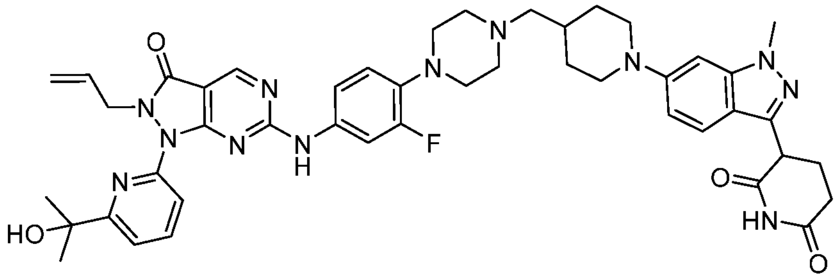

,

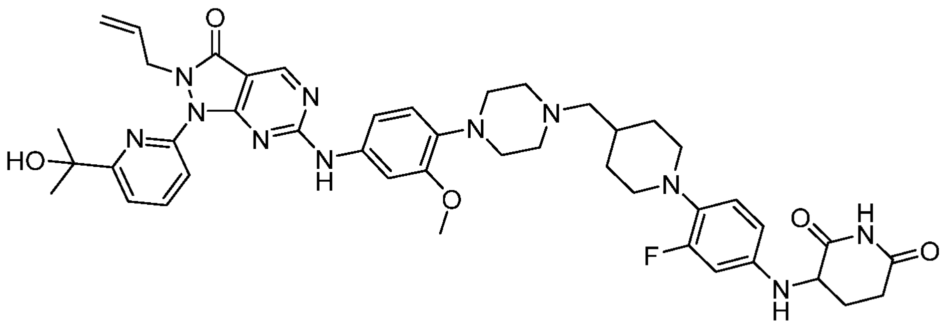

,

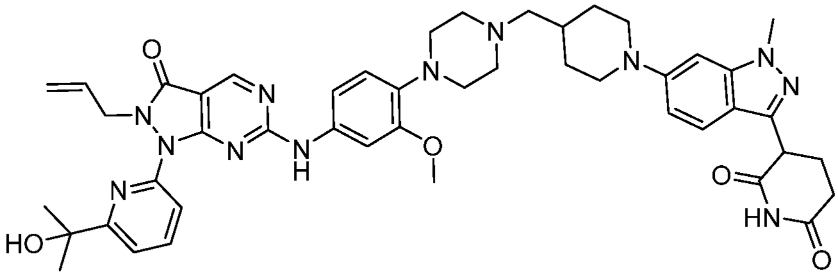

,

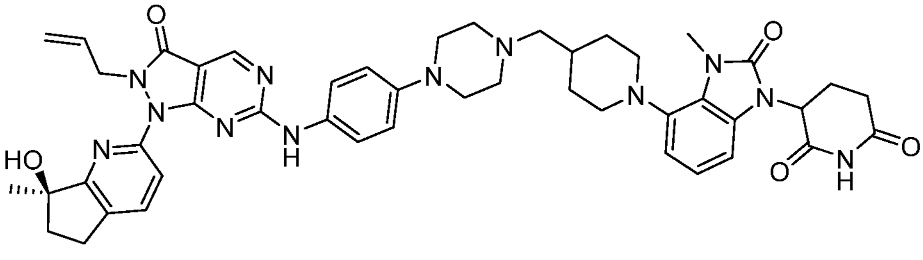

,

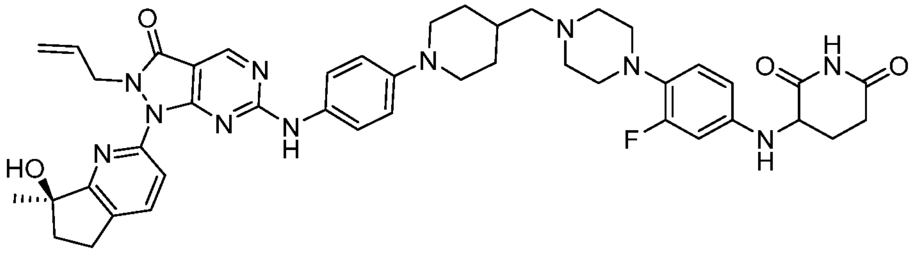

,

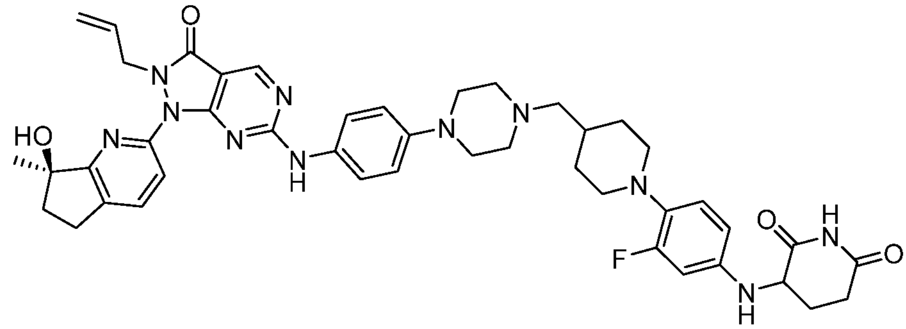

,

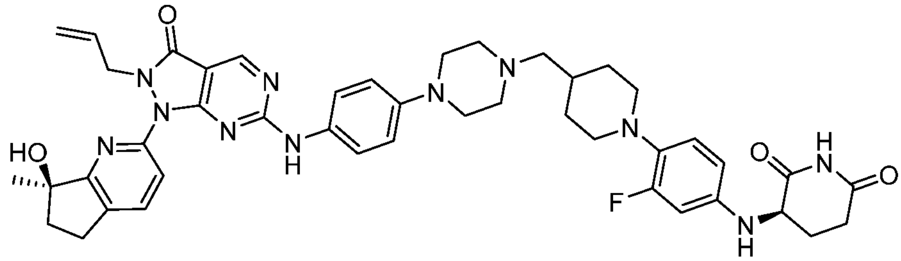

,

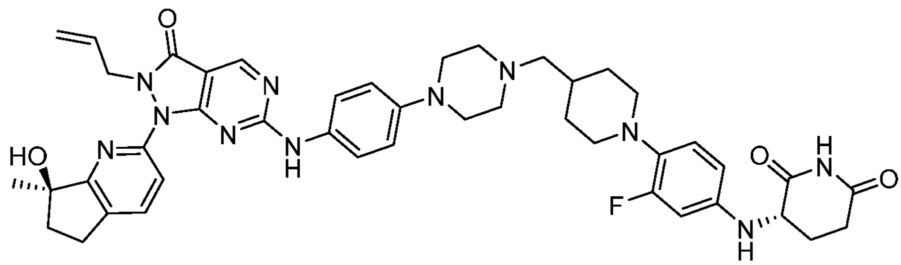

,

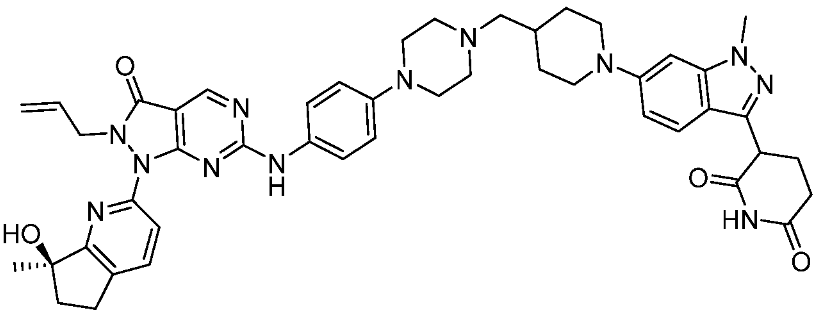

,

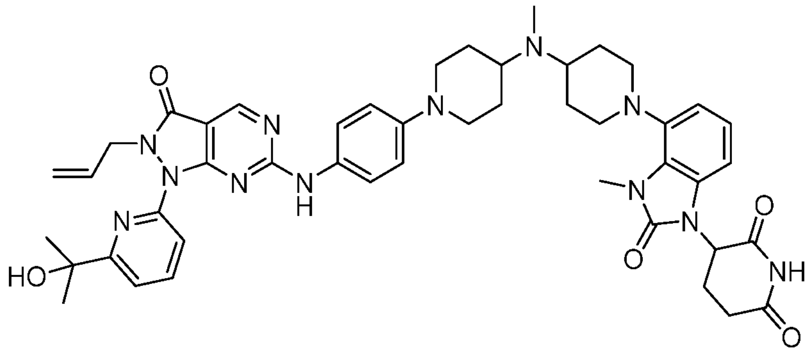

,

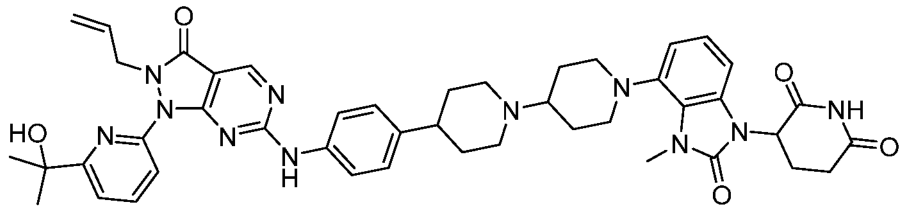

,

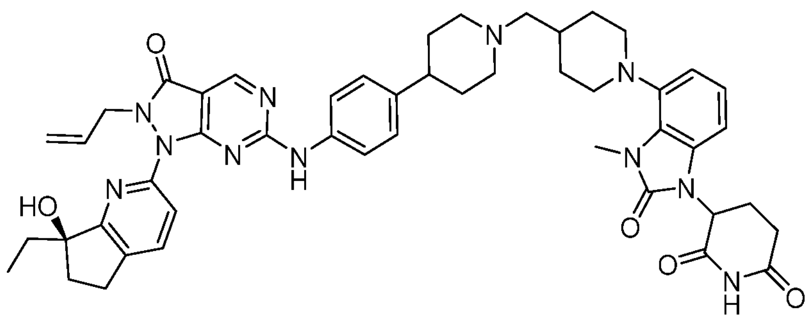

,

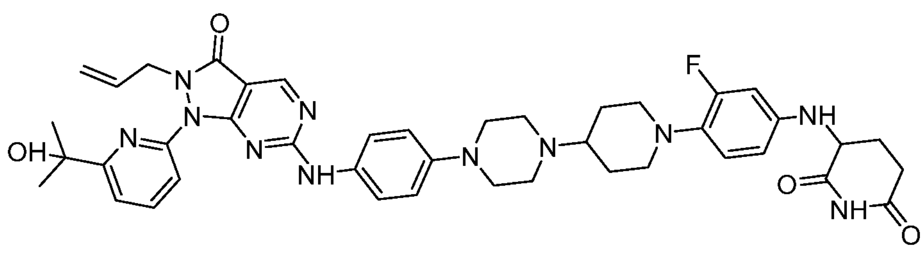

,

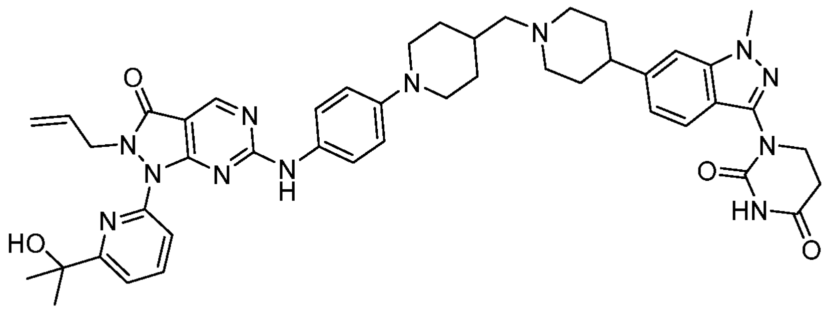

,

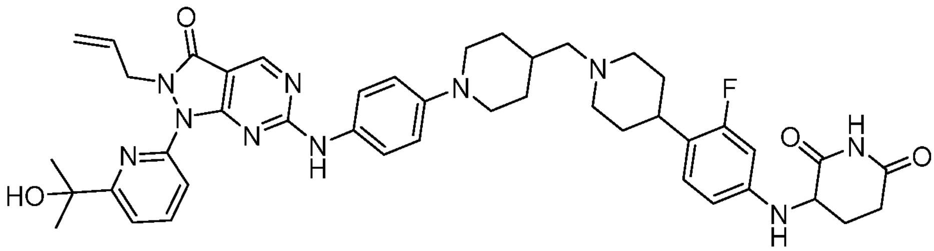

,

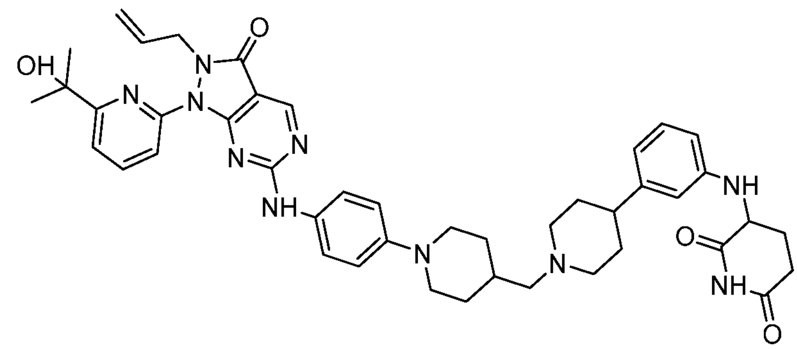

,

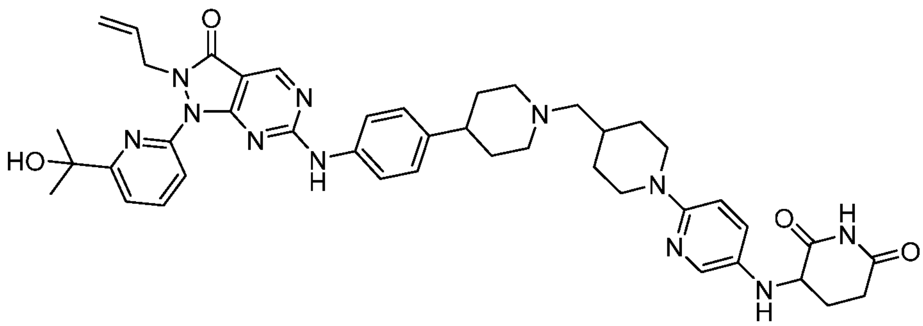

,

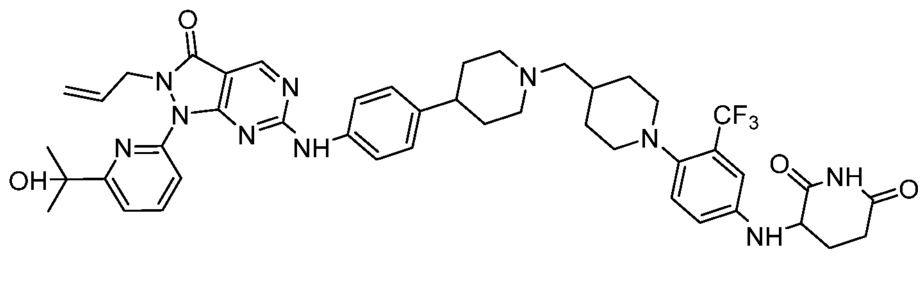

,

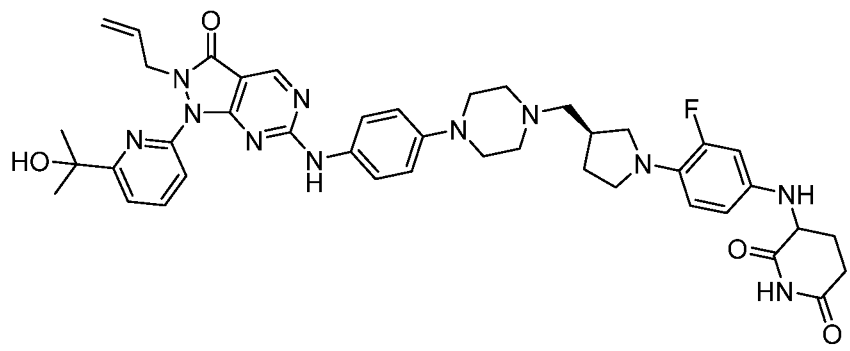
,

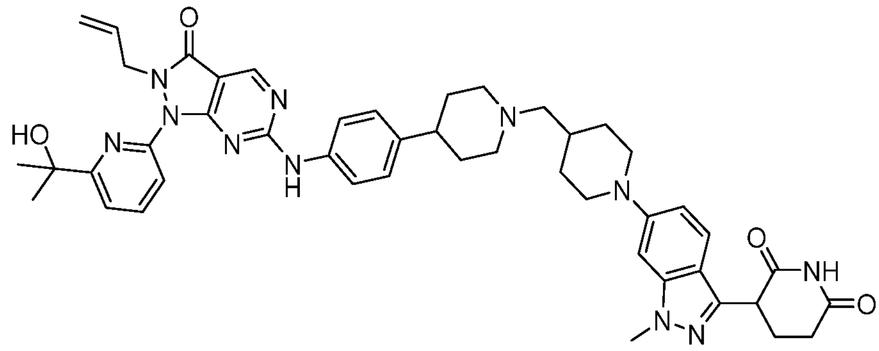
,

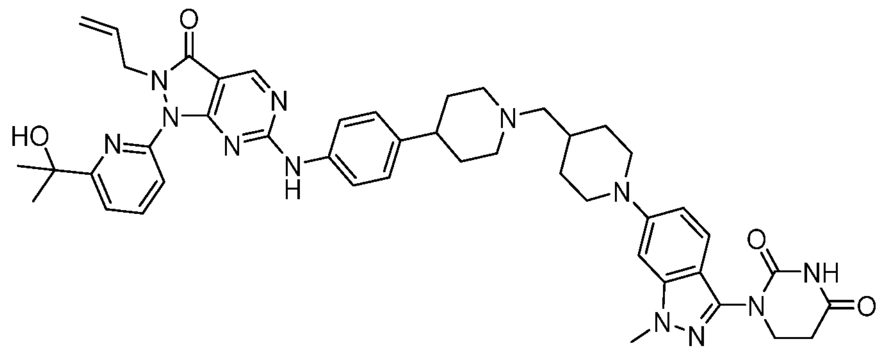
,

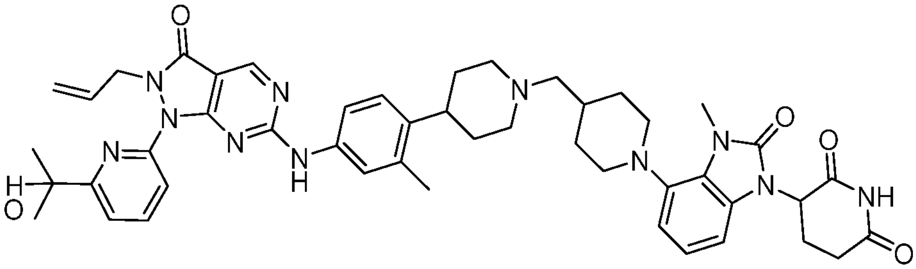
,

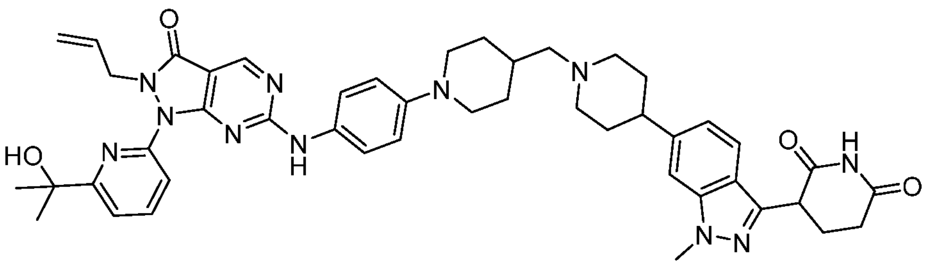
,

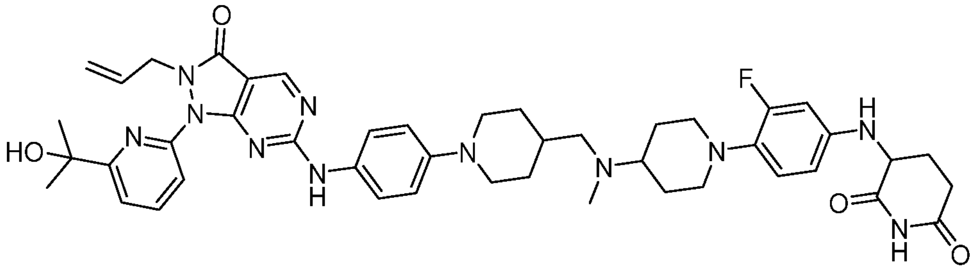

,

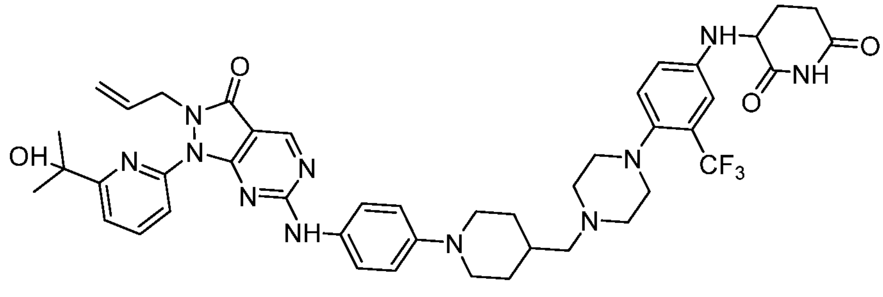

,

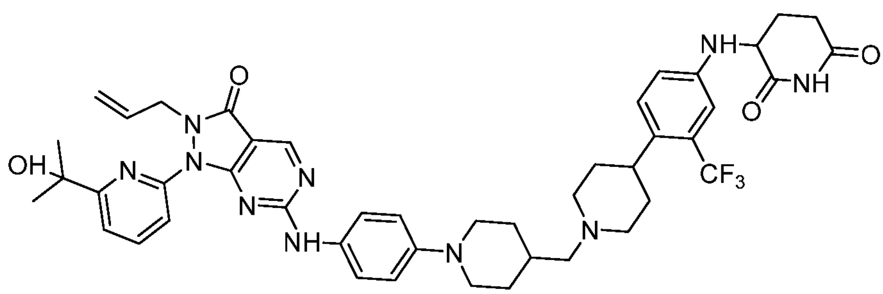

,

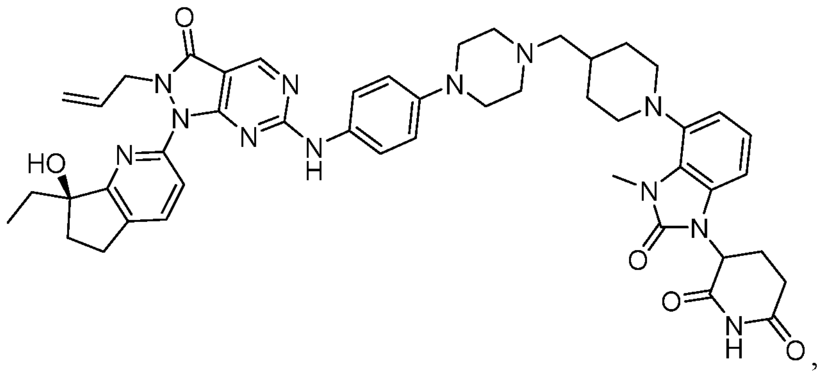

,

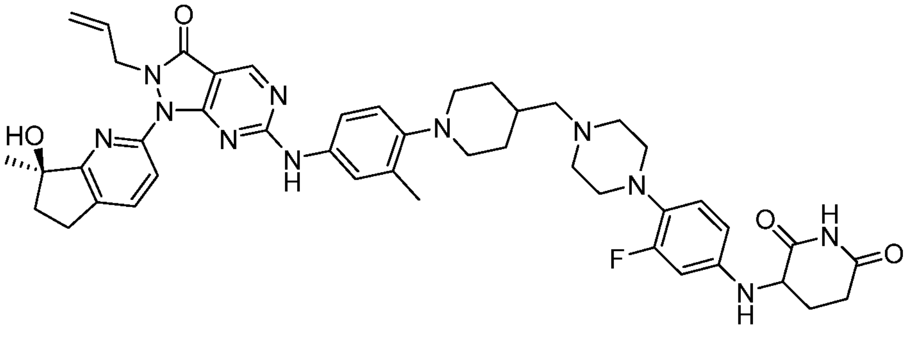

,

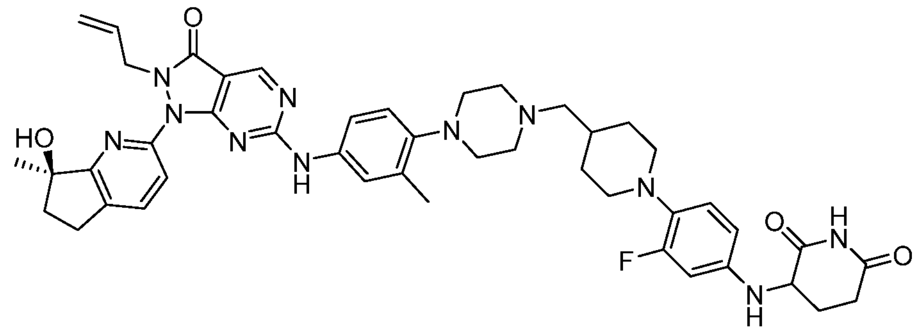

,

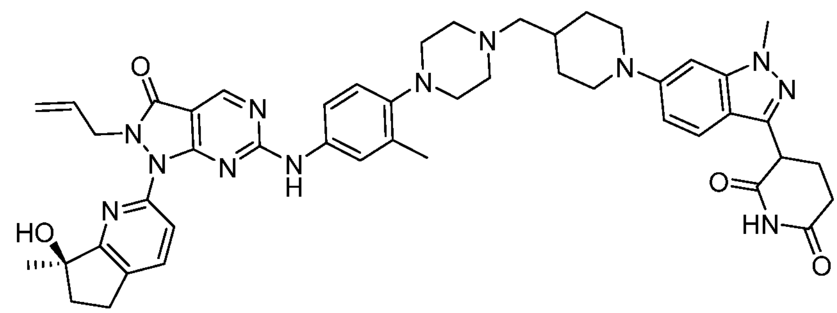

,

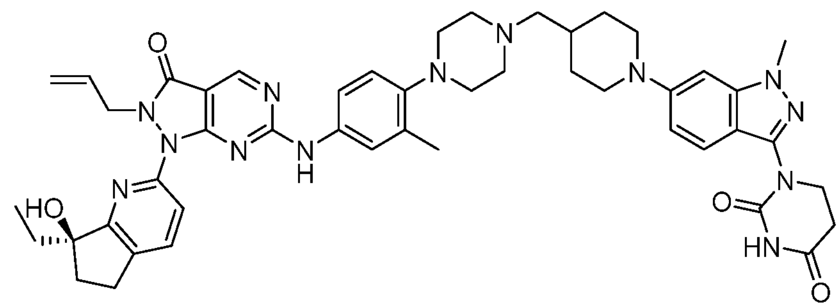

,

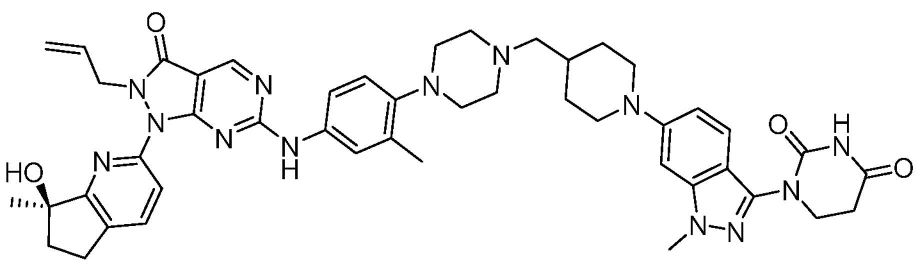

,

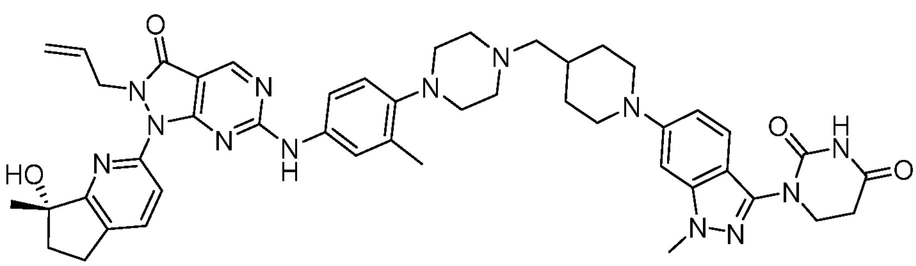

,

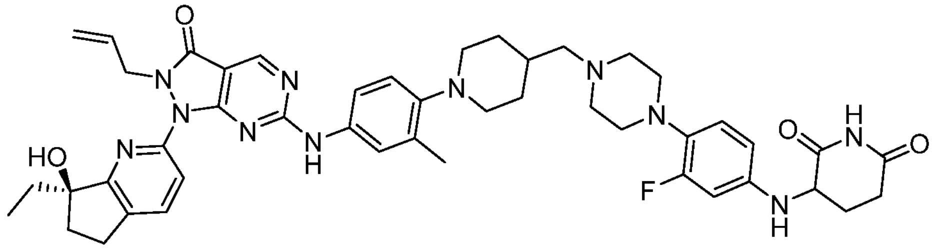

,

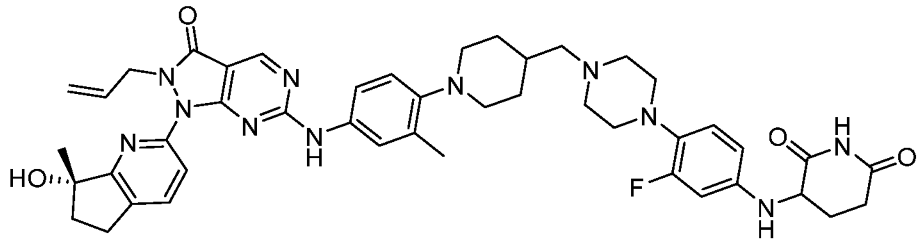

,

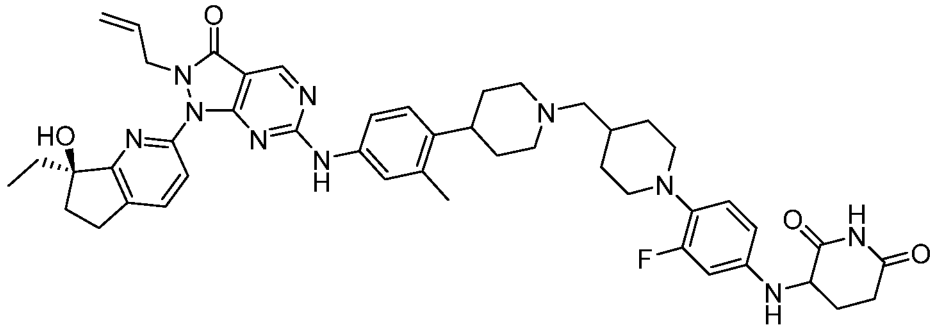

,

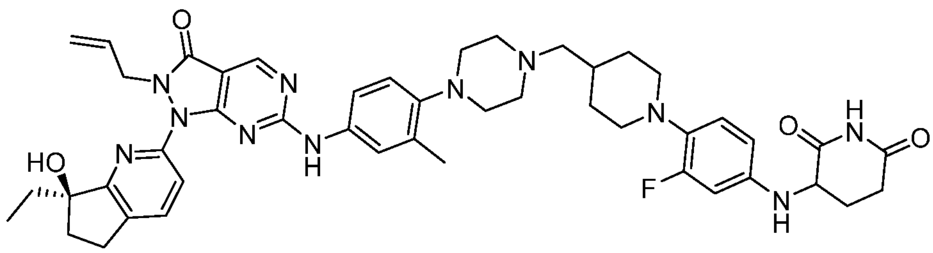

,

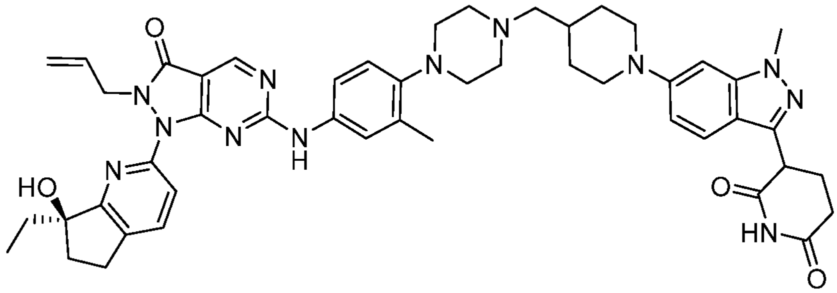

,

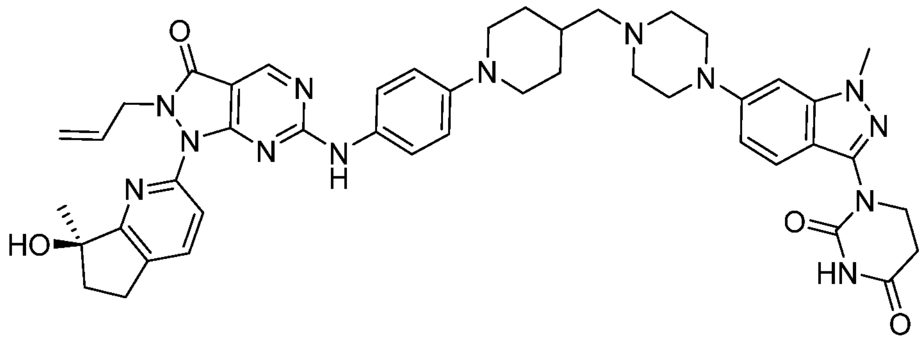
,

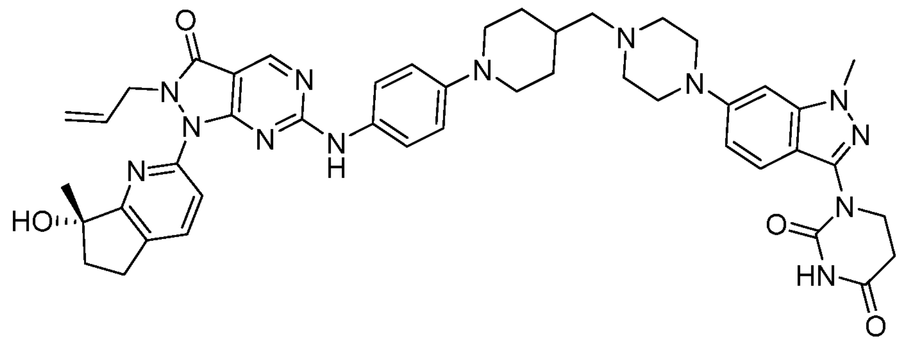
,

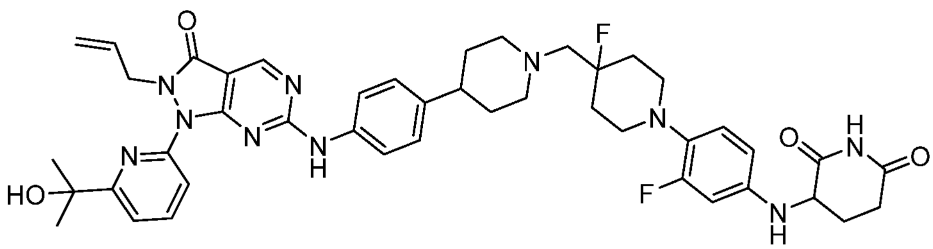
,

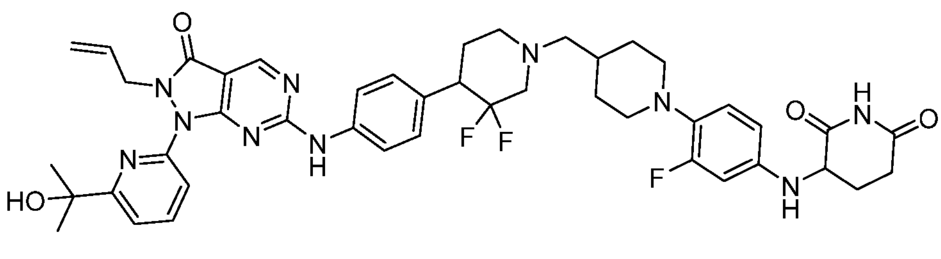
,

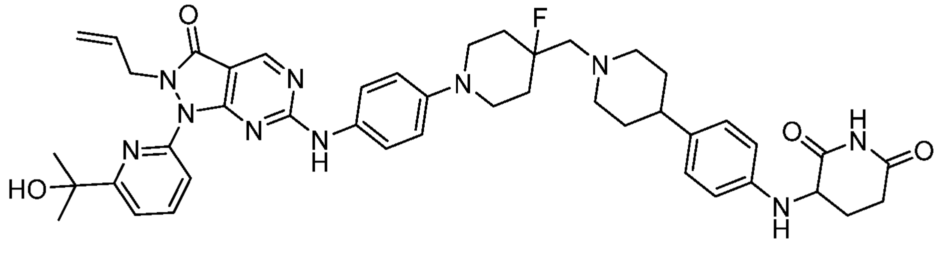
,

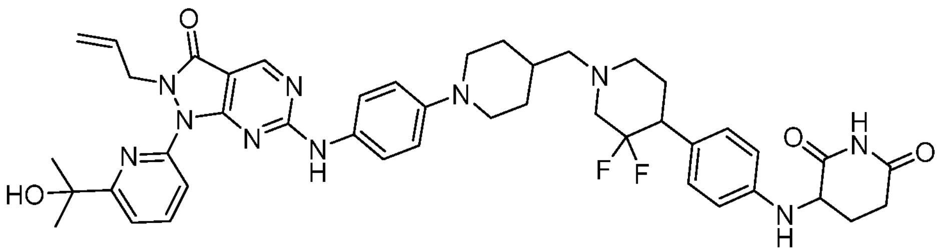

,

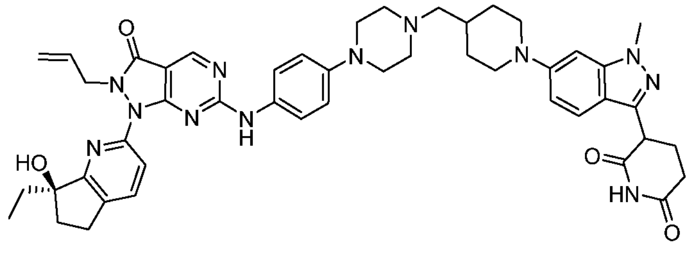

,

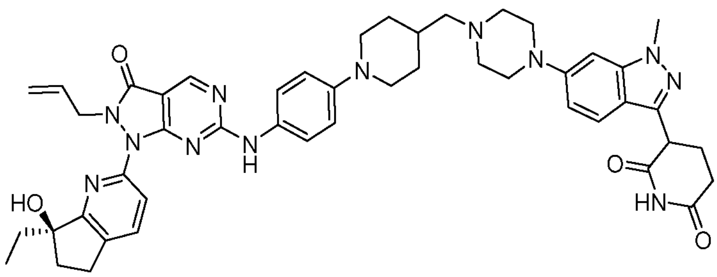

,

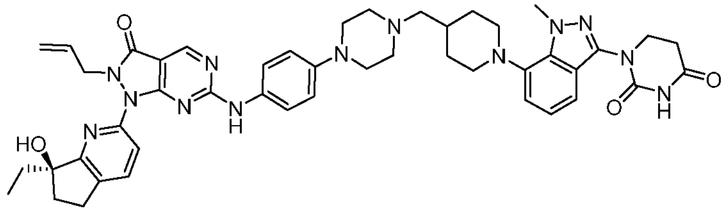

,

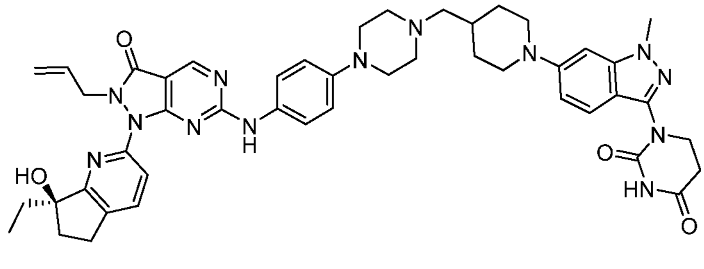

,

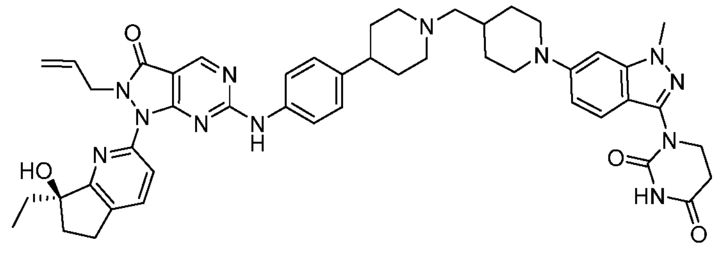,

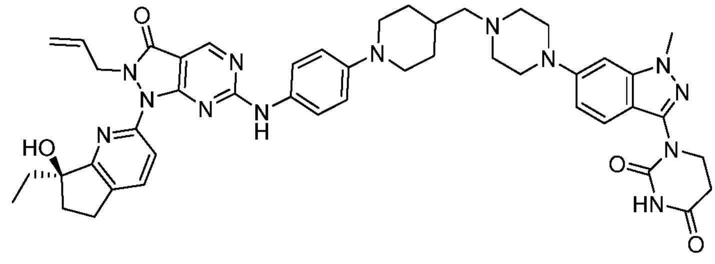,

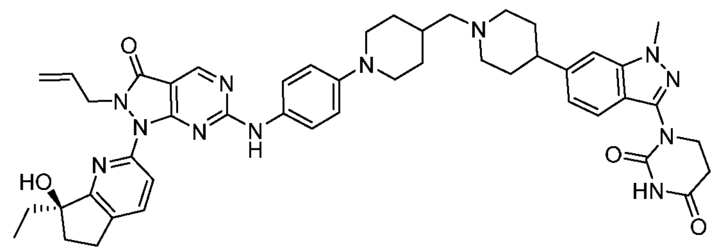,

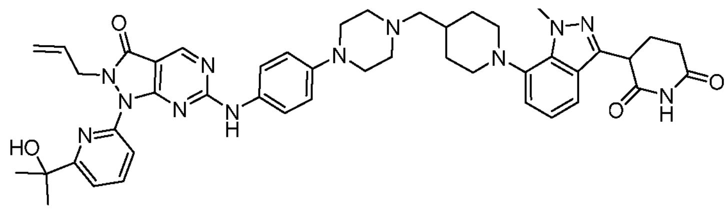,

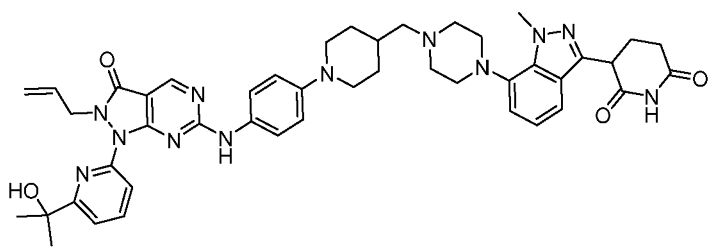,

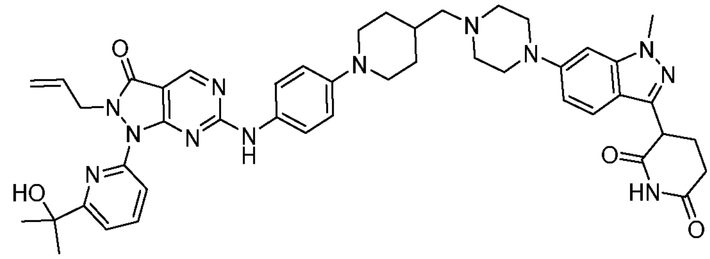,

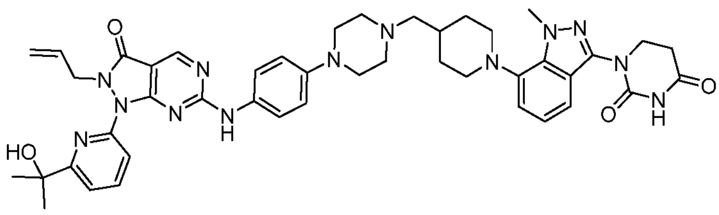

,

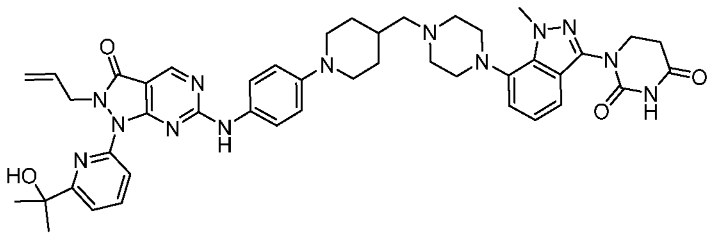

,

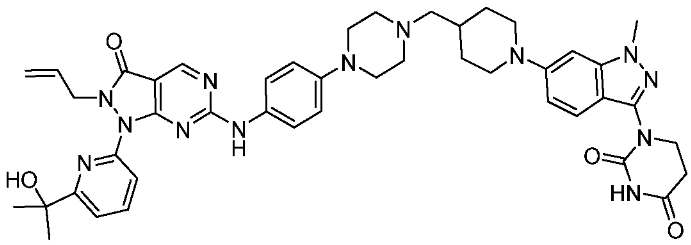

,

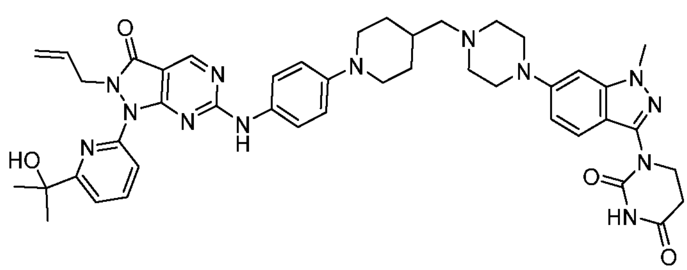

,

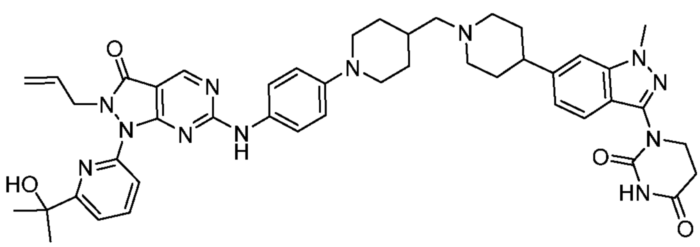

,

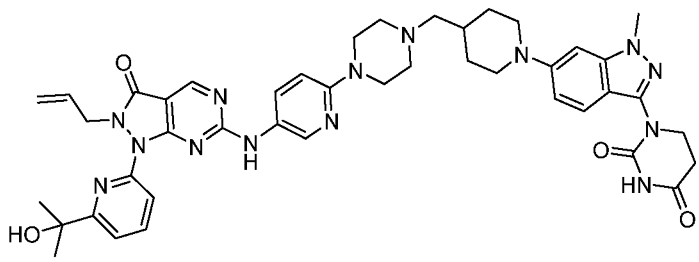

,

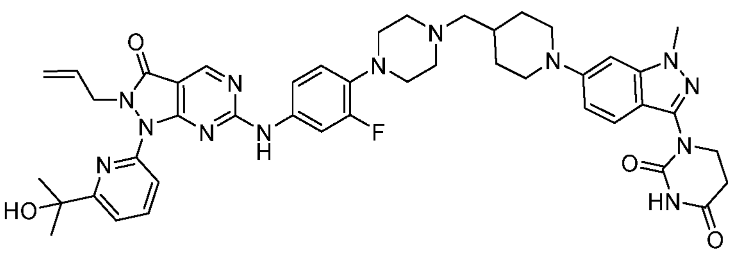,

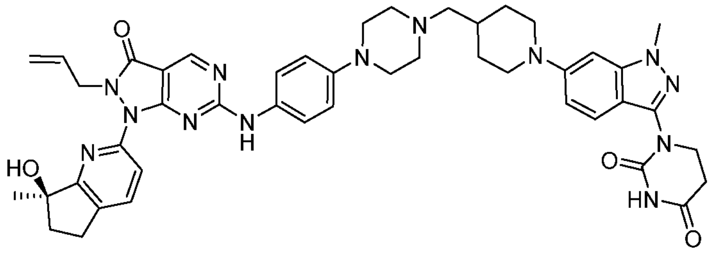,

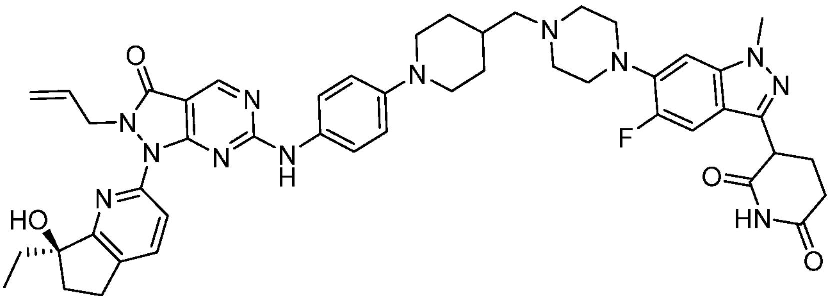,

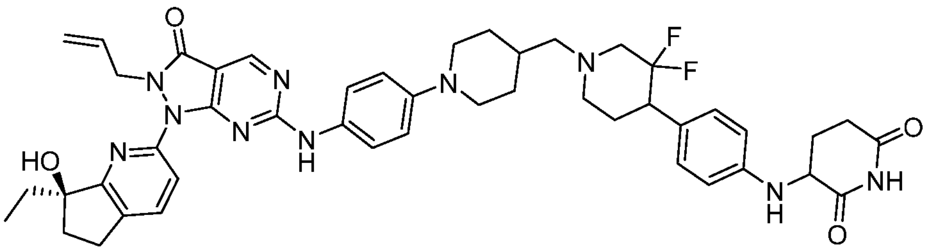,

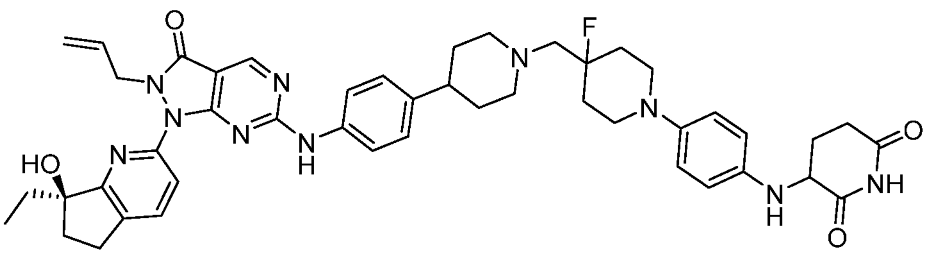,

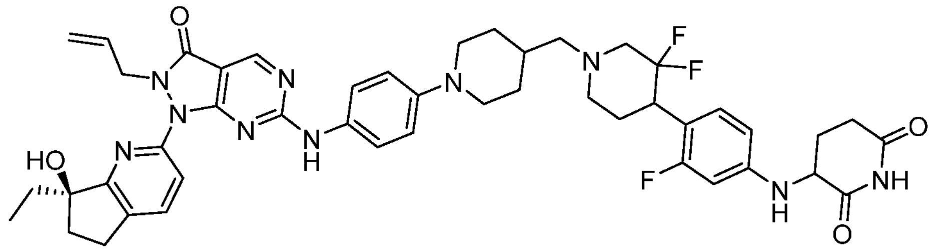

,

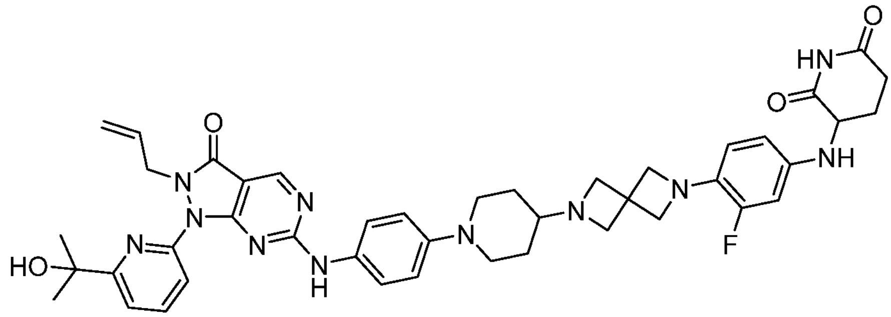

,

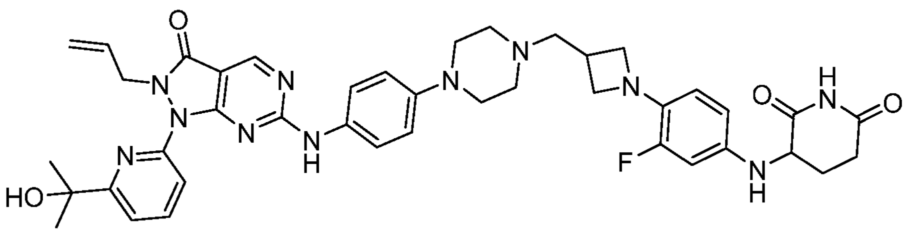

,

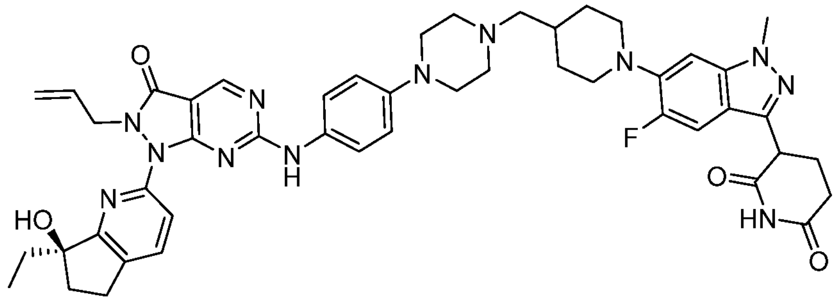

,

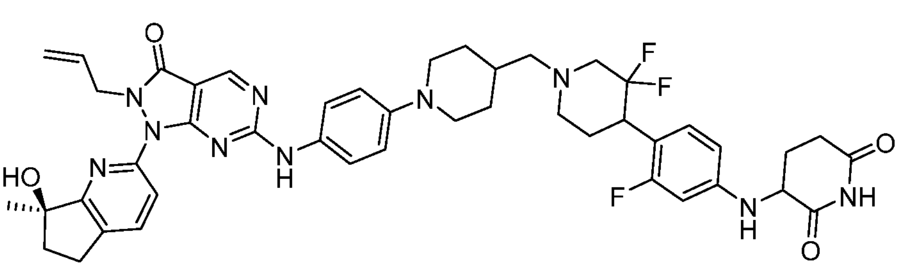

,

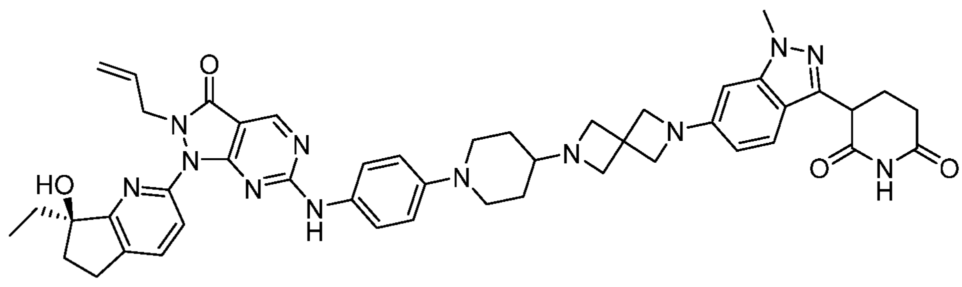

,

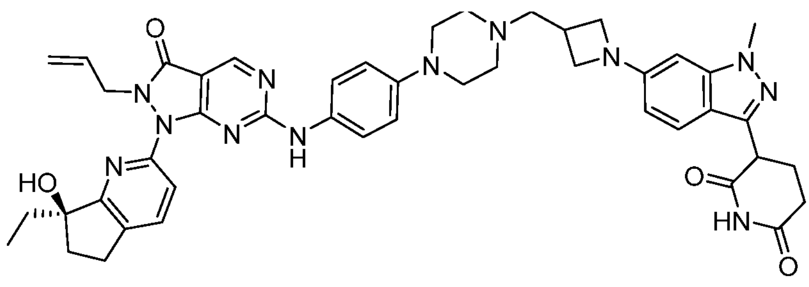

,

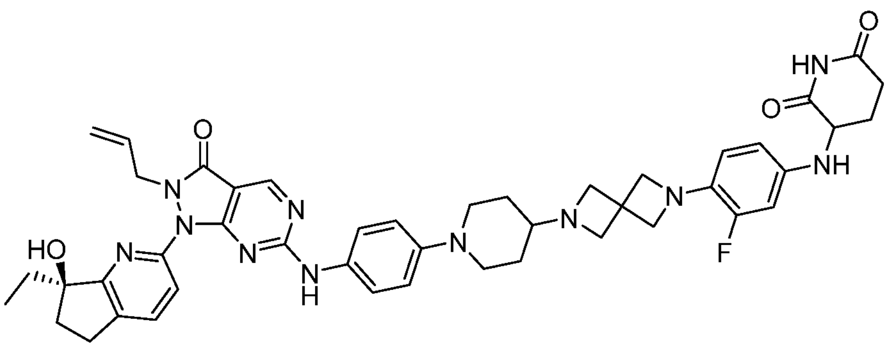

,

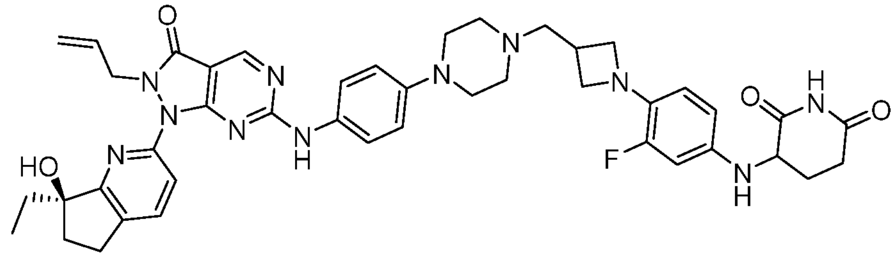

,

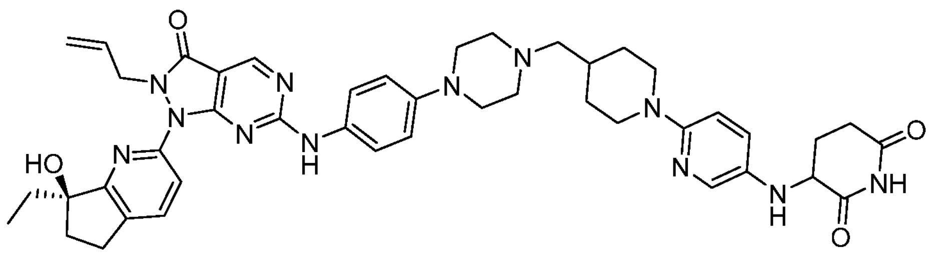

,

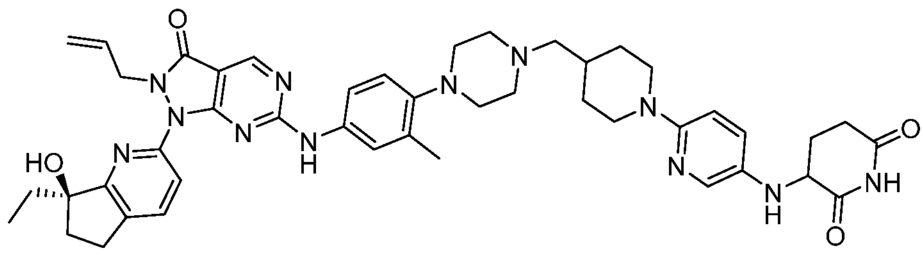

,

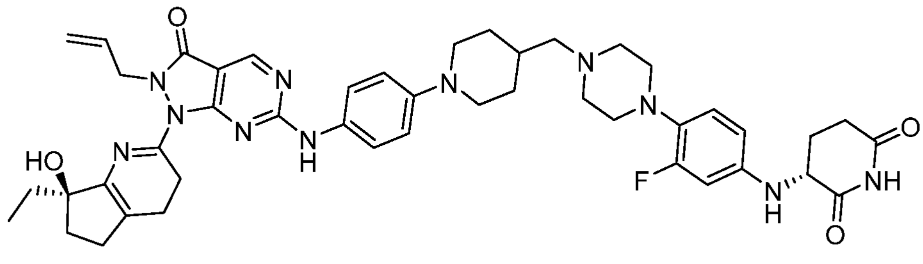

,

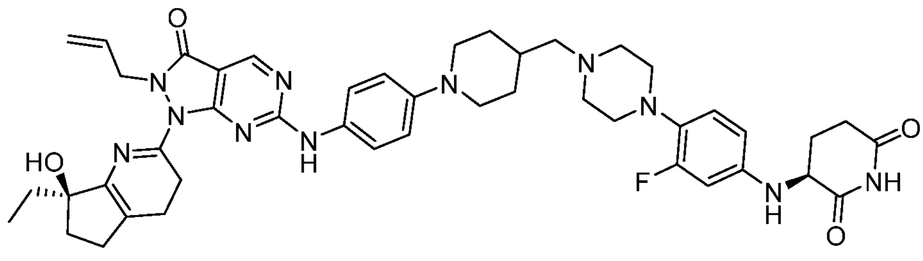

,

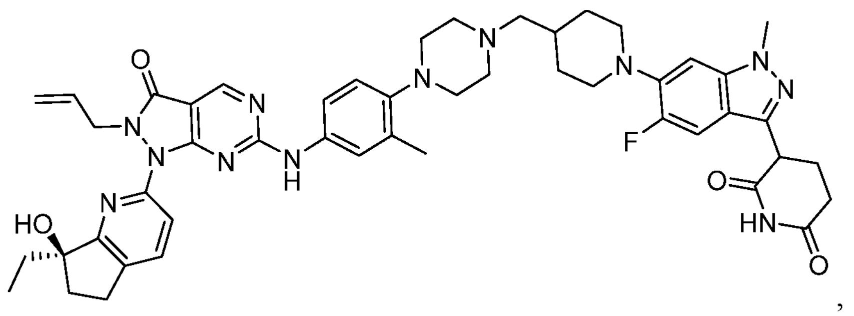

,

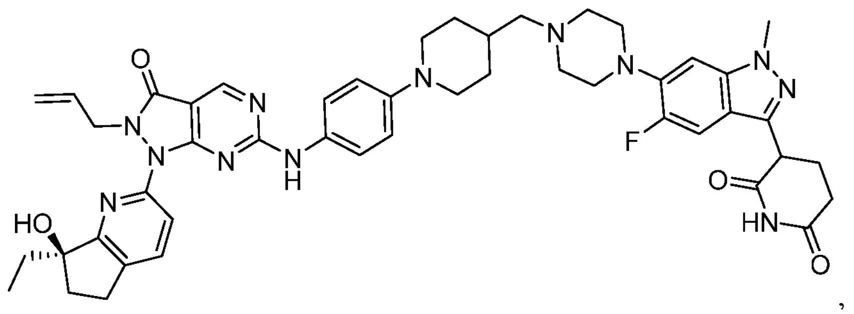

,

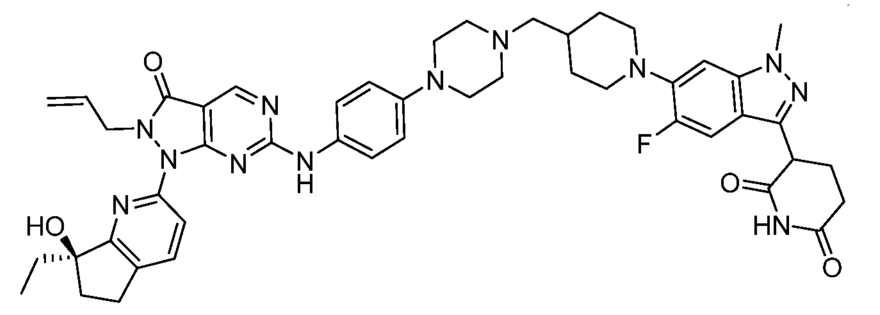

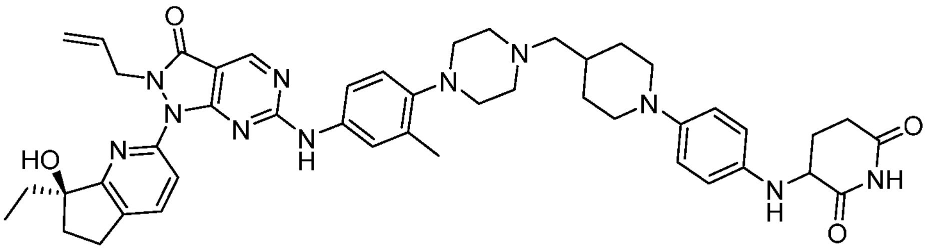

,

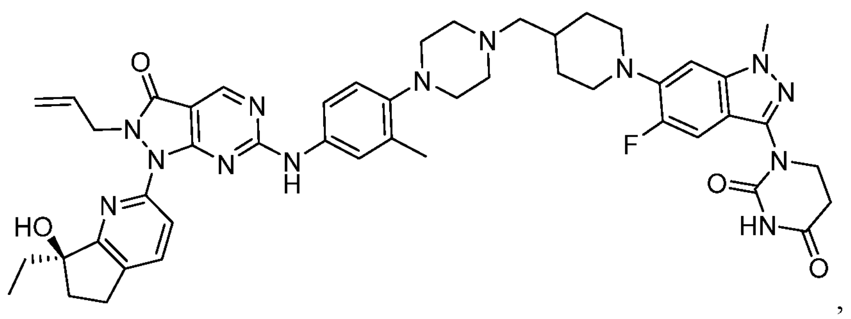

,

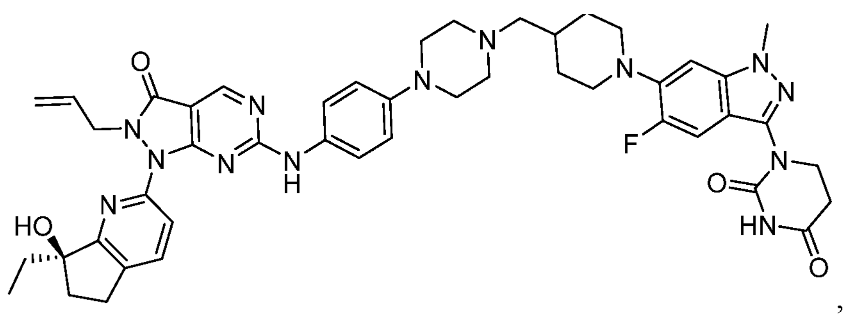

,

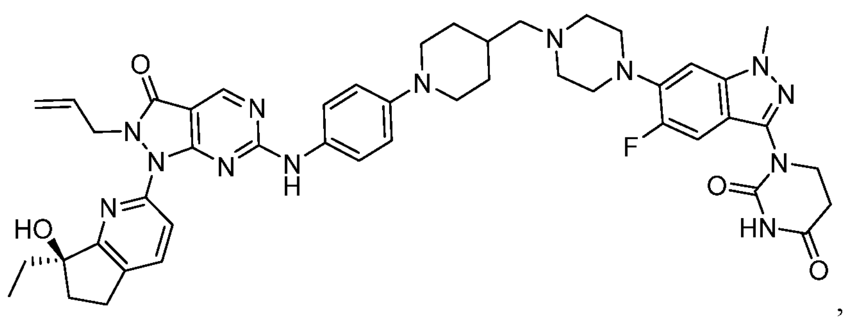

,

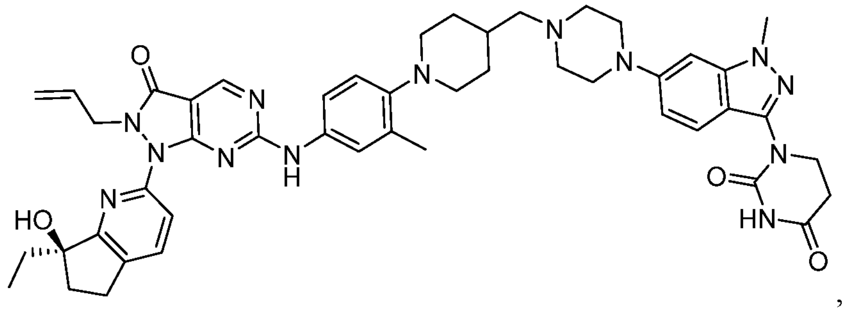

,

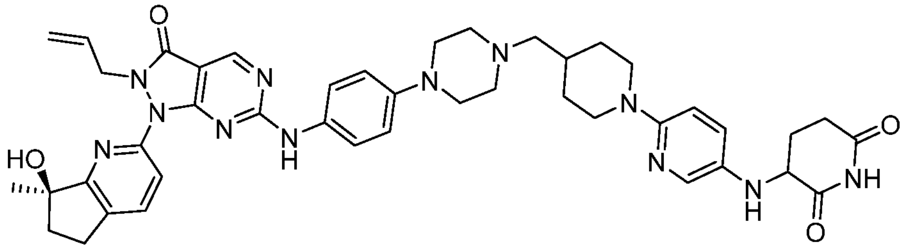

,

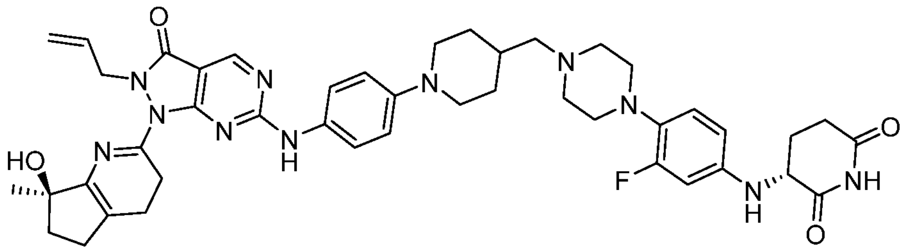

,

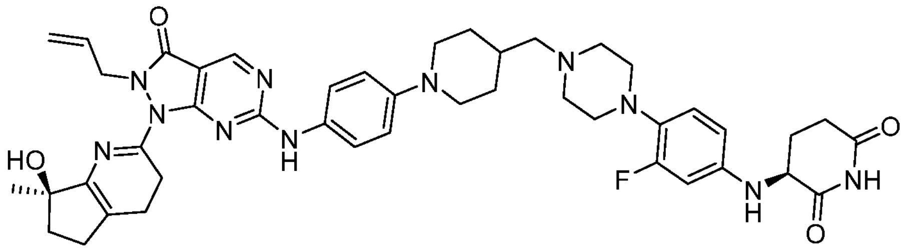

,

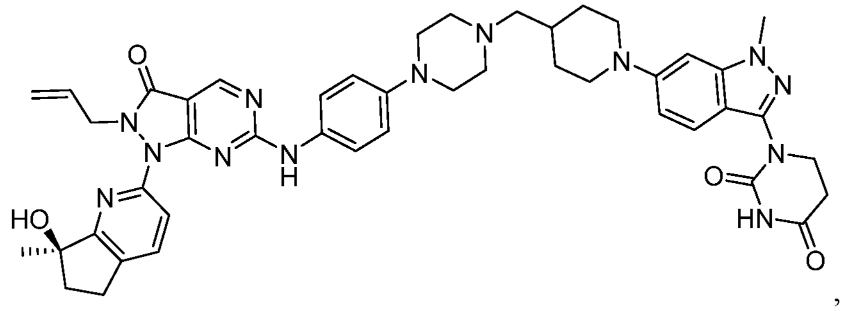

,

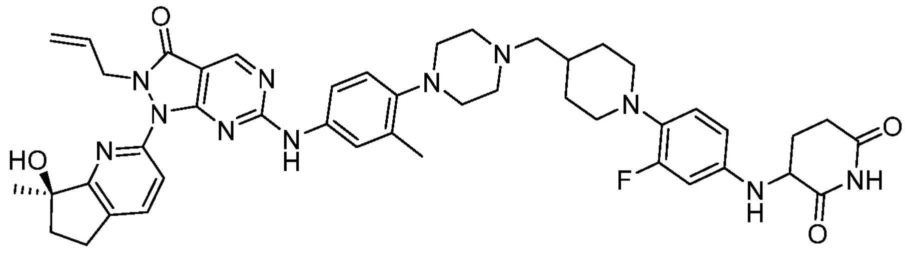
,

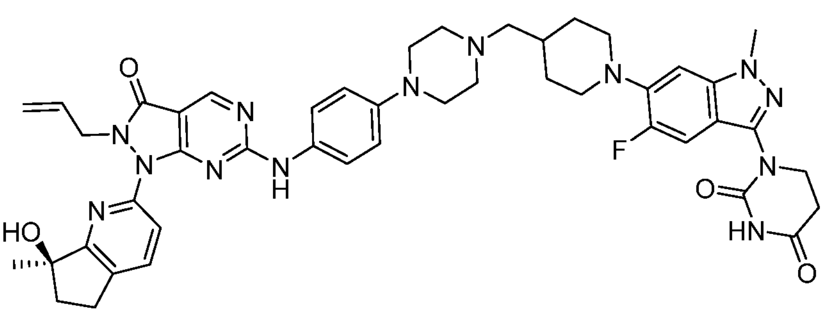
,

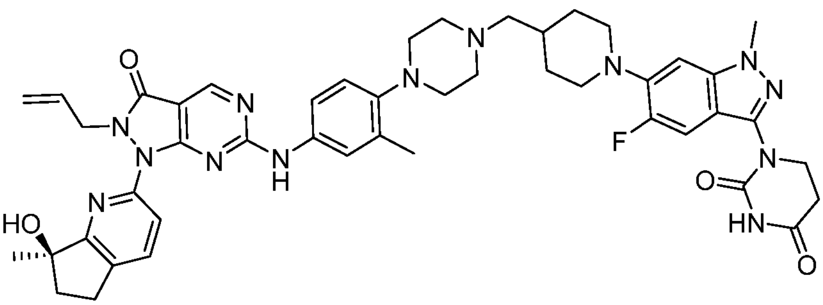
,

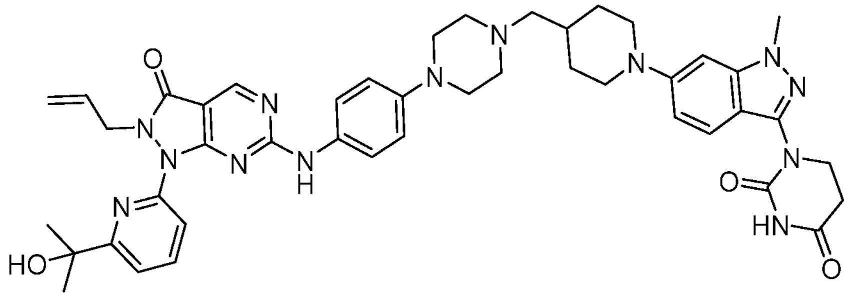
,

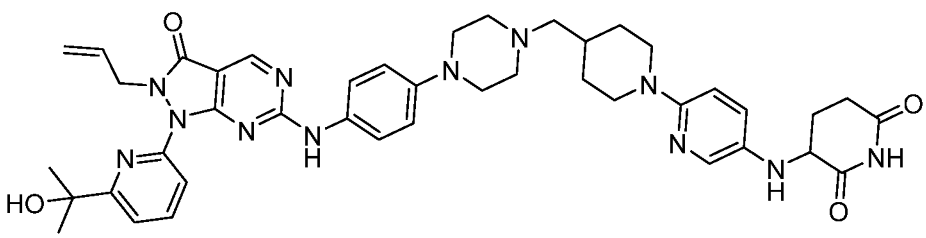
,

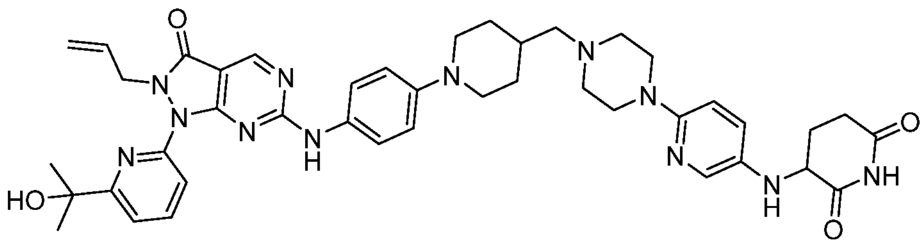

,

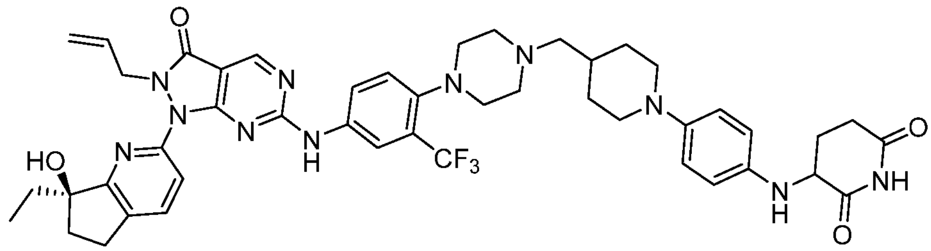

,

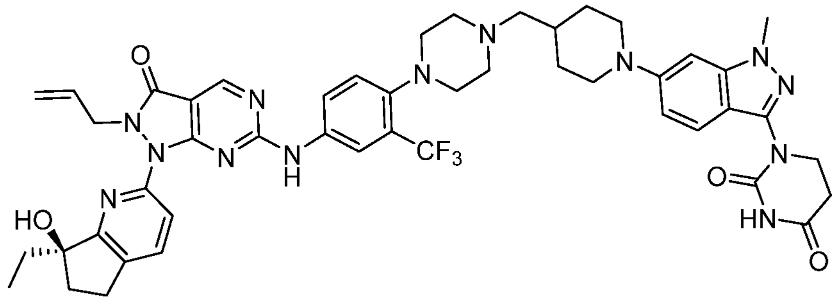

,

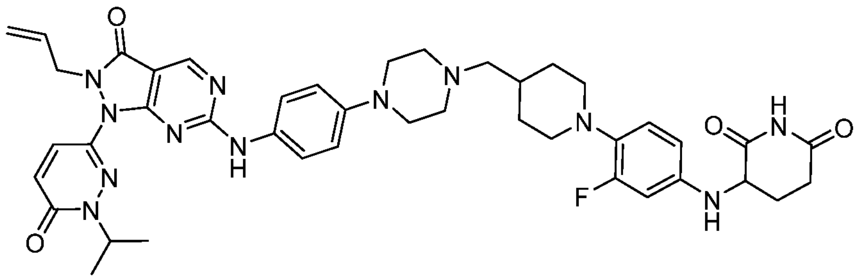

,

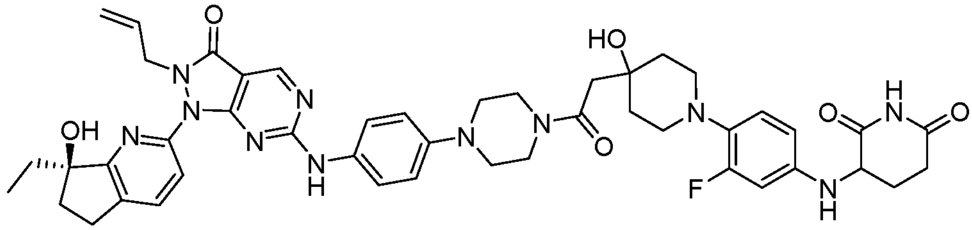

,

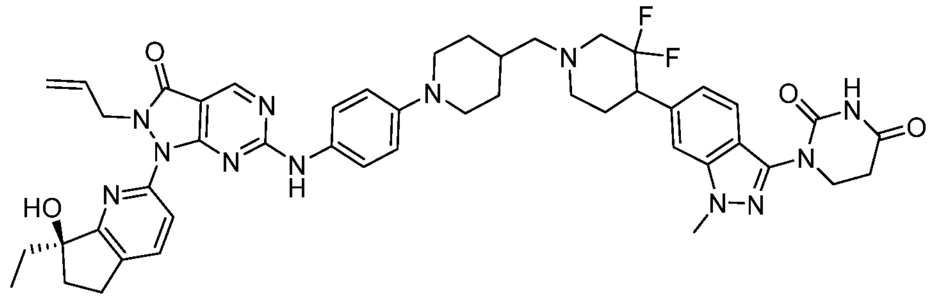

,

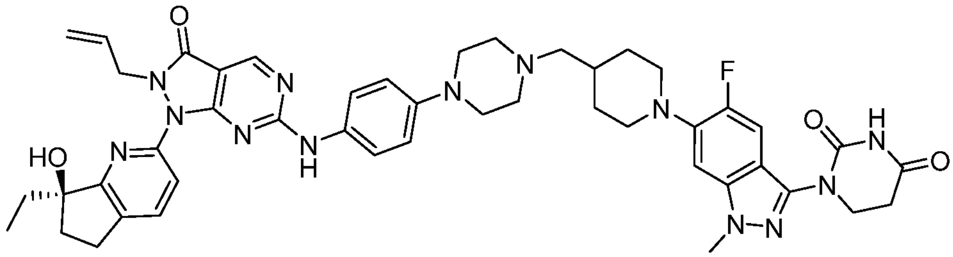

,

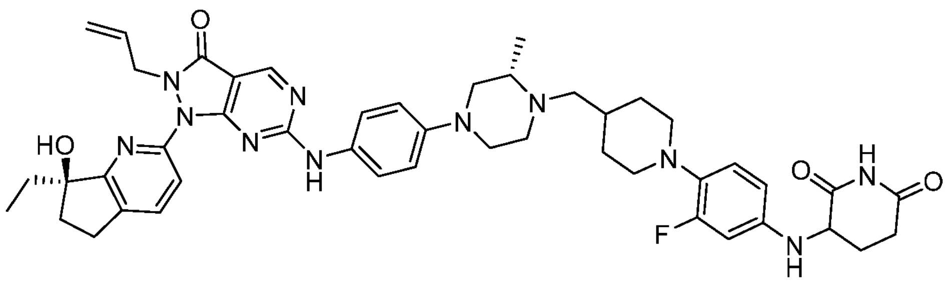

,

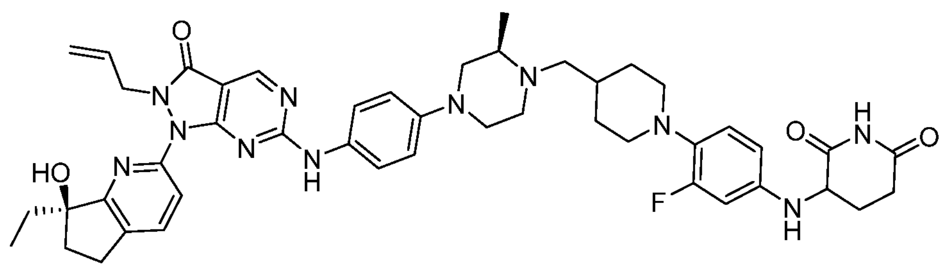

,

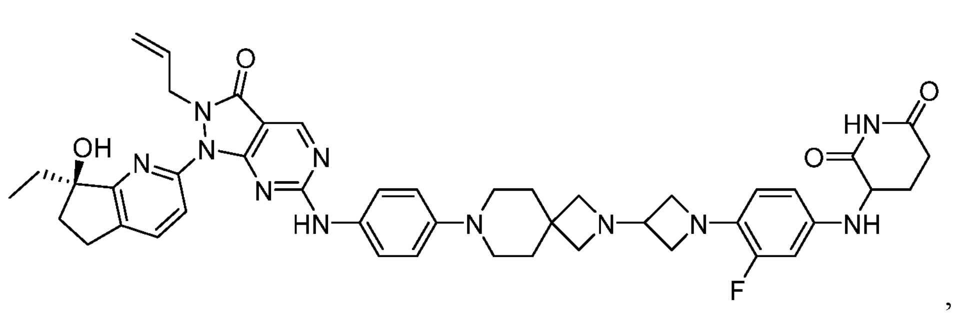

,

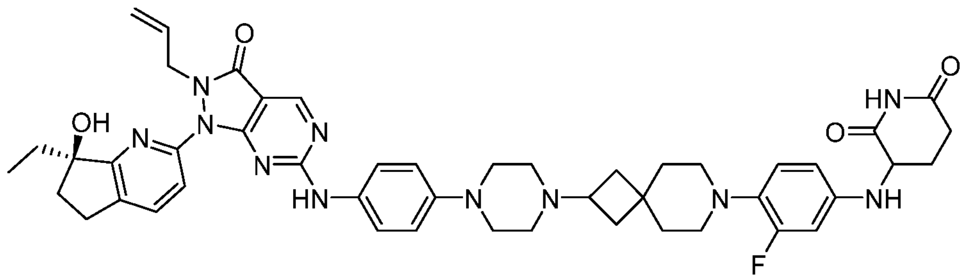

,

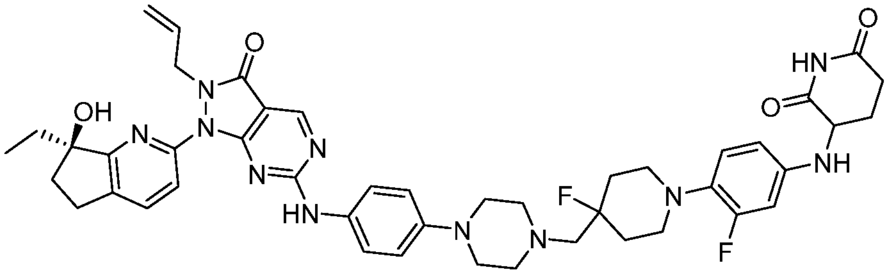

,

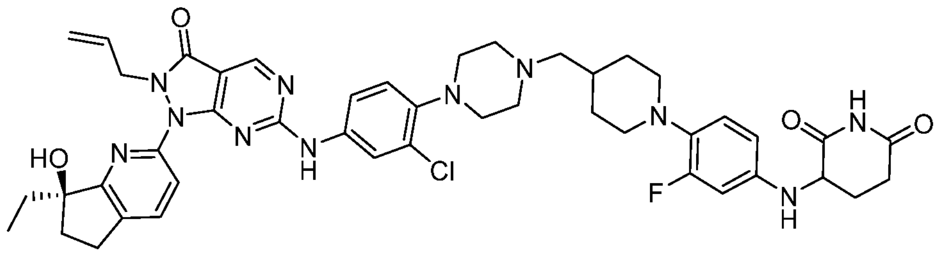

,

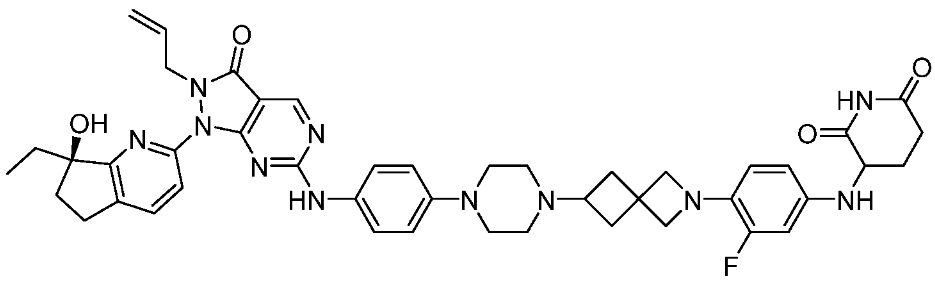

,

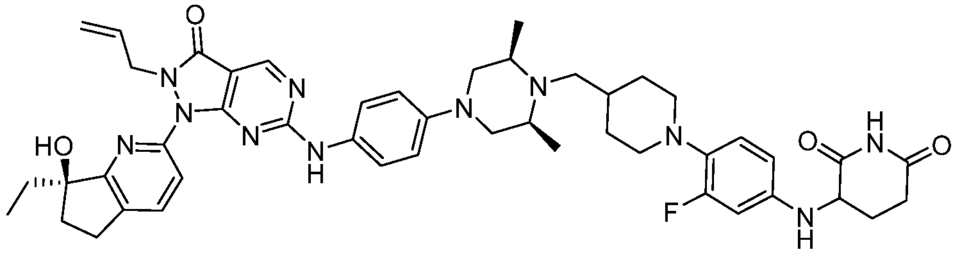

,

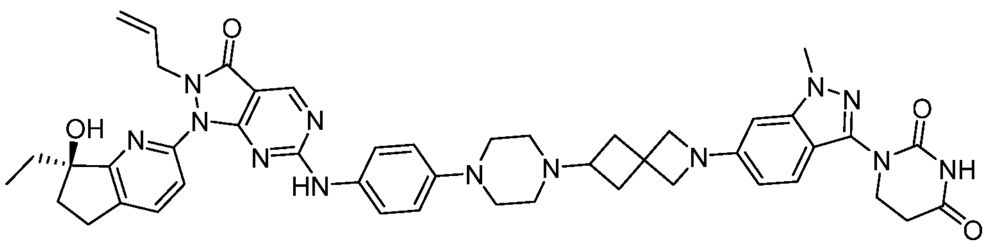,

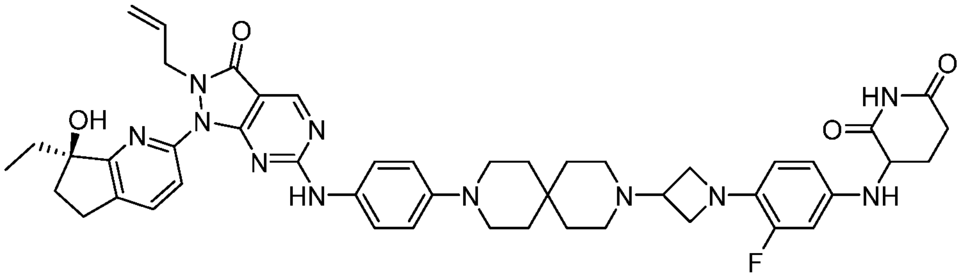,

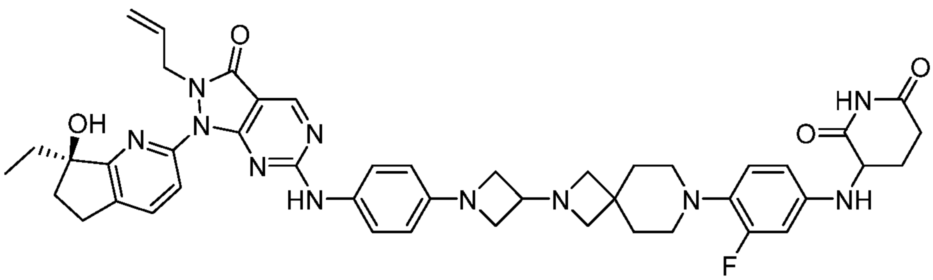,

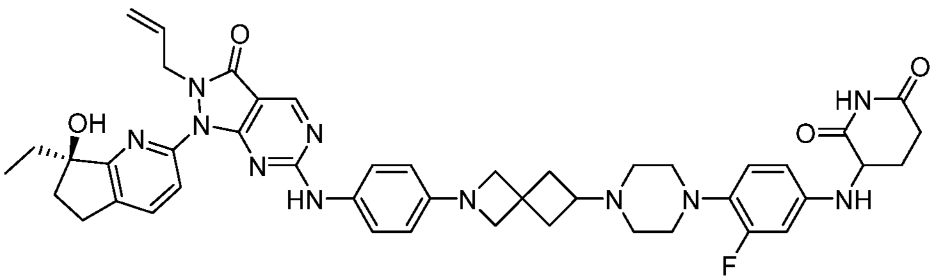,

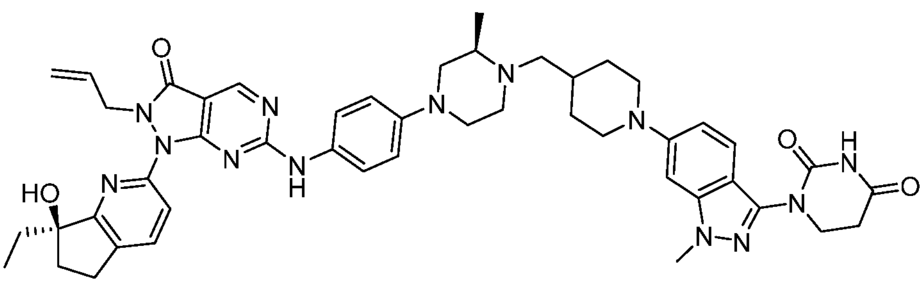,

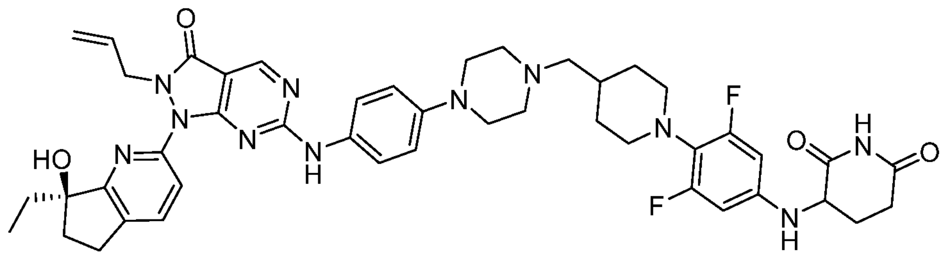

,

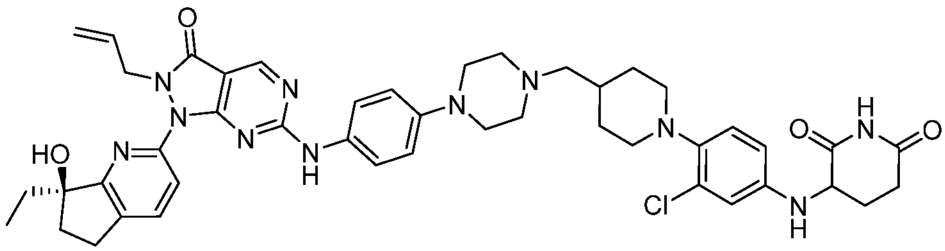

,

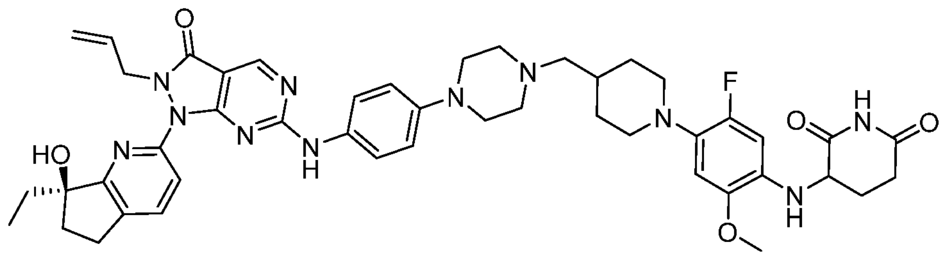

,

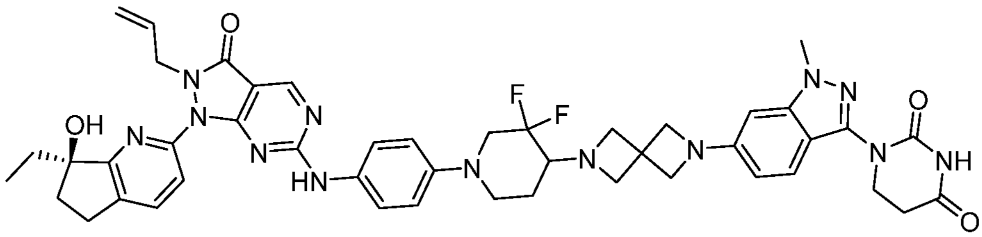

,

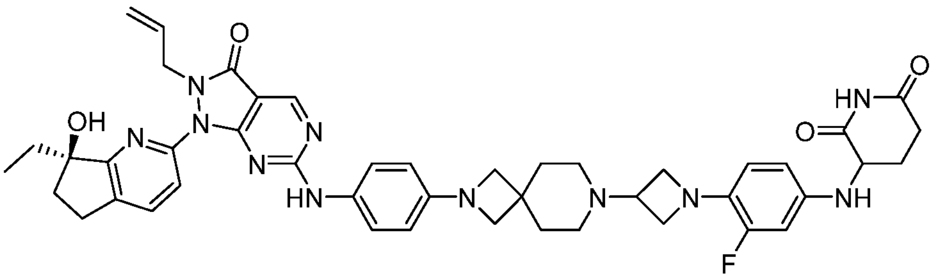

,

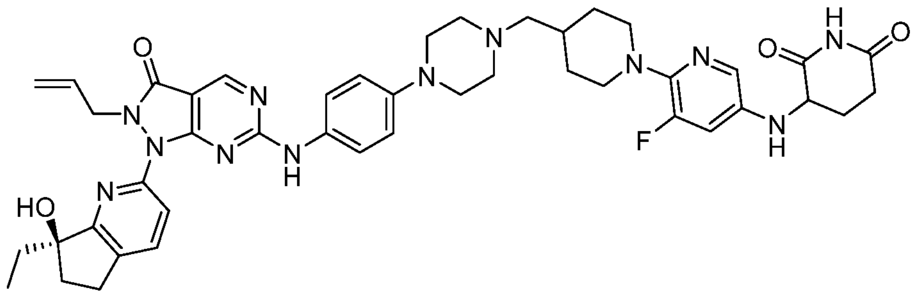

,

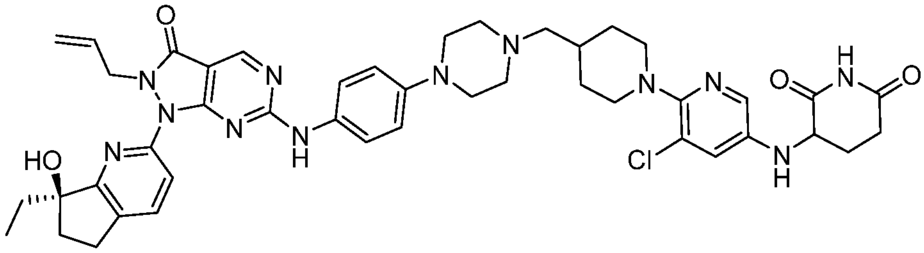

,

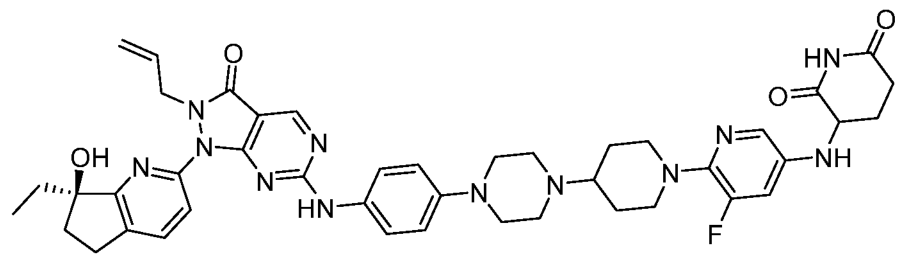

,

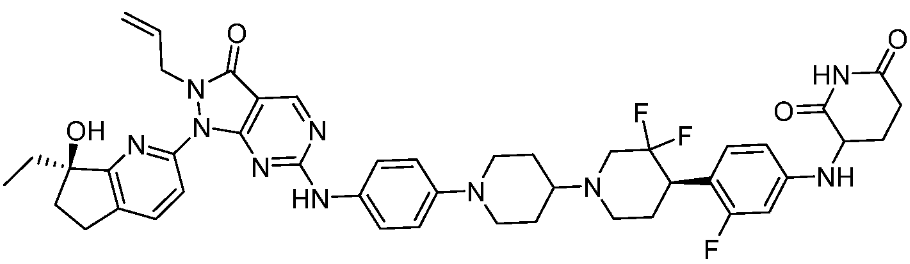

,

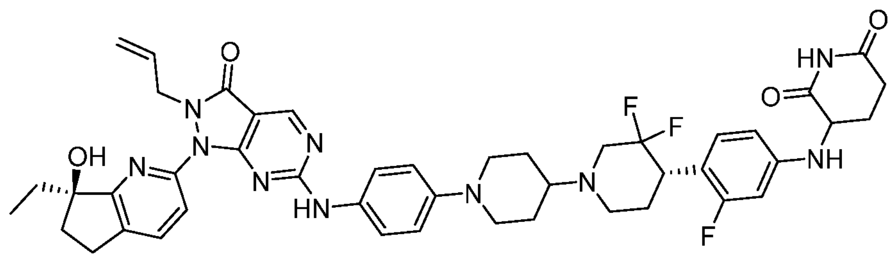

,

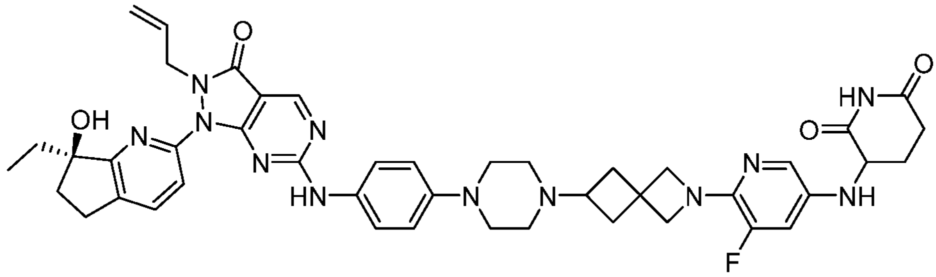

,

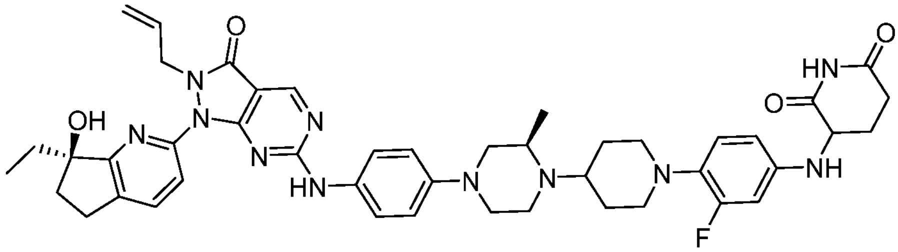

,

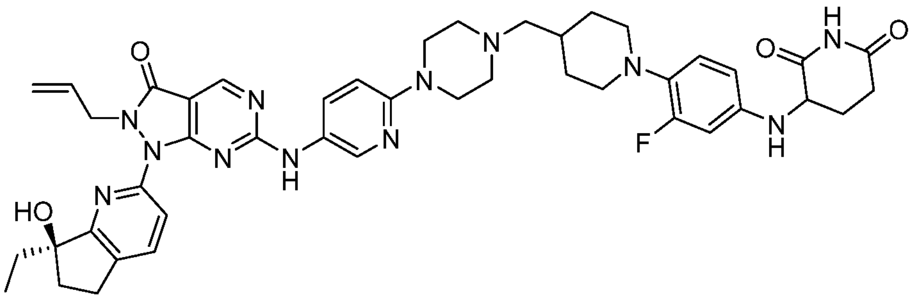

,

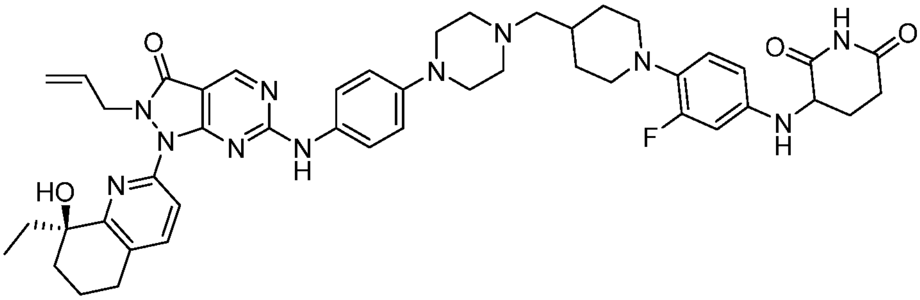

,

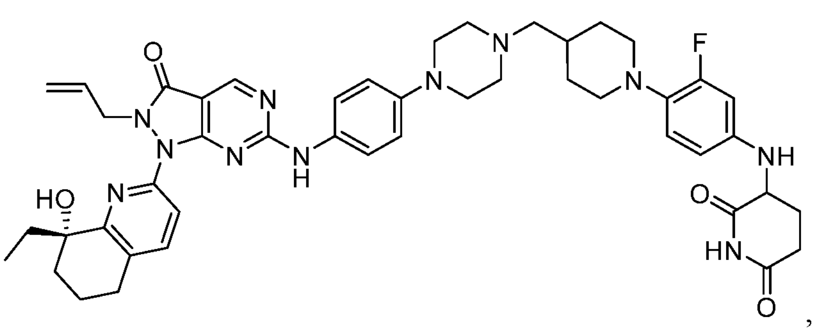

,

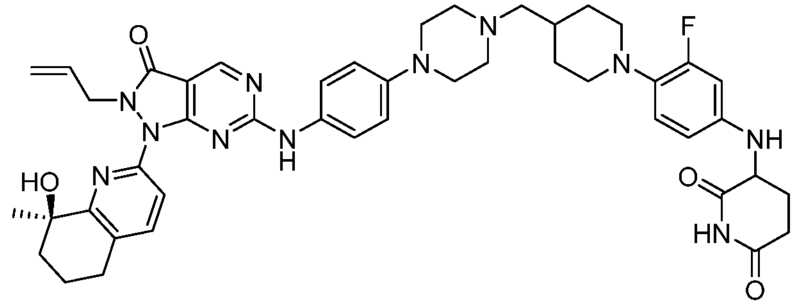

,

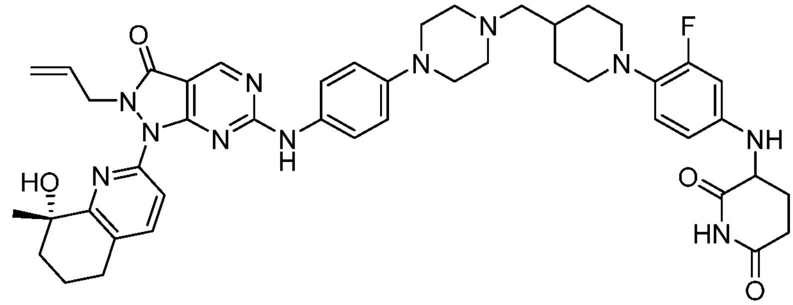

,

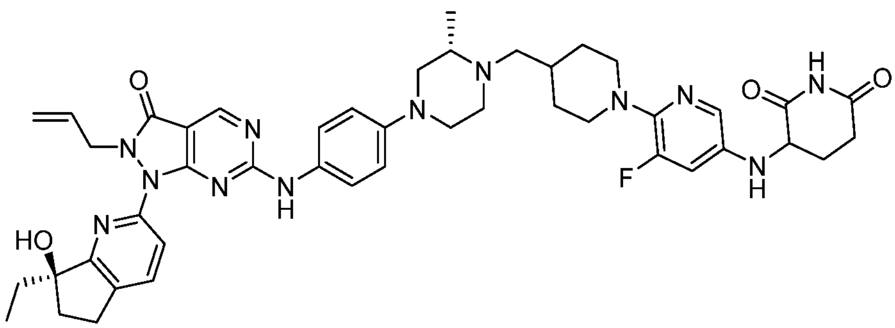

,

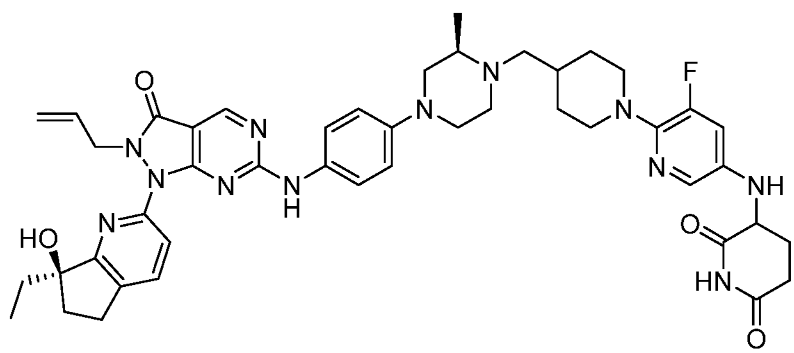

,

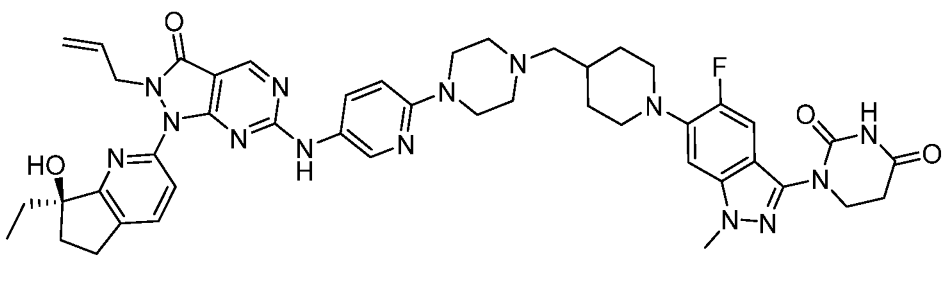

,

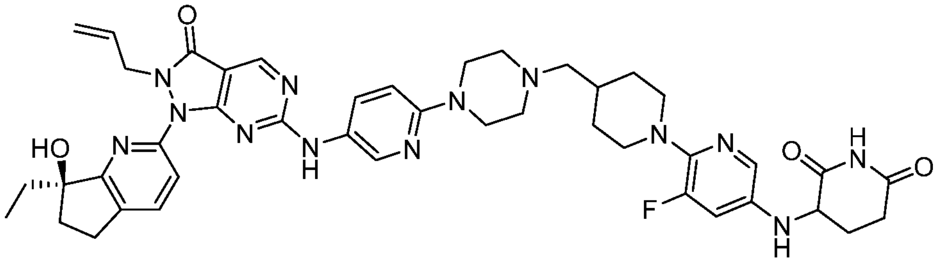

,

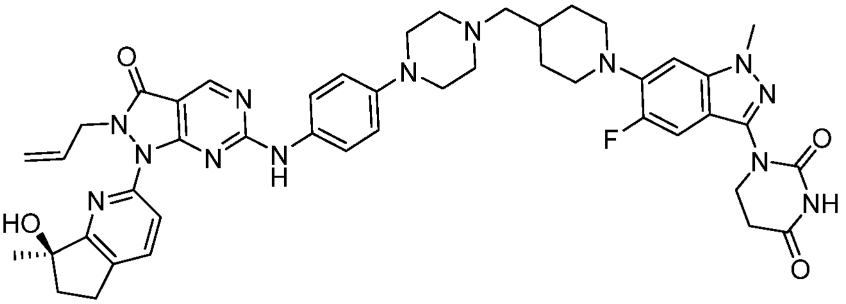

,

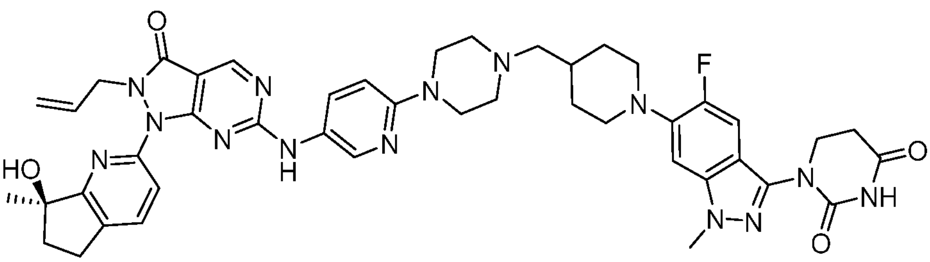

,

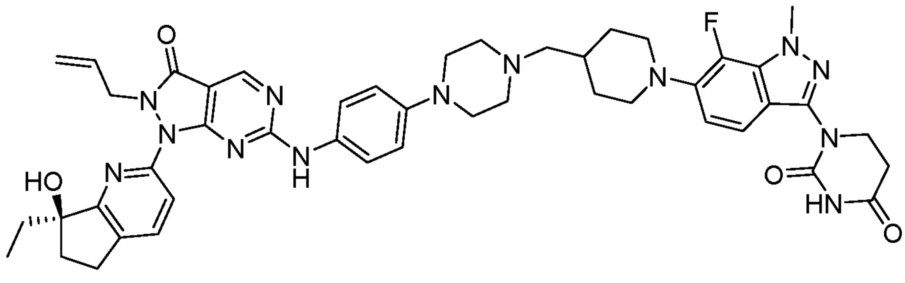

,

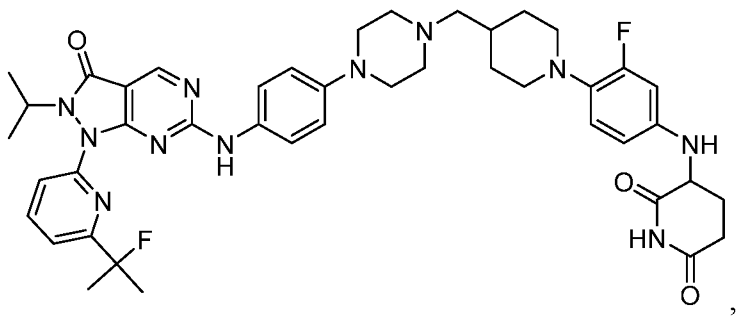

,

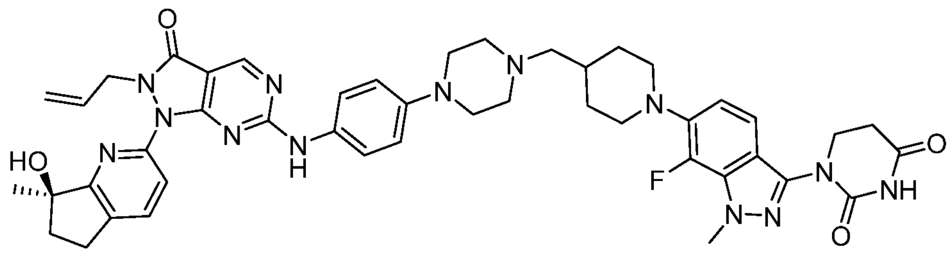

,

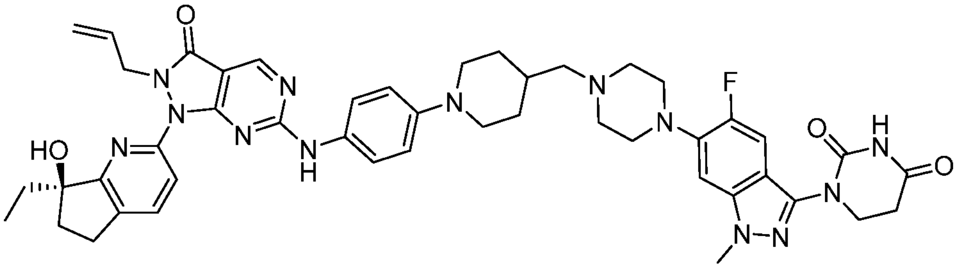

,

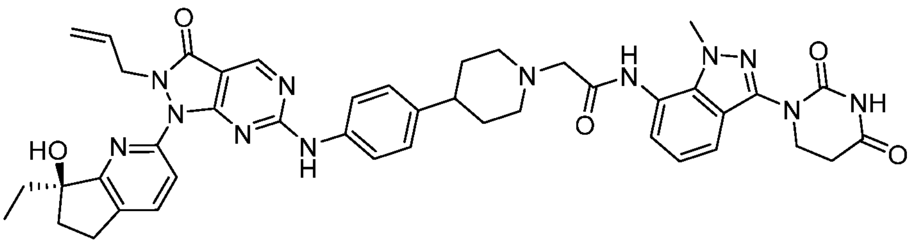

,

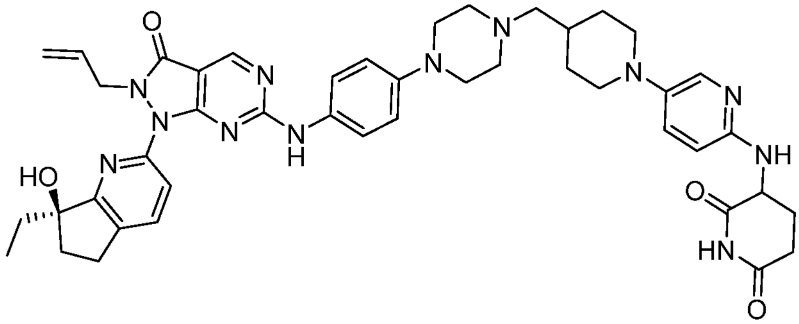

,

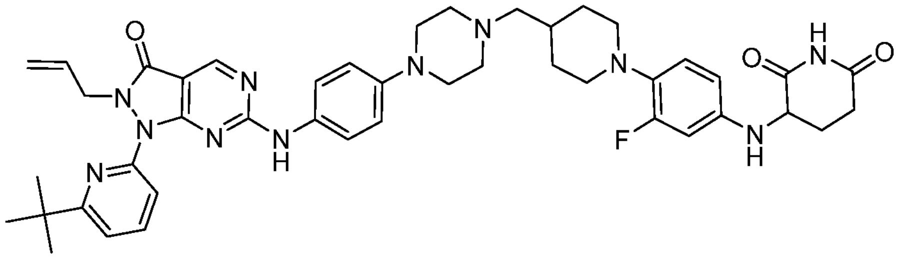

,

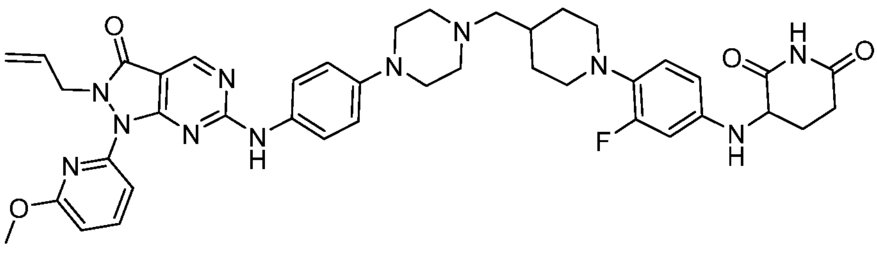

,

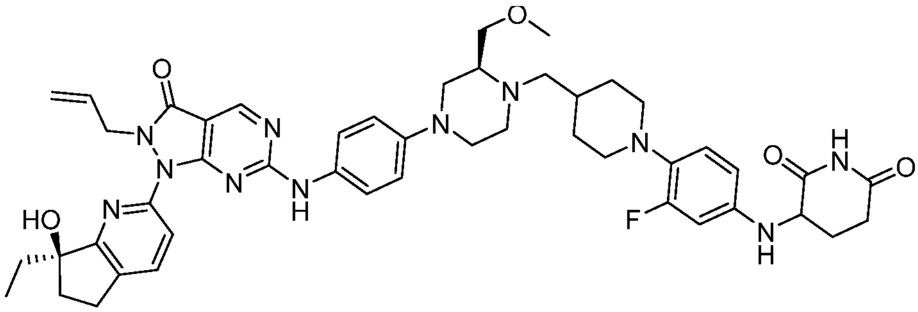

,

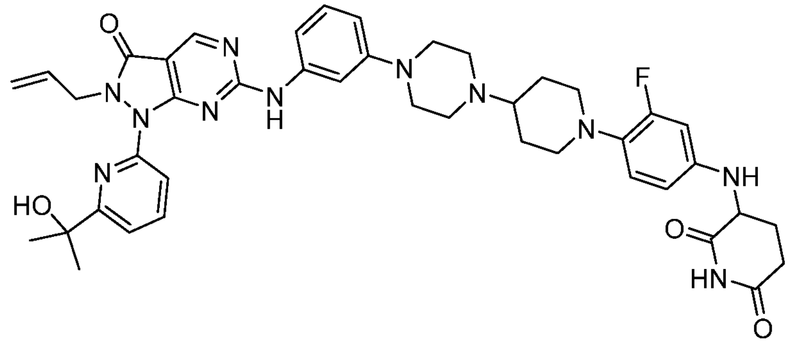

.

**74.** A pharmaceutical composition comprising the compound or a derivative, pharmaceutically acceptable salt, isomer, solvate, hydrate, adduct, complex or prodrug thereof according to any one of claims 1 to 73, and a pharmaceutically acceptable carrier.

**75.** The pharmaceutical composition according to claim 74, wherein the pharmaceutical composition is a tablet, a capsule, a granule, a syrup, a suspension, a solution, a dispersion, a slow release preparation for oral or non-oral administration, an intravenous injection preparation, a subcutaneous injection preparation, an inhalation preparation, a transdermal preparation, a rectal or vaginal suppository.

**76.** Use of the compound or a derivative, pharmaceutically acceptable salt, isomer, solvate, hydrate, adduct, complex or prodrug thereof according to any one of claims 1 to 73 in the manufacture of a medicament for the treatment of proliferative disorders.

**77.** The compound or a derivative, pharmaceutically acceptable salt, isomer, solvate, hydrate, adduct, complex or prodrug thereof according to any one of claims 1 to 73, for use in the treatment of a proliferative disorder.

**78.** A method of treating a proliferative disorder in a subject in need thereof, comprising administering to a subject in need thereof a therapeutically effective amount of the compound or a derivative, pharmaceutically acceptable salt, isomer, solvate, hydrate, adduct, complex or prodrug thereof according to any one of claims 1 to 73.

**79.** The use , the compound or a pharmaceutically acceptable salt thereof, or the method according to any one of claims 76 to 78, wherein the proliferative disorder comprises: breast cancer, colon cancer, brain cancer, prostate cancer, kidney cancer, pancreatic cancer, ovarian cancer, head and neck cancer, melanoma, colorectal cancer, gastric cancer, squamous cancer, small-cell lung cancer, non small-cell lung cancer, testicular cancer, Merkel cell carcinoma,

glioblastoma, neuroblastoma, cancers of lymphatic organs and hematological malignancy including Leukemia (Acute lymphoblastic leukemia (ALL), Acute myelogenous leukemia (AML), Chronic lymphocytic leukemia (CLL), Chronic myelogenous leukemia (CML), Acute monocytic leukemia (AMOL), Hairy cell leukemia (HCL), T-cell prolymphocytic leukemia (T-PLL), Large granular lymphocytic leukemia, Adult T-cell leukemia), Lymphoma (small lymphocytic lymphoma (SLL), Hodgkin's lymphomas (Nodular sclerosis, Mixed cellularity, Lymphocyte-rich, Lymphocyte depleted or not depleted, and Nodular lymphocyte-predominant Hodgkin lymphoma), Non-Hodgkin's lymphomas (all subtypes), Chronic lymphocytic leukemia/Small lymphocytic lymphoma, B-cell prolymphocytic leukemia, Lymphoplasmacytic lymphoma (such as Waldenström macroglobulinemia), Splenic marginal zone lymphoma, Plasma cell neoplasms (Plasma cell myeloma, Plasmacytoma, Monoclonal immunoglobulin deposition diseases, Heavy chain diseases), Extranodal marginal zone B cell lymphoma (MALT lymphoma), Nodal marginal zone B cell lymphoma (NMZL), Follicular lymphoma, Mantle cell lymphoma, Diffuse large B cell lymphoma, Mediastinal (thymic) large B cell lymphoma, Intravascular large B cell lymphoma, Primary effusion lymphoma, Burkitt lymphoma/leukemia, T cell prolymphocytic leukemia, T cell large granular lymphocytic leukemia, Aggressive NK cell leukemia, Adult T cell leukemia/lymphoma, Extranodal NK/T cell lymphoma (nasal type), Enteropathy-type T cell lymphoma, Hepatosplenic T cell lymphoma, Blastic NK cell lymphoma, Mycosis fungoides / Sezary syndrome, Primary cutaneous CD30-positive T cell lymphoproliferative disorders, Primary cutaneous anaplastic large cell lymphoma, Lymphomatoid papulosis, Angioimmunoblastic T cell lymphoma, Peripheral T cell lymphoma (unspecified), Anaplastic large cell lymphoma), multiple myeloma (plasma cell myeloma or Kahler's disease).

**80.** The use, the compound or a pharmaceutically acceptable salt thereof, or the method according to any one of claims 76 to 78, wherein the proliferative disorder comprises: acute lymphoblastic leukemia (ALL), acute myeloid leukemia (AML), multiple myeloma (MM), diffuse large B cell lymphoma (DLBCL), mantle cell lymphoma (MCL), malignant melanoma, gastric cancer, gliomas, ovarian carcinoma, uterine serous carcinoma (USC), colorectal cancer, pancreatic cancer, esophageal cancer, hepatocellular carcinoma, glioblastoma, non-small-cell lung cancer (NSCLC), small-cell lung cancer (SCLC), neuroblastoma, breast cancer, triple negative breast cancer (TNBC), neuroblastoma, head and neck squamous cell carcinoma (HNSCC).

## INTERNATIONAL SEARCH REPORT

International application No.
**PCT/CN2022/130700**

**A. CLASSIFICATION OF SUBJECT MATTER**

C07D 487/14(2006.01)i； C07D 471/14(2006.01)i； A61K 31/519(2006.01)i； A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNABS, CNTXT, SIPOABS, DWPI, WOTXT, EPTXT, USTXT, CNKI, 万方, WANFANG, 读秀, DUXIU, PUBMED, ISI_Web of Science, Science Direct, STNext: 杭州格博生物医药有限公司, 付利强, 结构检索, 增殖性疾病, structure search, proliferative, cancer

**C. DOCUMENTS CONSIDERED TO BE RELEVANT**

| Category* | Citation of document, with indication, where appropriate, of the relevant passages | Relevant to claim No. |
|---|---|---|
| PX | WO 2022251224 A1 (RECURIUM IP HOLDINGS, LLC) 01 December 2022 (2022-12-01)<br>description, paragraph 0120, and claims 1-63 | 1-80 |
| X | WO 2020069105 A1 (DANA-FARBER CANCER INSTITUTE, INC.) 02 April 2020 (2020-04-02)<br>description, page 53, line 3-page 54, line 2, page 58, lines 10-11, and page 66, line 1-page 68, line 14, and claims 1-18 | 1-80 |
| X | CN 113402520 A (SHANGHAI INSTITUTE OF MATERIA MEDICA, CHINESE ACADEMY OF SCIENCES) 17 September 2021 (2021-09-17)<br>claims 1-10 | 1-80 |
| Y | WO 2021127044 A1 (RECURIUM IP HOLDINGS, LLC) 24 June 2021 (2021-06-24)<br>claims 1-13, and figure 1 | 1-80 |
| Y | CN 111902413 A (RECURIUM IP HOLDINGS LLC) 06 November 2020 (2020-11-06)<br>claims 1-127 | 1-80 |
| Y | CN 103703005 A (ABBVIE INC.) 02 April 2014 (2014-04-02)<br>claims 1-20 | 1-80 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 January 2023** | **19 January 2023** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No. **PCT/CN2022/130700**

**C. DOCUMENTS CONSIDERED TO BE RELEVANT**

| Category* | Citation of document, with indication, where appropriate, of the relevant passages | Relevant to claim No. |
|---|---|---|
| Y | WO 2020259724 A2 (SHANGHAI PHARMACEUTICALS HOLDING CO., LTD.) 30 December 2020 (2020-12-30)<br>claims 1-25 | 1-80 |
| Y | WO 2021073491 A1 (SHANGHAI YINGPAI PHARMACEUTICAL CO., LTD.) 22 April 2021 (2021-04-22)<br>claims 1-15 | 1-80 |
| Y | CN 110603256 A (ALMAC DISCOVERY LIMITED) 20 December 2019 (2019-12-20)<br>claims 1-9 | 1-80 |
| Y | WO 2018090939 A1 (SHANGHAI YINGPAI PHARMACEUTICAL CO., LTD.) 24 May 2018 (2018-05-24)<br>claims 1-10 | 1-80 |

Form PCT/ISA/210 (second sheet) (January 2015)

**INTERNATIONAL SEARCH REPORT**

International application No.
**PCT/CN2022/130700**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **78，79**（部分），**80**（部分）
because they relate to subject matter not required to be searched by this Authority, namely:

[1] Claims 78, 79 (in part), and 80 (in part) set forth a method for treating a proliferative disease in a subject in need, which comprises a treatment plan for a human or animal body. Therefore, said claims do not comply with PCT Rule 39.1(iv). The search for claims 78, 79 (in part), and 80 (in part) was made on the basis of the following amendment: a use of the compound according to any one of claims 1-73 or derivative, pharmaceutically acceptable salt, isomer, solvate, hydrate, adduct, complex, or prodrug thereof in the preparation of a drug for treating a proliferative disease.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/130700**

| Patent document cited in search report | Publication date (day/month/year) | Patent family member(s) | Publication date (day/month/year) |
|---|---|---|---|
| WO 2022251224 A1 | 01 December 2022 | None | |
| WO 2020069105 A1 | 02 April 2020 | US 2022047709 A1 | 17 February 2022 |
| CN 113402520 A | 17 September 2021 | None | |
| WO 2021127044 A1 | 24 June 2021 | CA 3165472 A1 | 24 June 2021 |
| | | BR 112022012281 A2 | 30 August 2022 |
| | | AU 2020407070 A1 | 14 July 2022 |
| | | IL 294080 A | 01 August 2022 |
| | | KR 20220119442 A | 29 August 2022 |
| | | CN 115427042 A | 02 December 2022 |
| | | TW 202132299 A | 01 September 2021 |
| | | EP 4069235 A1 | 12 October 2022 |
| CN 111902413 A | 06 November 2020 | AU 2019231551 A1 | 20 August 2020 |
| | | CA 3089746 A1 | 12 September 2019 |
| | | EP 3762385 A1 | 13 January 2021 |
| | | BR 112020018286 A2 | 29 December 2020 |
| | | KR 20200130834 A | 20 November 2020 |
| | | IL 276802 A | 29 October 2020 |
| | | US 2021139482 A1 | 13 May 2021 |
| | | WO 2019173082 A1 | 12 September 2019 |
| | | SG 11202006906 X A | 29 September 2020 |
| | | US 2022024939 A1 | 27 January 2022 |
| | | RU 2020126590 A | 11 April 2022 |
| | | TW 202003512 A | 16 January 2020 |
| | | JP 2021516242 A | 01 July 2021 |
| CN 103703005 A | 02 April 2014 | UY 33925 A | 28 September 2012 |
| | | ES 2587514 T3 | 25 October 2016 |
| | | MX 2013009873 A | 12 March 2014 |
| | | JP 2014506929 A | 20 March 2014 |
| | | EP 2681221 A1 | 08 January 2014 |
| | | MX 327713 B | 06 February 2015 |
| | | WO 2012161812 A1 | 29 November 2012 |
| | | AR 085502 A1 | 09 October 2013 |
| | | CA 2827648 A1 | 29 November 2012 |
| | | TW 201247675 A | 01 December 2012 |
| | | US 2012220572 A1 | 30 August 2012 |
| WO 2020259724 A2 | 30 December 2020 | US 2022324868 A1 | 13 October 2022 |
| | | CO 2022000751 A2 | 29 April 2022 |
| | | BR 112021026620 A2 | 07 June 2022 |
| | | JP 2022539460 A | 09 September 2022 |
| | | IL 289395 A | 01 February 2022 |
| | | CL 2021003498 A1 | 19 August 2022 |
| | | EP 4008719 A2 | 08 June 2022 |
| | | CA 3145348 A1 | 30 December 2020 |
| | | AU 2020304472 A1 | 03 March 2022 |
| | | CN 112142748 A | 29 December 2020 |
| WO 2021073491 A1 | 22 April 2021 | CN 114502559 A | 13 May 2022 |
| CN 110603256 A | 20 December 2019 | JP 2020510040 A | 02 April 2020 |
| | | RS 62464 B1 | 30 November 2021 |
| | | PH 12019502049 A1 | 29 June 2020 |

Form PCT/ISA/210 (patent family annex) (January 2015)

**INTERNATIONAL SEARCH REPORT**
**Information on patent family members**

International application No.
**PCT/CN2022/130700**

| Patent document cited in search report | Publication date (day/month/year) | Patent family member(s) | Publication date (day/month/year) |
|---|---|---|---|
| | | PT 3592745 T | 20 October 2021 |
| | | AU 2018231671 A1 | 26 September 2019 |
| | | US 2021128560 A1 | 06 May 2021 |
| | | IL 269063 A | 28 November 2019 |
| | | EA 201992137 A1 | 05 February 2020 |
| | | MA 47736 A | 21 April 2021 |
| | | GB 201703881 D0 | 26 April 2017 |
| | | HR P20211652 T1 | 04 February 2022 |
| | | DK 3592745 T3 | 18 October 2021 |
| | | PL 3592745 T3 | 03 January 2022 |
| | | LT 3592745 T | 10 November 2021 |
| | | CA 3055874 A1 | 13 September 2018 |
| | | CL 2019002557 A1 | 27 December 2019 |
| | | US 2022194947 A1 | 23 June 2022 |
| | | KR 20190128673 A | 18 November 2019 |
| | | EP 3592745 A1 | 15 January 2020 |
| | | ES 2895365 T3 | 21 February 2022 |
| | | BR 112019018753 A2 | 07 April 2020 |
| | | MX 2019010554 A | 16 December 2019 |
| | | WO 2018162932 A1 | 13 September 2018 |
| | | SI 3592745 T1 | 31 December 2021 |
| | | SA 519410093 B1 | 16 July 2022 |
| | | SG 11201908114 U A | 30 October 2019 |
| | | UA 125093 C2 | 05 January 2022 |
| | | HU E056220 T2 | 28 February 2022 |
| WO 2018090939 A1 | 24 May 2018 | CA 3043945 A1 | 24 May 2018 |
| | | KR 20190099209 A | 26 August 2019 |
| | | US 2019308984 A1 | 10 October 2019 |
| | | EP 3543242 A1 | 25 September 2019 |
| | | JP 2020502065 A | 23 January 2020 |
| | | AU 2017359844 A1 | 27 June 2019 |
| | | US 2020385394 A1 | 10 December 2020 |
| | | CN 109906227 A | 18 June 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2019192668 A1
- WO 2018102725 A1


### Non-patent literature cited in the description


- **PAQUETTE, LEO A.** Principles of Modern Heterocyclic Chemistry. 1968
- The Chemistry of Heterocyclic Compounds, A series of Monographs. John Wiley & Sons, 1950
- *J. Am. Chem. Soc.,* 1960, vol. 82, 5566
- REMINGTON'S PHARMACEUTICAL SCIENCES. Mack Publishing Company, 1995